# EUROPEAN PATENT APPLICATION

(11) **EP 4 647 431 A1**
(43) Date of publication of application: **12.11.2025**
(21) Application number: 24738548.7
(22) Date of filing: 04.01.2024
(51) Int. Cl.: C07D 487/04, C07D 471/04, C07D 471/14, C07D 471/20, C07D 487/10, C07D 498/04, A61K 31/407, A61K 31/435, A61K 31/438, A61P 35/00

(54) **COMPOUND AND METHOD FOR TARGETED DEGRADATION OF ANDROGEN RECEPTOR**

(30) Priority: 04.01.2023 CN 202310021835; 28.07.2023 CN 202310939916
(71) Applicant: Gan & lee Pharm Co., Ltd., Beijing 101109 (CN)
(72) Inventor: CHEN, Wenmin, Beijing 101109 (CN); XU, Fuming, Beijing 101109 (CN); LIU, Xiaobing, Beijing 101109 (CN); MA, Yichao, Beijing 101109 (CN); LI, Xiaoguang, Beijing 101109 (CN); ZHAO, Qianqian, Beijing 101109 (CN); WANG, Kanghua, Beijing 101109 (CN)
(74) Representative: Kraus & Lederer PartGmbB
(86) International application number: PCT/CN2024/070694
(87) International publication number: WO 2024/146617

(57) **Abstract**

The present disclosure relates to a bifunctional compound suitable for degrading (and inhibiting) an androgen receptor. Specifically, the compound contains on one end a moiety that binds to E3 ubiquitin ligase and on the other end a moiety that binds to the androgen receptor, such that the androgen receptor is placed in proximity to the ubiquitin ligase to effect degradation (and inhibition) of the androgen receptor.

## Description

### Technical Field

The present disclosure relates to a bifunctional compound comprising an androgen receptor-binding moiety and an E3 ubiquitin ligase binding moiety, a pharmaceutical composition thereof, and pharmaceutical uses of the compound and pharmaceutical composition.

### Background

The concept of the Proteolysis Targeting Chimeras (PROTACs) was first proposed by Craig Crews et al. in 2001 (Proc. Natl. Acad. Sci. USA, 2001, 98, 8584). A PROTAC molecule is a bifunctional molecule that comprises an E3 ubiquitin ligase binding ligand at one terminus and a target protein-binding ligand at the other terminus, and the two parts are connected by a linker moiety. The linker moiety spatially positions the E3 ligase in proximity to the target protein, thereby inducing polyubiquitination of the target protein and its subsequent degradation by the proteasome. Compared to traditional small molecule drugs, PROTACs can achieve irreversible degradation of the target protein through transient binding sufficient for ubiquitin transfer, which makes PROTACs have the advantages of stronger degradation capability, longer-lasting efficacy, higher target selectivity, and the potential to overcome drug resistance caused by mutations in the target protein observed with traditional small molecule inhibitors.

Currently, the ligands of E3 ubiquitin ligases such as cereblon (CRBN), von Hippel-Lindau (VHL), mouse double minute 2 (MDM2), and inhibitor of apoptosis protein (IAP) are mainly used in the development of PROTAC technology. Among these, CRBN-based E3 ligase ligands are widely used, and the discovery of CRBN ligands is closely related to the study of thalidomide's mechanism of action. During the study of thalidomide's toxicity, researchers found that cereblon proteins are a type of thalidomide-binding proteins and part of the E3 ubiquitin ligase complex, which acts as a substrate receptor to selectively target ubiquitinated proteins. In addition, lenalidomide and pomalidomide, obtained by modification of thalidomide, have also been shown to bind cereblon proteins. Given CRBN ligands are widely used in the PROTAC field, and there is a pressing need to develop novel, highly selective CRBN ligands.

The androgen receptor (AR) is a steroid-induced transcription factor that regulates numerous genes involved in tumor progression (N. Lallous, Int. J. Mol. Sci. 14 (2013), 12496-12519). Prostate cancer is a typical AR-driven disease. Androgen deprivation therapy (ADT) is one of the conventional treatments for prostate cancer, such as surgical castration (bilateral orchiectomy) or pharmacological castration (e.g., goserelin injection). ADT demonstrates significant efficacy in the initial treatment phase. However, as the disease progresses, mutations may occur in the AR gene, and the mutated ARs become hypersensitive to low levels of androgens, thereby driving the progression of the disease into castration-resistant prostate cancer (CRPC). Currently approved oral drugs for the treatment of metastatic CRPC mainly include abiraterone and enzalutamide. Abiraterone is a novel androgen biosynthesis inhibitor, and enzalutamide is an androgen receptor inhibitor that competitively inhibits the binding of androgens to the receptor. However, these drugs become ineffective in advanced prostate cancer characterized by AR gene amplification, mutations, and alternative splicing (Lottrup, G.; J. Clin. Endocrinol. Metab. 2013, 98, 2223-2229).

The present disclosure is primarily based on the Proteolysis Targeting Chimeras (PROTACs) technology and provides a class of Selective Androgen Receptor Degraders (SARDs). These SARDs not only inhibit the androgen receptor and block its signaling transduction pathway, but also induce degradation of the receptor itself, which may provide prostate cancer patients with more benefits than known AR inhibitors.

### Summary

As described in the background, the present disclosure is based on the Proteolysis Targeting Chimera (PROTAC) technology, and provides a class of selective Androgen Receptor Degraders (SARDs). These compounds may not only inhibit the androgen receptor and block its signaling transduction pathway, but also induce degradation of the receptor itself, offering greater therapeutic benefits for prostate cancer patients compared to known androgen receptor inhibitors.

Accordingly, in one aspect, the present disclosure provides a compound having the structure of formula I:

PTM-L-CLM (formula I),

or an isomer, an isotopic derivative, a polymorph, a prodrug, or a pharmaceutically acceptable salt or a solvate thereof,
wherein:
   PTM is a moiety that binds to the androgen receptor;
   L is a bond or a chemical linker moiety that covalently connects CLM and the PTM, and has a structure of -(B^{L})_{q}-;
   each occurrence of B ^{L} is identical or different and is each independently selected from: a covalent bond, CR^{L1}R^{L2}, O, S, SO, SO₂, NR^{L3}, CO, SiR^{L1}R^{L2}, P(O)R^{L1}, P(O)OR^{L1}, C(=NCN), C(= CNO₂), C₂-C₆ alkenylene, C₂-C₆ alkynylene, C₃-C₁₁ cycloalkylene optionally substituted with 0-6 R^{L1} and/or R^{L2} groups, C₃-C₁₁ heterocyclylene optionally substituted with 0-6 R^{L1} and/or R^{L2} groups, arylene optionally substituted with 0-6 R^{L1} and/or R^{L2} groups, heteroarylene optionally substituted with 0-6 R^{L1} and/or R^{L2} groups, C₆-C₁₆ spirocyclylene optionally substituted with 0-6 R^{L1} and/or R^{L2} groups, and C₆-C₁₆ heterospirocyclylene optionally substituted with 0-6 R^{L1} and/or R^{L2} groups; preferably,
   R^{L1}, R^{L2}, and R^{L3} are each independently H, halogen, alkyl, haloalkyl, alkoxy, haloalkoxy, alkenyl, haloalkenyl, alkynyl, haloalkynyl, SR^{L4}, NR^{L4}R^{L5}, cycloalkyl, aryl, heteroaryl, heterocyclyl, OR^{L4}, OH, SO₂-R^{L4}, P(O)R^{L4}R^{L5}, Si(OH)₃, SiR^{L4}R^{L5}R^{L6}, COR^{L6}, CN, NO₂, SF₅, SO₂NR^{L4}R^{L5}, CONR^{L4}R^{L5}, - COOR^{L4}, N(R^{L4})CONR^{L5}R^{L4}, or N(R^{L4})SO₂NR^{L4}R^{L5};
   R^{L4} and R^{L5} are each independently H, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl;
   R^{L6} is each independently H, OH, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl; and
   q is an integer greater than or equal to 1;
   the CLM is a cereblon E3 ubiquitin ligase binding moiety, selected from the following structures: 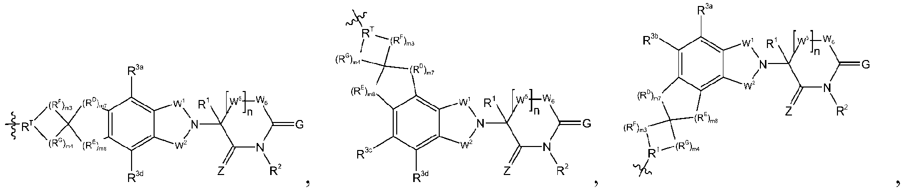 and 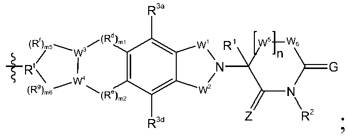 wherein:
      W¹ and W² are identical or different, each independently being CR^{a}R^{b}, C(=O), NR^{a}, or SO₂, and at least one of W¹ and W² is C(=O);
      G and Z are identical or different and are each independently selected from O, S, and Se;
      R^{3a}, R^{3b}, R^{3c}, and R^{3d} are each independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
      each occurrence of W⁵, W⁶, R^{d}, R^{e}, R^{f}, R^{g}, R^{D}, R^{E}, R^{F}, and R^{G} are each independently C(R^{m})₂, NR^{m}, O, or S;
      W³ and W⁴ are each independently CR^{m} or N;
      R^{t} and R^{T} are each independently N or CR^{2h}, and when all of R^{D}, R^{E}, R^{F}, and R^{G} are C(R^{m})₂, R^{T} are CR^{2h};
      m1 and m2 are each independently an integer of 0, 1, 2, 3, 4, 5, or 6, and m1+ m2≤6;
      m3 is an integer of 0, 1, 2, 3, 4, 5, 6, or 7, m4 is an integer of 1, 2, 3, 4, 5, 6, 7, or 8, and m3+m4≤8;
      m5 and m6 are each independently an integer of 0, 1, 2, 3, 4, 5, 6, or 7, and m5+ m6≤7;
      m7 and m8 are each independently an integer of 0, 1, 2, 3, 4, 5, 6, or 7, and m7+ m8≤7;
      each occurrence of R^{m} is independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, alkylacyl, alkoxycarbonyl, alkylaminocarbonyl, -C₁₋₆ alkylene -ONH₂, -NHO-C₁₋₆ alkyl, -C₁₋₆ alkylene -NH-C₁₋₆ alkylene -ONH₂, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
      R^{2h} is selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
      R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl and C₁-C₆ hydroxyalkyl; preferably, R¹ is H, F, Cl, Br, I, C₁-C₃ alkyl, or hydroxyl;
      R², R^{a}, and R^{b} are each independently selected from H, deuterium, F, Cl, Br, I, and C₁-C₆ alkyl, C₁-C₆ alkoxy, hydroxyl, C₁-C₆ haloalkyl, and C₁-C₆ hydroxyalkyl; and
      n is 0, 1, 2, or 3.

In one embodiment, the compound having the structure of formula I:

PTM-L-CLM (formula I),

or an isomer, an isotopic derivative, a polymorph, a prodrug, or a pharmaceutically acceptable salt or a solvate thereof,
wherein:
   PTM is a moiety that binds to the androgen receptor;
   L is a bond or a chemical linker moiety that covalently connects CLM and PTM, and has a structure of -(B^{L})_{q}-;
   each occurrence of B ^{L} is identical or different and is each independently selected from a covalent bond, CR^{L1}R^{L2}, O, S, SO, SO₂, NR^{L3}, CO, SiR^{L1}R^{L2}, P(O)R^{L1}, P(O)OR^{L1}, C(=NCN), C(=CNO₂), C₃-C₁₁ cycloalkylene optionally substituted with 0-6 R^{L1} and/or R^{L2} groups, C₃-C₁₁ heterocyclylene optionally substituted with 0-6 R^{L1} and/or R^{L2} groups, arylene optionally substituted with 0-6 R^{L1} and/or R^{L2} groups, heteroarylene optionally substituted with 0-6 R^{L1} and/or R^{L2} groups, C₆-C₁₆ spirocyclylene optionally substituted with 0-6 R^{L1} and/or R^{L2} groups, and C₆-C₁₆ heterospirocyclylene optionally substituted with 0-6 R^{L1} and/or R^{L2} groups;
   R^{L1}, R^{L2}, and R^{L3} are each independently H, halogen, alkyl, haloalkyl, alkoxy, haloalkoxy, alkenyl, haloalkenyl, alkynyl, haloalkynyl, SR^{L4}, NR^{L4}R^{L5}, cycloalkyl, aryl, heteroaryl, heterocyclyl, OR^{L4}, OH, SO₂-R^{L4}, P(O)R^{L4}R^{L5}, Si(OH)₃, Si R^{L4}R^{L5}R^{L6}, COR^{L6}, CN, NO₂, SF₅, SO₂NR^{L4}R^{L5}, CONR^{L4}R^{L5}, N(R^{L4})CONR^{L5}R^{L4}, or N(R^{L4})SO₂NR^{L4}R^{L5};
   R^{L4} and R^{L5} are each independently H, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl; R^{L6} is each independently H, OH, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl;
   q is an integer greater than or equal to 1; preferably, q is 1, 2, 3, 4, 5, 6, 7, 8, 9, 10, 11, 12, 13, 14, 15, 16, 17, 18, 19, or 20;
   the CLM is a cereblon E3 ubiquitin ligase binding moiety, selected from the following structures: 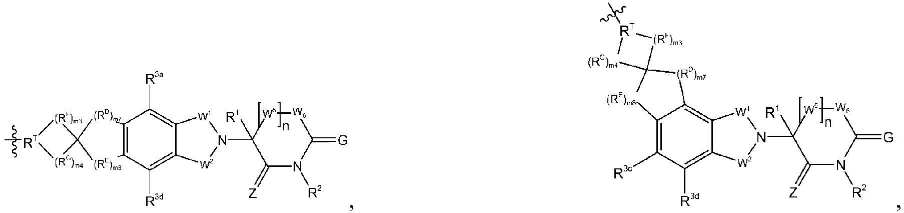 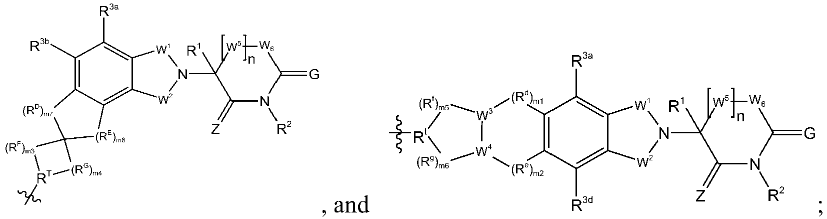
   wherein:
      W¹ and W² are identical or different, each independently being CR^{a}R^{b}, C(=O), NR^{a}, or SO₂, and at least one of W¹ and W² is C(=O);
      G and Z are identical or different and are each independently selected from O, S, and Se;
      R^{3a}, R^{3b}, R^{3c}, and R^{3d} are each independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
      each occurrence of W⁵, W⁶, R^{d}, R^{e}, R^{f}, R^{g}, R^{D}, R^{E}, R^{F}, and R^{G} are each independently C(R^{m})₂, NR^{m}, O, or S;
      W³ and W⁴ are each independently CR^{m} or N;
      R^{t} and R^{T} are each independently N or CR^{2h}, and when all of R^{D}, R^{E}, R^{F}, and R^{G} are C(R^{m})₂, R^{T} is CR^{2h};
      m1 and m2 are each independently an integer of 0, 1, 2, 3, 4, 5, or 6, and m1+ m2≤6;
      m3 is an integer of 0, 1, 2, 3, 4, 5, 6, or 7, m4 is an integer of 1, 2, 3, 4, 5, 6, 7, or 8, and m3+m4≤8;
      m5 and m6 are each independently an integer of 0, 1, 2, 3, 4, 5, 6, or 7, and m5+ m6≤7;
      m7 and m8 are each independently an integer of 0, 1, 2, 3, 4, 5, 6, or 7, and m7+ m8≤7;
      each occurrence of R^{m} is independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, alkylacyl, alkoxycarbonyl , alkylaminocarbonyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
      R^{2h} is selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
      R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl and C₁-C₆ hydroxyalkyl; preferably, R¹ is H, F, Cl, Br, I, C₁-C₃ alkyl, or hydroxyl;
      R², R^{a}, and R^{b} are each independently selected from H, deuterium, F, Cl, Br, I, C₁-C₆ alkyl, and C₁-C₆ alkoxy, hydroxyl, C₁-C₆ haloalkyl, and C₁-C₆ hydroxyalkyl; and
      n is 0, 1, 2, or 3.
      In one embodiment, W¹ and W² are identical or different, each independently being CH₂ or C(=O), and at least one of W¹ and W² is C(=O); and/or
      G is O; and/or
      Z is O; and/or
      R^{3a}, R^{3b}, R^{3c}, and R^{3d} are each independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₅-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₅-C₁₀ heteroaryl; preferably, R^{3a}, R^{3b}, R^{3c}, and R^{3d} are each independently selected from H, deuterium, F, Cl, Br, I, C₁-C₃ alkyl, and C₁-C₃ alkoxy; and/or
      each occurrence of R^{d}, R^{e}, R^{D}, and R^{E} are each independently C(R^{m})₂ or O; and/or
      each occurrence of R^{f}, R^{g}, R^{F}, and R^{G} are each independently C(R^{m})₂ or O, and preferably C(R^{m})₂; and/or
      W³ and W⁴ are CH; and/or
      W⁵ and W⁶ are C(R^{m})₂ or N(R^{m}), and preferably CH₂, CH (C₁-C₆ alkyl), CH (C₁-C₆ haloalkyl), CH(OH), or NH;
      R^{2h} is selected from H, deuterium, F, Cl, Br, I, C₁-C₆ alkyl, C₁-C₆ deuterated alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₅-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₅-C₁₀ heteroaryl; preferably, R^{2h} is selected from H, deuterium, F, Cl, Br, I, C₁-C₃ alkyl, and C₁-C₃ alkoxy; and/or
      m1 and m2 are each independently an integer of 0, 1, 2, or 3, and m1+ m2≤3, preferably, m1+m2=1 or m1+m2=2; and/or
      m3 is an integer of 0, 1, 2, 3, or 4, m4 is an integer of 1, 2, 3, 4, or 5, and m3+m4≤5, preferably, m3+m4=2, m3+m4=3, or m3+m4=4; and/or
      each occurrence of m5 and m6 are each independently an integer of 0, 1, 2, 3, or 4, and m5+ m6≤4, preferably, m5+m6=2 or m5+m6=3;
      each occurrence of m7 and m8 are each independently an integer of 0, 1, 2, 3, or 4, and m7+ m8≤4, preferably, m7+m8=2 or m7+m8=3; and /or
      each occurrence of R^{m} is independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₁-C₆ alkylacyl, C₁-C₆ alkoxycarbonyl , ,, C₁-C₆ alkylaminocarbonyl , -C₁₋₆ alkylene -ONH₂, -NHO-C₁₋₆ alkyl, -C₁₋₆ alkylene -NH-C₁₋₆ alkylene - ONH₂, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₅-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₅-C₁₀ heteroaryl; preferably, each occurrence of R^{m} is independently selected from H, deuterium, F, Cl, Br, I, C₁-C₃ alkyl, and C₁-C₃ alkoxy; and/or
      R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl, and C₁-C₆ hydroxyalkyl, preferably R¹ is H, F, Cl, Br, I, C₁-C₃ alkyl, or hydroxyl; and/or
      R² is selected from H, deuterium, F, Cl, Br, I, C₁-C₆ alkyl, C₁-C₆ alkoxy, hydroxyl, C₁-C₆ haloalkyl and C₁-C₆ hydroxyalkyl, preferably R² is H, F, Cl, Br, I, C₁-C₃ alkyl or hydroxyl; and /or
      n is 0 or 1.

In one embodiment, wherein:
W¹ and W² are identical or different, each independently being CH₂ or C(=O), and at least one of W¹ and W² is C(=O); and/or
G is O; and /or
Z is O; and /or
R^{3a}, R^{3b}, R^{3c}, and R^{3d} are each independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₅-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₅-C₁₀ heteroaryl; preferably, R^{3a}, R^{3b}, R^{3c}, and R^{3d} are each independently selected from H, deuterium, F, Cl, Br, I, C₁-C₃ alkyl, and C₁-C₃ alkoxy; and/or
each occurrence of R^{d}, R^{e}, R^{D}, and R^{E} are each independently C(R^{m})₂ or O; and/or
each occurrence of R^{f}, R^{g}, R^{F}, and R^{G} are each independently C(R^{m})₂ or O,and preferably C(R^{m})₂; and/or
W³ and W⁴ are CH; and/or
W⁵ and W⁶ are C(R^{m})₂ or N(R^{m}), and preferably CH₂, CH (C₁-C₆ alkyl), CH (C₁-C₆ haloalkyl), CH(OH), or NH;
R^{2h} is selected from H, deuterium, F, Cl, Br, I, C₁-C₆ alkyl, C₁-C₆ deuterated alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl and C₅-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₅-C₁₀ heteroaryl; preferably, R^{2h} is selected from H, deuterium, F, Cl, Br, I, C₁-C₃ alkyl, and C₁-C₃ alkoxy; and/or
m1 and m2 are each independently an integer of 0, 1, 2, or 3, and m1+ m2≤3, preferably, m1+m2=1 or m1+m2=2; and/or
m3 is an integer of 0, 1, 2, 3, or 4, m4 is an integer of 1, 2, 3, 4, or 5, and m3+m4≤5, preferably, m3+m4=2, m3+m4=3, or m3+m4=4; and/or
each occurrence of m5 and m6 are each independently an integer of 0, 1, 2, 3, or 4, and m5+ m6≤4, preferably, m5+m6=2 or m5+m6=3;
each occurrence of m7 and m8 are each independently an integer of 0, 1, 2, 3, or 4, and m7+ m8≤4, preferably, m7+m8=2 or m7+m8=3; and /or
each occurrence of R^{m} is independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₁-C₆ alkylacyl, C₁-C₆ alkoxycarbonyl , C₁-C₆ alkylaminocarbonyl, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₅-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₅-C₁₀ heteroaryl; preferably, each occurrence of R^{m} is independently selected from H, deuterium, F, Cl, Br, I, C₁-C₃ alkyl, and C₁-C₃ alkoxy; and/or
R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl, and C₁-C₆ hydroxyalkyl, preferably R¹ is H, F, Cl, Br, I, C₁-C₃ alkyl, or hydroxyl; and/or
R² is selected from H, deuterium, F, Cl, Br, I, C₁-C₆ alkyl, and C₁-C₆ alkoxy, hydroxyl, C₁-C₆ haloalkyl and C₁-C₆ hydroxyalkyl; preferably R² is H, F, Cl, Br, I, C₁-C₃ alkyl, or hydroxyl; and/or
n is 0 or 1.

In one embodiment,wherein, the CLM is selected from the following structures: 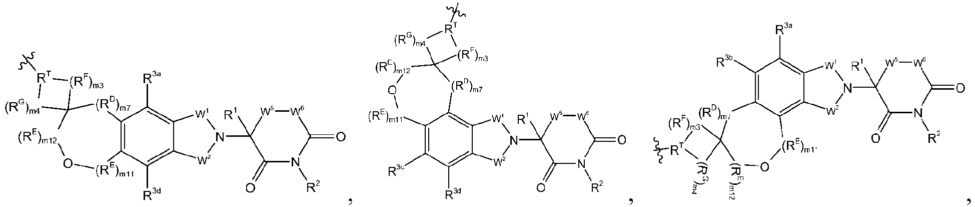 and 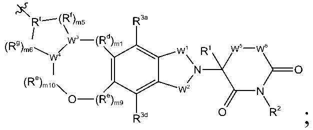 preferably, the CLM is the following structures: 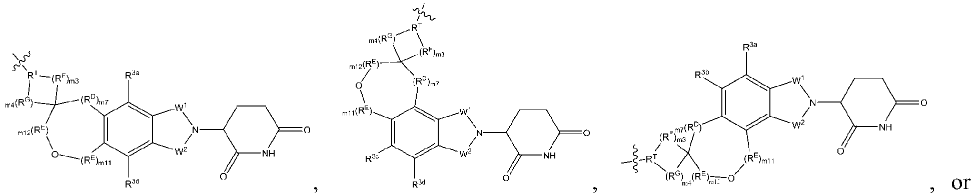 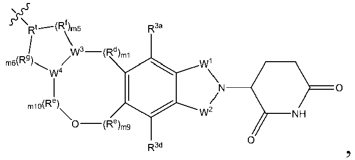 wherein:
W¹, W², W³, W⁴, W⁵, W⁶, R^{3a}, R^{3b}, R^{3c}, R^{3d}, R^{d}, R^{e}, R^{f}, R^{t}, R^{g}, R^{D}, R^{E}, R^{F}, R^{T}, R^{G}, R¹, R², m3, m4, m5, and m6 are as defined in claim 1, 2, 3, or 4;
m1, m9, and m10 are each independently an integer of 0, 1, 2, 3, 4, or 5, and m1+m9+m10≤5; preferably, m1, m9, and m10 are each independently an integer of 0, 1, or 2, and m1+m9+m10≤2; preferably, m1+m9+m10=1 or m1+m9+m10=0; and
m7, m11, and m12 are each independently an integer of 0, 1, 2, 3, 4, 5, or 6, and m7+m11+m12≤6; preferably, m7, m11, and m12 are each independently an integer of 0, 1, 2, or 3, and m7+m11+m12≤3; preferably, m7+m11+m12=1 or m7+m11+m 12=2.

In one embodiment, the CLM is selected from the following structures: 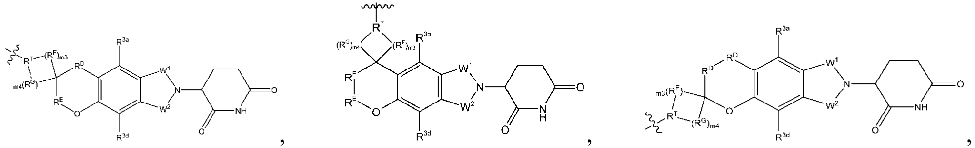 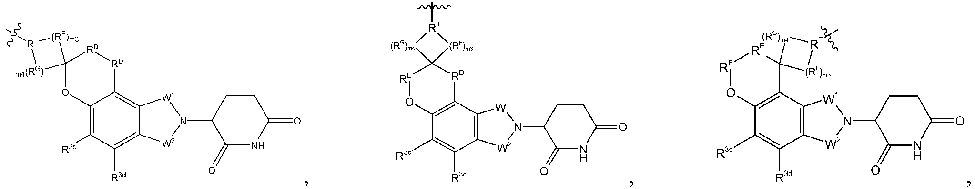 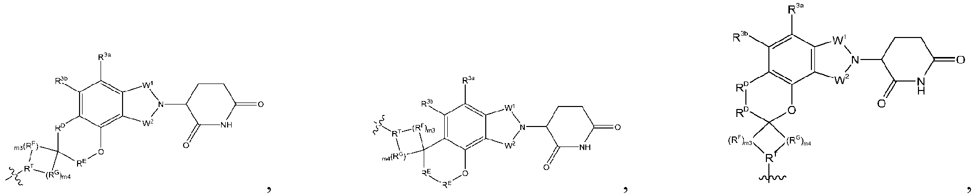 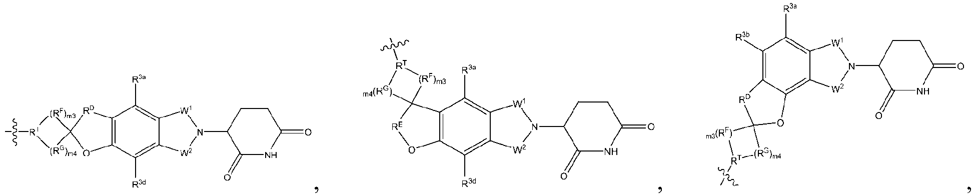 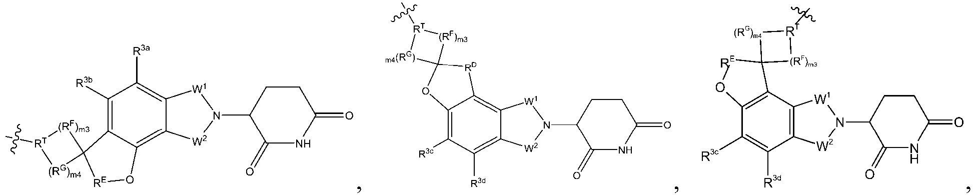 and

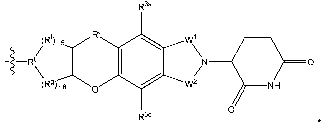

In one embodiment, wherein the CLM is selected from the following structures, or an isomer, an isotopic derivative, a polymorph, a prodrug, or a pharmaceutically acceptable salt or a solvate thereof: 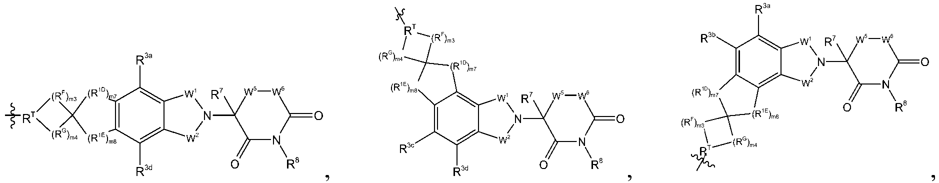 and 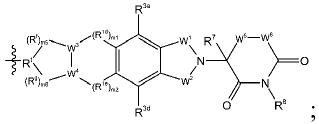 preferably, the CLM is the following structures: 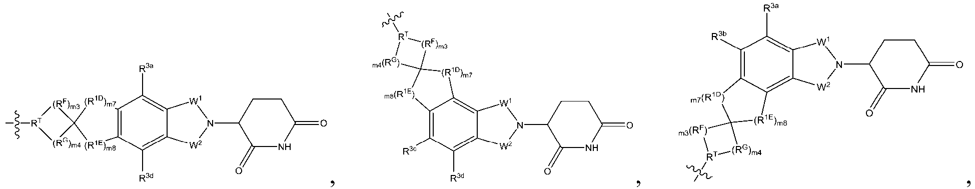 or 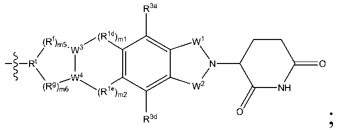 wherein:
W¹, W², W³, W⁴, W⁵, W⁶, R^{3a}, R^{3b}, R^{3c}, R^{3d}, R^{f}, R^{g}, R^{t}, R^{F}, R^{G}, R⁷, R⁸, m1, m2, m3, m4, m5, m6, m7, and m8 are as defined in claim 1, 2, 3, or 4; and
each occurrence of R^{1d}, R^{1e}, R^{1D}, and R^{1E} is independently C(R^{m})₂; and
R^{T} is N or CR^{2h}, and when R^{F} and R^{G} are both C(R^{m})₂, R^{T} is CR^{2h}; and
R^{2h} and R^{m} are as defined previously.

In one embodiment, the CLM is selected from the following structures: 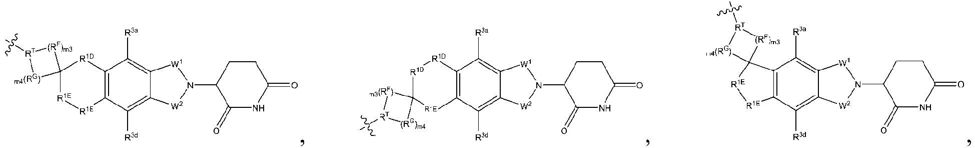 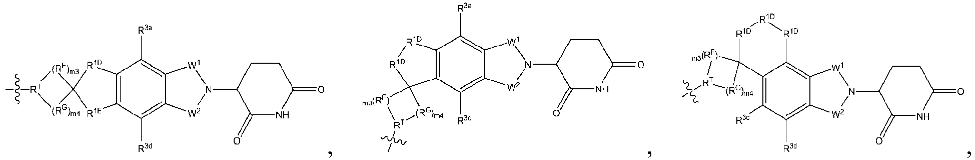 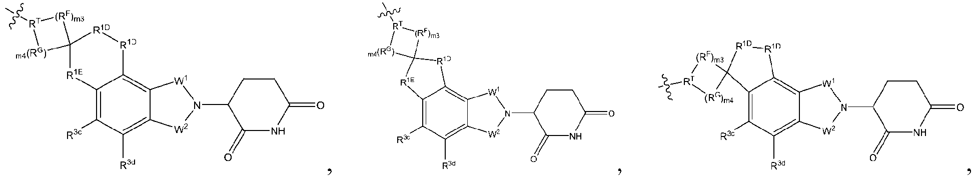 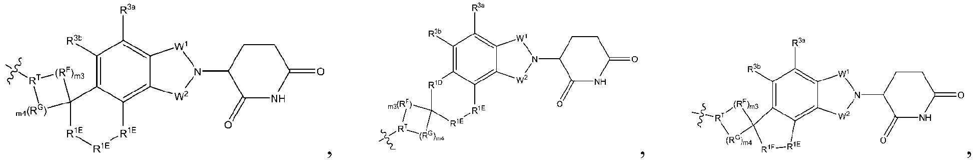 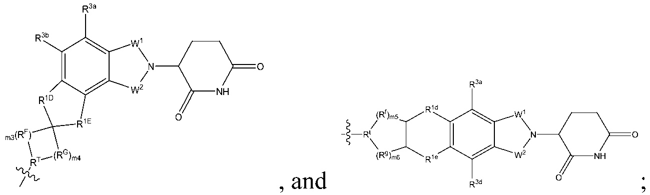 wherein W¹, W², R^{3a}, R^{3b}, R^{3c}, R^{3d}, R^{f}, R^{t}, R^{g}, R^{F}, R^{T}, R^{G}, R^{1d}, R^{1e}, R^{1D}, R^{1E}, m3, m4, m5, and m6 are as defined previously.

In one embodiment, wherein: each occurrence of B^{L} is identical or different and is each independently selected from: a covalent bond, CR^{L1}R^{L2}, O, S, SO, SO₂, NR^{L3}, CO, C₂₋₆ alkenylene, C₂₋₆ alkynylene, cycloalkylene, heterocyclylene, spirocyclylene, heterospirocyclylene, arylene, and heteroarylene, wherein the cycloalkylene, heterocyclylene, spirocyclylene, heterospirocyclylene, arylene, and heteroarylene are optionally substituted with 0-6 R^{L1} and/or R^{L2} groups; preferably, each occurrence of B^{L} is identical or different and is independently selected from a covalent bond, CR^{L1}R^{L2}, O, S, SO, SO₂, NR^{L3}, CO, cycloalkylene, heterocyclylene, spirocyclylene, heterospirocyclylene, arylene, and heteroarylene, wherein the cycloalkylene, heterocyclylene, spirocyclylene, heterospirocyclylene, arylene, and heteroarylene are optionally substituted with 0-6 R^{L1} and/or R^{L2} groups; and/or
each occurrence of R^{L1}, R^{L2}, and R^{L3} are each independently selected from H, F, Cl, Br, I, C₁₋₈ alkyl, C₃₋₁₁ cycloalkyl, C₃₋₁₁ heterocyclyl, C₆₋₁₀ aryl, C₅₋₁₀ heteroaryl, C₁-₈ alkoxy, C₁₋₈ alkylene-O-C₁₋₈ alkyl, C₁₋₈ alkylene C₃₋₁₁ cycloalkyl, -O-C₃₋₈ cycloalkyl, -O-C₃₋₁₁ heterocyclyl, -O- aryl, -O-heteroaryl, -NH-C₁₋₈ alkyl, -N(C₁₋₈ alkyl)₂, -NH-C₃₋₈ cycloalkyl, -N (C₃₋₈ cycloalkyl)₂, -N(C₃₋₈ cycloalkyl)(C₁₋₈ alkyl), -NH-C₃₋₈ heterocyclyl, -N (C₃₋₈ heterocyclyl)₂, -N(C₃₋₈ heterocyclyl)(C₁₋₈ alkyl), -NH- aryl, -N(aryl)(C₁₋₈ alkyl), -NH- heteroaryl, -N(heteroaryl)(C₁₋₈ alkyl), -OH, -NH₂, -CO-C₁₋₈ alkyl, -CO₂H, -CN, -CF₃, -CHF₂, -CH₂F, -NO₂, -CONH-C₁₋₈ alkyl, -CON(C₁₋₈ alkyl)₂, -N(C₁₋₈ alkyl)CONH(C₁₋₈ alkyl), -N(C₁₋₈ alkyl)CON(C₁₋₈ alkyl)₂, -NHCONH(C₁₋₈ alkyl), -NHCON(C₁₋₈ alkyl)₂, -NHCONH₂, and -COO-C₁₋₈ alkyl, wherein the alkyl, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl; preferably, each occurrence of R^{L1}, R^{L2}, and R^{L3} is independently selected from H, F, Cl, Br, I, C₁₋₈ alkyl, C₃₋₁₁ cycloalkyl, C₃₋₁₁ heterocyclyl, C₆₋₁₀ aryl, C₅₋₁₀ heteroaryl, C₁-₈ alkoxy, O-C₃₋₈ cycloalkyl, O-C₃₋₁₁ heterocyclyl, O- aryl, O- heteroaryl, NH-C₁₋₈ alkyl, N(C₁₋₈ alkyl)₂, NH-C₃₋₈ cycloalkyl, N (C₃₋₈ cycloalkyl)₂, N(C₃₋₈ cycloalkyl)(C₁₋₈ alkyl), NH-C₃₋₈ heterocyclyl, N (C₃₋₈ heterocyclyl)₂, N(C₃₋₈ heterocyclyl)(C₁₋₈ alkyl), NH- aryl, N(aryl)(C₁₋₈ alkyl), NH- heteroaryl, N(heteroaryl)(C₁₋₈ alkyl), OH, NH₂, CO-C₁₋₈ alkyl, CO₂H, CN, CF₃, CHF₂, CH₂F, NO₂, CONH-C₁₋₈ alkyl, CON(C₁₋₈ alkyl)₂, N(C₁₋₈ alkyl)CONH(C₁₋₈ alkyl), N(C₁₋₈ alkyl)CON(C₁₋₈ alkyl)₂, NHCONH(C₁₋₈ alkyl), NHCON(C₁₋₈ alkyl)₂, and NHCONH₂, wherein the alkyl, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl; and/or
q is an integer greater than or equal to 1; preferably 1, 2, 3, 4, 5, 6, 7, 8, 9, 10, 11, 12, 13, 14, 15, 16, 17, 18, 19, or 20.

In one embodiment, B^{L} is selected from one or more of the following structures: a covalent bond, -O-, -(CH₂)ₖ-, -CO-, -NH-, 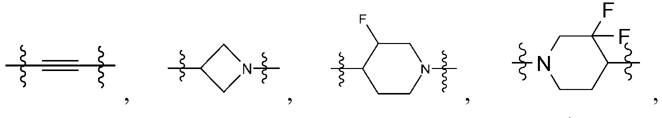 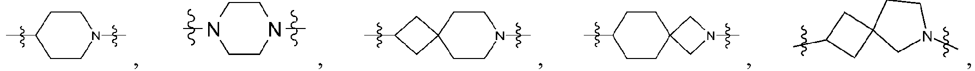 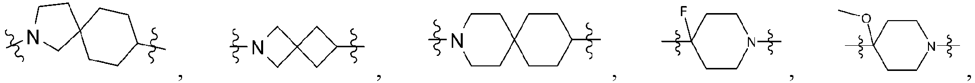 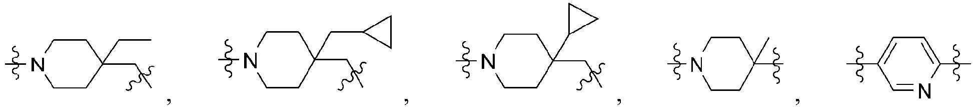 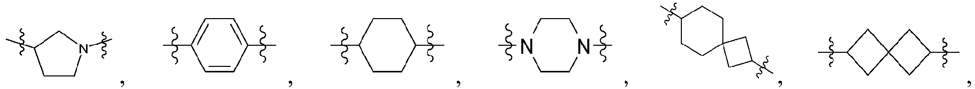 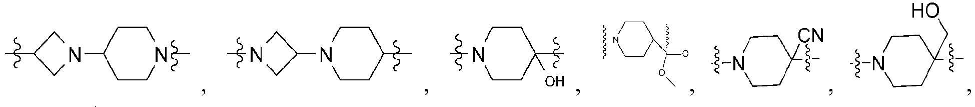 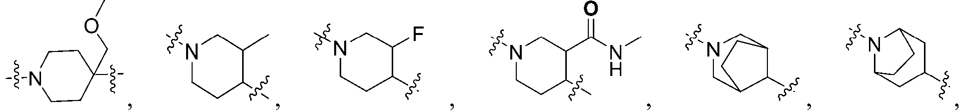 and 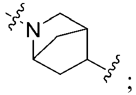 k is 1, 2, 3, 4, 5, 6, 7, 8, 9, or 10.

In one embodiment, wherein L is selected from the following structures:
a covalent bond, -(CH₂)ⱼ-, -(CH₂)ⱼ-CO-, -NH-(CH₂)ⱼ-, -(CH₂)ⱼ-NH-, -NH-(CH₂)ⱼ-NH-, 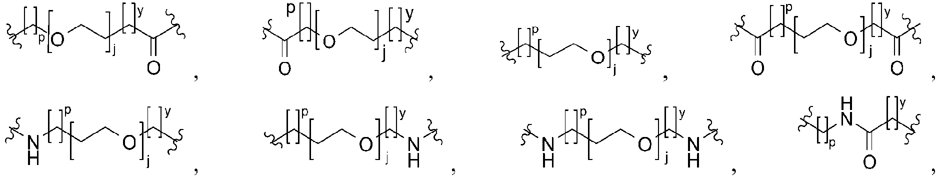 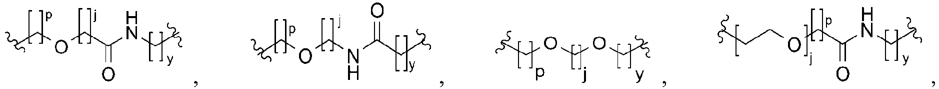 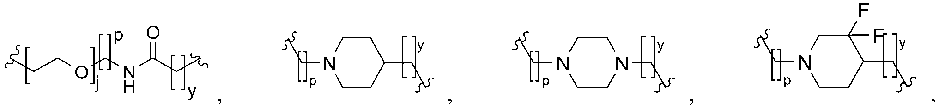 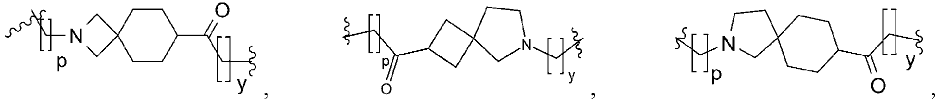 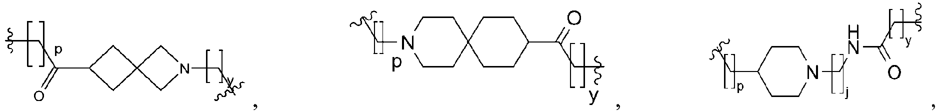 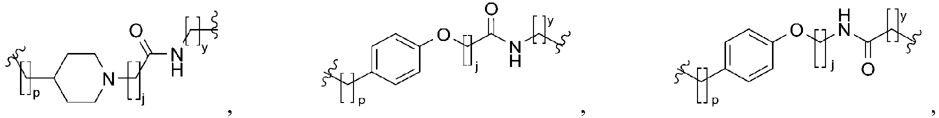 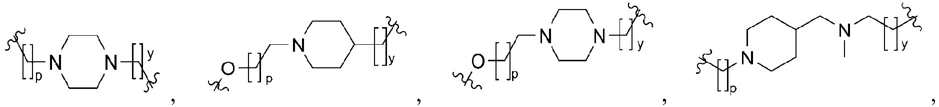 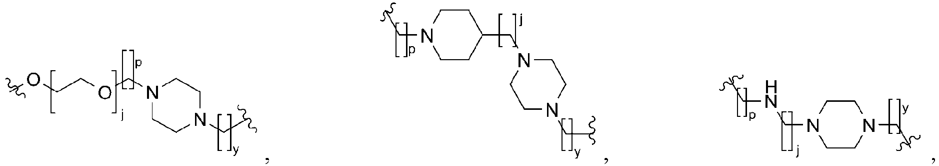 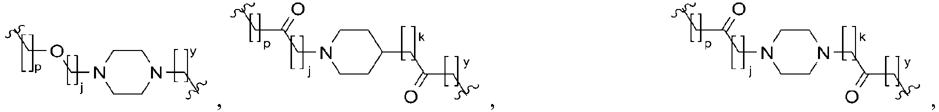 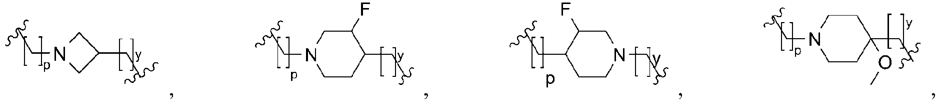 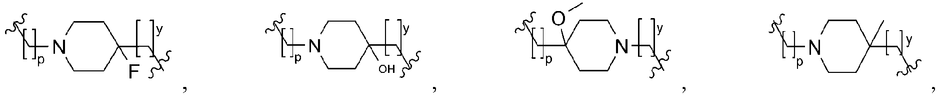 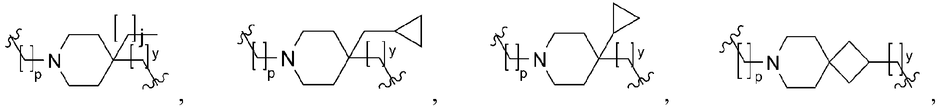 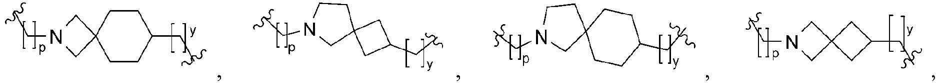 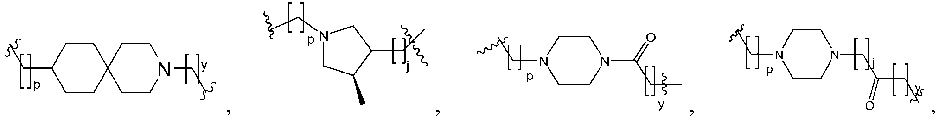 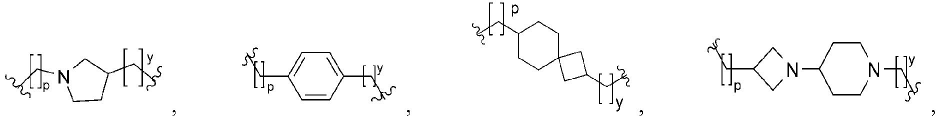 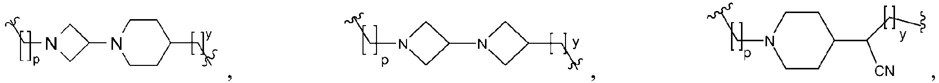 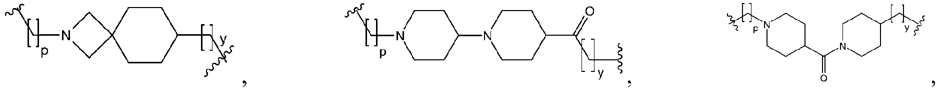 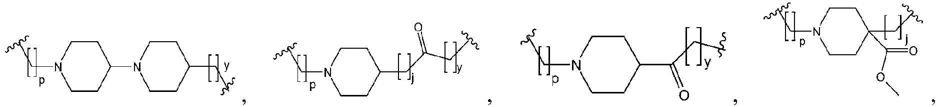 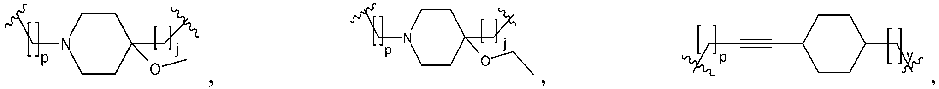 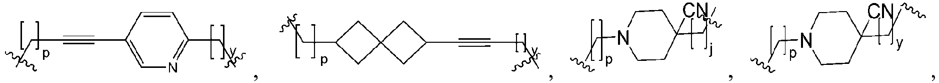 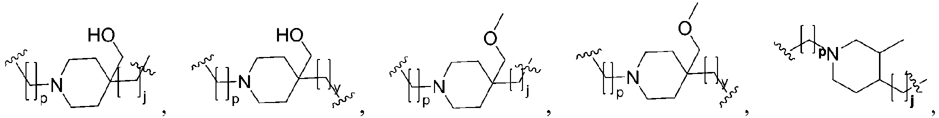 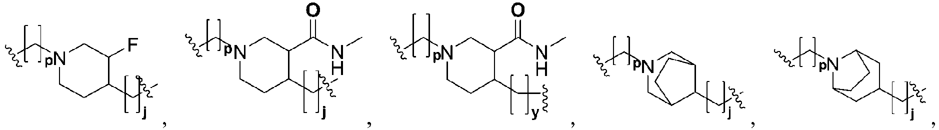 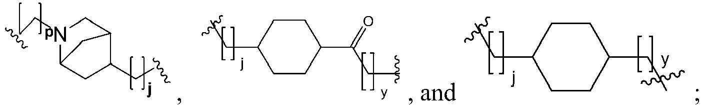
wherein j is 1, 2, 3, 4, 5, 6, 7, 8, 9, or 10;
p and y are 0, 1, 2, 3, 4, 5, 6, 7, 8, 9, or 10;
preferably, L is selected from a covalent bond, -CH₂-, -CH₂-CO-, -(CH₂)₂-, -(CH₂)₃-, -(CH₂)₄-, - (CH₂)₅-, -(CH₂)₆-, -(CH₂)₇-, -(CH₂)₈-, -NH-CH₂-, -NH-(CH₂)₂-, -NH-(CH₂)₃-, -NH-(CH₂)₄-, -NH-(CH₂)₅-, -NH-(CH₂)₆-, -NH-(CH₂)₇-, -NH-(CH₂)₈-, -CO-NH-CH₂-, -CO-NH-(CH₂)₂-, -CO-NH-(CH₂)₃-, -CO-NH-(CH₂)₄-, -CO-NH-(CH₂)₅-, -CO-NH-(CH₂)₆-, -CO-NH-(CH₂)₇-, -CO-NH-(CH₂)₈-, - CH₂-NH-, -(CH₂)₂-NH-, -(CH₂)₃-NH-, -(CH₂)₄-NH-, -(CH₂)₅-NH-, -(CH₂)₆-NH-, -(CH₂)₇-NH-, - (CH₂)₈-NH-, -NH-CH₂-NH-, -NH-(CH₂)₂-NH-, -NH-(CH₂)₃-NH-, -NH-(CH₂)₄-NH-, -NH-(CH₂)₅-NH-, -NH-(CH₂)₆-NH-, -NH-(CH₂)₇-NH-, -NH-(CH₂)₈-NH-, -CO-NH-CH₂-NH-, -CO-NH-(CH₂)₂-NH-, -CO-NH-(CH₂)₃-NH-, -CO-NH-(CH₂)₄-NH-, -CO-NH-(CH₂)₅-NH-, -CO-NH-(CH₂)₆-NH-, - CO-NH-(CH₂)₇-NH-, -CO-NH-(CH₂)₈-NH-, -(CH₂-CH₂-O)-CH₂-CH₂-, -(CH₂-CH₂-O)₂-CH₂-CH₂-, - (CH₂-CH₂-O)₃-CH₂-CH₂-, -NH-(CH₂-CH₂-O)-CH₂-CH₂-, -NH-(CH₂-CH₂-O)₂-CH₂-CH₂-, -NH-(CH₂-CH₂-O)₃-CH₂-CH₂-, -CO-NH-(CH₂-CH₂-O)-CH₂-CH₂-, -CO-NH-(CH₂-CH₂-O)₂-CH₂-CH₂-, -CO-NH-(CH₂-CH₂-O)₃-CH₂-CH₂-, -(CH₂-CH₂-O)-CH₂-CH₂-NH-, -(CH₂-CH₂-O)₂-CH₂-CH₂-NH-, -(CH₂-CH₂-O)₃-CH₂-CH₂-NH-, -NH-(CH₂-CH₂-O)-CH₂-CH₂-NH-, -NH-(CH₂-CH₂-O)₂-CH₂-CH₂-NH-, - NH-(CH₂-CH₂-O)₃-CH₂-CH₂-NH-, -CO-NH-(CH₂-CH₂-O)-CH₂-CH₂-NH-, -CO-NH-(CH₂-CH₂-O)₂-CH₂-CH₂-NH-, -CO-NH-(CH₂-CH₂-O)₃-CH₂-CH₂-NH-, -CH₂-CH₂-(O-CH₂-CH₂)-, -CH₂-CH₂-(O-CH₂-CH₂)₂-, -CH₂-CH₂-(O-CH₂-CH₂)₃-, -NH-CH₂-CH₂-(O-CH₂-CH₂)-, -NH-CH₂-CH₂-(O-CH₂-CH₂)₂-, -NH-CH₂-CH₂-(O-CH₂-CH₂)₃-, -CO-NH-CH₂-CH₂-(O-CH₂-CH₂)-, -CO-NH-CH₂-CH₂-(O-CH₂-CH₂)₂-, -CO-NH-CH₂-CH₂-(O-CH₂-CH₂)₃-, -CH₂-CH₂-(O-CH₂-CH₂)-NH-, -CH₂-CH₂-(O-CH₂-CH₂)₂-NH-, -CH₂-CH₂-(O-CH₂-CH₂)₃-NH-, -NH-CH₂-CH₂-(O-CH₂-CH₂)-NH-, -NH-CH₂-CH₂-(O-CH₂-CH₂)₂-NH-, -NH-CH₂-CH₂-(O-CH₂-CH₂)₃-NH-, -CO-NH-CH₂-CH₂-(O-CH₂-CH₂)-NH-, -CO-NH-CH₂-CH₂-(O-CH₂-CH₂)₂-NH-, -CO-NH-CH₂-CH₂-(O-CH₂-CH₂)₃-NH-,

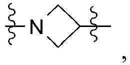

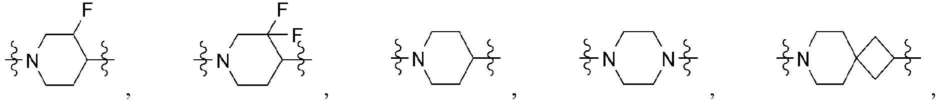

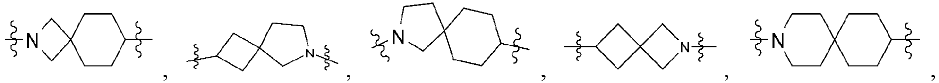

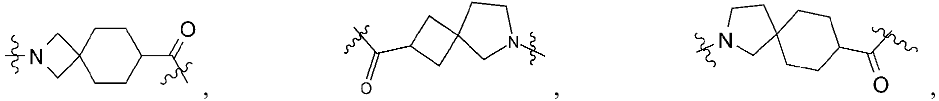

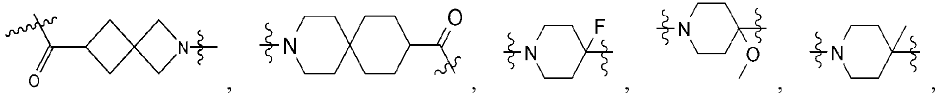

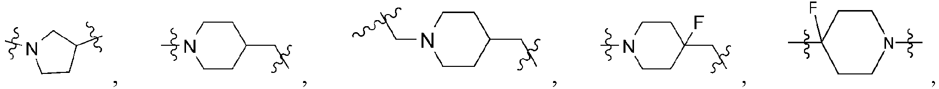

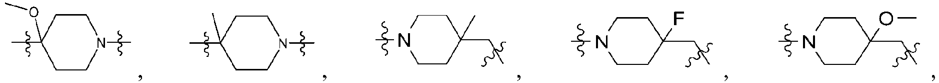

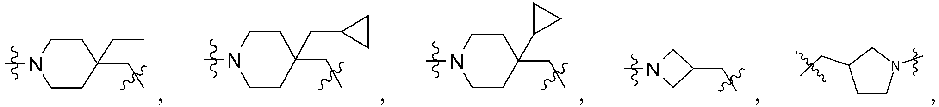

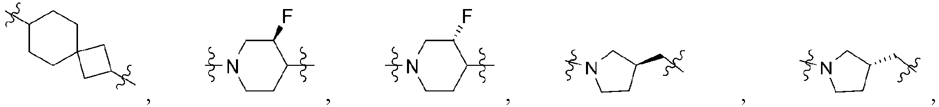

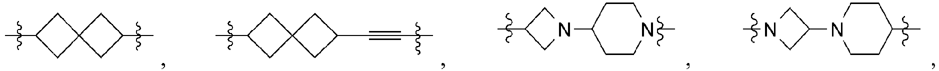

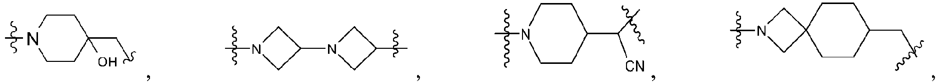

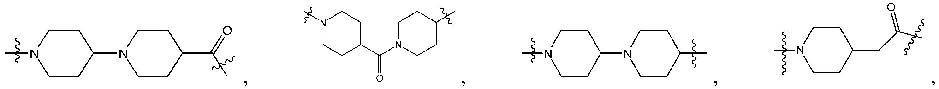

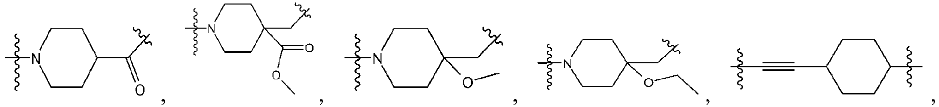

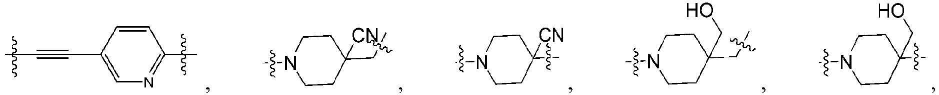

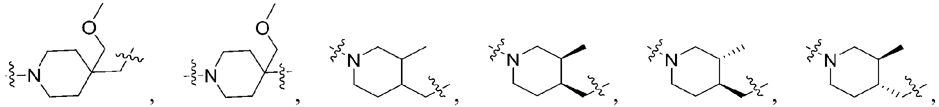

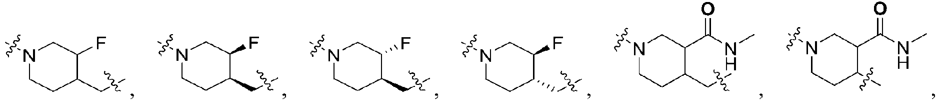

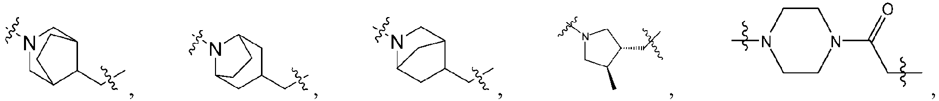

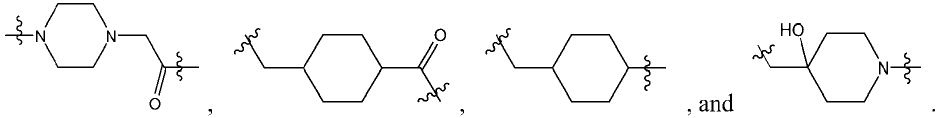

In one embodiment, the PTM is selected from the following structures: 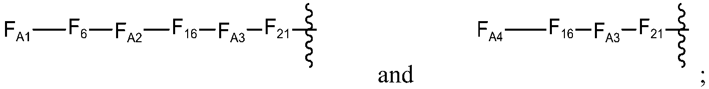
wherein each occurrence of F₆, F₁₆, and F₂₁ are each independently selected from a single bond, NH, SO, S, O, SO₂, alkylene, haloalkylene, heteroalkylene, alkyleneoxy, heteroalkyleneoxy, alkenylene, alkynylene, C(=O), OC(=O), C(=O)O, C(=O)NH, and NHC(=O), or a combination thereof; wherein the alkylene, alkyleneoxy, and alkenylene are optionally substituted with 0, 1, 2, 3, 4, 5, or 6 R_{c};
F_{A1} and F_{A4} are each independently aryl or heteroaryl; the aryl or heteroaryl is optionally substituted with 0, 1, 2, 3, 4, 5, or 6 R_{d}; preferably, F_{A4} is a 9-20 membered benzo- spiroheterocyclyl containing 1, 2, 3, 4, or 5 heteroatoms selected from N, O, and S, optionally substituted with 0, 1, 2, 3, 4, 5, or 6 R_{d};
each occurrence of F_{A3} is independently arylene or heteroarylene; the arylene or heteroarylene is optionally substituted with 0, 1, 2, 3, 4, 5, or 6 R_{c}, preferably substituted with 0, 1, 2, or 3 R_{c};
F_{A2} is cycloalkylene, spirocycloalkylene, heterocycloalkylene, or spiroheterocycloalkylene, and optionally substituted with 0, 1, 2, 3, 4, 5, or 6 R_{d};
each occurrence of R_{c} is independently selected from H, halogen, C₁₋₆ alkyl, C₁₋₆ haloalkly, C₁₋₆ heteroalkyl, C₁₋₆ alkoxy, C₁₋₆ heteroalkoxyl, C₃₋₇ cycloalkyl, C₃₋₇ heterocyclyl, C₆₋₈ aryl, C₅₋₈ heteroaryl, OH, NH₂, CN, and NO₂, preferably H, F, Cl, Br, I, CH₃, OCH₃, CF₃, OH, NH₂, CN, or NO₂;
each occurrence of R_{d} is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, oxo (=O), thioxo (=S), OH, NH₂, CN, and NO₂, preferably oxo (=O), thioxo (=S), H, F, Cl, Br, I, CH₃, OCH₃, CF₃, OH, NH₂, CN, or NO₂.

In one embodiment, wherein:
(1) the PTM is selected from the following structures: 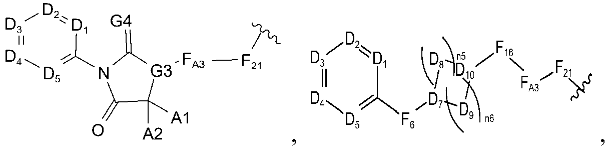 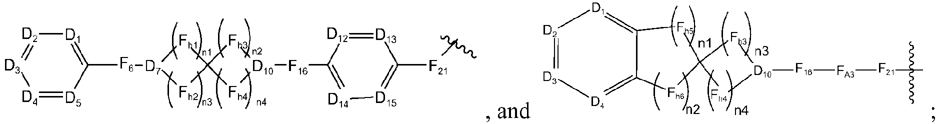 wherein:
   each occurrence of F₆ and F₁₆ are each independently selected from a single bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O), wherein the C₁₋₃ alkylene, C₁₋₃ alkyleneoxy, and C₂₋₃ alkenylene are optionally substituted with 0-6 R_{c}, preferably substituted with 0-4 R_{c}, preferably substituted with 0, 1, 2, or 3 R_{c};
   each occurrence of F_{A3} is independently a 6-10 membered arylene or a 5-13 membered heteroarylene containing 1, 2, 3, 4, or 5 heteroatoms selected from N, O, and/or S, the arylene or heteroarylene is optionally substituted with 0, 1, 2, 3, 4, 5, or 6 R_{c}; preferably, F_{A3} is selected from the following groups: 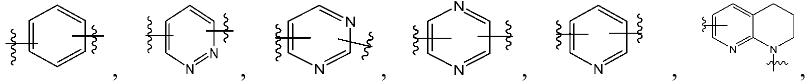 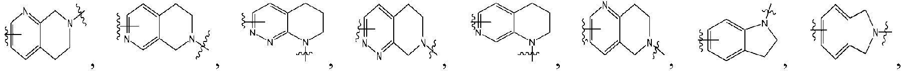 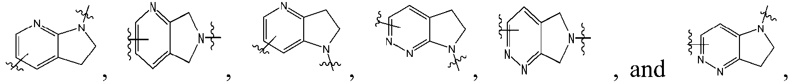 optionally substituted with 0, 1, 2, 3, 4, 5, or 6 R_{c}; wherein 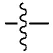 indicates a point of attachment, when the point of attachment on the F_{A3} group is not fixed, it indicates that 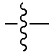 can be attached to any atom on the F_{A3} group that is capable of being connected;
   G3 is selected from N and C(R_{c});
   each occurrence of F₂₁ is independently selected from a single bond, NH, O, CO, C₁₋₆ alkylene, C₁₋₆ heteroalkylene, C₁₋₆ alkyleneoxy, and C₁₋₆ heteroalkyleneoxy, or a combination thereof, preferably selected from a single bond, NH, O, CO, C₁₋₆ alkylene, -C₁₋₆ alkylene-NH-C₁₋₆ alkylene-, -C₁₋₆ alkylene-O-C₁₋₆ alkylene-, -C₁₋₆ alkylene -C(O)-C₁₋₆ alkylene, -C₁₋₆ alkylene-O-C(O)-C₁₋₆ alkylene, - C₁₋₆ alkylene-C(O)-O-C₁₋₆ alkylene, -C₁₋₆ alkylene-NH-, -C₁₋₆ alkylene-O-, -C₁₋₆ alklyene-C(O)-, -C₁₋₆ alkylene-C(O)-O-, -C₁₋₆ alkylene-O-C(O)-, -NH-C₁₋₆ alkylene-, -O-C₁₋₆ alkylene-, -C(O)-C₁₋₆ alkylene, -C(O)-O-C₁₋₆ alkylene, -O-C(O)-C₁₋₆ alkylene, -NH- C₁₋₆ alkylene-NH-, -O-C₁₋₆ alkylene-O-, and -C(O)-C₁₋₆ alkylene-C(O)-, wherein the C₁₋₆ alkylene, C₁₋₆ heteroalkylene, C₁₋₆ alkyleneoxy, and C₁₋₆ heteroalkyleneoxy is optionally substituted with 0, 1, 2, 3, 4, 5, or 6 R_{c};
   each occurrence of A1 and A2 are independently selected from H, C₁₋₆ alkyl, halogen, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, or each occurrence of A1 and A2 together with the carbon atoms to which they are attached form a C₄₋₈ cycloalkyl or C₄₋₈ heteroalkyl, wherein the C₁₋₆ alkyl, C₁₋₆ haloalkyl, C₁₋₆ heteroalkyl, C₄₋₈ cycloalkyl, and C₄₋₈ heterocyclyl are optionally substituted with 0, 1, 2, 3, 4, 5, or 6 R_{c}; preferably, each occurrence of A1 and A2 is independently CH₃, or each occurrence of A1 and A2 together with the carbon atoms to which they are attached form a C₄₋₆ cycloalkyl or heterocyclyl;
   G4 is selected from O and S;
   each occurrence of D₁, D₂, D₃, D₄, D₅, D₁₂, D₁₃, D₁₄, D₁₅, D₇, and D₁₀ is independently selected from CR_{c} and N;
   each occurrence of D₈ and D₉ are independently selected from C(R_{d})₂ and NR_{d};
   each occurrence of n5 and n6 are independently selected from 0, 1, 2, and 3, and n5 and n6 are not simultaneously 0; preferably, n5 and n6 are selected from 1 and 2;
   each occurrence of Fₕ₁, Fₕ₂, Fₕ₃, and Fₕ₄ are independently selected from C(R_{c})₂ and NR_{c};
   each occurrence of Fₕ₅ and Fₕ₆ are independently selected from C(R_{c})₂, CO, CS, O, S, and NR_{c};
   each occurrence of n1, n2, n3, and n4 is independently selected from 1, 2, 3, 4, and 5; preferably, each occurrence of n1, n2, n3, and n4 are independently selected from 1 and 2;
   each occurrence of R_{c} is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably R_{c} is H, F, Cl, Br, I, CH₃, OCH₃, CF₃, OH, NH₂, CN, or NO₂;
   each occurrence of R_{d} is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, oxo (=O), thioxo (=S), OH, NH₂, CN, and NO₂, preferably R₄ is oxo (=O), thioxo (=S), H, F, Cl, Br, I, CH₃, CF₃, OCH₃, OH, NH₂, CN, or NO₂;
   preferably, 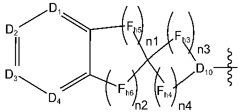 is selected from the following groups: 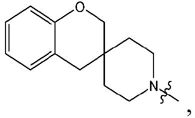 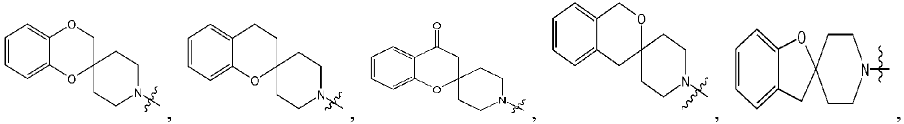 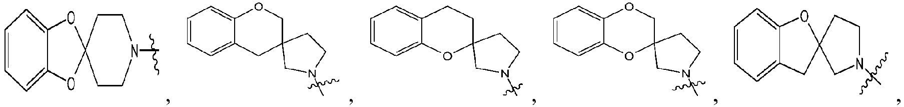 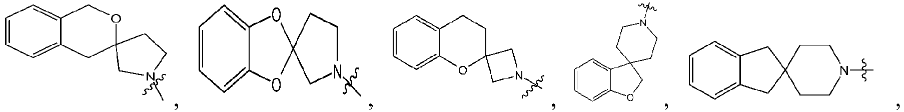 and 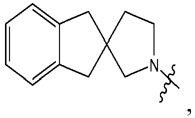 optionally substituted with 0, 1, 2, 3, or 4 R_{d}, wherein 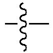 indicates a point of attachment; preferably, 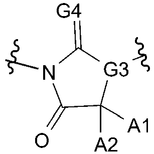 is selected from 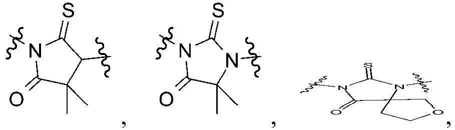 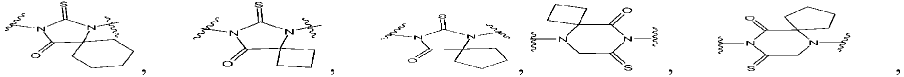 and 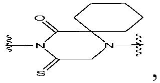 optionally substituted with 0-3 R_{d} ; 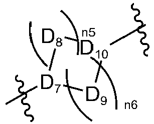 is selected from 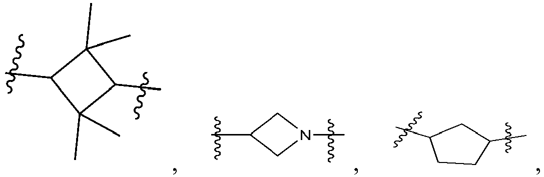 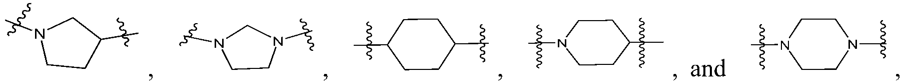 optionally substituted with 0-3 R_{d} ; 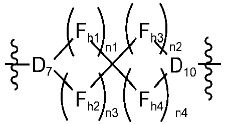 is selected from 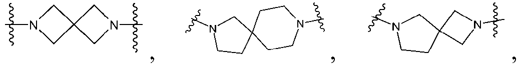 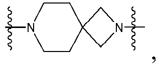 and 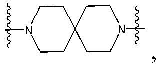 optionally substituted with 0-3 R_{d} ; wherein 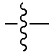 indicates a point of attachment;
      or,
(2) the PTM is selected from the following structures: 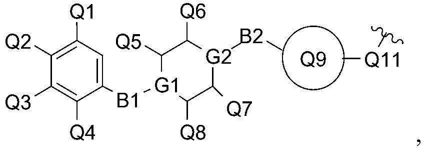 preferably, the PTM is selected from the following structures: 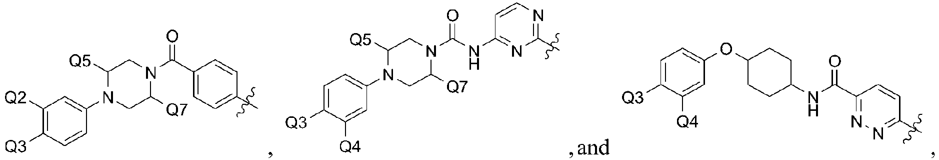 wherein:
   B1 and B2 are independently selected from a covalent bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O), wherein the C₁₋₃ alkylene, C₁₋₃ alkyleneoxy, and C₂₋₃ alkenylene are optionally substituted with 0-6 Q10, preferably substituted with 0-4 Q10, preferably substituted with 0, 1, 2, or 3 Q10;
   G1 and G2 are independently selected from N and C(Q10);
   Q9 is selected from aryl and heteroaryl, wherein the aryl and heteroaryl are optionally substituted with 0-4 Q10;
   Q11 is selected from one or more of a covalent bond, NH, O, CO, C₁₋₆ alkylene, C₁₋₆ heteroalkylene, C₁₋₆ alkyleneoxy, and C₁₋₆ heteroalkyleneoxy, preferably selected from a covalent bond, NH, O, CO, C₁₋₆ alkylene, -C₁₋₆ alkylene-NH- C₁₋₆ alkylene-, -C₁₋₆ alkylene-O-C₁₋₆ alkylene-, -C₁₋₆ alkylene-C(O)-C₁₋₆ alkylene, -C₁₋₆ alkylene-O-C(O)-C₁₋₆ alkylene, -C₁₋₆ alkylene-C(O)-O-C₁₋₆ alkylene, -C₁₋₆ alkylene-NH-, -C₁₋₆ alkylene-O-, -C₁₋₆ alkylene-C(O)-, -C₁₋₆ alkylene-C(O)-O-, -C₁₋₆ alkylene-O-C(O)-, -NH-C₁₋₆ alkylene-, -O-C₁₋₆ alkylene-, -C(O)-C₁₋₆ alkylene, -C(O)-O-C₁₋₆ alkylene, -O-C(O)-C₁₋₆ alkylene, -NH-C₁₋₆ alkylene -NH-, -O-C₁₋₆ alkylene -O-, and -C(O)-C₁₋₆ alkylene -C(O)-, wherein the C₁₋₆ alkylene, C₁₋₆ heteroalkylene, C₁₋₆ alkyleneoxy, and C₁₋₆ heteroalkyleneoxy are optionally substituted with 0-6 Q10, preferably substituted with 0, 1, 2, 3, 4, or 5 Q10;
   Q1, Q2, Q3, Q4, Q5, Q6, Q7, Q8, and Q10 are independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably H, F, Cl, Br, I, CH₃, CF₃, OH, NH₂, CN, or NO₂;
   preferably, the PTM is selected from the following structures: 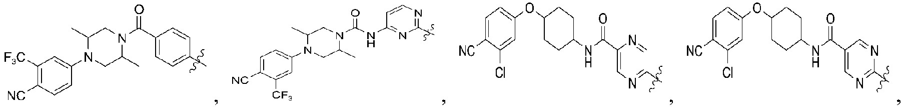 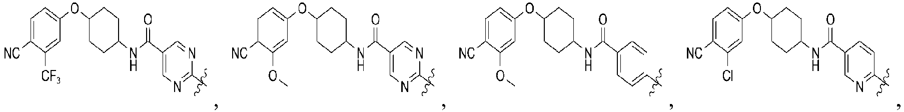 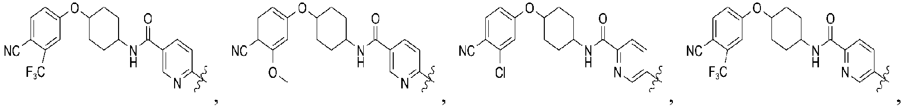 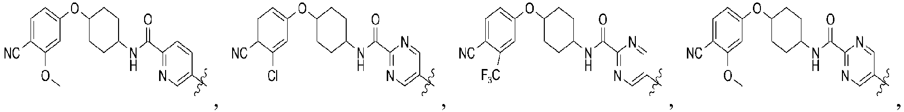 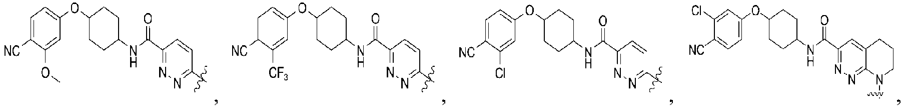 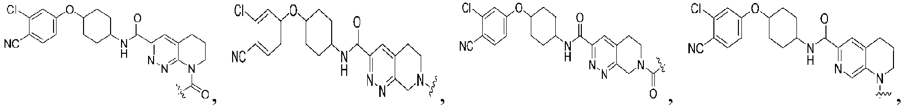 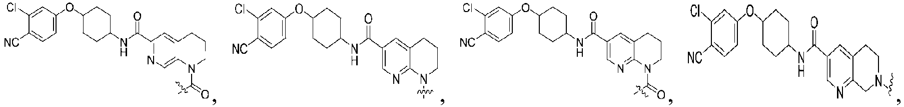 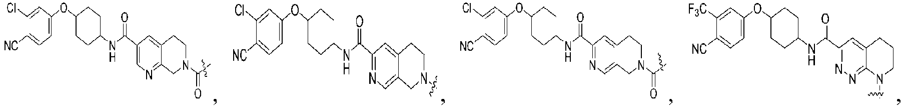 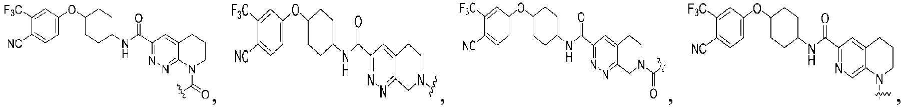 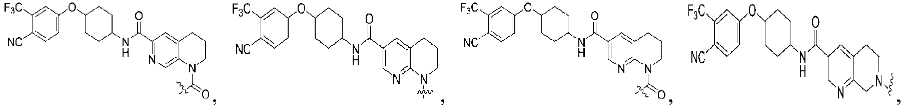 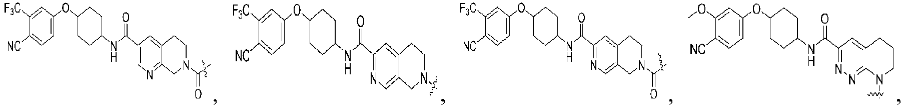 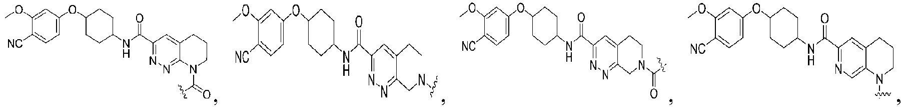 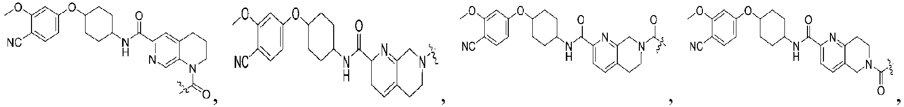 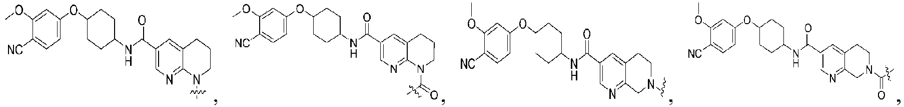 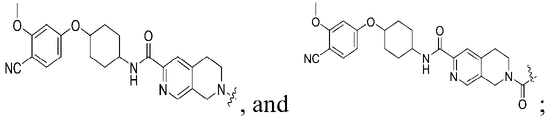
   preferably, the PTM is selected from the following structures: 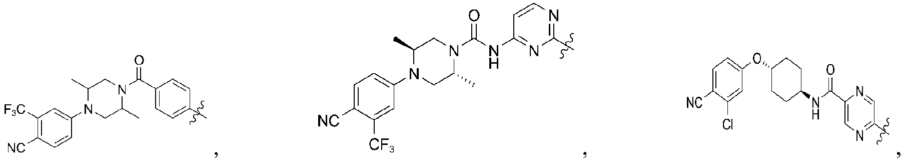 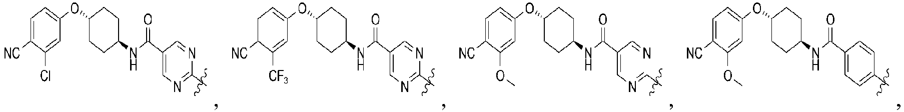 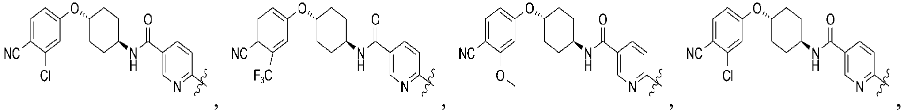 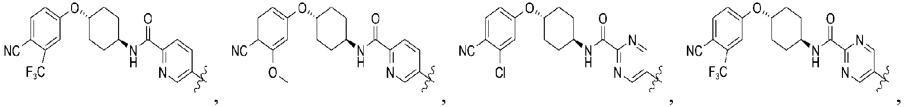 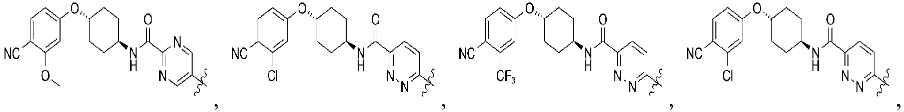 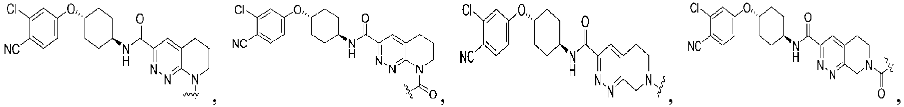 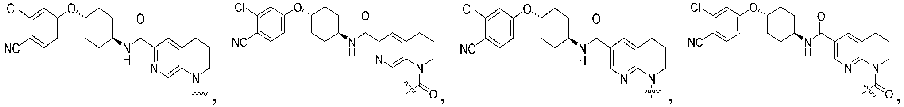 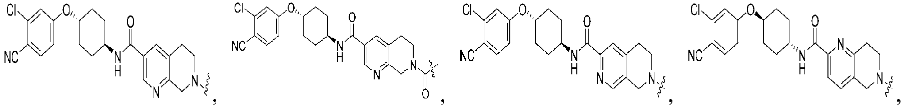 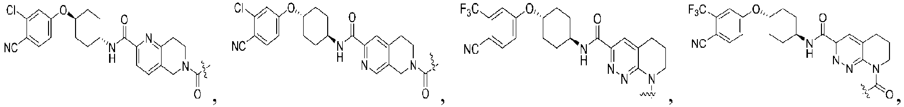 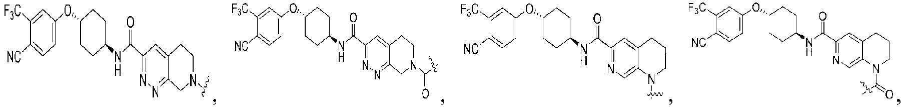 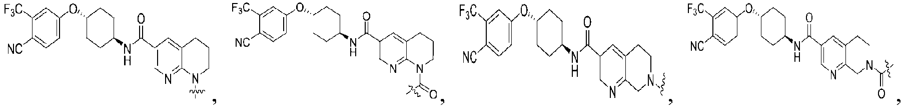 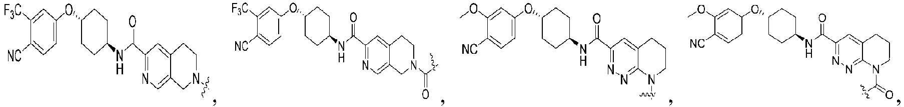 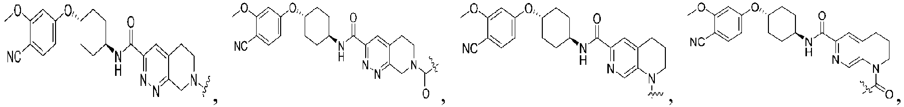 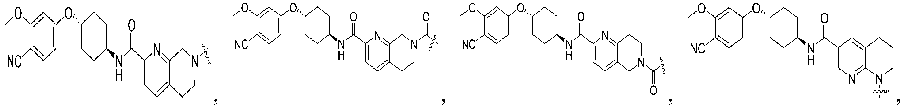 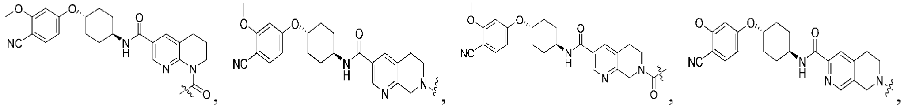 and 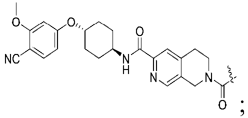 or,
(3) the PTM is selected from the following structures: 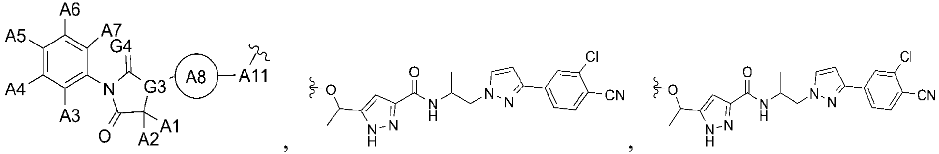 and 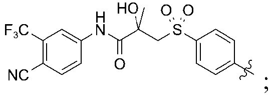 preferably, the PTM is selected from the following structures: 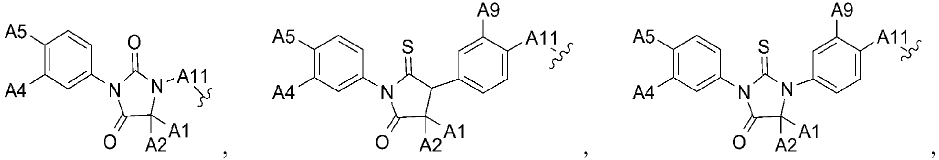 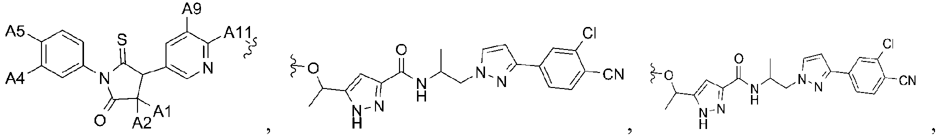 and 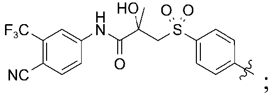 wherein:
   A1 and A2 are independently selected from C₁₋₆ alkyl, C₁₋₆ heteroalkyl, and C₄₋₈ cycloalkyl or C₄₋₈ heterocyclyl formed together with the carbon atom or heteroatom to which they are attached, wherein the C₁₋₆ alkyl, C₁₋₆ heteroalkyl, C₄₋₈ cycloalkyl, and C₄₋₈ heterocyclyl are optionally substituted with 0-6 A9, preferably substituted with 0, 1, 2, 3, 4, or 5 A9;
   A8 is selected from a covalent bond, aryl, and heteroaryl, wherein the aryl and heteroaryl are optionally substituted with 0-4 A9;
   A11 is selected from one or more of a covalent bond, NH, O, CO, C₁₋₆ alkylene, C₁₋₆ heteroalkylene, C₁₋₆ alkyleneoxy, and C₁₋₆ heteroalkyleneoxy, preferably selected from a covalent bond, NH, O, CO, C₁₋₆ alkylene, -C₁₋₆ alkylene -NH- C₁₋₆ alkylene -, -C₁₋₆ alkylene -O-C₁₋₆ alkylene -, -C₁₋₆ alkylene -C(O)- C₁₋₆ alkylene, -C₁₋₆ alkylene -O-C(O)-C₁₋₆ alkylene, -C₁₋₆ alkylene -C(O)-O-C₁₋₆ alkylene, -C₁₋₆ alkylene -NH-, -C₁₋₆ alkylene -O-, -C₁₋₆ alkylene -C(O)-, -C₁₋₆ alkylene -C(O)-O-, -C₁₋₆ alkylene -O-C(O)-, -NH-C₁₋₆ alkylene -, -O-C₁₋₆ alkylene -, -C(O)-C₁₋₆ alkylene, -C(O)-O- C₁₋₆ alkylene, -O-C(O)- C₁₋₆ alkylene, -NH- C₁₋₆ alkylene -NH-, -O-C₁₋₆ alkylene -O-, and -C(O)-C₁₋₆ alkylene -C(O)-, wherein the C₁₋₆ alkylene, C₁₋₆ heteroalkylene, C₁₋₆ alkyleneoxy, and C₁₋₆ heteroalkyleneoxy are optionally substituted with 0-6 A9, preferably substituted with 0, 1, 2, 3, 4, or 5 A9;
   G3 is selected from N and C(A10);
   G4 is selected from O and S; and
   each occurrence of A3, A4, A5, A6, A7, A9, and A10 is independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably H, F, Cl, Br, I, CH₃, CF₃, OH, NH₂, CN, or NO₂;
   preferably, the PTM is selected from the following structures: 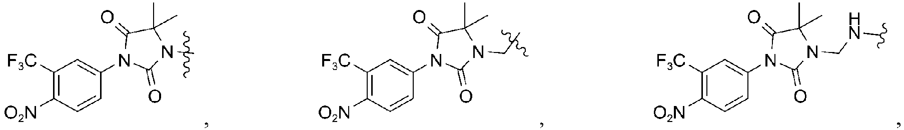 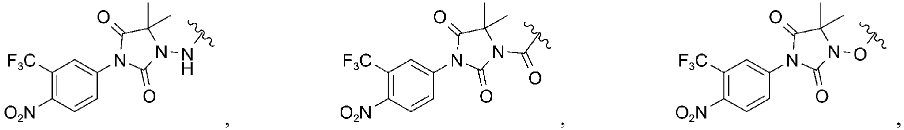 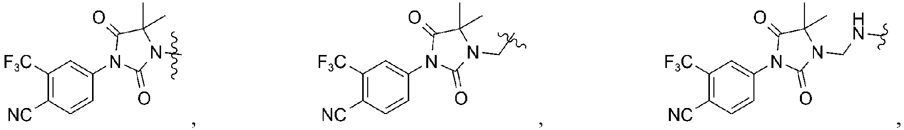 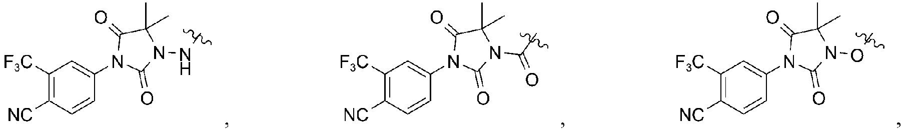 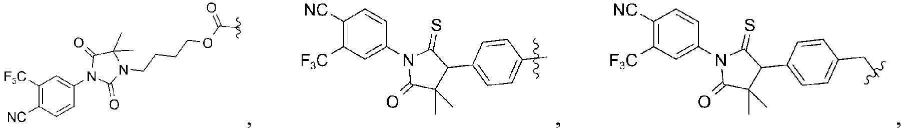 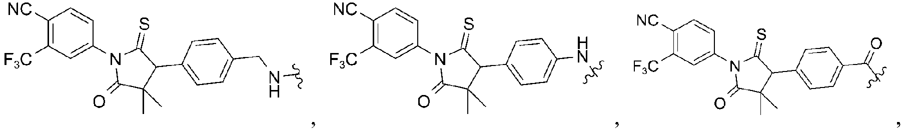 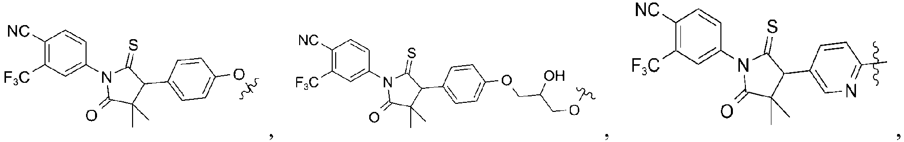 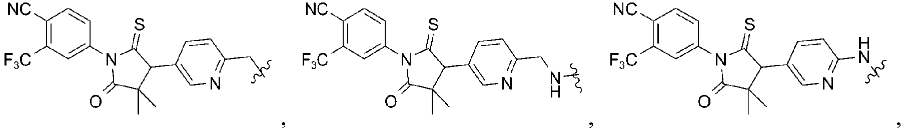 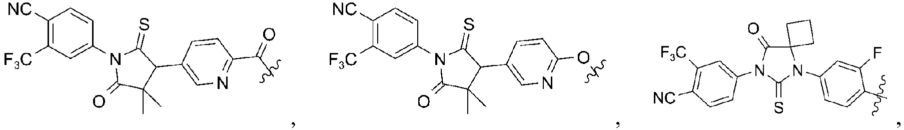 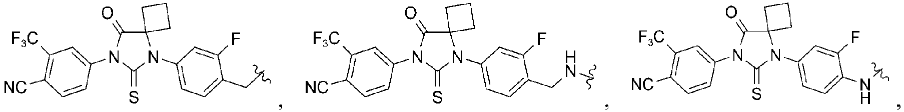 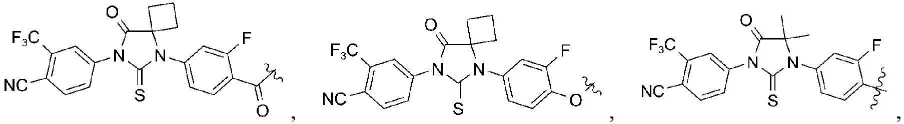 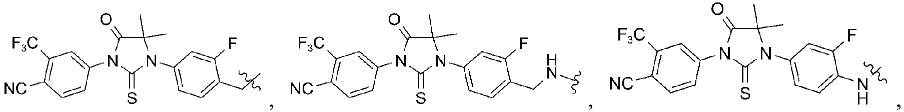 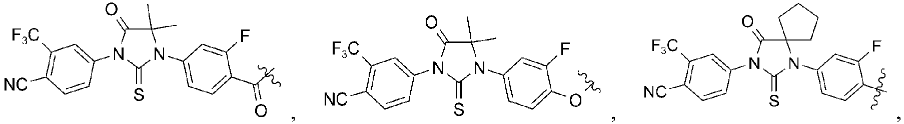 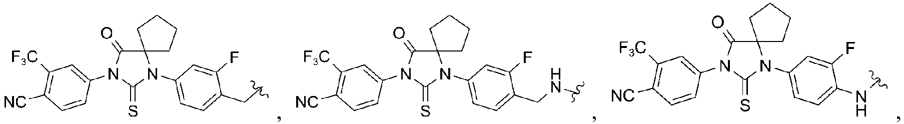 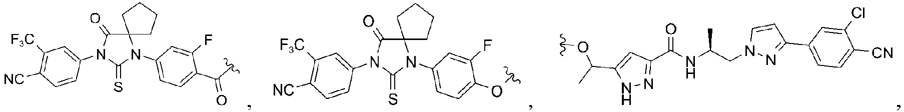 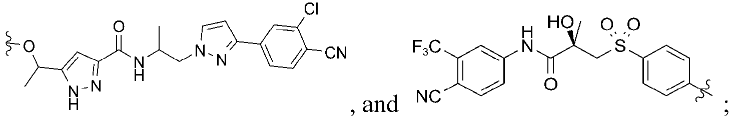
   preferably, the PTM is selected from the following structures: 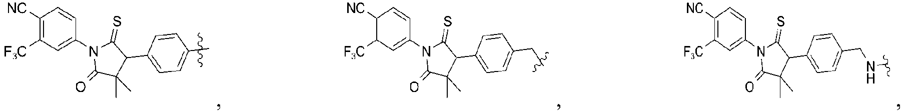 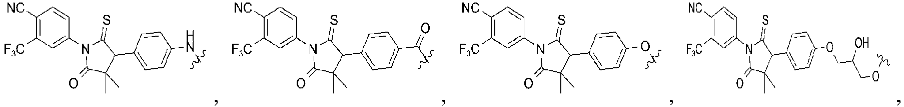 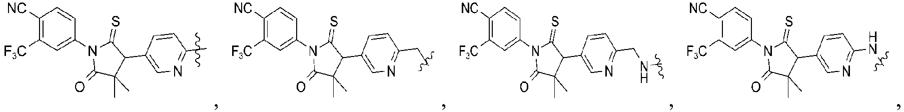 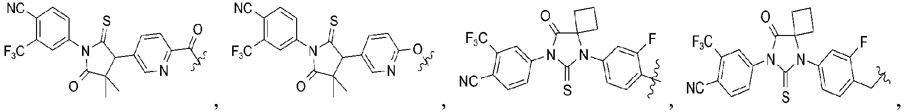 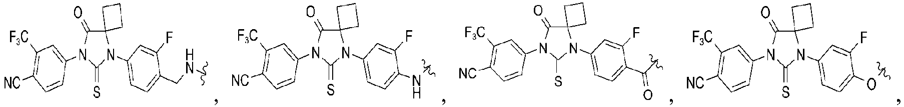 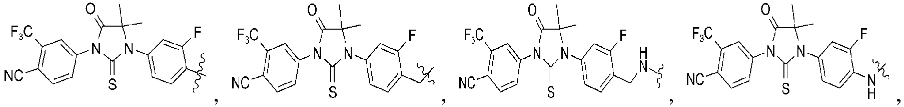 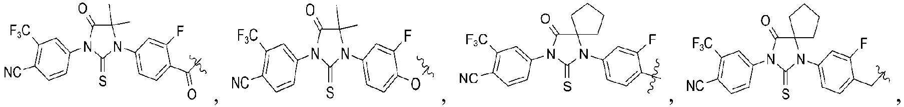 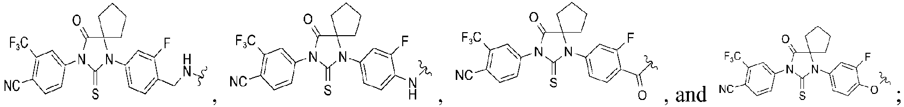 or,
(4) the PTM is selected from the following structures: 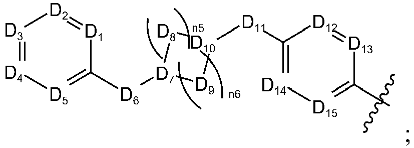 preferably, the PTM is selected from the following structures: 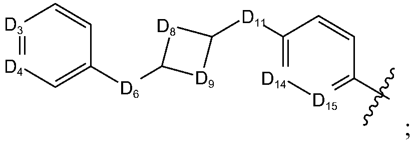
   wherein, D₆ and D₁₁ are independently selected from a single bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O); wherein the C₁₋₃ alkylene, C₁₋₃ alkyleneoxy, and C₂₋₃ alkenylene are optionally substituted with 0-6 Rₐₐ, preferably substituted with 0-4 Rₐₐ, preferably substituted with 0, 1, 2, or 3 Rₐₐ;
   each occurrence of D₁, D₂, D₃, D₄, D₅, D₁₂, D₁₃, D₁₄, D₁₅, D₇, and D₁₀ are independently selected from CRₐₐ and N;
   each occurrence of D₈ and D₉ are independently selected from C(Rₐₐ)₂ and NRₐₐ;
   n5 and n6 are independently selected from 1, 2, and 3, preferably selected from 1 and 2;
   each occurrence of Rₐₐ is independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably independently selected from H, F, Cl, Br, I, CH₃, CF₃, OH, NH₂, CN, and NO₂; preferably independently selected from H, F, Cl, Br, I, CH₃, CF₃, OH, NH₂, CN, OCH₃, and NO₂;
   preferably, the PTM is selected from the following structures: 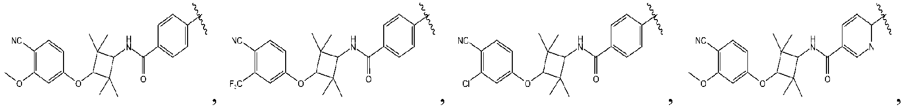 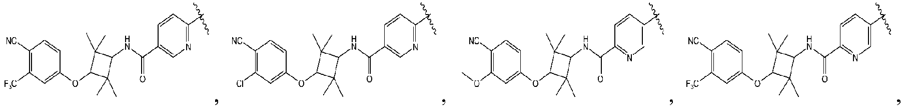 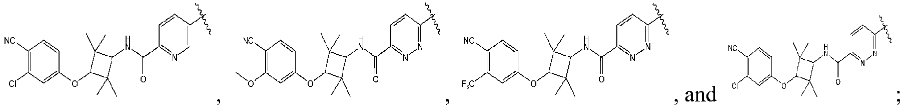
   preferably, the PTM is selected from the following structures: 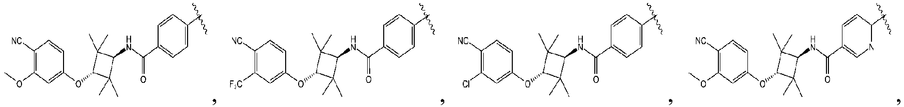 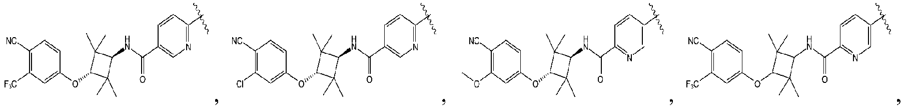 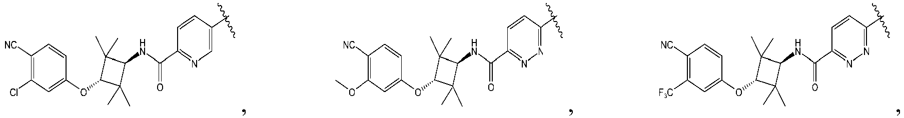 and 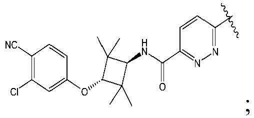 or,
(5) the PTM is selected from the following structures: 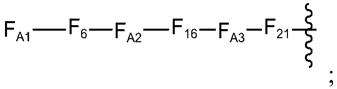 preferably, the PTM is selected from the following structures: 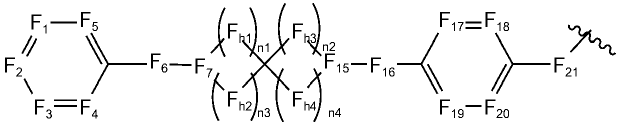 preferably, the PTM is selected from the following structures: 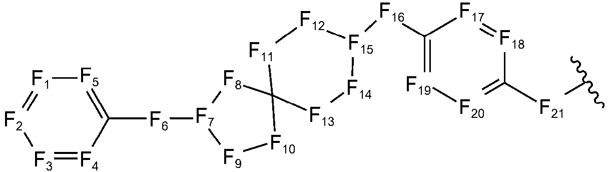 preferably, the PTM is selected from the following structures: 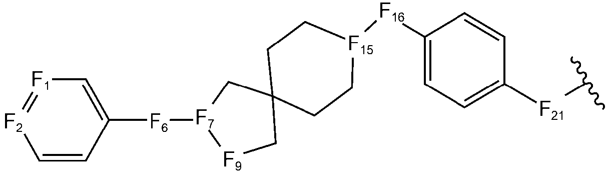
   wherein F₆, F₁₆, and F₂₁ are independently selected from a single bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O); wherein the C₁₋₃ alkylene, C₁₋₃ alkyleneoxy, and C₂₋₃ alkenylene are optionally substituted with 0-6 R_{c}, preferably substituted with 0-4 R_{c}, preferably substituted with 0, 1, 2, or 3 R_{c};
   F_{A1} and F_{A3} are independently a 6-10 membered aryl ring or a 5-8 membered heteroaryl ring; the aryl ring or heteroaryl ring is optionally substituted with 0-6 R_{c}, preferably substituted with 0-4 R_{c}, preferably substituted with 0, 1, 2, or 3 R_{c};
   F_{A2} is a 7-13 membered spirocycle or spiroheterocycle containing 0, 1, 2, 3, or 4 nitrogen atoms, and is optionally substituted with 0-6 R_{c}, preferably substituted with 0, 1, 2, or 3 R_{c};
   each occurrence of R_{c} is independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably selected from H, F, Cl, Br, I, CH₃, CF₃, OH, NH₂, CN, and NO₂;
   wherein:
      each occurrence of F₁, F₂, F₃, F₄, F₃, F₇, F₁₅, F₁₇, F₁₈, F₁₉, and F₂₀ are independently selected from CR_{c} and N;
      each occurrence of F₇ and F₁₅ are independently selected from CR_{c} and N;
      each occurrence of Fₕ₁, Fₕ₂, Fₕ₃, and Fₕ₄ are independently selected from C(R_{c})₂ and NR_{c};
      each occurrence of n1, n2, n3, and n4 are independently selected from 1, 2, 3, 4 and 5; preferably, each occurrence of n1, n2, n3, and n4 are independently selected from 1 and 2;
      wherein,
      each occurrence of F₈, F₉, F₁₀, F₁₁, F₁₂, F₁₃, and F₁₄ are independently selected from C(R_{c})₂ and NR_{c};
      preferably, the PTM is the following structures: 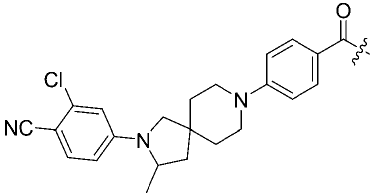 or

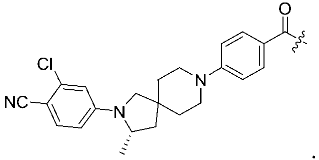

In another aspect, the present disclosure provides a pharmaceutical composition comprising any one of the compounds described above.

In a third aspect, the present disclosure provides a medicament comprising any one of the compounds described in the first aspect or the pharmaceutical composition described in the second aspect.

In a fourth aspect, the present disclosure provides a medicament for use in the treatment of prostate cancer, comprising any one of the compounds described in the first aspect or the pharmaceutical composition described in the second aspect.

In a fifth aspect, the present disclosure provides a use of any one of the compounds described in the first aspect or the pharmaceutical composition described in the second aspect in the manufacture of a medicament.

Preferably, the medicament is used for the treatment of prostate cancer.

### Detailed Description

### Terms and Definitions

The term "alkyl" as used herein refers to a saturated aliphatic hydrocarbon group, which is a straight or branched chain group containing 1 to 20 carbon atoms, preferably an alkyl group containing 1 to 12 (e.g., 1, 2, 3, 4, 5, 6, 7, 8, 9, 10, 11 and 12) carbon atoms, and more preferably an alkyl group containing 1 to 6 carbon atoms. Non-limiting examples of alkyl include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched chain isomers thereof. More preferably are lower alkyl containing 1 to 6 carbon atoms, non-limiting examples of which include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, when substituted, the substituent(s) may be substituted at any available attachment point, and are preferably optionally independently selected from one or more of the group consisting of: H atom, D atom, halogen, alkyl, alkoxy, haloalkyl, hydroxyl, hydroxyalkyl, cyano, amino, nitro, cycloalkyl, heterocyclyl, aryl, and heteroaryl.

The term "heteroalkyl" refers to an alkyl group in which one or more -CH₂- are substituted by heteroatoms selected from NH, O, and S, or one or more -CH- are substituted by N atoms; wherein the alkyl group is as defined above. The heteroalkyl may be optionally substituted or unsubstituted, when substituted, the substituent(s) may be substituted at any available attachment point, and are preferably optionally independently selected from one or more of the group consisting of: H atom, D atom, halogen, alkyl, alkoxy, haloalkyl, hydroxyl, hydroxyalkyl, cyano, amino, nitro, cycloalkyl, heterocyclyl, aryl, and heteroaryl.

The term "alkoxy" refers to -O-(alkyl) and -O-(unsubstituted cycloalkyl), wherein the alkyl or cycloalkyl is as defined herein. Non-limiting examples of alkoxy include: methoxy, ethoxy, propoxy, butoxy, cyclopropyloxy, cyclobutyloxy, cyclopentyloxy, cyclohexyloxy. The alkoxy may be optionally substituted or unsubstituted, when substituted, and the substituent(s) are preferably one or more of the following groups which are independently selected from: H atom, D atom, halogen, alkyl, alkoxy, haloalkyl, hydroxyl, hydroxyalkyl, cyano, amino, nitro, cycloalkyl, heterocyclyl, aryl, and heteroaryl.

The term "alkenyl" refers to an alkyl group containing carbon-carbon double bonds, wherein the definition of alkyl is as described above. The alkenyl group may be optionally substituted or unsubstituted, when substituted, the substituent(s) are preferably one or more of the following groups which are independently selected from: H atom, D atom, alkyl, alkoxy, halogen, haloalkyl, hydroxyl, hydroxyalkyl, cyano, amino, nitro, cycloalkyl, heterocyclyl, aryl, and heteroaryl.

The term "alkynyl" refers to an alkyl group containing carbon-carbon triple bonds in the molecule, wherein the definition of alkyl is as described above. The alkynyl may be optionally substituted or unsubstituted, when substituted, the substituent(s) are preferably one or more of the following groups which are independently selected from: H atom, D atom, alkyl, alkoxy, halogen, haloalkyl, hydroxyl, hydroxyalkyl, cyano, amino, nitro, cycloalkyl, heterocyclyl, aryl, and heteroaryl.

The term "cycloalkyl" refers to a saturated or partially unsaturated monocyclic hydrocarbon substituent, wherein the cycloalkyl ring containing 3 to 20 carbon atoms, preferably 3 to 12 carbon atoms, more preferably 3 to 8 (e.g., 3, 4, 5, 6, 7 and 8) carbon atoms, and even more preferably 4 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, and cyclooctyl, etc. The cycloalkyl may be optionally substituted or unsubstituted, when substituted, the substituent(s) may be substituted at any available attachment point and are preferably independently selected from one or more of the group consisting of: H atom, D atom, halogen, alkyl, alkoxy, haloalkyl, hydroxyl, hydroxyalkyl, cyano, amino, nitro, cycloalkyl, heterocyclyl group, aryl, and heteroaryl.

The term "heterocycloalkyl" or "heterocyclyl" refers to a saturated or partially unsaturated monocyclic hydrocarbon substituent containing 3 to 20 ring atoms, one or more of which are heteroatoms selected from nitrogen, oxygen, or S(O)ₘ (wherein m is an integer from 0 to 2), but excluding the ring part of -O-O-, -O-S- or -S-S-, and the remaining ring atoms are carbon. Preferably, it contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably, it contains 3 to 8 ring atoms, of which 1-3 are heteroatoms; more preferably, it contains 3 to 6 ring atoms, of which 1-3 are heteroatoms; most preferably, it contains 5 or 6 ring atoms, of which 1-3 are heteroatoms. Non-limiting examples of monocyclic heterocycloalkyl groups include pyrrolidinyl, tetrahydropyranyl, 1,2,3,6-tetrahydropyridinyl, piperidinyl, piperazinyl, morpholinyl, thiomorpholinyl, and homopiperazinyl, etc.

The heterocycloalkyl may be substituted or unsubstituted, when substituted, the substituent(s) may be substituted at any available attachment point and are preferably independently selected from one or more of the group consisting of: H atom, D atom, halogen, alkyl, alkoxy, haloalkyl, hydroxyl, hydroxyalkyl, cyano, amino, nitro, cycloalkyl, heterocyclyl, aryl, and heteroaryl.

The term "spirocycloalkyl" or "spirocyclyl" refers to a bicyclic structure formed by two saturated or unsaturated cycloalkyl groups connected through a single shared ring carbon atom.

The term "spiroheterocycloalkyl" or "spiroheterocyclyl" refers to a bicyclic structure formed by two saturated or unsaturated heterocyclyl groups connected through a single shared ring carbon atom, or a bicyclic structure formed by a saturated or unsaturated heterocycloalkyl group and a saturated or unsaturated cycloalkyl group connected through a single shared ring carbon atom.

The term "aryl" refers to a 6 to 14-membered all-carbon monocyclic or fused polycyclic (the fused polycyclic is a ring that shares adjacent pairs of carbon atoms) group with a conjugated π-electron system, preferably 6 to 10-membered, such as phenyl and naphthyl. The aryl ring includes an aryl ring as described above fused to a heteroaryl, heterocyclyl, spirocycloalkyl, spiroheterocycloalkyl, or cycloalkyl ring, wherein the ring connected to the parent structure is an aryl ring. The aryl may be optionally substituted or unsubstituted, when substituted, the substituent(s) can be substituted at any available attachment point, and the substituent are preferably independently selected from one or more of group consisting of: H atom, D atom, halogen, alkyl, alkoxy, haloalkyl, hydroxyl, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms, 5 to 14 ring atoms, wherein the heteroatoms are selected from oxygen, sulfur, and nitrogen. Heteroaryl is preferably 5 to 10-membered (e.g., 5, 6, 7, 8, 9, or 10-membered), more preferably 5-membered or 6-membered, such as furanyl, thienyl, pyridyl, pyrrolyl, N-alkylpyrrolyl, pyrimidyl, pyrazinyl, pyridazinyl, imidazolyl, pyrazolyl, triazolyl, and tetrazolyl, etc. The heteroaryl ring includes a heteroaryl fused to an aryl, heterocyclyl, or cycloalkyl ring as described above, wherein the ring connected to the parent structure is a heteroaryl ring. The heteroaryl may be optionally substituted or unsubstituted, when substituted, the substituent(s) may be substituted at any available attachment point and are preferably independently selected from one or more of the group consisting of: H atom, halogen, alkyl, alkoxy, haloalkyl, hydroxyl, hydroxyalkyl, cyano, amino, nitro, cycloalkyl, heterocyclyl, aryl, and heteroaryl.

The term "haloalkyl" refers to an alkyl group substituted with one or more halogen, wherein the alkyl is as defined above.

The term "hydroxyalkyl" refers to an alkyl group substituted with one or more hydroxy groups, wherein the alkyl is as defined above.

The term "halogen" refers to fluorine, chlorine, bromine, or iodine.

The term "amino" refers to -NH₂.

The term "cyano" refers to -CN.

The term "nitro" refers to -NO₂.

Unless otherwise indicated, the term "compounds of the present invention or disclosure" refers to compounds of formula I, sub-formulae thereof, and isomers thereof, such as stereoisomers (including diastereomers, enantiomers, and racemates), geometric isomers, conformational isomers (including rotamers and atropisomers), tautomers, isotopically labeled compounds (including deuterated substitutions), and inherently occurring forms (e.g., polymorphs, solvates, and/or hydrates). When moieties capable of forming salts are present, salts are also included, particularly pharmaceutically acceptable salts.

Those skilled in the art will recognize that the compounds of the present disclosure may contain chiral centers and therefore may exist in different isomeric forms. The term "isomers" as used herein refers to different compounds that have the same molecular formula but differ in the arrangement and configuration of atoms.

"Enantiomers" are a pair of stereoisomers that are non-superimposable mirror images of each other. A 1:1 mixture of a pair of enantiomers is a "racemic" mixture. The term is used to represent a suitable racemic mixture. When specifying the stereochemistry of the compounds of the present disclosure, the conventional R-S system is used to specify single stereoisomers with known relative and absolute configurations of two chiral centers (e.g., (1S, 2S)); single stereoisomers with known relative configurations but unknown absolute configurations are represented by asterisks (e.g., (1R*, 2R*)); and racemates denoted using the RS notation (e.g., (1RS, 2RS) as a racemic mixture of (1R, 2R) and (1S, 2S); (1RS, 2SR) as a racemic mixture of (1R, 2S) and (1S, 2R). "Diastereomers" are stereoisomers with at least two asymmetric atoms, but they are non-mirror images of each other. Absolute stereochemistry is specified according to the Cahn-Ingold-Prelog R-S system. When the compound is a pure enantiomer, the stereochemistry at each chiral carbon atom may be specified by R or S. For resolved compounds of unknown absolute configuration, they may be designated as (+) or (-) based on the direction of rotation of plane-polarized light at the wavelength of the sodium D line (dextrorotatory or levorotatory). Alternatively, the resolved compounds may be defined by chiral HPLC by the corresponding retention times of the corresponding enantiomers/diastereomers.

Certain compounds described herein contain one or more asymmetric centers or axes and may thus give rise to enantiomers, diastereomers, and other stereoisomeric forms that can be identified in terms of absolute stereochemistry as (R)- or (S)-.

Geometric isomers occur when a compound contains a double bond or some other feature that provides a certain amount of structural rigidity to the molecule. If the compound contains a double bond, its substituents may be in the E or Z configuration. If the compound contains a disubstituted cycloalkyl, the cycloalkyl substituents may have a cis- or trans-configuration.

Conformational isomers (or conformers) are isomers that can differ by the rotation of one or more bonds. Rotamers are conformers that differ by the rotation of only one bond.

The term "atropisomer" refers to structural isomers based on axial or planar chirality, resulting from restricted rotation in the molecule.

Unless otherwise indicated, the compounds of the present disclosure are intended to include all such possible isomers, including racemic mixtures, optionally pure forms and intermediate mixtures. Optically active (R)- and (S)-isomers can be prepared using chiral synthons or chiral reagents, or resolved using conventional techniques (e.g., separation on chiral SFC or HPLC columns, such as those available from DAICEL Corp. or other equivalent columns, using an appropriate solvent or solvents mixture to achieve effective separation).

The compounds of the present disclosure may be isolated in optically active or racemic forms. Optically active forms may be prepared by resolution of racemic forms or by synthesis of optically active starting materials. All methods for preparing the compounds of the present disclosure and intermediates prepared therein are considered to be part of the present disclosure. When enantiomeric or diastereomeric products are prepared, they may be separated by conventional methods, such as chromatography or fractional crystallization.

Depending on the process conditions, the final products of the present disclosure are obtained in free (neutral) or salt forms. Both the free forms and salts of these final products are within the scope of the present disclosure. If desired, one form of the compound can be converted to another form. A free base or acid can be converted to a salt; a salt can be converted to a free compound or another salt; a mixture of isomeric compounds of the present disclosure can be separated into individual isomers.

A pharmaceutically acceptable salt is preferred. However, other salts may be useful, for example, in isolation or purification steps that may be employed during preparation, and are therefore intended to be within the scope of the present disclosure.

As used herein, " a pharmaceutically acceptable salt" refer to a derivative of the disclosed compounds in which the parent compound is modified by preparing an acid or base salt thereof. For example, pharmaceutically acceptable salts include, but are not limited to, acetate, aspartate, benzoate, benzenesulfonate, bromide/hydrobromide, bicarbonate/carbonate, bisulfate/sulfate, camphorsulfonate, decanoate, chloride/hydrochloride, chlorourea acetate, citrate, edisylate, fumarate, gluconate, glucuronate, glutamate, glutarate, glycolate, hippurate, hydroiodide/iodide, isethionate, lactate, lactobionate, dodecyl sulfate, malate, malonate/hydroxymalonate, mandelate, methanesulfonate, methylsulfate, mucate, naphthoate, naphthalenesulfonate, nicotinate, nitrate, octadecanoate, oleate, oxalate, palmitate, pamoate, phenylacetate, phosphate/hydrogenphosphate/dihydrogenphosphate, polygalacturonate, propionate, salicylate, stearate, succinate, sulfamate, sulfosalicylate, tartrate, toluenesulfonate, trifluoroacetate, and xinafoate forms.

Pharmaceutically acceptable acid addition salts can be formed from inorganic acids and organic acids. Inorganic acids from which salts can be obtained include, for example, hydrochloric acid, hydrobromic acid, sulfuric acid, nitric acid, and phosphoric acid, etc. Organic acids from which salts can be obtained include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, and sulfosalicylic acid, etc.

Pharmaceutically acceptable base addition salts can be formed from inorganic bases and organic bases. Inorganic bases from which salts can be obtained include, for example, ammonium salts and metals in columns I to XII of the periodic table. In certain embodiments, salts are obtained from sodium, potassium, ammonium, calcium, magnesium, iron, silver, zinc, and copper; particularly suitable salts include ammonium salts, potassium salts, sodium salts, calcium salts, and magnesium salts. Organic bases from which salts can be obtained include, for example, primary, secondary, and tertiary amines, substituted amines (including naturally occurring substituted amines), cyclic amines, basic ion exchange resins, and the like. Certain organic amines include isopropylamine, benzathine, choline, diethanolamine, diethylamine, lysine, meglumine, piperazine, and tromethamine.

The pharmaceutically acceptable salts of the present disclosure can be synthesized by conventional chemical methods from a parent compound containing a basic or acidic moiety. Typically, such salts can be prepared by reacting the free acid or base forms of these compounds with a stoichiometric amount of an appropriate base or acid in water or in an organic solvent, or in a mixture of the two; typically, non-aqueous media such as ether, ethyl acetate, ethanol, isopropanol or acetonitrile are preferred. A list of suitable salts can be found in Remington: The Science and Practice of Pharmacy, 22nd edition, Allen, L.V., Jr., ed., Pharmaceutical Press, London, England, the disclosure of which is incorporated herein by reference.

Compounds of the present disclosure containing groups capable of serving as hydrogen bond donors and/or acceptors can form co-crystals with suitable co-crystal formers. These co-crystals can be prepared from compounds of the present disclosure by known co-crystal formation methods. Such methods include grinding, heating, co-subliming, co-melting compounds of the present disclosure and co-crystal formers under crystallization conditions or contacting compounds of formula (I) with co-crystal formers in solution and separating the co-crystals thus formed. Suitable co-crystal formers include those described in WO2004/078163. Therefore, the present disclosure also provides co-crystals comprising compounds of the present disclosure.

Any formula given herein is also intended to represent the unlabeled form of the compound as well as the isotopically labeled form. An isotopically labeled compound has a structure represented by the formula given herein, except that one or more atoms are replaced by atoms with a selected atomic mass or mass number. Examples of isotopes that can be incorporated into compounds of the present disclosure include isotopes of hydrogen, carbon, nitrogen, oxygen, phosphorus, fluorine, chlorine, and iodine, such as ²H, ³H, ¹¹C, ¹³C, ¹⁴C, ¹⁵N, ¹⁸F, ³¹P, ³²P, ³⁵S, ³⁶Cl, and ¹²⁵I, respectively. The present disclosure includes various isotopically labeled compounds as described herein, for example, those in which radioactive isotopes such as ³H and ¹⁴C are present, or those in which non-radioactive isotopes such as ²H and ¹³C are present. Such isotopically labeled compounds can be used in metabolic studies (using ¹⁴C), reaction kinetic studies (using, for example, ²H or ³H), drug or substrate tissue distribution assays including detection or imaging techniques such as positron emission tomography (PET) or single photon emission computed tomography (SPECT), or radiotherapy of patients. In particular, ¹⁸F-labeled compounds may be particularly desirable for PET or SPECT studies.

In addition, substitution with heavier isotopes, particularly deuterium (i.e., ²H or D) can provide certain therapeutic advantages due to higher metabolic stability, such as prolonged half-life in vivo, reduced dosage requirements, or improved therapeutic indices. It should be understood that deuterium described herein is considered to be a substituent of the compounds of the present disclosure. The concentration of such heavier isotopes (particularly deuterium) can be defined by an isotopic enrichment factor. As used herein, the term "isotopic enrichment factor" refers to the ratio between the isotopic abundance and the natural abundance of a particular isotope. If a substituent in a compound of the present disclosure is represented as deuterium, such compound has an isotopic enrichment factor for each designated deuterium atom of at least 3500 (52.5% deuterium incorporation for each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The isotopically labeled compounds of the present disclosure can generally be prepared by conventional techniques known to those skilled in the art or by methods disclosed in the schemes or examples and the preparations described below (or processes analogous to those described herein): by substituting an appropriate or readily available isotopically labeled reagent for the non-isotopically labeled reagent otherwise used. Such compounds have a variety of potential uses, for example, as standards and reagents for determining the ability of potential drug compounds to bind to target proteins or receptors, or for imaging of substances that bind to the disclosed biological receptors herein in vivo or in vitro.

The term "solvate" refers to a physical association of the compound of the present disclosure with one or more solvent molecules, whether organic or inorganic. The physical association includes hydrogen bonding. In some cases, the solvate will be able to separate, for example, when one or more solvent molecules are incorporated into the crystal lattice of a crystalline solid. The solvent molecules in the solvate may exist in either ordered or disordered arrangements. The solvate may contain stoichiometric or non-stoichiometric amounts of solvent molecules. "Solvate" includes solution phase and separable solvates. Exemplary solvates include, but are not limited to, hydrates, ethanolates, methanolates, and isopropanolates. Methods of solvation are generally known in the art.

The term "ubiquitin ligase" refers to a family of proteins that promote ubiquitin transfer to specific substrate proteins , thereby targeting the substrate proteins for degradation. For example, cereblon is an E3 ubiquitin ligase protein that , either alone or in combination with an E2 ubiquitin-conjugating enzyme, mediates the attachment of ubiquitin to lysine residues on target proteins, subsequently directing specific protein substrates for degradation via the proteasome. Therefore, E3 ubiquitin ligases alone or in combination with E2 ubiquitin conjugating enzymes are the cause of ubiquitin transfer to the target protein. In general, ubiquitin ligases participate in polyubiquitination so that a second ubiquitin is attached to a first ubiquitin, a third ubiquitin is attached to a second ubiquitin, and so on. Polyubiquitination marks proteins for degradation by the proteasome. However, there are some ubiquitination events limited to monoubiquitination, in which only a single ubiquitin is added to the substrate molecule by a ubiquitin ligase. Monoubiquitinated proteins are not targeted to the proteasome for degradation, but may instead change in their cellular location or function, such as by binding to other proteins with a domain that can bind ubiquitin. To make things more complicated, different lysines on ubiquitin can be targeted by E3 ligases to prepare chains. The most common lysine is Lys48 on the ubiquitin chain. This is the lysine used to make polyubiquitin chain, which is recognized by the proteasome.

The term "target protein" refers to proteins and peptides having any biological function or activity, including structural, regulatory, hormonal, enzymatic, genetic, immune, contractile, storage, transport, and signal transduction. In some embodiments, target proteins include structural proteins, receptors, enzymes, cell surface proteins, proteins integral to cellular functions, such as those involved in catalytic activity, aromatase activity, motor activity, helicase activity, metabolic processes (anabolism and catabolism), antioxidant activity, proteolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (protein, lipid carbohydrate), receptor activity, cell motility, membrane fusion, cell communication, regulation of biological processes, development, cell differentiation, stimulus response, behavioral proteins, cell adhesion proteins, proteins involved in cell death, proteins involved in transport (including protein transport activity, nuclear transport, ion transport activity, channel transport activity, carrier activity), permease activity, secretion activity, electron transport activity, pathogenesis, chaperone regulator activity, nucleic acid binding activity, transcription regulator activity, extracellular organization and biogenesis activity, translation regulator activity. The proteins include those derived from eukaryotic and prokaryotic organisms, including microbes, viruses, fungi and parasites, among many others, including humans, microorganisms, viruses, fungi and parasites as targets for drug therapy, other animals including domestic animals, microbes used as targets for testing antibiotics and other antimicrobials, plants, even viruses, and among many others.

"Optional" or "optionally" means that the subsequently described event or circumstance may, but need not, occur, and the description includes instances where the event or circumstance occurs or does not occur. For example, "optionally substituted cyclopropyl" means that cyclopropyl may but need not be substituted, and the description includes instances where cyclopropyl is substituted and instances where cyclopropyl is not substituted.

"Substituted" means that one or more hydrogen atoms, preferably up to 5, more preferably 1 to 3 hydrogen atoms in the group are replaced independently of each other by a corresponding number of substituents. It goes without saying that the substituents are only in their possible chemical positions, and those skilled in the art can determine (by experiment or theory) possible or impossible substitutions without undue effort.
The abbreviations used in this text are as follows:
MeOH is methanol;
H₂SO₄ is concentrated sulfuric acid;
NIS is N-iodosuccinimide;
TFA is trifluoroacetic acid;
Pd is palladium;
Sn is tin;
Pyrrolidine is pyrrolidine;
OH is hydroxyl;
Boc is tert-butoxycarbonyl;
NaBH₄ is sodium borohydride;
Et₃SiH is triethylsilane;
H₂ is hydrogen;
LiOH is lithium hydroxide;
NaOAc is sodium acetate;
AcOH is acetic acid;
PE is petroleum ether;
EA is ethyl acetate;
CDCl₃ is deuterated chloroform;
HNO₃ is nitric acid;
HBF₄ is tetrafluoroboric acid;
NaNO₂ is sodium nitrite;
NBS is N-bromosuccinimide;
KOtBu is potassium tert-butoxide;
NaH is sodium hydride;
B₂pin₂ is bis(pinacolato)diboron;
Oxone is potassium monoperoxysulfate;
Cbz is benzyloxycarbonyl;
PPh₃ is triphenylphosphine;
CBr₄ is carbon tetrabromide;
Zn is zinc;
NH₄Cl is ammonium chloride;
B is boron;
Br is bromine;
(TMS)₃SiH is tris(trimethylsilyl)silane;
AlBN is azobisisobutyronitrile;
DMF is N,N-dimethylformamide;
K₂CO₃ is potassium carbonate;
TBAB is tetrabutylammonium bromide;
Bn is benzyl;
LiAlH₄ is lithium aluminum hydride;
BH₃ is borane;
THF is tetrahydrofuran;
H₂O₂ is hydrogen peroxide;
DMP is Dess-Martin oxidant;
PBr₃ is phosphorus tribromide;
Pd(OAc)₂ is palladium(II) acetate;
BF₃Et₂O is boron trifluoride etherate;
S is sulfur;
MsCl is methanesulfonyl chloride;
TEA is triethylamine;
DBU is 1,8-diazabicyclo[5.4.0]undec-7-ene;
NaHCO₃ is sodium bicarbonate;
Malonic acid is malonic acid;
PPA is polyphosphoric acid;
HBr is hydrogen bromide;
Tf₂O is trifluoromethanesulfonic anhydride;
Pd/C is palladium on carbon;
DMSO is dimethyl sulfoxide;
UPLC is ultra-performance liquid chromatography;
MgCl₂ is magnesium chloride;
NADPH is nicotinamide adenine dinucleotide phosphate;
NaBH₃CN is sodium cyanoborohydride;
DIPEA is N,N-diisopropylethylamine;
Dioxane is 1,4-Dioxacyclohexane;
NaBH(OAc)₃ is sodium triacetoxyborohydride.

The following examples provide detailed descriptions of the preparation of the intermediate compounds and final products identified in the specification and synthetic schemes. While the chemical reactions described herein are disclosed based on their general applicability to the preparation of the compounds of the present invention, it is possible that such reactions may not be applicable to every compound within the scope of the present invention as described. Those of ordinary skill in the art will readily identify such compounds. In such cases, the reactions may be successfully conducted through routine modifications known to those skilled in the art. In all preparation methods, all starting materials are either known or can be readily prepared from known materials.

The starting materials, chemical reagents, and solvents used in the present disclosure are commercially available and sourced from companies such as Energy Chemical, Shanghai Bepharm , Beijing InnoChem, Jiangsu Aikon, China National Pharmaceutical Group Corporation (Sinopharm), Beijing J & K, and Yunnan Xinlanjing.

The structures of the compounds were determined by nuclear magnetic resonance (NMR) and/or mass spectrometry (MS). Nuclear magnetic resonance ( NMR ) measurements were performed using a Bruker AVANCE-400/600 NMR spectrometer with deuterated solvents including deuterated dimethyl sulfoxide (DMSO-d₆) , deuterated chloroform (CDCl₃), and deuterated methanol (CD₃OD), using tetramethylsilane (TMS) as the internal standard. Mass spectrometry (MS) measurements were conducted using a Waters Acquity UPLC^{®} Plus instrument. High-performance liquid chromatography (HPLC) preparation was performed using a Waters 2489 instrument. Medium-pressure flash preparative chromatography was carried out using a COMBIFLASH NEXTGEN 300+ instrument. Thin-layer chromatography (TLC) silica gel plates (aluminum plates with fluorescence indicator) were used, and the silica gel (100-200 mesh, 200-300 mesh) for TLC was purchased from InnoChem.

Reaction progress in the examples was monitored by thin-layer chromatography (TLC). The systems of developing solvents for reaction monitoring and eluents for column chromatographic purification of compounds included petroleum ether/ethyl acetate and dichloromethane/methanol systems.

### Example 1

### Synthesis of intermediate 1

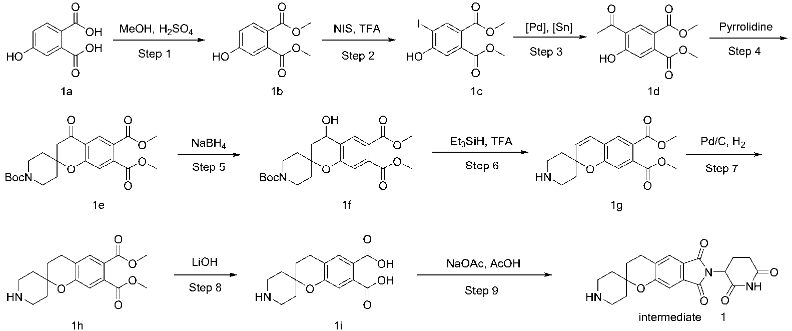

Step 1: Concentrated sulfuric acid (45 mL) was slowly added to a solution of intermediate 1a (30 g, 164.7 mmol) in methanol (300 mL). The reaction solution was stirred at 65 °C for 5 hours. The resulting mixture was poured into ice water, filtered and washed with water, and dried under vacuum to obtain a white solid intermediate 1b (30.0 g, 87%).
¹H NMR(600MHz,DMSO-*d*₆ )δ10.66(s,1H),7.70(d,*J*=8.5Hz,1H),6.96(dd,*J*=8.5,2.5Hz,1H),6.93(d,*J*= 2.5Hz,1H),3.79(s,3H),3.76(s,3H)
LC-MS(ESI):[M-H]⁺ =209.22

Step 2: N-iodosuccinimide (23.6 g, 104.7 mmol) was slowly added to a solution of intermediate 1b (20.0 g, 95.2 mmol) in trifluoroacetic acid (60 mL). The reaction solution was stirred at room temperature overnight. The resulting mixture was concentrated under vacuum and purified by C18 reversed-phase column to obtain a white solid intermediate 1c (16.3 g, 51%).
¹H NMR(600MHz,DMSO-*d*₆ )δ11.59(s,1H),8.11(s,1H),7.02(s,1H),3.79(s,3H),3.77(s,3H)
LC-MS(ESI):[M-H]⁺ =335.06

Step 3: Under the protection of nitrogen, the intermediate 1c (15.0 g, 44.6 mmol), tributyl (1-ethoxy vinyl) tin (32.2 g, 89.3 mmol) and bis (triphenylphosphine) palladium dichloride (II) (3.1 g, 4.5 mmol) were dissolved in tetrahydrofuran (75 mL), heated to 65 °C and stirred for 15 h. After the reaction was completed, 1 M dilute hydrochloric acid solution was added to the system and stirred for 0.5 h, followed by adding potassium fluoride. The mixture was filtered and concentrated to obtain a crude product. It was purified by column chromatography (PE: EA = 0-40%) to obtain a yellow oily intermediate 1d (9.8 g, 87%).
¹H NMR(400MHz, CDCl3 )δ12.65(s,1H),8.37(s,1H),7.12(s,1H),3.96(s,3H),3.92(s,3H),2.73(s,3H)
LC-MS(ESI):[M+H]⁺ =253.15

Step 4: The intermediate 1d (5.0 g, 19.8 mmol), N-(tert-butoxycarbonyl)-4-piperidone (1.4 g, 19.8 mmol) and tetrahydropyrrole (4.0 g, 19.8 mmol) were dissolved in methanol (50 mL), and the mixture was stirred at 70°C Overnight, spun dry, and purified by column chromatography (PE:EA=0-35%) to obtain a yellow oily intermediate 1e (6.9 g, 80%).
¹H NMR(600MHz,DMSO-*d*₆ )δ8.17(s,1H),7.36(s,1H),3.83(s,3H),3.82(s,3H),3.73(s,2H),3.13(d, *J*= 40.7Hz,2H),2.96(s,2H),1.95-1.86(m,2H),1.66(dq,*J*= 13.2,5.0Hz,2H),1.40(s,9H)
LC-MS(ESI):[M-H]⁺ =432.22

Step 5: Sodium borohydride (0.7 g, 17.3 mmol) was added to a solution of intermediate 1e (5.0 g, 11.5 mmol) in methanol (50 mL) under ice bath conditions. The reaction solution was stirred at 70°C overnight. The resulting mixture was concentrated under vacuum and purified by column chromatography (PE:EA=0-50%) to obtain a white solid intermediate 1f (3.3 g, 66%).
¹H NMR(600MHz, DMSO-*d*₆)δ7.92(s,1H),7.02(s,1H),5.73(d,*J*=6.2Hz,1H),4.74(dt,*J*= 9.3,6.1Hz,1H) 3.79(s,6H),3.74-3.64(m,2H),3.06(s,2H),2.18(dd,*J*=13.6,6.1Hz,1H),1.78(ddt,*J*= 23.0,13.5,6.3Hz ,2H),1.72-1.64(m,2H),1.57(ddd,*J*=13.7,11.3,4.7Hz,1H),1.40(s,9H)
LC-MS(ESI):[M-H]⁺ =464.39

Step 6: The intermediate 1f (3.0 g, 6.9 mmol) and triethylsilane (3.2 g, 27.6 mmol) were dissolved in trifluoroacetic acid (30 mL), and the mixture was stirred overnight at 80°C. The resulting mixture was concentrated under vacuum and purified by C18 reversed-phase column to obtain colorless oily intermediate 1g (1.8 g, 83%).
¹H NMR(400MHz, CDCl3 )δ7.53(s,1H),7.11(s,1H),6.51(d,*J*=9.9Hz,1H),5.72(d,*J*= 9.8Hz,1H),4.66(s ,2H),3.93(s,3H),3.89(s,3H),3.42-3.31(m,4H),2.23(d,*J*=14.6Hz,2H),2.12-2.02(m,2H)
LC-MS(ESI):[M+H]⁺ =318.25

Step 7: The intermediate 1g (1.6g, 5.0mmol) was dissolved in methanol (20mL) and then palladium on carbon (0.2g) was added. The reaction solution was stirred at room temperature under hydrogen overnight. The mixture was filtered and spun dry to obtain a yellow oily intermediate 1h (1.3g, 81%).
¹H NMR(400MHz, CDCl₃ )δ7.61(s,1H),7.14(s,1H),3.93(s,3H),3.89(s,3H),3.35(d,*J*=6.2Hz,4H),3.30 (s,2H),2.87(t,*J*=6.8Hz,2H),2.05-2.00(m,2H),1.95(t,*J*=6.7Hz,2H)
LC-MS(ESI):[M+H]⁺ =320.28

Step 8: The intermediate 1h (1.5 g, 4.7 mmol) was dissolved in a suspension of methanol and water, and lithium hydroxide (1.7 g, 70.5 mmol) was added. The reaction solution was stirred at room temperature overnight. The reaction mixture was concentrated in vacuo. The crude product is used directly in the next step without further purification.
LC-MS(ESI):[M+H]⁺ =292.21

Step 9: The intermediate 1i (1.3 g, 7.7 mmol) was dissolved in acetic acid (15 mL) solution, 3-aminopiperidine-2,6-dione hydrochloride (1.52 g, 9.2 mmol) and sodium acetate (2.1 g, 15.5 mmol) were added, and the reaction solution was stirred at 110 °C for 4 hours. After the reaction was completed, the mixture was purified by reversed-phase column to obtain a white solid intermediate 1 (1.6 g, 81%).
¹H NMR(400MHz, DMSO-*d*₆)311.10(s,1H),7.74(s,1H),7.38(s,1H),5.10(dd,*J*=12.9,5.4Hz,1H), 3.28-3.14(m,4H),2.95(t,*J*=6.8Hz,2H),2.91-2.83(m,1H),2.65-2.53(m,2H),2.07-1.99(m,1H),1.93(q,*J*= 6.2Hz,4H),1.88-1.78(m,2H).
LC-MS(ESI):[M+H]⁺ =384.32.

### Example 2

### Synthesis of intermediate 2

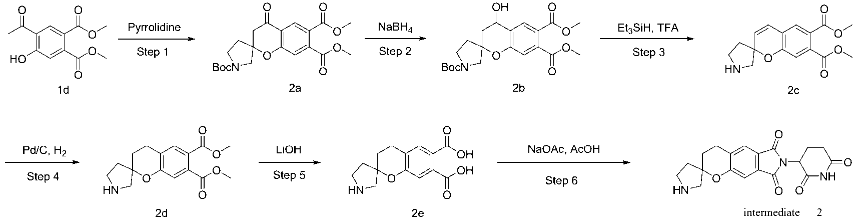

Step 1: The intermediate 1d was prepared by referring to steps 1 to 3 of Example 1, the prepared intermediate 1d (5.0 g, 19.8 mmol), 1-tert-butyloxycarbonyl-3-pyrrolidone (1.4 g, 19.8 mmol) and tetrahydropyrrole (4.0 g, 19.8 mmol) were dissolved in methanol (50 mL), and the mixture was stirred at 70°C Overnight, spun dry and purified by column chromatography (PE:EA=0-35%) to obtain a yellow oily intermediate 2a (5.0 g, 60%).
¹H NMR(400MHz,CDCl₃ )δ8.44(s,1H),7.18(s,1H),3.95(s,3H),3.91(s,3H),3.89-3.83(m,1H),3.76-3.64( m,1H), 3.62-3.50(m,1H),3.40(dd,*J*=17.4,12.4Hz,1H),3.06-2.86(m,2H),2.36-2.25(m,1H),1.97(ddd, *J*13.5,10.4,9.0Hz,1H), 1.47(d,9H)
LC-MS(ESI):[M-H]⁺ =418.40

Step 2: Sodium borohydride (0.7 g, 17.9 mmol) was added to a solution of intermediate 2a (5.0 g, 11.5 mmol) in methanol (50 mL) under ice bath conditions. The reaction solution was stirred at 70 °C overnight. The resulting mixture was concentrated under vacuum and purified by column chromatography (PE: EA = 0-50%) to obtain a white solid intermediate 2b (5.0 g, 99%). The resulting mixture is directly used for the next reaction.
LC-MS(ESI):[M+Na]⁺ =444.33

Step 3: The intermediate 2b (3.0 g, 7.1 mmol) and triethylsilane (3.3 g, 28.5 mmol) were dissolved in trifluoroacetic acid (30 mL), and the mixture was stirred overnight at 80°C. The resulting mixture was concentrated under vacuum and purified by C18 reversed-phase column to obtain colorless oily intermediate 2c (1.1 g, 51%).
¹H NMR(600MHz, CDCl3 )δ7.53(s,1H),7.09(s,1H),6.61(d,*J*=9.8Hz,1H),5.79(d,*J*=9.9Hz,1H),4.53(br s,2H),3.91(s,3H),3.89(s,3H),3.70-3.55(m,3H),3.27(d,*J*=12.5Hz,1H),2.54(dd,*J*=14.2,6.4Hz ,1H),2.14(ddd,J= 13.8,11.0,6.5Hz,1H)
LC-MS(ESI):[M+H]⁺ =304.27

Step 4: The intermediate 2c (1.0 g, 3.3 mmol) was dissolved in methanol (20 mL) and palladium on carbon (0.1 g) was added. The reaction solution was stirred under hydrogen at room temperature overnight. The mixture was filtered and spun dry to obtain a yellow oily intermediate 2d (1.0 g, 99%).
¹H NMR(600MHz, CDCl3 )δ7.61(s,1H),7.09(s,1H),3.90(s,3H),3.88(s,3H),3.52(dtd,*J*=17.8,11.4,7.8Hz, 2H),3.44(dd,*J*=12.7,1.6Hz,1H),3.25(d,*J*=12.6Hz,1H),2.90(dtd,*J*=17.3,10.7,6.9Hz,2H),2.28-2.23(m ,1H),2.14-2.10(m,2H),2.08-2.02(m,1H)
LC-MS(ESI):[M+H]⁺ =306.22

Step 5: The intermediate 2d (1.0 g, 3.3 mmol) was dissolved in methanol and water, and lithium hydroxide (1.2 g, 49.1 mmol) was added. The reaction solution was stirred at room temperature overnight. The reaction mixture was concentrated in vacuo. The crude product is used directly in the next step without further purification.
LC-MS(ESI):[M+H]⁺ =278.22

Step 6: The intermediate 2e (801 mg, 4.9 mmol) was dissolved in acetic acid (5 mL) solution, and 3-aminopiperidine-2,6-dione hydrochloride (0.97 g, 6.0 mmol) and sodium acetate (1.3 g, 9.7 mmol) were added, the reaction solution was stirred at 110 °Cfor 4 hours. After the reaction was completed, the mixture was purified by reversed-phase column to obtain a white solid intermediate 2 (312 mg, 26%).
¹H NMR(400MHz, DMSO-*d*₆)δ11.11(s,1H),7.78(s,1H),7.18(s,1H),5.10(dd,*J*=12.9,5.4Hz,1H),3.50-3.21( m,4H),3.03-2.84(m,4H),2.66-2.53(m,2H),2.24-2.00(m,4H)
LC-MS(ESI):[M+H]⁺ =370.34

### Example 3

### Synthesis of intermediate 3

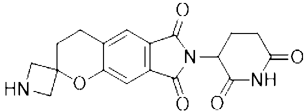

7'-(2,6-dioxapiperidin-3-yl)-3',4'-dihydro-6'H-spiro[azetidine-3,2'-pyrano[2,3-f]isoindole]-6',8'(7'H)-dione was prepared by similar procedures as Step 1 to Step 9 of Example 1.
¹H NMR(600MHz, DMSO-*d*₆)δ11.12(s,1H),9.12(d,*J*=49.0Hz,2H,NH),7.76(s,1H),7.30(s,1H),5.10(dd, *J*= 12.9,5.4Hz,1H),4.16(t,*J*=8.7Hz,4H),2.97(t,*J*=6.5Hz,2H),2.88(ddd,*J*=17.0,13.9,5.5Hz,1H), 2.59(dt,*J*= 17.1,3.1Hz,1H),2.54(dd,*J*=13.1,4.5Hz,1H),2.22(t,*J*=6.5Hz,2H),2.06-2.00(m,1H).
LC-MS(ESI):[M+H]⁺ =356.26

### Example 4

### Synthesis of intermediate 4

### Synthesis scheme

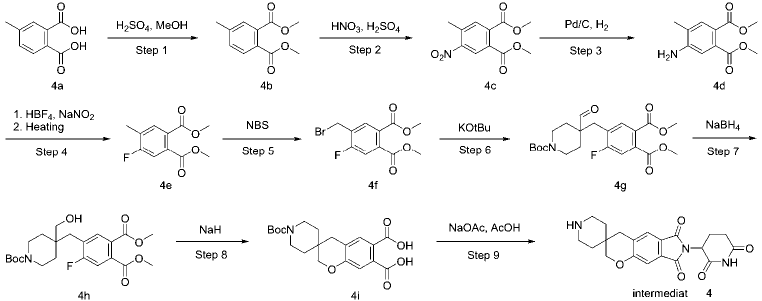

Step 1: The intermediate 4a (10 g, 55.56 mmol) was dissolved in 100 mL of methanol, then 15 mL of concentrated sulfuric acid was added and reacted at room temperature overnight. The reaction solution was poured into ice water, extracted with ethyl acetate and concentrated to obtain colorless oily intermediate 4b (10.6 g, 91%), which is used directly in the next step without purification.
¹H NMR(400MHz, CDCl3 )δ7.66(d,*J*=7.9Hz,1H),7.46(d,*J*=1.7Hz,1H),7.32(dd,*J*=7.9,1.8,1H),3.89(s, 3H),3.88(s,3H),2.40(s,3H)

Step 2: The intermediate 4b (10 g, 48 mmol) was dissolved in 100 mL of concentrated sulfuric acid, then concentrated nitric acid (25 mL, 68%) was slowly added, and reacted at room temperature overnight. After the reaction was completed, the reaction solution was poured into ice water, extracted with ethyl acetate and concentrated. The crude product was purified by column chromatography to obtain a white solid intermediate 4c (5.5 g, 45%).
¹H NMR(400MHz, CDCl₃ )δ8.43(s,1H),7.64(s,1H),3.97(s,3H),3.96(s,3H),2.69(s,3H)

Step 3: The intermediate 4c (5.1g, 20mmol) was dissolved in 100mL methanol, and then 0.51g palladium on carbon was added, and the reaction system was replaced with nitrogen and hydrogen, and the reaction was allowed to conduct overnight at room temperature. After the reaction was completed, diatomaceous earth was used for filtration, and the filtrate was collected and concentrated to obtain a yellow oily intermediate 4d (4g, 91%), which was used directly in the next step without purification.
¹H NMR(400MHz,DMSO-*d*₆ )δ7.46(s,1H),6.68(s,1H),5.91(s,2H),3.75(s,3H),3.71(s,3H)
LC-MS(ESI):[M+Na]⁺ =246.18

Step 4: The intermediate 4d (4 g, 17.92 mmol) was added to 10% fluoroboric acid (32 ml), and the solution is suspended. After being stirred for 0.5 h, cooled to 0-5 °C, and 4 mL of NaNO₂ (1.36 g, 19.71 mmol) aqueous solution was slowly added for diazotization reaction, and stirred in an ice bath for 0.5 h. The tetrafluoroborate was filtered and the filter cake was collected, and the solid was dried under vacuum. Then the solution of tetrafluoroborate in toluene was placed in a 110 °C oil bath and stirred. After the reaction was completed, it was extracted with ethyl acetate, the organic phase was washed with water and saturated brine, and dried with anhydrous sodium sulfate. It was concentrated to obtain a crude product, which is purified by column chromatography to obtain a light yellow oily intermediate 4e (2.31 g, 57%).
¹H NMR(400MHz, CDCl₃ )δ7.61(dd,*J*=7.2,0.9Hz,1H),7.38(d,*J*=9.4Hz,1H), 3.92(s,3H), 3.91(s,3H), 2.35 (d,*J*=2.0Hz,3H)

Step 5: The intermediate 4e (2.26 g, 10 mmol) was dissolved in carbon tetrachloride, then N-bromosuccinimide (2.14 g, 12 mmol) and azobisisobutyronitrile (0.16 g6, 1 mmol) were added, heated toreflux overnight. After concentration, it was purified by reversed-phase column to obtain white solid intermediate 4f (2.14 g, 70%).
¹H NMR(400MHz, CDCl3 )δ7.87(d,*J*=7.1Hz,1H),7.40(d,*J*=9.4Hz,1H),4.51(d,*J*= 1.0Hz,2H),3.94(s,3H ),3.93(s,3H)

Step 6: The intermediate 4f (2.14 g, 7 mmol) was dissolved in anhydrous tetrahydrofuran (30 mL), and potassium tert-butoxide (1.18 g, 10.5 mmol) was slowly added at -30 °C. After reacting for 0.5 h, a solution of 1-tert-butyloxycarbonylpiperidine-4-carboxaldehyde (1.79 g, 8.4 mmol) in tetrahydrofuran (5 mL) was added dropwise at the same temperature. The reaction was brought to room temperature and stirred overnight. The reaction system was quenched with saturated ammonium chloride and extracted with ethyl acetate. The organic phase was washed with saturated brine, dried with anhydrous sodium sulfate, concentrated, and purified by rapid silica gel column chromatography to obtain a colorless oily intermediate 4g (1.68 g, 55%).
¹H NMR(400MHz, CDCl₃ )δ9.58(d,*J*=2.3Hz,1H),7.50(d,*J*=7.1Hz,1H),7.34(d,*J*=9.5Hz,1H),3.96-3.84(m ,8H),2.91-2.74(m,4H),1.96(d,*J*=13.0Hz,2H),1.59-1.46(m,2H),1.44(s,9H)
LC-MS(ESI):[M-Boc+H]⁺=338.29

Step 7: The intermediate 4g (1.66g, 3.8mmol) was dissolved in methanol (15mL), and sodium borohydride (0.215g, 5.7mmol) was added under ice bath conditions, and stirred at room temperature for 3h. It was quenched with saturated ammonium chloride, extracted with ethyl acetate, the organic phase was washed with saturated brine, dried with anhydrous sodium sulfate, concentrated, and purified by column chromatography to obtain a colorless oily intermediate 4h (1.47g, 88%).
¹H NMR(400MHz, DMSO-*d*₆)δ7.74(d,*J*=7.0Hz,1H),7.54(d,*J*=9.7Hz,1H),4.81(t,*J*= 5.0Hz, 1H),3.82(s 3H),3.82(s,3H),3.48-3.39(m,2H),3.21(d,*J*= 5.0Hz,4H),2.74(s,2H),1.42-1.32(m,11H),1.27-1.14(m,2H)
LC-MS(ESI):[M-Boc+H]⁺=340.41

Step 8: The intermediate 4h (1.45 g, 3.3 mmol) was dissolved in dry N,N-dimethylformamide (6 mL), sodium hydride (0.4 g, 16.5 mmol) was added, and reacted at 110°C for 2 h. After the reaction was completed, it was quenched with acetic acid and purified with a reversed-phase column to obtain a white solid intermediate 4i (0.65 g, 51%).
¹H NMR(400MHz,DMSO-*d*₆ )δ12.93(s,2H),7.50(s,1H),6.92(s,1H),3.99(s,2H),3.53-3.42(m,2H),3.33-3.23(m,2H),2.75(s,2H),1.40(s,9H),1.39-1.28(m,4H)
LC-MS(ESI):[M-Boc+H]⁺=292.23

Step 9: The intermediate 4i (0.63 g, 1.6 mmol) was dissolved in acetic acid (4 mL), and then 3-amino-2,6-piperidindione hydrochloride (0.33 g, 2.0 mmol) and sodium acetate (0.39 g, 4.8 mmol) were added, and reacted overnight at 110°C. After the reaction was completed, it was purified by reversed-phase column to obtain a white solid intermediate 4 (0.5 g, 82%).
¹H NMR(400MHz,DMSO-*d*₆ )δ11.12(s,1H),8.51(s,2H),7.69(s,1H),7.29(s,1H),5.10(dd,*J*=12.7,5.4Hz, 1H),4.16(s,2H),3.25-3.05(m,4H),2.92(s,2H),2.90-2.82(m,1H),2.64-2.52(m,2H),2.05-1.99(m, 1H),1.67-1.51(m,4H)
LC-MS(ESI):[M+H]⁺ =384.36

### Example 5

### Synthesis of intermediate 5

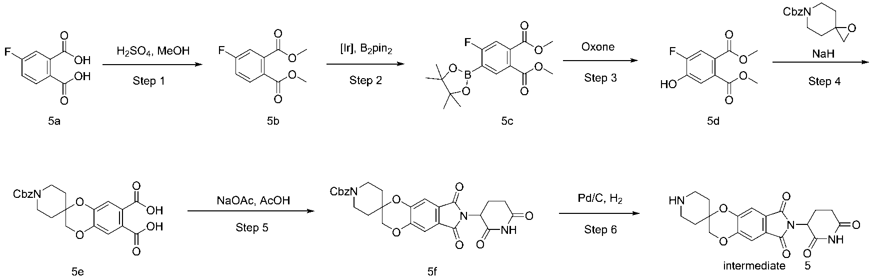

Step 1: Concentrated sulfuric acid (15 mL) was slowly added to a solution of intermediate 5a (5.5 g, 30.0 mmol) in methanol (100 mL). The reaction solution was stirred overnight at room temperature. The reaction solution was poured into ice water, extracted with ethyl acetate, the organic phases were combined, washed with saturated brine, and dried with anhydrous sodium sulfate. Filtered and concentrated under reduced pressure to obtain a colorless oily liquid intermediate 5b (5.7 g, 90%), which was used in the next step without purification.
¹H NMR(400MHz, CDCl3 )δ7.82(dd,*J*=8.6,5.3Hz,1H),7.38(dd,*J*=8.6,2.6Hz,1H),7.82(ddd,*J*= 8.6,5.3,2.6Hz, 1H),3.96(s,3H),3.92(s,3H)

Step 2: Bis(pinacolato)diboron (4.5g, 17.5mmol), 4,4'-di-tert-butyl-2,2'-bipyridine (0.13g, 0.47mmol) and methoxy(cyclooctadiene) iridium dimer (0.16g, 0.23mmol) were added to 10ml of methyl tert-butyl ether solution, a solution of intermediate 5b (2.5g, 11.8mmol) in methyl tert-butyl ether (10mL) was added, replaced with nitrogen, and the reaction solution was stirred at 100°C for 4h. After the reaction was completed, it was filtered through diatomaceous earth, the filtrate was concentrated to obtain a crude product (3.2g) that can be used for the next step without purification.

Step 3: Potassium peroxymonosulfonate (6.15g) was added to a solution of intermediate 5c (3.38g, 10mmol) in acetonitrile (50mL), and the reaction solution was stirred at room temperature overnight. The reaction solution was filtered and the filtrate was concentrated. The crude product was purified by silica gel column chromatography to obtain a white solid intermediate 5d (1.6 g, 70%).
¹H NMR(400MHz, CDCl3 )δ8.39(s,1H),7.50(d,*J*=10.8Hz,1H),7.19(d,*J*=8.1Hz,1H),3.88(s,3H),3.86(s ,3H).
LC-MS(ESI):[M+H]⁺ =229.18

Step 4: The intermediate 5d (23 mg, 0.1 mmol) was dissolved in 1 ml of ultra-dry N,N-dimethylformamide, then 1-oxa-6-azaspiro[2.5]octane-6-carboxylic acid benzyl ester (25 mg, 0.1 mmol) and sodium hydride (6 mg, 0.25 mmol) were added, and the reaction solution was heated to 110°C and stirred for 2 days. The reaction solution was purified by reversed-phase column to obtain a white solid intermediate 5e (13 mg, 31%).
¹H NMR(600MHz, CD3 OD)δ7.41-7.35(m,4H),7.33(dd,*J*=5.9,2.9Hz,1H),7.29(s,1H),7.27(s,1H),5.15(s, 2H),4.07(s,2H),4.01(dt,*J*=13.6,4.0Hz,2H),3.42-3.32(m,2H),1.82(d,*J*=13.9Hz,2H),1.76-1.67(m ,2H).
LC-MS(ESI):[M+H]⁺ =428.36

Step 5: 3-aminopiperidine-2,6-dione hydrochloride (6 mg, 0.036 mmol) and sodium acetate (7 mg, 0.09 mmol) were added to a solution of intermediate 5e (13 mg, 0.03 mmol) in acetic acid (1 mL), and the reaction solution was heated to 110°C and stirred for 4 hours. After the reaction was completed, the reaction solution was purified by reversed-phase column to obtain a white solid intermediate 5f (12 mg, 77%).
¹H NMR(600MHz, CD3 OD)δ7.42(s,1H),7.40-7.31(m,6H),5.15(s,2H),5.08(dd,*J*=12.9,5.5Hz,1H),4.14(s, 2H),4.01(dt,*J*=13.8,4.0Hz,2H),3.43-3.32(m,2H),2.91-2.83(m,1H),2.79-2.69(m,2H),2.14-2.09(m, 1H), 1.83 (d, *J* =13.9Hz, 2H), 1.74 (td, *J=*14.4*,* 12.9, 4.8Hz, 2H).
LC-MS(ESI):[M+H]⁺ =520.29

Step 6: Palladium on carbon (5 mg) was added to a solution of intermediate 5f (11 mg, 0.02 mmol) in methanol, replaced with hydrogen, and the reaction solution was stirred under hydrogen for 6 hours. The reaction solution was filtered through diatomaceous earth, and the filtrate was concentrated to obtain a white solid intermediate 5 (7 mg, 90%).
¹H NMR(400MHz, DMSO-*d*₆)δ11.11(s,1H),7.50(s,1H),7.48(s,1H),5.09(dd,*J*=12.8,5.3Hz,1H),4.27(s, 2H),3.25(d,*J*=12.7Hz,2H),3.18-3.11(m,2H),2.89(ddd,*J*=16.7,13.7,5.3Hz,1H),2.64-2.53(m,2H),2.03 (ddd,J= 13.3,5.7,3.4Hz,1H),1.92-1.86(m,4H).
LC-MS(ESI):[M+H]⁺ =386.32

### Example 6

### Synthesis of intermediate 6

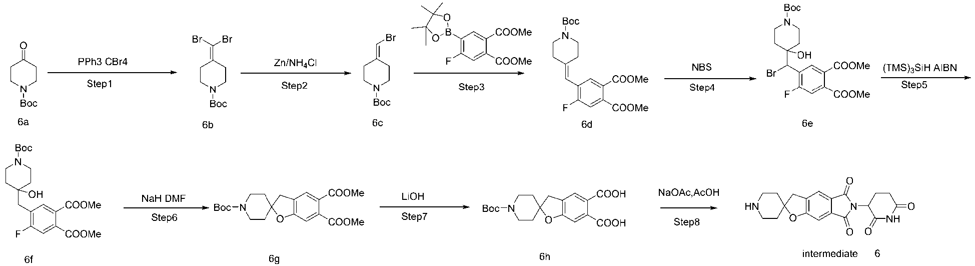

Step 1: Under the protection of nitrogen, an intermediate 6a (5 g, 25.1 mmol) and triphenylphosphine (26.3 g, 100.4 mmol) were dissolved in anhydrous acetonitrile (50 mL), and carbon tetrabromide (16.7 g, 50.2 mmol) was added to the reaction system in batches at 0°C. After reacting for 30 minutes, the reaction system was warmed to room temperature and reacted overnight. After the reaction was completed, the reaction system was filtered, and the filtrate was concentrated under reduced pressure to obtain a crude product, which was purified by column chromatography to obtain a white solid intermediate 6b (5.5 g, 62%).
¹H NMR (600MHz, DMSO-*d*₆) δ3.37 (s, 4H), 2.41 (dd, *J =* 7.2, 4.7Hz, 4H), 1.41 (d, *J =* 0.9Hz, 9H).

Step 2: Under the protection of nitrogen, the intermediate 6b (5.2 g, 18.9 mmol) was dissolved in tetrahydrofuran (34 mL) and methanol (17 mL). At 0°C, ammonium chloride (4.04 g, 75.6 mmol) was added to the reaction system. After reacting at 0°C for 30 min, zinc powder (4.9 g, 75.6 mmol) was added in batches and reacted overnight at room temperature. After the reaction was completed, the reaction system was filtered, the filter cake was washed with methanol, and the filtrate was spun dry under reduced pressure. The concentrated crude product was purified by column chromatography to obtain a white oily intermediate 6c (2.6 g, 50%).
¹H NMR(600MHz, DMSO-*d*₆)δ6.26(t,*J*=1.3Hz,1H),3.34(dt,*J*=16.4,6.3Hz,4H),2.32-2.27(m,2H),2.23(ddd, *J*=7.2,4.4,1.2Hz,2H),1.41 (s,9H).

Step 3: Dimethyl 4-(1-(tert-butyloxycarbonyl)piperidin-4-ylidene)methyl)-5-fluorophthalate (Intermediate 6d)

Under the protection of nitrogen, the intermediate 6c (2g, 7.3mmol), the intermediate 5c (2.64g, 7.8mmol) prepared by steps 1 to 2 of Example 5, palladium acetate (163mg, 0.73mmol), triphenylphosphine (382mg, 1.46mmol), cesium carbonate (7.14g, 21.9mmol) were added sequentially to the reaction flask, 1,4-dioxane (20mL) and water (2mL) were added, and after nitrogen replacement, the mixture was reacted at 110°C for 4 hours. After the reaction, the reaction system was cooled to room temperature, water was added, and then extracted with ethyl acetate. The organic phase was washed with saturated brine and dried with anhydrous sodium sulfate. The concentrated crude product was purified by column chromatography to obtain a light yellow oily intermediate 6d (1.4g, 47%).
¹H NMR(600MHz,DMSO-*d*₆ )δ7.65(d,*J*=7.0Hz,1H),7.60(d,*J*=9.7Hz,1H),6.32(s,1H),3.83(s,3H),3.82 (s,3H),3.43(t,*J*=5.8Hz,2H),3.35(s,1H),3.33(s,1H),2.37-2.32(m,2H),2.25(t,*J*=5.9Hz, 2H),1.42(s,9H).
LC-MS(ESI):[M-Boc+H]⁺ =308.28

Step 4: Under the protection of nitrogen, the intermediate 6d (7 g, 17.2 mmol) was dissolved in tetrahydrofuran (140 mL) and water (140 mL), and N-bromosuccinimide (6.12 g, 34.4 mmol) was added, and the reaction was allowed to proceed overnight at room temperature. After the reaction was completed, the excess tetrahydrofuran was removed by concentration under reduced pressure, and water was added, followed by extraction with ethyl acetate. The organic phase was washed with saturated brine and dried with anhydrous sodium sulfate. The concentrated crude product was purified by column chromatography to obtain a light yellow oily intermediate 6e (4.9 g, 56%).
LC-MS(ESI):[M-Boc+H]⁺ =404.30/406.30

Step 5: Under the protection of nitrogen, the intermediate 6e (10.9 g, 21.61 mmol) was dissolved in toluene (80 ml), and tris(trimethylsilyl)silane (8.06 g, 32.42 mmol) and azobisisobutyronitrile (357 mg, 2.16 mmol) were added, and the mixture was reacted overnight at 90°C. After the reaction, the excess toluene was removed by concentration under reduced pressure, and the concentrated crude product was purified by column chromatography to obtain a light yellow oily intermediate 6f (1.5 g, 16%).
¹H NMR(600MHz,DMSO-*d*₆ )δ7.77(d,*J*=6.9Hz,1H),7.52(d,*J*=9.5Hz,1H),4.62(s,1H),3.82(d,*J*= 2.1Hz ,6H),3.66(s,2H),3.34(s,4H),2.79(s,2H),1.38(s,9H).
LC-MS(ESI):[M-Boc+H]⁺=326.37

Step 6: Under the protection of nitrogen, the intermediate 6f (1.6 g, 3.76 mmol) was dissolved in N, N-dimethylformamide (2 ml), sodium hydride (300 mg, 7.52 mmol) was added, and the reaction was continued overnight at 110 °C. After the reaction was completed, water was added, and then extracted with ethyl acetate. The organic phase was washed with saturated brine and dried with anhydrous sodium sulfate. The concentrated crude product was purified by reversed-phase column to obtain a light yellow solid intermediate 6g (660 mg, 43%).
¹H NMR(600MHz, CDCl3 )δ7.58(s,1H),7.15(s,1H),3.93(s,3H),3.89(s,3H),3.56(t,*J*=5.7Hz,2H),3.42 (t,*J*= 5.8Hz,2H),2.42(t,*J*=5.9Hz,2H),2.24(t,*J*=5.9Hz,2H),1.61(s,2H),1.50(s,9H).
LC-MS(ESI):[M-Boc+H]⁺ =306.23

Step 7: Under the protection of nitrogen, the compound 6g (660 mg, 1.63 mmol) was dissolved in methanol (9 ml) and water (1 ml), and then the lithium hydroxide (389 mg, 16.28 mmol) was added, and the reaction was continued at room temperature overnight. The concentrated crude product was purified by reversed-phase column to obtain a light yellow solid intermediate 6h (600 mg, 97%).
¹ H NMR(400MHz, DMSO-*d*₆)δ7.39(s,1H),6.37(s,1H),3.17(s,2H),2.29(d,*J*=51.9Hz,4H),1.98(dd,*J*= 13.7,6.9Hz,2H),1.64(s,2H),1.41(s,9H).
LC-MS(ESI):[M-Boc+H]⁺ =278.26

Step 8: The intermediate 6h (400 mg, 1.06 mmol) was dissolved in acetic acid (5 mL) solution, 3-aminopiperidine-2,6-dione hydrochloride (169 mg, 1.32 mmol) and sodium acetate (260 mg, 3.18 mmol) were added, reacted at 110 °C, and stirred for 4 hours. After the reaction was completed, the mixture was purified by reversed-phase column to obtain a white solid intermediate 6 (170 mg, 43%).
¹H NMR(400MHz, DMSO-*d*₆)δ7.46(s,1H),7.11(s,1H),5.04(dd,*J*=12.8,5.4Hz,1H),2.99-2.79(m,5H),2.62-2.54(m,1H),2.53(s,1H),2.38(q,*J*=6.3Hz,4H),2.04-1.96(m,1H),1.90(s,2H).
LC-MS(ESI):[M+H]⁺ =370.34

### Example 7

### Synthesis of intermediate 7

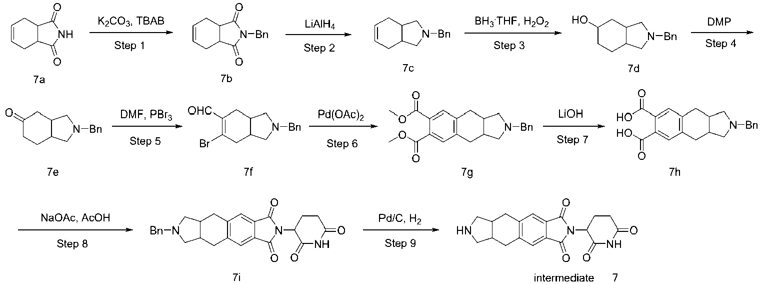

Step 1: The intermediate 7a (5 g, 33 mmol) was dissolved in anhydrous acetonitrile (50 ml). Tetrabutylammonium bromide (1.23 g, 3.3 mmol) and anhydrous potassium carbonate (14 g, 99 mmol) was added to the solution. Benzyl chloride (5.5 g, 43 mmol) was slowly added to the system and reacted at 30 °C overnight. Water was added to the reaction system and extracted with ethyl acetate. The organic phase was washed with saturated brine and dried with anhydrous sodium sulfate, and concentrated to obtain a white solid intermediate 7b (7.5 g, 94%).
¹H NMR(400MHz, CDCl3 )δ7.37-7.20(m,5H),5.95-5.83(m,2H),4.63(s,2H),3.20-3.01(m,2H),2.61(m,2H), 2.31-2.10(m,2H).
LC-MS(ESI):[M+H]⁺ =242.22

Step 2: At 0°C, lithium aluminum tetrahydride (3.2 g, 82 mmol) was added to 40 mL of anhydrous tetrahydrofuran, and then the intermediate 7b (5 g, 20.7 mmol) was added. After ice bathing for 5 min, the reaction system was moved to a 70°C oil bath and reacted for 3 h. After the reaction was completed, water was slowly added dropwise to the reaction solution under ice bath conditions until no bubbles were generated. The filtrate was collected by filtration, extracted with ethyl acetate, and the organic phase was washed with saturated brine and dried with anhydrous sodium sulfate, concentrated to obtain a yellow oily intermediate 7c (3.2 g, 73%).
¹H NMR(400MHz, CDCl3 )δ7.43-7.24(m,5H),5.87(t,*J*=2.7Hz,2H),3.67(s,2H),3.05-2.90(m,2H),2.44(m, 2H),2.29-2.16(m,4H),1.92(m,2H)

Step 3: At 0°C, the intermediate 7c (5 g, 23.44 mmol) was dissolved in 20 mL of tetrahydrofuran, and 25 mL of 2M borane-tetrahydrofuran complex was added under ice bath conditions. After reacting for 12 h, 13 mL of anhydrous methanol, 10.5 mL of 3M NaOH solution, and 10.5 mL of hydrogen peroxide were added to the reaction solution, and the reaction was continued at 60°C for 6 h. After the reaction was completed, the mixture was cooled to room temperature and extracted with ethyl acetate. The organic phase was washed with saturated brine and dried with anhydrous sodium sulfate. The crude product was obtained through concentration and purified by column chromatography to obtain the intermediate 7d (1.2 g, 22.2%).
¹H NMR(400MHz, CDCl3 )δ7.43-7.18(m,5H),3.84(m,1H),3.78(s,2H),2.94(dd,*J*=9.8,6.7Hz,1H),2.82(dd, *J =* 9.5,7.7Hz,1H),2.64(dd,*J*=9.5,8.4Hz,1H),2.60-2.44(m,2H),2.12(m,1H),1.89-1.72(m,3H),1.54 (m,1H),1.47-1.35(m,1H),1.35-1.22(m,2H).
LC-MS(ESI):[M+H]⁺ =232.31

Step 4: The intermediate 7d (1.2 g, 12.97 mmol) was dissolved in anhydrous dichloromethane, Dess-Martin periodinane (11 g, 25.95 mmol) was added under ice bath conditions, and reacted for 12 h. The reaction was quenched with a 1:1 (v/v) mixture of saturated sodium bicarbonate and saturated sodium thiosulfate, the filtrate was collected after filtration, and extracted with dichloromethane. The organic phase was washed with saturated brine, combined and dried with anhydrous sodium sulfate, then oncentrated to obtain the crude intermediate 7e (1 g). The crude product is directly used in the next step.

Step 5: N,N-dimethylformamide (1.01mL, 13.08mmol) was dissolved in 2mL dichloromethane at 0°C. Phosphorus tribromide (1.13mL, 11.77mmol) was slowly added dropwise and stirred at 0°C for 1 Hour. The dichloro solution of intermediate 7e (1g, 2.62mmol) was added dropwise to the above system, warmed to room temperature and reacted for 10h. After the reaction was completed, the reaction solution was placed at 0°C and saturated sodium bicarbonate was added until no bubbles were generated. The reaction solution was extracted with dichloromethane. The organic phase was washed with saturated brine and dried with anhydrous sodium sulfate, concentrated to obtain a crude product, which is purified by column chromatography to obtain the target compound intermediate 7f (270mg, 20%).
¹H NMR(400MHz, CDCl3 )δ10.04(s,1H),7.32(d,*J*=5.8Hz,5H),3.60(s,2H),3.21-3.10(m,2H),2.85-2.72(m, 3H),2.50-2.43(m,1H),2.33(dd,*J*=9.2,5.0Hz,1H),2.24-2.18(m,1H),1.83-1.74(m,2H)
LC-MS(ESI):[M+H]⁺ =320.16

Step 6: The intermediate 7f (270 mg, 0.84 mmol), dimethyl itaconate (133.4 mg, 0.84 mmol), palladium acetate (9.5 mg, 0.042 mmol), triphenylphosphine (22.11 mg, 0.084 mmol), sodium acetate (207.5 mg, 2.53 mmol) were added sequentially to a pressure vessel, dissolved with tetrahydrofuran, replaced with nitrogen, heated to 120 °C and reacted overnight. After the reaction system was cooled to room temperature, tetrahydrofuran was spun dry, and then separated and purified by preparative liquid chromatography to obtain the intermediate 7g (71 mg, 22%).
¹ H NMR(400MHz, CDCl3 )δ7.52-7.39(m,7H),4.38-4.14(m,3H),3.91(d,*J*=2.8Hz,7H),3.27(s,3H),2.94(d, *J =* 12.0Hz,1H),2.85-2.75(m,3H),1.55(d,*J*=6.6Hz,1H)
LC-MS(ESI):[M+H]⁺ =380.36

Step 7: The intermediate 7g (71mg, 0.19mmol) was dissolved in methanol and water, lithium hydroxide (90mg, 3.74mmol) was added, and reacted at room temperature for 20h. After the reaction, the water and methanol were spun dry to obtain a crude product (62mg, 95%). The crude product is directly used in the next step.
LC-MS (ESI): [M+H]⁺ =352.34

Step 8: The intermediate 7h (62 mg, 0.18 mmol) was dissolved in glacial acetic acid, sodium acetate (43.2 mg, 0.53 mmol) and 3-amino-2,6-piperidinedione hydrochloride (44 mg, 0.26 mmol) were added, and reacted overnight in an oil bath at 110°C. Then, methanol and water were spun dry, and the reaction solution was purified by preparative liquid chromatography to obtain the intermediate 7i (39 mg, 49%).
¹H NMR(400MHz, DMSO-*d*₆)δ11.14(s,1H),7.98-7.72(m,2H),7.64-7.41(m,5H),5.14(dd,*J*=12.9,5.4Hz,1H), 4.53-4.24(m,2H),4.01(dd,*J*=13.9,7.6Hz,1H),3.76(s,3H),3.12(d,*J*=9.7Hz,1H),3.06-2.91(m,2H) ,2.92-2.76(m,3H),2.67-2.54(m,2H),2.04(dd,*J*=12.5,6.2Hz,1H),1.90-1.59(m,2H)
LC-MS(ESI):[M+H]⁺ =444.41

Step 9: The intermediate 7i (39 mg, 0.088 mmol) was dissolved in 2 mL methanol, 4 mg palladium on carbon was added, replaced with hydrogen, and reacted at room temperature overnight. The palladium on carbon was removed by filtration and the methanol was spun dry, and the reaction solutuion was purified by preparative liquid chromatography to obtain the intermediate 7 (12 mg, 38%).
¹H NMR(400MHz, DMSO-*d*₆)δ11.13(s,1H),9.07-8.95(m,1H),7.95-7.68(m,2H),5.14(dd,*J*= 13.0,5.3Hz,1H),3.96-3.87(m,2H),3.51(m,2 H),3.12(m,1H),3.04-2.82(m,4H),2.72-2.55(m,2H),2.06(m,1H),1.86(m,1H),1.65-1.46(m,1H)
LC-MS(ESI):[M+H]⁺ =354.41

### Example 8

### Synthesis of intermediate 8

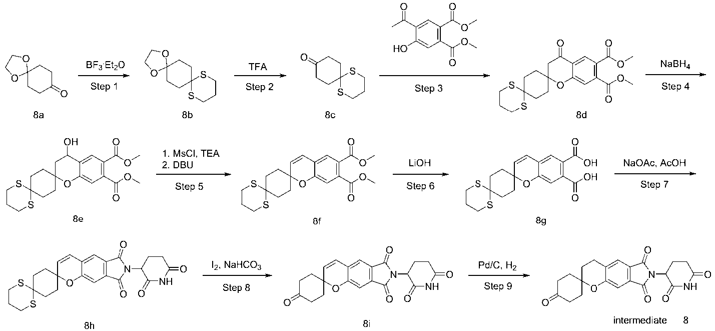

Step 1: The intermediate 8a (10.0 g, 64.0 mmol) was dissolved in 200 mL of dichloromethane, then 1,3-propanedithiol (7.0 g, 64.0 mmol) was added. 32 mmol of boron trifluoride ether solution was added dropwise at - 18 °C. After the addition was complete, the mixture was stirred at -18 °C for 4 h. After the reaction was complete, the solvent was removed under reduced pressure, 500 mL of water was added, the solid was filtered and purified by C18 column chromatography to obtain the intermediate 8b (2 g, 12.7%).
¹H NMR(400MHz,DMSO-*d*₆ )δ3.85(s,4H),2.84-2.78(m,4H),2.06-1.98(m,4H),1.93-1.80(m,2H), 1.70-1.59(m ,4H)
LC-MS(ESI):[M+H]⁺ =247.07

Step 2: The intermediate 8b (2.0 g, 8.0 mmol) was dissolved in 200 mL of dichloromethane, and then 100 mL of trifluoroacetic acid was added at room temperature. After the addition, the mixture was stirred at room temperature for 4 h. After the reaction was completed, sodium bicarbonate (aq) solution was added to the system, and the pH was adjusted to 7. After extraction, the organic phase was dried, concentrated, and purified by column chromatography to obtain the intermediate 8c (1.5 g, 91.0%).
¹H NMR (400 MHz, DMSO-*d*₆)δ2.93-2.84 (m, 4H), 2.41-2.33 (m, 4H), 2.31-2.25 (m, 4H), 1.94-1.88 (m, 2H)
LC-MS(ESI):[M+H]⁺ =203.05

Step 3: The intermediate 8c (2.0 g, 9.9 mmol) was dissolved in 200 mL tetrahydrofuran, then the intermediate 1d (2.0 g, 9.8 mmol) and tetrahydropyrrole (2.0 g, 28.0 mmol) were added at room temperature. After the addition, the mixture was stirred at 70°C for 4 h. After the reaction was completed, the system was decompressed to remove the solvent, and the residue was separated and purified by column chromatography to obtain the intermediate 8d (2.0 g, 46.0%).
¹H NMR(400MHz,DMSO-*d*₆ )δ8.18(s,1H),7.35(s,1H),3.83(s,3H),3.81(s,3H),2.92(s,2H),2.90-2.85(m,2H),2.81-2.74(m,2H),2.14 -2.10(m,2H),2.02-1.77(m,8H).
LC-MS(ESI):[M+H]⁺ =437.10

Step 4: The intermediate 8d (2.0 g, 4.5 mmol) was dissolved in 50 mL methanol and 50 mL tetrahydrofuran, then sodium borohydride (350.0 mg, 9.0 mmol) was added at room temperature. After the addition, the mixture was stirred at 70°C for 4 h. After the reaction was completed, the system was decompressed to remove the solvent, and the residue was separated and purified by column chromatography to obtain the intermediate 8e (1.5 g, 75.0%).
¹H NMR(600MHz, DMSO-*d*₆)δ7.90(s,1H),7.00(s,1H),5.68(d,*J*=6.2Hz,1H),4.77-4.71(m,1H),3.79(s, 6H),2.96-2.72(m,4H),2.16-2.01 (m,4H), 1.93-1.79(m,5H), 1.77-1. 70(m,3H)
LC-MS(ESI):[M+H]⁺ =439.12

Step 5: The intermediate 8e (700 mg, 1.5 mmol) was dissolved in 200 mL of dichloromethane, and then triethylamine (480.0 mg, 4.5 mmol) and 4-dimethylaminopyridine (100.0 mg, 0.8 mmol) were added at room temperature. Sulfonyl chloride (720.0 mg, 6.0 mmol) was added dropwise thereto at 0°C. After the addition, the mixture was stirred at 0°C for 4 h, and the reaction was monitored by TLC. After the reaction was completed, the solvent was removed under reduced pressure. After the addition was completed, the residue was dissolved in 100 mL of toluene, and then 1,8-diazabicycloundec-7-ene (1.6 g, 10.0 mmol) was added at room temperature. The mixture was stirred at 110°C for 16 h. After the reaction was completed, the system was decompressed to remove the solvent, and the residue was separated and purified by column chromatography to obtain the intermediate 8f (400 mg, 59.0%).
¹ H NMR(400MHz, DMSO-*d*₆)δ7.58(s,1H),7.05(s,1H),6.59(d,*J*=9.9Hz,1H),5.95(d,*J*=11.4Hz,1H),3.78 (s,3H),3.77(s,3H),2.93-2.85(m,2H),2.82-2.73(m,2H),2.18-2.11(m,2H),2.09-1.96(m,2H),1.92 -1.77(m,6H)
LC-MS(ESI):[M+H]⁺ =421.11

Step 6: The intermediate 8f (400.0 mg, 0.9 mmol) was dissolved in 50 mL methanol, 50 mL tetrahydrofuran, and 50 mL water, and then lithium hydroxide (120.0 mg, 4.5 mmol) was added at room temperature. After the addition, the mixture was stirred at room temperature for 16 h. After the reaction was completed, 1.0 M dilute hydrochloric acid solution was added to the system to adjust the pH to 6, and then extracted, the organic phase was dried, concentrated, and purified by column chromatography to obtain the intermediate 8g (350 mg, 94.0%).
¹H NMR(600MHz,DMSO-*d*₆ )δ12.90(s,2H),7.52(s,1H),6.99(s,1H),6.57(d,*J*=9.9Hz,1H),5.90(d,*J*= 9.9Hz,1H),2.92-2.86(m,2H),2.83-2.73(m,2H),2.11(m,2H),2.09-1.97(m,2H),1.94-1.76(m,6H)
LC-MS(ESI):[M+H]⁺ =393.08

Step 7: The intermediate 8g (300.0 mg, 0.7 mmol) was dissolved in 100 mL of acetic acid, then 3-aminopiperidine-2,6-dione hydrochloride (170.0 mg, 1.3 mmol) and sodium acetate (360.0 mg, 2.1 mmol) were added at room temperature. After the addition, the mixture was stirred at 110°C for 4 h. After the reaction was completed, the system was decompressed to remove the solvent, and the residue was separated and purified by column chromatography to obtain the intermediate 8h (280 mg, 75.0%).
¹ H NMR(400MHz, DMSO-*d*₆)δ11.12(s,1H),7.72(s,1H),7.30(s,1H),6.70(d,*J*=8.0Hz,1H),6.03(d,*J*= 8.8Hz,1H),5.13-5.07(m,1H),2.94-2.84(m,3H),2.84-2.76(m,2H),2.64-2.55(m,1H),2.18-1.98(m,6H) ,1.95-1.78(m,6H)
LC-MS(ESI):[M+H]⁺ =485.11

Step 8: The intermediate 8h (280.0 mg, 0.5 mmol) was dissolved in 50 mL of acetonitrile, then 50 mL of sodium bicarbonate solution and iodine (1.5 g, 5.0 mmol) were added at room temperature. After the addition, the mixture was stirred at room temperature for 4 h. After the reaction was complete, the solvent was removed under reduced pressure. After extraction, the organic phase was dried, concentrated, and purified by column chromatography to obtain the intermediate 8i (100 mg, 44.0%).
¹H NMR(600MHz,DMSO-*d*₆ )δ11.13(s,1H),7.77(s,1H),7.45(s,1H),6.76(d,*J*=10.0Hz, 1H),6.09(d,*J*= 9.9Hz,1H),5.13-5.09(m,1H),2.92-2.84(m,1H),2.78-2.70(m,2H),2.63-2.57(m,1H),2.26-2.15(m,4H) ,2.12-2.00(m,4H)
LC-MS(ESI):[M+H]⁺ =395.12

Step 9: The intermediate 8i (280.0 mg, 0.7 mmol) was dissolved in 50 mL tetrahydrofuran, then Pd/C (150.0 mg) was added at room temperature. After the addition, the mixture was stirred at 50 °C for 4 h under hydrogen. After the reaction was completed, the reaction solution was filtered through diatomaceous earth, the solvent was removed under reduced pressure, and purified by preparative liquid chromatography to obtain the intermediate 8 (195 mg, 70.0%).
¹H NMR(600MHz,DMSO-*d*₆ )δ11.11(s,1H),7.68(s,1H),7.35(s,1H),5.12-5.06(m,1H),2.99-2.93(m,2H), 2.92-2.84(m, 1H),2.68-2.55(m,3H),2.23-2.15(m,2H),2.13-2.00(m,4H), 1.99-1.91 (m,4H)
LC-MS(ESI):[M+H]⁺ =397.13

### Example 9

### Synthesis of intermediate 9

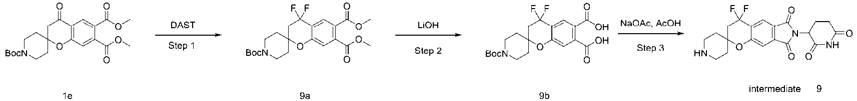

Step 1: Diethylaminosulfur trifluoride (4 mL) was added to the intermediate 1e (2 g, 4.6 mmol), and the reaction solution was stirred at 85°C for 2 hours. The resulting mixture was poured into ice water, extracted three times with ethyl acetate, and the organic phase was concentrated in vacuo. The mixture was purified by reversed-phase column to obtain a yellow oily liquid intermediate 9a (0.5 g, 24%).
¹H NMR(600MHz,DMSO-*d*₆ )δ8.02(s,1H),7.29(s,1H),3.82(s,3H),3.81(s,3H),3.77-3.68(m,2H),3.22-3.02(m,2H),2.72(t,*J*=14.7Hz,2H),1.85(d,*J*=13.9Hz,2H),1.70(td,*J*=14.0,11.6,4.7Hz,2H),1.40(s ,9H).
LC-MS(ESI):[M-tBu+H]⁺ =400.31

Step 2: lithium hydroxide (42 mg, 1.8 mmol) was slowly added to a solution of intermediate 9a (160 mg, 0.4 mmol) in methanol/tetrahydrofuran (2 ml). The reaction solution was stirred at room temperature overnight. The mixture was extracted three times with dichloromethane/methanol (10:1) solvent, and the organic phase was concentrated in vacuo to obtain a white solid intermediate 9b (0.1 g, 67%) .
¹H NMR(600MHz,DMSO-*d*₆ )δ13.27(br s,2H),8.12(s,1H),7.36(s,1H),3.21-3.06(m,2H),2.68(t,*J*= 14.7Hz ,2H),2.05-1.93(m,2H),1.84(d,*J*=13.8Hz,2H),1.68(td,*J*=14.0,12.9,4.7Hz,2H),1.40(s,9H).
LC-MS(ESI):[M-tBu+H]⁺ =372.27

Step 3: 3-aminopiperidine-2,6-dione hydrochloride (77 mg, 0.5 mmol) and sodium acetate (96 mg, 1.2 mmol) were slowly added to a solution of intermediate 9b (100 mg, 0.23 mmol) in acetic acid (1 ml), and the reaction solution was stirred for 2 hours at 110°C. After the reaction was completed, the mixture was purified by reversed-phase column to obtain a brown solid intermediate 9 (50 mg, 51%).
¹H NMR(600MHz,DMSO-*d*₆ )δ11.15(s,1H),8.10(s,1H),7.68(s,1H),5.16(dd,*J*=13.0,5.4Hz,1H),3.27-3.15( m,4H),2.92-2.82(m,3H),2.61(dt,*J*=17.2,3.4Hz,1H),2.56-2.51(m,1H),2.11(d,*J*=14.4Hz,2H),2.05 (ddt,*J*= 12.9,5.6,2.5Hz,1H),1.97-1.90(m,2H).
LC-MS(ESI):[M+H]⁺ =420.36

### Example 10

### Synthesis of intermediate 10

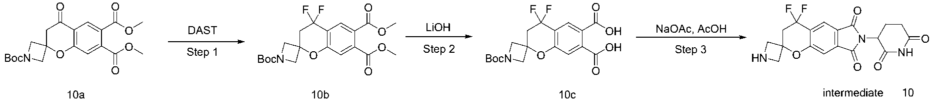

Step 1: Diethylaminosulfur trifluoride (5 mL) was slowly added to an intermediate 10a (1 g, 2.5 mmol). The reaction solution was stirred at 50 °C overnight. The resulting mixture was poured into ice water for quenching, extracted with ethyl acetate, dried with anhydrous sodium sulfate, filtered, and concentrated. The crude product was purified by column chromatography (PE: EA = 0-40%) to obtain a light yellow solid intermediate 10b (500 mg, 47%).
¹ H NMR(400MHz, DMSO-*d*₆)δ8.01(s,1H),7.34(s,1H),4.13(d,*J*=9.7Hz,2H),3.91(d,*J*=9.7Hz,2H),3.83 (s,3H),3.82(s,3H),3.04(t,*J*=13.4Hz,2H),1.39(s,9H).
LC-MS(ESI):[M-Boc+H]⁺=328.30

Step 2: The intermediate 10b (500 mg, 1.2 mmol) was dissolved in methanol (5 mL) and lithium hydroxide (140.1 mg, 5.9 mmol) aqueous solution was slowly added at room temperature. The reaction solution was stirred for 2 h at room temperature. The reaction solution was neutralized with acetic acid to pH = 7, concentrated under vacuum and extracted with ethyl acetate to obtain a white solid crude product, which is used directly in the next step without further purification.
LC-MS (ESI): [M+H]⁺ =399.28

Step 3: Under the protection of nitrogen, the intermediate 10c (100 mg, 0.3 mmol) was dissolved in acetic acid (1 mL), sodium acetate (61.6 mg, 0.8 mmol) and 3-aminopiperidine-2,6-dione hydrochloride (64.5 mg, 0.5 mmol) were added, the temperature was raised to 110°C and stirred for 3 h. After the reaction was completed, the mixture was purified by C18 reversed-phase column to obtain a white solid intermediate 10 (3.6 mg, 4%).
¹H NMR(600MHz,DMSO-*d*₆ )δ11.15(s,1H),8.13(s,1H),7.58(s,1H),5.18(dd,*J*=13.0,5.4Hz,1H),4.33(d,*J*= 11.7Hz,2H),4.22(d,*J*=11.6Hz,2 H),3.15(t,*J*=13.6Hz,2H),2.94-2.87(m,1H),2.65-2.59(m,1H),2.54-2.51(m,1H),2.10-2.05(m,1H).
LC-MS(ESI):[M+H]⁺ =392.30

### Example 11

### Synthesis of intermediate 11

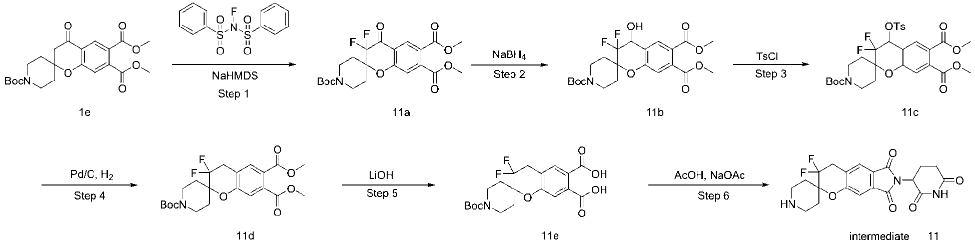

Step 1: The intermediate 1e (5 g, 11.5 mmol) was dissolved in anhydrous tetrahydrofuran (10 mL), cooled to - 78 °C and sodium bis(trimethylsilyl)amide (4.23 g, 23.07 mmol) was added dropwise and reacted for 2 h at this temperature, and N-fluorobisbenzenesulfonamide (7.3 g, 23.1 mmol) was added dropwise at -78 °C. The reaction solution was stirred at -78 °C overnight. The resulting mixture was poured into saturated ammonium chloride solution to quench, extracted with ethyl acetate, dried with anhydrous sodium sulfate, filtered and concentrated. The crude product was purified by column chromatography (PE: EA = 0-40%) to obtain a light yellow solid intermediate 11a (2.5 g, 46%).
¹H NMR(400MHz, DMSO-*d*₆)δ8.29(s,1H),7.62(s,1H),3.94(d,*J*= 11.6Hz,2H),3.85(s,3H),3.86(s,3H) ,3.08(s,2H),2.07(d,*J*=13.4Hz,2H),1.74(td,*J*=13.5,4.7Hz,2H),1.41(s,9H).
LC-MS(ESI):[M+H]⁺ =470.31

Step 2: The intermediate 11a (2.5 g, 5.3 mmol) was dissolved in ethanol (5 mL) and sodium borohydride (403 mg, 10.7 mmol) was slowly added at 0°C. The reaction solution was stirred at room temperature for 1 H. The reaction solution was quenched with acetone. After concentration under vacuum, the crude product was purified by column chromatography (PE:EA=0-40%) to obtain a white solid intermediate 11b (2.4 g, 95%).
¹H NMR(400MHz, DMSO-*d*₆)δ7.90(s,1H),7.23(s,1H),6.68(d,*J*=6.1Hz,1H),5.03(dt,*J*= 16.1,7.0Hz,1H) ,3.97(d,*J*=14.1Hz,1H),3.91-3.75(m,7H),3.04(d,*J*=55.0Hz,2H),1.99(d,*J*=13.3Hz,1H),1.84-1.74( m,2H),1.70-1.56(m,1H),1.41(s,9H).
LC-MS(ESI):[M-Boc+H]⁺ =372.34

Step 3: The intermediate 11b (1.5 g, 3.2 mmol) was dissolved in dichloromethane (30 mL), p-toluenesulfonyl chloride (727 mg, 3.8 mmol) and triethylamine (642 mg, 6.4 mmol) were added under ice bath conditions. The reaction solution was stirred at room temperature for 2 h. After concentration under vacuum, the crude product was purified by column chromatography (PE:EA = 0-40%) to obtain a white solid intermediate 11c (1.0 g, 50%).
¹H NMR(600MHz,DMSO-*d*₆ )δ7.98(d,*J*=8.2Hz,2H),7.56(d,*J*=8.1Hz,2H),7.41(s,1H),7.35(s,1H),6.25 (dd,J =12.9,8.1Hz,1H),3.90(dd,*J*=21.4,8.9Hz,2H),3.81(s,3H),3.79(s,3H),3.06(s,2H),2.48( s,3H),1.99-1.91(m,2H),1.67(dtd,*J*=39.4,13.3,4.7Hz,2H),1.41(s,9H).
LC-MS(ESI):[M-Boc+H]⁺ =526.34

Step 4: The intermediate 11c (1.0 g, 1.6 mmol) was dissolved in methanol (20 mL), palladium on carbon (100 mg) was added, stirred for 12 h under hydrogen atmosphere at room temperature, filtered, washed with methanol, and concentrated under vacuum to obtain a white solid intermediate 11d (700 mg, 96%).
¹H NMR(600MHz,DMSO-*d*₆ )δ7.70(s,1H),7.28(s,1H),3.95-3.88(m,2H),3.86-3.74(m,8H),3.51(t,*J*=15.8 Hz, 2H), 1.81 (d, *J=*13.2Hz, 2H), 1.69 (td, *J*=13.6*,* 4.9Hz, 2H), 1.42 (s, 9H).
LC-MS(ESI):[M-tBu+H]⁺ =400.44

Step 5: The intermediate 11d (700 mg, 1.5 mmol) was dissolved in methanol and water (10 mL), lithium hydroxide (184 mg, 7.7 mmol) was added, and stirred at room temperature for 2 h. Glacial acetic acid was added to neutralize to pH = 7. The reaction solution was concentrated under vacuum and extracted with ethyl acetate to obtain a white solid crude product, which is used directly in the next step without further purification.
LC-MS(ESI):[M-Boc+H]⁺ =32S.32

Step 6: Under the protection of nitrogen, the intermediate 11e (600 mg, 1.4 mmol) was dissolved in acetic acid (10 mL), sodium acetate (346 mg, 4.2 mmol) and 3-aminopiperidine-2,6-dione hydrochloride (360 mg, 2.8 mmol) were added, the temperature was raised to 110 °C and stirred for 3 h. After the reaction was completed, the mixture was purified by C18 reversed-phase column to obtain a white solid intermediate 11 (70 mg, 12%). ¹ H NMR(400MHz, CD3 OD)δ7.78(s,1H),7.55(s,1H),5.13(dd,*J*=12.6,5.4Hz,1H),3.60(t,*J*=15.5Hz,2H),3.50-3.34(m,4H),2.93-2.82(m,¹H),2.80-2.66(m,2H),2.24-2.09(m,5H).

LC-MS(ESI):[M+H]⁺ =420.41.

### Example 12

### Synthesis of intermediate 12

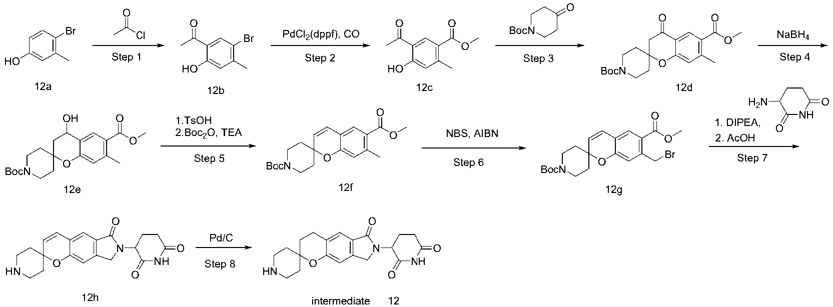

Step 1: A mixture of intermediate 12a (5.0 g, 26.7 mmol) and acetyl chloride (10.5 g, 134 mmol) was stirred at 60 °C for 1 hour, and then aluminum chloride (5.4 g, 40.1 mmol) was added at room temperature. After stirring at 160 °C for 2 hours, the mixture was cooled to room temperature, poured into a saturated ammonium chloride solution (50 mL), and extracted with EA (50 mL × 3). The combined organic layer was dried with anhydrous sodium sulfate, filtered, and concentrated to obtain the crude intermediate 12b as a grayish-white solid (5.5 g, 90%).
¹H NMR (600 MHz, DMSO-*d*₆) δ 11.81 (s, 1H), 8.01 (s, 1H), 7.00 (s, 1H), 2.62 (s, 3H), 2.34 (s, 3H).

Step 2: Triethylamine (9.72 g, 96.04 mmol) and Pd(dppf)Cl₂ (1.76 g, 2.40 mmol) were added to a solution of intermediate 12b (5.5 g, 24.01 mmol) in methanol (50 mL). The mixed system was purged with CO three times and then stirred at 60°C overnight. After the reaction, the system was filtered, and the filtrate was concentrated under reduced pressure. The resulting mixture was purified by column chromatography (PE:EA=6:1) to obtain a white solid intermediate 12c (3.26 g, 65%).
¹H NMR(600MHz,DMSO-*d*₆ )δ12.14(s,1H),8.34(s,1H),6.90(s,1H),3.82(s,3H),2.64(s,3H),2.53(s, 3H).

Step 3: N-tert-butyloxycarbonyl-3-azetidinone (2.8 g, 16.38 mmol) and tetrahydropyrrole (1.59 g, 22.33 mmol) were added to a solution of intermediate 12c (3.1 g, 14.89 mmol) in ethanol (120 mL). The reaction solution was stirred at 80 °C overnight. After the reaction was completed, the resulting mixture was concentrated under vacuum and purified by column chromatography (PE: EA = 0-20%) to obtain a yellow solid intermediate 12d (1.9 g, 35%).

¹H NMR(600MHz,DMSO-*d*₆ )δ8.25(s,1H),7.08(s,1H),3.82(s,3H),3.72(s,2H),3.14(s,2H),2.89(s, 2H),2.55(s,3H),1.91-1.84(m,2H),1.69-1.61(m,2H),1.40(s,9H).

LC-MS(ESI): [M-Boc+H]⁺ =290.27.

Step 4: Sodium borohydride (504 mg, 13.28 mmol) was added to a solution of intermediate 12d (1.6 g, 4.43 mmol) in methanol (40 mL). The reaction solution was stirred at room temperature for 4 hours. After the reaction was completed, the resulting mixture was concentrated under vacuum and purified by column chromatography (PE:EA = 0-40%) to obtain a yellow solid intermediate 12e (1.5 g, 93.23%).
¹H NMR(600MHz,DMSO-*d*₆ )δ8.01(s,1H),6.73(s,1H),5.52(d,*J*=4.7Hz,1H),4.68(s,1H),3.78(s,3H) ,3.68(dd,J =22.7,9.0Hz,2H),3.13(d,*J*=77.0Hz,2H),2.46(s,3H),2.13(dd,*J*=13.5,6.0Hz,1H),1.82-1.62(m,4H),1.59-1.50(m,1H),1.41(s,9H).
LC-MS(ESI):[M-Boc+H]⁺ =292.30

Step 5: p-Toluenesulfonic acid (712 mg, 4.13 mmol) was added to a solution of intermediate 12e (1.5 g, 4.13 mmol) in toluene (40 mL). The reaction solution was stirred overnight at 110 °C. After the reaction was completed, the system was concentrated under vacuum to obtain a mixture, which was dissolved in tetrahydrofuran (40 mL), triethylamine (2.23 g, 22.02 mmol) and di-tert-butyl dicarbonate (2.4 g, 11.01 mmol) were added thereto, and the mixture was stirred at room temperature overnight. After the reaction was completed, the system was concentrated under vacuum, and the resulting mixture was purified by column chromatography (PE: EA = 0-30%) to obtain a yellow solid intermediate 12f (1.1 g, 77%).
¹H NMR(600MHz,DMSO-*d*₆ )δ7.64(s,1H),6.80(s,1H),6.55(d,*J*=9.9Hz,1H),5.80(d,*J*=9.8Hz,1H),3.78 (s,3H),3.70(d,*J*=13.0Hz,2H),3.30-3.11(m,2H),2.47(s,3H),1.84-1.78 (m,2H),1.68-1.60(m,2H ),1.41(s,9H).
LC-MS(ESI):[M-Boc+H]⁺ =274.26

Step 6: N-bromosuccinimide (620 mg, 3.47 mmol) and azobisisobutyronitrile (48 mg, 290 µmol) were added to a solution of intermediate 12f (1 g, 2.90 mmol) in carbon tetrachloride (10 mL), the reaction system was replaced with a nitrogen atmosphere, and stirred overnight at 80 °C. After the reaction was completed, the system was concentrated under vacuum, and the crude product was directly used in the next step.

Step 7: The mixture obtained in the previous step was dissolved with acetonitrile (40 mL), diisopropylethylamine (1.12 g, 8.70 mmol) and 3-amino-2,6-piperidindione (446 mg, 3.48 mmol) were added, and the mixture was stirred at 80 °C overnight. After concentration, acetic acid (10 mL) was added and continued to react for 2 hours. After the reaction was completed, the system was concentrated under vacuum, and the resulting mixture was purified by column chromatography (PE: EA = 0-50%) to obtain 12 g (285 mg, 22%) of a black solid intermediate.

¹H NMR(600MHz, CD3 OD)δ7.52(s,1H),7.13(s,1H),6.69(d,*J*=9.9Hz,1H),5.83(d,*J*=9.9Hz,1H),5.13(dd ,*J*= 13.3,5.1Hz,1H),4.45(dd,*J*=35.2,17.3Hz,2H),3.48-3.40(m,2H),3.38-3.33(m,2H),2.91(ddd,*J*= 18.2,11.8,5.0Hz,2H),2.82-2.77(m,1H),2.49(ddd,*J*=17.9,12.7,3.8Hz,1H) ,2.29-2.22(m,2H),2.17(tdd,J= 13.4,6.7,4.2Hz,1H),2.03-1.95(m,2H).

LC-MS(ESI):[M+H]⁺ =368.38.

Step 8:The intermediate 12g of the product in the previous step was dissolved in methanol (10mL), palladium on carbon (150mg) was added thereto, the reaction system was replaced with hydrogen atmosphere, stirred at room temperature for 2 hours, after the reaction was completed, the mixture was filtered, the filtrate was concentrated under vacuum, and purified by preparative HPLC to obtain a white solid intermediate 12 (120mg, two-step yield 35%).

¹H NMR(600MHz,DMSO-*d*₆ )δ10.97(s,1H),7.51(s,1H),7.05(s,1H),5.07(dd,*J=*13.3,5.1Hz,1H),4.34(d, *J =* 16.9Hz,1H),4.22(d,*J*=16.9Hz,1H),3.26-3.20(m,2H),3.16-3.08(m,2H),2.94-2.86(m,3H),2.63-2.57 (m,1H),2.41-2.33(m,1H),2.00-1.87(m,5H),1.85-1.77(m,2H).

LC-MS(ESI):[M+H]⁺ =370.39.

### Example 13

### Synthesis of intermediate 13

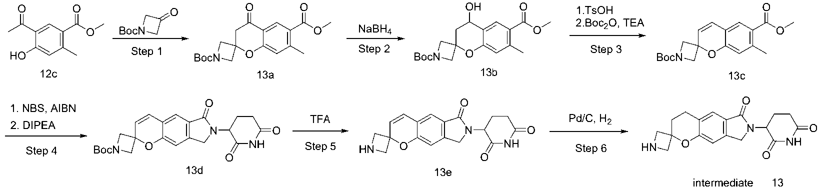

Step 1: N-tert-butyloxycarbonyl-3-azetidinone (2.8 g, 16.38 mmol) and tetrahydropyrrole (1.59 g, 22.33 mmol) were added to a solution of intermediate 12c (3.1 g, 14.89 mmol) in ethanol (120 mL). The reaction solution was stirred at 80 °C overnight. After the reaction was completed, the system was concentrated under vacuum, and the resulting mixture was purified by column chromatography (PE: EA = 0-20%) to obtain a yellow solid intermediate 13a (1.9 g, 35%).

¹H NMR(600MHz, DMSO-*d*₆)δ8.24(s,1H),7.15(s,1H),4.00(d,*J*=8.7Hz,2H),3.89(d,*J*=8.7Hz,2H),3.82 (s,3H),3.20(s,2H),2.56(s,3H),1.38(s,9H).

LC-MS(ESI): [M-Boc+H]⁺ =262.25.

Step 2: Sodium borohydride (504 mg, 13.28 mmol) was added to a solution of intermediate 13a (1.6 g, 4.43 mmol) in methanol (40 mL). The reaction solution was stirred at room temperature for 4 hours. After the reaction was completed, the system was concentrated under vacuum, and the resulting mixture was purified by column chromatography (PE:EA = 0-40%) to obtain a yellow solid intermediate 13b (1.5 g, 93.23%).

¹H NMR(600MHz, CD3 OD)δ8.02(s,1H),6.81(s,1H),4.82(t,*J*=5.2Hz,1H),4.28(d,*J*=9.6Hz,1H),4.04(dd ,*J*= 19.0,9.1Hz,2H),3.95(d,*J*=9.7Hz,1H),3.87-3.84(m,3H),2.54(s,3H),2.32(d,*J*=5.2Hz,2H),1.47(s,9H).

LC-MS(ESI): [M-Boc+H]⁺ =264.27.

Step 3: P-toluenesulfonic acid (712 mg, 4.13 mmol) was added to a solution of intermediate 13b (1.5 g, 4.13 mmol) in toluene (40 mL). The reaction solution was stirred overnight at 110 °C. The resulting mixture was concentrated under vacuum, dissolved in tetrahydrofuran (40 mL), triethylamine (2.23 g, 22.02 mmol) and di-tert-butyl dicarbonate (2.4 g, 11.01 mmol) were added thereto, and the mixture was stirred at room temperature overnight. The resulting mixture was concentrated under vacuum and purified by column chromatography (PE: EA = 0-30%) to obtain a yellow solid intermediate 13c (1.1 g, 77%).

¹H NMR(600MHz,DMSO-*d*₆ )δ7.67(s,1H),6.84(s,1H),6.65(d,*J*=9.9Hz,1H),6.16(d,*J*=9.9Hz,1H),4.04 (s,4H),3.78(s,3H),2.47(s,3H),1.40(s,9H).

LC-MS(ESI): [M-Boc+H]⁺ =246.23.

Step 4: N-bromosuccinimide (620 mg, 3.47 mmol) and azobisisobutyronitrile (48 mg, 290 µmol) were added to a solution of intermediate 13c (1 g, 2.90 mmol) in carbon tetrachloride (10 mL), the reaction system was replaced with a nitrogen atmosphere, and stirred at 80°C overnight. The resulting mixture was concentrated under vacuum, dissolved in acetonitrile (40 mL), and diisopropylethylamine (1.12 g, 8.70 mmol) and 3-amino-2,6-piperidindione (446 mg, 3.48 mmol) were added thereto, and the mixture was stirred at 80°C overnight. The resulting mixture was concentrated under vacuum and purified by column chromatography (PE:EA = 0-50%) to obtain a black solid intermediate 13d (285 mg, 22%).

¹H NMR(600MHz, CD3 OD)δ7.50(s,1H),7.09(s,1H),6.69(d,*J*=9.9Hz,1H),6.17(d,*J*=9.8Hz,1H),5.12(dd ,*J*= 13.2,5.2Hz,1H),4.50-4.40(m,2H),4.24-4.19(m,2H),4.10(d,*J*=8.9Hz,2H),2.94-2.86(m,1H), 2.82-2.77(m,1H),2.49(ddd,*J*=26.5,13.2,4.5Hz,1H),2.19-2.15(m,1H),1.48(s,9H).

LC-MS(ESI):[M+H]⁺ =440.38.

Step 5: The intermediate 13d (285 mg, 648.5 µmol) was dissolved in dichloromethane (5 mL), trifluoroacetic acid (636 mg, 6.49 mmol) was added, and the reaction solution was stirred at room temperature for 2 hours. After the reaction was completed, the reaction solution was concentrated and used directly in the next step.

LC-MS(ESI):[M+H]⁺ =340.32.

Step 6: The mixture from the previous step was dissolved in methanol (10 mL), palladium on carbon (10%, 150 mg) was added thereto, the reaction system was replaced with a hydrogen atmosphere, and stirred at room temperature for 2 hours. After the reaction was completed, the mixture was filtered, and the filtrate was concentrated under vacuum. The white solid intermediate 13 (120 mg, two-step yield 35%) was obtained through purification by preparative HPLC.

¹H NMR(400MHz, CD3 OD)δ7.57(s,1H),7.10(s,1H),5.11(dd,*J*=13.3,5.1Hz,1H),4.50-4.35(m,2H),4.24(s, 4H),2.97(t,*J*=5.8Hz,2H),2.91-2.84(m,1H),2.77(ddd,*J*=17.6,4.5,2.3Hz,1H),2.46(ddd,*J*=26.4,13.2, 4.7Hz,1H),2.28(t,*J*=6.1Hz,2H),2.14(dtd,*J*=12.7,5.2,2.3Hz,1H).

LC-MS(ESI):[M+H]⁺ =342.32.

### Example 14

### Synthesis of intermediate 14

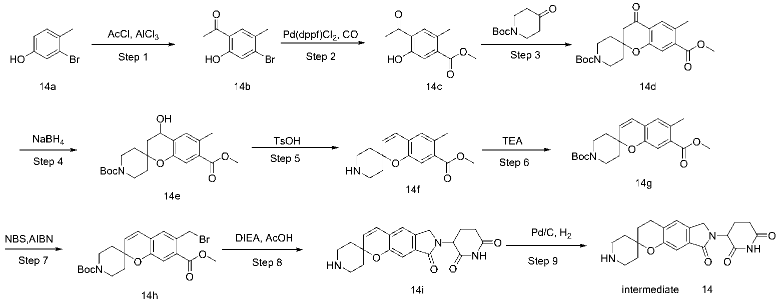

Step 1: The intermediate 14a (15.2 g, 81.27 mmol) was added to acetyl chloride (30 mL) and stirred at 60°C for 1 Hour. After the reaction solution was cooled to room temperature, aluminum chloride (16.25 g, 121.90 mmol) was slowly added, and after that, the reaction solution was stirred at 160°C for 3 hours. The resulting mixture was poured into ice water, filtered and washed with saturated ammonium chloride aqueous solution, concentrated under vacuum to obtain a mixture, and then purified by column chromatography (PE:EA=0-50%) to obtain a yellow-brown solid intermediate 14b (18.0 g, 96%).

¹H NMR (600MHz,CDCl3 )δ12.10(s,1H),7.57(s,1H),7.25(s,1H),2.63(s,3H),2.39(s,3H).

Step 2: The triethylamine (1.99g, 19.6mmol) and Pd(dppf)Cl₂ (1.42g, 1.96mmol) were added to a solution of the intermediate 14b (3.0g, 13.1mmol) in methanol (60mL). Under the protection of carbon monoxide gas, the reaction solution was stirred at 60°C overnight. After the reaction was completed, the reaction solution was filtered and the filtrate was concentrated under vacuum. The resulting mixture was purified by column chromatography (PE:EA=0-50%) to obtain a yellow-brown solid intermediate 14c (16.3g, 51%).

¹H NMR(600MHz, CDCl₃ )δ11.88(s,1H), 7.60(s,1H), 7.49(s,1H), 3.93(s,3H), 2.68(s,3H), 2.53(s,3H) .

Step 3: The intermediate 14c (10.11 g, 48.56 mmol), N-tert-butyloxycarbonyl-4-piperidone (9.67 g, 48.56 mmol) and tetrahydropyrrole (3.45 g, 48.56 mmol) were dissolved in methanol (50 mL) and refluxed at 80°C for 6 h. After the reaction was completed, the resulting mixture was extracted, filtered and concentrated to obtain a crude product, which was then purified by column chromatography (PE:EA=0-50%) to obtain a light yellow solid intermediate 14d (15.53 g, 82%).

¹H NMR(600MHz,DMSO-*d*₆ )δ7.64(s,1H),7.45(s,1H),3.85(s,3H),3.71(s,2H),3.12(d,*J*= 50.1Hz,2H) ,2.88(s,2H),2.43(s,3H),1.90-1.84(m,2H),1.62(td,*J*=12.7,4.6Hz,2H),1.40(s,9H).

LC-MS(ESI): [M-Boc+H]⁺ =290.26.

Step 4: Sodium borohydride (6.03 g, 159.51 mmol) was added to a solution of intermediate 14d (15.53 g, 39.88 mmol) in methanol (70 mL) under ice bath conditions. The reaction solution was stirred at room temperature for 2 hours. After the reaction was completed, the resulting mixture was concentrated under vacuum and purified by column chromatography (PE:EA = 0-50%) to obtain a yellow foamy intermediate 14e (15.39 g, 98%).

¹H NMR(600MHz, CDCl3 )δ7.40(s,1H),7.32(s,1H),4.83(t,*J*=7.0Hz,1H),3.90-3.84(m,5H),3.31-3.05(m, 2H),2.93(s,1H),2.49(s,3H),2.11(dd,*J*= 13.6,6.1Hz,1H),1.92-1.85(m,2H),1.78-1.72(m,1H),1.63 (td,*J=* 13.3,4.6Hz,1H),1.52(ddd,*J*=24.7,12.6,7.9Hz,1H),1.46(s,9H).

LC-MS(ESI):[M-Boc+H]- =292.27.

Step 5: The intermediate 14e (15.23 g, 38.91 mmol) and p-toluenesulfonic acid (6.7 g, 38.91 mmol) were dissolved in toluene and refluxed at 110°C overnight. The resulting light yellow oily mixture was concentrated under vacuum to obtain intermediate 14f (8.6 g, 80%). The crude product was used directly in the next step.

LC-MS(ESI):[M-Boc+H]⁺ =274.29.

Step 6: The intermediate 14f (8.6 g, 31.46 mmol), di-tert-butyl dicarbonate (13.73 g, 62.93 mmol) and triethylamine (9.55 g, 94.39 mmol) were dissolved in dichloromethane (30 mL), and the mixture was stirred at room temperature for 4 hours. The resulting mixture was concentrated under vacuum and purified by column chromatography (PE:EA=0-30%) to obtain a colorless solid intermediate 14g (1.8 g, 83%). ¹ H NMR(600MHz, CDCl₃ )δ7.41(s,1H),6.85(s,1H),6.37(d,*J*=9.8Hz,1H),5.65(d,*J*=9.6Hz,1H),3.98-3.77(m,5H),3.28(s,2H),2.50(s,3H ),1.97(d,*J*=13.4Hz,2H),1.59(td,*J*=13.4,4.7Hz,2H),1.47(s,9H).

LC-MS(ESI):[M-Boc+H]⁺ =274.24.

Step 7: The intermediate 14g (5.11g, 13.68mmol) and N-bromosuccinimide (2.73g, 15.33mmol) and azobisisobutyronitrile (0.112g, 6.84mmol) were dissolved in carbon tetrachloride (30mL), and the reaction soluiton was refluxed at 80°C overnight under nitrogen. The mixture was extracted and spun dry to obtain a colorless oily intermediate 14h (1.32g, 21%) . The crude product was used directly in the next step.

Step 8: The intermediate 14g (1.56g, 3.45mmol) was dissolved in acetonitrile (20mL) solution, 3-aminopiperidine-2,6-dione hydrochloride (0.53g, 4.14mmol) and N,N-diisopropylethylamine (1.33g, 10.35mmol) were added, and the reaction solution was stirred at 80°C overnight. Then, the mixture was spun dry and acetic acid (1 mL) was added and refluxed at 110°C for 2 hours. After the reaction was completed, the mixture was purified by reversed-phase column to obtain a brown solid intermediate 14i (0.96 g, 75%).

¹H NMR(600MHz,DMSO-*d*₆ )δ11.00(s,1H),7.36(s,1H),7.25(s,1H),6.68(d,*J*=9.8Hz,1H),5.99(d,*J*= 9.8Hz,1H),5.08(dd,*J*=13.3,5.2Hz,1H),4.37(d,*J*=16.9Hz,1H),4.25(d,*J*=16. 9Hz,1H),3.26-3.19(m,4H),2.90(ddd,*J* =17.3,13.7,5.5Hz,1H),2.64-2.57(m,1H),2.39(qd,*J*=13.2,4.5Hz, 1H),2.10-2.03(m,2H),2.00(dtd,*J*= 12.8,5.4,2.4Hz,1H),1.94-1.86(m,2H).

LC-MS(ESI):[M+H]⁺ =368.37.

Step 9: The intermediate 14i (0.668 g, 1.82 mmol) was dissolved in methanol (15 mL) and palladium on carbon (0.2 g) was added. The reaction solution was stirred at room temperature overnight under hydrogen. The mixture was filtered and spun dry. The mixture was purified by reversed-phase column to obtain a white solid intermediate 14 (0.536 g, 79%) .

¹H NMR(600MHz,DMSO-*d*₆ )δ10.99(s,1H),7.36(s,1H),7.18(s,1H),5.07(dd,*J*=13.3,5.1Hz,1H),4.34(d, *J=* 16.6Hz,1H),4.21(d,*J*=16.5Hz,1H),3.25-3.11(m,4H),2.94-2.87(m,3H),2.63-2.57(m,1H),2.39(qd ,*J=* 13.2,4.5Hz,1H),2.01-1.95(m,1H),1.93-1.87(m,4H),1.82-1.74(m,2H).

LC-MS(ESI):[M+H]⁺ =370.36.

### Example 15

### Synthesis of intermediate 15

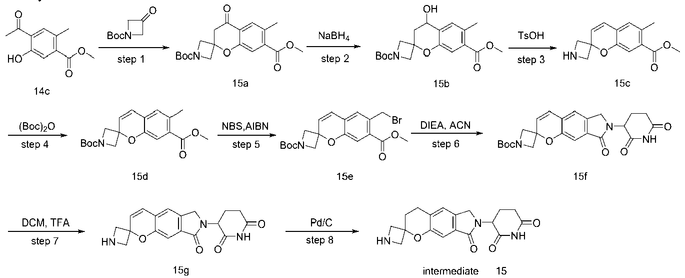

Step 1: The 1-Boc-3-azetidinone (9.9 g, 57.6 mmol) and tetrahydropyrrole (4.1 g, 57.6 mmol) were added to a solution of intermediate 14c (12 g, 57.6 mmol) in ethanol (200 mL). The reaction solution was stirred at 80 °C for 4 hours. 1-Boc-3-azetidinone (9.9 g, 57.6 mmol), tetrandrine (4.1 g, 57.6 mmol) were added to the reaction solution again and the reaction solution was stirred at 80 °C for 16 hours. The resulting mixture was concentrated under vacuum and purified by column chromatography (PE:EA=0-40%) to obtain a yellow solid intermediate 15a (8 g, 38%).

¹H NMR (600 MHz, CDCl₃) δ 7.74 (s, 1H), 7.59 (s, 1H), 4.09 (d, *J =* 9.6 Hz, 2H), 3.97 (d, *J =* 9.5 Hz, 2H), 3.94 (s, 3H), 3.07 (s, 2H), 2.53 (s, 3H), 1.46 (s, 9H).

LC-MS(ESI): [M-Boc+H]⁺=262.28.

Step 2: The sodium borohydride (786 mg, 20.8 mmol) was slowly added to a solution of intermediate 15a (5 g, 13.8 mmol) in methanol (100 mL) , the reaction solution was stirred at room temperature for 4 h. The reaction solution was extracted with ethyl acetate for three times, and the organic phase was collected and dried with anhydrous sodium sulfate, and the organic phase was concentrated under vacuum to obtain a yellow oily liquid intermediate 15b (4.2 g, 83%).

¹H NMR (600 MHz, CDCl₃) δ 7.50 (s, 1H), 7.24 (s, 1H), 4.89 (q, *J =* 5.2 Hz, 1H), 4.36 (q, *J =* 7.2 Hz, 1H), 4.27 - 4.23 (m, 1H), 4.10 (d, *J* = 9.3 Hz, 1H) , 4.00 (dd, *J =* 17.3, 9.6 Hz, 2H), 3.90 (s, 3H), 2.54 (s, 3H), 2.35 (d, *J=* 5.3 Hz, 2H), 1.47 (s, 9H).

LC-MS(ESI): [M-Boc+H]⁺=264.27.

Step 3: The hydrated p-toluenesulfonic acid (2.3 g, 12.1 mmol) was added to a solution of intermediate 15b (4 g, 13.8 mmol) in toluene (100 mL) , and the reaction solution was stirred at 110 °C for 18 h. The reaction solution was concentrated under vacuum to obtain a yellow solid crude product that was used directly in the next step without further purification.

LC-MS(ESI): [M+H]⁺ = 246.22.

Step 4: The di-tert-butyl dicarbonate (2.1 g, 9.8 mmol) and triethylamine (3.2 mL) were added to a solution of intermediate 15c (2 g, 8.2 mmol) in dichloromethane (30 mL). The reaction solution was stirred at room temperature for 4 h. The resulting mixture was concentrated under vacuum and purified by column chromatography (PE:EA=0-40%) to obtain a colorless liquid intermediate 15d (2.5 g, 88%).
¹H NMR (600 MHz, CDCl₃) δ 7.45 (s, 1H), 6.88 (s, 1H), 6.47 (d, *J =* 9.8 Hz, 1H), 6.06 (d, *J* = 9.7 Hz, 1H), 4.24 (d, *J* = 9.5, 2H), 4.01 (d, *J* = 9.5, 2H), 3.89 (s, 3H), 2.51 (s, 3H), 1.48 (s, 9H). 2.51 (s, 3H), 1.48 (s, 9H).
LC-MS(ESI): [M-tBu+H]⁺=290.20

Step 5: NBS (1.2 g, 6.4 mmol) and AIBN (99 mg, 0.6 mmol) were added to a solution of intermediate 15e (2 g, 5.8 mmol) in carbon tetrachloride (30 mL) . The reaction solution was stirred at 80 °C for 12 h. The resulting mixture was concentrated under vacuum and purified by column chromatography (PE:EA=0-40%) to obain a colorless liquid crude product which was used directly in the next step without further purification.

Step 6: 3-amino-2,6-piperidinedione hydrochloride (173 mg, 1.05 mmol) and N,N-diisopropylethylamine (0.4 mL) were added to a solution of intermediate 15e (2 g, 0.7 mmol) in acetonitrile (15 mL) . The reaction solution was stirred at 80 °C for 12 h. The resulting mixture was concentrated under vacuum and purified by a C18 reversed-phase column to obtain a gray solid intermediate 15f (120 mg, 38%).
¹H NMR (400 MHz, *DMSO-d*₆) *δ* 10.97 (s, 1H), 7.35 (s, 1H), 7.13 (s, 1H), 6.71 (d, *J* = 9.9 Hz, 1H), 6.33 (d, *J=* 9.8 Hz, 1H), 5.08 (dd, *J =* 13.3, 5.1 Hz, 1H), 4.41 - 4.19 (m, 2H), 4.15 - 3.96 (m, 4H), 2.97 - 2.85 (m, 1H), 2.66 - 2.55 (m, 1H), 2.41 - 2.30 (m, 1H), 2.03 - 1.95 (m, 1H), 1.40 (s, 9H).
LC-MS(ESI): [M-tBu+H]⁺=384.35

Step 7: Trifluoroacetic acid (1 mL) was added to a solution of intermediate 15f (120 mg, 0.7 mmol) in dichloromethane (3 mL). The reaction solution was stirred at room temperature for 2 h. The reaction mixture was concentrated under vacuum. The crude product was used directly in the next step without further purification.
LC-MS(ESI): [M+H]⁺=340.34

Step 8: Palladium carbon (5 mg) was added to a solution of intermediate 15g (50 mg, mmol) in methanol (3 mL) . The reaction solution was stirred at room temperature for 12 h. The reaction solution was filtered through diatomaceous earth, the filtrate was concentrated and a white solid intermediate 15 (30 mg, 59%) was obtained by preparative HPLC.
¹H NMR (400 MHz, *DMSO-d*₆) δ 10.97 (s, 1H), 7.36 (s, 1H), 7.11 (s, 1H), 5.07 (dd, *J =* 13.3, 5.1 Hz, 1H), 4.34 (d, *J* = 16.8 Hz, 1H), 4.21 (d, *J =* 16.8 Hz, 1H), 4.15 - 4.04 (m, 4H), 2.91 (q, *J =* 5.6, 4.4 Hz, 2H), 2.67 - 2.56 (m, 2H), 2.40 - 2.31 (m, 1H), 2.20 (t, *J=* 6.9 Hz, 2H), 1.98 (m, 1H). 1H).
LC-MS(ESI): [M+H]⁺=342.43

### Example 16

### Synthesis of intermediate 16

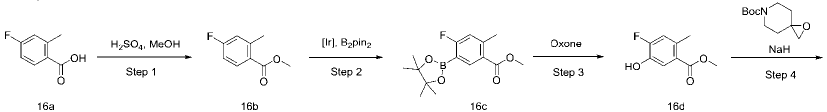

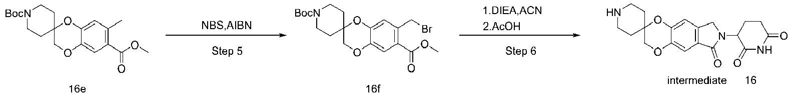

Step 1: Concentrated sulfuric acid (15 mL) was added slowly and dropwise to a solution of intermediate 16a (5.5 g, 35.7 mmol) in methanol (100 mL). The reaction solution was stirred at room temperature overnight. The reaction solution was poured into ice water, extracted with ethyl acetate, the organic phases were combined and dried with anhydrous sodium sulfate. The organic phase was filtered and concentrated under reduced pressure to obtain a colorless oily liquid intermediate 16b (5.0 g, 83%), the crude product can be used in the next reaction without purification.

¹H NMR (600 MHz, *DMSO-d*₆) δ 7.90 (dd, *J =* 8.7, 6.2 Hz, 1H), 7.22 (dd, *J =* 10.1, 2.8 Hz, 1H), 7.15 (td, *J =* 8.5, 2.7 Hz, 1H), 3.82 (s, 3H), 2.53 (s, 3H).

LC-MS(ESI): [M+H]⁺ = 169.19.

Step 2: Boronic acid pinacol ester (5.7 g, 22.3 mmol), 4,4'-di-tert-butyl-2,2'-dipyridine (0.16 g, 0.59 mmol) and methoxy(cyclooctadiene)iridium dimer (0.20 g, 0.30 mmol) were added to 10 ml of methyl tertiary-butyl ether solution, and intermediate 16b (2.5 g, 14.9 mmol) was added to the methyl tertiary-butyl ether (10 mL) solution, and the air in the system was replaced with nitrogen three times, and the reaction solution was stirred at 85 °C for 4 h. After the reaction was completed, the reaction solution was filtered through diatomaceous earth and the filtrate was concentrated to obtain the crude product (4.0 g), which was ready for use in the next step of the reaction without purification.

¹H NMR (600 MHz, *DMSO-d*₆) δ 8.16 (d, *J* = 6.3 Hz, 1H), 7.19 (dd, *J =* 10.4, 3.2 Hz, 1H), 3.83 (s, 3H), 2.55 (s, 3H), 1.30 (s, 12H).

Step 3: The potassium peroxomonosulfate (10.9 g) was added to a solution of intermediate 16c (4.0 g, 13.6 mmol) in acetonitrile (50 mL) and the reaction solution was stirred at room temperature overnight. The reaction solution was filtered and the filtrate was concentrated, and the crude product was chromatographed by silica gel column chromatography to obtain a white solid intermediate 16d (2.0 g, 80%).

¹H NMR (600 MHz, *DMSO-d*₆) δ 10.04 (s, 1H), 7.47 (d, *J =* 9.3 Hz, 1H), 7.13 (d, *J =* 12.2 Hz, 1H), 3.80 (s, 3H), 2.41 (s, 3H).

LC-MS(ESI): [M+H]⁻= 183.21.

Step 4: The intermediate 16d (1.0 g, 5.4 mmol) was dissolved in 10 ml of ultra-dry N,N-dimethylformamide, followed by the addition of tert-butyl 1-oxa-6-azaspiro[2.5]octane-6-carboxylate (1.2 g, 5.3 mmol) and sodium hydride (196 mg, 4.9 mmol), and the reaction solution was heated to 110 °C and stirred overnight. The reaction solution was chromatographed by silica gel column chromatography to obtain a white solid intermediate 16e (1.4 g, 68%).

¹H NMR (600 MHz, *DMSO-d*₆) δ 7.37 (s, 1H), 6.85 (s, 1H), 4.04 (s, 2H), 3.77 (s, 3H), 3.73 (d, *J =* 13.4 Hz, 2H), 3.24 - 3.04 (m, 2H), 2.42 (s, 3H), 1.66 - 1.61 (m, 4H), 1.41 (s, 9H).

LC-MS(ESI): [M+H]⁺ = 278.29.

Step 5: N-bromosuccinimide (613 mg, 3.4 mmol) and azobisisobutyronitrile (44 mg, 0.3 mmol) were added to a solution of intermediate 16e (1.0 g, 2.7 mmol) in carbon tetrachloride (10 mL), and the air in the system was replaced with nitrogen three times, and the reaction solution was stirred at 85 °C overnight under the protection of nitrogen. After completion of the reaction, the reaction solution was filtered and the filtrate was concentrated to obtain the crude product (1.2 g) which could be used for the next step without purification.

### Step 6: 3-(6'-oxa-6',8'-dihydro-3'H,7'H-spiro[piperidin-4,2'-[1,4]dioxino[2,3-f]isoindol]-7'-yl)piperidine-2,6-dione (intermediate 16)

3-aminopiperidine-2,6-dione hydrochloride (655 mg, 4.0 mmol) and N,N-diisopropylethylamine (1.0 g, 8.0 mmol) were added to a solution of intermediate 16f (1.2 g, 2.7 mmol) in acetonitrile (10 mL), the reaction solution was stirred at 80 °C overnight, and then the reaction solution was concentrated and dissolved in acetic acid (10 mL), and the reaction solution was stirred at 110 °C for 2 h. The reaction solution was purified by reversed-phase purification to obtain a white solid intermediate 16 (235 mg, 24%).

¹H NMR (400 MHz, *DMSO-d*₆) δ 10.96 (s, 1H), 7.22 (s, 1H), 7.17 (s, 1H), 5.06 (dd, *J =* 13.3, 5.1 Hz, 1H), 4.32 (d, *J= 16.8* Hz, 1H), 4.20 (d, *J* = 16.8 Hz, 1H), 4.19 - 4.11 (m, 2H), 3.30 - 3.25 (m, 2H), 3.18 - 3.08 (m, 2H), 2.90 (ddd, *J=* 17.3, 13.6, 5.4 Hz, 1H), 2.59 (dt, *J=* 16.6, 3.4 Hz, 1H), 2.42 - 2.30 (m, 1H), 2.00 - 1.93 (m, 1H), 1.92 - 1.85 (m, 4H).

LC-MS(ESI): [M+H]⁺= 373.35.

LC-MS(ESI): [M+H]⁺= 373.35.

### Example 17

### Synthesis of intermediate 17

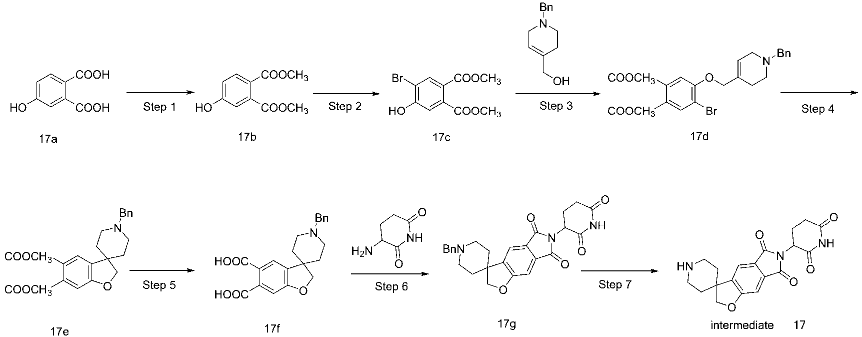

Step 1: The intermediate 17a (30 g, 165 mmol) was dissolved in methanol (300 mL) under the protection of nitrogen, concentrated sulfuric acid (36 mL) was slowly added to the system, and the system was heated up to 66°C for reaction for 8 hours. The reaction system was cooled to room temperature and then concentrated under reduced pressure, water (200 mL) was added and extracted with ethyl acetate (300 ml*3 times). The extracted solution was washed with saturated saline (300 ml), the organic phases were combined and dried with anhydrous sodium sulfate, and concentrated to obtain a crude light yellow solid intermediate 17b (31 g, 89.5%).

LC-MS(ESI): [M-OMe+H]⁺ = 179.12.

Step 2: The intermediate 17b (10 g, 47 mmol) was dissolved in trifluoroacetic acid (100 mL) under the protection of nitrogen, N-bromosuccinimide (4.24 g, 47 mmol) was added to the reaction system, and the reaction was carried out overnight at room temperature. The resulting reaction system was concentrated under reduced pressure and extracted with ethyl acetate (100 ml*3 times) after adding water (100 mL). Then it was washed by saturated saline (100 ml), and the organic phases were combined and dried with anhydrous sodium sulfate. The concentrated crude product was purified by reversed-phase column chromatography to obtain a light yellow solid intermediate 17c (4.6 g, 33%).

¹H NMR (600 MHz, CD₃OD) δ 7.98 (s, 1H), 7.07 (s, 1H), 3.88 (s, 3H), 3.86 (s, 3H).

LC-MS(ESI): [M+H]⁺ = 289.04.

Step 3: The intermediate 17c (8 g, 27 mmol, 1.0eq), (1-benzyl-1,2,3,6-tetrahydropyridin-4-yl) methanol (5.64 g, 27 mmol) and triphenylphosphine (7.2 g, 27 mmol) were dissolved in tetrahydrofuran (80 mL) under the protection of nitrogen, and the diethyl azodicarboxylate (4.08 mL, 27 mmol, 1.0eq) was added and the reaction was carried out for 4 h at room temperature. The resulting reaction system was concentrated under reduced pressure, water (50 mL) was added to the system and the system was extracted with ethyl acetate (100 ml*3 times). Then it was washed with saturated saline (100 ml) and the organic phases were combined and dried with anhydrous sodium sulfate. The combined organic phases were purified by column chromatography to obtain a reddish-brown solid intermediate 17d (6 g, 46%).

¹H NMR (600 MHz, CDCl₃) δ 8.04 (s, 1H), 7.47 (hd, *J* = 5.4, 1.9 Hz, 5H), 7.09 (s, 1H), 5.90 (tt, *J* = 3.2, 1.5 Hz, 1H), 4.67 - 4.55 (m, 2H), 4.38 (d, *J* = 12.9 Hz, 1H), 4.28 (d, *J* = 12.8 Hz, 1H), 3.98 (d, *J* = 16.7 Hz, 1H), 3.93 (s, 3H), 3.91 (s, 3H), 3.78 (dd, *J* = 19.0, 12.3 Hz, 1H), 3.48 (d, *J =* 16.5 Hz, 1H), 3.06 (s, 1H) , 2.75 (s, 1H), 2.54 (d, *J =* 18.6 Hz, 1H).

LC-MS(ESI): [M+H]⁺ = 474.26/476.27.

Step 4: Under the protection of nitrogen, the intermediate 17d (8.4 g, 17 mmol), tri-n-butyltin hydroxide (10.2 g, 34 mmol) and azobisisobutyronitrile (574 mg, 3.4 mmol) were dissolved in toluene (84 mL) and the reaction system was heated up to 110 °C for reaction for 4 hours. The reaction system was cooled to room temperature and concentrated under reduced pressure, water (100 mL) was added and extracted with ethyl acetate (100 ml*3 times). Then it was washed with saturated saline (100 ml), the organic phases were combined and dried with anhydrous sodium sulfate. The combined organic phases were purified by column chromatography to obtain a reddish-brown oily intermediate 17e (5 g, 71%).

¹H NMR (400 MHz, CDCl₃) δ 7.67 (s, 1H), 7.55 - 7.41 (m, 5H), 6.97 (s, 1H), 4.47 (s, 2H), 4.27 (s, 2H), 3.91 (s, 3H), 3.89 (s, 3H), 3.70 (d, *J =* 12.3 Hz, 2H), 2.78 - 2.63 (m, 2H), 2.52 (td, *J* = 14.3, 4.0 Hz, 2H), 1.94 (d, *J =* 14.5 Hz, 2H). LC-MS(ESI): [M+H]⁺= 396.40.

Step 5: The intermediate 17e (2.3 g, 5.8 mmol) was dissolved in methanol (20 mL) and water (2 mL) and lithium hydroxide (1.39 g, 58 mmol) was added to the system. The reaction was carried out at room temperature for 4 hours. The reaction system was concentrated under reduced pressure and purified by reversed-phase column to obtain a light reddish brown solid intermediate 17f (1.28 g, 60%).

¹H NMR (600 MHz, *DMSO-d*₆) δ 7.51 (ddd, *J* = 12.6, 6.6, 3.4 Hz, 6H), 6.98 (s, 1H), 4.64 (s, 2H), 4.36 (s, 2H), 3.39 (s, 2H), 3.13 (t, *J* = 13.4 Hz, 2H), 2.15 (dd, *J =* 20.6, 8.4 Hz, 2H), 1.96 (d, *J =* 14.1 Hz, 2H).

LC-MS(ESI): [M+H]⁺= 368.34.

Step 6: The intermediate 17f (3.0 g, 8 mmol), sodium acetate (3.3 g, 24 mmol) and 3-amino-2,6-piperidinedione hydrochloride (1.3 g, 10 mmol, 1.25 eq) were dissolved in acetic acid (30 mL) under the protection of nitrogen, and the system was reacted for 4 h at 110 °C. The reaction system was cooled to room temperature and concentrated under reduced pressure, water (50 mL) was added and extracted with ethyl acetate (100 ml*3 times). Then it was washed with saturated saline (100 ml), the organic phases were combined and dried with anhydrous sodium sulfate. The combined organic phases were purified by column chromatography to obtain a light yellow solid intermediate17g (1.8 g, 48%).

¹H NMR (600 MHz, CDCl₃) δ 7.69 (s, 1H), 7.54 - 7.43 (m, 5H), 7.25 (s, 1H), 4.96 (dd, *J =* 12.7, 5.4 Hz, 1H), 4.56 (s, 2H), 4.28 (s, 2H), 2.97 - 2.75 (m, 4H), 2.70 (t, *J =* 13.6 Hz, 2H), 2.63 - 2.53 (m, 2H), 1.99 (d, *J =* 14.6 Hz, 2H), 1.63 - 1.57 (m, 2H).

LC-MS(ESI): [M+H]⁺ = 460.34.

Step 7: Under the protection of nitrogen, the intermediate 17g (3.0 g, 6.5 mmol, 1.0 eq) was dissolved in methanol (30 mL), and 10% wet palladium/carbon (600 mg) was added to the system. After replaced with hydrogen for 3 times, the reaction was carried out overnight at room temperature. The reaction system was filtered and the filter cake was washed with methanol (15 mL) three times. The resulting filtrate was spun dried under reduced pressure, and the concentrated crude product was purified by reversed-phase column chromatography to obtain a white solid intermediate 17 (630 mg, 26%).

¹H NMR (600 MHz, CD₃OD) δ 7.78 (s, 1H), 7.26 (d, *J* = 0.6 Hz, 1H), 5.14 - 5.10 (m, 1H), 4.74 (s, 2H), 3.38 (dt, *J =* 12.7, 3.2 Hz, 2H), 3.05 (td, *J =* 13.1, 3.0 Hz, 2H), 2.90 - 2.85 (m, 1H), 2.78 (dd, *J* = 4.4, 2.6 Hz, 1H), 2.77 - 2.69 (m, 2H), 2.14 (ddt, *J* = 13.2, 11.0, 4.1 Hz, 4H).

LC-MS(ESI): [M+H]⁺ = 370.33.

### Example 18

### Synthesis of intermediate 18

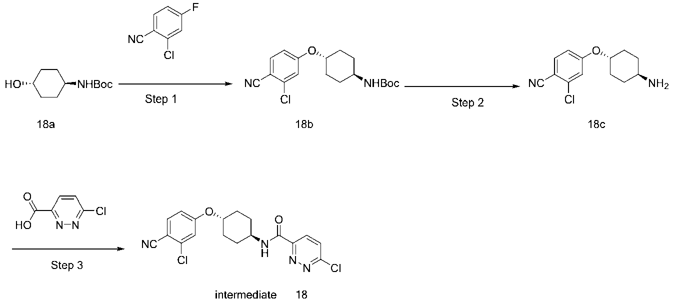

### Step 1:

The intermediate 18a (20 g, 92.9 mmol) was dissolved in a single-necked flask containing tetrahydrofuran (100 mL), cooled to 0 °C, and sodium hydrogen (8.36 g, 209.02 mmol) was slowly added, and stirred at 0 °C for 1 h. A solution of 2-chloro-4-fluorobenzonitrile (20 g, 92.9 mmol) in tetrahydrofuran (100 mL) was added to the single-necked flask. The reaction solution was stirred at 0 °C for 0.5 h. The reaction solution was transferred to room temperature and continued to stir for 3 h. After completion of the reaction, the reaction solution was cooled to 0 °C, quenched with ice water and extracted with ethyl acetate three times (100 mL), and the organic phase was washed with saturated brine and dried with anhydrous sodium sulfate, and filtered and concentratedto obtain a crude product. The crude product was purified by column purification (0-25% ethyl acetate) to obtain a white solid intermediate 18b (21.92 g).

¹H NMR (600 MHz, MeOD) δ 7.69 (d, *J =* 8.8 Hz, 1H), 7.18 (d, *J* = 2.4 Hz, 1H), 7.03 (dd, *J* = 8.8, 2.4 Hz, 1H), 4.43 (tt, *J* = 10.2, 4.1 Hz, 1H), 3.42 (dq, *J =* 10.9, 5.8 Hz, 1H), 2.18 - 2.10 (m, 2H), 2.07 - 1.97 (m, 2H), 1.61 - 1.51 (m, 2H), 1.46 (s, 9H).

Step 2: The intermediate 18b (21.92 g, 62.48 mmol) was added to a single-necked flask containing dioxane (25 mL). Solution of hydrochloric acid (25 mL) in dioxane was added to the reaction flask. After addition, the reaction solution was stirred at room temperature for 2 h. After completion of the reaction, the reaction solution was concentrated directly to obtain a crude white solid intermediate 18c (20.05 g).

¹H NMR (600 MHz, DMSO) δ 8.18 (s, 3H), 7.86 (d, *J* = 8.7 Hz, 1H), 7.42 (dd, *J* = 2.5, 0.9 Hz, 1H), 7.14 (ddd, *J* = 8.8, 2.4, 1.0 Hz, 1H), 4.52 (tt, *J =* 9.6, 4.2 Hz, 1H), 3.10 - 3.01 (m, 1H), 2.10 (dd, J = 12.6, 4.4 Hz, 1H), 2.00 (d, *J* = 11.8 Hz, 1H), 1.58 - 1.41 (m, 1H).

Step 3: The intermediate 18c (20.05g, 79.97mmol), triethylamine (32.37g, 319.87mmol), 1-propylphosphonic anhydride (12.2g, 96mmol), dichloromethane (200mL) were added sequentially into a single-necked flask, and then the reaction solution was stirred at room temperature for 5 minutes. 6-Chloro-3-pyridazinecarboxylic acid (16.48g, 103.96mmol) was slowly added into the single-necked flask , warmed up to 30 °C and continued to stir for 2.5 h. After completion of the reaction, it was concentrated and purified by slurrying (petroleum ether: ethyl acetate=1:1) to obtain a brown solid intermediate 18 (13.45 g).

¹H NMR (600 MHz, DMSO) δ 9.17 (d, *J* = 8.2 Hz, 1H), 8.23 (d, *J* = 8.9 Hz, 1H), 8.11 (d, *J* = 8.9 Hz, 1H), 7.87 (d, *J* = 8.7 Hz, 1H), 7.41 (d, *J* = 2.4 Hz, 1H), 7.15 (dd, *J =* 8.8, 2.4 Hz, 1H), 4.54 (td, *J* = 10.5, 5.1 Hz, 1H), 3.91 (dtd, *J* = 11.5, 7.7, 4.1 Hz, 1H), 2.13 (d, *J* = 12.3 Hz, 2H), 1.90 (s, 2H), 1.71 (q, *J =* 13.4 Hz, 2H), 1.57 - 1.48 (m, 2H).

### Example 19

### Synthesis of compound 1

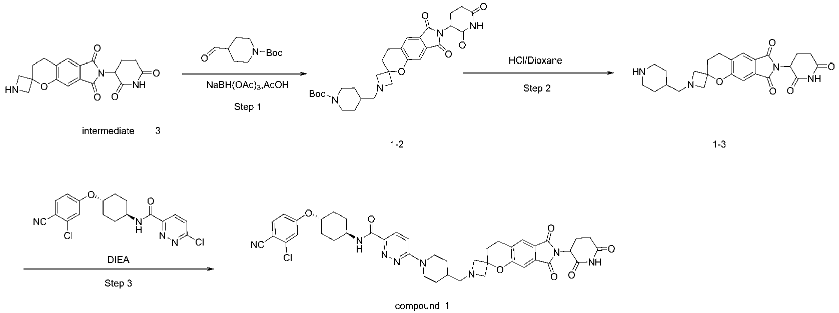

Step 1: The intermediate 3 (100 mg, 0.281 mmol) and tert-butyl 4-formylpiperidine-1-carboxylate (90 mg, 0.422 mmol) were added with solvents DCE (2 mL) and AcOH (4 drop) and reacted at room temperature for 1 h. Then NaBH(OAc)₃ (179 mg, 0.844 mmol) was added and the reaction continued for 1 h. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 1-2 (120 mg).
LC-MS(ESI): [M+H]⁺ = 497.40

Step 2: The compound 1-2 (100 mg, 0.181 mmol), Dioxane (1 mL) and 4M dioxane hydrochloride solution (1 mL) were reacted at room temperature for 0.5 h. The reaction solution was concentrated to obtain a white solid compound 1-3 (40 mg).

Step 3: The compound 1-3 (40 mg, 0.088 mmol), the intermediate 18 (69.17 mg, 0.176 mmol), DIEA (22.85 mg, 0.177 mmol), DMSO (1 mL) were added sequentially to an 8 mL tube. The reaction was carried out at 80 °C for 2 h under the protection of N₂. After the reaction was completed, it was cooled to room temperature, concentrated, and purified by Prep-HPLC preparation (15-50% acetonitrile) to obtain the white solid compound 1 (1.23 mg).

¹H NMR (400 MHz, *DMSO-d*₆) δ 11.11 (s, 1H), 8.57 (d, *J* = 8.2 Hz, 1H), 7.84 (dd, *J* = 11.0, 9.1 Hz, 2H), 7.78 (s, 1H), 7.41 - 7.34 (m, 2H), 7.29 (s, 1H), 7.13 (dd, *J =* 8.8, 2.5 Hz, 1H), 5.32 (s, 1H), 5.10 (dd, *J =* 12.8, 5.5 Hz, 2H), 4.43 (d, *J =* 65.0 Hz, 10H), 3.85 (s, 3H), 2.60 (d, *J =* 17.5 Hz, 3H), 2.25 (s, 3H), 2.15 - 1.86 (m, 8H), 1.78 (d, *J* = 12.5 Hz, 3H), 1.63 (q, *J* = 12.3 Hz, 3H).

LC-MS(ESI): [M+H]⁺ = 807.65

### Example 20

### Synthesis of compound 2

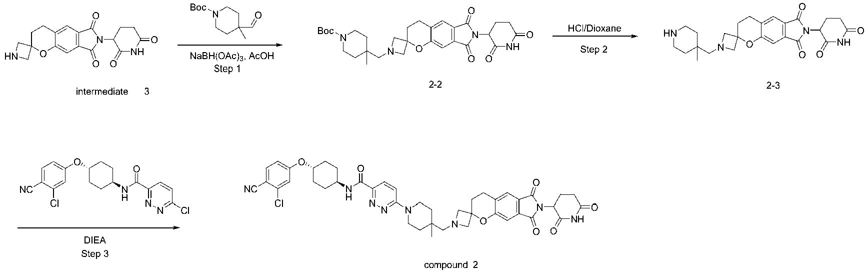

Step 1: The intermediate 3 (50 mg, 0.141 mmol) and tert-butyl acyl-4-methylpiperidine-1-carboxylate (48 mg, 0.211 mmol) were added to solvents DCE (1 mL) and AcOH (4 drops) and reacted at room temperature for 1 h. Then NaBH(OAc)₃(111 mg, 0.552 mmol) was added, and the reaction was continued for another 1 h at room temperature. The reaction solution was concentrated, washed with 5mL of saturated ammonium chloride aqueous solution, extracted with ethyl acetate, concentrated and purified by preparative thin-layer chromatography (DCM:MeOH=10:1) to obtain a white solid compound 2-2 (40 mg) .

Step 2: Compound 2-3 was prepared with reference to step 2 of Example 19.
¹H NMR (600 MHz, *DMSO-d*₆) δ 11.12 (s, 1H), 7.77 (s, 1H), 7.27 (s, 1H), 5.11 (dd, *J* = 12.9, 5.4 Hz, 1H), 4.57 (d, *J =* 44.1 Hz, 2H), 4.39 (s, 2H), 3.67 - 3.60 (m, 1H), 3.15 - 3.09 (m, 2H), 3.05 (d, *J =* 10.6 Hz, 2H), 2.97 (s, 2H), 2.89 (ddd, *J =* 17.2, 13.9, 5.4 Hz, 1H), 2.64 - 2.53 (m, 2H), 2.27 (s, 2H), 2.04 (dtd, *J* = 13.1, 5.4, 2.4 Hz, 1H), 1.65 (d, *J* = 13.0 Hz, 2H), 1.56 (s, 2H), 1.27 (dd, *J =* 9.7, 6.7 Hz, 2H), 1.07 (s, 3H) .
LC-MS(ESI): [M+H]⁺ = 467.41

Step 3: Compound 2 was prepared with reference to step 3 of Example 19.
¹H NMR (600 MHz, *DMSO-d*₆) δ 11.12 (s, 1H), 8.59 (d, *J =* 8.2 Hz, 1H), 7.85 (dd, *J =* 16.1, 9.1 Hz, 2H), 7.78 (s, 1H), 7.41 - 7.35 (m, 1H), 7.28 (d, *J =* 14.6 Hz, 1H), 7.20 - 7.01 (m, 2H), 5.11 (dd, *J =* 13.0, 5.5 Hz, 1H), 4.65 - 4.50 (m, 3H), 4.40 (s, 2H), 4.06 (d, *J =* 12.1 Hz, 2H), 3.91 - 3.82 (m, 1H), 3.44-3.47(m, 3H), 3.04 - 2.84 (m, 4H), 2.59 (s, 2H), 2.27 (d, *J =* 20.5 Hz, 2H), 2.15 - 2.00 (m, 3H), 1.93 - 1.86 (m, 2H), 1.69 - 1.60 (m, 2H), 1.60 - 1.50 (m, 3H), 1.48 (d, *J =* 19.5 Hz, 2H), 1.24 (s, 2H), 1.14 (s, 2H).
LC-MS(ESI): [M+H]⁺ = 821.61

### Example 21

### Synthesis of compound 3

### synthesis scheme

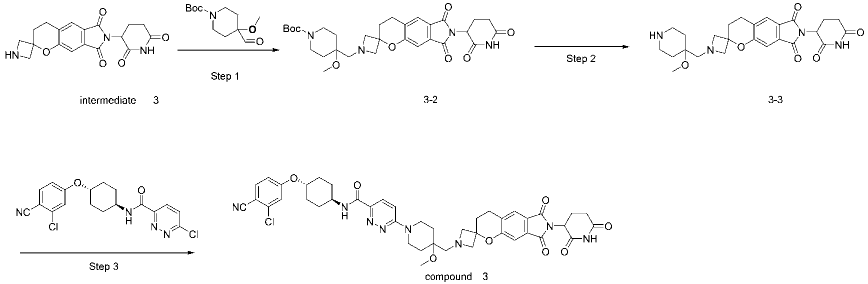

Step 1: The intermediate 3 (50 mg, 0.141 mmol) and tert-butyl 4-formyl-4-methoxypiperidine-1-carboxylate (51.35 mg, 0.211 mmol) were added to the solvent DCE (1 mL), AcOH (4 drop) and reacted at room temperature for 1 h. Then NaBH(OAc)₃₍111 mg, 0.552 mmol) was added and the reaction was continued for 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 3-2 (40 mg).

Step 2: Compound 3-3 was prepared with reference to step 2 of Example 19.
¹H NMR (600 MHz, *DMSO-d*₆) δ 11.12 (s, 1H), 7.77 (s, 1H), 7.28 (s, 1H), 5.11 (dd, *J =* 12.9, 5.4 Hz, 1H), 4.35 (s, 4H), 3.20 (s, 3H), 3.15 (ddt, *J =* 11.3, 7.4, 3.5 Hz, 3H), 3.00 - 2.90 (m, 4H), 2.90 - 2.85 (m, 2H), 2.74 (s, 1H), 2.63 - 2.53 (m, 2H), 2.30 (s, 2H), 2.09 - 2.01 (m, 1H), 1.95 (d, *J =* 14.4 Hz, 2H), 1.75 - 1.66 (m, 2H).
LC-MS(ESI): [M+H]⁺ = 483.42

Step 3: Compound 3 was prepared with reference to step 3 of Example 19.
¹H NMR (600 MHz, *DMSO-d*₆) δ 11.12 (d, *J =* 3.7 Hz, 1H), 8.62 (d, *J =* 8.2 Hz, 1H), 7.86 (dd, *J* = 9.1, 5.2 Hz, 2H), 7.78 (d, *J* = 2.4 Hz, 1H), 7.43 - 7.38 (m, 2H), 7.29 (d, *J* = 13.3 Hz, 1H), 7.14 (dd, *J =* 8.8, 2.4 Hz, 1H), 5.11 (dd, *J* = 13.0, 5.3 Hz, 1H), 4.61 - 4.48 (m, 4H), 4.35 (s, 2H), 4.21 (t, *J =* 16.1 Hz, 3H), 3.87 (s, 2H), 3.64 (s, 1H), 3.00 (s, 2H), 2.94 (s, 2H), 2.89 (s, 1H), 2.28 (d, *J =* 22.9 Hz, 3H), 2.11 (d, *J =* 11.7 Hz, 2H), 2.07 - 2.01 (m, 2H), 1.93 - 1.87 (m, 2H), 1.83 (d, *J =* 13.4 Hz, 2H), 1.69 - 1.57 (m, 4H), 1.56 - 1.48 (m, 3H).
LC-MS(ESI): [M+H]⁺ = 837.67

### Example 22

### Synthesis of compound 4

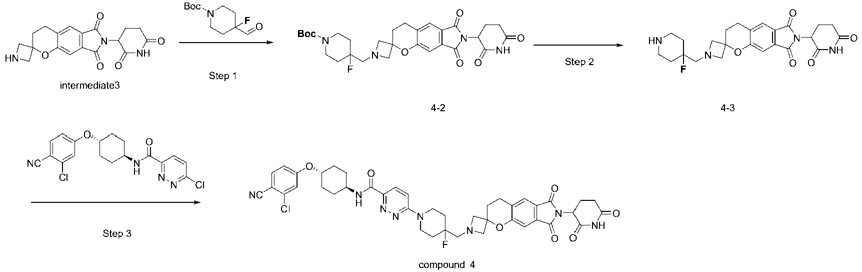

Step 1: The intermediate 3 (100 mg, 0.281 mmol) and tert-butyl 4-fluoro-4-formylpiperidine-1-carboxylate (97.62 mg, 0.422 mmol) were added to the solvents DCE (2 mL), AcOH (4 drop) and reacted at room temperature for 1 h. Then NaBH(OAc)₃ (111 mg, 0.552 mmol) was added and the reaction was continued for 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 4-2 (80 mg).

### Step 2.

Compound 4-3 was prepared with reference to the step 2 of Example 19.
¹H NMR (400 MHz, *DMSO-d*₆) δ 11.10 (s, 1H), 7.74 (s, 1H), 7.26 (s, 1H), 5.09 (dd, *J* = 12.8, 5.4 Hz, 1H), 3.28 (d, *J =* 12.8 Hz, 3H), 2.93 (ddt, *J =* 39.7, 13.8, 8.5 Hz, 5H), 2.68 - 2.53 (m, 2H), 2.49 (s, 2H), 2.23 (t, *J* = 6.4 Hz, 2H), 2.14 - 1.99 (m, 3H), 1.88 (dd, *J =* 35.3, 14.0 Hz, 2H).
LC-MS(ESI): [M+H]⁺ = 471.40

Step 3: (Compound 4) was prepared with reference to step 3 of Example 19.
¹ H NMR (400 MHz, DMSO-*d*₆) δ 11.10 (s, 1H), 8.61 (d, *J* = 8.2 Hz, 1H), 7.86 (dd, *J* = 9.1, 6.2 Hz, 2H), 7.77 (s, 1H), 7.45 (d, *J =* 9.6 Hz, 1H), 7.38 (d, *J =* 2.4 Hz, 1H), 7.28 (s, 1H), 7.13 (dd, *J =* 8.8, 2.4 Hz, 1H), 5.10 (dd, *J =* 12.8, 5.4 Hz, 1H), 4.59 - 4.29 (m, 6H), 3.92 - 3.67 (m, 4H), 3.31 (t, *J =* 12.6 Hz, 3H), 3.04 - 2.82 (m, 3H), 2.69 - 2.53 (m, 2H), 2.27 (t, *J =* 6.6 Hz, 2H), 2.07 (ddd, *J =* 25.1, 12.5, 4.9 Hz, 3H), 2.00 - 1.83 (m, 4H) 1.83 (m, 4H), 1.77 (d, *J =* 11.1 Hz, 1H), 1.70 - 1.58 (m, 2H), 1.58 - 1.45 (m, 2H).
LC-MS(ESI): [M+H]⁺ = 825.71

### Example 23

### Synthesis of compound 5

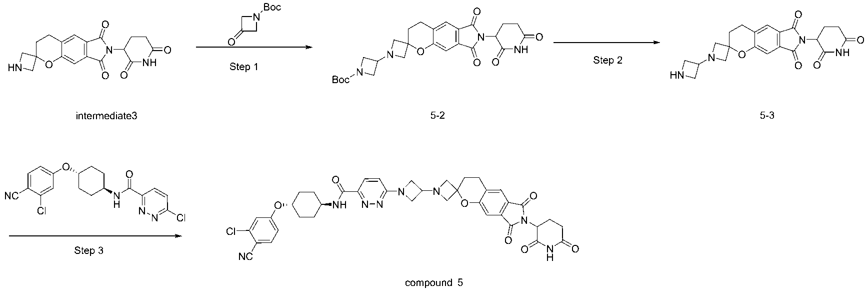

Step 1: The intermediate 3 (100 mg, 0.281 mmol) and tert-butyl 3-oxoazetidine-1-carboxylate (72.27 mg, 0.723 mmol) were added to the solvents DCE (2 mL), AcOH (4 drop) and reacted at room temperature for 1 h. Then NaBH(OAc)₃ (111 mg, 0.552 mmol) was added and the reaction was continued for another 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 5-2 (70 mg).
¹H NMR (600 MHz, DMSO-d₆) δ 11.11 (s, 1H), 7.72 (s, 1H), 7.26 (s, 1H), 5.63 (s, 3H), 5.09 (dd, *J* = 12.9, 5.4 Hz, 1H), 4.37 (t, *J* = 5.7 Hz, 3H), 3.98 (s, 6H), 3.86 (s, 2H), 3.58 (s, 8H), 3.50 - 3.45 (m, 1H), 3.40 (d, *J* = 8.5 Hz, 2H), 3.23 (d, *J =* 8.0 Hz, 2H), 2.98 - 2.85 (m, 3H), 2.61 (t, *J* = 3.3 Hz, 1H), 2.58 (d, *J* = 4.3 Hz, 1H), 2.55 (d, *J =* 13.0 Hz, 1H), 2.13 (t, *J* = 6.5 Hz, 2H), 2.03 (ddd, *J* = 10.8, 5.7, 3.5 Hz, 1H), 1.91 (s, 3H), 1.38 (s, 9H).
LC-MS(ESI): [M-tBu+H]⁺ = 455.40

Step 2: (Compound 5-3) were prepared with reference to step 2 of Example 19.
¹H NMR (600 MHz, DMSO-d₆) δ 11.10 (s, 1H), 7.75 (s, 1H), 7.27 (s, 1H), 5.10 (dd, *J* = 12.9, 5.4 Hz, 1H), 4.58 (q, *J* = 7.0 Hz, 1H), 4.05 (m, 5H), 2.95 (t, *J* = 6.5 Hz, 3H), 2.89-2.96 (m, 3H), 2.63 - 2.53 (m, 2H), 2.16 (t, *J* = 6.5 Hz, 2H), 2.07 - 2.01 (m, 1H), 1.53 (d, *J* = 7.0 Hz, 1H).
LC-MS(ESI): [M+H]⁺ = 411.47

Step 3: (Compound 5) was prepared with reference to step 3 of Example 19.
¹H NMR (600 MHz, DMSO-d₆) δ 11.11 (s, 1H), 8.62 (d, *J =* 8.2 Hz, 1H), 7.93 (d, *J =* 9.2 Hz, 1H), 7.87 (d, *J* = 8.7 Hz, 1H), 7.79 (s, 1H), 7.39 (d, *J* = 2.4 Hz, 1H), 7.33 (s, 1H), 7.14 (dd, *J* = 8.8, 2.5 Hz, 1H), 7.03 (d, *J* = 9.2 Hz, 1H), 5.11 (dd, *J* = 12.9, 5.4 Hz, 1H), 4.54 (td, *J* = 10.2, 5.1 Hz, 2H), 4.42 (d, *J* = 9.5 Hz, 3H), 4.19 - 4.14 (m, 2H), 3.91 - 3.83 (m, 2H), 2.98 (s, 2H), 2.90 (ddd, *J* = 17.0, 13.7, 5.4 Hz, 2H), 2.62 (s, 1H) , 2.57 (d, *J =* 25.3 Hz, 2H), 2.26 (t, *J* = 6.6 Hz, 2H), 2.14 - 2.09 (m, 2H), 2.05 (dd, *J* = 10.1, 4.9 Hz, 1H), 1.93 - 1.88 (m, 2H), 1.69 - 1.61 (m, 2H), 1.56 - 1.49 (m, 2H).
LC-MS(ESI): [M+H]⁺ = 765.60

### Example 24

### Synthesis of compound 6

### synthesis scheme

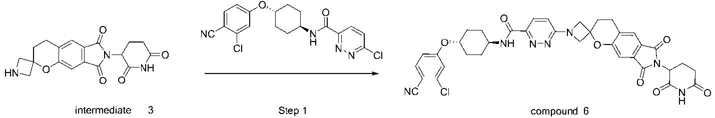

Step 1: (Compound 6) was prepared with reference to step 3 of Example 19.
¹H NMR (600 MHz, DMSO-d₆) δ 11.13 (s, 1H), 8.63 (d, *J* = 8.2 Hz, 1H), 7.92 (d, *J* = 9.2 Hz, 1H), 7.86 (d, *J* = 8.7 Hz, 1H), 7.78 (s, 1H), 7.40 (d, *J* = 2.4 Hz, 1H), 7.34 ( s, 1H), 7.14 (dd, *J* = 8.8, 2.4 Hz, 1H), 7.02 (d, *J* = 9.3 Hz, 1H), 5.11 (dd, *J* = 12.9, 5.4 Hz, 1H), 4.54 (tt, *J* = 10.1, 4.3 Hz, 1H), 4.35 (d, *J* = 9.6 Hz, 2H), 4.25 (d, *J* = 9.6 Hz, 2H), 3.87 (tdt, *J* = 11.6, 8.1, 4.0 Hz, 1H), 3.03 (t, *J=* 6.5 Hz, 2H), 2.89 (ddd, *J* = 17.0, 13.9, 5.5 Hz, 1H), 2.65 - 2.51 (m, 2H), 2.29 (t, *J* = 6.6 Hz 2H), 2.17 - 1.99 (m, 3H), 1.95 - 1.86 (m, 2H), 1.65 (qd, *J* = 13.2, 3.1 Hz, 2H), 1.57 - 1.46 (m, 2H).
LC-MS(ESI): [M+H]⁺ = 710.43

### Example 25

### Synthesis of compound 7

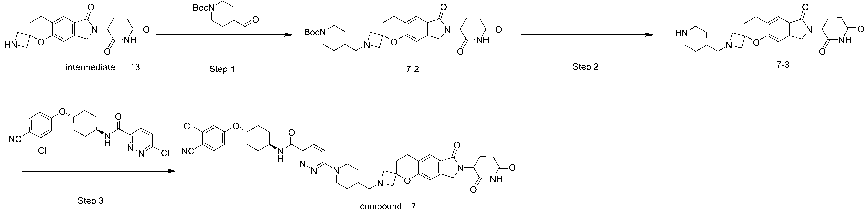

Step 1: The intermediate 13 (10 mg, 0.029 mmol), tert-butyl 4-formylpiperidine-1-carboxylate (9.37 mg, 0.044 mmol), sodium cyanoborohydride (18.63 mg, 0.812 mmol), tetraisopropyl titanate (15.4 mg, 0.059 mmol) were added to a single-necked flask containing tetrahydrofuran (2 mL) and stirred at 60 °C for 2 h. After the reaction was completed and cooled to room temperature, the reaction solution was purified by column chromatography (dichloromethane: methanol = 10:1) to obtain a white solid compound 7-2 (5 mg).
LC-MS(ESI): [M+H] ⁺ =539.52
¹H NMR (600 MHz, DMSO-d₆) δ 10.96 (s, 1H), 7.46 (s, 1H), 6.99 (s, 1H), 5.05 (dd, *J =* 13.4, 5.1 Hz, 1H), 4.33 (d, *J* = 16.7 Hz, 2H), 4.20 (d, *J* = 16.8 Hz, 2H), 3.90 ( s, 3H), 3.08 (s, 2H), 2.87 (d, *J* = 6.9 Hz, 2H), 2.60 (s, 2H), 2.40 - 2.35 (m, 4H), 2.07 (d, *J* = 7.3 Hz, 2H), 1.98 (d, *J* = 6.6 Hz, 2H), 1.64 (s, 2H), 1.46 (t , *J =* 7.2 Hz, 2H), 1.39 (s, 9H).

Step 2: The compound 7-2 was added to a single-necked flask containing dioxane (5 mL). A solution of hydrochloric acid (5 mL) in dioxane was added to the reaction flask. After addition, the reaction solution was stirred at room temperature for 2 h. After completion of the reaction, the reaction solution was concentrated directly to obtain a crude white solid compound 7-3 (10 mg).

Step 3: The compound 7-3 (10 mg, 0.023 mmol), the intermediate 18 (13.38 mg, 0.034 mmol), N,N-diisopropylethylamine (9.16 mg, 0.05 mmol), dimethylsulfoxide (2 mL) were added sequentially to a single-necked flask. The reaction solution was warmed to 80 °C and stirred for 2 h. After the reaction was completed, preparative purification (15-50% acetonitrile) was carried out to obtain a brown solid compound 7 (3 mg).

¹H NMR (600 MHz, Methanol-d₄) δ 7.94 (d, *J* = 9.6 Hz, 1H), 7.71 (d, *J* = 8.8 Hz, 1H), 7.61 (s, 1H), 7.35 (d, *J* = 9.6 Hz, 1H), 7.22 (d, *J* = 2.3 Hz, 1H), 7.15 (d, *J* = 17.4 Hz, 1H), 7.06 (dd, *J* = 8.8, 2.4 Hz, 1H), 5.14 (dd, *J* = 13.4, 5.1 Hz, 1H), 4.60 (d, *J* = 13.6 Hz, 3H), 4.48 - 4.39 (m, 4H), 4.00 (m, 2H), 3.86 (m, 1H), 3.40 (s, 2H), 3.35 (d, *J* = 3.7 Hz, 1H), 3.12 (t, *J* = 12.9 Hz, 2H), 3.00 (s, 3H), 2.92 (ddd, *J* = 18.5, 13.6, 5.4 Hz, 1H), 2.83 - 2.77 (m, 1H), 2.49 (qd, *J =* 13.3, 4.6 Hz, 1H), 2.33 (s, 3H), 2.22 (d, *J* = 7.4 Hz, 2H), 2.14 - 2.10 (m, 5H), 1.91 (d, *J* = 13.0 Hz, 2H).

LC-MS(ESI): [M+H]⁺ =793.66.

### Example 26

### Synthesis of compound 8

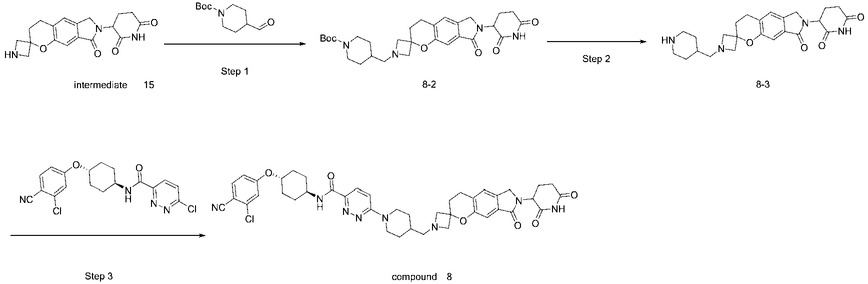

Step 1: The intermediate 15 (100 mg, 0.293 mmol) and tert-butyl 4-formylpiperidine-1-carboxylate (124.8 mg, 0.586 mmol) were added sequentially to the reaction flask, and the solvents DCE (1 mL) and AcOH (4 drops) were added dropwise, the reaction solution was stirred at room temperature for 1 h, and then NaBH(OAc) ₃₍186 mg, 0.879 mmol) was added, and the reaction solution was continued to be stirred at room temperature for 1 h. When the reaction was completed, it was concentrated, and then purified by preparative thin-layer chromatography (DCM:MeOH=10:1) to obtain a white solid compound 8-2 (56 mg).
¹H NMR (400 MHz, DMSO-d₆) δ 10.95 (s, 1H), 7.30 (s, 1H), 7.02 (s, 1H), 5.06 (dd, *J* = 13.3, 5.2 Hz, 1H), 4.31 (d, *J* = 16.6 Hz, 1H), 4.19 (d, *J* = 16.6 Hz, 1H), 3.90 (d, *J* = 13.0 Hz, 2H), 3.16 - 3.04 (m, 3H), 2.96 - 2.83 (m, 3H), 2.43 - 2.28 (m, 4H), 2.21 - 2.14 (m, 1H), 2.13 - 1.95 (m, 4H), 1.75 - 1.60 (m, 4H), 1.51 - 1.42 (m, 2H), 1.23 (s, 9H).
LC-MS: LC-MS(ESI): [M+H]⁺ =439.42

Step 2: (Compound 8-3) was prepared with reference to step 2 of Example 25.
LC-MS: [M+H]⁺ =439.44

Step 3: (Compound 8) was prepared with reference to step 3 of Example 25.
¹H NMR (600 MHz, DMSO-d₆) δ 10.99 (d, *J* = 2.2 Hz, 1H), 8.58 (d, *J* = 8.2 Hz, 1H), 7.86 (d, *J* = 8.8 Hz, 1H), 7.83 (d, *J* = 9.5 Hz, 1H), 7.38 (dd, *J* = 9.0, 2.2 Hz, 3H), 7.17 - 7.11 (m, 2H), 5.11 - 5.04 (m, 1H), 4.58 - 4.43 (m, 4H), 4.42 - 4.19 (m, 5H), 3.91 - 3.81 (m, 1H), 3.21-3.23 (m, 2H), 3.05 - 2.85 (m, 5H), 2.64 - 2.57 (m, 1H), 2.44 - 2.34 (m, 2H), 2.27 - 2.16 (m, 2H), 2.14 - 2.05 (m, 3H), 2.05 - 1.96 (m, 2H), 2.05 - 1.95 (m, 2H), 1.79 (d, *J* = 12.3 Hz, 2H), 1.72 - 1.60 (m, 2H), 1.58 - 1.48 (m, 2H).
LC-MS: [M+H]⁺ =793.67

### Example 27

### Synthesis of compound 9

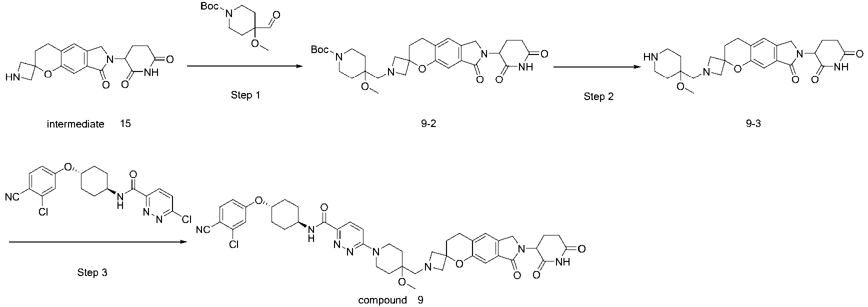

Step 1: The intermediate 15 (75 mg, 0.219 mmol), tert-butyl 4-formyl-4-methoxypiperidine-1-carboxylate (106.9 mg, 0.439 mmol) and Ti(O-iPr)₄ (125.0 mg, 0.439 mmol) were added sequentially in a reaction flask, and the solvent THF (1.0 mL) was added dropwise. The reaction solution was stirred at 60 °C for 1.5 h. Then NaBH₃CN (41.4 mg, 0.657 mmol) was added and the reaction solution was continued to be stirred at 60 °C for 1 h. After the reaction was completed, the reaction soluition was concentrated and then purified by TLC (DCM:MeOH=8:1) to obtain a white solid compound 9-2 (75 mg).

Step 2: (Compound 9-3) was prepared with reference to step 2 of Example 25.

Step 3: (Compound 9) was prepared with reference to step 3 of Example 25.

¹H NMR (400 MHz, DMSO-d₆) δ 10.96 (s, 1H), 8.58 (d, *J* = 8.2 Hz, 1H), 7.85 (t, *J* = 7.1 Hz, 2H), 7.37 (t, *J* = 6.7 Hz, 3H), 7.18 - 6.99 (m, 2H), 5.06 (dd, *J* = 13.2, 5.0 Hz, 1H), 4.61 - 4.47 (m, 1H), 4.26 (dd, *J* = 51.3, 16.4 Hz, 5H), 3.93 - 3.78 (m, 1H), 3.69 - 3.38 (m, 2H), 3.21 (m, 7H), 2.97 - 2.82 (m, 3H), 2.69 - 2.53 (m, 2H), 2.41 - 2.16 (m, 2H), 2.10 (d, *J* = 9.6 Hz, 3H), 2.02 - 1.73 (m, 5H), 1.57 (dq, *J* = 22.4, 10.6 Hz, 6H).
LC-MS(ESI): [M+H]⁺ = 823.70

### Example 28

### Synthesis of compound 10

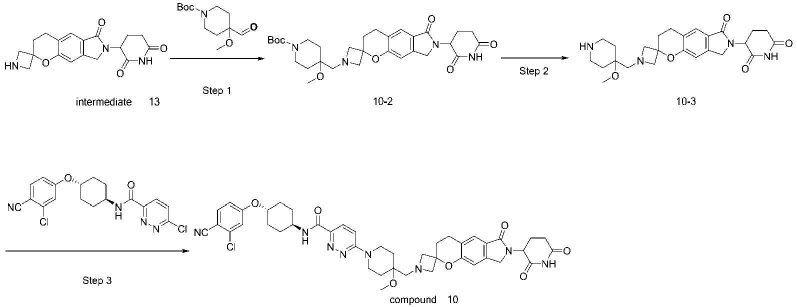

Step 1: The intermediate 13 (75 mg, 0.219 mmol) and tert-butyl 4-formyl-4-methoxypiperidine-1-carboxylate (106.4 mg, 0.438 mmol) were added sequentially to a reaction flask, and the solvent DCE (1 mL), AcOH (4 drop) were added dropwise, the reaction solution was stirred at room temperature for 1 h. Then NaBH(OAc)₃( 139 mg, 0.657 mmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 10-2 (50 mg).
¹H NMR (600 MHz, *DMSO-d*₆) *δ* 10.95 (s, 1H), 7.45 (s, 1H), 6.98 (s, 1H), 5.05 (dd, *J* = 13.4, 5.1 Hz, 1H), 4.32 (d, *J =* 16.9 Hz, 1H), 4.19 (d, *J =* 16.8 Hz, 1H), 3.61 (d, *J =* 13.1 Hz, 2H), 3.43 (d, *J =* 7.7 Hz, 1H), 3.19 (d, *J* = 7.6 Hz, 2H), 3.11 (s, 3H), 3.00 - 2.82 (m, 5H), 2.63 - 2.52 (m, 3H), 2.41 - 2.30 (m, 1H), 2.07 (t, *J* = 6.7 Hz, 2H), 2.03 - 1.89 (m, 2H), 1.65 (d, *J* = 13.6 Hz, 2H), 1.51 - 1.43 (m, 1H), 1.41 -1.30 (m , 10H).
LC-MS: [M+H]⁺ =569.56

Step 2: (Compound 10-3) was prepared with reference to step 2 of Example 25.
LC-MS: [M+H]⁺ =469.44

Step 3: (Compound 10) was prepared with reference to step 3 of Example 25.
¹H NMR (600 MHz, DMSO-*d*₆) δ 10.96 (s, 1H), 8.57 (d, *J* = 8.2 Hz, 1H), 7.83 (dd, *J* = 24.6, 9.2 Hz, 2H), 7.45 (s, 1H), 7.39 - 7.32 (m, 2H), 7.13 (dd, *J* = 8.8, 2.4 Hz, 1H), 6.99 (s, 1H), 5.03 (dd, *J =* 13.3, 5.1 Hz, 1H), 4.57 - 4.51 (m, 2H), 4.14-4.36 (m, 7H), 3.18 (s, 3H), 2.92 - 2.84 (m, 3H), 2.61 (s, 2H), 2.41 - 2.27 (m, 2H), 2.13 - 2.04 (m, 5H), 2.00 - 1.94 (m, 1H), 1.93 - 1.85 (m, 3H), 1.80 (d, *J* = 13.5 Hz, 2H), 1.69 - 1.58 (m, 3H), 1.56 - 1.45 (m, 4H).
LC-MS: [M+H]⁺ =823.64

### Example 29

### Synthesis of compound 11

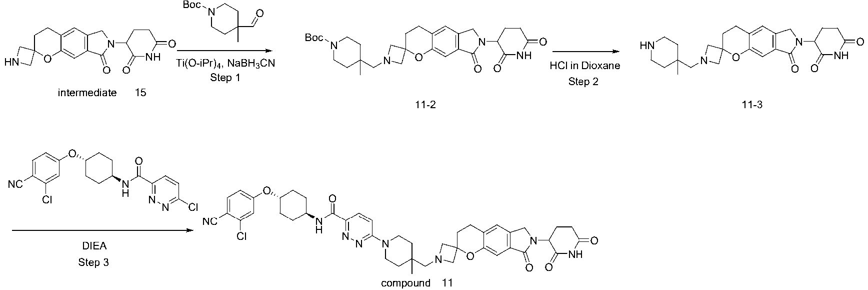

Step 1: The intermediate 15 (75 mg, 0.219 mmol) and tert-butyl 4-formyl-4-methylpiperidine-1-carboxylate (99.9 mg, 0.439 mmol) and Ti(O-iPr)₄₍133.3 mg, 0.439 mmol) were added sequentially in a glass vial, and the solvent THF (1 mL) was added dropwise. The reaction solution was stirred at 60 °C for 1.5 h, then NaBH₃CN (44.2 mg, 0.657 mmol) was added, and the reaction solution was continued to be stirred at 60 °C for 1 h. After the reaction was completed, it was concentrated, and then purified by TLC (DCM:MeOH=8:1) to obtain a white solid compound 11-2 (75 mg).

Step 2: (Compound 11-3) was prepared with reference to step 2 of Example 25.

Step 3: (Compound 11) was prepared with reference to step 3 of Example 25.
¹H NMR (400 MHz, DMSO-d₆) δ 10.95 (s, 1H), 8.57 (d, J = 8.2 Hz, 1H), 7.82 (dd, *J* = 23.0, 9.2 Hz, 2H), 7.35 (dd, *J* = 29.7, 5.3 Hz, 3H), 7.13 (dd, *J* = 8.8, 2.4 Hz, 1H) , 7.03 (s, 1H), 5.06 (dd, *J =* 13.2, 5.1 Hz, 1H), 4.59 - 4.48 (m, 1H), 4.25 (dd, *J* = 51.1, 16.7 Hz, 2H), 4.01 - 3.79 (m, 3H), 3.47 (d, *J* = 9.9 Hz, 3H), 3.22 (s, 2H), 2.95 - 2.80 (m, 3H), 2.69 - 2.52 (m, 2H), 2.47 - 2.29 (m, 5H), 2.09 (s, 3H), 2.03 - 1.82 (m, 3H), 1.71 - 1.27 (m, 8H), 0.99 (s, 2H).
LC-MS(ESI): [M+H]⁺ = 807.70

### Example 30

### Synthesis of compound 12

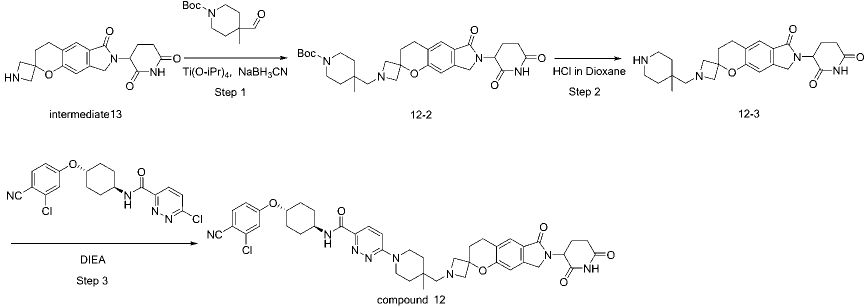

Step 1: The intermediate 13 (75 mg, 0.219 mmol), tert-butyl 4-formyl-4-methylpiperidine-1-carboxylate (99.9 mg, 0.439 mmol)H and Ti(O-iPr)₄₍124.9 mg, 0.439 mmol) were added sequentially in a glass vial, and the solvent THF (1.5 mL) was added dropwise, and the reaction solution was stirred at 60 °C for 1 h. Then NaBH₃CN (41.4 mg, 0.657 mmol) was added and the reaction solution was continued to be stirred at 60 °C for 1 h. After the reaction was completed, the reaction solution was concentrated and then purified by TLC (DCM:MeOH=10:1) to obtain a white solid compound 12-2 (75 mg).

Step 2: (Compound 12-3) was prepared with reference to Step 2 of Example 25.

Step 3: (Compound 12) was prepared with reference to step 3 of Example 25.
¹H NMR (400 MHz, DMSO-d₆) δ 10.94 (s, 1H), 8.57 (d, *J* = 8.2 Hz, 1H), 7.82 (dd, *J* = 22.3, 9.2 Hz, 2H), 7.48 - 7.26 (m, 3H), 7.13 (dd, *J* = 8.8, 2.4 Hz, 1H), 6.99 (s, 1H), 5.04 (dd, *J* = 13.2, 5.0 Hz, 1H), 4.59 - 4.48 (m, 1H), 4.26 (dd, *J* = 51.4, 16.9 Hz, 2H), 4.02 - 3.79 (m, 3H), 3.45 (t, *J* = 9.7 Hz, 3H), 3.23 (s, 2H), 2.88 (dd, *J* = 17.5, 11.7 Hz, 3H), 2.59 (dd, *J* = 34.5, 17.5 Hz, 2H), 2.45 - 2.29 (m, 5H), 2.09 (s, 3H), 2.00 - 1.84 (m, 3H), 1.57 (ddd, *J* = 32.3, 23.0, 10.2 Hz, 6H), 1.40 - 1.22 (m, 2H), 0.99 (s, 2H).
LC-MS(ESI): [M+H]⁺ = 807.66

### Example 31

### Synthesis of compound 13

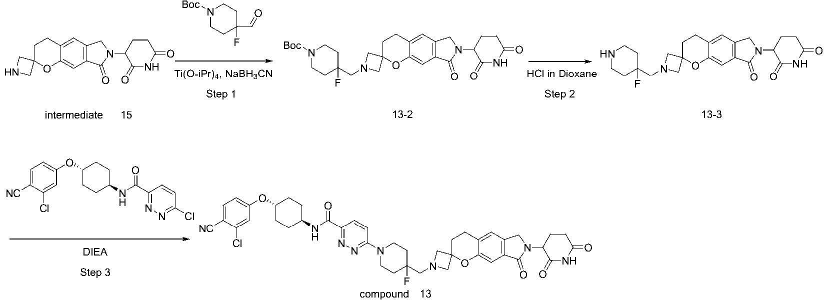

Step 1: The intermediate 15 (80 mg, 0.234 mmol) and tert-butyl 4-fluoro-4-formylpiperidine-1-carboxylate (108.4 mg, 0.468 mmol) and Ti(O-iPr)₄ (133.3 mg, 0.468 mmol) were added sequentially in a glass vial, and solvent THF (1.5 mL) was added dropwise, and the reaction solution was stirred at 60 °C for 1.5 h. Then NaBH₃CN (44.2 mg, 0.702 mmol) was added and the reaction solution was continued to be stirred at 60 °C for 1 h. After the reaction was completed, it was concentrated, and then purified by TLC (DCM:MeOH=10:1) to obtain a white solid compound 13-2 (80 mg).

Step 2: (Compound 13-3) was prepared with reference to step 2 of Example 25.

Step 3: (compound 13) was prepared with reference to the step 3 of Example 25.
¹H NMR (600 MHz, DMSO-d₆) δ 10.98 (s, 1H), 8.63 (d, *J* = 8.2 Hz, 1H), 7.87 (d, *J* = 8.8 Hz, 2H), 7.40 (d, *J* = 2.3 Hz, 3H), 7.14 (dd, *J* = 8.8, 2.3 Hz, 2H), 5.08 (dd, *J* = 13.2, 5.1 Hz, 1H), 4.59 - 4.50 (m, 1H), 4.27 (m, 5H), 3.93 - 3.83 (m, 1H), 3.46 (s, 1H), 3.27 (d, *J* = 39.4 Hz, 5H), 2.91 (dd, *J* = 14.6, 10.7 Hz, 3H), 2.77 (s, 2H), 2.64 - 2.53 (m, 2H), 2.42 - 2.32 (m, 2H), 2.11 (d, *J =* 9.7 Hz, 3H), 2.03 - 1.85 (m, 5H), 1.58 (ddd, *J* = 33.0, 23.6, 10.4 Hz, 4H).
LC-MS(ESI): [M+H]⁺ = 811.70

### Example 32

### Synthesis of compound 14

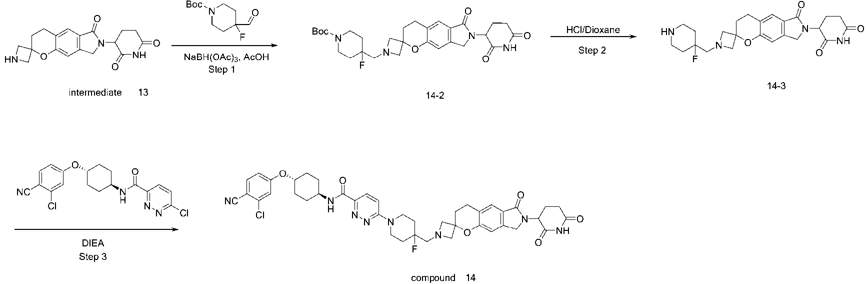

Step 1: The intermediate 13 (75 mg, 0.220 mmol) and tert-butyl 4-fluoro-4-formylpiperidine-1-carboxylate (76.22 mg, 0.330 mmol), Ti(oipr)₄₍73.13 mg, 0.261 mmol) were added to the solvent DCE (1 mL), AcOH (4 drops), and the reaction was continued at 60 °C for 1 h. Then NaBH(OAc)₃ (139.69 mg, 0.659 mmol) was added and the reaction was continued at 60 °C for 1 h. The reaction solution was concentrated, washed with 5 mL of saturated ammonium chloride aqueous solution, extracted with ethyl acetate, concentrated and then purified by preparative thin-layer chromatography (DCM:MeOH=10:1) to obtain a white solid compound 14-2 (50 mg).
¹H NMR (600 MHz, DMSO-d₆) δ 10.96 (s, 1H), 7.45 (s, 1H), 6.99 (s, 1H), 5.05 (dd, *J =* 13.3, 5.1 Hz, 1H), 3.73 *(d, J=* 13.0 Hz, 2H), 3.45 (d, *J* = 7.9 Hz, 2H), 3.21 (d *J=* 8.0 Hz, 2H), 2.89 (dt*, J =* 22.5, 7.4 Hz, 6H), 2.71 (d, *J* = 22.7 Hz, 2H), 2.59 (d, *J* = 17.2 Hz, 1H), 2.40 - 2.29 (m, 2H), 2.09 (t, *J* = 6.5 Hz, 2H), 2.00 ( dt, *J* = 26.7, 10.1 Hz, 3H), 1.77 (t, *J =* 12.1 Hz, 2H), 1.39 (d, *J* = 5.4 Hz, 9H).
LC-MS(ESI): [M+H]⁺ = 557.46

Step 2: (Compound 14-3) was prepared with reference to step 2 of Example 25.

Step 3: (Compound 14) was prepared with reference to step 3 of Example 19.
¹H NMR (600 MHz, DMSO-d₆) δ 10.89 (s, 1H), 8.55 (d, *J* = 8.2 Hz, 1H), 7.80 (t, *J* = 8.6 Hz, 2H), 7.45 (s, 1H), 7.38 (d, *J=* 9.6 Hz, 1H), 7.31 (d, *J =* 2.4 Hz, 1H), 7.08 - 6.95 (m, 3H), 4.99 (dd, *J =* 13.3, 5.1 Hz, 1H), 4.46 (tt, *J=* 10.0, 4.3 Hz, 2H), 4.28 (d, *J* = 18.8 Hz, 5H), 4.16 (d, *J* = 17.0 Hz, 2H), 3.79 (dt, *J* = 7.8, 3.7 Hz, 2H), 2.89 - 2.77 (m, 4H), 2.55 - 2.50 (m, 2H), 2.28 (dt, *J* = 13.1, 6.5 Hz, 2H), 2.22 - 2.11 (m, 3H), 2.06 - 1.99 (m, 2H), 1.89 (q, *J* = 11.5, 10.8 Hz, 3H), 1.85 - 1.80 (m, 2H), 1.57 (qd, *J* = 13.1, 3.2 Hz, 2H), 1.49 - 1.41 (m, 2H).
LC-MS(ESI): [M+H]⁺ = 811.70

### Example 33

### Synthesis of compound 15

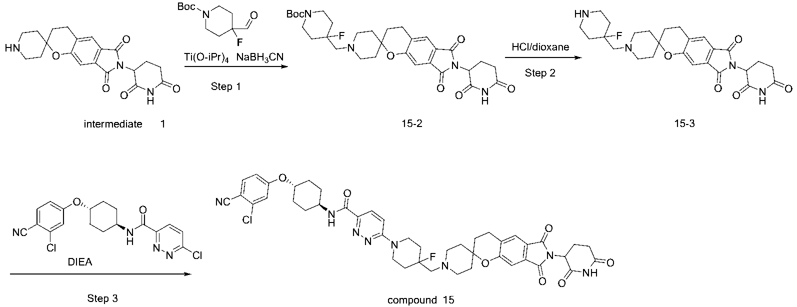

Step 1: The intermediate 1 (200 mg, 0.521 mmol) and tert-butyl 4-fluoro-4-formylpiperidine-1-carboxylate (180.96 mg, 0.782 mmol) were added sequentially to a glass vial, and the solvents THF (2 mL) and Ti(O-iPr)₄(296.52 mg, 1.04 mmol) were added, and the reaction solution was stirred at 60°C for 1 h. Then NaBH₃CN (98.34 mg, 1.56 mmol) was added, and the reaction solution was continued to be stirred at 60 °C for 1 h. After the reaction was completed, the reaction solution was concentrated and then purified by preparative thin-layer chromatography (DCM:MeOH=10:1) to obtain a white solid compound 15-2 (160 mg).
LC-MS: [M+H]⁺ =599.60

Step 2: (Compound 15-3) was prepared with reference to step 2 of Example 25.
LC-MS: [M+H]⁺ =499.46

Step 3: (Compound 15) was prepared with reference to step 3 of Example 25.
¹H NMR (600 MHz, DMSO-d₆) δ 11.11 (s, 1H), 9.50 (s, 1H), 8.64 (d, *J* = 8.2 Hz, 1H), 7.88 (dd, *J* = 15.2, 9.1 Hz, 2H), 7.76 (s, 1H), 7.48 (d, *J* = 9.6 Hz, 1H), 7.41 (s, 1H), 7.39 (d, *J* = 2.4 Hz, 1H), 7.14 (dd, *J* = 8.8, 2.4 Hz, 1H), 5.10 (dd, *J* = 12.9, 5.5 Hz, 1H), 4.54 (tt, *J* = 10.1, 4.3 Hz, 1H), 4.39 (d, *J* = 13.4 Hz, 2H), 3.62 (d, *J* = 19.6 Hz, 3H), 3.51 (s, 2H), 3.45 - 3.22 (m, 5H), 2.99 - 2.83 (m, 3H), 2.61 (dt, *J* = 17.1, 3.7 Hz, 1H), 2.57 - 2.52 (m, 1H), 2.17 - 1.95 (m, 9H), 1.91 (dq, *J* = 9.2, 4.7, 3.9 Hz, 4H), 1.82 (q, *J* = 7.8, 4.3 Hz, 1H), 1.65 (qd, *J* = 13.2, 3.2 Hz, 2H), 1.59 - 1.47 (m, 2H).
LC-MS: [M+H]⁺ =853.74

### Example 34

### Synthesis of compound 16

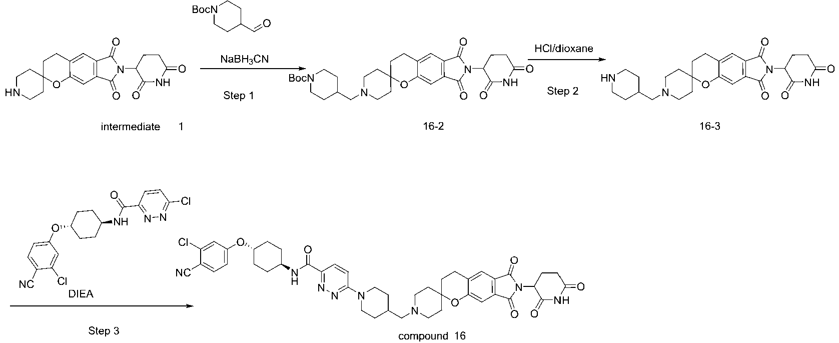

Step 1: The intermediate 1 (200 mg, 0.521 mmol) and tert-butyl 4-formylpiperidine-1-carboxylate (222.51 mg, 1.04 mmol) were added sequentially into a glass vial, and the solvent DCE (2 mL) was added, and the reaction solution was stirred at 25 °C for 16 h. Then NaBH₃CN (65.56 mg, 1.04 mmol) was added, and the reaction solution was continued to be stirred at 25 °C for 1 h. After the reaction was completed, the reaction solution was concentrated and then purified by preparative thin-layer chromatography (DCM:MeOH=10:1) to obtain a white solid compound 16-2 (130 mg).
¹H NMR (600 MHz, DMSO-d₆) δ 11.10 (s, 1H), 7.69 (s, 1H), 7.20 (s, 1H), 5.09 (dd, *J =* 12.9, 5.4 Hz, 1H), 4.46 (t, *J* = 5.3 Hz, 1H), 2.90 (td, *J =* 8.1, 7.6, 5.0 Hz, 3H ), 2.62 - 2.57 (m, 2H), 2.57 - 2.52 (m, 2H), 2.33 (s, 2H), 2.17 (d, *J* = 6.8 Hz, 2H), 2.05 - 2.00 (m, 1H), 1.85 (t, *J =* 6.7 Hz , 2H), 1.73 (d, *J* = 13.7 Hz, 2H), 1.67 (d, *J* = 12.4 Hz, 4H), 1.65 - 1.59 (m, 4H), 1.50 (ddd, *J=* 11.2, 7.0, 4.5 Hz, 2H), 1.40 (s, 9H).
LC-MS: [M+H]⁺ =581.50

Step 2: (Compound 16-3) was prepared with reference to step 2 of Example 19.
LC-MS: [M+H]⁺ =481.52

Step 3: (Compound 16) was prepared with reference to step 3 of Example 25.
¹H NMR (600 MHz, DMSO-d₆) δ 11.11 (s, 1H), 9.27 (s, 1H), 8.59 (d, *J* = 8.2 Hz, 1H), 7.85 (t, *J* = 9.4 Hz, 2H), 7.76 (s, 1H), 7.43 - 7.37 (m, 3H), 7.14 (dd, *J* = 8.8, 2.4 Hz, 1H), 5.10 (dd, *J* = 12.9, 5.5 Hz, 1H), 4.61 - 4.47 (m, 4H), 3.87 (dp, *J* = 11.3, 3.7 Hz, 1H), 3.49 (d, *J* = 11.7 Hz, 2H), 3.30 - 3.19 (m, 2H), 3.14 - 3.02 (m, 4H), 2.96 (q, *J* = 6.9 Hz, 2H), 2.89 (ddd, *J* = 17.1, 13.8, 5.5 Hz, 1H), 2.65 - 2.52 (m, 2H), 2.24 (tp, *J* = 11.3, 3.7 Hz, 1H), 3.30 (dp, *J* = 11.3, 3.7 Hz, 1H), 3.30 2.24 (tp, *J* = 13.2, 4.3, 3.7 Hz, 1H), 2.14 - 2.09 (m, 2H), 2.03 - 1.95 (m, 4H), 1.94 - 1.81 (m, 6H), 1.65 (qd, *J =* 13.2, 3.2 Hz, 2H) 3.2 Hz, 2H), 1.57 - 1.46 (m, 2H), 1.29 - 1.22 (m, 2H).
LC-MS: [M+H]⁺ =835.62

### Example 35

### Synthesis of compound 17

### Synthesis scheme:

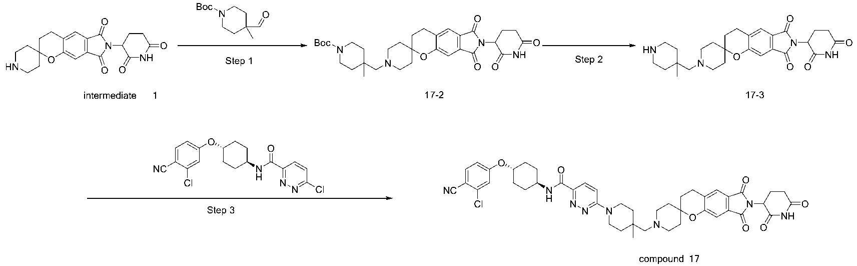

Step 1: The intermediate 1 (50 mg, 0.130 mmol), tert-butyl 4-formyl-4-methylpiperidine-1-carboxylate (44.48 mg, 0.196 mmol) and tetraisopropyl titanate (74.13 mg, 0.261) were added in 8 mL sample vial, and then the solvent THF (1ml) was added. The reaction solution was stired at 60 °C for 1h, then cooled down to room temperature. Then NaBH₃CN (16.39 mg, 0.261 mmol) was added, and the reaction solution was again heated up to 60 °C and stirred for 1 h. After the reaction was completed, it was concentrated under reduced pressure and then purified by preparative thin-layer chromatography (DCM:MeOH=10:1) to obtain a white solid compound 17-2 (50 mg, 93%).
¹H NMR (600 MHz, CDCl₃) δ 8.01 (s, 1H), 7.60 (s, 1H), 7.32 (s, 1H), 4.96 (dd, *J* = 12.5, 5.4 Hz, 1H), 3.90 (s, 2H), 3.70 (s, 3H), 3.19 - 3.07 (m, 4H), 2.97 - 2.88 (m, 3H), 2.86 - 2.79 (m, 1H), 2.78 - 2.70 (m, 2H), 2.68 (t, *J* = 10.5 Hz, 2H), 2.61 - 2.54 (m, 2H), 2.51 (dd, *J* = 16.2, 8.0 Hz, 1H), 2.20 (s, 2H), 2.17 - 2.11 (m, 1H), 2.05 (t*, J =* 11.1 Hz, 3H), 1.88 (dd, *J* = 19.1, 12.4 Hz, 2H), 1.77 (d, *J* = 12.7 Hz, 2H), 1.30 (d, *J* = 4.9 Hz, 9H), 0.96 (s, 3H).
LCMS(ESI): [M+H]⁺ = 595.54

Step 2: The compound 17-2 (40 mg, 0.067 mmol) was added to a 50 mL pear shaped vial, and solvents DCM (4 mL) and trifluoroacetic acid (1 mL) were added. The reaction solution was stirred at room temperature for 2 h. After completion of the reaction, the reaction solution was concentrated under reduced pressure to obtain a red oily compound 17-3 (47 mg, 92%).
LCMS(ESI): [M+H]⁺ = 495.46

Step 3: (Compound 17) was prepared with reference to step 3 of Example 25.
¹H NMR (600 MHz, DMSO-d₆) δ 11.03 (s, 1H), 8.52 (d, *J* = 8.2 Hz, 1H), 8.47 (s, 1H), 7.77 (t, *J* = 18.3, 8.3 Hz, 2H), 7.68 (s, 1H), 7.34 - 7.29 (m, 2H), 7.06 (dd, *J* = 8.8, 2.4 Hz, 1H), 5.02 (dd, *J =* 12.9, 5.4 Hz, 1H), 4.49 - 4.42 (m, 1H), 4.12 - 3.97 (m, 3H), 3.82 - 3.75 (m, 1H), 3.44 - 3.36 (m, 4H), 3.14 (t, *J* = 21.4 Hz, 4H), 2.91 - 2.76 (m, 4H), 2.55 - 2.44 (m, 2H), 2.06 - 1.92 (m, 5H), 1.91 - 1.78 (m, 5H), 1.61 - 1.38 (m, 7H), 1.20 - 1.14 (m, 3H).
LCMS(ESI): [M+H]⁺ = 849.67

### Example 36

### Synthesis of compound 18

### synthesis scheme

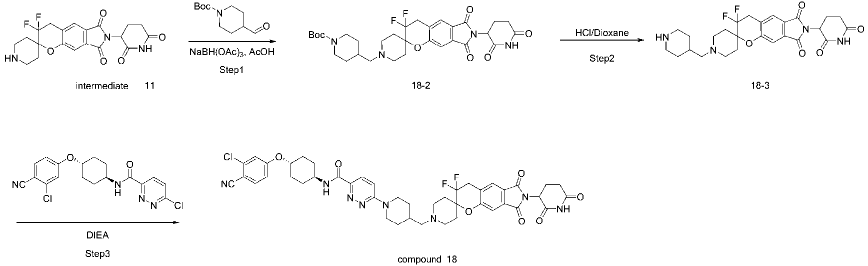

Step 1: The intermediate 11 (100 mg, 0.238 mmol) and tert-butyl 4-formylpiperidine-1-carboxylate (76.28 mg, 0.358 mmol) were added to the solvent DCE (1 mL), AcOH (4 drop), and the reaction was carried out at room temperature for 1 h. Then NaBH(OAc)₃ (111 mg, 0.552 mmol) was added, and the reaction was continued for 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 18-2 (80 mg).
¹H NMR (400 MHz, DMSO-d₆) δ 11.09 (s, 1H), 7.79 (s, 1H), 7.42 (s, 1H), 5.10 (dd, *J* = 12.7, 5.4 Hz, 1H), 3.90 (d, *J* = 12.8 Hz, 2H), 3.57 (t, *J* = 15.7 Hz, 2H), 2.87 (ddd, *J* = 16.6, 13.7, 5.2 Hz, 1H), 2.78 - 2.52 (m, 6H), 2.29 - 2.12 (m, 4H), 2.08 - 1.98 (m, 1H), 1.79 (d, *J* = 14.6 Hz, 4H), 1.66 (d, *J* = 12.0 Hz, 3H), 1.37 (s, 9H), 1.07 - 0.79 (m, 2H).
LC-MS(ESI): [M-tBu+H]⁺ = 561.40

Step 2: (Compound 18-3) was prepared with reference to step 2 of Example 19.
¹H NMR (600 MHz, DMSO-d₆) δ 11.13 (s, 1H), 9.71 (s, 1H), 7.85 (s, 1H), 7.58 (s, 1H), 5.13 (dd, *J* = 12.9, 5.4 Hz, 1H), 3.69 (t, *J* = 15.9 Hz, 3H), 3.32 (d, *J* = 12.7 Hz, 3H), 3.26 (s, 2H), 3.14 (s, 2H), 2.95 - 2.83 (m, 3H), 2.61 (dt, *J =* 17.3, 3.5 Hz, 1H), 2.56 (dd, *J* = 13.2, 4.5 Hz, 1H), 2.28 - 2.02 (m, 6H), 1.92 (d, *J* = 13.8 Hz, 2H), 1.38 (q, *J* = 12.0 Hz, 2H).
LC-MS(ESI): [M+H]⁺ = 517.42

Step 3: (Compound 18) was prepared with reference to step 3 of Example 19.
¹H NMR (600 MHz, DMSO-d₆) δ 11.13 (s, 1H), 9.14 (d, *J* = 22.4 Hz, 1H), 8.59 (d, *J* = 8.2 Hz, 1H), 7.88 - 7.81 (m, 2H), 7.60 (s, 1H), 7.42 - 7.38 (m, 2H), 7.14 (dd, *J* = 8.8, 2.4 Hz, 1H), 5.14 (dd, *J* = 12.9, 5.6 Hz, 1H), 4.54 (td, *J* = 10.8, 5.2 Hz, 3H), 3.91 - 3.83 (m, 1H), 3.70 (t, *J* = 16.0 Hz, 2H), 3.58 (m, 2H), 3.29 (d, *J* = 12.1 Hz, 2H), 3.13 (d, *J=* 7.3 Hz, 2H), 3.08 (t, *J* = 12.5 Hz, 2H), 2.93 - 2.85 (m, 1H), 2.61 (d, *J* = 15.6 Hz, 1H), 2.56 (td, J = 10.8, 5.2 Hz, 3H) 1H), 2.56 (dd, *J* = 12.7, 4.3 Hz, 1H), 2.24 (s, 3H), 2.09 (q, *J=* 14.5, 13.3 Hz, 5H), 1.89 (t, *J=* 15.4 Hz, 4H), 1.65 (q, *J* = 11.4 Hz, 2H), 1.57 - 1.48 (m 2H), 1.26 (d, *J* = 12.3 Hz, 2H).
LC-MS(ESI): [M+H]⁺ = 871.67

### Example 37

### Synthesis of compound 19

### synthesis scheme

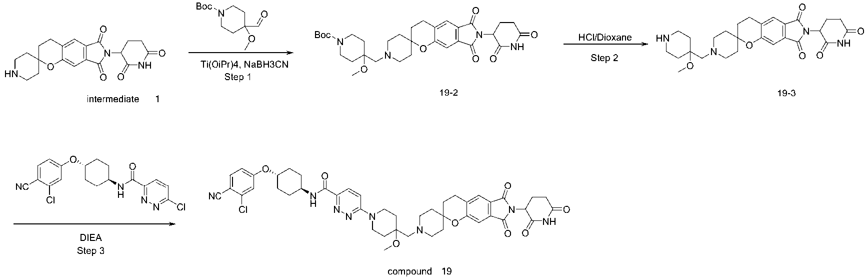

Step 1: The intermediate 1 (50 mg, 0.130 mmol) and 4-formyl-4-methoxypiperidine-1-carboxylic acid tert-butyl ester (47.59 mg, 0.196 mmol), Ti(oipr)₄ (73.13 mg, 0.261 mmol) were added to the solvents DCE (1 mL), AcOH (4 drop), and the reaction was continued for 1 h at 60 °C. Then NaBH₃CN (20.49 mg, 0.326 mmol) was added, and the reaction was continued for 1 h at 60 °C. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 19-2 (40 mg).
¹H NMR (600 MHz, DMSO-d₆) δ 11.08 (s, 1H), 7.68 (s, 1H), 7.19 (s, 1H), 5.07 (dd, *J* = 12.9, 5.5 Hz, 1H), 3.59 (d, *J* = 12.6 Hz, 2H), 3.24 (s, 1H), 3.22 (s, 1H), 3.10 (s, 3H), 2.91 - 2.83 (m, 4H), 2.64 (d, *J* = 9.6 Hz, 2H), 2.59 (d, *J* = 3.2 Hz, 1H), 2.57 - 2.51 (m, 2H), 2.37 (s, 2H), 2.04 - 1.97 (m, 2H), 1.82 (t, *J* = 6.8 Hz, 2H), 1.75 - 1.59 (m, 7H), 1.38 (d, *J* = 2.9 Hz, 9H).
LC-MS(ESI): [M+H]⁺ = 611.59

Step 2: (Compound 19-3) was prepared with reference to step 2 of Example 19.

Step 3: (Compound 19) was prepared with reference to step 3 of Example 19.
¹H NMR (600 MHz, DMSO-d₆) δ 11.09 (s, 1H), 8.92 (s, 1H), 8.60 (d, *J* = 8.1 Hz, 1H), 7.86 (s, 1H), 7.84 (s, 1H), 7.74 (s, 1H), 7.41 (d, *J* = 10.2 Hz, 2H), 7.38 (d, *J* = 2.4 Hz, 1H), 7.12 (dd, *J* = 8.8, 2.4 Hz, 1H), 5.09 (dd, *J* = 12.9, 5.5 Hz, 1H), 4.53 (dq, *J* = 10.5, 5.7, 5.1 Hz, 1H), 4.24 (dd, *J* = 10.8, 6.5 Hz, 2H), 3.86 (dt, *J* = 7.8, 3.7 Hz, 1H), 3.28 (s, 5H), 2.94 (t, *J* = 6.9 Hz, 2H), 2.87 (ddd, *J* = 16.9, 13.8, 5.5 Hz, 2H), 2.61 - 2.52 (m, 2H), 2.12 - 2.06 (m, 4H), 2.05 - 1.93 (m, 7H), 1.92 (s, 1H), 1.88 (t, *J* = 6.7 Hz, 4H), 1.63 (ddt, *J* = 16.0, 13.0, 5.5 Hz, 5H), 1.51 (qd,*J* = 13.0, 3.6 Hz, 3H).
LC-MS(ESI): [M+H]⁺ = 865.74

### Example 38

### Synthesis of compound 20

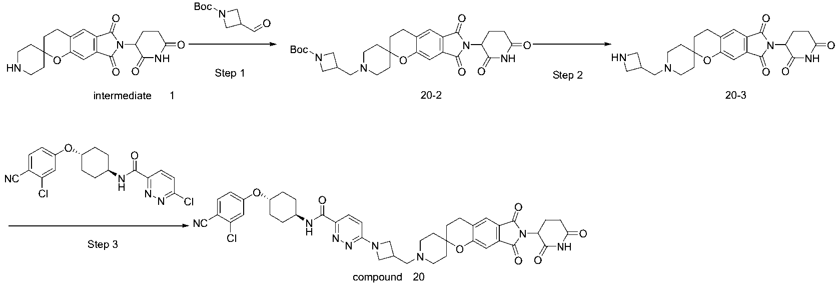

Step 1: The intermediate 1 (50 mg, 0.13 mmol) and tert-butyl 3-formylaniline-1-carboxylate (72.5 mg, 0.391 mmol) were added sequentially into a glass vial, solvents DCE (2 mL) and AcOH (1 drop) were added dropwise and the reaction solution was stirred at room temperature for 1 h. Then NaBH₃CN (24.6 mg, 0.39 mmol) was added and the reaction solution was continued to be stirred for 1 h at room temperature. After the reaction was completed, it was concentrated, and then purified by TLC (DCM:MeOH=10:1) to obtain a white solid (Compound 20-2) (50 mg).

Step 2: (Compound 20-3) was prepared with reference to step 2 of Example 19.

Step 3: The compound 20-3 (50 mg, 0.11 mmol), the intermediate 18 (86.5 mg, 0.22 mmol), K₂CO₃(30.5 mg, 0.22 mmol), and DMSO (1 mL) were added sequentially in a single-necked flask. The reaction solution was stirred at 80 °C for 2 h. When the reaction was completed, the reaction solution was cooled to room temperature, concentrated, and then purified by preparation to obtain white solid compound 20 (7.8 mg).
¹H NMR (600 MHz, DMSO-d₆) δ 11.11 (s, 1H), 8.59 (d, *J* = 8.2 Hz, 1H), 7.88 (dd, *J* = 13.1, 9.0 Hz, 2H), 7.76 (s, 1H), 7.43 - 7.37 (m, 2H), 7.14 (dd, *J* = 8.8, 2.3 Hz, 1H), 6.92 (d, J= 9.2 Hz, 1H), 5.10 (dd, *J* = 12.9, 5.4 Hz, 1H), 4.58 - 4.50 (m, 1H), 4.34 (t, *J* = 8.4 Hz, 2H), 3.99 (dd, *J* = 8.4, 5.9 Hz, 2H), 3.90 - 3.83 (m, 1H), 3.58 (d, *J=* 5.3 Hz, 2H), 3.34 (ddd, *J* = 41.3, 31.9, 10.6 Hz, 5H), 2.95 (dd, *J* = 30.3, 23.9 Hz, 3H), 2.58 (d, *J* = 43.6 Hz, 3H), 2.07 (dd, *J* = 50.5, 11.8 Hz, 5H), 1.97 - 1.85 (m, 5H), 1.71 - 1.47 (m, 4H).
LC-MS(ESI): [M+H]⁺ = 807.50

### Example 39

### Synthesis of compound 21

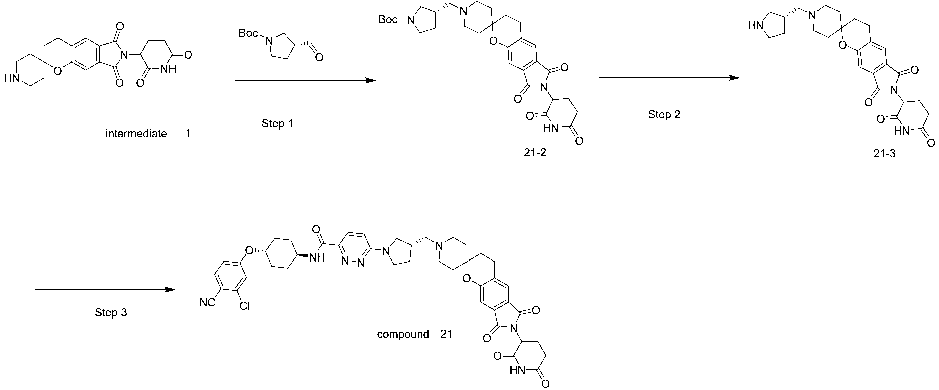

Step 1: The intermediate 1 (100 mg, 0.26 mmol) and tert-butyl (R)-3-formylpyrrolidine-1-carboxylate (103.9 mg, 0.52 mmol) were added sequentially in a glass vial, and the solvents DCM (2 mL), AcOH (2 drop) were added dropwise, and the reaction solution was stirred at room temperature for 3 h. Then, NaBH(OAc)₃ ( 165.8 mg, 0.78 mmol) was added, and the reaction solution was continued to be stirred at room temperature for 1 h. After the reaction was completed, it was concentrated, and then purified by TLC (DCM:MeOH=10:1) to obtain a white solid compound 21-2 (50 mg).
LC-MS: [M+H]⁺ =567.23

Step 2: The compound 21-2 (50 mg, 0.17 mmol), TFA/DCM (2 mL) solution were added sequentially in a single-necked flask. The reaction solution was stirred at room temperature for 1 h. After completion of the reaction, the reaction solution was concentrated directly to obtain a white solid compound 21-3 (40 mg).
LC-MS: [M+H]⁺ =467.32

Step 3: The (21-3) (80 mg, 0.17 mmol), the intermediate 18 (184.5 mg, 0.471 mmol), K₂CO₃ (130.3 mg, 0.94 mmol), and DMF (2 mL) were added sequentially in a single-necked flask, . The reaction solution was stirred at 80 °C for 1 h. When the reaction was completed, the reaction solution was cooled to room temperature, concentrated, and then purified by preparation to obtain white solid compound 21 (9 mg).
¹H NMR (600 MHz, DMSO-d₆) δ 11.12 (s, 1H), 9.26 (s, 1H), 8.52 (d, *J* = 8.0 Hz, 1H), 7.88 (t, *J* = 9.0 Hz, 1H), 7.77 (s, 1H), 7.66 - 7.53 (m, 1H), 7.42 - 7.36 (m, 1H), 7.14 (dt, *J* = 25.0, 20.0 Hz, 2H), 7.04 - 6.96 (m, 1H), 5.10 (dd, *J =* 12.9, 5.4 Hz, 1H), 4.59 - 4.49 (m, 1H), 3.94 - 3.80 (m, 2H), 3.51 (d, *J* = 4.8 Hz, 4H), 3.30 (s, 4H), 2.98 (t, *J* = 6.6 Hz, 2H), 2.92 - 2.83 (m, 6H), 2.64 - 2.54 (m, 1H), 2.29 (d, *J* = 5.1 Hz, 1H), 2.11 (d, *J* = 10.2 Hz, 2H), 2.06 - 2.00 (m, 2H), 2.01 - 1.89 (m, 5H), 1.86 (dd, *J* = 12.2, 8.8 Hz, 1H), 1.64 (dd, *J* = 23.9, 10.7 Hz, 1H), 1.53 (dd, *J =* 22.9, 9.4 Hz, 1H) .
LC-MS: [M+H]⁺ =821.63

### Example 40

### Synthesis of compound 22

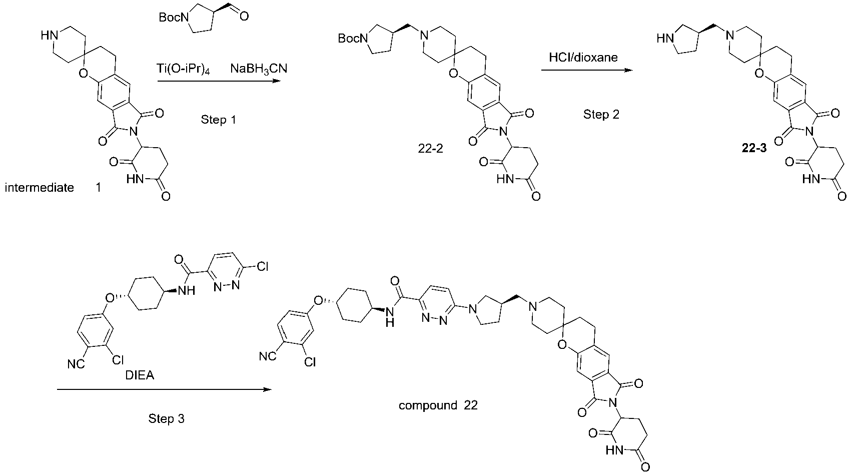

Step 1: The intermediate 1 (80 mg, 0.208 mmol) and tert-butyl (S)-3-formylpyrrolidine-1-carboxylate (83.15 mg, 0.417 mmol) were added sequentially to a glass vial, and the solvents THF (2 mL) and Ti(O-iPr)₄(118.61 mg, 0.417 mmol) were added. The reaction solution was stirred at 60 °C for 1h. Then NaBH₃CN (39.34 mg, 0.626 mmol) was added and the reaction solution was continued to be stirred at 60 °C for 1 h. After the reaction was completed, it was concentrated, and then purified by preparative thin-layer chromatography (DCM:MeOH=10:1) to obtain a white solid compound 22-2 (50 mg).
¹H NMR (600 MHz, Methanol-d₄) δ 7.65 (s, 1H), 7.26 (s, 1H), 5.10 (dd, *J =* 12.8, 5.4 Hz, 1H), 3.58 - 3.54 (m, 1H), 3.50 (q, *J* = 9.9, 9.0 Hz, 4H), 3.12 - 2.95 (m, 4H), 2.88 (ddd, *J* = 17.4, 13.9, 5.3 Hz, 2H), 2.77 - 2.71 (m, 2H), 2.61 - 2.46 (m, 4H), 2.11 - 1.99 (m, 2H), 1.95 - 1.84 (m, 4H), 1.84 - 1.75 (m, 2H), 1.48 (s, 9H).
LC-MS: [M+H]⁺ =567.49

Step 2: (Compound 22-3) was prepared with reference to step 2 of Example 19.
LC-MS: [M+H]⁺ =467.42

Step 3: (Compound 22) was prepared with reference to step 3 of Example 25.
¹H NMR (600 MHz, Methanol-d₄) δ 8.03 (d, *J =* 9.5 Hz, 1H), 7.73 - 7.70 (m, 2H), 7.43 (s, 1H), 7.22 (d, *J* = 2.4 Hz, 1H), 7.16 (d, *J* = 9.5 Hz, 1H), 7.06 (dd, *J* = 8.8, 2.5 Hz, 1H), 5.15 - 5.10 (m, 1H), 4.58 - 4.48 (m, 1H), 4.11 - 3.96 (m, 2H), 3.84 (s, 1H), 3.66 (d, *J* = 10.3 Hz , 3H), 3.47 (d, *J* = 23.5 Hz, 5H), 3.06 (t, *J* = 6.8 Hz, 2H), 2.99 (s, 1H), 2.90 - 2.83 (m, 1H), 2.79 - 2.69 (m, 2H), 2.47 (dtd, *J=* 12.9, 6.8 , 3.2 Hz, 1H), 2.22 (t*, J =* 7.1 Hz, 4H), 2.17 - 1.96 (m, 8H), 1.73 - 1.60 (m, 4H).
LC-MS: [M+H]⁺ =821.63

### Example 41

### Synthesis of compound 23

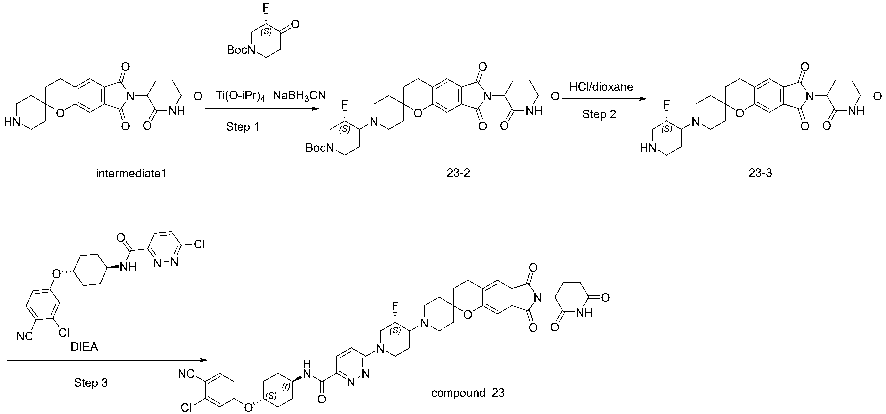

Step 1: The intermediate 1 (100 mg, 0.261 mmol) and tert-butyl (S)-3-fluoro-4-oxopiperidine-1-carboxylate (84.99 mg, 0.391 mmol) were added sequentially to a glass vial, and solvents THF (2 mL) and Ti(O-iPr)₄(148.26 mg, 0.521 mmol) were added, and the reaction solution was stirred at 60 °C for 1 h. Then NaBH₃CN (49.17 mg, 0.782 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 23-2 (60 mg).
LC-MS: [M+H]⁺ =585.56

Step 2: (Compound 23-3) was prepared with reference to step 2 of Example 19.
LC-MS: [M+H]⁺ =485.42

Step 3: (Compound 23) was prepared with reference to step 3 of Example 25.
¹H NMR (400 MHz, DMSO-d₆) δ 11.09 (s, 1H), 8.63 (d, *J* = 8.3 Hz, 1H), 7.85 (dd, *J* = 9.2, 4.1 Hz, 2H), 7.69 (s, 1H), 7.47 - 7.36 (m, 2H), 7.19 (s, 1H), 7.13 (dd, *J* = 8.8, 2.4 Hz, 1H), 5.08 (dd, *J* = 12.8, 5.4 Hz, 1H), 4.86 - 4.60 (m, 2H), 4.54 (ddt, *J =* 14.4, 9.7, 4.1 Hz, 1H), 4.27 (d, *J* = 13.1 Hz, 1H), 3.88 (d, *J =* 11.3 Hz, 1H), 3.29-3.11 (m, 4H), 2.90 (t, *J =* 6.7 Hz, 3H), 2.85 - 2.52 (m, 6H), 2.17 - 1.94 (m, 4H), 1.94 - 1.80 (m, 4H), 1.80 - 1.57 (m, 6H), 1.57 - 1.44 (m, 2H).
LC-MS: [M+H]⁺ =839.70

### Example 42

### Synthesis of compound 24

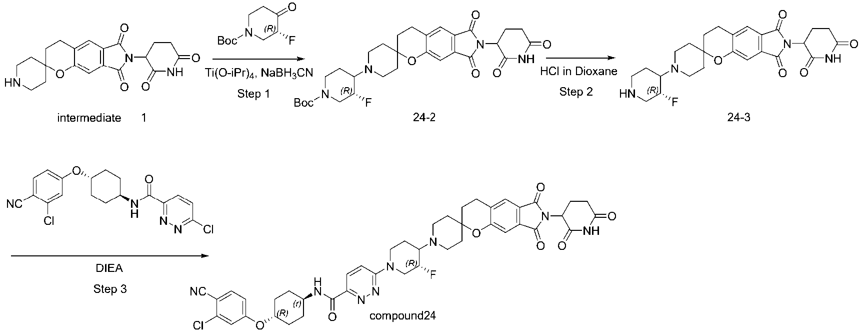

Step 1: The intermediate 1 (80 mg, 0.208 mmol) and tert-butyl (R)-3-fluoro-4-oxopiperidine-1-carboxylate (90.7 mg, 0.417 mmol) and Ti(O-iPr)₄ (118.6 mg, 0.417 mmol) were added sequentially in a glass vial, and then solvent THF (1.5 mL) was added dropwise, and the reaction solution was stirred at 60 °C for 1.5 h. Then NaBH₃CN (39.3 mg, 0.624 mmol) was added and the reaction solution was continued to be stirred at 60 °C for 1 h. After the reaction was completed, it was concentrated, and then purified by TLC (DCM:MeOH=10:1) to obtain a white solid compound 24-2 (100 mg).

Step 2: (Compound 24-3) was prepared with reference to step 2 of Example 19.

Step 3: (Compound 24) was prepared with reference to step 3 of Example 25.
¹H NMR (400 MHz, MeOD) δ 7.97 (d, *J* = 9.6 Hz, 1H), 7.73 - 7.67 (m, 2H), 7.47 - 7.36 (m, 2H), 7.20 (d, *J* = 2.3 Hz, 1H), 7.04 (dd, *J* = 8.8, 2.3 Hz, 1H), 5.63 - 5.28 (m, 2H), 5.09 (dt, *J* = 20.6, 10.5 Hz, 2H), 4.51 (d, *J* = 3.8 Hz, 1H), 4.00 (d, *J* = 3.9 Hz, 1H), 3.81 - 3.63 (m, 2H), 3.60 - 3.39 (m, 3H), 3.26 - 3.12 (m, 2H), 3.04 (t, *J* = 6.5 Hz, 2H), 2.93 - 2.60 (m, 4H), 2.38 (d, *J* = 10.8 Hz, 1H), 2.28 - 1.94 (m, 11H), 1.74 - 1.53 (m, 4H).
LC-MS(ESI): [M+H]⁺ = 839.69

### Example 43

### Synthesis of compound 25

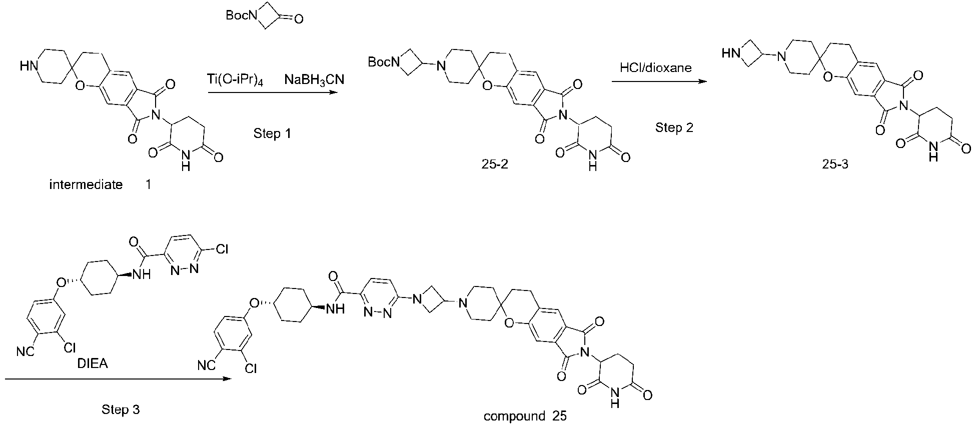

Step 1: The intermediate 1 (100 mg, 0.261 mmol) and tert-butyl 3-oxoazetidine-1-carboxylate (89.30 mg, 0.522 mmol) were added sequentially to a glass vial, and the solvents THF (2 mL) and Ti(O-iPr)₄ (148.26 mg, 0.522 mmol) were added, and the reaction solution was stirred at 60 °C for 1 h. Then NaBH₃CN (49.17 mg, 0.782 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 25-2 (72 mg).
¹H NMR (600 MHz, Methanol-d₄) δ 7.71 (s, 1H), 7.40 (s, 1H), 5.12 (dd, *J* = 12.8, 5.4 Hz, 1H), 4.28 (d, *J* = 16.9 Hz, 2H), 4.26 - 4.08 (m, 4H), 3.73 (dd, *J* = 9.3, 4.2 Hz, 1H), 3.50 (s, 2H), 3.04 (t, *J* = 6.8 Hz, 2H), 2.88 (ddd, *J* = 17.5, 14.0, 5.4 Hz, 1H), 2.79 - 2.69 (m, 2H), 2.21 (d, *J* = 15.1 Hz, 2H), 2.16 - 2.10 (m, 1H), 2.09 - 1.93 (m, 4H), 1.47 (d, *J* = 16.9 Hz, 9H).
LC-MS: [M+H]⁺ =539.46

Step 2: (Compound 25-3) was prepared with reference to step 2 of Example 19.
LC-MS: [M+H]⁺ =439.38

Step 3: (Compound 25) was prepared with reference to step 3 of Example 25.
¹H NMR (600 MHz, Methanol-d₄) δ 8.05 (d, *J* = 9.2 Hz, 1H), 7.71 (d, *J* = 8.6 Hz, 2H), 7.43 (s, 1H), 7.22 (d, *J* = 2.4 Hz, 1H), 7.11 - 7.02 (m, 2H), 5.12 (dd, *J* = 12.8, 5.4 Hz, 1H), 4.69 - 4.60 (m, 2H), 4.53 (dt, *J* = 9.4, 5.0 Hz, 1H), 4.47 (d, *J* = 8.1 Hz, 2H), 4.01 (dd, *J* = 10.2, 5.7 Hz, 1H), 3.67 - 3.54 (m, 2H), 3.43 (s, 2H), 3.06 (t, *J* = 6.8 Hz, 2H), 2.90 - 2.85 (m, 1H), 2.82 - 2.69 (m, 2H), 2.31 - 2.09 (m, 6H), 2.05 (d, *J* = 14.8 Hz, 3H), 1.67 (p, *J* = 6.3, 5.9 Hz, 3H), 1.41 - 1.29 (m, 4H). LC-MS: [M+H]⁺ =793.53

### Example 44

### Synthesis of compound 26

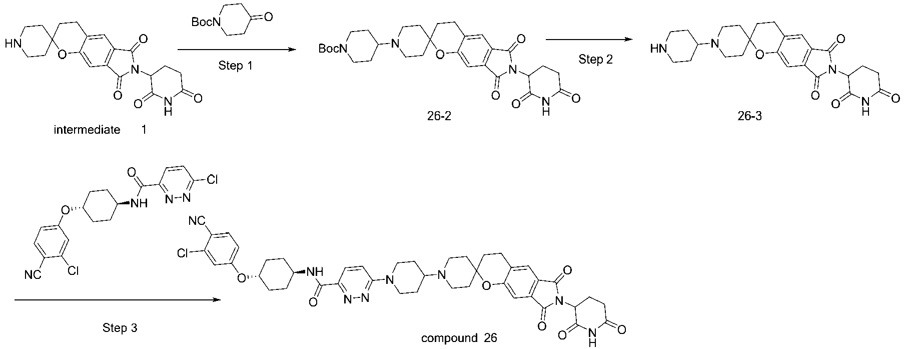

Step 1: The intermediate 1 (100 mg, 0.261 mmol) and tert-butyl 4-oxopiperidine-1-carboxylate (103.94 mg, 0.522 mmol) were added sequentially to a glass vial, and the solvents THF (2 mL) and Ti(O-iPr)₄ (148.36 mg, 0.522 mmol) were added, and the reaction solution was stirred at 60 °C for 1 h, then NaBH₃CN (32.78 mg, 0.522 mmol) was added and the reaction solution was continued to be stirred at 60 °C for 1 h. After the reaction was completed, it was concentrated, and then purified by preparative thin-layer chromatography (DCM:MeOH=10:1) to obtain a white solid compound 26-2 (50 mg).
¹H NMR (600 MHz, Methanol-d₄) δ 7.65 (s, 1H), 7.27 (s, 1H), 5.10 (dd, *J =* 12.8, 5.5 Hz, 1H), 4.15 (dd, *J* = 31.5, 13.5 Hz, 3H), 3.07 (s, 1H), 2.99 (t, *J* = 6.8 Hz, 2H), 2.87 (dq, *J* = 13.8, 5.4, 4.3 Hz, 3H), 2.76 (q, *J =* 13.3, 11.2 Hz, 4H), 2.63 (s, 1H), 2.12 (dd, *J* = 11.0, 5.3 Hz, 1H), 2.01 - 1.88 (m, 6H), 1.83 - 1.74 (m, 2H), 1.48 (d, *J =* 1.2 Hz, 11H).
LC-MS: [M+H]⁺ =567.55

Step 2: (Compound 26-3) was prepared with reference to step 2 of Example 19.
LC-MS: [M+H]⁺ =467.40

Step 3: (Compound 26) was prepared with reference to step 3 of Example 25.
¹H NMR (600 MHz, Methanol-d₄) δ 7.99 (dd, *J* = 9.6, 1.5 Hz, 1H), 7.75 - 7.68 (m, 2H), 7.47 - 7.40 (m, 2H), 7.22 (d, *J* = 2.4 Hz, 1H), 7.06 (dd, *J* = 8.8, 2.4 Hz, 1H), 5.15 - 5.10 (m, 1H), 4.80 (d, *J* = 13.7 Hz, 2H), 4.54 (d, *J* = 8.4 Hz, 1H), 4.01 (s, 1H), 3.74 - 3.66 (m, 1H), 3.60 (d, *J* = 12.2 Hz, 2H), 3.47 (t*, J* = 12.8 Hz, 2H), 3.17 (t, *J =* 13.0 Hz, 2H), 3.05 (t, *J* = 6.7 Hz, 2H), 2.96 - 2.68 (m, 4H), 2.36 (d, *J* = 11.8 Hz, 2H), 2.30 - 2.06 (m, 7H), 2.02 (tt, *J* = 14.5, 7.2 Hz, 3H), 1.91 - 1.80 (m, 2H), 1.75 - 1.60 (m, 4H).
LC-MS: [M+H]⁺ =821.64

### Example 45

### Synthesis of compound 27

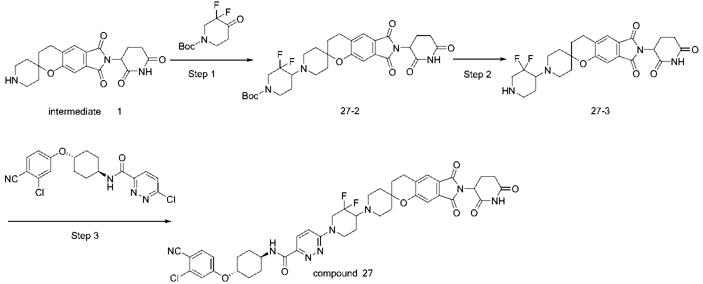

Step 1: The intermediate 1 (100 mg, 0.26 mmol) and tert-butyl 3,3-difluoro-4-oxopiperidine-1-carboxylate (122.7 mg, 0.52 mmol) and Ti(O-iPr)₄ (148.3 mg, 0.52 mmol) were added sequentially into a glass vial, solvent THF (2 mL) was added dropwise, and the reaction solution was stirred at 60 °C for 1.5 h_{.} Then NaBH₃CN (49.2 mg, 0.78 mmol) was added and the reaction solution was continued to be stirred at 60 °C for 1 h. After the reaction was completed, it was concentrated, and then purified by TLC (DCM:MeOH=10:1) to obtain a white solid compound 27-2 (70 mg).

Step 2: (Compound 27-3) was prepared with reference to step 2 of Example 19.

Step 3: The compound 27-3 (50 mg, 0.099 mmol), the intermediate 18 (77.9 mg, 0.199 mmol), KI (16.5 mg, 0.099 mmol), DIEA (12.9 mg, 0.495 mmol), and DMSO (2 mL) were added sequentially into a microwave tube. The reaction was carried out at 130 °C under microwave and N₂ for 3 h. After the reaction was completed, the reaction solution was cooled to room temperature, concentrated, and then purified by preparation to obtain white solid compound 27 (3 mg).
¹H NMR (400 MHz, MeOD) δ 8.01 (d, *J* = 9.4 Hz, 1H), 7.69 (d, *J* = 7.7 Hz, 2H), 7.50 (d, *J* = 9.6 Hz, 1H), 7.39 (s, 1H), 7.20 (t, *J* = 1.7 Hz, 1H), 7.04 (dd, *J* = 8.9, 2.1 Hz, 1H ), 5.10 (dd, *J* = 12.5, 5.5 Hz, 1H), 4.52 (d, *J* = 4.4 Hz, 1H), 4.24 (s, 1H), 3.99 (s, 1H), 3.73 - 3.46 (m, 5H), 3.02 (t, *J* = 6.7 Hz, 2H), 2.93 - 2.51 (m, 4H), 2.27 - 1.96 (m, 11H), 1.65 (t, *J* = 9.7 Hz, 4H), 1.40 - 1.24 (m, 4H).
LC-MS(ESI): [M+H]⁺ = 857.69

### Example 46

### Synthesis of compound 28

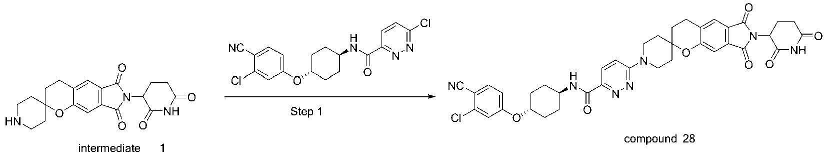

Step 1: The intermediate 1 (50 mg, 0.13 mmol), the intermediate 18 (102.1 mg, 0.26 mmol), DIEA (101.1 mg, 0.78 mmol), and DMSO (2 mL) were added sequentially in a single-necked flask. The reaction solution was stirred at 80 °C for 2 h. When the reaction was completed, it was cooled to room temperature, concentrated, and then purified by preparation to obtain white solid compound 28 (48.95 mg).
¹H NMR (600 MHz, DMSO-d₆) δ 11.11 (s, 1H), 8.63 (d*, J =* 8.2 Hz, 1H), 7.89 - 7.82 (m, 2H), 7.73 (s, 1H), 7.48 - 7.31 (m, 3H), 7.14 (dd, *J* = 8.8, 2.4 Hz, 1H), 5.10 (dd, *J =* 12.9, 5.4 Hz, 1H), 4.59 - 4.50 (m, 1H), 4.27 (d, *J =* 13.2 Hz, 2H), 3.91 - 3.81 (m, 2H), 3.53 (t, *J* = 11.9 Hz, 2H), 2.99 - 2.84 (m, 3H), 2.64 - 2.52 (m, 2H), 2.16 - 1.99 (m, 3H), 1.95 - 1.81 (m, 5H), 1.79 - 1.46 (m, 6H).
LC-MS(ESI): [M+H]⁺ = 738.41

### Example 47

### Synthesis of compound 29

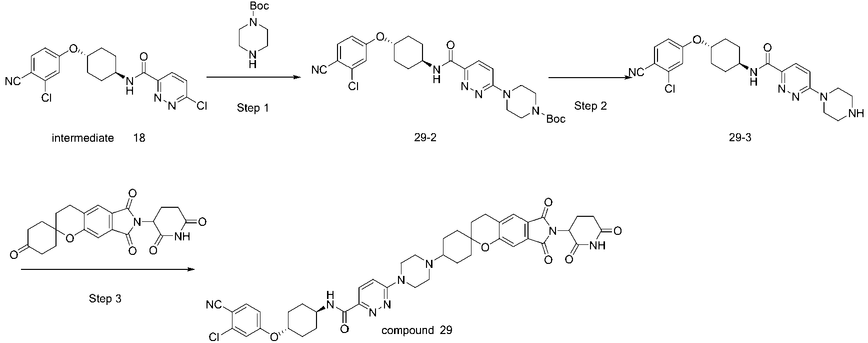

Step 1: The intermediate 18 (20 mg, 0.05 mmol) and tert-butyl piperazine-1-carboxylate (18.8 mg, 0.10 mmol) were added sequentially in a glass vial, and the solvents DCE (1.0 mL) and AcOH (1 drop) were added dropwise, and the reaction solution was stirred at room temperature for 1 h, then NaBH₃CN (9.5 mg, 0.15 mmol) was added. The reaction solution was continued to be stirred at room temperature for 1 h. After the reaction was completed, it was concentrated, and then purified by TLC (DCM:MeOH=10:1) to obtain a white solid compound 29-2 (5 mg).

Step 2: (Compound 29-3) was prepared with reference to step 2 of Example 19.

Step 3: The compound 29-3 (44 mg, 0.099 mmol), the intermediate 8 (20 mg, 0.05 mmol) were added sequentially in a single-necked flask, solvent DCM (1.0 mL), AcOH (1 drop) were added dropwise, and the reaction solution was stirred at room temperature for 1 h. Then NaBH(OAc)₃ (32.08 mg, 0.15 mmol) was added. The reaction solution was continued to be stirred at room temperature for 1 h. After the reaction was completed, it was concentrated, and then purified by preparation to obtain white solid compound 29 (6.25 mg).
¹H NMR (600 MHz, DMSO-d₆) δ 11.12 (s, 1H), 8.71 (d, *J* = 8.2 Hz, 1H), 7.96 (d, *J* = 9.5 Hz, 1H), 7.87 (d, *J =* 8.8 Hz, 1H), 7.74 (s, 1H), 7.51 (d, *J =* 9.6 Hz, 1H), 7.40 (d, *J =* 2.4 Hz, 1H), 7.28 (s, 1H), 7.14 (dd, *J =* 8.8, 2.4 Hz, 1H), 5.10 (dd, *J* = 12.9, 5.4 Hz, 1H), 4.67 (d, *J* = 13.2 Hz, 2H), 4.54 (s, 1H), 3.88 (d, *J* = 8.1 Hz, 2H), 3.68 (s, 3H), 3.41 - 3.31 (m, 3H), 3.26 (d, *J* = 10.7 Hz, 2H), 2.94 (s, 3H), 2.64 - 2.56 (m, 1H), 2.11 (d, *J* = 9.8 Hz, 2H), 1.90 (ddd, *J =* 28.5, 24.7, 9.2 Hz, 10H), 1.70 - 1.46 (m, 6H).
LC-MS(ESI): [M+H]⁺ = 821.60

### Example 48

### Synthesis of compound 30

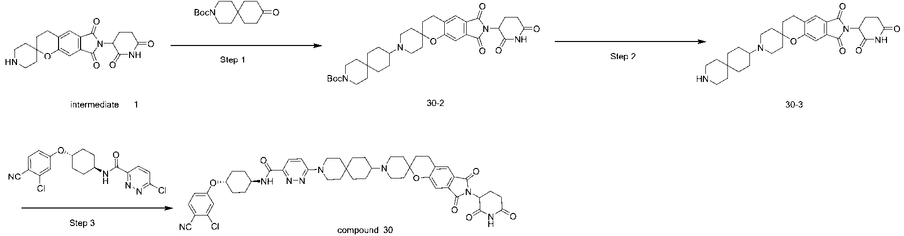

Step 1: The intermediate 1 (100 mg 0.26 mmol), tert-butyl 9-oxo-3-azaspiro[5.5]undecane-3-carboxylate (83.68 mg, 0.313 mmol), sodium cyanoborohydride (49.17 mg, 0.782 mmol), tetraisopropyl titanate (148.26 mg, 0.521 mmol) were added into a single-necked flask containing tetrahydrofuran (2 mL). The reaction solution was stirred at 60 °C for 2 h. After completion of the reaction, the reaction solution was cooled to room temperature and purified by TLC (dichloromethane:methanol=10:1) to obtain a white solid compound 30-3 (75 mg).

¹H NMR (500 MHz, Chloroform-d) δ 8.99 (s, 1H), 7.80 (t, *J* = 1.0 Hz, 1H), 7.58 (s, 1H), 5.43 (t, *J* = 7.0 Hz, 1H), 3.70 (t, *J* = 7.1 Hz, 4H), 2.86 - 2.80 (m, 2H), 2.80 - 2.77 (m, 1H), 2.68 (d*, J =* 7.1 Hz, 1H), 2.66 - 2.60 (m, 4H), 2.28 (t, *J =* 7.1 Hz, 2H), 2.20 - 2.12 (m, 2H), 2.12 - 2.07 (m, 1H), 2.06 (s, 1H), 2.04 (d, *J =* 5.3 Hz, 1H), 2.02 (s, 1H), 2.02 - 1.98 (m, 1H), 1.83 (t, *J =* 7.1 Hz, 4H), 1.74 - 1.66 (m, 2H), 1.64 - 1.56 (m, 2H), 1.51 - 1.48 (m, 4H), 1.46 (s, 9H).

Step 2: (Compound 30-3) was prepared with reference to Step 2 of Example 25.

Step 3: (compound 30) was prepared with reference to step 3 of Example 25.
LC-MS(ESI): [M+H]⁺ =889.63
¹H NMR (600 MHz, Methanol-d₄₎ . δ 7.94 (d, *J* = 9.3 Hz, 1H), 7.75 (s, 1H), 7.71 (d, *J* = 8.8 Hz, 1H), 7.26 (s, 1H), 7.23 (d, *J =* 2.4 Hz, 1H), 7.07 (dd, *J =* 8.8, 2.4 Hz, 1H), 6.90 (d, *J* = 9.4 Hz, 1H), 5.36- 5.31 (t, *J =* 5.36 Hz, 1H), 5.12 (dd, *J* = 12.8, 5.4 Hz, 1H), 4.56 - 4.50 (m, 1H), 4.00 (s, 1H), 3.91 (s, 2H), 2.89 (ddd, *J* = 17.4, 13.9, 5.3 Hz, 2H), 2.79 (dd, *J =* 4.5, 2.6 Hz, 2H), 2.77 - 2.70 (m, 2H), 2.21 (d, *J* = 14.4 Hz, 4H), 2.17 - 2.06 (m, 6H), 2.02 (d, *J* = 13.5 Hz, 2H), 1.73 (t, *J* = 13.1 Hz, 2H), 1.69 - 1.61 (m, 4H), 1.57 (t, *J* = 12.7 Hz, 2H), 1.33 (d, *J* = 20.4 Hz, 2H)

### Example 49

### Synthesis of compound 31

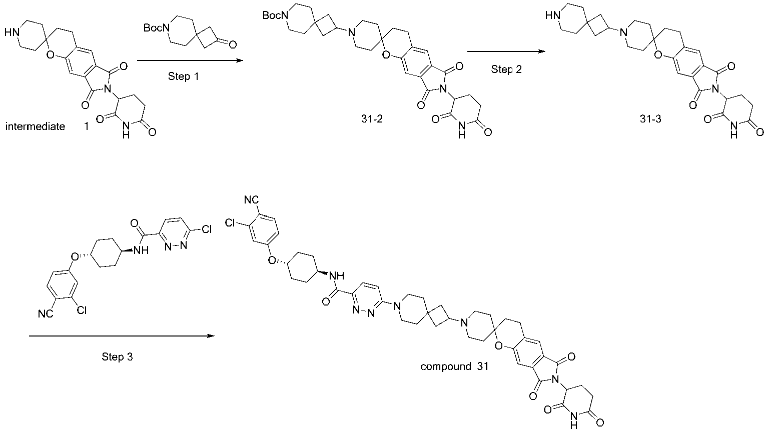

Step 1: The intermediate 1 (200 mg, 0.521 mmol) and tert-butyl 2-oxo-7-azaspiro[3.5]nonane-7-carboxylate (249.67 mg, 1.04 mmol) were added sequentially in a glass vial, and the solvents THF (2 mL) and Ti(O-iPr)₄(296.5 mg, 1.04 mmol) were added, and the reaction solution was stirred at 60 °C for 1 h. Then NaBH₃CN (98.3 mg, 1.56 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 31-2 (200 mg).
LC-MS: [M+H]⁺ =607.53

Step 2: The compound 31-2 (200 mg, 0.346 mmol), DCM (1 mL) solution and TFA (1 mL) were added sequentially in a single-necked flask. The reaction solution was stirred at room temperature for 1 h. After completion of the reaction, the reaction solution was concentrated directly to obtain a white solid compound 31-3 (150 mg).
¹H NMR (600 MHz, DMSO-d₆) δ 11.12 (s, 1H), 9.83 (s, 1H), 8.40 (s, 2H), 7.76 (d, *J* = 5.4 Hz, 1H), 7.35 (d, *J* = 5.3 Hz, 1H), 5.10 (dd, *J* = 12.9, 5.4 Hz, 1H), 3.29 (d *J* = 11.8 Hz, 2H), 3.12 - 2.93 (m, 8H), 2.89 (ddd, *J* = 17.0, 13.9, 5.6 Hz, 2H), 2.61 (d, *J* = 20.8 Hz, 1H), 2.25 (dd, *J=* 12.4, 7.9 Hz, 2H), 2.11 - 1.90 (m, 8H), 1.89 - 1.80 (m, 2H), 1.70 (dt, *J* = 25.8, 5.9 Hz, 4H).
LC-MS: [M+H]⁺ =507.50

Step 3: (Compound 31) was prepared with reference to step 3 of Example 25.
¹H NMR (600 MHz, DMSO-d₆) δ 11.11 (s, 1H), 9.84 (q, *J* = 9.5 Hz, 1H), 8.57 (d, *J* = 8.2 Hz, 1H), 7.84 (dd, *J* = 21.0, 9.1 Hz, 2H), 7.75 (d, *J =* 4.2 Hz, 1H), 7.42 - 7.33 (m, 3H), 7.14 (dd, *J* = 8.8, 2.4 Hz, 1H), 5.10 (dd, *J* = 12.9, 5.5 Hz, 1H), 4.54 (tt, *J* = 9.9, 4.4 Hz, 1H), 3.92 - 3.81 (m, 2H), 3.74 ( t, *J =* 5.5 Hz, 2H), 3.66 (t*, J =* 5.4 Hz, 2H), 3.33 (d, *J =* 11.4 Hz, 2H), 3.08 (q, *J =* 11.8 Hz, 2H), 2.97 (t, *J =* 7.0 Hz, 2H), 2.89 (ddd, *J* = 17.7, 13.9, 5.5 Hz, 1H), 2.64 - 2.52 (m, 2H), 2.27 (ddd, *J* = 12.0, 7.9 Hz, 2H), 2.14 - 2.01 (m, 7H), 1.92 (dddd, *J* = 27.9, 23.3, 16.9, 5.0 Hz, 6H), 1.72 - 1.57 (m, 6H), 1.57 - 1.47 (m, 2H).
LC-MS: [M+H]⁺ =861.66

### Example 50

### Synthesis of compound 32

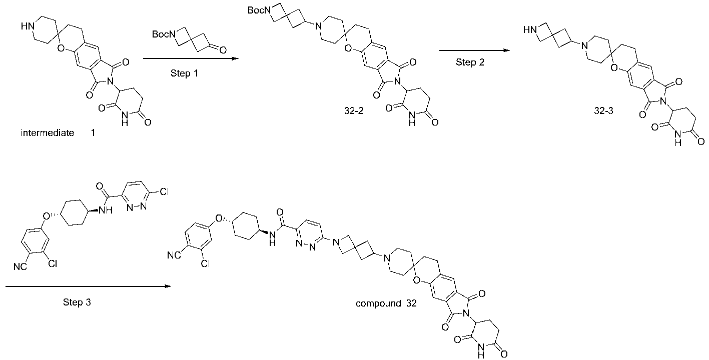

Step 1: The intermediate 1 (80 mg, 0.208 mmol) and tert-butyl 6-oxo-2-azaspiro[3.3]heptane-2-carboxylate (88.16 mg, 0.417 mmol) were added sequentially to a glass vial, and the solvents THF (2 mL) and Ti(O-iPr)₄ (177.91 mg, 0.626 mmol) were added. The reaction solution was stirred at 60 °C for 1 h. Then NaBH₃CN (26.22 mg, 0.417 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 32-2 (60 mg).
¹H NMR (600 MHz, Methanol-d₄) δ 7.67 (s, 1H), 7.31 (s, 1H), 5.11 (dd, *J* = 12.8, 5.4 Hz, 1H), 4.00 (s, 2H), 3.93 - 3.85 (m, 2H), 3.21 (p, *J* = 8.3 Hz 1H), 3.01 (t, *J* = 6.7 Hz, 2H), 2.90 - 2.82 (m, 1H), 2.81 - 2.68 (m, 3H), 2.51 (ddt, *J =* 10.1, 6.5, 3.6 Hz, 2H), 2.31 - 2.18 (m, 2H) 2.18 (m, 2H), 2.16 - 2.04 (m, 2H), 2.00 (s, 4H), 1.97 (t, *J =* 6.8 Hz, 2H), 1.89 - 1.79 (m, 2H), 1.44 (d, *J =* 5.7 Hz, 9H).
LC-MS: [M+H]⁺ =579.51

Step 2: (Compound 32-3) was prepared with reference to step 2 of Example 19.
LC-MS: [M+H]⁺ =479.40

Step 3: (Compound 32) was prepared with reference to step 3 of Example 25.
¹H NMR (600 MHz, Methanol-d₄) δ 8.04 (d, *J* = 9.4 Hz, 1H), 7.74 - 7.68 (m, 2H), 7.41 (s, 1H), 7.22 (d, *J* = 2.4 Hz, 1H), 7.09 - 7.04 (m, 2H), 5.12 (dd, *J* = 12.8, 5.5 Hz, 1H), 4.52 (dd, *J* = 9.6, 5.0 Hz, 1H), 4.42 (s, 2H), 4.32 (s, 2H), 3.99 (dd, *J* = 10.1, 5.8 Hz, 1H), 3.86 (p, *J* = 8.3 Hz, 1H), 3.49 (d, *J* = 12.1 Hz, 2H), 3.24 (t, *J* = 12.9 Hz, 2H), 3.04 (q, *J* = 5.8, 4.8 Hz, 2H), 2.92 - 2.69 (m, 5H), 2.64 (t, *J* = 11.2 Hz, 2H), 2.27 - 2.17 (m, 4H), 2.16 - 1.93 (m, 7H), 1.72 - 1.59 (m, 4H).
LC-MS: [M+H]⁺ =833.63

### Example 51

### Synthesis of compound 33

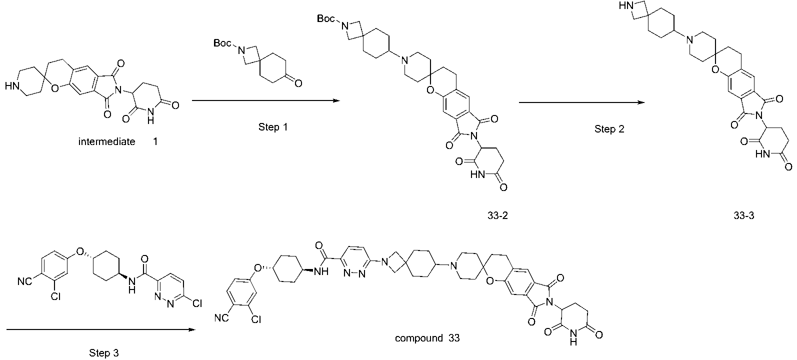

Step 1: The intermediate 1 (200 mg 0.52 mmol), tert-butyl 7-oxo-2-azaspiro[3.5]nonane-2-carboxylate (250.33 mg, 1.05 mmol), sodium cyanoborohydride (100 mg), and tetraisopropyl titanate (300 mg) were added to a single-necked flask containing tetrahydrofuran (2 mL) and stirred at 60 °C for 2 h. After the reaction was completed and cooled to room temperature, the reaction solution was purified using column chromatography (dichloromethane:methanol=10:1) to obtain a white solid compound 33-2 (142 mg).
LC-MS: [M+H]⁺ =607.03

Step 2: (Compound 33-3) was prepared with reference to step 2 of Example 39.
LC-MS: [M+H]⁺ =507.23

Step 3: (Compound 33) was prepared with reference to step 3 of Example 39.
¹H NMR (600 MHz, MeOD) δ 8.04 (d, *J* = 9.4 Hz, 1H), 7.73 - 7.68 (m, 2H), 7.42 (s, 1H), 7.22 (d, *J* = 2.3 Hz, 1H), 7.09 - 7.04 (m, 2H), 5.36 (s, 1H), 5.12 (dd, *J =* 12.7, 5.4 Hz, 1H), 4.56 - 4.49 (m, 1H), 4.05 (d, *J* = 52.5 Hz, 5H), 3.54 (s, 2H), 3.46 (dd, *J =* 21.7, 7.1 Hz, 2H), 3.05 (t, *J* = 6.5 Hz, 2H), 2.94 - 2.85 (m, 1H), 2.75 (s, 2H), 2.30 (d, *J* = 12.9 Hz, 2H), 2.22 (dd, *J* = 19.1, 11.4 Hz, 5H), 2.17 - 1.97 (m, 8H), 1.80 (s, 2H), 1.74 - 1.54 (m, 6H).
LC-MS: [M+H]⁺ =861.58

### Example 52

### Synthesis of compound 34

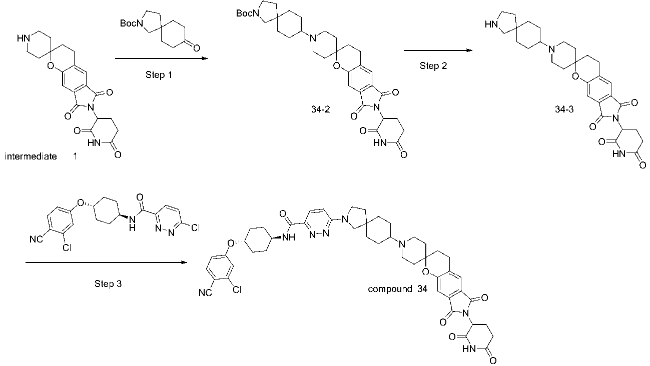

Step 1: The intermediate 1 (150 mg, 0.391 mmol) and tert-butyl 8-oxo-2-azaspiro[4.5]decane-2-carboxylate (198.23 mg, 0.782 mmol) were added sequentially to a glass vial, and the solvents THF (2 mL) and Ti(O-iPr)₄(222.39 mg, 0.782 mmol) were added. The reaction solution was stirred at 60 °C for 1 h. Then NaBH₃CN (73.76 mg, 1.17 mmol) was added and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 34-2 (100 mg).
¹H NMR (600 MHz, Methanol-d₄) δ 7.66 (s, 1H), 7.31 (d, *J* = 4.6 Hz, 1H), 5.10 (dd, *J =* 12.8, 5.4 Hz, 1H), 3.45 - 3.35 (m, 3H), 3.25 (d, *J =* 7.2 Hz, 1H ), 3.12 - 2.97 (m, 6H), 2.88 (ddd, *J* = 17.5, 13.9, 5.3 Hz, 1H), 2.76 (ddt, *J* = 18.1, 7.3, 3.5 Hz, 2H), 2.71 (dd, *J* = 13.1, 4.4 Hz, 1H), 2.16 - 2.10 (m, 1H), 2.08 - 1.93 (m, 6H), 1.89 - 1.69 (m, 6H), 1.58 (t, *J* = 12.5 Hz, 1H), 1.48 (d, *J =* 4.4 Hz, 12H).
LC-MS: [M+H]⁺ =621.59

Step 2: (Compound 34-3) was prepared with reference to step 2 of Example 19.
LC-MS: [M+H]⁺ =521.48

Step 3: (Compound 34) was prepared with reference to step 3 of Example 25.
¹H NMR (600 MHz, Methanol-d₄) δ 7.94 (dd, *J =* 9.5, 5.5 Hz, 1H), 7.74 - 7.66 (m, 2H), 7.33 (d, *J* = 4.4 Hz, 1H), 7.23 (d, *J* = 2.3 Hz, 1H), 7.09 - 6.97 (m, 2H), 5.11 (ddd, *J* = 12.7, 5.4, 2.6 Hz, 1H), 4.53 (d, *J =* 7.4 Hz, 1H), 3.99 (dt, *J =* 10.9, 6.3 Hz, 1H), 3.70 (s, 2H), 3.41 (s, 4H), 3.32 - 3.05 (m, 5H), 3.02 (t, *J* = 6.8 Hz, 2H), 2.94 - 2.81 (m, 2H), 2.80 - 2.70 (m, 2H), 2.23 (dt, *J* = 9.2*,* 5.0 Hz, 2H), 2.17 - 2.02 (m, 7H), 2.01 - 1.83 (m, 6H), 1.75 - 1.55 (m, 7H).
LC-MS: [M+H]⁺ =875.73

### Example 53

### Synthesis of compound 35

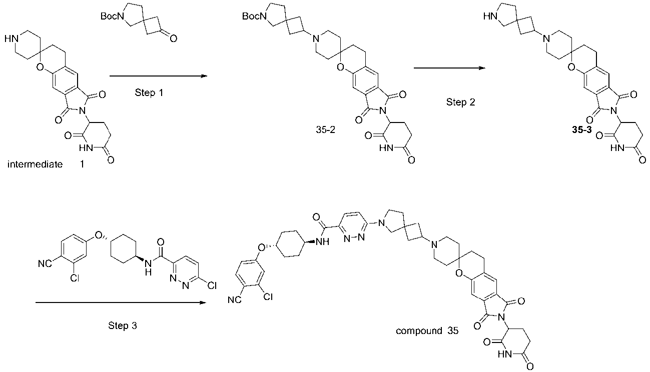

Step 1: The intermediate 1 (100 mg, 0.261 mmol) and tert-butyl 2-oxo-6-azaspiro[3.4]octane-6-carboxylate (117.52 mg, 0.522 mmol) were added sequentially to a glass vial, and the solvents THF (2 mL) and Ti(O-iPr)₄ (148.26 mg, 0.522 mmol) were added. The reaction solution was stirred at 60 °C for 1 h. Then NaBH₃CN (49.17 mg, 0.782 mmol) was added and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 35-2 (75 mg).
¹H NMR (600 MHz, Methanol-d₄) δ 7.68 (s, 1H), 7.33 (s, 1H), 5.11 (dd, *J* = 12.9, 5.4 Hz, 1H), 3.40 (d, *J* = 14.2 Hz, 2H), 3.31 - 3.22 (m, 2H), 3.08 (d, *J* = 13.2 Hz, 2H), 3.01 (t, *J* = 6.7 Hz, 2H), 2.93 - 2.68 (m, 5H), 2.34 - 2.29 (m, 2H), 2.15 - 2.10 (m, 2H), 2.05 (d, *J* = 14.1 Hz, 2H), 1.99 (s, 4H), 1.90 (dtd, *J* = 26.9, 14.0, 11.7, 5.3 Hz, 4H), 1.48 (d, *J* = 3.6 Hz, 9H).
LC-MS: [M+H]⁺ =593.53

Step 2: (Compound 35-3) was prepared with reference to step 2 of Example 19.
LC-MS: [M+H]⁺ =493.37

Step 3: (Compound 35) was prepared with reference to step 3 of Example 25.
¹H NMR (600 MHz, Methanol-d₄) δ 8.07 (dd, *J* = 9.6, 4.4 Hz, 1H), 7.71 (d, *J* = 9.1 Hz, 2H), 7.40 (d, *J* = 2.7 Hz, 1H), 7.28 (dd, *J* = 14.5, 9.7 Hz, 1H), 7.22 (dd, *J* = 2.5, 1.3 Hz, 1H), 7.06 (ddd, *J* = 8.8, 2.4, 1.2 Hz, 1H), 5.12 (dd, *J* = 12.8, 5.4 Hz, 1H), 4.55 (q, *J* = 7.0 Hz, 2H), 4.04 - 3.90 (m, 2H), 3.84 - 3.63 (m, 4H), 3.49 (d, *J* = 12.1 Hz, 2H), 3.25 (q, *J* = 10.6, 8.8 Hz, 2H), 3.04 (t, *J* = 6.7 Hz, 2H), 2.96 - 2.66 (m, 4H), 2.64 - 2.41 (m, 4H), 2.28 (t, *J* = 6.6 Hz, 1H), 2.25 - 2.09 (m, 8H), 2.04 (dt, *J* = 14.3, 6.7 Hz, 4H), 1.66 - 1.60 (m, 2H).
LC-MS: [M+H]⁺ =847.65

### Example 54

### Synthesis of compound 36

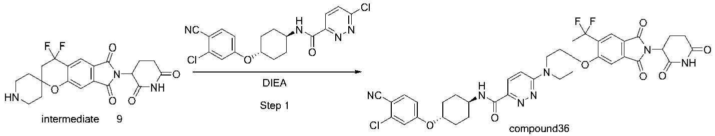

Step 1: The intermediate 9 (20 mg, 0.047 mmol), the intermediate 18 (37.3 mg, 0.095 mmol), DIEA (30.8 mg, 0.235 mmol), DMSO (1 mL) were added sequentially in a single-necked flask. The reaction solution was stirred at 80 °C for 2 h. After the reaction was completed, the reaction solution was cooled to room temperature, concentrated, and then purified by preparation to obtain white solid compound 36 (10.5 mg).
¹H NMR (600 MHz, DMSO-d₆) δ 11.15 (s, 1H), 8.64 (d, *J* = 8.2 Hz, 1H), 8.10 (s, 1H), 7.86 (dd, *J* = 9.2, 1.8 Hz, 2H), 7.65 (s, 1H), 7.42 (dd, *J* = 27.8, 6.0 Hz, 2H), 7.14 (dd, *J* = 8.8, 2.4 Hz, 1H), 5.17 (dd, *J* = 12.9, 5.4 Hz, 1H), 4.54 (s, 1H), 4.31 (d, *J =* 13.1 Hz, 2H), 3.87 (dd, *J* = 7.7, 3.7 Hz, 1H), 3.50 (t, *J* = 11.4 Hz, 2H), 2.96 - 2.76 (m, 3H), 2.67 - 2.53 (m, 2H), 2.06 (dd, *J* = 59.2, 12.3 Hz, 5H), 1.95 - 1.84 (m, 4H), 1.65 (d, *J* = 13.6 Hz, 2H), 1.52 *(d, J=* 13.5 Hz, 2H).
LC-MS(ESI): [M+H]⁺ = 774.62

### Example 55

### Synthesis of compound 37

### synthesis scheme

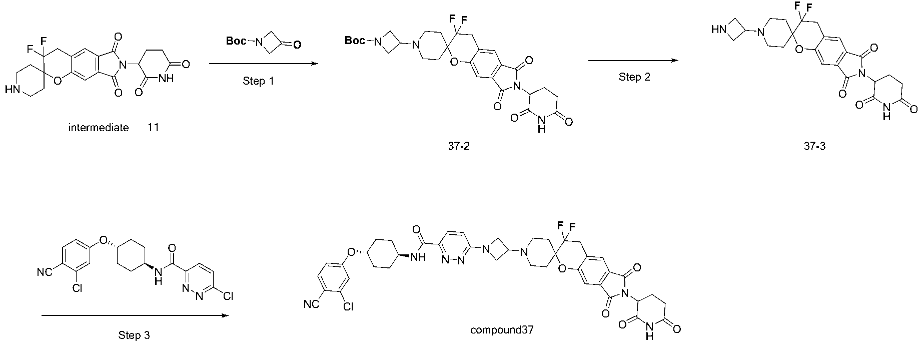

Step 1: The intermediate 11 (100 mg, 0.238 mmol) and tert-butyl 3-oxoazetidine-1-carboxylate (61.23 mg, 0.358 mmol) were added to the solvent DCE (1 mL), AcOH (4 drops) and reacted at room temperature for 1 h. Then NaBH(OAc)₃ (111 mg, 0.552 mmol) was added and the reaction was continued for 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 37-2 (60 mg).

Step 2: (Compound 37-3) was prepared with reference to step 2 of Example 19.
¹H NMR (600 MHz, DMSO-d₆) δ 11.12 (s, 1H), 7.82 (s, 1H), 7.50 (s, 1H), 5.12 (dd, *J =* 13.0, 5.4 Hz, 1H), 4.05 (s, 6H), 3.64 (t, *J* = 15.8 Hz, 4H), 2.99 - 2.84 (m, 3H), 2.64 - 2.53 (m, 2H), 2.05 (dtd, *J* = 10.5, 5.5, 2.8 Hz, 1H), 1.91 (d, *J* = 8.6 Hz, 4H).
LC-MS(ESI): [M+H]⁺ = 474.46

Step 3: (Compound 37) was prepared with reference to step 3 of Example 19.
¹H NMR (600 MHz, DMSO-d₆) δ 11.13 (s, 1H), 8.64 (d, *J* = 8.2 Hz, 1H), 7.95 (d, *J* = 9.2 Hz, 1H), 7.89 - 7.84 (m, 2H), 7.60 (s, 1H), 7.39 (d, *J* = 2.5 Hz, 1H), 7.14 (dd, *J* = 8.8, 2.5 Hz, 1H), 7.01 (d, *J* = 9.2 Hz, 1H), 5.14 (dd, *J* = 12.9, 5.6 Hz, 1H), 4.54 (p, *J* = 5.8 Hz, 1H), 4.47 (s, 2H), 4.41 (s, 2H), 3.92 - 3.83 (m, 2H), 3.71 (t, *J* = 15.9 Hz, 2H), 3.55 (s, 2H), 3.24 (s, 2H), 2.90 (ddd, *J* = 17.3, 14.0, 5.6 Hz, 1H), 2.65 - 2.53 (m, 2H), 2.20 - 2.02 (m, 7H), 1.94 - 1.87 (m, 2H), 1.66 (q, *J* = 12.0 Hz, 2H), 1.57 - 1.48 (m, 2H).
LC-MS(ESI): [M+H]⁺ = 829.65

### Example 56

### Synthesis of compound 38

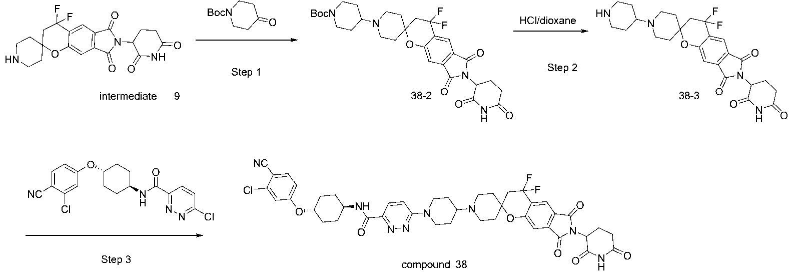

Step 1: The intermediate 9 (80 mg, 0.191 mmol) and tert-butyl 4-oxopiperidine-1-carboxylate (76.02 mg, 0.381 mmol) were added sequentially to a glass vial, and the solvents THF (2 mL) and Ti(O-iPr)₄(108.43 mg, 0.381 mmol) were added and the reaction solution was stirred at 60 °C for 1 h, followed by the addition of NaBH₃CN (35.96 mg, 0.572 mmol), and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 38-2 (80 mg).
LC-MS: [M+H]⁺ =603.51

Step 2: (Compound 38-3) was prepared with reference to step 2 of Example 19.
LC-MS: [M+H]⁺ =497.47

Step 3: (Compound 38) was prepared with reference to step 3 of Example 25.
¹H NMR (400 MHz, Chloroform-d) δ 8.16 (s, 1H), 8.00 (d, *J* = 9.5 Hz, 1H), 7.87 (d, *J =* 8.2 Hz, 1H), 7.56 (d, *J* = 8.7 Hz, 1H), 7.43 (s, 1H), 7.00 (dd, *J* = 6.0, 3.6 Hz, 1H), 6.85 (dd, *J* = 8.7, 2.4 Hz, 1H), 4.98 (dd, *J* = 12.2, 5.3 Hz, 1H), 4.58 (d, *J* = 13.3 Hz, 2H), 4.37 - 4.27 (m, 1H), 4.11 - 4.01 (m, 1H), 3.07 (t, *J* = 12.7 Hz, 2H), 2.98 - 2.88 (m, 1H), 2.88 - 2.73 (m, 4H), 2.73 - 2.61 (m, 2H), 2.51 (t, *J* = 14.1 Hz, 2H), 2.24 - 2.13 (m, 4H), 2.09 - 2.00 (m, 4H), 1.95 - 1.88 (m, 4H), 1.75 - 1.58 (m, 4H), 1.52 - 1.40 (m, 2H).
LC-MS: [M+H]⁺ =857.72

### Example 57

### Synthesis of compound 39

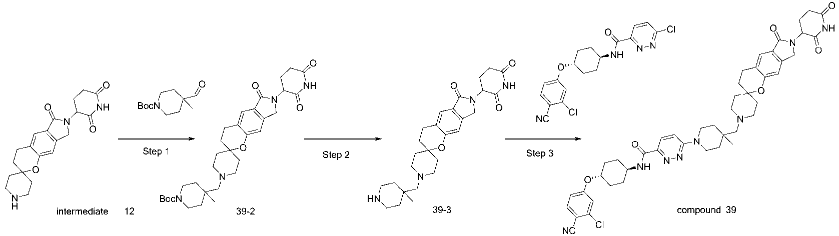

Step 1: The intermediate 12 (100 mg 0.271 mmol), tert-butyl 4-formyl-4-methylpiperidine-1-carboxylate (92.29 mg, 0.406 mmol), sodium cyanoborohydride (51.03 mg, 0.812 mmol), tetraisopropyl titanate (153.87 mg, 0.541 mmol) were added into a single-necked flask containing tetrahydrofuran ( 2 mL) and stirred at 60 °C for 2 h. After completion of the reaction, the reaction solution was cooled to room temperature and purified by TLC (dichloromethane:methanol=10:1) to obtain a white solid compound 39-2 (70 mg).
¹H NMR (600 MHz, DMSO-d₆) δ 10.96 (s, 1H), 7.45 (s, 1H), 6.96 (s, 1H), 5.05 (dd, *J* = 13.3, 5.2 Hz, 1H), 4.53 (t, *J* = 5.5 Hz, 1H), 4.32 (d, *J* = 16.6 Hz, 1H), 4.20 (d, *J* = 16.8 Hz, 1H), 3.53 (dd, *J* = 19.7, 15.3 Hz, 2H), 3.07 (s, 4H), 2.59 (d, *J =* 12.4 Hz, 3H), 2.36 (dd, *J =* 13.2, 4.5 Hz, 2H), 2.03 - 1.96 (m, 3H), 1.80 (t, *J =* 6.8 Hz, 1H), 1.65 (d, *J* = 14.8 Hz, 3H), 1.47 (q, *J* = 7.3 Hz, 2H), 1.39 (d, *J* = 2.2 Hz, 9H), 1.36 - 1.32 (m, 2H), 1.31 - 1.27 (m, 2H), 1.18 (t, *J =* 7.1 Hz, 2H), 1.13 (dt, *J* = 13.0, 4.3 Hz, 2H).
LC-MS(ESI): [M+H]⁺ =581.56

Step 2 (Compound 39-3) was prepared with reference to step 2 of Example 25.

Step 3 (compound 39) was prepared with reference to step 3 of Example 25.
¹H NMR (600 MHz, DMSO-d₆) δ 8.60 (d, *J* = 8.2 Hz, 1H), 8.36 (s, 2H), 7.86 (d, *J* = 8.8 Hz, 1H), 7.80 (d, *J* = 9.5 Hz, 1H), 7.46 (s, 1H), 7.40 (d, *J* = 2.4 Hz, 1H), 7.33 (d, *J* = 9.7 Hz, 1H), 7.14 (dd, *J* = 8.8, 2.5 Hz, 1H), 6.96 (s, 1H), 5.05 (dd, *J* = 13.3, 5.1 Hz, 1H), 4.54 (tt, *J* = 10.2, 4.3 Hz, 1H), 4.32 (d, *J* = 16.8 Hz, 1H), 4.19 (d, *J* = 16.8 Hz, 1H), 4.04 (dd, *J* = 11.9, 6.9 Hz, 2H), 3.86 (dq, *J* = 7.8, 3.9 Hz, 1H), 3.43 (s, 3H), 2.90 (ddd, *J* = 17.2, 13.6, 5.4 Hz, 2H), 2.83 (t, *J* = 6.9 Hz, 2H), 2.65 - 2.53 (m, 5H), 2.35 (td, *J* = 13.2, 4.6 Hz, 2H), 2.23 (s, 2H), 2.15 - 2.07 (m, 2H), 2.04 - 1.93 (m, 2H), 1.93 - 1.87 (m, 2H), 1.80 (t, *J* = 6.9 Hz, 2H), 1.73 - 1.59 (m, 5H), 1.58 - 1.44 (m, 4H), 1.38 - 1.31 (m, 2H).
LC-MS(ESI): [M+H]⁺ =835.66

### Example 58

### Synthesis of compound 40

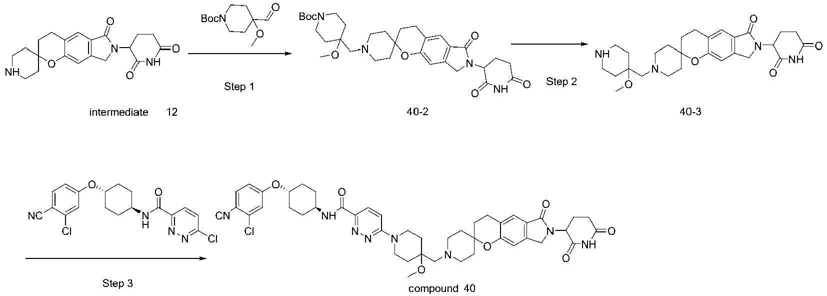

Step 1: The intermediate 12 (100 mg, 0.271 mmol) and tert-butyl 4-formyl-4-methoxypiperidine-1-carboxylate (98.79 mg, 0.406 mmol) were added sequentially to a glass vial, and the solvents THF (2 mL) and Ti(O-iPr)₄₍153.87 mg, 0.541 mmol) were added. The reaction solution was stirred at 60 °C for 1 h. Then NaBH₃CN (51.03 mg, 0.812 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 40-2 (60 mg).
LC-MS: [M+H]⁺ =597.58

Step 2: (Compound 40-3) was prepared with reference to step 2 of Example 19.
¹H NMR (400 MHz, DMSO-d₆) δ 10.95 (d, *J =* 2.9 Hz, 1H), 7.50 (s, 1H), 7.06 (s, 1H), 5.06 (dd, *J =* 13.2, 5.5 Hz, 1H), 4.28 (dd, *J* = 35.3, 8.1 Hz, 2H), 3.71 - 3.66 (m, 4H), 3.08 (d, *J* = 12.6 Hz, 4H), 2.97 - 2.91 (m, 3H), 2.83 (s, 1H), 2.59 (d, *J =* 15.2 Hz, 1H), 2.38 - 2.22 (m, 3H) , 2.10 (d, *J* = 14.3 Hz, 4H), 1.92 - 1.84 (m, 4H), 1.64 (td, *J* = 14.2, 13.7, 4.4 Hz, 6H).
LC-MS: [M+H]⁺ =497.47

Step 3: (Compound 40) was prepared with reference to step 3 of Example 25.
¹H NMR (600 MHz, DMSO-d₆) δ 10.96 (s, 1H), 8.61 (d, *J* = 8.2 Hz, 1H), 7.86 (d, *J* = 8.8 Hz, 1H), 7.81 (d, *J* = 9.5 Hz, 1H), 7.46 (s, 1H), 7.40 (d, *J* = 2.4 Hz, 1H), 7.35 (d, *J* = 9.7 Hz, 1H), 7.14 (dd, *J* = 8.8, 2.4 Hz, 1H), 6.96 (s, 1H), 5.05 (dd, *J* = 13.3, 5.2 Hz, 1H), 4.54 (tt, *J =* 10.1, 4.3 Hz, 1H), 4.32 (d, *J* = 16.8 Hz, 1H), 4.20 (d, *J* = 16.8 Hz, 1H), 4.17 - 4.08 (m, 2H), 3.87 (dtd, *J* = 11.4, 7.6, 4.1 Hz, 1H), 3.28 (s, 3H), 3.19 (s, 3H), 2.92 - 2.86 (m, 1H), 2.83 (t, *J =* 6.9 Hz, 2H), 2.67 (dt, *J* = 11.7, 4.4 Hz, 2H), 2.63 - 2.53 (m, 2H), 2.43 (s, 2H), 2.35 (td, *J* = 13.2, 4.7 Hz, 2H), 2.15 - 2.07 (m, 2H), 1.99 - 1.74 (m, 7H), 1.72 - 1.48 (m, 9H).
LC-MS: [M+H]⁺ =851.72

### Example 59

### Synthesis of compound 41

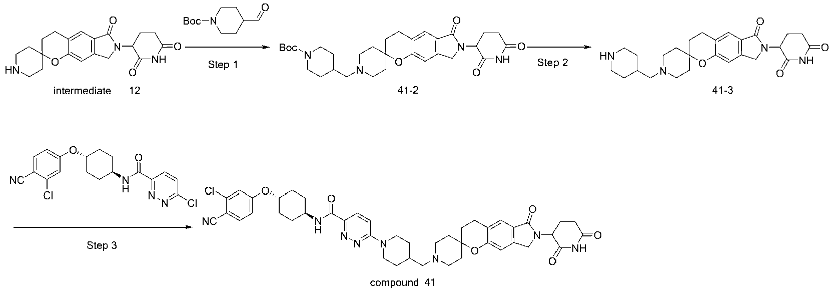

Step 1: The intermediate 12 (100 mg, 0.27 mmol) and tert-butyl 4-formylpiperidine-1-carboxylate (57.7 mg, 0.27 mmol) were added sequentially in a glass vial, and the solvents DCM (2 mL) and AcOH (2 drop) were added dropwise, and the reaction solution was stirred at room temperature for 1 h, and then NaBH(OAc)₃ (172 mg, 0.81 mmol) was added and the reaction solution was continued to be stirred at room temperature for 1 h. After the reaction was completed, the reaction solution was concentrated, and then purified by TLC (DCM:MeOH=10:1) to obtain a white solid compound 41-2 (70 mg).

Step 2: (Compound 41-3) was prepared with reference to step 2 of Example 19.

Step 3: (Compound 41) was prepared with reference to step 2 of Example 25.
¹H NMR (400 MHz, MeOD) δ 7.96 (d, *J* = 9.7 Hz, 1H), 7.69 (d, *J* = 8.8 Hz, 1H), 7.58 (s, 1H), 7.43 (d, *J* = 9.7 Hz, 1H), 7.20 (d*, J =* 2.3 Hz, 1H), 7.11 - 7.01 (m, 2H), 5.11 (dd, *J* = 13.3, 5.1 Hz, 1H), 4.47 (ddd, *J =* 42.3, 30.0, 15.1 Hz, 5H), 4.06 - 3.93 (m, 1H), 3.59 (d, *J =* 11.5 Hz, 2H), 3.47 - 3.34 (m, 2H), 3.18 (dd, *J* = 15.3, 8.3 Hz, 3H), 3.04 - 2.73 (m, 4H), 2.55 - 2.26 (m, 2H), 2.26 - 1.93 (m, 13H), 1.71 - 1.56 (m, 4H), 1.51 - 1.26 (m, 3H).
LC-MS(ESI): [M+H]⁺ = 821.68

### Example 60

### Synthesis of compound 42

### synthesis scheme

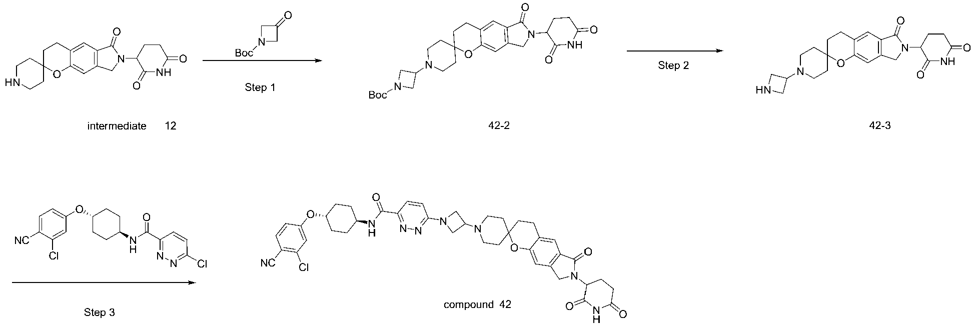

Step 1: The intermediate 12 (100 mg, 0.271 mmol) and tert-butyl 3-oxoazetidine-1-carboxylate (139.03 mg, 0.812 mmol) were added to the solvent DCE (1 mL), AcOH (4 drops), and the reaction was carried out at room temperature for 1 h. Then NaBH(OAc)₃(172.11 mg, 0.812mmol) was added and the reaction was continued at room temperature for 1 h. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 42-2 (109 mg).

Step 2: (Compounds 42-3) were prepared with reference to step 2 of Example 19.

Step 3: (Compound 42) was prepared with reference to step 3 of Example 19.
¹H NMR (400 MHz, DMSO-d₆) δ 10.95 (s, 1H), 8.60 (d, *J* = 8.1 Hz, 1H), 7.93 (d, *J* = 9.2 Hz, 1H), 7.85 (d, *J* = 8.8 Hz, 1H), 7.51 (s, 1H), 7.37 (d, *J =* 2.4 Hz, 1H), 7.12 (dd, *J* = 8.8, 2.4 Hz, 1H), 7.06 - 6.96 (m, 2H), 5.05 (dd, *J* = 13.3, 5.1 Hz, 1H), 4.57 - 4.29 (m, 6H), 4.21 (d, *J* = 17.0 Hz, 1H), 3.32-3.12 ( s,5H), 2.89 (m, 2H), 2.58 (d, *J* = 16.7 Hz, 2H), 2.36 (d, *J* = 13.2 Hz, 2H), 2.17 - 1.77 (m, 11H), 1.64 (q, *J* = 11.5 Hz, 2H), 1.57 - 1.43 (m 2H).
LC-MS(ESI): [M+H]⁺ = 779.63

### Example 61

### Synthesis of compound 43

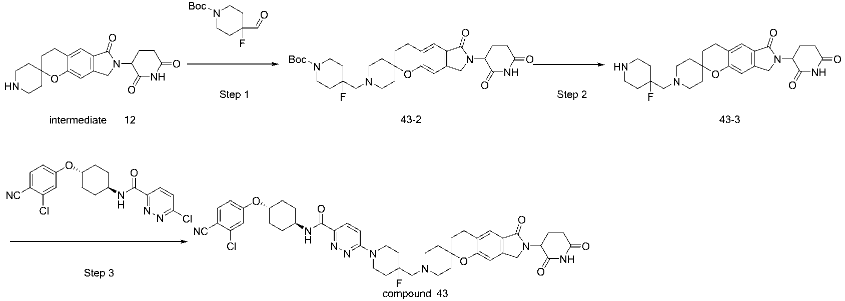

Step 1: The intermediate 12 (60 mg, 0.162 mmol) and tert-butyl 4-fluoro-4-formylpiperidine-1-carboxylate (75.1 mg, 0.324 mmol) and Ti(O-iPr)₄₍92.0 mg, 0.324 mmol) were added sequentially in a glass vial, and the solvent THF (1 mL) was added dropwise, and the reaction solution was stirred at 60 °C for 1.5 h. Then, NaBH₃CN (30.6 mg, 0.486 mmol) was added, and the reaction solution was continued to be stirred at 60 °C for 1 h. After the reaction was completed, the reaction solution was concentrated, and then purified by TLC (DCM:MeOH=10:1) to obtain a white solid compound 43-2 (56 mg).

Step 2: (Compound 43-3) was prepared with reference to step 2 of Example 19.

Step 3: (Compound 43) was prepared with reference to step 3 of Example 25.
¹H NMR (600 MHz, MeOD) δ 7.97 (d, *J* = 9.6 Hz, 1H), 7.71 (d, *J* = 8.8 Hz, 1H), 7.60 (s, 1H), 7.42 (d*, J =* 9.6 Hz, 1H), 7.22 (d*, J =* 2.2 Hz, 1H), 7.14 - 7.04 ( m, 2H), 5.14 (dd*, J =* 13.3, 5.1 Hz, 1H), 4.58 - 4.34 (m, 5H), 4.06 - 3.94 (m, 1H), 3.73 - 3.41 (m, 6H), 3.05 - 2.86 (m, 3H), 2.84 - 2.76 (m, 1H), 2.49 (ddd, *J =* 26.4, 13.2, 4.5 Hz, 1H), 2.29 - 1.86 (m, 14H), 1.67 (p, *J* = 11.9 Hz, 4H ), 1.42 - 1.28 (m, 3H).
LC-MS(ESI): [M+H]⁺ = 839.77

### Example 62

### Synthesis of compound 44

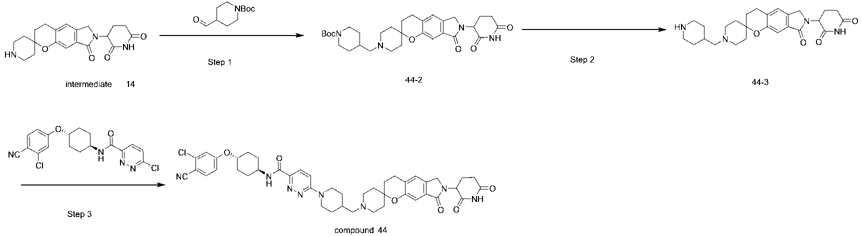

Step 1: The intermediate 14 (120 mg, 0.325 mmol), tert-butyl 4-formylpiperidine-1-carboxylate (104 mg, 0.487 mmol), sodium cyanoborohydride (51.04 mg, 0.812 mmol), and tetraisopropyl titanate (153.89 mg, 0.541 mmol) were added to a single-necked flask containing tetrahydrofuran (2 mL) and stirred at 60 °C for 2 h. After the reaction was completed, the reaction solution was cooled to room temperature and purified using TLC (dichloromethane:methanol = 10:1) to obtain a white solid compound 44-2 (68 mg).
LC-MS(ESI): [M+H]⁺ =567.52

Step 2: (Compound 44-3) was prepared with reference to step 2 of Example 25.

Step 3: (Compound 44) was prepared with reference to step 3 of Example 25.
¹H NMR (600 MHz, Methanol-d₄) δ 7.96 (d, *J* = 9.6 Hz, 1H), 7.71 (d, *J =* 8.7 Hz, 1H), 7.39 (d, *J* = 9.7 Hz, 1H), 7.37 (s, 1H), 7.34 (s, 1H), 7.22 (d, *J* = 2.4 Hz, 1H), 7.07 (dd, *J* = 8.7, 2.4 Hz, 1H), 5.15 (dd, *J* = 13.4, 5.2 Hz, 1H), 4.61 (d, *J =* 13.4 Hz, 2H), 4.53 (d, *J* = 4.1 Hz, 1H), 4.47 - 4.37 (m, 2H), 4.00 (d, *J* = 8.4 Hz, 1H), 3.60 (d, *J =* 12.5 Hz, 2H), 3.46 - 3.39 (m, 2H), 3.21 (d, *J* = 7.0 Hz, 1H), 3.17 (t, *J =* 12.2 Hz, 2H), 3.02 (t, *J* = 6.9 Hz, 2H), 2.92 (ddd, *J =* 18.5, 13.6, 5.4 Hz, 1H), 2.80 (ddd, *J* = 17.6, 4.7, 2.4 Hz, 1H), 2.51 (qd, *J* = 13.2, 4.6 Hz, 1H), 2.35 (td, *J* = 7.3, 3.6 Hz, 1H), 2.24 - 2.15 (m, 5H), 2.12 (s, 2H), 2.06 - 2.03 (m, 1H), 2.03 - 1.96 (m, 4H), 1.66 (q, *J* = 12.5, 11.0 Hz, 4H), 1.43 (tt, *J* = 12.1, 6.1 Hz, 2H), 1.35 (s, 2H).
LC-MS(ESI): [M+H]⁺ =821.66

### Example 63

### Synthesis of compound 45

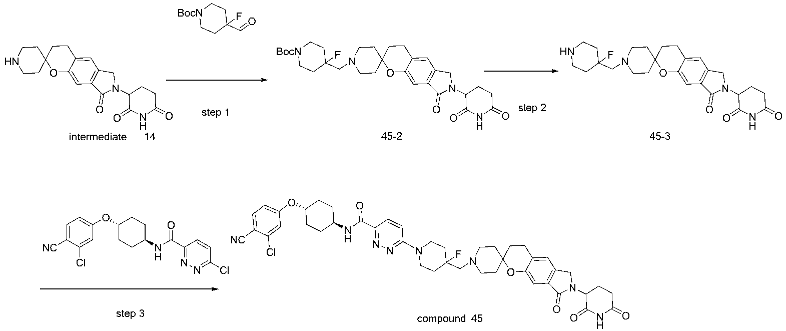

Step 1: The intermediate 14 (100 mg, 0.271 mmol) and tert-butyl 4-fluoro-4-formylpiperidine-1-carboxylate (93.90 mg, 0.406 mmol) were added sequentially to a glass vial, and the solvents THF (2 mL) and Ti(O-iPr)₄(153.87 mg, 0.541 mmol) were added, and the reaction solution was stirred at 60 °C for 1 h. Then NaBH₃CN (51.03 mg, 0.812 mmol) was added and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 45-2 (70 mg).
LC-MS: [M+H]⁺ =585.61

Step 2: (Compound 45-3) was prepared with reference to step 2 of Example 19.
LC-MS: [M+H]⁺ =485.48

Step 3: (Compound 45) was prepared with reference to step 3 of Example 25.
¹H NMR (600 MHz, DMSO-d₆) δ 10.99 (s, 1H), 8.63 (d, *J* = 8.3 Hz, 1H), 7.85 (dd, *J* = 14.8, 9.2 Hz, 2H), 7.46 - 7.37 (m, 2H), 7.31 (s, 1H), 7.14 (dd, *J* = 8.8, 2.4 Hz, 1H), 7.02 (s, 1H), 5.33 (t, *J* = 5.1 Hz, 1H), 5.07 (dd, *J* = 13.3, 5.2 Hz, 1H), 4.54 (tt, *J* = 10.0, 4.3 Hz, 1H), 4.37 - 4.15 (m, 4H), 3.87 (tdt, *J* = 11.7, 8.3, 4.1 Hz, 1H), 2.94 - 2.81 (m, 3H), 2.67 - 2.63 (m, 1H), 2.61 (s, 1H), 2.59 - 2.56 (m, 1H), 2.55 (s, 1H), 2.38 (qd, *J* = 13.2, 4.5 Hz, 2H), 2.10 (dt, *J* = 13.7, 6.7 Hz, 2H), 2.05 - 1.85 (m, 6H), 1.85 - 1.59 (m, 8H), 1.58 - 1.42 (m, 3H), 1.33 - 1.25 (m, 3H),.
LC-MS: [M+H]⁺ =839.73

### Example 64

### Synthesis of compound 46

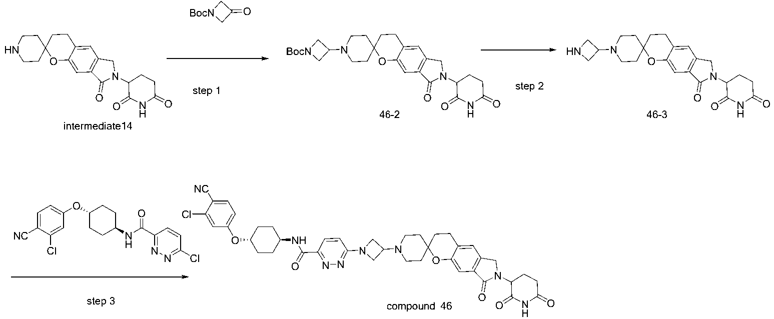

Step 1: The intermediate 14 (100 mg, 0.271 mmol) and tert-butyl 3-oxoazetidine-1-carboxylate (55.61 mg, 0.325 mmol) were added sequentially to a glass vial, and the solvents THF (2 mL) and Ti(O-iPr)₄ (153.87 mg, 0.541 mmol) were added, and the reaction solution was stirred at 25 °C for 1 h. Then NaBH₃CN (51.03 mg, 0.812 mmol) was added and the reaction solution was continued to be stirred at 25°C for 1 h. After the reaction was completed, the reaction solution was concentrated and then purified by preparative thin-layer chromatography (DCM:MeOH=10:1) to obtain awhite solid compound 46-2 (70 mg).
¹H NMR (600 MHz, Methanol-d₄) δ 7.30 (s, 1H), 7.20 (s, 1H), 5.13 (dd, *J* = 13.4, 5.1 Hz, 1H), 4.02 (s, 2H), 3.83 (s, 2H), 3.37 (s, 2H), 3.23 (ddt, *J* = 12.4, 7.0, 4.0 Hz, 1H), 2.95 (t, *J* = 7.0 Hz, 2H), 2.92 - 2.86 (m, 1H), 2.79 (ddt, J = 7.0 Hz, 2H). 4.0 Hz, 1H), 2.95 (t, *J* = 7.0 Hz, 2H), 2.92 - 2.86 (m, 1H), 2.79 (ddd, *J* = 17.6, 4.6, 2.4 Hz, 1H), 2.67 (d, *J* = 11.1 Hz, 2H), 2.49 (qd, *J* = 13.3, 4.6 Hz, 1H ), 2.40 (t, *J* = 11.8 Hz, 2H), 2.17 (dtd, *J* = 12.7, 5.2, 2.2 Hz, 1H), 1.89 (q, *J* = 6.6 Hz, 4H), 1.78 - 1.69 (m, 2H), 1.46 (s, 9H).
LC-MS: [M+H]⁺ =525.44

Step 2: (Compound 46-3) was prepared with reference to step 2 of Example 19.
LC-MS: [M+H]⁺ =439.36

Step 3: (Compound 46) was prepared with reference to step 3 of Example 25.
¹H NMR (600 MHz, DMSO-d₆) δ 11.04 - 10.92 (m, 1H), 8.57 (d, *J* = 8.2 Hz, 1H), 8.50 (s, 1H), 7.85 (dd, *J =* 11.2, 9.0 Hz, 2H), 7.39 (d, *J* = 2.4 Hz, 1H), 7.32 (s, 1H), 7.14 (dd, *J* = 8.8, 2.4 Hz, 1H), 7.05 (s, 1H), 6.87 (d, *J* = 9.2 Hz, 1H), 5.08 (dd, *J* = 13.3, 5.1 Hz, 1H), 4.54 (tt, *J =* 10.1, 4.3 Hz, 1H), 4.33 (d, *J* = 16.5 Hz, 1H), 4.22 (s, 1H), 4.21 - 4.16 (m, 2H), 3.96 (dd, *J* = 9.0, 5.1 Hz, 2H), 3.86 (dtt, *J =* 11.6, 8.1, 4.0 Hz, 1H), 2.95 - 2.89 (m, 1H), 2.87 (t, *J* = 6.7 Hz, 2H), 2.66 - 2.56 (m, 3H), 2.38 (dd, *J =* 13.2, 4.5 Hz, 1H), 2.36 - 2.28 (m, 2H), 2.15 - 2.06 (m, 2H), 1.99 (ddt, *J* = 13.0, 10.2, 6.2 Hz, 2H), 1.94 - 1.86 (m, 2H), 1.83 (t, *J* = 6.9 Hz, 2H), 1.80 - 1.72 (m, 2H), 1.71 - 1.58 (m, 4H), 1.56 - 1.47 (m, 2H). LC-MS: [M+H]⁺ =779.70

### Example 65

### Synthesis of compound 47

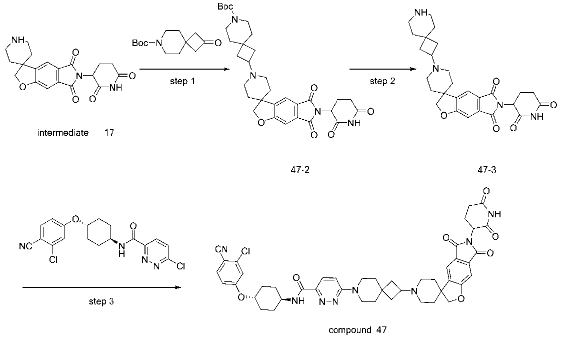

Step1: The intermediate 17 (20 mg, 0.054 mmol) and tert-butyl 2-oxo-7-azaspiro[3.5]nonane-7-carboxylate (25.9 mg, 0.108 mmol) and Ti(O-iPr)_{4 (}30 mg, 0.10 mmol) were added sequentially to a glass vial, and the solvent THF (1 mL) was added dropwise, and the reaction solution was stirred at 60 °C for 2 h. Then NaBH₃CN (11 mg, 0.162 mmol) was added and the reaction solution was continued to be stirred at 60 °C for 1 h. After the reaction was completed, the reaction solution was concentrated, and then purified by TLC (DCM:MeOH=10:1) to obtain a white solid compound 47-2 (20 mg).

Step 2: (Compound 47-3) was prepared with reference to step 2 of Example 39.

Step 3: (Compound 47) was prepared with reference to step 3 of Example 39.
¹H NMR (600 MHz, DMSO-d₆) δ 11.13 (s, 1H), 8.59 (t, *J =* 6.9 Hz, 1H), 7.90 - 7.80 (m, 2H), 7.66 (s, 1H), 7.44 - 7.34 (m, 3H), 7.14 ( dd, *J* = 8.8, 2.4 Hz, 1H), 5.16 - 5.08 (m, 1H), 4.74 (d, *J* = 12.9 Hz, 3H), 4.55 (dd, *J =* 9.3, 5.2 Hz, 2H), 4.02-3.99 (m, 1H), 3.93 - 3.61 (m , 6H), 3.47 (dd, *J =* 10.8 Hz, 1H), 3.28 (dd, *J* = 29.5, 23.9 Hz, 2H), 2.98 - 2.85 (m, 2H), 2.61 (dd, *J =* 9.5*,* 7.6 Hz, 1H), 2.32 - 2.22 (m, 2H) , 2.09 (ddd, *J* = 27.3, 21.5, 20.0 Hz, 7H), 1.90 (d, *J* = 12.2 Hz, 3H), 1.79 - 1.46 (m, 7H).
LC-MS(ESI): [M+H]⁺ = 847.65

### Example 66

### Synthesis of compound 48

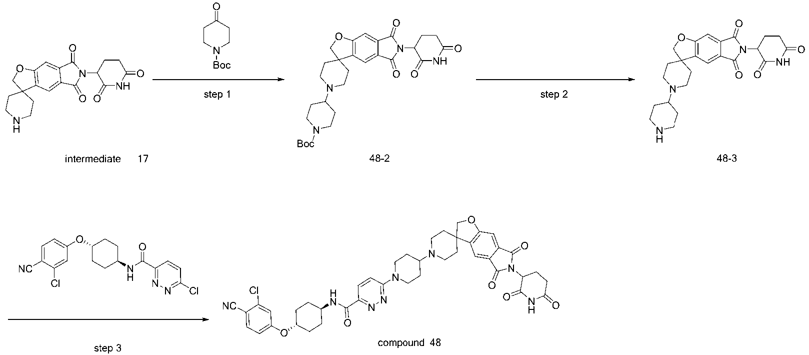

Step 1: The intermediate 17 (150 mg, 0.406 mmol) and tert-butyl 4-oxopiperidine-1-carboxylate (161.5 mg, 0.812 mmol) were added sequentially to a reaction flask, and the solvents DCE (2 mL) and AcOH (4 drops) were added dropwise, and the reaction solution was stirred at room temperature for 1 h, then NaBH(OAc)₃₍258 mg, 1.22 mmol) was added, and the reaction solution was continued to be stirred at room temperature for 1 h. After the reaction was completed, the reaction solution was concentrated, and then purified by preparative thin-layer chromatography (DCM:MeOH=10:1) to obtain a white solid compound 48-2 (120 mg).
¹H NMR (600 MHz, DMSO-d₆) δ 11.10 (s, 1H), 7.80 (s, 1H), 7.27 (s, 1H), 5.08 (dd, *J =* 12.9, 5.4 Hz, 1H), 4.59 (s, 2H), 3.96 (s, 2H), 3.47 - 3.38 (m , 1H), 2.93 - 2.80 (m, 3H), 2.62 - 2.56 (m, 1H), 2.27 - 2.15 (m, 2H), 2.05 - 1.98 (m, 1H), 1.93 (t, *J* = 11.2 Hz, 2H), 1.71 (dd, *J* = 25.2, 12.2 Hz, 4H), 1.38 (s, 9H), 1.32 - 1.18 (m, 4H), 1.04 (t, *J =* 7.0 Hz, 1H).
LC-MS: [M+H]⁺ =553.46

Step 2: (Compound 48-3) was prepared with reference to step 2 of Example 19.

Step 3: (Compound 48) was prepared with reference to step 3 of Example 25.
¹H NMR (600 MHz, DMSO-d₆) δ 11.11 (s, 1H), 8.63 (d, *J =* 8.2 Hz, 1H), 8.27 (s, 1H), 7.99 - 7.79 (m, 2H), 7.61 (s, 1H), 7.48 (dd, *J =* 19.7, 9.6 Hz, 1H ), 7.37 (dd, *J* = 14.3, 3.3 Hz, 1H), 7.22 - 7.10 (m, 1H), 5.37 - 5.27 (m, 1H), 5.17 - 5.05 (m, 1H), 4.82 - 4.64 (m, 2H) 4.64 (m, 2H), 4.57 - 4.47 (m, 1H), 3.93 - 3.80 (m, 1H), 3.67 - 3.47 (m, 2H), 3.19 - 2.99 (m, 3H), 2.94 - 2.82 (m, 1H), 2.64 - 2.51 (m, 6H), 2.42 - 2.36 (m, 1H), 2.33 - 2.15 (m, 2H), 2.14 - 1.94 (m, 3H), 1.93 - 1.84 (m, 1H), 1.75 - 1.59 (m, 2H), 1.58 - 1.41 (m, 3H), 1.35 - 1.16 (m, 4H).
LC-MS: [M+H]⁺ =807.60

### Example 67

### Synthesis of compound 49

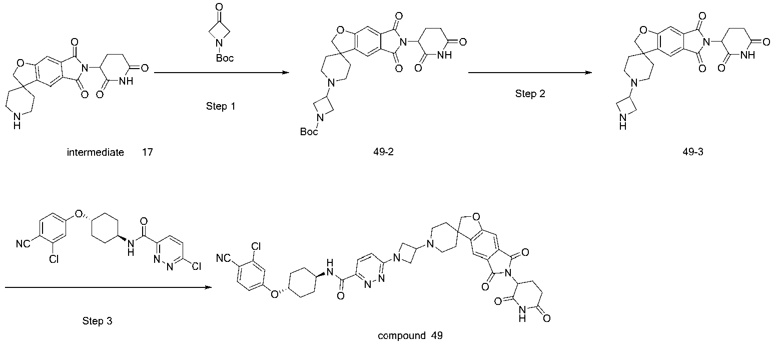

Step 1: The intermediate 17 (150 mg, 0.406 mmol) and tert-butyl 3-oxoazetidine-1-carboxylate (138.9 mg, 0.812 mmol) were added sequentially to a reaction flask, and the solvents DCE (2 mL) and AcOH (4 drops) were added dropwise, and the reaction solution was stirred at room temperature for 1 h, and then NaBH(OAc)₃(258 mg, 1.22 mmol) was added and the reaction solution was continued to be stirred at room temperature for 1 h. After the reaction was completed, the reaction solution was concentrated, and then purified by preparative thin-layer chromatography (DCM:MeOH=10:1) to obtain a white solid compound 49-2 (120 mg).
¹H NMR (600 MHz, DMSO-d₆) δ 11.13 (s, 1H), 7.84 (s, 1H), 7.30 (s, 1H), 5.11 (dd, *J* = 12.9, 5.4 Hz, 1H), 4.63 (s, 2H), 4.44 - 4.32 (m, 1H), 4.04 - 3.96 (m, 2H), 3.92 -3.81 (m, 2H), 3.76 - 3.67 (m, 2H), 3.62 - 3.57 (m, 2H), 3.14 - 3.07 (m, 1H), 2.94 - 2.86 (m, 1H), 2.80 (d, *J* = 9.8 Hz, 2H), 2.64 - 2.58 (m, 1H), 1.92 - 1.87 (m, 1H), 1.73 (d, *J* = 12.2 Hz, 2H), 1.40 (s, 9H).
LC-MS: [M+H]⁺ =425.39

Step 2: (Compound 49-3) was prepared with reference to step 2 of Example 19.
LC-MS: [M+H]⁺ =425.39

Step 3: (Compound 49) was prepared with reference to step 3 of Example 25.
¹H NMR (400 MHz, DMSO-d₆) δ 11.09 (s, 1H), 8.55 (d, *J* = 8.2 Hz, 1H), 7.91 - 7.76 (m, 3H), 7.39 (d, *J =* 2.3 Hz, 1H), 7.29 (s, 1H), 7.13 (dd, *J* = 8.8, 2.3 Hz, 1H), 6.87 (d, *J* = 9.3 Hz, 1H), 5.36 - 5.27 (m, 1H), 5.08 (dd, *J* = 12.8, 5.4 Hz, 1H), 4.64 (s, 2H), 4.58 - 4.48 (m, 1H), 4.21 (t, *J* = 7.8 Hz, 2H), 4.05 - 3.95 (m, 2H), 3.92 - 3.79 (m, 1H), 2.94 - 2.84 (m, 2H), 2.69 - 2.55 (m, 2H), 2.17 - 1.84 (m, 9H), 1.81 - 1.41 (m, 7H).
LC-MS: [M+H]⁺ =779.57

### Example 68

### Synthesis of compound 50

### synthesis scheme

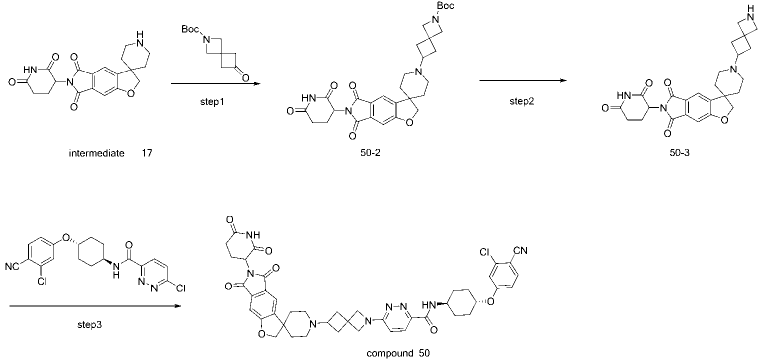

Step 1: The intermediate 17 (100 mg, 0.271 mmol) and tert-butyl 4-formylpiperidine-1-carboxylate (85.8 mg, 0.406 mmol) were added to the solvent DCE (1 mL), AcOH (4 drops), and the reaction was carried out at room temperature for 1 h. Then NaBH(OAc)₃ (111mg, 0.552 mmol) was added, and the reaction was continued for 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 50-2 (70 mg).
¹H NMR (600 MHz, DMSO-d₆) δ 11.11 (s, 1H), 7.80 (s, 1H), 7.28 (s, 1H), 5.09 (dd*, J =* 12.9, 5.4 Hz, 1H), 4.59 (s, 1H), 3.86 (s, 4H), 2.89 (ddd, *J* = 17.1, 13.9, 5.5 Hz, 1H), 2.75 (dt, *J* = 12.2, 3.4 Hz, 3H), 2.67 - 2.54 (m, 2H), 2.26 (m, 2H). 5.5 Hz, 1H), 2.75 (dt, *J* = 12.2, 3.4 Hz, 3H), 2.67 - 2.54 (m, 2H), 2.26 (ddd, *J* = 10.0, 7.2, 3.0 Hz, 2H), 2.03 (dtd, *J* = 12.9, 5.4, 2.3 Hz, 1H), 1.98 - 1.84 (m, 5H), 1.84 - 1.72 (m, 2H), 1.68 (d, *J* = 12.5 Hz, 2H), 1.37 (s, 9H).
LC-MS(ESI): [M-tBu+H]⁺ = 565.42

Step 2: (Compounds 50-3) were prepared with reference to step 2 of Example 19.
LC-MS(ESI): [M+H]⁺ = 465.42

Step 3: (Compound 50) was prepared with reference to step 3 of Example 19.
¹H NMR (400 MHz, DMSO-d₆) δ 11.11 (s, 1H), 8.53 (d, *J* = 8.1 Hz, 1H), 7.86 (dd, *J* = 8.9, 1.6 Hz, 2H), 7.67 (s, 1H), 7.41 - 7.34 (m, 2H), 7.13 (dd, *J* = 8.8, 2.5 Hz, 1H), 6.89 (dd, *J* = 9.3, 5.8 Hz, 1H), 5.11 (dd, *J =* 12.6, 5.6 Hz, 1H), 4.72 (d, *J* = 12.0 Hz, 2H), 4.60 - 4.48 (m, 2H), 4.26 (d, *J* = 19.0 Hz, 2H), 4.15 (s, 2H), 3.85-3.65 m, 4H), 3.45-3.44 (m, 2H) 2.65 (d, *J* = 12.5 Hz, 3H), 2.56 (d, *J =* 11.5 Hz, 2H), 2.23 (d, *J* = 13.5 Hz, 2H), 2.17 - 1.99 (m, 5H), 1.89 (s, 3H), 1.64 (q, *J* = 12.1 Hz, 2H), 1.52 (q, *J* = 11.5 Hz, 2H).
LC-MS(ESI): [M+H]⁺ = 819.77

### Example 69

### Synthesis of compound 51

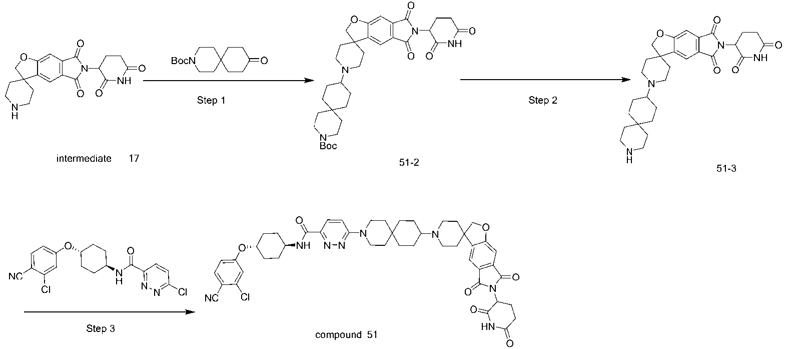

Step 1: The intermediate 17 (100 mg 0.27 mmol), tert-butyl 9-oxo-3-azaspiro[5.5]undecane-3-carboxylate (144.77 mg, 0.541 mmol), sodium cyanoborohydride (51.04 mg, 0.812 mmol), tetraisopropyl titanate (153.89 mg, 0.541 mmol) were added into a single-necked flask containing tetrahydrofuran (2 mL), and stirred at 60 °C for 2 h. After completion of the reaction, the reaction solution was cooled to room temperature and purified by TLC (dichloromethane:methanol=10:1) to obtain a white solid compound 51-2 (106 mg).
¹H NMR (600 MHz, Chloroform-d) δ 8.99 (s, 1H), 7.92 (s, 1H), 7.57 (s, 1H), 5.43 (t, *J* = 7.0 Hz, 1H), 4.18 (s, 2H), 3.70 (t, *J =* 7.1 Hz, 4H), 2.95 - 2.89 (m, 1H), 2.89 (d, *J =* 7.1 Hz, 1H), 2.81 (dt, *J* = 12.5, 7.1 Hz, 2H), 2.67 - 2.59 (m, 2H), 2.58 (q, *J* = 7.0 Hz, 1H), 2.22 - 1.98 (m, 6H), 1.83 (t, *J* = 7.0 Hz, 6H) 1.83 (t, *J* = 7.1 Hz, 4H), 1.72 (dq, *J=* 12.3, 7.0 Hz, 2H), 1.65 - 1.55 (m, 2H), 1.52 - 1.47 (m, 4H), 1.46 (s, 9H).
LC-MS(ESI): [M+H]⁺ =621.59

Step 2: (Compound 51-3) was prepared with reference to step 2 of Example 25.

Step 3: (Compound 51) was prepared with reference to step 3 of Example 25.
¹H NMR (600 MHz, Methanol-d₄) δ 7.99 (d, *J* = 9.6 Hz, 1H), 7.74 (s, 1H), 7.71 (dd, *J* = 8.7, 0.8 Hz, 1H), 7.46 (d, *J* = 9.7 Hz, 1H), 7.30 (s, 1H), 7.22 (dd, *J =* 2.4, 0.8 Hz, 1H), 7.06 (ddd, *J =* 8.8, 2.4, 0.8 Hz, 1H), 5.13 (dd, *J =* 12.9, 5.4 Hz, 1H), 4.79 (s, 2H), 4.52 (d, *J* = 4.7 Hz, 1H), 4.04 - 3.96 (m, 1H), 3.81 (dt, *J* = 15.2, 5.9 Hz, 4H), 3.74 - 3.69 (m, 2H), 3.23 (t, *J* = 12.7 Hz, 2H), 2.89 (ddd, *J* = 18.6, 14.0, 5.3 Hz, 1H), 2.80 - 2.70 (m, 2H), 2.30 (t, *J* = 13.1 Hz, 2H), 2.25 - 2.17 (m, 4H), 2.17 - 2.01 (m, 8H), 1.89 - 1.78 (m, 4H), 1.66 (q, *J* = 13.1, 11.3 Hz, 4H), 1.60 (d, *J* = 6.0 Hz, 2H), 1.42 (t, *J* = 12.3 Hz, 2H).
LC-MS(ESI): [M+H]⁺ =875.69

### Example 70

### Synthesis of compound 52

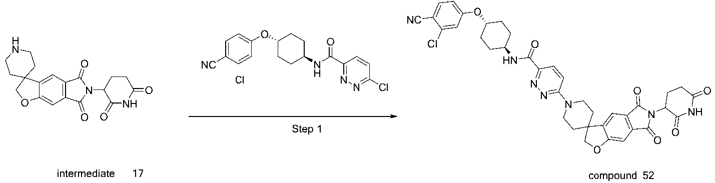

Step 1: The intermediate 17 (50 mg, 0.135 mmol) and the intermediate 18 (105.92 mg, 0.271 mmol), DIEA (34.99 mg, 0.271 mmol) were added to the solvent DMSO (1 mL), and the reaction was carried out at 80 °C for 2 h. After the reaction was completed, the reaction solution was cooled down to room temperature, concentrated and purified by Prep-HPLC (15-50% acetonitrile) to obtain a white solid compound 52 (1.87 mg).
¹H NMR (600 MHz, DMSO-d₆) δ11.11 (s, 1H), 8.68 (d, *J =* 8.2 Hz, 1H), 7.91 (s, 1H), 7.88 (d, *J* = 1.3 Hz, 1H), 7.86 (s, 1H), 7.47 (d, *J* = 9.6 Hz, 1H), 7.41 (d, *J* = 2.4 Hz, 1H), 7.32 (s, 1H), 7.19 - 7.12 (m, 1H), 5.09 (dd, *J* = 12.9, 5.4 Hz, 1H), 4.83 (s, 2H), 4.55 (tt, *J* = 8.6, 5.6, 4.8 Hz, 2H), 3.88 (ddd, *J* = 11.6, 9.7, 6.1 Hz, 2H), 3.18 (t, *J* = 13.1 Hz, 2H), 2.88 (ddd, *J* = 17.3, 13.9, 5.5 Hz, 1H), 2.66 - 2.52 (m, 2H), 2.15 - 1.96 (m, 5H), 1.94 - 1.88 (m, 2H), 1.82 (d, *J* = 12.9 Hz, 2H), 1.66 (q*, J =* 12.0, 11.3 Hz, 2H), 1.53 (td, *J* = 13.9, 7.1 Hz, 2H).
LC-MS(ESI): [M+H]⁺ = 724.44

### Example 71

### Synthesis of compound 53

### synthetic route

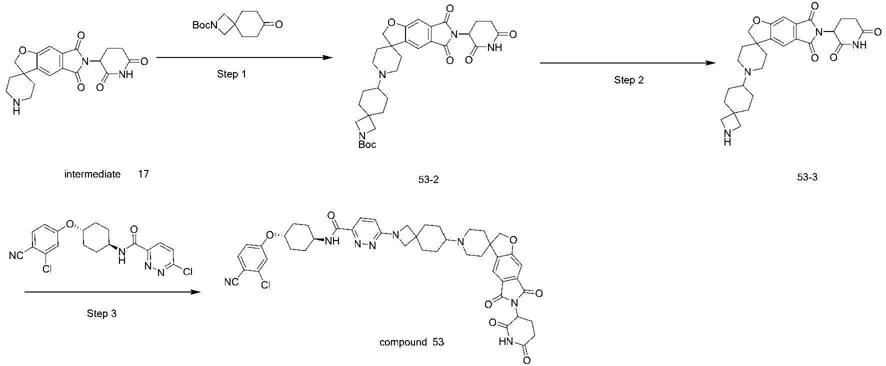

Step 1: The intermediate 17 (100 mg, 0.27 mmol), tert-butyl 7-oxo-2-azaspiro[3.5]nonane-2-carboxylate (129.58 mg, 0.541 mmol), sodium cyanoborohydride (51.04 mg, 0.812 mmol), tetraisopropyl titanate (153.89 mg, 0.541 mmol) were added into a single-necked flask containing tetrahydrofuran ( 2 mL) and stirred at 60 °C for 2 h. After the reaction was completed, the reaction solution was purified by TLC (dichloromethane:methanol=10:1) to obtain a white solid compound 53-2 (75 mg).
¹H NMR (600 MHz, Methanol-d₄) δ 7.72 (s, 1H), 7.21 (d, *J =* 0.5 Hz, 1H), 5.10 (dd, *J* = 12.7, 5.5 Hz, 1H), 4.64 (s, 2H), 3.67 (d, *J* = 6.3 Hz, 3H), 3.48 (s, 2H), 3.25 (s, 2H), 3.03 (d, *J* = 12.3 Hz, 2H), 2.91 - 2.84 (m, 1H), 2.79 - 2.71 (m, 2H), 2.43 (d, *J* = 12.9 Hz, 2H), 2.17 - 2.10 (m, 1H), 2.08 - 2.03 (m, 2H), 2.03 - 1.99 (m, 2H), 1.86 (d, *J* = 13.2 Hz, 2H), 1.58 - 1.52 (m, 2H), 1.46 (s, 9H), 1.37 (d, *J* = 12.3 Hz, 2H).
LC-MS(ESI): [M+H]⁺ = 593.68

Step 2: (Compound 53-3) was prepared with reference to step 2 of Example 25.

Step 3: (Compound 53) was prepared with reference to step 3 of Example 25.
¹H NMR (600 MHz, Methanol-d₄). δ 7.94 (d, *J* = 9.3 Hz, 1H), 7.75 (s, 1H), 7.71 (d, *J* = 8.8 Hz, 1H), 7.26 (s, 1H), 7.23 (d, *J =* 2.4 Hz, 1H), 7.07 (dd, *J =* 8.8, 2.4 Hz, 1H), 6.90 (d, *J* = 9.4 Hz, 1H), 5.12 (dd, *J* = 12.8, 5.4 Hz, 1H), 4.71 (s, 2H), 4.56 - 4.50 (m, 1H), 4.00 (s, 3H), 3.91 (s, 2H), 3.36-3.33 (m, 1H), 2.89 (ddd, *J* = 17.4, 13.9, 5.3 Hz, 2H), 2.79 (dd, *J* = 4.5, 2.6 Hz, 2H), 2.77 - 2.70 (m, 2H), 2.21 (d, *J* = 14.4 Hz, 4H), 2.17 - 2.06 (m, 6H), 2.02 (d, *J* = 13.5 Hz, 2H), 1.73 (t, *J* = 13.1 Hz, 2H), 1.69 - 1.61 (m, 4H), 1.57 (t, *J* = 12.7 Hz, 2H), 1.33 (d, *J* = 20.4 Hz, 2H).
LC-MS(ESI): [M+H]⁺ =847.66

### Example 72

### Synthesis of compound 54

### synthesis scheme

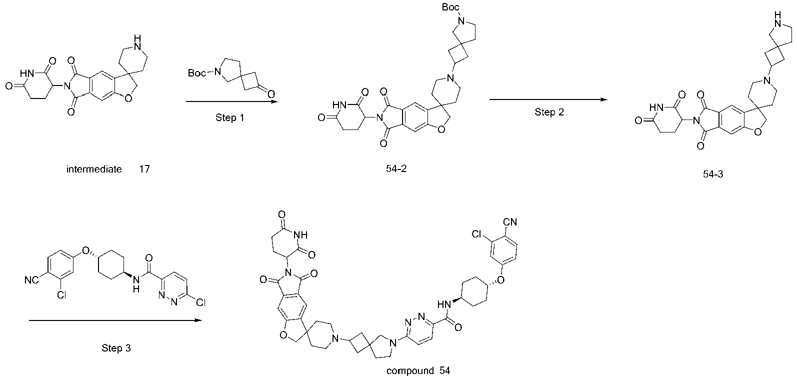

Step 1: The intermediate 17 (100 mg, 0.271 mmol) and tert-butyl 4-formylpiperidine-1-carboxylate (91.5 mg, 0.406 mmol) were added to the solvent DCE (1 mL), AcOH (4 drops), and the reaction was carried out at room temperature for 1 h. Then NaBH(OAc)₃ (115 mg, 0.542 mmol) was added, and the reaction was continued for 1 h. The reaction solution was concentrated and washed with 5 mL of saturated ammonium chloride aqueous solution, extracted with ethyl acetate, concentrated and purified by preparative thin-layer chromatography (DCM:MeOH=10:1) to obtain a white solid compound 54-2 (80 mg).
¹H NMR (600 MHz, DMSO-d₆) δ 11.11 (s, 1H), 7.82 (dd, *J* = 7.7, 5.4 Hz, 1H), 7.28 (s, 1H), 5.09 (dd, *J* = 12.9, 5.4 Hz, 1H), 4.60 (s, 1H), 3.24 (t, *J =* 6.3 Hz, 2H), 3.19 (q, *J* = 7.5 Hz, 1H), 3.12 (d, *J =* 9.9 Hz, 1H), 2.89 (ddd, *J* = 17.1, 13.9, 5.4 Hz, 1H), 2.81 - 2.66 (m, 2H), 2.63 - 2.52 (m, 2H), 2.03 (dtd, *J* = 13.1, 5.8, 5.3, 2.2 Hz, 2H), 1.91 (s, 5H), 1.88 - 1.72 (m, 5H), 1.69 (d, J = 12.5 Hz, 2H), 1.40 (d, *J* = 6.5 Hz, 9H), 0.98 (t, *J* = 7.2 Hz, 1H).
LC-MS(ESI): [M-tBu+H]⁺ = 579.50

Step 2: (Compound 54-3) was prepared with reference to step 2 of Example 19.
LC-MS(ESI): [M+H]⁺ = 479.41

Step 3: (Compound 54) was prepared with reference to step 3 of Example 19.
¹H NMR (600 MHz, DMSO-d₆) δ 11.13 (s, 1H), 9.80 (d, *J =* 73.2 Hz, 1H), 8.56 (t, *J =* 7.3 Hz, 1H), 8.26 (s, 1H), 7.95 - 7.81 (m, 1H), 7.67 (s, 1H), 7.39 (dd, *J* = 6.9, 2.0 Hz, 1H), 7.14 (dd, *J* = 8.8, 2.4 Hz, 1H), 6.91 (dd, *J* = 9.2, 1.9 Hz, 1H), 5.12 (dt, *J* = 13.0, 4.8 Hz, 1H), 4.73 (d, *J* = 19.0 Hz, 2H), 4.54 (td, *J* = 10.4, 9.9, 5.3 Hz, 2H), 4.39 - 3.99 (m, 5H), 3.77 (ddd, *J* = 97.6, 13.4, 7.5 Hz, 5H), 3.56 - 3.09 (m, 3H), 3.09 - 2.81 (m, 2H), 2.81 - 2.55 (m, 4H), 2.49 - 1.97 (m, 6H), 1.91 (d, *J* = 12.5 Hz, 2H), 1.69 - 1.45 (m, 4H).
LC-MS(ESI): [M+H]⁺ = 833.58

### Example 73

### Synthesis of compound 55

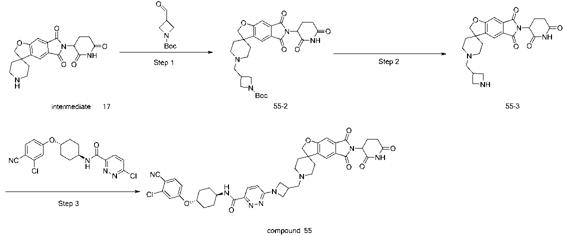

Step 1: The intermediate 17 (150 mg, 0.406 mmol) and tert-butyl 3-formylazetidine-1-carboxylate (150.4 mg, 0.812 mmol) were added sequentially in a glass vial, and the solvents DCE (2 mL), AcOH (4 drops) were added dropwise, and the reaction solution was stirred at room temperature for 1 h, then NaBH(OAc)₃ (258 mg, 1.22 mmol) was added and the reaction solution was continued to be stirred at room temperature for 1 h. After the reaction was completed, the reaction solution was concentrated, and then purified by preparative thin-layer chromatography (DCM:MeOH=10:1) to obtain a white solid compound 55-2 (150 mg).

Step 2: (Compound 55-3) was prepared with reference to step 2 of Example 19.
LC-MS: [M+H]⁺ =439.37

Step 3: (Compound 55) was prepared with reference to step 3 of Example 25.
¹H NMR (600 MHz, DMSO-d₆) δ 11.12 (s, 1H), 8.59 (t, *J* = 8.1 Hz, 1H), 8.29 (s, 1H), 7.98 - 7.84 (m, 2H), 7.66 (s, 1H), 7.42 - 7.33 (m, 1H), 7.14 (dd, *J* = 8.8, 2.4 Hz, 1H), 6.94 (dd, *J* = 24.4, 9.3 Hz, 1H), 5.12 (dd, *J* = 12.9, 5.4 Hz, 1H), 4.80 - 4.68 (m, 1H), 4.58 - 4.49 (m, 1H), 4.35 (t, *J =* 8.3 Hz, 1H), 4.04 - 3.94 (m, 1H), 3.91 - 3.80 (m, 1H), 3.73 - 3.54 (m, 4H), 3.18 - 3.06(m, 2H), 2.94 - 2.84 (m, 1H), 2.73 (s, 1H), 2.65 - 2.58 (m, 1H), 2.57 - 2.51 (m, 6H), 2.40 - 2.37 (m, 1H), 2.33 - 2.23 (m, 1H), 2.18 (t, *J =* 13.3 Hz, 1H), 2.11 (d, *J =* 10.3 Hz, 1H), 2.07 - 2.01 (m, 1H), 1.90 (d, *J =* 10.9 Hz, 2H), 1.73 - 1.57 (m, 2H), 1.57 - 1.47 (m, 2H).
LC-MS: [M+H]⁺ = 793.58

### Example 74

### Synthesis of compound 56

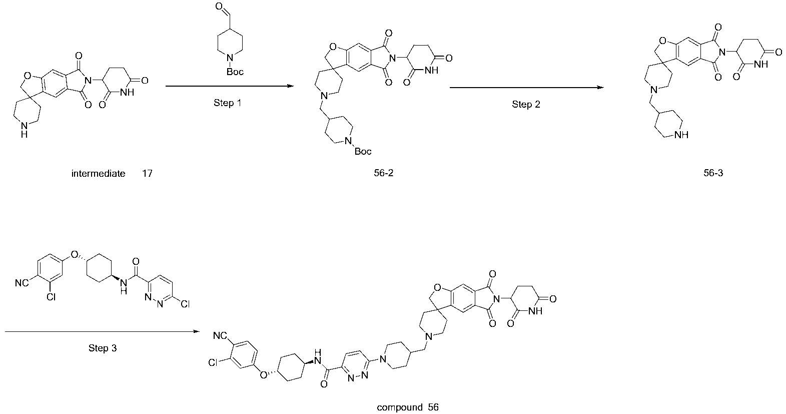

Step 1: The intermediate 17 (150 mg, 0.406 mmol) and tert-butyl 4-formylpiperidine-1-carboxylate (172.9 mg, 0.812 mmol) were added sequentially in a reaction flask, and the solvents DCE (2 mL) and AcOH (4 drops) were added dropwise, and the reaction solution was stirred at room temperature for 1 h. Then NaBH(OAc)₃(258 mg, 1.22 mmol) was added and the reaction solution was continued to be stirred at room temperature for 1 h. After the reaction was completed, the reaction solution was concentrated, and then purified by preparative thin-layer chromatography (DCM:MeOH=10:1) to obtain a white solid compound 56-2 (130 mg).
¹H NMR (600 MHz, DMSO) δ 11.10 (s, 1H), 7.81 (s, 1H), 7.27 (s, 1H), 5.13 - 5.04 (m, 1H), 4.6 (s, 1H), 3.92 (s, 2H), 2.94 - 2.76 (m, 3H), 2.52 - 2.62 (m, 2H), 2.21-2.12 (m, 2H), 1.92 - 2.05 (m, 4H), 1.90 (s, 1H), 1.68 (t, J=10.8 Hz, 3H), 1.38 (s, 9H), 1.3 - 1.22 (m, 5H), 1.15 - 1.06 (m, 2H).
LC-MS: [M+H]⁺ =567.46

Step 2: (Compound 56-3) was prepared with reference to step 2 of Example 19.
LC-MS: [M+H]⁺ =467.38

Step 3: (Compound 56) was prepared with reference to step 3 of Example 25.
¹H NMR (600 MHz, DMSO-d₆) δ 11.11 (s, 1H), 8.58 (d, *J =* 8.1 Hz, 1H), 8.31 (s, 1H), 7.85 (t, J = 9.1 Hz, 2H), 7.62 (s, 1H), 7.46 - 7.30 (m, 2H), 7.12 (d, *J* = 21.9, 10.9 Hz, 1H), 5.39 - 5.26 (m, 1H), 5.19 - 5.04 (m, 1H), 4.74 (d, *J* = 19.0 Hz, 2H), 4.61 - 4.46 (m, 2H), 3.92 - 3.81 (m, 1H), 3.61 (d, *J* = 11.1 Hz, 1H), 3.16 - 2.97 (m, 3H), 2.96 - 2.82 (m, 1H), 2.63 - 2.53 (m, 7H), 2.38 (s, 1H), 2.31 - 2.17 (m, 2H), 2.15 - 1.95 (m, 4H), 1.88 (dd, *J =* 23.9, 10.2 Hz, 3H), 1.64 (dd, *J =* 23.9, 10.8 Hz, 2H), 1.56 - 1.42 (m, 2H), 1.33 - 1.24 (m, 3H).
LC-MS: [M+H]⁺ = 821.71

### Example 75

### Synthesis of compound 57

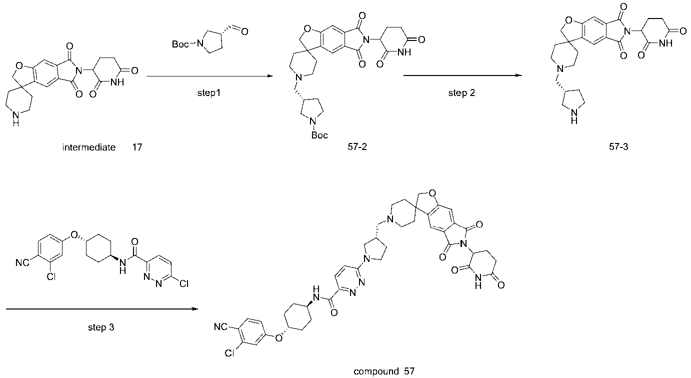

Step 1: The intermediate 17 (150 mg, 0.406 mmol) and tert-butyl tert-butyl (R)-3-pyrrolidine-1-carboxylate (161.58 mg, 0.812 mmol) were added sequentially into a glass vial, and then solvents DCE (2 mL) and AcOH (4 drops) were added dropwise, and the reaction solution was stirred at room temperature for 1 h. Then NaBH(OAc)₃(258 mg, 1.22 mmol) was added and the reaction solution was continued to be stirred at room temperature for 1 h. After the reaction was completed, the reaction solution was concentrated, and then purified by preparative thin-layer chromatography (DCM:MeOH=10:1) to obtain a white solid compound 57-2 (130 mg).
LC-MS: [M+H]⁺ =453.45

Step 2: (Compound 57-3) was prepared with reference to step 2 of Example 19.
LC-MS: [M+H]⁺ =453.41

Step 3: (Compound 57) was prepared with reference to step 3 of Example 25.
¹H NMR (600 MHz, DMSO-d₆) δ 11.11 (s, 1H), 8.52 (dd, *J =* 14.1, 8.1 Hz, 1H), 7.87 (dd, *J* = 14.0, 9.0 Hz, 2H), 7.65 (s, 1H), 7.43 - 7.31 (m, 2H), 7.13 (dd, *J =* 8.8, 2.4 Hz, 1H), 6.99 (d, *J =* 9.4 Hz, 1H), 5.15 - 5.08 (m, 1H), 4.80 - 4.69 (m, 2H), 4.58 - 4.49 (m, 1H), 3.98 - 3.78 (m, 2H), 3.78 - 3.60 (m, 4H), 3.37 - 3.26 (m, 3H), 3.22 - 3.05 (m, 2H), 2.93 - 2.80 (m, 2H), 2.63 - 2.50 (m, 3H), 2.34 - 2.19 (m, 3H), 2.16 - 1.80 (m, 7H), 1.68 - 1.59 (m, 2H), 1.58 - 1.43 (m, 2H).
LC-MS: [M+H]⁺ =807.61

### Example 76

### Synthesis of compound 58

### synthesis scheme

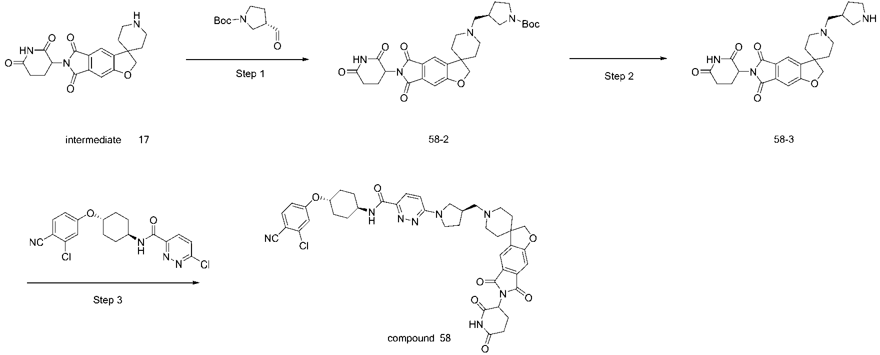

Step 1: The intermediate 17 (100 mg, 0.271 mmol) and tert-butyl (S)-3-formylpyrrolidine-1-carboxylate (80.9 mg, 0.406 mmol) were added to the solvent DCE (1 mL), AcOH (4 drops), and the reaction was carried out at room temperature for 1 h. Then NaBH(OAc)₃ (115 mg, 0.542 mmol) was added, and the reaction was continued for 1 h at room temperature. The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 58-2 (80 mg).
¹H NMR (600 MHz, DMSO-d₆) δ 7.85 - 7.70 (m, 1H), 7.25 (dd, *J =* 37.3, 3.4 Hz, 1H), 5.09 (dd, *J =* 12.9, 5.4 Hz, 1H), 4.59 (s, 2H), 3.98 (d, *J =* 30.5 Hz, 1H), 3.85 (d, *J =* 19.9 Hz, 1H), 3.68 (s, 1H), 3.62 - 3.52 (m, 1H), 3.47 (s, 1H), 3.38 (d, *J* = 7.3 Hz, 1H), 3.21 (q, *J =* 7.2 Hz, 1H), 3.16 - 3.07 (m, 1H), 3.06 - 2.96 (m, 1H), 2.89 (ddd, *J =* 17.0, 14.0, 5.5 Hz, 1H), 2.75 (d, *J =* 11.0 Hz, 2H), 2.65 - 2.56 (m, 1H), 2.33 - 2.20 (m, 2H), 2.13 (d, *J =* 8.7 Hz, 1H), 2.07 - 1.99 (m, 1H), 1.91 (d, *J =* 11.7 Hz, 3H), 1.87 (s, 2H), 1.77 (d, *J =* 7.7 Hz, 2H), 1.68 (d, *J =* 11.5 Hz, 2H), 1.29 - 1.20 (m, 2H), 1.16 (t, *J =* 7.2 Hz, 1H), 0.99 (t, *J =* 7.2 Hz, 1H), 0.72 (d, *J =* 5.7 Hz, 1H).
LC-MS(ESI): [M+H]⁺ = 553.45

Step 2: (Compound 58-3) was prepared with reference to step 2 of Example 19.

Step 3: (Compound 58) was prepared with reference to step 3 of Example 19.
¹H NMR (600 MHz, DMSO-d₆) δ 11.13 (d, *J =* 2.9 Hz, 1H), 9.55 (d, *J =* 130.4 Hz, 1H), 8.58 - 8.31 (m, 1H), 7.95 - 7.83 (m, 2H), 7.65 (s, 1H), 7.42 - 7.34 (m, 1H), 7.14 (dd, *J =* 8.8, 2.4 Hz, 1H), 7.00 (d, *J =* 9.5 Hz, 1H), 5.16 - 5.09 (m, 1H), 4.76 (d, *J =* 19.3 Hz, 2H), 4.55 (dt, *J =* 10.7, 5.8 Hz, 2H), 3.87 (qd, *J =* 11.5, 5.7 Hz, 2H), 3.67 (t, *J* = 13.7 Hz, 3H), 3.57 -3.27 (m, 4H), 3.15 *(d, J=* 10.9 Hz, 2H), 2.97 - 2.79 (m, 2H), 2.66 - 2.51 (m, 2H), 2.41 - 2.20 (m, 3H), 2.17 - 1.97 (m, 4H), 1.97 - 1.79 (m, 3H), 1.65 (q, *J =* 11.8 Hz, 2H), 1.53 (q, *J =* 11.4 Hz, 2H).
LC-MS(ESI): [M+H]⁺ =807.55

### Example 77

### Synthesis of compound 59

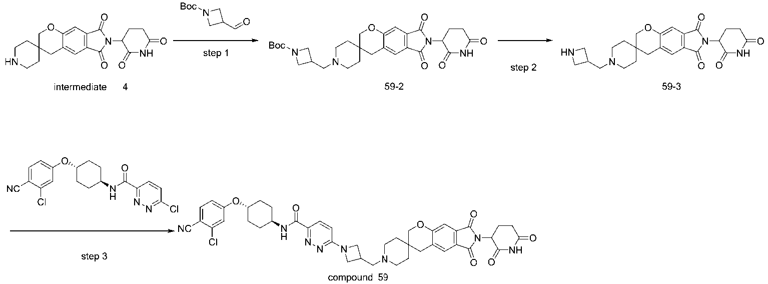

Step 1: The intermediate 4 (50 mg, 0.13 mmol) and tert-butyl 3-formylaniline-1-carboxylate (48.3 mg, 0.26 mmol) were added sequentially in a glass vial, and the solvents DCM (1 mL), AcOH (1 drop) were added dropwise, and the reaction solution was stirred at room temperature for 3 h. Then NaBH(OAc)₃ (83 mg, 0.39 mmol) was added, and the reaction solution was continued to be stirred at room temperature for 1 h. After the reaction was completed, the reaction solution was concentrated, and then purified by TLC (DCM:MeOH=10:1) to obtain a white solid compound 59-2 (45.3 mg).

Step 2: (Compound 59-3) was prepared with reference to step 2 of Example 19.

Step 3: (Compound 59) was prepared with reference to step 3 of Example 39.
¹H NMR (600 MHz, DMSO-d₆) δ 11.12 (s, 1H), 8.60 (d, *J =* 8.2 Hz, 1H), 7.88 (dd, *J =* 11.2, 9.0 Hz, 2H), 7.68 (d, *J* = 61.7 Hz, 1H), 7.39 (d, *J =* 2.4 Hz, 1H), 7.31 (d, *J =* 4.7 Hz, 1H), 7.14 (dd, *J =* 8.8, 2.4 Hz, 1H), 6.91 (dd, *J =* 9.3, 1.5 Hz, 1H), 5.11 (dd, *J =* 12.9, 5.4 Hz, 1H), 4.58 - 4.49 (m, 1H), 4.37 - 4.29 (m, 3H), 4.05 (s, 2H), 4.01 - 3.93 (m, 2H), 3.90 - 3.82 (m, 3H), 3.45 - 3.35 (m, 3H), 3.31 (s, 1H), 3.17 (d, *J =* 12.2 Hz, 2H), 3.09 (s, 1H), 2.92 - 2.83 (m, 1H), 2.81 (s, 1H), 2.59 (dd, *J =* 31.0, 14.6 Hz, 1H), 2.15 - 2.07 (m, 2H), 2.06 - 1.99 (m, 1H), 1.93 - 1.86 (m, 2H), 1.68 (dd, *J =* 42.2, 28.4 Hz, 5H), 1.52 (d, *J =* 13.0 Hz, 2H).
LC-MS(ESI): [M+H]⁺ = 807.60

### Example 78

### Synthesis of compound 60

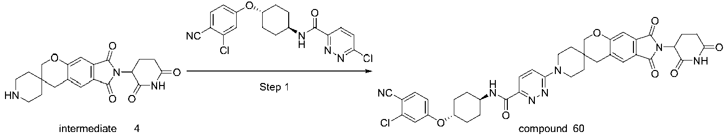

Step 1: The intermediate 4 (50 mg, 0.13 mmol), the intermediate 18 (102.1 mg, 0.26 mmol), DIEA (100.6 mg, 0.78 mmol), DMSO (2 mL) were added sequentially in a single-necked flask. The reaction solution was stirred at 80 °C for 2 h. After the reaction was completed, the reaction solution was cooled to room temperature, concentrated, and then purified to obtain a white solid compound 60 (44 mg).
¹H NMR (600 MHz, DMSO-d₆) δ 11.11 (s, 1H), 8.62 (d, *J =* 8.2 Hz, 1H), 7.86 (dd, *J =* 9.2, 5.1 Hz, 2H), 7.69 (s, 1H), 7.40 (dd, *J =* 11.0, 6.0 Hz, 2H), 7.28 (s, 1H), 7.14 (dd, *J =* 8.8, 2.4 Hz, 1H), 5.10 (dd, *J =* 12.9, 5.4 Hz, 1H), 4.59 - 4.50 (m, 1H), 4.16 (s, 3H), 3.88 (ddd, *J =* 14.9, 7.9, 3.8 Hz, 2H), 3.71 (d, *J* = 9.2 Hz, 2H), 3.00 - 2.84 (m, 3H), 2.65 - 2.53 (m, 2H), 2.15 - 2.00 (m, 3H), 1.90 (d, *J =* 10.6 Hz, 2H), 1.70 - 1.45 (m, 8H).
LC-MS(ESI): [M+H]⁺ = 738.49

### Example 79

### Synthesis of compound 61

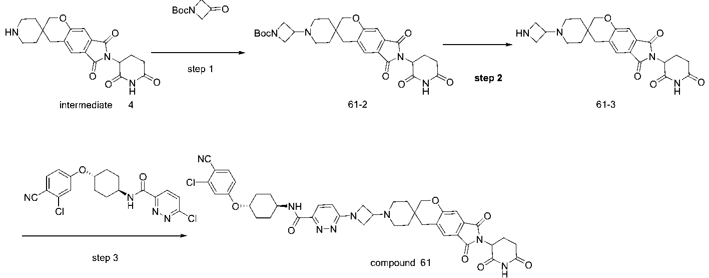

Step 1: The intermediate 4 (200 mg, 0.522 mmol) and tert-butyl 3-oxoazetidine-1-carboxylate (178.61 mg, 1.04 mmol) were added sequentially to a glass vial, and the solvents THF (2 mL) and Ti(O-iPr)₄(296.52 mg, 1.04 mmol) were added and the reaction solution was stirred at 60 °C for 1 h. Then NaBH₃CN (98.34 mg, 1.56 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 61-2 (170 mg).
¹H NMR (600 MHz, Methanol-d₄) δ 7.68 (s, 1H), 7.30 (s, 1H), 5.14 - 5.10 (m, 1H), 4.36 - 4.24 (m, 4H), 4.14 (t*, J =* 11.1 Hz, 4H), 3.52 (s, 2H), 3.14 (s, 4H), 2.96 - 2.82 (m, 4H), 2.79 - 2.69 (m, 3H), 1.48 (s, 9H).
LC-MS: [M+H]⁺ =539.43

Step 2: (Compound 61-3) was prepared with reference to step 2 of Example 19.
LC-MS: [M+H]⁺ =439.36

Step 3: (Compound 61) was prepared with reference to step 3 of Example 25.
¹H NMR (400 MHz, DMSO-d₆) δ 11.09 (s, 1H), 8.55 (d, *J =* 8.2 Hz, 1H), 7.84 (dd, *J =* 9.0, 7.2 Hz, 2H), 7.69 (s, 1H), 7.38 (d, *J =* 2.4 Hz, 1H), 7.25 (s, 1H), 7.13 (dd, *J =* 8.8, 2.4 Hz, 1H), 6.85 (d, *J* = 9.3 Hz, 1H), 5.08 (dd, *J =* 12.8, 5.4 Hz, 1H), 4.53 (dt, *J =* 10.3, 5.6 Hz, 1H), 4.26 - 4.12 (m, 2H), 4.07 (s, 2H), 3.95 (dd, *J =* 9.0, 5.0 Hz, 2H), 3.86 (d, *J* = 11.6 Hz, 1H), 2.86 (s, 3H), 2.69 - 2.51 (m, 3H), 2.46 (d, *J =* 6.1 Hz, 2H), 2.32 (d, *J =* 11.0 Hz, 2H), 2.16 - 2.06 (m, 2H), 2.06 - 1.93 (m, 2H), 1.90 (dt, *J =* 8.1, 4.1 Hz, 2H), 1.71 - 1.58 (m, 2H), 1.52 (td, *J =* 9.7, 4.6 Hz, 3H), 1.42 (d, *J =* 14.3 Hz, 2H).
LC-MS: [M+H]⁺ =793.8

### Example 80

### Synthesis of compound 62

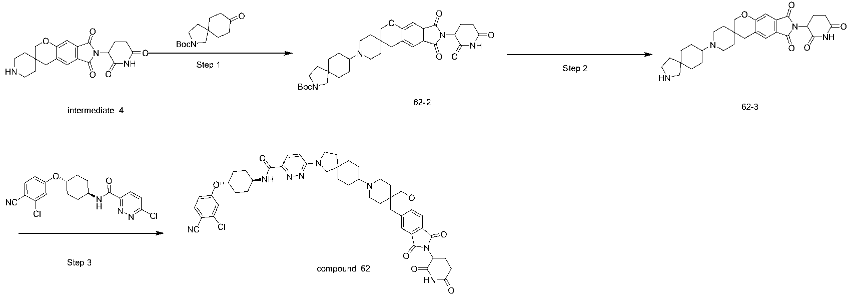

Step 1: The intermediate 4 (100 mg 0.261 mmol), tert-butyl 8-oxo-2-azaspiro[4.5]decane-2-carboxylate (132.15 mg, 0.522 mmol), sodium cyanoborohydride (49.17 mg, 0.782 mmol), tetraisopropyl titanate (148.26 mg, 0.521 mmol) were added to a single-necked flask containing tetrahydrofuran (2 mL) and stirred at 60 °C for 2 h. After the reaction was completed, the reaction solution was cooled to room temperature and purified by TLC (dichloromethane:methanol=10:1) to obtain a white solid compound 62-2 (30 mg).
¹H NMR (500 MHz, Chloroform-d) δ 8.99 (s, 1H), 7.78 (t, *J =* 1.0 Hz, 1H), 7.54 (s, 1H), 5.43 (t, *J* = 7.0 Hz, 1H), 3.96 (s, 2H), 3.50 (t, *J* = 7.1 Hz, 2H), 3.44 (d, *J* = 0.8 Hz, 2H), 2.78 - 2.56 (m, 9H), 2.22 - 2.06 (m, 2H), 1.92 (t, *J* = 7.0 Hz, 1H), 1.86 - 1.42 (m, 13H), 1.46 (s, 9H).
LC-MS: [M+H]⁺=621.53

Step 2: (Compound 62-3) was prepared with reference to step 2 of Example 25.

Step 3 (compound 62) was prepared with reference to step 3 of Example 25.
¹H NMR (600 MHz, Methanol-d₄) δ 7.95 (dd, *J =* 9.4, 7.5 Hz, 1H), 7.73 - 7.67 (m, 2H), 7.29 (s, 1H), 7.24 - 7.21 (m, 1H), 7.08 - 7.05 (m, 1H), 7.04 - 6.99 (m, 1H), 5.37-5.35 (t, J= 4.9Hz, 1H), 5.14 - 5.09 (m, 1H), 4.62 (s, 1H), 4.53 (s, 2H), 4.17 (s, 2H), 4.00 (s, 1H), 3.77 - 3.56 (m, 5H), 3.05 - 2.71 (m, 8H), 2.28 - 2.19 (m, 4H), 2.13 (d, *J = 10.7* Hz, 4H), 2.05 (q, *J =* 6.4 Hz, 2H), 1.96 (dt, *J =* 26.1, 9.2 Hz, 3H), 1.81 (s, 4H), 1.67 (t, *J =* 9.3 Hz, 4H).
LC-MS: [M+H]⁺ =875.41

### Example 81

### Synthesis of compound 63

### synthesis scheme

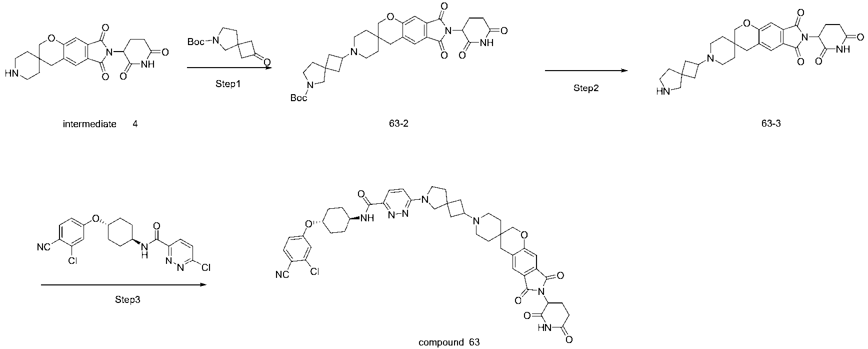

Step 1: The intermediate 4 (100 mg, 0.271 mmol) and tert-butyl 2-oxo-6-azaspiro[3.4]octane-6-carboxylate (88.14 mg, 0.391 mmol) were added to the solvent DCE (1 mL), AcOH (4 drops), and the reaction was carried out at room temperature for 1 h. The reaction was continued for another 1 h at room temperature with adding NaBH(OAc)₃ (115 mg, 0.542 mmol). The reaction solution was concentrated, washed with 5 mL of saturated ammonium chloride aqueous solution, extracted with ethyl acetate, concentrated and purified by preparative thin-layer chromatography (DCM:MeOH=10:1) to obtain a white solid compound 63-2 (80 mg).
¹H NMR (600 MHz, Chloroform-d) δ 8.04 (s, 1H), 7.58 (s, 1H), 4.96 (dd, *J =* 12.7, 5.3 Hz, 1H), 4.03 (s, 2H), 3.44 - 3.28 (m, 4H), 2.96 - 2.72 (m, 6H), 2.36 (d, *J* = 102.0 Hz, 3H), 2.21 - 2.13 (m, 2H), 1.89 (q, *J* = 7.5, 7.0 Hz, 2H), 1.55 (d, *J* = 12.9 Hz, 5H), 1.52 - 1.42 (m, 9H), 1.29 (d, *J =* 19.2 Hz, 3H).
LC-MS(ESI): [M+H]⁺ = 593.55

Step 2: (Compound 63-3) was prepared with reference to step 2 of Example 19.
LC-MS(ESI): [M+H]⁺ =493.38

Step 3: (Compound 63) was prepared with reference to step 3 of Example 19.
¹H NMR (600 MHz, DMSO-d₆) δ 11.12 (s, 1H), 9.64 (s, 1H), 8.55 (dd, *J =* 8.2, 4.1 Hz, 1H), 7.90 - 7.84 (m, 1H), 7.67 (d, *J* = 57.2 Hz, 1H), 7.39 (d, *J* = 2.4 Hz, 1H), 7.31 (dd, *J* = 5.5, 2.2 Hz, 1H), 7.14 (dd, *J* = 8.8, 2.4 Hz, 1H), 6.98 (dd, *J* = 9.4, 2.2 Hz, 1H), 5.10 (dd, *J =* 12.9, 5.5 Hz, 1H), 4.66 - 4.45 (m, 2H), 4.32 (s, 3H), 4.07 (s, 2H), 3.86 (d, *J* = 10.8 Hz, 2H), 3.47 (d, *J =* 5.3 Hz, 4H), 3.33 (t, *J =* 13.9 Hz, 2H), 3.14 - 2.78 (m, 4H), 2.77 - 2.53 (m, 1H), 2.35 (d, *J* = 9.5 Hz, 4H), 2.15 - 1.96 (m, 4H), 1.91 (d, *J =* 12.2 Hz, 2H), 1.78 (d, *J =* 14.2 Hz, 1H), 1.72 - 1.58 (m, 4H), 1.52 (q, *J =* 11.8 Hz, 2H).
LC-MS(ESI): [M+H]⁺ =847.64

### Example 82

### Synthesis of compound 64

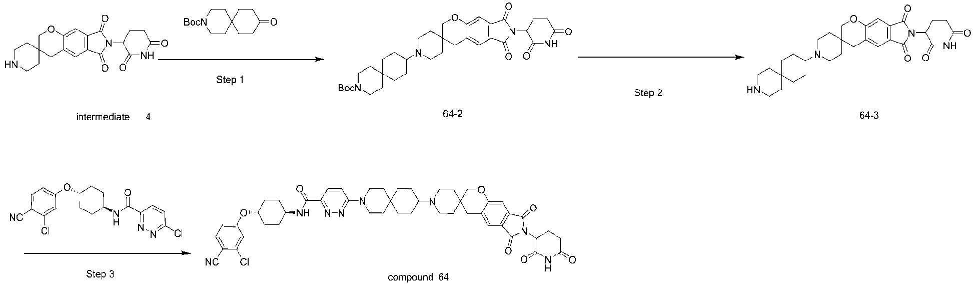

Step 1: The intermediate 4 (100 mg 0.26 mmol), tert-butyl 9-oxo-3-azaspiro[5.5]undecane-3-carboxylate (139.47 mg, 0.522 mmol), sodium cyanoborohydride (49.17 mg, 0.782 mmol), tetraisopropyl titanate (148.26 mg, 0.521 mmol) were added into a single-necked flask containing tetrahydrofuran (2 mL) and stirred at 60 °C for 2 h. After completion of the reaction, the reaction solution was cooled to room temperature and purified by TLC (dichloromethane:methanol=10:1) to obtain a white solid compound 64-2 (70 mg).
LC-MS(ESI): [M+H]⁺ =635.59

Step 2: (Compound 64-3) was prepared with reference to step 2 of Example 25.

Step 3: (Compound 64) was prepared with reference to step 3 of Example 25.
¹H NMR (600 MHz, Methanol-d₄) δ 7.99 (d, *J* = 9.7 Hz, 1H), 7.73 -7.65 (m, 2H), 7.46 (d, *J =* 9.8 Hz, 1H), 7.30 (d, *J* = 1.3 Hz, 1H), 7.22 (d, *J* = 2.4 Hz, 1H), 7.06 (dd, *J* = 8.7, 2.4 Hz, 1H), 5.14 - 5.10 (m, 1H), 4.52 (d, *J* = 4.5 Hz, 1H), 4.35 (s, 1H), 4.03 (d, *J =* 41.1 Hz, 2H), 3.80 (dt, *J =* 12.2, 6.0 Hz, 4H), 3.56 (t, *J =* 11.8 Hz, 2H), 3.29 (d, *J =* 12.9 Hz, 2H), 3.12 (s, 1H), 2.92 - 2.83 (m, 2H), 2.80 - 2.70 (m, 2H), 2.22 (d, *J =* 8.9 Hz, 2H), 2.16 - 1.94 (m, 8H), 1.88 - 1.74 (m, 6H), 1.71 - 1.62 (m, 4H), 1.59 (t, *J =* 5.9 Hz, 2H), 1.40 (t, *J =* 13.1 Hz, 2H), 1.34 (d, *J =* 21.0 Hz, 2H).
LC-MS(ESI): [M+H]⁺ = 889.68

### Example 83

### Synthesis of compound 65

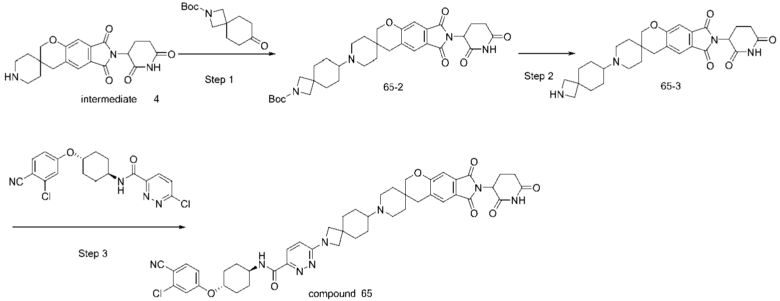

Step 1: The intermediate 4 (50 mg, 0.13 mmol) and tert-butyl 7-oxo-2-azaspiro[3.5]nonane-2-carboxylate (62.4 mg, 0.26 mmol) were added sequentially into a glass vial, and the solvents DCM (1 mL), AcOH (1 drop) were added dropwise, and the reaction solution was stirred at room temperature for 1 h, and then NaBH(OAc)₃ (24.6 mg, 0.39 mmol) was added, and the reaction solution was continued to be stirred at room temperature for 1 h. After the reaction was completed, the reaction solution was concentrated, and then purified by TLC (DCM:MeOH=10:1) to obtain a white solid compound 65-2 (21.5 mg).

Step 2: (Compound 65-3) was prepared with reference to step 2 of Example 39.

Step 3: (Compound 65) was prepared with reference to step 3 of Example 39.
¹H NMR (600 MHz, DMSO-d₆) δ 11.12 (s, 1H), 8.56 (d, *J =* 8.0 Hz, 1H), 7.91 - 7.83 (m, 2H), 7.69 (d, *J =* 52.0 Hz, 1H), 7.39 (d, *J* = 2.3 Hz, 1H), 7.31 (d, *J =* 2.8 Hz, 1H), 7.14 (dd, *J =* 8.8, 2.3 Hz, 1H), 6.91 - 6.85 (m, 1H), 5.11 (dd, *J* = 12.9, 5.4 Hz, 1H), 4.59 - 4.50 (m, 1H), 4.33 (s, 1H), 4.04 (s, 1H), 3.86 (d, *J =* 41.0 Hz, 1H), 3.70 (s, 3H), 3.41 - 3.32 (m, 5H), 3.20 (dd, *J* = 32.2, 20.5 Hz, 4H), 3.09 (s, 1H), 2.88 (dd, *J* = 21.9, 8.9 Hz, 2H), 2.79 (s, 1H), 2.60 (d, *J* = 18.0 Hz, 1H), 2.18 - 1.98 (m, 6H), 1.95 - 1.86 (m, 2H), 1.80 - 1.44 (m, 10H).
LC-MS(ESI): [M+H]⁺ = 861.62

### Example 84

### Synthesis of compound 66

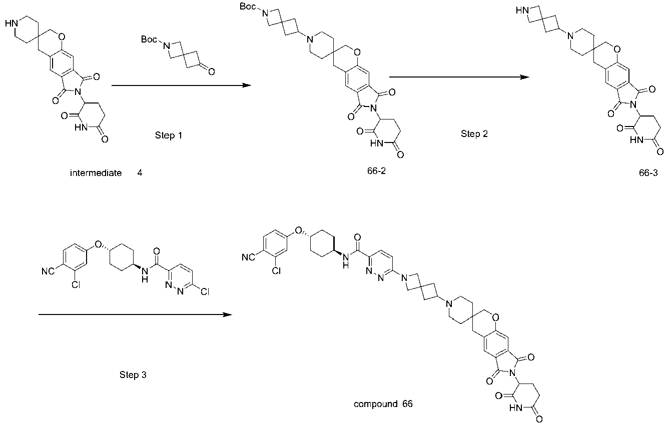

Step 1: The intermediate 4 (80 mg, 0.208 mmol) and tert-butyl 6-oxo-2-azaspiro[3.3]heptane-2-carboxylate (88.16 mg, 0.417 mmol) were added sequentially to a glass vial, and the solvents THF (2 mL) and Ti(O-iPr)₄₍ 177.91 mg, 0.626 mmol) were added. The reaction solution was stirred at 60 °C for 1 h. Then NaBH₃CN (26.22 mg, 0.417 mmol) was added and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 66-2 (45 mg).
LC-MS: [M+H]⁺= 579.40

Step 2: (Compound 66-3) was prepared with reference to step 2 of Example 49.
LC-MS: [M+H]⁺= 479.10

Step 3: (Compound 66) was prepared with reference to step 3 of Example 25.
¹H NMR (600 MHz, DMSO-d₆) δ 11.12 (s, 1H), 8.55 (dd, *J =* 8.1, 3.9 Hz, 1H), 7.87 (dd, *J =* 9.0, 2.5 Hz, 1H), *7.67* (d, *J* = 55.4 Hz, 1H), 7.39 (d, *J =* 2.3 Hz, 1H), 7.31 (d, *J* = 4.4 Hz, 1H), 7.14 (dd, *J* = 8.8, 2.4 Hz, 1H), 6.88 (dd, *J* = 24.7, 9.3 Hz, 1H), 5.33 (t, *J* = 5.0 Hz, 1H), 5.10 (dd, *J* = 12.9, 5.4 Hz, 1H), 4.59 - 4.48 (m , 1H), 4.16 (dt, *J* = 32.2, 26.0 Hz, 6H), 3.90 - 3.70 (m, 3H), 3.31 - 3.21 (m, 4H), 2.93 (ddd, *J =* 35.1, 19.9, 8.0 Hz, 3H), 2.69 - 2.58 (m, 2H), 2.15 - 1.87 (m, 8H), 1.70 - 1.58 (m, 4H), 1.57 - 1.42 (m, 3H).
LC-MS: [M+H]⁺= 834.31

### Example 85

### Synthesis of compound 67

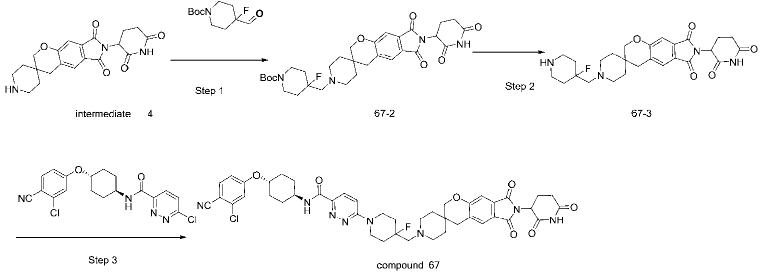

Step 1: The intermediate 4 (200 mg, 0.522 mmol) and tert-butyl 4-fluoro-4-formylpiperidine-1-carboxylate (180.96 mg, 0.782 mmol) were added sequentially to a glass vial, and the solvents THF (2 mL) and Ti(O-iPr)₄ (296.52 mg, 1.04 mmol) were added, and the reaction solution was stirred at 60°C for 1 h. Then NaBH₃CN (98.34 mg, 1.56 mmol) was added and the reaction solution was continued to be stirred at 60 °C for 1 h. After the reaction was completed, the reaction solution was concentrated and then purified by preparative thin-layer chromatography (DCM:MeOH=10:1) to obtain a white solid compound 67-2 (180 mg).
¹H NMR (400 MHz, Methanol-d₄) δ 7.61 (s, 1H), 7.20 (s, 1H), 5.07 (dd, *J =* 12.5, 5.4 Hz, 1H), 4.05 (s, 2H), 3.89 - 3.81 (m, 2H), 2.82 (d, *J =* 6.3 Hz, 2H), 2.79 - 2.70 (m, 2H), 2.65 - 2.56 (m, 4H), 1.92 - 1.83 (m, 2H), 1.82 - 1.63 (m, 2H), 1.58 (ddd, *J =* 12.0, 8.3, 3.7 Hz, 4H), 1.51 (ddd, *J =* 7.1, 4.0 Hz, 2H), 1.46 (s, 13H).
LC-MS: [M+H]⁺ =599.59

Step 2: (Compound 67-3) was prepared with reference to step 2 of Example 19.
LC-MS: [M+H]⁺= 499.50

Step 3: (Compound 67) was prepared with reference to step 3 of Example 25.
¹H NMR (400 MHz, Methanol-d₄) δ 8.32 (s, 2H), 7.92 (d, *J =* 9.6 Hz, 1H), 7.70 (d, *J =* 8.7 Hz, 1H), 7.63 (s, 1H), 7.35 (d, *J =* 9.7 Hz, 1H), 7.25 - 7.19 (m, 2H), 7.06 (dd, *J =* 8.8, 2.4 Hz, 1H), 5.09 (dd, *J =* 12.5, 5.4 Hz, 1H), 4.34 (d, *J =* 13.6 Hz, 2H), 3.99 (s, 1H), 3.44 (t, *J =* 12.3 Hz, 3H), 2.93 - 2.81 (m, 3H), 2.81 - 2.60 (m, 8H), 2.28 - 2.00 (m, 7H), 1.90 - 1.69 (m, 3H), 1.69 - 1.45 (m, 7H).
LC-MS: [M+H]⁺ =853.75

### Example 86

### Synthesis of compound 68

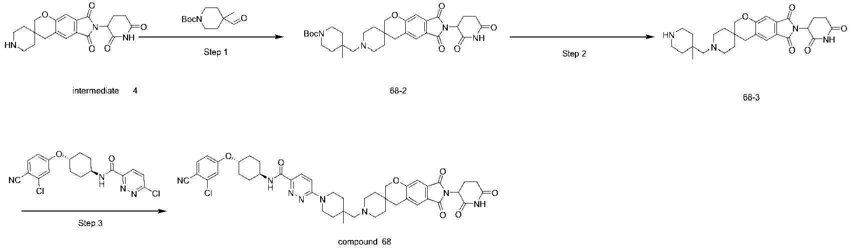

Step 1: The intermediate 4 (100 mg, 0.26 mmol), tert-butyl 4-formyl-4-methylpiperidine-1-carboxylate (88.93 mg, 0.391 mmol), sodium cyanoborohydride (49.17 mg, 0.782 mmol), and tetraisopropyl titanate (148.26 mg, 0.521 mmol) were added to a single-necked flask containing tetrahydrofuran (2 mL). The reaction solution was stirred at 60 °C for 2 h. After completion of the reaction, the reaction solution was cooled to room temperature and purified by TLC (dichloromethane:methanol=10:1) to obtain a white solid compound 68- 2 (55 mg).
¹H NMR (500 MHz, Chloroform-d) δ 8.99 (s, 1H), 7.80 (t, *J =* 1.0 Hz, 1H), 7.58 (s, 1H), 5.45 (t, *J =* 7.0 Hz, 1H), 3.70 (dt, *J =* 12.4, 7.1 Hz, 2H), 3.51 (dt, *J =* 12.5, 7.1 Hz, 2H), 2.83 (td, *J =* 7.1, 1.0 Hz, 2H), 2.72 - 2.55 (m, 6H), 2.47 (s, 2H), 2.28 (t*, J =* 7.0 Hz, 2H), 2.20 - 2.11 (m, 2H), 2.08 - 1.96 (m, 3H), 1.84 (t*, J =* 7.1 Hz, 4H), 1.45 (s, 9H), 1.11 (s, 3H).
LC-MS: [M+H]⁺ =595.61

Step 2: (Compound 68-3) was prepared with reference to step 2 of Example 25.

Step 3: (Compound 68) was prepared with reference to step 3 of Example 25.
¹H NMR (400 MHz, Methanol-d₄) δ 7.93 (d, *J =* 9.6 Hz, 1H), 7.69 (d, J = 8.8 Hz, 1H), 7.67 (s, 1H), 7.33 (d, *J =* 9.7 Hz, 1H), 7.28 (s, 1H), 7.20 (d, *J =* 2.4 Hz, 1H), 7.04 (dd, *J =* 8.8, 2.4 Hz, 1H), 5.38 - 5.30 (m, 1H), 5.09 (dd, *J =* 12.5, 5.4 Hz, 1H), 4.51 (s, 1H), 4.29 (s, 1H), 4.22 (d, *J =* 13.9 Hz, 2H), 4.08 (s, 1H), 3.98 (d, *J =* 4.0 Hz, 1H), 3.59 (s, 2H), 3.54 - 3.46 (m, 2H), 3.37 (s, 2H), 3.25 (s, 2H), 3.09 (s, 1H), 2.92 - 2.85 (m, 2H), 2.79 - 2.75 (m, 1H), 2.73 (s, 1H), 2.71 - 2.65 (m, 1H), 2.21 (s, 1H), 2.18 (d, *J* = 7.8 Hz, 1H), 2.11 (dt, *J* = 7.7, 2.8 Hz, 2H), 2.03 (d, *J =* 3.8 Hz, 1H), 1.91 (s, 2H), 1.79 (d, *J =* 16.8 Hz, 1H), 1.72 (t, *J =* 4.7 Hz, 3H), 1.65 (t, *J =* 9.4 Hz, 3H), 1.36 (s, 4H).
LC-MS: [M+H]⁺ =849.72

### Example 87

### Synthesis of compound 69

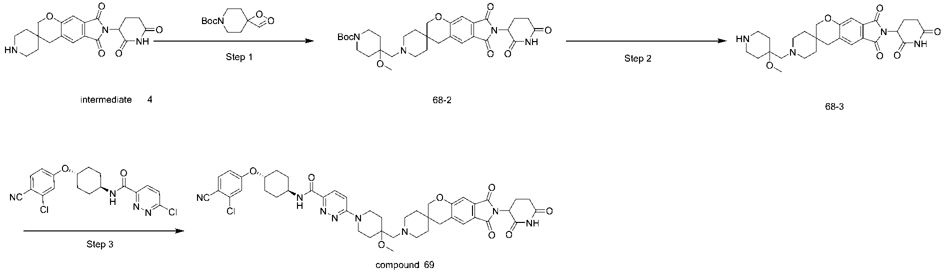

Step 1: The intermediate 4 (150 mg, 0.391 mmol), tert-butyl 4-formyl-4-methoxypiperidine-1-carboxylate (142.78 mg, 0.589 mmol), sodium cyanoborohydride (73.76 mg, 1.17 mmol), tetraisopropyl titanate (222.39 mg, 0.782 mmol) were added into a single-ported flask containing tetrahydrofuran (2 mL) and stirred at 60°C for 2 h. After completion of the reaction, the reaction solution was cooled to room temperature and purified by TLC (dichloromethane:methanol=10:1) to obtain a white solid compound 69-2 (75 mg).
¹H NMR (500 MHz, Chloroform-d) δ 8.99 (s, 1H), 7.78 (t, *J =* 1.0 Hz, 1H), 7.54 (s, 1H), 5.45 (t, *J =* 7.0 Hz, 1H), 3.96 (s, 2H), 3.83 - 3.59 (m, 4H), 3.18 (s, 3H), 2.86 (s, 2H), 2.77 - 2.68 (m, 4H), 2.67 - 2.58 (m, 4H), 2.22 - 2.09 (m, 2H), 2.09 - 1.94 (m, 4H), 1.76 (td, *J* = 7.1, 1.2 Hz, 4H), 1.45 (s, 9H).
LC-MS: [M+H]⁺ =611.59

Step 2: (Compound 69-3) was prepared with reference to step 2 of Example 25.

Step 3: (Compound 69) was prepared with reference to step 3 of Example 25.
¹H NMR (400 MHz, Methanol-d₄) δ 7.92 (d, *J =* 9.4 Hz, 1H), 7.70 (d, *J =* 8.8 Hz, 1H), 7.64 (s, 1H), 7.33 (d, *J* = 9.6 Hz, 1H), 7.24 (s, 1H), 7.21 (d, *J* = 2.4 Hz, 1H), 7.06 (dd, *J =* 8.8, 2.4 Hz, 1H), 5.09 (dd, *J* = 12.4, 5.4 Hz, 1H), 4.58 (s, 5H), 4.24 (d, *J* = 12.9 Hz, 2H), 4.10 (s, 2H), 3.99 (s, 2H), 3.46 - 3.37 (m, 1H), 3.01 - 2.86 (m,4H), 2.83 (d, J = 5.7 Hz, 1H), 2.78 (dd, *J* = 4.4, 2.4 Hz, 1H), 2.73 (s, 1H), 2.70 (d, *J* = 4.2 Hz, 1H), 2.20 (t, *J* = 7.6 Hz, 3H), 2.15 - 2.09 (m, 3H), 2.07 - 1.98 (m,4H), 1.71-1.60 (m, 8H).
LC-MS: [M+H]⁺ =865.70

### Example 88

### Synthesis of compound 70

### synthesis scheme

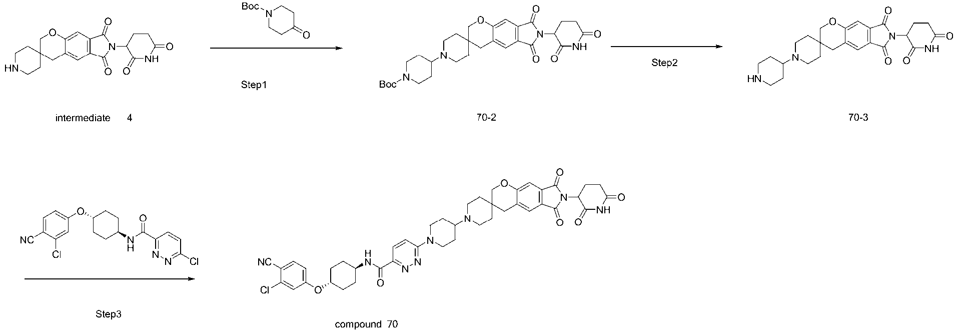

Step 1: The intermediate 4 (100 mg, 0.271 mmol) and tert-butyl 2-oxo-6-azaspiro[3.4]octane-6-carboxylate (77.9 mg, 0.391 mmol) were added to the solvent DCE (1 mL), AcOH (4 drops), and the reaction was carried out at room temperature for 1 h. The reaction was continued for another 1 h at room temperature by adding NaBH(OAc)₃ (115 mg, 0.542 mmol). The reaction solution was concentrated, washed by adding 5 mL of saturated ammonium chloride aqueous solution, extracted with ethyl acetate, concentrated and purified by preparative thin-layer chromatography (DCM:MeOH=10:1) to obtain a white solid compound 70-2 (90 mg).
¹H NMR (600 MHz, Chloroform-d) δ 8.04 (s, 1H), 7.58 (s, 1H), 4.96 (dd, *J =* 12.7, 5.3 Hz, 1H), 4.03 (s, 2H), 3.52 (s, 1H), 3.44 - 3.26 (m, 3H), 2.96 - 2.72 (m, 4H), 2.36 (d, *J =* 102.0 Hz, 3H), 2.25 - 2.13 (m, 2H), 2.08 - 1.53 (m, 10H), 1.52 - 1.23 (m, 9H).
LC-MS(ESI): [M+H]⁺ = 567.49

Step 2: (Compound 70-3) was prepared with reference to step 2 of Example 19.
LC-MS(ESI): [M+H]⁺ =467.39

Step 3: (Compound 70) was prepared with reference to step 3 of Example 19.
¹H NMR (600 MHz, DMSO-d₆) δ 11.12 (s, 1H), 9.31 (s, 1H), 8.65 (dd, *J =* 8.2, 1.8 Hz, 1H), 7.88 (t, *J* = 9.5 Hz, 1H), 7.68 (d, *J* = 51.7 Hz, 1H), 7.47 (d, *J* = 9.6 Hz, 1H), 7.40 (d, *J* = 2.4 Hz, 1H), 7.30 (d, *J =* 2.4 Hz, 1H), 7.14 (dd, *J =* 8.8, 2.5 Hz, 1H), 5.10 (dd, *J =* 12.9, 5.4 Hz, 1H), 4.95 - 4.16 (m, 7H), 4.04 (s, 1H), 3.87 (td, *J =* 10.7, 9.2, 5.6 Hz, 1H), 3.61 (d, *J =* 8.0 Hz, 1H), 3.43 (d, *J =* 11.9 Hz, 2H), 3.17 (s, 2H), 3.13 - 2.96 (m, 2H), 2.96 - 2.84 (m, 1H), 2.79 (s, 1H), 2.75 - 2.52 (m, 1H), 2.19 (s, 1H), 2.10 (dd, *J =* 14.2, 10.3 Hz, 2H), 2.07 - 2.00 (m, 1H), 1.99 - 1.81 (m, 2H), 1.77 (d, *J =* 14.3 Hz, 1H), 1.66 (h, *J =* 13.5, 10.9 Hz, 5H), 1.52 (td, *J =* 14.5, 13.8, 7.2 Hz, 2H), 1.46 - 1.16 (m, 1H).
LC-MS(ESI): [M+H]⁺ = 826.61

### Example 89

### Synthesis of compound 71

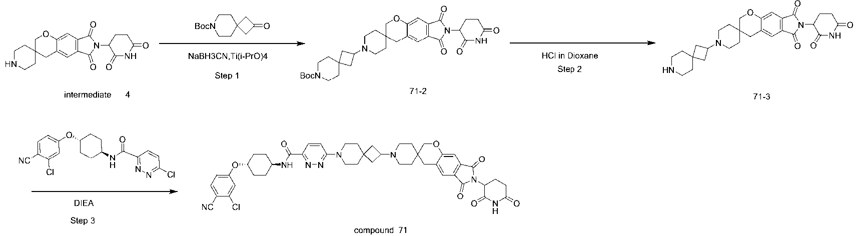

Step 1: The intermediate 4 (100 mg 0.26 mmol), tert-butyl 2-oxo-7-azaspiro[3.5]nonane-7-carboxylate (124.84 mg, 0.522 mmol), sodium cyanoborohydride (49.17 mg, 0.782 mmol), tetraisopropyl titanate (148.26 mg, 0.521 mmol) were added into a single-necked flask containing tetrahydrofuran (2 mL) and stirred at 60 °C for 2 h. After completion of the reaction, the reaction solution was cooled to room temperature and purified by TLC (dichloromethane:methanol=10:1) to obtain a white solid compound 71-2 (62 mg).
¹H NMR (600 MHz, Methanol-d₄) δ 7.66 (s, 1H), 7.26 (s, 1H), 5.51 (s, 1H), 5.11 (dd, *J =* 12.9, 5.4 Hz, 1H), 4.63 (s, 2H), 4.12 (s, 2H), 3.43 (s, 1H), 2.92 (s, 2H), 2.90 - 2.83 (m, 1H), 2.78 (d, *J* = 3.5 Hz, 1H), 2.75 (d, *J* = 3.4 Hz, 1H), 2.72 (d, *J* = 4.3 Hz, 2H), 2.21 (dd, *J =* 13.1, 7.3 Hz, 3H), 2.15 - 2.09 (m, 1H), 2.08 - 2.03 (m, 1H), 1.98 (s, 2H), 1.80 (t, *J =* 10.2 Hz, 2H), 1.70 (d, *J =* 6.9 Hz, 1H), 1.68 - 1.60 (m, 3H), 1.55 (t, *J =* 5.7 Hz, 2H), 1.47 (s, 9H), 1.38 (s, 1H).
LC-MS(ESI): [M+H]⁺ =607.58

Step 2: (Compound 71-3) was prepared with reference to step 2 of Example 25.

Step 3: (Compound 71) was prepared with reference to step 3 of Example 25.
¹H NMR (600 MHz, Methanol-d₄) δ 7.95 (d, *J =* 9.6 Hz, 1H), 7.71 (d, *J =* 8.8 Hz, 1H), 7.69 (d, *J =* 11.0 Hz, 1H), 7.38 (d, *J* = 9.7 Hz, 1H), 7.30 (d, *J =* 1.5 Hz, 1H), 7.23 (d, *J* = 2.4 Hz, 1H), 7.06 (dd, *J =* 8.8, 2.4 Hz, 1H), 5.14 - 5.10 (m, 1H), 4.53 (s, 2H), 4.35 (s, 1H), 4.09 (s, 1H), 4.00 (s, 2H), 3.82 (t*, J =* 5.7 Hz, 2H), 3.76 (t, *J =* 5.7 Hz, 2H), 3.50 (t, *J* = 12.6 Hz, 2H), 3.12 (s, 1H), 3.08 (d, *J =* 12.9 Hz, 2H), 2.87 (d, *J =* 7.8 Hz, 2H), 2.81 - 2.69 (m, 4H), 2.48 (t, *J* = 10.0 Hz, 2H), 2.21 (t, *J =* 7.6 Hz, 4H), 2.05 (q, *J =* 6.7, 6.2 Hz, 4H), 1.85- 1.83 (m, 2H), 1.78 (d, *J =* 5.6 Hz, 2H), 1.66 (t, *J =* 9.5 Hz, 2H), 1.62 (t, *J* = 7.3 Hz, 2H).
LC-MS(ESI): [M+H]⁺ =861.66

### Example 90

### Synthesis of compound 72

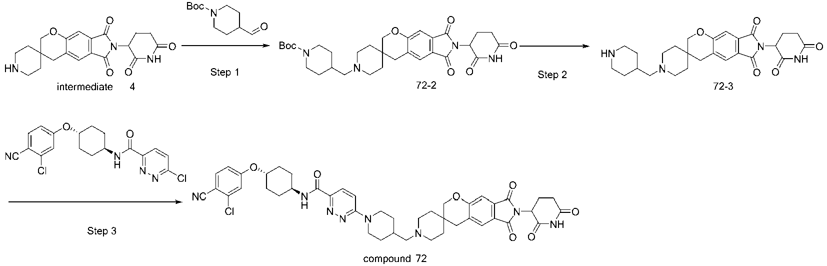

Step 1: The intermediate 4 (200 mg, 0.521 mmol) and tert-butyl 4-formylpiperidine-1-carboxylate (222.5 mg, 1.04 mmol) were added sequentially into a glass vial, and the solvents DCM (2 mL), AcOH (4 drops) were added dropwise, and the reaction solution was stirred at room temperature for 1 h, then NaBH(OAc)_{3 (}332 mg, 1.56 mmol) was added and the reaction solution was continued to be stirred at room temperature for 1 h. After the reaction was completed, the reaction solution was concentrated, and then purified by TLC (DCM:MeOH=10:1) to obtain a white solid compound 72-2 (230 mg).
¹H NMR (600 MHz, CD₃OD) δ 7.65 (s, 1H), 7.25 (s, 1H), 5.10 (dd, *J =* 12.9, 5.4 Hz, 1H), 4.10 (d, *J* = 17.4 Hz, 4H), 2.93 - 2.67 (m, 10H), 2.54 (d, *J =* 7.0 Hz, 2H), 2.16 - 2.09 (m, 1H), 1.89 - 1.88 (m, 1H), 1.79 (d, *J* = 12.7 Hz, 2H), 1.74 - 1.67 (m, 2H), 1.66 - 1.60 (m, 2H), 1.47 (s, 9H), 1.32 (s, 1H), 1.16 - 1.09 (m, 2H).

Step 2: (Compound 72-3) was prepared (180 mg) with reference to step 2 of Example 19.
¹H NMR (600 MHz, CD₃OD) δ 7.68 (s, 1H), 7.30 (s, 1H), 5.12 (dd, *J =* 12.8, 5.5 Hz, 1H), 4.35 (s, 1H), 4.07 (s, 1H), 3.60 (s, 2H), 3.47 (t, *J =* 13.9 Hz, 2H), 3.28 - 3.16 (m, 3H), 3.12 - 3.04 (m, 3H), 2.92 - 2.85 (m, 2H), 2.79 - 2.70 (m, 2H), 2.29 (s, 1H), 2.14 - 2.06 (m, 3H), 1.87 (d, *J* = 52.1 Hz, 4H), 1.55 (dd, *J =* 25.3, 12.3 Hz, 2H), 1.32 (s, 2H).

Step 3: (Compound 72) was prepared with reference to step 3 of Example 25.
¹H NMR (600 MHz, DMSO-d₆) δ 11.12 (s, 1H), 8.76 (d, *J =* 41.7 Hz, 1H), 8.60 (d, *J =* 8.2 Hz, 1H), 7.85 (dd, *J* = 14.8, 9.2 Hz, 2H), 7.68 (d, *J =* 72.8 Hz, 1H), 7.44 - 7.36 (m, 1H), 7.31 (d, *J =* 4.9 Hz, 1H), 7.14 (dd, *J =* 8.8, 2.4 Hz, 1H), 5.11 (dd, *J =* 12.9, 5.4 Hz, 1H), 4.59 - 4.50 (m, 3H), 4.33 (s, 1H), 4.04 (s, 1H), 3.89 - 3.83 (m, 2H), 3.52 - 3.40 (m, 2H), 3.16 - 3.04 (m, 5H), 2.94 - 2.83 (m, 1H), 2.80 (s, 1H), 2.65 - 2.52 (m, 1H), 2.42 - 2.36 (m, 1H), 2.20 (s, 2H), 2.12 - 2.01 (m, 2H), 1.94 - 1.82 (m, 3H), 1.80 - 1.61 (m, 5H), 1.56 - 1.47 (m, 3H), 1.30 - 1.17 (m, 3H).
LC-MS(ESI): [M+H]⁺ = 834.66

### Example 91

### Synthesis of compound 73

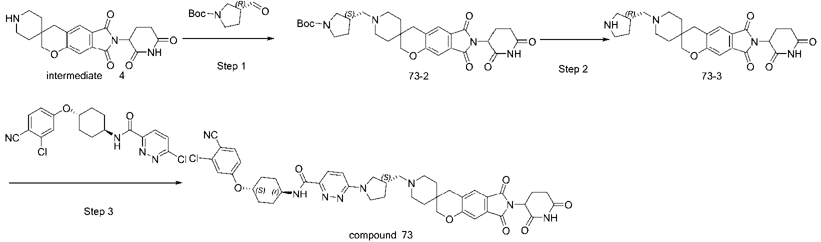

Step 1: The intermediate 4 (100 mg, 0.26 mmol) and tert-butyl (R)-3-formylpyrrolidine-1-carboxylate (103.9 mg, 0.52 mmol) were added sequentially in a glass vial, and then solvents DCM (2 mL) and AcOH (2 drop) were added dropwise, and the reaction solution was stirred at room temperature for 3 h. Then NaBH(OAc)₃( 165.8 mg, 0.78 mmol) was added, and the reaction solution was continued to be stirred at room temperature for 1 h. After the reaction was completed, the reaction solution was concentrated, and then purified by TLC (DCM:MeOH=10:1) to obtain a white solid compound 73-2 (142 mg).

Step 2: (Compound 73-3) was prepared with reference to step 2 of Example 39.

Step 3: (Compound 73) was prepared with reference to step 3 of Example 39.
¹H NMR (600 MHz, CD₃OD) δ 8.08 (d, *J =* 9.6 Hz, 1H), 7.70 (t, *J =* 7.9 Hz, 2H), 7.32 - 7.26 (m, 2H), 7.22 (d, *J* = 2.3 Hz, 1H), 7.06 (dd, *J =* 8.8, 2.3 Hz, 1H), 5.12 (dd, *J* = 12.8, 5.4 Hz, 1H), 4.53 (dd, *J =* 9.1, 4.8 Hz, 1H), 4.37 (s, 1H), 4.15 - 3.94 (m, 3H), 3.86 (s, 1H), 3.67 (d, *J =* 8.0 Hz, 3H), 3.46 (dd, *J =* 10.7, 6.2 Hz, 3H), 3.30-3.28 (m, 1H), 3.14 (s, 1H), 3.04 - 2.95 (m, 1H), 2.89 (ddd, *J =* 17.5, 14.0, 5.3 Hz, 2H), 2.82 - 2.67 (m, 2H), 2.54 - 2.43 (m, 1H), 2.23 (d, *J =* 2.8 Hz, 2H), 2.18 - 2.07 (m, 3H), 2.04 - 1.79 (m, 5H), 1.66 (t, *J =* 10.1 Hz, 4H), 1.33 (d, *J =* 15.8 Hz, 1H).
LC-MS(ESI): [M+H]⁺ = 821.61

### Example 92

### Synthesis of compound 74

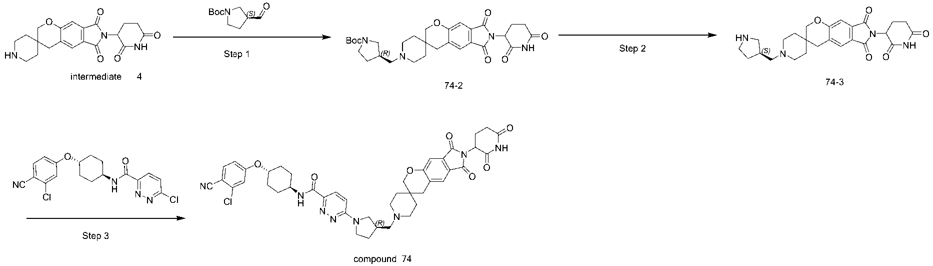

Step 1: The intermediate 4 (100 mg, 0.261 mmol), tert-butyl tert-butyl pyrrolidine-1-carboxylate (104 mg, 0.391 mmol), sodium cyanoborohydride (49.17 mg, 0.782 mmol), and tetraisopropyl titanate (148.26 mg, 0.521 mmol) were added to a single-necked flask containing tetrahydrofuran (2 mL). The reaction solution was stirred at 60 °C for 2 h. After completion of the reaction, the reaction solution was cooled to room temperature and purified by TLC (dichloromethane: methanol = 10:1) to obtain a white solid compound 74-2 (65 mg).
¹H NMR (600 MHz, Methanol-d₄) δ 7.65 (s, 1H), 7.24 (s, 1H), 5.10 (dd, *J =* 12.8, 5.4 Hz, 1H), 4.11 (s, 2H), 3.62 - 3.57 (m, 1H), 3.50 - 3.44 (m, 1H), 3.29 (d, *J* = 20.0 Hz, 1H), 3.03 (dt, *J =* 10.5, 8.0 Hz, 1H), 2.90 (s, 2H), 2.89 - 2.85 (m, 2H), 2.80 - 2.77 (m, 2H), 2.76 - 2.68 (m, 4H), 2.56 (dt, *J =* 15.4, 7.7 Hz, 1H), 2.16 - 2.07 (m, 2H), 1.74 - 1.60 (m, 6H), 1.48 (d, J = 2.1 Hz, 9H).
LC-MS(ESI): [M+H]⁺ = 567.47

The second step (compound 74-3) was prepared with reference to step 2 of Example 25.

The third step (compound 74) was prepared with reference to step 3 of Example 25.
¹H NMR (600 MHz, Methanol-d₄) δ 8.04 (d, *J* = 9.5 Hz, 1H), 7.71 (t, *J* = 7.4 Hz, 2H), 7.31 (s, 1H), 7.22 (d, *J* = 2.4 Hz, 1H), 7.19 (d, *J* = 9.5 Hz, 1H), 7.06 (dd, *J* = 8.8, 2.5 Hz, 1H), 5.12 (dd, *J* = 12.8, 5.4 Hz, 1H), 4.56 - 4.50 (m, 1H), 4.37 (s, 1H), 4.08 (d, *J* = 19.6 Hz, 2H), 4.05 - 3.95 (m, 2H), 3.84 (s, 1H), 3.73 - 3.60 (m, 4H), 3.45 (dt, *J =* 10.4, 5.1 Hz, 4H), 3.14 (s, 1H), 2.97 (p, *J* = 7.7 Hz, 1H), 2.89 (ddd, *J* = 17.3, 13.9, 5.3 Hz, 2H), 2.80 - 2.69 (m, 2H), 2.46 (dtd, *J =* 10.0, 6.7, 3.4 Hz, 1H), 2.22 (q, *J =* 7.8, 6.2 Hz, 2H), 2.16 - 2.09 (m, 3H), 1.98 (dt, *J =* 12.5, 9.1 Hz, 2H), 1.88 (s, 3H), 1.69 - 1.62 (m, 4H).
LC-MS(ESI): [M+H]⁺ = 821.42

### Example 93

### Synthesis of compound 75

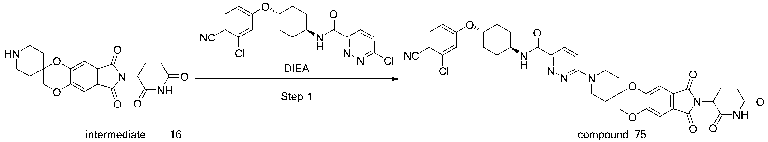

The intermediate 5 (20 mg, 0.051 mmol), the intermediate 18 (39.78 mg, 0.102 mmol), DIEA (65.79 mg, 0.51 mmol), DMSO (1 mL) were added sequentially in a single-necked flask. The reaction solution was stirred at 80 °C for 2 h. After the reaction was completed, the reaction solution was cooled to room temperature, concentrated, and then purified by preparation (15-50% acetonitrile) to obtain a white solid compound 75 (2 mg).
¹H NMR (400 MHz, DMSO-d₆) δ 11.10 (s, 1H), 8.63 (d, *J =* 8.2 Hz, 1H), 7.86 (dd, *J* = 9.2*,* 2.1 Hz, 2H), 7.50 - 7.44 (m, 2H), 7.43 (s, 1H), 7.39 (d, *J =* 2.4 Hz, 1H), 7.13 (dd, *J =* 8.8, 2.4 Hz, 1H), 5.09 (dd, *J =* 12.8, 5.4 Hz, 1H), 4.62 - 4.48 (m, 1H), 4.34 - 4.20 (m, 4H), 3.94 - 3.80 (m, 1H), 3.62 - 3.48 (m, 2H), 2.95 - 2.81 (m, 1H), 2.16 - 2.07 (m, 2H), 2.06 - 1.95 (m, 2H), 1.94 - 1.86 (m, 2H), 1.85 - 1.76 (m, 4H), 1.71 - 1.59 (m, 2H), 1.58 - 1.43 (m, 3H).
LC-MS: [M+H]⁺ =740.46

### Example 94

### Synthesis of compound 76

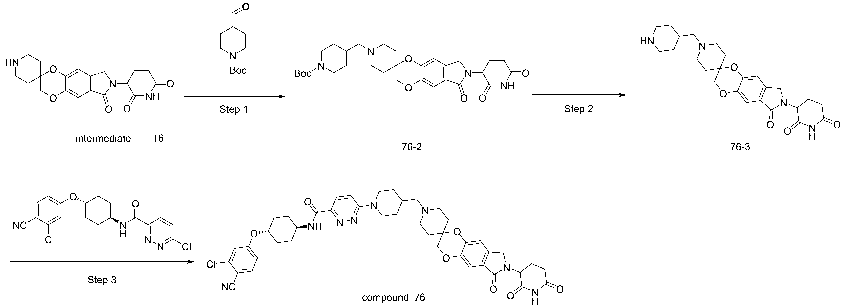

Step 1: The intermediate 16 (150 mg, 0.404 mmol) and tert-butyl 4-formylpiperidine-1-carboxylate (172.1 mg, 0.808 mmol) were added sequentially to a reaction flask, and the solvents DCE (2 mL) and AcOH (4 drops) were added dropwise, and the reaction solution was stirred at room temperature for 1 h, then NaBH(OAc)₃(257 mg, 1.21 mmol) was added, and the reaction solution was continued to be stirred at room temperature for 1 h. After the reaction was completed, the reaction solution was concentrated, and then purified by preparative thin-layer chromatography (DCM:MeOH=10:1) to obtain a white solid compound 76-2 (130 mg).
¹H NMR (600 MHz, DMSO-d₆) δ 10.95 (s, 1H), 7.16 (s, 1H), 7.09 (s, 1H), 5.05 (dd, *J* = 13.4, 5.1 Hz, 1H), 4.46 (t, *J* = 5.3 Hz, 1H), 4.30 (d, *J* = 16.6 Hz, 1H), 4.17 (d, *J* = 16.7 Hz, 1H), 4.09 - 4.00 (m, 2H), 3.91 (s, 3H), 3.27 - 3.18 (m, 1H), 2.94 - 2.83 (m, 2H), 2.63 - 2.53 (m, 3H), 2.40 - 2.24 (m, 3H), 2.17 - 2.12 (m, 2H), 2.04 - 1.91 (m, 2H), 1.74 - 1.57 (m, 6H), 1.38 (s, 9H).
LC-MS: [M+H]⁺ =469.50

Step 2: (Compound 76-3) was prepared with reference to step 2 of Example 19.
LC-MS: [M+H]⁺ =469.50

Step 3: (Compound 76) was prepared with reference to step 3 of Example 25.
¹H NMR (400 MHz, DMSO-d₆) δ 10.96 (s, 1H), 8.57 (d, *J =* 8.2 Hz, 1H), 7.90 - 7.80 (m, 2H), 7.44 - 7.35 (m, 2H), 7.24 (s, 1H), 7.18 - 7.10 (m, 2H), 5.07 (dd, *J =* 13.3, 5.0 Hz, 1H), 4.60 - 4.46 (m, 2H), 4.39 - 4.28 (m, 1H), 4.26 - 4.10 (m, 2H), 3.92 - 3.80 (m, 1H), 3.63 - 3.45 (m, 10H), 3.23 - 3.00 (m, 5H), 2.98 - 2.83 (m, 1H), 2.68 - 2.56 (m, 1H), 2.43 - 2.28 (m, 1H), 2.26 - 2.07 (m, 3H), 2.04 - 1.81 (m, 6H), 1.71 - 1.44 (m, 4H).
LC-MS: [M+H]⁺ =823.77

### Example 95

### Synthesis of compound 77

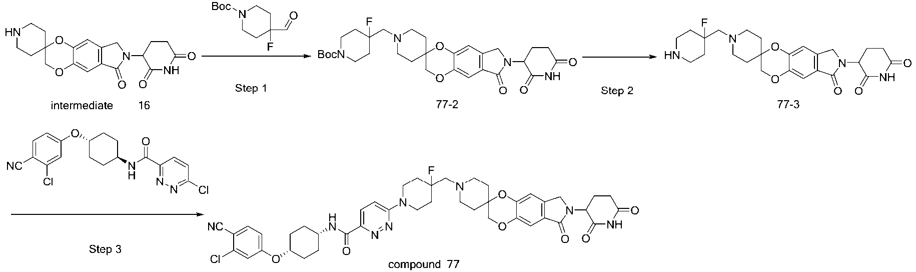

Step 1: The intermediate 16 (80 mg, 0.215 mmol) and tert-butyl 4-fluoro-4-formylpiperidine-1-carboxylate (99.6 mg, 0.43 mmol) and Ti(O-iPr)₄ (122.6 mg, 0.43 mmol) were added sequentially in a glass vial, and the solvent THF (1.5 mL) was added dropwise, and the reaction solution was stirred at 60 °C for 1.5 h. Then NaBH₃CN (40.6 mg, 0.645 mmol) was added, and the reaction solution was continued bo be stirred at 60 °C for 1 h. After the reaction was completed, the reaction solution was concentrated, and then purified by TLC (DCM:MeOH=8:1) to obtain a white solid compound 77-2 (53.8 mg).

Step 2: (Compound 77-3) was prepared with reference to step 2 of Example 19.

Step 3: (Compound 77) was prepared with reference to step 3 of Example 25.
¹H NMR (600 MHz, MeOD) δ 7.98 (d, *J =* 9.6 Hz, 1H), 7.71 (d, *J =* 8.8 Hz, 1H), 7.43 (d, *J =* 9.6 Hz, 1H), 7.35 (s, 1H), 7.23 - 7.18 (m, 2H), 7.06 (dd, *J =* 8.8, 2.3 Hz, 1H), 5.13 (dd, *J =* 13.3, 5.1 Hz, 1H), 4.58 - 4.35 (m, 5H), 4.16 (s, 2H), 4.01 (d, *J =* 4.1 Hz, 1H), 3.75 - 3.43 (m, 8H), 2.96 - 2.88 (m, 1H), 2.83 - 2.76 (m, 1H), 2.68 (s, 1H), 2.49 (ddd, *J* = 26.5, 13.3, 4.5 Hz, 1H), 2.26 - 2.06 (m, 10H), 2.04 - 1.88 (m, 2H), 1.72 - 1.62 (m, 4H).
LC-MS(ESI): [M+H]⁺ = 841.73

### Example 96

### Synthesis of compound 78

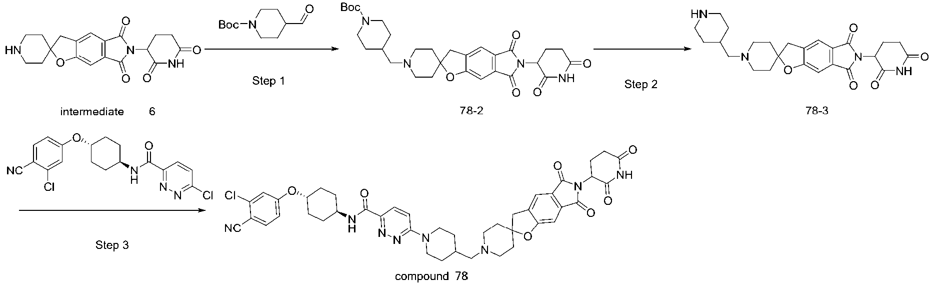

Step 1: The intermediate 6 (80 mg, 0.216 mmol) and tert-butyl 4-formylpiperidine-1-carboxylate (92.4 mg, 433 mmol) were added sequentially in a glass vial, and the solvents DCM (2 mL) and AcOH (2 drops) were added dropwise, and the reaction solution was stirred at room temperature for 1 h, then NaBH(OAc)₃ (138 mg, 648 mmol) was added, and the reaction solution was continued to be stirred at room temperature for 1 h. After the reaction was completed, the reaction solution was concentrated, and then purified by TLC (DCM:MeOH=10:1) to obtain a white solid compound 78-2 (67.3 mg).

Step 2: (Compound 78-3) was prepared with reference to step 2 of Example 19.

Step 3: (Compound 78) was prepared with reference to step 3 of Example 25.
¹H NMR (600 MHz, MeOD) δ 7.99 (d, *J =* 9.7 Hz, 1H), 7.71 (d, *J =* 8.8 Hz, 1H), 7.63 (s, 1H), 7.47 (d, *J =* 9.7 Hz, 1H), 7.28 (s, 1H), 7.21 (d, *J =* 2.3 Hz, 1H), 7.06 (dd, *J =* 8.8, 2.3 Hz, 1H), 5.11 (dd, *J =* 12.8, 5.4 Hz, 1H), 4.62 - 4.49 (m, 3H), 4.05 - 3.96 (m, 1H), 3.75 (dd, *J =* 59.4, 9.9 Hz, 2H), 3.25 - 3.03 (m, 6H), 2.94 - 2.60 (m, 8H), 2.39 - 2.28 (m, 1H), 2.27 - 1.97 (m, 8H), 1.66 (dd, *J =* 13.0, 7.1 Hz, 4H), 1.42 (ddd, *J =* 77.8, 40.4, 18.7 Hz, 3H).
LC-MS(ESI): [M+H]⁺ = 821.68

### Example 97

### Synthesis of compound 79

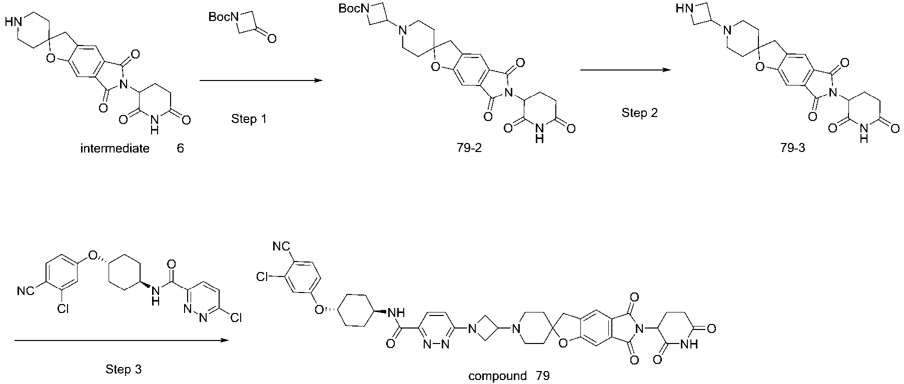

Step 1: The intermediate 6 (60 mg, 0.162 mmol) and tert-butyl 3-oxoazetidine-1-carboxylate (41.71 mg, 0.243 mmol) were added sequentially to a glass vial, and the solvents THF (2 mL) and Ti(O-iPr)₄(92.33 mg, 0.324 mmol) were added, and the reaction solution was stirred at 60 °C for 1 h, then NaBH₃CN (30.62 mg, 0.487 mmol) was added and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 79-2 (30 mg).
LC-MS: [M+H]⁺ =525.43

Step 2: (Compound 79-3) was prepared with reference to step 2 of Example 49.
LC-MS: [M+H]⁺ =425.43

Step 3: (Compound 79) was prepared with reference to step 3 of Example 25.
¹H NMR (400 MHz, Methanol-d₄) δ 7.97 (d, *J =* 8.4 Hz, 1H), 7.69 (d, *J =* 8.7 Hz, 1H), 7.60 (s, 1H), 7.25 (s, 1H), 7.20 (d, *J =* 2.4 Hz, 1H), 7.05 (dd, *J =* 8.8, 2.4 Hz, 1H), 6.95 (d, *J =* 8.2 Hz, 1H), 6.48 (s, 1H), 5.34 (s, 1H), 4.42 (s, 3H), 4.24 (s, 2H), 3.98 (s, 1H), 3.89 - 3.58 (m, 3H), 3.04 - 2.51 (m, 9H), 2.27 - 1.96 (m, 6H), 1.63 (q, *J =* 13.6, 11.5 Hz, 4H).
LC-MS: [M+H]⁺ =779.59

### Example 98

### Synthesis of intermediate 39

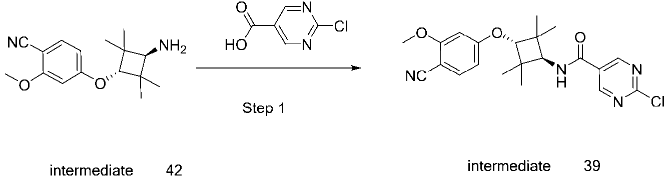

Step 1: The intermediate 42 (100.00 mg, 0.36 mmol), 2-chloropyrimidine-5-carboxylic acid (115.57 mg, 0.73 mmol), T₃P (139.17 mg, 0.44 mmol), and TEA (147.53 mg, 1.46 mmol) were added sequentially to a glass vial, and solvent DCM (3 mL) was added. The reaction solution was stirred at room temperature for 2 h. After the reaction was completed, the reaction solution was concentrated, and then purified by preparative thin-layer chromatography (DCM:MeOH=10:1) to obtain the white solid compound Intermediate 39 (85 mg).
LC-MS: [M+H]⁺ =416.30

### Example 99

### Synthesis of intermediate 40

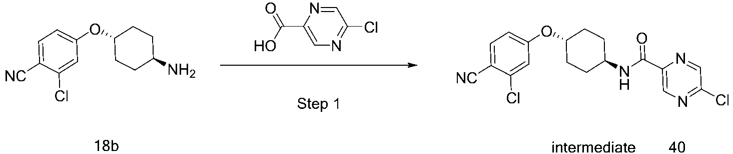

Step 1: Compound 18b (50.00 mg, 0.2 mmol) and 5-chloropyrazine-2-carboxylic acid (37.94 mg, 0.24 mmol), N,N-diisopropylethylamine (77.32 mg, 0.60 mmol), Carter's condensate (90.99 mg, 0.24 mmol) were added sequentially to a glass vial, and then solvent DMF (2 mL) was added, and stirred at room temperature for 2 h. After the reaction was completed, the reaction solution was concentrated, and then purified to obtain the white solid compound intermediate 40 (44 mg).
LC-MS: [M+H]⁺ =391.19

### Example 100

### Synthesis of intermediate 41

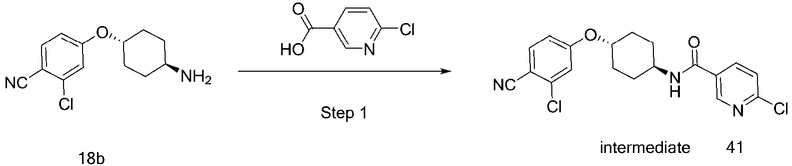

Step 1: Compound 18b (50.00 mg, 0.20 mmol), 5-chloropyridine-2-carboxylic acid (37.94 mg, 0.24 mmol), N,N-diisopropylethylamine (77.32 mg, 0.60 mmol), and Carter's condensate (90.99 mg, 0.24 mmol) were added sequentially to a glass vial, solvent DMF (2 mL) was added and the reaction solution was stirred at room temperature for 2 h. After the reaction was completed, the reaction solution was concentrated, and then purified by preparation to obtain the white solid compound Intermediate 41 (44 mg).
LC-MS: [M+H]⁺ =390.19

### Example 101

### Synthesis of intermediate 42

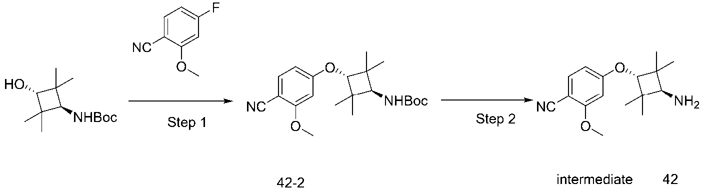

Step 1: In a glass vial, tert-butyl (1r,3r)-3-hydroxy-2,2,4,4-tetramethylcyclobutyl)carbamate (2 g, 8.22 mmol) and the solvent DMF (3 mL) were added, and sodium hydrogen (657.44 mg, 16.44 mmol) was added in batches at 0°C. The reaction solution was stirred at 0°C for 1 h. 4-fluoro-2-methoxybenzyl nitrile (1.37 g, 9.04 mmol) was added, and the reaction solution was continued to be stirred at room temperature for 1 h. After completion of the reaction, the reaction was quenched with water (100 mL), the reaction solution was extracted with ethyl acetate (3x100 mL), and the organic phase was concentrated and purified by normal phase purification to obtain the white solid compound 42-2 (2.5 g).
LC-MS: [M+H]⁺ = 375.49

Step 2: Compound 179-2 (2.5 g, 6.68 mmol), solvent dioxane (20 mL), HCl/dioxane (10 mL) were added to a glass vial and allowed to complete the reaction, the reaction solution was concentrated to obtain a white solid compound 179 (2.2 g).
¹H NMR (600 MHz, DMSO-d6) δ 8.48 (d, *J =* 6.0 Hz, 3H), 7.64 (d, *J =* 8.6 Hz, 1H), 6.63 (d, J = 2.2 Hz, 1H), 6.53 (dd, *J* = 8.6, 2.2 Hz, 1H), 4.31 (s, 1H), 3.90 (s, 3H), 3.07 (d, J = 5.4 Hz, 1H) 1.32 (s, 6H), 1.11 (s, 6H).
LC-MS: [M+H]⁺ = 275.37

### Example 102

### Synthesis of intermediate 19

### synthesis scheme

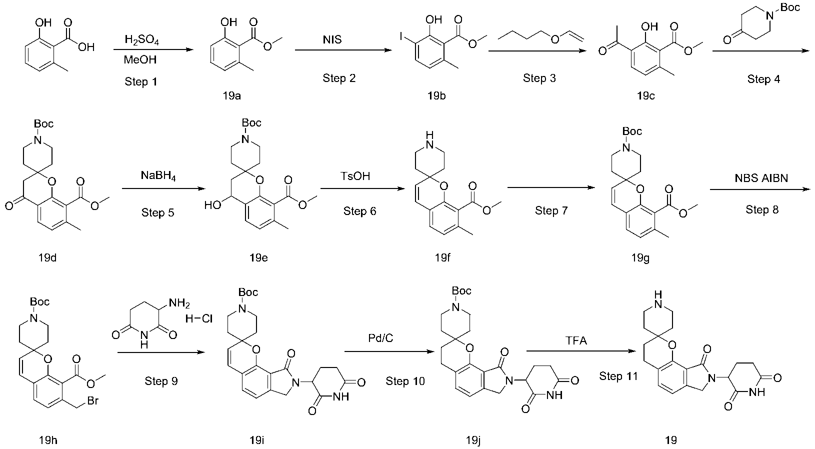

Step 1: Under the protection of nitrogen, 2-hydroxy-6-methylbenzoic acid (10 g, 65.79 mmol) was dissolved in methanol (30 mL), concentrated sulfuric acid (10 mL) was added, and the reaction solution was carried out at 80 °C for 16 h. After completion of the reaction indicated by TLC, the excess of concentrated sulfuric acid was neutralized with 1 m/L NaOH, washed with water, and extracted by ethyl acetate, and the organic phases were combined and concentrated to obtain intermediate 19a (10 g, 91.6%), which could be directly used in the next step without purification.
¹H NMR (600 MHz, CDCl₃) δ 11.31 (s, 1H), 7.31-7.28 (m, 1H), 6.87-6.86 (m, 1H), 6.75-6.74 (m, 1H), 3.99 (s, 3H), 2.56 (s, 3H).
LC-MS (ESI): [M+H]⁺ = 167.13

Step 2: Under the protection of nitrogen, the intermediate 19a (10 g, 60.24 mmol) was dissolved in trifluoroacetic acid (20 mL), and NIS (16.2 g, 72.00 mmol) was added, the reaction was carried out at room temperature for 16 h. After completion of the reaction indicated by TLC, the reaction was quenched with saturated NaS₂O₃ aqueous solution, washed with water, extracted with ethyl acetate, dried with anhydrous sodium sulfate, and concentrated to obtain a crude product, which was purified by reverse-phased column to obtain intermediate 19b (3.8 g, 21.6%).

¹H NMR (600 MHz, CDCl₃) δ 12.19 (s, 1H), 7.77 (d, *J =* 8.0 Hz, 1H), 6.56 (dd, *J =* 8.0, 0.8 Hz, 1H), 4.01 (s, 3H), 2.54 (s, 3H).

Step 3: Under the protection of nitrogen, the intermediate 19b (3.8 g, 13.02 mmol) and butyl vinyl ether (3.9 g, 39.00 mmol) were dissolved in methanol (20 ml), and then Pd(dppf)Cl₂(951 mg, 1.30 mmol) and TEA (3.95 g, 39.00 mmol) were added, and the reaction was carried out at 60 °C for 16 h, the mixture was purged with nitrogen three times. After completion of the reaction indicated by TLC, the reaction solution was spun dry, dissolved by adding dichloromethane, washed with 5 M/L HCl, washed with saturated brine, combined with the organic phases, dried with anhydrous sodium sulfate, and concentrated through a column to obtain the intermediate 19c (2.0 g, 73.8%).
¹H NMR (600 MHz, CDCl₃) δ 12.74 (s, 1H), 7.70 (d, *J =* 8.2 Hz, 1H), 6.79 (d, *J =* 8.2 Hz, 1H), 3.97 (s, 3H), 2.64 (s, 3H), 2.38 (s, 3H).
LC-MS (ESI): [M-OCH₃+H]⁺ = 177.14

Step 4: Under the protection of nitrogen, the intermediate 19c (1 g, 4.80 mmol) was dissolved in methanol (30 mL), tetrandrine (340 mg, 4.80 mmol) and N-tert-butyloxycarbonyl-4-piperidone (960 mg, 4.80 mmol) were slowly added to the system, and the reaction was carried out at 70 °C for 12 h. After completion of the reaction indicated by TLC, the system was cooled to room temperature and concentrated under reduced pressure, and then extracted with ethyl acetate and washed with saturated brine after addition of water, and the organic phases were combined and dried with anhydrous sodium sulfate. After purification by column chromatography, the yellow solid intermediate 19d (1.5 g, 80%) was obtained.
¹H NMR (600 MHz, DMSO-d₆) δ 7.72 (d, *J =* 8.0 Hz, 1H), 6.99 (d, *J =* 8.0 Hz, 1H), 3.87 (s, 3H), 3.78 (br, 2H), 2.99 (br, 2H), 2.84 (s, 2H), 2.28 (s, 3H), 1.96-1.78 (m, 2H), 1.59 (td, *J =* 13.3, 4.8 Hz, 2H), 1.40 (s, 9H).
LC-MS (ESI): [M+H]⁺ = 390.35

Step 5: Under the protection of nitrogen, the intermediate 19d (1.5 g, 3.85 mmol) was dissolved in methanol (25 mL), and NaBH₄(340 mg, 7.7 mmol) was added to the reaction system, and the reaction was carried out at room temperature for 1 h. After completion of the reaction indicated by TLC, the resulting reaction system was concentrated under reduced pressure, water was added, and then the reaction system was extracted with ethyl acetate, washed with saturated saline. The organic phases were combined and dried with anhydrous sodium sulfate. The combined organic phase was concentrated to obtain the crude intermediate 19e (1.5 g, 99%).
¹H NMR (600 MHz, DMSO-d₆) δ 7.37 (d, *J =* 7.8 Hz, 1H), 6.80 (d, *J =* 7.9 Hz, 1H), 5.44 (d, *J =* 5.5 Hz, 1H), 4.67 (q, *J* = 6.7 Hz, 1H), 3.80 (s, 3H), 3.76 (br, 2H), 2.97 (br, 2H), 2.17 (s, 3H), 2.06 (dd, *J* = 13.5, 6.2 Hz, 1H), 1.85-1.66 (m, 3H), 1.60-1.47 (m, 2H), 1.41 (s, 9H).
LC-MS (ESI): [M+H]⁺ = 392.40

Step 6: Under the protection of nitrogen, the intermediate 19e (1.5 g, 3.83 mmol) was dissolved in toluene (25 mL), p-toluenesulfonic acid (659.8 mg, 3.83 mmol) was added and the reaction was carried out at 110 °C for 12 h. After completion of the reaction indicated by TLC, the resulting reaction system was concentrated under reduced pressure and the crude intermediate 19f was directly used in the next step ( 850 mg, 81%).
LC-MS (ESI): [M+H]⁺ = 274.25

Step 7: Under the protection of nitrogen, the intermediate 19f (850 mg, 3.11 mmol) was dissolved in dichloromethane (20 mL), and then Boc₂O (1.36 g, 6.22 mmol), TEA (600 mg, 6.22 mmol) were added, and the reaction was carried out at room temperature 25 °C for 1 h. After completion of the reaction indicated by TLC, the reaction system was cooled to room temperature, concentrated under reduced pressure, added with water, extracted with ethyl acetate, washed with saturated brine, and the organic phases were combined and dried with anhydrous sodium sulfate. After purification by column chromatography, a white solid intermediate 19 g (1 g, 86%) was obtained.
¹H NMR (600 MHz, DMSO-d₆) δ 7.07 (d, *J* = 7.6 Hz, 1H), 6.78 (d, *J =* 7.6 Hz, 1H), 6.48 (d, *J =* 9.8 Hz, 1H), 5.72 (d, *J* = 9.8 Hz, 1H), 3.83 (s, 5H), 3.07 (s, 2H), 2.18 (s, 3H), 1.81 (dq, *J =* 14.3, 2.6 Hz, 2H), 1.57 (td, *J =* 13.1, 4.8 Hz, 2H), 1.41 (s, 9H).
LC-MS(ESI): [M+H]⁺ = 374.40

Step 8: Under the protection of nitrogen, the intermediate 19 g (1 g, 2.68 mmol) was dissolved in carbon tetrachloride (20 mL), NBS (620 mg, 3.50 mmol) and AIBN (43 mg, 0.27 mmol) were added to the system, and the reaction was carried out at 85 °C for 12 h. After completion of the reaction indicated by TLC, the reaction system was concentrated under reduced pressure. The concentrated crude intermediate 19h was directly used in the next step (1.2 g, 99%).
LC-MS(ESI): [M+H]⁺ = 452.25

Step 9: Under the protection of nitrogen, the intermediate 19h (1.2 g, 2.65 mmol), DIPEA (1 g, 7.95 mmol) and 3-amino-2,6-piperidinedione hydrochloride (654.9 mg, 3.98 mmol) were dissolved in acetonitrile (30 mL), and the system was reacted at 85 °C for 16 h. After completion of the reaction indicated by TLC, the reaction system was cooled to room temperature and concentrated under reduced pressure, added with water, extracted with ethyl acetate, washed with saturated brine, and the organic phases were combined and dried with anhydrous sodium sulfate. After purification by column chromatography, a gray solid intermediate 19i (400 mg, 32%) was obtained.
¹H NMR (600 MHz, DMSO-d₆) δ 11.00 (s, 1H), 7.31 (d, *J =* 7.5 Hz, 1H), 7.04 (d, *J =* 7.5 Hz, 1H), 6.57 (d, *J* = 9.9 Hz, 1H), 5.79 (d, *J* = 9.9 Hz, 1H), 4.99 (d, *J =* 5.1 Hz, 1H), 4.44 - 4.16 (m, 2H), 3.79 (s, 2H), 3.19 (d, *J =* 22.8 Hz, 4H), 2.87 (ddd, *J =* 18.2, 13.6, 5.4 Hz, 1H), 2.73-2.56 (m, 1H), 2.51 (p, *J =* 1.9 Hz, 1H), 2.06-1.56 (m, 3H), 1.41 (s, 9H).
LC-MS(ESI): [M-Boc+H]⁺ = 368.19

Step 10: Under the protection of nitrogen, the intermediate 19i (400 mg, 0.85 mmol) was dissolved in THF (10 mL) and 10% wet palladium/carbon (250 mg) was added to the system. After purging with hydrogen three times, the reaction was carried out overnight at 50 °C. After completion of the reaction indicated by TLC, the reaction system was filtered, the filter cake was washed three times with methanol (15 mL), the resulting filtrate was spun dry under reduced pressure, and the concentrated crude solid intermediate 19j was directly used in the next step (380 mg, 94%).
LC-MS(ESI): [M-Boc+H]⁺ = 370.29

Step 11: Under the protection of nitrogen, the intermediate 19j (380 mg, 0.80 mmol) was dissolved in dichloromethane (5 mL) and TFA (2 mL) was added to the system. The reaction was carried out at 25 °C for 1 h. After completion of the reaction indicated by TLC, the reaction solution was spun dry under reduced pressure and a yellow solid intermediate 19 (290 mg, 97%) was prepared by pre-HPLC.
¹H NMR (600 MHz, DMSO-d₆) δ 10.98 (s, 1H), 7.34 (d, *J* = 7.7 Hz, 1H), 7.04 (d, *J* = 7.6 Hz, 1H), 4.97 (dd, *J =* 13.3, 5.2, 1H), 4.43 - 4.20 (m, 2H), 3.30-3.08 (m, 4H), 2.97-2.78 (m, 3H), 2.61 (dt, J = 17.1, 3.7 Hz, 1H), 2.61 (dt, J = 17.1, 3.7 Hz) 3.30-3.08 (m, 4H), 2.97-2.78 (m, 3H), 2.61 (dt*, J =* 17.1, 3.7 Hz, 1H), 2.47-2.31 (m, 1H), 2.04-1.88 (m, 5H), 1.80 (tt, *J =* 12.6, 5.0 Hz, 2H).
LC-MS(ESI): [M+H]⁺ = 370.39

### Example 103

### Synthesis of intermediate 20

### synthesis scheme

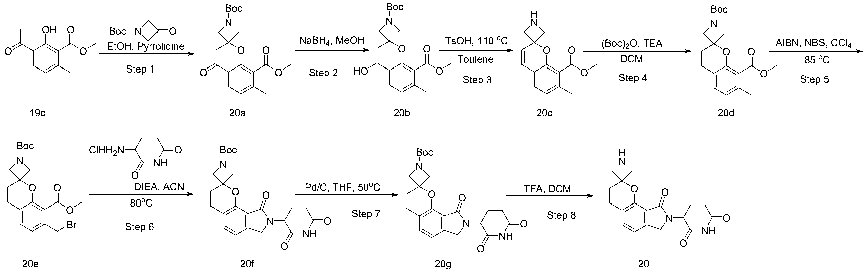

Step 1: Under the protection of nitrogen, the intermediate 19c (2.0 g, 9.61 mmol) was dissolved in ethanol (15 mL), 1-Boc-3-azetidinone (1.63 g, 9.61 mmol) and tetrandrine (682 mg, 9.61 mmol) were added and the reaction was continued at 70 °C for 4 h. The reaction was detected by TLC, 1-Boc-3-azetidinone (0.82 g, 4.81 mmol) and tetrandrine (340 mg, 4.81 mmol) were added additionally, the reaction was continued at 70 °C for 16 h. After completion of the reaction indicated by TLC, the reaction solution was spun dry to obtain the crude product, which was purified through a column to obtain intermediate 20a (1.0 g, 28.8%).
¹H NMR (600 MHz, CDCl₃) δ 7.84 (d, *J =* 8.0 Hz, 1H), 6.96 (d, *J =* 8.0 Hz, 1H), 4.09 (d, *J* = 9.5 Hz, 2H), 3.99 (s, 3H), 3.96 (d, *J =* 9.5 Hz, 2H), 3.06 (s, 2H), 2.39 (s, 3H). 3H), 1.46 (s, 9H).
LC-MS (ESI): [M-Boc+H]⁺ = 262.17

Step 2: Under the protection of nitrogen, the intermediate 20a (1.0 g, 2.77 mmol) was dissolved in methanol (15 mL), NaBH₄ (157 mg, 4.16 mmol) was added, and reacted at room temperature for 2 h. After completion of the reaction indicated by TLC, the reaction was quenched by the addition of saturated NH₄Cl, washed with water, extracted with ethyl acetate, washed with saturated brine, and spun dry to obtain intermediate 20b (900.0 mg, 89.5%), which was directly used in the next step without purification.
LC-MS (ESI): [M-Boc+H]⁺ = 264.17

Step 3: Under the protection of nitrogen, the intermediate 20b (900.0 mg, 2.48 mmol) was dissolved in toluene (10 mL) , and TsOH (471 mg, 2.48 mmol) was added, and the reaction was carried out at 110 °C for 2 h. After completion of the reaction indicated by TLC, the reaction solution was spun dry to obtain the crude intermediate 20c (1.1 g, 181.1%), which could be directly used in the next step without purification.
LC-MS (ESI): [M+H]⁺ = 246.11

Step 4: Under the protection of nitrogen, the intermediate 20c (1.1 g, 1 eq) was dissolved in dichloromethane (15 mL), and (Boc)₂O(1.95 g, 2.0 eq) and TEA (1.36 g mg, 3.0 eq) were added and the reaction was carried out at room temperature for 2 h. After completion of the reaction indicated by TLC, the reaction solution was spun dry and purified through a column to obtain a intermediate 20d (300.0 mg, 19.4%).
¹H NMR (600 MHz, CDCl₃) δ 6.95 (d, *J =* 7.6 Hz, 1H), 6.76 (d, *J =* 7.6 Hz, 1H), 6.45 (d, *J =* 9.8 Hz, 1H), 5.93 (d, *J* = 9.8 Hz, 1H), 4.24 (d, *J* = 9.5 Hz, 2H), 3.99 (d, *J =* 9.5 Hz, 2H), 3.96 (s, 3H), 2.30 (s, 3H), 1.48 (s, 9H).
LC-MS (ESI): [M-Boc+H]⁺ = 256.17

Step 5: Under the protection of nitrogen, the intermediate 20d (400.0 mg, 1 eq) was dissolved in CCl₄ (10 mL) , and AIBN (19 mg, 0.1 eq) and NBS (268 mg, 1.3 eq) were added to the reaction solution, and the reaction was carried out at 85°C for 16 h. After completion of the reaction indicated by TLC, the reaction solution was spun dry to obtain the crude intermediate 20e (600 mg), which could be directly used in the next step without purification.
LC-MS (ESI): [M-Boc+H]⁺ = 324.07

Step 6: Under the protection of nitrogen, the intermediate 20e (600 mg, 1 eq) was dissolved in CAN (10 mL) , 3-aminopiperidine-2,6-dione hydrochloride (287 mg, 1.5 eq) and DIEA (448 mg, 3.0 eq) were added to the reaction solution, and the reaction was carried out at 80°C for 16 h. After completion of the reaction indicated by TLC, the reaction solution was spun dry and purified through a column to obtain a intermediate 20f (60 mg, 21.4% combined yield of two steps).
¹H NMR (600 MHz, DMSO-d₆) δ 11.00 (s, 1H), 7.34 (d, *J =* 7.6 Hz, 1H), 7.08 (d, *J =* 7.6 Hz, 1H), 6.67 (d, *J =* 9.9 Hz, 1H), 6.20 (d, *J* = 9.9 Hz, 1H), 4.99 (dd, *J* = 13.2, 5.2 Hz, 1H), 4.37 (d, *J* = 17.6 Hz, 1H), 4.25 (d, *J =* 17.6 Hz, 1H), 4.04-4.01 (m, 4H), 2.93-2.87 (m, 1H), 2.62-2.57 (m, 1H), 2.41-2.33 (m, 1H), 2.00-1.96 (m, 1H), 1.41 (s, 9H).
LC-MS (ESI): [M-Boc+H]⁺ =340.19

Step 7: Under the protection of nitrogen, the intermediate 20f (60.0 mg, 1 eq) was dissolved in THF (10 mL), Pd/C (60 mg, 1 eq) was added, the reaction was purged with hydrogen three times, and the reaction was carried out at 50 °C for 16 h. After completion of the reaction indicated by TLC, the reaction solution was spun dry to obtain the crude intermediate 20g (80 mg), which could be directly used in the next step without purification.
LC-MS (ESI): [M-Boc+H]⁺ = 342.17

Step 8: Under the protection of nitrogen, the intermediate 20g (80 mg, 1 eq) was dissolved in dichloromethane (5 mL), and TFA (1 mL) was added, and the reaction was carried out at room temperature for 2 h. After completion of the reaction indicated by TLC, the reaction solution was spun dry to prepare the product intermediate 20 (15 mg, 24.3% combined yield of two steps) by isolation and lyophilization.
¹H NMR (600 MHz, DMSO-d₆) δ 10.98 (s, 1H), 7.35 (d, *J =* 7.6 Hz, 1H), 7.08 (d, *J =* 7.6 Hz, 1H), 4.97 (dd, *J* = 13.2, 5.2 Hz, 1H), 4.35 (d, *J =* 17.3 Hz, 1H), 4.23 (d, *J =* 17.3 Hz, 1H), 4.14-4.10 (m, 4H), 2.93 - 2.85 (m, 3H), 2.62 - 2.58 (m, 1H), 2.42-2.35 (m, 1H), 2.22 - 2.19 (m, 2H), 1.99-1.95 (m, 1H).
LC-MS (ESI): [M+H]⁺ = 342.29

### Example 104

### Synthesis of intermediate 21

### Synthesis scheme:

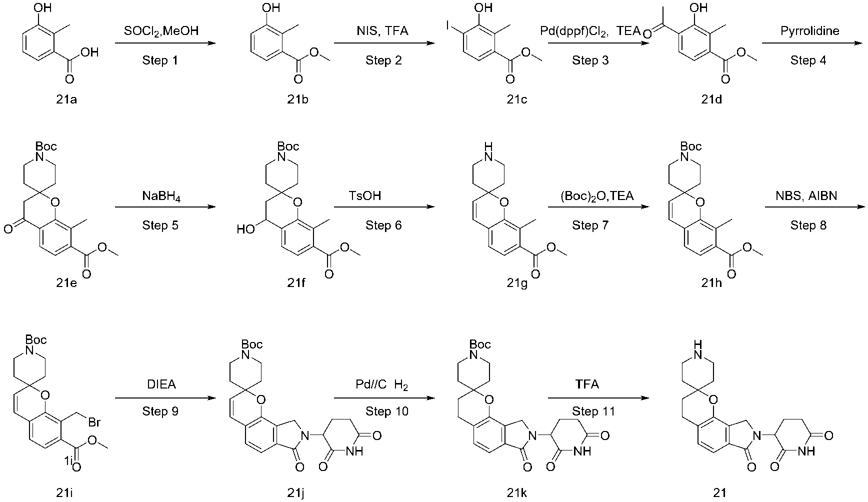

Step 1: Dichlorosulfoxide (15.64 g, 131.45 mmol) was slowly added to a solution of 3-hydroxy-2-methylbenzoic acid 21a (10 g, 65.73 mmol) in methanol (100 mL). The reaction solution was stirred at 80 °C for 2 hours. The reaction solution was spun dry to obtain a gray solid intermediate 21b (10.0 g, 91.56%).
¹H NMR (600 MHz, DMSO-d₆) δ 9.71 (s, 1H), 7.19 (dd, *J* = 7.7, 1.3 Hz, 1H), 7.09 (t, *J =* 7.9 Hz, 1H), 7.02 (dd, *J=* 8.0, 1.4 Hz, 1H), 3.80 (s, 3H), 2.29 (s, 3H).
LC-MS(ESI): [M+H]⁺ = 167.23.

Step 2: N-iodosuccinimide (17.6 g, 78.23 mmol) was slowly added to a solution of 3-hydroxy-2-methylbenzoic acid 21b (10.0 g, 60.18 mmol) in trifluoroacetic acid (80 mL) and methanol (40 mL). The reaction solution was stirred at room temperature for 2 hours. The resulting mixture was concentrated under vacuum and purified by a C18 reversed-phase column to obtain a white solid 21c (4 g, 22.76%).

¹H NMR (600 MHz, DMSO-d₆) *δ* 9.36 (s, 1H), 7.67 (d, *J* = 8.2 Hz, 1H), 7.03 (d, *J =* 8.2 Hz, 1H), 3.80 (s, 3H), 2.38 (s, 3H).

LC-MS(ESI): [M+H]⁺ = 364.22.

Step 3: The intermediate 21c (5.4 g, 18.49 mmol), butyl vinyl ether (5.56 g, 55.47 mmol) and Pd(dppf)Cl₂ (1.35 g, 1.85 mmol) were dissolved in methanol (80 mL) under the protection of nitrogen, and triethylamine (5.61 g, 55.47 mmol) was added. The reaction solution was warmed to 60°C and stirred for 4 h. At the end of the reaction, the mixture was filtered and the filtrate was concentrated to obtain a crude product. The crude product was purified by column chromatography (PE:EA=20%) to obtain a yellow oily product 21d (3 g, 77.93%).

¹H NMR (600 MHz, CD₃OD) δ 7.28 (d, *J =* 8.2 Hz, 1H), 7.18 (d, *J =* 8.3 Hz, 1H), 3.87 (s, 3H), 2.38 (s, 3H), 1.59 (s, 3H).

LC-MS(ESI): [M+H]⁺ = 209.15.

Step 4: The intermediate 21d (3.0 g, 14.41 mmol), N-Boc-4-piperidone (3.16 g, 15.85 mmol) and tetrandrine (1.13 g, 15.85 mmol) were dissolved in methanol (60 mL), and the mixture was stirred at 70 °C overnight. The mixture was spun dry and purified by column chromatography (PE:EA=30%) to obtain a yellow solid 21e (5 g, 89.11%).

¹H NMR (600 MHz, DMSO-d₆) *δ* 7.65 (d, *J =* 8.2 Hz, 1H), 7.33 (d, *J =* 8.2 Hz, 1H), 3.86 (s, 3H), 2.89 (s, 2H), 2.38 (s, 3H), 1.92 (d, *J* = 2.5 Hz, 1H), 1.89 (q, *J* = 2.8 Hz, 1H), 1.64 (td, *J* = 13.1, 4.8 Hz, 2H), 1.43 (s, 4H), 1.40 (s, 9H).

LC-MS(ESI): [M-Boc+H]⁺ = 290.18.

Step 5: Sodium borohydride (0.97 g, 25.68 mmol) was added to a solution of intermediate 21e (5.0 g, 12.84 mmol) in methanol (80 mL) in an ice bath. The reaction solution was stirred at 25°C for 1 hour. The reaction solution was quenched with ammonium chloride solution and extracted with ethyl acetate, and the solvent was spun dry to obtain a yellow oily product 21f (5.5 g) which was used directly in the next step of the reaction.

LC-MS(ESI): [M-Boc+H]⁺ = 292.28.

Step 6: The intermediate 21f (5.5 g, 14.05 mmol) and p-toluenesulfonic acid (2.66 g, 15.45 mmol) were dissolved in toluene (80 mL) and the mixture was stirred at 110 °C for 5 hours. The mixture was concentrated under vacuum to obtain a brown oily product 21 g (3.84 g) which was used directly in the next step.

LC-MS(ESI): [M+H]⁺ = 274.47.

Step 7: The intermediate 21 g (3.84 g, 14.05 mmol) and Boc anhydride (6.13 g, 28.10 mmol) were dissolved in dichloromethane (100 mL) and triethylamine (4.26 g, 442.15 mmol) was added. The reaction solution was stirred at room temperature for 2 hours. At the end of the reaction, the mixture was spun dry and purified by column chromatography (PE:EA=25%) to obtain a yellow oily product 21h (5 g, 95.30%).

¹H NMR (600 MHz, CD₃OD) δ 7.37 (d, *J =* 7.9 Hz, 1H), 6.94 (d, *J =* 7.9 Hz, 1H), 6.48 (d, *J =* 9.8 Hz, 1H), 5.77 (d, *J* = 9.7 Hz, 1H), 3.87 (s, 3H), 2.45 (s, 3H), 1.97 - 1.93 (m, 2H), 1.66 (td, *J =* 13.9, 12.2, 4.8 Hz, 2H), 1.58 (s, 2H), 1.53 (s, 2H), 1.49 (s, 9H).

LC-MS(ESI): [M-Boc+H]⁺ = 274.17.

Step 8: 21h (3.3 g, 8.84 mmol), N-bromosuccinimide (1.89 g, 10.60 mmol) and azobisisobutyronitrile (145.11 mg, 883.65 umol) were dissolved in carbon tetrachloride (50 mL). The reaction solution was stirred at 80 °C overnight. The reaction solution was concentrated under vacuum to obtain a brown solid 21i (4 g) which was used directly in the next step of the reaction.

LC-MS(ESI): [M+H]⁺ = 316.08.

Step 9: The intermediate 21i (4 g, 8.84 mmol) was dissolved in a solution of acetonitrile (100 mL), 3-aminopiperidine-2,6-dione hydrochloride (2.18 g, 13.26 mmol) and N,N-diisopropylethylamine (3.43 g, 26.53 mmol) were added and the reaction solution was stirred at 80°C overnight. After completion of the reaction, the mixture was purified by normal phase column (100% EA) to obtain a blue solid 21j (1 g, 24.19%).

LC-MS(ESI): [M-tBu+H]⁺ = 412.29.

Step 10: The intermediate 21j (90 mg, 192.50 umol) was dissolved in a solution of tetrahydrofuran (5 mL), palladium carbon (20 mg) was added, the mixture was purged with hydrogen three times, and the reaction solution was stirred at 50 °C overnight. Upon completion of the reaction, the mixture was filtered to obtain a white solid 21k (90 mg, 99.57%).

LC-MS(ESI): [M-tBu+H]⁺ = 414.39.

Step 11: The intermediate 21k (90 mg, 191.68 umol) was dissolved in a solution of dichloromethane (2 mL), trifluoroacetic acid (1 mL) was added, and the reaction solution was stirred at room temperature for 2 hours. After completion of the reaction, it was spun dry and purified by preparative liquid phase to obtain a white solid 21 (50 mg, 70.61%).
¹H NMR (400 MHz, CD₃OD) δ 7.36 (d, *J =* 7.8 Hz, 1H), 7.32 (d, *J =* 7.8 Hz, 1H), 5.18 (dd, *J =* 13.3, 5.2 Hz, 1H), 4.55 - 4.40 (m, 2H), 3.38 (dd, *J =* 12.5, 3.3 Hz, 4H), 3.03 - 2.89 (m, 3H), 2.81 (m, *J =* 17.6, 4.6, 2.4 Hz, 1H), 2.51 (m, *J =* 13.3, 4.7 Hz, 1H), 2.24 - 2.09 (m, 3H), 2.02 (t, *J =* 6.8 Hz, 2H), 1.98 - 1.87 (m, 2H).
LC-MS(ESI): [M+H]⁺ = 370.39

### Example 105

### Synthesis of intermediate 22

### Synthesis scheme:

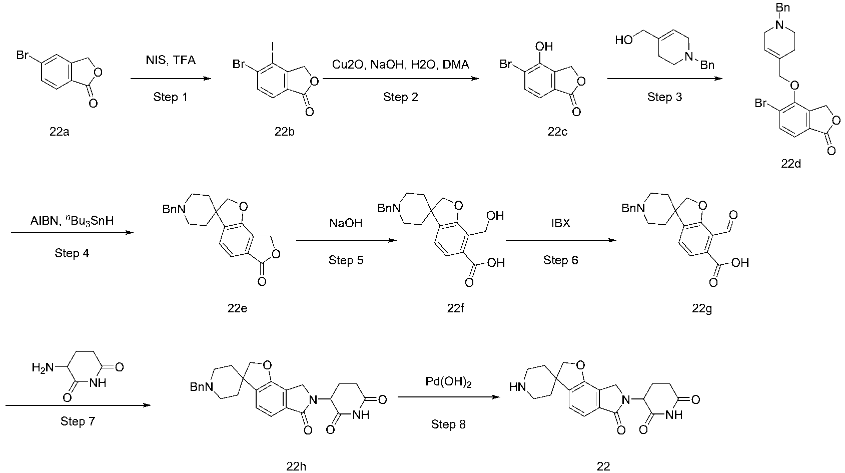

Step 1:22a (1 g, 4.69 mmol) was dissolved in trifluoromethanesulfonic acid (10 mL), the solution was protected with nitrogen and cooled down to 0 °C. N-iodosuccinimide (1.16 g, 5.16 mmol) was added to the reaction solution at 0 °C. The reaction solution was brought to room temperature and reacted overnight. The resulting mixture was poured into ice water and filtered. The filter cake was washed with water and purified by column chromatography (EA:PE=0%-15%) to obtain a white solid 22b (800 mg, 50.28%).

¹H NMR (600 MHz, DMSO-d₆) δ 7.94 - 7.91 (m, 1H), 7.78 (dd, *J =* 8.1, 1.1 Hz, 1H), 5.21 (s, 2H).

LC-MS(ESI): [M-H]⁻= 230.86.

Step 2: The solution of sodium hydroxide (413.04 mg, 10.33 mmol) in water (7 mL) was added to a solution of intermediate 22b in N,N-dimethylaniline (3.5 mL),. Cuprous oxide (59.11 mg, 413.07 µmol) was added to the above solution. The reaction solution was warmed to 80 °C and stirred overnight. The reaction solution was neutralized with 1N hydrochloric acid to neutral state, extracted with ethyl acetate and the organic phase was washed with saturated brine. The organic phase was concentrated under vacuum and the resulting mixture was purified by column chromatography (EA:PE=0%-35%) to obtain a white solid 22c (249 mg, 52.64%).

¹H NMR (600 MHz, DMSO-d₆) *δ* 10.91 (s, 1H), 7.73 (d, *J* = 8.0 Hz, 1H), 7.24 (d, *J* = 8.0 Hz, 1H), 5.35 (s, 2H).

LC-MS(ESI): [M-H]⁻= 230.86.

Step 3: The intermediate 22c (100 mg, 436.63 µmol), (1-benzyl1,2,3,6-tetrahydropyridin-4-yl)methanol (106.51 mg, 523.95 µmol) were dissolved in tetrahydrofuran (1.0 mL) under the protection of nitrogen, and the system was cooled down to 0 °C, then triphenylphosphine (229.05 mg, 873.25 µmol) was added, and diethyl azodicarboxylate (152.08 mg, 873.25 µmol) was added slowly. The reaction solution was warmed to room temperature and stirred overnight. The mixture was purified by preparative column chromatography to obtain ayellow oily product 22d (105 mg, 58.05%).

¹H NMR (600 MHz, DMSO-d₆) δ 7.83 (d, *J =* 8.0 Hz, 1H), 7.45 (d, *J =* 8.0 Hz, 1H), 7.37 - 7.30 (m, 4H), 7.29 - 7.19 (m, 1H), 5.84 (s, 1H), 5.68 (s, 2H), 4.64 (s, 2H), 3.56 (s, 2H), 2.93 (s, 2H), 2.57 (t, *J =* 5.7 Hz, 2H), 2.25 (s, 2H).LC-MS(ESI): [M+H]⁺ = 414.20.

Step 4: The intermediate 22d (100 mg, 241.37 µmol), azobisisobutyronitrile (79.27 mg, 482.74 µmol) and tri-n-butyltin hydride (210.77 mg, 724.11 µmol) were dissolved in toluene (1.0 mL), and the mixture was stirred at 110°C overnight. The reaction solution was concentrated under vacuum and purified by C18 reversed-phase column to obtain a yellow oily product 22e (27 mg, 33.35%).

¹H NMR (600 MHz, CDCl₃) δ 7.56 (d, *J =* 7.7 Hz, 1H), 7.52 - 7.46 (m, 6H), 5.26 (s, 2H), 4.54 (s, 2H), 4.29 (s, 2H), 2.73 (t, *J =* 13.1 Hz, 2H), 2.60 (td, *J =* 14.6, 4.0 Hz, 2H), 2.12 - 1.91 (m, 4H).

LC-MS(ESI): [M-H]⁻= 336.30.

Step 5: The solution of sodium hydroxide (120.44 mg, 3.01 mmol) in water (2.0 mL) was added to a solution of intermediate 22e (202 mg, 602.26 µmol) in tetrahydrofuran (2.0 mL). The reaction solution was stirred at room temperature overnight. The resulting mixture was concentrated under vacuum and purified by reversed-phase column to obtain a white solid 22f (98 mg, 46.04%).

LC-MS(ESI): [M-H]⁻= 354.59.

Step 6: The intermediate 22f (20 mg, 56.59 µmol) and IBX (31.69 mg, 113.18 µmol) were dissolved in tetrahydrofuran and dimethylsulfoxide = 20:1 (0.5 mL), and the mixture was stirred at 80 °C for 2 h. The reaction solution was filtered and the resulting mixture was concentrated under vacuum, and the crude was used directly in the next step without purification.

LC-MS(ESI): [M+H]⁺ = 352.30.

Step 7: Compound 22g (48 mg, 136.60 µmol) and 3-aminopiperidine-2,6-dione hydrochloride (44.96 mg, 273.19 µmol) were dissolved in tetrahydrofuran and dimethylsulfoxide = 20:1 (1.0 mL) and a drop of N,N-diisopropylethylamine was added. The reaction solution was stirred at 50 °C for 1 h. The mixed solution was cooled down to room temperature, and sodium triacetoxyborohydride (86.85 mg, 409.79 µmol) and a drop of acetic acid were added and the reaction solution was warmed up to 50 °C for 1 h. The mixture was concentrated under vacuum and purified by reversed-phase HPLC to obtain a white solid 22h (16 mg, 26.29%).

¹H NMR (600 MHz, DMSO-d₆) δ 11.00 (s, 1H), 7.46 - 7.56 (m, 5H), 7.34 (d, *J =* 7.6 Hz, 1H), 7.26 (d, *J =* 7.7 Hz, 1H), 5.09 (dd, *J =* 13.3, 5.1 Hz, 1H), 4.67 (t, *J =* 6.3 Hz, 2H), 4.43 - 4.34 (m, 3H), 4.24 (d, *J =* 17.1 Hz, 1H), 3.16 (d, *J =* 14.4 Hz, 2H), 2.91 (ddd, *J =* 18.0, 13.7, 5.4 Hz, 1H), 2.64 - 2.56 (m 1H), 2.44 (td, *J =* 13.2, 4.6 Hz, 2H), 2.12 (d, *J =* 15.4 Hz, 2H), 2.03 - 1.89 (m, 4H).

LC-MS(ESI): [M+H]⁺ = 446.35.

Step 8: The intermediate 22h (150 mg, 336.69 µmol) was dissolved in a solution of hexafluoroisopropanol (4.5 mL), 20% palladium/carbon hydroxide (75 mg) was added, hydrogen substitution was performed for three times and the reaction solution was stirred at room temperature for 5 hours under hydrogen. After completion of the reaction, the reaction solution was filtered, concentrated under reduced pressure and purified by reversed-phase HPLC to obtain a white solid (35 mg, 29.25%).

¹H NMR (600 MHz, CD₃OD) δ 7.46 - 7.40 (m, 2H), 5.16 (dd, *J =* 13.4, 5.2 Hz, 1H), 4.70 (s, 2H), 4.52 - 4.40 (m, 2H), 3.54 - 3.47 (m, 2H), 3.24 - 3.14 (m, 2H), 2.96 - 2.87 (m, 2H), 2.80 (m, *J =* 17.7, 4.6, 2.2 Hz, 1H), 2.52 (m, *J* = 13.3, 4.6 Hz, 1H), 2.25 - 2.16 (m, 3H), 2.05 - 2.01 (m, 1H).

LC-MS(ESI): [M+H]⁺ = 356.29.

### Example 106

### Synthesis of intermediate 23

### synthesis scheme

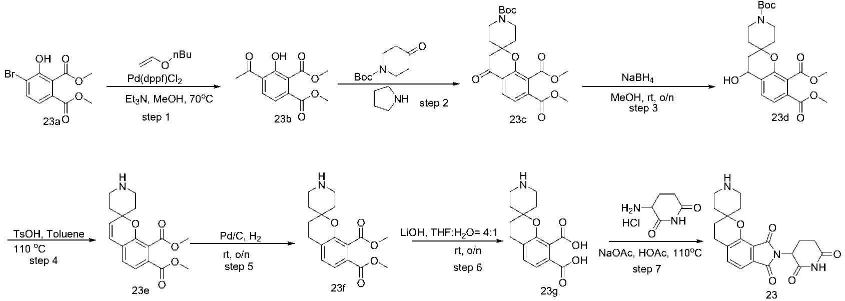

Step 1: Under the protection of nitrogen, dimethyl 4-bromo-3-hydroxyphthalate (10 g, 34.59 mmol) was dissolved in MeOH (100 mL), and 1-(vinyloxy)butane (13.86 g, 138.87 mmol), Pd(dppf)Cl_{2 (}2.53 g, 3.46 mmol) and triethylamine ( 10.50 g, 103.78 mmol) were added. The reaction solution was stirred at 70 °C for 12 h. At the end of the reaction, HCl-1,4-dioxane (100 mL) was added and stirred at room temperature for 30 min. Then, it was extracted three times with EA, the organic phases were combined, washed with saturated sodium chloride solution, dried with anhydrous sodium sulfate and concentrated to obtain a yellow oily crude product compound 23b (8 g, 91.6%), which was directly used in the next step.
LC-MS(ESI): [M-32+H]⁺ = 211.06

Step 2: The intermediate 23b (8.0 g, 31.72 mmol), N-Boc-4-piperidone (7.72 g, 31.72 mmol) and tetrahydropyrrole (2.26 g, 31.72 mmol) were dissolved in 80 mL of ethanol and the reaction was carried out at 85 °C for 12 h. The reaction was completed and the reactdion solution was purified by column chromatography (EA:PE =29%) to obtain a yellow oily intermediate 23c (4.3 g, 31.28%).
¹H NMR (600 MHz, CDCl₃) δ 7.93 (d, *J =* 8.2 Hz, 1H), 7.68 (d, *J =* 8.2 Hz, 1H), 4.00 (s, 3H), 3.95 (s, 3H), 3.30 (t, *J* = 9.7 Hz, 2H), 3.17 (t, *J =* 9.7 Hz, 2H), 3.14 (s, 2H). 2H), 2.47 (t, *J =* 9.7 Hz, 2H), 2.06 (t, *J =* 9.7 Hz, 2H).
LC-MS(ESI): [M+H]⁺ = 334.19

Step 3: The intermediate 23c (4.30 g, 9.92 mmol) was dissolved in 40 mL of MeOH, sodium borohydride (1.13 g, 29.76 mmol) was added in an ice bath and stirred overnight. At the end of the reaction, it was concentrated, water was added, and the reaction solution was extracted by ethyl ester and spun dryto obtain a crude yellow oily product intermediate 23d (2.8 g, 64.81%). It was used directly in the next step.
LC-MS (ESI): [M+H]⁺ = 336.29

Step 4: The intermediate 23d (2.80 g, 6.43 mmol) was dissolved in 3 mL of toluene, p-toluenesulfonic acid (2.21 g, 12.86 mmol) was added, and the reaction was carried out at 110 °C at reflux overnight. At the end of the reaction, it was concentrated, water was added, and the reaction solution was extracted by ethyl ester, spun dry and purified by column chromatography to obtain a yellow oily intermediate 23e (1.4 g, 68.6%).
LC-MS(ESI): [M+H]⁺ = 318.38

Step 5: The intermediate 23e (1.40 g, 4.41 mmol) was dissolved in MeOH, Pd/C (700 mg) was added and the reaction solution was stirred under hydrogen for 4 h after hydrogen substitution. At the end of the reaction, a yellow oily compound intermediate 23f (1.3 g, 93.8%) was obtained by filtration and concentration, and the crude product was directly used in the next step.
LC-MS(ESI): [M+H]⁺ = 320.58

Step 6: Compound 23f (1.4 g, 4.38 mmol) was dissolved in a mixture of THF: H₂O (4:1), LiOH (629.87 mg, 26.30 mmol) was added and stirred at room temperature overnight. The reaction solution was concentrated under reduced pressure to obtain a yellow oily crude product 23f (1.35 g, 96.84%).
LC-MS (ESI): [M+H]⁺ = 306.58

Step 7: Compound 23g (1.50 g, 4.91 mmol), 3-aminopiperidine-2,6-dione hydrochloride (970.30 mg, 5.90 mmol) and sodium acetate (806.02 mg, 9.83 mmol) were dissolved in acetic acid (5 mL). The mixture was stirred and reacted at 110 °C for 5 h. At the end of the reaction, filtration was carried out and the filtrate was concentrated under reduced pressure and purified by prep-HPLC (PhaseA=water, PhaseB=ACN, 0.1% TFA, B%=0%~50% in 30 min) to obtain a white solid compound 23 (0.55 g, 31.3%).
¹H NMR (600 MHz, DMSO-d₆) δ 11.11 (s, 1H), 7.62 (d, *J =* 7.5 Hz, 1H), 7.39 (d, *J =* 7.4 Hz, 1H), 5.08 (dd, *J* = 12.9, 5.5 Hz, 1H), 3.28 (t, *J =* 11.8 Hz, 2H), 3.09 (t, *J =* 11.8 Hz, 2H), 2.96 (t, *J =* 12,4 Hz, 2H), 2.91 - 2.78 (m, 3H), 2.63 - 2.52 (m, 3H), 2.05 -1.94 (m, 3H), 1.91 - 1.81 (m, 2H).
LC-MS (ESI): [M+H]⁺ = 384.19

### Example 107

### Synthesis of intermediate 24

### synthesis scheme

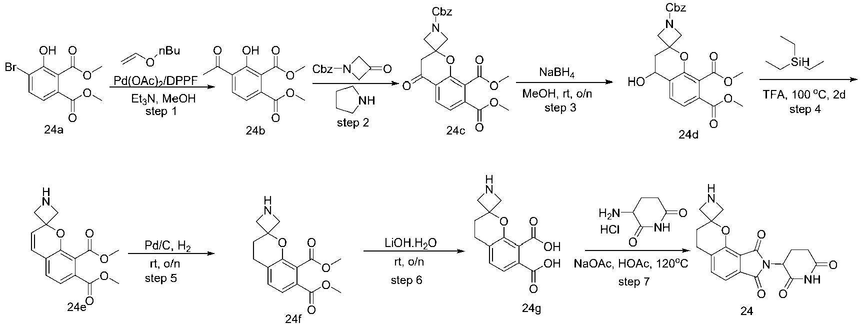

Step 1: Under the protection of nitrogen, dimethyl 4-bromo-3-hydroxyphthalate (2.40 g, 8.30 mmol), butyl vinyl ether (4.15 g, 41.5 mmol ), [1,1'-bis(diphenylphosphino)ferrocene]palladium dichloride (610 mg, 834.47 mmol) and triethylamine (2.53 g, 25.04 mmol) were dissolved in methanol and stirred at 70°C overnight in an oil bath. After the reaction was completed, the reaction solution was spun dry and the crude product was purified by silica gel column (EA/PE=28%) to synthesize a yellow oily intermediate 24b ( 418 mg, 20.3%).
¹H NMR (600 MHz, DMSO-d₆) *δ* 10.70 (s, 1H), 7.48 (d, *J =* 7.5 Hz, 1H), 7.12 (d, *J =* 7.4 Hz, 1H), 3.88 (s, 3H), 3.87 (s, 3H), 2.42 (s, 3H).
LC-MS(ESI): [M-32+H]⁺ = 221.16

Step 2: Compound 24b (325 mg, 1.58 mmol) was dissolved in methanol, then pyrrolidine (125 mg, 1.76 mmol) was added and stirred at 80°C in an oil bath overnight. After the reaction was completed, the reaction solution was spun dry, and the crude product was isolated and purified by silica gel columns (EA/PE=30%) to obtain 24c (310 mg, 44.5%), which was a yellow oily intermediate with a purity with >90% purity.
¹H NMR (600 MHz, CDCl₃) δ 7.99 (dd, *J =* 8.2, 0.9 Hz, 1H), 7.71 (dd, *J =* 8.2, 1.0 Hz, 1H), 7.42 - 7.32 (m, 5H), 5.13 (s, 2H), 4.20 (d, *J =* 9.7 Hz, 2H), 4.06 (d, J = 9.7 Hz, 2H), 4.00 (s, 3H), 3.95 (s, 3H), 3.14 (s, 2H).
LC-MS(ESI): [M+H]⁺ = 438.20

Step 3: Compound 24c (310 mg, 705.48 umol) was dissolved in methanol and sodium borohydride (80 mg, 2.11 mmol) was added batchwise in an ice-water bath and stirred at room temperature for 3 hours. After the reaction was completed, the reaction solution was spun dry and the crude product was purified by silica gel column (DCM/MeOH=5%) to obtain a yellow oily intermediate 24d (180 mg, 57%).
¹H NMR (600 MHz, DMSO-d₆) δ 7.63 (d, *J =* 7.5 Hz, 1H), 7.44 (d, *J =* 7.0 Hz, 1H), 7.42 - 7.30 (m, 5H), 5.11 (s, 2H), 4.50 (s, 1H), 4.25 -4.15 (m, 1H), 4.10 (d, *J =* 9.7 Hz, 4H), 3.88 (s, 3H), 3.86 (s, 3H), 2.60 (d, *J =* 17.6, 2H).
LC-MS(ESI): [M+H]⁺ = 442.29

Step 4: Compound 24d (180 mg, 407.76 umol) was dissolved in trifluoroacetic acid, then triethylsilane (330 mg, 2.84 mmol) was added and stirred at 100°C in an oil bath for 6 hours, then cooled to room temperature. Then another batch of triethylsilane (330 mg, 2.84 mmol) was added, and after the reaction was completed, the reaction solution was spun dry, the crude product was dissolved in methanol and stirred for 1 h, then spun dry, and supernatant was poured off, and the lower layer was the product, which was a yellow oily intermediate 24e (160 mg, 96.6%) and used for the next step without purification.
LC-MS(ESI): [M+H]⁺ =289.98

Step 5: Compound 24e (160 mg, 409.28 umol) was dissolved in methanol under the protection of nitrogen, Pd/C (50 mg, 10%) was added, followed by hydrogen substitution, and stirred overnight at room temperature under hydrogen. At the end of the reaction, the reaction solution was filtered and the filtrate was spun dry to obtain ayellow oily compound 24f (130 mg, 81.3%), which was used in the next step without purification.
LC-MS(ESI): [M+H]⁺ =292.18

Step 6: Compound 24f (130 mg, 410.57 umol) was dissolved in a solvent mixture of tetrahydrofuran/methanol/water=1:1:5 and lithium hydroxide (150 mg, 6.26 mmol) was added to the mixture in an ice-water bath, and stirred at room temperature overnight. At the end of the reaction, the pH was adjusted to 7 with aqueous hydrochloric acid and the filtrate was spun dry to obtain a yellow oily product 24 g (140 mg, 105%), which was used in the next step without purification.
LC-MS(ESI): [M+H]⁺ =264.17

Step 7: Compound 24g (200 mg, 1.22 mmol) and sodium acetate (200 mg, 2.44 mmol) were dissolved in acetic acid and stirred at 120 degrees Celsius overnight in an oil bath; after being spun dry, it was dissolved in methanol, and purified by reversed-phase purification, ACN/H₂O (0-20% for 40 min) to obtain the white solid intermediate 24 (80 mg, 18.4%).
¹H NMR (600 MHz, *DMSO-d*₆) δ 11.13 (s, 1H), 7.63 (d, *J =* 7.5 Hz, 1H), 7.44 (d, *J =* 7.0 Hz, 1H), 5.09 (dd, *J =* 12.9, 5.5 Hz, 1H), 4.15 (s, 4H), 2.96 (t, *J =* 6.5 Hz, 2H), 2.94-2.87 (m, 1H), 2.60-2.54 (m, 2H), 2.31 - 2.20 (m, 2H), 2.03-1.94 (m, 1H).
LC-MS(ESI): [M+H]⁺ = 356.30

### Example 108

### Synthesis of intermediate 25

### synthesis scheme

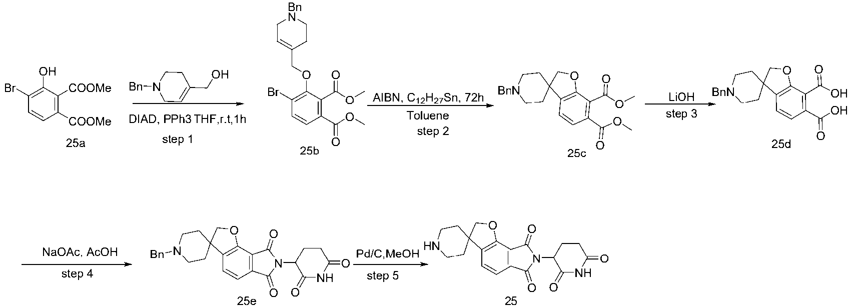

Step 1: Dimethyl 4-bromo-3-hydroxyphthalate (1.80 g, 6.23 mmol) was dissolved in tetrahydrofuran (10 mL) under the protection of nitrogen. (1-benzyl 1,2,3,6-tetrahydropyridin-4-yl) methanol (1.90 g, 9.34 mmol) and triphenylphosphine (3.27 g, 12.45 mmol) were added, and then azo Diisopropyl azodicarboxylate (2.52 g, 12.45 mmol) was added slowly. After the addition, the reaction solution was stirred at room temperature for 2 hours. At the end of the reaction, the solvent was removed under vacuum and the crude product was purified by column chromatography (H2O:ACN=0 ~30%) to obtain a yellow oily target compound (2.10 g, 71.10 %).
¹H NMR (600 MHz, DMSO-d₆) δ 7.95 (d, *J =* 8.4 Hz, 1H), 7.70 (d, *J =* 8.4 Hz, 1H), 7.58 - 7.45 (m, 5H), 5.87 - 5.83 (m, 1H), 4.45 (s, 2H), 3.84 (s, 3H), 3.83 (s, 3H), 3.81 (s, 2H), 3.47 - 3.30 (m, 2H), 3.25 - 3.10 (m, 2H), 2.47 - 2.34 (m, 2H).
LC-MS(ESI): [M+H]⁺ = 474.09

Step 2: Compound 25b (2.10 g, 4.43 mmol) was dissolved in toluene (25 mL) under the protection of nitrogen, and azobisisobutyronitrile (2.18 g, 13.28 mmol) and tri-n-butyltin hydroxide (2.58 g, 8.85 mmol) were added. The reaction solution was stirred at 110°C for 4 hours. At the end of the reaction, the solvent was removed under vacuum and the crude product was purified by column chromatography (H2O:ACN=0 ~30%) to obtain a yellow oily target compound (1.50 g, 85.68 %).
¹H NMR (600 MHz, *DMSO-d*₆) δ 7.55-7.47 (m, 5H), 7.46 (d, *J =* 8.4 Hz, 1H), 7.31 (d, *J =* 8.4 Hz, 1H), 4.65 (s, 2H), 4.35 (s, 2H), 3.80 (s, 3H), 3.78 (s, 3H), 3.43 - 3.30 (m, 2H), 3.15 - 3.06 (m, 2H), 2.17 - 2.07 (m, 2H), 2.01 - 1.94 (m, 2H).
LC-MS(ESI): [M+H]⁺ =360.29

Step 3: The intermediate 25c (1.50 g, 3.79 mmol) was dissolved in a mixture of methanol and water (3:1) (15 ml), and then lithium hydroxide (454.17 mg, 18.97 mmol) was added. The reaction solution was stirred at 50°C for 2 hours. At the end of the reaction, the filtrate was concentrated to obtain a crude product, i.e. a yellow solid (1.35 g) which could be used in the next step of the reaction without purification.
LC-MS(ESI): [M+H]⁺ =367.99

Step 4: The intermediate 25d (1.35 g, 3.67 mmol) was dissolved in acetic acid (12 mL) and 3-aminopiperidine-2,6-dione hydrochloride (1.21 g, 7.35 mmol) and sodium acetate (1.21 g, 14.70 mmol) were added. The reaction solution was stirred at 115°C overnight. At the end of the reaction, it was concentrated under vacuum and the crude product was purified by column chromatography (H₂O:ACN=0~30%) to obtain a purple oily compound (1.52 g, 90.02%).
¹H NMR (600 MHz, *DMSO-d*₆) δ 11.10 (s, 1H), 7.62 (d, *J =* 7.4 Hz, 1H), 7.55-7.47 (m, 5H), 7.43 (d, *J* = 7.3 Hz, 1H), 5.11 (dd, *J =* 13.0, 5.4 Hz, 1H), 4.81 (s, 2H), 3.96 (s, 2H), 3.38 (d, J = 13.0 Hz, 2H), 3.09 - 3.01 (m, 2H), 2.91 - 2.84 (m, 1H), 2.63-2.58 (m, 2H), 2.08 - 2.00 (m, 3H), 1.98 - 1.90 (m, 2H).
LC-MS(ESI): [M+H]⁺ =460.49

Step 5: The intermediate 25e (489 mg, 1.06 mmol) was dissolved in methanol, palladium carbon (180 mg) was added, and hydrogen substitution was performed for twice to three times. The reaction solution was stirred at room temperature for 2 hours. At the end of the reaction, it was filtered through diatomaceous earth and after the organic phase was dried and concentrated, the crude product was purified by column chromatography (H₂O:ACN=15 ~55%) to obtain a white solid target compound (170 mg, 43.25%).
¹H NMR (600 MHz, *DMSO-d*₆) δ 11.12 (s, 1H), 7.61 (d, *J* = 7.4 Hz, 1H), 7.49 (d, *J* = 7.3 Hz, 1H), 5.11 (dd, *J* = 13.0, 5.4 Hz, 1H), 4.81 (s, 2H), 3.38 (d, *J =* 13.0 Hz, 2H), 3.09 - 3.00 (m, 2H), 2.92 - 2.85 (m, 1H), 2.62-2.59 (m, 2H), 2.09 - 2.01 (m, 3H), 1.98 - 1.91 (m, 2H).
LC-MS(ESI): [M+H]⁺ =370.09

### Example 109

### Synthesis of intermediate 26

### synthesis scheme

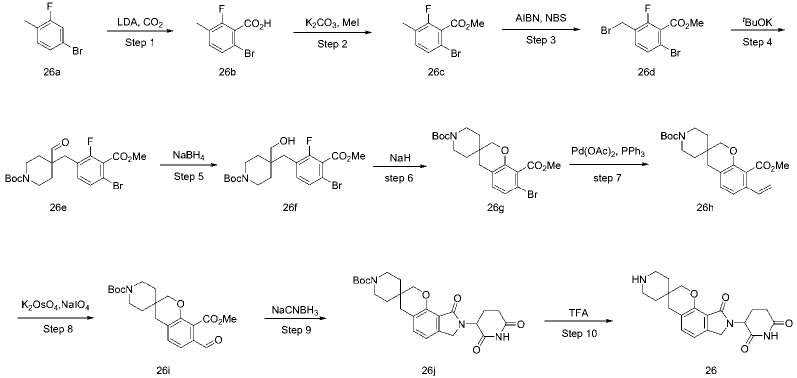

Step 1: Compound 26a (2.0 g, 10.6 mmol) was added into lithium diisopropylammonium (1.3 M in THF/hexane, 6.9 mL, 13 mmol) in tetrahydrofuran (10.6 mL) at -78 °C and reacted for 2 h at this temperature, quickly poured into dry ice. After it was brought to room temperature, the solvent was spun dry to obtain a white solid compound 26b (2.84 g, 115%), which was directly used in the next step.
LC-MS(ESI): [M-H]⁻= 230.95

Step 2: Iodomethane (2.5 g, 17.8 mmol), potassium carbonate (3.7 g, 26.7 mmol) were added to a solution of intermediate 26b (2.1 g, 8.9 mmol) in N,N-dimethylformamide (12 mL). The reaction was carried out at 80°C for 1 hour. At the end of the reaction, water (50 mL) was added, extracted with ethyl acetate (100 mL*3 times), washed with saturated saline (100 mL), and the organic phases were combined and dried with anhydrous sodium sulfate. The combined organic phase was spun dry and purified by column chromatography (PE:EA=0 - 20%) to obtain a yellow oily intermediate 26c (1.9 g, 86%).

¹H NMR (600 MHz, CDCl₃) δ 7.27 (d, *J =* 9.2 Hz, 1H), 7.14 - 7.09 (m, 1H), 3.97 (s, 3H), 2.25 (d, *J =* 1.9 Hz, 3H).

LC-MS(ESI): [M+H]⁺ = 247.66.

Step 3: N-Bromosuccinimide (1.6 g, 9.2 mmol) and azodiisobutyronitrile (62.8 mg, 0.38 mmol) were added to a solution of the compound 26d (1.9 g, 7.6 mmol) in carbon tetrachloride (98 mL) and reacted at 85°C overnight under nitrogen atmosphere. The solvent was spun dry and purified by column chromatography (PE:EA=0-30%) to obtain a yellow oily intermediate 26d (219.9 mg, 8%).

¹H NMR (600 MHz, CDCl₃) δ 7.39 (d, *J =* 8.4 Hz, 1H), 7.35 - 7.31 (m, 1H), 4.45 (s, 2H), 3.98 (s, 3H).

Step 4: Potassium tert-butanolate (75.7 mg, 0.67 mmol) was added to a solution of 1-Boc-piperidine-4-carbaldehyde (158.4 mg, 0.74 mmol) in tetrahydrofuran (4 mL) under nitrogen atmosphere at -30 °C and reacted at this temperature for 1 hour, after then, a solution of compound 26d (219.9 mg, 0.67 mmol) in tetrahydrofuran (3 mL) was added slowly, and reacted at this temperature for 2 hours. Then brought to room temperature and reacted overnight. The solvent was spun dry and purified by column chromatography (PE:EA= 0 - 40%) to obtain a colorless oily intermediate 26e (147.6 mg, 48%).

¹H NMR (600 MHz, CDCl₃) δ 9.55 (s, 1H), 7.30 (d, *J =* 8.3 Hz, 1H), 7.00 - 6.95 (m, 1H), 3.96 (s, 3H), 4.00 - 3.75 (m, 2H), 2.95 - 2.70 (m, 2H), 2.77 (s, 2H), 1.94 (d, *J =* 13.0 Hz, 2H), 1.58 - 1.46 (m, 2H), 1.43 (s, 9H).

LC-MS(ESI): [M-Boc+H]⁺ = 358.19.

Step 5: Sodium borohydride (1.9 g, 51.4 mmol) was added slowly and batchwise to a solution of compound 26e (4.7 g, 10.3 mmol) in methanol (50 mL) at 0°C , and the reaction solution was subsequently brought to room temperature and reacted for 2 hours. The reaction was quenched by slowly addition of water, spun dry, and purified by column chromatography (PE:EA=0-50%) to obtain a colorless oily liquid intermediate 26f (4.14 g, 88% yield).

¹H NMR (600 MHz, DMSO-*d*₆) δ 7.51 (d, *J =* 8.3 Hz, 1H), 7.41 - 7.36 (m, 1H), 4.78 (t, *J =* 5.0 Hz, 1H), 3.90 (s, 3H), 3.46 - 3.39 (m, 2H) , 3.20 (d, *J =* 4.5 Hz, 4H), 2.66 (s, 2H), 1.99 (s, 2H), 1.42 - 1.32 (m, 11H).

LC-MS(ESI): [M-Boc+H]⁺ = 360.29.

Step 6: The intermediate 26f (125 mg, 0.27 mmol) was slowly added to a solution of sodium hydride (11.9 mg, 0.27 mmol, 60 % dispersed in liquid paraffin) in N,N-dimethylformamide (1 mL) under nitrogen atmosphere, broughtto 110°C and reacted for 1 hour. The mixture was cooled to room temperature and water (2 mL) was slowly added to quench the reaction. After addition of water (10 mL), the reaction solution was extracted with ethyl acetate (20 mL*3 times), washed with saturated saline (40 mL), and the organic phases were combined and purified by column chromatography (PE:EA= 0-40%) to obtain a white solid intermediate 26 g (73.0 mg, 61%).
¹H NMR (600 MHz, CDCl₃) δ 7.03 (d, *J* = 8.2 Hz, 1H), 6.92 (d, *J=* 8.2 Hz, 1H), 3.92 (s, 3H), 3.90 (s, 2H), 3.59-3.47 (m, 2H), 3.37 - 3.28 (m, 2H), 2.61 (s, 2H), 1.50 - 1.40 (m, 13H).
LC-MS(ESI): [M-Boc+H]⁺ = 339.99

Step 7: Under nitrogen atmosphere, the intermediate 26 g (73.0 mg, 0.17 mmol)was dissolved in a solvent mixture of tetrahydrofuran/water (10:1, 1.1 mL), and potassium vinyltrifluoroborate (33.5 mg, 0.25 mmol), palladium acetate (7.5 mg, 0.033 mmol), triphenylphosphine (6.1 mmol, 0.023 mmol) and cesium carbonate (108.2 mg, 0.33 mmol) were added, and the reaction was carried out at 85°C overnight in an oil bath. The solvent was spun dry and purified by column chromatography (PE:EA= 0-40%) to obtain a white solid intermediate 26h (38.9 mg, 45%).
¹H NMR (600 MHz, CDCl₃) δ 7.08 (d, *J =* 7.9 Hz, 1H), 7.04 (d, *J =* 7.8 Hz, 1H), 6.63 (dd, *J =* 17.4, 11.0 Hz, 1H), 5.69 (d, *J* = 17.4 Hz, 1H), 5.28 (d, *J =* 11.0 Hz, 1H), 3.96 - 3.88 (m, 5H), 3.60 - 3.50 (m, 2H), 3.38 - 3.29 (m, 2H), 2.67 (s, 2H), 1.52 - 1.42 (m, 13H).
LC-MS(ESI): [M-Boc+H]⁺ = 288.18

Step 8: Sodium periodate (64.9 mg, 0.3 mmol) and potassium osmiocyanate dihydrate (1.4 mg, 0.003 mmol) were added to a solution of compound 26h (29.4 mg, 0.076 mmol) in an acetone/water mixture (5:1, 2.7 mL) and the reaction was allowed to proceed overnight at room temperature. The solvent was spun dry and purified by column chromatography (PE:EA= 0-40%) to obtain a white solid intermediate 26i (8.9 mg, 30%).
¹H NMR (600 MHz, CDCl₃) δ 9.89 (s, 1H), 7.37 (d, *J =* 7.7 Hz, 1H), 7.25 (d, *J =* 6.4 Hz, 1H), 4.00 - 3.94 (m, 5H), 3.59 - 3.48 (m, 2H), 3.42 - 3.33 (m, 2H), 2.75 (s, 2H), 1.54 - 1.48 (m, 2H), 1.48 - 1.41 (m, 11H).
LC-MS(ESI): [M-Boc+H]⁺ = 290.18

Step 9: The intermediate 26i (500 mg, 1.3 mmol) was mixed with 3-amino-2,6-piperidinedione hydrochloride (316 mg, 1.9 mmol) in a solvent mixture of dichloromethane/methanol (1/2, 39 mL), and reacted at room temperature for 1 hour, followed by the addition of sodium cyanoborohydride (241 mg, 3.8 mmol). The reaction was carried out overnight at room temperature. The solvent was spun dry and the mixture was purified by a reversed-phase column to obtain a white solid intermediate 26j (233 mg, 39%).
¹H NMR (600 MHz, *DMSO-d₆*) δ 10.95 (s, 1H), 7.28 (d, *J* = 7.6 Hz, 1H), 7.00 (d, *J* = 7.6 Hz, 1H), 5.02 (dd, *J =* 13.3, 5.1 Hz, 1H), 4.30 (d, *J =* 17.2 Hz, 1H), 4.17 (d, *J* = 17.1 Hz, 1H), 4.01 (s, 2H), 3.50 - 3.43 (m, 2H), 3.30 - 3.25 (m, 2H) 2.94 - 2.87 (m, 1H), 2.73 (s, 2H), 2.60 - 2.55 (m, 1H), 2.38 - 2.30 (m, 1H), 1.99 - 1.91 (m, 1H), 1.45 - 1.35 (m, 11H), 1.35 - 1.29 (m, 2H).
LC-MS(ESI): [M+H]⁺ = 470.40

Step 10: Trifluoroacetic acid (2 mL) was added to a solution of compound 26j (230 mg, 0.49 mmol) in dichloromethane (5 mL), and reacted at room temperature for 1.5 hours. The solvent was spun dry and the mixture was purified by a preparative column to obtain a white solid intermediate 26 (151.2 mg, 84%).
¹H NMR (600 MHz, *DMSO-d₆*) δ 10.96 (s, 1H), 7.30 (d, *J* = 7.6 Hz, 1H), 7.04 (d, *J* = 7.4 Hz, 1H), 5.01 (dd, *J =* 13.2, 4.9 Hz, 1H), 4.31 (d, *J* = 17.1 Hz, 1H), 4.18 (d, *J* = 17.2 Hz, 1H), 4.11 - 4.04 (m, 2H), 3.20 - 3.06 (m, 4H), 2.94 - 2.86 (m, 1H), 2.94 - 2.86 (m, 1H) 2.77 (s, 2H), 2.60 - 2.53 m, 1H), 2.39 - 2.30 (m, 1H), 1.98 - 1.91 (m, 1H), 1.69 - 1.53 (m, 4H).
LC-MS(ESI): [M+H]⁺ = 370.29

### Example 110

### Synthesis of intermediate 27

### synthesis scheme

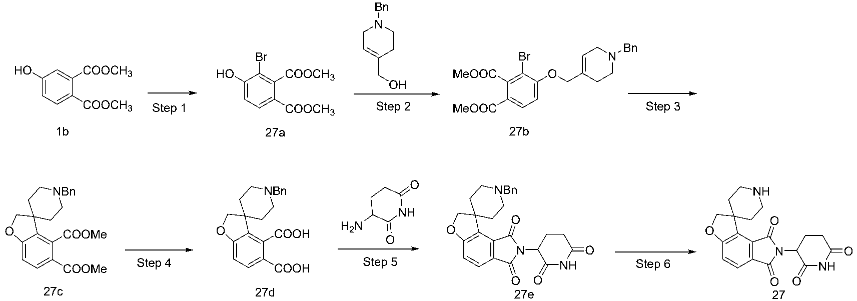

Step 1: The intermediate 1b (33.6 g, 160 mmol, 1.0 eq) was dissolved in trifluoroacetic acid (100 mL), N-bromosuccinimide (34.2 g, 192 mmol, 1.2 eq) was added to the reaction system and the reaction was carried out at room temperature overnight. The resultingreaction system was concentrated under reduced pressure and extracted with ethyl acetate (100 ml*3 times) after adding water (100 mL). Then it was washed by saturated saline (100 mL) and the organic phases were combined and dried with anhydrous sodium sulfate. The concentrated crude product was purified by reversed-phase column chromatography to obtain a pale yellow solid intermediate 27a (9.8 g, 21.3%).
¹H NMR (600 MHz, *DMSO-d₆*) δ 11.68 (s, 1H), 7.87 (d, *J =* 12.0 Hz, 1H), 7.10 (d, *J =* 12.0 Hz, 1H), 3.84 (s, 3H), 3.79 (s, 3H).
LCMS (ESI): [M-H]= 287.09.

Step 2: The intermediate 27a (9.8 g, 33.9 mmol, 1.0 eq), (1-benzyl-1,2,3,6-tetrahydropyridin-4-yl)methanol (10.3 g, 50.8 mmol, 1.5 eq) and triphenylphosphorane (33.3 g, 127 mmol, 2.5 eq) were dissolved in tetrahydrofuran (80 mL), diethyl azodicarboxylate ( 22.1 g, 127 mmol, 2.5 eq) was added and the reaction was carried out at room temperature for 4 hours. The resulting reaction system was concentrated under reduced pressure, water (50 mL) was added to the system, extracted with ethyl acetate (100 mL*3 times), then it was washed (100 mL) by saturated saline, the organic phases were combined and dried with anhydrous sodium sulfate. The crude product was purified by column chromatography to obtain a reddish-brown solid intermediate 27b (8.7 g, 51.3%).
¹H NMR (600 MHz, *DMSO-d*₆) δ 8.00 (d, *J =* 8.8 Hz, 1H), 7.40 - 7.29 (m, 5H), 7.27 - 7.24 (m, 1H), 5.84 (tt, *J* = 3.4, 1.6 Hz, 1H), 4.68 (s, 2H), 3.86 (s, 3H), 3.81 (s 3H), 3.54 (s, 2H), 2.93 - 2.92 (m, 2H), 2.54 (t, *J =* 5.7 Hz, 2H), 2.16 (s, 2H).
LCMS (ESI): [M+H]⁺ = 474.09.

Step 3: The intermediate 27b (8.7 g, 18.3 mmol, 1.0 eq), tri-n-butyltin hydroxide (10.6 g, 36.6 mmol, 2.0 eq) and azobis(isobutyronitrile) (0.6 g, 3.7 mmol, 0.2 eq) were dissolved in toluene (84 mL) and reacted at 110 °C for 4 h under the protection of nitrogen. The reaction system was cooled to room temperature and concentrated under reduced pressure, water (100 mL) was added and extracted with ethyl acetate (50 mL*3 times). Then it was washed by saturated saline (100 mL), and the organic phases were combined and dried with anhydrous sodium sulfate. The crude product was purified by column chromatography to obtain a reddish brown oily intermediate 27c (4.7 g, 64.8%).
¹H NMR (600 MHz, *DMSO-d*₆) δ 7.84 (d, *J* = 8.5 Hz, 1H), 7.38 -7.29 (m, 4H), 7.29 - 7.23 (m, 1H), 7.00 (d, *J =* 8.5 Hz, 1H), 4.52 (s, 2H), 3.86 (s, 3H), 3.78 (s, 3H), 3.48 (s, 2H), 2.75 (dd, *J =* 11.6, 3.7 Hz, 2H), 2.08 ~ 1.93 (m, 4H), 1.58 (d, *J* = 12.2 Hz, 2H).
LCMS (ESI): [M+H]⁺= 395.71

Step 4: The intermediate 27c (4.7 g, 11.9 mmol, 1.0 eq) was dissolved in methanol (10 mL) and water (10 mL), and potassium hydroxide (33.3 g, 594 mmol, 50 eq) was added to the system. the reaction was carried out at 90 °C for 12 h. The reaction system was concentrated under reduced pressure and the crude product was purified by column chromatography to obtain a white solid intermediate 27d (4.3 g, 92.1%).
¹H NMR (400 MHz, *DMSO-d*₆) δ 7.81 (d, *J =* 8.5 Hz, 1H), 7.60 -7.65 (m, 2H), 7.44 - 7.39 (m, 3H), 6.94 (d, *J* = 8.5 Hz, 1H), 4.61 (s, 2H), 4.25 (s, 2H), 3.16 (s, 4H), 2.39 (s, 2H), 1.78 (d, *J =* 12.2 Hz, 2H). 2.39 (s, 2H), 1.78 (d, *J* = 12.2 Hz, 2H).
LCMS (ESI) [M+H]⁺= 368.30

Step 5: The intermediate 27d (4.3 g, 11.7 mmol, 1.0 eq), sodium acetate (3.8 g, 46.8 mmol, 4.0 eq) and compound 3-amino-2,6-piperidinedione hydrochloride (2.99 g, 23.4 mmol, 2.0 eq) were dissolved in acetic acid (30 mL) and the system was reacted at 110°C for 12 h. The reaction system was cooled to room temperature and then concentrated under reduced pressure, water (50 mL) was added, extracted with ethyl acetate (50 mL*3 times), washed with saturated saline (80 mL), the organic phases were combined and dried with anhydrous sodium sulfate. The crude product was purified by column chromatography to obtain a white solid intermediate 27e (2.5 g, 47.2%).
¹H NMR (400 MHz, CD₃OD) δ 7.79 (d, *J =* 8.2 Hz, 1H), 7.69 - 7.44 (m, 5H), 7.19 (d, *J =* 8.1 Hz, 1H), 5.13 (dd, *J* = 12.6, 5.4 Hz, 1H), 4.80 (s, 2H), 4.38 (s, 2H), 3.55 (d, *J =* 13.0 Hz, 2H), 3.35 - 3.36 (m, 1H), 3.17 (t, *J* = 13.4 Hz, 2H), 2.97 - 2.85 (m, 2H), 2.81 - 2.65 (m, 2H), 2.17 - 1.98 (m, 3H).
LCMS (ESI) [M+H]⁺ = 460.29

Step 6: The intermediate 27e (50 mg, 1.1 mmol, 1.0 eq) was dissolved in methanol (50 mL) and palladium/carbon hydroxide (14 mg, 0.1 mmol, 0.1 eq) was added to the system. After hydrogen substitution for 3 times, the reaction was carried out at room temperature for 2 h. The reaction system was filtered and the filter cake was washed three times with methanol (10 mL), the resulting filtrate was spun dry under reduced pressure, and the concentrated crude was purified by reversed-phase column chromatography to obtain the white solid intermediate 27 (16 mg, 26.2%).
¹H NMR (600 MHz, CD₃OD) δ 7.79 (dd, *J* = 8.2, 1.2 Hz, 1H), 7.19 (dd, *J =* 8.1, 1.3 Hz, 1H), 5.15 (dd, *J* = 12.9, 5.4 Hz, 1H), 4.78 (s, 2H), 3.49 (dt, *J =* 13.3, 2.7 Hz, 2H), 3.14 (td, *J =* 13.7, 2.8 Hz, 2H), 2.94 - 2.87 (m, 3H), 2.80 - 2.70 (m, 2H), 2.18 - 2.14 (m, 1H), 2.04 (d, *J =* 14.3 Hz, 2H).
LCMS (ESI) [M+H]⁺= 370.39

### Example 111

### Synthesis of intermediate 28

### synthesis scheme

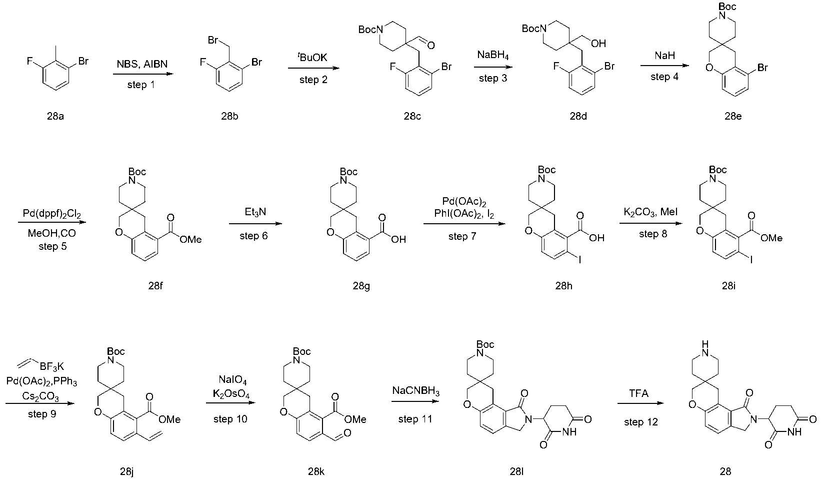

Step 1: N-Bromosuccinimide (1.2 g, 6.9 mmol) and azodiisobutyronitrile (173.7 mg, 1.1 mmol) were added to a solution of intermediate 28a (1 g, 5.3 mmol) in carbon tetrachloride (20 mL) under nitrogen atmosphere and reacted at 80°C overnight. The solvent was spun dry and purified by column chromatography (PE:EA=0-30%) to obtain a colorless oily intermediate 28b (1.3 g, 90%).

¹H NMR (600 MHz, CDCl₃) δ 7.39 (d, *J =* 8.1 Hz, 1H), 7.21 - 7.15 (m, 1H), 7.08 - 7.03 (m, 1H), 4.65 (d, *J =* 1.7 Hz, 2H).

Step 2: Potassium tert-butanolate (592.5 mg, 5.3 mmol) was added to a solution of 1-Boc-piperidine-4-carboxaldehyde (1.3 g, 4.8 mmol) in tetrahydrofuran (10 mL) under nitrogen atmosphere at -30 °C and reacted at this temperature for 1 hour, after then, a solution of compound 28b (1.3 g, 4.8 mmol) in tetrahydrofuran (10 mL) was slowly added and reacted at this temperature for 2 h. The solution was then broughtto room temperature and reacted overnight. The solvent was spun dry and purified by column chromatography (PE:EA= 0 - 20%) to obtain a colorless oily intermediate 28c (710.7 mg, 37%).

¹H NMR (600 MHz, CDCl₃) δ 9.65 (d, *J =* 2.9 Hz, 1H), 7.37 (d, *J =* 8.0 Hz, 1H), 7.14 - 7.08 (m, 1H), 7.04 - 6.98 (m, 1H), 4.15 - 3.70 (m, 2H), 3.02 (d, *J =* 2.4 Hz, 2H), 2.90 - 2.60 (m, 2H), 2.18 - 1.94 (m, 2H), 1.76 - 1.62 (m, 2H), 1.44 (s, 9H).

LC-MS(ESI): [M-Boc+H]⁺ = 300.06.

Step 3: Sodium borohydride (1.9 g, 51.4 mmol) was added slowly and batchwise to a solution of compound 28c (4.7 g, 10.3 mmol) in methanol (50 mL) at 0°C, and then broughtto room temperature and reacted for 2 hours. The reaction was quenched by slowly addition of water, spun dry, and purified by column chromatography (PE:EA=0-50%) to obtain a colorless oily liquid intermediate 28d (4.14 g, 88% yield).

¹H NMR (600 MHz, CDCl₃) δ 7.39 (d, *J* = 8.0 Hz, 1H), 7.12 - 7.07 (m, 1H), 7.05 - 6.99 (m, 1H), 3.80 - 3.65 (m, 2H), 3.62 (d, *J* = 6.2 Hz, 2H), 3.11 (t, J = 11.0 Hz, 2H), 2.94 (d, J = 2.6 Hz, 2H), 1.67 - 1.62 (m, 2H), 1.62 - 1.59 (m, 1H), 1.55 - 1.47 (m, 2H), 1.44 (s, 9H).

LC-MS(ESI): [M-Boc+H]⁺ = 301.98.

Step 4: The intermediate 28d (659.4 mg, 1.64 mmol) was slowly added to a solution of sodium hydride (72.1 mg, 1.8 mmol, 60 % dispersed in liquid paraffin) in N,N-dimethylformamide (7 mL) under nitrogen atmosphere and brought to 110°C and reacted for 1 hour. The mixture was cooled to room temperature and the reaction was quenched by slowly adding water (10 mL). After addition of water (10 mL), the reaction solution was extracted with ethyl acetate (40 mL*3 times), Then it was washed (80 mL) by saturated saline, and the organic phases were combined and purified by column chromatography (PE:EA= 0-10%) to obtain a white solid intermediate 28e (583.1 mg, 93%).

¹H NMR (400 MHz, CDCl₃) δ 7.15 (dd, *J* = 7.9, 1.1 Hz, 1H), 7.02 - 6.95 (m, 1H), 6.78 (dd, *J =* 8.2, 1.1 Hz, 1H), 3.85 (s, 2H), 3.64 - 3.51 (m, 2H), 3.45 - 3.32 (m, 2H), 2.64 (s, 2H), 1.52 - 1.44 (m, 13H).

LC-MS(ESI): [M-tBu+H]⁺ = 325.98.

Step 5: Triethylamine (2.5 g, 24.6 mmol) and Pd(dppf)₂Cl₂• CH₂Cl_{2 (}670.7 mg, 0.8 mmol) was added to a solution (60 mL) of intermediate 28e (3.1 g, 8.2 mmol) in methanol in an atmosphere of carbon monoxide (60 mL) and then brought to 65°C and reacted overnight. At the end of the reaction, it was spun dry and purified by column chromatography (PE:EA=0-20%) to obtain a colorless oily liquid intermediate 28f (2.7 g, 90%).

¹H NMR (400 MHz, CDCl₃) δ 7.52 (d, *J =* 7.5 Hz, 1H), 7.20 - 7.11 (m, 1H), 6.99 (d, *J =* 8.0 Hz, 1H), 3.90 (s, 2H), 3.88 (s, 3H), 3.60 - 3.46 (m, 2H), 3.46 - 3.34 (m, 2H), 3.00 (s, 2H), 1.55 - 1.40 (m, 13H).

LC-MS(ESI): [M-Boc+H]⁺ = 262.17.

Step 6: An aqueous solution of potassium hydroxide (1.5 g, 37 mmol) (20 mL) was added to a solution (1/1, 40 mL) of compound 28f in a mixed methanol/tetrahydrofuran at 0°C and broughtto an oil bath at 60 °C and reacted overnight. The solution was spun dry, 1M hydrochloric acid solution (50 mL) was added, extracted with ethyl acetate (40 mL * 3 times), Then it was washed (80 mL) by saturated saline, then the organic phases were combined and spun dry to obtain a white solid intermediate 28 g (2.5 g, 97%), directly used in the next step.

LC-MS(ESI): [M- H]⁻= 346.20.

Step 7: The iodine monomer (62.0 mg, 0.244 mmol), diethyl iodate (78.7 mg, 0.244 mmol), and palladium acetate (2.7 mg, 0.012 mmol) were added to a solution of compound 28g (84.9 mg, 0.244 mmol) in N,N-dimethylformamide (1 mL) under air atmosphere. The reaction was carried out in an oil bath at 80 °C for half an hour. The reaction solution was extracted with ethyl acetate (10 mL*3 times).Then it was washed by saturated saline (20 mL), and the organic phases were combined, spun dry, subjected to the prepared liquid phase, and lyophilized to obtain a white solid intermediate 28h (72.3 mg, 65%).

¹H NMR (400 MHz, CDCl₃) δ 7.54 (d, *J =* 8.6 Hz, 1H), 6.63 (d, *J =* 8.5 Hz, 1H), 3.89 (s, 2H), 3.64 - 3.45 (m, 2H), 3.45 - 3.26 (m, 2H), 2.71 (s, 2H), 1.52 - 1.36 (m, 13H). 2.71 (s, 2H), 1.52 - 1.36 (m, 13H).

LC-MS(ESI): [M- H]⁻= 472.10.

Step 8: Iodomethane (120 mg, 0.85 mmol), potassium carbonate (175.2 mg, 1.27 mmol) were added to a solution of intermediate 28h (200.0 mg, 0.42 mmol) in N,N-dimethylformamide (5 mL). The reaction was carried out at 80°Cfor 1 hour. At the end of the reaction, water (20 mL) was added, extracted with ethyl acetate (40 mL*3 times), then it was washed by saturated saline (40 mL), and the organic phases were combined and dried with anhydrous sodium sulfate. The combined organic phase was spun dry and purified by column chromatography (PE:EA=0 - 20%) to obtain a yellow oily intermediate 28i (203.9 mg, 99%).

¹H NMR (600 MHz, CDCl₃) δ 7.51 (d, *J =* 8.7 Hz, 1H), 6.62 (d, *J =* 8.7 Hz, 1H), 3.95 (s, 3H), 3.88 (s, 2H), 3.57 - 3.47 (m, 2H), 3.40 - 3.27 (m, 2H), 2.59 (s, 2H), 1.49 - 1.41 (m, 13H).

LC-MS(ESI): [M-Boc+H]⁺ = 388.19.

Step 9: The intermediate 28i (1.36 g, 2.8 mmol) was dissolved in a solvent mixture of tetrahydrofuran/water (10:1, 33 mL) under nitrogen atmosphere, followed by the addition of potassium vinyl trifluoroborate (560.7 mg, 4.2 mmol), palladium acetate (125.3 mg, 0.558 mmol), triphenylphosphine (102.5 mg, 0.391 mmol) and cesium carbonate (1.82 g, 5.58 mmol). The reaction was carried out at 85°C overnight in an oil bath. The solvent was spun dry and purified by column chromatography (PE:EA= 0-40%) to obtain a colorless oily liquid intermediate 28j (790 mg, 73%), which was directly used in the next step.

LC-MS(ESI): [M+H]⁺ = 388.09.

Step 10: Sodium periodate (6.07 g, 28.4 mmol) and potassium osmioglycate dihydrate (126.8 mg, 0.284 mmol) were added to a solution of intermediate 28j (2.75 g, 7.1 mmol) in a mixture of acetone/water (5:1, 210 mL) and reacted overnight at room temperature. The solvent was spun dry and purified by column chromatography (PE:EA= 0-40%) to obtain a white solid intermediate 28k (930 mg, 33%).

¹H NMR (600 MHz, CDCl₃) δ 9.83 (s, 1H), 7.65 (d, *J =* 8.5 Hz, 1H), 6.99 (d, *J =* 8.5 Hz, 1H), 4.05 - 3.95 (m, 5H), 3.58 - 3.47 (m, 2H), 3.40 - 3.31 (m, 2H), 2.64 (s, 2H), 1.55 - 1.29 (m, 13H).

LC-MS(ESI): [M-tBu+H]⁺ = 334.19.

Step 11: The intermediate 28k (451 mg, 1.16 mmol) was mixed with 3-amino-2,6-piperidinedione hydrochloride (286.2 mg, 1.74 mmol) in a solvent mixture of dichloromethane/methanol (1/2, 21 mL) and reacted at room temperature for 1 hour, then sodium cyanoborohydride (218.5 mg, 3.5 mmol) was added. The reaction was carried out overnight at room temperature. The solvent was spun dry and the mixture was purified by a reversed-phase column to obtain a white solid intermediate 281 (30.2 mg, 5%).

¹H NMR (600 MHz, *DMSO-d₆*) δ 10.99 (s, 1H), 7.29 (d, *J =* 8.2 Hz, 1H), 7.02 (d, *J =* 8.2 Hz, 1H), 5.01 (dd, *J* = 13.2, 5.1 Hz, 1H), 4.30 (d, *J* = 16.9 Hz, 1H), 4.19 (d, *J =* 16.9 Hz, 1H), 3.98 (s, 2H), 3.47 - 3.38 (m, 2H), 3.31 - 3.24 (m, 2H), 3.12 - 3.02 (m, 2H), 2.92 - 2.85 (m, 1H), 2.63 - 2.56 (m, 1H), 2.42 - 2.32 (m, 1H), 2.00 - 1.94 (m, 1H), 1.51 - 1.30 (m, 11H), 1.38 - 1.30 (m, 2H).

LC-MS(ESI): [M-Boc+H]⁺ = 370.29.

Step 12: Trifluoroacetic acid (1 mL) was added to compound 281 (30.2 mg, 0.064 mmol), reacted overnight at room temperature. The mixture was purified by preparative column to obtain white solid intermediate 28 (21.7 mg, 91%).

¹H NMR (600 MHz, *DMSO-d₆*) δ 11.00 (s, 1H), 7.31 (d, *J* = 8.2 Hz, 1H), 7.04 (d, *J* = 8.2 Hz, 1H), 5.01 (dd,*J* = 13.3, 5.1 Hz, 1H), 4.31 (d, *J =* 17.0 Hz, 1H), 4.20 (d, *J =* 17.0 Hz, 1H), 4.05 (s, 2H), 3.21 - 3.03 (m, 6H), 2.94 - 2.84 (m, 1H), 2.64 - 2.56 (m, 1H), 2.38 (qd, *J* = 13.0, 4.2 Hz, 1H), 2.02 - 1.91 (m, 1H), 1.70 - 1.52 (m, 4H).

LC-MS(ESI): [M+H]⁺ = 370.29.

### Example 112

### Synthesis of intermediate 29

### synthesis scheme

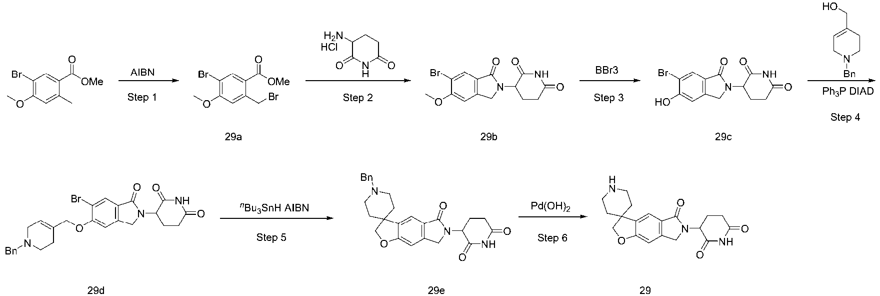

Step 1: Methyl 5-bromo-4-methoxy-2-methylbenzoate (5 g, 19.30 mmol), NBS (3.61 g, 20.30 mmol) and AIBN (316.9 mg, 1.90 mmol) were dissolved in carbon tetrachloride (50 mL) under the protection of nitrogen, and the reaction was carried out at 75 °C overnight. after completion of the reaction indicated by TLC was completely completed , the reaction system was cooled to room temperature and saturated aqueous sodium thiosulfate was added, stirred for 30 min, extracted with dichloromethane, washed with saturated saline, and the organic phases were combined and dried with anhydrous sodium sulfate. After concentration, the crude product was purified by column chromatography to obtain a white solid intermediate 29a (6.9 g, 96.7%).
LC-MS (ESI): [M+H]⁺ =336.90

Step 2: The intermediate 29a (7 g, 20.70 mmol) and 3-amino-2,6-piperidinedione hydrochloride (5.1 g, 31.10 mmol) were dissolved in acetonitrile (100 mL) under the protection of nitrogen, and N,N-diisopropylethylamine (13.4 g, 103.60 mmol) was added to the reaction system, and the reaction was carried out at 80 °C overnight. The reaction system was concentrated under reduced pressure, acetic acid (50 mL) was added and the reaction was carried out at 120 °C for 2 h. After completion of the reaction indicated by TLC , the reaction system was concentrated under reduced pressure and purified by reversed-phase column chromatography to obtain a solid intermediate 29b (5.3 g, 72.5%).
LC-MS(ESI): [M+H]⁺ = 353.15

Step 3: The intermediate 29b (5 g, 14.20 mmol) was dissolved in dichloromethane (10 mL) under the protection of nitrogen, solution of boron tribromide (1M, 140 mL) in dichloromethane was added slowly at 0°C, and the reaction was brought to room temperature and reacted overnight. After completion of the reaction indicated by TLC, the resulting reaction system was concentrated under reduced pressure to remove most of the boron tribromide, and then dichloromethane (50 mL) was added to the system and quenched with a small amount of methanol. The reaction system was concentrated under reduced pressure and purified by reversed-phase column chromatography to obtain a off-white solid intermediate 29c (3.6 g, 75.0%).
LC-MS(ESI): [M+H]⁺ = 338.99

Step 4: The intermediate 29c (180 mg, 0.53 mmol), (1-benzyl-1,2,3,6-tetrahydropyridin-4-yl)methanol(162 mg, 0.80 mmol) and triphenylphosphine (320 mg, 1.22 mmol) were dissolved in tetrahydrofuran (10 mL) under the protection of nitrogen, and diethyl azodicarboxylate (0.2 mL, 1.2 mmol) was added, and reacted at room temperature for 4 h. After completion of the reaction indicated by TLC , the resulting reaction system was concentrated under reduced pressure and purified by column chromatography to obtain a light yellow solid intermediate 29d (147 mg, 53 %).
¹H NMR (600 MHz, *DMSO-d₆*) δ 11.00 (s, 1H), 7.90 (s, 1H), 7.55- 7.46 (m, 5H), 7.42 (s, 1H), 5.91 (s, 1H), 5.08 (dd, *J* = 13.3, 5.1 Hz, 1H), 4.75 (s, 2H), 4.45- 4.34 (m, 3H), 4.27 (d, *J* = 17.5 Hz, 1H), 3.71 (s, 2H), 3.56 (s, 1H), 3.17 (s, 1H), 2.91 (ddd, *J* = 17.3, 13.6, 5.5 Hz, 1H), 2.63- 2.57 (m, 1H), 2.41 (ddd, *J* = 16.9, 16.2, 11.7 Hz, 1H) 16.2, 11.7 Hz, 3H), 2.00 (dtd, *J =* 12.7, 5.4, 2.3 Hz, 1H).
LC-MS(ESI): [M+H]⁺ = 524.09

Step 5: The intermediate 28d (120 mg, 0.23 mmol), tri-n-butyltin hydroxide (80 mg, 0.27 mmol) and azobisisobutyronitrile (19 mg, 0.12 mmol) were dissolved in toluene (10 mL) under the protection of nitrogen, and the reaction solution was heated up to 110 °C and reacted for 2 h. After completion of the reaction indicated by TLC, the reaction system was cooled down to room temperature and concentrated under reduced pressure. After being purified by reversed-phase column chromatography, a white solid intermediate 29e (51 mg, 50 %) was obtained.
¹H NMR (600 MHz, *DMSO-d₆*) *δ* 10.97 (s, 1H), 7.56-7.47 (m, 5H), 7.42 (s, 1H), 7.04 (s, 1H), 5.03 (dd, *J* = 13.2, 4.9 Hz, 1H), 4.64 (q, *J =* 9.3 Hz, 2H), 4.39-4.33 (m, 3H), 4.23 (d, *J =* 17.3 Hz, 1H), 3.33-3.24 (m, 2H), 3.16 (d, *J =* 16.2 Hz, 2H), 2.94-2.84 (m, 1H), 2.59 (d, *J =* 16.7 Hz, 1H), 2.41-2.32 (m, 1H), 2.16 (t, *J =* 12.4 Hz, 2H), 2.00-1.92 (m, 3H).
LC-MS(ESI): [M+H]⁺ = 446.35

Step 6: The intermediate 29e (25 mg, 0.06 mmol) was dissolved in methanol (2 mL) under the protection of nitrogen, and 10% wet palladium/carbon (24 mg) was added to the system. After hydrogen substitution for 3 times, the reaction was carried out overnight at room temperature. The reaction system was filtered and the filter cake was washed three times with methanol (5 mL). The resulting filtrate was spun dry under reduced pressure, and the concentrated crude product was purified by reversed-phase column chromatography to obtain a white solid intermediate 29 (8.7 mg, 43%).
¹H NMR (600 MHz, *DMSO-d₆*) δ 10.97 (s, 1H), 7.47 (s, 1H), 7.04 (s, 1H), 5.06 (dd, *J =* 13.3, 5.1 Hz, 1H), 4.65-4.60 (m, 2H), 4.36 (d, *J =* 17.2 Hz, 1H), 4.24 (d, *J* = 17.2 Hz, 1H), 3.36-3.34 (m, 2H), 3.04 (d, *J* = 8.5 Hz, 2H), 2.95-2.86 (m, 1H), 2.60 (d, *J* = 17.1 Hz, 1H), 2.38 (qd, *J* = 13.2, 4.4 Hz, 1H), 2.10-2.03 (m, 2H), 2.00- 1.95 (m, 1H), 1.91-1.85 (m, 2H).
LC-MS(ESI): [M+H]⁺ = 356.35

### Example 113

### Synthesis of intermediate 30

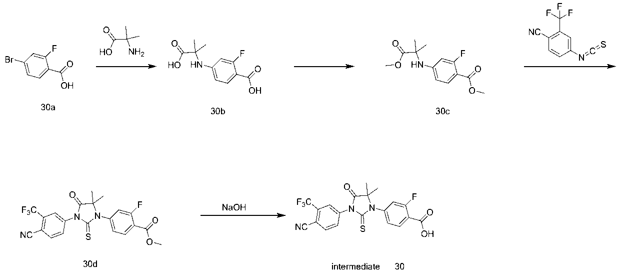

Step 1: In a glass vial, 4-bromo-2-fluorobenzoic acid (5 g, 22.83 mmol), 2-methylalanine (7.06 g, 68.49 mmol), copper powder (2.90 g, 45.66 mmol), cuprous iodide (8.70 g, 45.66 mmol), potassium carbonate (15.78 g, 114.15 mmol) and N N-dimethylglycine (1.17 g, 11.41 mmol) were added sequentially, solvents DMF (100 mL) and water (10 mL) were added, and stirred at 110 °C for 16 h. The reaction solution was cooled to room temperature, then iced water (200 mL) was added, and the pH was adjusted to 3-4 with dropwise addition of iced hydrochloric acid, then extracted with ethyl acetate for three times (500 mL*3). The organic phase was concentrated and then purified by a normal phase column (ethyl acetate:petroleum ether=35%) to obtain a yellow solid (Intermediate 30b) (4 g).
LC-MS: [M+H]⁺= 242.2

Step 2: The intermediate 30b (3 g, 12.44 mmol) and solvent methanol (50 mL) were added sequentially in a single-necked flask. Sulfoxide chloride (3.70 g, 31.09 mmol) was added dropwise in an ice bath and the reaction solution was stirred at 80°C for 16 h. Afterthe reaction was completed, the reaction solution was concentrated directly to obtain a yellow solid (intermediate 30c) (3 g).
LC-MS: [M+H]⁺= 270.07

Step 3: The intermediate 30c (1 g, 3.71 mmol), 4-isothiocyanato-2-(trifluoromethyl)benzonitrile (1.02 g, 4.46 mmol), isopropyl acetate (10 mL), and DMSO (5 mL) were added sequentially in a single-necked flask. The reaction was carried out at 95 °C for 60 h under the protection of nitrogen. After the reaction was completed, the reaction solution was concentrated until no solvent was evaporated, methanol was added dropwise, and the crystals were precipitated by stirring at 0-5 °C, and the intermediate 30d (500 mg) was obtained by filtration.
¹H NMR (600 MHz, DMSO-d6) δ 8.41 (d, *J* = 8.2 Hz, 1H), 8.30 (d, *J* = 1.9 Hz, 1H), 8.10 - 8.09 (m, 2H), 7.52 (dd, *J* = 11.2, 1.9 Hz, 1H), 7.42 (dd, *J* = 8.3, 1.9 Hz, 1H), 3.90 (s, 3H), 1.56 (s, 6H).
LC-MS(ESI): [M+H]⁺ = 466.08

Step 4: The intermediate 30d (0.5 g, 1.07 mmol), sodium hydroxide (214.84 mg, 5.37 mmol), solvents methanol (5 mL), tetrahydrofuran (5 mL) and water (2.5 mL) were added sequentially in a single-necked flask. The reaction was carried out at 40 °C for 2 h. After the reaction was completed, water (10 mL) was added, the pH was adjusted to 3-4 by dropwise addition of iced hydrochloric acid, and the reaction solution was extracted with ethyl acetate for three times (10 mL*3), and the organic phase was concentrated, and then purified by a reversed-phase column (acetonitrile:water=60%) to obtain a white solid (intermediate 30) (300 mg).
¹H NMR (600 MHz, DMSO-d6) δ 13.55 (s, 1H), 8.41 (d, *J* = 8.2 Hz, 1H), 8.30 (d, *J* = 1.9 Hz, 1H), 8.13 - 8.01 (m, 2H), 7.47 (dd, *J* = 11.0, 1.9 Hz, 1H), 7.38 (dd, *J =* 8.3, 1.9 Hz, 1H).
LC-MS(ESI): [M+H]⁻= 450.22

### Example 114

### Synthesis of intermediate 31

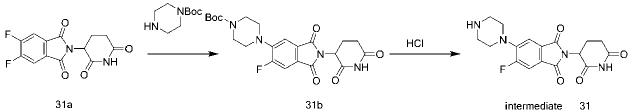

Step 1: In a glass vial, 2-(2,6-dioxopiperidin-3-yl)-5,6-difluoroisoindoline-1,3-dione (1.4 g, 4.76 mmol), tert-butyl piperazine-1-carboxylate (887 mg, 4.76 mmol), diisopropylethylamine (1.23 g, 9.52 mmol) were added sequentially, solvent DMSO was added, and the reaction solution was stirred at 110 °C for 16 h. After the reaction solution was cooled to room temperature, water (100 mL) was added, the reaction solution was extracted with ethyl acetate 3 times (50 mL*3), the organic phase was concentrated, and then purified by normal-phase column (ethyl acetate:petroleum ether=45%) to obtain a yellow solid (intermediate 31b) (850 mg).
LC-MS: [M+H-56]+=405.3

Step 2: The intermediate 31b (850 mg, 1.85 mmol) and solvent dioxane (10 mL) were added sequentially in a single-necked flask. Dioxane hydrochloride solution (2 mL) was added dropwise at room temperature and the reaction solution was stirred at room temperature for 2 h. After the reaction was completed, the reaction solution was concentrated directly to obtain a yellow solid (intermediate 31c) (610 mg).
LC-MS: [M+H]+=361.36
¹H NMR (600 MHz, MeOD) δ 7.66 (d, *J =* 10.8 Hz, 1H), 7.61 (d, *J =* 7.2 Hz, 1H), 5.13 (dd, *J =* 12.9, 5.4 Hz, 1H), 3.55 (m, *J* = 6.7, 3.7 Hz, 4H), 3.45 (m, *J =* 6.5, 3.8 Hz, 4H), 2.89 (m, *J* = 17.5, 14.0, 5.4 Hz, 1H), 2.81 (m, 2H), 2.14 (m, *J* = 13.1, 5.6, 2.7 Hz, 1H).

### Example 115

### Synthesis of compound 80

### synthesis scheme

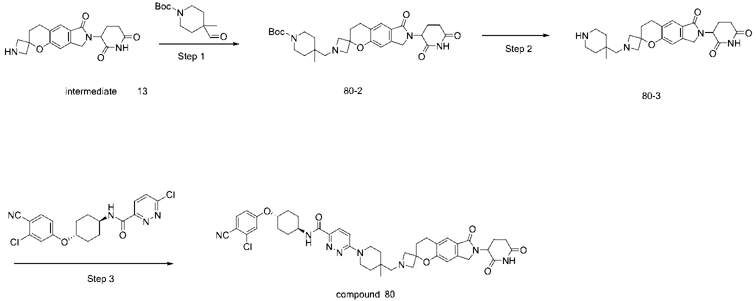

Step 1: (Compound 80-2) was prepared with reference to step 1 of Example 19.

Step 2: (Compound 80-3) was prepared with reference to step 2 of Example 19.

Step 3: (Compound 80) was prepared with reference to step 3 of Example 19.
¹H NMR (400 MHz, DMSO-d6) δ 10.94 (s, 1H), 8.57 (d, *J =* 8.2 Hz, 1H), 7.82 (dd, *J =* 22.3, 9.2 Hz, 2H), 7.49 - 7.24 (m, 3H), 7.13 (dd, *J =* 8.8, 2.4 Hz, ¹H ), 6.99 (s, 1H), 5.04 (dd, *J =* 13.2, 50 Hz, 1H), 4.62 - 4.45 (m, 1H), 4.26 (dd, *J =* 51.4, 16.9 Hz, 2H), 4.05 - 3.74 (m, 3H), 3.45 (t, *J =* 9.7 Hz, 3H), 3.23 (s, 1H), 2.97 - 2.78 (m, 3H), 2.59 (m, 4H), 2.46 - 2.23 (m, 3H), 2.09 (s, 3H), 2.00 - 1.83 (m, 3H), 1.57 (m, 6H), 1.38 - 1.13 (m, 2H), 0.99 (s, 3H).
LC-MS(ESI): [M+H]⁺ = 808.35

### Example 116

### Synthesis of compound 81

### synthesis scheme

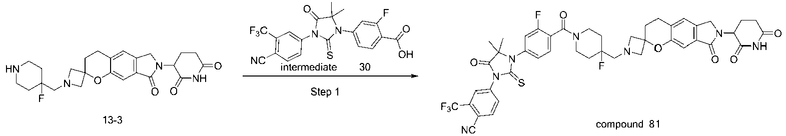

Step 1: Compound 13-3 (40 mg, 0.08 mmol), the intermediate 30 (52.2 mg, 0.12 mmol), DIEA (3 eq), BOP (3 eq) and DMF (2 mL) were added sequentially to an 8 mL capped tube. The reaction was carried out at room temperature under the condition of N₂ for 3 h. When the reaction was completed, it was cooled to room temperature, concentrated, and then purified by preparation to obtain a white solid compound 81 (5 mg).
¹H NMR (600 MHz, MeOD) δ 8.21 (t, *J =* 20.7 Hz, 2H), 8.02 (dd, *J =* 8.2, 1.8 Hz, 1H), 7.63 (s, 1H), 7.45 - 7.37 (m, 1H), 7.29 (s, 1H), 7.23 (s, 1H), 5.13 (dd, *J =* 13.3, 5.2 Hz, 1H), 4.57 (d, *J =* 13.2 Hz, 1H), 4.39 (q, *J =* 16.5 Hz, 2H), 3.64 (d, *J =* 8.6 Hz, 2H), 3.56 (d, *J =* 12.9 Hz, 1H), 3.47 (dd, *J =* 26.8, 4.7 Hz, 3H), 3.24 (td, *J =* 12.9, 2.4 Hz, 1H), 3.00 - 2.87 (m, 5H), 2.82 - 2.75 (m, 1H), 2.49 (ddd, *J =* 26.5, 13.3, 4.6 Hz, 1H), 2.22 (t, *J =* 6.5 Hz, 2H), 2.18 - 2.13 (m, 1H), 2.12 - 2.05 (m, 1H), 1.96 *(d, J=* 4.7 Hz, 1H), 1.78 (ddd, *J =* 19.9, 19.0, 5.4 Hz, 2H), 1.63 (s,6H).
LC-MS(ESI): [M+H]⁺ = 890.27

### Example 117

### Synthesis of compound 82

### synthesis scheme

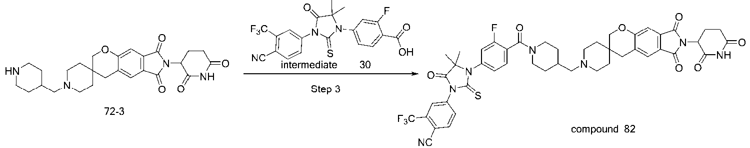

Step 1: (Compound 82) was prepared with reference to step 1 of example 116.
¹H NMR (600 MHz, DMSO-d6) δ 11.11 (s, 1H), 8.42 (d, *J =* 8.2 Hz, 1H), 8.30 (d, *J =* 1.3 Hz, 1H), 8.09 (dd, *J =* 8.2, 1.5 Hz, 1H), 7.73 (s, 1H), 7.61 (t, *J =* 7.7 Hz, 1H), 7.46 (dd, J = 9.9, 1.4 Hz, 1H), 7.37 - 7.29 (m, 2H), 5.10 (dd, *J* = 12.9, 1.4 Hz, 1.4 Hz) 4.54 (d, *J =* 11.9 Hz, 1H), 4.32 (s, 1H), 4.04 (s, 1H), 3.45 (d, *J =* 13.0 Hz, 4H), 3.21 - 3.02 (m, 7H), 2.88 (dd, *J =* 20.1, 9.0 Hz, 2H), 2.80 (s, 1H), 2.57 (dd, *J =* 38.4, 10.8 Hz, 2H), 2.16 (s, 1H), 2.06 - 2.00 (m, 1H), 1.91 (t, *J =* 5.7 Hz, 1H), 1.81 - 1.68 (m, 4H), 1.62 (d, *J =* 13.6 Hz, 1H), 1.57 (s, 6H).
LC-MS(ESI): [M+H]⁺ = 914.29

### Example 118

### Synthesis of compound 83

### synthesis scheme

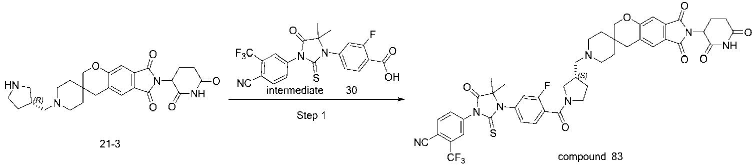

Step 1: (Compound 83) was prepared with reference to step 1 of example 116.
¹H NMR (600 MHz, DMSO-d6) δ 11.11 (s, 1H), 8.42 (dd, *J =* 8.2, 2.6 Hz, 1H), 8.30 (s, 1H), 8.09 (d, *J =* 8.2 Hz, 1H), 7.65 (ddd, *J* = 28.3, 20.7, 13.8 Hz, 2H), 7.52 - 7.44 (m, 1H), 7.39 - 7.34 (m, 1H), 7.30 (dd, *J =* 13.4, 5.0 Hz, 1H), 5.10 (dt, *J=* 12.3, 6.0 Hz, 1H), 4.31 (d, *J* = 32.9 Hz, 1H), 4.04 (d,*J* = 16.9 Hz, 1H), 3.89 (dd, *J =* 12.0, 7.7 Hz, 1H), 3.67 (d, *J* = 8.8 Hz, 1H), 3.40 (s, 3H), 3.30 (s, 2H), 3.24 - 2.98 (m, 5H), 2.92 - 2.83 (m 1H), 2.77 (dd, *J =* 32.9, 11.9 Hz, 2H), 2.63 - 2.56 (m, 1H), 2.21 - 1.98 (m, 3H), 1.81 - 1.60 (m, 5H), 1.56 (s, 5H).
LC-MS(ESI): [M+H]⁺ = 890.27

### Example 119

### Synthesis of compound 84

### synthesis scheme

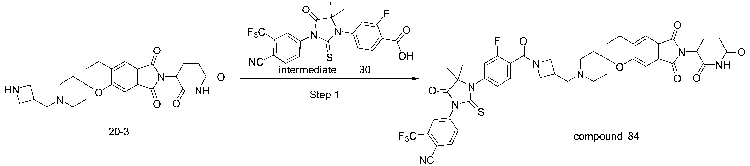

Step 1: (Compound 84) was prepared with reference to step 1 of example 116.
¹H NMR (600 MHz, DMSO-d6) δ 11.10 (s, 1H), 8.42 (d, *J =* 8.2 Hz, 1H), 8.29 (d, *J =* 1.2 Hz, 1H), 8.10 - 8.07 (m, 1H), 7.77 - 7.71 (m, 2H), 7.47 (dd, *J =* 10.4, 1.4 Hz, 1H), 7.42 - 7.34 (m, 2H), 5.10 (dd, *J* = 12.9, 5.4 Hz, 1H), 4.26 (t, *J =* 9.1 Hz, 2H), 3.96 (m, 3H), 3.52 (s, 2H), 3.37 (dd, *J* = 24.5, 10.7 Hz, 2H), 3.21 (dd, *J =* 13.3, 7.9 Hz, 3H), 2.99 - 2.84 (m, 3H), 2.60 (d, *J =* 17.4 Hz, 2H), 2.09 - 1.95 (m, 3H), 1.90 (dd, *J =* 18.2, 11.2 Hz, 3H), 1.55 (s, 6H).
LC-MS(ESI): [M+H]⁺ = 886.26

### Example 120

### Synthesis of compound 85

### synthesis scheme

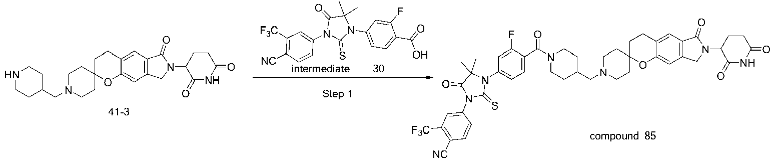

Step 1: (Compound 85) was prepared with reference to step 1 of example 116.
¹H NMR (600 MHz, DMSO-d6) δ 10.97 (s, 1H), 8.42 (d, *J =* 8.2 Hz, 1H), 8.30 (d, *J =* 1.4 Hz, 1H), 8.09 (dd, *J* = 8.3, 1.5 Hz, 1H), 7.61 (t, *J =* 7.8 Hz, 1H), 7.52 (d, *J =* 5.6 Hz, 1H), 7.46 (dd, *J =* 10.0, 1.3 Hz, 1H), 7.37 - 7.32 (m, 1H), 7.03 (d, *J =* 17.1 Hz, 1H), 5.07 (dd, *J* = 13.3, 5.2 Hz, 1H), 4.55 (d, *J =* 12.8 Hz, 1H), 4.34 ( t, *J=* 13.7 Hz, 2H), 4.26 - 4.19 (m, 2H), 3.46 (d, *J =* 10.5 Hz, 3H), 3.16 (d, *J =* 29.2 Hz, 5H), 2.94 - 2.82 (m, 4H), 2.60 (d*, J =* 17.0 Hz, 1H) , 2.37 (dd, *J =* 13.2, 4.6 Hz, 1H), 2.19 (s, 1H), 2.09 - 2.03 (m, 1H), 1.96 (m, 7H), 1.76 (s, 1H), 1.57 (s, 6H).
LC-MS(ESI): [M+H]⁺ = 890.31

### Example 121

### Synthesis of compound 86

### synthesis scheme

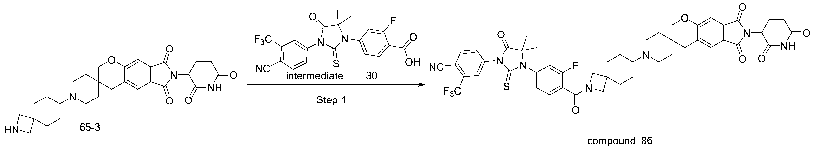

Step 1: (Compound 86) was prepared with reference to step 1 of Example 116.
¹H NMR (600 MHz, DMSO-d6) δ 11.11 (d, *J =* 4.8 Hz, 1H), 8.42 (d, *J =* 8.3 Hz, 1H), 8.30 (s, 1H), 8.09 (d, *J =* 8.3 Hz, 1H), 7.71 (ddd, *J* = 62.7, 35.5, 12.2 Hz, 2H), 7.46 (ddd, *J* = 10.4, 4.8, 1.6 Hz, 1H), 7.39 - 7.33 (m, 1H), 7.29 (ddd, *J* = 11.7, 3.9 Hz, 1H), 5.12 - 5.07 (m, 1H), 4.31 (d, *J =* 19.1 Hz, 1H), 4.02 (d, *J* = 17.4 Hz, 1H), 3.78 (dd, *J* = 39.2, 11.7 Hz, 4H), 3.40 - 3.29 (m, 2H), 3.14 (ddd, *J =* 50.3, 31.4, 16.0 Hz, 4H), 2.93 - 2.83 (m, 1H), 2.77 (d, *J* = 17.9 Hz, 1H), 2.63 - 2.55 (m, 3H), 2.12 - 1.86 (m, 5H), 1.78 - 1.40 (m, 13H).
LC-MS(ESI): [M+H]⁺ =940.30

### Example 122

### Synthesis of compound 87

### synthesis scheme

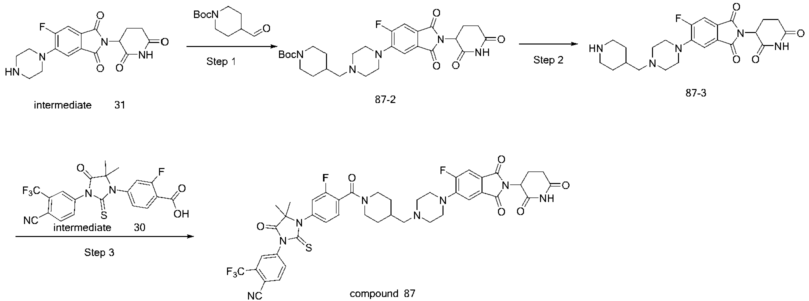

Step 1: The intermediate 31 (50 mg, 0.13 mmol) and tert-butyl 4-formylpiperidine-1-carboxylate (51.35 mg, 0.21 mmol) were added sequentially in a glass vial, and solvents DCE (2 mL), AcOH (1 drop) were added, and the reaction solution was stirred at room temperature for 3 h, then NaBH(OAc)₃ (59 mg, 0.28 mmol) was added and the reaction solution was continued to be stirred at room temperature for 1 h. When the reaction was completed, the reaction solution was concentrated, and then purified by TLC (DCM:MeOH=10:1) to obtain a white solid compound 87-2 (45 mg).

Step 2: Compound 87-2 (45 mg, 0.08 mmol), Dioxane (1 mL) and 4M dioxane hydrochloride solution (1 mL) were reacted at room temperature for 0.5 h. The reaction solution was concentrated to obtain a white solid compound 87-3 (40 mg).

Step 3: (Compound 87) was prepared with reference to step 1 of Example 115.
¹H NMR (600 MHz, DMSO-d6) δ 11.13 (s, 1H), 8.42 (d, *J =* 8.3 Hz, 1H), 8.30 (d, *J =* 1.2 Hz, 1H), 8.11 - 8.07 (m, 1H), 7.84 (d, *J* = 11.1 Hz, 1H), 7.62 (dd, *J =* 7.3, 4.7 Hz, 2H), 7.46 (dd, *J =* 10.0, 1.4 Hz, 1H), 7.36 (dd, *J* = 8.1, 1.4 Hz, 1H), 5.14 (dd, *J* = 12.9, 5.4 Hz, 1H), 4.55 (d, *J* = 12.9 Hz, 1H), 3.80 (d, *J =* 12.5 Hz, 3H), 3.68 - 3.57 (m, 3H), 3.46 (d, *J =* 13.2 Hz, 1H), 3.33 (d, *J =* 11.9 Hz, 4H), 3.16 (s, 3H), 2.89 (dd, *J =* 21.4, 9.5 Hz, 2H), 2.67 - 2.53 (m, 2H ), 2.19 (s, 1H), 2.10 - 2.00 (m, 1H), 1.93 (d, *J =* 11.8 Hz, 1H), 1.76 (s, 1H), 1.57 (s, 6H).
LC-MS(ESI): [M+H]⁺ =891.26

### Example 123

### Synthesis of compound 88

### synthesis scheme

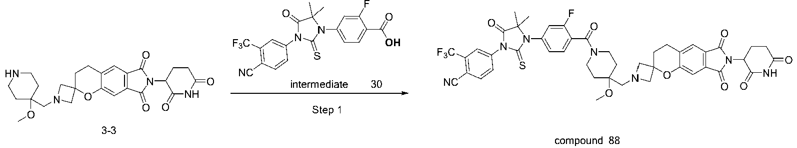

Step 1: (Compound 88) was prepared with reference to step 1 of example 116.
¹H NMR (600 MHz, DMSO-d6) δ 11.12 (s, 1H), 8.41 (t, *J =* 13.1 Hz, 1H), 8.30 - 8.24 (m, 1H), 8.10 (t, *J* = 11.0 Hz, 1H), 7.79 (d, *J* = 13.4 Hz, 1H), 7.64 - 7.57 (m, 1H), 7.46 (d, *J =* 9.8 Hz, 1H), 7.36 (d, *J =* 7.8 Hz, 1H), 7.29 (s, 1H), 5.11 (dd, *J =* 12.9, 5.4 Hz, 1H), 4.61 - 4.21 (m, 6H), 3.66 (s, 1H), 3.56 (s, 1H), 3.26 (d, *J* = 21.9 Hz, 2H), 3.23 (s, 3H), 3.11 (d*, J =* 9.6 Hz, 1H), 3.00 - 2.83 (m, 4H), 2.63 - 2.54 (m, 2H), 2.29 (s , 2H), 2.08 - 1.99 (m, 1H), 1.87 (d, *J =* 9.9 Hz, 1H), 1.76 (s, 1H), 1.59 (s, 6H).
LC-MS(ESI): [M+H]⁺ = 916.92

### Example 124

### Synthesis of compound 89

### synthesis scheme

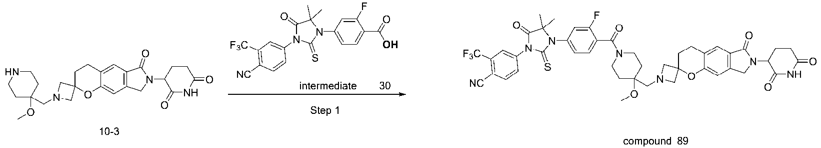

Step 1: (Compound 89) was prepared with reference to step 1 of example 116.
¹H NMR (600 MHz, DMSO-d6) δ 10.97 (s, 1H), 8.42 (d, *J =* 8.3 Hz, 1H), 8.29 (d, *J =* 1.4 Hz, 1H), 8.09 (dd, *J* = 8.2, 1.6 Hz, 1H), 7.61 (t, *J* = 7.8 Hz, 1H), 7.52 (s, 1H), 7.46 (d, *J* = 9.9 Hz, 1H), 7.36 (d, *J* = 8.1 Hz, 1H), 7.05 (t, *J =* 22.5 Hz, 1H), 5.07 (dd, *J =* 13.3, 5.0 Hz, 1H), 4.55 (s, 1H), 4.44 (s, 1H), 4.41 - 4.20 (m 4H), 3.68 - 3.51 (m, 3H), 3.28 (s, 2H), 3.22 (s, 3H), 3.11 (d, *J =* 9.5 Hz, 1H), 2.95 - 2.85 (m, 3H), 2.63 - 2.56 (m, 1H), 2.42 - 2.32 (s, 1H), 2.25 (s, 2H), 2.03 - 1.94 (m, 1H), 1.87 (d, *J =* 10.6 Hz, 1H), 1.76 (s, 1H), 1.68 - 1.58 (m, 1H), 1.56 (s, 6H).
LC-MS(ESI): [M+H]⁺ = 902.93

### Example 125

### Synthesis of compound 90

### synthesis scheme

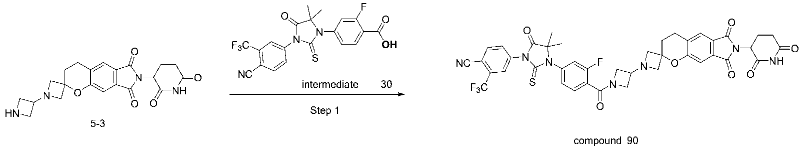

Step 1: (Compound 90) was prepared with reference to step 1 of example 116.
¹H NMR (600 MHz, DMSO-d6) δ 11.12 (s, 1H), 8.42 (d, *J =* 8.3 Hz, 1H), 8.28 (t, *J =* 19.0 Hz, 1H), 8.16 - 7.98 (m, 1H), 7.81 - 7.67 (m, 2H), 7.48 (dt, *J =* 26.3, 13.1 Hz, 1H), 7.41 - 7.29 (m, 1H), 7.30 (s, 1H), 5.11 (dd, *J =* 12.9, 5.4 Hz, 1H), 4.45 - 3.99 (m, 8H), 2.95 (t, *J =* 6.0 Hz, 2H), 2.89 (ddd, *J =* 17.1, 14.1, 5.4 Hz, 1H), 2.61 (m, 2H), 2.22 (t, *J =* 6.2 Hz, 2H), 2.07 - 1.98 (m, 2H), 1.55 (s, 6H).
LC-MS(ESI): [M+H]⁺ = 902.93

### Example 126

### Synthesis of compound 91

### synthesis scheme

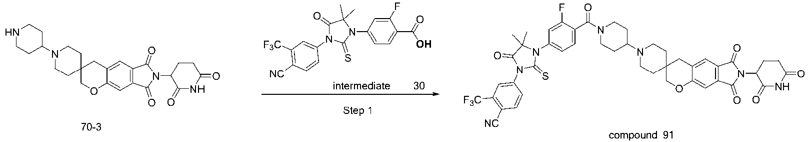

Step 1: (Compound 91) was prepared with reference to step 1 of example 116.
¹H NMR (600 MHz, DMSO-d6) δ 11.11 (s, 1H), 8.42 (d, *J =* 8.3 Hz, 1H), 8.30 (s, 1H), 8.09 (dd, *J =* 8.2, 1.3 Hz, 1H), 7.75 - 7.60 (m, 2H), 7.48 (dd, *J =* 10.0, 1.4 Hz, 1H), 7.36 (dt, *J =* 12.7, 6.3 Hz, 1H), 7.30 (d, *J = 3.9* Hz, 1H), 5.10 (dd, *J =* 12.9, 5.4 Hz, 1H), 4.70 (d, *J =* 12.3 Hz, 1H), 4.33 (s, 1H), 4.04 (s, 1H), 3.63 - 3.51 (m, 3H), 3.18 (d, *J =* 11.1 Hz, 3H), 3.08 (t, *J =* 21.2 Hz, 1H), 2.95 - 2.83 (m, 2H), 2.80 (s, 1H), 2.66 - 2.56 (m, 2H) , 2.22 (s, 1H), 2.09 (d, *J =* 12.4 Hz, 1H), 2.01 (ddd, *J =* 19.3, 11.2, 6.0 Hz, 2H), 1.69 (dd, *J =* 33.8, 13.8 Hz, 6H), 1.57 (s, 6H).
LC-MS(ESI): [M+H]⁺ = 900.92

### Example 127

### Synthesis of compound 92

### synthesis scheme

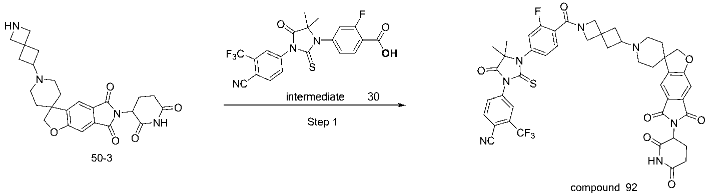

Step 1: (Compound 92) was prepared with reference to step 1 of example 116.
¹H NMR (600 MHz, DMSO-d6) δ 11.14 - 11.08 (m, 1H), 8.42 (d, *J* = 8.2 Hz, 1H), 8.29 (t,*J* = 9.1 Hz, 1H), 8.13 - 8.03 (m, 1H), 7.74 (dt, *J* = 7.8, 4.7 Hz, 1H), 7.66 (d, *J =* 13.0 Hz, 1H), 7.47 (t, *J =* 10.4 Hz, 1H), 7.35 (dd, *J =* 22.0, 13.7 Hz, 2H), 5.12 (ddd, *J =* 12.6, 11.9, 5.6 Hz, 1H), 4.72 (dd, *J =* 18.7, 3.4 Hz, 2H), 4.25 - 4.16 (m, 2H), 4.10 (s, 2H), 3.33 - 3.17 (m, 2H), 2.97 - 2.82 (m, 3H), 2.68 - 2.56 (m, 3H), 2.55 - 2.45 (m, 1H), 2.44 - 2.36 (m, 2H), 2.28 - 2.08 (m, 2H), 2.02 (dd, *J* = 23.3, 18.5 Hz, 3H), 1.85 (s, 1H), 1.55 (s, 6H ).
LC-MS(ESI): [M+H]⁺ = 898.90

### Example 128

### Synthesis of compound 93

### synthesis scheme

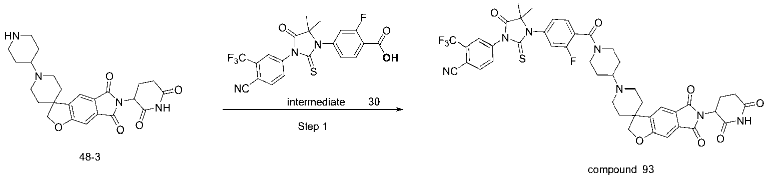

Step 1: (Compound 93) was prepared with reference to step 1 of example 116.
¹H NMR (600 MHz, DMSO-d6) δ 11.12 (s, 1H), 8.42 (d, *J =* 8.3 Hz, 1H), 8.28 (t, *J =* 14.1 Hz, 1H), 8.09 (dd, *J* = 8.2, 1.6 Hz, 1H), 7.65 (d, *J =* 12.6 Hz, 2H), 7.52 - 7.45 (m, 1H), 7.43 - 7.34 (m, 2H), 5.16 - 5.07 (m, 1H), 4.79 - 4.68 (m, 3H), 3.59 (d, *J =* 10.8 Hz, 4H), 3.18 (dd, *J =* 8.2, 1.6 Hz, 1.6 Hz, 1H), 7.65 (d, *J* = 12.6 Hz, 2H), 7.52 3.18 (dd, *J =* 57.5, 11.3 Hz, 3H), 2.94 - 2.83 (m, 2H), 2.69 - 2.56 (m, 2H), 2.23 (s, 3H), 2.06 (dd, *J =* 20.0, 6.9 Hz, 3H), 1.97 - 1.85 (m, 1H), 1.72 (d, *J =* 47.8 Hz, 2H), 1.57 (s, 6H).
LC-MS(ESI): [M+H]⁺ = 886.89

### Example 129

### Synthesis of compound 94

### synthesis scheme

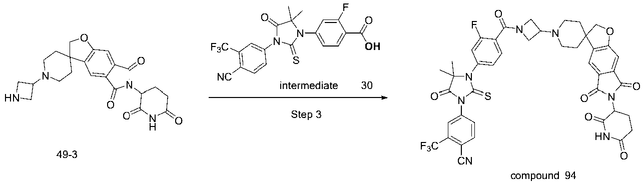

Step 1: (Compound 94) was prepared with reference to step 1 of example 116.
¹H NMR (600 MHz, DMSO-d6) δ 11.12 (s, 1H), 8.41 (d, *J =* 8.3 Hz, 1H), 8.30 (d, *J =* 1.6 Hz, 1H), 8.19 - 8.04 (m, 1H), 7.76 (t, *J =* 7.7 Hz, 2H), 7.48 (d, *J =* 10.0 Hz, 1H), 7.41 - 7.25 (m, 2H), 5.10 (dd, *J =* 13.0, 5.3 Hz, 1H), 4.66 (s, 2H), 4.07 (m, 7H), 2.93 - 2.83 (m, 2H), 2.64 - 2.55 (m, 2H), 2.02 (ddd, *J =* 20.5, 19.7, 14.6 Hz, 4H), 1.76 (s, 2H), 1.55 (s, 6H).
LC-MS(ESI): [M+H]⁺ = 858.84

### Example 130

### Synthesis of compound 95

### synthesis scheme

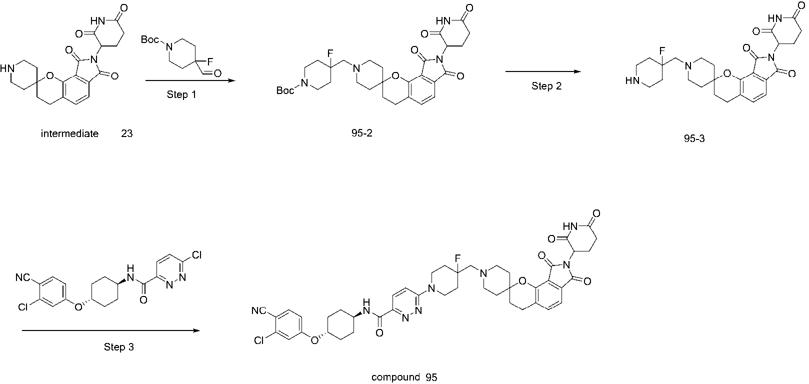

Step 1: The intermediate 23 (50 mg, 0.13 mmol) and tert-butyl 4-formyl-4-methoxypiperidine-1-carboxylate (45 mg, 0.20 mmol) were added sequentially in a glass vial, and solvents DCE (2 mL), AcOH (1 drop) were added, and the reaction solutioin was stirred at room temperature for 3 h. Then NaBH(OAc)₃ (55 mg, 0.26 mmol) was added and the reaction solution was continued to be stirred at room temperature for 1 h. After the reaction was completed, it was concentrated, and then purified by TLC (DCM:MeOH=10:1) to obtain a white solid compound 95-2 (46 mg).

Step 2: Compound 95-2 (46 mg, 0.08 mmol), Dioxane (1 mL) and 4M dioxane hydrochloride solution (1 mL) were reacted at room temperature for 0.5 h. The reaction solution was concentrated to obtain a white solid compound 95-3 (40 mg).

Step 3: Compound 95-3 (40 mg, 0.08 mmol), Intermediate 18 (52.2 mg, 0.12 mmol), DIEA (3 eq), and DMSO (2 mL) were added sequentially to an 8 mL capped tube. The reaction was carried out at room temperature under the condition of N₂ for 3 h. After completion of the reaction, the reaction solution was cooled down to room temperature, concentrated, and then purified by preparation to obtain a white solid compound compound 95 (3 mg).
¹H NMR (600 MHz, DMSO-d6) δ 11.12 (s, 1H), 8.41 (d, *J =* 8.3 Hz, 1H), 8.30 (d, *J =* 1.6 Hz, 1H), 8.19 - 8.04 (m, 1H), 7.76 (t, *J =* 7.7 Hz, 2H), 7.48 (d, *J =* 10.0 Hz, 1H), 7.41 - 7.25 (m, 2H), 5.10 (dd, *J =* 13.0, 5.3 Hz, 1H), 4.66 (s, 2H), 4.07 (m, 7H), 2.93 - 2.83 (m, 2H), 2.64 - 2.55 (m, 2H), 2.02 (ddd, *J =* 20.5, 19.7, 14.6 Hz, 4H), 1.76 (s, 2H), 1.55 (s, 6H).
LC-MS(ESI): [M+H]⁺ = 853.35

### Example 131

### Synthesis of compound 96

### synthesis scheme

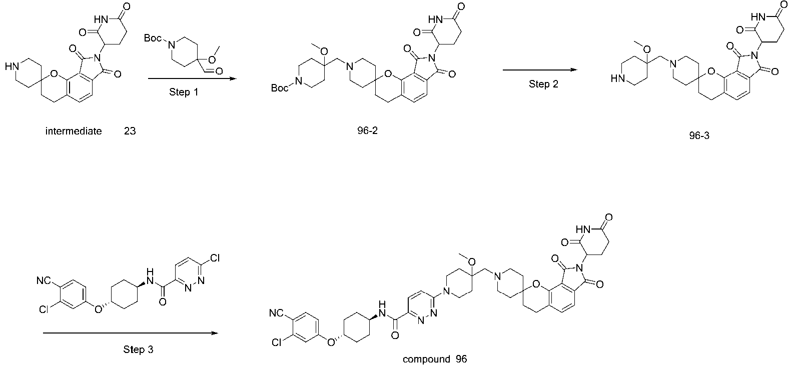

Step 1: The intermediate 23 (50 mg, 0.13 mmol) and tert-butyl 4-formyl-4-methoxypiperidine-1-carboxylate (45 mg, 0.20 mmol) were added sequentially in a glass vial, solvents DCE (2 mL), AcOH (1 drop) were added, and the reaction solution was stirred at room temperature for 3 h. Then NaBH(OAc)₃ (55 mg, 0.26 mmol) was added and the reaction solution was continued to be stirred at room temperature for 1 h. After the reaction was completed, it was concentrated, and then purified by TLC (DCM:MeOH=10:1) to obtain a white solid compound 96-2 (38 mg).

Step 2: Compound 96-2 (38 mg, 0.08 mmol), Dioxane (1 mL) and 4M dioxane hydrochloride solution (1 mL) were reacted at room temperature for 0.5 h. The reaction solution was concentrated to obtain a white solid compound 96-3 (30 mg).

Step 3: (Compound 96) was prepared with reference to step 3 of Example 130.
¹H NMR (600 MHz, DMSO-d6) δ 11.13 (s, 1H), 8.65 (t, *J =* 11.0 Hz, 1H), 7.89 - 7.80 (m, 2H), 7.72 - 7.60 (m, 1H), 7.47 - 7.35 (m, 3H), 7.13 (dt, *J =* 12.2, 6.1 Hz, 1H), 5.08 (dd, *J* = 12.8, 5.5 Hz, 1H), 4.54 (ddd, *J =* 14.6, 10.2, 4.2 Hz, 1H), 4.23 (s, 2H), 3.86 (m, 1H), 3.35 (dd, *J =* 25.7, 12.1 Hz, 6H), 3.27 (m, 5H), 2.90 (m, 3H), 2.61 - 2.54 (m, 2H), 2.20 - 2.08 (m, 4H), 1.98 (m, 7H), 1.89 (d, *J =* 10.5 Hz, 2H), 1.65 (dd, *J =* 22.7, 11.4 Hz 4H), 1.50 (dt, *J =* 20.1, 8.6 Hz, 2H).
LC-MS(ESI): [M+H]⁺ = 865.39

### Example 132

### Synthesis of compound 97

### synthesis scheme

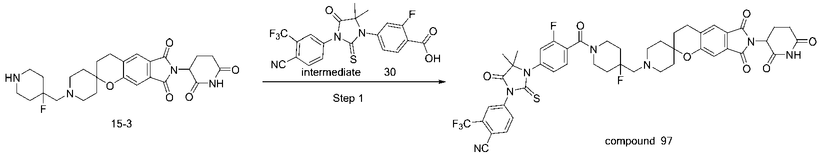

Step 1: (Compound 97) was prepared with reference to step 1 of Example 116.
¹H NMR (600 MHz, DMSO-d6) δ 11.10 (s, 1H), 8.42 (d, *J* = 8.2 Hz, 1H), 8.30 (s, 1H), 8.09 (d, J = 8.2 Hz, 1H), 7.70 (s, 1H), 7.64 (t, *J =* 7.8 Hz, 1H), 7.45 (dd, *J =* 10.0, 1.4 Hz, 1H), 7.35 (d, *J =* 8.1 Hz, 1H), 7.22 (s, 1H), 5.09 (dd, *J =* 12.9 1.4 Hz, 1H), 4.31 (d, *J =* 13.0 Hz, 2H), 3.20 - 3.12 (m, 2H), 2.95 - 2.87 (m, 2H), 2.71 - 2.54 (m, 6H), 2.06 - 1.94 (m, 3H), 1.92 - 1.81 (m, 3H), 1.75 - 1.62 (m, 5H), 1.54 (d, *J* = 36.8 Hz, 2H) *J =* 36.8 Hz, 5H).
LC-MS(ESI): [M+H]⁺ = 932.01

### Example 133

### Synthesis of compound 98

### synthesis scheme

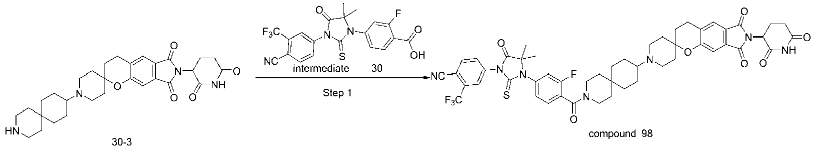

Step 1: (Compound 98) was prepared with reference to step 1 of Example 116.
¹H NMR (600 MHz, DMSO-d6) δ 11.10 (s, 1H), 8.41 (d, *J =* 8.3 Hz, 1H), 8.30 (d, *J =* 1.5 Hz, 1H), 8.09 (ddd, *J* = 8.2, 1.7 Hz, 1H), 7.69 (d, *J =* 8.4 Hz, 1H), 7.60 (td, *J =* 7.8 , 4.9 Hz, 1H), 7.43 (ddd, *J =* 9.9, 4.4, 1.7 Hz, 1H), 7.38 - 7.29 (m, 1H), 7.20 (d, *J =* 14.6 Hz, 1H), 5.12 - 5.04 (m, 1H), 3.62 (d, *J* = 26.6 Hz, 2H), 3.23 (d, *J =* 7.3 Hz, 5H), 2.88 (m, 3H), 2.66 - 2.56 (m, 4H), 2.32 (d, *J* = 10.9 Hz, 1H), 2.02 (dd, *J =* 11.3, 5.2 Hz, 1H), 1.79 (m, 5H), 1.64 (d, *J* = 27.9 Hz, 5H), 1.56 (s, 5H), 1.47 - 1.21 (m, 6H), 1.17 - 1.06 (m, 2H).
LC-MS(ESI): [M+H]⁺ = 968.34

### Example 134

### Synthesis of compound 99

### synthesis scheme

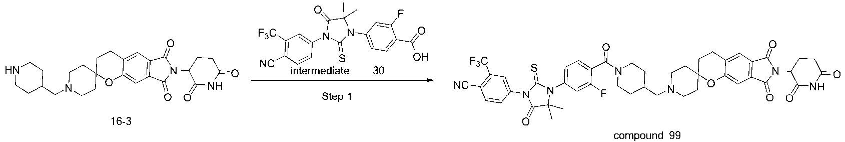

Step 1: (Compound 99) was prepared with reference to step 1 of Example 116.
¹H NMR (600 MHz, DMSO-d6) δ 11.10 (s, 1H), 8.41 (d, *J =* 8.2 Hz, 1H), 8.30 (d, *J =* 1.6 Hz, 1H), 8.09 (dd, *J =* 8.2, 1.6 Hz, 1H), 7.67 (d, *J =* 30.4 Hz, 1H), 7.61 (d, *J =* 6.9 Hz, 1H), 7.44 (dd, *J =* 9.9, 1.7 Hz, 1H), 7.37 - 7.30 (m, 1H), 7.20 (s, 1H), 5.09 (dd, *J =* 12.9, 5.5 Hz, 1H), 4.52 (d, *J =* 12.9 Hz, 1H), 3.42 (d, *J =* 21.6 Hz , 3H), 3.08 (d, *J =* 35.2 Hz, 2H), 2.93 - 2.78 (m, 4H), 2.66 - 2.53 (m, 3H), 2.40 - 2.29 (m, 2H), 2.26 - 2.15 (m, 2H), 2.02 (dd, *J* = 14.2, 8.9 Hz, 1H), 1.83 (dd, *J* = 23.5, 17.1 Hz, 3H), 1.76 - 1.61 (m, 5H), 1.58 - 1.38 (m, 7H).
LC-MS(ESI): [M+H]⁺ = 914.29

### Example 135

### Synthesis of compound 100

### synthesis scheme

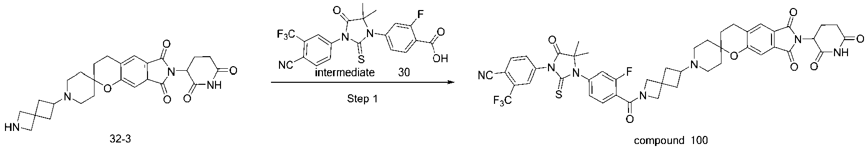

Step 1: (Compound 100) was prepared with reference to step 1 of Example 116.
¹H NMR (600 MHz, DMSO-d6) δ 11.08 (t, *J =* 22.1 Hz, 1H), 8.40 (dt, *J =* 49.2, 24.6 Hz, 1H), 8.27 (d, *J =* 36.8 Hz, 1H), 8.09 (d, *J =* 8.2 Hz, 1H), 7.77 - 7.62 (m, 2H), 7.47 - 7.39 (m, 1H), 7.37 - 7.28 (m, 1H), 7.28 (d, *J =* 9.9 Hz, 1H), 5.07 (ddd, *J =* 64.2, 34.7, 29.5 Hz, 1H), 4.15 (s, 2H), 4.04 (s, 2H) , 3.59 (dd, *J =* 13.2, 7.4 Hz, 2H), 2.89 (m, 4H), 2.64 - 2.55 (m, 2H), 2.48 - 2.41 (m, 2H), 2.00 (m, 1H), 1.93 - 1.76 (m, 4H), 1.59 - 1.39 (m, 11H).
LC-MS(ESI): [M+H]⁺ = 912.27

### Example 136

### Synthesis of compound 101

### synthesis scheme

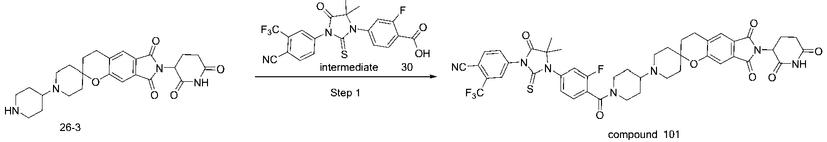

Step 1: (Compound 101) was prepared with reference to step 1 of Example 116.
¹H NMR (600 MHz, MeOD) δ8.19 (m, 2H), 8.02 (d, *J =* 8.2 Hz, 1H), 7.71 (s, 1H), 7.65 (s, 1H), 7.44 (m, 3H), 5.12 (dd, *J =* 12.8, 5.5 Hz, 1H), 4.95 (d, *J =* 12.6 Hz, 1H), 3.88 (d, *J =* 12.8 Hz, 1H), 3.63 (d, *J* = 35.9 Hz, 3H), 3.52 - 3.42 (m, 2H), 3.02 (dt, *J =* 23.9, 8.8 Hz, 3H), 2.88 (m, 2H), 2.80 - 2.69 (m, 3H ), 2.39 (s, 1H), 2.24 (d, *J =* 13.4 Hz, 2H), 2.13 (dd, *J =* 8.8, 3.7 Hz, 2H), 2.01 (t, *J* = 29.6 Hz, 3H), 1.84 (s, 2H), 1.63 (s, 6H).
LC-MS(ESI): [M+H]⁺ = 900.27

### Example 137

### Synthesis of compound 102

### synthesis scheme

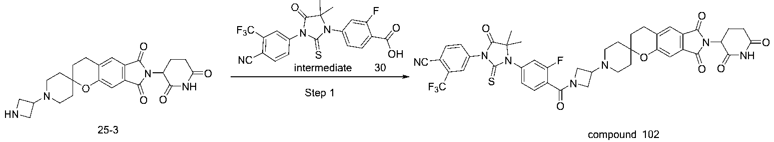

Step 1: (Compound 102) was prepared with reference to step 1 of example 116.
¹H NMR (600 MHz, MeOD) δ 8.20 - 8.18 (m, 2H), 8.01 (d, *J* = 8.3 Hz, 1H), 7.79 (t, *J =* 7.7 Hz, 1H), 7.70 (s, 1H), 7.45 (dd, *J =* 16.7, 9.3 Hz, 1H), 7.38 (s 1H), 5.11 (dd, *J =* 12.9, 5.4 Hz, 1H), 4.58 - 4.52 (m, 2H), 4.45 *(*d*, J =* 36.2 Hz, 2H), 4.25 (s, 1H), 3.52 - 3.37 (m, 2H), 3.29 - 3.16 (m, 2H), 3.04 (t, *J =* 6.4 Hz, 2H), 2.94 - 2.67 (m, 4H), 2.24 - 2.09 (m, 3H), 2.03 (d, *J =* 6.3 Hz,3H), 1.62 (s, 6H).
LC-MS(ESI): [M+H]+ = 872.24

### Example 138

### Synthesis of compound 103

### synthesis scheme

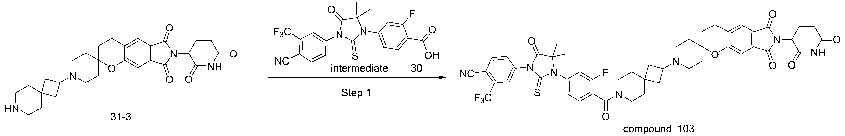

Step 1: (Compound 103) was prepared with reference to step 1 of example 116.
¹H NMR (600 MHz, MeOD) δ 8.19 (d, *J =* 5.7 Hz, 2H), 8.02 (d, *J =* 8.3 Hz, 1H), 7.70 (s, 1H), 7.60 (dd, *J =* 14.3, 7.2 Hz, 1H), 7.45 - 7.35 (m, 3H), 5.16 - 5.07 (m, 1H), 3.83 (t, *J =* 42.4 Hz, 3H), 3.56 - 3.36 (m, 4H), 3.21 (dd, *J =* 6.4, 4.8 Hz, 2H), 3.04 (s, 2H), 2.93 - 2.67 (m , 4H), 2.47 (m, 2H), 2.19-2.13 (m, 4H), 2.01 (t, *J =* 29.6 Hz, 4H), 1.77 (m, 4H), 1.63 (s, 6H).
LC-MS(ESI): [M+H]+ = 940.30

### Example 139

### Synthesis of compound 104

### synthesis scheme

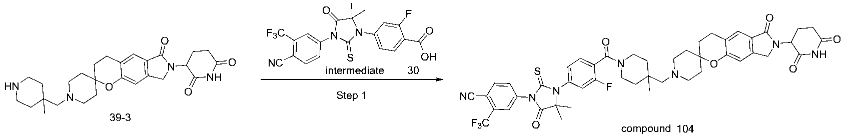

Step 1: (Compound 104) was prepared with reference to step 1 of example 116.
¹H NMR (600 MHz, MeOD) δ 8.19 (dd, *J* = 4.9, 3.1 Hz, 2H), 8.01 (dd, *J* = 8.3, 1.7 Hz, 1H), 7.62 (dd, *J* = 14.7, 6.7 Hz, 2H), 7.45 - 7.39 (m, 2H), 7.10 (d, *J =* 35.3 Hz, 1H), 5.13 (dd, *J =* 13.3, 5.1 Hz, 1H), 4.41 (dt, *J =* 33.6, 16.8 Hz, 3H), 3.71 - 3.35 (m, 10H), 3.04 - 2.97 (m, 2H), 2.92 (m, 2H) , 2.85 - 2.72 (m, 1H), 2.49 (ddd, *J =* 26.4, 13.3, 4.3 Hz, 2H), 2.14 (m, 6H), 1.99 (t, *J =* 6.7 Hz, 2H), 1.79 - 1.58 (m, 9H).
LC-MS(ESI): [M+H]+ = 914.32

### Example 140

### Synthesis of compound 105

### synthesis scheme

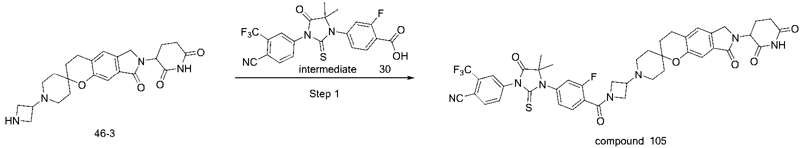

Step 1: (Compound 105) was prepared with reference to step 1 of example 116.
¹H NMR (600 MHz, MeOD) δ 8.22 - 8.13 (m, 2H), 8.01 (d, *J =* 8.2 Hz, 1H), 7.80 (t, *J =* 7.8 Hz, 1H), 7.52 - 7.41 (m, 2H), 7.36 (s, 1H), 7.32 (s, 1H), 5.14 (dd, *J =* 13.2, 4.8 Hz, 1H), 4.63 - 4.55 (m, 2H), 4.52 (m, 1H), 4.47 - 4.33 (m, 2H), 4.48 - 4.33 (m, 4H). 3.63 - 3.35 (m, 4H), 3.01 (t, *J =* 6.5 Hz, 2H), 2.95 - 2.87 (m, 1H), 2.84 - 2.75 (m, 1H), 2.52 - 2.43 (m, 1H), 2.25 - 2.11 (m, 3H), 2.06 - 1.91 (m, 4H), 1.62 (s, 6H).
LC-MS(ESI): [M + H]+ = 858.26

### Example 141

### Synthesis of compound 106

### synthesis scheme

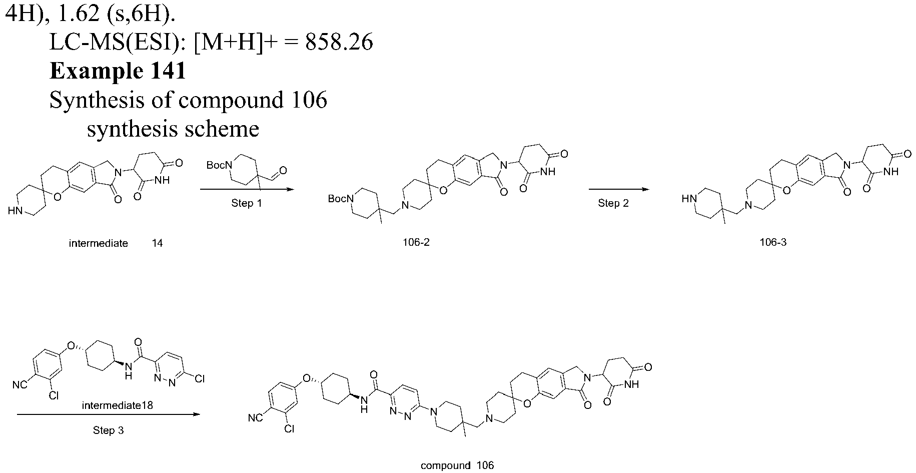

Step 1: (Compound 106-2) was prepared with reference to step 1 of example 130.

Step 2: (Compound 106-3) was prepared with reference to step 2 of example 130.

Step 3: Compound 106-3 (40 mg, 83.23 umol), the intermediate 2 (39.08 mg, 99.87 umol), DIEA (53.79 mg, 416.14 umol), DMSO (2 mL) were added sequentially in a single-necked flask. The reaction solution was stirred at 80 °C for 2 h. After the reaction was completed, it was cooled to room temperature, concentrated, and then purified by preparation (15-50% acetonitrile) to obtain a white solid compound 106 (6 mg).
¹H NMR (600 MHz, MeOD) δ 7.97 (d, *J =* 9.6 Hz, 1H), 7.71 (d, *J =* 8.8 Hz, 1H), 7.41 - 7.28 (m, 3H), 7.22 (d, *J= 2.4* Hz, 1H), 7.06 (dd, *J =* 8.8, 2.4 Hz, 1H), 5.14 (dd, *J* = 13.3, 5.1 Hz, 1H), 4.53 (d, *J* = 3.8 Hz, 1H), 4.42 (dd, *J* = 3.8 Hz, 1H) , 4.25 (dd, *J* = 30.8, 13.7 Hz, 2H), 4.03 - 3.96 (m, 1H), 3.70 (d , *J =* 11.8 Hz, 1H), 3.56 (dd, *J =* 25.7, 15.5 Hz, 4H), 3.38 - 3.34 (m, 2H), 3.30 - 3.26 (m, 2H), 3.01 (t, *J =* 6.5 Hz, 2H), 2.92 (ddd, *J* = 18.6 , 13.6, 5.4 Hz, 1H), 2.85 - 2.77 (m, 1H), 2.50 (ddd, *J =* 26.5, 13.3, 4.6 Hz, 1H), 2.25 - 2.16 (m, 3H), 2.09-2.03 (m, 5H), 1.98 (m, 2H), 1.75 (dd, *J =* 33.1, 18.5 Hz, 4H), 1.66 (dd, *J =* 20.4, 10.8 Hz, 4H), 1.38 (d, *J =* 14.9 Hz, 3H).
LC-MS(ESI): [M+H]+ = 835.36

### Example 142

### Synthesis of compound 107

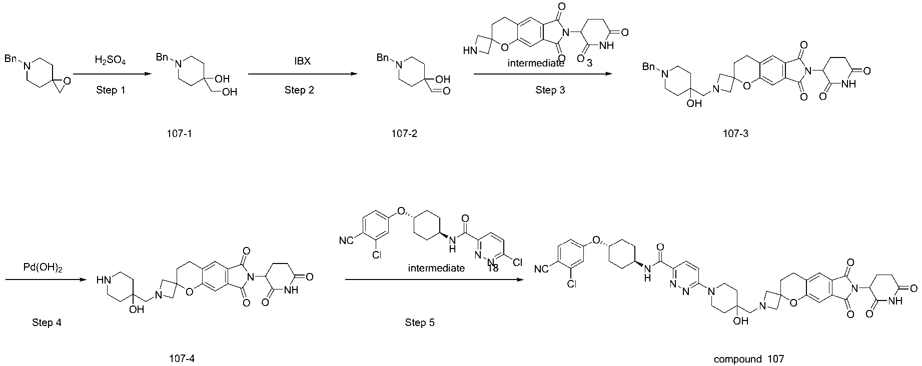

Step 1: 6-benzyl-1-oxa-6-azaspiro[2.5]octane (500 mg, 2.46 mmol) was added to a single-necked glass flask, then the solvent, i.e. 0.2 M aqueous sulfuric acid (30 mL) , was added, and the reaction solution was continued to be stirred at room temperature overnight. After the reaction was completed, the reaction solution was neutralized with aqueous sodium bicarbonate and extracted with ethyl acetate, and then the organic phase was spun dry and purified by flash to obtain a yellow oily compound 107-1 (380 mg).
LC-MS(ESI): [M+H]⁺ = 222.28

Step 2: Compound 107-1 (380 mg, 1.72 mmol), 2-iodoacylbenzoic acid (721.24 mg, 2.58 mmol) and solvent acetonitrile (4 mL) were added in a single-necked glass flask, heated up to 80 °C with stirring, the reaction solution was filtered and concentrated to obtain a yellow oily compound 107-2 (120 mg).
LC-MS(ESI): [M+H]⁺ = 220.2

Step 3: The intermediate 3 (97.23 mg, 0.274 mmol) and compound 107-2 (90 mg, 0.410 mmol) were added sequentially in a glass vial, and the solvents tetrahydrofuran (2 mL) and tetraisopropyl titanate (155.53 mg, 0.547 mmol) were added, and the reaction solution was stirred at room temperature for 1 h. Then sodium cyanoborohydride (34.39 mg, 0.547 mmol) was added and the reaction solution was continued to be stirred at room temperature for 2 h. After the reaction was completed, it was concentrated, and then purified by TLC (DCM:MeOH=10:1) to obtain a yellow solid compound 107-3 (55 mg).
LC-MS(ESI): [M+H]⁺ = 558.97

Step 4: Compound 107-3 (35 mg, 0.063 mmol) was dissolved in methanol (2 mL), 20% palladium charcoal hydroxide (17.5 mg) was added, hydrogen substitution was performed for three times and the reaction was carried out at room temperature for 2 h. A yellow oil compound 107-4 (28 mg) was obtainedby filtration and concentration.
LC-MS(ESI): [M+H]⁺ = 469.18

Step 5: Compound 107-4 (30 mg, 0.064 mmol), the intermediate 18 (37.58 mg, 0.096 mmol), DIEA (28.97 mg, 0.224 mmol) and DMSO (1 mL) were added sequentially into a glass vial. The reaction was carried out at 80 °C under the condition of N₂ for 2 h. After the reaction was completed, the reaction solution was cooled to room temperature, concentrated, and then purified by preparation to obtain a white solid compound 107 (4.99 mg).
¹H NMR (600 MHz, DMSO-d6) δ 11.12 (s, 1H), 8.60 (d, *J =* 8.2 Hz, 1H), 7.86 (dd, *J =* 11.4, 9.2 Hz, 2H), 7.78 (s, 1H), 7.43 - 7.37 (m, 2H), 7.29 (d, *J =* 10.7 Hz, 1H), 7.14 (dd, *J* = 8.8, 2.3 Hz, 1H), 5.33 (s, 1H), 5.11 (dd, *J* = 12.8, 5.2 Hz, 1H), 4.60 - 4.46 (m, 3H), 4.40 (s, 2H), 4.20 (t, *J =* 15.5 Hz, 2H), 3.86 (dt, *J* = 15.5 Hz, 2H), 3.86 (dt, *J* = 15.5 Hz, 2H), 3.30 (dt, *J* = 15.5 Hz, 2H) 3.86 (dt, *J =* 11.2, 9.5 Hz, 2H), 3.42 - 3.22 (m, 5H), 3.06 - 2.83 (m, 3H), 2.60 (d, *J =* 18.1 Hz, 2H), 2.34 - 2.22 (m, 2H), 2.15 - 2.00 (d, *J =* 15.5 Hz, 2H) 2.15 - 2.00 (m, 3H), 1.93 - 1.86 (m, 2H), 1.65 (d, *J =* 23.2 Hz,5H), 1.52 (dd, *J =* 23.0, 10.0 Hz, 2H).
LC-MS(ESI): [M+H]⁺ = 822.99

### Example 143

### Synthesis of compound 108

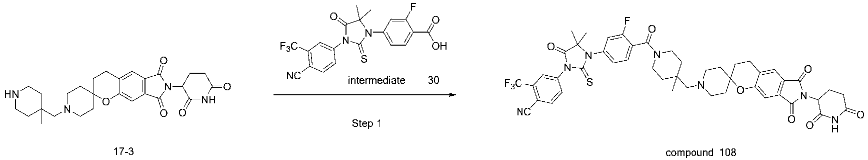

Step 1: (Compound 108) was prepared with reference to step 1 of example 116.
¹H NMR (600 MHz, DMSO-d6) δ 11.11 (s, 1H), 8.42 (d, *J =* 8.2 Hz, 1H), 8.30 (s, 1H), 8.09 (d, *J* = 8.2 Hz, 1H), 7.75 (s, 1H), 7.61 (t, *J =* 7.8 Hz, 1H), 7.48 - 7.32 (m, 3H), 5.10 (dd, *J =* 12.9, 5.4 Hz, 1H), 4.08 (s, 2H), 3.22 (m, 10H), 2.96 (s, 2H), 2.88 (m, 1H), 2.66 - 2.52 (m, 5H), 2.17 - 1.83 (m, 7H), 1.67 - 1.46 (m, 8H).
LC-MS(ESI): [M+H]⁺ = 928.03

### Example 144

### Synthesis of compound 109

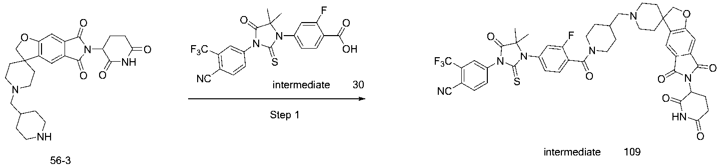

Step 1: (Compound 109) was prepared with reference to step 1 of example 116.
¹H NMR (600 MHz, DMSO-d6) δ 11.12 (s, 1H), 8.42 (d, *J=* 8.3 Hz, 1H), 8.30 (t, *J=* 4.3 Hz, 1H), 8.09 (dd, *J* = 8.2, 1.6 Hz, 1H), 7.63 (dd, *J =* 19.8, 11.9 Hz, 2H), 7.46 ( dd, *J =* 14.3, 12.8 Hz, 1H), 7.39 - 7.30 (m, 2H), 5.15 - 5.08 (m, 1H), 4.74 (d, *J =* 19.1 Hz, 2H), 4.56 (d, *J =* 12.3 Hz, 1H), 3.59 (d, *J=* 13.2 Hz, 2H), 3.37 - 3.30 (m, 2H), 3.22 - 3.01 (m, 5H), 2.94 - 2.82 (m, 2H), 2.65 - 2.56 (m, 2H), 2.23 (m, 3H), 2.10 - 1.88-1.79 (m, 6H), 1.57 (s, 6H).
LC-MS(ESI): [M+H]⁺ = 857.69

### Example 145

### Synthesis of compound 110

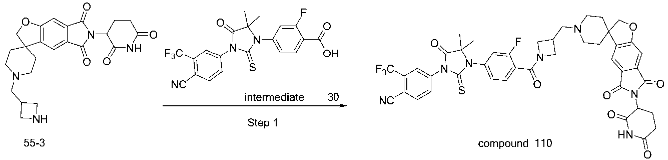

Step 1: (Compound 110) was prepared with reference to step 1 of example 116.
¹H NMR (600 MHz, DMSO-d6) δ 11.12 (s, 1H), 8.42 (d, *J* = 8.3 Hz, 1H), 8.29 (t, *J =* 7.9 Hz, 1H), 8.09 (dd, *J* = 8.3, 1.4 Hz, 1H), 7.77 - 7.66 (m, 1H), 7.64 (s, 1H), 7.49 (dd, *J* = 10.5, 1.6 Hz, 1H), 7.37 (dd, *J =* 13.9, 5.7 Hz, 2H), 5.15 - 5.07 (m, 1H), 4.78 - 4.65 (m, 2H), 4.29 (dd, *J =* 16.0, 8.6 Hz, 2H), 7.77 - 7.66 (m, 1H), 7.64 8.6 Hz, 2H), 4.01 - 3.90 (m, 2H), 3.34 (s, 2H), 3.25 - 3.03 (m, 3H), 2.94 - 2.84 (m, 1H), 2.64 - 2.52 (m 2H), 2.24 (s, 1H), 2.14 (t, *J =* 13.2 Hz,2H), 2.03 (dd, *J =* 17.3, 10.0 Hz, 3H), 1.93 - 1.84 (m, 1H), 1.55 (s, 6H).
LC-MS(ESI): [M+H]⁺ = 872.09

### Example 146

### Synthesis of compound 111

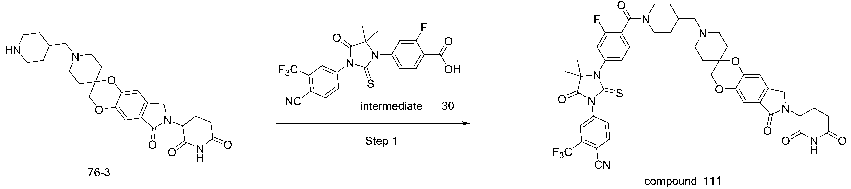

Step 1: (Compound 111) was prepared with reference to step 1 of example 116.
¹H NMR (600 MHz, DMSO-d6) δ 10.98 (s, 1H), 8.42 (d, *J =* 8.3 Hz, 1H), 8.30 (d, *J =* 1.7 Hz, 1H), 8.09 (dd, *J* = 8.2, 1.7 Hz, 1H), 7.61 (t, *J =* 7.8 Hz, 1H), 7.46 (dd, *J =* 10.0, 1.5 Hz, 1H), 7.36 (dd, *J=* 8.1, 1.5 Hz, 1H), 7.23 (d, *J* = 7.4 Hz, 1H), 7.12 (d, *J* = 25.8 Hz, 1H), 5.11 - 5.04 (m, 1H), 4.55 (d, *J =* 12.3 Hz, 1H), 4.38 - 4.26 (m, 2H), 4.18 (m, 4H), 3.60 - 3.40 (m, 4H), 3.20 (d, *J =* 60.7 Hz, 5H), 2.91 (dt, *J =* 24.4, 8.1 Hz, 2H), 2.60 (d, *J* = 16.8 Hz, 1H), 2.42 - 2.30 (d, *J* = 16.8 Hz, 1H), 2.16 (s, 2H), 2.02 - 1.89 (m, 5H), 1.76 (s, 1H), 1.57 (s, 5H).
LC-MS(ESI): [M+H]⁺ = 902.17

### Example 147

### Synthesis of compound 112

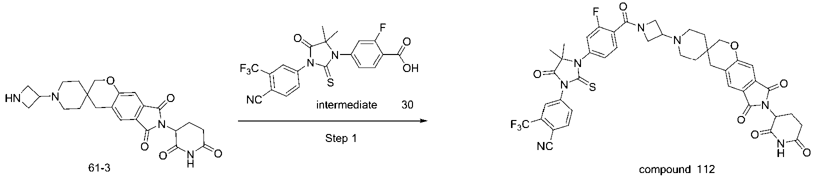

Step 1: (Compound 112) was prepared with reference to step 1 of example 116.
¹H NMR (600 MHz, MeOD) δ 8.19 (d, *J =* 7.6 Hz, 2H), 8.02 - 7.98 (m, 1H), 7.79 (t, *J =* 7.8 Hz, 1H), 7.67 (s, 1H), 7.45 (ddd, *J =* 9.8, 9.4, 1.5 Hz,2H), 7.29 (s, 1H), 5.11 (dd, *J =* 12.8, 5.4 Hz, 1H), 4.61 - 4.54 (m, 2H), 4.51 (dd, *J =* 10.7, 4.9 Hz, 1H), 4.44 (dd, *J =* 11.8, 4.8 Hz, 1H), 4.33 - 4.11 (m, 4H), 3.42 (m, 1H), 3.01 (s, 3H), 2.92 - 2.66 (m, 4H), 2.14 - 2.07 (m, 1H), 1.82 (t, *J =* 48.7 Hz, 4H), 1.62 (s, 6H).
LC-MS(ESI): [M+H]⁺ = 872.36

### Example 148

### Synthesis of compound 113

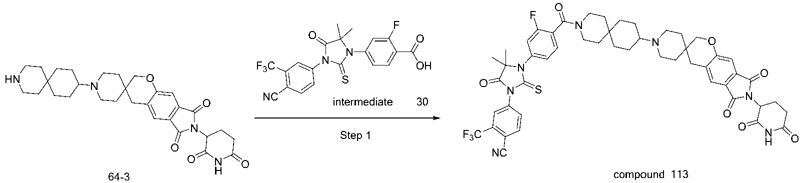

Step 1: (Compound 113) was prepared with reference to step 1 of example 116.
¹H NMR (600 MHz, DMSO-d6) δ 11.11 (d, *J =* 4.4 Hz, 1H), 8.42 (d, *J =* 8.3 Hz, 1H), 8.30 (s, 1H), 8.09 (d, *J =* 8.4 Hz, 1H), 7.64 (ddd, *J =* 31.3, 21.8, 7.8 Hz, 2H), 7.49 - 7.40 (m, 1H), 7.38 - 7.25 (m, 2H), 5.14 - 5.06 (m, 1H), 4.32 (d, *J =* 11.2 Hz, 1H), 4.03 (d, *J =* 14.1 Hz, 1H), 3.43 - 3.32 (m, 7H), 3.28 - 3.10 (m, 5H), 3.07 (d, *J =* 12.8 Hz, 1H), 2.89 (dd, *J =* 20.1, 10.2 Hz, 1H), 2.79 (d, *J =* 13.4 Hz, 1H), 2.58 (m, 2H), 2.08 - 1.98 (d, *J* = 11.2 Hz, 1H), 1.89 (s, 3H), 1.79 - 1.61 (m, 4H), 1.53 (m, 7H), 1.47 (d, *J* = 27.9 Hz, 1H), 1.40 (s, 1H), 1.32 (s, 1H), 1.26 - 1.12 (m, 2H).
LC-MS(ESI): [M+H]⁺ = 968.45

### Example 149

### Synthesis of compound 114

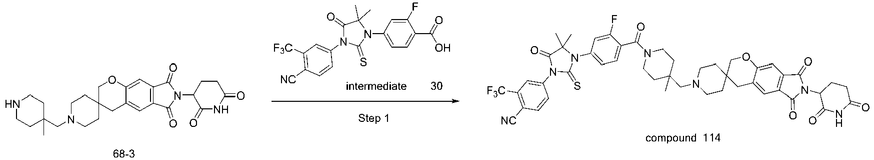

Step 1: (Compound 114) was prepared with reference to step 1 of example 116.
¹H NMR (600 MHz, DMSO-d6) δ 11.11 (s, 1H), 8.42 (d, *J =* 8.2 Hz, 1H), 8.29 (s, 1H), 8.09 (d, *J* = 8.2 Hz, 1H), 7.75 -7.61 (m, 2H), 7.46 (d, *J =* 9.9 Hz, 1H), 7.36 (d, *J =* 8.0 Hz, 1H), 7.30 (s, 1H), 5.10 (dd, *J =* 12.9, 5.4 Hz, 1H), 4.30 (s, 1H), 4.10 (s, 1H), 4.05 (s, 1H), 3.32 (s, 11H), 3.17 (s, 2H), 3.07 (s, 1H), 2.95 - 2.84 (m, 1H), 2.81 (s, 1H), 2.58 (m, 1H), 2.08 - 1.99 (m, 1H), 1.85 (dd, *J=* 34.1, 28.7 Hz, 3H), 1.71 (s, 1H), 1.64 - 1.42 (m, 10H).
LC-MS(ESI): [M+H]⁺ = 928.45

### Example 150

### Synthesis of compound 115

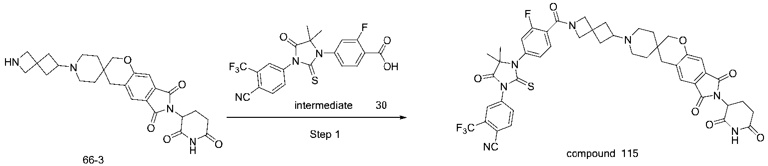

Step 1: (Compound 115) was prepared with reference to step 1 of example 116.
¹H NMR (600 MHz, DMSO-d6) δ 11.10 (s, 1H), 8.42 (d, *J=* 8.2 Hz, 1H), 8.29 (s, 1H), 8.09 (d, *J* = 8.2 Hz, 1H), 7.72 (dd, *J =* 15.9, 5.9 Hz, 1H), 7j61 (d, *J =* 7.4 Hz, 1H), 7.46 (dd, *J* = 10.4, 3.4 Hz, 1H), 7.35 (d, *J* = 6.8 Hz, 1H), 7.30 (d, *J* = 6.8 Hz, 1H), 5.10 (dd, *J =* 12.8, 5.2 Hz, 1H), 4.30 (d, *J =* 5.9 Hz, 1H), 4.18 (s, 1H), 4.09 (s, 1H), 4.05 (d, *J =* 5.0 Hz, 1H), 3.68 (m, 2H), 3.32 - 3.22 (m, 3H), 3.05 (d, *J =* 6.5 Hz, 1H), 3.02 - 2.83 (m, 3H), 2.80 (d, *J =* 5.8 Hz, 1H), 2.60 (d , *J =* 16.0 Hz, 2H), 2.48 - 2.35 (m, 2H), 2.05 - 2.00 (m, 1H), 1.78 - 1.72 (m, 1H), 1.62 (m, 8H).
LC-MS(ESI): [M+H]⁺ = 912.45

### Example 151

### Synthesis of compound 116

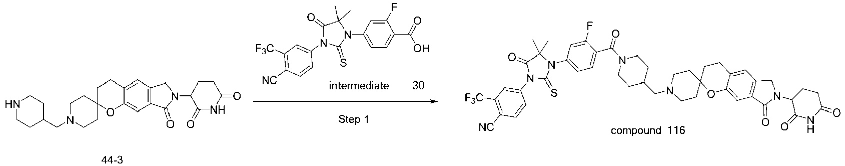

Step 1: (Compound 116) was prepared with reference to step 1 of example 116.
¹H NMR (600 MHz, DMSO-d6) δ 10.98 (s, 1H), 8.42 (d, *J =* 8.2 Hz, 1H), 8.30 (s, 1H), 8.09 (d, *J* = 8.3 Hz, 1H), 7.61 (d, *J =* 7.7 Hz, 1H), 7.46 (d, *J* = 9.9 Hz, 1H), 7.35 (d *J =* 8.3 Hz, 2H), 7.24 (s, 1H), 5.07 (dd, *J =* 13.2, 5.2 Hz, 1H), 4.55 (d, *J =* 12.6 Hz, 1H), 4.26 m, 2H), 3.46 (m, 3H), 3.30 - 3.07 (m, 6H), 2.89 (dd, *J =* 19.3, 6.0 Hz, 4H), 2.64 - 2.55 (m, 1H), 2.42 - 2.34 (m, 1H), 2.19 (s, 1H), 2.02 - 1.81 (m, 8H), 1.76 (s, 1H), 1.57 (s, 6H).
LC-MS(ESI): [M+H]⁺ = 900.45

### Example 152

### Synthesis of compound 117

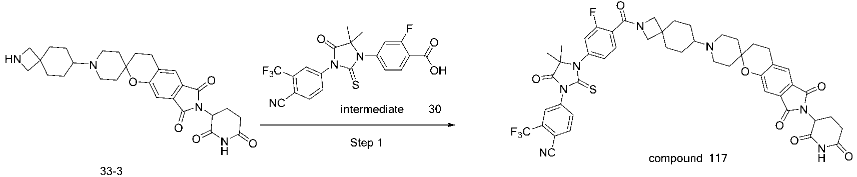

Step 1: (Compound 117) was prepared with reference to step 1 of example 116.
¹H NMR (600 MHz, DMSO-d6) δ 11.11 (d, *J =* 6.2 Hz, 1H), 8.42 (d, *J =* 8.2 Hz, 1H), 8.29 (s, 1H), 8.09 (d, *J =* 8.2 Hz, 1H), 7.80 - 7.67 (m, 2H), 7.49 - 7.42 (m, 1H), 7.40 (s, 1H), 7.38 - 7.32 (m, 1H), 5.14 - 5.02 (m, 1H), 3.85 - 3.72 (m, 5H), 3.44 - 3.15 (m, 7H), 3.00 - 2.79 (m, 4H), 2.60 (d, *J=* 18.0 Hz, 1H), 2.48 - 2.42 (m, 1H), 2.12 - 1.80 (m, 10H), 1.63 - 1.40 (m, 9H).
LC-MS(ESI): [M+H]⁺ = 940.45

### Example 153

### Synthesis of compound 118

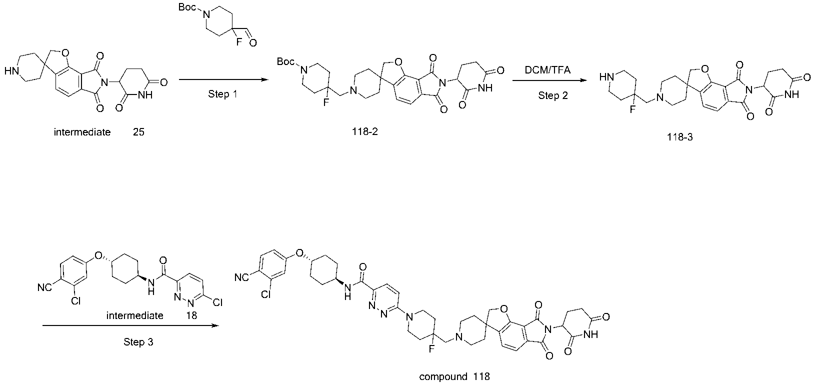

Step 1: (Compound 118-2) was prepared with reference to step 1 of example 116.
LC-MS(ESI): [M+H-C_{(4) H9}]⁺ = 529.49

Step 2: (118-3) was prepared with reference to step 2 of Example 39.
LC-MS(ESI): [M+H]⁺ = 485.29

Step 3: (Compound 118) was prepared with reference to step 3 of Example 19.
¹H NMR (600 MHz, DMSO-d6) δ 11.12 (s, 1H), 8.66 (d, *J =* 8.1 Hz, 1H), 7.87 (d, *J =* 8.8 Hz, 1H), 7.73 (s, 1H), 7.62 - 7.34 (m, 4H), 7.14 (dd, *J =* 8.8, 2.2 Hz, 1H), 5.10 (d, *J =* 8.0 Hz, 1H), 4.82 (s, 1H), 4.69 (s, 1H), 4.58 - 4.48 (m, 1H), 4.34 (d, *J* = 71.2 Hz, 2H), 3.8 (m, 1H), 3.59 (s, 2H), 3.39 (d, *J* = 6.1 Hz, 1H), 3.30 - 3.19 (m, 2H), 2.98 - 2.81 (m, 2H), 2.64 - 2.55 (m,3H), 2.49 - 2.43 (m, 1H), 2.25 (d, *J =* 46.5 Hz, 2H), 2.13-1.89 (m, 8H), 1.77 - 1.59 (m, 5H), 1.52 (dd, *J =* 22.8, 10.3 Hz, 2H).
LC-MS(ESI): [M+H]⁺ = 839.46

### Example 154

### Synthesis of compound 119

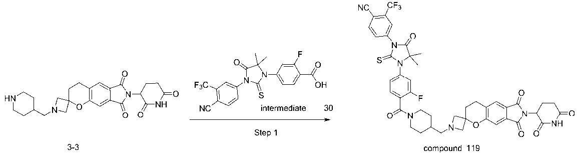

Step 1: (Compound 119) was prepared with reference to step 1 of example 116.
¹H NMR (600 MHz, DMSO-d6) δ 11.12 (s, 1H), 8.42 (d, *J =* 8.2 Hz, 1H), 8.29 (dd, *J =* 1.3 Hz, 1H), 8.09 (dd, *J =* 8.2, 1.4 Hz, 1H), 7.79 (s, 1H), 7.60 (t, *J =* 7.7 Hz, 1H), 7.46 (dd, *J =* 9.9, 1.3 Hz, 1H), 7.35 (dd, *J =* 8.1, 1.3 Hz, 1H), 7.30 (d, *J =* 12.7 Hz, 1H), 5.11 (dd, *J =* 12.9, 5.4 Hz, 1H), 4.56 - 4.29 (m, 5H), 3.33 (s, 2H ), 3.25 (s, 2H), 3.12 (s, 1H), 3.00 (d, *J =* 5.9 Hz, 1H), 2.96 - 2.79 (m, 3H), 2.64 - 2.54 (m, 2H), 2.25 (d, *J =* 5.8 Hz, 2H), 2.09 - 1.88 (m, 3H), 1.84 (d, *J =* 11.4 Hz, 1H), 1.69 (s, 1H), 1.56 (s, 6H).
LC-MS(ESI): [M+H]⁺ = 886.11

### Example 155

### Synthesis of compound 120

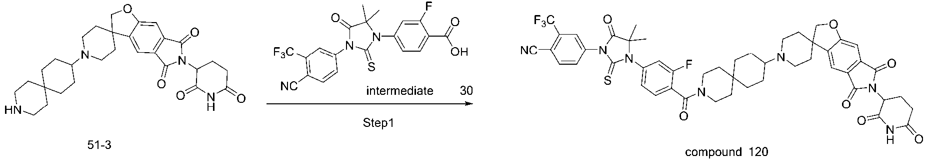

Step 1: (Compound 120) was prepared with reference to step 1 of example 116.
¹H NMR (600 MHz, DMSO-d6) δ 11.12 (d, *J =* 4.6 Hz, 1H), 8.42 (d, *J =* 8.2 Hz, 1H), 8.29 (d, *J* = 7.1 Hz, 1H), 8.09 (d, *J =* 8.3 Hz, 1H), 7.68 - 7.52 (m, 2H), 7.48 - 7.42 (m, 1H), 7.40 - 7.30 (m, 2H), 5.16 - 5.06 (m, 1H), 4.81 - 4.68 (m, 2H), 3.68 (s, 2H), 3.62 - 3.53 (m, 1H), 3.25 (dd, *J =* 12.5, 5.2 Hz, 4H), 3.17 - 3.02 (m, 2H), 2.96 - 2.83 (m, 1H), 2.64 - 2.56 (m, 2H), 2.26 - 2.14 (m, 2H), 2.09 - 1.98 (m, 3H), 1.90 (d, *J = 9.5* Hz, 4H), 1.69 (d, *J =* 11.3 Hz, 1H), 1.63 - 1.48 (m, 8H), 1.42 - 1.27 (m, 5H).
LC-MS(ESI): [M+H]⁺ = 954.11

### Example 156

### Synthesis of compound 121

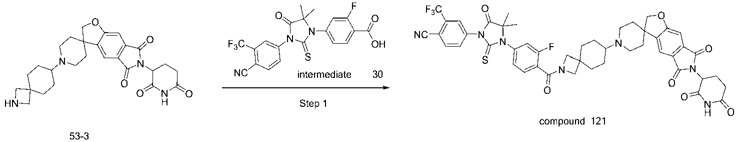

Step 1: (Compound 121) was prepared with reference to step 1 of example 116.
¹H NMR (600 MHz, DMSO-d6) δ 11.12 (d, *J =* 5.1 Hz, 1H), 8.42 (d, *J =* 8.3 Hz, 1H), 8.30 (s, 1H), 8.09 (d, *J =* 8.3 Hz, 1H), 7.74 (dt, *J =* 19.1, 7.9 Hz, 1H), 7.62 (d, *J =* 11.4 Hz, 1H), 7.50 - 7.42 (m, 1H), 7.36 (dd, *J =* 12.1, 7.9 Hz, 2H), 5.20 - 5.02 (m, 1H), 4.83 - 4.67 (m, 2H), 3.81 (t, *J =* 16.5 Hz, 2H), 3.79 - 3.70 (m, 2H), 3.54 (d, *J =* 11.1 Hz, 2H), 3.26 (s, 2H), 3.10 (m, 2H), 2.97 - 2.82 (m, 1H), 2.60 (dd, *J =* 24.1, 8.5 Hz. 1H), 2.26 - 1.81 (m, 10H), 1.66 - 1.41 (m, 9H).
LC-MS(ESI): [M+H]⁺ = 926.15

### Example 157

### Synthesis of compound 122

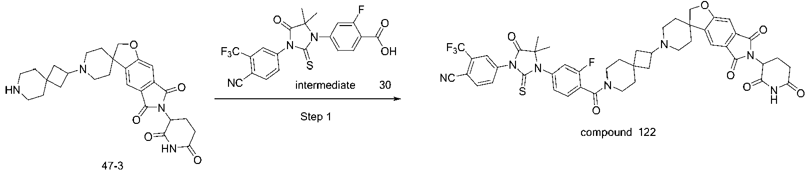

Step 1: (Compound 122) was prepared with reference to step 1 of example 116.
¹H NMR (600 MHz, DMSO-d6) δ 11.15 - 11.09 (m, 1H), 8.42 (d, *J =* 8.2 Hz, 1H), 8.30 (s, 1H), 8.09 (d, *J =* 8.4 Hz, 1H), 7.66 (t, *J =* 11.8 Hz, 1H), 7.62 (td, *J =* 8.0, 3.4 Hz, 1H), 7.46 (t, *J =* 9.9 Hz, 1H), 7.36 (dd, *J =* 12.8, 10.0 Hz, 2H), 5.16 - 5.07 (m, 1H), 4.73 (dd, *J =* 15.6, 9.0 Hz, 2H), 3.79 - 3.56 (m, 3H), 3.32 - 3.14 (m, 4H), 2.90 (dd, *J =* 16.5, 14.0 Hz, 3H), 2.63 (t, *J* = 24.7 Hz, 2H), 2.29 (m, 3H), 2.17 - 1.98 (m, 6H), 1.66 (s, 2H), 1.57 (s, 8H).
LC-MS(ESI): [M+H]⁺ = 926.11

### Example 158

### Synthesis of compound 123

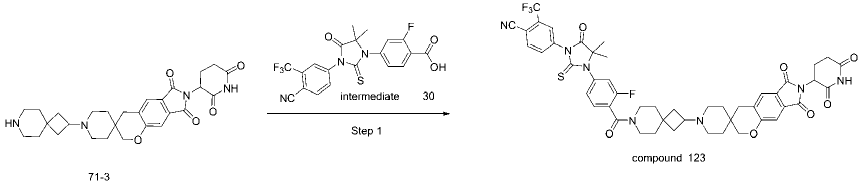

Step 1: (Compound 123) was prepared with reference to step 1 of example 116.
¹H NMR (400 MHz, DMSO-d6) δ 11.11 (s, 1H), 8.42 (d, *J =* 8.3 Hz, 1H), 8.30 (s, 1H), 8.09 (d, *J* = 8.3 Hz, 1H), 7.71 (t, *J =* 5.5 Hz, 1H), 7.64 - 7.55 (m, 1H), 7.45 (dd, *J =* 9.9, 2.5 Hz, 1H), 7.37 - 7.22 (m, 2H), 5.10 (dd, *J =* 12.7, 5.3 Hz, 1H), 4.31 (s, 1H), 4.06 (s, 1H), 3.79 (m, 2H), 3.37 - 3.13 (m, 5H), 3.09 - 2.77 (m, 6H), 2.68 - 2.55 (m, 2H), 2.25 (d, *J =* 9.4 Hz, 2H), 2.13 - 1.95 (m, 3H), 1.77 (d, *J =* 11.7 Hz, 1H), 1.66- 1.56 (s, 12H).
LC-MS(ESI): [M+H]⁺ = 940.35

### Example 159

### Synthesis of compound 124

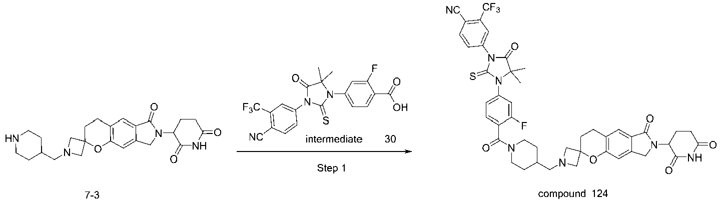

Step 1: (Compound 124) was prepared with reference to step 1 of example 116.
¹H NMR (600 MHz, DMSO-d6) δ 10.97 (s, 1H), 8.42 (d, *J =* 8.2 Hz, 1H), 8.29 (s, 1H), 8.09 (d, *J* = 8.1 Hz, 1H), 7.60 (t, *J =* 7.5 Hz, 1H), 7.53 (s, 1H), 7.45 (d, *J =* 10.0 Hz 1H), 7.35 (dd, *J =* 8.0, 1.2 Hz, 1H), 7.07 (d, *J =* 19.3 Hz, 1H), 5.07 (dd, *J =* 13.2, 4.8 Hz, 1H), 4.53 - 4.42 (m, 2H), 4.37 - 4.19 (m, 6H), 3.11 (s, 2H), 2.95 - 2.77 (m, 4H), 2.60 (d, *J =* 17.8 Hz, 1H), 2.40 - 2.28 (m, 1H), 2.27 - 2.13 (m, 2H), 2.03 - 1.88 (m, 2H), 1.83 (s, 1H), 1.69 (s, 1H), 1.56 (s, 6H), 1.32 - 1.11 (m, 3H).
LC-MS(ESI): [M+H]⁺ = 872.36

### Example 160

### Synthesis of compound 125

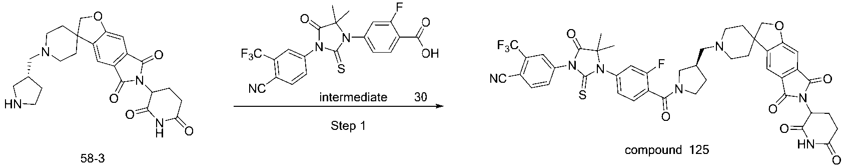

Step 1: (Compound 125) was prepared with reference to step 1 of example 116.
¹H NMR (600 MHz, DMSO-d6) δ 11.12 (d, *J =* 5.7 Hz, 1H), 8.42 (d, *J =* 8.2 Hz, 1H), 8.30 (s, 1H), 8.09 *(d, J=* 8.2 Hz, 1H), 7.74 - 7.59 (m, 2H), 7.48 (dd, *J* = 12.6, 7.5 Hz, 1H), 7.36 (dd, *J* = 16.4*,* 9.9 *Hz,* 2H), 5.17 - 5.03 (m, 1H), 4.80 - 4.67 (m, 2H), 3.91 (m, 1H), 3.39-3.31 (m, 4H), 3.23- 3.04 (m, 3H), 2.96 - 2.83 (m, 2H), 2.83 - 2.65 (m, 1H), 2.61 (d, *J =* 16.4 Hz, 1H), 2.57 - 2.52 (m, 1H), 2.35 - 2.11 (m, 3H), 2.10 - 1.97 (m, 3H), 1.97 - 1.68 (m, 2H), 1.56 (s, 6H).
LC-MS(ESI): [M+H]⁺ = 886.36

### Example 161

### Synthesis of compound 126

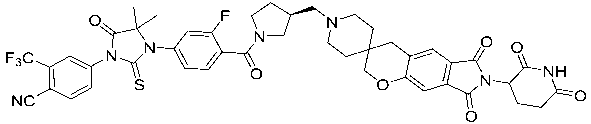

Step 1: (Compound 126) was prepared with reference to step 1 of example 116.
¹H NMR (600 MHz, DMSO-d6) δ 11.11 (d, *J =* 3.9 Hz, 1H), 8.42 (d, *J =* 8.2 Hz, 1H), 8.30 (s, 1H), 8.09 (d, J= 8.2 Hz, 1H), 7.75 - 7.58 (m, 2H), 7.47 (d, *J =* 10.1 Hz, 1H), 7.36 (dd, *J =* 7.9, 2.7 Hz, 1H), 7.31 - 7.24 (m, 1H), 5.14 - 5.04 (m, 1H), 4.32 (d, *J =* 32.5 Hz, 1H), 4.03 (d, *J =* 18.4 Hz, 1H) , 3.89 (dd, *J =* 12.0, 7.7 Hz, 1H), 3.68 (t, *J =* 8.9 Hz, 1H), 3.61 - 3.44 (m, 3H), 3.37-3.14 (m, 7H), 2.89 (dd, *J =* 21.8, 9.2 Hz, 1H), 2.80 - 2.70 (m, 2H), 2.65 - 2.57 (m, 1H), 2.16 (dd, *J =* 46.3, 3.8 Hz, 1H), 2.05 - 2.00 (m, 1H), 1.80 - 1.59 (m, 5H), 1.56 (s, 6H).
LC-MS(ESI): [M+H]⁺ = 900.46

### Example 162

### Synthesis of compound 127

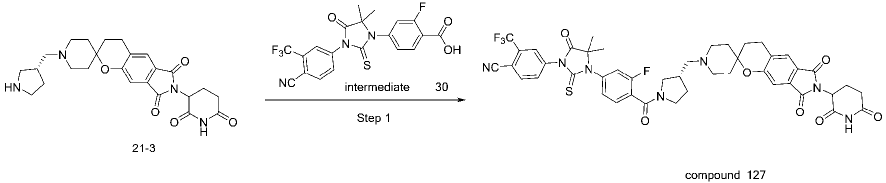

Step 1: (Compound 127) was prepared with reference to step 1 of example 116.
¹H NMR (600 MHz, DMSO-d6) δ 11.11 (d, *J =* 4.5 Hz, 1H), 8.42 (d, *J =* 8.2 Hz, 1H), 8.30 (s, 1H), 8.09 (d, *J =* 8.2 Hz, 1H), 7.75 (d, *J =* 12.2 Hz, 1H), 7.71 - 7.61 (m, 1H), 7.52 - 7.44 (m, 1H), 7.38 - 7.33 (m, 1H), 5.16 - 5.05 (m, 1H), 3.90 (dd, *J =* 11.8, 7.7 Hz, 1H), 3.69 (dd, *J =* 14.6, 5.8 Hz, 1H), 3.62 - 3.48 (m, 3H), 3.45 - 3.32 (m, 3H), 3.22 - 3.08 (m, 2H), 3.02 - 2.83 (m, 3H), 2.83 - 2.67 (m, 3H) 2.66 - 2.57 (m, 1H), 2.23 - 2.09 (m, 2H), 2.08 - 1.83 (m, 7H), 1.81 - 1.67 (m, 1H), 1.56 (s, 6H).
LC-MS(ESI): [M+H]⁺ = 900.45

### Example 163

### Synthesis of compound 128

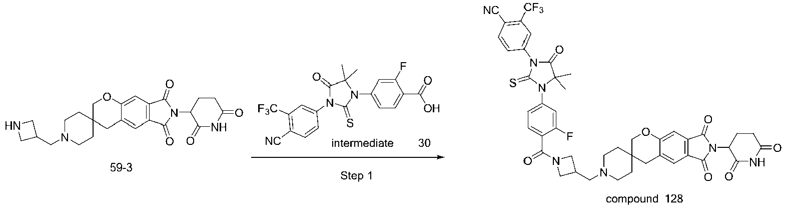

Step 1: (Compound 128) was prepared with reference to step 1 of example 116.
¹H NMR (600 MHz, DMSO-d6) δ 11.11 (s, 1H), 8.42 (d, *J* = 8.2 Hz, 1H), 8.28 (d, *J* = 18.1 Hz, 1H), 8.09 (d, *J =* 8.4 Hz, 1H), 7.78 - 7.68 (m, 1H), 7.60 (s 1H), 7.47 (d, *J =* 10.4 Hz, 1H), 7.37 (d, *J =* 8.2 Hz, 1H), 7.30 (*d, J =* 5.7 Hz, 1H), 5.10 (dd, *J* = 12.9, 5.4 Hz, 1H), 4.30 (d, *J* = 13.7 Hz, 1H), 4.25 (t, *J =* 8.8 Hz, 2H), 4.03 (s, 1H), 3.99 - 3.88 (m, 3H), 3.47 (s, 2H), 3.41 - 3.29 (m, 2H), 3.13 (m, 4H), 2.94 - 2.83 (m, 1H), 2.78 (s, 1H), 2.64 - 2.55 (m, 1H), 2.06 - 1.99 (m, 1H), 1.74 - 1.58 (m, 4H), 1.55 (s, 6H).
LC-MS(ESI): [M+H]⁺ = 886.36

### Example 164

### Synthesis of compound 129

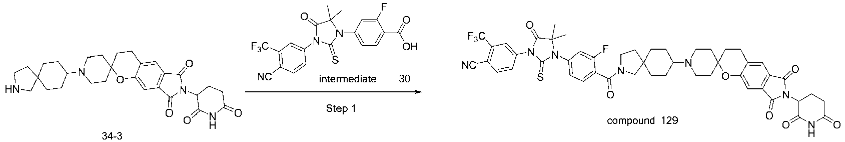

Step 1: Compound 129 was prepared with reference to step 1 of example 116.
¹H NMR (600 MHz, DMSO-d6) δ 11.11 (s, 1H), 8.41 (t, *J =* 9.4 Hz, 1H), 8.29 (d, *J =* 13.3 Hz, 1H), 8.08 (dd, *J =* 14.8, 8.5 Hz, 1H), 7.78 - 7.71 (m, 1H), 7.69 - 7.61 (m, 1H), 7.50 - 7.39 (m, 2H), 7.39 - 7.29 (m, 1H), 5.10 (m, 1H), 5.10 (m, 1H), 3.50 - 3.21 (m, 7H), 3.09 (s, 1H), 3.01 - 3.01 (m, 7H), 2.63 - 2.56 (m, 1H), 2.09 - 1.97 (m, 5H), 1.96 - 1.87 (m, 4H), 1.86 - 1.68 (m, 4H), 1.67 - 1.60 (m, 2H), 1.60 - 1.50 (m, 7H), 1.42 (dt, *J =* 23.8, 11.7 Hz, 2H).
LC-MS(ESI): [M+H]⁺ = 954.45

### Example 165

### Synthesis of compound 130

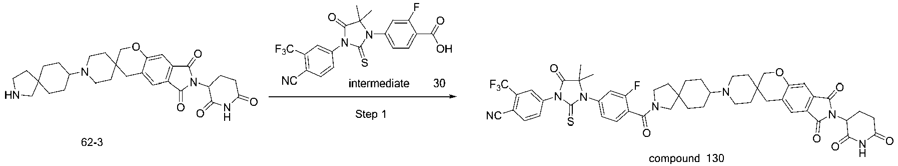

Step 1: Compound 130 was prepared with reference to step 1 of example 116.
¹H NMR (600 MHz, DMSO-d6) δ 11.11 (s, 1H), 8.42 (d, *J =* 8.2 Hz, 1H), 8.33 - 8.26 (m, 1H), 8.08 (t, *J =* 10.5 Hz, 1H), 7.78 - 7.58 (m, 2H ), 7.45 (d, *J =* 10.2 Hz, 1H), 7.35 - 7.27 (m, 2H), 5.15 - 5.03 (m, 1H), 4.33 (d, *J =* 8.5 Hz, 1H), 4.03 (t, *J =* 12.5 Hz, 1H), 3.49 - 3.30 (m, 6H), 3.27 - 3.03 (m, 6H), 2.93 - 2.83 (m, 1H), 2.77 (dd, *J =* 28.0, 9.5 Hz, 1H), 2.64 - 2.55 (m, 1H), 2.09 - 1.94 (m, 3H), 1.91 - 1.66 (m, 7H), 1.63 - 1.53 (m, 7H), 1.50 (d, *J =* 2.8 Hz, 1H), 1.45 - 1.34 (m , 2H).
LC-MS(ESI): [M+H]⁺ = 954.35

### Example 166

### Synthesis of compound 131

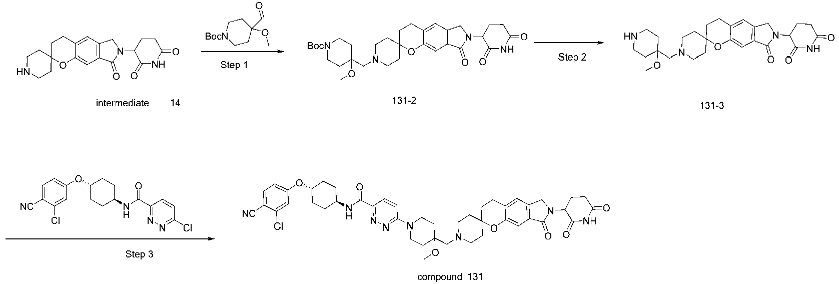

Step 1: The intermediate 14 (30 mg, 0.081 mmol) and tert-butyl 4-formyl-4-methoxypiperidine-1-carboxylate (39.52 mg, 0.162 mmol) were added sequentially to a glass vial, Ti(Oi-Pr)₄ (69.24 mg, 0.244 mmol), and the solvent THF:DMSO=4:1 (1.5 mL) were added. The reaction solution was stirred at 60°C for 1 h. Then NaBH(OAc)₃ (43.03 mg, 0.203 mmol) was added and the reaction solution was continued to be stirred at 60°C for 1 h. After the reaction was completed, the reaction solution was concentrated and then purified by TLC (DCM:MeOH=10:1) to obtain a white solid compound 131-2 (33 mg).

Step 2: (Compound 131-3) was prepared with reference to step 2 of Example 39.

Step 3: (Compound 131) was prepared with reference to step 3 of Example 19.
¹H NMR (400 MHz, DMSO-d6) δ 10.97 (s, 1H), 8.61 (d, *J =* 8.1 Hz, 1H), 7.86 (d, *J =* 9.0 Hz, 2H), 7.48 - 7.23 (m,4H), 7.14-7.08 (m, 1H), 5.06 (dd, *J =* 13.1, 4.7 Hz, 1H), 4.52 (d, *J=* 9.8 Hz, 1H), 4.41 - 4.16 (m, 6H), 3.86 (d, *J =* 7.9 Hz, 3H), 3.40 (dd, *J =* 46.7, 19.4 Hz, 7H), 2.90 (s, 3H), 2.69 - 2.56 (m, 2H), 2.15 - 1.79 (m, 12H), 1.73 - 1.42 (m, 7H).
LC-MS(ESI): [M+H]⁺ = 851.46

### Example 167

### Synthesis of compound 132

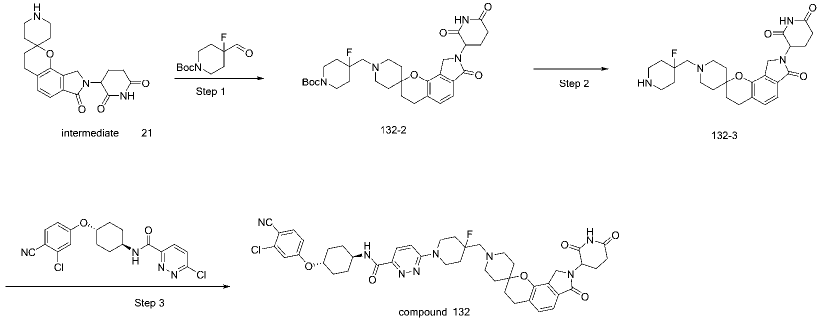

Step 1: The intermediate 21 (20 mg, 0.054 mmol) and tert-butyl 4-fluoro-4-formylpiperidine-1-carboxylate (18.78 mg, 0.081 mmol), Ti(Oi-Pr)₄ (38.47 mg, 0.135 mmol) were added sequentially to a glass vial, the solvent THF (1 mL) was added, and the reaction solution was stirred at room temperature for 1 h, then NaBH₃CN (8.51 mg, 0.135 mmol) was added and the reaction solution was continued to be stirred at room temperature for 1 h. After the reaction was completed, it was concentrated, and then purified by TLC (DCM:MeOH=10:1) to obtain a white solid compound 132-2 (23 mg).

Step 2: (Compounds 132-3) was prepared with reference to step 2 of Example 39.

Step 3: (Compound 132) was prepared with reference to step 3 of example 19.
¹H NMR (600 MHz, DMSO-d6) δ 11.03 (d, *J=* 12.9 Hz, 1H), 8.65 (d, *J=* 8.2 Hz, 1H), 7.86 (dd, *J* = 25.2, 18.2, 9.5 Hz, 2H), 7.49 (d, *J =* 9.6 Hz, 1H), 7.38 (t, *J =* 11.6 Hz, 1H), 7.27 (dd, *J =* 29.0, 7.6 Hz, 2H), 7.18 - 7.07 (m, 1H), 5.15 (dd, *J* = 29.0, 7.6 Hz, 2H) 4.54 (m, 2H), 4.48 - 4.36 (m, 3H), 4.30 (d, *J =* 17.4 Hz, 2H), 3.95 - 3.83 (m, 2H), 3.43 - 3.35 (m, 3H), 3.33 - 3.23 (m, 2H), 3.04 - 2.85 (m, 3H), 2.64 (dd *J =* 25.0, 7.5 Hz, 1H), 2.42 - 2.31 (m, 2H), 2.20 - 1.95 (m, 8H), 1.85 (dd, *J =* 61.0, 19.2 Hz, 6H), 1.65 (dd, *J =* 24.1, 10.6 Hz, 2H), 1.52 (dd, *J =* 23.0, 9.9 Hz, 2H).
LC-MS(ESI): [M+H]⁺ = 839.36

### Example 168

### Synthesis of compound 133

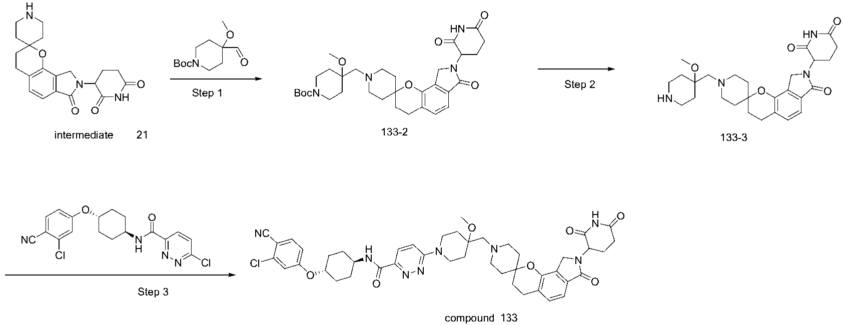

Step 1: The intermediate 21 (50 mg, 0.135 mmol) and tert-butyl 4-formyl-4-methoxypiperidine-1-carboxylate (65.86 mg, 0.271 mmol) were added sequentially in a glass vial with 2 drops of AcOH, the solvent DCE:DMSO=5:1 was added, and the reaction solution was stirred at room temperature for 1 h. Then NaBH(OAc)₃( 86.06 mg, 0.406 mmol) was added, and the reaction solution was continued to be stirred at room temperature for 1 h. When the reaction was completed, it was concentrated, and then purified by TLC (DCM:MeOH=10:1) to obtain a white solid compound 133-2 (40 mg).

Step 2: (133-3) was prepared with reference to step 2 of example 39.

Step 3: (Compound 133) was prepared with reference to step 3 of example 19.
¹H NMR (600 MHz, DMSO-d6) δ 11.00 (s, 1H), 8.63 (d, *J =* 8.2 Hz, 1H), 8.22 (s, 1H), 7.88 - 7.79 (m, 2H), 4.57 - 4.49 (m, 1H), 4.32 (t, *J =* 14.9 Hz, 1H), 4.17 (dd, *J =* 35.1, 14.9 Hz, 3H), 3.85 (dd, *J =* 11.4, 7.6 Hz, 1H), 3.28 (d, *J =* 11.4 Hz, 4H), 3.16 (d, *J =* 31.1 Hz, 3H), 2.96 - 2.79 (m, 4H), 2.74 - 2.65 (m, 2H ), 2.41 (d, *J =* 21.8 Hz, 2H), 2.11 (d, *J =* 10.5 Hz, 2H), 1.93 (m, 7H), 1.76 (s, 1H), 1.71 - 1.42 (m, 10H).
LC-MS(ESI): [M+H]⁺ = 851.36

### Example 169

### Synthesis of intermediate 43

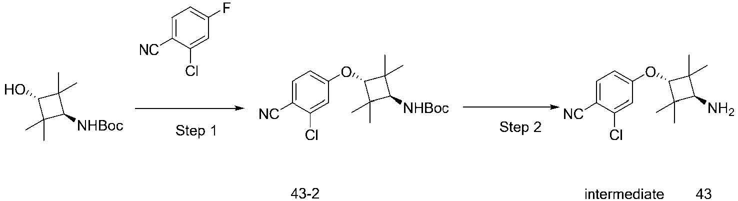

Step 1: In a glass vial, tert-butyl (1r,3r)-3-hydroxy-2,2,4,4-tetramethylcyclobutyl)carbamate (2 g, 8.22 mmol) and solvent DMF (3 mL) were added, sodium hydrogen (657.44 mg, 16.44 mmol) was added in batches at 0°C with stirring at 0°C for 1 h. Then 4-chloro-2-fluorobenzonitrile (1.40g, 9.04mmol) was added and continued for 1 h at room temperature with stirring, after the reaction was completed, the reaction was quenched with water (100 mL), extracted with ethyl acetate (3x100 mL), and the organic phase was concentrated and purified by normal phase to obtain the white solid compound 43-2 (2.3 g).
LC-MS: [M+H]⁺ = 378.9

Step 2: (Intermediate 43) was prepared with reference to step 2 of Example 254.
LC-MS: [M+H]⁺ =278.8

### Example 170

### Synthesis of intermediate 44

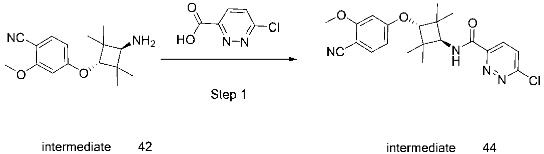

Step 1: (Intermediate 44) was prepared with reference to step 1 of Example 249.
LC-MS: [M+H]⁺ =414.89

### Example 171

### Synthesis of compound 134

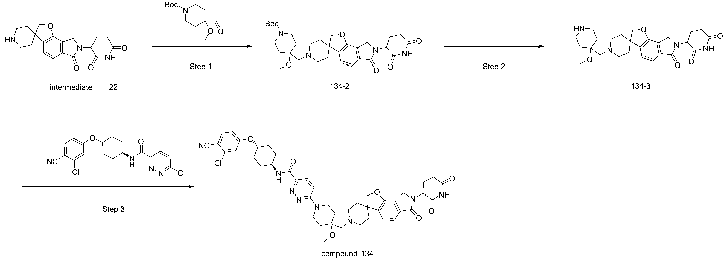

Step 1: The intermediate 22 (50.0 mg, 140.69 µmol) and tert-butyl 4-formyl-4-methoxypiperidine-1-carboxylate (136.92 mg, 562.76 µmol) were added sequentially to a glass vial, the solvents DCE:DMSO = 5 : 1 (1.2 mL) and acetic acid (1 drop) were added, and the reaction solution was stirred at room temperature for 1 h. Then NaBH(OAc)₃₍89.45 mg, 422.07 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 134-2 (100 mg, crude).
LC-MS: [M+H]⁺ =583.36

Step 2: (Compound 134-3) was prepared with reference to step 2 of example 39.
LC-MS: [M+H]⁺ =483.29

Step 3: (Compound 134) was prepared with reference to step 3 of Example 19.
¹H NMR (400 MHz, *DMSO-d*₆) δ 11.02 (s, 1H), 8.67 (d, *J =* 8.2 Hz, 1H), 7.87 (d, *J =* 9.0 Hz, 2H), 7.48 - 7.34 (m, 3H), 7.27 (d, *J =* 7.6 Hz, 1H), 7.14 (dd , *J =* 8.8, 2.5 Hz, 1H), 5.10 (dd, *J =* 13.3, 5.1 Hz, 1H), 4.74 - 4.61 (m, 2H), 4.50-4.57 (m, 1H), 4.40 (d, *J =* 17.2 Hz, 1H), 4.21-4.29 (m, 3H), 3.62 (d, *J =* 11.8 Hz, 3H), 3.41 - 3.20 (m, 9H), 2.88 - 2.97 (m, 1H), 2.65 - 2.55 (m, 1H), 2.47 - 2.28 (m, 3H), 2.17 - 2.05 (m, 2H), 2.05 - 1.83 (m, 7H), 1.72 - 1.59 (m, 4H), 1.58 - 1.44 (m, 2H).
LC-MS: [M+H]⁺ =837.36

### Example 172

### Synthesis of compound 135

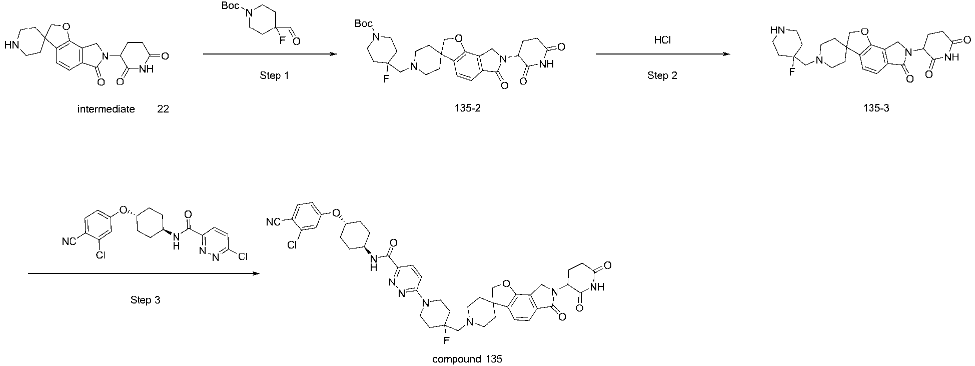

Step 1: The intermediate 22 (50.0 mg, 140.69 µmol) and tert-butyl 4-fluoro-4-formylpiperidine-1-carboxylate (65.07 mg, 281.38 µmol) were added sequentially in a glass vial, and the solvents DCE:DMSO = 5 : 1 (1.2 mL) and acetic acid (1 drop) were added, and the reaction solution was stirred at room temperature for 1 h, and then NaBH( OAc)₃ (89.45 mg, 422.07 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 135-2 (100 mg, crude).
LC-MS: [M+H]⁺ =571.50

Step 2: (Compound 135-3) was prepared with reference to step 2 of Example 39.
LC-MS: [M+H]⁺ =471.29

Step 3: (Compound 135) was prepared with reference to step 3 of Example 19.
¹H NMR (400 MHz, *DMSO-d*₆) δ 11.02 (s, 1H), 8.70 (d, *J =* 8.2 Hz, 1H), 7.88 (dd, *J =* 9.2, 6.5 Hz, 2H), 7.49 (*d, J =* 9.6 Hz, 1H), 7.40 *(d, J =* 2.4 Hz, 1H), 7.36 (d, *J =* 7.5 Hz, 1H), 7.28 (d, *J =* 7.9 Hz, 1H), 7.14 (dd, *J* = 8.8, 2.4 Hz, 1H), 5.10 (dd, *J =* 13.3, 5.1 Hz, 1H), 4.67 (s, 2H), 4.50-4.57 (m, 1H), 4.36-4.42 (m, 3H), 4.23 (d, *J =* 17.2 Hz, 1H), 3.54-3.62 (m, 4H), 3.38 (t, *J =* 12.3 Hz, 3H), 3.24 (s, 2H), 2.87-2.96 (m, 1H), 2.65 - 2.55 (m, 1H), 2.46 - 2.23 (m, 3H), 2.17 - 2.03 (m, 4H), 2.02 - 1.76 (m, 7H), 1.60-1.70 (m, 2H), 1.57 - 1.44 (m, 2H).
LC-MS: [M+H]⁺ =825.36

### Example 173

### Synthesis of compound 136

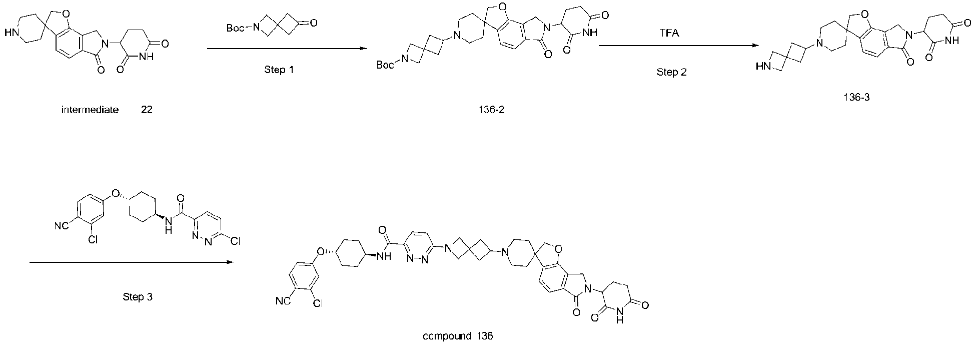

Step 1: The intermediate 22 (50.0 mg, 140.69 µmol) and tert-butyl 6-oxo-2-azaspiro[3.3]heptane-2-carboxylate (59.44 mg, 281.38 µmol) were added sequentially to a glass vial with solvent DCE:DMSO = 3 : 1 (1.6 mL), and the reaction solution was stirred at room temperature for 1 h, then NaBH( OAc)₃(89.45 mg, 422.07 µmol) and 1 drop of acetic acid were added, and the reaction solution was continued to be stirred at room temperature for 1 h. After the reaction was completed, the reaction solution was concentrated, and then purified by preparative thin-layer chromatography (DCM:MeOH=10:1) to obtain a white solid compound 136-2 (40 mg, 51.63%).
LC-MS: [M+H]⁺ =551.38

Step 2: (Compound 136-3) was prepared with reference to step 2 of example 39.
LC-MS: [M+H]⁺ =451.40

Step 3: (Compound 136) was prepared with reference to step 3 of Example 19.
¹H NMR (600 MHz, *DMSO-d*₆) δ 11.01 (s, 1H), 8.56 (d, *J =* 8.2 Hz, 1H), 7.91 - 7.83 (m, 2H), 7.43 - 7.27 (m, 3H), 7.14 (dd, *J =* 8.8, 2.4 Hz, 1H), 6.90 (d, *J =* 9.3 Hz, 1H), 5.11 (dd, *J =* 13.2, 5.0 Hz, 1H), 4.71 - 4.62 (m, 2H), 4.62 - 4.50 (m, 2H), 4.40 (d, *J =* 17.1 Hz, 1H), 4.29 - 4.21 (m, 3H), 4.16 (s, 2H), 3.91 - 3.82 (m, 1H), 3.72 - 3.65 (m, 1H), 2.99 - 2.85 (m, 3H), 2.71 - 2.54 (m, 5H), 2.45 - 2.32 (m, 2H), 2.14 - 1.95 (m, 6H), 1.95 - 1.81 (m, 3H), 1.64 (dd, *J =* 23.5, 11.1 Hz. 2H), 1.52 (dd, *J =* 22.8, 9.8 Hz, 2H).
LC-MS: [M+H]⁺ =805.46

### Example 174

### Synthesis of compound 137

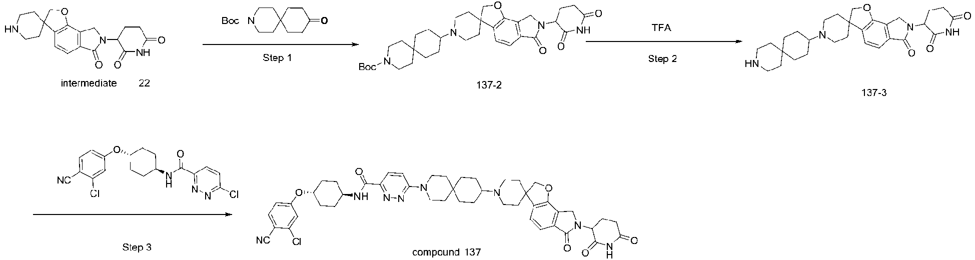

Step 1: The intermediate 22 (50.0 mg, 140.69 µmol) and tert-butyl 9-oxo-3-azaspiro[5.5]undecane-3-carboxylate (75.23 mg, 281.38 µmol) were added sequentially to a glass vial with the solvent DCE:DMSO = 3 : 1 (1.6 mL), and the reaction solution was stirred at room temperature for 1 h, followed by addition of NaBH ( OAc)₃ (89.45 mg, 422.07 µmol) and 1 drop of acetic acid, and the reaction solution was continued to be stirred at 50°C for 1 h. After the reaction was completed, the reaction solution was concentrated and then purified by preparative thin-layer chromatography (DCM:MeOH=10:1) to obtain a yellow solid compound 137-2 (40 mg, 46.86%).
LC-MS: [M+H]⁺ =607.48

Step 2: (Compound 137-3) was prepared with reference to step 2 of Example 39.
LC-MS: [M+H]⁺ =507.50

Step 3: (Compound 137) was prepared with reference to step 3 of Example 19.
¹H NMR (600 MHz, *DMSO-d*₆) δ 11.02 (s, 1H), 8.60 (d, *J =* 8.2 Hz, 1H), 7.91 - 7.76 (m, 2H), 7.42 - 7.31 (m, 3H), 7.31 - 7.24 (m, 1H), 7.14 (dd, *J =* 8.8, 2.4 Hz, 1H), 5.11 (dd, *J =* 13.3, 5.0 Hz, 1H), 4.68 (q, *J =* 9.6 Hz, 2H), 4.61 - 4.48 (m, 2H), 4.40 (d, *J =* 17.2 Hz, 1H), 4.24 (d, *J* = 17.2 Hz, 1H), 3.90 - 3.81 (m, 4H), 3.61 - 3.51 (m, 2H), 3.30 - 3.20 (m, 1H), 3.13 (dd, *J =* 22.9, 10.7 Hz, 2H), 2.97 - 2.88 (m, 1H), 2.79 - 2.63 (m, 1H), 2.61 (d, *J =* 17.0 Hz, 1H), 2.48 - 2.37 (m, 1H), 2.21 (t, *J =* 12.3 Hz, 2H), 2.16 - 2.07 (m, 2H), 2.04 - 1.96 (m, 3H), 1.96 - 1.84 (m, 5H), 1.74 - 1.57 (m, 6H), 1.52 (dd, *J =* 22.9, 9.9 Hz, 2H), 1.42 (dd, *J =* 22.9, 9.9 Hz, 2H), 1.27 - 1.17 (m, 2H).
LC-MS: [M+H]⁺ =861.46

### Example 175

### Synthesis of compound 138

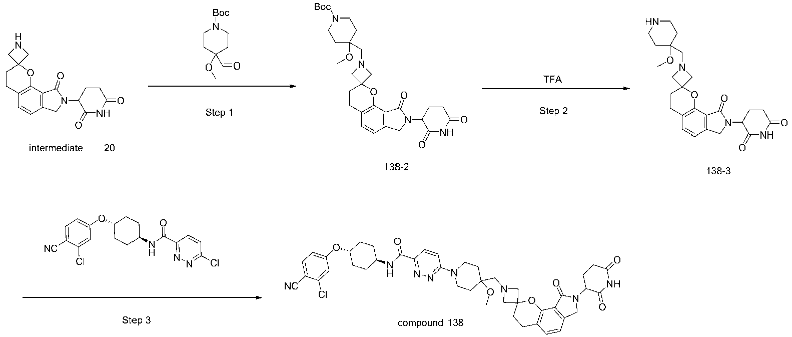

Step 1: The intermediate 20 (40.0 mg, 117.18 µmol) and tert-butyl 4-formyl-4-methoxypiperidine-1-carboxylate (57.02 mg, 234.35 µmol) were added sequentially into a glass vial with solvent DCE:DMSO = 5 : 1 (1.2 mL), and the reaction solution was stirred at room temperature for 1 h, followed by addition ofNaBH(OAc)₃ (74.50 mg, 351.53 µmol), and the reaction solutioin was continued to be stirred at room temperature for 1 h. After the reaction was completed, it was concentrated, and then purified by preparative thin-layer chromatography (DCM:MeOH=9:1) to obtain a yellow oily compound 138-2 (41 mg, 61.53%).
LC-MS: [M+H]⁺ =569.38

Step 2: (Compound 138-3) was prepared with reference to step 2 of Example 39.
LC-MS: [M+H]⁺ =469.45

Step 3: (Compound 138) was prepared with reference to step 3 of Example 19.
¹H NMR (600 MHz, *DMSO-d*₆) δ 10.98 (d, *J =* 5.7 Hz, 1H), 8.63 (d, *J =* 8.2 Hz, 1H), 7.86 (t, *J =* 9.3 Hz, 2H), 7.45 - 7.32 (m, 3H), 7.18 - 7.05 (m, 2H), 4.97 (dd, *J =* 13.0, 5.2 Hz, 1H), 4.62 (s, 1H), 4.56 - 4.46 (m, 2H), 4.39 - 4.30 (m, 2H), 4.27 - 4.16 (m, 4H) , 3.90 - 3.83 (m, 2H), 3.56 (s, 1H), 3.36 - 3.22 (m, 5H), 2.94 - 2.79 (m, 3H), 2.64 - 2.55 (m, 1H), 2.44 - 2.34 (m, 1H), 2.31 - 2.19 (m, 2H), 2.11 (d, *J =* 10.1 Hz, 2H), 2.02 - 1.86 (m, 3H), 1.82 (d, *J =* 13.3 Hz, 2H), 1.70 - 1.46 (m, 6H).
LC-MS: [M+H]⁺ =823.50

### Example 176

### Synthesis of compound 139

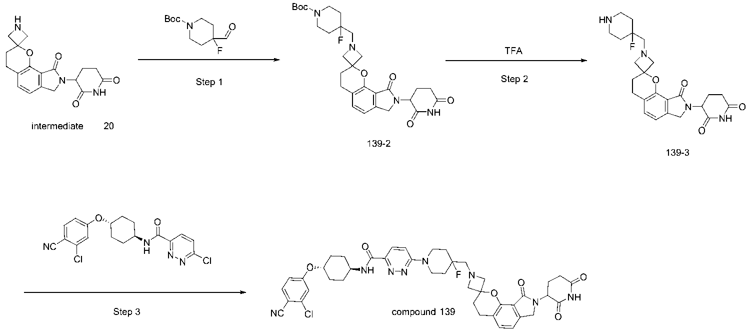

Step 1: The intermediate 20 (40.0 mg, 117.18 µmol) and tert-butyl 4-fluoro-4-formylpiperidine-1-carboxylate (54.20 mg, 234.35 µmol) were added sequentially into a glass vial with solvent DCE:DMSO = 5 : 1 (1.2 mL), and the reaction solution was stirred at room temperature for 1 h, then NaBH(OAc)₃( 74.50 mg, 351.53 µmol) was added, and the reaction solution was continued to be stirred at room temperature for 1 h. After the reaction was completed, it was concentrated, and then purified by preparative thin-layer chromatography (DCM:MeOH=9:1) to obtain a yellow oily compound 139-2 (80 mg, crude).
LC-MS: [M+H]⁺ =557.38

Step 2: (Compound 139-3) was prepared with reference to step 2 of Example 39.
LC-MS: [M+H]⁺ =457.40

Step 3: (Compound 139) was prepared with reference to step 3 of Example 19.
¹H NMR (600 MHz, *DMSO-d*₆) *δ* 10.98 (s, 1H), 8.65 (d, *J =* 8.2 Hz, 1H), 7.87 (dd, *J =* 9.1, 4.5 Hz, 2H), 7.50 - 7.32 (m, 3H), 7.17 - 7.06 (m, 2H), 4.97 (dd, 1H), 4.62 (s, 1H), 4.57 - 4.47 (m, 2H), 4.43 - 4.33 (m, 4H), 4.24 (d, *J =* 17.3 Hz, 3H), 3.91 - 3.82 (m, 3H), 3.31 (dd, *J* = 9.1, 4.5 Hz, 3H), 2.94 - 2.78 (m, 3H), 2.60 (d, *J* = 17.3 Hz, 1H), 2.54 (s, 1H), 2.44 - 2.34 (m, 1H), 2.30 - 2.20 (m, 2H), 2.15 - 2.07 (m, 2H), 2.01 - 1.92 (m, 3H), 1.92 - 1.87 (m, 2H), 1.65 (dd, *J =* 24.1, 10.8 Hz, 2H), 1.57 - 1.46 (m, 2H).
LC-MS: [M+H]⁺ =811.46

### Example 177

### Synthesis of compound 140

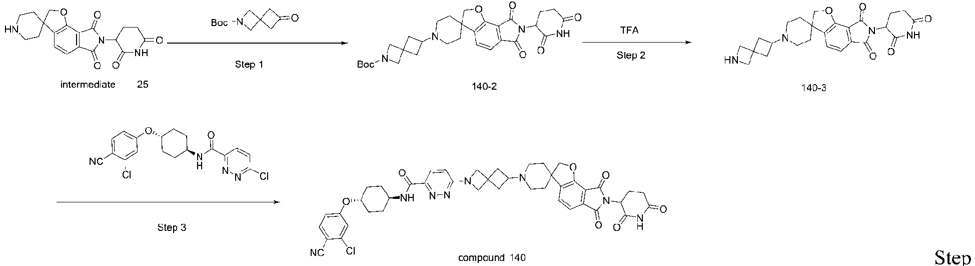

1: The intermediate 25 (30 mg, 81.22 µmol) and tert-butyl 6-oxo-2-azaspiro[3.3]heptane-2-carboxylate (34.32 mg, 162.44 µmol) were added sequentially to a glass vial, the solvent THF:DMSO= 5 : 1 (1.2 mL) was added, and finally 1 drop of acetic acid was added, and the reaction solution was stirred at room temperature for 2.0 h. Then NaBH₃CN (10.21 mg, 162.44 µmol) was added and the reaction solution was continued to be stirred at room temperature for 2.0 h. After the reaction was completed, the reaction solution was concentrated and then purified by preparative thin-layer chromatography (DCM:MeOH=10:1) to obtain a yellow oily compound 140-2 (90 mg, crude).
LC-MS: [M+H]⁺ =565.38

Step 2: (Compound 140-3) was prepared with reference to step 2 of Example 39.
LC-MS: [M+H]⁺ =465.45

Step 3: (Compound 140) was prepared with reference to step 3 of Example 19.
¹H NMR (600 MHz, *DMSO-d*₆) *δ* 11.11 (s, 1H), 8.55 (d, *J =* 8.1 Hz, 1H), 7.87 (d, *J =* 8.8 Hz, 2H), 7.60 (d, *J =* 7.3 Hz, 1H), 7.51 (d, *J=* 7.3 Hz, 1H), 7.39 (d, *J =* 2.3 Hz, 1H), 7.14 (dd, *J* = 8.8, 2.3 Hz, 1H), 6.90 (d, *J* = 9.2 Hz, 1H), 5.12 (dd, *J* = 9.2 Hz, 1H), 4.85 - 4.74 (m, 2H), 4.61 - 4.50 (m, 2H). 4.26 (s, 2H), 4.15 (s, 2H), 3.47 (d, *J =* 11.2 Hz, 2H), 3.36 (s, 1H), 3.00 - 2.81 (m, 3H), 2.71 - 2.63 (m, 2H), 2.63 - 2.54 (m, 3H), 2.28 - 2.07 (m, 5H), 2.07 - 1.97 (m, 2H), 1.91 (d, *J =* 10.1 Hz, 2H), 1.70 - 1.59 (m, 2H), 1.56 - 1.47 (m, 3H).
LC-MS: [M+H]⁺ =819.46

### Example 178

### Synthesis of compound 141

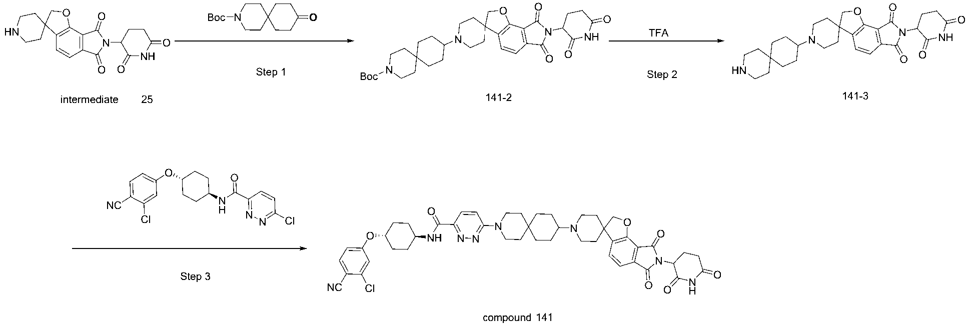

Step 1: The intermediate 25 (50 mg, 135.36 µmol) and tert-butyl 9-oxo-3-azaspiro[5.5]undecane-3-carboxylate (54.29 mg, 203.04 µmol) were added sequentially to a glass vial, and the solvent THF (2.0 mL) was added, and finally tetraisopropyl titanate (76.95 mg, 270.73 µmol) was added. The reaction solution was stirred at room temperature for 1 h, then NaBH₃CN (17.01 mg, 270.73 µmol) was added, and the reaction solution was continued to be stirred at room temperature for 1 h. After the reaction was completed, it was concentrated, and then purified by preparative thin-layer chromatography (DCM:MeOH=10:1) to obtain a yellow solid compound 141-2 (52 mg, 61.89%).
LC-MS: [M+H]⁺ =621.46

Step 2: (Compound 141-3) was prepared with reference to step 2 of Example 39.
LC-MS: [M+H]⁺ =521.50

Step 3: (Compound 141) was prepared with reference to step 3 of Example 19.
¹H NMR (600 MHz, *DMSO-d*₆) δ 11.12 (s, 1H), 8.59 (d, *J =* 8.2 Hz, 1H), 7.84 (dd, *J =* 25.3, 9.2 Hz, 2H), 7.59 (d, *J* = 7.3 Hz, 1H), 7.50 (d, *J =* 7.3 Hz, 1H), 7.41 - 7.32 (m, 2H), 7.14 (dd, *J =* 8.7, 2.3 Hz, 1H), 5.11 (dd, *J =* 12.9, 5.4 Hz, 1H), 4.79 (s, 2H), 4.61 - 4.49 (m, 2H), 3.92 - 3.82 (m, 1H), 3.72 (s, 4H), 3.29 - 3.20 (m, 2H), 3.18 - 3.08 (m, 2H), 2.95 - 2.82 (m, 1H), 2.60 (d, *J =* 17.1 Hz, 1H), 2.57 - 2.52 (m, 1H), 2.27 - 2.15 (m, 2H), 2.13 - 2.09 (m, 2H), 2.09 - 2.00 (m, 3H), 2.00 - 1.84 ( m, 6H), 1.74 - 1.57 (m, 6H), 1.57 - 1.48 (m, 4H), 1.48 - 1.39 (m, 2H), 1.24 (s, 1H).
LC-MS: [M+H]⁺ =876.75

### Example 179

### Synthesis of compound 142

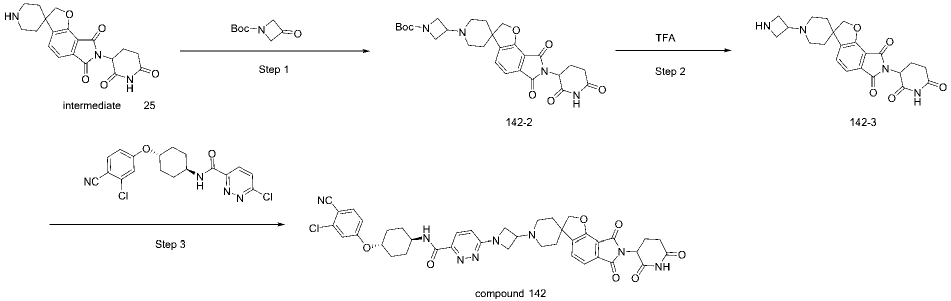

Step 1: The intermediate 25 (80 mg, 216.58 µmol) and tert-butyl 3-oxoazetidine-1-carboxylate (55.62 mg, 324.87 µmol) were added sequentially to a glass vial, and the solvent THF (2.0 mL) was added, and tetraisopropyl titanate (123.11 mg, 433.16 µmol) was added in the end, and the reaction solution was stirred at room temperature for 1.5 h. Then NaBH₃CN (40.83 mg, 649.74 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 142-2 (70 mg, 61.61%).
LC-MS: [M+H]⁺ =525.89

Step 2: (Compound 142-3) was prepared with reference to step 2 of example 39.
LC-MS: [M+H]⁺ =425.30

Step 3: (Compound 142) was prepared with reference to step 3 of Example 19.
¹H NMR (600 MHz, *DMSO-d*₆) δ 11.12 (s, 1H), 8.64 (d, *J =* 8.2 Hz, 1H), 7.95 (d, *J =* 9.2 Hz, 1H), 7.87 (d, *J =* 8.8 Hz, 1H), 7.61 (s, 1H), 7.50 (d, *J =* 7.3 Hz, 1H), 7.40 (d, *J =* 2.4 Hz, 1H), 7.15 (d, *J =* 2.4 Hz, 1H), 6.99 (d, *J =* 9.2 Hz, 1H), 5.12 (dd, *J =* 12.9, 5.4 Hz, 1H), 4.83 (s, 2H), 4.62 - 4.50 (m, 2H), 4.50 - 4.34 (m, 4H), 4.27 (s, 1H), 3.94 - 3.81 (m, 2H), 3.05 (s, 1H), 2.93 - 2.85 (m, 1H), 2.66 - 2.57 (m, 1H), 2.57 - 2.53 (m, 1H), 2.23 - 1.96 (m, 7H), 1.91 (d, *J =* 10.7 Hz, 2H), 1.71 - 1.60 (m, 2H), 1.57 - 1.48 (m, 3H ).
LC-MS: [M+H]⁺ =779.46

### Example 180

### Synthesis of compound 143

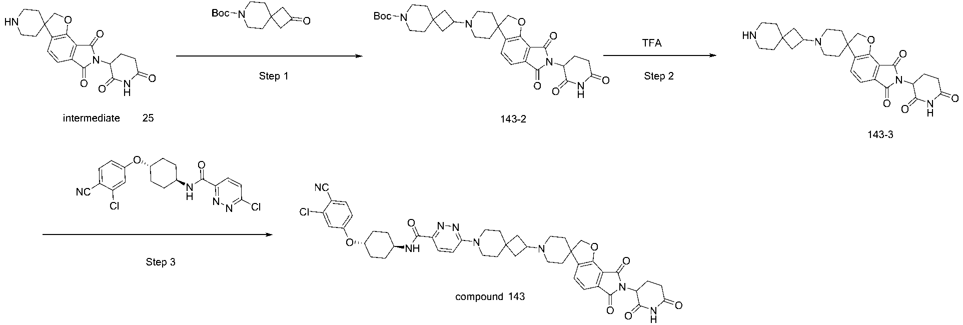

Step 1: The intermediate 25 (60 mg, 162.44 µmol) and tert-butyl 2-oxo-7-azaspiro[3.5]nonane-7-carboxylate (58.31 mg, 243.65 µmol) were added sequentially to a glass vial, and the solvent THF (2.0 mL) was added, and tetraisopropyl titanate (92.33 mg, 324.87 µmol) was added in the end. The reaction solution was stirred at room temperature for 1 h, then NaBH₃CN (31.62 mg, 487.31 µmol) was added, and the reaction solution was continued to be stirred at room temperature for 1 h. After the reaction was completed, it was concentrated, and then purified by preparative thin-layer chromatography (DCM:MeOH=10:1) to obtain a light yellow oily compound 143-2 (50 mg, 51.93%).
LC-MS: [M+H]⁺ =593.45

Step 2: (Compound 143-3) was prepared with reference to step 2 of Example 39.
LC-MS: [M+H]⁺ =493.45

Step 3: (Compound 143) was prepared with reference to step 3 of Example 19.
¹H NMR (600 MHz, *DMSO-d*₆) δ 11.12 (s, 1H), 8.60 (d, *J =* 8.2 Hz, 1H), 7.85 (dd, *J =* 14.9, 9.2 Hz, 2H), 7.60 (d, *J* = 7.4 Hz, 1H), 7.51 (d, *J =* 7.3 Hz, 1H), 7.42 - 7.35 (m, 2H), 7.14 (dd, *J =* 8.8, 2.4 Hz, 1H), 5.12 (dd, *J =* 12.8, 5.4 Hz, 1H), 4.87 - 4.73 (m, 2H), 4.59 - 4.47 (m, 1H), 3.92 - 3.81 (d, *J* = 7.4 Hz, 1H), 3.75 (s, 3H), 3.67 (s, 4H), 3.47 (d, *J =* 11.7 Hz, 2H), 3.01 - 2.81 (m, 3H), 2.69 - 2.54 (m, 2H), 2.36 - 2.17 (m, 3H), 2.17 - 1.97 (m, 6H), 1.90 (d, *J =* 11.1 Hz, 2H), 1.71 - 1.45 (m, 6H).
LC-MS: [M+H]⁺ =847.46

### Example 181

### Synthesis of compound 144

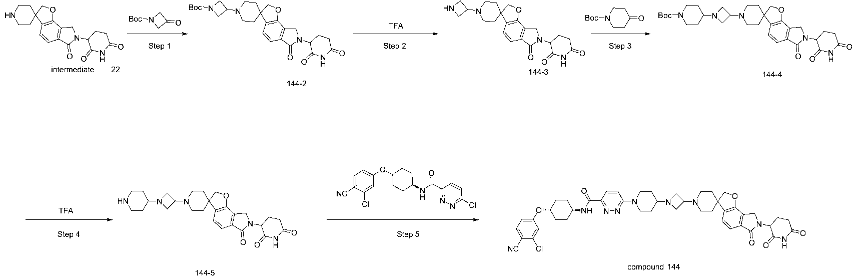

Step 1: The intermediate 22 (60 mg, 168.83 µmol) and tert-butyl 3-oxoazetidine-1-carboxylate (43.35 mg, 253.24 µmol) were added sequentially to a glass vial, and the solvent THF (2.0 mL) was added, and tetraisopropyl titanate (95.97 mg, 337.65 µmol) was added in the end, and the reaction solution was stirred at room temperature for 1.5 h. Then NaBH₃CN (31.83 mg, 506.48 µmol) was added and the reaction solution was continued to be stirred at room temperature for 1 h. After the reaction was completed, the reaction solution was concentrated and then purified by preparative thin-layer chromatography (DCM:MeOH=10:1) to obtain a white solid compound 144-2 (65 mg, 75.40%).
LC-MS: [M+H]⁺ =511.39

Step 2: (Compound 144-3) was prepared with reference to step 2 of Example 39.
LC-MS: [M+H]⁺ =411.40

Step 3: (Compound 144-4) Compound 144-3 (50 mg, 121.81 µmol) and tert-butyl 4-oxopiperidine-1-carboxylate (36.41 mg, 182.72 µmol) were added sequentially to a glass vial, the solvent THF (2.0 mL) was added, and tetraisopropyl titanate (69.24 mg, 243.62 µmol) was added in the end. The reaction solution was stirred at room temperature for 1.5 h, then NaBH₃CN (22.96 mg, 365.43 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 144-4 (30 mg, 41.48%).
LC-MS: [M+H]⁺ =594.55

Step 4: (Compound 144-5) was prepared with reference to step 2 of Example 39.
LC-MS: [M+H]⁺ =494.50

Step 5: (Compound 144) was prepared with reference to step 3 of Example 19.
¹H NMR (600 MHz, CD₃OD) δ 7.98 (d, *J =* 9.6 Hz, 1H), 7.72 (d, *J =* 8.8 Hz, 1H), 7.42 (dd, *J =* 8.6, 3.8 Hz, 2H), 7.37 (d, *J =* 7.6 Hz, 1H), 7.22 (d, *J =* 2.3 Hz, 1H), 7.07 ( dd, *J =* 8.8, 2.3 Hz, 1H), 5.16 (dd, *J =* 13.3, 5.1 Hz, 1H), 4.71 (d, *J =* 13.6 Hz, 2H), 4.66 - 4.58 (m, 2H), 4.53 (d, *J =* 4.0 Hz, 1H), 4.51 - 4.41 (m, 2H), 4.38 (dd, *J =* 17.5, 6.7 Hz, 2H), 4.28 - 4.23 (m, 2H), 4.04 - 3.97 (m, 1H), 3.63 - 3.54 (m, 2H), 3.16 (t, *J =* 12.4 Hz, 2H), 3.08 (d, *J =* 11.0 Hz, 2H), 2.96 - 2.87 (m, 1H), 2.82 - 2.75 (m, 1H), 2.56 - 2.48 (m, 2H), 2.32 (t, *J =* 11.6 Hz , 2H), 2.26 - 2.07 (m, 8H), 1.97 - 1.88 (m, 2H), 1.71 - 1.61 (m, 4H), 1.55 - 1.45 (m, 2H).
LC-MS: [M+H]⁺ =848.56

### Example 182

### Synthesis of compound 145

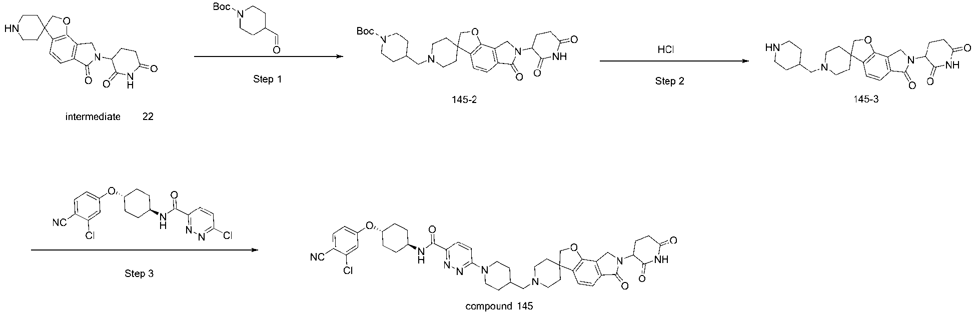

Step 1: The intermediate 22 (50.0 mg, 140.69 µmol) and tert-butyl 4-formylpiperidine-1-carboxylate (45.01 mg, 211.03 µmol) were added sequentially to a glass vial, the solvent THF (2.0 mL) was added, and then tetraisopropyl titanate (79.97 mg, 281.38 µmol) was added in the end, and the reaction solution was stirred at room temperature for 1.5 h. Then NaBH₃CN (26.52 mg, 422.07 µmol) was added and the reaction solution was continued to be stirred at room temperature for 1 h. After the reaction was completed, the reaction solution was concentrated and then purified by preparative thin-layer chromatography (DCM:MeOH=10:1) to obtain a white solid compound 145-2 (20 mg, 25.72%).
LC-MS: [M+H]⁺ =553.50

Step 2: (Compound 145-3) was prepared with reference to step 2 of Example 39.
LC-MS: [M+H]⁺ =453.45

Step 3: (Compound 145) was prepared with reference to step 3 of Example 19.
¹ H NMR (600 MHz, *DMSO-d*₆ *)* δ 10.99 (s, 1H), 8.60 (d, *J =* 8.2 Hz, 1H), 7.85 (dd, *J =* 21.6, 9.1 Hz, 2H), 7.43 - 7.19 (m, 4H), 7.14 (dd, *J =* 8.8, 2.4 Hz, 1H), 5.10 (dd, *J =* 13.2, 5.1 Hz, 1H), 4.69 - 4.44 (m, 4H), 4.43 - 4.19 (m, 2H), 3.92 - 3.78 (m, 1H), 3.05 (t, *J =* 11.9 Hz, 2H), 2.98 - 3.78 (m, 1H), 3.05 (t, *J =* 11.9 Hz 2H), 2.98 - 2.79 (m, 2H), 2.65 - 2.53 (m, 2H), 2.47 - 2.35 (m, 2H), 2.25 - 2.03 (m, 4H), 2.03 - 1.94 (m, 2H), 1.94 - 1.72 (m, 5H), 1.65 (dd, *J =* 23.9, 10.8 Hz, 2H), 1.57 - 1.37 (m, 3H), 1.35 (d, *J=* 8.0 Hz, 1H), 1.32 - 1.02 (m, 5H).
LC-MS: [M+H]⁺ =807.46

### Example 183

### Synthesis of compound 146

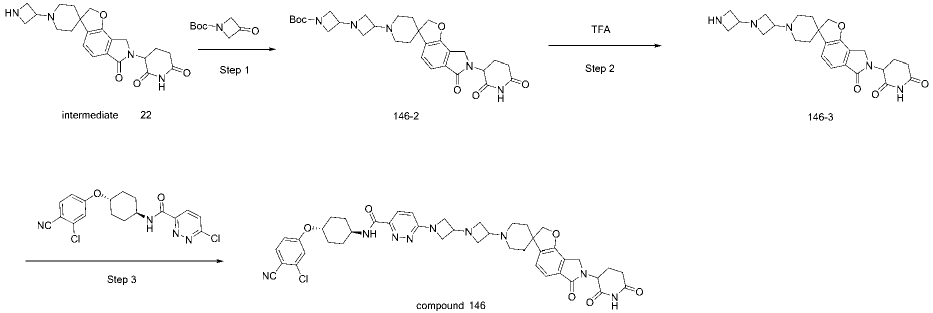

Step 1: The intermediate 22 (58 mg, 141.30 µmol) and tert-butyl 3-oxoazetidine-1-carboxylate (48.36 mg, 282.60 µmol) were added sequentially to a glass vial, solvent THF (1.0 mL) was added, and tetraisopropyl titanate (80.32 mg, 282.60 µmol) was added in the end, and the reaction solution was stirred at room temperature for 1.5 h.Then NaBH₃CN (26.64 mg, 423.90 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 146-2 (14 mg, 21.28%).
LC-MS: [M+H]⁺ =566.50

Step 2: (Compound 146-3) was prepared with reference to step 2 of Example 39.
LC-MS: [M+H]⁺ =466.45

Step 3: (Compound 146) was prepared with reference to step 3 of Example 19.
¹H NMR (600 MHz, *DMSO-d*₆) δ 10.98 (s, 1H), 8.56 (d, *J =* 8.2 Hz, 1H), 8.14 (s, 1H), 7.86 (t, *J =* 17.3, 8.7 Hz, 2H), 7.47 - 7.35 (m, 1H), 7.29 (d, *J =* 7.6 Hz, 1H), 7.14 (dd, *J =* 8.8, 2.3 Hz, 1H), 5.09 (dd, *J =* 13.3, 5.1 Hz, 1H), 4.58 - 4.50 (m, 2H), 4.38 (d, *J =* 17.1 Hz, 1H), 4.25 - 4.20 (m , 1H), 4.17 (t, *J =* 7.8 Hz, 1H), 3.96 - 3.82 (m, 4H), 3.75 (s, 1H), 3.53 (s, 2H), 3.12 (d, *J =* 6.5 Hz, 2H), 2.96 - 2.88 (m, 2H), 2.86 - 2.78 (m, 2H), 2.14 - 2.07 (m, 3H), 2.03 - 1.87 (m, 6H), 1.77 - 1.70 (m, 2H), 1.67 - 1.60 (m, 2H), 1.55 - 1.47 (m, 2H), 1.38 - 1.32 (m, 2H).
LC-MS: [M+H]⁺ =820.50

### Example 184

### Synthesis of compound 147

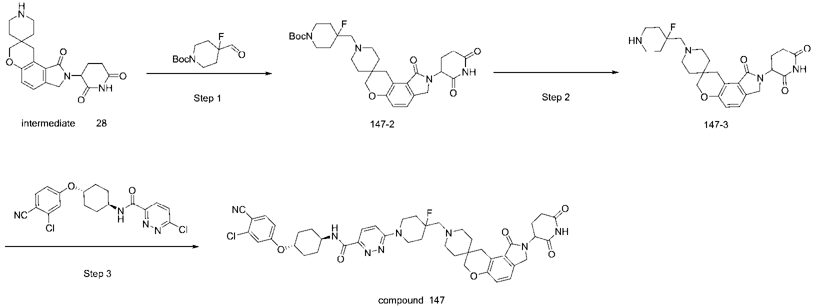

Step 1: The intermediate 28 (45 mg, 121.81 µmol) and tert-butyl 4-fluoro-4-formylpiperidine-1-carboxylate (84.51 mg, 365.44 µmol) were added sequentially to a glass vial, the solvents DCE:DMSO= 4 : 1 (1.5 mL) and DIEA (1 drop) were added, and the reaction solution was stirred at room temperature for 1 h, followed by addition of NaBH(OAc)₃ (64.54 mg, 304.53 µmol) and acetic acid (1 drop), and the reaction solution was continued to be stirred at room temperature for 1 h. After the reaction was completed, it was concentrated, and then purified by preparative thin-layer chromatography (DCM:MeOH=10:1) to obtain a white solid compound 147-2 (51 mg).
LC-MS: [M+H]⁺ = 585.38

Step 2: (Compound 147-3) was prepared with reference to step 2 of Example 39.
LC-MS: [M+H]⁺= 485.29

Step 3: (Compound 147) was prepared with reference to step 3 of Example 19.
¹H NMR (600 MHz, *DMSO-d*₆) δ 11.00 (s, 1H), 8.65 (d, *J =* 8.2 Hz, 1H), 7.87 (dd, *J =* 12.8, 9.1 Hz, 2H), 7.48 (d, *J* = 9.6 Hz, 1H), 7.39 (d, *J =* 2.4 Hz, 1H), 7.33 (d, *J =* 8.2 Hz, 1H), 7.13 (dd, *J =* 8.8, 2.4 Hz, 1H), 7.06 (d, *J =* 8.2 Hz, 1H), 5.03 (d, *J =* 15.5 Hz, 1H), 4.54 (s, *J =* 10.0, 4.3 Hz, 1H), 4.35 (dd, *J =* 33.3, 15.1 Hz, 3H), 4.21 (d *J =* 13.4 Hz, 2H), 3.87 (d, *J =* 15.3, 7.8, 3.7 Hz, 2H), 3.41 - 3.13 (m, 6H), 3.05 - 2.98 (m, 1H), 2.89 (d, *J* = 18.1, 13.5, 5.5 Hz, 1H), 2.61 (d, *J =* 17.4, 3.4 Hz, 1H), 2.45 - 2.34 (m, 1H), 2.15 - 1.95 (m, 5H), 1.93 - 1.72 (m, 7H), 1.66 (pd, *J =* 15.5, 13.1, 4.1 Hz. 3H), 1.57 - 1.43 (m, 2H), 1.36 - 1.12 (m, 3H).
LC-MS: [M+H]⁺= 840.46

### Example 185

### Synthesis of compound 148

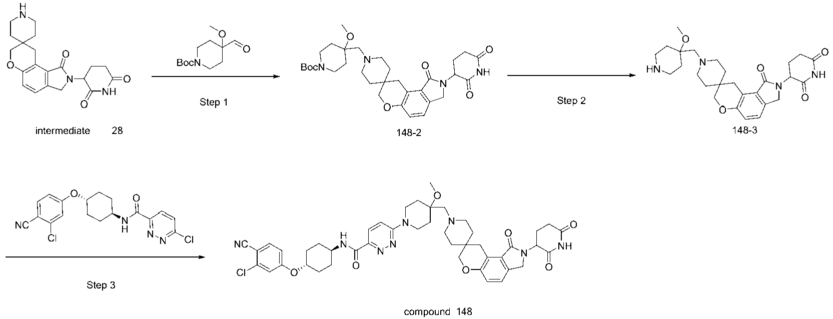

Step 1: The intermediate 28 (70 mg, 189.49 µmol) and tert-butyl 4-formyl-4-methoxypiperidine-1-carboxylate (138.31 mg, 568.46 µmol) were added sequentially to a glass vial, and the solvents DCE:DMSO= 4 : 1 (1.5 mL) and DIEA (1 drop) were added, and the reaction solution was stirred at room temperature for 1 h. Then NaBH(OAc)₃ (100.40 mg, 473.71 µmol) and acetic acid (1 drop) were added, and the reaction solution was continued to be stirred at room temperature for 1 h. After the reaction was completed, the reaction solution was concentrated, and then purified by preparative thin-layer chromatography (DCM:MeOH=10:1) to obtain a white solid compound 148-2 (57 mg).
LC-MS: [M+H]⁺ = 597.28

Step 2: (Compound 148-3) was prepared with reference to step 2 of Example 39.
LC-MS: [M+H]⁺= 497.29

Step 3: (Compound 148) was prepared with reference to step 3 of Example 19.
¹H NMR (600 MHz, *DMSO-d*₆) δ 11.00 (s, 1H), 8.63 (d, *J =* 8.2 Hz, 1H), 7.86 (d, *J =* 8.8 Hz, 1H), 7.81 (d, *J =* 9.5 Hz, 1H), 7.40 (d, *J =* 2.4 Hz, 1H), 7.35 (d, *J =* 9.6 Hz, 1H), 7.28 (d, *J =* 8.2 Hz, 1H), 7.14 (d, *J* = 8.8, 2.4 Hz, 1H), 7.00 (d, *J =* 8.8, 2.4 Hz, 1H), 7.00 (d, *J =* 8.8, 2.4 Hz, 1H) 1H), 5.02 (dd, *J =* 13.2, 5.2 Hz, 1H), 4.54 (tt, *J =* 10.2, 4.2 Hz, 1H), 4.30 (d, *J =* 16.8 Hz 1H), 4.16 (dd, *J =* 34.8, 14.7 Hz, 3H), 3.92 - 3.83 (m, 3H), 3.29 (dd, *J =* 24.7, 2.8 Hz, 2H), 3.18 (s, 3H), 3.07 (s, 2H), 2.93 - 2.85 (m 1H), 2.61 (tt, *J* = 14.2, 10.7, 5.2 Hz, 3H), 2.48 - 2.34 (m, 5H), 2.14 - 2.08 (m, 2H), 2.02 - 1.95 (m, 1H), 1.93 - 1.81 (m, 4H), 1.69 - 1.33 (m, 10H).
LC-MS: [M+H]⁺= 852.36

### Example 186

### Synthesis of compound 149

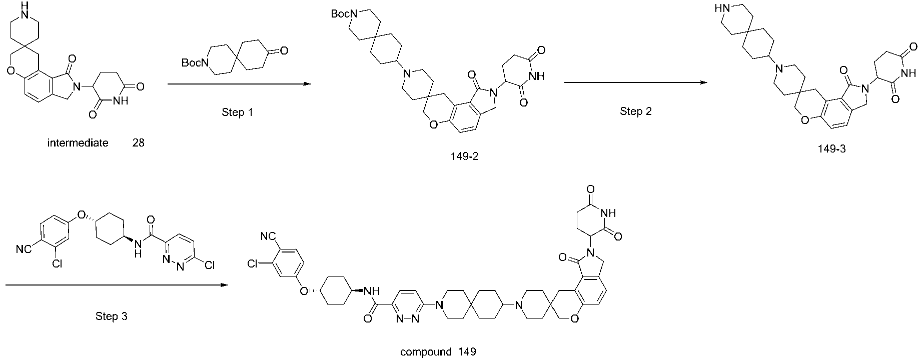

Step 1: The intermediate 28 (70 mg, 189.49 µmol) and tert-butyl 9-oxo-3-azaspiro[5.5]undecane-3-carboxylate (138.31 mg, 568.46 µmol) were added sequentially to a glass vial, and the solvents DCE:DMSO= 4 : 1 (1.5 mL) and DIEA (1 drop) were added, the reaction solution was stirred at room temperature for 1 h. Then NaBH(OAc)₃ (100.40 mg, 473.71 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 149-2 (57 mg).
LC-MS: [M+H]⁺ = 621.48

Step 2: (Compound 149-3) was prepared with reference to step 2 of Example 39.
LC-MS: [M+H]⁺= 521.39

Step 3: (Compound 149) was prepared with reference to step 3 of Example 19.
¹H NMR (600 MHz, *DMSO-d*₆) δ 11.00 *(d, J =* 2.6 Hz, 1H), 8.61 - 8.58 (m, 1H), 7.87 (d, *J =* 8.8 Hz, 1H), 7.82 (dd, *J* = 9.5, 2.5 Hz, 1H), 7.40 (d, *J =* 2.5 Hz, 1H), 7.37 - 7.32 (m, 2H), 7.14 (d, *J =* 8.8, 2.4 Hz, 1H), 7.06 (dd,*J* = 8.2, 2.7 Hz, 1H), 5.03 (dd, *J =* 12.9, 5.2 Hz, 1H), 4.54 (tt, *J =* 10.1, 4.4 Hz, 1H), 4.33 (d, *J =* 17.0, 6.2 Hz, 1H), 4.21 (d, *J =* 15.6 Hz, 2H), 3.93 - 3.84 (m, 2H), 3.75 - 3.68 (m, 4H), 3.20 (s, 2H), 3.04 (dd, *J =* 27.7, 10.0 Hz, 3H), 2.90 (ddt, *J =* 18.9, 11.9, 5.8 Hz, 2H), 2.61 (d, *J =* 18.9 Hz, 2H), 2.43 - 2.35 (m, 2H), 2.14 - 2.08 (m, 2H), 2.01 - 1.86 (m, 7H), 1.80 (d, *J =* 9.1 Hz, 2H), 1.76 - 1.57 (m, 9H), 1.52 (dt, *J =* 13.8, 10.1 Hz, 2H), 1.41 (q, *J =* 6.2 Hz, 2H).
LC-MS: [M+H]⁺=876.56

### Example 187

### Synthesis of compound 150

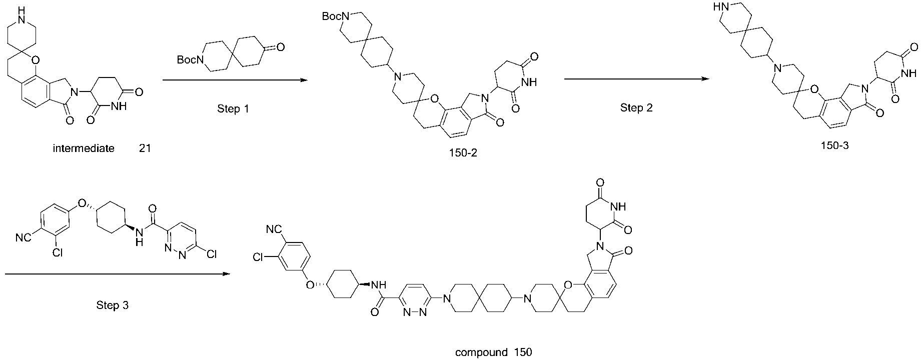

Step 1: The intermediate 21 (70 mg, 189.49 µmol), tert-butyl 9-oxo-3-azaspiro[5.5]undecane-3-carboxylate (101.33 mg, 378.97 µmol), Ti(OiPr)₄ (80.78 mg, 284.23 µmol) were added sequentially to a glass vial, the solvent THF:DMSO= 4 : 1 (1.5 mL) was added, and the reaction solution was stirred at room temperature for 1 h. Then NaBH₃CN (29.77 mg, 473.71 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 150-2 (68 mg).
LC-MS: [M+H]⁺ = 621.48

Step 2: (Compound 150-3) was prepared with reference to step 2 of Example 39.
LC-MS: [M+H]⁺= 521.39

Step 3: (Compound 150) was prepared with reference to step 3 of Example 19.
¹H NMR (600 MHz, *DMSO-d*₆) δ 11.01 *(d, J =* 16.6 Hz, 1H), 8.58 (d, *J =* 8.2 Hz, 1H), 7.87 (d, *J =* 8.7 Hz, 1H), 7.82 (d, *J =* 9.5 Hz, 1H), 7.40 (d, *J = 2.4* Hz, 1H), 7.35 (d, *J =* 9.6 Hz, 1H), 7.30 (d, *J =* 7.8 Hz, 1H), 7.25 (d, *J =* 7.6 Hz, 1H), 7.14 (dd, *J =* 8.8, 2.4 Hz, 1H), 5.12 (dd, *J =* 13.2, 5.2 Hz, 1H), 4.54 (tt, *J =* 10.1, 4.4 Hz, 1H), 4.43 (d, *J =* 17.4 Hz, 1H), 4.28 (d, *J =* 17.4 Hz, 1H), 3.86 (tdt, *J =* 12.0, 8.7, 4.0 Hz, 2H), 3.53 - 3.38 (m, 4H), 3.29 - 3.16 (m, 3H), 2.93 (ddt, *J =* 31.3, 13.2, 5.9 Hz, 3H), 2.62 (dd, *J =* 22.0, 9.5 Hz, 1H), 2.38 (qd, *J =* 13.3, 4.6 Hz, 1H), 2.16 - 1.84 (m, 15H), 1.64 (ddd, *J =* 34.9, 18.7, 9.1 Hz, 6H), 1.52 (dt, *J =* 12.8, 9.4 Hz, 2H), 1.41 (t, *J =* 5.6 Hz, 2H), 1.22 (tt, *J =* 13.3, 5.3 Hz, 3H).
LC-MS: [M+H]⁺=876.66

### Example 188

### Synthesis of compound 151

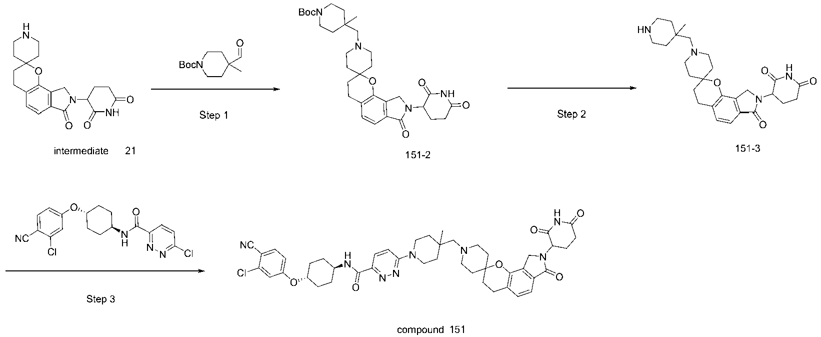

Step 1: The intermediate 21 (70 mg, 189.49 µmol), tert-butyl 4-formyl-4-methylpiperidine-1-carboxylate (86.14 mg, 378.97 µmol), Ti(OiPr)₄ (80.78 mg, 284.23 µmol) were added sequentially to a glass vial, the solvent THF:DMSO= 4 : 1 (1.5 mL) was added, and the reaction solution was stirred at room temperature for 1 h. Then NaBH₃CN (29.77 mg, 473.71 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 151-2 (55 mg).
LC-MS: [M+H]⁺ = 581.48

Step 2: (Compound 151-3) was prepared with reference to step 2 of Example 39.
LC-MS: [M+H]⁺= 481.39

Step 3: (Compound 151) was prepared with reference to step 3 of Example 19.
¹H NMR (600 MHz, Methanol-*d*₄) δ 7.97 (d, *J =* 9.5 Hz, 1H), 7.71 (d, *J =* 8.7 Hz, 1H), 7.36 (td, *J =* 25.1, 8.7 Hz, 3H), 7.22 (d, *J= 2.4* Hz, 1H), 7.06 (dd, *J* = 8.8, 2.4 Hz, 1H), 5.19 (dd, *J =* 13.2, 5.2 Hz, 1H), 4.58 - 4.41 (m, 3H), 4.22 (d, *J =* 13.9 Hz, 2H), 4.00 (s, 1H), 3.67 - 3.44 (m, 5H), 3.31 (s, 4H), 3.05 - 2.90 (m, 3H), 2.81 (ddd, *J =* 17.8, 4.7, 2.3 Hz, 1H), 2.49 (qd, *J =* 13.1, 4.4 Hz, 1H), 2.27 - 1.97 (m, 10H), 1.77 (s, 3H), 1.66 (q, *J =* 12.5, 10.9 Hz, 4H), 1.35 (d, *J =* 53.8 Hz, 4H).
LC-MS: [M+H]⁺=835.46

### Example 189

### Synthesis of compound 152

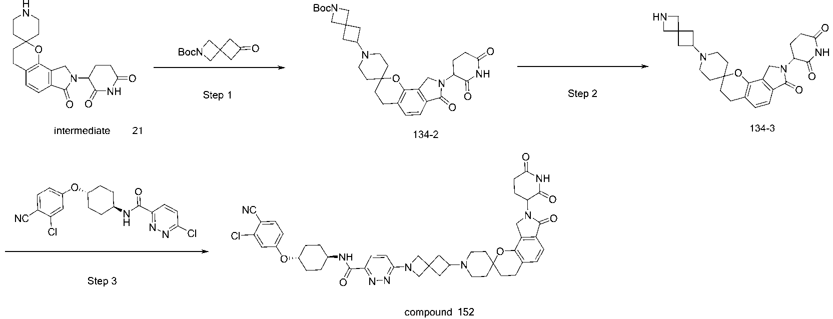

Step 1: The intermediate 21 (70 mg, 189.49 µmol), tert-butyl 6-oxo-2-azaspiro[3.3]heptane-2-carboxylate (80.06 mg, 378.97 µmol), Ti(OiPr)₄ (80.78 mg, 284.23 µmol) were added sequentially to a glass vial, the solvents THF:DMSO= 4 : 1 ( 1.5 mL) was added, and the reaction solution was stirred at room temperature for 1 h. Then NaBH₃CN (29.77 mg, 473.71 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 152-2 (73 mg).
LC-MS: [M+H]⁺ = 565.48

Step 2: (Compound 152-3) was prepared with reference to step 2 of Example 39.
LC-MS: [M+H]⁺= 465.39

Step 3: (Compound 152) was prepared with reference to step 3 of Example 19.
¹H NMR (600 MHz, Methanol-*d*₄) δ 8.00 (d, *J =* 9.3 Hz, 1H), 7.71 (d, *J =* 8.7 Hz, 1H), 7.39 - 7.29 (m, 2H), 7.22 (d, *J =* 2.4 Hz, 1H), 7.06 (dd, *J =* 8.7, 2.4 Hz, 1H), 6.99 (d, *J =* 9.3 Hz, 1H), 5.20 (dd, *J =* 13.4, 5.1 Hz, 1H), 4.57 - 4.44 (m, 3H), 4.38 (s, 2H), 4.28 (s, 2H), 3.99 (d, *J =* 4.4 Hz, 1H), 3.88 (s, 1H), 3.48 (d, *J =* 12.1 Hz, 2H), 3.19 (d, *J =* 16.1 Hz, 2H), 3.07 - 2.92 (m, 3H), 2.87 - 2.77 (m, 3H), 2.68 - 2.50 (m, 3H), 2.22 (d, *J =* 9.0 Hz, 5H), 2.15 - 1.93 (m, 6H), 1.72 - 1.58 (m, 4H).
LC-MS: [M+H]⁺=819.46

### Example 190

### Synthesis of compound 153

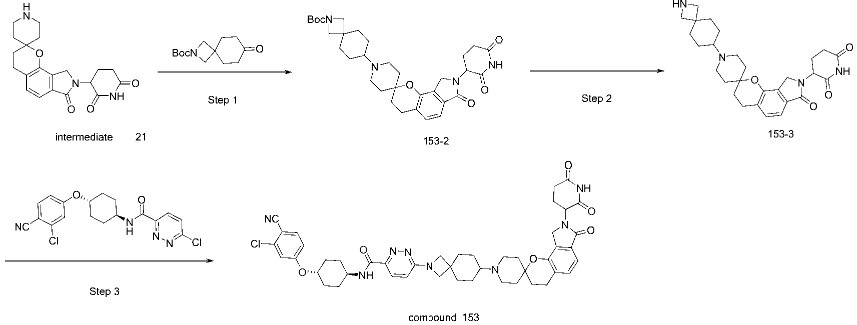

Step 1: The intermediate 21 (70 mg, 189.49 µmol), tert-butyl 7-oxo-2-azaspiro[3.5]nonane-2-carboxylate (90.69 mg, 378.97 µmol), Ti(OiPr)₄ (80.78 mg, 284.23 µmol) were added sequentially to a glass vial, the solvent THF:DMSO= 4 : 1 (1.5 mL) was added, and the reaction solution was stirred at room temperature for 1 h. Then NaBH₃CN (29.77 mg, 473.71 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 153-2 (61 mg).
LC-MS: [M+H]⁺ = 593.48

Step 2: (Compound 153-3) was prepared with reference to step 2 of Example 39.
LC-MS: [M+H]⁺= 493.39

Step 3: (Compound 153) was prepared with reference to step 3 of Example 19.
¹H NMR (600 MHz, Methanol-*d*₄) δ 8.00 (d, *J* = 9.5 Hz, 1H), 7.71 (d, *J =* 8.7 Hz, 1H), 7.39 - 7.30 (m, 2H), 7.22 (d, *J = 2.3* Hz, 1H), 7.06 (dd, *J =* 8.8, 2.4 Hz, 1H), 7.01 (dd, *J =* 9.6*,* 3.2 Hz, 1H), 5.19 (dd, *J =* 13.4, 5.2 Hz, 1H), 4.56 - 4.44 (m, 3H), 4.06 (s, 2H), 3.97 (s, 3H), 3.55 (d, *J =* 12.0 Hz, 2H), 3.48 - 3.37 (m, 3H), 3.03 - 2.96 (m, 2H), 2.83 - 2.78 (m, 1H), 2.52 (dd, *J =* 13.3, 4.6 Hz, 1H), 2.26 (d, *J =* 44.2 Hz, 9H), 2.11 (s, 3H), 2.03 (t, *J =* 6.6 Hz, 4H), 1.79 (t, *J =* 12.8 Hz, 2H), 1.67 (q, *J =* 11.5, 9.3 Hz, 6H).
LC-MS: [M+H]⁺= 847.56

### Example 191

### Synthesis of compound 154

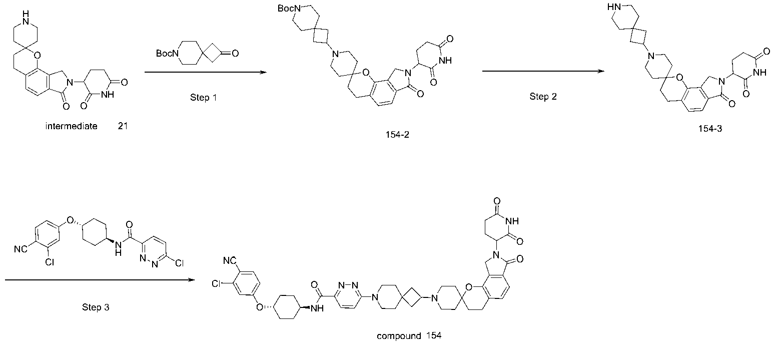

Step 1: The intermediate 21 (70 mg, 189.49 µmol), tert-butyl 2-oxo-7-azaspiro[3.5]nonane-7-carboxylate (90.69 mg, 378.97 µmol), and Ti(OiPr)₄ (80.78 mg, 284.23 µmol) were added sequentially into a glass vial, the solvent THF:DMSO = 4 : 1 ( 1.5 mL) was added, and the reaction solution was stirred at room temperature for 1 h. Then NaBH₃CN (29.77 mg, 473.71 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 154-2 (65 mg).
LC-MS: [M+H]⁺ = 593.58

Step 2: (Compound 154-3) was prepared with reference to step 2 of Example 39.
LC-MS: [M+H]⁺= 493.49

Step 3: (Compound 154) was prepared with reference to step 3 of Example 19.
¹H NMR (600 MHz, Methanol-*d*₄) δ 7.92 (d, *J* = 9.5 Hz, 1H), 7.71 (d, *J =* 8.7 Hz, 1H), 7.40 - 7.26 (m, 3H), 7.22 (d, *J =* 2.4 Hz, 1H), 7.06 (dd, *J =* 8.8, 2.4 Hz, 1H), 5.19 (dd, *J =* 13.4, 5.1 Hz, 1H), 4.57 - 4.42 (m, 3H), 3.99 (s, 1H), 3.78 (dt, *J =* 45.9, 5.3 Hz, 5H), 3.27 (s, 2H), 3.05 - 2.90 (m, 4H), 2.82 (ddd, *J =* 17.5, 4.5, 2.3 Hz, 1H), 2.52 (qd, *J =* 13.3, 4.5 Hz, 1H), 2.39 (s, 2H), 2.28 - 2.18 (m, 3H), 2.18 - 1.89 (m, 9H), 1.78 (dt, *J =* 35.4, 5.6 Hz, 4H), 1.65 (q, *J* = 12.2, 10.8 Hz, 4H), 1.42 - 1.27 (m, 2H).
LC-MS: [M+H]⁺= 847.56

### Example 192

### Synthesis of compound 155

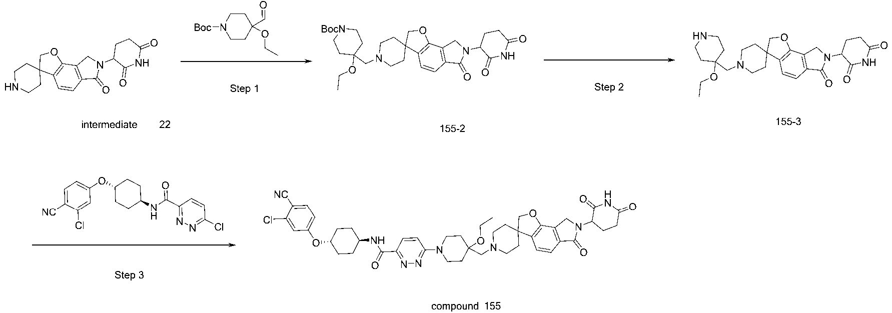

Step 1: The intermediate 22 (40 mg, 112.55 µmol), tert-butyl 4-ethoxy-4-formylpiperidine-1-carboxylate (86.89 mg, 337.65 µmol), Ti(OiPr)₄ (47.98 mg, 168.83 µmol) were added sequentially to a glass vial, the solvent THF:DMSO= 4 : 1 (1.5 mL) was added, and the reaction solution was stirred at room temperature for 1 h. Then NaBH₃CN (17.68 mg, 281.38 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 155-2 (59 mg).
LC-MS: [M+H]⁺ = 597.50

Step 2: (Compound 155-3) was prepared with reference to step 2 of example 39.
LC-MS: [M+H]⁺= 497.29

Step 3: (Compound 155) was prepared with reference to step 3 of Example 19.
¹H NMR (600 MHz, Methanol-*d*₄) δ 7.96 (s, 1H), 7.71 (d, *J =* 8.7 Hz, 1H), 7.53 - 7.32 (m, 3H), 7.22 (d, *J =* 2.3 Hz, 1H), 7.07 (dd, *J =* 8.7, 2.4 Hz, 1H) , 5.16 (dd, *J =* 13.3, 5.1 Hz, 1H), 4.74 (s, 2H), 4.57 - 4.47 (m, 2H), 4.43 (d, *J =* 17.0 Hz, 1H), 4.35 (s, 2H), 4.00 (s, 1H), 3.87 (s, 1H), 3.65 (d, *J =* 6.9 Hz, 2H), 3.47 (d, *J =* 29.4 Hz, 4H), 2.93 (ddd, *J =* 18.2, 13.5, 5.4 Hz, 1H), 2.80 (ddd, *J* = 17.8, 4.7, 2.3 Hz, 1H), 2.58 - 2.35 (m, 3H), 2.31 - 2.00 (m, 9H), 1.79 (s, 2H), 1.66 (d, *J =* 10.5 Hz, 4H), 1.41 - 1.25 (m, 6H).
LC-MS: [M+H]⁺= 851.46

### Example 193

### Synthesis of compound 156

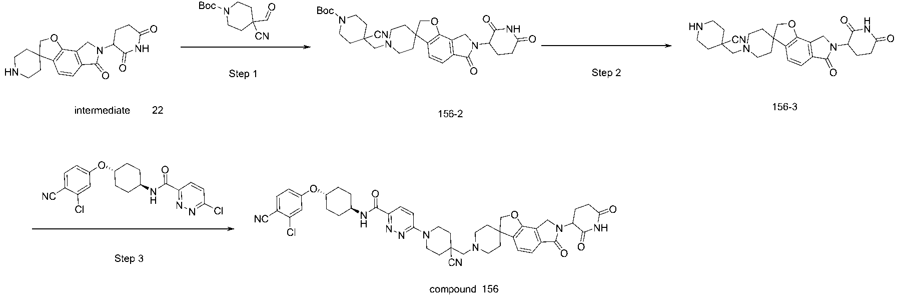

Step 1: The intermediate 22 (45 mg, 126.62 µmol), tert-butyl 4-cyano-4-formylpiperidine-1-carboxylate (181.03 mg, 759.72 µmol), Ti(OiPr)₄ (53.98 mg, 189.93 µmol) were added sequentially to a glass vial and the solvent THF:DMSO= 4 : 1 (1.5 mL) was added, and the reaction solution was stirred at room temperature for 1 h. Then NaBH₃CN (31.83 mg, 506.48 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 156-2 (20 mg).
LC-MS: [M+H]⁺ =578.39

Step 2: (Compound 156-3) was prepared with reference to step 2 of example 39.
LC-MS: [M+H]⁺= 478.29

Step 3: (Compound 155) was prepared with reference to step 3 of Example 19.
¹H NMR (400 MHz, *DMSO-d*₆) δ 10.99 (s, 1H), 7.87 (dd, *J =* 9.2, 2.4 Hz, 2H), 7.49 - 7.35 (m, 3H), 7.27 (tt, *J =* 14.7, 7.4 Hz, 2H), 7.14 (dd, *J =* 8.8, 2.5 Hz, 1H), 5.09 (dd, *J =* 13.2, 5.1 Hz, 1H), 4.62 - 4.48 (m, 4H), 4.38 (d, *J =* 17.1 Hz, 1H), 4.21 (d, *J =* 17.1 Hz, 1H), 3.87 (q, *J =* 10.2, 8.3 Hz, 1H), 3.17 (t, *J =* 12.8 Hz, 2H), 2.91 (ddd, *J =* 23.3, 12.7, 6.8 Hz, 3H), 2.63 (d, *J =* 15.8 Hz, 2H), 2.40 (dd, *J =* 14.5, 10.1 Hz, 3H), 2.15 - 1.85 (m, 10H), 1.65 (q, *J* = 12.9 Hz, 5H), 1.57 - 1.38 (m, 4H).
LC-MS: [M+H]⁺= 832.46

### Example 194

### Synthesis of compound 157

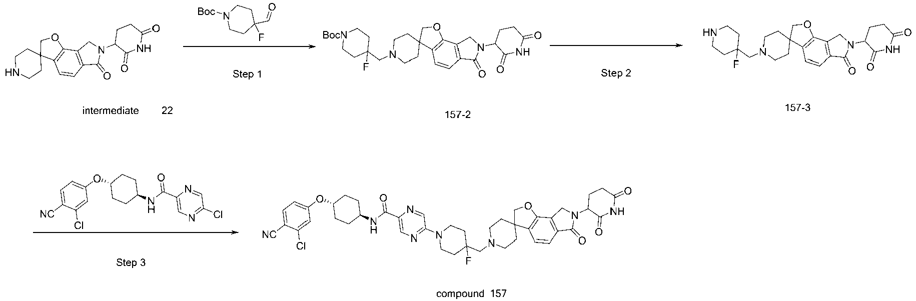

Step 1: The intermediate 22 (55 mg, 154.76 µmol), tert-butyl 4-fluoro-4-formylpiperidine-1-carboxylate (107.37 mg, 464.27 µmol), Ti(OiPr)₄ (65.98 mg, 232.14 µmol) were added sequentially to a glass vial, the solvent THF:DMSO= 4 : 1 (1.5 mL) was added, and the reaction solution was stirred at room temperature for 1 h. Then NaBH₃CN (24.31 mg, 386.89 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 157-2 (57 mg).
LC-MS: [M+H]⁺ = 571.59

Step 2: (Compound 157-2) was prepared with reference to step 2 of Example 39.
LC-MS: [M+H]⁺= 471.49

Step 3: (Compound 157) was prepared with reference to step 3 of Example 19.
¹H NMR (600 MHz, *DMSO-d*₆) δ 10.98 (s, 1H), 8.63 (s, 1H), 8.33 (s, 1H), 8.13 (d*, J =* 8.2 Hz, 1H), 7.87 (d, *J =* 8.8 Hz, 1H), 7.45 - 7.35 (m, 2H), 7.28 (d, *J =* 7.5 Hz, 1H), 7.13 (dd, *J =* 8.8, 2.5 Hz, 1H), 5.33 (t, *J =* 5.0 Hz, 1H), 5.09 (dd, *J =* 13.3, 5.1 Hz, 1H), 4.53 (dd, *J =* 9.2, 4.5 Hz, 3H), 4.38 (d, *J =* 17.0 Hz, 1H), 4.31 - 4.18 (m, 3H), 3.84 (dtd, *J =* 11.5, 7.6, 4.0 Hz, 1H), 2.91 (dq, *J =* 12.5, 6.5, 5.7 Hz, 3H), 2.61 (d, *J =* 4.3 Hz, 1H), 2.58 (d, *J =* 5.9 Hz, 1H), 2.42 (dd, *J =* 13.1, 4.7 Hz, 1H), 2.22 (t, *J =* 11.8 Hz, 2H), 2.10 (dd, *J =* 14.8, 5.3 Hz, 2H), 1.95 (dddt, *J =* 47.5, 17.8, 13.4, 5.6 Hz, 8H), 1.82 - 1.58 (m, 6H), 1.56 - 1.44 (m, 3H).
LC-MS: [M+H]⁺=825.56

### Example 195

### Synthesis of compound 158

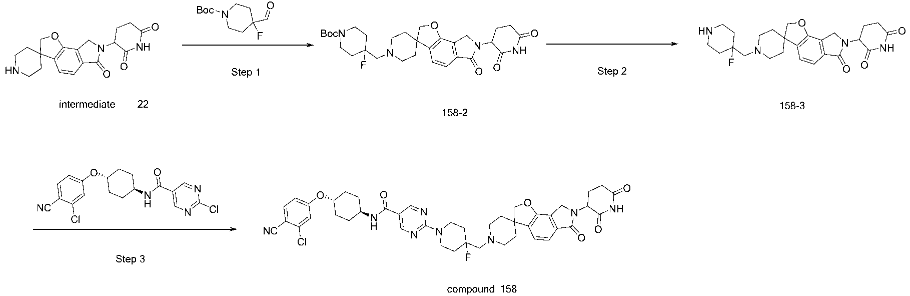

Step 1: The intermediate 22 (55 mg, 154.76 µmol), tert-butyl 4-fluoro-4-formylpiperidine-1-carboxylate (107.37 mg, 464.27 µmol), Ti(OiPr)₄ (65.98 mg, 232.14 µmol) were added sequentially to a glass vial, the solvent THF:DMSO= 4 : 1 (1.5 mL) was added, and the reaction solution was stirred at room temperature for 1 h. Then NaBH₃CN (24.31 mg, 386.89 µmol) was added and the reaction solution was continued to be stirred at room temperature for 1 h. After the reaction was completed, the reaction solution was concentrated, and then purified by preparative thin-layer chromatography (DCM:MeOH=10:1) to obtain a white solid compound 158-2 (55 mg).
LC-MS: [M+H]⁺ = 571.59

Step 2: (Compound 158-3) was prepared with reference to step 2 of Example 39.
LC-MS: [M+H]⁺= 471.49

Step 3: (Compound 158) was prepared with reference to step 3 of Example 19.
¹H NMR (600 MHz, *DMSO-d*₆) δ 10.98 (s, 1H), 8.79 (s, 2H), 8.17 (d, *J =* 7.3 Hz, 1H), 7.87 (d, *J =* 8.7 Hz, 1H), 7.45 - 7.37 (m, 2H), 7.27 (d, *J =* 7.6 Hz, 1H), 7.15 (dd, *J =* 8.8, 2.5 Hz, 1H), 5.09 (dd, *J =* 13.3, 5.1 Hz, 1H), 4.60 - 4.44 (m, 5H), 4.38 (d, *J =* 17.0 Hz, 1H), 4.22 (d, *J =* 17.0 Hz, 1H), 3.81 (ddt, *J =* 10.6, 7.0, 3.4 Hz, 1H), 3.33 (d, *J* = 11.2 Hz, 2H), 2.96 - 2.87 (m, 3H), 2.64 - 2.54 (m, 3H), 2.43 (td, *J =* 13.2, 4.6 Hz, 1H), 2.27 - 2.17 (m, 2H), 2.10 (dq, *J =* 11.9, 6.7, 5.1 Hz, 2H), 2.03 - 1.86 (m, 7H), 1.77 - 1.60 (m, 4H), 1.58 - 1.44 (m, 4H).
LC-MS: [M+H]⁺=825.46

### Example 196

### Synthesis of compound 159

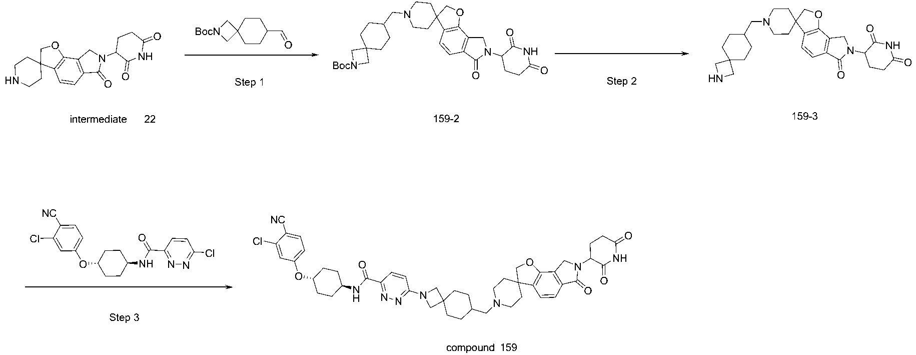

Step 1: The intermediate 22 (55 mg, 154.76 µmol), tert-butyl 7-formyl-2-azaspiro[3.5]nonane-2-carboxylate (117.62 mg, 464.27 µmol), Ti(OiPr)₄ (65.98 mg, 232.14 µmol) were added sequentially to a glass vial, and the solvent THF:DMSO= 4 : 1 (1.5 mL) was added, and the reaction solution was stirred at room temperature for 1 h. Then NaBH₃CN (24.31 mg, 386.89 µmol) was added and the reaction solution was continued to be stirred at room temperature for 1 h. After the reaction was completed, it was concentrated, and then purified by preparative thin-layer chromatography (DCM:MeOH=10:1) to obtain a white solid compound 159-2 (56 mg).
LC-MS: [M+H]⁺ = 593.48

Step 2: (Compound 159-3) was prepared with reference to step 2 of Example 39.
LC-MS: [M+H]⁺= 493.50

Step 3: (Compound 159) was prepared with reference to step 3 of Example 19.
¹H NMR (600 MHz, *DMSO-d*₆) δ 10.99 (s, 1H), 8.54 (d, *J =* 8.2 Hz, 1H), 7.85 (dd, *J =* 15.2, 9.0 Hz, 2H), 7.39 (d, *J* = 2.4 Hz, 1H), 7.36 (d, *J =* 7.4 Hz, 1H), 7.29 (td, *J =* 13.9, 13.2, 7.5 Hz, 1H), 7.15 - 7.13 (m, 1H), 6.86 (d, *J= 9.3* Hz, 1H), 5.10 (dd, *J* = 13.3, 5.1 Hz, 1H), 4.65 (s, 2H), 4.54 (tt, *J =* 10.2, 4.4 Hz, 1H), 4.40 (*d, J =* 17.2 Hz, 1H), 4.24 (d, *J =* 17.1 Hz, 1H), 3.83 (d, *J =* 32.5 Hz, 4H), 3.11 - 2.86 (m, 5H), 2.59 (s, 1H), 2.43 (dt, *J =* 13.2, 6.6 Hz, 2H), 2.33 - 2.07 (m, 5H), 1.97 (ddd, *J =* 36.5, 16.7, 11.5 Hz, 6H), 1.77 (d, *J =* 13.9 Hz, 4H), 1.70 - 1.48 (m, 7H), 1.38 (s, 2H)
LC-MS: [M+H]⁺=847.71

### Example 197

### Synthesis of compound 160

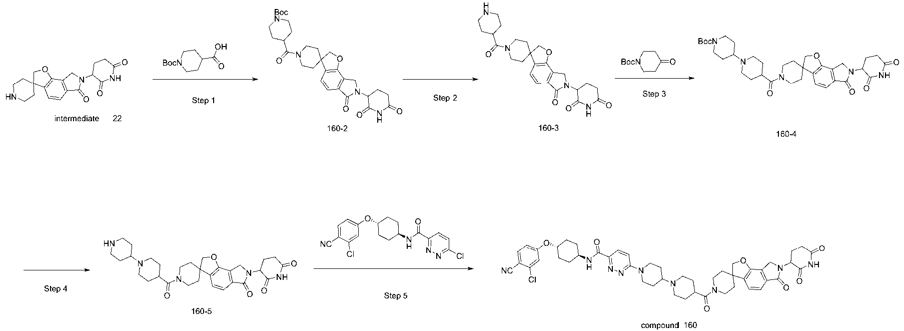

Step 1: The intermediate 22 (90 mg, 253.24 µmol), 1-(tert-butoxycarbonyl)piperidine-4-carboxylic acid (69.76 mg, 303.89 µmol), BOP (336.01 mg, 759.72 µmol) were added sequentially to a glass vial the solvent DMF (3 mL) was added, then DIEA (98.19 mg, 759.72 µmol) was added, and the reaction solution was stirred at room temperature for 2 h. After the reaction was completed, it was concentrated, and then purified by flash to obtain a white solid compound 160-2 (89 mg).
LC-MS: [M+H]⁺ = 567.40

Step 2: (Compound 160-3) was prepared with reference to step 2 of Example 39.
LC-MS: [M+H]⁺= 467.39

Step 3: Compound 159-3 (60 mg, 128.61 µmol), tert-butyl 4-oxopiperidine-1-carboxylate (51.25 mg, 257.21 µmol), Ti(OiPr)₄ (54.83 mg, 192.91 µmol) were added sequentially to a glass vial, the solvent THF:DMSO= 4 : 1 (1.5 mL) was added, and the reaction solution was stirred at room temperature for 1 h. Then NaBH₃CN (32.33 mg, 514.43 µmol) was added, and the reaction solution was continued to be stirred at room temperature for 1 h. After the reaction was completed, it was concentrated, and then purified by preparative thin-layer chromatography (DCM:MeOH=10:1) to obtain a white solid compound 160-4 (66 mg).
LC-MS: [M+H]⁺ = 650.47

Step 4: (Compound 160-5) was prepared with reference to step 2 of Example 39.
LC-MS: [M+H]⁺ = 550.38

Step 5: (Compound 160) was prepared with reference to step 3 of Example 19.
¹ H NMR (600 MHz, *DMSO-d*₆ ) δ 10.98 (s, 1H), 8.59 (d, *J* = 8.2 Hz, 1H), 7.85 (dd, *J* = 17.9, 9.1 Hz, 2H), 7.43 - 7.23 (m, 4H), 7.14 (dd, *J* = 8.8, 2.5 Hz, 1H), 5.10 (dd, *J* = 13.3, 5.1 Hz, 1H), 4.74 -4.35 (m, 8H), 4.24 (d, *J* = 17.0 Hz, 2H), 3.91 - 3.83 (m, 1H), 3.54 (s, 3H), 3.11 (d, *J* = 31.1 Hz, 5H), 2.92 (ddd, *J* = 17.7, 13.5, 5.2 Hz, 1H), 2.59 (d, *J* = 3.4 Hz, 2H), 2.43 (dd, *J* = 13.2, 4.7 Hz, 2H), 2.14 (t, *J* = 23.7 Hz, 5H), 1.95 (dd, *J* = 48.6, 12.0 Hz, 6H), 1.80 - 1.70 (m, 2H), 1.70 - 1.47 (m, 7H).
LC-MS: [M+H]⁺= 904.45

### Example 198

### Synthesis of compound 161

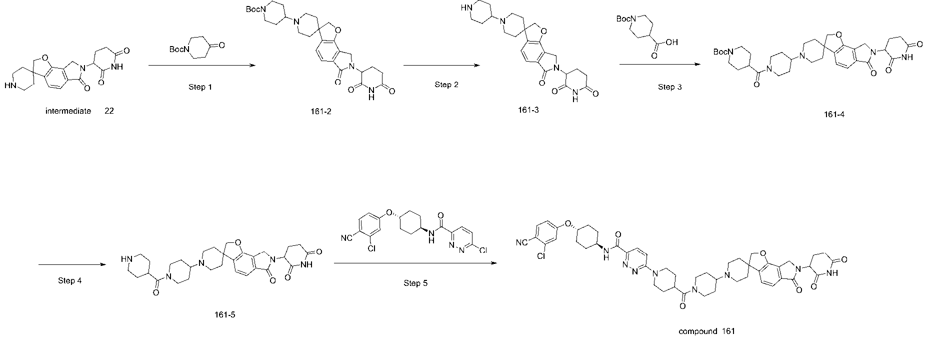

Step 1: The intermediate 22 (90 mg, 253.24 µmol), tert-butyl 4-oxopiperidine-1-carboxylate (151.37 mg, 759.72 µmol), Ti(OiPr)₄ (107.96 mg, 379.86 µmol) were added sequentially into a glass vial, the solvent THF:DMSO= 4 : 1 (1.5 mL) was added. The reaction solution was stirred at room temperature for 1 h. Then NaBH₃CN (47.74 mg, 759.72 µmol) was added, and the reaction solution was continued to be stirred at room temperature for 1 h. After the completion of the reaction, it was concentrated, and then purified by preparative thin-layer chromatography (DCM:MeOH=10:1) to obtain a white solid compound 161-2 (77 mg).
LC-MS: [M+H]⁺ = 539.38

Step 2: (Compound 161-3) was prepared with reference to step 2 of Example 39.
LC-MS: [M+H]⁺= 439.40

Step 3: Compound 161-3 (70 mg, 159.62 µmol), 1-(tert-butoxycarbonyl)piperidine -4-carboxylic acid (43.92 mg, 191.55 µmol), and BOP (211.80 mg, 478.87 µmol) were added sequentially into a glass vial , the solvent DMF (3 mL) was added, and then DIEA (61.89 mg, 478.87 µmol) was added, and the reaction solution was stirred at room temperature for 2 h. After the reaction was completed, it was concentrated, and then purified by flash to obtain a white solid compound 161-4 (68 mg).
LC-MS: [M+H]⁺ = 650.38

Step 4: (Compound 161-5) was prepared with reference to step 2 of Example 39.
LC-MS: [M+H]⁺ = 550.48

Step 5: (Compound 161) was prepared with reference to step 3 of Example 19.
¹H NMR (600 MHz, DMSO-*d*₆) δ 10.98 (s, 1H), 8.59 (d, *J* = 8.2 Hz, 1H), 7.85 (dd, *J* = 17.8, 9.1 Hz, 2H), 7.43 - 7.23 (m, 4H), 7.14 (dd, *J* = 8.8, 2.4 Hz, 1H), 5.10 (dd, *J* = 13.3, 5.1 Hz, 1H), 4.73 - 4.45 (m, 6H), 4.40 (d, *J* = 17.1 Hz, 1H), 4.24 (d, *J* = 17.0 Hz, 2H), 3.93 - 3.82 (m, 1H), 3.55 (s, 2H), 3.10 (d, *J* = 42.2 Hz, 5H), 2.97 - 2.85 (m, 2H), 2.57 (d, *J* = 25.9 Hz, 2H), 2.43 (dd, *J* = 13.2, 4.7 Hz, 2H), 2.27 - 2.07 (m, 5H), 1.95 (dd, *J* = 48.4, 12.0 Hz, 6H), 1.79 - 1.71 (m, 2H), 1.71 - 1.42 (m, 8H).
LC-MS: [M+H]⁺=904.65

### Example 199

### Synthesis of compound 162

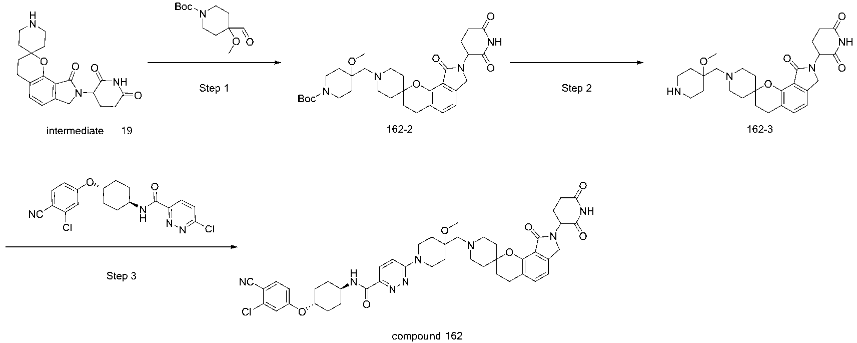

Step 1: The intermediate 19 (40.0 mg, 108.35 µmol) and tert-butyl 4-formyl-4-methoxypiperidine-1-carboxylate (52.7 mg, 216.7 µmol) were added sequentially to a glass vial, followed by the addition of the solvents THF:DMSO = 2 : 1 (1.0 mL) and Ti(O-iPr)₄ (61.6 mg, 216.7 µmol), and the reaction solution was stirred at 60°C for 1 h. Then NaBH(OAc)₃ (45.9 mg, 216.7 µmol) was added, and the reaction solution was continued to be stirred at 60°C for 1 h. When the reaction was completed, the reaction solution was concentrated, and then purified by preparation to obtain a white solid compound 162-2 (20 mg).
LC-MS: [M+H]⁺ =597.38

Step 2: (Compound 162-3) was prepared with reference to step 2 of Example 39.
LC-MS: [M+H]⁺ =497.29

Step 3: (Compound 162) was prepared with reference to step 3 of Example 19.
¹H NMR (600 MHz, DMSO-d6) δ 11.00 (s, 1H), 8.66 (d, *J* = 8.2 Hz, 1H), 7.87 (dd, *J* = 9.1, 4.8 Hz, 2H), 7.45 - 7.39 (m, 2H), 7.37 - 7.33 (m, 1H), 7.14 (dd, *J* = 8.8, 2.3 Hz, 1H), 7.05 (t, *J* = 7.2 Hz, 1H), 5.01 (dd, *J* = 13.2, 5.1 Hz, 1H), 4.54 (t, *J* = 10.4 Hz, 1H), 4.35 (d, *J* = 17.3 Hz, 1H), 4.24 (t, *J* = 16.2 Hz, 3H), 3.87 (d, *J* = 8.1 Hz, 2H), 3.35 (dd, *J* = 22.3, 11.6 Hz, 6H), 3.23 (s, 2H), 2.85 (dd, *J* = 18.7, 12.4 Hz, 3H), 2.61 (d, *J* = 13.9 Hz, 1H), 2.43 - 2.36 (m 2H), 2.11 (d, *J* = 11.9 Hz, 4H), 2.03 - 1.85 (m, 9H), 1.70 - 1.59 (m, 4H), 1.52 (dd, *J* = 23.0, 10.0 Hz, 3H).
LC-MS: [M+H]⁺ =851.60

### Example 200

### Synthesis of compound 163

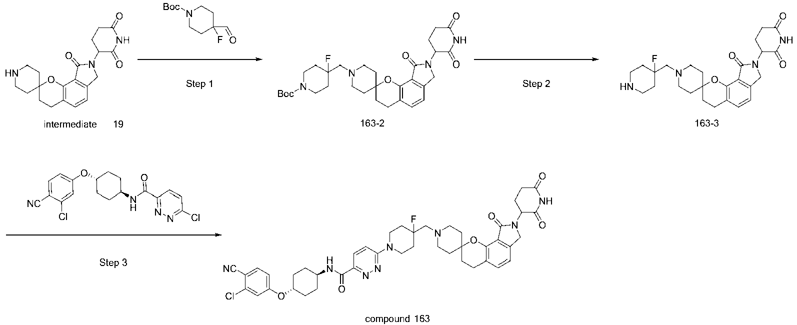

Step 1: The intermediate 19 (25.0 mg, 67.67 µmol) and tert-butyl 4-fluoro-4-formylpiperidine-1-carboxylate (15.7 mg, 67.67 µmol) were added sequentially to a glass vial, and the solvents THF:DMSO = 2 : 1 (1.0 mL) and acetic acid (1 drop) were added, and the reaction solution was stirred at 60 °C for 1 h. Then NaBH(OAc)₃ (28.7 mg, 135.34 µmol) was added, and the reaction solution was continued to be stirred at 60 °C for 1 h. After the reaction was completed, it was concentrated, and then purified by preparative to obtain a white solid compound 163-2 (20 mg).
LC-MS: [M+H]⁺ =585.38

Step 2: (Compound 163-3) was prepared with reference to step 2 of example 39.
LC-MS: [M+H]⁺ =485.45

Step 3: (Compound 163) was prepared with reference to step 3 of Example 19.
¹H NMR (600 MHz, DMSO-d6) δ 11.00 (s, 1H), 8.66 (d, *J* = 8.2 Hz, 1H), 7.88 (dd, *J* = 12.1, 9.2 Hz, 2H), 7.48 (d, *J* = 9.6 Hz, 1H), 7.41 - 7.34 (m, 2H), 7.14 (dd, *J* = 8.8, 2.4 Hz, 1H), 7.05 (dd, *J* = 7.5, 3.6 Hz, 1H), 5.02 (dd, *J* = 13.3, 5.1 Hz, 1H), 4.58 - 4.50 (m, 1H), 4.36 (dd, *J* = 16.7, 13.6 Hz, 3H), 4.25 (t, *J* = 17.9 Hz, 2H), 3.91 - 3.84 (m, 2H), 3.42 - 3.24 (m, 7H), 2.86 (d, *J* = 21.2 Hz, 3H), 2.65 - 2.59 (m, 1H), 2.17 - 1.80 (m, 16H), 1.65 (dd, *J* = 24.0, 10.6 Hz, 2H), 1.52 (dd, *J* = 23.0, 9.9 Hz, 2H).
LC-MS: [M+H]⁺ =839.36

### Example 201

### Synthesis of compound 164

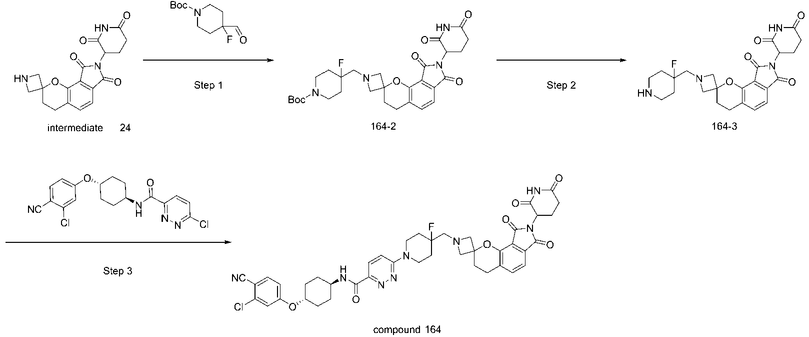

Step 1: The intermediate 24 (40.0 mg, 112.57 µmol) and tert-butyl 4-fluoro-4-formylpiperidine-1-carboxylate (26.1 mg, 112.57 µmol) were added sequentially to a glass vial, the solvents THF:DMSO = 2 : 1 (1.0 mL) and acetic acid (1 drop) were added, and the reaction solution was stirred at 60°C for 1 h. Then NaBH(OAc)₃(47.7 mg, 225.14 µmol) was added, and the reaction solution was continued to be stirred at 60°C for 1 h. After the reaction was completed, it was concentrated, and then purified by preparative to obtain a white solid compound 164-2 (30 mg).
LC-MS: [M+H]⁺ =571.45

Step 2: (Compound 164-3) was prepared with reference to step 2 of Example 39.
LC-MS: [M+H]⁺ =471.19

Step 3: (Compound 164) was prepared with reference to step 3 of Example 19.
¹H NMR (600 MHz, DMSO-d6) δ 11.12 (s, 1H), 8.64 (d, *J* = 8.2 Hz, 1H), 7.87 (dd, *J* = 9.1, 6.1 Hz, 2H), 7.64 (d, *J* = 7.6 Hz, 1H), 7.49 - 7.38 (m, 3H), 7.14 (dd, *J* = 8.8, 2.4 Hz, 1H), 5.09 (s, 1H), 4.69 - 4.50 (m, 3H), 4.41 (d,*J* = 37.7 Hz, 4H), 3.93 - 3.85 (m, 3H), 3.32 (t, *J* = 12.0 Hz, 2H), 2.95 (d, *J* = 35.4 Hz, 3H), 2.64 - 2.57 (m, 1H), 2.31 (s, 2H), 2.11 (d, *J* = 10.4 Hz, 2H), 2.07 - 1.73 (m, 8H), 1.65 (dd, *J* = 24.1, 10.7 Hz, 2H), 1.52 (dd, *J* = 22.9, 10.0 Hz, 2H).
LC-MS: [M+H]⁺ =825.36

### Example 202

### Synthesis of compound 165

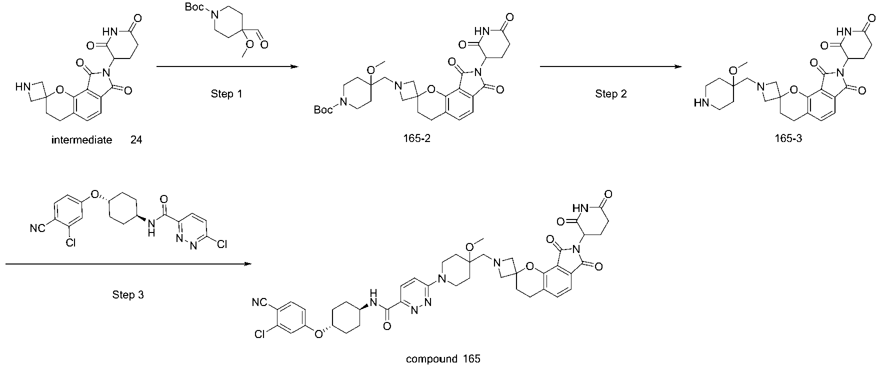

Step 1: The intermediate 24 (50.0 mg, 140.71 µmol) and tert-butyl 4-formyl-4-methoxypiperidine-1-carboxylate (34.3 mg, 140.71 µmol) were added sequentially to a glass vial, and the solvents THF:DMSO = 2 : 1 (1.0 mL) and acetic acid (2 drops) were added, and the reaction solution was stirred at 60 °C for 1 h. Then NaBH (OAc)₃ (59.6 mg, 281.42 µmol) was added, and the reaction solution was continued to be stirred at 60 °C for 1 h. After the reaction was completed, it was concentrated, and then purified by preparative to obtain a white solid compound 165-2 (46 mg).
LC-MS: [M+H]⁺ =583.50

Step 2: (Compound 165-3) was prepared with reference to step 2 of Example 39.
LC-MS: [M+H]⁺ =483.29

Step 3: (Compound 165) was prepared with reference to step 3 of Example 19.
¹H NMR (600 MHz, DMSO-d6) δ 11.13 (d, *J* = 8.5 Hz, 1H), 8.63 (d, *J* = 8.2 Hz, 1H), 7.86 (t, *J* = 8.4 Hz, 2H), 7.64 (d, *J* = 7.5 Hz, 1H), 7.47 - 7.38 (m, 3H), 7.14 (dd, *J* = 8.8, 2.4 Hz, 1H), 5.08 (dd, *J* = 12.7, 5.4 Hz, 1H), 4.57 (ddd, *J* = 31.1, 14.8, 6.8 Hz, 4H), 4.34 (dd, *J* = 27.6, 10.0 Hz, 2H), 4.22 (d, *J* = 12.4 Hz, 2H), 3.91 - 3.82 (m, 1H), 3.61 (d, *J* = 52.6 Hz, 2H), 3.37 - 3.24 (m, 5H), 3.01 - 2.86 (m, 3H), 2.59 (d, *J* = 16.4 Hz, 1H), 2.32 (s, 2H), 2.11 (d, *J* = 10.1 Hz, 2H), 2.06 - 1.98 (m, 1H), 1.89 (d, *J* = 10.7 Hz, 2H), 1.82 (d, *J* = 13.0 Hz, 2H), 1.70 - 1.49 (m, 6H).
LC-MS: [M+H]⁺ =837.46

### Example 203

### Synthesis of compound 166

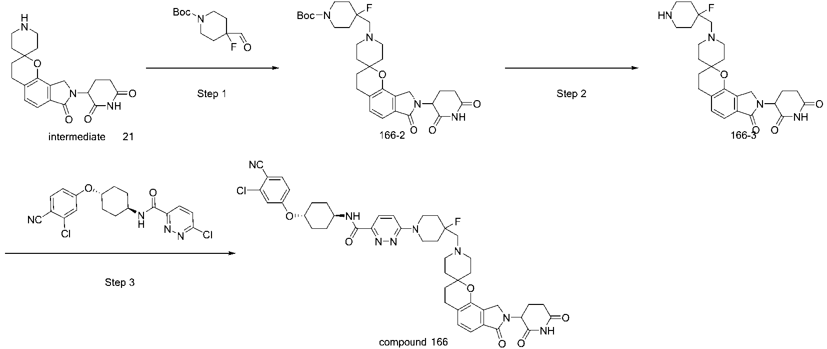

Step 1: The intermediate 21 (50.0 mg, 135.5 µmol) and tert-butyl 4-fluoro-4-formylpiperidine-1-carboxylate (62.6 mg, 271.0 µmol) were added sequentially into a glass vial, and the solvents DCE:DMSO = 2 : 1 (2 mL) and acetic acid (1 drop) were added, and the reaction soluton was stirred at room temperature for 1 h, and then NaBH(OAc)₃ (57.4 mg, 271.0 µmol) was added, and the reaction solution was continued to be stirred at room temperature for 1 h. After the reaction was completed, it was concentrated, and then purified by preparation to obtain a white solid compound 166-2 (32 mg).
LC-MS: [M+H]⁺ =585.38

Step 2: (Compound 166-3) was prepared with reference to step 2 of Example 39.
LC-MS: [M+H]⁺ =485.45

Step 3: (Compound 166) was prepared with reference to step 3 of Example 19.
¹H NMR (600 MHz, DMSO-d6) δ 7.98 (d, *J* = 9.2 Hz, 1H), 7.71 (d, *J* = 8.7 Hz, 1H), 7.43 (d, *J* = 9.6 Hz, 1H), 7.38 - 7.31 (m, 2H), 7.22 (d, *J* = 2.2 Hz, 1H), 7.06 (dd, *J* = 8.8, 2.2 Hz, 1H), 5.20 (dd, *J* = 13.3, 5.0 Hz, 1H), 4.61 - 4.44 (m, 5H), 4.01 (s, 1H), 3.69 (s, 4H), 3.56 - 3.43 (m, 4H), 3.04 - 2.92 (m, 3H), 2.82 (d, *J* = 16.1 Hz, 1H), 2.51 (ddd, *J* = 26.3, 13.2, 4.3 Hz, 1H), 2.18 (dd, *J* = 47.8, 17.0 Hz, 10H), 2.04 (d, *J* = 11.2 Hz, 4H), 1.72 - 1.60 (m, 4H), 1.31 - 1.35 (m, 1H)
LC-MS: [M+H]⁺ =839.46

### Example 204

### Synthesis of compound 167

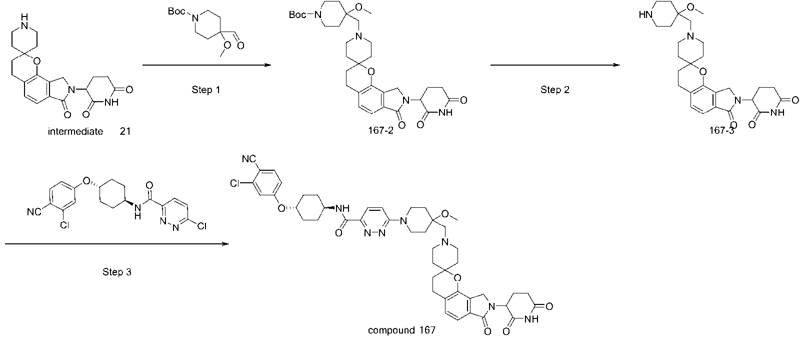

Step 1: The intermediate 21 (50.0 mg, 135.50 µmol) and tert-butyl 4-formyl-4-methoxypiperidine-1-carboxylate (49.4 mg, 203.25 µmol) were added sequentially to a glass vial, followed by the addition of the solvents THF:DMSO = 2 : 1 (2 mL) and Ti(O-iPr)₄ (77.0 mg, 271.0 µmol), and the reaction solution was stirred at 60°C for 1 h. Then NaBH(OAc)₃(57.4 mg, 271.0 µmol) was added and the reaction solution was continued to be stirred at 60°C for 1 h. After the reaction was completed, the reaction solution was concentrated, and then purified by the preparation to obtain a white solid compound 167-2 (46 mg)
LC-MS: [M+H]⁺ =597.48

Step 2: (Compound 167-3) was prepared with reference to step 2 of Example 39.
LC-MS: [M+H]⁺ = 497.09

Step 3: (Compound 167) was prepared with reference to step 3 of Example 19.
¹H NMR (600 MHz, DMSO) δ 11.03 (s, 1H), 8.63 (d, *J* = 8.2 Hz, 1H), 7.87 (d, *J* = 9.0 Hz, 2H), 7.41 (dd, *J* = 22.9, 6.0 Hz, 2H), 7.32 - 7.23 (m, 2H), 7.14 (dd, *J* = 8.8, 2.3 Hz, 1H), 5.14 (dd, *J* = 13.3, 5.0 Hz, 1H), 4.57 - 4.51 (m, 1H), 4.44 (d, *J* = 17.5 Hz, 1H), 4.37 (d, *J* = 17.3 Hz, 1H), 4.31 (d, *J* = 17.4 Hz, 1H), 4.24 (d, *J* = 12.7 Hz, 3H), 3.87 (dt, *J* = 11.2, 9.4 Hz, 1H), 3.49 (dd, *J* = 41.2, 18.3 Hz, 4H), 3.34 (t, *J* = 11.4 Hz, 2H), 3.29 (s, 3H), 2.99 - 2.87 (m, 3H), 2.63 (d, *J* = 17.5 Hz, 1H), 2.35 (ddd, *J* = 26.3, 13.2, 4.1 Hz, 1H), 2.11 (d, *J* = 9.6 Hz, 4H), 1.96 (ddd, *J* = 27.2, 23.4, 15.6 Hz, 9H), 1.65 (dd, *J* = 23.1, 11.2 Hz, 4H), 1.52 (dd, *J* = 22.9, 9.9 Hz, 2H).
LC-MS: [M+H]⁺ =851.36

### Example 205

### Synthesis of compound 168

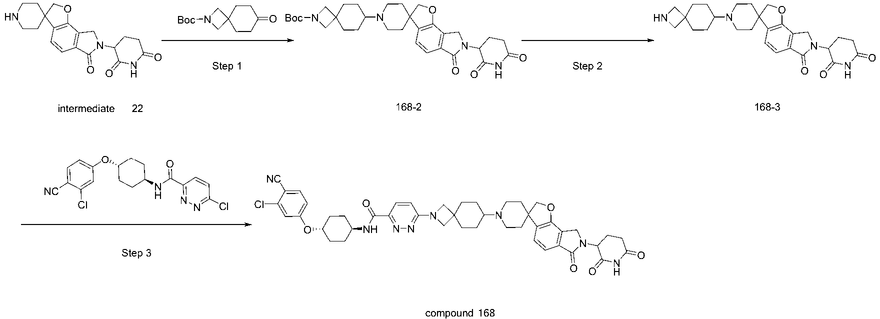

Step 1: The intermediate 22 (50.0 mg, 140.84 µmol) and tert-butyl 7-oxo-2-azaspiro[3.5]nonane-2-carboxylate (50.6 mg, 211.26 µmol) were added sequentially to a glass vial, and the solvents THF:DMSO = 2 : 1 (4 mL) and Ti(O-iPr)₄(80.0 mg, 281.68 µmol) were added, and the reaction solution was stirred at 60°C for 1 h. Then NaBH₃CN (17.7 mg, 281.68 µmol) was added, and the reaction solution was continued to be stirred at 60°C for 1 h. After the reaction was completed, the reaction solution was concentrated, and then purified by preparation to obtain a white solid compound 168-2 (36 mg).
LC-MS: [M+H]⁺ =579.18

Step 2: (Compound 168-3) was prepared with reference to step 2 of Example 39.
LC-MS: [M+H]⁺ =479.29

Step 3: (Compound 168) was prepared with reference to step 3 of Example 19.
¹H NMR (600 MHz, DMSO-d6) δ 11.00 (s, 1H), 8.56 (d, *J* = 8.2 Hz, 1H), 7.87 (dd, *J* = 9.0, 2.6 Hz, 2H), 7.38 (dd, *J* = 17.6, 4.9 Hz, 2H), 7.30 (d, *J* = 7.6 Hz, 1H), 7.14 (dd, *J* = 8.8, 2.3 Hz, 1H), 6.88 (d, *J* = 9.4 Hz, 1H), 5.11 (dd, *J* = 13.3, 5.0 Hz, 1H), 4.69 (q, *J* = 9.5 Hz, 2H), 4.57 - 4.52 (m, 1H), 4.40 (d, *J* = 17.2 Hz, 1H), 4.25 (d, *J* = 17.2 Hz, 1H), 3.90 (s, 2H), 3.58 - 3.51 (m, 3H), 3.28 (s, 1H), 3.17 - 3.08 (m, 2H), 2.96 - 2.89 (m, 1H), 2.61 (d, *J* = 16.5 Hz, 1H), 2.46 - 2.39 (m, 1H), 2.22 (t, *J* = 13.0 Hz, 2H), 2.16 - 1.96 (m, 10H), 1.90 (d, *J* = 11.6 Hz, 2H), 1.68 - 1.48 (m, 9H). LC-MS: [M+H]⁺ =833.46

### Example 206

### Synthesis of compound 169

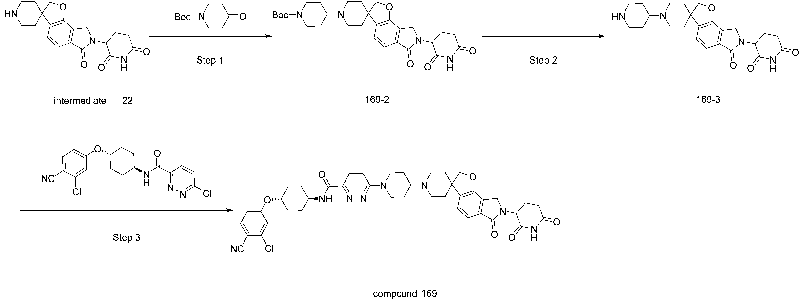

Step 1: The intermediate 22 (50.0 mg, 140.84 µmol) and tert-butyl 4-oxopiperidine-1-carboxylate (42.1 mg, 211.27 µmol) were added sequentially to a glass vial, and the solvents THF:DMSO = 2:1 (2 mL) and Ti(O-iPr)₄ (80.0 mg, 281.68 µmol) were added, and the reaction solution was stirred at 60°C for 1 h. Then NaBH₃CN (17.7 mg, 281.68 µmol) was added, and the reaction solution was continued to be stirred at 60°C for 1 h. After the reaction was completed, it was concentrated, and then purified by preparation to obtain a white solid compound 169-2 (30 mg).
LC-MS: [M+H]⁺ =539.38

Step 2: (Compound 169-3) was prepared with reference to step 2 of Example 39.
LC-MS: [M+H]⁺ =439.40

Step 3: (Compound 169) was prepared with reference to step 3 of Example 19.
¹H NMR (600 MHz, DMSO-d6) δ 11.00 (s, 1H), 8.65 (d, *J* = 8.2 Hz, 1H), 7.88 (dd, *J* = 13.8, 9.1 Hz, 2H), 7.48 (d, *J* = 9.7 Hz, 1H), 7.38 (dd, *J* = 24.8, 5.0 Hz, 2H), 7.29 (d, *J* = 7.6 Hz, 1H), 7.14 (dd, *J* = 8.8, 2.4 Hz, 1H), 5.10 (dd, *J* = 13.2, 5.1 Hz, 1H), 4.68 (dd, *J* = 14.4, 9.7 Hz, 4H), 4.58 - 4.52 (m, 1H), 4.40 (d, *J* = 17.2 Hz , 1H), 4.24 (d, *J* = 17.2 Hz, 1H), 3.92 - 3.85 (m, 1H), 3.52 (s, 2H), 3.18 - 3.03 (m, 4H), 2.96 - 2.88 (m, 1H), 2.63 - 2.57 (m, 1H), 2.46 - 2.38 (m, 1H), 2.22 - 2.08 (m, 6H), 2.04 - 1.87 (m, 6H), 1.74 - 1.61 (m, 4H), 1.57 - 1.49 (m, 2H).
LC-MS: [M+H]⁺ =793.36

### Example 207

### Synthesis of compound 170

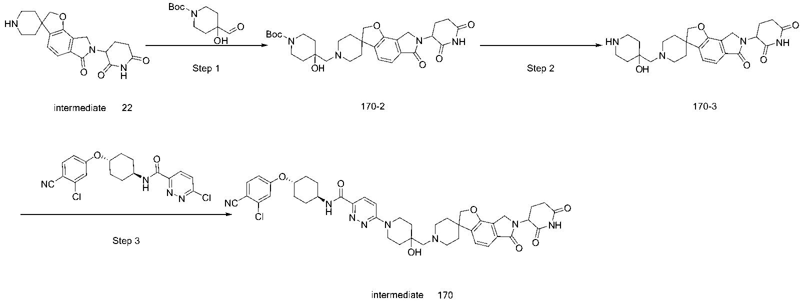

Step 1: The intermediate 22 (200.0 mg, 563.14 µmol) and tert-butyl 4-formyl-4-hydroxypiperidine-1-carboxylate (258.1 mg, 1126.3 µmol) were added sequentially to a glass vial, and the solvents THF:DMSO = 2 : 1 (6 mL) and Ti(O-iPr)₄(319.9 mg, 1126.3 µmol) were added, and the reaction solution was stirred at 60°C for 1 h. Then NaBH₃CN (70.8 mg, 1126.3 µmol) was added and the reaction solution was continued to be stirred at 60°C for 1 h. After the reaction was completed, the reaction solution was concentrated, and then purified by preparation to obtain a white solid compound 170-2 (190 mg).
LC-MS: [M+H]⁺ =569.45

Step 2: (Compound 170-3) was prepared with reference to step 2 of Example 39.
LC-MS: [M+H]⁺ =469.45

Step 3: (Compound 170) was prepared with reference to step 3 of Example 19.
¹ H NMR (600 MHz, MeOD) δ 7.96 (d, *J* = 9.6 Hz, 1H), 7.71 (d, *J* = 8.8 Hz, 1H), 7.43 (dd, *J* = 31.3, 8.6 Hz, 3H), 7.22 (d, *J* = 2.3 Hz, 1H), 7.07 (dd, *J* = 8.8, 2.3 Hz, 1H), 5.17 (dd, *J* = 13.3, 5.1 Hz, 1H), 4.73 (s, 2H), 4.58 - 4.40 (m, 5H), 4.33 (d, *J* = 12.0 Hz, 2H), 4.00 (s, 1H), 3.86 (d, *J* = 11.2 Hz, 2H), 3.60 (t, *J* = 11.7 Hz, 4H), 2.97 - 2.88 (m, 1H), 2.84 - 2.77 (m, 1H), 2.51 (ddd, *J* = 32.2, 16.0, 11.4 Hz, 3H), 2.26 - 2.16 (m, 3H), 2.15 - 2.02 (m, 4H), 1.99 - 1.91 (m, 2H), 1.84 (s, 2H).
LC-MS: [M+H]⁺ =823.46

### Example 208

### Synthesis of compound 171

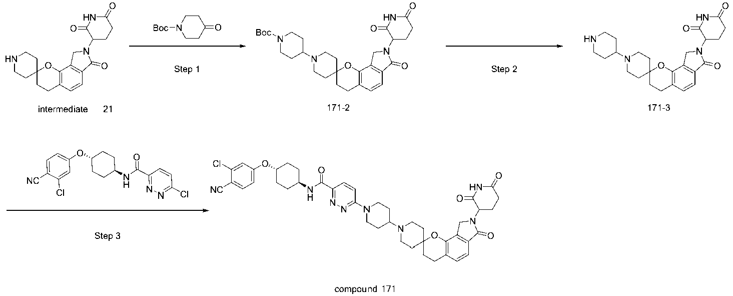

Step 1: The intermediate 21 (50.0 mg, 135.44 µmol) and tert-butyl 4-oxopiperidine-1-carboxylate (27.0 mg, 135.44 µmol) were added sequentially to a glass vial, and solvents THF:DMSO = 2 : 1 (2 mL) and Ti(O-iPr)₄ (77.0 mg, 270.88 µmol) were added and the reaction solution was stirred at 60°C for 1 h. Then NaBH₃CN (17.1 mg, 270.88 µmol) was added, and the reaction solution was continued to be stirred at 60°C for 1 h. After the reaction was completed, it was concentrated, and then purified by preparation to obtain a white solid compound 171-2 (38.1 mg).
LC-MS: [M+H]⁺ =553.38

Step 2: (Compound 171-3) was prepared with reference to step 2 of example 39.
LC-MS: [M+H]⁺ =453.40

Step 3: (Compound 171) was prepared with reference to step 3 of Example 19.
¹H NMR (600 MHz, DMSO-d6) δ 11.04 (s, 1H), 8.63 (d, *J =* 8.2 Hz, 1H), 7.88 (dd, *J* = 12.1, 9.1 Hz, 3H), 7.47 (d, *J* = 9.6 Hz, 2H), 7.40 (d, *J* = 2.4 Hz, 1H), 7.29 (d, *J* = 7.7 Hz, 1H), 7.25 (d, *J* = 7.6 Hz, 1H), 7.14 (dd, *J* = 8.8, 2.4 Hz, 1H), 5.14 (dd, *J* = 13.3, 5.1 Hz, 1H), 4.68 (d, *J* = 12.8 Hz, 2H), 4.58 - 4.51 (m, 1H), 4.43 (d, *J* = 17.4 Hz, 1H), 4.28 (d, *J* = 17.4 Hz, 1H), 3.91 - 3.83 (m, 1H), 3.63 (s, 2H), 3.24 - 3.15 (m, 2H), 3.05 (t, *J* = 12.9 Hz, 2H), 3.00 - 2.86 (m, 3H), 2.68 - 2.60 (m, 1H), 2.38 (d, *J* = 12.8 Hz, 1H), 2.23 (s, 2H), 2.14 - 1.96 (m, 6H), 1.94 - 1.83 (m, 5H), 1.65 (dd, *J* = 23.9, 10.9 Hz, 4H), 1.52 (dd, *J* = 22.8, 10.5 Hz, 2H).
LC-MS: [M+H]⁺ =807.46

### Example 209

### Synthesis of compound 172

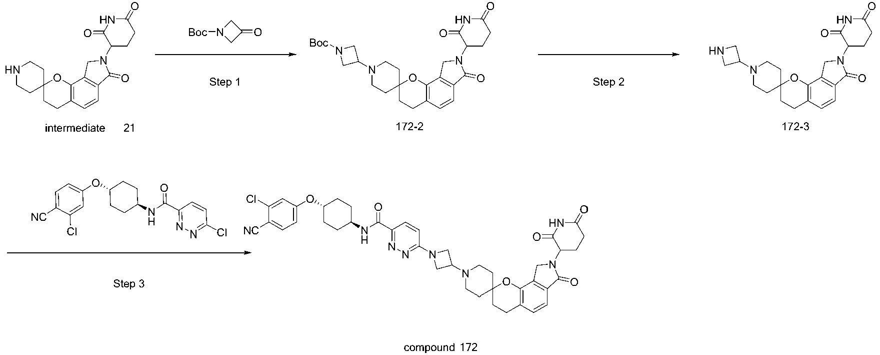

Step 1: The intermediate 21 (50.0 mg, 135.44 µmol) and tert-butyl 3-oxoazetidine-1-carboxylate (23.2 mg, 135.44 µmol) were added sequentially to a glass vial, solvents THF/DMSO (2 mL) and Ti(O-iPr)₄(76.9 mg, 270.88 µmol) were added, and the reaction solution was stirred at 60°C for 1 h. Then NaBH₃CN (17.0 mg, 270.88 µmol) was added and the reaction solution was continued to be stirred at 60°C for 1 h. After the reaction was completed, it was concentrated, and then purified by preparation to obtain a white solid compound 172-2 (20 mg).
LC-MS: [M+H]⁺ =525.19

Step 2: The intermediate 42-2 (20.0 mg, 38.15 µmol) was added to a glass vial, solvent TFA/DCM (1 mL) was added, and the reaction solution was stirred at room temperature for 0.5 h. When the reaction was completed, the reaction solution was concentrated and then lyophilized to obtain a white solid compound 172-3 (18 mg).
LC-MS: [M+H]⁺ =425.40

Step 3: (Compound 172) was prepared with reference to step 3 of Example 19.
¹H NMR (600 MHz, DMSO-d6) δ 11.05 (s, 1H), 8.65 (d, *J* = 8.1 Hz, 1H), 7.95 (d, *J* = 9.2 Hz, 1H), 7.87 (d, *J* = 8.8 Hz, 1H), 7.39 (d, *J* = 2.4 Hz, 1H), 7.27 (dd, *J* = 28.4, 7.7 Hz, 2H), 7.14 (dd, *J* = 8.8, 2.4 Hz, 1H), 7.03 - 6.98 (m, 1H), 5.15 (dd, *J* = 13.0, 4.4 Hz, 1H), 4.54 (ddd, *J* = 14.5, 10.3, 4.1 Hz, 2H), 4.36 (dd, *J* = 71.6, 19.4 Hz, 7H), 3.91 - 3.83 (m, 2H), 3.17 (s, 2H), 2.93 (dt, *J* = 13.2, 10.1 Hz, 3H), 2.68 - 2.60 (m, 1H), 2.37 (dd, *J* = 22.8, 6.7 Hz, 1H), 2.16 - 2.00 (m, 5H), 1.90 (d, *J* = 11.8 Hz, 6H), 1.66 (dd, *J* = 24.0, 10.7 Hz, 2H), 1.52 (dd, *J* = 23.0, 10.0 Hz, 2H).
LC-MS: [M+H]⁺ =779.46

### Example 210

### Synthesis of compound 173

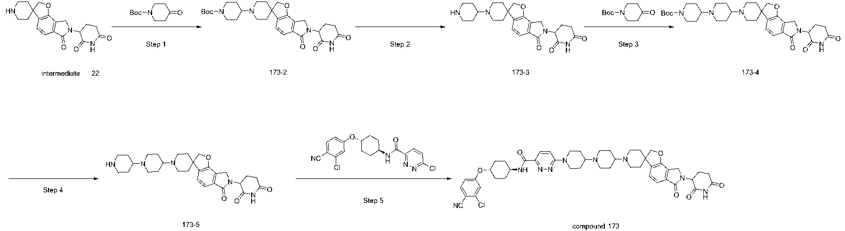

Step 1: The intermediate 22 (70.0 mg, 197.10 µmol) and tert-butyl 4-oxopiperidine-1-carboxylate (58.8 mg, 295.65 µmol) were added sequentially to a glass vial, solvents THF:DMSO = 2 : 1 (2 mL) and Ti(O-iPr)₍₄₎(112.0 mg, 394.20 µmol) were added, and then the reaction solution was stirred at 60 °C for 1 h. Then NaBH₃CN (24.8 mg, 394.20 µmol) was added and the reaction solution was continued to be stirred at 60 for 1 h. When the reaction was completed, it was concentrated, and then purified by preparation to obtain a white solid compound 173-2 (78 mg).
LC-MS: [M+H]⁺ =539.18

Step 2: (Compound 173-3) was prepared with reference to step 2 of example 39.
LC-MS: [M+H]⁺ =439.29

Step 3: The intermediate 173-4 (43.8 mg, 99.95 µmol) and tert-butyl 4-oxopiperidine-1-carboxylate (29.9 mg, 149.92 µmol) were added sequentially to a glass vial, the solvents THF:DMSO = 2 : 1 (2 mL) and Ti(O-iPr)₄ (56.8 mg, 199.90 µmol) were added. The reaction solution was stirred at 60°C for 1 h. Then NaBH₃CN (12.6 mg, 199.90 µmol) was added and the reaction solution was continued to be stirred at 60°C for 1 h. After the reaction was completed, the reaction solution was concentrated, and then purified by preparation to obtain a white solid compound 173-4 (27.2 mg).
LC-MS: [M+H]⁺ =622.58

Step 4: (Compound 173-5) was prepared with reference to step 2 of Example 39.
LC-MS: [M+H]⁺ =522.39

Step 5: (Compound 173) was prepared with reference to step 3 of Example 19.
¹H NMR (600 MHz, DMSO-d6) δ 7.87 (d, *J* = 9.3 Hz, 1H), 7.80 (d, *J* = 8.7 Hz, 1H), 7.36 (dd, *J* = 33.6, 23.1 Hz, 4H), 7.08 (d, *J* = 8.7 Hz, 1H), 5.00 (d, *J* = 8.4 Hz, 1H), 4.71 - 4.58 (m, 3H), 4.51 (s, 1H), 4.40 (d, *J* = 17.3 Hz, 1H), 4.25 (d, *J* = 17.3 Hz, 1H), 3.84 (s, 2H), 3.57 (dd, *J* = 78.8, 22.7 Hz, 6H), 3.18 (dd, *J* = 32.4, 20.2 Hz, 2H), 2.88 - 2.79 (m, 1H), 2.62 (d, *J* = 12.4 Hz, 1H), 2.37 (d, *J* = 29.6 Hz, 4H), 2.23 - 1.85 (m, 13H), 1.68 - 1.45 (m, 6H), 1.23 - 1.14 (m, 3H).
LC-MS: [M+H]⁺ =876.46

### Example 211

### Synthesis of compound 174

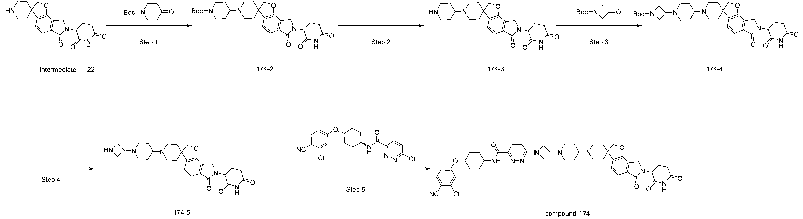

Step 1: The intermediate 22 (150.0 mg, 422.36 µmol) and tert-butyl 4-oxopiperidine-1-carboxylate (126.2 mg, 633.54 µmol) were added sequentially to a glass vial, and the solvents THF:DMSO = 2 : 1 (5 mL) and Ti(O-iPr)₄ (240 mg, 844.72 µmol) were added, and the reaction solution was stirred at 60 °C for 1 h. Then NaBH₃)CN (53.1 mg, 844.54 µmol) was added, and the reaction solution was continued to be stirred at 60 for 1 h. After the reaction was completed, it was concentrated, and then purified by preparation to obtain a white solid compound 174-2 (100 mg).
LC-MS: [M+H]⁺ =539.48

Step 2: (Compound 174-3) was prepared with reference to step 2 of example 39.
LC-MS: [M+H]⁺ =439.40

Step 3: The intermediate 174-3 (72.0 mg, 164.30 µmol) and tert-butyl 3-oxoazetidine-1-carboxylate (280.9 mg, 164.3 µmol) were added sequentially to a glass vial, and the solvents THF (2 mL) and Ti(O-iPr)₄(933.2 mg, 328.6 µmol) were added, and the reaction solution was stirred at 60°C for 1 h. Then NaBH₃CN (206.5 mg, 328.6 µmol) was added, and the reaction solution was continued to be stirred at 60°C for 1 h. After the reaction was completed, it was concentrated, and then purified by preparation to obtain a white solid compound 174-4 (43 mg).
LC-MS: [M+H]⁺ =594.08

Step 4: The intermediate 174-4 (43.0 mg, 72.47 µmol) was added to a glass vial, solvent TFA/DCM (2 mL) was added, and the reaction solution was stirred at room temperature for 0.5 h. When the reaction was completed, the reaction solution was concentrated and then lyophilized to obtain a white solid compound 174-5 (40.2 mg).
LC-MS: [M+H]⁺ =494.50

Step 5: (Compound 174) was prepared with reference to step 3 of Example 19.
¹H NMR (600 MHz, DMSO-d6) δ 11.00 (s, 1H), 8.57 (d, *J* = 8.1 Hz, 1H), 7.87 (dd, *J* = 8.9, 3.1 Hz, 2H), 7.39 (d, *J* = 2.4 Hz, 1H), 7.34 (t, *J* = 14.7 Hz, 2H), 7.14 (dd, *J* = 8.8, 2.4 Hz, 1H), 6.89 (d, *J* = 9.1 Hz, 1H), 5.10 (dd, *J* = 13.3, 5.0 Hz, 1H), 4.72 - 4.62 (m, 2H), 4.58 - 4.51 (m, 1H), 4.41 (dd, *J* = 17.2, 8.5 Hz, 1H), 4.23 (t, *J* = 19.1 Hz, 3H), 4.00 (s, 2H), 3.90 - 3.82 (m, 1H), 3.57 (s, 2H), 3.23 - 3.17 (m, 1H), 3.11 (dd, *J* = 7.2, 4.7 Hz, 4H), 2.97 - 2.89 (m, 1H), 2.60 (d, *J* = 17.8 Hz, 1H), 2.42 (ddd, *J* = 20.5, 10.3, 5.3 Hz, 1H), 2.23 - 2.07 (m, 6H), 2.03 - 1.86 (m, 6H), 1.76 - 1.60 (m, 4H), 1.52 (dd, *J* = 23.0, 9.9 Hz, 3H).
LC-MS: [M+H]⁺ =848.46

### Example 212

### Synthesis of compound 175

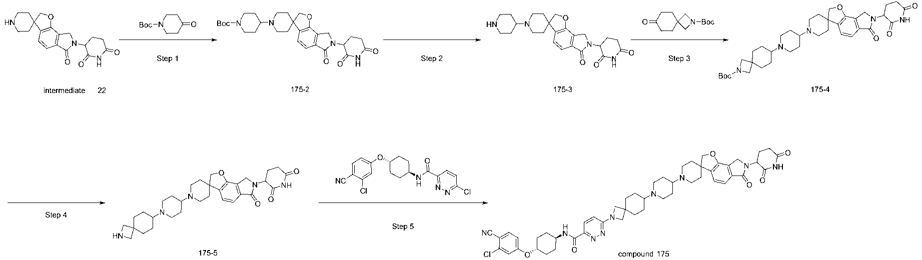

Step 1: The intermediate 22 (150.0 mg, 422.36 µmol) and tert-butyl 4-oxopiperidine-1-carboxylate (126.2 mg, 633.54 µmol) were added sequentially to a glass vial, and the solvents THF:DMSO = 2 : 1 (5 mL) and Ti(O-iPr)₄ (240 mg, 844.72 µmol) were added, and the reaction solution was stirred at 60°C for 1 h. Then NaBH₃CN (53.1 mg, 844.54 µmol) was added, and the reaction solution was continued to be stirred at 60 °C for 1 h. After the reaction was completed, it was concentrated, and then purified by preparation to obtain a white solid compound 175-2 (100 mg).
LC-MS: [M+H]⁺ =539.38

Step 2: (Compound 175-3) was prepared with reference to step 2 of example 39.
LC-MS: [M+H]⁺ =439.45

Step 3: The intermediate 175-3 (100.0 mg, 228.19 µmol) and tert-butyl 7-oxo-2-azaspiro[3.5]nonane-2-carboxylate (109.1 mg, 456.38 µmol) were added sequentially to a glass vial, followed by the addition of solvents THF:DMSO = 2 : 1 (4 mL) and Ti(O-iPr)₄ (129.6 mg, 456.38 µmol), and the reaction solution was stirred at 60°C for 1 h. Then NaBH₃CN (28.7 mg, 456.38 µmol) was added, and the reaction solution was continued to be stirred at 60°C for 1 h. When the reaction was completed, the reaction was concentrated, and then purified by preparation to obtain a white solid compound 175-4 (110 mg).
LC-MS: [M+H]⁺ =662.60

Step 4: (Compound 175-5) was prepared with reference to step 2 of Example 39.
LC-MS: [M+H]⁺ =562.55

Step 5: (Compound 175) was prepared with reference to step 3 of Example 19.
¹H NMR (600 MHz, DMSO-d6) δ 10.99 (s, 1H), 8.55 (d, *J* = 8.2 Hz, 1H), 8.40 (s, 1H), 7.84 (dd, *J* = 23.8, 9.0 Hz, 2H), 7.41 - 7.38 (m, 1H), 7.27 (d, *J* = 7.6 Hz, 1H), 7.14 (dd, *J =* 8.8, 2.4 Hz, 1H), 6.83 (d, *J* = 9.3 Hz, 1H), 5.09 (dd, *J* = 13.3, 5.1 Hz, 1H), 4.56 - 4.48 (m, 3H), 4.38 (d, *J* = 17.1 Hz, 1H), 4.22 (d, *J* = 17.0 Hz, 1H), 3.84 (s, 3H), 3.77 (s, 3H), 2.89 (dd, *J* = 21.6, 8.3 Hz, 5H), 2.60 (dd, *J* = 12.8, 10.2 Hz, 1H), 2.41 (dd, *J* = 13.1, 8.5 Hz, 1H), 2.31 - 2.25 (m, 1H), 2.23 - 2.07 (m, 7H), 2.01 - 1.94 (m, 3H), 1.89 (dd, *J* = 17.1, 8.7 Hz, 4H), 1.68 (m, 7H), 1.52 (d, *J* = 9.5 Hz , 4H), 1.41 (dd, *J* = 20.6, 11.1 Hz, 2H), 1.30 (dd, *J* = 22.6, 10.8 Hz, 2H).
LC-MS: [M+H]⁺ =916.55

### Example 215

### Synthesis of compound 178

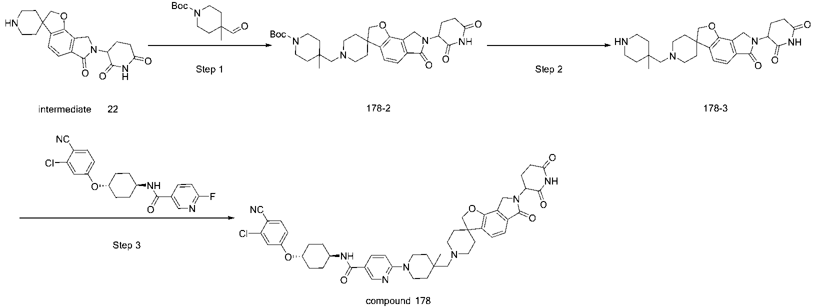

Step 1: The intermediate 22 (80.0 mg, 225.26 µmol) and tert-butyl 4-formyl-4-methylpiperidine-1-carboxylate (51.2 mg, 225.26 µmol) were added sequentially to a glass vial, and the solvents THF:DMSO = 1 : 2 (3 mL) and Ti(O-iPr)₄ (128 mg, 450.51 µmol) were added, and the reaction solution was stirred at 60°C for 1 h. Then NaBH₃CN (28.3 mg, 450.51 µmol) was added and the reaction solution was continued to be stirred at 60°C for 1 h. After the reaction was completed, the reaction solution was concentrated, and then purified by preparation to obtain a white solid compound 178-2 (90 mg).
LC-MS: [M+H]⁺ =567.78

Step 2: (Compound 178-3) was prepared with reference to step 2 of Example 39.
LC-MS: [M+H]⁺ =467.39

Step 3: (Compound 178) was prepared with reference to step 3 of Example 19.
¹H NMR (600 MHz, DMSO-d6) δ 10.99 (s, 1H), 8.61 (d, *J* = 2.2 Hz, 1H), 8.09 - 7.96 (m, 2H), 7.87 (d, *J* = 8.8 Hz, 1H), 7.38 (dd, *J* = 15.5, 4.9 Hz, 2H), 7.29 (d, *J* = 7.6 Hz, 1H), 7.15 (dd, *J* = 8.8, 2.4 Hz, 1H), 6.90 (d, *J* = 9.1 Hz, 1H), 5.10 (dd, *J* = 13.2, 4.9 Hz, 1H), 4.67 (q, *J* = 9.6 Hz, 2H), 4.59 - 4.51 (m, 1H), 4.40 (dd, *J* = 17.1, 6.2 Hz, 1H), 4.24 (d, *J* = 17.2 Hz, 1H), 4.03 (d, *J* = 13.6 Hz, 2H), 3.80 (dd, *J* = 7.9, 4.1 Hz, 1H), 3.39 - 3.13 (m, 7H), 2.96 - 2.88 (m, 1H), 2.60 (d, *J* = 17.3 Hz, 1H), 2.45 - 2.27 (m, 4H), 2.11 (d, *J* = 9.6 Hz, 2H), 2.02 - 1.87 (m, 5H), 1.53 (ddd, *J* = 24.8, 19.8, 13.7 Hz, 9H), 1.27 (s, 3H).
LC-MS: [M+H]⁺ =820.65

### Example 216

### Synthesis of compound 179

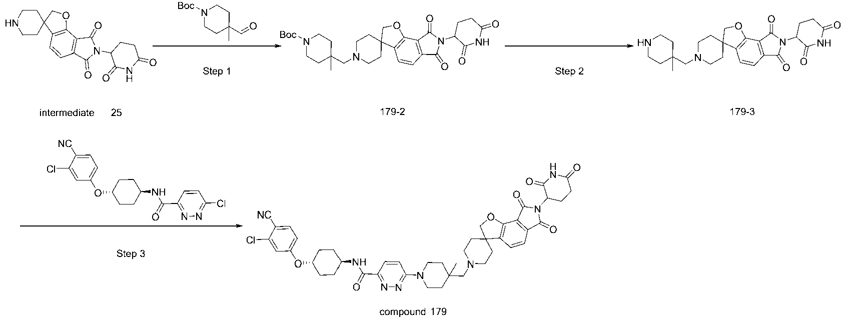

Step 1: The intermediate 25 (100.0 mg, 270.72 µmol) and tert-butyl 4-formyl-4-methylpiperidine-1-carboxylate (123.1 mg, 541.45 µmol) were added sequentially to a glass vial, and the solvents THF:DMSO = 1 : 2 (4 mL) and Ti(O-iPr)₄ (153.9 mg, 541.45 µmol) were added, and the reaction solution was stirred at 60°C for 1 h. Then NaBH₃CN (34.0 mg, 541.45 µmol) was added, and the reaction solution was continued to be stirred at 60°C for 1 h. When the reaction was completed, the reaction solution was concentrated, and then purified by preparation to obtain a white solid compound 179-2 (40 mg).
LC-MS: [M+H]⁺ =581.48

Step 2: (Compound 179-3) was prepared with reference to step 2 of Example 39.
LC-MS: [M+H]⁺ =481.40

Step 3: (Compound 179) was prepared with reference to step 3 of Example 19.
¹H NMR (400 MHz, DMSO) δ 11.12 (s, 1H), 8.61 (d, *J* = 8.2 Hz, 1H), 7.86 (dd, *J* = 9.1, 5.2 Hz, 2H), 7.58 - 7.49 (m, 2H), 7.39 (dd, *J* = 5.9, 3.5 Hz, 2H), 7.14 (dd, *J* = 9.0, 2.5 Hz, 1H), 5.11 (dd, *J* = 12.8, 5.4 Hz, 1H), 4.82 (s, 2H), 4.59 - 4.48 (m, 1H), 4.12 (d, *J* = 13.7 Hz, 2H), 3.86 (dd, *J* = 11.6, 7.4 Hz, 1H), 3.47 (s, 1H), 3.27 (dd, *J* = 45.0, 34.2 Hz, 7H), 2.95 - 2.83 (m, 1H), 2.60 (d, *J* = 18.8 Hz, 1H), 2.42 - 2.30 (m, 2H), 2.00 (ddd, *J* = 40.7, 30.1, 9.4 Hz, 7H), 1.71 - 1.46 (m, 8H), 1.28 (s, 3H).
LC-MS: [M+H]⁺ =835.55

### Example 217

### Synthesis of compound 180

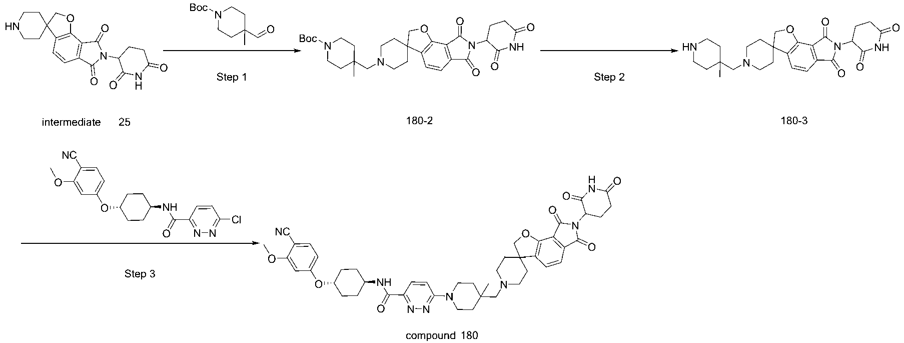

Step 1: The intermediate 25 (100.0 mg, 270.72 µmol) and tert-butyl 4-formyl-4-methylpiperidine-1-carboxylate (123.1 mg, 541.45 µmol) were added sequentially to a glass vial, and the solvents THF:DMSO = 1 : 2 (4 mL) and Ti(O-iPr)₄ (153.9 mg, 541.45 µmol) were added, and the reaction solution was stirred at 60°C for 1 h. Then NaBH₃CN (34.0 mg, 541.45 µmol) was added, and the reaction solution was continued to be stirred at 60°C for 1 h. When the reaction was completed, the reaction solution was concentrated, and then purified by preparation to obtain a white solid compound 180-2 (40 mg).
LC-MS: [M+H]⁺ =581.48

Step 2: (Compound 180-3) was prepared with reference to step 2 of example 39.
LC-MS: [M+H]⁺ =481.40

Step 3: (Compound 180) was prepared with reference to step 3 of Example 19.
¹ H NMR (600 MHz, DMSO) δ 11.11 (s, 1H), 8.59 (d, *J* = 8.2 Hz, 1H), 7.85 (d, *J* = 9.5 Hz, 1H), 7.62 (d, *J* = 9.2 Hz, 1H), 7.54 (dd, *J* = 34.3, 7.3 Hz, 2H), 7.39 (d, *J* = 9.6 Hz, 1H), 6.72 (d, *J* = 7.1 Hz, 2H), 5.11 (dd, *J* = 12.9, 5.4 Hz, 1H), 4.82 (s, 2H), 4.50 (ddd, *J* = 14.3, 10.1, 3.9 Hz, 1H), 4.12 (d, *J* = 13.4 Hz, 2H), 3.90 (s, 3H), 3.50 - 3.45 (m, 3H), 3.39 - 3.14 (m, 6H), 2.93 - 2.85 (m, 1H), 2.63 - 2.57 (m, 1H), 2.42 - 2.27 ( m, 2H), 2.13 (d, *J* = 10.1 Hz, 2H), 1.98 (ddd, *J* = 46.8, 22.2, 8.0 Hz, 5H), 1.70 - 1.47 (m, 9H), 1.29 (s, 3H).
LC-MS: [M+H]⁺ =831.56

### Example 218

### Synthesis of compound 181

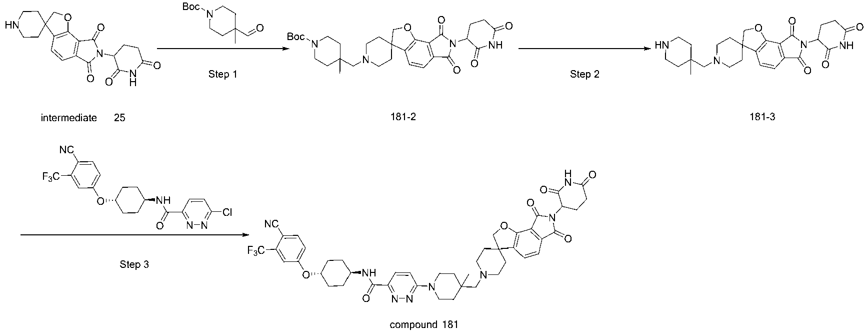

Step 1: The intermediate 25 (100.0 mg, 270.72 µmol) and tert-butyl 4-formyl-4-methylpiperidine-1-carboxylate (123.1 mg, 541.45 µmol) were added sequentially to a glass vial, and the solvents THF:DMSO = 1 : 2 (4 mL) and Ti(O-iPr)₄(153.9 mg, 541.45 µmol) were added, and the reaction solution was stirred at 60°C for 1 h. Then NaBH₃CN (34.0 mg, 541.45 µmol) was added, and the reaction solution was continued to be stirred at 60°C for 1 h. When the reaction was completed, the reaction solution was concentrated, and then purified by preparation to obtain a white solid compound 181-2 (40 mg).
LC-MS: [M+H]⁺ =581.48

Step 2: (Compound 181-3) was prepared with reference to step 2 of Example 39.
LC-MS: [M+H]⁺ =481.40

Step 3: (Compound 181) was prepared with reference to step 3 of Example 19.
¹H NMR (400 MHz, DMSO) δ 11.12 (s, 1H), 8.62 (d, *J* = 8.2 Hz, 1H), 8.08 (d, *J* = 8.3 Hz, 1H), 7.86 (d, *J* = 9.5 Hz, 1H), 7.58 - 7.46 (m, 4H), 7.40 (d, *J* = 9.6 Hz, 1H), 5.11 (dd, *J* = 12.7, 5.4 Hz, 1H), 4.82 (s, 2H), 4.69 - 4.59 (m, 1H), 4.12 (d, *J* = 13.8 Hz, 3H), 3.40 (ddd, *J* = 69.1, 49.2, 22.5 Hz, 10H), 2.94 - 2.83 (m, 1H), 2.60 (d, *J* = 18.6 Hz, 1H), 2.42 - 2.28 (m, 2H), 2.01 (ddd, *J =* 35.6, 31.4, 12.2 Hz, 7H), 1.59 (dt, *J* = 22.7, 10.8 Hz, 8H), 1.29 (s, 3H).
LC-MS: [M+H]⁺ =869.56

### Example 219

### Synthesis of compound 182

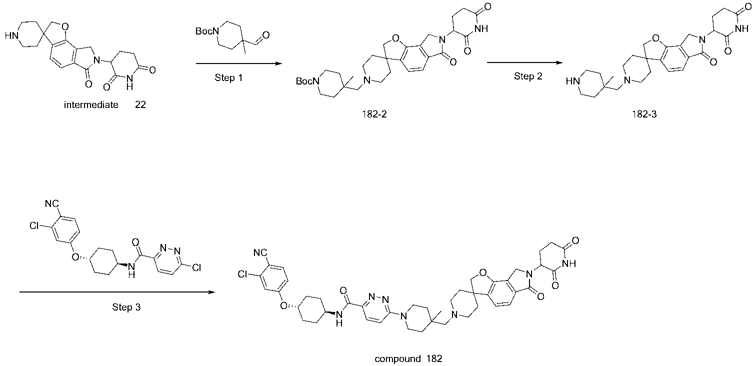

Step 1: The intermediate 22 (50 mg, 0.141 mmol) and tert-butyl 4-formyl-4-methylpiperidine-1-carboxylate (38.37 mg, 0.169 mmol) were added sequentially to a glass vial, and the solvent THF (2 mL) and Ti(O-iPr)₄ (79.97 mg, 0.281 mmol) were added and the reaction solution was stirred at 60°C for 1 h. Then NaBH₃CN (26.52 mg, 0.422 mmol) was added and the reaction solution was continued to be stirred at 60°C for 1 h. After the reaction was completed, the reaction solution was concentrated, and then purified by preparative thin-layer chromatography (DCM:MeOH=10:1) to obtain a white solid (compound 182-2) (25 mg).

Step 2: (Compound 182-3) was prepared with reference to step 2 of example 39.
LC-MS: [M+H]⁺= 467.49

Step 3: (Compound 182) was prepared with reference to step 3 of Example 19.
¹H NMR (600 MHz, DMSO-d6) δ 11.00 (s, 1H), 8.62 (d, *J* = 8.2 Hz, 1H), 8.57 (s, 1H), 7.86 (dd, *J* = 9.2, 7.5 Hz, 2H), 7.44-7.34 (m, 3H), 7.28 (d, *J* = 7.6 Hz , 1H), 7.14 (dd, *J* = 8.8, 2.5 Hz, 1H), 5.36-5.30 (m, 1H), 5.10 (dd, *J* = 13.3, 5.0 Hz, 1H), 4.70-4.63 (m, 2H), 4.54 (dt, *J* = 10.5, 6.0 Hz , 2H), 4.40 (d, *J* = 17.2 Hz, 1H), 4.24 (d, *J* = 17.2 Hz, 1H), 4.13 (d, *J* = 13.8 Hz, 2H), 3.87 (dd, *J* = 7.9, 3.9 Hz, 2H), 3.29-3.23 (m, 2H), 3.21 -3.14 (m, 2H), 2.92 (td, *J* = 17.3, 15.4, 5.4 Hz, 2H), 2.59 (s, 2H), 2.48 - 2.30 (m, 4H), 2.11 (d, *J* = 11.4 Hz, 2H), 2.00- 1.94 (m, 2H), 1.91 (d, *J* = 14.8 Hz, 3H), 1.65 (d, *J* = 13.2 Hz, 5H), 1.58-1.41 (m, 4H).
LC-MS: [M+H]+=821.56

### Example 220

### Synthesis of compound 183

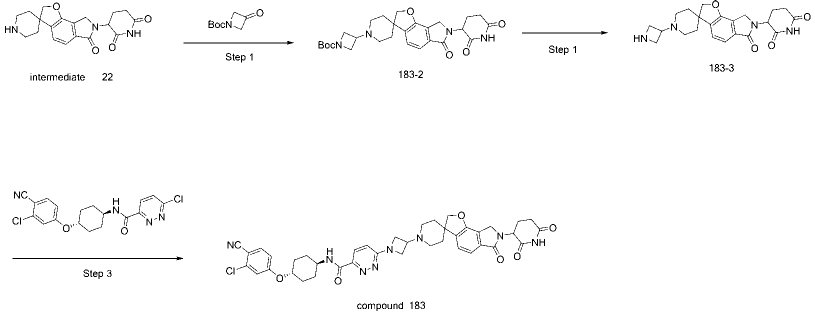

Step 1: The intermediate 22 (200.0 mg, 562.76 µmol) and tert-butyl 3-oxoazetidine-1-carboxylate (115.61 mg, 675.31 µmol) were added sequentially to a glass vial, solvent THF (2 mL) and Ti(O-iPr)₄ (319.89 mg, 1.13 mmol) were added, and the reaction solution was stirred at 60°C for 1 h. Then NaBH₃CN (106.09 mg, 1.69 mmol) was added and the reaction solution was continued to be stirred at 60°C for 1 h. After the reaction was completed, it was concentrated, and then purified by preparation to obtain a white solid compound 183-2 (40 mg).
LC-MS: [M+H]⁺ =511.39

Step 2: (Compound 183-3) was prepared with reference to step 2 of example 39.

Step 3: (Compound 183) was prepared with reference to step 3 of example 19.

¹H NMR (600 MHz, DMSO-d6) δ 11.00 (s, 1H), 8.64 (d, *J* = 8.2 Hz, 1H), 7.95 (d, *J* = 9.2 Hz, 1H), 7.87 (d, *J* = 8.8 Hz, 1H), 7.40 (d, *J* = 2.4 Hz, 1H), 7.37 - 7.19 (m, 2H), 7.14 (dd, *J* = 8.8, 2.4 Hz, 1H), 6.99 (d, *J* = 9.1 Hz, 1H), 5.11 (dd, *J* = 13.3, 5.1 Hz, 1H), 4.67 (s, 2H), 4.54 (tt, *J* = 10.0, 4.3 Hz, 1H), 4.50 - 4.17 (m, 6H), 3.88 (tdt, *J* = 11.7, 8.2, 4.1 Hz, 1H), 3.59 (s, 2H), 3.10 (s, 2H), 2.95 - 2.87 (m, 1H), 2.64 - 2.56 (m, 1H), 2.47 - 2.38 (m, 1H), 2.28 - 1.83 (m, 9H), 1.66 (qd, *J* = 13.2, 3.2 Hz, 2H), 1.52 (dt, *J* = 13.1, 9.6 Hz, 2H), 1.24 (s, 1H).

### Example 221

### Synthesis of compound 184

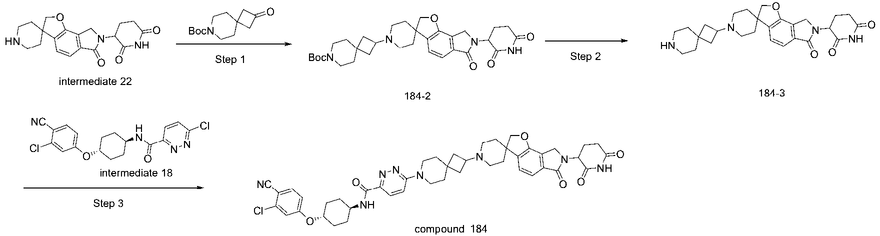

Step 1: The intermediate 22 (50.0 mg, 140.69 µmol) and tert-butyl 2-oxo-7-azaspiro[3.5]nonane-7-carboxylate (40.40 mg, 168.83 µmol) were added sequentially to a glass vial, and the solvent THF (2 mL) and Ti(O-iPr)₄(79.97 mg, 281.32 µmol) were added, and the reaction solution was stirred at 60°C for 1 h. Then NaBH₃CN (26.52 mg, 422.07 umol) was added, and the reaction solution was continued to be stirred at 60°C for 1 h. When the reaction was completed, the reaction solution was concentrated, and then purified by preparation to obtain a white solid compound 184-2 (40 mg).
¹H NMR (600 MHz, DMSO-d6) δ 11.00 (s, 1H), 7.36 (d, *J* = 7.5 Hz, 1H), 7.30 (d, *J* = 7.5 Hz, 1H), 5.10 (dd, *J* = 13.3, 5.0 Hz, 1H), 4.72 - 4.61 (m, 2H), 4.40 (d, *J* = 17.2 Hz, 1H), 4.24 (d, *J* = 17.1 Hz, 1H), 3.27 (d, *J* = 43.3 Hz, 6H), 2.96 - 2.83 (m, 3H), 2.48 - 2.35 (m, 2H), 2.25 - 1.95 (m, 10H), 1.49 (q, *J* = 7.1, 4.8 Hz, 4H), 1.40 (d, *J* = 3.0 Hz, 9H).
LC-MS: [M+H]⁺ =579.50

Step 2: (Compound 184-3) was repared with reference to step 2 of example 39.
LC-MS: [M+H]⁺ = 479.49

Step 3: (Compound 184) was prepared with reference to step 3 of example 19.
¹H NMR (600 MHz, DMSO-d6) δ 11.01 (s, 1H), 9.85 (d, *J* = 58.3 Hz, 2H), 8.60 (dd, *J =* 8.3, 3.2 Hz, 1H), 7.89 - 7.80 (m, 2H), 7.40 (d, *J* = 2.5 Hz, 1H), 7.39 - 7.29 (m, 2H), 7.14 (dd, *J* = 8.8, 2.4 Hz, 1H), 5.11 (dd, *J* = 13.3, 5.1 Hz, 1H), 4.71 - 4.48 (m, 4H), 4.41 (d, *J* = 17.2 Hz, 1H), 4.25 (d, *J* = 17.1 Hz, 1H), 3.89 - 3.84 (m, 2H), 3.74 (d, *J* = 6.9 Hz, 4H), 3.46 (s, 2H), 3.00 - 2.84 (m, 3H), 2.59 (d, *J* = 3.4 Hz, 1H), 2.43 (dt, *J* = 13.5, 6.7 Hz, 2H), 2.28 (t, *J* = 10.2 Hz, 2H), 2.18 - 1.94 (m, 8H), 1.93 - 1.87 (m, 2H), 1.64 (tt, *J* = 12.9, 5.1 Hz, 5H), 1.52 (td, *J* = 13.7, 7.2 Hz, 2H).
LC-MS: [M+H]⁺ = 833.46

### Example 222

### Synthesis of compound 185

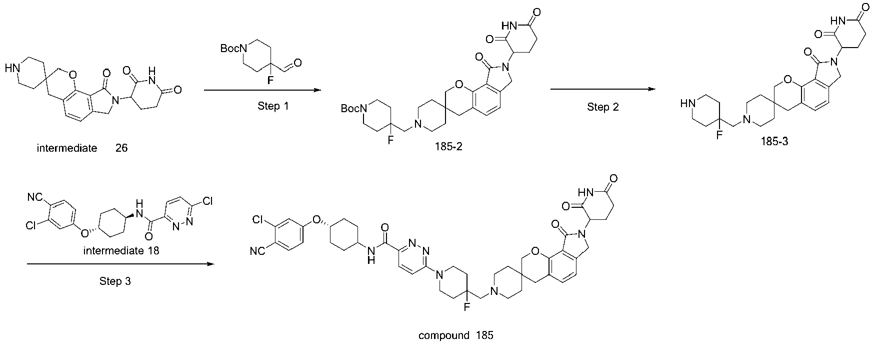

Step 1: The intermediate 26 (50.0 mg, 135.35 µmol) and tert-butyl 4-fluoro-4-formylpiperidine-1-carboxylate (37.55 mg, 162.42 µmol) were added sequentially to a glass vial, solvent THF (2 mL) and Ti(O-iPr)₄ (76.94 mg, 270.69 µmol) were added, and the reaction solution was stirred at 60°C for 1 h. Then NaBH₃CN (25.52 mg, 406.04 umol) was added, and the reaction solution was continued to be stirred at 60°C for 1 h. After the reaction was completed, it was concentrated, and then purified by preparation to obtain a white solid compound 185-2 (40 mg).
LC-MS: [M+H]⁺ = 585.38

Step 2: (Compound 185-3) was prepared with reference to step 2 of example 39.
¹H NMR (600 MHz, DMSO-d6) δ 10.98 (s, 1H), 7.35 - 7.25 (m, 1H), 7.06 (s, 1H), 5.01 (d, *J* = 11.4 Hz, 1H), 4.37 - 4.15 (m, 5H), 3.53 (s, 2H), 3.32 (d, *J* = 13.1 Hz, 4H), 3.06 (q, *J* = 11.8, 11.3 Hz, 2H), 2.98 - 2.83 (m, 2H), 2.66 (s, 1H), 2.62 - 2.56 (m, 1H), 2.35 (td, *J* = 13.2, 4.5 Hz, 1H), 2.18 (s, 2H), 2.06 - 1.70 (m, 6H), 1.65 (d, *J* = 14.0 Hz, 1H).
LC-MS: [M+H]⁺ = 485.45

Step 3: (Compound 185) was prepared with reference to step 3 of example 19.
¹H NMR (600 MHz, DMSO-d6) δ 10.97 (s, 1H), 9.34 (d, *J* = 33.3 Hz, 2H), 8.65 (d, *J* = 8.1 Hz, 1H), 7.88 (dd, *J* = 11.6, 9.2 Hz, 2H), 7.49 (d, *J* = 9.6 Hz, 1H), 7.40 (d, *J* = 2.4 Hz, 1H), 7.31 (dd, *J* = 22.7, 7.7 Hz, 1H), 7.14 (dd,*J* = 8.8, 2.4 Hz, 1H), 7.06 (t, *J* = 7.9 Hz, 1H), 5.02 (ddd, *J* = 14.6, 9.9, 5.1 Hz, 1H), 4.54 (tt, *J* = 9.9, 4.2 Hz, 1H ), 4.39 (d, *J* = 13.0 Hz, 2H), 4.35 - 4.15 (m, 5H), 3.45 (d, *J* = 13.8 Hz, 2H), 3.37 (t, *J* = 12.3 Hz, 2H), 3.33 - 3.25 (m, 2H), 2.96 (d, *J* = 6.5 Hz, 1H), 2.93 - 2.87 (m, 1H), 2.67 (s, 1H), 2.64 - 2.55 (m, 2H), 2.35 (t, *J* = 13.5 Hz, 2H), 2.15 - 2.02 (m, 4H), 1.98 - 1.87 (m, 4H), 1.86 - 1.71 (m, 4H), 1.69 - 1.60 (m, 3H), 1.52 (dt, *J* = 13.4, 10.1 Hz, 2H).
LC-MS: [M+H]⁺ = 839.46

### Example 223

### Synthesis of compound 186

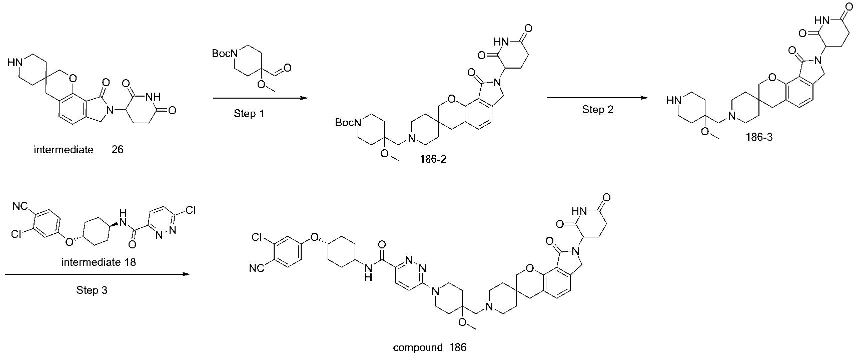

Step 1: The intermediate 26 (50.0 mg, 135.35 µmol) and tert-butyl 4-formyl-4-methoxypiperidine-1-carboxylate (39.52 mg, 162.42 µmol) were added sequentially to a glass vial, solvent THF (2 mL) and Ti(O-iPr)₄ (76.94 mg, 270.69 µmol) were added, and the reaction solution was stirred at 60 °C for 1 h. Then NaBH₃CN (25.52 mg, 406.04 umol) was added and the reaction solution was continued to be stirred at 60 °C for 1 h. After the reaction was completed, it was concentrated, and then purified by preparation to obtain a white solid compound 186-2 (35 mg).
LC-MS: [M+H]⁺ = 597.48

Step 2: Compound 186-3 was prepared with reference to step 2 of Example 39.
LC-MS: [M+H]⁺ = 497.50

Step 3: Compound 186 was prepared with reference to step 3 of Example 19.
¹H NMR (600 MHz, DMSO-d6) δ 10.97 (d, *J* = 2.1 Hz, 1H), 8.83 (d, *J* = 35.5 Hz, 1H), 8.63 (d, *J* = 8.2 Hz, 1H), 7.87 (dd, *J* = 9.1, 2.7 Hz, 2H), 7.43 (d, *J* = 9.6 Hz, 1H), 7.40 (d, *J* = 2.4 Hz, 1H), 7.31 (dd, *J* = 21.4, 7.7 Hz, 1H), 7.14 (dd, *J* = 8.8, 2.4 Hz, 1H), 7.06 (dd, *J* = 10.4, 7.6 Hz, 1H), 5.02 (ddd, *J* = 13.3, 11.5, 5.2 Hz, 1H), 4.54 (tt, *J* = 10.0, 4.2 Hz, 1H), 4.37 - 4.15 (m, 6H), 3.95 (s, 2H), 3.91 - 3.83 (m, 2H), 3.48 (d, *J* = 13.8 Hz, 2H), 3.37 (d, *J* = 5.2 Hz, 2H), 3.28 (d, *J* = 6.1 Hz, 6H), 2.98 - 2.86 (m, 2H), 2.67 - 2.56 (m, 2H), 2.36 (dt, *J* = 13.2, 4.8 Hz, 1H), 2.16 - 2.07 (m, 2H), 2.00 - 1.80 (m, 6H), 1.73 (d, *J* = 12.8 Hz, 1H), 1.67 - 1.56 (m, 4H), 1.52 (dt, *J* = 13.7, 9.8 Hz, 2H).
LC-MS: [M+H]⁺ = 851.56

### Example 224

### Synthesis of compound 187

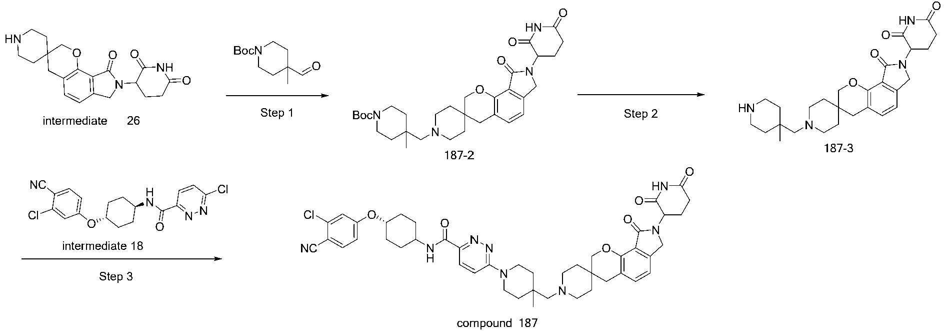

Step 1: The intermediate 26 (50.0 mg, 135.35 µmol) and tert-butyl 4-formyl-4-methylpiperidine-1-carboxylate (35.92 mg, 162.42 µmol) were added sequentially to a glass vial, solvent THF (2 mL) and Ti(O-iPr)₄ (76.94 mg, 270.69 µmol) were added, and the reaction solution was stirred at 60°C for 1 h. Then NaBH₃CN (25.52 mg, 406.04 umol) was added, and the reaction solution was continued to be stirred at 60°C for 1 h. After the reaction was completed, it was concentrated, and then purified by preparation to obtain a white solid compound 187-2 (35 mg).
LC-MS: [M+H]⁺ = 581.48

Step 2: Compound 187-3 was prepared with reference to step 2 of Example 39, LC-MS: [M+H]⁺ = 481.39

Step 3: Compound 187 was prepared with reference to step 3 of Example 19.
¹H NMR (600 MHz, DMSO-d6) δ 10.96 (s, 1H), 8.63 - 8.47 (m, 1H), 7.86 (dd, *J* = 11.6, 9.1 Hz, 2H), 7.45 - 7.24 (m, 3H), 7.14 (dd, *J* = 8.8, 2.4 Hz, 1H), 7.06 (dd, *J =* 7.6, 6.1 Hz, 1H), 5.02 (ddd, *J* = 14.3, 9.6, 5.1 Hz, 1H), 4.54 (tt, *J* = 10.2, 4.2 Hz, 1H), 4.33 (d, *J* = 17.2 Hz, 1H), 4.28 - 4.17 (m, 2H), 4.17 - 4.07 (m, 2H), 3.98 (s, 1H), 3.87 (dtd, *J* = 11.2, 7.5, 4.0 Hz, 2H), 3.49 - 3.35 (m, 6H), 3.27 (t, *J* = 11.4 Hz, 3H), 3.17 (d, *J* = 4.0 Hz, 2H), 2.95 - 2.85 (m, 2H), 2.71 - 2.56 (m, 2H), 2.39 - 2.32 (m, 1H), 2.11 (dt, *J* = 14.0, 7.1 Hz, 2H), 2.04 - 1.79 (m, 5H), 1.74 (d, *J* = 12.9 Hz, 1H), 1.69 - 1.42 (m, 8H).
LC-MS: [M+H]⁺ =835.46

### Example 225

### Synthesis of compound 188

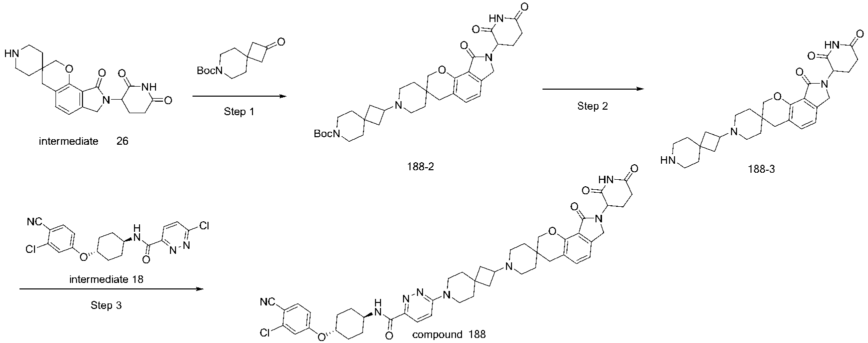

Step 1: The intermediate 26 (50.0 mg, 135.35 µmol) and tert-butyl 2-oxo-7-azaspiro[3.5]nonane-7-carboxylate (38.87 mg, 162.42 µmol) were added sequentially to a glass vial, followed by the addition of solvent THF (2 mL) and Ti(O-iPr)₄ (76.94 mg, 270.69 umol), and the reaction solution was stirred at 60°C for 1 h. Then NaBH₃CN (25.52 mg, 406.04 umol) was added, and the reaction solution was continued to be stirred at 60°C for 1 h. When the reaction was completed, the reaction solution was concentrated, and then purified by preparation to obtain a white solid compound 188-2 (35 mg).
LC-MS: [M+H]⁺ = 593.48

Step 2: (Compound 188-3) was prepared with reference to step 2 of Example 39.
LC-MS: [M+H]⁺ = 493.39

Step 3: (Compound 188) was prepared with reference to step 3 of Example 19.
¹H NMR (600 MHz, DMSO-d6) δ 10.96 (d, *J* = 1.9 Hz, 1H), 9.32 (d, *J* = 14.6 Hz, 2H), 8.58 (d, *J* = 8.2 Hz, 1H), 7.85 (dd, *J* = 22.7, 9.1 Hz, 2H), 7.42 - 7.34 (m, 2H), 7.31 (dd, *J* = 17.0, 7.8 Hz, 1H), 7.14 (dd, *J* = 8.8, 2.5 Hz, 1H), 7.06 (dd, *J* = 7.6, 5.8 Hz, 1H), 5.02 (dt, *J* = 9.9, 5.2 Hz, 1H), 4.54 (dt, *J* = 10.4, 6.0 Hz, 1H), 4.33 (d, *J* = 17.2 Hz, 1H), 4.27 - 4.17 (m, 2H), 4.00 (s, 1H), 3.70 (d, *J* = 46.0 Hz, 4H), 2.93 (d, *J* = 4.4 Hz, 4H), 2.69 (s, 1H), 2.63 - 2.52 (m, 2H), 2.40 - 2.32 (m, 2H), 2.26 (q, *J* = 10.4 Hz, 2H), 2.14 - 2.08 (m, 2H), 2.02 (q, *J* = 8.2, 7.0 Hz, 2H), 1.95 (d, *J* = 11.6 Hz, 1H), 1.93 - 1.86 (m, 2H), 1.85 - 1.74 (m, 2H), 1.73 - 1.57 (m, 7H), 1.52 (dt, *J* = 13.3, 9.8 Hz, 3H). 1.24 (s, 2H).
LC-MS: [M+H]⁺ = 847.56

### Example 226

### Synthesis of compound 189

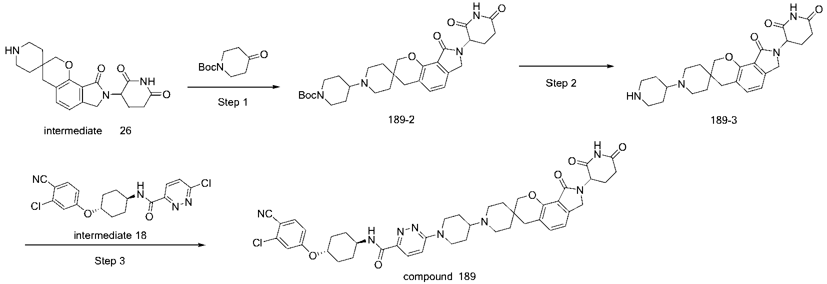

Step 1: The intermediate 26 (50.0 mg, 135.35 µmol) and tert-butyl 4-oxopiperidine-1-carboxylate (32.35 mg, 162.42 µmol) were added sequentially to a glass vial, and the solvent THF (2 mL) and Ti(O-iPr)₄ (76.94 mg, 270.69 µmol) were added, and the reaction solution was stirred at 60°C for 1 h. Then NaBH₃CN (25.52 mg, 406.04 umol) was added and the reaction solution was continued to be stirred at 60°C for 1 h. After the reaction was completed, it was concentrated and then purified by preparation to obain a white solid compound 189-2 (50 mg).
LC-MS: [M+H]⁺ = 553.38

Step 2: (Compound 189-3) was prepared with reference to step 2 of Example 39.
LC-MS: [M+H]⁺ = 453.39

Step 3: (Compound 189) was prepared with reference to step 3 of Example 19.
¹H NMR (600 MHz, DMSO-d6) δ 10.96 (s, 1H), 9.15 (s, 1H), 8.63 (dd, *J* = 8.3, 3.1 Hz, 1H), 7.88 (dd, *J* = 10.5, 9.0 Hz, 2H), 7.47 (dd, *J* = 9.7, 3.0 Hz, 1H), 7.39 (d, *J* = 2.4 Hz, 1H), 7.31 (t, *J* = 8.5 Hz, 1H), 7.14 (dd, *J* = 8.8, 2.5 Hz, 1H), 7.06 (dd, *J* = 10.6, 7.6 Hz, 1H), 5.02 (dt, *J* = 13.3, 5.2 Hz, 1H), 4.67 (d, *J* = 13.0 Hz, 2H), 4.54 (tt, *J* = 10.2, 4.3 Hz, 1H), 4.33 (d, *J* = 17.3 Hz, 1H), 4.26 (q, *J* = 11.4 Hz, 1H), 4.20 (d, *J* = 17.2 Hz, 1H), 3.96 (s, 1H), 3.92 - 3.82 (m, 2H), 3.19 (s, 2H), 3.05 (t, *J* = 12.9 Hz, 2H), 2.96 - 2.88 (m, 2H), 2.66 (s, 1H), 2.59 (dd, *J* = 17.0, 3.7 Hz, 1H), 2.43 - 2.30 (m, 2H), 2.24 - 2.16 (m, 2H), 2.15 - 2.09 (m, 2H), 2.03 - 1.93 (m, 2H), 1.93 - 1.87 (m, 2H), 1.79 (q, *J* = 12.5, 10.8 Hz, 1H), 1.67 (q, *J* = 11.8, 11.2 Hz, 6H), 1.57 - 1.42 (m, 3H).
LC-MS: [M+H]⁺ = 807.46

### Example 227

### Synthesis of compound 190

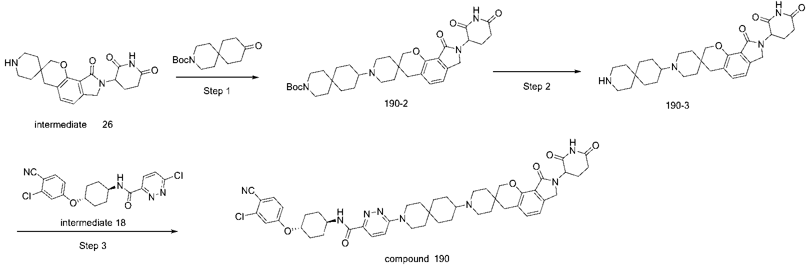

Step 1: The intermediate 26 (50.0 mg, 135.35 µmol) and tert-butyl 9-oxo-3-azaspiro[5.5]undecane-3-carboxylate (43.43 mg, 162.42 µmol) were added sequentially to a glass vial, followed by the addition of solvent THF (2 mL) and Ti(O-iPr)₄ (76.94 mg, 270.69 umol), and the reaction solution was stirred at 60°C for 1 h. Then NaBH₃CN (25.52 mg, 406.04 umol) was added, and the reaction solution was continued to be stirred at 60°C for 1 h. After the reaction was completed, the reaction solution was concentrated, and then purified by preparation to obtain a white solid compound 190-2 (40 mg).
LC-MS: [M+H]⁺ = 621.58

Step 2: (Compound 190-3) was prepared with reference to step 3 of Example 39.
LC-MS: [M+H]⁺ = 521.49

Step 3: (Compound 190) was prepared with reference to step 3 of Example 39.
¹H NMR (600 MHz, DMSO-d6) δ 10.96 (s, 1H), 8.95 (s, 1H), 8.57 (d, *J* = 8.2 Hz, 1H), 7.84 (dd, *J* = 25.5, 9.1 Hz, 2H), 7.42 - 7.25 (m, 3H), 7.14 (dd, *J* = 8.8, 2.5 Hz, 1H), 7.06 (t, *J* = 8.3 Hz, 1H), 5.33 (t, *J* = 5.0 Hz, 1H), 5.03 (dt, *J* = 13.3, 5.0 Hz, 1H), 4.54 (dt, *J* = 10.6, 5.9 Hz, 1H), 4.33 (d, *J* = 17.3 Hz, 1H), 4.26 (q, *J* = 11.6 Hz, 1H), 4.20 (d, *J* = 17.2 Hz, 1H), 3.97 (s, 1H), 3.88 - 3.83 (m, 2H), 3.72 (s, 5H), 3.20 (d, *J* = 25.1 Hz, 4H), 2.96 - 2.88 (m, 2H), 2.66 (s, 1H), 2.62 - 2.57 (m, 1H), 2.41 - 2.31 (m, 2H), 2.15 - 2.07 (m, 2H), 2.00 (dt, *J* = 19.1, 7.1 Hz, 2H), 1.92 (t, *J* = *15.3* Hz, 5H), 1.80 (t, *J* = 13.6 Hz, 1H), 1.74 - 1.62 (m, 5H), 1.59 (s, 2H), 1.56 - 1.48 (m, 2H), 1.46 (t, *J* = 7.0 Hz, 1H), 1.41 (d, *J* = 6.1 Hz, 2H).
LC-MS: [M+H]⁺ = 875.56

### Example 228

### Synthesis of compound 191

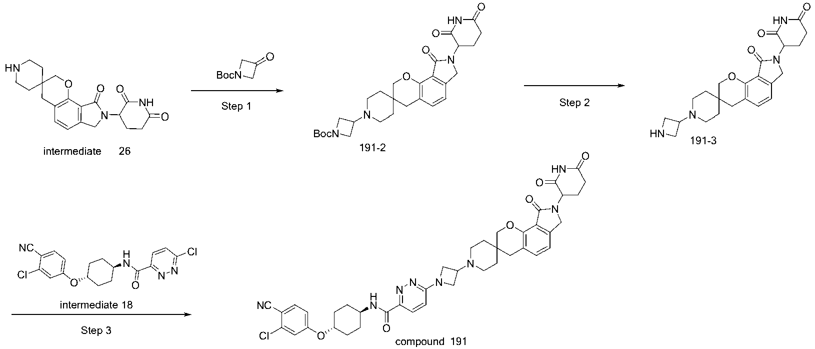

Step 1: The intermediate 26 (50.0 mg, 135.35 µmol) and tert-butyl 3-oxoazetidine-1-carboxylate (27.81 mg, 162.42 µmol) were added sequentially to a glass vial, solvent THF (2 mL) and Ti(O-iPr)₄(76.94 mg, 270.69 µmol) were added, and the reaction solution was stirred at 60°C for 1 h. Then NaBH₃CN (25.52 mg, 406.04 umol) was added and the reaction solution was continued to be stirred at 60°C for 1 h. After the reaction was completed, it was concentrated, and then purified by preparation to obtain a white solid compound 191-2 (40 mg).
LC-MS: [M+H]⁺ = 525.59

Step 2: (Compound 191-3) was prepared with reference to step 2 of Example 39.
LC-MS: [M+H]⁺ = 425.39

Step 3: (Compound 191) was prepared with reference to step 3 of Example 39.
¹H NMR (600 MHz, Methanol-d4) δ 8.04 (d, *J* = 9.3 Hz, 1H), 7.71 (d, *J* = 8.7 Hz, 1H), 7.38 (d, *J* = 7.7 Hz, 1H), 7.22 (d, *J* = 2.4 Hz, 1H), 7.10 (d, *J* = 7.7 Hz, 1H), 7.06 (dd, *J* = 8.8, 2.4 Hz, 1H), 7.04 (d, *J* = 9.3 Hz, 1H), 5.11 (dd, *J* = 13.3, 5.2 Hz, 1H), 4.61 (dd, *J* = 10.2, 7.5 Hz, 2H), 4.55 - 4.50 (m, 1H), 4.47 - 4.43 (m, 2H), 4.39 (d, *J* = 17.1 Hz, 2H), 4.17 (s, 3H), 4.03 - 3.98 (m, 1H), 3.79 - 3.61 (m, 2H), 3.37 (s, 2H), 2.92 (ddd, *J* = 18.4, 13.6, 5.3 Hz, 2H), 2.80 (ddd, *J* = 17.6, 4.7, 2.5 Hz, 1H), 2.50 (qd, *J* = 13.2, 4.6 Hz, 1H), 2.22 (dd, *J* = 13.2, 5.9 Hz, 2H), 2.17 (ddd, *J* = 10.4, 5.2, 2.7 Hz, 1H), 2.12 (d, *J* = 8.8 Hz, 2H), 2.05 (s, 1H), 1.89 (d, *J* = 26.5 Hz, 4H), 1.70 - 1.60 (m, 4H).
LC-MS: [M+H]⁺ = 779.46

### Example 229

### Synthesis of compound 192

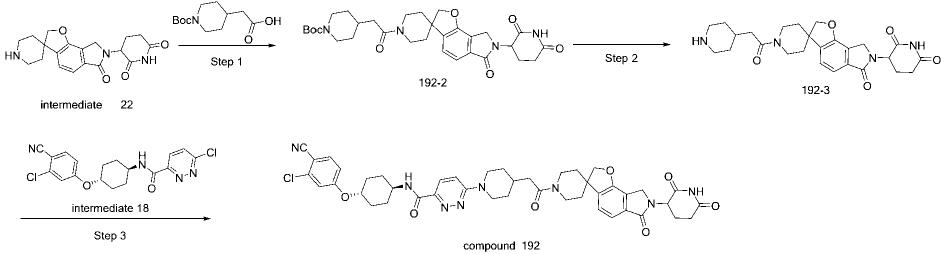

Step 1: The intermediate 22 (30 mg, 84.41 umol) and 2-(1-(tert-butoxycarbonyl)piperidin-4-yl)acetic acid (24.65 mg, 101.30 umol) were added sequentially to a glass vial, and the solvent DMF (2 mL) and BOP (74.69 mg, 168.82 umol), DIEA (54.55 mg, 422.07 umol) were added, and the reaction solution was stirred at room temperature for 2 h. After the reaction was completed, it was concentrated, and then purified by a normal-phase column to obtain a white solid (192-2) (30 mg)
LC-MS: [M+H]⁺ = 581.19

Step 2: (Compound 192-3) was prepared with reference to step 2 of Example 39.
LC-MS: [M+H]⁺ = 481.29

Step 3: (Compound 192) was prepared with reference to step 3 of Example 19.
¹H NMR (600 MHz, DMSO-d6) δ 10.98 (s, 1H), 8.60 (d, *J* = 8.2 Hz, 1H), 7.86 (d, *J* = 8.8 Hz, 1H), 7.82 (dd, *J* = 9.8, 3.0 Hz, 1H), 7.41 (d, *J* = 7.5 Hz, 1H), 7.40 (d, *J* = 2.4 Hz, 1H), 7.37 (t, *J* = 7.5 Hz, 1H), 7.28 (d, *J* = 7.6 Hz, 1H), 7.14 (dd, *J* = 8.8, 2.4 Hz, 1H), 5.10 (dt, *J* = 13.4, 4.5 Hz, 1H), 4.69 - 4.59 (m, 2H), 4.54 (tt, *J* = 9.9, 4.2 Hz, 3H), 4.45 - 4.35 (m, 2H), 4.23 (dd, *J* = 17.1, 6.9 Hz, 1H), 3.94 (d, *J* = 13.4 Hz, 1H), 3.86 (dtt, *J* = 11.6, 8.1, 4.0 Hz, 1H), 3.17 (t, *J* = 12.7 Hz, 1H), 3.06 (t, *J* = 12.7 Hz, 2H), 2.92 (ddd, *J* = 18.1, 13.4, 5.5 Hz, 1H), 2.74 (t, *J* = 11.9 Hz, 1H), 2.63 - 2.56 (m, 1H), 2.53 (m, 3H), 2.47 - 2.29 (m, 3H), 2.14 - 2.07 (m, 2H), 2.03 - 1.95 (m, 1H), 1.94 - 1.78 (m, 4H), 1.74 (t, *J* = 13.2 Hz, 2H), 1.64 (qd, *J* = 13.2, 3.1 Hz, 2H), 1.57 - 1.45 (m, 2H), 1.21 (dd, *J* = 24.6, 12.0 Hz, 2H).
LC-MS: [M+H]⁺ = 835.46

### Example 230

### Synthesis of compound 193

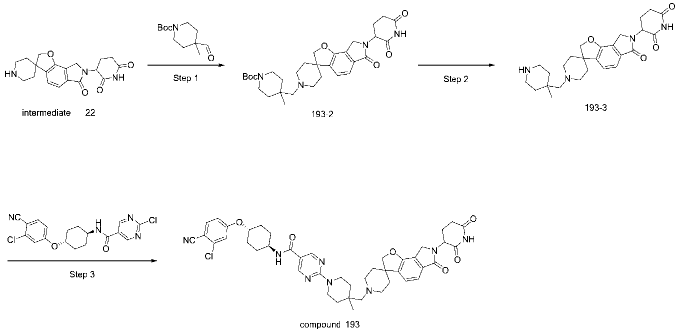

Step 1: The intermediate 22 (50.0 mg, 140.69 µmol) and tert-butyl 4-formyl-4-methylpiperidine-1-carboxylate (38.37 mg, 168.83 µmol) were added sequentially to a glass vial, solvent THF (2 mL) and Ti(O-iPr)₄(79.97 mg, 281.38 µmol) were added, and the reaction solution was continued to be stirred at 60°C for 1 h. Then NaBH₃CN (26.52 mg, 422.07 umol) was added, and the reaction solution was continued to be stirred at 60°C for 1 h. After the reaction was completed, it was concentrated, and then purified by preparation to obtain a white solid compound 193-2 (30 mg).
LC-MS: [M+H]⁺ = 567.38

Step 2: (Compound 193-3) was prepared with reference to step 2 of Example 39.
LC-MS: [M+H]⁺ = 467.39

Step 3: (Compound 193) was prepared with reference to step 3 of Example 19.
¹H NMR (600 MHz, DMSO-d6) δ 10.99 (s, 1H), 8.79 (s, 2H), 8.15 (d, *J* = 7.5 Hz, 1H), 7.87 (d, *J* = 8.7 Hz, 1H), 7.43 - 7.33 (m, 2H), 7.28 (d, *J* = 7.6 Hz, 1H ), 7.15 (dd, *J* = 8.8, 2.4 Hz, 1H), 5.33 (t, *J* = 5.1 Hz, 1H), 5.10 (dt, *J* = 13.4, 4.8 Hz, 1H), 4.72 - 4.62 (m, 2H), 4.56 (dt, *J* = 10.2, 5.6 Hz, 1H), 4.45 - 4.30 (m, 3H), 4.24 (d, *J* = 17.2 Hz, 1H), 3.81 (s, 2H), 3.57 (s, 2H), 3.26 (d, *J* = 12.1 Hz, 3H), 3.15 (d, *J* = 3.8 Hz, 2H), 2.92 (ddd, *J* = 17.9, 13.7, 5.4 Hz, 2H), 2.57 (d, *J* = 25.6 Hz, 2H), 2.46 - 2.33 (m, 3H), 2.29 (s, 1H), 2.16 - 2.06 (m, 2H), 2.04 - 1.96 (m, 2H), 1.92 (d, *J* = 12.8 Hz, 3H), 1.67 - 1.41 (m, 7H).
LC-MS: [M+H]⁺ = 821.46

### Example 231

### Synthesis of compound 194

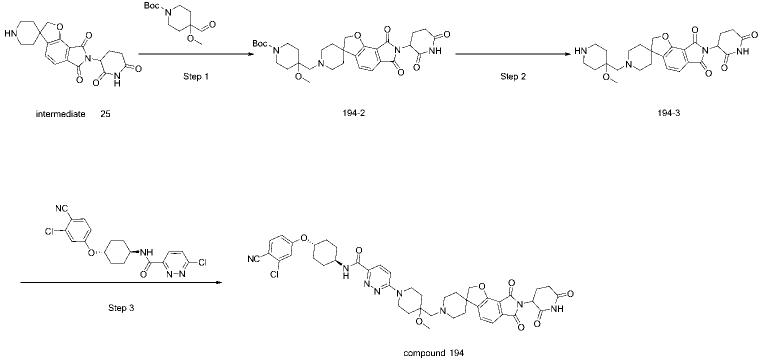

Step 1: The intermediate 25 (100.0 mg, 270.72 µmol) and tert-butyl 4-formyl-4-methoxypiperidine-1-carboxylate (123.1 mg, 541.45 µmol) were added sequentially to a glass vial, and the solvents THF:DMSO = 1 : 2 (4 mL) and Ti(O-iPr)₄(153.9 mg, 541.45 µmol) were added, and the reaction solution was stirred at 60°C for 1 h. Then NaBH₃CN (34.0 mg, 541.45 µmol) was added, and the reaction solution was continued to be stirred at 60°C for 1 h. When the reaction was completed, the reaction solution was concentrated, and then purified by preparation to obtain a white solid compound 194-2 (40 mg).
LC-MS: [M+H]⁺ =596.68

Step 2: (Compound 194-3) was prepared with reference to step 2 of Example 39.
LC-MS: [M+H]⁺ =496.56

Step 3: (Compound 194) was prepared with reference to step 3 of Example 19.
¹H NMR (600 MHz, DMSO-d6) δ 11.12 (s, 1H), 8.63 (d, *J* = 8.2 Hz, 1H), 7.87 (dd, *J* = 9.1, 3.1 Hz, 2H), 7.56 - 7.50 (m, 2H), 7.44 (d, *J* = 9.6 Hz, 1H), 7.39 (d, *J* = 2.4 Hz, 1H), 7.14 (dd, *J* = 8.8, 2.4 Hz, 1H), 5.11 (dd, *J* = 12.9, 5.5 Hz, 1H), 4.81 (d, *J* = 17.6 Hz, 2H), 4.55 (tq, *J* = 10.3, 4.9, 4.2 Hz, 1H), 4.27 (d, *J* = 13.0 Hz, 2H), 3.87 (dtd, *J* = 11.4, 7.7, 4.2 Hz, 2H), 3.60-3.65 (m, 2H),3.37 (d, *J* = 11.3 Hz, 3H), 3.33 (d, *J* = 5.0 Hz, 1H), 3.30 (s, 4H), 2.89 (ddd, *J* = 17.1, 13.9, 5.5 Hz, 1H), 2.64 - 2.52 (m, 2H), 2.35 (t, *J* = 13.4 Hz, 2H), 2.15 - 2.09 (m, 2H), 2.00 (q, *J* = 14.5, 13.0 Hz, 5H), 1.93 - 1.87 (m, 2H), 1.71 - 1.60 (m, 4H), 1.52 (dt, *J* = 12.7, 9.3 Hz, 2H).
LC-MS: [M+H]+=851.36

### Example 232

### Synthesis of compound 195

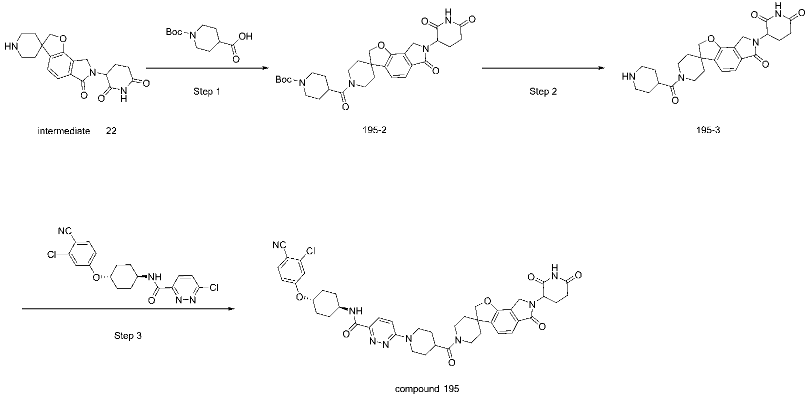

Step 1: The intermediate 22 (100.0 mg, 270.72 µmol) and 1-(tert-butoxycarbonyl)piperidine-4-carboxylic acid (123.1 mg, 541.45 µmol) were added sequentially to a glass vial, and solvent DMF (2 mL), BOP (237.07 mg, 561.79 µmol), DIEA (72.42 mg, 561.79 µmol) were added, and the reaction solution was stirred at room temperature for 2 h. After the reaction was completed, it was concentrated, and then purified by preparation to obtain a white solid compound 195-2 (60 mg).
LC-MS: [M+H]⁺ =566.66

Step 2: (Compound 195-3) was prepared with reference to step 2 of Example 39.
LC-MS: [M+H]⁺ =466.54

Step 3: Compound 195 was prepared with reference to step 3 of Example 19.
¹H NMR (600 MHz, DMSO-d6) δ 10.99 (s, 1H), 8.62 (d, *J* = 8.2 Hz, 1H), 7.85 (dd, *J* = 12.8, 9.1 Hz, 2H), 7.47 - 7.37 (m, 3H), 7.29 (d, *J* = 7.5 Hz, 1H), 7.14 (dd, *J* = 8.8, 2.4 Hz, 1H), 5.10 (dt, *J* = 13.3, 5.1 Hz, 1H), 4.66 (dt, *J* = 31.8, 9.6 Hz, 2H), 4.58 - 4.46 (m, 3H), 4.40 (dd, *J* = 16.9, 4.0 Hz, 2H), 4.24 (dd, *J* = 17.0, 8.4 Hz, 1H), 4.13 - 4.06 (m, 1H), 3.87 (dtd, *J* = 11.3, 7.6, 4.0 Hz, 1H), 3.25 (t, *J* = 13.0 Hz, 1H), 3.13 (dt, *J* = 30.8, 12.2 Hz, 3H), 2.92 (ddd, *J* = 18.1, 13.6, 5.4 Hz, 1H), 2.74 (t, *J* = 12.2 Hz, 1H), 2.64 - 2.56 (m, 1H), 2.48 - 2.38 (m, 2H), 2.11 (dt, *J* = 15.8, 7.8 Hz, 2H), 1.99 (tt, *J* = 7.9, 2.4 Hz, 1H), 1.90 (ddd, *J* = 23.6, 14.3, 9.2 Hz, 3H), 1.84 - 1.72 (m, 4H), 1.69 - 1.47 (m, 6H).
LC-MS: [M+H]⁺ =851.36

### Example 233

### Synthesis of compound 196

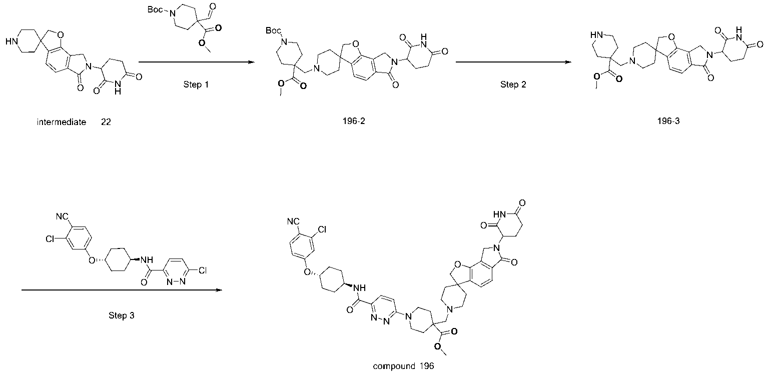

Step 1: The intermediate 22 (100.0 mg, 281.38 µmol) and 1-(tert-butyl)4-methyl 4-formyl piperidine-1,4-dicarboxylate (114.51 mg, 422.07 µmol) were added sequentially to a glass vial, solvents THF:DMSO = 1 : 2 (4 mL) and Ti(O-iPr)₄(153.9 mg, 541.45 µmol) were added, and the reaction solution was stirred at 60°C for 1 h. Then NaBH₃CN (34.0 mg, 541.45 µmol) was added, and the reaction solution was continued to be stirred at 60°C for 1 h. After the reaction was completed, it was concentrated, and then purified by preparation to obtain a white solid compound 196-2 (30 mg).
LC-MS: [M+H]⁺ =610.71

Step 2: (Compound 196-3) was prepared with reference to step 2 of Example 39.
LC-MS: [M+H]⁺ =510.59

Step 3: Compound 196 was prepared with reference to step 3 of Example 19.
¹H NMR (600 MHz, DMSO-d6) δ 10.99 (s, 1H), 8.63 (d, *J* = 8.2 Hz, 1H), 7.87 (t, *J* = 8.8 Hz, 2H), 7.45 - 7.33 (m, 3H), 7.27 (d, *J* = 7.6 Hz, 1H), 7.14 (dd, *J* = 8.8, 2.4 Hz, 1H), 5.10 (dd, *J* = 13.3, 5.1 Hz, 1H), 4.64 (d, *J* = 14.5 Hz, 2H), 4.54 (tt, *J* = 9.8, 4.3 Hz, 1H), 4.40 (d, *J* = 17.1 Hz, 1H), 4.24 (d, *J* = 17.1 Hz, 1H), 4.15 (d *J* = 13.1 Hz, 2H), 3.87 (dtd, *J* = 11.6, 7.6, 4.1 Hz, 2H), 3.80 (s, 3H), 3.17 (s, 3H), 2.92 (ddd, *J* = 18.1, 13.7, 5.5 Hz, 2H), 2.60 (d, *J* = 18.4 Hz, 1H), 2.44 (dt, *J* = 13.2 , 6.7 Hz, 2H), 2.26 (s, 2H), 2.19 - 2.07 (m, 4H), 2.02 - 1.86 (m, 5H), 1.76 (s, 2H), 1.65 (qd, *J* = 13.0, 3.2 Hz, 2H), 1.58 - 1.48 (m, 2H), 1.39 (d, *J* = 4.5 Hz, 1H), 1.33 - 1.21 (m, 1H).
LC-MS: [M+H]⁺ =865.39

### Example 236

### Synthesis of compound 199

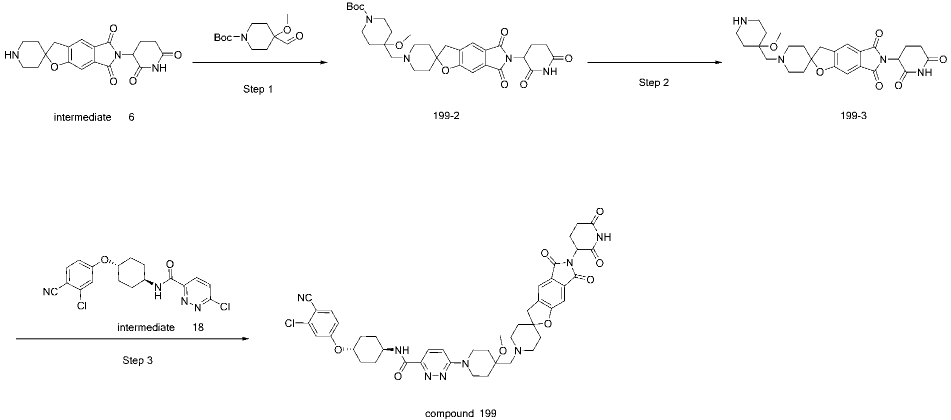

Step 1: The intermediate 6 (23.00 mg, 0.06 mmol) and tert-butyl 4-formyl-4-methoxypiperidine-1-carboxylate (30.30 mg, 0.12 mmol) were added sequentially to a glass vial, the solvent THF (2 mL) and tetraisopropyl titanate (35.39 mg, 0.12 mmol) were added, and the reaction solution was stirred at 60°C for 1 h. Then NaBH₃CN (11.74 mg, 0.19 mmol) was added and the reaction solution was continued to be stirred at 60°C for 1 h. After the reaction was completed, the reaction solution was concentrated and then purified by preparative thin-layer chromatography (DCM:MeOH=10:1) to obtain a white solid compound 199-2 (18 mg, crude).
LC-MS: [M+H]⁺ =597.48

Step 2: Compound 3-1 (18.00 mg, 14.97 mmol) and dioxane hydrochloride solution were added sequentially in a glass vial, and the reaction solution was stirred at room temperature for 2 h. The reaction solution was concentrated to obtain a white solid compound 199-3 (25 mg, crude).
LC-MS: [M+H]⁺ =497.45

Step 3: Compound 3-2 (25.00 mg, 0.05 mmol), the intermediate 18 (39.33 mg, 0.10 mmol) and N,N-diisopropylethylamine (32.44 mg, 0.25 mmol) were added sequentially to a glass vial, the solvent DMSO (1.0 mL) was added, and the reaction solution was stirred at 80°C for 2 h. Then a gray solid compound 199 (2 mg) was obtained by purification.

Preparation with reference to step 3 of Example 19
¹H NMR (600 MHz, DMSO) δ 11.11 (s, 1H), 8.63 (d, *J* = 8.2 Hz, 1H), 7.86 (d, *J* = 9.0 Hz, 2H), 7.24 (s, 2H), 7.15 (d, *J* = 5.1 Hz, 2H), 7.07 (s, 2H), 4.53 (ddd, *J* = 14.3. 10.2, 4.1 Hz, 1H), 4.24 (d, *J* = 11.7 Hz, 2H), 3.29 (s, 9H), 2.93 - 2.85 (m, 1H), 2.64 - 2.51 (m, 2H), 2.31 - 1.86 (m, 14H) , 1.58 (dq, *J* = 23.0, 10.0 Hz, 8H).
LC-MS: [M+H]⁺ =851.46

### Example 237

### Synthesis of compound 200

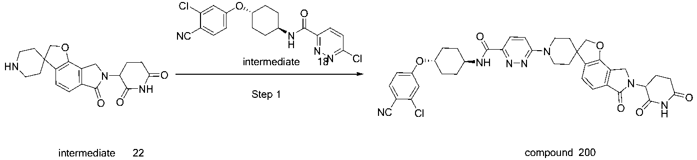

Step 1: The intermediate 3(36.33 mg, 0.09 mmol), the intermediate XX (30.00 mg, 0.84 mmol) and DIEA (54.55 mg, 0.42 mmol) were added sequentially to a glass vial, the solvent DMSO (1 mL) was added, and the reaction solution was stirred at 80°C for 2 h. When the reaction was completed, the reaction solution was concentrated, and then purified by preparative thin chromatography (DCM:MeOH=10:1) to obtain a white solid compound 200 (12 mg).
¹H NMR (600 MHz, DMSO) δ 10.98 (s, 1H), 8.64 (d, *J* = 8.2 Hz, 1H), 7.86 (d, *J* = 9.0 Hz, 2H), 7.48 - 7.37 (m, 3H), 7.26 (d, *J* = 7.6 Hz, 1H), 7.14 (dd, *J* = 8.8, 2.3 Hz, 1H), 5.09 (dd, *J* = 13.3, 5.1 Hz, 1H), 4.73 (q, *J* = 9.3 Hz, 2H), 4.54 - 4.50 (m, 2H), 4.40 (d, *J* = 17.1 Hz, 1H), 4.24 (d, *J* = 17.1 Hz, 1H), 3.20 (t, *J* = 12.5 Hz, 2H), 2.95 - 2.86 (m, 1H), 2.59 (d, *J* = 17.4 Hz, 1H), 2.43 (dd, *J* = 13.2, 4.6 Hz, 1H), 2.11 (d, *J* = 9.7 Hz, 2H), 2.03 - 1.80 ( m, 8H), 1.70 - 1.60 (m, 2H), 1.53 (dd, *J* = 12.8, 8.5 Hz, 3H).
LC-MS: [M+H]⁺ = 710.37

### Example 238

### Synthesis of compound 201

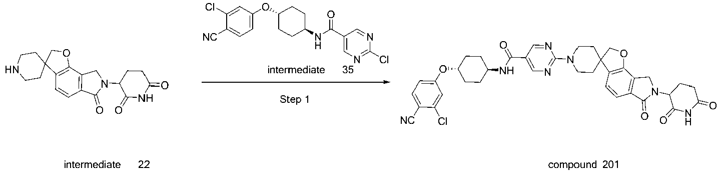

Step 1: The intermediate 22 (36.33 mg, 0.93 mmol), the intermediate 35 (30.00 mg, 0.84 mmol) and DIEA (54.55 mg, 0.42 mmol) were added sequentially to a glass vial, the solvent DMSO (1 mL) was added, and the reaction solution was stirred at 80°C for 2 h. When the reaction was completed, the reaction solution was concentrated, and then purified by preparative thin chromatography (DCM:MeOH=10:1) to obtain a white solid compound 201.
¹H NMR (600 MHz, DMSO) δ 10.99 (s, 1H), 8.80 (s, 2H), 8.17 (d, *J* = 7.4 Hz, 1H), 7.87 (d, *J* = 8.8 Hz, 1H), 7.41 (dd, *J* = 8.6, 4.9 Hz, 2H), 7.26 (d, *J* = 7.6 Hz, 1H), 7.18 - 7.12 (m, 1H), 5.09 (dd, *J* = 13.3, 5.0 Hz, 1H), 4.78 - 4.69 (m, 4H), 4.41 (d, *J* = 17.1 Hz, 1H), 4.24 (d, *J* = 17.0 Hz, 1H), 3.18 (t, *J* = 12.2 Hz, 2H), 2.95 - 2.88 (m, 1H), 2.60 (d, *J* = 18.0 Hz, 1H), 2.43 (dd, *J* = 13.1, 4.4 Hz, 1H), 2.04 - 1.75 (m, 9H), 1.58 - 1.46 ( m, 6H).
LC-MS: [M+H]⁺ = 710.27

### Example 239

### Synthesis of compound 202

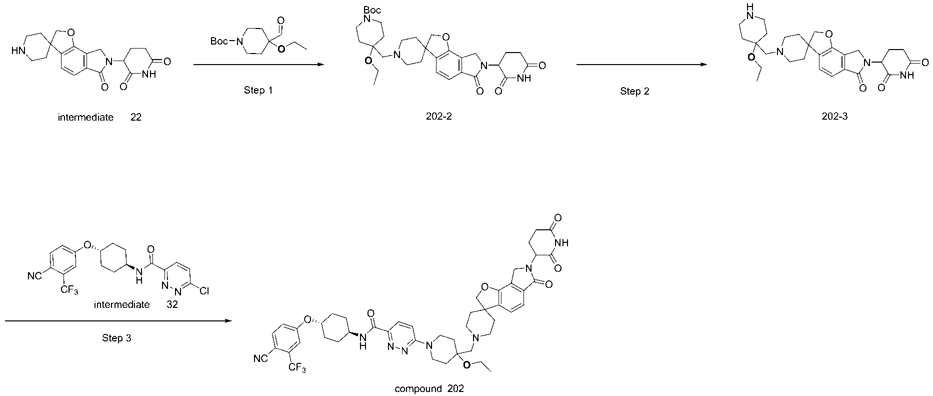

Step 1: The intermediate 22 (50.00 mg, 0.14 mmol) and tert-butyl 4-formyl-4-ethoxypiperidine-1-carboxylate (72.41 mg, 0.28 mmol) were added sequentially to a glass vial, solvents THF (2 mL) and tetraisopropyl titanate (79.97 mg, 0.28 mmol) were added, and the reaction solution was stirred at 60°C for 1 h. Then NaBH₃CN (26.52 mg, 0.42 mmol) was added and the reaction solution was continued to be stirred at 60°C for 1 h. After the reaction was completed, the reaction solution was concentrated and then purified by preparative thin-layer chromatography (DCM:MeOH=10:1) to obtain a white solid compound 202-2 (18 mg, crude).
LC-MS: [M+H]⁺ =597.58

Step 2: Compound 7-1(18.00 mg, 140.69 µmol) and dioxane hydrochloride solution were added sequentially in a glass vial, and the reaction solution was stirred at room temperature for 2 h. The reaction solution was concentrated to obtain a white solid compound 202-3 (28 mg, crude).
LC-MS: [M+H]⁺ =497.50

Step 3: Compound 7-2 (28.00 mg, 0.06 mmol), the intermediate 32 (47.88 mg, 0.12 mmol) and N,N-diisopropylethylamine (36.33 mg, 0.30 mmol) were added sequentially to a glass vial, the solvent DMSO (1.0 mL) was added, and the reaction solution was stirred at 80°C for 2 h. Then a gray solid compound 202 (3.2 mg) was obtained by purification.
¹H NMR (600 MHz, DMSO) δ 10.99 (s, 1H), 8.62 (d, *J* = 8.2 Hz, 1H), 8.07 (d, *J* = 8.5 Hz, 1H), 7.87 (d, *J* = 9.5 Hz, 1H), 7.49 (dd, *J* = 11.3, 2.5 Hz, 2H), 7.43 (d, *J* = 9.6 Hz, 1H), 7.36 (d, *J* = 7.6 Hz, 1H), 7.27 (d, *J* = 7.6 Hz, 1H), 5.09 (dd, *J* = 13.3, 5.0 Hz, 1H), 4.70 - 4.59 (m, 3H), 4.39 (d, *J* = 17.1 Hz, 1H), 4.25 (t, *J* = 12.7 Hz, 3H), 3.63 (d, *J* = 12.5 Hz, 2H), 3.53 (dd, *J* = 13.6, 6.7 Hz, 2H), 3.39 - 3.19 (m, 6H), 2.95 - 2.87 (m, 1H), 2.61 (s, 1H), 2.35 (s, 4H), 2.13 (d, *J* = 10.7 Hz, 2H), 2.03 - 1.88 (m, 7H), 1.70 - 1.50 (m, 6H), 1.19 (t, *J* = 6.8 Hz, 3H).
LC-MS: [M+H]⁺ =885.46

### Example 242

### Synthesis of compound 205

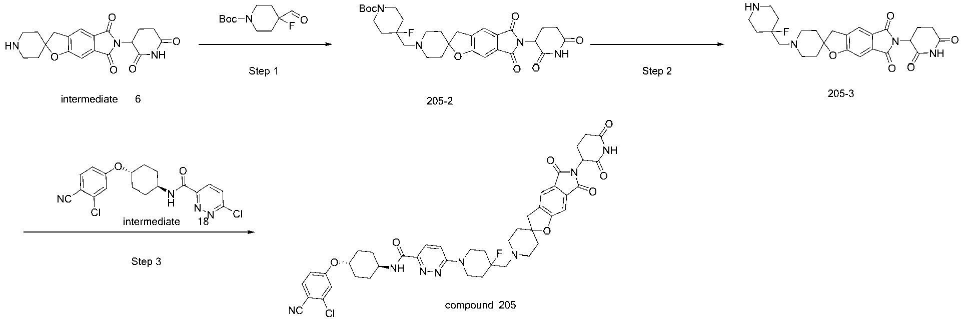

Step 1: The intermediate 2 (50.00 mg, 0.14 mmol) and tert-butyl 4-fluoro-4-formylpiperidine-1-carboxylate (136.92 mg, 0.56 mmol) were sequentially added to a glass vial, and the solvents DCE:DMSO = 5 : 1 (1.2 mL) and acetic acid (1 drop) were added, and the reaction solution was stirred at room temperature for 1 h. Then NaBH(OAc)₃(89.45 mg, 0.42 mmol) was added and the reaction solution was continued to be stirred at room temperature for 1 h. After the reaction was completed, it was concentrated, and then purified by preparative thin-layer chromatography (DCM:MeOH=10:1) to obtain a white solid compound 205-2 (70 mg, crude).
LC-MS: [M+H]⁺ =585.46

Step 2: Compound 17-1 (50.00 mg, 0.09 mmol) and dioxane hydrochloride solution were added sequentially in a glass vial, and the reaction solution was stirred at room temperature for 2 h. The reaction solution was concentrated to obtain a white solid compound 205-3 (78 mg, crude).
LC-MS: [M+H]⁺ =485.29

Step 3: Compound 17-2 (20.00 mg, 0.04 mmol), intermediate 18 (19.38 mg, 0.05 mmol) and N,N-diisopropylethylamine (77.32 mg, 0.60 mmol) were added sequentially to a glass vial, the solvent DMSO (1.0 mL) was added, and the reaction solution was stirred at 80°C for 2 h. Then a gray solid compound 205 (9 mg) was obtained by purification.
LC-MS: [M+H]⁺ =839.46
¹H NMR (600 MHz, Methanol-d4) δ 7.99 (d, *J* = 9.6 Hz, 1H), 7.77 (s, 1H), 7.71 (d, *J* = 8.7 Hz, 1H), 7.44 (d, *J* = 9.7 Hz, 1H), 7.27 (s, 1H), 7.22 (d, *J* = 2.4 Hz, 1H), 7.06 (dd, *J* = 8.8, 2.4 Hz, 1H), 5.12 (dd, *J* = 12.8, 5.4 Hz, 1H), 4.53 (d, *J* = 12.5 Hz, 3H), 4.04 - 3.97 (m, 1H), 3.82 - 3.58 (m, 8H), 3.52 - 3.43 (m, 2H), 3.36 - 3.34 (m, 2H), 2.92 - 2.84 (m, 2H), 2.80 - 2.77 (m, 1H), 2.76 - 2.68 ( m, 2H), 2.36 - 2.28 (m, 2H), 2.26 - 2.19 (m, 4H), 2.18 - 2.09 (m, 4H), 2.04 - 1.98 (m, 1H), 1.98 - 1.90 (m, 2H).

### Example 243

### Synthesis of compound 206

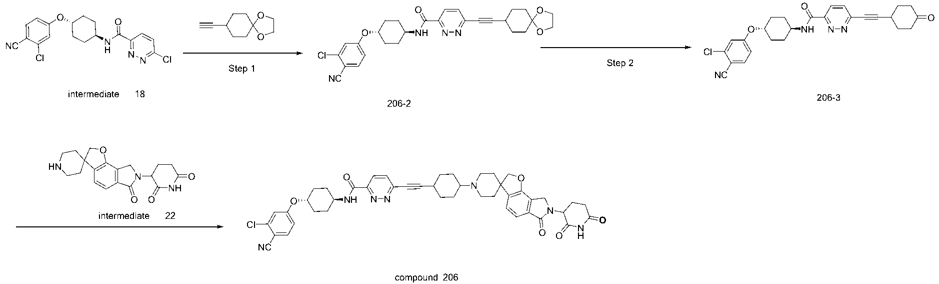

Step 1: The intermediate 18 (50.00 mg, 0.13 mmol) and 8-ethynyl-1,4-dioxaspiro[4.5]decane (25.49 mg, 0.15 mmol) were added sequentially to a glass vial. Solvent DMSO (2 mL), copper iodide (7.3 mg, 0.04 mmol), bis(triphenylphosphine)palladium chloride (8.97 mg, 0.02 mmol), and cesium carbonate (12.91 mg, 0.38 mmol) were added. The reaction solution was heated to 110 °C and stirred for 2 hours. The reaction solution was purified by preparative chromatography to obtain a white solid compound 206-2(40 mg, crude).
LC-MS: [M+H]⁺ =521.29

Step 2: Compound 20-1 (40.00 mg, 0.14 mmol) and dioxane hydrochloride solution were added sequentially in a glass vial and the reaction solution was stirred at room temperature for 2 h. The reaction solution was concentrated to obtain a white solid compound 206-3 (57 mg, crude).
LC-MS: [M+H]⁺ =477.35

Step 3: Compound 20-2 (16.00 mg, 0.03 mmol) and the intermediate 3 (17.88 mg, 0.05 mmol) were added sequentially into a glass vial, the solvents DCE:DMSO = 5 : 1 (1.2 mL) and acetic acid (1 drop) were added, and the reaction solution was stirred at room temperature for 1 h. Then NaBH(OAc)₃(6.32 mg, 0.10 mmol) was added, and the reaction solution was continued to be stirred at room temperature for 1 h. After the reaction was completed, a white solid compound 206 (5 mg) was obtained by preparative purification.
LC-MS: [M+H]⁺ =816.56
¹H NMR (600 MHz, Methanol-d4) δ 8.26 (d, *J* = 8.6 Hz, 1H), 7.90 (d, *J* = 8.6 Hz, 1H), 7.72 (d, *J* = 8.8 Hz, 1H), 7.46 (d, *J* = 7.7 Hz, 1H), 7.38 (d, *J* = 7.7 Hz, 1H), 7.23 (d, *J* = 2.4 Hz, 1H), 7.07 (dd, *J* = 8.8, 2.4 Hz, 1H), 5.17 (dd, *J* = 13.4, 5.2 Hz, 1H), 4.57 - 4.52 (m, 1H), 4.51 - 4.40 (m, 2H), 4.09 - 4.00 (m, 1H), 3.67 (d, *J* = 12.6 Hz, 1H), 3.42 - 3.37 (m, 1H), 3.23 (t, 2H), 2.96 - 2.89 (m, 1H), 2.83 - 2.78 (m, 1H), 2.77 - 2.74 (m, 1H), 2.56- 2.50 (m, 1H), 2.44-2.37 (m, 2H), 2.33 - 2.27 (m, 3H), 2.07 - 2.03 (m, 1H).

### Example 244

### Synthesis of intermediate 32

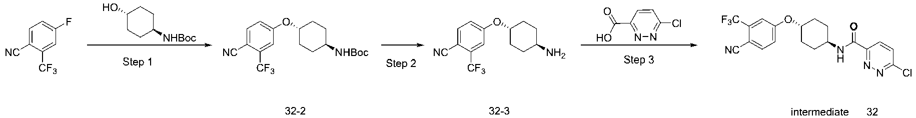

Step 1: In a glass vial, tert-butyl (1r,4r)-4-hydroxycyclohexylcarbamate (200.00 mg, 0.93 mmol) and sodium hydride (111.48 mg, 4.65 mmol) were added sequentially, and the solvent THF (5 mL) was added, and the reaction solution was stirred at 0°C for 0.5 h. Then 4-fluoro-2 - (trifluoromethyl) benzyl nitrile (209.91 mg, 1.11 mmol) was added, and the reaction solution was brought to room temperature and stirred for 2 h. A white solid compound 32-2 (180 mg, crude) was obtained by preparative purification.
LC-MS: [M+H]⁺ =384.40

Step 2: Compound 21-1 (180.00 mg, 0.47 mmol) and dioxane hydrochloride solution were added sequentially in a glass vial, and the reaction solution was stirred at room temperature for 2 h. The reaction solution was concentrated to obtain a white solid compound 32-3 (223 mg, crude).
LC-MS: [M+H]⁺ =285.19

Step 3: Compound 21-2 (223 mg, 0.78 mmol), 6-chloropyridazine-3-carboxylic acid (124.05 mg, 0.78 mmol), BOP (691.29 mg, 1.56 mmol) and DIEA (302.63 mg, 2.35 mmol) were added sequentially to a glass vial, the solvent DMF (5 mL) was added, and the reaction solution was stirred at room temperature for 2 h. After the reaction was completed, a white solid compound Intermediate 32 (165 mg) was obtained by preparative purification.
LC-MS: [M+H]⁺ =425.46
¹H NMR (600 MHz, DMSO-*d*₆) δ 9.14 (d, *J* = 8.3 Hz, 1H), 8.22 (dd, *J* = 8.9, 1.5 Hz, 1H), 8.12 - 8.04 (m, 2H), 7.52 - 7.45 (m, 2H), 4.67 - 4.58 (m, 1H), 3.96 - 3.87 (m, 1H), 2.18 - 2.10 (m, 2H), 1.95 - 1.87 (m, 2H), 1.76 - 1.66 (m, 2H), 1.59 - 1.49 (m, 2H).

### Example 245

### Synthesis of compound 414

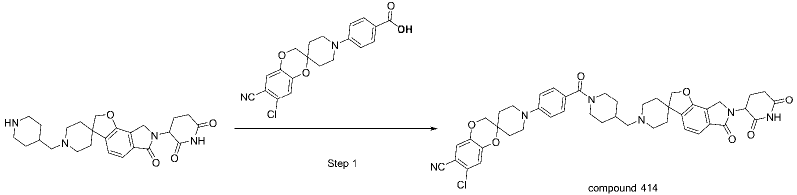

Step 3: (Compound 414) was prepared with reference to step 3 of Example 267.
¹H NMR (600 MHz, DMSO-d6) δ 11.01 (s, 1H), 7.64 (s, 1H), 7.38 - 7.32 (m, 2H), 7.29 (dt, *J* = 7.8, 2.6 Hz, 3H), 7.02 (dd, *J* = 8.9, 2.6 Hz, 2H), 5.10 (dd, *J* = 13.3, 5.0 Hz, 1H), 4.71 - 4.63 (m, 2H), 4.40 (d, *J* = 17.2 Hz, 1H), 4.24 (d, *J* = 17.2 Hz, 1H), 4.19 (s, 2H), 3.65 - 3.55 (m, 5H), 3.22 ( ddd, *J* = 13.5, 10.4, 3.2 Hz, 2H), 3.15 - 3.03 (m, 4H), 2.92 (ddd, *J* = 17.3, 13.6, 5.5 Hz, 2H), 2.62 - 2.57 (m, 1H), 2.44 (m, 1H), 2.23 (t, *J* = 13.7 Hz, 2H), 2.15 (d, *J* = 4.0 Hz, 1H), 2.04 - 1.72 (m, 10H), 1.30 - 1.14 (m, 3H). LC-MS: [M+H]⁺ =819.56

### Example 246

### Synthesis of compound 415

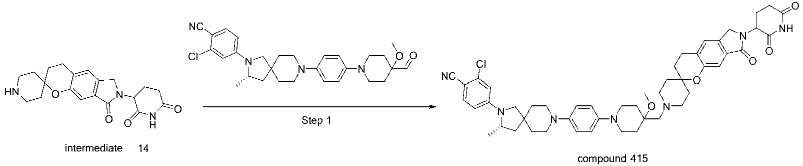

Step 1: (Compound 415) was prepared with reference to step 1 of example 267 LC-MS: [M+H]⁺ =860.76

¹H NMR (600 MHz, Methanol-d4) δ 7.53 (d, *J* = 8.8 Hz, 1H), 7.51 - 7.45 (m, 2H), 7.37 (s, 1H), 7.35 (s, 1H), 7.19 (s, 2H), 6.83 (s, 1H), 6.71 (d, *J* = 8.8 Hz, 1H), 5.15 (dd, *J* = 13.4, 5.1 Hz, 1H), 4.50 - 4.34 (m, 3H), 4.17 - 4.07 (m, 1H), 3.75 - 3.64 (m, 4H), 3.62 - 3.55 (m, 4H), 3.49 - 3.43 (m, 2H), 3.39 (s, 3H), 3.25 - 3.18 (m, 2H), 3.02 (s, 2H), 2.96 - 2.87 (m, 3H), 2.84 - 2.77 (m, 2H), 2.56 - 2.46 (m, 2H), 2.24 - 2.16 (m, 2H), 2.15 - 2.08 (m, 4H), 2.08 - 2.03 (m, 2H), 1.99 (s, 2H), 1.94 - 1.83 (m, 4H), 1.78 (dd, *J* = 12.9, 6.9 Hz, 2H), 1.33 (d, *J* = 6.1 Hz, 3H).

### Example 247

### Synthesis of intermediate 35

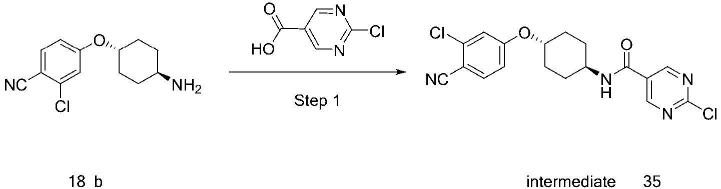

Step 1: Compound 18b (2.00 g, 7.98 mmol), p-2-chloropyrimidine-5-carboxylic acid (1.52 g, 9.57 mmol), T₃P (6.09 g, 9.57 mmol), and TEA (3.23 g, 31.91 mmol) were added sequentially into a glass vial, the solvent DCM (30 mL) was added, and the reaction solution was stirred at room temperature for 1 h. After the reaction was completed, the reaction solution was concentrated and then purified by preparative thin-layer chromatography (DCM:MeOH=10:1) to obtain a white solid compound Intermediate 35 (30 mg, crude).

Preparation with reference to step 3 of Example 18
LC-MS: [M+H]⁺ =391.29

### Example 248

### Synthesis of intermediate 36

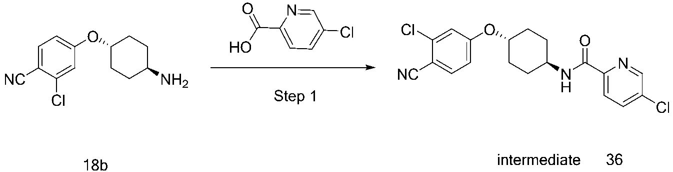

Step 1: 5-chloropyridine-2-carboxylic acid (188.52 mg, 1.20 mmol), HATU (545.95 mg, 1.44 mmol) and DIEA (773.24 mg, 5.98 mmol) were added sequentially to a glass vial, the solvent DMF (1 mL) was added, and the reaction solution was stirred at room temperature for 1 h. The intermediate 1 (300.00 mg, 1.20 mmol) was added and the reaction solution was continued to be stirred at room temperature for 1 h. After the reaction was completed, it was concentrated and then purified by preparative thin-layer chromatography (DCM:MeOH=10:1) to obtain a white solid compound intermediate 36 (105 mg, crude).
LC-MS: [M+H]⁺ =390.30

### Example 249

### Synthesis of compound 416

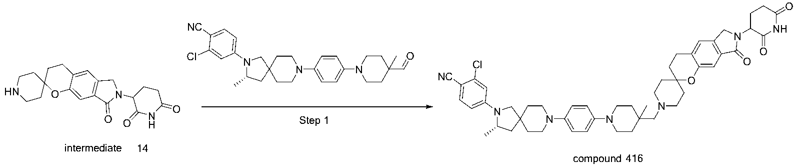

Step 1: (Compound 416) was prepared with reference to step 1 of Example 267.
¹H NMR (600 MHz, Methanol-d4) δ 7.53 (d, *J* = 8.8 Hz, 1H), 7.46 (d, *J* = 8.4 Hz, 2H), 7.37 (s, 1H), 7.34 (s, 1H), 7.25 (d, *J* = 8.0 Hz, 2H), 6.83 (d, *J* = 2.4 Hz, 1H), 6.71 (dd, *J* = 8.9, 2.4 Hz, 1H), 5.15 (dd, *J* = 13.4, 5.1 Hz, 1H), 4.47 - 4.36 (m, 2H), 4.12 (h, *J* = 6.5 Hz, 1H), 3.70 (s, 1H), 3.66 - 3.53 (m, 9H), 3.48 (d, *J* = 10.5 Hz, 1H), 3.38 - 3.34 (m, 4H), 3.01 (t, *J* = 6.8 Hz, 2H), 2.92 (ddd, *J* = 18.5, 13.6, 5.3 Hz, 2H), 2.80 (ddd, *J* = 17.5, 4.6, 2.4 Hz, 1H), 2.56 - 2.41 (m, 2H), 2.25 - 2.15 (m, 4H), 2.13 - 2.04 (m, 4H), 1.99 (d, *J* = 6.9 Hz, 2H), 1.93 - 1.84 (m, 2H), 1.84 - 1.73 (m, 4H), 1.35 (s, 3H), 1.33 (d, *J* = 6.1 Hz, 3H).LC-MS: [M+H]⁺ =844.66

### Example 250

### Synthesis of intermediate 38

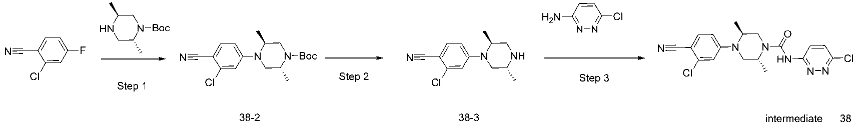

°CStep 1: Compounds 2-chloro-4-fluorobenzene (10.00 mg, 0.06 mmol), tert-butyl (2R,5S)-2,5-dimethylpiperazine-1-carboxylate (13.78 mg, 0.06 µmol) and potassium carbonate (17.77 mg, 0.13 mmol) were added sequentially in a glass vial, the solvent DMSO (1 mL) was added. The reaction solution was stirred at 80 °C for 12 h. After the reaction was completed, it was concentrated and then purified by preparative thin-layer chromatography (DCM:MeOH=10:1) to obtain a white solid compound 38-2 (12 mg).
LC-MS: [M+H]⁺ =294.18

Step 2: Compound 11-1 (1.00 g, 2.86 mmol), dioxane hydrochloride (10 ml) were added sequentially in a glass vial , the reaction solution was stirred at room temperature for 1 h. After the reaction was completed, the reaction solution was concentrated to obtain a white solid compound 38-3 (1.23 g).
LC-MS: [M+H]⁺ =250.26

Step 3: Compound 6-chloropyridazin-3-amine (5.00 mg, 0.03 mmol), CDI (9.10 mg, 0.06 mmol) and TEA (5.68 mg, 0.06 mmol) were added sequentially to a glass vial, the solvent DMF (1 mL) was added, the reaction solution was stirred at room temperature for 0.5 h. Compound 38-3 (21.02 mg, 84.16 µmol) was added, the reaction solution was stirred at room temperature for 2 h. After the reaction was completed, it was concentrated, and then purified by preparative thin-layer chromatography (DCM:MeOH=10:1) to obtain a white solid compound intermediate 38 (5.3 mg).
LC-MS: [M+H]⁺ =405.28

### Example 251

### Synthesis of compound 417

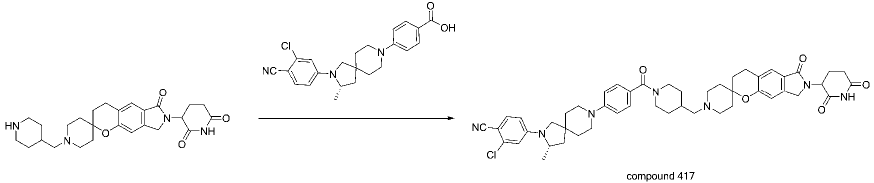

Step 1: (Compound 417) was prepared with reference to step 3 of Example 267.
¹ H NMR (400 MHz, *DMSO-d*₆ ) δ 10.98 (s, 1H), 7.60 (d, *J* = 8.9 Hz, 1H), 7.51 (d, *J* = 3.9 Hz, 1H), 7.26 (d, *J* = 8.4 Hz, 2H), 7.02 (d, *J* = 11.6 Hz, 1H), 6.96 (d, *J* = 8.6 Hz, 2H), 6.80 (d, *J* = 2.3 Hz, 1H), 6.66 (dd, *J* = 8.9, 2.3 Hz, 1H), 5.07 (dt, *J* = 13.3, 4.4 Hz, 1H), 4.34 (dd, *J* = 17.0, 7.0 Hz, 1H), 4.22 (d, *J* = 17.0 Hz, 1H), 4.04 (m, 1H), 3.32 (d, *J* = 10.8 Hz, 3H), 3.27 - 3.13 (m, 6H), 3.10 (d, *J* = 6.1 Hz, 2H), 2.91 (tt, *J* = 12.5, 5.7 Hz, 5H), 2.64 - 2.56 (m, 1H), 2.43 - 2.29 (m, 2H), 2.24 (dd, *J* = 12.8, 7.7 Hz, 1H), 2.13 (s, 1H), 1.96 (q, *J* = 13.2, 10.0 Hz, 5H), 1.87 (t, *J* = 6.7 Hz, 2H), 1.83 - 1.69 (m, 4H), 1.58 (dd, *J* = 12.9, 6.5 Hz, 1H), 1.50 (q, *J* = 5.3, 4.5 Hz, 2H), 1.24 (d, *J* = 3.5 Hz, 2H), 1.21 (d, *J* = 6.0 Hz, 4H), 1.18 - 1.12 (m, 1H).LC--MS: [M+H]⁺ = 858.66

### Example 252

### Synthesis of compound 418

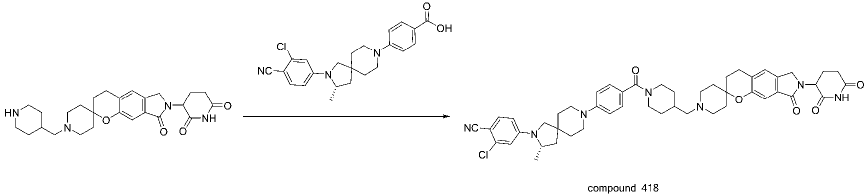

Step 1: (Compound 418) was prepared with reference to step 3 of Example 267.
¹H NMR (400 MHz, DMSO-*d*₆) δ 10.99 (s, 1H), 7.60 (d, *J* = 8.9 Hz, 1H), 7.36 (s, 1H), 7.28 - 7.22 (m, 3H), 6.97 (d, *J* = 8.3 Hz, 2H), 6.80 (d, *J* = 2.3 Hz, 1H), 6.66 (dd, *J* = 8.9, 2.4 Hz, 1H), 5.07 (dt, *J* = 13.3, 4.4 Hz, 1H), 4.34 (d, *J* = 16.8 Hz, 1H), 4.22 (d, *J* = 16.8 Hz, 1H), 4.04 (q, *J* = 6.6 Hz, 1H), 3.34 (t, *J* = 9.8 Hz, 4H), 3.27 - 3.15 (m, 5H), 3.12 (d, *J* = 6.1 Hz, 2H), 2.98 - 2.84 (m, 5H), 2.60 (d, *J* = 17.5 Hz, 1H), 2.39 (dd, *J* = 13.2, 4.6 Hz, 2H), 2.24 (dd, *J* = 12.8, 7.7 Hz, 1H), 2.13 (s, 1H), 2.03 - 1.92 (m, 4H), 1.89 (q, *J* = 6.6, 5.9 Hz, 3H), 1.82 - 1.69 (m, 4H), 1.58 (dd, *J* = 12.8, 6.6 Hz, 1H), 1.50 (t, *J* = 4.8 Hz, 2H), 1.24 (d, *J* = 3.6 Hz, 2H), 1.21 (d, *J* = 6.0 Hz, 4H), 1.17 (d, *J* = 3.7 Hz, 1H).LC-MS: [M+H]⁺ =858.56

### Example 253

### Synthesis of compound 419

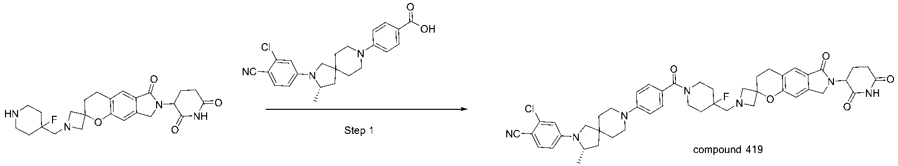

Step 1: (Compound 419) was prepared with reference to step 3 of Example 267.
¹H NMR (400 MHz, *DMSO-d*₆) δ 10.98 (s, 1H), 7.60 (d, *J* = 8.9 Hz, 1H), 7.52 (s, 1H), 7.29 (d, *J* = 8.7 Hz, 2H), 7.06 (s, 1H), 6.97 (d, *J* = 8.8 Hz, 2H), 6.80 (d, *J* = 2.3 Hz, 1H), 6.66 (dd, *J* = 9.0, 2.3 Hz, 1H), 5.07 (dd, *J* = 13.2, 5.0 Hz, 1H), 4.50 - 4.18 (m, 5H), 4.04 (dd, *J* = 13.5, 6.5 Hz, 2H), 3.46 - 3.30 (m, 5H), 3.21 (ddd, *J* = 15.3, 9.0, 3.6 Hz, 5H), 2.98 - 2.81 (m, 3H), 2.64 - 2.54 (m, 1H), 2.42 - 2.30 (m, 1H), 2.24 (dd, *J* = 12.4, 7.6 Hz, 3H), 1.98 (dt, *J* = 21.3, 11.9 Hz, 2H), 1.90 (d, *J* = 9.4 Hz, 2H), 1.73 (dd, *J* = 15.9, 11.7 Hz, 3H), 1.58 (dd, *J* = 12.8, 6.5 Hz, 1H), 1.48 (d, *J* = 14.8 Hz, 2H ), 1.23 (d, *J* = 5.3 Hz, 2H), 1.21 (d, *J* = 6.1 Hz, 3H).LC-MS: [M+H]⁺ = 848.56

### Example 254

### Synthesis of compound 420

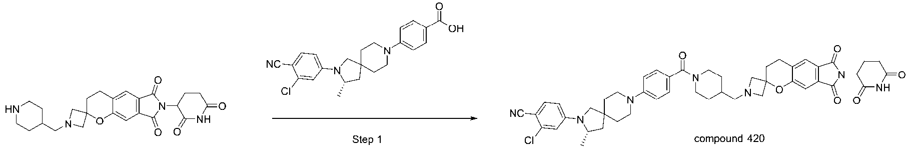

Step 1: (Compound 420) was prepared with reference to step 3 of Example 267.
¹H NMR (400 MHz, DMSO-*d*₆) δ 11.12 (s, 1H), 7.78 (s, 1H), 7.60 (d, *J* = 8.9 Hz, 1H), 7.28 (s, 1H), 7.25 (d, *J* = 8.6 Hz, 2H), 6.95 (d, *J* = 8.8 Hz, 2H), 6.80 (d, *J* = 2.2 Hz, 1H), 6.66 (dd, *J* = 9.0, 2.2 Hz, 1H), 5.11 (dd, *J* = 12.8, 5.4 Hz, 1H), 4.57 - 4.23 (m, 4H), 4.04 (dd, *J* = 13.3, 6.5 Hz, 1H), 3.40-3.28 (d, *J* = 11.1 Hz, 6H), 3.43 - 3.28 (m, 4H), 3.27 - 3.14 (m, 3H), 2.92 (dd, *J* = 35.5, 18.3 Hz, 4H), 2.60 (d, *J* = 18.3 Hz, 1H), 2.53 (d, J = 11.0 Hz, 1H), 2.24 (dd, *J* = 12.3, 7.6 Hz, 2H), 2.09 - 1.98 (m, 1H), 1.91 (s, 1H), 1.80 - 1.62 (m, 3H), 1.62 - 1.51 (m, 1H), 1.47 (d, *J* = 14.8 Hz, 2H), 1.21 (t, *J* = 7.6 Hz, 5H).LC-MS: [M+H]⁺ = 844.60

### Example 255

### Synthesis of compound 421

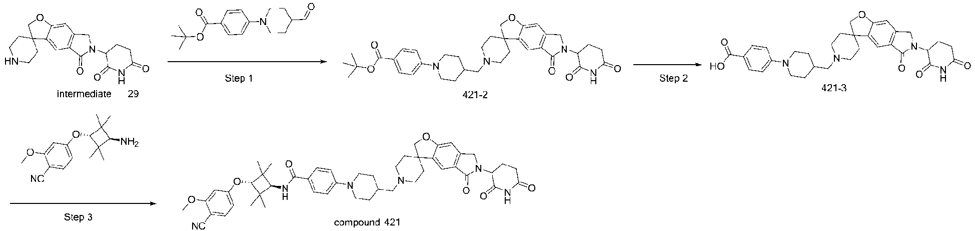

Step 1: (Compound 421-2) was prepared by reference to step 3 of Example 267. LC-MS: [M+H]⁺ = 629.48

Step 2: (Compound 421-3) was prepared in reference to step 2 of Example 267. LC-MS: [M+H]⁺ = 573.50

Step 3: (Compound 421) was prepared with reference to step 3 of Example 267. LC-MS: [M+H]⁺ = 829.66
¹H NMR (600 MHz, DMSO) δ 10.99 (s, 1H), 7.77 (d, *J* = 8.7 Hz, 2H), 7.66 (d, *J* = 8.6 Hz, 1H), 7.53 (d, *J* = 9.2 Hz, 1H), 7.43 (s, 1H), 7.05 (d, *J* = 11.1 Hz, 1H), 7.01 (d, *J* = 8.9 Hz, 2H), 6.64 (d, *J* = 2.0 Hz, 1H), 6.54 (dd, *J* = 8.6, 2.1 Hz, 1H), 5.07 (ddd, *J* = 34.3, 13.2, 5.0 Hz, 1H), 4.64 (m, 2H), 4.37 (d, *J* = 17.3 Hz, 1H), 4.30 - 4.21 (m, 2H), 4.06 (d, *J* = 9.2 Hz, 1H), 3.92 (d, *J* = 9.9 Hz, 5H), 3.08 (s, 3H), 2.95 - 2.81 (m, 3H), 2.60 (d, *J* = 18.0 Hz, 1H), 2.39 (m, 1H), 2.24 (t, *J* = 13.6 Hz, 2H), 2.09 (d, *J* = 13.3 Hz, 1H), 2.01 - 1.93 (m, 3H), 1.87 (dd, *J* = 28.3, 11.6 Hz, 3H), 1.33 (dd, *J* = 20.9, 11.5 Hz, 2H), 1.23 (s, 8H), 1.15 (s, 6H).

### Example 256

### Synthesis of compound 424

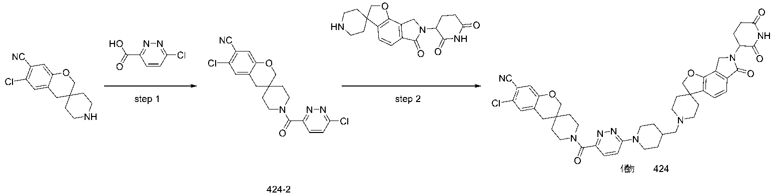

### Step 1: (Compound 424-2) was prepared with reference to step 3 of Example 267. LC-MS: [M+H]⁺ =403.29

### Step 2: (Compound 424) was prepared with reference to step 3 of example 19. LC-MS: [M+H]+=819.56

¹H NMR (600 MHz, DMSO-*d₆*) δ 11.01 (s, 1H), 7.58 (d, *J* = 9.5 Hz, 1H), 7.48 (d, *J* = 12.2 Hz, 2H), 7.43 - 7.34 (m, 2H), 7.29 (d, *J* = 7.6 Hz, 1H), 5.11 (dd, *J* = 13.2, 5.1 Hz, 1H), 4.72 - 4.63 (m, 2H), 4.50 (d, *J* = 13.0 Hz, 2H), 4.40 (d, *J* = 17.1 Hz, 1H), 4.24 (d, *J* = 17.1 Hz, 1H), 4.10 - 4.02 (m, 2H), 3.83 (d, *J* = 10.5 Hz, 1H), 3.70 (s, 2H), 3.58 (d, *J* = 11.7 Hz, 6H), 3.12 - 2.99 (m, 6H), 2.96 - 2.83 (m, 3H), 2.21 (dd, *J* = 19.0, 9.0 Hz, 3H), 2.04 - 1.90 (m, 4H), 1.86 (d, *J* = 13.0 Hz, 2H), 1.54 - 1.41 (m, 4H).

### Example 257

### Synthesis of compound 207

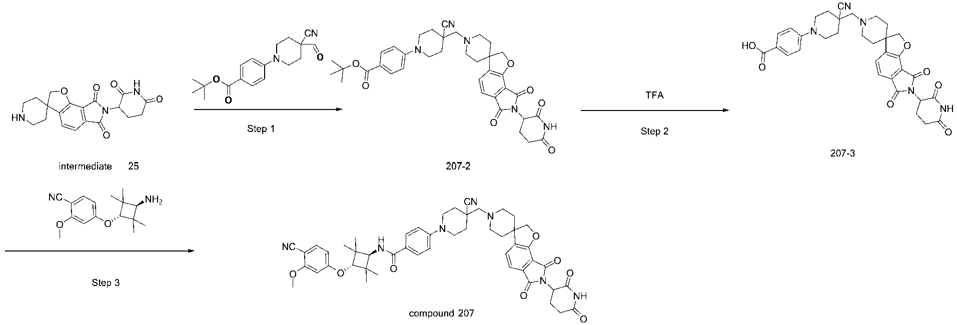

### Step 1: (Compound 207-2) LC-MS: [M+H]⁺ =667.30

The intermediate 25 (50.0 mg, 140.69 µmol) and tert-butyl 4-(4-cyano-4-formylpiperidin-1-yl)benzoate (59.44 mg, 281.38 µmol) were added sequentially in a glass vial, and solvent DCE:DMSO = 3 : 1 (1.6 mL) was added, and the reaction solution was stirred at room temperature for 1 h, and then NaBH(OAc)₃ (89.45 mg, 422.07 µmol) and 1 drop of acetic acid were added, and the reaction solution was continued to be stirred at room temperature for 1 h. After the reaction was completed, the reaction solution was concentrated and then purified by preparative thin-layer chromatography (DCM:MeOH=10:1) to obtain a white solid compound 207-2 (20 mg, 51.63%).

### Step 2: (Compound 207-3) was prepared with reference to step 2 of example 39. LC-MS: [M+H]⁺ =611.66

### Step 3: (Compound 207) was prepared with reference to step 3 of Example 18. LC-MS: [M+H]⁺ =867.40

¹ H NMR (600 MHz, *DMSO-d*₆ ) δ 11.11 (s, 1H), 7.80 (d, *J* = 8.6 Hz, 2H), 7.66 (d, *J* = 8.6 Hz, 2H), 7.57 (d, *J* = 9.3 Hz, 1H), 7.49 (d, *J* = 6.7 Hz, 1H), 7.07 (d, *J* = 8.5 Hz, 2H), 6.64 (d, *J* = 2.2 Hz, 1H), 6.55 (dd, *J* = 8.6, 2.2 Hz, 1H), 5.11 (dd, *J* = 12.9, 5.5 Hz, 1H), 4.79 (s, 2H), 4.28 (s, 1H), 4.07 (d, *J* = 9.2 Hz, 1H), 3.98 (d, *J* = 12.9 Hz 2H), 3.91 (s, 3H), 3.02 - 2.84 (m, 5H), 2.63 - 2.52 (m, 2H), 2.26 - 2.07 (m, 5H), 2.06 - 1.85 (m, 4H), 1.77 (s, 3H), 1. 1.23 (s, 7H), 1.16 (s, 6H).

### Example 258

### Synthesis of compound 208

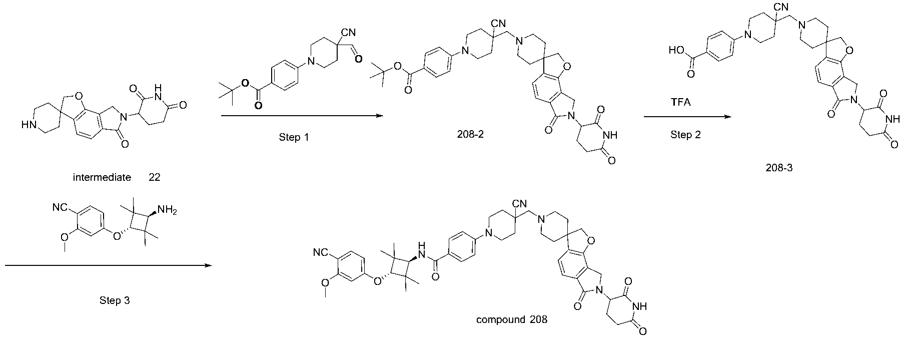

### Step 1: (Compound 208-2) was prepared with reference to step 1 of example 257. LC-MS: [M+H]⁺ =653.32

### Step 2: Compound 208-3 was prepared with reference to step 2 of Example 39. LC-MS: [M+H]⁺ =597.26

### Step 3: Prepared with reference to step 3 of Example 18. LC-MS: [M+H]⁺ =853.4

¹H NMR (600 MHz, DMSO-*d*₆) δ 10.99 (d, *J* = 3.1 Hz, 1H), 7.80 (d, *J* = 8.6 Hz, 2H), 7.66 (d, *J* = 8.6 Hz, 1H), 7.57 (d, *J* = 9.2 Hz, 1H), 7.34 (dq, *J* = 16.6, 7.6 Hz, 2H), 7.07 (d, *J* = 8.6 Hz, 2H), 6.64 (d, *J* = 2.3 Hz, 1H), 6.55 (dd, *J* = 8.6, 2.2 Hz, 1H), 5.09 (dd, *J* = 13.3, 5.1 Hz, 1H), 4.70 - 4.60 (m, 2H), 4.40 (d, *J* = 17.0 Hz, 1H), 4.29 - 4.22 (m, 2H), 4.07 (d, *J* = 9.2 Hz, 1H), 3.98 (d, *J* = 13.0 Hz, 2H), 3.91 (s, 3H), 3.01 - 3.66 - 3.52 (m, 1H),2.87 (m, 4H), 2.59 (t, *J* = 15.4 Hz, 2H), 2.48 - 2.38 (m, 2H), 2.34 - 2.09 (m, 5H), 2.03 - 1.96 (m, 2H), 1.90 (d, *J* = 18.4 Hz, 2H), 1.76 (s, 2H), 1.23 (s, 6H), 1.16 (s, 6H).

### Example 259

### Synthesis of compound 209

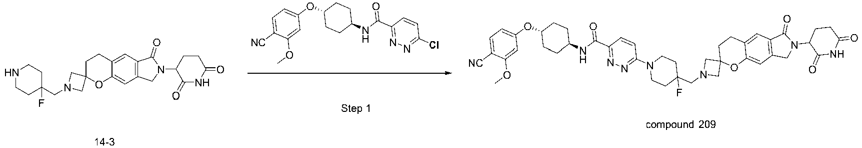

Step 1: (Compound 209) was prepared with reference to step 3 of Example 19.
¹H NMR (400 MHz, DMSO-*d*₆) δ 10.97 (s, 1H), 8.62 (d, *J* = 8.3 Hz, 1H), 7.88 (d, *J* = 9.5 Hz, 1H), 7.64 - 7.60 (m, 1H), 7.52 (s, 1H), 7.46 (d, *J* = 9.6 Hz, 1H), 7.04 (s, 1H), 6.75 - 6.70 (m, 2H), 5.07 (dd, *J* = 13.3, 5.1 Hz, 1H), 4.49 (m, 2H), 4.36 (d, *J* = 15.7 Hz, 4H), 4.23 (d, *J* = 17.3 Hz, 2H), 3.89 (s, 3H), 3.32 (t, *J* = 12.5 Hz, 4H), 2.91 (t, *J* = 12.8 Hz, 4H), 2.60 (d, *J* = 17.2 Hz, 2H), 2.40 - 2.31 (m, 2H), 2.23 (s, 2H), 2.13 (d, *J* = 11.2 Hz, 2H), 2.03 - 1.86 (m, 6H), 1.68 - 1.59 (m, 2H), 1.53 (t, *J* = 11.6 Hz, 2H). LC-MS: [M+H]⁺ =806.36

### Example 260

### Synthesis of compound 210

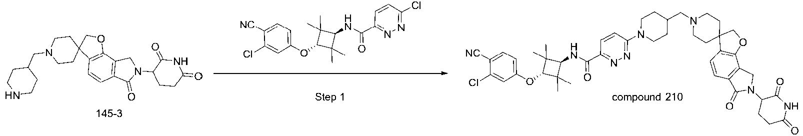

Step 1: (Compound 210) was prepared with reference to step 3 of Example 19.
¹H NMR (600 MHz, DMSO-*d*₆) δ 10.99 (s, 1H), 8.24 (d, *J* = 9.1 Hz, 1H), 7.91 (d, *J* = 8.7 Hz, 1H), 7.87 (d, *J* = 9.5 Hz, 1H), 7.44 (d, *J=* 9.6 Hz, 1H), 7.37 (d, *J* = 7.5 Hz, 1H), 7.30 (d, *J* = 7.6 Hz, 1H), 7.26 (d, *J* = 2.2 Hz, 1H), 7.04 (dd, *J* = 8.7, 2.1 Hz, 1H), 5.10 (dd, *J* = 13.2, 5.0 Hz, 1H), 4.68 (q, *J* = 9.4 Hz, 2H), 4.62 - 4.51 (m, 3H), 4.47 (s, 1H), 4.41 (d, *J* = 17.1 Hz, 1H), 4.25 (d, *J* = 17.1 Hz, 1H), 4.02 (d, *J* = 9.1 Hz, 2H), 3.67 - 3.59 (m, 2H), 3.15 - 3.04 (m, 5H), 2.97 - 2.88 (m, 1H), 2.61 (d, *J* = 15.9 Hz, 1H), 2.47 - 2.36 (m, 1H), 2.31 - 2.18 (m, 3H), 2.03 - 1.85 (m, 5H), 1.53 (d, *J* = 7.0 Hz, 1H), 1.23 (s, 6H), 1.15 (s, 6H). LC-MS: [M+H]⁺ =835.56

### Example 261

### Synthesis of compound 211

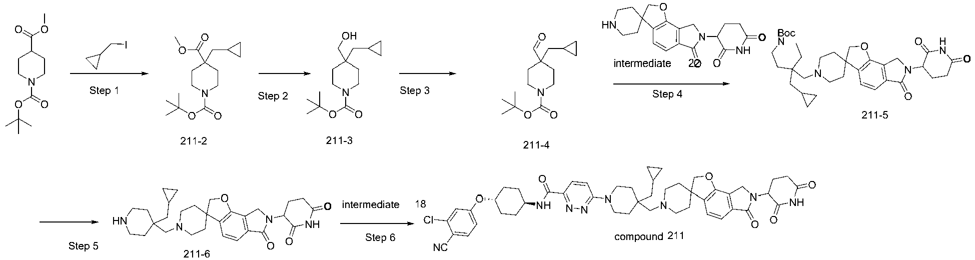

### Step 1: (Compound 211-2)

In a glass vial, 1-(tert-butyl)4-methylpiperidine-1,4-dicarboxylate (1.0 g, 4.11 mmol) and solvent-dried tetrahydrofuran (10.0 mL) were added, cooled down to -78 °C, then LDA (2 M, 5.14 mL, 10.28 mmol) was slowly added and the reaction solution was stirred at -78 °C for 1 h. Then (iodomethyl) cyclopropane was added (897.67 mg, 4.93 mmol), then slowly brought to room temperature and continued to be stirred overnight. After the reaction was completed, the reaction was quenched with saturated ammonium chloride aqueous solution and extracted with ethyl acetate, the organic phase was concentrated, and then purified by Flash to obtain a yellow oily compound 211-2 (10 mg, 66.27%).

### Step 2: (Compound 211-3)

In a glass vial, 211-2 (810.0 mg, 2.72 mmol) and solvent-dried tetrahydrofuran (10.0 mL) were added, and after cooling down to 0 °C, lithium aluminum hydride (206.74 mg, 5.45 mmol) was added and the reaction solution was stirred at 0 °C for 0.5 h. The reaction solution was then slowly brought to room temperature and continued to be stirred for 3 h. After the reaction was completed, it was quenched with 10% sodium hydroxide solution, diluted with ethyl acetate and dried with anhydrous sodium sulfate, filtered and concentrated to obtain a colorless oily compound 211-3 (667 mg).

### Step 3: (Compound 211-4)

Compound 211-3 (677.0 mg, 2.51 mmol) and IBX (1.41 g, 5.03 mmol) were added sequentially in a glass vial, the solvent MeCN (15.0 mL) was added, the reaction solution was raised to 80 °C and continued to be stirred for 2 h. After the reaction was completed, the reaction solution was concentrated to obtain a crude white solid compound 211-4 (840 mg).

### Step 4: (Compound 211-5) LC-MS: [M+H]⁺ =607.48

The intermediate 18 (50 mg, 140.69 µmol) and 211-4 (188.08 mg, 703.44 µmol) were added sequentially in a glass vial, and the solvent THF:DMSO= 5 : 1 (2.4 mL) was added, and finally tetraisopropyl titanate (79.97 mg, 281.38 µmol) was added, and the reaction solution was raised to 50 °C and stirred at 50 °C for 1 h. Then NaBH₃CN (26.52 mg, 422.07 µmol) was added, and the reaction solution was continued to be stirred at 50 °C for 1.5 h. After the reaction was completed, it was concentrated, and then purified by Flash to obtain a white solid compound 211-5 (50 mg).

### Step 5: (Compound 211-6) was prepared with reference to step 2 of Example 39. LC-MS: [M+H]⁺ =507.45

Step 6: (Compound 211) was prepared with reference to step 3 of Example 19.
¹H NMR (600 MHz, DMSO-*d*₆) δ 10.99 (s, 1H), 8.61 (d, *J =* 8.0 Hz, 1H), 7.88 - 7.83 (m, 2H), 7.43 - 7.33 (m, 3H), 7.28 (d, *J =* 7.5 Hz, 1H), 7.14 (dd, *J =* 8.8, 2.2 Hz, 1H), 5.10 (dd, *J =* 13.3, 5.1 Hz, 1H), 4.71 - 4.62 (m, 2H), 4.60 - 4.50 (m, 2H), 4.43 - 4.34 (m, 1H), 4.24 (d, *J =* 17.2 Hz, 1H), 4.08 - 4.01 (m, 1H), 4.01 - 3.94 (m, 2H), 3.92 - 3.84 (m, 2H), 3.66 - 3.58 (m, 4H), 2.98 - 2.87 (m, 2H), 2.61 (d, *J =* 17.2 Hz, 2H), 2.46 - 2.35 (m, 3H), 2.12 (d, *J =* 8.1 Hz, 2H), 2.04 - 1.96 (m, 2H), 1.96 - 1.87 (m, 3H), 1.81 (s, 2H), 1.76 (s, 1H), 1.69 - 1.60 (m, 4H), 1.57 - 1.48 (m, 3H), 0.52 (d, *J =* 7.7 Hz, 2H), 0.17 (d, *J =* 4.4 Hz, 2H). LC-MS: [M+H]⁺ =861.56

### Example 262

### Synthesis of compound 212

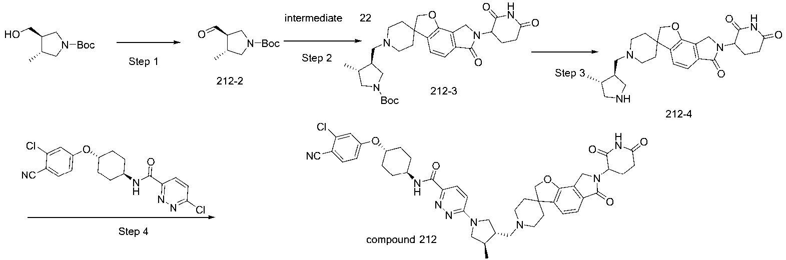

### Step 1: (Compound 212-2) was prepared with reference to step 3 of Example 211.

### Step 2: (Compound 212-3) was prepared with reference to step 1 of example 257. LC-MS: [M+H]⁺ =553.38

### Step 3: (Compound 212-4) was prepared with reference to step 2 of example 39. LC-MS: [M+H]⁺ =453.45

Step 4: (Compound 212) was prepared with reference to step 3 of Example 19.
¹H NMR (600 MHz, DMSO-*d*₆) δ 10.99 (s, 1H), 9.82 (s, 1H), 8.51 (s, 1H), 7.91 - 7.83 (m, 2H), 7.41 - 7.29 (m, 2H), 7.14 (dd, *J =* 8.8, 2.1 Hz, 1H), 6.97 (t, *J* = 9.1 Hz, 1H), 5.11 (dd, *J =* 13.3, 4.9 Hz, 1H), 4.74 - 4.64 (m, 2H), 4.61 - 4.50 (m, 2H), 4.41 (d, *J* = 17.1 Hz, 1H), 4.25 (d, *J =* 17.1 Hz, 1H), 3.95 - 3.80 (m, 2H), 3.72 (d, *J =* 12.4 Hz, 1H), 3.66 - 3.54 (m, 1H), 3.39 (d, *J =* 20.1 Hz, 2H), 3.28 (s, 1H), 3.24 - 3.05 (m, 3H), 2.97 - 2.86 (m, 1H), 2.66 - 2.57 (m, 1H), 2.48 - 2.35 (m, 2H), 2.32 - 2.22 (m, 2H), 2.22 - 2.15 (m, 1H), 2.11 (d, *J =* 10.4 Hz, 2H), 2.06 - 1.95 (m, 3H), 1.92 (d, *J =* 10.4 Hz, 2H), 1.65 (dd, *J =* 23.8, 11.0 Hz, 2H), 1.53 (dd, *J =* 22.7, 10.2 Hz, 2H), 1.30 - 1.12 (m, 3H). LC-MS: [M+H]⁺ =807.46

### Example 263

### Synthesis of compound 213

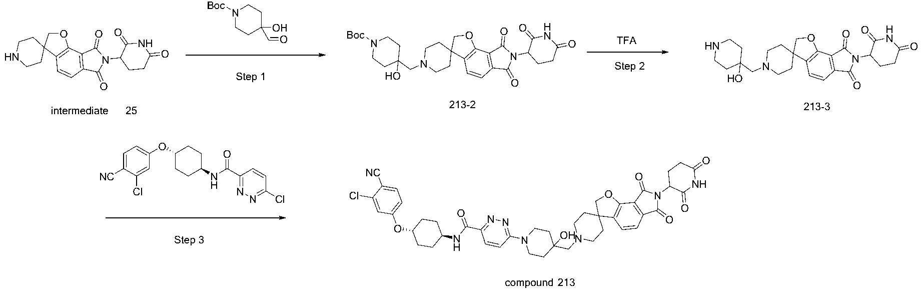

### Step 1: (Compound 213-2) was prepared with reference to step 1 of Example 257. LC-MS: [M+H]⁺ =583.38

### Step 2: (Compound 213-3) was prepared with reference to step 2 of Example 39. LC-MS: [M+H]⁺ =483.29

Step 3: (Compound 213) was prepared with reference to step 3 of Example 19.
¹H NMR (600 MHz, DMSO-*d*₆) δ 11.11 (s, 1H), 8.61 (d, *J =* 8.3 Hz, 1H), 7.89 - 7.82 (m, 2H), 7.54 (dd, *J =* 22.2, 7.2 Hz, 2H), 7.45 - 7.36 (m, 2H), 7.16 - 7.13 (m, 1H), 5.11 (dd, *J =* 12.8, 5.4 Hz, 1H), 4.82 (s, 2H), 4.62 - 4.50 (m, 2H), 4.23 (d, *J =* 12.1 Hz, 2H), 3.91 - 3.82 (m, 1H), 3.66 (d, *J =* 11.1 Hz, 2H), 3.24 (s, 2H), 3.21 (s, 2H), 2.93 - 2.83 (m, 1H), 2.60 (d, *J* = 17.6 Hz, 1H), 2.39 (t, *J =* 12.4 Hz, 2H), 2.15 - 2.08 (m, 2H), 2.05 - 1.87 (m, 6H), 1.80 (d, *J* = 12.7 Hz, 2H), 1.73 - 1.60 (m, 4H), 1.56 - 1.47 (m, 4H). LC-MS: [M+H]⁺ =837.46

### Example 264

### Synthesis of compound 214

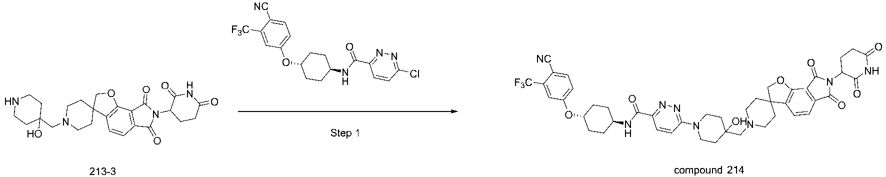

Step 1: (Compound 214) was prepared with reference to step 3 of Example 19. LC-MS: [M+H]⁺ =871.56
¹H NMR (600 MHz, DMSO-*d*₆) δ 11.11 (s, 1H), 8.62 (d, *J =* 8.2 Hz, 1H), 8.08 (d, *J =* 8.6 Hz, 1H), 7.86 (d, *J* = 9.5 Hz, 1H), 7.56 (d, *J =* 7.3 Hz, 1H), 7.54 - 7.46 (m, 3H), 7.42 (d, *J =* 9.6 Hz, 1H), 5.11 (dd, *J =* 12.9, 5.4 Hz, 1H), 4.82 (s, 2H), 4.64 (dd, *J =* 12.3, 8.1 Hz, 1H), 4.23 (d, *J =* 12.1 Hz, 2H), 3.93 - 3.84 (m, 2H), 3.29 - 3.16 (m, 4H), 2.94 - 2.83 (m, 2H), 2.60 (d, *J =* 17.6 Hz, 1H), 2.57 - 2.53 (m, 1H), 2.40 (t, *J =* 12.1 Hz, 2H), 2.29 (s, 1H), 2.17 - 2.10 (m, 2H), 2.05 - 2.00 (m, 1H), 1.95 (dd, *J =* 34.0, 12.0 Hz, 4H), 1.80 (d, *J =* 13.0 Hz, 2H), 1.74 - 1.49 (m, 8H).

### Example 265

### Synthesis of compound 215

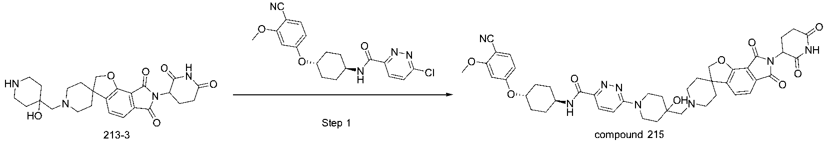

Step 1: (Compound 215) was prepared with reference to step 3 of Example 19.
¹H NMR (600 MHz, DMSO-*d*₆) δ 11.12 (s, 1H), 8.61 (d, *J =* 8.2 Hz, 1H), 7.86 (d, *J =* 9.5 Hz, 1H), 7.62 (d, *J* = 9.2 Hz, 1H), 7.55 (d, *J =* 6.9 Hz, 1H), 7.52 (d, *J =* 7.2 Hz, 1H), 7.43 (d, *J =* 9.6 Hz, 1H), 6.75 - 6.69 (m, 2H), 5.57 (s, 1H), 5.11 (dd, *J =* 12.9, 5.4 Hz, 1H), 4.82 (s, 2H), 4.58 (q, *J* = 7.0 Hz, 1H), 4.53 - 4.45 (m, 1H), 4.23 (d, *J =* 12.5 Hz, 2H), 3.92 - 3.82 (m, 4H), 3.66 (d, *J =* 10.0 Hz, 1H), 3.28 - 3.18 (m, 3H), 2.94 - 2.85 (m, 1H), 2.60 (d, *J =* 17.8 Hz, 1H), 2.44 - 2.34 (m, 2H), 2.29 (s, 1H), 2.17 - 2.08 (m, 2H), 2.07 - 1.86 (m, 5H), 1.83 - 1.75 (m, 2H), 1.73 - 1.61 (m, 4H), 1.56 - 1.45 (m, 5H). LC-MS: [M+H]⁺ =833.66

### Example 266

### Synthesis of compound 216

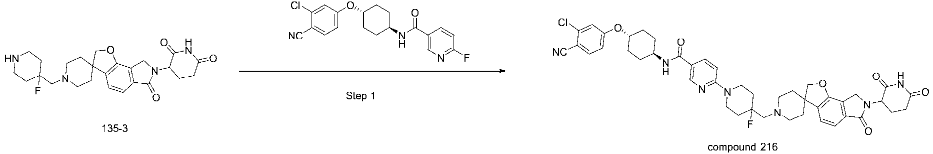

Step 1: (Compound 216) was prepared with reference to step 3 of Example 19.
¹H NMR (600 MHz, DMSO-*d*₆) δ 10.99 (s, 1H), 8.64 (s, 1H), 8.11 - 7.96 (m, 2H), 7.87 (d, *J =* 8.8 Hz, 1H), 7.39 (d, *J =* 2.3 Hz, 1H), 7.33 (d, *J =* 36.4 Hz, 2H), 7.15 (dd, *J =* 8.8, 2.3 Hz, 1H), 6.96 (s, 1H), 5.10 (dd, *J =* 13.2, 5.0 Hz, 1H), 4.73 - 4.50 (m, 3H), 4.40 (d, *J =* 17.1 Hz, 1H), 4.35 - 4.14 (m, 3H), 3.81 (d, *J =* 6.9 Hz, 1H), 3.67 - 3.50 (m, 2H), 3.27 (t, *J* = 11.8 Hz, 3H), 2.97 - 2.87 (m, 2H), 2.60 (d, *J =* 16.9 Hz, 1H), 2.46 - 2.38 (m, 2H), 2.34 (s, 1H), 2.15 - 2.08 (m, 2H), 2.06 - 1.85 (m, 7H), 1.85 - 1.61 (m, 4H), 1.58 - 1.45 (m, 4H). LC-MS: [M+H]⁺ = 824.46

### Example 267

### Synthesis of compound 217

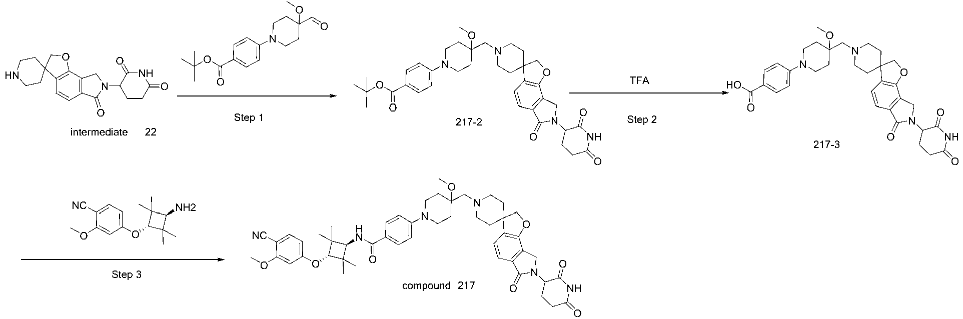

### Step 1: (Compound 217-2) LC-MS: [M+H]⁺ =659.57

The intermediate 22 (200.0 mg, 562.76 µmol) and tert-butyl 4-(4-formyl-4-methoxypiperidin-1-yl)benzoate (215.69 mg, 675.31 µmol) were added sequentially to a glass vial, and the solvent THF (3 mL), Ti(O-iPr)₄₍319.89 mg, 1.13 mmol) were added. The reaction solution was stirred at 60 °C for 1 h. Then NaBH₃CN (106.09 mg, 1.69 mmol) was added and the reaction solution was continued to be stirred at 60 °C for 1 h. After the reaction was completed, the reaction solution was concentrated, and then purified by preparative thin-layer chromatography (DCM:MeOH=10:1) to obtain a white solid compound 217-2 (60 mg, 16.18%).

### Step 2: (Compound 217-3) LC-MS: [M+H]⁺ =603.48

In a glass vial, 217-2 (60.0 mg, 91.08 µmol) was added, solvent DCM (1 mL) was added, then TFA (1 mL) was added, and the reaction solution was stirred at room temperature for 1 h. After the reaction was completed, it was concentrated, and then purified by a reversed-phase column (MeCN in Water=30%) to obtain a white solid compound 217-3 (44 mg, 80.16%).

### Step 3: (Compound 217)

In a glass vial, 217-3 (40.0 mg, 66.37 µmol) and the intermediate 42 (21.85 mg, 675.31 µmol), EDCI (19.04 mg, 99.56 µmol), HOBT (13.44 mg, 99.56 µmol) were added sequentially, and the solvent DMF (1 mL), DIEA ( 25.73 mg, 199.11 umol) were added, the reaction solution was stirred at room temperature for 2 h. After the reaction was completed, it was concentrated, and then purified by high-performance liquid phase preparation to obtain a white solid compound 217 (33 mg, 57.88%).
¹H NMR (600 MHz, DMSO-d6) δ 10.99 (s, 1H), 7.79 (d, *J =* 8.4 Hz, 2H), 7.66 (d, *J =* 8.6 Hz, 1H), 7.53 (d, *J* = 9.3 Hz, 1H), 7.37 (d, *J =* 7.6 Hz, 1H), 7.28 (d, *J =* 7.5 Hz, 1H) , 7.03 (d, J = 8.5 Hz, 2H), 6.64 (d, *J =* 2.2 Hz, 1H), 6.55 (dd, *J =* 8.6, 2.2 Hz, 1H), 5.10 (dd, *J =* 13.3, 5.1 Hz, 1H), 4.72 - 4.60 (m, 2H), 4.41 (dd, *J =* 17.1 , 4.5 Hz, 1H), 4.31 - 4.21 (m, 2H), 4.06 (d, *J* = 9.2 Hz, 1H), 3.91 (s, 3H), 3.72 - 3.65 (m, 5H), 3.39 - 3.35 (m, 2H), 3.29 ( d, *J =* 17.3 Hz, 4H), 3.09 (t, *J =* 11.6 Hz, 2H), 2.96 - 2.88 (m, 1H), 2.61 (dd, *J =* 15.4, 4.6 Hz, 1H), 2.48 - 2.31 (m, 3H), 2.05 - 1.84 (m, 5H), 1.69 (t, *J =* 11.5 Hz, 2H), 1.23 (s, 6H), 1.16 (s, 6H). LC-MS: [M+H]⁺ =859.66

### Example 268

### Synthesis of compound 218

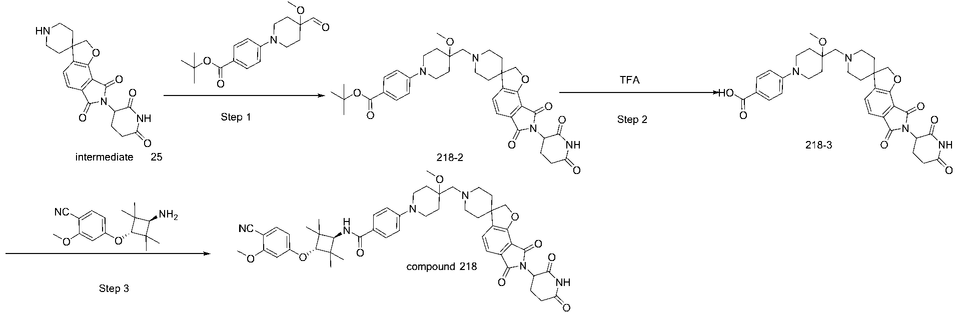

### Step 1: (Compound 218-2) was prepared with reference to step 1 of example 257. LC-MS: [M+H]⁺ =673.37

### Step 2: (Compound 218-3) was prepared in reference to step 2 of example 39. LC-MS: [M+H]⁺ =617.48

### Step 3: (Compound 218) was prepared with reference to step 3 of example 267. LC-MS: [M+H]⁺ =873.56

¹H NMR (600 MHz, DMSO-d6) δ 11.12 (s, 1H), 7.79 (d, *J =* 8.3 Hz, 2H), 7.65 (dd, *J =* 8.7, 3.3 Hz, 1H), 7.61 - 7.44 (m, 3H), 7.03 (d, *J =* 8.8 Hz, 2H), 6.64 (d, *J* = 2.1 Hz, 1H), 6.54 (dd, *J =* 8.7, 2.1 Hz, 1H), 5.11 (dd, *J =* 12.9, 5.5 Hz, 1H), 4.81 (d, *J =* 16.1 Hz, 2H), 4.29 (s, 1H), 4.06 (d, *J =* 9.2 Hz, 2H), 3.91 (s, 4H), 3.67 (d, *J =* 11.8 Hz, 4H), 3.29 (d, *J* = 16.0 Hz, 5H), 3.09 (t, *J =* 11.6 Hz, 2H), 2.89 (ddd, *J =* 16.9, 13.7, 5.5 Hz, 1H), 2.60 (d, *J =* 18.3 Hz, 2H), 2.41 - 2.31 (m, 2H), 2.00 (dd, *J =* 32.1, 12.6 Hz, 5H), 1.71 (d, *J =* 12.6 Hz, 2H), 1.23 (s, 6H), 1.16 (s, 6H).

### Example 269

### Synthesis of compound 219

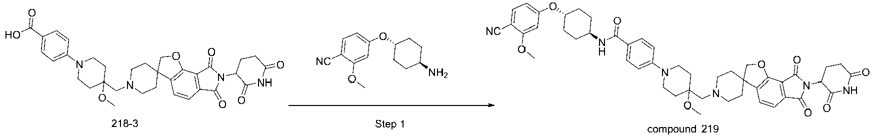

### Step 1: (Compound 219) was prepared with reference to step 3 of example 267. LC-MS: [M+H]⁺ =845.56

¹H NMR (600 MHz, DMSO-d6) δ 11.12 (s, 1H), 7.99 (d, *J =* 7.7 Hz, 1H), 7.77 (d, *J =* 8.4 Hz, 2H), 7.62 (d, *J =* 8.6 Hz, 1H), 7.58 - 7.45 (m, 2H), 7.00 (d, *J =* 8.6 Hz, 2H), 6.77 - 6.69 (m, 2H), 5.11 (dd, *J =* 12.9, 5.5 Hz, 1H), 4.81 (d, *J* = 16.5 Hz, 2H), 4.49 (tt, *J =* 9.2, 4.3 Hz, 1H), 3.89 (s, 3H), 3.87 - 3.77 (m, 5H), 3.37 (s, 2H), 3.28 (d, *J =* 15.8 Hz, 5H), 3.07 (t, *J* = 11.7 Hz, 2H), 2.89 (ddd, *J* = 17.8, 13.9, 5.5 Hz, 1H), 2.64 - 2.52 (m 2H), 2.35 (t, *J =* 14.2 Hz, 2H), 2.13 (dd, *J =* 9.6, 5.0 Hz, 2H), 2.06 - 1.94 (m, 5H), 1.93 - 1.87 (m, 2H), 1.74 - 1.64 (m, 2H ), 1.59 - 1.42 (m, 4H).

### Example 270

### Synthesis of compound 220

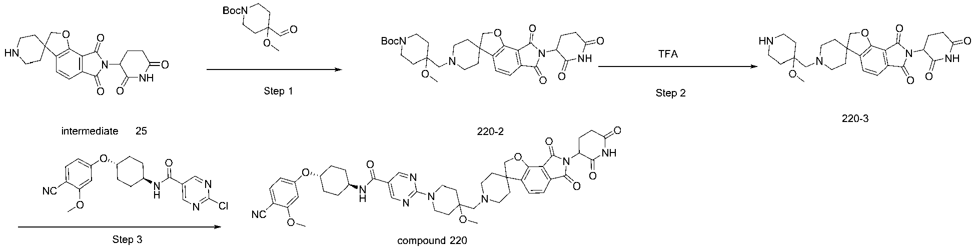

### Step 1: (Compound 220-2) was prepared with reference to step 1 of Example 257. LC-MS: [M+H]⁺ =597.48

### Step 2: (Compound 220-3) was prepared with reference to step 2 of Example 39. LC-MS: [M+H]⁺ =497.39

### Step 3: (Compound 220) was prepared with reference to step 3 of Example 19. LC-MS: [M+H]⁺ =847.66

¹H NMR (600 MHz, DMSO-d6) δ 11.11 (s, 1H), 8.80 (s, 2H), 8.15 (dd, *J* = 7.7, 2.9 Hz, 1H), 7.63 (d, *J =* 8.6 Hz, 1H), 7.59 - 7.47 (m, 2H), 6.77 - 6.67 (m, 2H), 5.11 (dd, J = 12.9, 5.5 Hz, 1H), 4.81 (d, *J =* 18.1 Hz, 2H), 4.56 - 4.41 (m, 4H), 3.89 (s, 3H), 3.81 (d, *J =* 9.2 Hz, 2H), 3.64 (d, *J =* 12.5 Hz, 2H), 3.37 (s, 2H), 3.29 (d, *J =* 10.0 Hz, 5H), 2.89 (ddd, *J =* 18.0, 13.8, 5.5 Hz, 1H), 2.64 - 2.57 (m, 1H), 2.55 (d, *J =* 13.2 Hz, 1H), 2.40 - 2.25 (m, 2H), 2.18 - 2.06 (m, 2H), 2.07 - 1.83 (m, 7H), 1.59 (m, 2H), 1.51 (m, 4H).

### Example 271

### Synthesis of compound 221

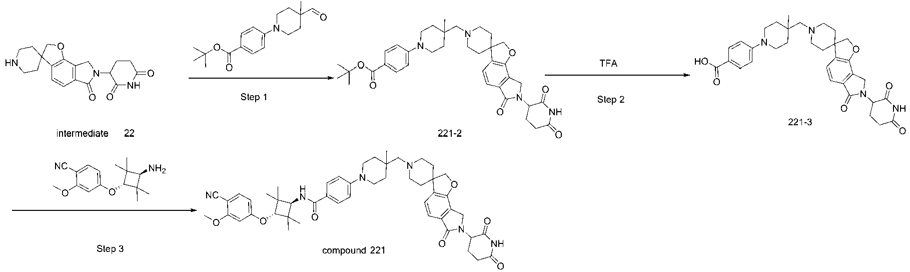

### Step 1: (Compound 221-2) was prepared with reference to step 1 of Example 257. LC-MS: [M+H]⁺ =643.18

### Step 2: (Compound 221-3) was prepared with reference to step 2 of Example 39. LC-MS: [M+H]⁺ =587.50

### Step 3: (Compound 221) was prepared with reference to step 3 of Example 18. LC-MS: [M+H]⁺ =843.65

¹H NMR (600 MHz, DMSO-d6) δ 10.99 (s, 1H), 7.78 (d, *J =* 8.7 Hz, 2H), 7.65 (d, *J =* 8.6 Hz, 1H), 7.53 (d, *J =* 9.2 Hz, 1H), 7.33 (dd, *J =* 40.6, 7.3 Hz, 2H), 7.00 (d, *J =* 8.8 Hz, 2H), 6.64 (d, *J =* 1.8 Hz, 1H), 6.54 (dd, *J* = 8.6, 1.9 Hz, 1H), 5.10 (dd, *J =* 13.3, 4.9 Hz, 1H), 4.67 (s, 2H), 4.57 (q, *J =* 7.0 Hz, 1H), 4.40 *(d, J=* 17.1 Hz, 1H), 4.30 - 4.22 (m, 2H), 4.06 (d, *J =* 9.2 Hz, 1H), 3.91 (s, 3H), 3.58 (s, 4H), 3.15 (s, 4H), 2.95 - 2.88 (m, 1H), 2.60 *(d, J=* 17.3 Hz, 1H), 2.45 - 2.38 (m, 2H), 2.03 - 1.87 (m, 3H), 1.73 - 1.58 (m, 4H), 1.52 (d, *J =* 7.0 Hz, 2H), 1.23 (s, 9H), 1.15 (s, 6H).

### Example 272

### Synthesis of compound 222

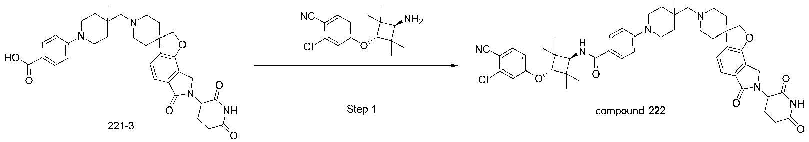

Step 1: (Compound 222) was prepared with reference to step 3 of Example 267.
¹H NMR (400 MHz, DMSO-d6) δ 10.99 (s, 1H), 7.91 (d, *J =* 8.8 Hz, 1H), 7.78 (d, *J =* 8.8 Hz, 2H), 7.51 (d, *J* = 9.2 Hz, 1H), 7.37 (d, *J =* 7.5 Hz, 1H), 7.29 (d, *J =* 7.6 Hz, 1H), 7.21 (d, *J =* 2.4 Hz, 1H), 7.01 (dd, *J =* 8.8, 2.5 Hz, 3H), 5.10 (dd, *J =* 13.3, 5.0 Hz, 1H), 4.67 (s, 2H), 4.45 - 4.21 (m, 3H), 4.07 (d, *J* = 9.1 Hz, 1H), 3.59 (s, 4H), 3.40 - 3.11 (m, 6H), 2.92 (ddd, *J =* 18.9, 13.9, 5.3 Hz, 1H), 2.68 - 2.54 (m, 1H), 2.46 - 2.32 (m, 3H), 2.02 - 1.88 (m, 3H), 1.75 - 1.57 (m, 4H), 1.23 (d, *J =* 6.0 Hz, 9H), 1.13 (s, 6H).LC-MS: [M+H]⁺=847.66

### Example 273

### Synthesis of compound 223

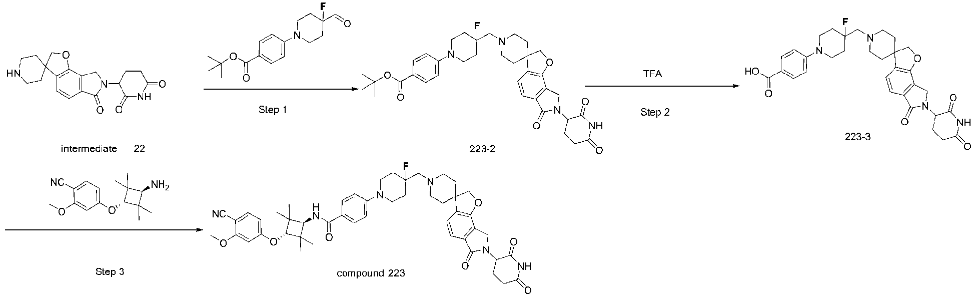

### Step 1: (Compound 223-2) was prepared with reference to step 1 of Example 27.1 LC-MS: [M+H]⁺ =647.70

### Step 2: (Compound 223-3) was prepared with reference to step 2 of Example 39. LC-MS: [M+H]⁺ =591.38

### Step 3: (Compound 223) was prepared with reference to step 3 of Example 267. LC-MS: [M+H]⁺ =847.56

¹H NMR (600 MHz, DMSO-d6) δ 10.99 (s, 1H), 7.80 (d, *J =* 8.7 Hz, 2H), 7.66 (d, *J =* 8.6 Hz, 1H), 7.55 (d, *J =* 9.2 Hz, 1H), 7.36 (d, *J =* 7.5 Hz, 1H), 7.30 (s, 1H), 7.06 (d, *J =* 9.0 Hz, 2H), 6.65 (d, *J =* 1.8 Hz, 1H), 6.55 (dd, *J =* 8.6, 1.9 Hz, 1H), 5.10 (dd, *J =* 13.2, 5.0 Hz, 1H), 4.67 (s, 2H), 4.40 (d, *J =* 17.1 Hz, 1H), 4.30 - 4.22 (m, 2H), 4.07 (d, *J* = 9.2 Hz, 1H), 3.91 (s, 3H), 3.80 (d, *J =* 12.6 Hz, 2H), 3.26 (m, 3H), 3.15 (t, *J =* 11.8 Hz, 2H), 2.97 - 2.87 (m, 1H), 2.61 (d, *J =* 16.9 Hz, 1H), 2.55 (s, 2H), 2.48 - 2.25 (m, 3H), 2.10 - 1.81 (m, 8H), 1.23 (s, 6H), 1.16 (s, 6H).

### Example 274

### Synthesis of compound 224

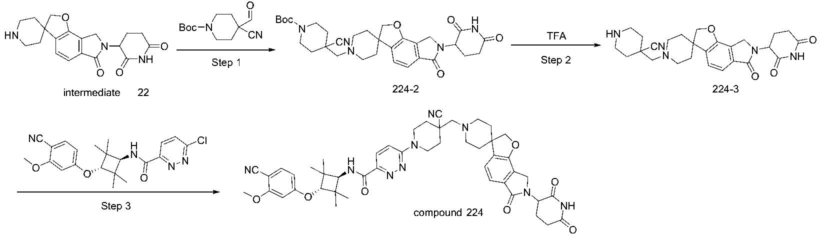

### Step 1: (Compound 224-2) was prepared with reference to step 1 of Example 257. LC-MS: [M+H]⁺ =578.49

### Step 2: (Compound 224-3) was prepared with reference to step 2 of Example 39. LC-MS: [M+H]⁺ =478.59

### Step 3: (Compound 224) was prepared with reference to step 3 of Example 19. LC-MS: [M+H]⁺ =856.66

¹ H NMR (600 MHz, DMSO-*d*₆) δ 10.98 (s, 1H), 8.29 (d, *J* = 9.2 Hz, 1H), 7.90 (d, *J* = 9.5 Hz, 1H), 7.65 (d, *J =* 8.6 Hz, 1H), 7.47 (d, *J* = 9.6 Hz, 1H), 7.41 (d, *J = 7.6* Hz, 1H), 7.28 (d, *J* = 7.5 Hz, 1H), 6.67 (d, *J =* 2.2 Hz, 1H), 6.57 (dd, *J =* 8.7, 2.2 Hz, 1H), 5.09 (dd, *J =* 13.3, 5.1 Hz, 1H), 4.64 - 4.52 (m, 4H), 4.45 - 4.36 (m, 2H), 4.22 (d, *J =* 17.0 Hz, 1H), 4.02 (d, *J* = 9.2 Hz, 1H), 3.92 (s, 3H), 3.25 - 3.15 (m, 2H), 3.01 - 2.87 (m, 3H), 2.73 - 2.54 (m, 4H), 2.47 - 2.34 (m, 3H), 2.08 (d, *J =* 13.0 Hz, 2H), 2.04 - 1.88 (m, 3H), 1.75 - 1.58 (m, 4H), 1.24 (s, 6H), 1.17 (s, 5H).

### Example 275

### Synthesis of compound 225

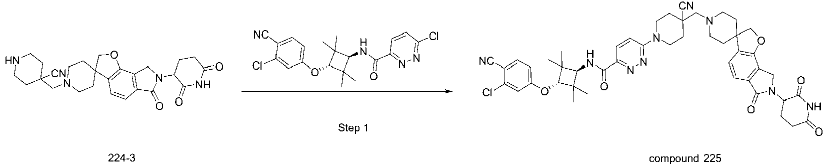

Step 1: (Compound 225) was prepared with reference to step 3 of Example 19.
¹H NMR (600 MHz, DMSO-*d*₆) δ 10.98 (s, 1H), 8.28 (d, *J* = 9.2 Hz, 1H), 7.90 (dd, *J =* 12.6, 9.1 Hz, 2H), 7.47 (d, *J* = 9.6 Hz, 1H), 7.41 (d, *J* = 7.6 Hz, 1H), 7.31 - 7.24 (m, 2H), 7.04 (dd, *J =* 8.8, 2.4 Hz, 1H), 5.09 (dd, *J =* 13.3, 5.1 Hz, 1H), 4.62 - 4.52 (m, 3H), 4.47 (s, 1H), 4.38 (d, *J =* 17.0 Hz, 1H ), 4.22 (d, *J =* 17.0 Hz, 1H), 4.02 (d, *J* = 9.2 Hz, 1H), 2.99 - 2.87 (m, 3H), 2.69 - 2.57 (m, 3H), 2.45 - 2.37 (m, 3H), 2.08 ( d, *J =* 13.2 Hz, 2H), 2.04 - 1.90 (m, 4H), 1.73 - 1.59 (m, 4H), 1.46 (p, *J* = 7.3 Hz, 1H), 1.28 - 1.20 (m, 9H), 1.15 (s, 4H). LC-MS: [M+H]⁺ =860.56

### Example 276

### Synthesis of compound 226

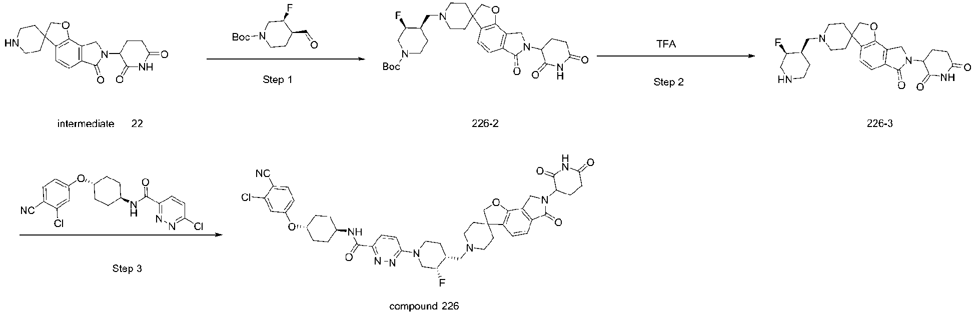

### Step 1: (Compound 226-2) was prepared with reference to step 1 of Example 257. LC-MS: [M+H]⁺ =571.48

### Step 2: (Compound 226-3) was prepared with reference to step 2 of Example 39. LC-MS: [M+H]⁺ =471.59

### Step 3: (Compound 226) was prepared with reference to step 3 of Example 19. LC-MS: [M+H]⁺ =825.56

¹ H NMR (600 MHz, *DMSO-d*₆ ) δ 10.99 (s, 1H), 8.60 (d, *J =* 8.2 Hz, 1H), 7.86 (t, *J =* 9.8 Hz, 2H), 7.44 (d, *J =* 9.6 Hz, 1H), 7.39 (d, *J* = 2.4 Hz, 1H), 7.36 (d, *J* = 7.5 Hz, 1H), 7.31 (d, *J* = 7.2 Hz, 1H), 7.14 *(dd, J=* 8.9, 2.4 Hz, 1H), 5.10 (dd, *J =* 13.3, 5.1 Hz, 1H), 4.87 (t, *J =* 13.0 Hz, 1H), 4.72 - 4.64 (m, 2H), 4.54 (tt, *J =* 10.1, 4.2 Hz, 1H), 4.40 (d, *J =* 17.1 Hz, 1H), 4.25 (d, *J =* 17.1 Hz, 1H), 3.87 (dt, *J =* 7.9, 5.6 Hz, 1H), 3.69 (s, 2H), 3.28 (d, *J =* 14.4 Hz, 2H), 3.19 - 3.02 (m, 4H), 2.97 - 2.88 (m, 1H), 2.61 (d, *J =* 20.5 Hz, 1H), 2.43 (dt, *J =* 13.3, 6.6 Hz, 2H), 2.22 (d, *J =* 31.4 Hz, 2H), 2.15 - 2.07 (m, 2H ), 2.05 - 1.78 (m, 7H), 1.70 - 1.57 (m, 3H), 1.57 - 1.43 (m, 3H).

### Example 277

### Synthesis of compound 227

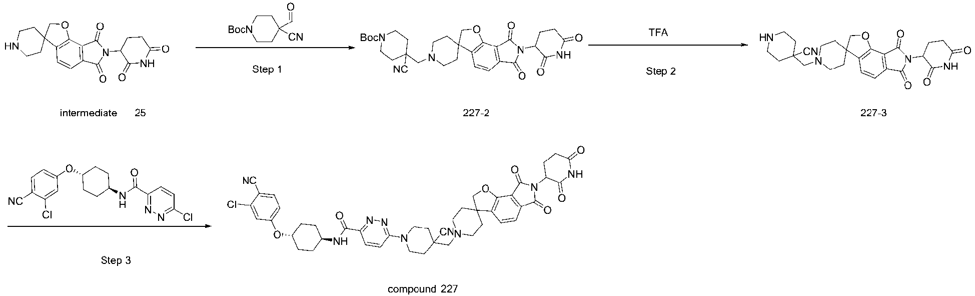

### Step 1: (Compound 227-2) was prepared with reference to step 1 of Example 257. LC-MS: [M+H]⁺ =592.49

### Step 2: (Compound 227-3) was prepared in reference to step 2 of Example 39. LC-MS: [M+H]⁺ =492.49

Step 3: (Compound 227) was prepared with reference to step 3 of Example 19.
¹H NMR (600 MHz, DMSO-*d*₆) δ 11.10 (s, 1H), 8.65 (d, *J =* 8.2 Hz, 1H), 7.88 (dd, *J =* 16.7, 9.1 Hz, 2H), 7.68 (s, 1H), 7.47 (d, *J* = 9.4 Hz, 2H), 7.39 (d, *J =* 2.4 Hz, 1H), 7.14 (dd, *J =* 8.8, 2.4 Hz, 1H), 5.10 (dd, *J =* 12.9, 5.4 Hz, 1H), 4.76 (s, 2H), 4.67 - 4.48 (m, 3H), 3.88 (dtd, *J =* 11.3, 7.6, 4.0 Hz, 2H), 3.20 (t, *J =* 13.1 Hz, 5H), 2.89 (m, 2H), 2.64 - 2.57 (m, 1H), 2.57 - 2.52 (m, 1H), 2.27 - 2.07 (m, 5H), 2.06 - 1.97 (m, 2H), 1.96 - 1.79 (m, 4H), 1.76 - 1.61 (m, 4H), 1.52 (m, 3H). LC-MS: [M+H]⁺ =846.46

### Example 278

### Synthesis of compound 228

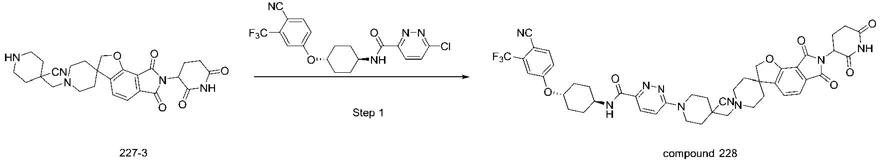

Step 1: (Compound 228) was prepared with reference to step 3 of Example 19.
¹H NMR (600 MHz, DMSO-*d*₆) δ 11.10 (s, 1H), 8.65 (d, *J =* 8.2 Hz, 1H), 8.08 (d, *J =* 8.5 Hz, 1H), 7.89 (d, *J =* 9.4 Hz, 1H), 7.69 (s, 1H), 7.54 - 7.42 (m, 4H), 5.10 (dd, *J =* 12.9, 5.5 Hz, 1H), 4.76 (s, 2H), 4.69 - 4.52 (m, 3H), 3.94 - 3.84 (m, 1H), 3.20 (t, *J =* 13.0 Hz, 4H), 2.94 - 2.84 (m, 2H), 2.63 - 2.57 (m, 1H), 2.27 - 1.97 (m, 9H), 1.96 - 1.79 (m, 4H), 1.78 - 1.62 (m, 4H), 1.61 - 1.39 (m, 4H). LC-MS: [M+H]⁺ =880.46

### Example 279

### Synthesis of compound 229

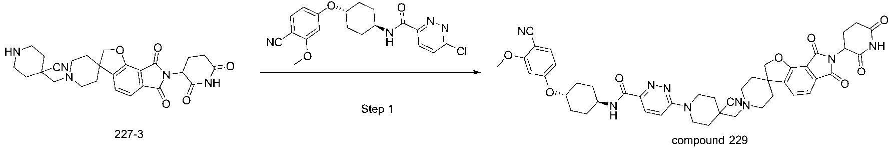

Step 1: (Compound 229) was prepared with reference to step 3 of Example 19.
¹H NMR (600 MHz, DMSO-*d*₆) δ 11.09 (s, 1H), 8.64 (d, *J* = 8.2 Hz, 1H), 7.87 (d, *J* = 9.5 Hz, 1H), 7.74 (d, *J* = 7.3 Hz, 1H), 7.62 (d, *J* = 9.2 Hz, 1H), 7.42 (dd, *J =* 12.9, 8.5 Hz, 2H), 6.74 - 6.70 (m, 2H), 5.09 (dd, *J =* 12.9, 5.5 Hz, 1H), 4.70 (s, 2H), 4.60 - 4.47 (m, 3H), 3.89 (s, 4H), 3.21 - 3.13 (m, 2H), 3.00 - 2.84 (m, 3H), 2.66 (s, 2H), 2.43 - 2.36 (m, 2H), 2.16 - 1.98 (m, 6H), 1.97 - 1.88 (m, 4H), 1.75 - 1.59 (m, 6H), 1.56 - 1.43 (m, 3H). LC-MS: [M+H]⁺ =842.56

### Example 280

### Synthesis of compound 230

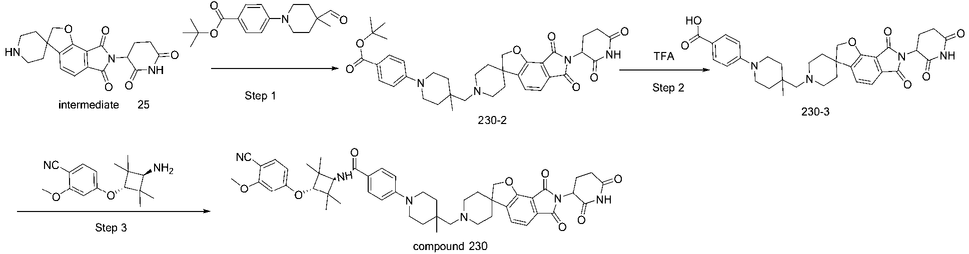

### Step 1: (Compound 230-2) was prepared with reference to step 1 of Example 257. LC-MS: [M+H]⁺ =657.47

### Step 2: (Compound 230-3) was prepared with reference to step 2 of Example 39. LC-MS: [M+H]⁺ =601.59

### Step 3: (Compound 230) was prepared with reference to step 3 of Example 267. LC-MS: [M+H]⁺ =857.66

¹H NMR (600 MHz, DMSO-*d*₆) δ 11.11 (s, 1H), 7.78 (d, *J =* 8.5 Hz, 2H), 7.66 (d, *J =* 8.6 Hz, 1H), 7.60 - 7.47 (m, 3H), 7.01 (d, *J =* 8.7 Hz, 2H), 6.65 (d, *J* = 2.2 Hz, 1H), 6.54 (dd, *J =* 8.7, 2.2 Hz, 1H), 5.11 (dd, *J =* 13.0, 5.4 Hz, 1H), 4.82 (s, 2H), 4.28 (s, 1H), 4.06 (d, *J* = 9.1 Hz, 1H), 3.91 (s, 3H), 3.40 - 3.21 (m, 4H), 3.20 - 3.08 (m, 4H), 2.89 (ddd, *J =* 16.9, 13.9, 5.5 Hz, 1H), 2.64 - 2.54 (m, 2H), 2.43 - 2.29 (m, 3H), 2.07 - 1.95 (m, 3H), 1.75 - 1.58 (m, 4H), 1.24 (d, *J =* 5.9 Hz, 10H), 1.15 (s, 6H).

### Example 281

### Synthesis of compound 231

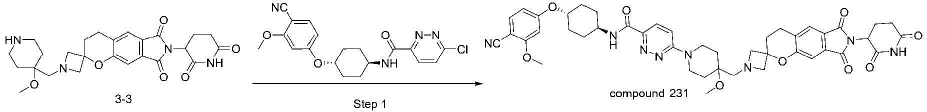

Step 1: (Compound 231) was prepared with reference to step 3 of Example 19.
¹H NMR (600 MHz, DMSO-*d*₆) δ 11.10 (s, 1H), 8.59 (d, *J =* 8.2 Hz, 1H), 7.81 (d, *J =* 9.4 Hz, 1H), 7.70 (s, 1H), 7.61 (d, *J =* 9.1 Hz, 1H), 7.34 (d, *J* = 9.6 Hz, 1H), 7.24 ( s, 1H), 6.73 (d, *J =* 6.4 Hz, 2H), 5.09 (dd, *J =* 12.9, 5.4 Hz, 1H), 4.50 (tt, *J* = 9.8, 4.2 Hz, 1H), 4.14 (d, *J =* 12.9 Hz, 2H), 3.89 (s, 4H), 3.47 (d, *J =* 7.6 Hz, 2H), 3.25 (s , 4H), 3.19 (s, 3H), 2.99 - 2.84 (m, 3H), 2.68 - 2.56 (m, 3H), 2.56 - 2.53 (m, 1H), 2.12 (q, *J =* 6.1 Hz, 4H), 2.06 - 1.97 (m, 1H), 1.94 - 1.86 (m, 2H), 1.81 (d, *J =* 13.5 Hz, 2H), 1.69 - 1.60 (m, 2H), 1.56 - 1.44 (m, 4H). LC-MS: [M+H]⁺ =832.46

### Example 282

### Synthesis of compound 232

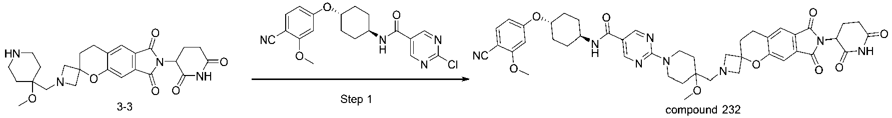

Step 1: (Compound 232) was prepared with reference to step 3 of Example 19.
¹H NMR (600 MHz, DMSO-*d*₆) δ 11.10 (s, 1H), 8.75 (s, 2H), 8.11 (d, *J =* 7.4 Hz, 1H), 7.70 (s, 1H), 7.62 (d, *J =* 8.5 Hz, 1H), 7.24 (s, 1H), 6.77 - 6.69 (m, 2H), 5.09 (dd, *J =* 12.9, 5.4 Hz, 1H), 4.51 (dt, *J =* 10.0, 5.2 Hz, 1H), 4.38 (dt, *J =* 13.4, 4.2 Hz, 2H), 3.89 (s, 3H), 3.84 - 3.76 (m, 1H), 3.47 (s, 2H), 3.29 - 3.15 (m, 7H), 2.98 - 2.84 (m, 3H), 2.68 - 2.56 (m, 3H), 2.55 (d, *J* = 13.1 Hz, 1H), 2.12 (t, *J =* 6.4 Hz, 4H), 2.03 (dd, *J =* 12.3, 6.1 Hz, 1H), 1.93 (dd,*J =* 9.6, 4.3 Hz, 2H), 1.79 (d, *J =* 13.4 Hz, 2H), 1.57 - 1.39 (m, 6H). LC-MS: [M+H]⁺ =832.56

### Example 283

### Synthesis of compound 233

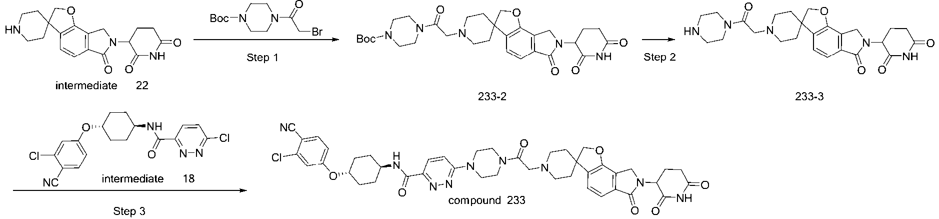

### Step 1: (Compound 233-2) LC-MS: [M+H]⁺ =582.48

The intermediate 22 (70.00 mg, 0.20 mmol) and tert-butyl 4-(2-bromoacetyl)piperazine-1-carboxylate (90.76 mg, 0.30 mmol) were added sequentially to a glass vial, the solvent DMF (1 mL) and DIEA (109.00 mg, 1.00 mmol) were added, and the reaction solution was stirred at room temperature for 2 h. After the reaction was completed, the reaction solution was extracted, concentrated and purified by preparative thin-layer chromatography (DCM:MeOH=10:1) to obtain a white solid compound 233-2 (50 mg, crude).

### Step 2: (Compound 233-3) was prepared with reference to step 2 of Example 39. LC-MS: [M+H]⁺ =482.40

### Step 3: (Compound 233) was prepared with reference to step 3 of Example 19. LC-MS: [M+H]⁺ =836.56

¹H NMR (600 MHz, DMSO-*d*₆) δ 10.99 (s, 1H), 8.64 (d, *J =* 8.0 Hz, 1H), 7.92 (d, *J* = 9.5 Hz, 1H), 7.87 (d, *J* = 8.8 Hz, 1H), 7.43 (d, *J =* 9.6 Hz, 1H), 7.41 - 7.36 (m, 2H), 7.32 (d, *J* = 7.5 Hz, 1H), 7.14 (dd, *J =* 8.8, 2.4 Hz, 1H), 5.10 (dd, *J =* 13.3, 5.1 Hz, 1H), 4.72 - 4.50 (m, 5H), 4.46 - 4.20 (m, 6H), 3.92 - 3.76 (m, 9H), 2.97 - 2.88 (m, 2H), 2.16 - 1.87 (m, 9H), 1.71 - 1.48 ( m, 5H).

### Example 284

### Synthesis of compound 234

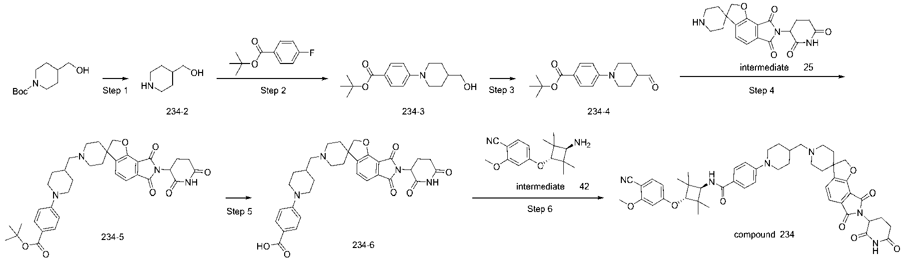

### Step 1: Synthesis of compound piperidin-4-ylmethanol (compound 234-2)

In a glass vial, tert-butyl 4-(hydroxymethyl)piperidine-1-carboxylate (1.0 g, 4.6 mmol) and trifluoroacetic acid (2 ml) were added sequentially, and solvent DCM (5 mL) was added, and the reaction solution was stirred at room temperature for 2 h. After the reaction was completed, the reaction solution was concentrated to obtain a yellow oily compound 234-2 (1.25 g, crude).

### Step 2: (Compound 234-3) LC-MS: [M+H]⁺ =292.18

Compound 234-2 (1.25 g, 10.8 mmol) and tert-butyl 4-fluorobenzoate (2.12 g, 10.8 mmol) were added sequentially in a glass vial, and the solvent DMSO (10 mL) was added and the reaction solution was stirred at 120°C for 2 h. After the reaction was completed, the reaction solution was extracted and concentrated, and then purified by preparative column chromatography (EA:PE=1:1) to obtain a white solid compound 234-3 (1 g, crude).

### Step 3: (Compound 234-4) LC-MS: [M+H]⁺ =290.17

Compound 234-3 (715 mg, 2.46 mmol) and 2-iodoacylbenzoic acid (1.03 g, 3.68 mmol) were added sequentially in a glass vial, solvent ACN (10 mL) was added, and the reaction solution was stirred at 80°C for 2 h. After the reaction was completed, it was filtered, concentrated and purified by preparative column chromatography (EA:PE=1:1) to obtain a white solid compound 234-4 (715 mg, crude^{).}

### Step 4: (Compound 234-5) LC-MS: [M+H]⁺ =643.31

Compound 234-4 (50 mg, 0.173 mmol), compound 25 (63.82 mg, 0.173 mmol) and tetraisopropyl titanate (98.26 mg, 0.346 mmol) were added sequentially into a glass vial, and the solvent THF (2 mL) was added, and the reaction solution was stirred at 60°C for 1 h. After that, sodium cyanoborohydride (32.70 mg, 0.52 mmol) was added, and the reaction solution was continued at 60°C for 1 h. After the reaction was completed, it was filtered and concentrated, and then purified by preparative thin-layer chromatography (MeOH:DCM=1:10) to obtain a white solid compound 234-5 (25 mg, crude).

### Step 5: (Compound 234-6) was prepared with reference to step 2 of Example 39. LC-MS: [M+H]⁺ =587.24

Step 6: (Compound 234) was prepared with reference to step 3 of Example 267.
¹H NMR (6600 MHz, DMSO-*d*₆) δ 11.12 (s, 1H), 7.77 (d, *J =* 8.8 Hz, 2H), 7.65 (d, *J =* 8.6 Hz, 1H), 7.58 (d, *J* = 7.3 Hz, 1H), 7.53 - 7.50 (m, 2H), 7.02 - 6.98 (m, 2H), 6.64 (d, *J =* 2.1 Hz, 1H), 6.55 (dd, *J =* 8.6, 2.1 Hz, 1H), 5.12 (d, *J =* 5.4 Hz, 1H), 5.10 (d, *J =* 5.4 Hz, 1H), 4.82 (s, 2H), 4.28 (s, 1H), 4.06 (d, *J =* 9.2 Hz, 2H), 3.91 (s, 5H), 3.62 (d, *J =* 10.5 Hz, 3H), 3.09 (d, *J* = 21.3 Hz, 5H), 2.92 - 2.81 (m, 5H), 2.64 - 2.58 (m, 2H), 2.40 - 2.38 (m, 1H), 2.24 - 2.16 (m, 3H), 2.06 - 1.96 (m, 7H), 1.84 (d, *J =* 11.5 Hz, 2H), 1.49 - 1.42 (m, 3H). LC-MS: [M+H]⁺ =843.40

### Example 285

### Synthesis of compound 235

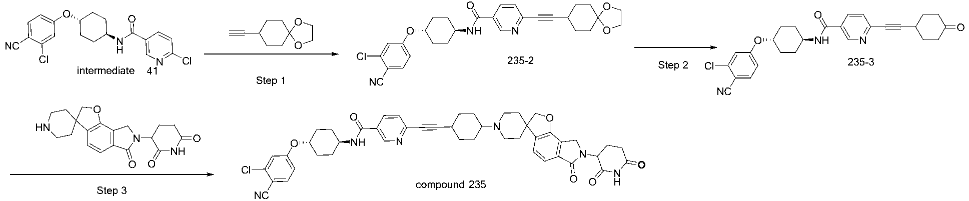

### Step 1: (Compound 235-2) LC-MS: [M+H]⁺ =520.29

The intermediate 41 (50.00 mg, 127.79 µmol) and 8-ethynyl-1,4-dioxaspiro[4.5]decane (25.49 mg, 153.35 µmol) were added sequentially to a glass vial. Solvent DMSO (2 mL), copper(I) iodide (7.3 mg, 38.34 µmo), bis(triphenylphosphine)palladium(II) chloride (8.97 mg, 0.02 mmol), and cesium carbonate (12.91 mg, 383.38µmol) were added. The reaction solution was heated to 110 °C and stirred for 2 hours. The reaction solution was purified by preparative chromatography to obtain a white solid compound 235-2(40 mg, crude). Step 2: (Compound 235-3) LC-MS: [M+H]⁺ =476.35

Compound 235-2 (40.0 mg, 140.69 µmol) and dioxane hydrochloride solution were added sequentially in a glass vial, and the reaction solution was stirred at room temperature for 2 h. The reaction solution was concentrated to obtain a white solid compound 235-3 (57 mg, crude).

### Step 3: (Compound 235)

Compound 235-3 (16.0 mg, 33.55 µmol) and the intermediate 22 (17.88 mg, 50.32 µmol) were added sequentially into a glass vial, and the solvents DCE:DMSO = 5 : 1 (1.2 mL) and acetic acid (1 drop) were added, and the reaction solution was stirred at room temperature for 1 h, and then NaBH(OAc)₃ (6.32 mg, 100.64 µmol) was added. and the reaction solution was continued to be stirred at room temperature for 1 h. After the reaction was completed, a white solid compound 235 (5 mg) was obtained by preparative purification.

¹H NMR (600 MHz, Methanol-d4) δ 8.93 (dd, *J =* 19.9, 2.3 Hz, 1H), 8.22 (m, 1H), 7.71 (d, *J =* 8.8 Hz, 1H), 7.64 (d, *J =* 8.2 Hz, 1H), 7.46 (dd, *J* = 7.6, 3.5 Hz, 1H), 7.37 (dd, *J =* 7.6, 2.4 Hz, 1H), 7.21 (t, *J =* 1.9 Hz, 1H), 7.06 (d, *J =* 8.8 Hz, 1H), 5.17 (m, 1H), 4.76 - 4.69 (m, 2H), 4.53 - 4.47 (m, 2H), 4.43 (dd, J = 17.0, 3.0 Hz, 2H), 3.98 (s, 2H), 3.74 (d, *J* = 12.7 Hz, 1H), 3.66 (d, *J =* 12.8 Hz, 2H), 3.26 - 3.18 (m, 2H), 2.93 (m, 1H), 2.84 - 2.77 (m, 1H), 2.73 - 2.65 (m, 1H), 2.59 - 2.47 (m, 2H), 2.36 (s, 1H), 2.29 (s, 2H), 2.27 - 2.24 (m, 2H), 2.23 - 2.15 (m, 5H), 2.12 (d, *J =* 10.2 Hz, 2H), 2.05 (d, *J =* 6.7 Hz, 2H), 1.83 (t, *J =* 13.7 Hz, 2H), 1.76 - 1.68 (m, 2H). LC-MS: [M+H]⁺ =815.45

### Example 286

### Synthesis of compound 236

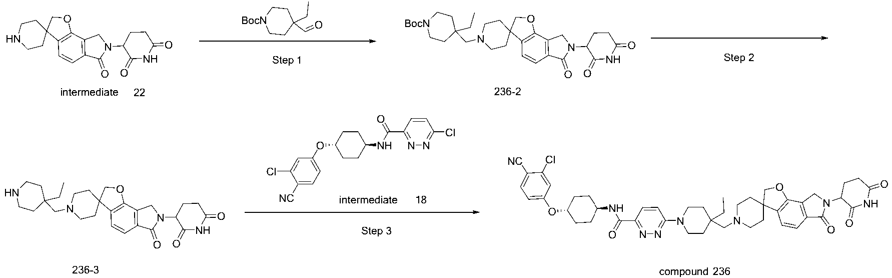

### Step 1: (Compound 236-2) was prepared with reference to step 1 of Example 257. LC-MS: [M+H]⁺ =581.35

### Step 2: (Compound 236-3) was prepared with reference to step 2 of Example 39. LC-MS: [M+H]⁺ =481.40

Step 3: (Compound 236) was prepared with reference to step 3 of Example 19.
¹H NMR (600 MHz, Methanol-d4) δ 8.00 (d, *J =* 9.7 Hz, 1H), 7.71 (d, *J* = 8.7 Hz, 1H), 7.45 (dd, *J =* 8.6, 4.3 Hz, 2H), 7.34 (d, *J* = 6.8 Hz, 1H), 7.22 (d, *J* = 2.4 Hz, 1H), 7.06 (dd, *J =* 8.8, 2.4 Hz, 1H), 5.16 - 4.89 (m,1H), 4.73 (s, 2H), 4.56 - 4.52 (m, 1H), 4.44 - 4.28 (m, 2H), 4.12 (d, *J =* 12.5 Hz, 2H), 4.04 - 3.97 (m, 1H), 3.75 (d, *J =* 12.7 Hz, 2H), 3.67 - 3.60 (m, 3H), 3.41 - 3.34 (m, 1H), 2.93 - 2.88 (m, 2H), 2.84 - 2.75 (m, 1H), 2.52 - 2.41 (m, 3H), 2.25 - 2.17 (m, 4H), 2.15 - 2.10 (m, 2H), 2.07 (d, *J =* 16.0 Hz, 2H), 1.92 - 1.83 (m, 4H), 1.81 - 1.75 (m, 2H), 1.67 (t, *J* = 9.5 Hz, 3H), 1.62 (d, *J =* 10.1 Hz, 1H). LC-MS: [M+H]⁺ =835.46

### Example 287

### Synthesis of compound 237

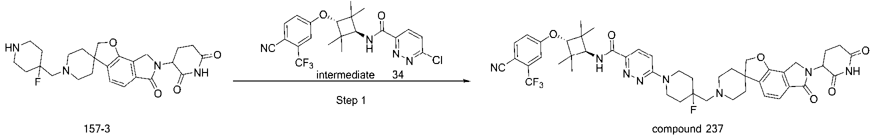

### Step 1: (Compound 237) was prepared with reference to step 3 of Example 19. LC-MS: [M+H]⁺ =887.40

¹H NMR (600 MHz, Methanol-d4) δ 8.00 (d, *J* = 9.6 Hz, 1H), 7.95 (d, *J =* 8.6 Hz, 1H), 7.47 (d, *J =* 2.9 Hz, 1H), 7.46 (s, 1H), 7.40 (d, *J* = 2.5 Hz, 1H), 7.29 (dd, *J =* 8.7, 2.5 Hz, 1H), 7.14 (m, 1H), 5.17 (m, 1H), 4.73 (s, 2H), 4.55 (d, *J* = 13.8 Hz, 2H), 4.48 (s, 1H), 4.45 - 4.40 (m, 2H), 4.15 (s, 1H), 3.81 (s, 2H), 3.63 (s, 2H), 3.50 (t, *J* = 12.5 Hz, 2H), 3.44 - 3.35 (m, 1H), 2.97 - 2.90 (m, 1H), 2.88 - 2.83 (m, 1H), 2.82 - 2.76 (m, 1H), 2.53 (m, 1H), 2.42 (d, *J =* 14.3 Hz, 2H), 2.25 (d, 2H), 2.22 - 2.16 (m, 1H), 2.11 (d, *J =* 14.1 Hz,2H), 2.04 - 1.92 (m, 2H), 1.34 (s, 6H), 1.26 (s, 6H).

### Example 288

### Synthesis of compound 238

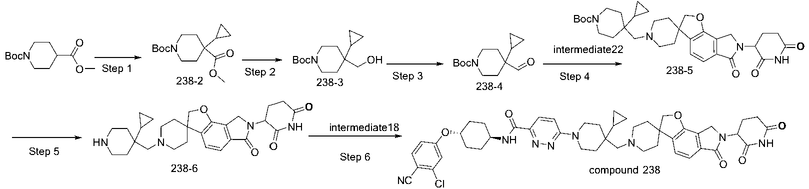

### Step 1: (Compound 238-2)

The commercially available compound 1-(tert-butyl)4-methylpiperidine-1,4-dicarboxylate (1.0 g, 4.11 mmol) was added sequentially to a three-necked flask, the solvent THF (20 mL) was added and the temperature was cooled down to -78 °C, and bromocyclopropane (546.95 mg 4.52 mmol) was added under the protection of nitrogen, and the reaction solution was stirred at a low temperature for 2 h. The reaction solution was brought to room temperature for 12 h. The reaction soluition was purified by forward purification to obtain a white oily compound 238-2 (750 mg).

### Step 2: (Compound 238-3)

Compound 238-2 (100.0 mg, 352.90 µmol) and lithium aluminum hydride (26.79 mg, 705.80 µmol) were added sequentially to a glass vial, the solvent THF (2 mL) was added, and the reaction solution was stirred at room temperature for 2 h, and a oily compound 238-3 (55 mg) was obtained by forward purification.

### Step 3: Synthesis of the compound tert-butyl 4-cyclopropyl-4-formylpiperidine-1-carboxylate (Compound 238-4)

Compound 238-3 (50.0 mg, 195.80 µmol) and 2-iodoylbenzoic acid (82.24 mg 293.71 µmol) were added sequentially in a glass vial, and the reaction solution was warmed to 80 °C and stirred at 80 °C for 2 h. A white solid compound 238-4 (35 mg, crude) was obtained by filtration and concentration.

### Step 4: (Compound 238-5) was prepared with reference to step 1 of Example 257. LC-MS: [M+H]+ =593.55

### Step 5: (Compound 238-6) LC-MS: [M+H]⁺ =493.45

238-5 (40.0 mg, 140.69 µmol) and a solution of dioxane hydrochloride were added sequentially in a glass vial, and the reaction solution was stirred at room temperature for 2 h. A white solid compound 238-6 (33 mg, crude) was obtained by concentration.

Step 6: (Compound 238) was prepared with reference to step 3 of Example 19.
¹H NMR (600 MHz, Methanol-d4) δ 7.97 (d, 1H), 7.71 (dd, *J =* 8.8, 0.8 Hz, 1H), 7.47 (d, *J* = 7.6 Hz, 1H), 7.38 (d, *J* = 7.9 Hz, 1H), 7.35 (d, *J* = 9.6 Hz, 1H), 7.22 (d, *J* = 2.4 Hz, 1H), 7.06 (dd, *J* = 8.7, 2.3 Hz, 1H), 5.17 (m, 1H), 4.89 (s, 2H), 4.75 (q, *J =* 3.8 Hz, 1H), 4.57 - 4.52 (m, 1H), 4.45 (q, 2H), 4.16 (d, *J =* 14.0 Hz, 2H), 4.00 (q, 1H), 3.93 (d, *J =* 12.7 Hz, 1H), 3.59 (q, *J =* 14.3, 11.7 Hz, 2H), 3.45 - 3.36 (m, 2H), 2.97 - 2.89 (m, 2H), 2.84 - 2.77 (m, 2H), 2.57 - 2.50 (m, 2H), 2.46 (d, *J =* 15.0 Hz, 2H), 2.25 - 2.21 (m, 2H), 2.20 - 2.16 (m, 2H), 2.16 - 2.08 (m, 4H), 2.08 - 2.02 (m, 2H), 1.78 - 1.72 (m, 2H), 1.71 - 1.61 (m, 5H). LC-MS: [M+H]⁺ =847.56

### Example 289

### Synthesis of compound 239

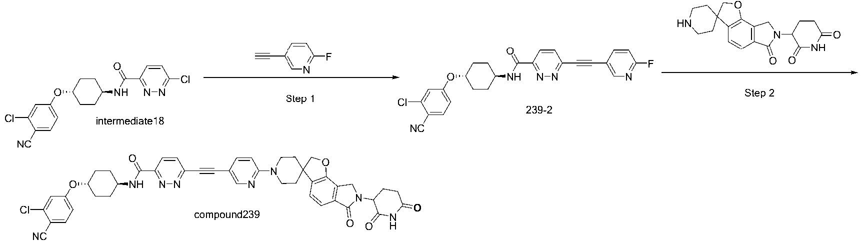

### Step 1: (Compound 239-2) LC-MS: [M+H]⁺ =457.09[M+H]

The intermediate 18 (50.0 mg, 127.79 µmol) and 5-ethynyl-2-fluoropyridine 5 (25.49 mg, 153.35 µmol) were added sequentially to a glass vial, and the solvent DMSO (2 mL), cuprous iodide (7.3 mg, 38.34 µmol), bis(triphenylphosphine)dichloropalladium (8.97 mg, 17.78 µmol) and cesium carbonate (12.91 mg, 383.38 µmol) were added, then heated up to 110 °C and stirred for 2 h. A white solid compound 239-2 (40 mg, crude) was obtained By preparative purification.

### Step 2: (Compound 239) was prepared with reference to step 3 of Example 19.

¹H NMR (600 MHz, Methanol-d4) δ 9.29 (d, *J* = 2.3 Hz, 1H), 9.01 (d, *J =* 8.7 Hz, 1H), 8.86 (d, *J =* 8.6 Hz, 1H), 8.68 (d, *J* = 8.8 Hz, 1H), 8.63 (d, J = 8.3 Hz, 1H), 8.23 (d, *J* = 7.6 Hz, 2H), 8.08 (d, *J* = 7.5 Hz, 1H), 7.96 (dd, *J* = 8.8, 2.4 Hz, 1H), 7.85 (d, *J* = 9.1 Hz, 1H), 5.91 (dd, *J =* 13.3, 5.1 Hz, 1H), 5.56 - 5.48 (m, 2H), 5.36 (d, *J* = 5.9 Hz, 1H), 5.27 (d, *J* = 6.7 Hz, 2H), 5.22 (d, *J =* 17.1 Hz, 1H), 5.06 (d, *J =* 17.1 Hz, 1H), 3.94 (t, *J =* 13.3 Hz, 2H), 3.77 - 3.68 (m, 1H), 3.45 - 3.38 (m, 1H), 3.29 - 3.18 (m, 2H), 2.98 - 2.91 (m, 2H), 2.84 - 2.78 (m, 2H), 2.77 - 2.71 ( m, 4H), 2.67 - 2.60 (m, 2H), 2.57 - 2.50 (m, 2H). LC-MS: [M+H]⁺ =811.46[M+H]

### Example 290

### Synthesis of compound 241

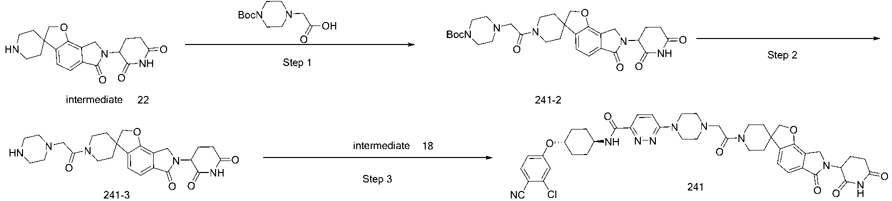

### Step 1: (Compound 241-2) LC-MS: [M+H]+ =582.58

The intermediate 22 (150 mg, 422.07 umol), 2-(4-(tert-butoxycarbonyl)piperazin-1-yl)acetic acid (154.66 mg, 633.1 umol), BOP (373.35 mg, 844.13 umol) and N,N-diisopropylethylamine (272.75 mg 2.11 mmol) were added sequentially to a glass vial containing N,N-dimethylformamide. The reaction solution was stirred at room temperature for 2 hours, prepared and purified to obtain compound 241-2 (144mg).

### Step 2: (Compound 241-3) was prepared with reference to step 2 of Example 39. LC-MS: [M+H]⁺ =482.39

### Step 3: (Compound 241) was prepared with reference to step 3 of Example 19. LC-MS: [M+H]⁺ =836.46

¹H NMR (600 MHz, Methanol-*d*₄) δ 8.06 (d, *J* = 9.5 Hz, 1H), 7.71 (d, *J =* 8.8 Hz, 1H), 7.47 (d, *J* = 9.5 Hz, 1H), 7.42 (d, *J =* 7.7 Hz, 1H), 7.38 (d, *J =* 7.7 Hz, 1H), 7.22 (d, *J =* 2.4 Hz, 1H), 7.06 (dd, *J* = 8.7, 2.4 Hz, 1H), 5.21 - 5.09 (m, 1H), 4.75 - 4.67 (m, 2H), 4.59 - 4.55 (m, 1H), 4.55 - 4.52 (m, 1H), 4.51 - 4.47 (m, 2H), 4.45 - 4.38 (m, 3H), 4.01-3.88 (m, 1H), 3.77 (d, *J =* 14.2 Hz, 1H), 3.60 (s, 4H), 3.35 (s, 2H), 3.32 (s, 2H), 3.06 - 2.98 (m, 1H), 2.96 - 2.88 (m, 1H), 2.84 - 2.77 (m, 1H), 2.58 - 2.46 (m, 1H), 2.22 (d, *J =* 11.5 Hz, 2H), 2.18 (d, *J =* 6.4 Hz, 1H), 2.15 - 2.10 (m, 2H), 2.10 - 2.03 (m, 1H), 2.02 - 1.95 (m, 1H), 1.91 (d, *J* = 12.1 Hz, 2H), 1.72 - 1.61 (m, 4H).,

### Example 291

### Synthesis of compound 242

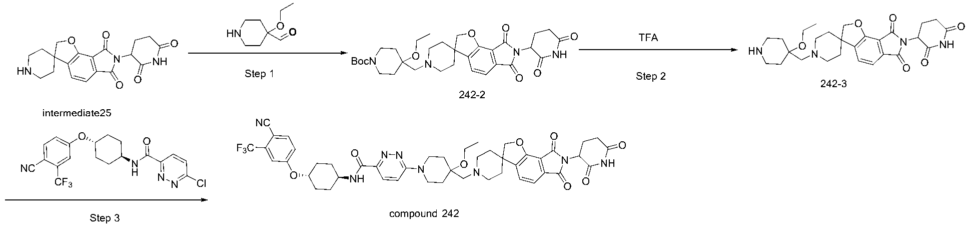

### Step 1: (Compound 242-2) was prepared with reference to step 1 of Example 257. LC-MS: [M+H]⁺ =611.58

### Step 2: (Compound 242-3) was prepared with reference to step 2 of Example 39. LC-MS: [M+H]⁺ =511.40

Step 3: (Compound 242) was prepared with reference to step 3 of Example 19.
¹ H NMR (600 MHz, DMSO-*d*₆) δ 11.11 (s, 1H), 8.63 (d, *J =* 8.2 Hz, 1H), 8.08 (d, *J =* 8.5 Hz, 1H), 7.88 (d, *J* = 9.6 Hz, 1H), 7.55 (d, *J* = 7.4 Hz, 1H), 7.52 (d, *J* = 7.4 Hz, 1H), 7.50 (s, 1H), 7.49 (s, 1H), 7.44 (d, *J =* 9.6 Hz, 1H), 5.14 - 5.07 (m, 1H), 4.83 (s, 1H), 4.66 - 4.60 (m, 1H), 4.30 - 4.21 (m, 1H), 3.40 - 3.33 (m, 4H), 3.31 - 3.20 (m, 4H), 2.94 - 2.83 (m, 1H), 2.64 - 2.57 (m, 1H), 2.55 - 2.52 (m, 1H), 2.40 - 2.32 (m, 2H), 2.31 - 2.22 (m, 1H), 2.16 - 2.10 (m, 2H), 2.05 - 2.02 (m, 1H), 2.02 - 1.95 (m, 4H), 1.95 - 1.88 (m, 2H), 1.71 - 1.61 (m, 4H), 1.59 - 1.50 (m, 2H), 1.48 - 1.42 (m, 1H), 1.32 - 1.27 (m, 1H), 1.26 - 1.23 (m, 2H), 1.20 (t, *J =* 6.8 Hz, 3H). LC-MS: [M+H]⁺ =899.65

### Example 292

### Synthesis of compound 243

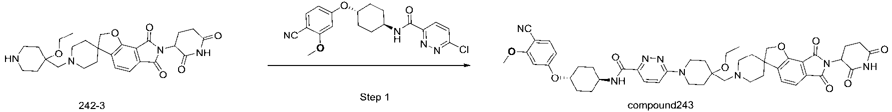

Step 1: (Compound 243) was prepared with reference to step 3 of Example 19.
¹H NMR (600 MHz, DMSO-*d*₆) δ 11.11 (s, 1H), 8.61 (d, *J =* 8.2 Hz, 1H), 7.88 (d, *J* = 9.5 Hz, 1H), 7.62 (d, *J* = 9.2 Hz, 1H), 7.56 - 7.49 (m, 2H), 7.44 (d, *J =* 9.6 Hz, 1H), 6.75 - 6.69 (m, 2H), 5.15 - 5.04 (m, 1H), 4.83 (s, 1H), 4.54 - 4.44 (m, 1H), 4.26 (d, *J =* 13.0 Hz, 2H), 3.90 (s, 3H), 3.87 - 3.82 (m, 2H), 3.57 - 3.49 (m, 2H), 3.42 - 3.32 (m, 4H), 3.31 - 3.20 (m, 2H), 2.94 - 2.83 (m, 1H), 2.64 - 2.57 (m, 1H), 2.54 - 2.50 (m, 1H), 2.41 - 2.31 (m, 2H), 2.16 - 2.09 (m, 2H), 2.06 - 2.02 (m, 1H), 2.01 - 1.95 (m, 4H), 1.94 - 1.87 (m, 2H), 1.71 - 1.60 (m, 4H), 1.52 (m,2H), 1.29 (d, *J =* 6.5 Hz, 1H), 1.26 - 1.23 (m, 2H), 1.20 (t, *J =* 6.8 Hz, 3H). LC-MS: [M+H]⁺ =861.86

### Example 293

### Synthesis of compound 244

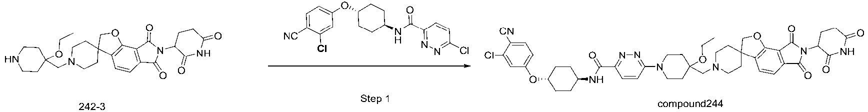

Step 1: (Compound 244) was prepared with reference to step 3 of Example 19.
¹H NMR (600 MHz, DMSO-*d*₆) δ 11.11 (s, 1H), 8.62 (d, *J =* 8.2 Hz, 1H), 7.89 - 7.85 (m, 2H), 7.56 - 7.49 (m, 2H), 7.44 (d, *J =* 9.6 Hz, 1H ), 7.39 (d, *J =* 2.4 Hz, 1H), 7.14 (dd, *J =* 8.8, 2.4 Hz, 1H), 5.14 - 5.07 (m, 1H), 4.83 (s, 2H), 4.57 - 4.50 (m, 1H), 4.26 (d, *J =* 12.9 Hz, 2H ), 3.65 (d, *J =* 12.2 Hz, 2H), 3.56 - 3.51 (m, 2H), 3.41 - 3.32 (m, 4H), 3.32 - 3.23 (m, 2H), 2.94 - 2.84 (m 1H), 2.63 - 2.57 (m, 1H), 2.56 - 2.51 (m, 1H), 2.40 - 2.33 (m, 2H), 2.15 - 2.07 (m, 2H), 2.05 - 2.03 (m, 1H), 2.01 - 1.94 (m, 4H), 1.94 - 1.88 (m, 2H), 1.70 - 1.60 (m, 4H), 1.57 - 1.49 ( m, 2H), 1.31 - 1.23 (m, 2H), 1.20 (t, *J =* 6.8 Hz, 3H). LC-MS: [M+H]⁺ =865.56

### Example 294

### Synthesis of compound 245

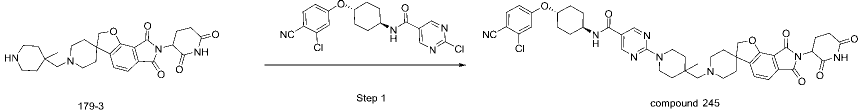

Step 1: (Compound 245) was prepared with reference to step 3 of Example 19.
¹H NMR (600 MHz, Methanol-*d*₄) *δ* 8.78 (s, 2H), 7.71 (d, *J =* 8.8 Hz, 1H), 7.59 (d, *J =* 7.4 Hz, 1H), 7.51 (d, *J =* 7.4 Hz, 1H), 7.21 (d, *J* = 2.4 Hz, 1H), 7.05 (dd, *J =* 8.8 , 2.4 Hz, 1H), 5.17 - 5.09 (m, 1H), 4.89 (s, 2H), 4.55 *(d, J=* 14.0 Hz, 2H), 4.51 - 4.43 (m, 1H), 3.98 - 3.90 (m, 1H), 3.77 (d, *J =* 12.9 Hz, 2H), 3.51 (dd, *J =* 14.0, 7.4 Hz, 2H), 3.25 (s, 2H), 2.93 - 2.67 (m, 2H), 2.42 (t, *J =* 14.1 Hz, 2H), 2.22 (dd, *J =* 13.5, 8.0 Hz, 2H), 2.16 - 2.12 (m, 2H), 2.11 - 2.03 (m, 4H), 1.73 - 1.67 (m, 4H), 1.65 - 1.55 (m, 4H), 1.39 (s, 3H). LC-MS: [M+H]⁺ =835.56

### Example 295

### Synthesis of compound 246

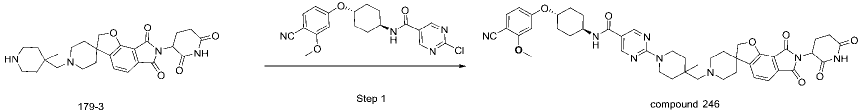

Step 1: (Compound 246) was prepared with reference to step 3 of Example 19.
¹H NMR (600 MHz, Methanol-*d*₄) δ 8.78 (s, 2H), 7.60 (d, *J* = 7.4 Hz, 1H), 7.54 - 7.48 (m, 2H), 6.69 - 6.63 (m, 2H), 5.16 - 5.06 (m, 1H), 4.89 (s, 2H), 4.55 (d, *J =* 14.0 Hz, 2H), 4.49 - 4.40 (m, 1H), 3.98 - 3.94 (m, 1H), 3.93 (s, 3H), 3.77 (d, *J =* 12.9 Hz, 2H), 3.62 - 3.46 (m, 2H), 3.24 (s, 2H), 2.93 - 2.66 (m, 4H), 2.47 - 2.37 (m, 2H), 2.24 (d, *J =* 11.1 Hz, 2H), 2.16 - 2.12 (m, 2H), 2.11 - 2.04 (m, 4H), 1.72 - 1.67 (m, 4H), 1.65 - 1.55 (m, 4H), 1.39 (s, 3H). LC-MS: [M+H]⁺ =831.56

### Example 296

### Synthesis of compound 247

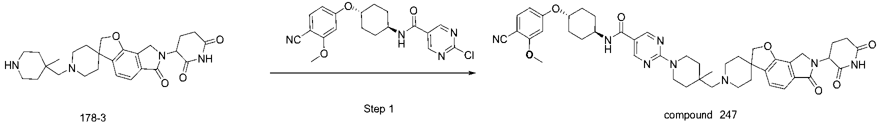

Step 1: (Compound 247) was prepared with reference to step 3 of Example 19.
¹H NMR (600 MHz, Methanol-*d*₄) δ 8.78 (d, *J =* 0.7 Hz, 2H), 7.52 (d, *J =* 8.3 Hz, 1H), 7.46 (d, *J =* 7.6 Hz, 1H), 7.35 (d, *J =* 7.7 Hz, 1H), 6.67 (d, *J* = 8.4 Hz, 2H), 5.19 - 5.14 (m, 1H), 4.89 (d, *J =* 0.8 Hz, 2H), 4.76 - 4.67 (m, 2H), 4.59 - 4.52 (m, 2H), 4.51 - 4.37 (m, 3H), 3.99 - 3.95 (m, 1H), 3.93 (s, 2H), 3.76 (d, *J =* 12.8 Hz, 1H), 3.56 - 3.47 (m, 2H), 3.34 - 3.30 (m, 1H), 3.24 (s, 1H), 2.95 - 2.88 (m, 1H), 2.84 - 2.77 (m, 1H), 2.57 - 2.50 (m, 1H), 2.48 - 2.39 (m, 2H), 2.27 - 2.21 (m, 2H), 2.20 - 2.15 (m, 1H), 2.11 - 2.02 (m, 4H), 1.72 - 1.66 (m, 4H), 1.61 (q, *J =* 13.5 Hz, 4H), 1.39 (s, 3H). LC-MS: [M+ H]⁺ =817.66

### Example 297

### Synthesis of compound 248

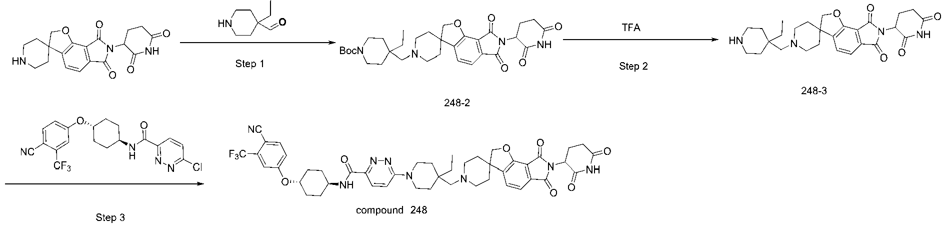

### Step 1: (Compound 248-2) was prepared with reference to step 1 of Example 257. LC-MS: [M+H]⁺ =595.68

### Step 2: (Compound 248-3) was prepared with reference to Step 2 of Example 39. LC-MS: [M+H]⁺ =495.40

Step 3: (Compound 248) was prepared with reference to step 3 of Example 19.
¹H NMR (600 MHz, Methanol-*d*₄) δ 7.96 (d, *J* = 9.6 Hz, 1H), 7.92 (d, *J =* 8.6 Hz, 1H), 7.59 (d, *J* = 7.0 Hz, 1H), 7.52 (d, *J =* 7.1 Hz, 1H), 7.40 (d, *J =* 2.4 Hz, 1H), 7.39 - 7.33 (m, 2H), 5.15 - 5.11 (m, 1H), 4.88 (s, 2H), 4.62 (s, 1H), 4.18 - 3.96 (m, 3H), 3.80 - 3.56 (m, 4H), 3.47 - 3.41 (m, 2H), 3.39 - 3.34 (m, 2H), 2.92 - 2.83 (m, 2H), 2.81 - 2.70 (m, 2H), 2.48 - 2.36 (m, 2H), 2.24 (s, 2H), 2.14 (s, 2H), 2.05 (s, 4H), 1.90 - 1.74 (m, 4H), 1.73 - 1.67 (m, 2H), 1.62 (d, *J =* 7.6 Hz, 3H), 1.05 (s, 2H). LC-MS: [M+H]⁺ = 883.56

### Example 298

### Synthesis of compound 249

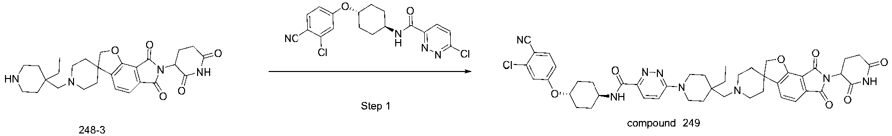

Step 1: (Compound 249) was prepared with reference to step 3 of Example 19.
¹H NMR (600 MHz, Methanol-*d*₄) δ 7.98 (d, *J* = 9.6 Hz, 1H), 7.71 (d, *J =* 8.8 Hz, 1H), 7.59 (d, *J =* 7.4 Hz, 1H), 7.52 (d, *J* = 7.3 Hz, 1H), 7.39 (d, *J* = 9.7 Hz, 1H), 7.22 (d, *J =* 2.4 Hz, 1H), 7.06 (dd, *J =* 8.8, 2.4 Hz, 1H), 5.13 (dd, *J =* 12.8, 5.4 Hz, 1H), 4.80 (s, 2H), 4.58 - 4.46 (m, 1H), 4.13 (d, *J =* 13.6 Hz, 2H), 4.01 (s, 1H), 3.76 (d, *J =* 13.1 Hz, 2H), 3.63 (t, *J =* 11.2 Hz, 2H), 3.51 (s, 2H), 3.43 - 3.35 (m, 2H), 2.93 - 2.84 (m, 2H), 2.81 - 2.66 (m, 2H), 2.44 (t, *J* = 14.0 Hz, 2H), 2.27 - 2.19 (m, 2H), 2.17 - 2.09 (m, 4H), 2.05 (s, 2H), 1.86 (s, 4H), 1.80 (d, *J =* 10.0 Hz, 2H), 1.69 - 1.64 (m, 3H), 1.63 (s, 2H). LC-MS: [M+H]⁺ =849.56

### Example 299

### Synthesis of compound 250

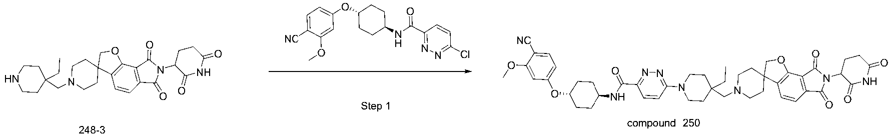

### Step 1: (Compound 250) was prepared with reference to step 3 of Example 19. LC-MS: [M+H]⁺ =845.66

¹H NMR (600 MHz, Methanol-*d*₄) δ 7.97 (d, *J* = 9.6 Hz, 1H), 7.59 (d, *J =* 7.4 Hz, 1H), 7.54 - 7.49 (m, 2H), 7.36 (d, *J* = 9.6 Hz, 1H), 6.71 - 6.65 (m, 2H), 5.15 - 5.09 (m, 1H), 4.80 (s, 2H), 4.51 (s, 1H), 4.13 (d, *J =* 14.4 Hz, 2H), 4.01 (s, 1H), 3.94 (s, 3H), 3.75 (s, 2H), 3.62 (t, *J =* 11.4 Hz, 2H), 3.50 (d, *J =* 17.8 Hz, 2H), 3.37 (d, *J =* 8.4 Hz, 2H), 2.93 - 2.84 (m, 2H), 2.78 - 2.69 (m, 2H), 2.43 (t, *J =* 14.1 Hz, 2H), 2.24 (d, *J* = 4.3 Hz, 2H), 2.21 (t, *J =* 7.7 Hz, 2H), 2.15 - 2.10 (m, 4H), 2.05 (d, *J =* 5.1 Hz, 2H), 1.85 (s, 4H), 1.79 (d, *J =* 10.4 Hz, 2H), 1.67 (t, *J =* 9.4 Hz, 3H), 1.63 (s, 2H).

### Example 300

### Synthesis of compound 251

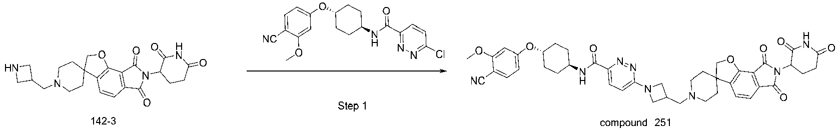

### Step 1: (Compound 251) was prepared with reference to step 3 of Example 19. LC-MS: [M+H]⁺ =789.56

¹H NMR (600 MHz, Methanol-*d*₄) δ 8.11 (d, *J* = 9.4 Hz, 1H), 7.64 (m, 2H), 7.52 (dd, *J* = 7.9, 4.2 Hz, 1H), 7.18 (d, *J* = 9.5 Hz, 1H), 6.73 - 6.55 (m, 2H), 5.13 (dd, *J =* 12.6, 5.5 Hz, 1H), 4.89 (s, 2H), 4.60 (t, *J =* 8.9 Hz, 1H), 4.50 (s, 2H), 4.20 (d, *J =* 8.7 Hz, 2H), 4.00 (s, 1H), 3.93 (s, 3H), 3.68 (s, 2H), 3.42 (d, *J =* 24.9 Hz, 2H), 2.96 - 2.83 (m, 2H), 2.83 - 2.71 (m, 2H), 2.68 (s, 2H), 2.38 - 2.28 (m, 2H), 2.26 - 2.17 (m, 3H), 2.16 - 2.08 (m, 2H), 2.08 - 2.01 (m, 2H), 1.71 - 1.53 (m, 4H).

### Example 301

### Synthesis of compound 252

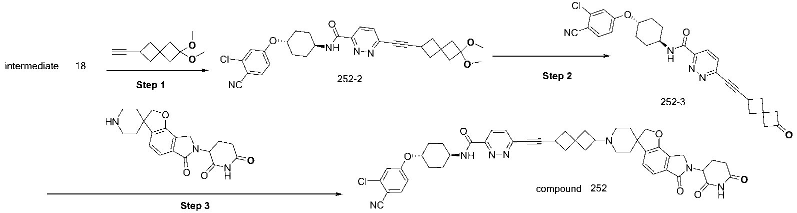

### Step 1: (Compound 252-2)

The intermediate 18 (50 mg, 0.128 mmol), 6-ethynyl-2,2-dimethoxyspiro[3.3]heptane (27.64 mg, 0.153 mmol), CuI (7.30 mg, 0.038 mmol), Cs₂CO₃ (125 mg, 0.383 mmol) and Pd(PPh₃) ₂Cl₂ (9 mg, 0.13 mmol) were added sequentially in a glass vial, then solvent DMF (1 mL) was added, and the reaction solution was stirred at 110 °C for 1 h. When the reaction was completed, it was diluted with water, extracted with EA and then concentrated, and then purified by a separation column (PE/EA=0-60%) to obtain a yellow solid compound 252-2 (45 mg, 65.81%).

Step 2: (Compound 252-3) was prepared with reference to step 2 of Example 267.

Step 3: (Compound 252) was prepared with reference to step 1 of Example 257.
¹H NMR (600 MHz, Methanol-d4) δ 8.23 (d, *J* = 8.7 Hz, 1H), 7.87 (d, *J =* 8.7 Hz, 1H), 7.69 (d, *J =* 8.8 Hz, 1H), 7.43 (d, *J =* 7.6 Hz, 1H), 7.35 (s, 1H), 7.20 (d, *J =* 2.4 Hz, 1H), 7.04 (dd, *J =* 8.8, 2.4 Hz, 1H), 5.14 (dd, J = 13.3, 5.1 Hz, 1H), 4.67 (s, 2H), 4.51 (ddd, J = 14.8, 9.7, 4.5 Hz, 1H), 4.47 - 4.37 (m, 2H), 4.08 - 3.99 (m, 1H), 3.63 (s, 1H), 3.54 (s, 2H), 3.44 - 3.37 (m, 1H), 2.91 (ddd, *J =* 23.2, 12.6, 5.7 Hz, 3H), 2.82 - 2.74 (m, 1H), 2.72 - 2.61 (m, 2H), 2.61 - 2.37 (m, 5H), 2.36 - 2.28 (m, 2H), 2.24 - 2.08 (m, 9H), 1.75 - 1.60 (m, 4H).LC-MS(ESI): [M+H]⁺ = 828.50

### Example 302

### Synthesis of compound 253

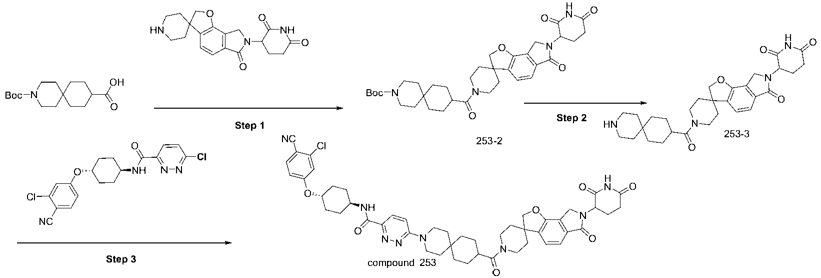

Step 1: (Compound 253-2) was prepared with reference to step 3 of Example 267.

Step 2: (Compound 253-3) was prepared with reference to step 2 of example 267.

Step 3: (Compound 253) was prepared with reference to step 3 of Example 19. LC-MS(ESI): [M+H]⁺ = 889.66
¹H NMR (600 MHz, DMSO-d6) δ 10.98 (s, 1H), 8.61 - 8.56 (m, 1H), 7.86 (d, *J =* 8.8 Hz, 1H), 7.80 (dd, *J =* 9.5, 4.9 Hz, 1H), 7.44 - 7.40 (m, 1H), 7.39 (s, 1H), 7.31 (dd, *J =* 9.6, 5.8 Hz, 1H), 7.27 (d, *J* = 7.6 Hz, 1H), 7.18 - 7.11 (m, 1H), 5.14 - 5.05 (m, 1H), 4.72 - 4.59 (m, 1H), 4.54 (dt, *J =* 10.2, 5.5 Hz, 1H), 4.39 (d, *J =* 16.9 Hz, 1H), 4.23 (dd, *J* = 17.0, 5.0 Hz, 1H), 3.98 (d, *J =* 16.0 Hz, 1H), 3.91 - 3.81 (m, 1H), 3.79 - 3.62 (m, 5H), 3.20 (t, *J =* 12.9 Hz, 1H), 2.91 (td, *J =* 16.1, 13.9, 5.4 Hz, 1H), 2.77 - 2.55 (m, 2H), 2.48 - 2.37 (m, 1H), 2.09 (d, *J =* 22.6 Hz, 2H), 2.03 - 1.94 (m, 1H), 1.90 (d, *J =* 11.3 Hz, 2H), 1.87 - 1.46 (m, 17H), 1.40 (s, 3H), 1.21 (dt, *J =* 33.0, 9.0 Hz, 3H).

### Example 303

### Synthesis of compound 254

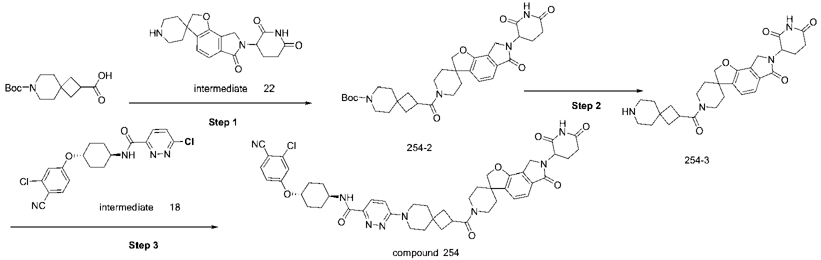

Step 1: (Compound 254) was prepared with reference to step 1 of Example 267.

Step 2: (Compound 254-3) was prepared with reference to step 2 of Example 39.

Step 3: (Compound 254) was prepared with reference to step 3 of Example 19.
¹H NMR (600 MHz, DMSO-d6) δ 10.98 (s, 1H), 8.58 (d, *J =* 8.2 Hz, 1H), 7.86 (d, *J =* 8.8 Hz, 1H), 7.80 (d, *J* = 9.5 Hz, 1H), 7.42 (d, *J =* 7.6 Hz, 1H), 7.39 (s, 1H), 7.35 (d, *J* = 9.7 Hz, 1H), 7.27 (d, *J =* 7.6 Hz, 1H), 7.17 - 7.11 (m, 1H), 5.12 - 5.06 (m, 1H), 4.69 - 4.59 (m, 2H), 4.53 (dt, *J =* 10.4, 6.1 Hz, 1H), 4.42 - 4.35 (m, 2H), 4.23 (dd, *J =* 17.1, 8.1 Hz, 1H), 3.86 (dq, *J =* 11.2, 7.9, 5.6 Hz, 1H), 3.80 - 3.58 (m, 5H), 3.40 (q, *J* = 8.4 Hz, 1H), 3.11 (t, *J* = 12.0 Hz, 1H), 2.96 - 2.86 (m, 1H), 2.74 (t, *J =* 12.4 Hz, 1H), 2.59 (dd, *J =* 14.4, 3.2 Hz, 1H), 2.48 - 2.37 (m, 1H), 2.08 (m, 5H), 1.99 (m, 7.2 Hz, 2H), 1.90 (d, J = 10.7 Hz, 2H), 1.71 (m, 8H), 1.57 - 1.47 (m, 4H). LC-MS(ESI): [M+H]⁺ = 861.76

### Example 304

### Synthesis of compound 255

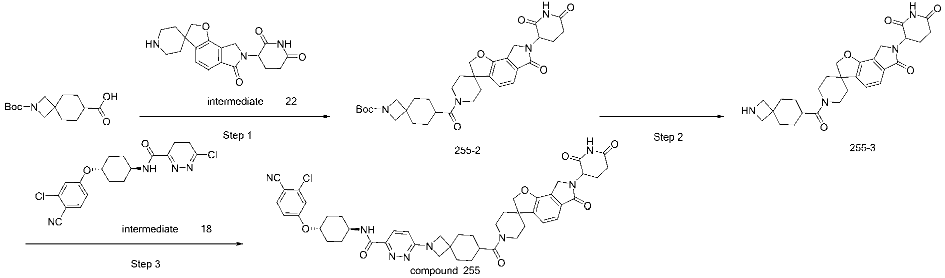

Step 1: (Compound 255-2) was prepared with reference to step 1 of Example 267.

Step 2: (Compound 255-3) was prepared with reference to step 2 of Example 39.

Step 3 (compound 255) was prepared with reference to step 3 of Example 19.
¹H NMR (600 MHz, DMSO-d6) δ 10.98 (s, 1H), 8.56 (d, *J =* 8.2 Hz, 1H), 7.85 (dd, *J =* 12.3, 9.0 Hz, 2H), 7.45 - 7.36 (m, 2H), 7.28 (d, *J =* 7.6 Hz, 1H), 7.14 (d, *J* = 11.1 Hz, 1H), 6.88 (d, *J* = 9.3 Hz, 1H), 5.13 - 5.07 (m, 1H), 4.70 - 4.59 (m, 2H), 4.54 (dt, *J =* 10.3, 6.1 Hz, 1H), 4.40 (d, *J =* 16.8 Hz, 2H), 4.23 (dd, *J =* 17.0, 6.2 Hz, 1H), 4.00 (d, J = 12.1 Hz, 1H), 3.86 (d, *J =* 22.8 Hz, 3H), 3.79 (s, 2H), 3.19 (s, 1H), 2.91 (t, *J =* 15.5 Hz, 1H), 2.70 (dd, *J =* 28.3, 12.1 Hz, 2H), 2.59 (d, *J =* 14.5 Hz, 1H), 2.49 - 2.38 (m, 1H), 2.10 (d, *J =* 10.1 Hz, 2H), 2.05 - 1.95 (m, 3H), 1.90 (d, *J* = 11.2 Hz, 3H), 1.80 - 1.71 (m, 3H), 1.66 - 1.58 (m, 5H), 1.51 (q, *J =* 12.4, 11.4 Hz, 3H), 1.41 (d, *J =* 12.9 Hz, 2H). LC-MS(ESI): [M+H]⁺ = 861.56

### Example 305

### Synthesis of compound 256

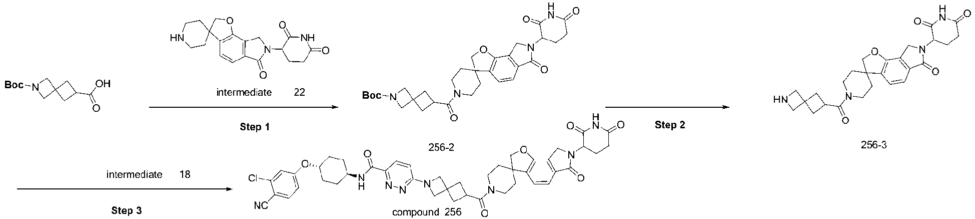

Step 1: (Compound 256-2) was prepared with reference to step 3 of Example 267.

Step 2: (Compound 256-3) was prepared with reference to the step 2 of Example 39.

Step 3: (Compound 256) was prepared with reference to step 3 of Example 19. LC-MS(ESI): [M+H]⁺ = 834.46
¹H NMR (600 MHz, Methanol-d4) δ 7.95 - 7.91 (m, 1H), 7.69 (d, *J =* 8.8 Hz, 1H), 7.68 - 7.62 (m, 1H), 7.38 (t, *J =* 4.9 Hz, 1H), 7.19 (d, *J =* 6.7 Hz, 1H), 7.06 - 7.01 (m, 1H), 6.93 - 6.87 (m, 1H), 5.17 - 5.10 (m, 1H), 4.66 (d, *J =* 11.8 Hz, 2H), 4.54 - 4.46 (m, 2H), 4.44 (s, 1H), 4.39 (dd, *J =* 16.9, 5.5 Hz, 1H), 4.30 (s, 1H), 4.17 (q, *J =* 9.2 Hz, 1H), 4.01 - 3.93 (m, 1H), 3.89 (d, *J =* 13.6 Hz, 1H), 3.44 (dd, *J =* 16.5, 8.3 Hz, 1H), 3.27 - 3.17 (m, 2H), 2.93 - 2.87 (m, 1H), 2.78 (dd, *J =* 17.1, 4.3 Hz, 1H), 2.68 - 2.62 (m, 1H), 2.58 (dd, *J =* 12.9, 9.0 Hz, 2H), 2.54 - 2.45 (m, 1H), 2.24 - 2.13 (m, 3H), 2.13 - 2.02 (m, 2H), 1.96 - 1.79 (m, 3H), 1.69 - 1.57 (m, 4H), 1.38 - 1.24 (m, 4H).

### Example 306

### Synthesis of compound 257

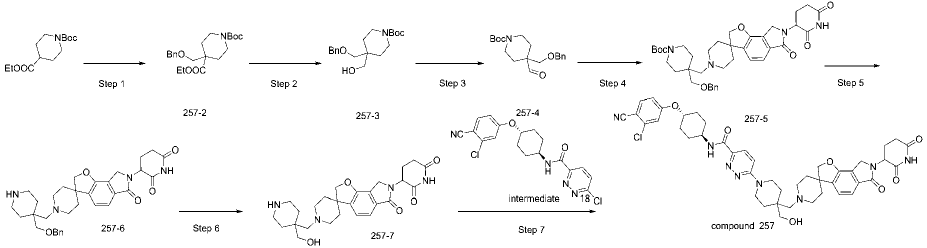

### Step 1: (Compound 257-2)

1-(tert-butyl)4-ethylpiperidine-1,4-dicarboxylate (5 g, 19.43 mmol) was added to a three-necked flask, the solvent THF (20 mL) was added and under the protection of nitrogen, the reaction solution was stirred at -78 °C for ten minutes. Then LDA (2.08 g, 19.43 mmol) was added to the flask, the reaction solution was continued to be stirred at -78 °C for half hour. BOM-Cl (3.04 g, 19.43 mmol) was slowly added to the flask, which was then brought to room temperature and stirred for another 2 h. After completion of the reaction, the reaction was quenched with a saturated solution of NH₄Cl, extracted with EA, concentrated, and purified by a separatory column (PE/EA=0-30%) to obtain a yellow oily compound 257-2 (6.07 g, 79.8%).

### Step 2: (Compound 257-3)

Compound 257-2 (3 g, 7.66 mmol) was added to the reaction vial, the solvent THF (100 mL) was added, LiAiH₄ (436.24 mg, 11.49 mmol) was added to the vial at 0 °C and the reaction solution was stirred at room temperature overnight. When the reaction was completed, it was quenched with distilled water, extracted with EA, concentrated and purified by separation column (PE/EA=0-40%) to obtain a colorless oily compound 257-3 (2.55 g, 99%).

Step 3: (Compound 257-4) was prepared with reference to step 3 of Example 211.

Step 4: (Compound 257-5) was prepared with reference to step 1 of Example 267.

Step 5: (Compound 257-6) was prepared with reference to step 2 of Example 267.

### Step 6: (Compound 257-7)

Compound 257-7 (30 mg, 0.052 mmol) and Pd(OH)₂/C (30 mg , 0.197 mmol) were added sequentially in a glass vial, the solvent HFIP (5 mL) was added, the hydrogen substitutionwas performed and the reaction solution was stirred at room temperature for 48 h. When the reaction was completed, the reaction solution was filtered and concentrated to obtain a white solid compound 257-7 (25 mg, 99%).

### Step 7: (Compound 257) was prepared with reference to step 3 of Example 19. LC-MS (ESI): [M+H]⁺ = 837.55

¹H NMR (600 MHz, Methanol-d4) δ 7.94 (d, *J* = 9.6 Hz, 1H), 7.69 (d, *J =* 8.7 Hz, 1H), 7.43 (d, *J* = 7.6 Hz, 1H), 7.39 (d, *J =* 7.6 Hz, 1H), 7.33 (d, *J =* 9.7 Hz, 1H), 7.20 (s, 1H), 7.04 (dd, *J =* 8.8, 2.2 Hz, 1H), 5.14 (dd, *J =* 13.3, 5.1 Hz, 1H), 4.74 - 4.67 (m, 1H), 4.49 (d, *J =* 11.9 Hz, 1H), 4.49 - 4.37 (m, 2H), 4.16 - 4.03 (m, 2H), 4.03 - 3.96 (m, 1H), 3.93 (s, 2H), 3.79 (d, *J =* 12.3 Hz, 2H), 3.68 - 3.60 (m, 2H), 3.55 (d, *J =* 9.7 Hz, 2H), 3.36 (s, 3H), 2.97 - 2.86 (m, 1H), 2.78 (d, *J =* 15.4 Hz, 1H), 2.65 (s, 0H), 2.58 - 2.44 (m, 1H), 2.43 - 2.31 (m, 2H), 2.20 (d, *J =* 6.6 Hz, 3H), 2.11 (s, 4H), 1.90 (d, *J* = 12.8 Hz, 2H), 1.74 (t, *J =* 12.0 Hz, 2H), 1.65 (p, *J =* 11.4, 10.3 Hz, 5H).

### Example 307

### Synthesis of compound 258

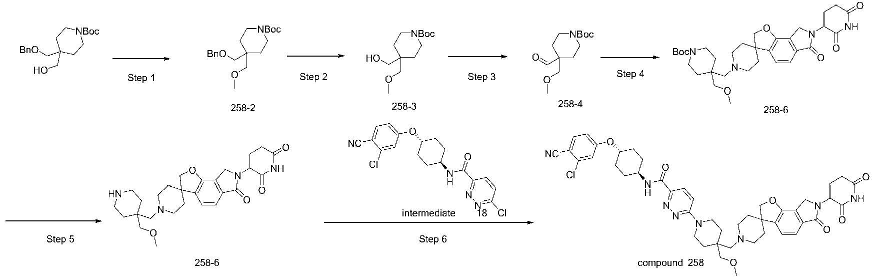

### Step 1: (Compound 258-2)

The compound 4-(benzyloxy)methyl)-4-(hydroxymethyl)piperidine-1-carboxylic acid tert-butyl ester (1 g, 2.98 mmol) was added to a glass vial, the solvent THF (10 mL) was added, and NaH (357.70 mg, 8.94 mmol) was added in an ice-water bath, and the reaction solution was continued to be stirred for half an hour, then iodomethane (846.28 mg, 5.96 mmol) was added and the reaction solution was stirred at room temperature overnight. When the reaction was completed, it was quenched with water, extracted by EA and purified by TLC (PE/EA=5/1) to obtain a colorless oily compound 258-2 (400 mg, 38.39%).

Step 2: (Compound 258-3) was prepared with reference to step 6 of Example 306.

Step 3: (Compound 258-4) was prepared with reference to step 1 of Example 257.

Step 4: (Compound 258-5) was prepared with reference to step 1 of Example 257.

Step 5: (Compound 258-6) was prepared with reference to step 2 of Example 267.

Step 6: (Compound 258) was prepared with reference to step 3 of Example 19.
¹H NMR (600 MHz, Methanol-d4) δ 7.90 (d, *J =* 9.6 Hz, 1H), 7.69 (d, *J =* 8.8 Hz, 1H), 7.41 - 7.33 (m, 2H), 7.28 (d, *J =* 9.7 Hz, 1H), 7.20 (d, *J = 2.3* Hz, 1H ), 7.04 (dd, *J =* 8.8, 2.3 Hz, 1H), 5.13 (dd, *J =* 13.4, 5.1 Hz, 1H), 4.59 (d, *J* = 8.9 Hz, 2H), 4.55 - 4.48 (m, 1H), 4.41 (q, *J =* 16.9 Hz, 2H), 3.98 (t, *J* = 9.9 Hz, 1H), 3.89 (dd, *J =* 8.9, 4.8 Hz, 2H), 3.73 - 3.65 (m, 2H), 3.46 (s, 2H), 3.36 (s, 2H), 3.04 - 2.85 (m, 3H), 2.77 (dd, *J =* 17.5, 4.3 Hz 1H), 2.65 - 2.42 (m, 5H), 2.25 - 2.01 (m, 8H), 1.79 - 1.61 (m, 10H).LC-MS(ESI): [M+H]⁺ = 852.56

### Example 308

### Synthesis of compound 259

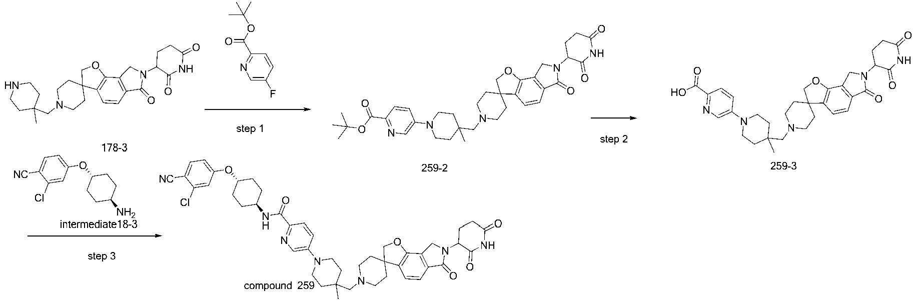

### Step 1: (Compound 259-2)

Compound 178-3 (440 mg, 0.876 mmol), tert-butyl 5-fluoropyridinecarboxylate (518.53 mg, 2.63 mmol) and DIEA (566.39 mg, 4.38 mmol) were added sequentially in a glass vial, the solvent DMF (10 mL) was added, and the reaction solution was stirred at 100 °C overnight. When the reaction was completed, a yellow solid compound 259-5 (40 mg, 7%) was obtained by HPLC (ACN/H₂O=5%-45%, 50 min, P: 24 MIN).

Step 2: (Compound 259-3) was prepared with reference to step 2 of Example 267.

Step 3: (Compound 259) was prepared with reference to step 3 of Example 267.
¹H NMR (600 MHz, Methanol-d4) δ 8.30 (s, 1H), 7.91 (d, *J =* 8.8 Hz, 1H), 7.71 - 7.66 (m, 1H), 7.42 - 7.33 (m, 3H), 7.20 (s, 1H), 7.06 - 7.02 (m, 1H), 5.17 - 5.10 (m, 1H), 4.62 (s, 2H), 4.54 - 4.48 (m, 1H), 4.46 - 4.37 (m, 2H), 3.99 - 3.90 (m, 1H), 3.67 (d, *J =* 12.8 Hz, 2H), 3.25 (t, *J =* 10.9 Hz, 3H), 2.90 (m, 2H), 2.77 (d, *J =* 17.5 Hz, 2H), 2.49 (m, 1H), 2.17 (dd, *J =* 22.4, 8.1 Hz, 5H) , 2.08 (d, *J* = 9.5 Hz, 2H), 1.87 (s, 1H), 1.79 (t, *J =* 12.1 Hz, 2H), 1.63 (m, 7H), 1.40 - 1.27 (m, 3H), 1.19 (s, 3H). LC-MS(ESI): [M+H]⁺ = 820.56

### Example 309

### Synthesis of compound 260

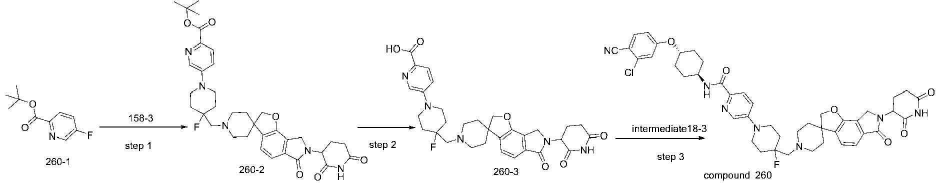

### Step 1: (Compound 260-2)

Compound 260-1 (350 mg, 0.743 mmol), tert-butyl 5-fluoropyridinecarboxylate (440.06 mg, 2.23 mmol) and DIEA (480.68 mg, 3.72 mmol) were added sequentially in a glass vial, the solvent DMF (10 mL) was added, and the reaction solution was stirred at 100 °C overnight. When the reaction was completed, a yellow solid compound 260-2 (42 mg, 8.72%) was obtained by HPLC (ACN/H₂O=5%-55%, 40 min, P: 22 MIN).

Step 2: (Compound 260-3) was prepared with reference to step 2 of Example 39.

### Step 3: (Compound 260) was prepared with reference to step 3 of Example 267. LC-MS(ESI): [M+H]⁺ = 824.56

¹H NMR (600 MHz, Methanol-d4) δ 8.36 (s, 1H), 7.95 (d, *J =* 8.8 Hz, 1H), 7.69 (d, *J =* 8.7 Hz, 1H), 7.48 (d, *J =* 11.1 Hz, 1H), 7.44 (d, *J =* 7.5 Hz, 1H), 7.37 (s, 1H), 7.20 (s, 1H), 7.04 (d, *J =* 10.3 Hz, 1H), 5.14 (dd, *J =* 13.4, 5.2 Hz, 1H), 4.71 (s, 2H), 4.54 - 4.38 (m, 3H), 3.93 (d, *J =* 12.4 Hz, 3H), 3.79 (s, 2H), 3.68 - 3.53 (m, 2H), 2.90 (m, 1H), 2.83 - 2.75 (m, 1H), 2.72 - 2.68 (m, 1H), 2.50 (m, 1H), 2.45 - 2.33 (m, 2H), 2.23 - 2.14 (m, 5H), 2.14 - 1.91 (m, 7H), 1.63 (m, 4H), 1.40 - 1.26 (m, 2H).

### Example 310

### Synthesis of compound 265

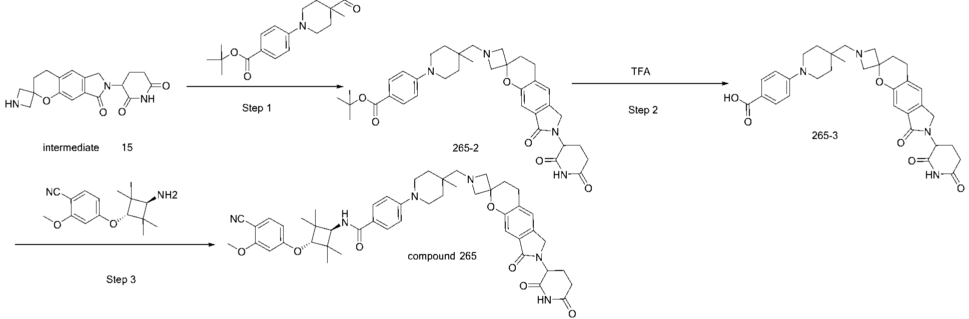

### Step 1: (Compound 265-2) was prepared with reference to step 1 of Example 267. LC-MS: [M+H]⁺ =629.58

### Step 2: (Compound 265-3) was prepared with reference to step 2 of Example 267. LC-MS: [M+H]⁺ =573.48

Step 3: (Compound 265) was prepared with reference to step 3 of Example 267.
¹H NMR (600 MHz, DMSO-d6) δ 10.98 (d, *J =* 2.4 Hz, 1H), 7.77 (d, *J =* 8.5 Hz, 2H), 7.66 (d, *J =* 8.6 Hz, 1H), 7.51 (dd, *J* = 9.3, 1.9 Hz, 1H), 7.38 (s, 1H), 7.13 (d, *J* = 23.9 Hz, 1H), 7.03 - 6.92 (m, 2H), 6.64 (d, *J = 2.2* Hz, 1H), 6.54 (dd, *J =* 8.7, 2.2 Hz, 1H), 5.08 (ddd, J = 13.3, 5.2, 2.4 Hz, 1H), 4.61 - 4.55 (m, 1H), 4.53 - 4.47 (m, 1H), 4.44 - 4.30 (m, 3H), 4.28 (s, 1H), 4.23 (d, *J =* 16.7 Hz, 1H), 4.06 (d, *J = 9.2* Hz, 1H), 3.91 (s, 3H), 3.42 (d, *J =* 4.8 Hz, 2H), 3.33 (d, *J* = 5.5 Hz, 1H), 3.17 - 3.09 (m, 2H), 2.92 (m, 3H), 2.60 (d, *J =* 15.7 Hz, 1H), 2.43 - 2.16 (m, 4H), 2.00 (m, 1H), 1.67 - 1.55 (m, 2H), 1.48 (m, 2H), 1.23 (s, 6H), 1.15 (s, 6H), 1.10 (d, J = 3.1 Hz, 3H). LC-MS: [M+H]⁺ =829.66

### Example 311

### Synthesis of compound 266

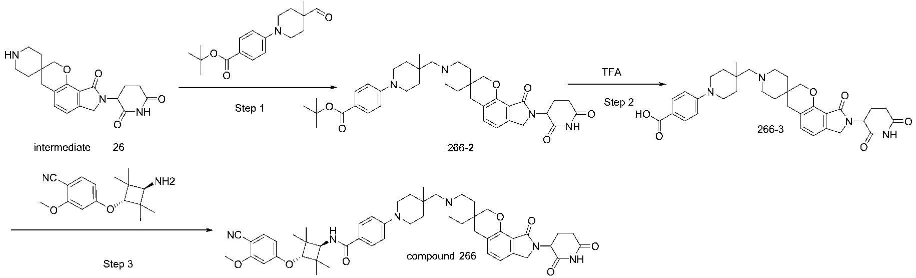

### Step 1: (Compound 266-2) was prepared with reference to step 1 of Example 267. LC-MS: [M+H]⁺ =657.57

### Step 2: (Compound 266-3) was prepared with reference to step 2 of Example 267. LC-MS: [M+H]⁺ =601.48

Step 3: (Compound 266) was prepared with reference to step 3 of Example 267.
¹H NMR (600 MHz, DMSO-d6) δ 10.96 (s, 1H), 7.77 (d, *J =* 8.6 Hz, 2H), 7.66 (d, *J =* 8.6 Hz, 1H), 7.51 (d, *J* = 9.2 Hz, 1H), 7.31 (dd, *J =* 24.3, 7.7 Hz, 1H), 7.06 (t, *J =* 6.9 Hz, 1H), 7.00 (d, *J =* 8.5 Hz, 2H), 6.64 (d, *J* = 2.1 Hz, 1H), 6.54 (dd, J = 8.7, 2.2 Hz, 1H), 5.05 - 4.99 (m, 1H), 4.33 (d, *J =* 17.2 Hz, 1H), 4.28 (s, 1H), 4.28 - 4.21 (m, 1H), 4.20 (dd, *J =* 17.4, 4.3 Hz, 1H), 4.06 (d, *J* = 9.2 Hz, 1H), 3.98 (s, 1H), 3.91 (s, 3H), 3.56 (s, 2H), 3.30 (m, 1H),3.18 - 3.10 (m, 4H), 2.99 - 2.84 (m, 3H), 2.68 (s, 1H), 2.63 - 2.56 (m, 1H), 2.42 - 2.30 (m, 2H), 2.04 - 1.92 (m, 2H), 1.90 - 1.79 (m, 2H), 1.78 - 1.72 (m, 1H), 1.73 - 1.63 (m, 2H), 1.60 (m, 3H), 1.23 (s, 6H), 1.20 (s, 3H), 1.15 (s, 6H). LC-MS: [M+H]⁺ =857.76

### Example 312

### Synthesis of compound 267

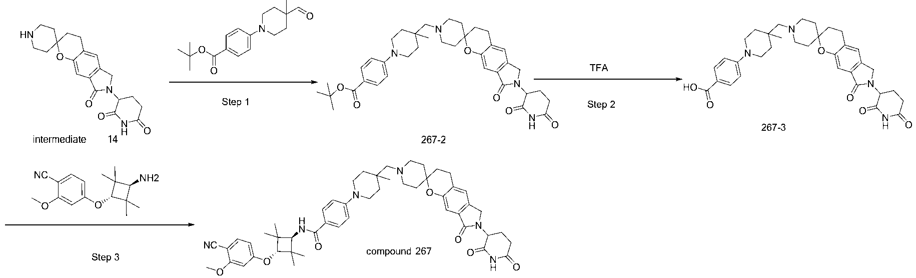

### Step 1: (Compound 267-2) was prepared with reference to step 1 of Example 267. LC-MS: [M+H]⁺ =657.47

### Step 2: (Compound 267-3) was prepared with reference to step 2 of Example 267. LC-MS: [M+H]⁺ =601.48

### Step 3: (Compound 267) was prepared with reference to step 3 of Example 267. LC-MS: [M+H]⁺ =857.66

¹H NMR (600 MHz, DMSO-d6) δ 10.98 (s, 1H), 7.77 (d, *J =* 8.4 Hz, 2H), 7.65 (d, *J =* 8.6 Hz, 1H), 7.52 (d, *J* = 9.3 Hz, 1H), 7.37 (d, *J =* 4.5 Hz, 1H), 7.26 (s, 1H), 7.00 (d, *J =* 8.6 Hz, 2H), 6.64 (d, *J* = 2.1 Hz, 1H), 6.54 (dd, *J* = 8.6, 2.1 Hz, 1H), 5.06 (dd, *J =* 13.2, 5.3 Hz, 1H), 4.35 (d, *J =* 16.6 Hz, 1H), 4.28 (s, 1H), 4.22 (dd, *J =* 16.7, 4.3 Hz, 1H), 4.06 (d, *J =* 9.2 Hz, 1H), 3.91 (s, 3H), 3.63 - 3.52 (m, 3H), 3.39 (m, 3), 3.24 - 3.18 (m, 2H), 3.14 (d, *J =* 10.1 Hz, 2H), 2.95 - 2.85 (m, 3H), 2.62 (d, *J =* 3.9 Hz, 1H), 2.39 (m, 1H), 2.18 - 2.02 (m, 2H), 2.03 - 1.84 (m, 5H), 1.68 (m, 2H), 1.63 - 1.52 (m, 2H), 1.22 (d, *J =* 8.2 Hz, 9H), 1.15 (s, 6H).

### Example 313

### Synthesis of compound 268

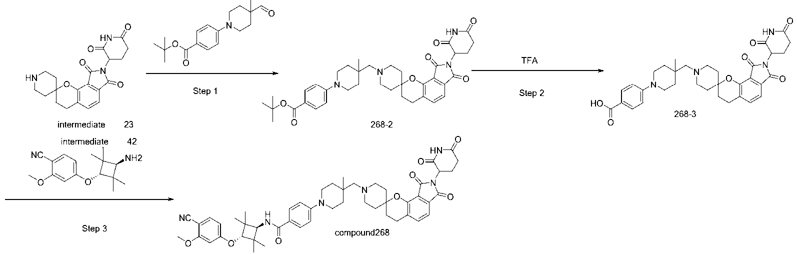

### Step 1: (Compound 268-2) was prepared with reference to step 1 of Example 267. LC-MS: [M+H]⁺ =671.57

### Step 2: (Compound 268-3) was prepared with reference to step 2 of Example 267. LC-MS: [M+H]⁺ =615.48

### Step 3: (Compound 268) was prepared with reference to step 3 of Example 267. LC-MS: [M+H]⁺ =871.66

¹H NMR (600 MHz, DMSO-d6) δ 11.11 (d, *J =* 5.7 Hz, 1H), 7.77 (d, *J =* 8.3 Hz, 2H), 7.65 (t, *J =* 9.8 Hz, 2H), 7.54 - 7.49 (m, 1H), 7.41 (dd, *J* = 7.4, 2.8 Hz, 1H), 7.00 (d, *J =* 8.7 Hz, 2H), 6.64 (d, *J* = 2.1 Hz, 1H), 6.54 (dd, *J =* 8.6, 2.2 Hz, 1H), 5.08 (m, 1H), 4.28 (s, 1H), 4.06 (d, *J* = 9.2 Hz, 1H), 3.91 (s, 3H), 3.58 (s, 4H), 3.25 (d, J = 22.8 Hz, 4H), 3.13 (d, J = 6.5 Hz, 2H), 2.98 - 2.85 (m, 3H), 2.61 (d, *J =* 20.4 Hz, 1H), 2.28 (t, *J =* 9.6 Hz, 1H), 2.22 - 2.11 (m, 2H), 2.09 - 1.92 (m, 5H), 1.74 - 1.64 (m, 2H), 1.59 (dd, *J =* 22.4, 13.7 Hz, 2H), 1.21 (d, *J =* 24.3 Hz, 9H), 1.15 (s, 6H).

### Example 314

### Synthesis of compound 269

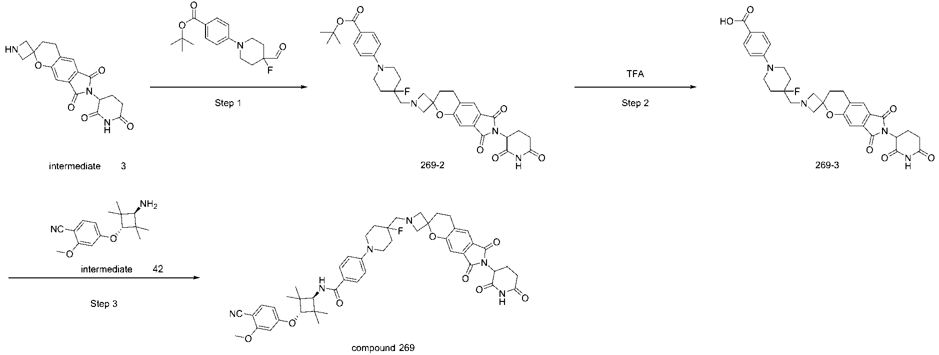

### Step 1: (Compound 269-2) was prepared with reference to step 1 of Example 267. LC-MS: [M+H]⁺ =647.47

### Step 2: (Compound 269-3) was prepared with reference to step 2 of Example 267. LC-MS: [M+H]⁺ =591.40

Step 3: (Compound 269) was prepared with reference to step 3 of Example 267.
¹H NMR (600 MHz, DMSO-*d*₆) δ 11.10 (s, 1H), 7.76 (d, *J =* 8.8 Hz, 2H), 7.70 (s, 1H), 7.65 (d, *J =* 8.6 Hz, 1H), 7.52 (d, *J* = 9.2 Hz, 1H), 7.25 (s, 1H), 7.00 (d, *J =* 8.9 Hz, 2H), 6.64 (d, *J* = 2.1 Hz, 1H), 6.54 (dd, *J =* 8.6, 2.1 Hz, 1H), 5.09 (dd, *J =* 12.9, 5.5 Hz, 1H), 4.29 (d, *J =* 13.9 Hz, 1H), 4.05 (d, *J =* 9.2 Hz, 1H), 3.91 (s, 3H), 3.65 ( d, *J =* 13.0 Hz, 2H), 3.48 (t, *J =* 10.6 Hz, 2H), 3.29 (d, *J =* 8.1 Hz, 3H), 3.08 (t, *J =* 11.0 Hz, 2H), 2.95 (t, *J =* 6.3 Hz, 2H), 2.93 - 2.84 (m, 1H), 2.76 (d, *J =* 22.7 Hz, 2H), 2.64 - 2.57 (m, 1H), 2.14 (t, *J =* 6.4 Hz, 2H), 2.06 - 1.99 (m, 1H), 1.93 - 1.84 (m, 2H), 1.80 - 1.66 (m, 2H), 1.23 (s, 6H), 1.15 (s, 6H). LC-MS: [M+H]⁺ =847.66

### Example 315

### Synthesis of compound 270

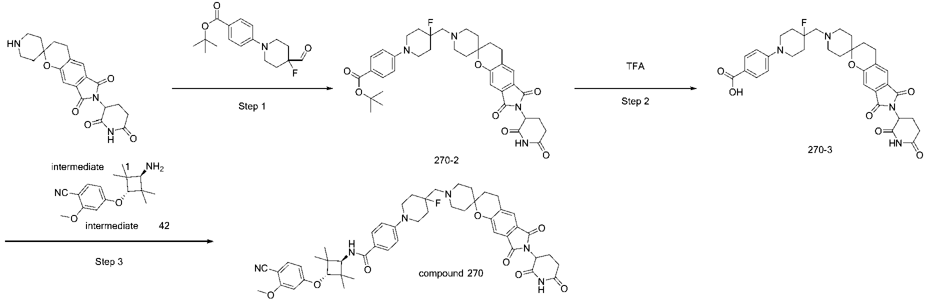

### Step 1: (Compound 270-2) was prepared with reference to step 1 of Example 267. LC-MS: [M+H]⁺ =675.47

### Step 2: (Compound 270-3) was prepared with reference to step 2 of Example 267. LC-MS: [M+H]⁺ =619.40

Step 3: (Compound 270) was prepared with reference to step 3 of Example 267.
¹H NMR (600 MHz, DMSO-*d*₆) δ 11.10 (s, 1H), 7.76 (d, *J =* 8.8 Hz, 2H), 7.70 (s, 1H), 7.65 (d, *J =* 8.6 Hz, 1H), 7.52 (d, *J* = 9.2 Hz, 1H), 7.22 (s, 1H), 7.01 (d, *J =* 9.0 Hz, 2H), 6.64 (d, *J* = 2.1 Hz, 1H), 6.54 (dd, *J =* 8.7, 2.2 Hz, 1H), 5.09 (dd, *J =* 12.9, 5.4 Hz, 1H), 4.28 (s, 1H), 4.06 (d, *J =* 9.2 Hz, 1H), 3.91 (s, 3H), 3.65 (d, *J =* 12.8 Hz, 2H), 3.12 (t, *J* = 10.9 Hz, 2H), 2.93 - 2.84 (m, 3H), 2.67 (s, 2H), 2.64 - 2.52 (m, 6H), 2.06 - 1.99 (m, 1H), 1.95 - 1.88 (m, 2H), 1.85 (t, *J* = 6.7 Hz, 2H), 1.82 - 1.64 (m, 6H), 1.24 (s, 6H), 1.15 (s, 6H).LC-MS: [M+H]⁺ =875.76

### Example 316

### Synthesis of compound 271

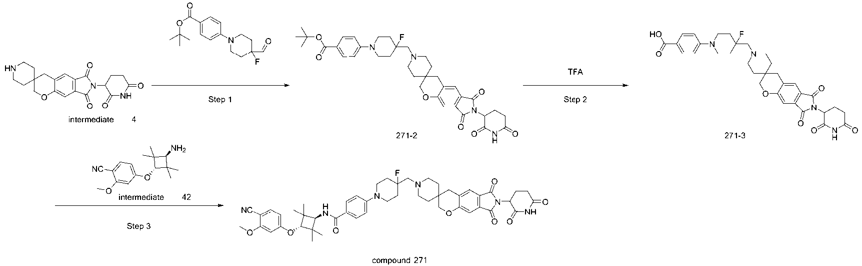

### Step 1: (Compound 271-2) was prepared with reference to step 1 of Example 267. LC-MS: [M+H]⁺ =675.57

### Step 2: (Compound 271-3) was prepared with reference to step 2 of Example 267. LC-MS: [M+H]⁺ =619.40

Step 3: (Compound 271) was prepared with reference to step 3 of Example 267.
¹H NMR (600 MHz, DMSO-*d*₆) δ 11.10 (s, 1H), 7.76 (d, *J =* 8.1 Hz, 2H), 7.72 - 7.64 (m, 2H), 7.52 (d, *J =* 8.9 Hz, 1H), 7.33 - 7.22 (m, 1H), 7.08 - 6.96 (m, 2H), 6.64 (d, *J* = 2.1 Hz, 1H), 6.54 (dd, *J =* 8.7, 2.2 Hz, 1H), 5.09 (dd, *J =* 12.7, 4.9 Hz, 1H), 4.28 (s, 1H), 4.06 (d, *J =* 8.7 Hz, 3H), 3.91 (s, 3H), 3.79 (s, 1H), 3.67 - 3.53 (m, 2H), 3.47 (s, 1H), 3.12 (t, *J* = 11.7 Hz, 2H), 2.94 - 2.77 (m, 3H), 2.66 - 2.52 (m, 4H), 2.09 - 1.97 (m, 2H), 1.95 - 1.68 (m, 5H), 1.64 (s, 1H), 1.44 (d, *J =* 57.2 Hz, 3H), 1.23 (s, 6H), 1.15 (s, 6H).LC-MS: [M+H]⁺ = 875.66

### Example 317

### Synthesis of compound 272

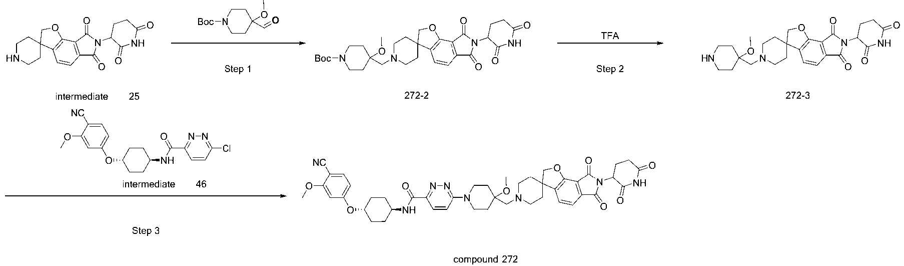

### Step 1: (Compound 272-2) was prepared with reference to step 1 of Example 267. LC-MS: [M+H]⁺ =597.48

### Step 2: (Compound 272-3) was prepared with reference to step 2 of Example 267. LC-MS: [M+H]⁺ =497.40

Step 3: (Compound 272) was prepared with reference to step 3 of Example 19.
¹H NMR (600 MHz, DMSO-*d*₆) δ 11.10 (s, 1H), 8.61 (d, *J =* 8.2 Hz, 1H), 7.83 (d, *J =* 9.6 Hz, 1H), 7.71 (d, *J =* 7.4 Hz, 1H), 7.63 - 7.60 (m, 1H), 7.38 (dd, *J =* 17.3, 8.5 Hz, 2H), 6.75 - 6.70 (m, 2H), 5.09 (dd, *J =* 12.9, 5.4 Hz, 1H), 4.68 (s, 2H), 4.55 - 4.46 (m, 1H), 4.15 (d, *J =* 12.8 Hz, 2H), 3.92 - 3.83 (m, 4H), 3.30 (s, 2H), 3.20 (s, 3H), 2.95 (d, *J* = 11.6 Hz, 2H), 2.92 - 2.83 (m, 1H), 2.62 - 2.55 (m, 1H), 2.55 - 2.52 (m, 1H), 2.41 (d, *J* = 27.8 Hz, 2H), 2.26 - 2.16 (m, 2H), 2.13 (d, *J =* 10.1 Hz, 2H), 2.05 - 1.98 (m, 1H), 1.89 (d, *J =* 13.1 Hz, 6H), 1.71 - 1.56 (m, 6H), 1.56 - 1.46 (m, 2H).LC-MS: [M+H]⁺ =847.66

### Example 318

### Synthesis of compound 273

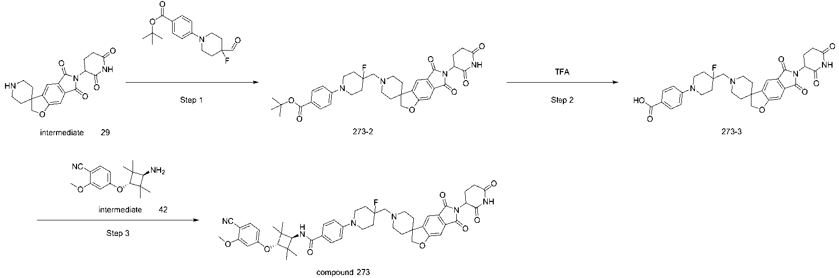

### Step 1: (Compound 273-2) was prepared with reference to step 1 of Example 267. LC-MS: [M+H]⁺ =661.47

### Step 2: (Compound 273-3) was prepared with reference to step 2 of Example 267. LC-MS: [M+H]⁺ =605.38

Step 3: (Compound 273) was prepared with reference to step 3 of Example 267.
¹H NMR (600 MHz, DMSO-*d*₆) δ 11.10 (s, 1H), 7.84 (s, 1H), 7.77 (d, *J =* 8.8 Hz, 2H), 7.65 (d, *J =* 8.6 Hz, 1H), 7.52 (d, *J* = 9.2 Hz, 1H), 7.28 (s, 1H), 7.03 (d, *J =* 9.0 Hz, 2H), 6.65 (d, *J* = 2.1 Hz, 1H), 6.55 (dd, *J =* 8.6, 2.2 Hz, 1H), 5.10 (dd, *J =* 12.9, 5.5 Hz, 1H), 4.63 (s, 2H), 4.28 (s, 1H), 4.06 (d, *J* = 9.2 Hz, 1H), 3.91 (s, 3H), 3.67 (d, *J =* 12.9 Hz, 2H), 3.13 (t, *J =* 11.0 Hz, 2H), 2.97 - 2.83 (m, 3H), 2.64 - 2.52 (m, 4H), 2.22 (t, *J =* 11.6 Hz, 2H), 2.07 - 1.92 (m, 5H), 1.86 - 1.73 (m, 2H), 1.67 (d, *J =* 12.6 Hz, 2H), 1.23 (s, 6H), 1.15 (s, 6H).LC-MS: [M+H]⁺ =861.55

### Example 319

### Synthesis of compound 274

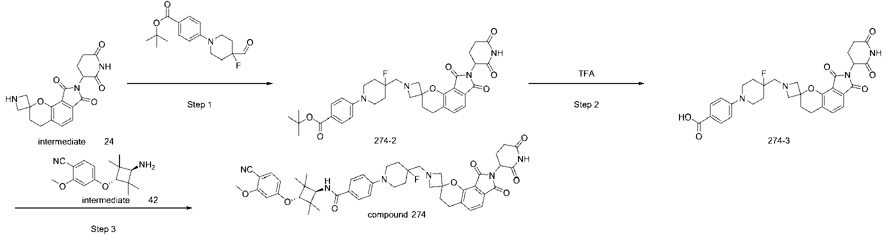

### Step 1: (Compound 274-2) was prepared with reference to step 1 of Example 267. LC-MS: [M+H]⁺ =647.47

### Step 2: (Compound 274-3) was prepared with reference to step 2 of Example 267. LC-MS: [M+H]⁺ =591.38

Step 3: (Compound 274) was prepared with reference to step 3 of Example 267.
¹H NMR (600 MHz, DMSO-*d*₆) δ 11.11 (s, 1H), 7.77 (d, *J =* 7.5 Hz, 2H), 7.64 (dd, *J =* 17.6, 12.6 Hz, 2H), 7.52 (d, *J* = 9.2 Hz, 1H), 7.38 (s, 1H), 7.02 (d, *J =* 6.6 Hz, 2H), 6.64 (d, *J* = 2.1 Hz, 1H), 6.54 (dd, *J =* 8.6, 2.2 Hz, 1H), 5.13 - 5.02 (m, 1H), 4.72 - 4.51 (m, 2H), 4.51 - 4.30 (m, 2H), 4.28 (s, 1H), 4.06 (d, *J =* 9.2 Hz, 1H), 3.91 (s, 3H), 3.77 (d, *J* = 20.8 Hz, 4H), 3.14 - 3.04 (m, 2H), 3.00 - 2.86 (m, 3H), 2.64 - 2.57 (m, 1H), 2.37 - 2.11 (m, 3H), 2.07 - 1.99 (m, 1H), 1.92 (s, 2H), 1.30 - 1.08 (m, 12H). LC-MS: [M+H ]⁺ =847.66

### Example 320

### Synthesis of compound 275

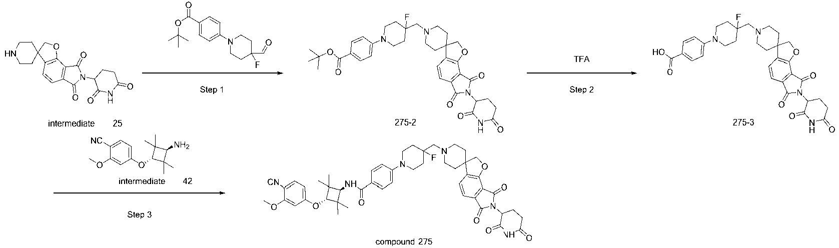

### Step 1: (Compound 275-2) was prepared with reference to step 1 of Example 267. LC-MS: [M+H]⁺ =661.47

### Step 2: (Compound 275-3) was prepared with reference to step 2 of Example 267. LC-MS: [M+H]⁺ =605.48

### Step 3: (Compound 275) was prepared with reference to step 3 of Example 267. LC-MS: [M+H]⁺ =861.66

¹ H NMR (600 MHz, DMSO-*d*₆) δ 11.10 (s, 1H), 7.78 (s, 2H), 7.66 (d, *J =* 8.6 Hz, 1H), 7.53 (d, *J =* 8.8 Hz, 2H), 7.42 (s, 1H), 7.04 (s, 2H), 6.65 (d, *J* = 2.0 Hz, 1H), 6.55 (dd, *J =* 8.6, 2.1 Hz, 1H), 5.14 - 5.06 (m, 1H), 4.83 (s, 1H), 4.69 (s, 1H), 4.28 (s, 1H), 4.07 (d, *J =* 9.2 Hz, 1H), 3.92 (s, 3H), 3.82 (s, 1H), 3.73 - 3.50 (m, 3H), 3.14 (t, *J =* 11.4 Hz, 2H), 2.99 - 2.83 (m, 2H), 2.65 - 2.54 (m, 2H), 2.36 - 2.15 (m, 3H), 2.11 - 1.87 (m, 6H), 1.87 - 1.66 (m, 3H), 1.20 (d, *J* = 46.3 Hz, 12H).

### Example 321

### Synthesis of compound 276

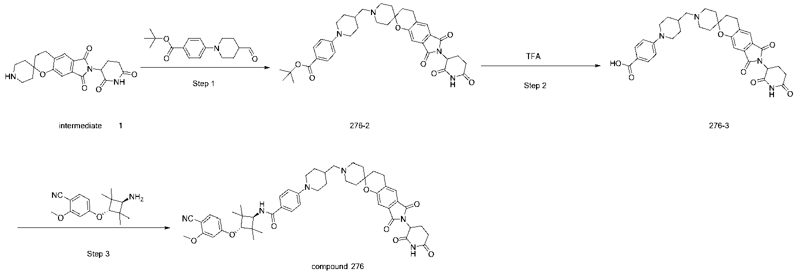

### Step 1: (Compound 276-2) was prepared with reference to step 1 of Example 267. LC-MS: [M+H]⁺ =657.47

### Step 2: (Compound 276-3) was prepared with reference to step 2 of Example 267. LC-MS: [M+H]⁺ =601.69

### Step 3: (Compound 276) was prepared in reference to step 3 of Example 267. LC-MS: [M+H]⁺ =857.66

¹H NMR (600 MHz, DMSO-*d*₆) δ 11.11 (s, 1H), 7.79 - 7.70 (m, 3H), 7.65 (d, *J =* 8.6 Hz, 1H), 7.51 (d, *J* = 9.2 Hz, 1H), 7.26 (s, 1H), 6.97 (d, *J =* 8.4 Hz, 2H), 6.64 (s, 1H), 6.54 (d, *J =* 8.6 Hz, 1H), 5.09 (dd, *J =* 12.9, 5.5 Hz, 1H), 4.28 (s, 1H), 4.05 (d, *J* = 9.1 Hz, 1H), 3.91 (s, 5H), 2.99 - 2.85 (m, 4H), 2.79 (t, *J =* 12.0 Hz, 3H), 2.64 - 2.57 (m, 2H), 2.57 - 2.52 (m, 1H), 2.02 (ddt, *J =* 20.7, 13.2, 6.0 Hz, 2H), 1.96 - 1.63 (m, 9H), 1.49 (d, *J* = 29.9 Hz, 1H), 1.19 (d, *J* = 46.8 Hz, 15H).

### Example 322

### Synthesis of compound 277

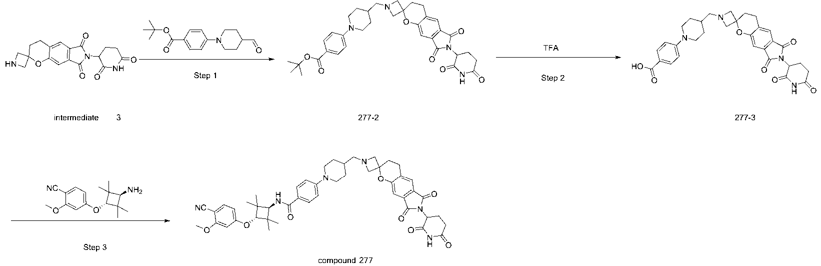

### Step 1: (Compound 277-2) was prepared with reference to step 1 of Example 267. LC-MS: [M+H]⁺ =629.58

### Step 2: (Compound 277-3) was prepared with reference to step 2 of Example 267. LC-MS: [M+H]⁺ =573.48

Step 3: (Compound 277) was prepared with reference to step 3 of Example 267.
¹H NMR (600 MHz, DMSO-*d*₆) δ 11.11 (s, 1H), 7.74 (d, *J =* 8.2 Hz, 3H), 7.65 (d, *J =* 8.6 Hz, 1H), 7.51 (d, *J* = 9.3 Hz, 1H), 7.26 (s, 1H), 6.96 (d, *J =* 8.5 Hz, 2H), 6.64 (d, *J* = 2.2 Hz, 1H), 6.54 (dd, *J* = 8.6, 2.2 Hz, 1H), 5.10 (dd, *J* = 12.9, 5.4 Hz, 1H), 4.27 (s, 1H), 4.05 (d, *J* = 9.2 Hz, 1H), 3.91 (s, 3H), 3.85 (t, *J =* 7.7 Hz, 2H), 3.05 - 2.84 (m, 4H), 2.75 (t, *J =* 12.2 Hz, 3H), 2.61 (d, *J =* 3.3 Hz, 1H), 2.58 (t, *J* = 3.4 Hz, 1H), 2.55 (dd, *J =* 13.1, 4.5 Hz, 1H), 2.28 - 1.95 (m, 4H), 1.87 - 1.43 (m, 4H), 1.19 (d, *J =* 46.5 Hz, 15H). LC-MS: [M+H]⁺ =829.66

### Example 323

### Synthesis of compound 278

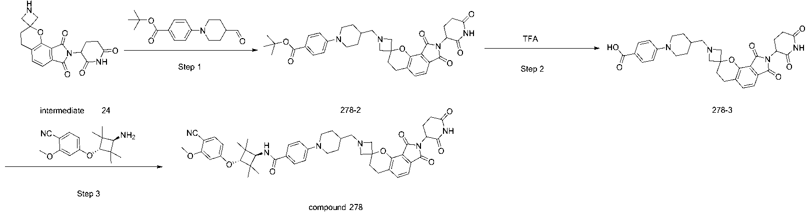

### Step 1: (Compound 278-2) was prepared with reference to step 1 of Example 267. LC-MS: [M+H]⁺ =629.58

### Step 2: (Compound 278-3) was prepared with reference to step 2 of Example 267. LC-MS: [M+H]⁺ =573.38

Step 3: (Compound 278) was prepared with reference to step 3 of Example 267.
¹H NMR (400 MHz, DMSO-*d*₆) δ 11.12 (s, 1H), 7.76 (d, *J =* 8.6 Hz, 2H), 7.65 (dd, *J* = 8.2, 2.6 Hz, 2H), 7.52 (d, *J* = 9.2 Hz, 1H), 7.45 (d, *J* = 7.4 Hz, 1H), 6.98 (d, *J =* 8.6 Hz, 2H), 6.64 (d, *J* = 2.2 Hz, 1H), 6.54 (dd, *J =* 8.6, 2.2 Hz, 1H), 5.09 (dd, *J =* 12.7, 5.5 Hz, 1H), 4.62 - 4.44 (m, 2H), 4.38 (dd, *J =* 12.5, 4.9 Hz, 1H), 4.29 (d, *J =* 10.7 Hz, 2H), 4.05 (d, *J =* 9.2 Hz, 1H), 3.91 (s, 5H), 3.34 (d, *J =* 6.7 Hz, 2H), 3.25 (t, *J =* 6.1 Hz, 1H), 2.93 (m, 3H), 2.77 (t, *J =* 11.7 Hz, 2H), 2.66 - 2.56 (m, 1H), 2.30 (t, *J =* 6.6 Hz, 2H), 2.03 (m, 1H), 1.83 - 1.71 (m, 2H), 1.19 (d, *J* = 30.2 Hz, 15H). LC-MS: [M+H]⁺ =829.66

### Example 324

### Synthesis of compound 279

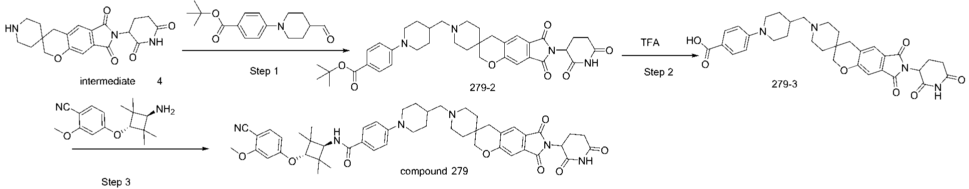

### Step 1: (Compound 279-2) was prepared with reference to step 1 of Example 267. LC-MS: [M+H]⁺ =657.47

### Step 2: (Compound 279-3) was prepared with reference to step 2 of Example 267. LC-MS: [M+H]⁺ =601.48

Step 3: (Compound 279) was prepared with reference to step 3 of Example 267.
¹H NMR (400 MHz, DMSO-*d*₆) δ 11.11 (s, 1H), 7.81 - 7.59 (m, 4H), 7.52 (d, *J* = 9.2 Hz, 1H), 7.31 (d, *J =* 2.7 Hz, 1H), 6.99 (d, *J* = 8.9 Hz, 2H), 6.64 (d *J* = 2.2 Hz, 1H), 6.54 (dd, *J =* 8.7, 2.2 Hz, 1H), 5.10 (dd, *J =* 12.8, 5.4 Hz, 1H), 4.30 (d, *J =* 19.6 Hz, 2H), 4.05 (d, *J =* 9.8 Hz, 2H), 3.91 (s, 5H), 3.22 - 3.01 (m, 5H), 2.96 - 2.75 (m, 4H), 2.70 - 2.54 (m, 2H), 2.13 - 1.95 (m, 2H), 1.89 - 1.56 (m, 6H), 1.55 - 1.41 (m, 1H), 1.36 - 1.11 (m, 15H). LC-MS: [M+H]⁺ =857.76

### Example 325

### Synthesis of compound 280

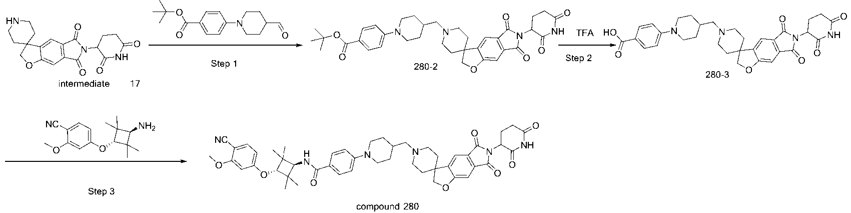

### Step 1: (Compound 280-2) was prepared with reference to step 1 of Example 267. LC-MS: [M+H]⁺ =643.48

### Step 2: (Compound 280-3) was prepared with reference to step 2 of Example 267. LC-MS: [M+H]⁺ =587.48

Step 3: (Compound 280) was prepared with reference to step 3 of Example 267.
¹H NMR (400 MHz, DMSO-*d*₆) δ 11.12 (s, 1H), 7.77 (d, *J =* 8.5 Hz, 2H), 7.70 - 7.60 (m, 2H), 7.53 (d, *J* = 9.2 Hz, 1H), 7.36 (d, *J* = 8.6 Hz, 1H), 7.07 - 6.93 (m, 2H), 6.64 (d, *J* = 2.2 Hz, 1H), 6.54 (dd, *J* = 8.6, 2.2 Hz, 1H), 5.11 (dd, *J =* 12.7, 5.5 Hz, 1H), 4.74 (d, *J =* 13.1 Hz, 2H), 4.28 (s, 1H), 4.06 (d, *J* = 9.2 Hz, 1H), 3.91 (s, 5H), 3.62 (d, *J* = 12.2 Hz, 2H), 3.17 - 3.01 (m, 3H), 2.86 (ddd, *J =* 15.5, 12.0, 8.7 Hz, 3H), 2.66 - 2.57 (m, 1H), 2.36 - 2.22 (m, 2H), 2.18 - 1.74 (m, 7H), 1.19 (d, *J =* 30.8 Hz, 15H). LC-MS: [M+H]⁺ =843.76

### Example 326

### Synthesis of compound 281

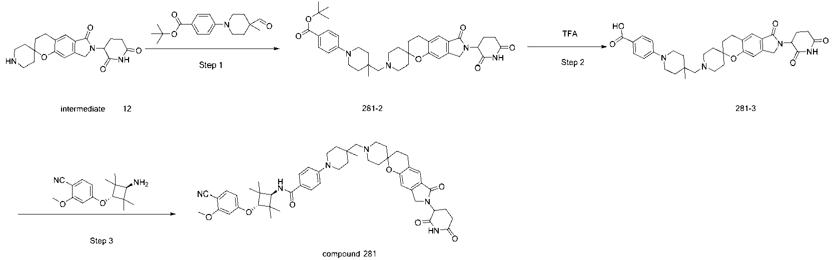

### Step 1: (Compound 281-2) was prepared with reference to step 1 of Example 267. LC-MS: [M+H]⁺ =657.57

### Step 2: (Compound 281-3) was prepared with reference to step 2 of Example 267. LC-MS: [M+H]⁺ =601.48

### Step 3: (Compound 281) was prepared with reference to step 3 of Example 267. LC-MS: [M+H]⁺ =857.66

¹H NMR (600 MHz, DMSO-*d*₆) δ 10.97 (s, 1H), 7.77 (d, *J =* 8.4 Hz, 2H), 7.66 (d, *J =* 8.6 Hz, 1H), 7.52 (d, *J* = 5.0 Hz, 2H), 7.06 (s, 1H), 7.03 - 6.97 (m, 2H), 6.64 (d, *J* = 2.2 Hz, 1H), 6.54 (dd, *J* = 8.7, 2.2 Hz, 1H), 5.07 (dt, *J =* 13.6, 4.0 Hz, 1H), 4.35 (dd, *J =* 17.0, 3.8 Hz, 1H), 4.28 (s, 1H), 4.23 (d, *J =* 16.6 Hz, 1H), 4.06 (d, *J* = 9.2 Hz, 1H), 3.91 (s, 3H), 3.56 (d, *J =* 12.2 Hz, 2H), 3.48 (s, 2H), 3.40 - 3.29 (m, 2H), 3.22 (d, *J =* 3.8 Hz, 2H), 3.14 (mz, 2H), 2.91 (m, 3H), 2.64 - 2.55 (m, 1H), 2.42 - 2.32 (m, 1H), 2.17 - 2.05 (m, 2H), 2.04 - 1.84 (m, 5H), 1.69 (t, *J* = 11.2 Hz, 2H), 1.64 - 1.53 (m, 2H), 1.31 - 1.10 (m, 15H).

### Example 327

### Synthesis of compound 282

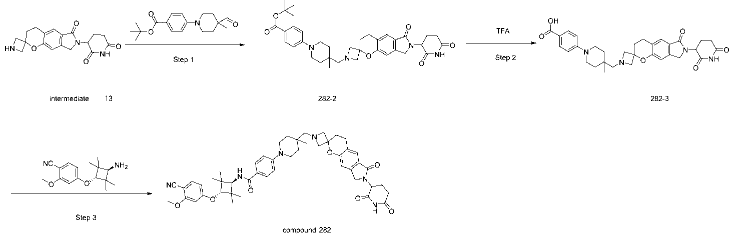

### Step 1: (Compound 282-2) was prepared with reference to step 1 of Example 267. LC-MS: [M+H]⁺ =629.58

### Step 2: (Compound 282-3) was prepared with reference to step 2 of Example 267. LC-MS: [M+H]⁺ =573.48

### Step 3: (Compound 282) was prepared with reference to step 3 of Example 267. LC-MS: [M+H]⁺ =829.66

¹H NMR (600 MHz, DMSO-*d*₆) δ 10.97 (s, 1H), 7.77 (d, *J =* 8.5 Hz, 2H), 7.65 (d, *J =* 8.6 Hz, 1H), 7.52 (d, *J =* 12.1 Hz, 2H), 7.07 (d, *J =* 17.6 Hz, 1H), 6.98 (d, *J =* 8.5 Hz, 2H), 6.64 (d, *J =* 2.2 Hz, 1H), 6.54 (dd, *J =* 8.7, 2.2 Hz, 1H), 5.07 (dd, *J =* 13.4, 5.0 Hz, 1H), 4.61 (s, 1H), 4.55 - 4.46 (m, 1H), 4.44 - 4.31 (m, 3H), 4.30 - 4.19 (m, 3H), 4.06 (d, *J =* 9.2 Hz, 2H), 3.52 (dt, *J =* 13.4, 4.8 Hz, 2H), 3.34 (d, *J =* 5.4 Hz, 3H), 3.13 (t, *J =* 11.4 Hz, 2H), 2.97 - 2.83 (m, 3H), 2.64 - 2.56 (m, 1H), 2.37 (q, *J =* 12.8 Hz, 1H), 2.31 - 2.17 (m, 2H), 2.00 (dd, *J =* 13.3, 7.9 Hz, 1H), 1.62 ( d, *J =* 11.2 Hz, 2H), 1.49 (d, *J =* 10.8 Hz, 2H), 1.23 (s, 6H), 1.15 (s, 6H), 1.10 (d, *J =* 3.3 Hz, 3H).

### Example 328

### Synthesis of compound 283

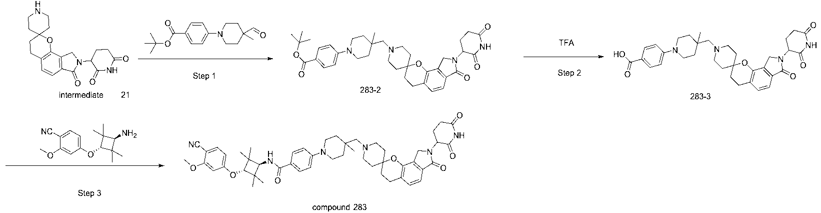

### Step 1: (Compound 283-2) was prepared with reference to step 1 of Example 267. LC-MS: [M+H]⁺ =657.57

### Step 2: (Compound 283-3) was prepared with reference to step 2 of Example 267. LC-MS: [M+H]⁺ =601.48

Step 3: (Compound 283) was prepared with reference to step 3 of Example 267.
¹H NMR (400 MHz, DMSO-*d*₆) δ 11.01 (d, *J* = 4.5 Hz, 1H), 7.77 (d, *J =* 8.6 Hz, 2H), 7.65 (d, *J =* 8.6 Hz, 1H), 7.51 (d, *J =* 9.0 Hz, 1H), 7.33 - 7.22 (m, 2H), 7.00 (d, *J =* 8.6 Hz, 2H), 6.64 (d, *J* = 2.2 Hz, 1H), 6.54 (dd, *J =* 8.6, 2.2 Hz, 1H), 5.14 (dd, *J =* 13.2, 5.5 Hz, 1H), 4.44 (d, *J =* 17.3 Hz, 1H), 4.30 (d, *J =* 16.4 Hz, 2H), 4.06 (d, *J* = 9.2 Hz, 2H), 3.61 - 3.44 (m, 4H), 3.34 (t, *J =* 11.7 Hz, 2H), 3.30 - 3.05 (m, 5H), 3.00 - 2.83 (m, 3H), 2.63 (d, *J =* 17.1 Hz, 1H), 2.42 - 2.29 (m, 1H), 2.14 (t, *J =* 13.6 Hz, 2H), 2.08 - 1.81 (m, 6H), 1.66 (dt, *J =* 29.5, 12.1 Hz, 4H), 1.19 (d, *J* = 30.1 Hz, 15H).LC-MS: [M+H]⁺ =857.47

### Example 329

### Synthesis of compound 284

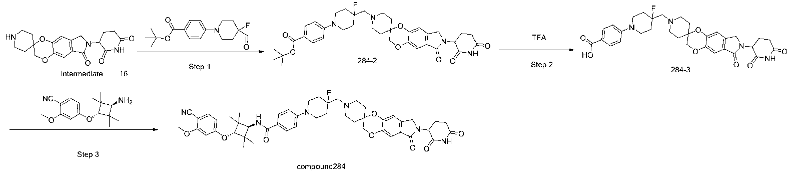

### Step 1: (Compound 284-2) was prepared with reference to step 1 of Example 267. LC-MS: [M+H]⁺ =663.57

### Step 2: (Compound 284-3) was prepared with reference to step 2 of Example 267. LC-MS: [M+H]⁺ =607.48

Step 3: (Compound 284) was prepared with reference to step 3 of Example 267.
¹H NMR (600 MHz, DMSO-*d*₆) δ 10.97 (s, 1H), 7.79 (d, *J =* 8.5 Hz, 2H), 7.66 (d, *J =* 8.6 Hz, 1H), 7.53 (d, *J* = 9.2 Hz, 1H), 7.23 (s, 1H), 7.16 (s, 1H), 7.05 (d, *J =* 8.5 Hz, 2H), 6.64 (d, *J* = 2.2 Hz, 1H), 6.54 (dd, *J* = 8.7, 2.2 Hz, 1H), 5.07 (dd, *J =* 13.3, 5.1 Hz, 1H), 4.57 (m, 1H), 4.34 (d, *J =* 16.9 Hz, 1H), 4.28 (s, 1H), 4.21 (d, *J =* 16.8 Hz, 1H), 4.13 (s, 1H), 4.06 (d, *J* = 9.2 Hz, 1H), 3.91 (s, 3H), 3.81 (d, *J =* 30.3 Hz, 3H), 3.61 (d, *J =* 55.3 Hz, 2H), 3.13 (t, *J* = 11.6 Hz, 2H), 2.91 (ddd, *J =* 17.9, 11.0, 5.4 Hz, 1H), 2.63 - 2.57 (m, 1H), 2.35 (td, *J =* 13.2, 4.6 Hz, 2H), 2.20 - 1.93 (m, 6H), 1.86 (d, *J* = 34.5 Hz, 3H), 1.52 (d, *J =* 7.0 Hz, 1H), 1.46 (m, 1H), 1.34 - 1.09 (m, 12H). LC-MS: [M+H]⁺ =863.66

### Example 330

### Synthesis of compound 285

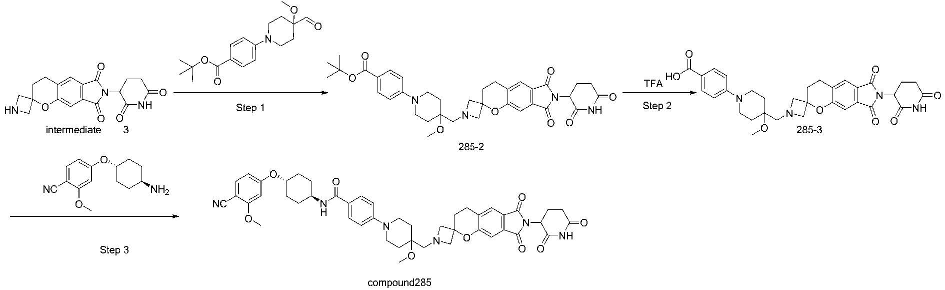

### Step 1: (Compound 285-2) was prepared with reference to step 1 of Example 267. LC-MS: [M+H]⁺ =659.07

### Step 2: (Compound 285-3) was prepared with reference to step 2 of Example 267. LC-MS: [M+H]⁺ =603.08

Step 3: Prepared with reference to step 3 of Example 267.
¹ H NMR (600 MHz, *DMSO-d*₆ ) δ 11.11 (s, 1H), 7.98 (d, *J =* 7.6 Hz, 1H), 7.81 - 7.72 (m, 3H), 7.62 (d, *J =* 8.6 Hz, 1H), 7.29 (s, 1H), 6.97 (d, *J =* 8.8 Hz, 2H), 6.73 (dd, *J =* 13.2, 4.5 Hz, 2H), 5.11 (dd, *J =* 12.8, 5.3 Hz, 1H), 4.65 - 4.31 (m, 5H), 3.89 (s, 3H), 3.85 - 3.78 (m, 2H), 3.64 (s, 2H ), 3.23 (s, 3H), 3.11 - 2.84 (m, 6H), 2.60 (d, *J =* 17.3 Hz, 1H), 2.31 (s, 2H), 2.16 - 2.01 (m, 3H), 1.86 (m, 4H), 1.69 - 1.44 (m, 7H). LC-MS: [M+H]⁺ =831.66

### Example 331

### Synthesis of compound 286

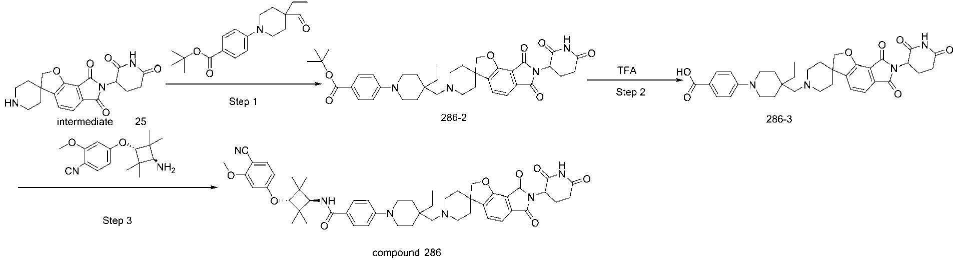

### Step 1: (Compound 286-2) was prepared with reference to step 1 of Example 267. LC-MS: [M+H]⁺ =671.50

### Step 2: (Compound 286-3) was prepared with reference to step 2 of Example 267. LC-MS: [M+H]⁺ =615.48

Step 3: (Compound 286) was prepared with reference to step 3 of Example 267.
¹H NMR (600 MHz, DMSO-*d*₆) δ 11.12 (s, 1H), 7.79 (t, *J =* 9.5 Hz, 1H), 7.66 (d, *J =* 8.6 Hz, 1H), 7.57 - 7.45 (m, 4H), 7.01 (d, *J =* 8.7 Hz, 2H), 6.64 (s, 1H), 6.54 (d, *J =* 8.6 Hz, 1H), 5.11 (dd, *J =* 12.8, 5.4 Hz, 1H), 4.82 (s, 2H), 4.66 (s, 1H), 4.28 (s, 1H), 4.05 (dd, *J =* 14.4, 7.5 Hz, 3H), 3.91 (s, 3H), 3.21 (s, 3H), 2.94 - 2.86 (m, 2H), 2.60 (d, *J* = 18.0 Hz, 1H), 2.44 - 2.34 (m, 5H), 2.05 - 1.95 (m, 3H), 1.74 - 1.57 (m, 7H), 1.23 (s, 6H), 1.19 - 1.13 (m, 7H).LC-MS: [M+H]⁺ =871.60

### Example 332

### Synthesis of compound 287

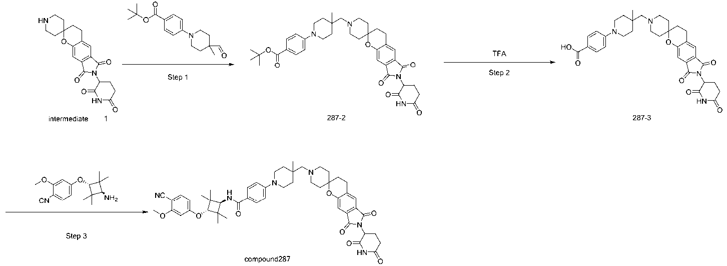

### Step 1: (Compound 287-2) was prepared with reference to step 1 of Example 267. LC-MS: [M+H]⁺ =671.57

### Step 2: (Compound 287-3) was prepared with reference to step 2 of Example 267. LC-MS: [M+H]⁺ =615.48

Step 3: (Compound 287) was prepared with reference to step 3 of Example 267.
¹ H NMR (600 MHz, DMSO-*d*₆) δ 11.11 (s, 1H), 7.80 - 7.74 (m, 3H), 7.65 (d, *J =* 8.6 Hz, 1H), 7.52 (d, *J =* 9.3 Hz, 1H), 7.41 (s, 1H), 7.00 (d, *J =* 8.9 Hz, 2H), 6.64 (d, *J* = 2.1 Hz, 1H), 6.54 (dd, *J* = 8.7, 2.1 Hz, 1H), 5.10 (dd, *J =* 12.9, 5.4 Hz, 1H), 4.58 (dd, *J =* 13.9, 7.0 Hz, 1H), 4.28 (s, 1H), 4.06 (d, *J* = 9.2 Hz, 1H), 3.91 (s, 3H), 3.61 - 3.37 (m, 6H), 3.26 - 3.11 (m, 4H), 2.92 (ddd, *J =* 31.1, 19.4, 9.1 Hz, 3H), 2.60 (d, *J =* 16.9 Hz, 1H), 2.16 - 1.89 (m, 6H), 1.64 (dd, *J =* 43.0, 11.3 Hz, 4H), 1.53 (d, *J =* 7.0 Hz, 1H), 1.23 (d, *J* = 4.2 Hz, 9H), 1.15 (s, 6H). LC-MS: [M+H]⁺ =871.76

### Example 333

### Synthesis of compound 288

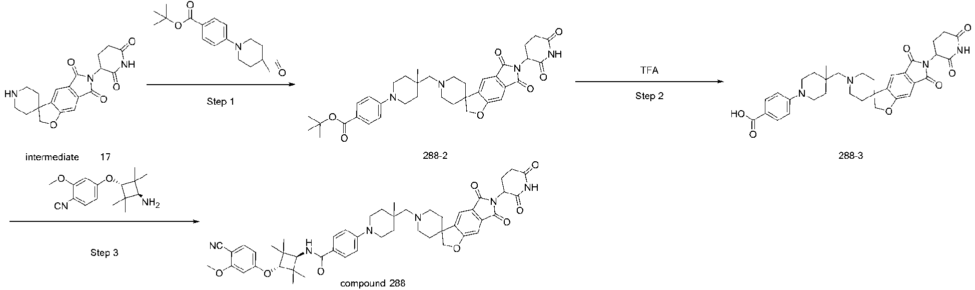

### Step 1: (Compound 288-2) was prepared with reference to step 1 of Example 267. LC-MS: [M+H]⁺ =657.57

### Step 2: (Compound 288-3) was prepared with reference to step 2 of Example 267. LC-MS: [M+H]⁺ =601.45

Step 3: (Compound 288) was prepared with reference to step 3 of Example 267.
¹H NMR (600 MHz, DMSO-*d*₆) δ 11.12 (s, 1H), 7.78 (d, *J =* 8.8 Hz, 2H), 7.67 - 7.59 (m, 2H), 7.53 (d, *J* = 9.1 Hz, 1H), 7.36 (d, *J* = 8.6 Hz, 1H), 7.01 (d, *J =* 8.8 Hz, 2H), 6.64 (d, *J* = 2.0 Hz, 1H), 6.54 (dd, *J =* 8.6, 2.0 Hz, 1H), 5.11 (dd, *J =* 12.6, 5.5 Hz, 1H), 4.76 (s, 2H), 4.28 (s, 1H), 4.06 (d, *J* = 9.2 Hz, 1H), 3.91 (s, 3H), 3.67 - 3.55 (m, 4H), 3.31 (d, *J* = 43.1 Hz, 4H), 3.16 (s, 3H), 2.95 - 2.83 (m, 1H), 2.61 (d, *J =* 16.6 Hz, 1H), 2.46 - 2.34 (m, 2H), 2.09 - 1.88 (m, 3H), 1.75 - 1.57 (m, 4H), 1.23 (s, 9H), 1.15 (s, 6H).LC-MS: [M+H]⁺ =857.66

### Example 334

### Synthesis of compound 289

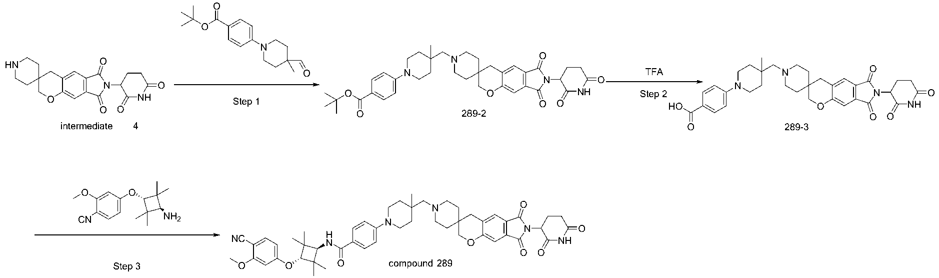

### Step 1: (Compound 289-2) was prepared with reference to step 1 of Example 267. LC-MS: [M+H]⁺ =671.57

### Step 2: (Compound 289-3) was prepared with reference to step 2 of Example 267. LC-MS: [M+H]⁺ =615.48

### Step 3: (Compound 289) Pwas prepared with reference to step 3 of Example 267. LC-MS: [M+H]⁺ =871.76

¹H NMR (600 MHz, DMSO-*d*₆) δ 11.11 (s, 1H), 7.80 - 7.73 (m, 2H), 7.68 - 7.60 (m, 2H), 7.52 (d, *J* = 9.2 Hz, 1H), 7.31 (d, *J =* 3.0 Hz, 1H), 6.99 (d, *J =* 8.6 Hz, 2H), 6.64 (d, *J =* 2.1 Hz, 1H), 6.54 (dd, *J =* 8.6, 2.1 Hz, 1H), 5.10 (dd, *J =* 12.9, 5.4 Hz, 1H), 4.29 (d, *J =* 14.3 Hz, 2H), 4.08 - 4.03 (m, 3H), 3.91 (s, 4H), 3.56 (d, *J =* 8.1 Hz, 3H), 3.50 - 3.39 (m, 3H), 3.29 (s, 2H), 3.12 (dd, *J =* 27.8, 16.9 Hz, 5H), 2.93 - 2.85 (m, 1H), 2.81 (s, 1H), 2.60 (d, *J =* 17.6 Hz, 1H), 2.07 - 2.01 (m, 1H), 1.94 - 1.80 (m, 2H), 1.74 - 1.64 (m, 3H), 1.60 (d, *J =* 10.1 Hz, 3H), 1.53 (d, *J* = 7.0 Hz, 1H), 1.22 (d, *J =* 16.2 Hz, 9H), 1.15 (s, 6H).

### Example 335

### Synthesis of compound 290

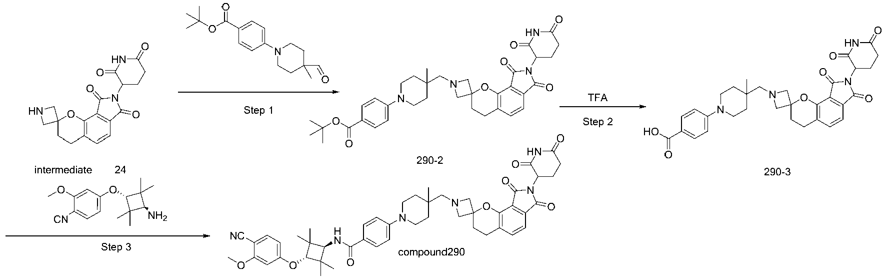

### Step 1: (Compound 290-2) was prepared with reference to step 1 of Example 267. LC-MS: [M+H]⁺ =643.48

### Step 2: (Compound 290-3) was prepared with reference to step 2 of Example 267. LC-MS: [M+H]⁺ =587.58

### Step 3: (Compound 290) was prepared with reference to step 3 of Example 267. LC-MS: [M+H]⁺ =843.66

¹H NMR (600 MHz, DMSO-*d*₆) δ 11.12 (s, 1H), 7.76 (d, *J =* 8.5 Hz, 2H), 7.65 (t, *J =* 7.7 Hz, 2H), 7.51 (d, *J = 9.2* Hz, 1H), 7.45 (d, *J =* 7.4 Hz, 1H), 6.98 (d, *J =* 8.9 Hz, 2H), 6.64 (d, *J* = 2.0 Hz, 1H), 6.54 (dd, *J =* 8.6, 2.1 Hz, 1H), 5.08 (dd, *J=* 12.6, 5.6 Hz, 1H), 4.62 (d, *J* =32.3 Hz, 2H), 4.45 - 4.32 (m, 2H), 4.28 (s, 1H), 4.06 (d, *J* = 9.2 Hz, 1H), 3.91 (s, 3H), 3.52 (d, *J=* 12.1 Hz, 3H), 3.17 - 3.09 (m, 3H), 2.92 (dd, *J =* 41.8, 22.5 Hz, 4H), 2.63 - 2.57 (m, 1H), 2.33 (d, *J* = 5.2 Hz, 2H),2.06 - 1.98 (m, 1H), 1.63 (d, *J =* 8.9 Hz, 2H), 1.48 (s, 2H), 1.23 (s, 6H), 1.15 (s, 6H), 1.09 (d, *J* = 6.9 Hz, 3H).

### Example 336

### Synthesis of compound 291

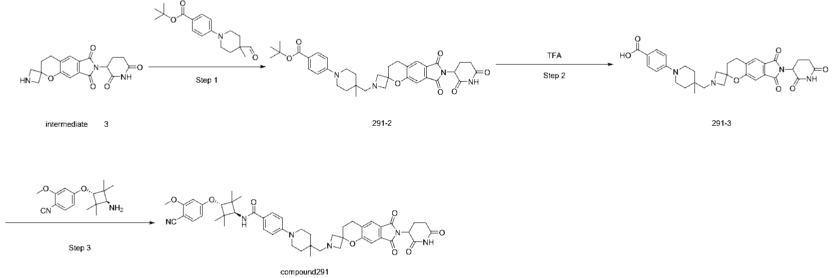

### Step 1: (Compound 291-2) was prepared with reference to step 1 of Example 267. LC-MS: [M+H]⁺ =643.48

### Step 2: (Compounds 291-3) was prepared with reference to step 2 of Example 267. LC-MS: [M+H]⁺ =587.48

### Step 3 (Compound 291) was prepared with reference to step 3 of Example 267. LC-MS: [M+H]⁺ =843.66

¹ H NMR (400 MHz, DMSO-*d*₆) δ 11.12 (s, 1H), 7.77 (d, *J =* 10.4 Hz, 3H), 7.65 (d, *J =* 8.6 Hz, 1H), 7.51 (d, *J = 9.2* Hz, 1H), 7.29 (d, *J =* 9.2 Hz, 1H), 6.98 (d, *J =* 8.9 Hz, 2H), 6.64 (d, *J* = 2.1 Hz, 1H), 6.54 (dd, *J* = 8.7, 2.1 Hz, 1H), 5.11 (dd, *J =* 12.8, 5.4 Hz, 1H), 4.67 - 4.34 (m, 4H), 4.28 (s, 1H), 4.06 (d, *J* = 9.2 Hz, 1H), 3.91 (s, 3H), 3.52 (d, *J =* 13.5 Hz, 2H), 3.14 (dd, *J =* 19.5, 9.4 Hz, 3H), 2.92 (dd, *J =* 37.9, 18.2 Hz, 4H), 2.69 - 2.57 (m, 1H), 2.37 - 2.22 (m, 2H), 2.10 - 1.96 (m, 2H), 1.61 (s, 2H), 1.47 (d, *J* = 11.2 Hz, 2H), 1.23 (d, *J =* 5.2 Hz, 6H), 1.15 (s, 6H), 1.09 (s, 3H).

### Example 337

### Synthesis of compound 294

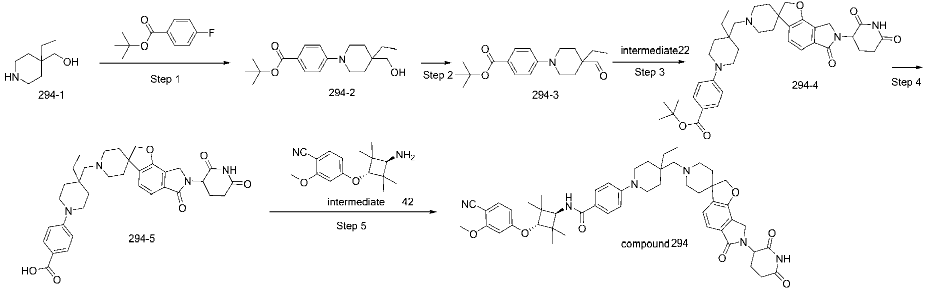

Step 1: (Compound 294-2) was prepared with reference to step 2 of Example 284.

### Step 2: (Compound 294-3) was prepared wth reference to step 3 of Example 284 LC-MS: [M+H]⁺ =318.43

### Step 3: (Compound 294-4) was prepared with reference to step 4 of Example 284 LC-MS: [M+H]⁺ =657.36

### Step 4: (Compound 294-5) was prepared with reference to step 2 of Example 267. LC-MS: [M+H]⁺ =601.29

### Step 5: (Compound 294) was prepared with reference to step 3 of Example 267. LC-MS: [M+H]⁺ =857.45

¹ H NMR (600 MHz, *DMSO-d*₆ ) δ 10.99 (s, 1H), 7.79 (d, *J =* 8.4 Hz, 2H), 7.66 (d, *J =* 8.6 Hz, 1H), 7.52 (d, *J* = 9.1 Hz, 1H), 7.37 (d, *J =* 7.5 Hz, 1H), 7.28 (d, *J =* 7.6 Hz, 1H), 7.01 (d, *J =* 8.6 Hz, 2H), 6.64 (s, 1H), 6.55 (d, *J* = 8.6 Hz, 1H), 5.10 (dd, *J =* 13.1, 5.2 Hz, 1H), 4.72 - 4.62 (m, 2H), 4.41 (dd, *J =* 17.2, 7.8 Hz, 1H) , 4.30 - 4.23 (m, 2H), 4.06 (d, *J=* 9.1 Hz, 1H), 3.91 (s, 3H), 3.21 (s, 4H), 2.97 - 2.87 (m, 2H), 2.61 (d, *J =* 16.4 Hz, 2H), 2.45 - 2.29 (m, 5H), 2.04 - 1.87 (m, 4H), 1.70 (dd, *J =* 40.7, 21.6 Hz, 7H), 1.23 (s, 6H), 1.15 (s, 6H), 0.90 (t, *J =* 7.3 Hz, 3H).

### Example 338

### Synthesis of compound 295

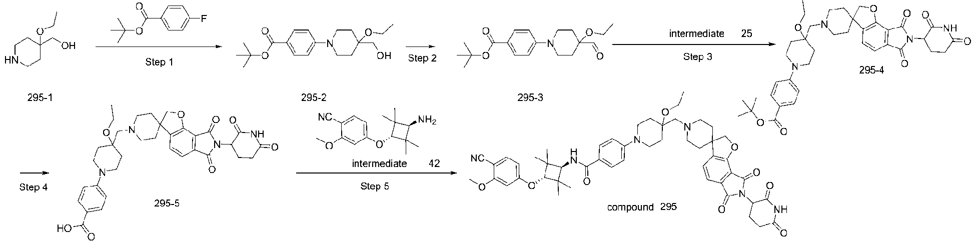

### Step 1: (Compound 295-2) was prepared with reference to step 2 of Example 284

### Step 2: (Compound 295-3) was prepared with reference to step 3 of Example 284. LC-MS: [M+H]⁺ =334.19

### Step 3: (Compound 295-4) was prepared with reference to step 4 of Example 284. LC-MS: [M+H]⁺ =687.33

### Step 4: (Compound 295-5) was prepared with reference to step 2 of Example 267. LC-MS: [M+H]⁺ =631.27

### Step 5: (Compound 295) was prepared with reference to step 3 of Example 267. LC-MS: [M+H]⁺ =887.43

¹ H NMR (600 MHz, DMSO-d₆) δ 11.11 (s, 1H), 7.78 (d, *J =* 8.4 Hz, 2H), 7.69 - 7.45 (m, 4H), 7.01 (d, *J =* 8.5 Hz, 2H), 6.65 (s, 1H), 6.55 (d, *J* = 8.7 Hz, 1H), 5.11 (dd, *J =* 12.8, 5.3 Hz, 1H), 4.80 (s, 2H), 4.29 (s, 1H), 4.06 *(d, J=* 9.2 Hz, 1H), 3.91 (s, 3H), 3.56 (d, *J =* 78.6 Hz, 6H), 3.11 (t, *J =* 11.3 Hz, 3H), 2.95 - 2.84 (m, 2H), 2.65 - 2.56 (m, 2H), 2.54 - 2.51 (m, 2H), 2.01 (dd, *J =* 27.9, 21.8 Hz, 5H), 1.78 - 1.66 (m, 3H), 1.50 *(d, J =* 6.9 Hz, 2H), 1.23 (s, 6H), 1.16 (s, 8H).

### Example 339

### Synthesis of compound 296

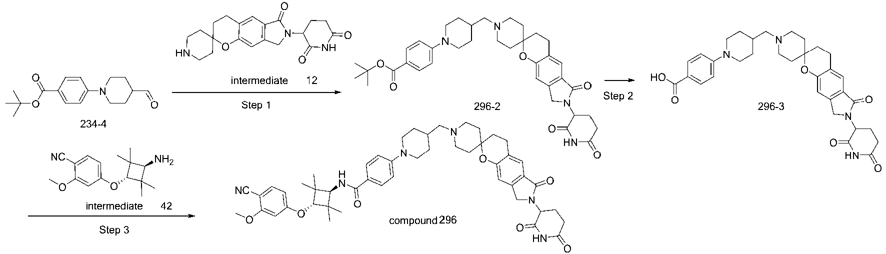

### Step 1: (Compound 296-2) was prepared with reference to step 4 of Example 284.

### Step 2: (Compound 296-3) was prepared with reference to step 2 of Example 267 LC-MS: [M+H]⁺ =587.28

Step 3: (Compound 296) was prepared with reference to step 3 of Example 267.
¹H NMR (600 MHz, DMSO-d₆) δ 10.97 (s, 1H), 7.77 (d, *J =* 8.8 Hz, 2H), 7.66 (d, *J =* 8.6 Hz, 1H), 7.54 - 7.49 (m, 2H), 7.02 (dd, *J =* 21.5, 13.1 Hz, 3H), 6.64 (d, *J* = 2.0 Hz, 1H), 6.54 (dd, *J =* 8.6, 2.1 Hz, 1H), 5.07 (dd, *J =* 13.3, 5.1 Hz, 1H), 4.39 - 4.20 (m, 4H), 4.06 (d, *J* = 9.2 Hz, 1H), 3.91 (s, 4H), 3.15 (d, *J =* 29.5 Hz, 5H), 2.95 - 2.79 (m, 5H), 2.60 (d, *J =* 18.5 Hz, 2H), 2.40 - 2.31 (m, 2H), 2.15 - 1.81 (m, 10H), 1.35 - 1.22 (m, 8H), 1.15 (s, 5H). LC-MS: [M+H]⁺ =843.44

### Example 340

### Synthesis of compound 297

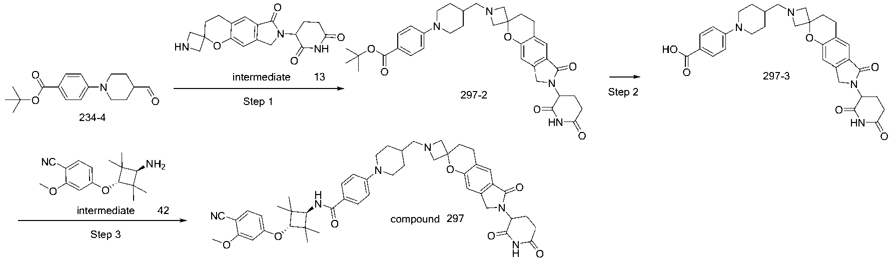

Step 1: (Compound 297-2) was prepared with reference to step 4 of Example 284.

### Step 2: (Compound 297-3) was prepared with reference to step 2 of Example 267. LC-MS: [M+H]⁺ =559.25

Step 3: (Compound 297) was prepared with reference to step 3 of Example 267.
¹H NMR (600 MHz, *DMSO-d*₆) δ 10.97 (s, 1H), 7.76 (d, *J =* 8.8 Hz, 2H), 7.66 (d, *J =* 8.6 Hz, 1H), 7.56 - 7.49 (m, 2H), 7.11 - 7.04 (m, 1H), 7.01 - 6.95 (m, 2H), 6.64 (d, *J* = 2.1 Hz, 1H), 6.54 (dd, *J* = 8.6, 2.1 Hz, 1H), 5.07 (d, *J* = 12.8 Hz, 1H), 4.51 - 4.21 (m, 9H), 4.06 (d, *J = 9.2* Hz, 1H), 3.90 (d, *J =* 15.1 Hz, 5H), 3.27 (d, *J =* 40.9 Hz, 5H), 2.98 - 2.85 (m, 4H), 2.77 (t, *J =* 12.2 Hz, 2H), 2.60 (d, *J =* 18.0 Hz, 1H), 2.37 (d, *J =* 21.1 Hz, 2H), 2.26 - 2.17 (m, 3H), 2.04 - 1.95 (m, 2H), 1.91 - 1.73 (m, 4H), 1.15 (s, 4H). LC-MS: [M+H]⁺=815.41

### Example 341

### Synthesis of compound 298

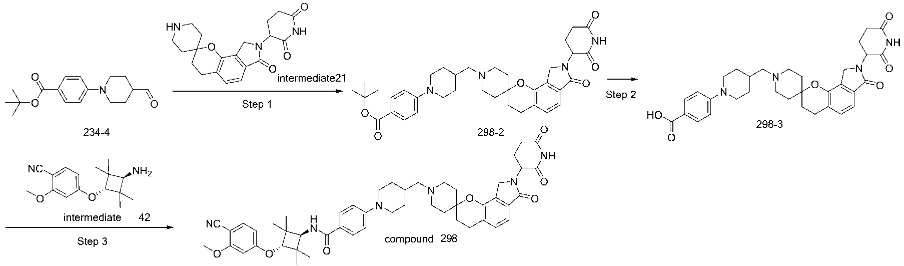

Step 1: (Compound 298-2) was prepared with reference to step 4 of Example 284.

### Step 2: (Compound 298-3) was prepared with reference to step 2 of Example 267. LC-MS: [M+H]⁺ =587.28

Step 3: (Compound 298) was prepared with reference to step 3 of Example 267.
¹H NMR (600 MHz, *DMSO-d*₆) δ 11.03 (s, 1H), 7.77 (d, *J=* 8.7 Hz, 2H), 7.66 (d, *J =* 8.6 Hz, 1H), 7.51 (d, *J = 9.2* Hz, 1H), 7.27 (dd, *J =* 28.8, 7.7 Hz, 2H), 7.00 (d, *J =* 8.8 Hz, 2H), 6.64 (d, *J =* 1.7 Hz, 1H), 6.54 (dd, *J* = 8.7, 1.8 Hz, 1H), 5.14 (dd, *J =* 13.1, 5.2 Hz, 1H), 4.44 (d, *J =* 17.4 Hz, 1H), 4.30 (d, *J =* 18.1 Hz, 2H), 4.06 (d, *J* = 9.3 Hz, 1H), 3.91 (s, 5H), 3.26 - 3.09 (m, 6H), 3.00 - 2.78 (m, 6H), 2.63 (d, *J =* 17.6 Hz, 2H), 2.41 - 2.30 (m, 2H), 2.14 - 1.77 (m, 12H), 1.32 (dd, *J =* 24.9, 12.3 Hz, 3H), 1.15 (s, 6H).LC-MS: [M+H]⁺ =843.44

### Example 342

### Synthesis of compound 299

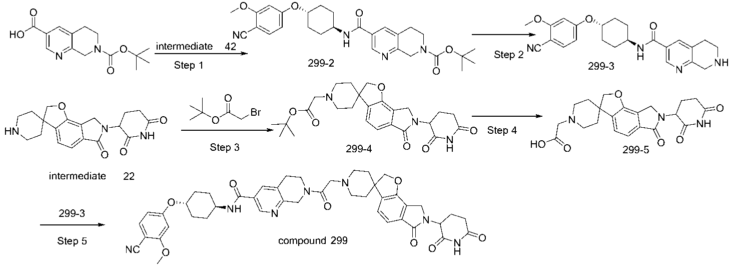

### Step 1: (Compound 299-2) was prepared with reference to step 3 of Example 267. LC-MS: [M+H]+ =511.39 [M+H]

### Step 2: (Compound 299-3) was prepared with reference to step 2 of Example 267. LC-MS: [M+H]+ =411.39 [M+H]

### Step 3: (Compound 300-4) LC-MS: [M+H]+=470.51 [M+H]

Compound 22 (100 mg, 281.38 umol), tert-butyl bromoacetate (82.33 mg, 422.07 umol), N,N-diisopropylethylamine (181.84 mg 1.41 mmol) were added sequentially to an glass vial containing N,N-dimethylformamide, and the reaction solution was stirred at room temperature for 2 hours, and compound 299-4 (70 mg) was obtained by preparative purification.

### Step 4: (Compound 299-5) was prepared with reference to step 2 of Example 267. LC-MS: [M+H]+ =414.39 [M+H]

Step 5: (Compound 299) was prepared with reference to step 3 of Example 267.
¹H NMR (600 MHz, Methanol-d4) δ 8.85 (d, 1H), 8.11 (s, 1H), 7.53 (d, *J =* 8.3 Hz, 1H), 7.47 (d, *J =* 7.6 Hz, 1H), 7.42 (d, 1H), 6.68 (d, *J = 2.2* Hz, 1H), 6.67 (s, 1H), 5.19 - 5.14 (m, 1H), 4.92 (s, 1H), 4.74 (d, *J =* 9.4 Hz, 2H), 4.53 - 4.39 (m, 5H), 4.01 - 3.96 (m, 2H), 3.93 (s, 3H), 3.78 (s, 3H), 3.61 (s, 1H), 3.26 (d, *J =* 13.5 Hz, 1H), 3.12 (s, 1H), 3.02 (t, *J =* 6.0 Hz, 1H), 2.96 - 2.88 (m, 1H), 2.83 - 2.77 (m, 1H), 2.57 - 2.48 (m, 1H), 2.46 - 2.37 (m, 2H), 2.25 (d, *J =* 9.5 Hz, 2H), 2.23 - 2.17 (m, 1H), 2.17 - 2.08 (m, 4H), 1.63 (m, 5H). LC-MS: [M+H]+ =802.66[M+H]

### Example 343

### Synthesis of compound 300

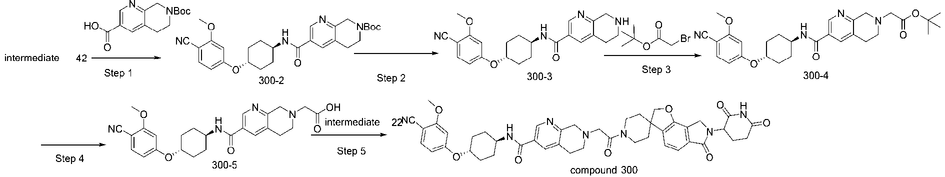

### Step 1: (Compound 300-2) was prepared with reference to step 3 of Example 267. LC-MS: [M+H]+ =525.49 [M+H]

### Step 2: (Compound 300-3) was prepared with reference to step 2 of Example 39. LC-MS: [M+H]+ =469.19 [M+H]

### Step 3: (Compound 300-4) LC-MS: [M+H]+ =521.49 [M+H]

301-3 (70 mg, 172.21 umol), tert-butyl bromoacetate (50.39 mg, 258.31 umol), N,N-diisopropylethylamine (111.29 mg 861.04 umol) were added sequentially to a glass vial containing N,N-dimethylformamide, and the reaction solution was stirred at room temperature for 2 h, pufified by preparation to obtaincompound 300-4 ( 112mg).

### Step 4: (Compound 300-5) was prepared with reference to step 2 of Example 39. LC-MS: [M+H]+ =465.39 [M+H]

### Step 5: (Compound 300) was prepared with reference to step 3 of Example 267. LC-MS: [M+H]+ =802.66[M+H]

¹H NMR (600 MHz, Methanol-d4) δ 8.92 (s, 1H), 8.18 (s, 1H), 7.53 (d, *J =* 8.4 Hz, 1H), 7.44 - 7.36 (m, 2H), 6.69 (d, *J =* 2.4 Hz, 1H), 6.67 (s, 1H), 5.20 - 5.11 (m, 1H), 4.75 - 4.70 (m, 2H), 4.70 - 4.63 (m, 2H), 4.61 - 4.55 (m, 2H), 4.55 - 4.48 (m, 3H), 4.47 - 4.39 (m, 2H), 4.02 - 3.97 (m, 1H), 3.80 (d, *J =* 14.3 Hz, 1H), 3.37 (s, 2H), 3.35 (s, 5H), 3.07 - 3.00 (m, 1H), 2.96 - 2.88 (m, 1H), 2.83 - 2.76 (m, 1H), 2.56 - 2.48 (m, 1H), 2.26 (d, *J =* 9.9 Hz, 2H), 2.13 (d, *J* = 9.5 Hz, 2H), 2.08 - 1.96 (m, 1H), 1.94 - 1.88 (m, 2H), 1.70 - 1.57 (m, 4H).

### Example 344

### Synthesis of compound 301

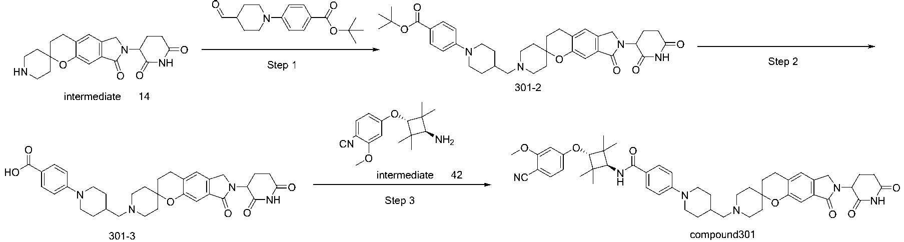

### Step 1: (Compound 301-2) was prepared with reference to step 1 of Example 267. LC-MS: [M+H]⁺ =643.59

### Step 2: (Compound 301-3) was prepared with reference to step 2 of Example 39. LC-MS: [M+H]⁺ =587.40

Step 3: (Compound 301) was prepared with reference to step 3 of Example 267.
¹H NMR (600 MHz, Methanol-d4) δ 7.77 (d, *J =* 8.5 Hz, 2H), 7.54 (d, *J =* 8.6 Hz, 1H), 7.37 (s, 1H), 7.34 (s, 1H), 7.05 (d, *J* = 8.6 Hz, 2H), 6.65 (d, *J =* 1.6 Hz, 1H), 6.58 (dd, *J =* 8.7, 2.2 Hz, 1H), 5.17 - 5.11 (m, 1H), 4.47 - 4.37 (m, 2H), 4.28 (s, 1H), 4.15 (s, 1H), 3.98 (d, *J =* 13.1 Hz, 2H), 3.95 (s, 3H), 3.59 (d, *J =* 12.5 Hz, 2H), 3.47 - 3.39 (m, 2H), 3.21 (d, *J =* 6.9 Hz, 2H), 3.02 (t, *J =* 6.9 Hz, 2H), 2.97 - 2.88 (m, 3H), 2.83 - 2.75 (m, 1H), 2.56 - 2.44 (m, 1H), 2.25 - 2.12 (m, 4H), 2.11 - 2.03 (m, 2H), 2.00 (t, *J =* 6.7 Hz, 2H), 1.95 (d, *J =* 12.9 Hz, 2H), 1.54 - 1.45 (m, 2H), 1.31 (s, 6H), 1.26 (s, 6H). LC-MS: [M+H]⁺ =843.66

### Example 345

### Synthesis of compound 302

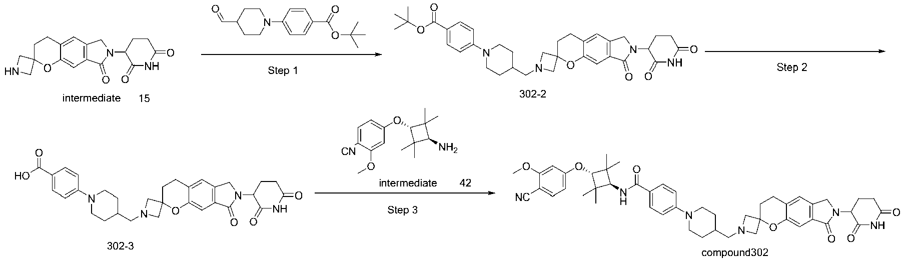

### Step 1: (Compound 302-2) was prepared with reference to step 1 of Example 267. LC-MS: [M+H]⁺ =615.39

### Step 2: (Compound 302-3) was prepared with reference to step 2 of Example 39. LC-MS: [M+H]⁺ =559.24

### Step 3: (Compound 302) was prepared with reference to step 3 of Example 267. LC-MS: [M+H]⁺ =815.66

¹H NMR (600 MHz, Methanol-d4) δ 7.77 (d, *J=* 8.5 Hz, 2H), 7.54 (d, *J =* 8.6 Hz, 1H), 7.36 (d, *J =* 22.9 Hz, 2H), 7.03 (d, *J* = 8.5 Hz, 2H), 6.65 (d, *J =* 2.2 Hz, 1H), 6.57 (dd, *J* = 8.6, 2.2 Hz, 1H), 5.19 - 5.10 (m, 1H), 4.59 - 4.46 (m, 2H), 4.46 - 4.40 (m, 2H), 4.40 - 4.33 (m, 2H), 4.28 (s, 1H), 4.15 (s, 1H), 3.97 (d, 2H), 3.95 (s, 3H), 3.46 - 3.37 (m, 2H), 3.03 (d, *J =* 21.8 Hz, 2H), 2.95 - 2.86 (m, 3H), 2.83 - 2.75 (m, 1H), 2.55 - 2.45 (m, 1H), 2.32 (s, 2H), 2.24 - 2.11 (m, 2H), 2.08 - 1.94 (m, 2H), 1.88 (d, *J =* 12.8 Hz, 2H), 1.46 (d, *J =* 12.2 Hz, 2H), 1.30 (s, 6H), 1.26 (s, 6H).

### Example 346

### Synthesis of compound 303

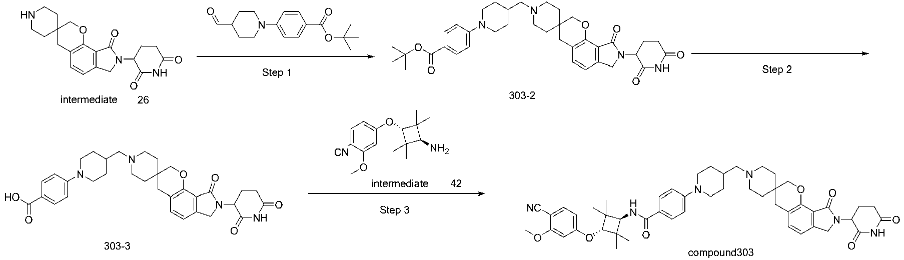

### Step 1: (Compound 303-2) was prepared with reference to step 1 of Example 267. LC-MS: [M+H]⁺ =643.39

### Step 2: (Compound 303-3) was prepared with reference to step 2 of Example 39. LC-MS: [M+H]⁺ =587.34

### Step 3: (Compound 303) was prepared with reference to step 3 of Example 267. LC-MS: [M+H]⁺ =843.66

¹H NMR (600 MHz, Methanol-d4) δ 7.85 (d, *J =* 8.8 Hz, 1H), 7.55 (d, *J =* 8.6 Hz, 1H), 7.39 (dd, *J =* 25.9, 7.7 Hz, 1H), 7.25 (d, *J* = 8.5 Hz, 1H), 7.09 (dd, *J =* 7.7, 4.6 Hz, 1H), 6.65 (d, *J* = 2.2 Hz, 1H), 6.58 (dd, *J =* 8.6, 2.2 Hz, 1H), 5.11 (dd, *J =* 13.4, 5.3 Hz, 1H), 4.42 (q, *J =* 17.1 Hz, 1H), 4.30 (d, *J =* 15.8 Hz, 1H), 4.18 - 4.13 (m, 1H), 4.04 (s, 1H), 3.95 (s, 2H), 3.94 (s, 1H), 3.61 (t, *J=* 12.7 Hz, 1H), 3.29 - 3.25 (m, 0H), 3.24 - 3.19 (m, 1H), 3.18 - 3.12 (m, 1H), 3.03 (d, J = 5.7 Hz, 0H), 2.95 - 2.87 (m, 1H), 2.83 - 2.74 (m, 1H), 2.54 - 2.45 (m, 0H), 2.30 - 2.24 (m, 1H), 2.22 (t, J = 7.6 Hz, 0H), 2.19 - 2.14 (m, 0H), 2.04 (d, *J=* 13.1 Hz, 1H), 1.87 (d, J = 9.9 Hz, 2H), 1.68 - 1.61 (m, 1H), 1.62 - 1.54 (m, 1H), 1.39 - 1.33 (m, 1H), 1.31 (s, 4H), 1.26 (s, 3H).

### Example 347

### Synthesis of compound 304

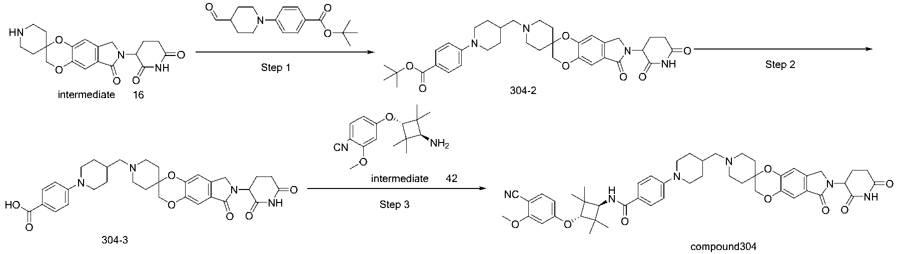

### Step 1: (Compound 304-2) was prepared with reference to step 1 of Example 267. LC-MS: [M+H]⁺ =645.51

### Step 2: (Compound 304-3) was prepared with reference to step 2 of Example 39. LC-MS: [M+H]⁺ =589.34

Step 3: (Compound 304) was prepared with reference to step 3 of Example 267.
¹H NMR (600 MHz, Methanol-d4) δ 7.77 (d, *J =* 8.9 Hz, 2H), 7.54 (d, *J =* 8.6 Hz, 1H), 7.36 (s, 1H), 7.20 (s, 1H), 7.05 (d, 2H), 6.65 (d, *J =* 2.2 Hz, 1H), 6.58 (dd, *J* = 8.6, 2.2 Hz, 1H), 5.13 (dd, J = 13.4, 5.1 Hz, 1H), 4.41 (d, *J* = 16.9 Hz, 2H), 4.28 (d, *J* = 0.9 Hz, 1H), 4.20 - 4.09 (m, 3H), 3.98 (d, *J =* 13.1 Hz, 2H), 3.95 (s, 3H), 3.66 (d, *J =* 12.7 Hz, 2H), 3.40 (d, *J =* 12.6 Hz, 2H), 3.25 - 3.19 (m, 2H), 2.97 - 2.91 (m, 2H), 2.89 (d, *J =* 5.4 Hz, 1H), 2.83 - 2.75 (m, 1H), 2.53 - 2.42 (m, 1H), 2.23 - 2.15 (m, 4H), 2.10 - 2.03 (m, 2H), 1.95 (d, *J* = 12.9 Hz, 2H), 1.50 (dd, *J* = 12.3, 3.7 Hz, 2H), 1.31 (s, 6H), 1.26 (s, 6H). LC-MS: [M+H]⁺ =845.76

### Example 348

### Synthesis of compound 305

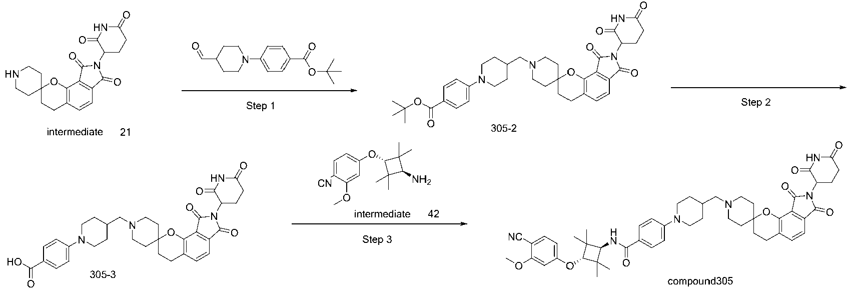

### Step 1: (Compound 305-2) was prepared with reference to step 1 of Example 267. LC-MS: [M+H]⁺ =657.33

### Step 2: (Compound 305-3) was prepared with reference to step 2 of Example 39. LC-MS: [M+H]⁺ =601.34

Step 3: (Compound 305) was prepared with reference to step 3 of Example 267.
¹H NMR (600 MHz, Methanol-d4) δ 7.77 (dd, *J =* 9.7, 2.7 Hz, 2H), 7.62 (d*, J =* 7.5 Hz, 1H), 7.54 (d, *J =* 8.6 Hz, 1H), 7.43 (d, *J =* 7.5 Hz, 1H), 7.07 - 7.03 (m, 2H), 6.65 (d, *J =* 2.2 Hz, 1H), 6.58 (dd, *J =* 8.6, 2.2 Hz, 1H), 5.10 (dd, *J =* 12.5, 5.5 Hz, 1H), 4.28 (d, *J =* 1.0 Hz, 1H), 4.16 - 4.14 (m, 1H), 3.98 (s, 1H), 3.95 (s, 3H), 3.61 (d, J = 12.2 Hz, 2H), 3.55 - 3.45 (m, 2H), 3.24 - 3.15 (m, 2H), 3.04 (t, *J =* 6.7 Hz, 2H), 2.99 - 2.90 (m , 2H), 2.88 - 2.81 (m, 1H), 2.77 - 2.69 (m, 2H), 2.28 - 2.18 (m, 4H), 2.17 - 2.11 (m, 1H), 2.10 - 2.03 (m, 4H), 1.98 (d, *J =* 13.5 Hz, 2H), 1.55 - 1.46 (m, 2H), 1.31 (s, 6H), 1.26 (s, 6H). LC-MS: [M+H]⁺=857.76

### Example 349

### Synthesis of compound 306

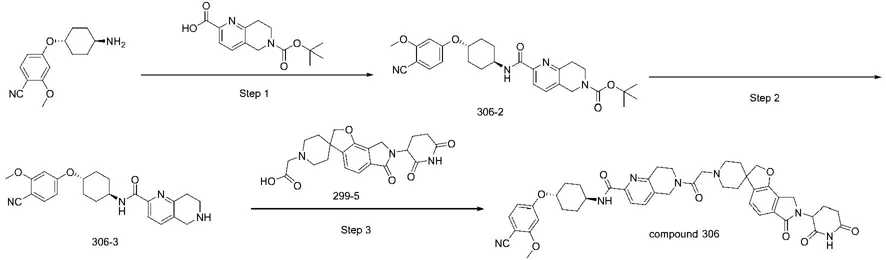

### Step 1: (Compound 306-2) was prepared with reference to step 3 of Example 267. LC-MS: [M+H]⁺ =511.39

### Step 2: (Compound 307-3) was prepared with reference to step 2 of Example 39. LC-MS: [M+H]⁺ =411.39

Step 3: (Compound 307) was prepared with reference to step 3 of Example 267.
¹H NMR (600 MHz, Methanol-d4) δ 7.99 (d, *J =* 8.1 Hz, 1H), 7.83 (d, *J=* 8.0 Hz, 1H), 7.53 (d, *J =* 8.4 Hz, 1H), 7.47 (d, *J* = 7.6 Hz, 1H), 7.41 (d, *J =* 7.7 Hz, 1H), 6.69 (d, *J =* 2.1 Hz, 1H), 6.68 - 6.66 (m, 1H), 5.17 (dd, *J =* 13.3, 5.1 Hz, 1H), 4.92 (s, 2H), 4.77 (s, 1H), 4.74 (s, 2H), 4.55 - 4.37 (m, 5H), 4.05 (t, *J =* 6.1 Hz, 1H), 4.02 - 3.96 (m, 1H), 3.94 (s, 3H), 3.85 (s, 1H), 3.78 (d, J = 12.4 Hz, 2H), 3.26 - 3.19 (m, 2H), 3.13 (t, *J =* 6.1 Hz, 1H), 2.97 - 2.88 (m, 1H), 2.83 - 2.76 (m, 1H), 2.58 - 2.48 (m, 1H), 2.46 - 2.36 (m, 2H), 2.27 - 2.21 (m, 2H), 2.22 - 2.17 (m, 1H), 2.17 - 2.08 (m, 4H), 1.73 - 1.58 (m, 4H). LC-MS: [M+H]⁺ =802.66

### Example 350

### Synthesis of compound 307

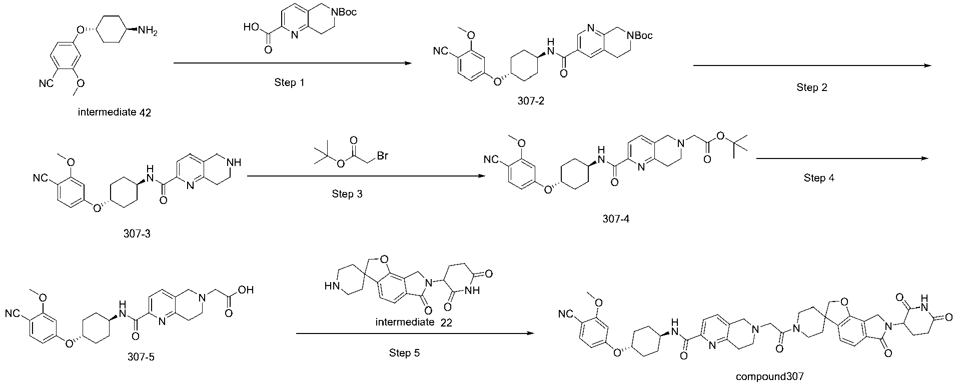

### Step 1: (Compound 307-2) was prepared with reference to step 3 of Example 267. LC-MS: [M+H]⁺ =525.49

### Step 2: (Compound 307-3) was prepared with reference to step 2 of Example 267. LC-MS: [M+H]⁺ =469.19

### Step 3: (Compound 307-4) was prepared with reference to step 3 of Example 343 LC-MS: [M+H]⁺ =521.49

### Step 4: Compound 307-5) was prepared with reference to step 2 of Example 267. LC-MS: [M+H]⁺ =465.39

Step 5: (Compound 307) was prepared with reference to step 3 of Example 267.
¹H NMR (400 MHz, Methanol-d4) δ 8.06 (d, J = 8.0 Hz, 1H), 7.87 (d, *J* = 8.1 Hz, 1H), 7.53 (d, 1H), 7.42 (d, *J* = 7.7 Hz, 1H), 7.38 (d, *J =* 7.7 Hz, 1H), 6.71 - 6.69 (m, 1H), 6.67 (s, 1H), 5.19 - 5.11 (m, 1H), 4.77 - 4.67 (m, 3H), 4.63 - 4.56 (m, 2H), 4.52 (s, 1H), 4.49 - 4.46 (m, 2H), 4.45 - 4.37 (m, 1H), 4.06 - 3.96 (m, 1H), 3.94 (s, 3H), 3.90 - 3.74 (m, 3H), 3.52 - 3.43 (m, 2H), 3.09 - 2.99 (m, 1H), 2.96 - 2.86 (m, 1H), 2.83 - 2.76 (m, 1H), 2.58 - 2.46 (m, 1H), 2.29 - 2.15 (m, 4H), 2.14 - 2.04 (m, 2H), 2.01 - 1.85 (m, 4H), 1.77 - 1.56 (m, 4H). LC-MS: [M+H]⁺ =802.56

### Example 351

### Synthesis of compound 308

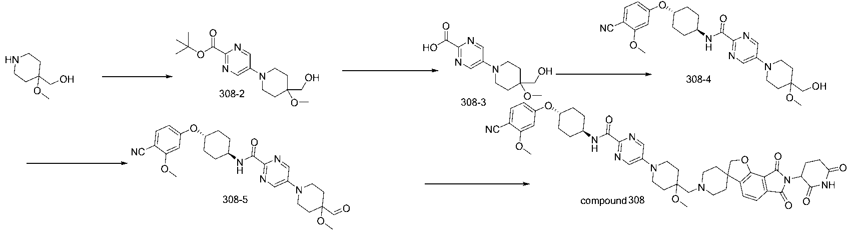

### Step 1: (Compound 308-2) LC-MS: [M+H]⁺ =324.18

In a 50 mL three-necked flask, (4-methoxypiperidin-4-yl)methanol (100.0 mg, 550.48 µmol), tert-butyl 5-bromopyrimidine-2-carboxylate (142.63 mg, 550.48 µmol), palladium acetate (12.36 mg, 55.05 µmol), 1,1'-binaphthyl-2,2'-bisdiphenylphosphine (73.26 mg, 110.49 µmol) and cesium carbonate (896.78 mg, 2.75 mmol) were added, then the solvent 1,4-dioxane was added, and the reaction solution was stirred at 110°C overnight under the protection of nitrogen, and when the reaction was completed, the reaction solution was concentrated and then purified by preparative thin-layer chromatography (DCM:MeOH=10:1) to obtain a white solid compound 308-2 (80 mg, 44.49%).

### Step 2: (Compound 308-3) was prepared with reference to step 2 of Example 267. LC-MS: [M+H]⁺ =268.12

Step 3: (Compound 308-4) was prepared with reference to step 3 of Example 267.

### Step 4: (Compound 308-5)

Compound 308-4 (100.0 mg, 201.8 µmol), 2-iodoacylbenzoic acid (84.8 mg, 302.7 µmol) were added sequentially to a 50 mL three-necked flask, the solvent acetonitrile was added and the reaction solution was stirred at 80°C for 2 h. When the reaction was completed, the reaction solution was concentrated and then purified by preparative thin-layer chromatography (DCM:MeOH=10:1) to obtain a white solid compound 308-5 (70 mg,70.29%).

Step 4: (Compound 308) was prepared with reference to step 1 of Example 267.
¹H NMR (600 MHz, Methanol-*d*₄) δ 8.57 (s, 2H), 7.62 (s, 1H), 7.52 (dd, *J =* 8.1, 4.8 Hz, 2H), 6.68 (d, *J* = 9.3 Hz, 2H), 5.13 (dd, *J =* 13.1, 5.6 Hz, 1H), 4.50 (s, 1H), 3.99 (s, 1H), 3.94 (d, *J =* 1.4 Hz, 3H), 3.85 (d, *J =* 13.5 Hz, 4H), 3.68 (s, 1H), 3.53 (s, 1H), 3.46 (s, 2H), 3.42 (s, 3H), 3.37 (s, 2H), 2.93 - 2.82 (m, 2H), 2.81 - 2.69 (m, 3H), 2.47 - 2.38 (m, 2H), 2.28 - 2.07 (m, 9H), 1.83 (t, *J =* 12.5 Hz, 2H), 1.67 (q, *J =* 11.0, 9.4 Hz, 4H). LC-MS: [M+H]⁺ =847.37

### Example 352

### Synthesis of compound 309

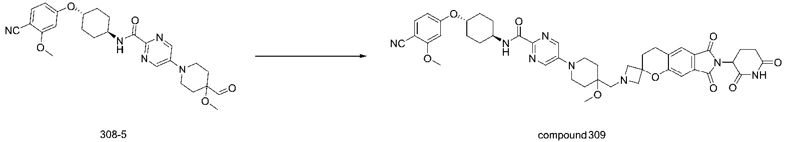

### Step 1: (Compound 309) was prepared with reference to step 1 of Example 267. LC-MS: [M+H]⁺ =833.35

¹H NMR (600 MHz, DMSO-*d*₆) δ 11.11 (s, 1H), 8.56 (s, 2H), 8.31 (d, *J =* 8.2 Hz, 1H), 7.76 (d, *J =* 23.7 Hz, 1H), 7.63 (d, *J =* 9.1 Hz, 1H), 7.28 (s, 1H), 6.72 (dq, *J =* 3.8, 2.2 Hz, 2H), 5.11 (dd, *J =* 13.1, 5.3 Hz, 1H), 4.59 (s, 1H), 4.52 - 4.47 (m, 1H), 4.36 (s, 2H), 3.89 (s, 2H), 3.82 (d, *J* = 9.9 Hz, 1H), 3.75 (s, 2H), 3.65 (s, 1H), 3.23 (s, 3H), 3.14 (s, 3H), 3.02 - 2.85 (m, 4H), 2.59 (s, 2H), 2.29 (d, *J =* 17.2 Hz, 2H), 2.12 (d, *J =* 11.6 Hz, 2H), 2.08 - 2.00 (m, 2H), 1.91 (d, *J* = 12.0 Hz, 2H), 1.83 (d, *J =* 13.4 Hz, 2H), 1.70 - 1.56 (m, 4H), 1.52 (q, *J =* 11.8 Hz, 2H).

### Example 353

### Synthesis of compound 310

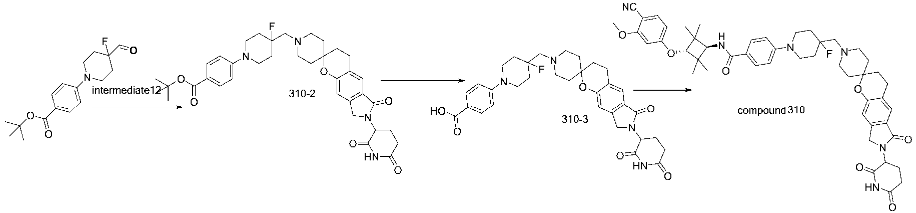

Step 1: (Compound 310-2) was prepared with reference to step 1 of Example 267.

Step 2: (Compound 310-3) was prepared with reference to step 2 of Example 267.

Step 3: (Compound 310) was prepared with reference to step 3 of Example 267.
¹H NMR (600 MHz, *DMSO-d*₆) δ 10.97 (s, 1H), 7.79 (d, *J =* 8.6 Hz, 2H), 7.65 (d, *J =* 8.6 Hz, 1H), 7.58 - 7.50 (m, 2H), 7.05 (d, *J =* 8.8 Hz, 3H), 6.64 (d *J = 2.2* Hz, 1H), 6.54 (dd, *J =* 8.6, 2.2 Hz, 1H), 5.06 (dd, *J =* 13.3, 5.2 Hz, 1H), 4.36 (d, *J =* 16.8 Hz, 1H), 4.28 (s, 1H), 4.23 (d, *J=* 16.8 Hz, 1H), 4.06 (d, *J = 9.2* Hz, 1H) , 3.91 (s, 3H), 3.80 (dt, *J =* 13.4, 4.0 Hz, 2H), 3.63 (d, *J =* 22.8 Hz, 3H), 3.34 (s, 3H), 3.17 - 3.08 (m, 2H), 2.90 (tt, *J =* 10.6, 4.9 Hz, 3H), 2.60 (dt, *J =* 17.6, 3.1 Hz, 1H), 2.42 - 2.31 (m, 1H), 2.11 - 1.94 (m, 7H), 1.87 (dtd, *J =* 25.8, 13.6, 11.9, 6.7 Hz, 4H), 1.23 (s, 6H), 1.15 (s, 6H). LC-MS: [M+H]⁺=861.03

### Example 354

### Synthesis of compound 311

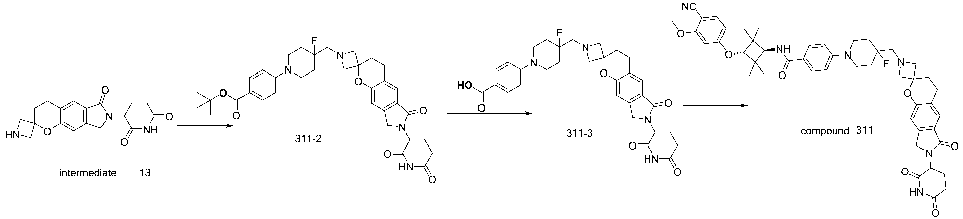

Step 1: (Compound 311-2) was prepared with reference to step 1 of Example 267.

Step 2: (Compound 311-3) was prepared with reference to step 2 of Example 267.

Step 3: Prepared with reference to step 3 of Example 267.
¹H NMR (600 MHz, DMSO-d₆) δ 10.97 (s, 1H), 7.78 (d, *J =* 8.7 Hz, 2H), 7.65 (d, *J =* 8.6 Hz, 1H), 7.57 - 7.51 (m, 2H), 7.07 (s, 1H), 7.04 (d, *J =* 8.9 Hz, 2H), 6.64 (d, *J = 2.2* Hz, 1H), 6.54 (dd, *J =* 8.6, 2.2 Hz, 1H), 5.06 (dd, *J =* 13.3, 5.1 Hz, 1H), 4.55 (s, 1H), 4.43 (s, 2H), 4.37 (d, *J =* 16.9 Hz, 2H), 4.30 - 4.20 (m, 2H), 4.06 (d, *J = 9.2* Hz, 1H), 3.91 (s, 3H), 3.76 (s, 4H), 3.07 (s, 2H), 2.98 - 2.84 (m, 3H), 2.60 (d, *J =* 16.7 Hz, 1H), 2.35 (td, *J =* 13.2, 4.6 Hz, 1H), 2.25 (s, 2H), 2.01 - 1.90 (m, 3H), 1.83 (d, *J =* 37.0 Hz, 2H), 1.22 (s, 6H), 1.15 (s, 6H). LC-MS: [M+H]⁺ =832.97

### Example 355

### Synthesis of compound 312

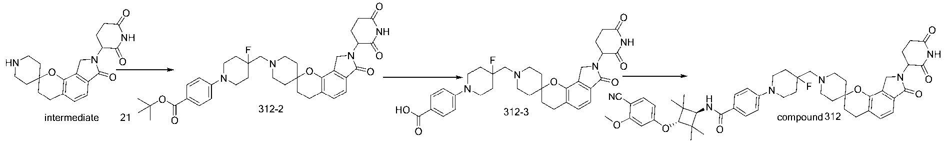

Step 1: (Compound 312-2) was prepared with reference to step 1 of Example 267.

Step 2: (Compound 312-3) was prepared with reference to step 2 of Example 267.

Step 3: (Compound 312) was prepared with reference to step 3 of Example 267.
¹H NMR (600 MHz, DMSO-d*₆*) δ 11.03 (s, 1H), 7.79 (d, *J =* 8.8 Hz, 2H), 7.65 (d, *J =* 8.6 Hz, 1H), 7.55 (d, *J =* 9.3 Hz, 1H), 7.29 (d, *J =* 7.7 Hz, 1H), 7.25 (d, *J* = 7.7 Hz, 1H), 7.05 (d, *J =* 8.8 Hz, 2H), 6.64 (d, *J =* 2.2 Hz, 1H), 6.54 (dd, *J =* 8.6, 2.2 Hz, 1H), 5.14 (dd, *J =* 13.3, 5.2 Hz, 1H), 4.45 (d, *J =* 17.5 Hz, 1H), 4.28 (s, 2H), 4.06 (d, *J* = 9.2 Hz, 1H), 3.91 (s, 3H), 3.80 (dd, *J =* 13.0, 3.8 Hz, 2H), 3.66 (s, 3H), 3.40 - 3.20 (m, 3H), 3.18 - 3.08 (m, 2H), 3.00 - 2.84 (m, 3H), 2.63 (d, *J* = 20.5 Hz, 1H), 2.43 - 2.30 (m, 1H), 2.12 - 1.95 (m, 7H), 1.94 - 1.81 (m, 4H), 1.23 (s, 6H), 1.15 (s, 6H). LC-MS: [M+H]⁺=861.03

### Example 356

### Synthesis of compound 317

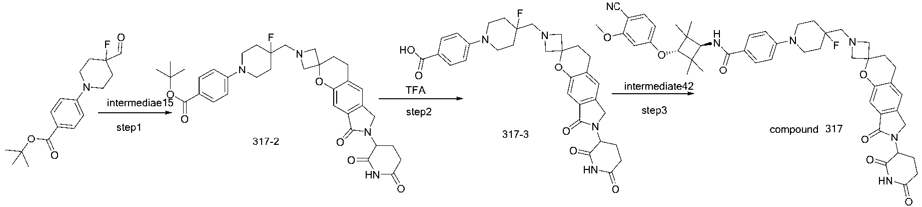

### Step 1: (Compound 317-2) was prepared with reference to step 1 of Example 257 LC-MS: [M-56+H]⁺ =577.48

### Step 2: (Compound 317-3) was prepared with reference to step 2 of Example 267. LC-MS: [M+H]⁺ =577.32

### Step 3: (Compound 317) was prepared with reference to step 3 of Example 267. LC-MS: [M+H]⁺ =833.66

¹ H NMR (600 MHz, DMSO-d6) δ 10.98 (s, 1H), 7.79 (s, 2H), 7.66 (s, 1H), 7.54 (s, 1H), 7.38 (s, 1H), 7.11 (s, 1H), 7.04 (d, *J* = 8.7 Hz, 2H), 6.64 (d, *J = 2.2* Hz, 1H), 6.55 (s, 1H), 5.07 (d, *J* = 5.2 Hz, 1H), 4.36 (d, *J =* 16.7 Hz, 2H), 4.28 (s, 1H), 4.23 (d, *J* = 16.7 Hz, 1H), 4.06 (d, *J* = 9.2 Hz, 1H), 3.93 (s, 3H), 3.75 (s, 4H), 3.08 (s 2H), 2.92 (d, *J =* 24.6 Hz, 4H), 2.60 (d, *J =* 15.6 Hz, 1H), 2.38 (s, 2H), 2.23 (s, 2H), 1.86 (d, *J =* 64.2 Hz, 6H), 1.23 (s, 6H), 1.15 (s, 6H).

### Example 357

### Synthesis of compound 319

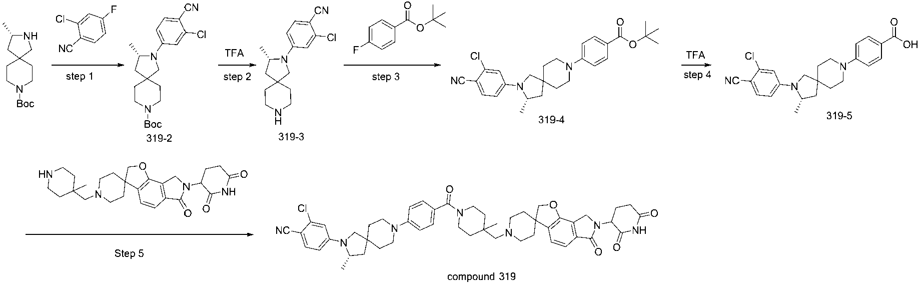

### Step 1: (Compound 319-2) LC-MS: [M-t-Bu+H]⁺=334.39

In a glass vial, tert-butyl compound (S)-3-methyl-2,8-diazaspiro[4.5]deca-8-carboxylate (4 g, 15.72 mmol), 2-chloro-4-fluorobenzonitrile (4.89 g, 31.45 mmol), and DIEA (6.1 g, 47.16 mmol) were added sequentially, and the solvent DMSO (40 mL) was added. The reaction solution was stirred at 100 °C for 4 h under nitrogen atmosphere. After the reaction was completed, the temperature was lowered to 0 °C. Water and ethyl acetate were added to extract the partition, and the residue obtained from concentration of the organic phase under vacuum was purified by normal-phase chromatography (PE:EA=4:1) to obain a yellow solid compound 319-2 (4.3 g, 70.13%).

### Step 2: (Compound 319-3) was prepared with reference to step 2 of Example 39. LC-MS: [M+H]⁺ =290.38

### Step 3: (Compound 319-4) LC-MS: [M+H]⁺ = 466.39

Compound 319-3 (1 g, 3.45 mmol), tert-butyl 4-fluorobenzoate (1.02 g, 5.18 mmol) and K₂CO₃ (2.4 g, 17.25 mmol) were added sequentially in a glass vial, the solvent DMSO (10 mL) was added, and the reaction solution was stirred at 100 °C for 12 h under nitrogen atmosphere. After the reaction was completed, the temperature was lowered to 0°C, water and ethyl acetate were added to extract the partition, and the residue obtained by concentration of the organic phase under vacuum was purified by normal-phase chromatography (PE:EA=4:1) to obtain a yellow solid compound 319-4 (660 mg, 41.04%).

Step 4: (Compounds 319-5) was prepared with reference to step 2 of Example 39.

Step 5: (Compound 319) was prepared with reference to step 3 of Example 267.
¹H NMR (600 MHz, DMSO) δ 10.99 (s, 1H), 7.60 (d, *J =* 8.8 Hz, 1H), 7.36 (d, *J* = 7.5 Hz, 1H), 7.29 (d, *J =* 8.4 Hz, 3H), 6.98 (d, *J* = 8.5 Hz, 2H), 6.80 (d, *J =* 1.8 Hz, 1H), 6.67 (dd, *J* = 8.9, 1.9 Hz, 1H), 5.10 (dd, *J =* 13.1, 5.1 Hz, 1H), 4.67 (q, *J =* 9.5 Hz, 2H), 4.41 (dd, *J =* 17.0, 8.8 Hz, 1H), 4.24 (d, *J =* 17.2 Hz, 2H), 4.07 - 4.01 (m, 5H), 3.58 (d, *J =* 9.6 Hz, 2H), 3.44 (d, *J =* 10.8 Hz, 1H), 3.34 (t, *J =* 13.0 Hz, 4H), 3.24 (dd, *J =* 20.8, 11.2 Hz, 5H), 3.15 (s, 1H), 2.96 - 2.87 (m, 1H), 2.60 (d, *J =* 16.9 Hz, 1H), 2.43 (dd, *J =* 13.2, 4.4 Hz, 1H), 2.37 (t, *J =* 12.9 Hz, 2H), 2.25 (dd, *J =* 12.7, 7.8 Hz, 1H), 2.02 - 1.97 (m, 1H), 1.91 (t, *J =* 14.5 Hz, 2H), 1.74 (dq, *J =* 13.1, 8.8 Hz, 2H), 1.59 (dd, *J =* 12.8, 6.4 Hz, 2H), 1.52 (dd, *J =* 13.7, 6.2 Hz, 3H), 1.24 (s, 3H), 1.21 (d, *J =* 5.9 Hz, 3H). LC-MS: [M+H]⁺ = 858.66

### Example 358

### Synthesis of compound 320

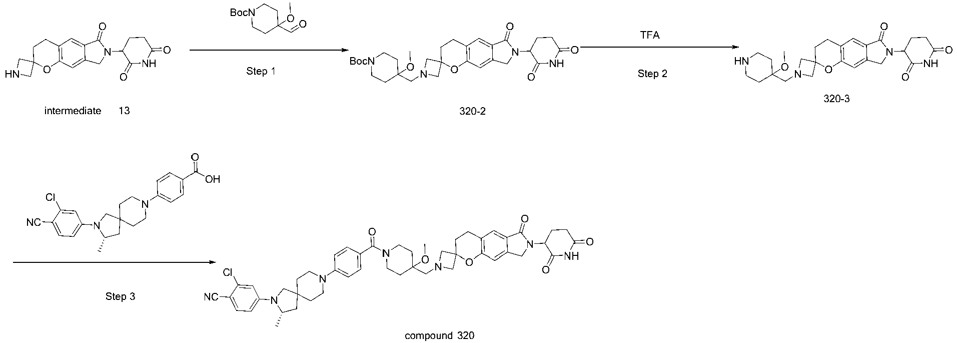

### Step 1: (Compound 320-2) was prepared with reference to step 1 of Example 267. LC-MS: [M+H]⁺ =569.48

### Step 2: (Compound 320-3) was prepared with reference to step 2 of Example 267. LC-MS: [M+H]⁺ =469.29

### Step 3: (Compound 320) was prepared with reference to step 3 of Example 267. LC-MS: [M+H]⁺ =860.66

¹H NMR (600 MHz, DMSO-d6) δ 10.97 (s, 1H), 7.60 (d, *J =* 8.9 Hz, 1H), 7.52 (s, 1H), 7.27 (dd, *J =* 8.7, 2.3 Hz, 2H), 7.08 (d, *J* = 8.2 Hz, 1H), 6.99 - 6.95 (m, 2H), 6.80 (d, *J* = 2.4 Hz, 1H), 6.66 (dd, *J* = 9.0, 2.4 Hz, 1H), 5.06 (dd, *J =* 13.3, 5.1 Hz, 1H), 4.55 (d, *J =* 10.7 Hz, 1H), 4.44 (s, 1H), 4.35 (t, *J =* 18.1 Hz, 3H), 4.24 (d, *J =* 17.0 Hz, 1H), 4.04 (m, 1H), 3.60 (s, 3H), 3.33 *(d, J =* 10.7 Hz, 4H), 3.21 (s, 6H), 3.15 (d, *J =* 10.2 Hz, 2H), 2.90 (m, 3H), 2.64 - 2.57 (m, 1H), 2.41 - 2.32 (m, 1H), 2.24 (dd, *J =* 12.7, 7.7 Hz, 3H), 2.02 - 1.95 (m, 1H), 1.74 (m, 4H), 1.60 - 1.47 (m, 5H), 1.21 (d, J = 6.1 Hz, 3H).

### Example 359

### Synthesis of compound 321

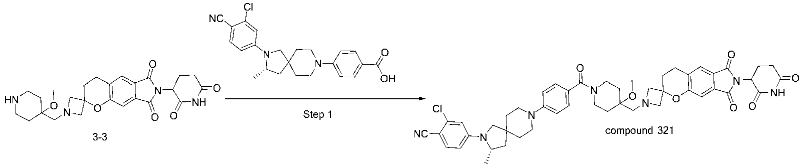

### Step 1: (Compound 321-2) was prepared with reference to step 1 of Example 267. LC-MS: [M+H]⁺ =583.68

### Step 2: (Compound 321-3) was prepared with reference to step 2 of Example 267. LC-MS: [M+H]⁺ =483.49

Step 3: (Compound 321) was prepared with reference to step 3 of Example 267.
¹H NMR (600 MHz, DMSO-d6) δ 11.11 (s, 1H), 7.77 (s, 1H), 7.60 (d, *J =* 8.9 Hz, 1H), 7.31 - 7.24 (m, 3H), 6.99 - 6.93 (m, 2H), 6.80 (d, *J =* 2.4 Hz, 1H), 6.66 (dd, *J* = 9.0, 2.4 Hz, 1H), 5.11 (dd, *J =* 12.9, 5.5 Hz, 1H), 4.56 (s, 1H), 4.48 (s, 1H), 4.35 (s, 2H), 4.04 (m, 1H),3.61 (s, 2H) 3.32 (d, *J =* 10.1 Hz, 4H), 3.26 - 3.17 (m, 6H), 3.14 (s, 2H), 3.03 - 2.82 (m, 4H), 2.64 - 2.57 (m, 1H), 2.57 - 2.52 (m, 1H), 2.31 - 2.20 (m, 3H), 2.08 - 2.00 (m, 1H), 1.74 (qt, J = 14.8, 6.1 Hz, 4H), 1.62 - 1.46 (m, 5H), 1.21 (d, J = 6.0 Hz, 3H). LC-MS: [M+H]⁺ =874.66

### Example 360

### Synthesis of compound 322

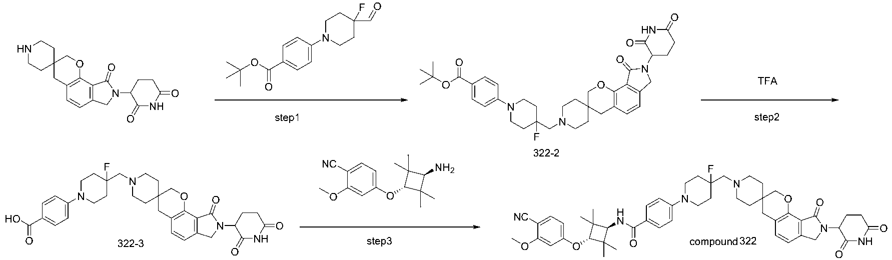

### Step 1: (Compound 322-2) was prepared with reference to step 1 of Example 267. LC-MS: [M-56+H]⁺ =605.48

### Step 2: (Compound 322-3) was prepared with reference to step 2 of Example 39. LC-MS: [M+H]⁺ =605.48

### Step 3: Compound 322) was prepared with reference to step 3 of Example 267. LC-MS: [M+H]⁺ =861.66

¹H NMR (600 MHz, Methanol-d4) δ 7.79 (d, *J =* 8.5 Hz, 2H), 7.54 (d, *J =* 8.6 Hz, 1H), 7.38 (s, 1H), 7.11 - 7.06 (m, 3H), 6.65 (d, *J = 2.2* Hz, 1H), 6.58 (dd, *J =* 8.6, 2.2 Hz, 1H), 5.11 (dd, *J =* 13.3, 5.2 Hz, 1H), 4.42 (q, *J =* 17.1 Hz, 3H), 4.28 (s, 1H), 4.15 (s, 1H), 4.06 (d, *J* = 9.7 Hz, 3H), 3.95 (s, 3H), 3.87 (d, *J* = 13.1 Hz, 2H), 3.60 (d, *J =* 21.0 Hz, 3H), 3.24 (t, *J* = 12.7 Hz, 3H), 2.91 (ddd, *J =* 18.5, 13.5, 5.4 Hz, 2H), 2.80 (ddd, *J =* 17.5, 4.6, 2.4 Hz, 2H), 2.49 (m, 2H), 2.19 - 2.12 (m, 3H), 2.01 (s, 1H), 1.94 (s, 4H), 1.87 (s, 2H), 1.30 (s, 6H), 1.26 (s, 6H).

### Example 361

### Synthesis of compound 323

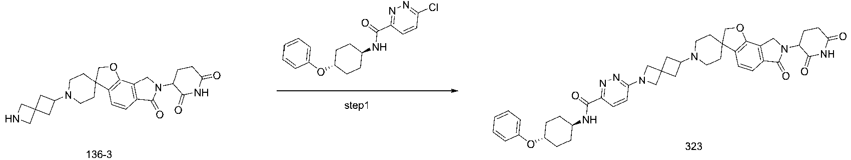

### Step 1: (Compound 323) was prepared with reference to Step 3 of Example 19 LC-MS: [M+H]+=746.66

¹H NMR (600 MHz, DMSO-d6) δ 10.98 (s, 1H), 8.51 (s, 1H), 7.85 (s, 1H), 7.35 (s, 1H), 7.29 (s, 3H), 6.92 (d, *J =* 30.9 Hz, 4H), 5.11 (s, 1H), 4.66 (s, 2H), 4.38 (s, 1H), 4.23 (s, 4H), 4.14 (s, 2H), 3.85 (s, 1H), 3.68 (s, 2H), 3.18 (d, *J* = 20.2 Hz, 1H), 2.91 (s, 3H), 2.66 (s, 2H), 2.58 (s, 3H), 2.41 (s, 1H), 2.11 (s, 4H), 1.98 (s, 3H), 1.90 (s, 2H), 1.60 (s, 2H), 1.47 (s, 2H).

### Example 362

### Synthesis of compound 324

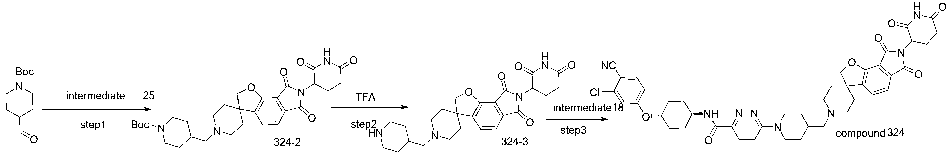

### Step 1: (Compound 324-2) was prepared with reference to step 1 of Example 267. LC-MS: [M+H]+ = 567.66

### Step 2: (Compound 324-3) was prepared with reference to step 2 of Example 267. LC-MS: [M+H]+ = 467.66

### Step 3: (Compound 324) was prepared with reference to step 3 of Example 19 LC-MS: [M+H]+=821.56

¹ H NMR (600 MHz, DMSO-d6) δ 11.11 (s, 1H), 8.57 (s, 1H), 7.84 (s, 2H), 7.57 (s, 1H), 7.51 (d, *J =* 7.3 Hz, 1H), 7.38 (s, 2H), 7.14 (s, 1H), 5.09 (d, *J =* 5.4 Hz, 1H), 4.81 (s, 2H), 4.51 (s, 4H), 3.85 (s, 2H), 3.06 (d, *J =* 10.5 Hz, 6H), 2.85 (s, 1H), 2.58 (s, 1H), 2.19 (s, 3H), 2.10 (s, 2H), 2.02 (s, 3H), 1.88 (d, *J =* 28.0 Hz, 4H), 1.63 (s, 2H), 1.51 (s, 2H), 1.25 (s, 3H).

### Example 363

### Synthesis of compound 325

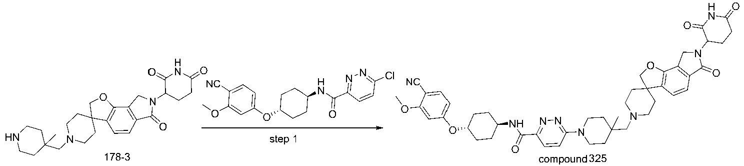

### Step 1: (Compound 325-2) was prepared with reference to step 1 of Example 267. LC-MS: [M+H]+ = 567.66

### Step 2: (Compound 325-3) was prepared with reference to step 2 of Example 267. LC-MS: [M+H]+ = 467.33

Step 3: (Compound 325) was prepared with reference to step 3 of Example 267.
¹ H NMR (600 MHz, DMSO-d6) δ 10.98 (s, 1H), 8.57 (s, 1H), 7.84 (s, 1H), 7.61 (s, 1H), 7.37 (d, *J =* 16.1 Hz, 2H), 7.29 (s, 1H), 6.71 (s, 2H), 5.07 (s, 1H), 4.66 (s, 2H), 4.49 (s, 1H), 4.38 (s, 1H), 4.22 (s, 1H), 4.11 (s, 2H), 3.88 (s, 4H), 3.58 (d, *J* = 12.6 Hz, 4H), 3.25 (q, *J* = 12.0 Hz, 2H), 3.19 - 3.13 (m, 2H), 2.88 (s, 1H), 2.63 - 2.54 (m, 1H), 2.36 (s, 3H), 2.11 (s, 2H), 1.89 (s, 5H), 1.63 (s, 6H), 1.50 (s, 2H), 1.28 (s, 3H). LC-MS: [M+H]+=817.66

### Example 364

### Synthesis of compound 326

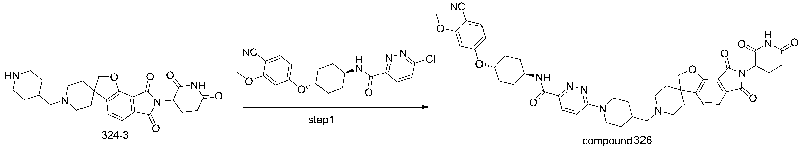

Step 1: (Compound 326) was prepared with reference to step 3 of Example 19.
¹H NMR (600 MHz, DMSO-d6) δ 11.12 (s, 1H), 8.59 (d, *J =* 8.2 Hz, 1H), 7.85 (d, *J* = 9.5 Hz, 1H), 7.64 - 7.60 (m, 1H), 7.58 (d, *J =* 7.2 Hz, 1H), 7.51 (d, *J =* 7.4 Hz, 1H), 7.41 (d, *J =* 9.6 Hz, 1H), 6.73 (d, *J =* 7.3 Hz, 2H), 5.12 (dd, *J* = 12.9, 5.4 Hz, 1H), 4.79 (d, *J=* 33.9 Hz, 2H), 4.58 - 4.46 (m, 3H), 3.90 (s, 4H), 3.64 - 3.61 (m, 4H), 3.09 (q, *J =* 11.2 Hz, 5H), 2.89 (ddd, *J =* 17.0, 13.8, 5.4 Hz, 1H), 2.64 - 2.57 (m, 1H), 2.29 - 2.17 (m, 3H), 2.17 - 2.10 (m, 2H), 2.03 (m, 3H), 1.95 - 1.83 (m, 4H), 1.65 (m, 2H), 1.57 - 1.46 (m, 2H), 1.27 (m, 2H). LC-MS: [M+H]+=817.66

### Example 365

### Synthesis of compound 327

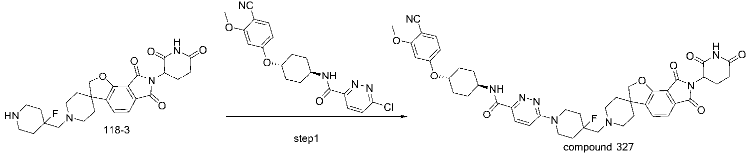

### Step 1: (Compound 327-2) was prepared with reference to step 1 of Example 267. LC-MS: [M+H]+ = 585.66

### Step 2: (Compound 327-3) was prepared with reference to step 2 of Example 267. LC-MS: [M+H]+ = 485.66

Step 3: Compound 327) was prepared with reference to step 3 of Example 19.
¹H NMR (600 MHz, DMSO-d₆) δ 11.11 (s, 1H), 8.61 (s, 1H), 7.89 (d, *J =* 9.5 Hz, 1H), 7.63 - 7.59 (m, 1H), 7.57 (s, 1H), 7.49 (d, *J =* 25.2 Hz, 2H), 6.71 (s, 2H), 5.08 (s, 1H), 4.80 (s, 2H), 4.48 (s, 1H), 4.38 (s, 2H), 3.88 (s, 3H), 3.87 - 3.83 (m, 1H), 2.85 (s, 2H), 2.74 (s, 1H), 2.69 - 2.55 (m, 2H), 2.38 (m, 1H), 2.27 (s, 3H), 2.07 (s, 5H), 1.98 (s, 4H), 1.90 (d, *J* = 11.0 Hz, 5H), 1.61 (s, 2H), 1.48 (s, 2H), 1.25 - 1.21 (m, 1H). LC-MS: [M+H]⁺ =835.76

### Example 366

### Synthesis of compound 328

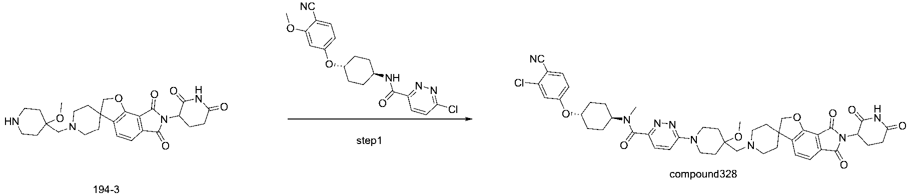

Step 1: (Compound 328) was prepared with reference to step 3 of Example 19.
¹H NMR (600 MHz, *DMSO-d*₆) δ 11.12 (s, 1H), 7.87 (dd, *J =* 12.2, 8.6 Hz, 1H), 7.60 - 7.48 (m, 3H), 7.46 - 7.31 (m, 2H), 7.10 (ddd, *J =* 36.6, 8.7, 2.4 Hz, 1H), 5.11 (dd, *J =* 12.9, 5.5 Hz, 1H), 4.82 (s, 2H), 4.54 (ddd, *J =* 21.6, 10.8, 5.3 Hz, 1H), 4.22 (d, *J =* 12.9 Hz, 2H), 3.86 (d, *J =* 13.0 Hz, 1H), 3.65 (d, *J =* 12.3 Hz, 3H), 3.33 - 3.23 (m, 8H), 2.95 - 2.86 (m, 4H), 2.64 - 2.57 (m, 1H), 2.34 (t, J = 13.4 Hz, 2H), 2.20 - 2.12 (m, 1H), 2.02 (dt, *J =* 39.2, 12.2 Hz, 6H), 1.94 - 1.78 (m, 3H), 1.76 - 1.61 (m, 3H), 1.56 (q, *J =* 12.2 Hz, 1H), 1.34 - 1.16 (m, 2H).LC-MS: [M+H]⁺ =865.56

### Example 367

### Synthesis of compound 329

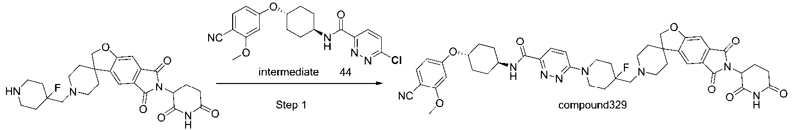

Step 1: (Compound 329) was prepared with reference to step 3 of Example 19.
1H NMR (600 MHz, Methanol-d4) δ 7.99 (d, *J =* 9.6 Hz, 1H), 7.77 (s, 1H), 7.55 - 7.51 (m, 1H), 7.44 (d, *J =* 9.6 Hz, 1H), 7.30 (s, 1H), 6.69 (d, *J =* 2.2 Hz, 1H), 6.67 (s, 1H), 5.13 (dd, J = 12.8, 5.4 Hz, 1H), 4.78 (s, 2H), 4.52 (d, 2H), 4.51 (s, 1H), 4.05 - 3.97 (m, 1H), 3.94 (s, 3H), 3.86 - 3.72 (m, 2H), 3.68 - 3.56 (m, 2H), 3.48 (t, *J =* 13.0 Hz, 2H), 2.93 - 2.85 (m, 1H), 2.81 - 2.74 (m, 2H), 2.74 - 2.67 (m, 1H), 2.45 - 2.37 (m, 2H), 2.28 - 2.21 (m, 4H), 2.21 - 2.16 (m, 2H), 2.15 - 2.07 (m, 4H), 2.03 - 1.92 (m, 2H), 1.67 (t, *J =* 9.5 Hz, 4H). LC-MS: [M+H]⁺ =835.66

### Example 368

### Synthesis of compound 330

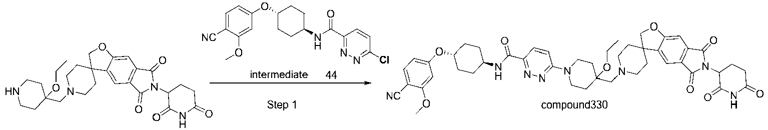

Step 1: (Compound 330) was prepared with reference to step 3 of Example 19.
¹H NMR (600 MHz, Methanol-d4) δ 8.00 (d, *J* = 9.6 Hz, 1H), 7.74 (s, 1H), 7.54 - 7.51 (m, 1H), 7.47 (d, *J =* 9.7 Hz, 1H), 7.29 (s, 1H), 6.69 - 6.67 (m, 1H), 6.67 (s, 1H), 5.13 (dd, J = 12.8, 5.5 Hz, 1H), 4.79 (s, 2H), 4.55 - 4.46 (m, 1H), 4.33 (d, *J=* 13.4 Hz, 2H), 4.04 - 3.97 (m, 1H), 3.93 (s, 3H), 3.91 - 3.84 (m, 2H), 3.65 (q, *J =* 6.9 Hz, 2H), 3.52 (t, *J* = 12.5 Hz, 2H), 3.46 (s, 2H), 3.37 - 3.34 (m, 1H), 2.93 - 2.85 (m, 1H), 2.81 - 2.69 (m, 2H), 2.48 - 2.40 (m, 2H), 2.24 (d, *J =* 3.8 Hz, 2H), 2.19 (d, *J =* 13.9 Hz, 2H), 2.15 - 2.10 (m, 4H), 1.85 - 1.75 (m, 2H), 1.69 - 1.62 (m, 4H), 1.32 (t, J = 6.9 Hz, 4H). LC-MS: [M+H]⁺ =861.66

### Example 369

### Synthesis of compound 331

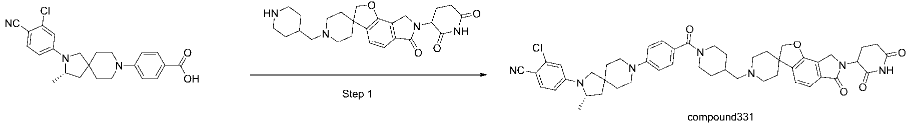

Step 1: (Compound 331) was prepared with reference to step 3 of Example 267.
¹H NMR (600 MHz, Methanol-d4) δ 7.51 (d, *J =* 8.8 Hz, 1H), 7.46 (d, *J =* 7.7 Hz, 1H), 7.40 (d, *J=* 8.5 Hz, 2H), 7.37 (d, *J =* 7.7 Hz, 1H), 7.15 (d, *J =* 8.4 Hz, 2H), 6.78 (d, *J =* 2.3 Hz, 1H), 6.68 (dd, *J* = 8.9, 2.4 Hz, 1H), 5.16 (dd, *J* = 13.4, 5.2 Hz, 1H), 4.72 (d, *J =* 3.0 Hz, 2H), 4.46 (q, 3H), 4.13 - 4.05 (m, 2H), 3.73 (d, *J =* 12.8 Hz, 2H), 3.48 (d, *J =* 10.6 Hz, 2H), 3.40 (d, *J* = 10.5 Hz, 2H), 3.38 - 3.34 (m, 1H), 3.22 - 3.19 (m, 2H), 3.17 (d, *J =* 7.6 Hz, 2H), 2.97 - 2.88 (m, 2H), 2.83 - 2.77 (m, 1H), 2.66 (d, *J =* 9.4 Hz, 1H), 2.57 - 2.48 (m, 1H), 2.42 - 2.36 (m, 1H), 2.35 - 2.25 (m, 3H), 2.24 - 2.16 (m, 2H), 2.15 - 2.09 (m, 2H), 2.05 (s, 1H), 1.97 -1.85 (m, 4H), 1.74 - 1.66 (m, 3H), 1.65 - 1.57 (m, 1H), 1.31 (d, *J =* 6.0 Hz, 3H). LC-MS: [M+H]+ =844.66[M+H]

### Example 370

### Synthesis of compound 332

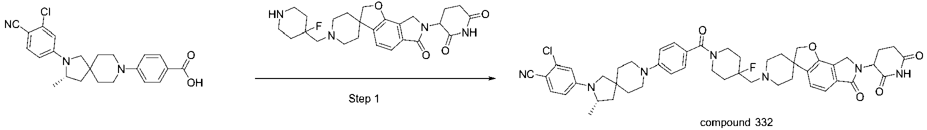

### Step 1: (Compound 332) was prepared with reference to step 3 of Example 267. LC-MS: [M+H]+ =862.66 [M+H]

¹H NMR (600 MHz, Methanol-d4) δ 7.50 (d, *J =* 8.8 Hz, 1H), 7.45 (dd, *J =* 8.1, 5.1 Hz, 3H), 7.20 - 7.16 (m, 2H), 6.78 (d, *J =* 2.3 Hz, 1H), 6.67 (dd, *J* = 8.9, 2.4 Hz, 1H), 5.16 (dd, *J =* 13.4, 5.1 Hz, 1H), 4.71 (s, 2H), 4.55 - 4.37 (m, 2H), 4.09 (q, *J* = 6.7 Hz, 1H), 3.79 (s, 2H), 3.61 (d, *J =* 20.1 Hz, 2H), 3.51 - 3.43 (m, 4H), 3.42 - 3.38 (m, 4H), 3.36 - 3.34 (m, 4H), 2.96 - 2.87 (m, 1H), 2.83 - 2.76 (m, 1H), 2.57 - 2.48 (m, 1H), 2.45 - 2.35 (m, 3H), 2.23 - 2.16 (m, 1H), 2.14 - 2.05 (m, 4H), 2.00 - 1.83 (m, 4H), 1.72 - 1.66 (m, 3H), 1.39 - 1.34 (m, 1H), 1.31 (d, *J* = 6.1 Hz, 3H).

### Example 371

### Synthesis of compound 333

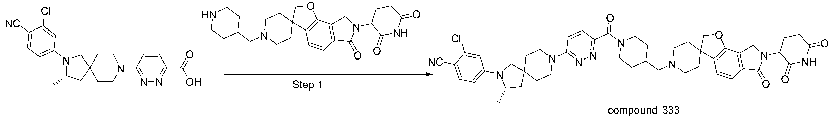

Step 1: (Compound 333) was prepared with reference to step 3 of Example 267.
¹H NMR (600 MHz, Methanol-d4) δ 7.68 (d, *J =* 9.7 Hz, 1H), 7.51 (dd, *J =* 9.3, 6.1 Hz, 2H), 7.46 (d, *J* = 7.7 Hz, 1H), 7.38 (d, *J =* 7.6 Hz, 1H), 6.79 (d, *J =* 2.3 Hz, 1H), 6.68 (dd, *J =* 8.9, 2.4 Hz, 1H), 5.16 (dd, *J =* 13.3, 5.1 Hz, 1H), 4.77 - 4.69 (m, 2H), 4.53 - 4.37 (m, 2H), 4.24 (d, *J =* 13.6 Hz, 1H), 4.14 - 4.07 (m, 1H), 3.93 - 3.84 (m, 2H), 3.83 - 3.71 (m, 4H), 3.53 - 3.40 (m, 4H), 3.35 (m, 1H), 3.22 - 3.19 (m, 1H), 3.19 - 3.15 (m, 2H), 3.02 - 2.95 (m, 1H), 2.93 - 2.88 (m, 1H), 2.84 - 2.76 (m, 1H), 2.57 - 2.48 (m, 1H), 2.45 - 2.39 (m, 1H), 2.37 - 2.28 (m, 3H), 2.23 - 2.17 (m, 2H), 2.16 - 2.09 (m, 2H), 2.07 - 1.98 (m, 2H), 1.93 - 1.81 (m, 3H), 1.75 - 1.69 (m, 1H), 1.67 - 1.60 (m, 2H), 1.37 - 1.34 (m, 1H), 1.32 (d, *J =* 6.1 Hz, 2H).LC-MS: [M+H]+ =846.66[M+H]

### Example 372

### Synthesis of compound 334

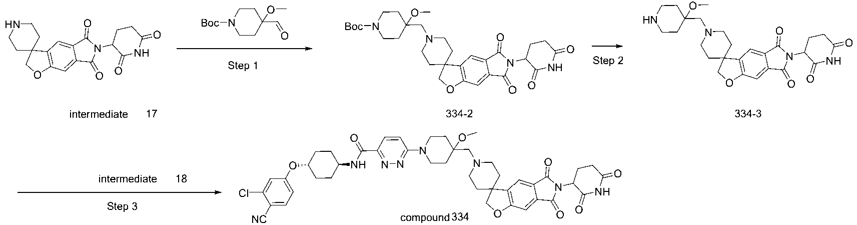

### Step 1: (Compound 334-2) was prepared with reference to step 3 of Example 267. LC-MS: [M+H]⁺ =597.28

### Step 2: Compound 334-3) was prepared with reference to step 2 of Example 39. LC-MS: [M+H]⁺ = 497.23

Step 3: Compound 334) was prepared with reference to step 3 of Example 19.
¹H NMR (600 MHz, DMSO) δ 11.11 (s, 1H), 8.62 (d, *J =* 8.1 Hz, 1H), 7.86 (d, *J =* 8.7 Hz, 2H), 7.45 - 7.35 (m, 3H), 7.17 -7. 12 (m, 2H), 5.33 (t, *J* = 4.8 Hz, 1H), 5.11 (d, *J =* 7.4 Hz, 2H), 4.76 (s, 2H), 4.54 (t, *J =* 10.1 Hz, 2H), 4.24 (s, 2H), 3.87 (d, *J* = 7.7 Hz, 2H), 3.64 (s, 2H), 2.88 (dd, *J =* 21.7, 9.4 Hz, 2H), 2.65 - 2.55 (m, 3H), 2.13 - 2.07 (m, 3H), 1.91 (d, *J* = 11.0 Hz, 4H), 1.73 - 1.41 (m, 11H), 0.86 (t, *J =* 6.9 Hz, 2H). LC-MS: [M+H]⁺ =851.32

### Example 373

### Synthesis of compound 335

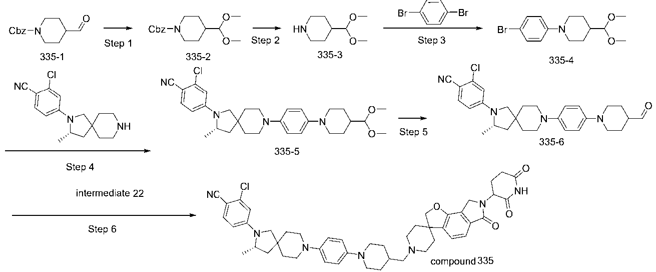

### Step 1: (Compound 335-2)

Benzyl 4-formylpiperidine-1-carboxylate (2 g, 8.097 mmol) and trimethyl orthoformate (2.57 g, 24.291 mmol) were added sequentially to an eggplant shaped flask, and the solvent MeOH (20 mL) was added, and hydrochloric acid (0.1 ml) was added dropwise, and then the reaction solution was stirred at 70°C for 2 h. After the reaction was completed, and the reaction solution was concentrated, and purified by preparative thin layer column chromatography (EA:PE=1:1) to obtain a transparent oily compound 335-2 (2 g, 84.38 %).

### Step 2: (Compound 335-3)

335-2 (2 g, 6.825 mmol) and Pd(OH)₂/C (400 mg) were added sequentially in an eggplant shaped flask, and the solvent MeOH (20 mL) was added, and the reaction solution was stirred at room temperature for 3 h under H₂ atmosphere. After completion of the reaction, the reaction solution was filtered and concentrated, and spun dry to obtain a yellow oily compound 335-3 (800 mg, 73.73 %).

Step 3: (Compound 335-4) LC-MS: [M+H]⁺ =314.07

335-3 (800 mg, 5.031 mmol), 1,4-dibromobenzene (3.52 g, 15.093 mmol), Pd(OAc)₂ (113.19 mg, 0.503 mmol), BINAP (625.73 mg, 1.006 mmol), and Cs₂CO₃ (4.9 g, 15.093 mmol) were added sequentially in an eggplant shaped flask, then the solvent dioxane (8 mL) was added, and the reaction solution was stirred at 110°C for 2 h under N₂ atmosphere. After the reaction was completed, the reaction solution was filtered and concentrated, and then purified by preparative thin-layer column chromatography (EA:PE=1:5) to obtain a yellow solid compound 335-4 (730 mg, 44.58 %).

Step 4: (Compound 335-5) LC-MS: [M+H]⁺ =523.28

In an eggplant shaped flask, 335-3 (730 mg, 2.325 mmol), (S)-2-chloro-4-(3-methyl-2,8-diazaspiro[4.5]decan-2-yl)benzonitrile (806 mg, 2.79 mmol), Pd₂(dba)₃ (213.43 mg, 0.233 mmol), BINAP (289.85 mg, 0.466 mmol) and tBuOK (780 mg, 6.97 mmol) were added sequentially, then solvent toluene (8 mL) was added, and the reaction solution was stirred at 110°C for 2 h under the atmosphere of N₂, and after the reaction was completed, the reaction solution was filtered and concentrated, and purified by preparative thin-layer column chromatography (EA:PE=1:1) to obtain a yellow solid compound 335-5 (190 mg, 15.83 %).

Step 5: (Compound 335-6)

335-5 (190 mg, 0.363 mmol), TFA (0.4 ml) and H₂O (0.1 ml) were added sequentially in an eggplant shaped flask, the solvent DCM (0.2 mL) was added and the reaction solution was stirred at room temperature for 1 h. After the reaction was completed, it was concentrated, and spun dry to obtain a crude blue solid compound 335-6 (220 mg).

Step 6: (Compound 335)

In a glass vial, 335-6 (20.0 mg, 0.042 mmol) and the intermediate 22 (17.89 mg, 0.050 mmol) were added sequentially, and the solvent THF (1 mL) was added, and the reaction solution was stirred at 60°C for 1 h. Then NaBH₃CN (7.938 mg, 0.126 mmol) was added, and the reaction solution was continued to be stirred at 60°C for 1 h. After the reaction was completed, it was concentrated, and then purified by column chromatography preparation to obtain a white solid compound 335 (3 mg, 8.8 %).
¹H NMR (600 MHz, DMSO) δ 10.98 (s, 1H), 7.60 (d, *J =* 8.9 Hz, 2H), 7.20 (s, 1H), 6.85 (s, 3H), 6.80 (d, *J* = 2.2 Hz, 1H), 6.66 (dd, *J* = 8.9, 2.2 Hz, 2H), 5.10 (dd, *J =* 13.3, 5.0 Hz, 1H), 3.52 (s, 3H), 3.09 (s, 4H), 3.01 - 2.87 (m, 5H), 2.64 - 2.54 (m, 4H), 2.44 - 2.37 (m, 3H), 2.23 (dd, *J =* 12.6, 7.8 Hz, 2H), 2.04 - 1.95 (m, 5H), 1.85 - 1.72 (m, 5H), 1.60 - 1.43 (m, 6H), 1.32 - 1.27 (m, 4H), 1.21 (d, *J =* 6.1 Hz, 2H). LC-MS: [M+H]⁺ =816.39

### Example 374

### Synthesis of compound 336

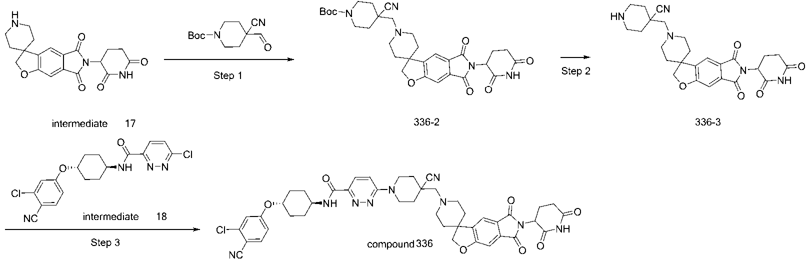

### Step 1: (Compound 336-2) was prepared with reference to step 1 of Example 267. LC-MS: [M+H]⁺ =592.27

### Step 2: (Compound 336-3) was prepared with reference to step 2 of Example 39. LC-MS: [M+H]⁺ =492.22

### Step 3: (Compound 336) was prepared with reference to step 3 of Example 19 LC-MS: [M+H]⁺ =846.31

¹H NMR (600 MHz, DMSO) δ 8.63 (d, *J =* 8.4 Hz, 1H), 7.89 - 7.83 (m, 1H), 7.45 - 7.35 (m, 2H), 7.27 (t, *J =* 10.8 Hz, 1H), 7.13 (d, *J =* 8.8 Hz, 1H), 6.84 (s, 1H), 6.66 (d, *J =* 13.2 Hz, 2H), 5.35 - 5.30 (m, 1H), 5.08 (dd, *J =* 12.9, 5.5 Hz, 1H), 4.65 - 4.51 (m, 2H), 3.86 (s, 1H), 3.41 - 3.26 (m, 6H), 3.20 - 3.14 (m, 1H), 3.00 - 2.73 (m, 4H), 2.67 - 2.52 (m, 2H), 2.41 (d, *J =* 18.5 Hz, 4H), 2.12 - 1.84 (m, 5H), 1.56 (m, 6H), 1.31 - 1.25 (m, 1H), 1.11 - 1.03 (m, 1H).

### Example 375

### Synthesis of compound 337

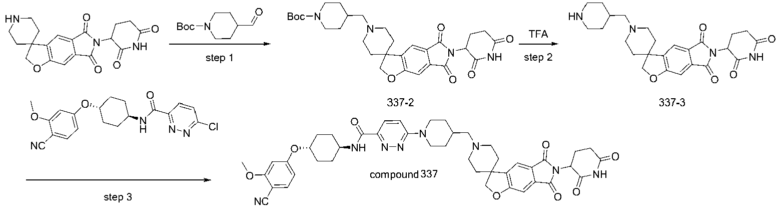

### Step 1: (Compound 337-2) was prepared with reference to step 1 of Example 267. LC-MS: [M+H]⁺ =567.40

### Step 2: (Compound 337-3) was prepared with reference to step 2 of Example 39. LC-MS: [M+H]⁺ =467.39

Step 3: (Compound 337) was prepared with reference to step 3 of Example 19.
¹H NMR (600 MHz, *DMSO-d₆*) δ 11.11 (s, 1H), 8.58 (d, *J =* 8.4 Hz, 1H), 7.83 (d, *J =* 9.6 Hz, 1H), 7.64 - 7.56 (m, 2H), 7.37 (s, 2H), 6.70 (s, 2H), 5.10 (dd, *J =* 12.8, 5.7 Hz, 1H), 4.74 (s, 2H), 4.51 (s, 3H), 3.88 (s, 3H), 3.59 (d, *J =* 12.1 Hz, 2H), 3.17 - 3.02 (m, 7H), 2.87 (d, *J =* 12.6 Hz, 1H), 2.61- 2.60 (m, 1H), 2.31 (t, *J=* 13.5 Hz, 2H), 2.22 (s, 2H), 2.11 (d, *J=* 11.6 Hz, 2H), 2.04-1.99 (m, 3H), 1.88 (t, *J =* 13.7 Hz, 4H), 1.62 (t, *J =* 12.3 Hz, 2H), 1.51 (q, *J =* 11.7 Hz, 2H), 1.30 - 1.21 (m, 2H). LC-MS: [M+H]⁺ =817.

### Example 376

### Synthesis of compound 338

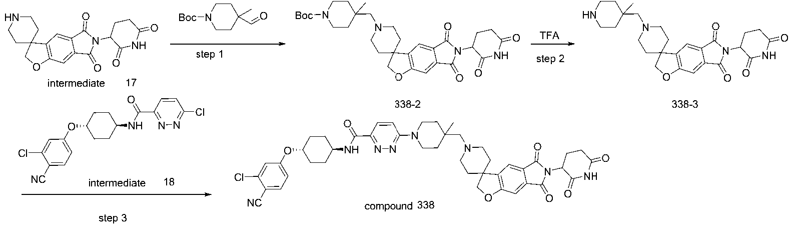

### Step 1: (Compound 338-2) was prepared with reference to step 1 of Example 267. LC-MS: [M-tBu+H]⁺ =481.39

### Step 2: (Compound 338-3) was prepared with reference to step 2 of Example 39. LC-MS: [M+H]⁺ =481.39

Step 3: Compound 338) was prepared with reference to step 3 of Example 19.
¹H NMR (600 MHz, DMSO-*d₆*) δ 11.10 (d, *J =* 4.1 Hz, 1H), 8.59 (dd, *J =* 8.2, 2.7 Hz, 1H), 7.84 (dd, *J = 9.2,* 7.1 Hz, 2H), 7.6-7.5 (s, 1H), 7.42 - 7.29 (m, 3H), 7.12 (dd, *J* = 8.7, 2.4 Hz, 1H), 5.09 (dd, *J =* 12.7, 6.0 Hz, 1H), 4.75 (d, *J =* 10.1 Hz, 2H), 4.52 (dt, *J =* 10.8, 5.9 Hz, 1H), 4.21 - 4.07 (m, 2H), 3.93 - 3.80 (m, 1H), 3.57 (d, *J =* 12.4 Hz, 2H), 3.45 (d, *J =* 5.1 Hz, 4H), 3.26 (d, *J* = 12.2 Hz, 3H), 3.15 (s, 2H), 2.87 (t, *J =* 13.0 Hz, 1H), 2.10 (d, *J =* 11.8 Hz, 2H), 2.02 (d, *J =* 5.9 Hz, 1H), 1.92 (dd, *J=* 36.5, 12.7 Hz, 4H), 1.69 - 1.55 (m, 6H), 1.52 (t, *J =* 11.7 Hz, 2H), 1.33 - 1.18 (m, 4H). LC-MS: [M+H]⁺=835.66

### Example 377

### Synthesis of compound 339

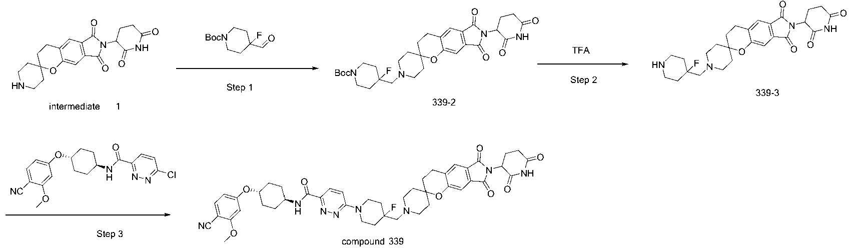

### Step 1: (Compound 339-2) was prepared with reference to step 1 of Example 267. LC-MS: [M+H]⁺ = 599.48

### Step 2: (Compound 339-3) was prepared with reference to step 2 of Example 267. LC-MS: [M+H]⁺ = 499.49

### Step 3: (Compound 339) was prepared with reference to step 3 of Example 19 LC-MS: [M+H]⁺ =849.66

¹H NMR (600 MHz, DMSO-*d*₆) δ 11.11 (s, 1H), 8.63 (d, *J* = 8.2 Hz, 1H), 7.89 (d, *J* = 9.5 Hz, 1H), 7.76 (s, 1H), 7.70 -7.56 (m, 1H), 7.48 (d, *J* = 9.6 Hz, 1H), 7.37 (d, *J =* 48.1 Hz, 1H), 6.79 - 6.49 (m, 2H), 5.10 (dd, *J =* 12.9, 5.5 Hz, 1H), 4.50 m, 1H), 4.39 (d, *J =* 13.3 Hz, 2H), 3.90 (m, 4H), 3.73 - 3.58 (m, 7H), 3.41 - 3.34 (m, 3H), 2.96 (t, *J* = 6.9 Hz, 2H), 2.89 (ddd, J = 17.0, 13.9, 5.5 Hz, 1H), 2.63 - 2.58 (m, 1H), 2.58 - 2.52 (m, 1H), 2.20 - 2.11 (m, 2H), 2.10 - 1.95 (m, 6H), 1.94 - 1.78 (m, 5H), 1.65 (m, 2H), 1.58 - 1.47 (m, 2H).

### Example 378

### Synthesis of compound 340

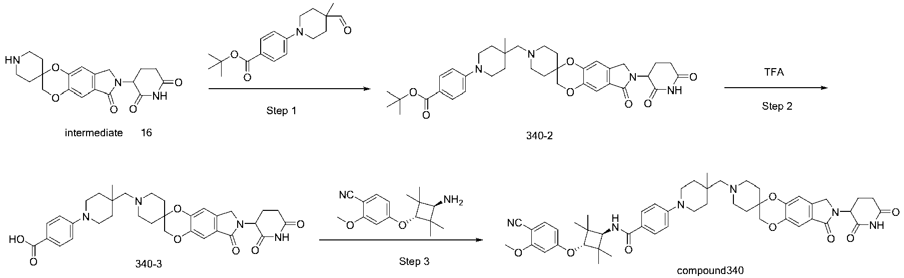

### Step 1: (Compound 340-2) was prepared with reference to step 1 of Example 267. LC-MS: [M+H]⁺ = 659.57

### Step 2: (Compound 340-3) was prepared with reference to step 2 of Compound 267. LC-MS: [M+H]⁺ = 603.58

Step 3: (Compound 340) was prepared with reference to step 3 of Example 267.
¹H NMR (600 MHz, DMSO-*d*₆) δ 10.97 (s, 1H), 7.78 (d, *J =* 8.5 Hz, 2H), 7.65 (d, *J =* 8.6 Hz, 1H), 7.52 (d, *J = 9.2* Hz, 1H), 7.24 (s, 1H), 7.17 (s, 1H), 7.00 (d, *J =* 8.6 Hz, 2H), 6.64 (d, *J =* 2.2 Hz, 1H), 6.54 (dd, *J* = 8.6, 2.2 Hz, 1H), 5.08 (dd, *J =* 13.4, 4.9 Hz, 1H), 4.34 (dd, *J =* 16.9, 7.0 Hz, 2H), 4.28 (s, 1H), 4.23 (td, *J =* 16.6, 16.0, 7.6 Hz, 2H), 4.16 - 4.09 (m, 2H), 4.06 (d, *J* = 9.2 Hz, 1H), 3.91 (s, 3H), 3.63 - 3.43 (m, 3H), 3.39 - 3.20 (m, 4H), 3.19 - 3.09 (m, 2H), 2.91 (ddd, J = 18.0, 13.5, 5.5 Hz, 1H), 2.65 - 2.57 (m, 1H), 2.36 (qd, *J =* 13.1, 4.2 Hz, 1H), 2.16 (t, *J =* 13.1 Hz, 2H), 2.05 - 1.87 (m, 2H), 1.69 (td, *J =* 12.2, 10.6, 4.3 Hz, 2H), 1.64 - 1.55 (m, 2H), 1.22 (d, J = 7.0 Hz, 9H), 1.15 (s, 6H).LC-MS: [M+H]⁺ = 859.66

### Example 379

### Synthesis of compound 341

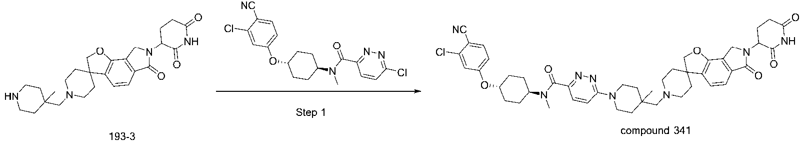

### Step 3: (Compound 341) was prepare with reference to step 3 of Compound 19.

¹H NMR (600 MHz, Methanol- *d*₄) δ 7.77 - 7.64 (m, 2H), 7.57 (dd, J = 19.8, 9.6 Hz, 1H), 7.45 (t, *J* = 6.6 Hz, 1H), 7.37 (d, *J* = 14.3 Hz, 1H), 7.20 (dd, *J =* 35.4, 2.4 Hz, 1H), 7.04 (ddd, *J =* 31.1, 8.8, 2.4 Hz, 1H), 5.16 (dd, *J =* 13.4, 5.1 Hz, 1H), 4.73 (s, 2H), 4.59 - 4.37 (m, 3H), 4.20 (d, *J=* 13.7 Hz, 2H), 3.98 (m, 1H), 3.77 (d, *J =* 12.8 Hz, 2H), 3.70 - 3.44 (m, 3H), 3.33 (m, 3H), 3.27 (s, 1H), 3.06 (d, *J =* 4.8 Hz, 3H), 2.92 (ddd, *J =* 17.4, 13.6, 5.4 Hz, 1H), 2.80 (ddd, *J* = 17.6, 4.7, 2.4 Hz, 1H), 2.52 (m, 3H), 2.30 (d, *J =* 12.5 Hz, 1H), 2.19 (d, J = 5.5 Hz, 2H), 2.08 (d, *J =* 14.3 Hz, 2H), 2.02 - 1.96 (m, 2H), 1.93 (m, 1H), 1.83 (d, *J =* 35.4 Hz, 4H), 1.68 (m, 1H), 1.41 (s, 4H). LC-MS: [M+H]⁺ =835.66

### Example 380

### Synthesis of compound 342

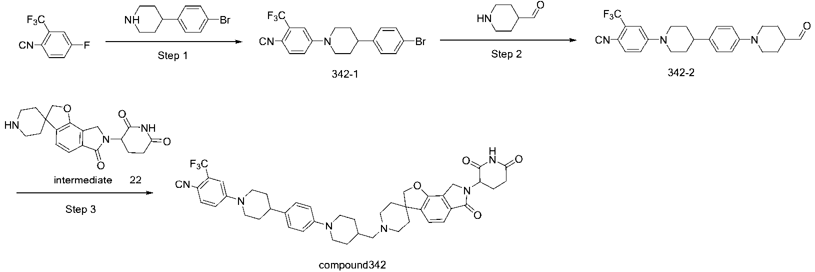

### Step 1: (Compound 342-1) LC-MS: [M+H]⁺ =410.2

4-Fluoro-2-trifluoromethylbenzonitrile (150.0 mg, 793.18 µmol) and 4-(4-bromophenyl)piperidine (228.57 mg, 951.81 µmol) were sequentially added to a glass vial, and the solvent DMSO (2 mL), potassium carbonate (328.86 mg, 2.38 mmol) were added, and the reaction solution was stirred at 80 °C for 2 h, and then a white solid compound 342-1 (196 mg, 60.38%) was obtained by preparative purification.

### Step 2: (Compound 342-2) LC-MS: [M+H]⁺ =488.29

342-1 (190.0 mg, 464.26 µmol) and 4-piperidine carboxaldehyde (105.07 mg, 928.53 µmol) were added sequentially in a glass vial, and the solvent Dioxane (2 mL), cesium carbonate (453.80 mg, 1.39 mmol) were added, and the reaction solution was stirred at 100 °C for 12 h. A white solid compound 342-1 (77 mg, 37.57%) was obtained by preparative purification.

Step 3: (Compound 342) was prepared with reference to step 1 of Example 267.
¹H NMR (600 MHz, Methanol-d4) δ 7.72 (d, *J =* 8.8 Hz, 1H), 7.46 (d, *J* = 7.5 Hz, 1H), 7.39 (s, 1H), 7.31 (s, 1H), 7.30 (s, 2H), 7.23 (d, *J =* 2.7 Hz, 1H), 7.22 (d, *J =* 2.8 Hz, 2H), 5.17 (dd, *J =* 13.3, 5.2 Hz, 1H), 4.73 (s, 2H), 4.53 - 4.41 (m, 2H), 4.18 (d, *J =* 12.9 Hz, 2H), 3.74 (d, *J =* 11.7 Hz, 4H), 3.27 - 3.16 (m, 4H), 3.15 - 3.08 (m, 2H), 2.97 - 2.89 (m, 1H), 2.88 - 2.77 (m, 2H), 2.57 - 2.48 (m, 1H), 2.40 - 2.31 (m, 2H), 2.26 - 2.16 (m, 3H), 2.16 - 2.07 (m, 4H), 1.97 (d, *J =* 13.2 Hz, 2H), 1.83 - 1.74 (m, 2H), 1.72 - 1.60 (m, 2H). LC-MS: [M+H]⁺ =761.66

### Example 381

### Compound 343 synthesis

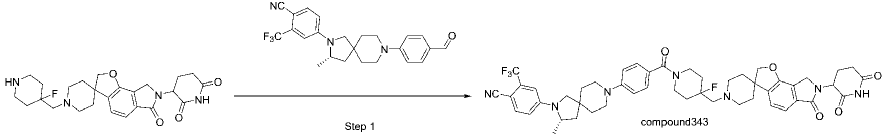

Step 1: (Compound 343) was prepared with reference to step 1 of Example 267.
¹H NMR (600 MHz, Methanol-d4) δ 7.69 (d, *J =* 8.7 Hz, 1H), 7.56 (d, *J =* 8.2 Hz, 2H), 7.45 (d, 1H), 7.43 (d, *J* = 8.3 Hz, 2H), 7.38 (d, *J =* 7.6 Hz, 1H), 7.01 (d, *J* = 2.5 Hz, 1H), 6.94 (dd, *J* = 8.8, 2.5 Hz, 1H), 5.16 (dd, *J =* 13.4, 5.1 Hz, 1H), 4.73 (s, 2H), 4.54 - 4.39 (m, 3H), 4.22 - 4.14 (m, 2H), 3.81 (d, *J =* 12.9 Hz, 2H), 3.65 - 3.56 (m, 5H), 3.51 (d, *J* = 10.7 Hz, 2H), 2.97 - 2.87 (m, 2H), 2.84 - 2.76 (m, 2H), 2.57 - 2.44 (m, 2H), 2.43 - 2.35 (m, 2H), 2.24 - 2.16 (m, 2H), 2.11 (d, *J =* 15.9 Hz, 4H), 2.05 - 1.98 (m, 2H), 1.86 - 1.80 (m, 2H), 1.78 (dd, *J =* 13.0, 7.0 Hz, 2H), 1.66 - 1.58 (m, 2H), 1.48 (s, 1H), 1.34 (d, *J* = 6.1 Hz, 3H).LC-MS: [M+H]⁺ =896.56

### Example 382

### Synthesis of compound 344

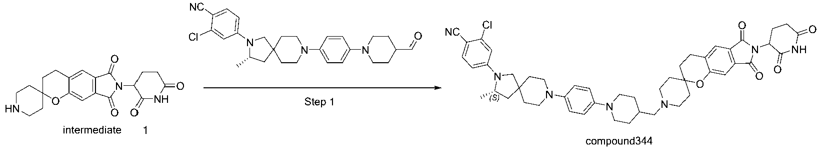

Step 1: (Compound 344) was prepared with reference to step 1 of Example 267.
¹H NMR (600 MHz, Methanol-d4) δ 7.71 (s, 1H), 7.52 (d, *J =* 8.8 Hz, 1H), 7.44 (d, 2H), 7.42 (s, 1H), 7.23 (d, *J =* 8.7 Hz, 2H), 6.82 (d, *J* = 2.4 Hz, 1H), 6.70 (dd, *J* = 8.9, 2.4 Hz, 1H), 5.12 (dd, *J =* 12.8, 5.4 Hz, 1H), 4.15 - 4.08 (m, 1H), 3.85 (d, *J =* 12.4 Hz, 2H), 3.62 (d, *J =* 12.3 Hz, 2H), 3.59 - 3.51 (m, 4H), 3.47 (d, *J =* 10.5 Hz, 1H), 3.45 - 3.38 (m, 2H), 3.23 (d, *J =* 6.9 Hz, 2H), 3.09 (s, 2H), 3.05 (t, *J =* 6.8 Hz, 2H), 2.92 - 2.85 (m, 1H), 2.80 - 2.69 (m, 2H), 2.45 (s, 1H), 2.27 - 2.16 (m, 4H), 2.15 - 2.07 (m, 4H), 2.06 - 1.99 (m, 4H), 1.86 (s, 2H), 1.77 (dd, *J =* 13.0, 6.9 Hz, 1H), 1.64 - 1.54 (m, 2H), 1.35 (s, 1H), 1.32 (d, *J =* 6.1 Hz, 3H).LC-MS: [M+H]⁺ =844.76

### Example 383

### Synthesis of compound 345

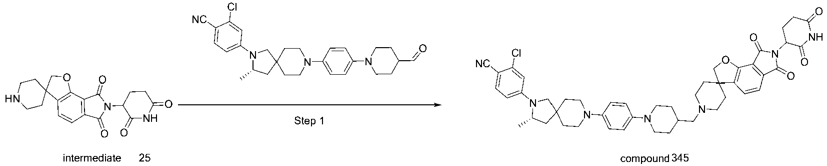

### Step 1: (Compound 345) was prepared with reference to step 1 of Example 267. LC-MS: [M+H]⁺ =830.43

¹H NMR (600 MHz, DMSO-*d*₆) δ 11.12 (s, 1H), 7.63 - 7.48 (m, 3H), 6.97 - 6.63 (m, 6H), 5.11 (dd, *J =* 12.9, 5.4 Hz, 1H), 4.79 (s, 2H), 4.04 (dd, *J =* 13.0, 6.4 Hz, 1H), 3.58 (d, *J =* 32.2 Hz, 4H), 3.38 (d, *J =* 15.6 Hz, 2H), 3.33 - 3.30 (m, 4H), 3.17 - 2.84 (m, 9H), 2.69 - 2.56 (m, 3H), 2.22 (s, 3H), 2.07 - 1.92 (m, 4H), 1.90 - 1.69 (m, 4H), 1.61 - 1.47 (m, 3H), 1.37 (s, 2H).

### Example 384

### Synthesis of compound 346

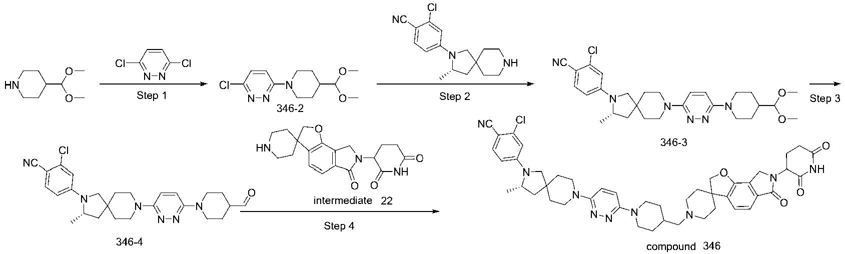

### Step 1: (Compound 346-2) LC-MS: [M+H]⁺ =272.75

4-(Dimethoxymethyl)piperidine (800 mg, 5.031 mmol), 1,4-dichlorobenzene (2.23 g, 15.093 mmol), Pd(OAc)₂(113.19 mg, 0.503 mmol), BINAP (625.73 mg, 1.006 mmol), and Cs₂CO₃(4.9 g, 15.093 mmol) were added sequentially to an eggplant shaped flask. Then solvent dioxane (8 mL) was added, and the reaction solution was stirred at 110°C for 2 h under N₂ atmosphere. After the reaction was completed, it was filtrated and concentrated, and then purified by preparative thin-layer column chromatography (EA:PE=1:5) to obtain a yellow solid compound 346-2 (730 mg, 53.55 %).

### Step 2: (Compound 346-3) LC-MS: [M+H]⁺ =525.09

Inan eggplant shaped flask, 346-2 (730 mg, 2.69 mmol), (S)-2-chloro-4-(3-methyl-2,8-diazaspiro[4.5]decan-2-yl)benzonitrile (933.8 mg, 3.23 mmol), Pd₂(dba)₃ (246.4 mg, 0.269 mmol), BINAP (334.63 mg, 0.538 mmol) and tBuOK (903.84 mg, 8.07 mmol) were added, solvent toluene (8 mL) was added, and the reaction solution was stirred at 110°C for 2 h under the atmosphere of N₂. After the reaction was completed, the reaction solution was filtered and concentrated, then purified by preparative thin-layer column chromatography (EA:PE=1:1) to obtain a solid compound 346-3 (190 mg, 13.46 %).

### Step 3: (Compound 346-4)

In an eggplant shaped flask, 346-3 (190 mg, 0.363 mmol), TFA (0.4 ml) and H₂O (0.1 ml) were added sequentially, the solvent DCM (0.2 mL) was added and the reaction solution was stirred at room temperature for 1 h. After the reaction was completed, the reaction solution was concentrated and spun dry to obtain a crude blue solid compound 346-4 (220 mg of the crude product).

### Step 4: (Compound 346) was prepared with reference to step 1 of Example 267. LC-MS: [M+H]⁺ =818.42

¹H NMR (600 MHz, DMSO-d₆) δ 11.00 (s, 1H), 7.60 (d, *J =* 8.8 Hz, 1H), 7.34 (m, 3H), 7.23 (d, *J* = 7.7 Hz, 1H), 6.80 (d, *J* = 2.0 Hz, 1H), 6.69 - 6.64 (m, 1H), 5.10 (dd, *J =* 13.2, 5.3 Hz, 1H), 4.65 (d, *J =* 8.9 Hz, 2H), 4.40 (d, *J =* 17.2 Hz, 1H), 4.26 - 4.21 (m, 1H), 4.16 - 4.01 (m, 3H), 3.55 (m, 5H), 3.14 - 2.98 (m, 5H), 2.97 - 2.77 (m, 5H), 2.62 (d, *J =* 22.8 Hz, 3H), 2.25 (dd, *J =* 12.7, 7.6 Hz, 2H), 2.04 - 1.82 (m, 7H), 1.69 (m, 3H), 1.58 (dd, *J =* 12.7, 6.5 Hz, 2H), 1.46 (s, 3H).

### Example 385

### Synthesis of compound 347

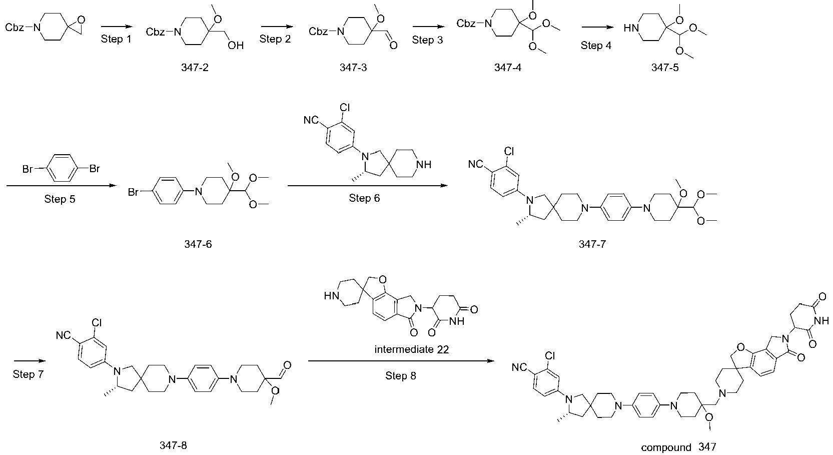

### Step 1: (Compound 347-2)

Benzyl 1-oxa-6-azaspiro[2.5]octane-6-carboxylate (10 g, 40.5 mmol) and p-toluenesulfonic acid (208.98 mg, 1.22 mmol) were added sequentially to an eggplant shaped flask, and the solvent MeOH (10 mL) was added, the reaction solution was stirred at room temperature for 12 h, and after the reaction was completed, the reaction solution was concentrated, and then purified by preparative thin layer column chromatography (EA: PE=1:1) to obtain a transparent oily compound 347-2 (5.2 g, 46.06 %).

### Step 2: (Compound 347-3)

The intermediate 347-2 (5.2 g, 18.63 mmol) and 2-iodoacylbenzoic acid (7.8 g, 27.95 mmol) were added sequentially to the eggplant shaped flask, solvent ACN (60 mL) was added. The reaction solution was stirred at room temperature for 2 h, and after the reaction was completed, the reaction solution was concentrated, and then purified by preparative thin layer column chromatography (EA: PE=1:1) to obtain a transparent oily compound 347-3 (3.8 g, 73.64 %).

### Step 3: (Compound 347-4)

In an eggplant shaped flask, 347-3 (3.8 g, 13.72 mmol) and trimethyl orthoformate (4.36 g, 41.16 mmol) were added sequentially, and the solvent MeOH (20 mL) was added, hydrochloric acid (0.1 ml) was added dropwise, and the reaction solution was stirred at 70°C for 2 h. After the reaction was completed, the reaction solution was concentrated, and purified by preparative thin-layer column chromatography (EA:PE=1:3) to obtain a transparent oily compound 347-4 (2 g, 63.2 %).

### Step 4: (Compound 347-5)

In an eggplant shaped flask, 347-4 (2 g, 6.19 mmol) and Pd(OH)₂/C (400 mg) were added sequentially, and the solvent MeOH (20 mL) was added, and the reaction solution was stirred at room temperature for 3 h under the atmosphere of H₂. After completion of the reaction, the reaction solution was filtrated and concentrated, and spun dry to obgtain a yellow oily compound 347-5 (800 mg, 68.37 %).

### Step 5: (Compound 347-6) LC-MS: [M+H]⁺ =344.25

In an eggplant shaped flask, 347-5 (800 mg, 4.23 mmol), 1,4-dibromobenzene (2.97 g, 12.69 mmol), Pd(OAc)₂ (95.17 mg, 0.423 mmol), BINAP (526.21 mg, 0.846 mmol) and Cs₂CO₃ (4.12 g, 12.69 mmol) were added sequentially. Then solvent dioxane (8 mL) was added and the reaction solution was stirred at 110°C for 2 h under N₂ atmosphere. After the reaction was completed, the reaction solution was filtrated and concentrated, and purified by preparative thin-layer column chromatography (EA:PE=1:5) to obtain a yellow solid compound 347-6 (730 mg, 50.31 %).

### Step 6: (Compound 347-7) LC-MS: [M+H]⁺ =553.14

In an eggplant shaped vial, 347-6 (730 mg, 2.13 mmol), (S)-2-chloro-4-(3-methyl-2,8-diazaspiro[4.5]decan-2-yl)benzonitrile (738 mg, 2.55 mmol), Pd₂ (dba)₃ (233.33 mg, 0.255 mmol), BINAP ( 317.22 mg, 0.51 mmol) and tBuOK (715.68 mg, 6.39 mmol) were added, then solvent toluene (8 mL) was added, the reaction solution was stirred at 110°C for 2 h under the atmosphere of N₂, and after the reaction was completed, the reaction solution was filtrated and concentrated and purified by preparative thin-layer column chromatography (EA: PE=1:1) to obtain a yellow solid compound 347-7 (170 mg, 14.46 %).

### Step 7: (Compounds 347-8)

In an eggplant shaped flask, 347-7 (170 mg, 0.308 mmol), TFA (0.4 ml) and H₂O (0.1 ml) were added sequentially, the solvent DCM (0.2 mL) was added and the reaction solution was stirred at room temperature for 1 h. After the reaction was completed, the reaction solution was concentrated and spun dry to obtain a crude blue solid compound 347-8 (190 mg).

### Step 8: (Compound 347) was prepared with reference to step 1 of Example 267. LC-MS: [M+H]⁺ =846.47

¹H NMR (600 MHz, DMSO-*d*₆) δ 10.98 (s, 1H), 8.14 (s, 1H), 7.60 (d, *J =* 8.8 Hz, 1H), 7.40 (s, 1H), 7.28 (d, *J* = 5.4 Hz, 1H), 6.86 (s, 3H), 6.80 (d, *J* = 2.2 Hz, 1H), 6.66 (dd, *J* = 8.9, 2.2 Hz, 1H), 5.09 (dd, *J =* 13.3, 5.0 Hz, 1H), 4.55 (s, 2H), 4.38 (d, *J* = 17.0 Hz, 1H), 4.22 (d, *J =* 17.0 Hz, 1H), 4.04 (m, 1H), 3.40 (d, *J* = 10.8 Hz, 2H), 3.31 (d, *J =* 11.0 Hz, 3H), 3.17 (m, 5H), 3.12 - 3.04 (m, 3H), 2.94 (m, 5H), 2.84 (t, *J =* 10.6 Hz, 2H), 2.61 (d, *J =* 12.1, 10.2 Hz, 2H), 2.46 - 2.38 (m, 3H), 2.23 (m, 2H), 2.03 - 1.96 (m, 2H), 1.85 (s, 2H), 1.73 (m, 6H), 1.55 (m, 3H).

### Example 386

### Synthesis of compound 348

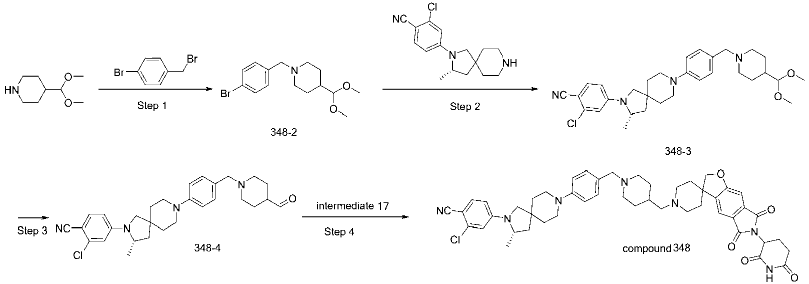

### Step 1: (Compound 348-2)

4-(Dimethoxymethyl)piperidine (2 g, 12.57 mmol), 1-bromo-4-(bromomethyl)benzene (3.73 g, 15.08 mmol) and potassium carbonate (5.2 g, 37.71 mmol) were added sequentially into an eggplant shaped flask, solvent EtOH (20 mL) was added, and the reaction solution was stirred at 80°C for 2 h. When the reaction was completed, the reaction solution was concentrated and then purified by preparative thin-layer column chromatography (EA:PE=1:2) to obtain atransparent oily compound 348-2 (2.2 g, 53.66 %).

### Step 2: (Compound 348-3) was prepared with reference to step 6 of Example 385. LC-MS: [M+H]⁺ =537.14

Step 3: (Compound 348-4) was prepared with reference to step 7 of Example 385.

Step 4: (Compound 348) was prepared with reference to Step 1 of Example 267.
¹H NMR (400 MHz, DMSO-*d*₆) δ 11.12 (s, 1H), 7.61 (d, *J* = 8.2 Hz, 2H), 7.31 (d, *J* = 8.5 Hz, 2H), 7.02 (d, *J* = 8.1 Hz, 2H), 6.80 (d, *J =* 2.0 Hz, 1H), 6.67 (d, *J* = 9.0 Hz, 2H), 5.11 (dd, *J =* 12.4, 5.0 Hz, 1H), 4.73 (d, *J* = 9.9 Hz, 3H), 4.17 (s, 2H), 4.04 (dd, *J =* 13.3, 6.5 Hz, 2H), 3.21 (m, 6H), 3.04 (s, 4H), 2.88 (d, *J =* 12.8 Hz, 5H), 2.36 - 2.17 (m, 6H), 2.01 (d, *J =* 13.1 Hz, 6H), 1.74 (s, 3H), 1.50 (s, 6H). LC-MS: [M+H]⁺ =844.45

### Example 387

### Synthesis of compound 349

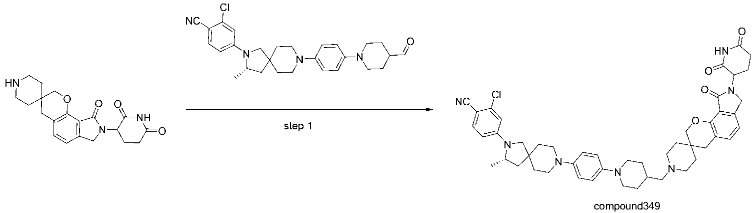

Step 1: (Compound 349) was prepared with reference to step 1 of Example 267.
¹H NMR (600 MHz, DMSO-*d₆*) δ 10.97 (s, 1H), 7.62 (d, *J =* 8.8 Hz, 1H), 7.31 (dd, *J =* 12.8, 7.7 Hz, 1H), 7.25 - 7.00 (m, 4H), 6.98 (d, *J =* 3.2 Hz, 1H), 6.81 (d, *J* = 2.3 Hz, 1H), 6.67 (dd, *J* = 8.9, 2.3 Hz, 1H), 5.02 (dd, 1H), 4.40 - 4.10 (m, 3H), 4.09 - 3.99 (m, 1H), 3.97 (s, 1H), 3.63 (s, 2H), 3.10 (s, 4H), 3.00 (s, 1H), 2.93 (d, *J =* 16.5 Hz, 3H), 2.76 (d, *J* = 15.4 Hz, 1H), 2.67 (d, *J =* 4.3 Hz, 2H), 2.65 - 2.54 (m, 2H), 2.43 - 2.30 (m, 2H), 2.25 (s, 1H), 2.07 - 1.92 (m, 3H), 1.85 (s, 3H), 1.75 (s, 4H), 1.62 (d, *J =* 13.5 Hz, 4H), 1.40 (s, 3H), 1.30 - 1.23 (m, 1H), 1.21 (d, *J* = 6.1 Hz, 3H), 1.18 (t, *J* = 7.1 Hz, 1H). LC-MS: [M+H]⁺ =830.76

### Example 388

### Synthesis of compound 350

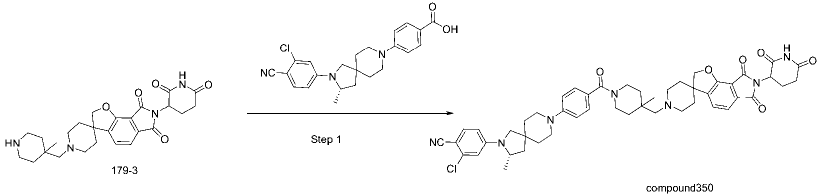

Step 3: (Compound 350) was prepared with reference to step 3 of Example 267.
¹H NMR (600 MHz, DMSO-d6) δ 11.11 (s, 1H), 7.60 (d, *J =* 8.8 Hz, 1H), 7.57 - 7.49 (m, 2H), 7.28 (d, *J =* 8.3 Hz, 2H), 6.97 (d, *J =* 8.4 Hz, 2H), 6.80 (d, *J =* 2.3 Hz, 1H), 6.67 (dd, *J =* 8.9, 2.3 Hz, 1H), 5.11 (dd, *J =* 12.9, 5.4 Hz, 1H), 4.82 (d, *J =* 4.4 Hz, 2H), 4.05 (m, 1H), 3.59 (d, J = 12.3 Hz, 2H), 3.25 (d, *J =* 11.6 Hz, 3H), 3.15 (s, 2H), 2.89 (ddd, *J =* 17.8, 14.1, 5.5 Hz, 2H), 2.64 - 2.56 (m, 2H), 2.40 - 2.20 (m, 5H), 2.05 - 1.94 (m, 4H), 1.74 (m, 3H), 1.63 - 1.40 (m, 8H), 1.32 - 1.17 (m, 9H). LC-MS: [M+H]⁺ =872.76

### Example 389

### Synthesis of compound 351

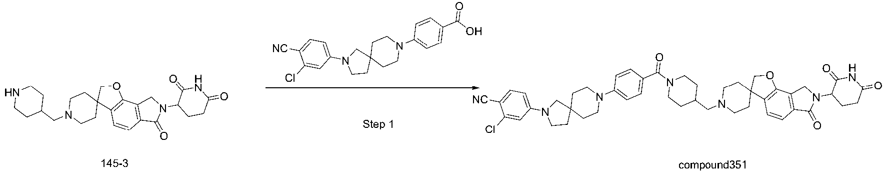

Step 1: (Compound 351) was prepared with reference to step 3 of Example 267. LC-MS: [M+H]⁺ =830.66

¹H NMR (600 MHz, DMSO-d6) δ 10.99 (s, 1H), 7.62 (d, *J* = 8.8 Hz, 1H), 7.36 (d, *J* = 7.5 Hz, 1H), 7.33 - 7.23 (m, 3H), 6.99 (dd, *J* = 8.6, 2.3 Hz, 2H), 6.76 (d, *J* = 2.3 Hz, 1H), 6.60 (dd, *J =* 8.9, 2.3 Hz, 1H), 5.10 (dd, *J =* 13.3, 5.0 Hz, 1H), 4.71 - 4.63 (m, 2H), 4.40 (d, *J =* 17.2 Hz, 1H), 4.26 (s, 1H), 3.59 (s, 2H), 3.41 (m, 5H), 3.30 (s, 2H), 3.25 (dd, *J =* 13.1, 5.4 Hz, 2H), 3.08 (m, 3H), 3.01 - 2.87 (m, 3H), 2.59 (d, *J* = 3.1 Hz, 1H), 2.48 - 2.38 (m, 2H), 2.22 (s, 4H), 2.02 - 1.89 (m, 5H), 1.84 - 1.75 (m, 2H), 1.71 - 1.62 (m, 4H), 1.25 - 1.18 (m, 2H).

### Example 390

### Synthesis of compound 352

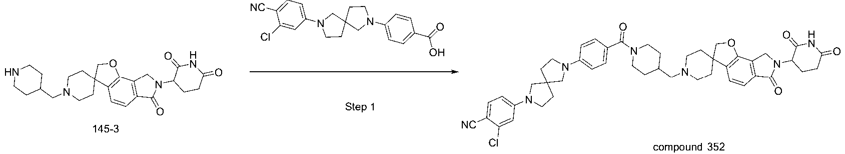

Step 3: (Compound 352) was prepared with reference to step 3 of Example 267.
¹H NMR (400 MHz, DMSO-d6) δ 10.99 (s, 1H), 7.62 (d, *J =* 8.8 Hz, 1H), 7.36 (d, *J =* 7.4 Hz, 1H), 7.27 (dd, *J =* 8.3, 6.7 Hz, 3H), 6.75 (d, *J* = 2.3 Hz, 1H), 6.60 (dd, *J* = 8.9, 2.4 Hz, 1H), 6.57 - 6.51 (m, 2H), 5.10 (dd, *J =* 13.3, 5.1 Hz, 1H), 4.66 (d, *J* = 2.6 Hz, 2H), 4.40 (d, *J=* 17.2 Hz, 1H), 4.24 (d, *J =* 17.2 Hz, 1H), 4.13 (s, 2H), 3.70 - 3.47 (m, 7H), 3.07 (s, 4H), 2.92 (dt, *J =* 17.4, 9.2 Hz, 4H), 2.60 (d, *J =* 16.7 Hz, 2H), 2.42 (d, *J* = 4.6 Hz, 2H), 2.19 (d, *J* =32.2 Hz, 4H), 2.10 - 2.01 (m, 4H), 2.01 - 1.84 (m, 4H), 1.83 - 1.74 (m, 2H).LC-MS: [M+H]⁺ =816.56

### Example 391

### Synthesis of compound 353

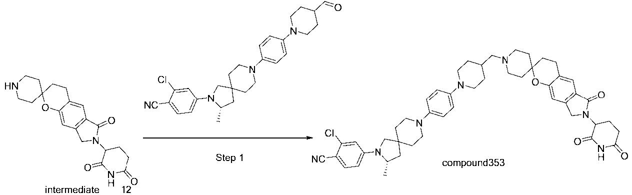

### Step 1: (Compound 353) was prepared with reference to step 1 of Example 267. LC-MS: [M+H]⁺ =830.76

¹H NMR (600 MHz, DMSO-d6) δ 10.98 (d, *J* = 2.9 Hz, 1H), 7.63 (d, *J=* 8.8 Hz, 2H), 7.52 (d, *J =* 5.9 Hz, 2H), 7.05 (s, 1H), 6.84 - 6.79 (m, 2H), 6.68 (d, *J =* 8.7 Hz, 2H), 5.08 (d, *J* = 7.9 Hz, 1H), 4.35 (dd, *J =* 17.0, 10.7 Hz, 2H), 4.22 (dd, *J=* 16.9, 8.7 Hz, 2H), 4.06 (d, *J =* 6.9 Hz, 2H), 3.20 (s, 4H), 3.15 (s, 2H), 2.96 - 2.85 (m, 6H), 2.63 - 2.57 (m, 2H), 2.41 - 2.32 (m, 3H), 2.09 - 2.04 (m, 2H), 2.01 - 1.96 (m, 6H), 1.88 (t, *J* = 7.0 Hz, 4H), 1.63 (s, 4H), 1.22 (d, *J* = 6.0 Hz, 6H).

### Example 392

### Synthesis of compound 354

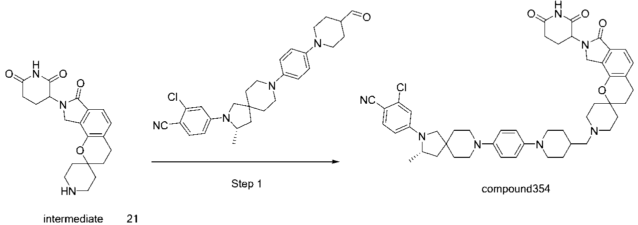

Step 1: (Compound 354) was prepared with reference to Step 1 of Example 267.
¹H NMR (600 MHz, DMSO-d6) δ 11.04 (s, 1H), 7.63 (d, *J =* 8.8 Hz, 1H), 7.30 (d, *J* = 7.7 Hz, 2H), 7.25 (d, *J =* 7.6 Hz, 2H), 6.82 (d, *J =* 2.3 Hz, 2H), 6.71 - 6.65 (m, 2H), 5.15 (d, *J* = 8.2 Hz, 1H), 4.45 (d, *J =* 17.5 Hz, 2H), 4.29 (d, *J* = 17.4 Hz, 2H), 4.06 (d, *J =* 6.8 Hz, 2H), 3.50 (s, 4H), 3.16 (s, 2H), 2.97 (dd, *J* = 15.5, 10.3 Hz, 2H), 2.90 (t, *J* = 7.0 Hz, 4H), 2.65 - 2.60 (m, 2H), 2.34 (td, *J =* 13.2, 4.6 Hz, 3H), 2.07 - 2.04 (m, 2H), 2.03 (s, 2H), 2.01 - 1.97 (m, 4H), 1.91 (m, 4H), 1.64 (s, 4H), 1.22 (d, *J* = 6.0 Hz, 6H).LC-MS: [M+H]⁺ =830.86

### Example 393

### Synthesis of compound 355

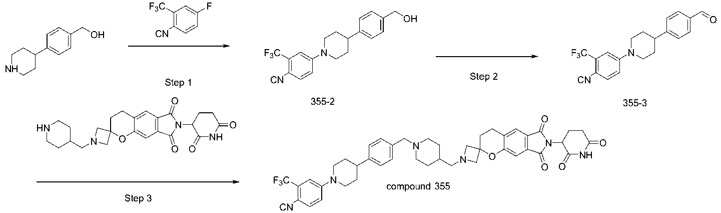

### Step 1: (Compound 355-2) LC-MS: [M+H]⁺ =361.39

Compound (4-(piperidin-4-yl)phenyl)methanol (750.0 mg, 3.92 mmol) and 4-fluoro-2-(trifluoromethyl)benzonitrile (741.53 mg, 3.92 mmol) were added sequentially into a glass vial, solvent DMSO (8.0 mL) was added, and finally N,N-diisopropylethylamine (1.52 g, 11.76 mmol) was added. The reaction solution was heated up to 120 °C and stirred overnight. After the reaction was completed, the reaction solution was cooled down to room temperature and then purified by Flash column chromatography to obtain a white solid compound 355-2 (938 mg, 66.38%).

### Step 2: (Compound 335-3) LC-MS: [M+H]⁺ =359.39

In a glass vial, 355-2 (400 mg, 1.11 mmol) was added, followed by the addition of solvent acetonitrile (8.0 mL), and finally 2-iodoacylbenzoic acid (621.60 mg, 2.22 mmol) was added, and then the reaction solution was stirred at room temperature for 2 h. After the reaction was completed, a crude compound 355-3 was obtained by filtration and used directly in the next step.

Step 3: (Compound 355) was prepared with reference to step 1 of Example 267.
¹H NMR (600 MHz, DMSO-*d*₆) δ 11.11 (s, 1H), 7.83 (d, *J =* 8.9 Hz, 1H), 7.77 (s, 1H), 7.42 (d, *J* = 7.5 Hz, 2H), 7.37 (d, *J =* 7.6 Hz, 2H), 7.34 (s, 1H), 7.28 (d, *J =* 13.2 Hz, 2H), 5.11 (dd, *J =* 12.9, 5.4 Hz, 1H), 4.25 (dd, *J =* 45.3, 31.5 Hz, 7H), 3.08 (t, *J =* 12.3 Hz, 2H), 3.03 - 2.79 (m, 6H), 2.64 - 2.52 (m, 2H), 2.23 (s, 2H), 2.08 - 1.95 (m, 2H), 1.95 - 1.84 (m, 4H), 1.84 - 1.73 (m, 1H), 1.73 - 1.63 (m, 2H), 1.51 - 1.16 (m, 6H). LC-MS: [M+H]⁺ =795.66

### Example 394

### Synthesis of compound 356

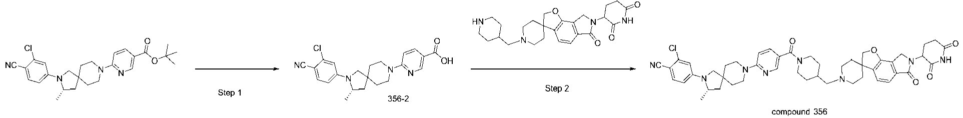

### Step 1: (Compound 356-2) was prepared with reference to step 2 of Example 267. LC-MS: [M+H]⁺ =411.39

Step 2: Compound 356) was prepared with reference to step 3 of Example 267.
¹H NMR (600 MHz, DMSO-*d*₆) δ 10.99 (s, 1H), 8.16 (d, *J =* 2.0 Hz, 1H), 7.61 (d, *J =* 8.8 Hz, 1H), 7.55 (dd, *J =* 8.8, 2.1 Hz, 1H), 7.39 (d, *J =* 7.5 Hz, 1H), 7.28 (d, *J =* 7.5 Hz, 1H), 6.85 (d, *J =* 8.9 Hz, 1H), 6.81 (d, *J* = 2.0 Hz, 1H), 6.67 (dd, *J =* 8.9, 2.0 Hz, 1H), 5.09 (dd, *J =* 13.3, 5.0 Hz, 1H), 4.54 (q, *J =* 9.2 Hz, 2H), 4.42 - 4.34 (m, 1H), 4.22 (d, *J* = 17.0 Hz, 1H), 4.09 - 4.00 (m, 1H), 3.73 (t, *J =* 14.8 Hz, 2H), 3.59 - 3.49 (m, 2H), 3.47 (d, *J* = 10.8 Hz, 1H), 3.05 - 2.74 (m, 5H), 2.59 (d, *J =* 16.9 Hz, 1H), 2.52 (s, 2H), 2.49 - 2.37 (m, 2H), 2.31 - 2.14 (m, 3H), 2.13 - 1.88 (m, 5H), 1.85 (s, 1H), 1.81 - 1.61 (m, 6H), 1.58 (dd, *J =* 12.8, 6.6 Hz, 1H), 1.49 - 1.39 (m, 2H), 1.27 - 1.17 (m, 3H), 1.09 (dd, *J =* 21.0, 11.6 Hz, 2H).LC-MS: [M+H]⁺=845.66

### Example 395

### Synthesis of compound 357

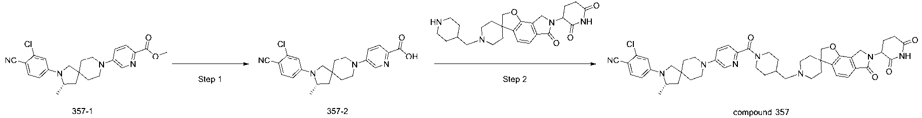

### Step 1: (Compound 357-2) LC-MS: [M+H]⁺ =411.39

Compound 357-1 (180 mg, 385.43 µmol) and solvent tetrahydrofuran:methanol:water=3:1:1 (2.0 mL) were added in a glass vial, after that lithium hydroxide (56.36 mg, 2.35 mmol) was added and the reaction solution was stirred at room temperature for 2.0 h, and when the reaction was completed, then purified by Flash to obtain a light yellow solid compound 357 -2 (109 mg, 56.36%).

Step 2: (Compound 357) was prepared with reference to step 3 of Example 267.
¹ H NMR (600 MHz, *DMSO-d*₆ ) δ 10.99 (s, 1H), 8.27 (d, *J =* 2.6 Hz, 1H), 7.61 (d, *J =* 8.8 Hz, 1H), 7.44 (d, *J =* 8.7 Hz, 1H), 7.41 - 7.36 (m, 2H), 7.28 (d, *J =* 7.5 Hz, 1H), 6.81 (d, *J =* 2.2 Hz, 1H), 6.67 (dd, *J =* 8.9, 2.2 Hz, 1H), 5.09 (dd, *J =* 13.3, 5.0 Hz, 1H), 4.54 (q, *J =* 9.2 Hz, 2H), 4.47 (d, *J* = 9.7 Hz, 1H), 4.38 (d, *J* = 17.1 Hz, 1H), 4.22 (d, *J =* 17.0 Hz, 1H), 4.10 - 4.00 (m, 2H), 3.50 - 3.39 (m, 3H), 3.31 - 3.21 (m, 3H), 3.03 (s, 1H), 2.97 - 2.83 (m, 3H), 2.76 (s, 1H), 2.59 (d, *J =* 17.0 Hz, 1H), 2.47 - 2.37 (m, 1H), 2.25 (dd, *J =* 12.7, 7.7 Hz, 3H), 2.17 - 2.01 (m, 2H), 2.01 - 1.89 (m, 3H), 1.85 (s, 2H), 1.80 - 1.63 (m, 5H), 1.59 (dd, *J =* 12.8, 6.5 Hz, 1H), 1.56 - 1.45 (m, 2H), 1.21 (d, *J =* 6.1 Hz, 3H), 1.16 - 1.03 (m, 2H).LC-MS: [M+H]⁺ =845.66

### Example 396

### Synthesis of compound 358

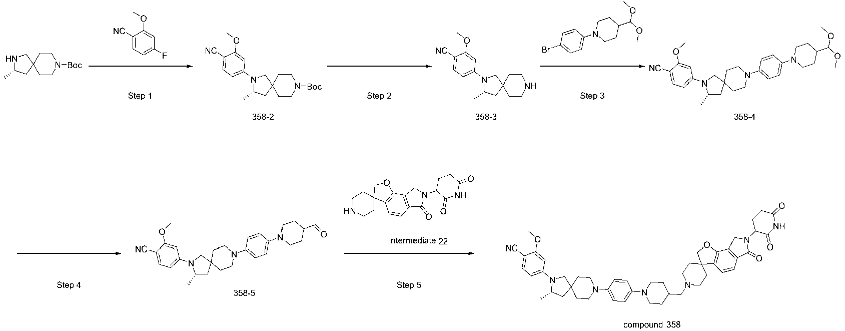

### Step 1: (Compound 358-2) was prepared with reference to step 1 of Example 393. LC-MS: [M+H]⁺ =386.35

### Step 2: (Compound 358-3) was prepared with reference to step 2 of Example 39. LC-MS: [M+H]⁺ =286.38

### Step 3: (Compound 358-4) was prepared with reference to step 6 of Example 385. LC-MS: [M+H]⁺ =519.45

### Step 4: (Compound 358-5) was prepared with reference to step 7 of Example 385. LC-MS: [M+H]⁺ =473.49

### Step 5: Compound 358) was prepared with reference to step 1 of Example 267. LC-MS: [M+H]⁺ =812.76

¹H NMR (600 MHz, *DMSO-d*₆) *δ* 11.00 (s, 1H), 7.56 - 6.94 (m, 7H), 6.29 (d, *J =* 8.5 Hz, 1H), 6.21 (s, 1H), 5.11 (dd, *J =* 13.3, 5.0 Hz, 1H), 4.68 (q, *J* = 9.4 Hz, 2H), 4.40 *(d, J =* 17.2 Hz, 1H), 4.25 (d, *J* = 17.1 Hz, 1H), 4.06 (d, *J =* 6.2 Hz, 1H), 3.89 (s, 4H), 3.47 - 3.20 (m, 8H), 3.19 - 3.01 (m, 4H), 2.97 - 2.83 (m, 2H), 2.65 - 2.57 (m, 1H), 2.48 - 2.36 (m, 2H), 2.31 - 2.17 (m, 3H), 2.17 - 2.04 (m, 2H), 2.04 - 1.78 (m, 7H), 1.78 - 1.51 (m, 4H), 1.45 (s, 2H), 1.24 (d, *J =* 5.8 Hz, 3H).

### Example 397

### Synthesis of compound 359

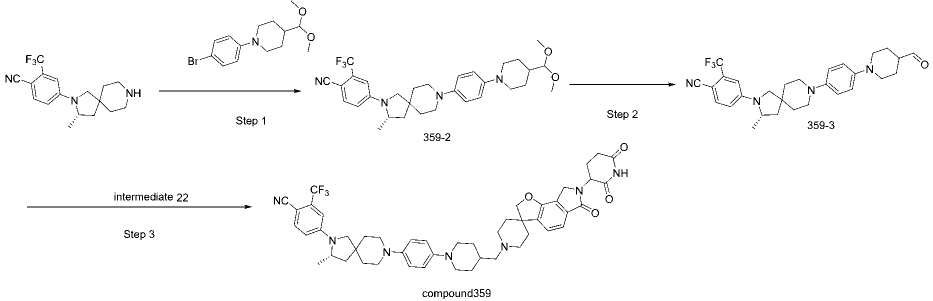

Step 1: (Compound 359-2) was prepared with reference to step 2 of Example 385.
LC-MS: [M+H]⁺ =557.48

### Step 2: (Compound 359-3) was prepared with reference to step 2 of Example 39. LC-MS: [M+H]⁺ =511.45

Step 3: Compound 359) was prepared with reference to step 1 of Example 267.
¹H NMR (600 MHz, DMSO-*d*₆) δ 10.99 (s, 1H), 7.86 - 7.77 (m, 1H), 7.37 (d, *J =* 7.4 Hz, 1H), 7.29 (d, *J =* 7.6 Hz, 1H), 7.18 (m, 4H), 6.99 (s, 1H), 6.94 (d, *J* = 8.9 Hz, 1H), 5.10 (dd, *J =* 13.3, 5.0 Hz, 1H), 5.10 (dd, *J =* 13.3, 5.0 Hz, 1H), 4.68 (q, *J =* 15.4, 9.4 Hz, 2H), 4.60 (dd, *J =* 19.4, 8.7 Hz, 1H), 4.40 (d, *J =* 17.2 Hz, 1H), 4.25 (d, *J =* 17.1 Hz, 1H), 4.14 (d, *J =* 6.2 Hz, 2H), 3.60 (s, 7H), 3.14 (d, *J =* 38.5 Hz, 4H), 2.98 - 2.86 (m, 2H), 2.66 - 2.56 (m, 1H), 2.47 -2.36(m, 2H), 2.32 - 2.17 (m, 3H), 2.17 - 2.05 (m, 2H), 2.05 - 1.81 (m, 6H), 1.81 - 1.55 (m, 4H), 1.45 (s, 3H), 1.24 (d, *J* = 6.0 Hz, 3H) LC-MS: [M+H]⁺ =850.76

### Example 398

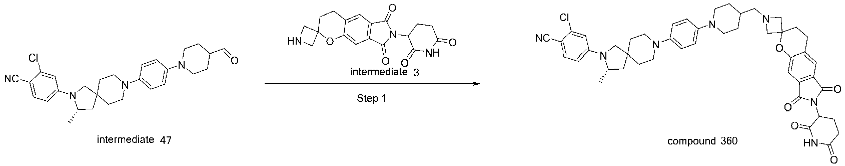

### Step 1: (Compound 360) was prepared with reference to step 1 of Example 267. LC-MS: [M+H]⁺ =816.76

¹H NMR (400 MHz, DMSO-*d*₆) δ 11.11 (s, 1H), 7.71 (s, 1H), 7.59 (d, *J =* 8.8 Hz, 1H), 7.25 (s, 1H), 6.88 - 6.75 (m, 5H), 6.65 (dd, *J* = 9.0, 2.1 Hz, 1H), 5.09 (dd, *J =* 12.8, 5.4 Hz, 1H), 4.03 (dd, *J =* 13.3, 6.5 Hz, 1H), 3.63 - 3.37 (m, 7H), 3.28 (s, 3H), 3.15 - 3.01 (m, 3H), 3.01 - 2.82 (m, 5H), 2.64 - 2.54 (m, 2H), 2.46 - 2.38 (m, 1H), 2.22 (dd, *J =* 12.8, 7.7 Hz, 1H), 2.18 - 2.09 (m, 2H), 2.09 - 1.97 (m, 1H), 1.82 - 1.66 (m, 4H), 1.59 - 1.48 (m, 3H), 1.48 - 1.33 (m, 1H), 1.33 - 1.14 (m, 5H).

### Example 399

### Synthesis of compound 361

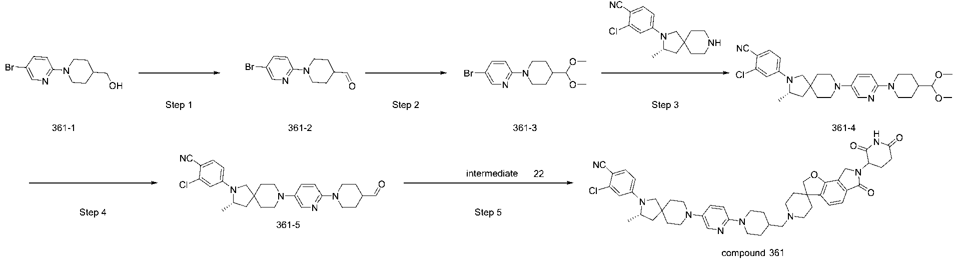

### Step 1: (Compound 361-2) LC-MS: [M+H]⁺ =386.35

Intermediate 361-1 (200.0 mg, 737.58 µmol) was added into a glass vial, the solvent acetonitrile (4.0 mL) was added, then 2-iodoylbenzoic acid (413.07 mg, 1.48 mmol) was added, and the reaction solution was heated up to 80°C and stirred for 4 h. After the reaction was completed, the reaction solution was filtered to obtain a crude yellow oily compound 361-2 (280 mg, crude) which was used directly in the next step.

### Step 2: (Compound 269.17) was prepared with reference to step 2 of Example 385. LC-MS: [M+H]⁺ =315.20

### Step 3: (Compound 361-4) was prepared with reference to step 6 of Example 385. LC-MS: [M+H]⁺ =524.49

### Step 4: (Compound 361-5) was prepared with reference to step 7 of Example 385. LC-MS: [M+H]⁺ =478.40

### Step 5: (Compound 361) was prepared with reference to step 1 of Example 267. LC-MS: [M+H]⁺ =817.66

¹H NMR (600 MHz, DMSO-d₆) δ 10.99 (s, 1H), 7.85 (d, *J =* 2.8 Hz, 1H), 7.60 (d, *J =* 8.9 Hz, 1H), 7.44 - 7.23 (m, 3H), 6.82 - 6.73 (m, 2H), 6.66 (dd, *J =* 9.0, 2.2 Hz, 1H), 5.10 (dd, *J =* 13.3, 5.0 Hz, 1H), 4.57 (s, 2H), 4.39 (d, *J* = 17.0 Hz, 1H), 4.23 (d, *J* = 17.0 Hz, 1H), 4.13 (d, *J* = 11.2 Hz, 2H), 4.08 - 3.99 (m, 1H), 3.76 - 3.46 (m, 2H), 3.40 (d, *J* = 10.8 Hz, 1H), 3.31 (d, *J =* 10.8 Hz, 1H), 3.12 - 2.76 (m, 7H), 2.69 (s, 2H), 2.60 (d, *J = 17.0* Hz, 1H), 2.48 - 2.37 (m, 1H), 2.23 (dd, *J =* 12.7, 7.7 Hz, 3H), 2.09 - 1.85 (m, 4H), 1.85 - 1.61 (m, 6H), 1.61 - 1.48 (m, 3H), 1.26 - 1.03 (m, 5H).

### Example 400

### Synthesis of compound 362

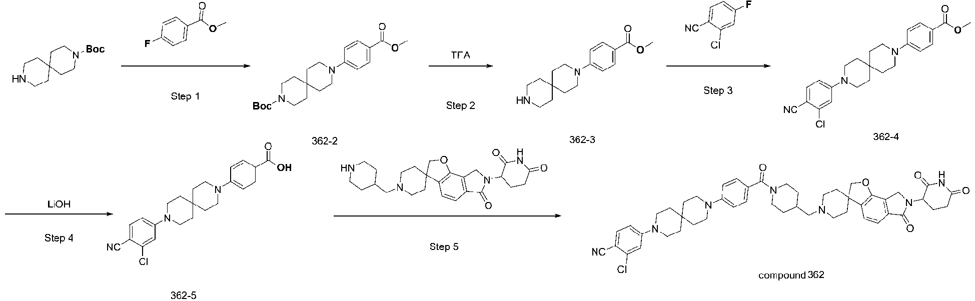

### Step 1: (Compound 362-2) LC-MS: [M+H]⁺ = 389.49

In a glass vial, tert-butyl 3,9-diazaspiro[5.5]undecane-3-carboxylate (1 g, 3.93 mmol) and methyl 4-fluorobenzoate (605.96 mg, 3.93 mmol) were added sequentially, and the solvent DMSO (5 mL) was added, followed by the addition of DIEA (762.15 mg, 5.90 mmol), and the reaction solution was stirred at 120 °C for 14 h. After the reaction was completed, it was concentrated and then purified by preparative thin-layer chromatography (EA:PE=1:5) to obtain a white solid 362-2 (780 mg, 51.07%).

### Step 2: (Compounds 362-3) was prepared with reference to step 2 of Example 39. LC-MS: [M+H]⁺ = 289.38

### Step 3: (Compound 362-4) LC-MS: [M+H]⁺ = 424.39

In a glass vial, 362-3 (740 mg, 2.57 mmol) and 2-chloro-4-fluorobenzonitrile (399.15 mg, 2.57 mmol) were added sequentially, and the solvent DMSO (5 mL) was added, followed by the addition of DIEA (497.46 mg, 3.85 mmol), and the reaction solution was stirred at 120 °C for 14 h. After the reaction was completed, the reaction solution was concentrated and then purified by preparative thin layer chromatography (EA:PE=1:5) to obtain a white solid 362-4 (446 mg, 41.00%).

### Step 4: (Compound 362-5) was prepared with reference to step 1 of Example 395 LC-MS: [M+H]⁺ =410.49

Step 5: (Compound 362) was prepared with reference to step 3 of Example 267.
¹H NMR (600 MHz, DMSO-*d*₆) δ 10.99 (s, 1H), 7.63 (d, *J =* 8.9 Hz, 1H), 7.37 (d, *J* =7.5 Hz, 1H), 7.28 (t, *J =* 8.2 Hz, 3H), 7.13 (d, *J =* 2.4 Hz, 1H), 6.98 (dd, *J* = 9.1, 2.5 Hz, 3H), 5.10 (dd, *J =* 13.3, 5.1 Hz, 1H), 4.71 - 4.56 (m, 2H), 4.40 (d, *J =* 17.2 Hz, 1H), 4.24 (d, *J =* 17.1 Hz, 1H), 4.13 (s, *J =* 17.1 Hz, 1H), 3.59 (d, *J =* 13.5 Hz, 3H), 3.27 (t, *J =* 5.6 Hz, 6H), 3.14 - 3.03 (m, 4H), 3.02 - 2.83 (m, 3H), 2.61 (d, *J =* 18.1 Hz, 1H), 2.48 - 2.37 (m, 2H), 2.22 (dd, *J =* 16.2, 12.3 Hz, 2H), 2.18 - 2.06 (m, 1H), 2.03 - 1.90 (m, 3H), 1.79 (d, *J =* 12.3 Hz, 2H), 1.59 (m, *J =* 29.3, 5.8 Hz, 8H), 1.22 (m, *J =* 20.2, 8.0, 6.2 Hz, 3H). LC-MS: [M+H]⁺ =844.66

### Example 401

### Synthesis of compound 363

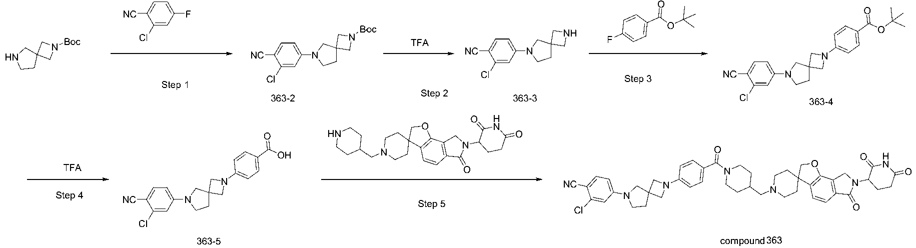

### Step 1: (Compound 363-2) was prepared with reference to step 1 of Example 400. LC-MS: [M+H]⁺ = 248.27

### Step 2: (Compound 363-3) was prepared with reference to step 2 of Example 39. LC-MS: [M+H]⁺ = 248.37

### Step 3: (Compounds 363-4) was prepared with reference to step 1 of Example 400. LC-MS: [M+H]⁺ = 368.29

### Step 4: Compounds 363-5) was prepared with reference to step 1 of Example 395. LC-MS: [M+H]⁺ = 368.39

Step 5: Compound 363) was prepared with reference to step 3 of Example 267.
¹H NMR (400 MHz, DMSO-*d*₆) δ 11.00 (s, 1H), 7.64 (d, *J =* 8.8 Hz, 1H), 7.36 (d, *J =* 7.5 Hz, 1H), 7.32 - 7.20 (m, 3H), 6.76 (d, *J =* 2.3 Hz, 1H), 6.60 (dd, *J =* 8.9, 2.3 Hz, 1H), 6.50 - 6.43 (m, 2H), 5.10 (dd, *J =* 13.3, 5.1 Hz, 1H), 4.73 - 4.55 (m, 2H), 4.40 (d, *J =* 17.2 Hz, 1H), 4.24 (d, *J =* 17.2 Hz, 1H), 4.11 (s, *J =* 17.1 Hz, 1H), 3.87 (s, 4H), 3.44 (t, *J =* 6.8 Hz, 3H), 3.16 - 3.01 (m, 4H), 3.01 - 2.84 (m, 3H), 2.66 - 2.55 (m, 1H), 2.43 (dd, *J =* 13.1, 4.7 Hz, 1H), 2.28 (t, *J =* 6.8 Hz, 2H), 2.22 (d, *J =* 12.7 Hz, 2H), 2.15 (s, 1H), 2.04 - 1.90 (m, 3H), 1.79 (d, *J =* 11.9 Hz, 2H), 1.30 - 1.10 (m, 2H). LC-MS: [M+H]⁺ = 802.56

### Example 402

### Synthesis of compound 364

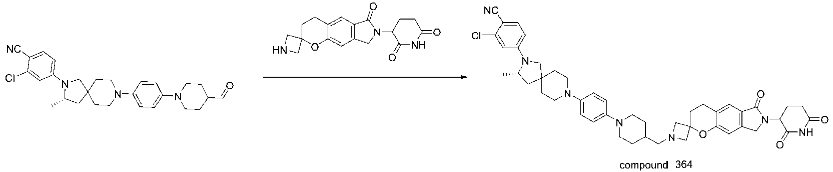

Step 1: (Compound 364) was prepared with reference to step 1 of Example 267.
¹H NMR (600 MHz, DMSO-*d*₆) δ 10.98 (s, 1H), 7.59 (d, *J =* 8.8 Hz, 1H), 7.34 (s, 1H), 7.07 (s, 1H), 6.84 (d, *J* = 2.1 Hz, 4H), 6.80 (d, *J =* 2.3 Hz, 1H), 6.66 (dd, *J =* 8.9, 2.4 Hz, 1H), 5.08 (dd, *J =* 13.4, 5.1 Hz, 1H), 4.34 (d, *J =* 16.7 Hz, 1H), 4.21 (d, *J* = 16.6 Hz, 1H), 4.03 (h, *J =* 6.3 Hz, 1H), 3.48 (d, *J =* 11.6 Hz, 2H), 3.39 (d, *J =* 10.8 Hz, 2H), 3.30 (d, *J* = 10.7 Hz, 4H), 3.13 - 3.03 (m, 3H), 3.01 - 2.86 (m, 6H), 2.63 - 2.57 (m, 1H), 2.38 (m, *J =* 13.3, 4.5 Hz, 2H), 2.22 (dd, *J* = 12.8, 7.8 Hz, 1H), 2.15 (s, 2H), 1.99 (m, *J* = 7.7, 5.3, 2.5 Hz, 1H), 1.74 (m, *J* = 21.2, 13.0, 8.9, 3.7 Hz, 4H), 1.60 - 1.43 (m, 4H), 1.34 - 1.23 (m, 3H), 1.20 (d, *J =* 6.0 Hz, 3H). LC-MS: [M+H]⁺ = 401.99

### Example 403

### Synthesis of compound 365

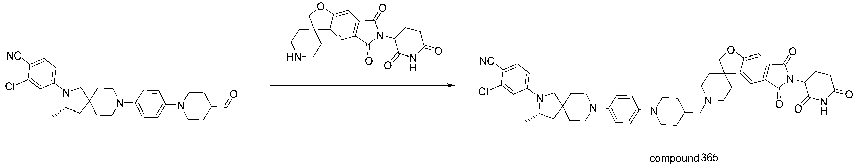

### Step 1: (Compound 365) was prepared with reference to step 1 of Example 267. LC-MS: [M+H]⁺ = 415.99

¹ H NMR (600 MHz, DMSO-*d*₆) δ 11.10 (s, 1H), 7.77 (s, 1H), 7.60 (d, *J =* 8.8 Hz, 1H), 7.22 (s, 1H), 6.84 (s, 4H), 6.80 (d, *J =* 2.3 Hz, 1H), 6.66 (dd, *J =* 9.0, 2.4 Hz, 1H), 5.09 (dd, *J =* 12.9, 5.4 Hz, 1H), 4.03 (m, *J =* 6.3 Hz, 1H), 3.50 (d, *J =* 11.2 Hz, 2H), 3.39 (d, *J =* 10.8 Hz, 1H), 3.30 (d, *J =* 10.8 Hz, 3H), 3.18 (s, 2H), 3.07 (m, *J =* 7.0 Hz, 2H), 3.01 - 2.92 (m, 2H), 2.90 - 2.85 (m, 1H), 2.66 - 2.52 (m, 5H), 2.22 (dd, *J =* 12.8, 7.7 Hz, 3H), 2.03 (m, *J =* 7.5, 7.0, 3.6 Hz, 2H), 1.97 - 1.69 (m, 8H), 1.57 (dd, *J =* 12.8, 6.5 Hz, 1H), 1.54 - 1.49 (m, 2H), 1.24 (s, 2H), 1.21 (d, *J* = 6.1 Hz, 4H).

### Example 404

### Synthesis of compound 366

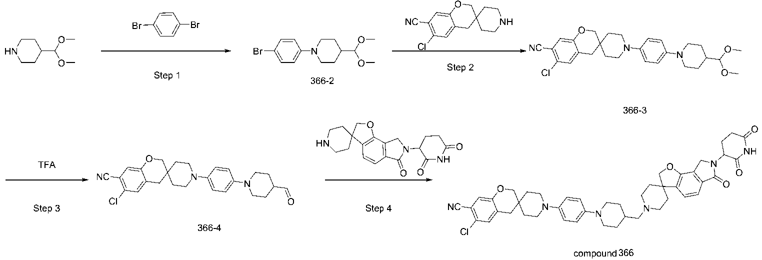

### Step 1: (Compound 366-2) was prepared with reference to step 5 of Example 385. LC-MS: [M+H]⁺ = 314.28

### Step 2: (Compound 366-3) was prepared with reference to step 6 of Example 385. LC-MS: [M+H]⁺ = 496.39

### Step 3: (Compound 366-4) was prepared with reference to step 2 of Example 267. LC-MS: [M+H]⁺ = 482.49

### Step 4: (Compound 366) was prepared with reference to step 1 of Example 267. LC-MS: [M+H]⁺ = 789.56

### Example 405

### Synthesis of compound 367

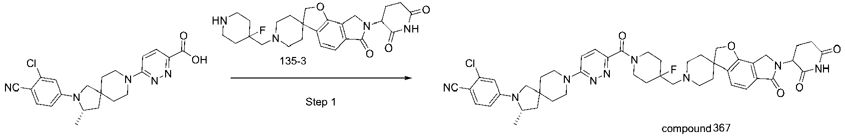

### Step 1: (Compound 367) was prepared with reference to step 3 of Example 267. LC-MS: [M+H]⁺ = 864.76

¹H NMR (600 MHz, DMSO-*d*₆) δ 11.00 (s, 1H), 7.61 (dd, *J =* 14.5, 9.2 Hz, 2H), 7.40 (d, *J =* 9.6 Hz, 1H), 7.37 (d, *J* = 7.5 Hz, 1H), 7.28 (d, *J =* 7.6 Hz, 1H), 6.81 (d, *J =* 2.3 Hz, 1H), 6.67 (dd, *J* = 9.0, 2.3 Hz, 1H), 5.10 (dd, *J =* 13.3, 5.1 Hz, 1H), 4.73 - 4.65 (m, 2H), 4.44 (s, 1H), 4.40 (d, *J =* 17.4 Hz, 2H), 4.24 (d*, J =* 17.1 Hz, 2H), 4.12 (d, *J* = 13.2 Hz, 1H), 4.06 (d, *J =* 6.7 Hz, 1H), 3.88 - 3.81 (m, 3H), 3.67 - 3.55 (m, 5H), 3.50 (d, *J =* 10.8 Hz, 1H), 3.36 (d, *J* = 10.7 Hz, 2H), 3.27 (s, 2H), 3.14 (t, *J=* 12.2 Hz, 1H), 2.97 - 2.87 (m, 1H), 2.64 - 2.57 (m, 1H), 2.43 (dd, *J =* 13.4, 4.8 Hz, 1H), 2.29 (dd, *J* = 12.9, 7.7 Hz, 3H), 2.14 (s, 1H), 2.05 (s, 1H), 2.01 - 1.95 (m, 3H), 1.77 - 1.66 (m, 2H), 1.61 (dd, *J =* 12.9, 6.7 Hz, 1H), 1.49 (t, *J* = 5.9 Hz, 2H), 1.22 (d, *J=* 6.0 Hz, 3H).

### Example 406

### Synthesis of compound 368

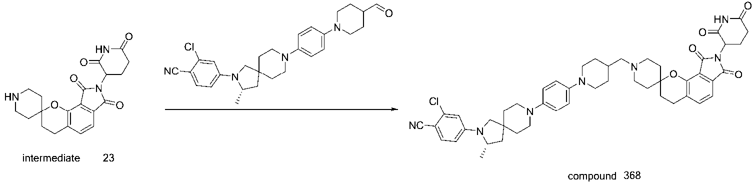

### Step 1: (Compound 368) was prepared with reference to step 1 of Example 267. LC-MS: [M+H]⁺ = 422.99

¹H NMR (600 MHz, *DMSO-d*₆) δ 11.11 (d, *J =* 8.0 Hz, 1H), 7.64 (dd, *J =* 7.6, 4.2 Hz, 1H), 7.60 (d, *J =* 8.8 Hz, 1H), 7.41 (d, *J =* 7.4 Hz, 1H), 6.86 (s, 4H), 6.80 (d, *J* = 2.3 Hz, 1H), 6.66 (dd, *J* = 9.0, 2.4 Hz, 1H), 5.08 (dd, *J =* 12.8, 5.5 Hz, 1H), 4.04 (q, *J =* 6.6 Hz, 1H), 3.56 (d, *J =* 16.3 Hz, 4H), 3.43 - 3.38 (m, 1H), 3.24 - 3.20 (m, 1H), 3.19 - 3.03 (m, 5H), 2.95 (t, *J =* 6.8 Hz, 4H), 2.87 (t, *J =* 15.2 Hz, 1H), 2.64 - 2.55 (m, 3H), 2.19 (d, *J =* 43.2 Hz, 2H), 2.11 - 1.92 (m, 8H), 1.87 (s, 3H), 1.75 (s, 2H), 1.54 (d, *J =* 30.5 Hz, 3H), 1.36 (s, 2H), 1.21 (d, *J =* 6.1 Hz, 3H).

### Example 407

### Synthesis of compound 369

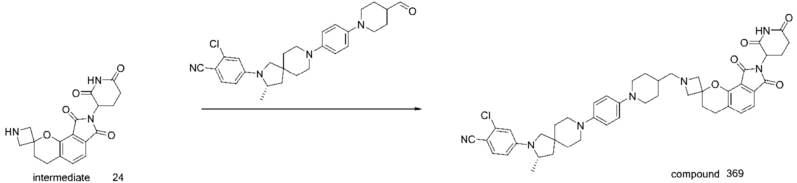

Step 1: (Compound 369) was prepared with reference to step 1 of Example 267.
¹H NMR (600 MHz, DMSO-d₆) δ 11.12 (s, 1H), 7.64 (t, *J =* 6.9 Hz, 1H), 7.62 - 7.57 (m, 1H), 7.45 (d, *J =* 7.5 Hz, 1H), 6.86 (s, 4H), 6.80 (d, *J* = 2.3 Hz, 1H), 6.66 (dd, *J =* 8.9, 2.4 Hz, 1H), 5.09 (dd, *J =* 12.5, 5.4 Hz, 1H), 4.55 (s, 1H), 4.51 - 4.45 (m, 1H), 4.39 (dd, *J =* 12.4, 5.4 Hz, 1H), 4.31 (s, 1H), 4.04 (p, *J =* 6.5 Hz, 1H), 3.52 (s, 2H), 3.26 (s, 2H), 3.10 (s, 2H), 2.93 (m, *J =* 31.3, 17.0, 5.2 Hz, 5H), 2.64 - 2.60 (m, 1H),2.59 (d, *J* = 3.5 Hz, 1H), 2.55 (s, 1H), 2.35 - 2.16 (m, 4H), 2.04 (q, *J =* 7.1 Hz, 2H), 1.78 (d, *J* = 12.4 Hz, 5H), 1.68 (s, 1H), 1.52 (s, 3H), 1.33 (s, 2H), 1.21 (d, *J* = 6.0 Hz, 3H). LC-MS: [M+H]⁺ = 409.09

### Example 408

### Synthesis of compound 370

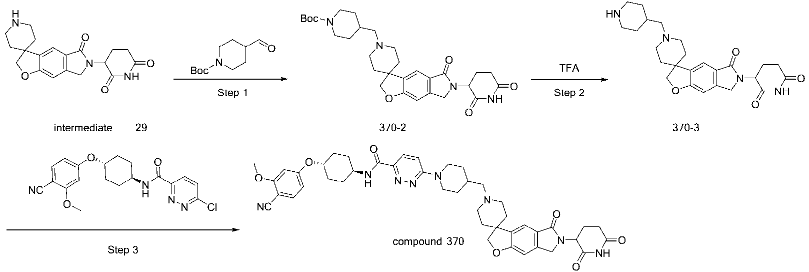

### Step 1: (Compound 370-2) was prepared with reference to step 1 of Compound 267. LC-MS: [M+H]⁺ = 553.48

### Step 2: (Compound 370-3) was prepared with reference to step 2 of Compound 267. LC-MS: [M+H]⁺ = 453.39

### Step 3: (Compound 370) was prepared with reference to step 3 of Compound 19. LC-MS: [M+H]⁺ = 803.66

¹H NMR (400 MHz, DMSO-*d*₆) δ 10.97 (s, 1H), 8.58 (d, *J =* 8.2 Hz, 1H), 7.84 (d, *J =* 9.5 Hz, 1H), 7.61 (d, *J = 9.2* Hz, 1H), 7.45 (s, 1H), 7.40 (d, *J =* 9.7 Hz, 1H), 7.04 (s, 1H), 6.75 - 6.67 (m, 2H), 5.03 (dd, *J =* 13.3, 5.1 Hz, 1H), 4.68 - 4.60 (m, 2H), 4.52 (d, *J =* 13.0 Hz, 3H), 4.36 (d, *J =* 17.4 Hz, 1H), 4.24 (d, *J =* 17.3 Hz, 1H), 3.88 (s, 3H), 3.84 (dd, *J =* 11.5, 3.3 Hz, 1H), 3.59 (d, *J =* 11.0 Hz, 2H), 3.08 (d, *J =* 11.8 Hz, 5H), 2.96 - 2.83 (m, 1H), 2.59 (d, *J* =16.7 Hz, 1H), 2.45 - 2.31 (m, 1H), 2.25 (t, *J =* 12.0 Hz, 3H), 2.12 (d, *J =* 9.9 Hz, 2H), 1.91 (dd, *J =* 30.0, 15.0 Hz, 7H), 1.64 (dd, *J =* 23.9, 11.1 Hz, 2H), 1.51 (dd, *J =* 22.8, 10.2 Hz, 2H), 1.33 - 1.18 (m, 3H).

### Example 409

### Synthesis of compound 371

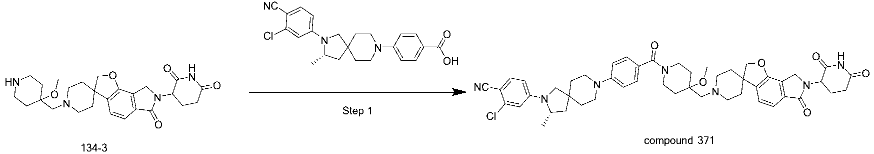

Step 3: (Compound 371) was prepare with reference to step 3 of Compound 267.
¹H NMR (600 MHz, *DMSO-d*₆) δ 10.99 (s, 1H), 7.61 (d, *J =* 8.8 Hz, 1H), 7.36 (d, *J =* 7.6 Hz, 1H), 7.28 (dd, *J =* 16.6, 8.0 Hz, 3H), 6.98 (d, *J =* 8.5 Hz, 2H), 6.81 (d, *J =* 2.0 Hz, 1H), 6.67 (dd, *J = 8.9,* 2.0 Hz, 1H), 5.10 (dd, *J* = 13.2, 5.0 Hz, 1H), 4.68 (q, *J* = 9.6 Hz, 2H), 4.40 (d, *J =* 17.2 Hz, 2H), 4.24 (d, *J =* 17.1 Hz, 2H), 4.08 - 4.02 (m, 3H), 3.62 (d, *J* = 10.2 Hz, 2H), 3.45 (d, *J* = 10.8 Hz, 1H), 3.35 (dd, *J =* 15.9, 7.3 Hz, 6H), 3.26 (s, 3H), 3.24 - 3.18 (m, 3H). 2.96 - 2.87 (m, 1H), 2.61 (d, *J* = 17.1 Hz, 1H), 2.43 (dd, *J* = 13.1, 4.4 Hz, 1H), 2.37 (t, *J =* 14.8 Hz, 2H), 2.25 (dd, *J* = 12.7, 7.7 Hz, 1H), 2.01 - 1.96 (m, 1H), 1.91 (t, *J* = 14.3 Hz, 4H), 1.74 (ddd, *J* = 17.7, 14.2, 9.3 Hz, 2H), 1.59 (dd, *J* = 12.8, 6.5 Hz, 3H), 1.51 (d, *J* = 10.9 Hz, 2H), 1.21 (d, *J =* 6.0 Hz, 3H). LC-MS: [M+H]⁺ = 874.56

### Example 410

### Synthesis of compound 372

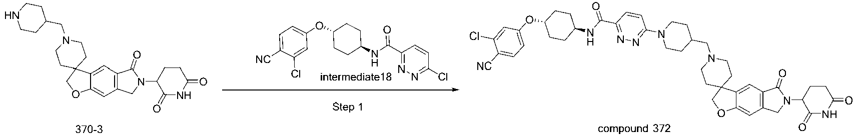

Step 1: (Compound 372) was prepared with reference to step 3 of Compound 19.
¹H NMR (600 MHz, DMSO-*d*₆) δ 10.98 (s, 1H), 8.61 (d, *J =* 8.0 Hz, 1H), 7.86 (dd, *J =* 11.2, 9.2 Hz, 2H), 7.41 (dd, *J =* 11.5, 7.1 Hz, 3H), 7.14 (dd, *J* = 8.8, 2.3 Hz, 1H), 7.05 (d, *J =* 10.2 Hz, 1H), 5.04 (dd, *J =* 13.3, 4.8 Hz, 1H), 4.65 (q, *J =* 9.4 Hz, 2H), 4.54 (d, *J* = 10.8 Hz, 3H), 4.37 (d, *J =* 17.2 Hz, 2H), 4.24 (dd, *J =* 17.1, 8.8 Hz, 2H), 3.92 - 3.82 (m, 3H), 3.60 (s, 2H), 3.38 (s, 1H), 2.95 - 2.86 (m, 1H), 2.60 (d, *J =* 16.8 Hz, 1H), 2.39 (dt, *J =* 13.2, 8.8 Hz, 1H), 2.33 -2.19 (m, 3H), 2.11 (d, *J* = 10.0 Hz, 2H), 1.94 (dd, *J =* 39.8, 7.8 Hz, 7H), 1.65 (dd, *J =* 23.5, 11.3 Hz, 2H), 1.53 (dd, *J =* 22.8, 10.0 Hz, 2H), 1.33 - 1.19 (m, 3H).LC-MS: [M+H]⁺ = 807.46

### Example 411

### Synthesis of compound 375

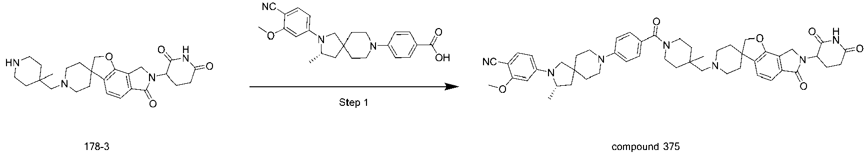

Step 1: (Compound 375) was prepare with reference to step 3 of Compound 267.
¹H NMR (600 MHz, DMSO-*d*₆) δ 11.00 (s, 1H), 7.39 - 7.33 (m, 2H), 7.28 (d, *J =* 8.2 Hz, 3H), 6.98 (d, *J =* 8.1 Hz, 2H), 6.27 (d, *J* = 8.7 Hz, 1H), 6.19 (s, 1H), 5.10 (dd, *J =* 13.2, 5.2 Hz, 1H), 4.67 (t, *J =* 10.9 Hz, 2H), 4.41 (t, *J =* 8.5 Hz, 1H), 4.24 (d, *J =* 17.1 Hz, 2H), 4.03 (d, *J =* 6.6 Hz, 2H), 3.87 (s, 3H), 3.58 (d, *J* = 9.2 Hz, 2H), 3.43 (d, *J =* 10.6 Hz, 1H), 3.37 - 3.31 (m, 4H), 3.30-3.20 (m, 6H), 3.14 (s, 2H), 2.97 - 2.87 (m, 1H), 2.60 (d, *J =* 17.2 Hz, 1H), 2.45 - 2.41 (m, 1H), 2.38 (d, *J =* 13.0 Hz, 2H), 2.25 (dd, *J =* 12.7, 7.7 Hz, 1H), 2.02 - 1.96 (m, 1H), 1.91 (t, *J =* 14.6 Hz, 2H), 1.78 - 1.69 (m, 2H), 1.58 (dd, *J =* 12.7, 6.3 Hz, 2H), 1.51 (s, 3H), 1.24 (d, *J* = 3.7 Hz, 4H), 1.22 (d, *J =* 6.9 Hz, 3H).LC-MS: [M+H]⁺ = 854.66

### Example 412

### Synthesis of compound 377

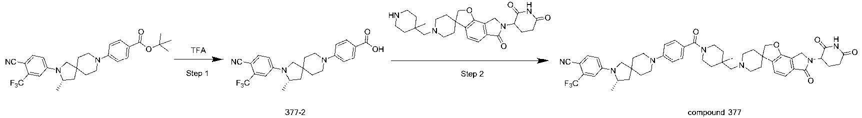

Step 1: (Compound 377-2) was prepared with reference to step 2 of Compound 267.
LC-MS: [M+H]⁺ = 444.29

Step 2: (Compound 377) was prepared with reference to step 3 of Compound 267.
¹H NMR (600 MHz, *DMSO-d*₆) δ 10.99 (s, 1H), 7.78 (d, *J =* 8.8 Hz, 1H), 7.36 (d, *J =* 7.5 Hz, 1H), 7.29 (d, *J =* 8.1 Hz, 3H), 6.98 (dd, *J =* 10.3, 5.1 Hz, 3H), 6.92 (dd, *J* = 8.9, 1.8 Hz, 1H), 5.10 (dd, *J =* 13.2, 5.0 Hz, 1H), 4.67 (q, *J =* 9.7 Hz, 2H), 4.40 (d, *J =* 17.2 Hz, 1H), 4.24 (d, *J =* 17.1 Hz, 1H), 4.12 (dd, *J =* 13.1, 6.5 Hz, 1H), 3.58 (d, *J =* 10.5 Hz, 2H), 3.50 (d, *J =* 10.8 Hz, 1H), 3.39 (d, *J =* 10.8 Hz, 1H), 3.35 (dd, *J =* 13.2, 5.1 Hz, 3H), 3.31 - 3.19 (m, 6H), 3.14 (s, 2H), 2.96 - 2.88 (m, 1H), 2.62 (s, 1H), 2.48 - 2.42 (m, 1H), 2.41 - 2.34 (m, 2H), 2.27 (dd, *J =* 13.0, 7.6 Hz, 1H), 2.02 - 1.95 (m, 1H), 1.91 (t, *J =* 14.6 Hz, 2H), 1.80 - 1.70 (m, 3H), 1.63 - 1.46 (m, 7H), 1.25 - 1.21 (m, 6H).LC -MS: [M+H]⁺ = 892.66

### Example 413

### Synthesis of compound 378

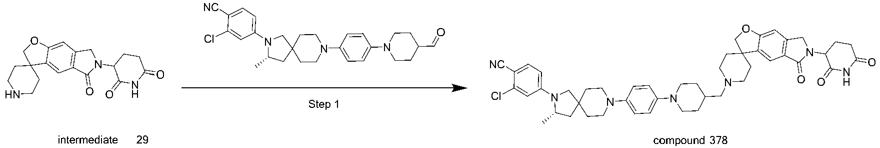

Step 1: (Compound 378) was prepared with reference to step 1 of Compound 267.
¹H NMR (600 MHz, DMSO-*d*₆) δ 10.98 (s, 1H), 7.63 (d, *J =* 8.8 Hz, 1H), 7.43 (s, 1H), 7.17 (d, *J =* 51.1 Hz, 3H), 7.05 (d, *J =* 9.1 Hz, 2H), 6.82 (s, 1H), 6.68 (d, *J =* 8.8 Hz, 1H), 5.04 (dd, *J =* 13.3, 5.1 Hz, 1H), 4.64 (dt, *J =* 27.3, 13.6 Hz, 2H), 4.37 (d, *J =* 17.2 Hz, 1H), 4.24 (dd, *J =* 17.2, 6.6 Hz, 1H), 4.10 - 4.02 (m, 1H), 3.70 (s, 2H), 3.39 (s, 4H), 3.10 (s, 5H), 2.95 - 2.85 (m, 2H), 2.71 (dd, *J =* 60.0, 15.4 Hz, 1H), 2.62 - 2.56 (m, 1H), 2.42 - 2.32 (m, 2H), 2.26 (t, *J* = 11.5 Hz, 3H), 2.08 (s, 1H), 2.01 (s, 1H), 1.99 - 1.93 (m, 4H), 1.91 (s, 3H), 1.64 (s, 3H), 1.46 (s, 2H), 1.24 (s, 1H), 1.22 (d, *J =* 6.0 Hz, 3H).LC-MS: [M+H]⁺ = 816.66

### Example 414

### Synthesis of compound 378

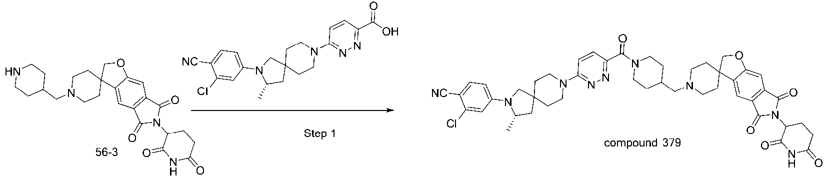

Step 1: (Compound 379) ws prepared by reference to step 3 of Compound 267.
LC-MS: [M+H]⁺ = 860.56
¹H NMR (600 MHz, DMSO-*d*₆) δ 11.12 (s, 1H), 7.62 (d, *J =* 5.9 Hz, 1H), 7.60 (s, 1H), 7.55 (d, *J* = 9.5 Hz, 1H), 7.37 (t, *J =* 4.9 Hz, 2H), 6.81 (s, 1H), 6.67 (d, *J=* 9.4 Hz, 1H), 5.11 (dd, *J =* 13.0, 5.7 Hz, 1H), 4.74 (d, *J =* 20.6 Hz, 2H), 4.52 (d, *J* = 12.7 Hz, 1H), 4.22 - 3.99 (m, 2H), 3.92 - 3.78 (m, 2H), 3.76 - 3.57 (m, 4H), 3.24 - 3.03 (m, 5H), 2.98 - 2.82 (m, 2H), 2.61 (d, *J=* 17.0 Hz, 1H), 2.29 (dd, *J =* 12.8, 7.2 Hz, 3H), 2.20 (s, 1H), 2.14 - 1.96 (m, 3H), 1.91 (d, *J= 12.8* Hz, 2H), 1.83 - 1.64 (m, 3H), 1.60 (dd, *J =* 12.9, 6.6 Hz, 1H), 1.48 (m, 2H), 1.40 - 1.14 (m, 7H).

### Example 415

### Synthesis of compound 380

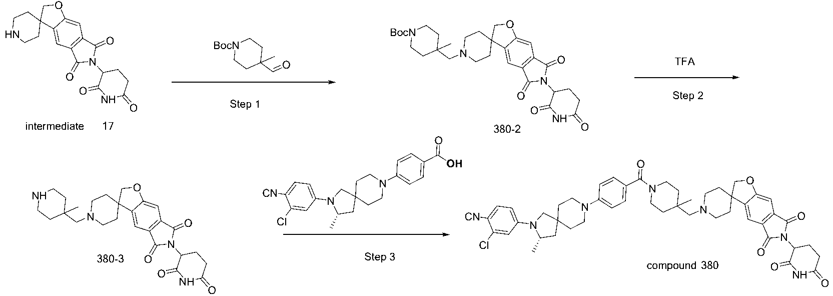

### Step 1: (Compound 380-2) was prepared with reference to step lof Compound 267. LC-MS: [M+H]⁺ = 581.48

### Step 2: (Compound 380-3) was prepared with reference to step 2 of Compound 267. LC-MS: [M+H]⁺ = 481.39

Step 3: (Compound 380) was prepare with reference to step 3 of Compound 267.
¹H NMR (600 MHz, DMSO-*d*₆) δ 11.12 (s, 1H), 7.67 - 7.54 (m, 2H), 7.36 (d, *J* = 9.2 Hz, 1H), 7.29 (d, *J =* 8.2 Hz, 2H), 6.98 (d, *J* = 8.4 Hz, 2H), 6.80 (d, *J =* 2.3 Hz, 1H), 6.67 (dd, *J* = 8.9, 2.3 Hz, 1H), 5.11 (dd, *J =* 12.8, 5.5 Hz, 1H), 4.76 (d, *J* = 8.8 Hz, 2H), 4.05 (q, *J =* 6.6 Hz, 1H), 3.44 (d*, J* = 11.0 Hz, 3H), 3.38 - 3.18 (m, 12H), 3.15 (s, 1H), 2.89 (ddd, *J* = 18.1, 13.8, 5.5 Hz, 1H), 2.61 (d, *J* = 16.9 Hz, 1H), 2.44 - 2.32 (m, 2H), 2.25 (dd, *J* = 12.8, 7.7 Hz, 1H), 2.05 (m, 1H), 1.94 (dd, *J* = 24.9, 14.3 Hz, 2H), 1.74 (m, 2H), 1.54 (m, 7H), 1.33 - 1.13 (m, 7H). LC-MS: [M+H]⁺ = 873.76

### Example 416

### Synthesis of compound 381

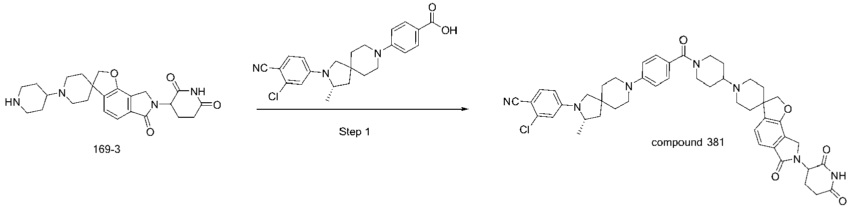

### Step 1: (Compound 381) was prepared with reference to step 3 of Compound 267. LC-MS: [M+H]⁺ = 830.56

¹H NMR (600 MHz, DMSO-*d*₆) δ 10.99 (s, 1H), 7.58 (d, *J =* 8.8 Hz, 1H), 7.41 - 7.24 (m, 4H), 6.98 (d, *J =* 8.4 Hz, 2H), 6.79 (d, *J* = 2.3 Hz, 1H), 6.65 (dd, *J* = 8.9, 2.4 Hz, 1H), 5.06 (dd, *J* = 13.3, 5.1 Hz, 1H), 4.66 (s, 2H), 4.40 (d, *J* = 17.3 Hz, 1H), 4.24 (d, *J* = 17.3 Hz, 1H), 4.02 (m, 2H), 3.45 - 3.28 (m, 6H ), 3.28 - 3.16 (m, 3H), 3.10 (s, 2H), 2.89 (m, 3H), 2.65 - 2.54 (m, 1H), 2.39 (m, 1H), 2.27 - 2.06 (m, 5H), 2.03 - 1.90 (m, 3H), 1.73 (m, 2H), 1.69 - 1.53 (m, 3H), 1.50 (s, 2H), 1.21 (t, *J =* 5.8 Hz, 4H).

### Example 417

### Synthesis of compound 382

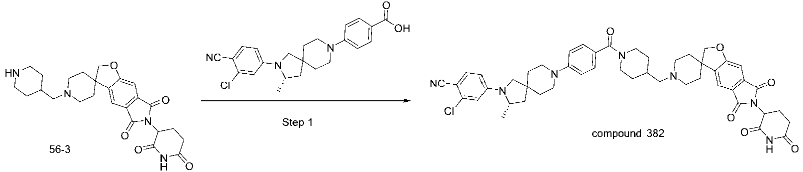

Step 3: (Compound 382) was prepared with reference to step 3 of Compound 267.
¹H NMR (600 MHz, DMSO-*d*₆) δ 11.13 (s, 1H), 7.73 (s, 1H), 7.58 (d, *J =* 8.9 Hz, 1H), 7.35 (s, 1H), 7.27 (d, *J =* 8.4 Hz, 2H), 6.98 (d, *J* = 8.5 Hz, 2H), 6.79 (d, *J=* 2.4 Hz, 1H), 6.66 (dd, *J =* 9.0, 2.3 Hz, 1H), 5.09 (dd, *J =* 12.9, 5.4 Hz, 1H), 4.71 (d, *J =* 15.9 Hz, 2H), 4.03 (m, 2H), 3.45 - 3.28 (m, 7H), 3.28 - 3.16 (m, 3H), 3.12 - 2.78 (m, 6H), 2.65 - 2.54 (m, 1H), 2.31 - 2.09 (m, 4H), 2.07 - 1.93 ( m, 3H), 1.75 (m, 4H), 1.58 (dd, *J =* 12.9, 6.6 Hz, 1H), 1.50 (d, *J =* 5.0 Hz, 2H), 1.21 (t, *J =* 5.8 Hz, 6H).
LC-MS: [M+H]⁺ = 858.66

### Example 418

### Synthesis of compound 383

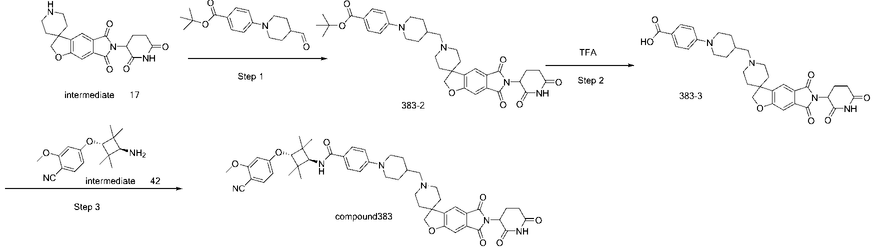

### Step 1: (Compound 383-2) was prepared with reference to step 1 of Compound 267. LC-MS: [M+H]⁺ = 643.48

### Step 2: Compound 383-3) was prepared with reference to step 2 of Compound 267. LC-MS: [M+H]⁺ = 587.48

(Step 3: Compound 383) was prepare with reference to step 3 of Compound 267.
¹H NMR (600 MHz, DMSO-*d*₆) δ 11.12 (s, 1H), 7.77 (d, *J =* 8.4 Hz, 2H), 7.70 - 7.59 (m, 2H), 7.52 (d, *J =* 9.2 Hz, 1H), 7.38 (s, 1H), 7.00 (d, *J =* 8.6 Hz, 2H), 6.64 *(d, J=* 2.2 Hz, 1H), 6.55 (dd, *J =* 8.6, 2.2 Hz, 1H), 5.11 (dd, *J =* 12.9, 5.5 Hz, 1H), 4.74 (d, *J=* 19.7 Hz, 2H), 4.28 (s, 1H), 4.06 (d, *J =* 9.1 Hz, 1H), 3.91 (s, 5H), 3.62 (m, 2H), 3.40 - 3.32 (m, 1H), 3.14 - 3.03 (m, 3H), 2.92 - 2.79 (m, 3H), 2.66 - 2.58 (m, 1H), 2.30 (m, 2H), 2.17 - 1.98 (m, 4H), 1.95 - 1.81 (m, 3H), 1.42 - 1.28 (m, 2H), 1.23 (s, 6H), 1.15 (s, 6H).LC-MS: [M+H]⁺ = 843.76

### Example 419

### Synthesis of compound 385

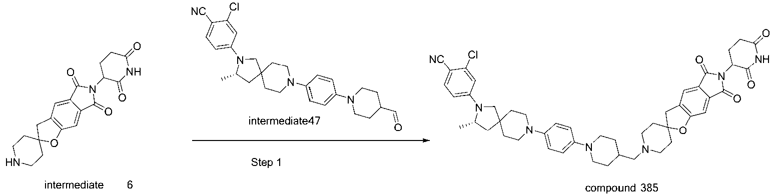

Step 1: (Compound 385) was prepared with reference to step 3 of Compound 267.
¹H NMR (600 MHz, DMSO-*d*₆) δ 11.11 (s, 1H), 7.83 (d, *J =* 12.3 Hz, 1H), 7.63 (d, *J =* 8.8 Hz, 1H), 7.54 - 6.94 (m, 5H), 6.82 (d, *J* = 2.3 Hz, 1H), 6.68 (dd, *J =* 8.9, 2.3 Hz, 1H), 5.10 (dd, *J =* 12.9, 5.4 Hz, 1H), 4.06 (q, *J =* 6.6 Hz, 1H), 3.72 (s, 3H), 3.46 - 3.31 (m, 7H), 3.28 (s, 1H), 3.26 - 3.10 (m, 5H), 2.90 (ddd, J= 17.9, 13.8, 5.5 Hz, 2H), 2.64 - 2.58 (m, 1H), 2.54 (d, *J =* 4.0 Hz, 1H), 2.19 (m, 5H), 2.09 - 1.98 (m, 2H), 1.92 (s, 4H), 1.77 - 1.55 (m, 3H), 1.38 (d, *J* = 51.7 Hz, 2H), 1.22 (d, *J =* 6.0 Hz, 4H).LC-MS: [M+H]⁺ = 830.66

### Example 420

### Synthesis of compound 386

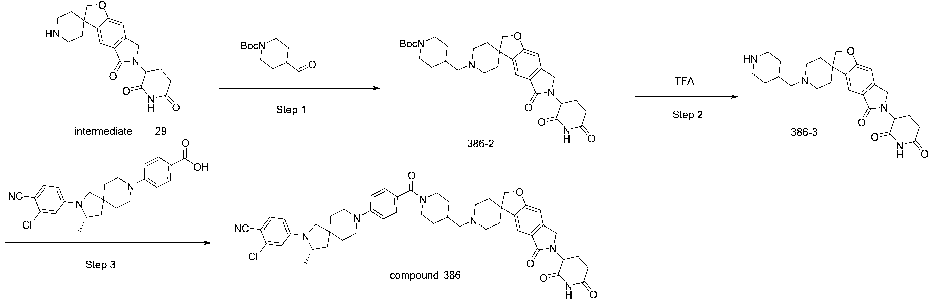

### Step 1: (Compound 386-2) was prepared with reference to step 1 of Compound 267. LC-MS: [M+H]⁺ = 553.45

### Step 2: (Compound 386-3) was prepared with reference to step 2 of Compound 267. LC-MS: [M+H]⁺ = 453.59

Step 3: (Compound 386) was prepared with reference to step 3 of Compound 267.
¹H NMR (600 MHz, DMSO-*d*₆) δ 10.98 (s, 1H), 7.61 (d, *J =* 8.8 Hz, 1H), 7.42 (s, 1H), 7.35 - 7.23 (m, 2H), 7.04 (d, *J= 10.4* Hz, 1H), 7.01 - 6.90 (m, 2H), 6.81 (d, *J* = 2.3 Hz, 1H), 6.67 (dd, *J =* 9.0, 2.3 Hz, 1H), 5.04 (dd, *J =* 13.3, 5.2 Hz, 1H), 4.70 - 4.54 (m, 2H), 4.36 (dd, *J =* 17.4, 3.0 Hz, 2H), 4.24 (dd, *J =* 17.2, 9.0 Hz, 2H), 4.05 (m, 2H), 3.59 (d, *J* = 11.8 Hz, 2H), 3.45 (d, *J* = 10.8 Hz, 1H), 3.36 (m, 4H), 3.22 (m, 2H), 3.07 (q, *J =* 8.6, 6.7 Hz, 3H), 3.01 - 2.86 (m, 3H), 2.65 - 2.56 (m, 1H), 2.38 (m, 1H), 2.24 (m, 3H), 2.15 (s, 1H), 1.95 (d, *J =* 14.0 Hz, 2H), 1.87 - 1.70 (m, 4H), 1.59 (dd, *J =* 12.8, 6.5 Hz, 1H), 1.51 (m, 2H), 1.27 - 1.12 (m, 5H).LC-MS: [M+H]⁺ = 844.76

### Example 421

### Synthesis of compound 387

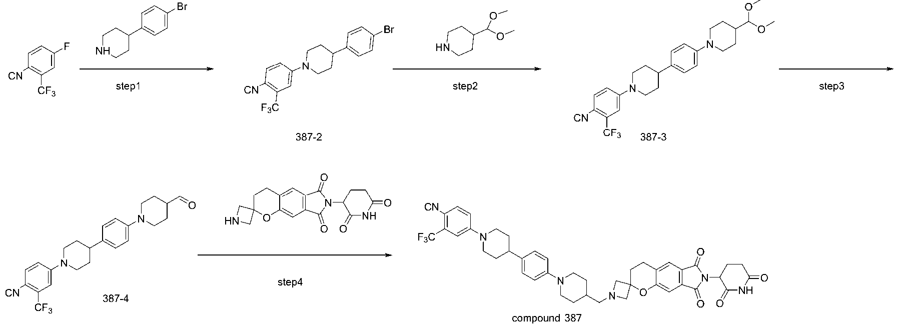

### Step 1: (Compound 387-2) LC-MS: [M+H]⁺ = 409.19

In a glass vial, 4-fluoro-2-(trifluoromethyl)benzonitrile (100.00 mg, 528.79 µmol), 4-(4-bromophenyl)piperidine (152.38 mg, 634.54 µmol) and K₂CO₃(146.16 mg, 1060 µmol) were added sequentially, and the solvent DMSO (4.0 mL) was added, and the reaction solution was stirred at 80° for 8 h. After the reaction was completed, the reaction solution was concentrated, and then purified by normal-phase Flash (PE:EA=3:1) to obtain a white solid compound 387-2 (120 mg, 55.45%).

### Step 2: (Compound 387-2) LC-MS: [M+H]⁺ = 488.30

In a glass vial, 387-2 (100.00 mg, 244.35 µmol), 4-(dimethoxymethyl)piperidine (77.81 mg, 324.87 µmol), Cs₂CO₃ (238.84 mg, 733.05 µmol), Ruphos (4.43 mg, 24.43 µmol) and Ruphos-G3 (20.48 mg, 24.48 µmol) were added sequentially, the solvent dioxane (2.0 mL) was added, and the reaction solution was stirred at 100° for 8 h. When the reaction was completed, the reaction solution was concentrated and then purified by normal phase Flash (PE:EA=2:1) to obtain awhite solid compound 387-3 (70 mg, 58.76%).

### Step 3: (Compound 387-4) LC-MS: [M+H]⁺ = 442.30

In a glass vial, 387-3 (30.0 mg, 61.53 µmol) and TFA (0.3 mL) were added sequentially, and the solvent DCM /H₂O(1.2 mL) was added, and the reaction solution was stirred at room temperature for 1 h, and when the reaction was completed, it was concentrated, and then purified by reversed-phase Flash (ACN:H₂O) to obtain a white solid compound 387-4 (20 mg, 73.62%).

### Step 4: (Compound 387) was prepared with reference to step 1 of Example 267. LC-MS: [M+H]⁺ =781.56

¹H NMR (600 MHz, DMSO-*d*₆) δ 11.12 (s, 1H), 7.81 (d, *J =* 8.8 Hz, 1H), 7.78 (s, 1H), 7.34 - 7.29 (m, 2H), 7.26 (dd, *J =* 9.0, 2.5 Hz, 1H), 7.13 (d, *J =* 69.3 Hz, 4H), 5.10 (dd, *J=* 12.9, 5.5 Hz, 1H), 4.41 (dd, *J =* 60.3, 29.0 Hz, 4H), 4.17 (d, *J =* 12.7 Hz, 2H), 3.64 (d, *J =* 11.9 Hz, 3H), 3.04 (m, 4H), 2.91 (d, *J =* 34.1 Hz, 4H), 2.77 (s, 2H), 2.59 (s, 1H), 2.26 (s, 2H), 2.09 - 1.99 (m, 1H), 1.83 (d, *J =* 12.9 Hz, 5H), 1.61 (d, *J =* 12.7 Hz, 2H), 1.41 (s, 2H).

### Example 422

### Synthesis of compound 388

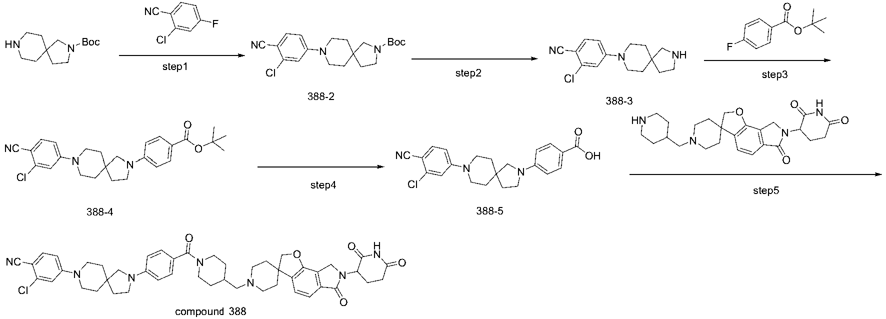

### Step 1: (Compound 388-2) was prepared with reference to step 1 of Example 421. LC-MS: [M-56+H]⁺ =320.28

### Step 2: (Compound 388-3) was prepared with reference to step 2 of Example 267. LC-MS: [M+H]⁺ =276.37

### Step 3: (Compound 388-4) was prepared with reference to step 1 of Example 421. LC-MS: [M+H]⁺ =453.40

### Step 4: (Compound 388-5) was prepared with reference to step 2 of Example 267. LC-MS: [M+H]⁺ =396.30

### Step 5: Prepared with reference to step 3 of Example 267. LC-MS: [M+H]⁺ =830.55

¹H NMR (600 MHz, DMSO-*d*₆) δ 11.00 (s, 1H), 7.64 (d, *J =* 8.9 Hz, 1H), 7.36 (d, *J =* 7.5 Hz, 1H), 7.27 (t, *J =* 8.1 Hz, 3H), 7.18 (d, *J =* 2.5 Hz, 1H), 7.01 (dd, *J* = 9.1, 2.5 Hz, 1H), 6.60 - 6.49 (m, 2H), 5.10 (dd, *J=* 13.3, 5.0 Hz, 1H), 4.72 - 4.55 (m, 2H), 4.40 *(d, J=* 17.1 Hz, 1H), 4.24 (d, *J=* 17.2 Hz, 1H), 4.13 (s, 2H), 3.54 (d, *J =* 5.7 Hz, 2H), 3.50 - 3.43 (m, 3H), 3.37 (d, *J =* 13.9 Hz, 3H), 3.22 (s, 2H), 3.07 (s, 4H), 2.92 (m, 3H), 2.43 (dd, *J =* 13.1, 4.4 Hz, 1H), 2.22 (m, 3H), 2.06 - 1.89 (m, 5H), 1.78 (d, *J=* 11.8 Hz, 2H), 1.62 (s, 4H), 1.21 (d, J = 16.8 Hz, 3H).

### Example 423

### Synthesis of compound 389

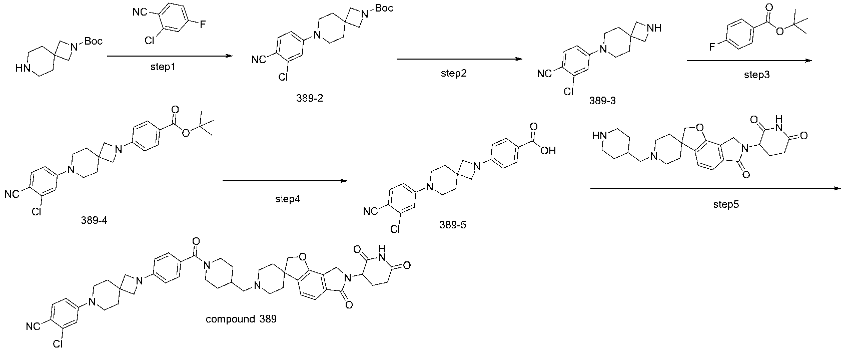

### Step 1: (Compound 389-2) LC-MS: [M-100+H]⁺ =262.27

In a glass vial, tert-butyl 2,7-diazaspiro[3.5]nonane-2-carboxylate (100.0 mg, 441.85 µmol), 2-chloro-4-fluorobenzonitrile (82.48 mg, 530.22 µmol) and DIEA (171.32 mg, 1330 µmol) were added sequentially, and the solvent DMSO (4.0 mL) was added. The reaction solution was stirred at 80 °C for 8 h. After the reaction was completed, the reaction solution was concentrated, and then purified by normal phase Flash (PE:EA=4:1) to obtain a yellow solid compound 389-2 (100 mg, 62.54%).

### Step 2: (Compound 389-3) was purified with reference to step 2 of Example 267. LC-MS: [M+H]⁺ =276.37

### Step 3: (Compound 389-4) was purified with reference to step 3 of Example 422. LC-MS: [M+H]⁺ =438.29

### Step 4: Compound 389-5) was purified with reference to step 2 of Example 267. LC-MS: [M+H]⁺ =382.25

### Step 5: (Compound 389) was purified with reference to step 3 of Example 267. LC-MS: [M+H]⁺ =816.10

¹H NMR (600 MHz, *DMSO*-*d*₆) δ 10.99 (s, 1H), 7.64 (d, *J =* 8.9 Hz, 1H), 7.36 (d, *J* = 7.6 Hz, 1H), 7.27 (dd, *J =* 15.0, 8.1 Hz, 3H), 7.19 (d, *J =* 2.4 Hz, 1H), 7.02 (dd, *J =* 9.1, 2.5 Hz, 1H), 6.43 (dd, *J=* 9.0, 2.4 Hz, 2H), 5.10 (dd, *J =* 13.3, 5.1 Hz, 1H), 4.70 - 4.63 (m, 2H), 4.43 - 4.36 (m, 3H), 4.24 (d, *J* = 17.1 Hz, 4H), 3.59 (d, *J=* 12.7 Hz, 2H), 3.49 - 3.41 (m, 5H), 3.13 - 3.03 (m, 4H), 3.00 - 2.85 (m, 3H), 2.65 - 2.57 (m, 1H), 2.42 (m, 1H), 2.24 (m, 2H), 2.01 - 1.93 (m, 3H), 1.87 - 1.75 (m, 7H), 1.21 (qd, J = 12.5, 6.7 Hz, 2H).

### Example 424

### Synthesis of compound 390

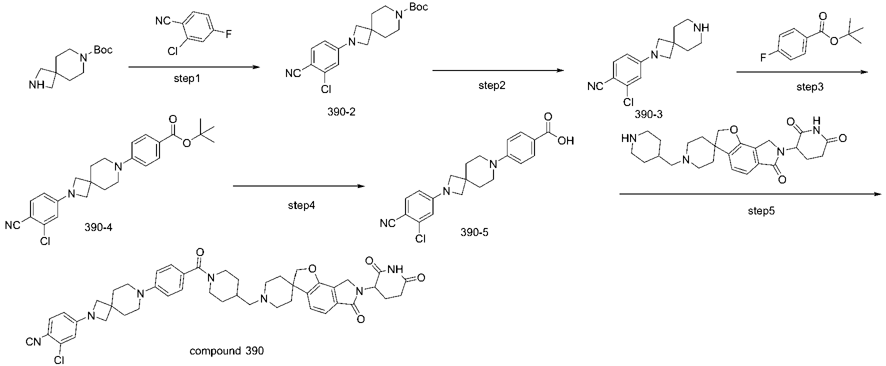

### Step 1: (Compound 390-2)

In a glass vial, tert-butyl 2,7-diazaspiro[3.5]nonane-7-carboxylate (100.0 mg, 416.07 µmol), 2-chloro-4-fluorobenzonitrile (77.67 mg, 499.28 µmol) and DIEA (161.33 mg, 1250 µmol) were added sequentially, and the solvent DMSO (4.0 mL) was added. The reaction solution was stirred at 80 °C for 8 h. After the reaction was completed, it was concentrated and then purified by normal-phase Flash (PE:EA=3:1) to obtain a white solid compound 390-2 (130 mg, 81.30%).LC-MS: [M+H]⁺ =362.30

### Step 2: (Compound 390-3) was prepared with reference to step 2 of Example 267. LC-MS: [M+H]⁺ =262.25

### Step 3: (Compound 390-4) was prepared with reference to step 3 of Example 422. LC-MS: [M+H]⁺ =438.20

### Step 4: (Compound 390-5) was prepared with reference to step 2 of Example 267. LC-MS: [M+H]⁺ =382.25

Step 5: (Compound 390) was purifiedn with reference to step 3 of Example 267.
¹H NMR (600 MHz, DMSO-*d*₆)δ 10.99 (s, 1H), 7.62 (d, *J =* 8.6 Hz, 1H), 7.36 (d, *J* = 7.5 Hz, 1H), 7.28 (dd, *J* = 8.1, 3.7 Hz, 3H), 7.03 - 6.95 (m, 2H), 6.60 (d, *J* = 2.2 Hz, 1H), 6.43 (dd, *J* = 8.7, 2.2 Hz, 1H), 5.10 (dd, *J =* 13.3, 5.1 Hz, 1H), 4.72 - 4.57 (m, 2H), 4.40 (d, *J =* 17.2 Hz, 1H), 4.24 (d, *J =* 17.2 Hz, 1H), 3.69 - 3.54 (m, 8H), 3.30 - 3.22 (m, 4H), 3.16 - 3.00 (m, 4H), 3.01 - 2.86 (m, 3H), 2.70 - 2.56 (m, 1H), 2.42 (m, 1H), 2.19 (m, 3H), 2.07 - 1.92 (m, 3H), 1.91 - 1.72 (m, 6H), 1.27 - 1.17 (m, 2H). LC-MS: [M+H]⁺ =816.55

### Example 425

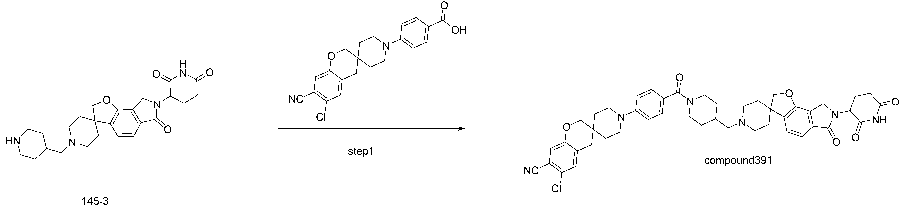

Step 1: (Compound 391) was prepared with reference to step 3 of Example 267.
1H NMR (600 MHz, DMSO-d6) δ 11.00 (s, 1H), 9.10 (s, 1H),7.52 - 7.33 (m, 2H), 7.31 - 7.15 (m, 3H), 7.12 - 6.91 (m, 2H), 5.10 (dd, *J* = 13.0, 5.1 Hz, 1H), 4.66 (s, 2H), 4.49 - 3.95 (m, 5H), 3.07 (m, 4H), 2.91 (d, *J* = 14.8 Hz, 3H), 2.79 (d, *J* = 36.4 Hz, 2H), 2.63 (d, *J =* 5.7 Hz, 2H), 2.28 - 2.09 (m, 2H), 2.06 - 1.90 (m, 3H), 1.79 (s, 1H), 1.52 (dd, *J =* 31.1, 11.5 Hz, 4H), 1.24 (s, 9H), 0.90 - 0.82 (m, 1H). LC-MS: [M+H]⁺ =817.56

### Example 426

### Synthesis of compound 392

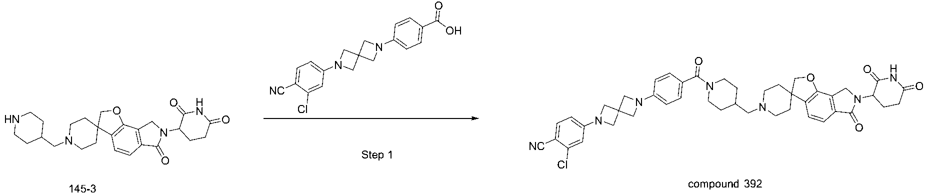

Step 1: (Compound 392) was prepared with reference to step 3 of Example 267.
¹H NMR (400 MHz, DMSO-*d*₆) δ 11.00 (s, 1H), 7.64 (d, *J =* 8.6 Hz, 1H), 7.36 (d, *J =* 7.5 Hz, 1H), 7.31 - 7.23 (m, 3H), 6.65 (d, *J* = 2.2 Hz, 1H), 6.50 - 6.43 (m, 3H), 5.10 (dd, *J =* 13.3, 5.1 Hz, 1H), 4.71 - 4.63 (m, 2H), 4.40 (d, *J =* 17.2 Hz, 1H), 4.26 (s, 1H), 4.20 (s, 4H), 4.07 (s, 4H), 3.59 (d, *J =* 12.3 Hz, 3H), 3.33 (d, *J =* 14.8 Hz, 1H), 3.16 - 2.84 (m, 7H), 2.65 - 2.56 (m, 1H), 2.42 (td, *J =* 13.2, 4.6 Hz, 1H), 2.29 - 2.10 (m, 3H), 2.04 - 1.90 (m, 3H), 1.78 (d, *J =* 12.2 Hz, 2H), 1.20 (td, *J =* 12.7, 12.0, 8.1 Hz, 2H).LC-MS: [M+H]⁺ =788.35

### Example 427

### Synthesis of compound 393

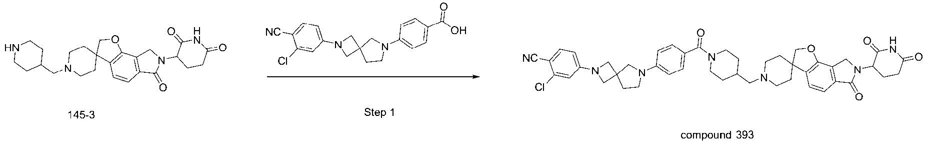

Step 1: (Compound 392) was prepared with reference to step 3 of Example 267.
¹H NMR (400 MHz, DMSO-*d*₆) δ 10.99 (s, 1H), 7.87 - 7.60 (m, 2H), 7.36 (d, *J =* 7.6 Hz, 1H), 7.28 (dd, *J =* 8.1, 3.2 Hz, 2H), 6.76 (d, *J =* 8.4 Hz, 1H), 6.67 (dd, *J =* 16.2, 2.2 Hz, 1H), 6.59 - 6.52 (m, 1H), 6.48 (ddd, *J =* 11.0, 8.5, 2.2 Hz, 1H), 5.10 (dd, *J =* 13.3, 5.1 Hz, 1H), 4.71 - 4.62 (m, 2H), 4.40 (d, *J =* 17.2 Hz, 1H), 4.24 (d, *J =* 17.2 Hz, 1H), 4.15 (d, *J =* 8.3 Hz, 1H), 4.09 - 3.96 (m, 3H), 3.87 (dd, *J =* 13.6, 7.8 Hz, 1H), 3.79 (s, 1H), 3.37 (t, *J =* 6.7 Hz, 3H), 3.14 - 3.05 (m, 4H),3.00 - 2.85 (m, 3H), 2.69 - 2.53 (m, 2H), 2.48 - 2.35 (m, 2H), 2.34 - 2.09 (m, 5H), 1.97 (t, *J =* 13.0 Hz, 3H), 1.79 (d, *J =* 13.0 Hz, 2H), 1.21 (d, *J =* 14.2 Hz, 3H).LC-MS: [M+H]⁺ =802.37

### Example 428

### Synthesis of compound 394

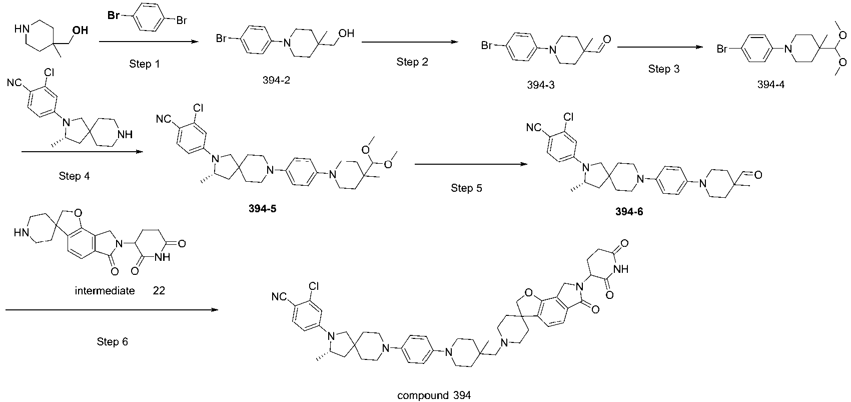

### Step 1: (Compound 394-2) LC-MS: [M+H]⁺ =284.20

(4-methylpiperidin-4-yl)methanol (2 g, 15.48 mmol) and 1,4-dibromobenzene (10.96 g, 46.44 mmol) were added sequentially to a 100 mL round bottomed flask, then solvent 1,4-dioxane (50 mL) was added, and palladium acetate(0.1eq), 1,1'-binaphthyl-2,2'-bisdiphenylphosphine (0.2eq), cesium carbonate (3eq) were added under the protection of nitrogen, and the reaction solution was stirred at 80°C for 4 h. After the reaction was completed, it was concentrated, then purified by column chromatography (PE:EA=5:1) to obtain a white solid compound 394-2 (1.28 g, 29.10%).

### Step 2: (Compounds 392-3)

In a 100 mL round bottomed flask, 394-2 (1 g, 15.48 mmol) and 2-iodobenzoic acid (1.5 eq) were added sequentially, solvent acetonitrile (50 mL) was added, and the reaction solution was stirred at 80°C for 1.5 h. After the reaction was completed, the reaction solution was concentrated, and then purified by column chromatography (PE:EA=5:1) to obtain a white solid compound 394-3 ( 800 mg, 80.57%) LC-MS: [M+H]⁺ =282.18

### Step 3: (Compounds 392-4)

In a 100 mL round bottomed flask, 394-3 (500 mg, 1.77 mmol) and trimethyl orthoformate (3eq) were added sequentially, solvent methanol (50 mL) and concentrated hydrochloric acid (1 mL) were added, and the reaction solution was stirred at 70°C for 3 h. After the reaction was completed, the reaction solution was concentrated, and then purified by column chromatography (PE:EA=5:1) to obtain awhite solid compound 394-4 (300 mg, 51.58%).
LC-MS: [M+H]⁺ =328.25

Step 4: (Compounds 392-5) was prepared with reference to step 3 of Example 443.

### Step 5: (Compounds 392-6)

394-5 (200 mg, 372.34 µmol) and the solvent 10% concentrated hydrochloric acid (5 mL) was added sequentially to a 100 mL round bottomed flask, and the reaction solution was stirred at 50°C for 2 h. When the reaction was completed, it was concentrated, then purified by column chromatography (PE:EA=5:1) to obtain a white solid compound 394-6 (100 mg, 54.69%) LC -MS: [M+H]⁺ =491.08

### Step 6: (Compound 392) was prepared with reference to step 1 of Example 267. LC-MS: [M+H]⁺ =830.47

¹H NMR (600 MHz, DMSO-*d*₆) δ 10.99 (s, 1H), 7.63 (d, *J =* 8.8 Hz, 2H), 7.37 (d, *J =* 7.6 Hz, 2H), 7.29 (d, *J =* 7.7 Hz, 1H), 6.82 (d, *J =* 2.3 Hz, 2H), 6.69 (d, *J* = 9.0 Hz, 2H), 5.10 (dd, *J =* 13.3, 5.1 Hz, 1H), 4.71 - 4.63 (m, 3H), 4.40 (d, *J =* 17.2 Hz, 2H), 4.25 (d, *J =* 17.2 Hz, 2H), 4.07 (d, *J =* 7.5 Hz, 2H), 3.21-3.17 (m, 3H), 2.92 (ddd, *J =* 17.3, 13.6, 5.5 Hz, 2H), 2.59 (s, 3H), 2.48 - 2.33 (m, 5H), 2.02 - 1.89 (m, 6H), 1.69 (d, *J =* 48.1 Hz, 10H), 1.23 (t, *J* = 6.8 Hz, 7H).

### Example 429

### Synthesis of compound 395

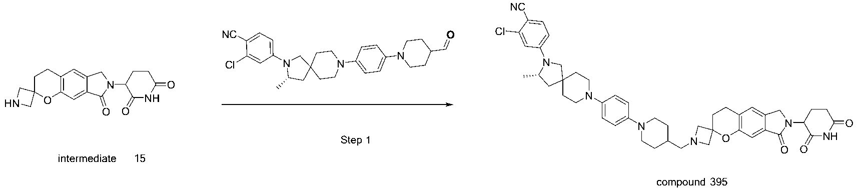

Step 1: (Compound 395) was prepared with reference to step 1 of Example 267.
¹ H NMR (600 MHz, DMSO-*d*₆) δ 10.99 (s, 1H), 7.63 (d, *J =* 8.8 Hz, 1H), 7.39 (s, 1H), 7.20 - 6.98 (m, 5H), 6.81 (d, *J* = 2.3 Hz, 1H), 6.68 (d, *J* = 8.5 Hz, 1H), 5.08 (dd, *J =* 13.3, 5.1 Hz, 1H), 4.47 (s, 1H), 4.38 (d, *J* = 11.3 Hz, 2H), 4.34 (s, 2H), 4.31 - 4.26 (m, 1H), 4.23 (d, *J* = 16.8 Hz, 1H), 4.06 (d, *J* = 6.2 Hz, 2H), 3.36 (d, *J =* 27.7 Hz, 5H), 3.25 (s, 2H), 2.99 - 2.85 (m, 4H), 2.60 (d, *J =* 15.0 Hz, 2H), 2.44 - 2.34 (m, 2H), 2.24 (q, *J =* 7.9 Hz, 4H), 2.05 - 1.95 (m, 2H), 1.87 (d, *J* = 47.6 Hz, 5H), 1.48 - 1.29 (m, 3H), 1.22 (d, *J=* 6.0 Hz, 3H).LC-MS: [M+H]⁺ =802.42

### Example 430

### Synthesis of compound 397

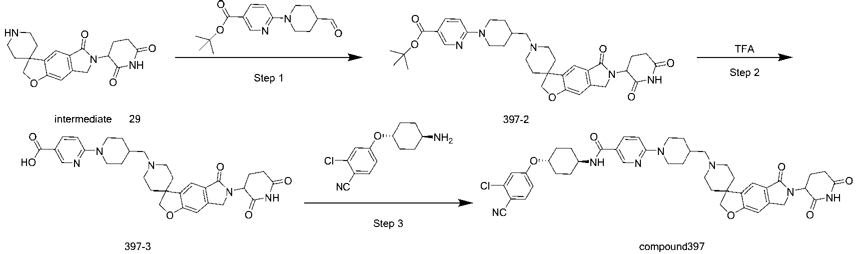

### Step 1: (Compound 397-2) was prepared with reference to step 1 of Example 267. LC-MS: [M+H]⁺ = 630.58

### Step 2: (Compound 397-3) was prepared with reference to step 2 of Compound 267. LC-MS: [M+H]⁺ = 574.38

Step 3: (Compound 397) was prepared with reference to step 3 of Compound 267.
¹H NMR (600 MHz, DMSO-*d*₆) δ 10.98 (s, 1H), 8.60 (d, *J =* 2.3 Hz, 1H), 8.09 - 7.95 (m, 2H), 7.87 (d, *J =* 8.8 Hz, 1H), 7.45 - 7.37 (m, 2H), 7.15 (dd, *J =* 8.8, 2.4 Hz, 1H), 7.04 (d, *J =* 10.0 Hz, 1H), 6.92 (d, *J=* 9.1 Hz, 1H), 5.07 (ddd, *J =* 33.6, 13.3, 5.1 Hz, 1H), 4.65 (q, *J* = 9.4 Hz, 1H), 4.57 - 4.51 (m, 1H), 4.44 (d, *J =* 13.0 Hz, 2H), 4.37 (d, *J* = 17.2 Hz, 1H), 4.24 (dd, *J =* 17.2, 8.7 Hz, 1H), 3.81 (d, *J =* 5.8 Hz, 2H), 3.37 (s, 2H), 3.08 (d, *J=* 22.3 Hz, 3H), 2.99 - 2.87 (m, 3H), 2.60 (d, *J =* 16.7 Hz, 1H), 2.39 (qd, *J =* 13.2, 4.4 Hz, 1H), 2.26 (t, *J =* 13.4 Hz, 2H), 2.18 (s, 1H), 2.14 - 2.07 (m, 2H), 1.97 (d, *J =* 5.1 Hz, 2H), 1.91 (d, *J =* 8.0 Hz, 3H), 1.85 (d, *J =* 11.6 Hz, 2H), 1.57 - 1.45 (m, 4H), 1.28 - 1.17 (m, 3H). LC-MS: [M+H]⁺ = 806.56

### Example 431

### Synthesis of compound 398

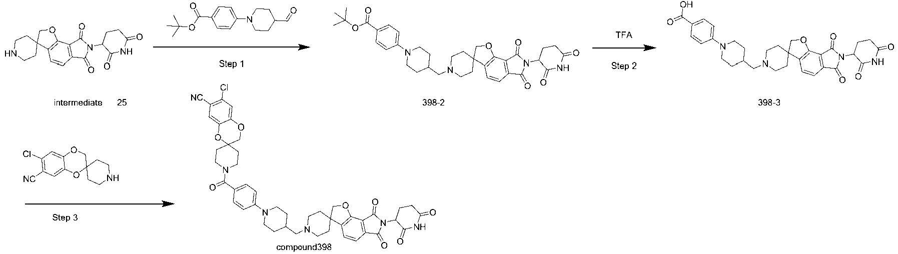

### Step 1: (Compound 398-2) was prepared with reference to step 1 of Compound 267. LC-MS: [M+H]⁺ = 643.48

### Step 2: (Compound 398-3) was prepared with reference to step 2 of Compound 267. LC-MS: [M+H]⁺ = 587.48

Step 3: (Compound 398) was prepared with reference to step 3 of Compound 267.
¹H NMR (600 MHz, DMSO-*d*₆) δ 11.13 (s, 1H), 7.60 - 7.55 (m, 2H), 7.51 (d, *J* = 7.3 Hz, 1H), 7.36 (s, 1H), 7.31 (d, *J= 8.6* Hz, 2H), 6.99 (d, *J=* 8.8 Hz, 2H), 5.12 (dd, *J =* 12.9, 5.4 Hz, 1H), 4.82 (s, 2H), 4.21 (s, 2H), 3.86 (d, *J=* 12.4 Hz, 2H), 3.62 (d, *J =* 11.8 Hz, 3H), 3.36 (d, *J =* 31.6 Hz, 3H), 3.10 (dd, *J =* 21.3, 8.2 Hz, 4H), 2.89 (td, *J =* 17.4, 5.4 Hz, 1H), 2.80 (t, *J =* 11.6 Hz, 2H), 2.60 (d, *J =* 17.3 Hz, 1H), 2.54 (dd, *J =* 13.2, 4.4 Hz, 1H), 2.23 (t*, J =* 12.3 Hz, 2H), 2.09 - 2.05 (m, 1H), 2.03 (d, *J =* 14.5 Hz, 3H), 1.84 (d, *J =* 11.7 Hz, 2H), 1.74 (dd, *J =* 17.9, 7.4 Hz, 4H), 1.32 (dd, *J =* 21.0, 11.5 Hz, 2H).LC-MS: [M+H]⁺ = 833.56

### Example 432

### Synthesis of compound 399

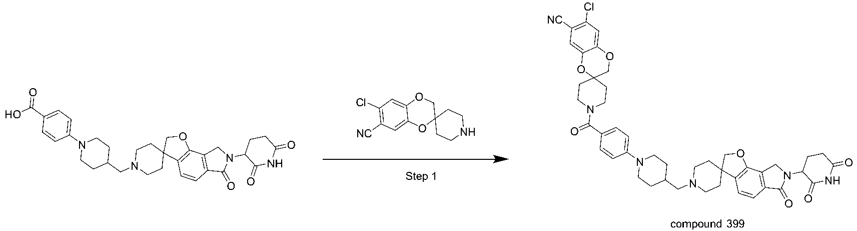

Step 1: (Compound 399) was prepare with reference to step 3 of Compound 267.
¹H NMR (600 MHz, DMSO-*d*₆) δ 11.01 (s, 1H), 7.59 (s, 1H), 7.37 (d, *J =* 7.3 Hz, 2H), 7.31 (d, *J =* 8.6 Hz, 2H), 7.29 (d, *J* = 7.6 Hz, 1H), 6.99 (d, *J =* 8.8 Hz, 2H), 5.10 (dd, *J =* 13.3, 5.0 Hz, 1H), 4.67 (q, *J =* 9.4 Hz, 2H), 4.40 (d, *J =* 17.2 Hz, 1H), 4.24 (d, *J =* 17.1 Hz, 1H), 4.21 (s, 2H), 3.86 (d, *J =* 12.3 Hz, 3H), 3.33 (s, 3H), 3.13 - 3.04 (m, 4H), 2.97 - 2.88 (m, 1H), 2.80 (t, *J =* 12.3 Hz, 3H), 2.60 (d, *J =* 16.9 Hz, 1H), 2.43 (qd, *J =* 13.2, 4.5 Hz, 1H), 2.24 (t, *J =* 13.4 Hz, 2H), 2.11 - 2.04 (m, 1H), 1.97 (t, *J* = 13.7 Hz, 3H), 1.85 (d, *J =* 11.7 Hz, 2H), 1.76 - 1.67 (m, 4H), 1.32 (dd, *J =* 20.9, 11.4 Hz, 2H), 1.24 (s, 1H).LC-MS: [M+H]⁺ = 819.56

### Example 433

### Synthesis of compound 400

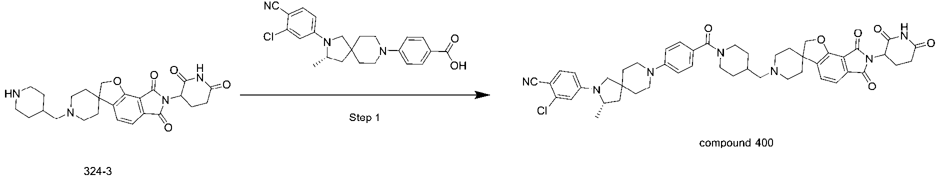

### Step 1: (Compound 400-2) was prepared with reference to step 1 of Compound 267. LC-MS: [M+H]⁺ = 567.46

### Step 2: (Compound 400-3) was prepared with reference to step 2 of Compound 267. LC-MS: [M+H]⁺ = 467.39

Step 3: (Compound 400) was prepare with reference to step 3 of Compound 267.
¹ H NMR (600 MHz, DMSO-*d*₆) δ 11.13 (s, 1H), 7.61 (d, *J =* 8.9 Hz, 1H), 7.57 (d, *J =* 7.3 Hz, 1H), 7.50 (d, *J =* 7.3 Hz, 1H), 7.27 (d, *J =* 8.3 Hz, 2H), 6.98 (d, *J =* 7.5 Hz, 2H), 6.81 (d, *J=* 2.1 Hz, 1H), 6.67 (dd, *J* = 8.9, 2.1 Hz, 1H), 5.11 (dd, *J =* 12.9, 5.4 Hz, 1H), 4.81 (s, 2H), 4.05 (dd, *J =* 13.2, 6.5 Hz, 2H), 3.45 (d, *J =* 10.8 Hz, 2H), 3.40 - 3.29 (m, 5H), 3.23 (dd, *J =* 18.9, 8.8 Hz, 3H), 3.14 - 3.03 (m, 4H), 2.94 - 2.85 (m, 2H), 2.60 (d, *J =* 17.2 Hz, 1H), 2.25 (dd, *J =* 12.7, 7.8 Hz, 3H), 2.15 (s, 1H), 2.02 (d, *J =* 11.4 Hz, 3H), 1.82 - 1.70 (m, 4H), 1.59 (dd, *J =* 12.8, 6.5 Hz, 1H), 1.55 - 1.46 (m, 3H), 1.24 (s, 1H), 1.21 (d, *J =* 6.0 Hz, 3H), 1.18 (s, 1H).LC-MS: [M+H]⁺ = 858.56

### Example 434

### Synthesis of compound 401

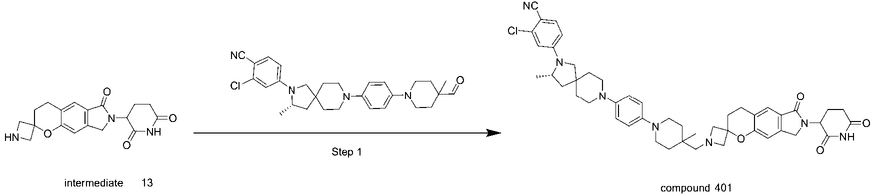

### Step 1: (Compound 401) was prepared with reference to step 1 of Compound 267. LC-MS: [M+H]⁺ = 816.66

¹H NMR (400 MHz, *DMSO*-*d*₆) δ 10.99 (s, 1H), δ 7.60 (d, *J =* 8.8 Hz, 1H), 7.52 (s, 1H), 7.34 (s, 2H), 7.12 (d, *J =* 25.1 Hz, 3H), 6.79 (d, *J =* 2.0 Hz, 1H), 6.67 (dd, *J =* 9.0, 2.0 Hz, 1H), 5.03 (dd, *J =* 13.3, 5.1 Hz, 1H), 4.51 (d, *J =* 34.5 Hz, 2H), 4.36 (d, *J =* 17.1 Hz, 3H), 4.23 (d, *J =* 17.2 Hz, 1H), 4.04 (dd, *J =* 13.0, 6.5 Hz, 1H), 3.38 (d *J* = 10.3 Hz, 8H), 3.14 (d, *J =* 12.5 Hz, 2H), 2.95 - 2.80 (m, 3H), 2.62 (dd, *J =* 18.8, 16.1 Hz, 1H), 2.41 - 2.31 (m, 2H), 2.27 *(d, J =* 22.7 Hz, 3H), 2.03 - 1.87 (m, 3H), 1.75 - 1.58 (m, 5H), 1.56 (s, 2H), 1.20 (d, *J =* 6.0 Hz, 4H), 1.09 (s, 3H).

### Example 435

### Synthesis of compound 402

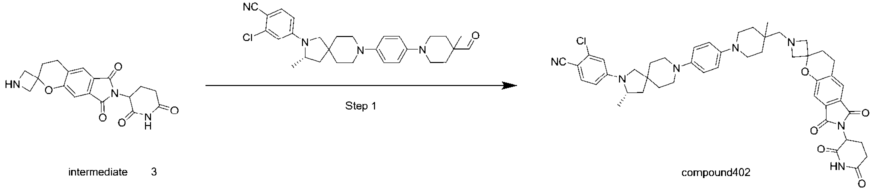

Step 1: (Compound 402) was prepared with reference to step 1 of Compound 267.
¹H NMR (400 MHz, DMSO-*d*₆) δ 11.13 (s, 1H), δ 7.76 (s, 1H), 7.61 (d, *J =* 8.8 Hz, 1H), 7.38 (s, 2H), 7.32 (s, 1H), 7.17 (s, 2H), 6.80 (d, *J =* 1.9 Hz, 1H), 6.67 (dd, *J =* 9.0, 2.0 Hz, 1H), 5.09 (dd, *J =* 12.8, 5.5 Hz, 1H), 4.46 *(d, J=* 41.7 Hz, 4H), 4.05 (dd, *J =* 13.2, 6.5 Hz, 1H), 3.42 (dd, *J=* 19.9, 8.2 Hz, 8H), 3.15 (s, 2H), 2.94 (d, *J =* 16.3 Hz, 2H), 2.94 - 2.78 (m, 2H), 2.68 - 2.54 (m, 1H), 2.30 (d, *J =* 25.9 Hz, 3H), 2.02 (dd, *J =* 18.6, 11.3 Hz, 2H), 1.94 (s, 2H), 1.65 (dd, *J =* 12.6, 6.3 Hz, 5H), 1.56 (s, 2H), 1.21 (d, *J =* 6.0 Hz, 4H), 1.09 (s, 3H). LC-MS: [M+H]⁺ = 830.66

### Example 436

### Synthesis of compound 403

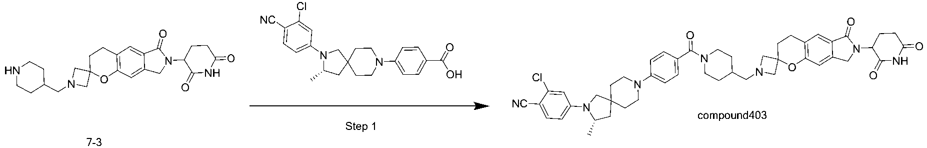

Step 3: (Compound 403) was prepare with reference to step 3 of Compound 267.
¹H NMR (600 MHz, DMSO-*d*₆) δ 10.98 (s, 1H), 7.61 (d, *J* = 8.8 Hz, 1H), 7.53 (s, 1H), 7.26 (d, *J =* 7.6 Hz, 2H), 7.07 (d, *J =* 14.4 Hz, 1H), 6.97 (d, *J =* 8.6 Hz, 2H), 6.80 (d, *J =* 2.2 Hz, 1H), 6.66 (dd, *J* = 8.9, 2.2 Hz, 1H), 5.07 (dd, *J =* 13.3, 5.0 Hz, 1H), 4.47 (d, *J =* 6.3 Hz, 1H), 4.41 - 4.31 (m, 4H), 4.29 (s, 1H), 4.24 (dd, *J=* 17.1, 4.2 Hz, 1H), 4.04 (dd, *J =* 13.4, 6.6 Hz, 2H), 3.44 (d, *J =* 10.8 Hz, 1H), 3.40 - 3.31 (m, 4H), 3.29 (s, 1H), 3.26 - 3.18 (m, 3H), 2.97 - 2.83 (m, 5H), 2.60 (d, *J =* 17.0 Hz, 1H), 2.41 - 2.32 (m, 1H), 2.27 - 2.18 (m, 3H), 1.98 (dd, *J =* 7.2, 5.2 Hz, 1H), 1.89 (d, *J =* 24.4 Hz, 1H), 1.73 (ddd, *J=* 17.1, 14.3, 9.2 Hz, 4H), 1.58 (dd, *J=* 12.8, 6.5 Hz, 1H), 1.52 - 1.48 (m, 2H), 1.21 (d, *J =* 6.1 Hz, 3H), 1.17 (d, *J =* 12.5 Hz, 1H).LC-MS: [M+H]⁺ = 830.56

### Example 437

### Synthesis of compound 404

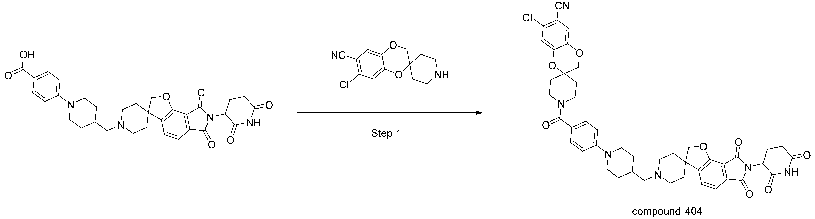

Step 1: (Compound 404) was prepared with reference to step 3 of Compound 267.
¹H NMR (600 MHz, DMSO-*d*₆) δ 11.13 (s, 1H), 7.63 (s, 1H), 7.57 (d, *J* = 7.3 Hz, 1H), 7.51 (d, *J =* 7.3 Hz, 1H), 7.35 - 7.29 (m, 3H), 6.99 (d, *J* = 8.6 Hz, 2H), 5.12 (dd, *J =* 12.9, 5.5 Hz, 1H), 4.82 (s, 2H), 4.15 (s, 3H), 3.62 (d, *J =* 12.4 Hz, 3H), 3.47 - 3.21 (m, 4H), 3.17 - 3.02 (m, 4H), 2.89 (m, 1H), 2.84 - 2.73 (m, 2H), 2.60 (m, 1H), 2.22 (m, 2H), 2.12 - 1.93 (m, 5H), 1.90 - 1.80 (m, 2H), 1.80 - 1.65 (m, 4H), 1.32 (qd, *J =* 12.4, 4.0 Hz, 2H). LC-MS: [M+H]⁺ = 833.66

### Example 438

### Synthesis of compound 405

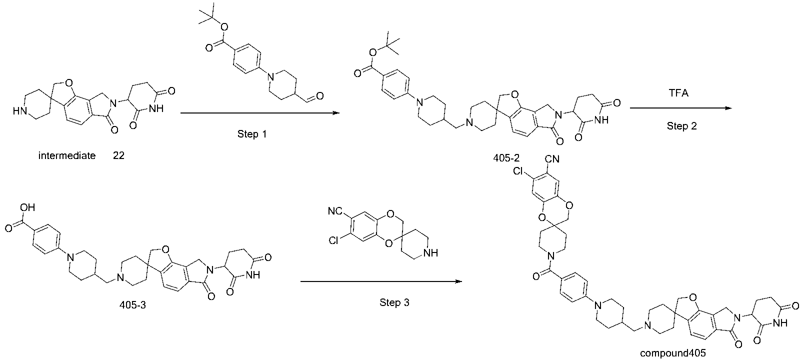

### Step 1: (Compound 405-2) was prepared with reference to step 3 of Compound 267. LC-MS: [M+H]⁺ = 629.58

### Step 2: (Compound 405-3) was prepared with reference to step 2 of Compound 267. LC-MS: [M+H]⁺ = 573.38

### Step 3: (Compound 405) was prepared with reference to step 3 of Compound 267. LC-MS: [M+H]⁺ = 819.46

¹H NMR (600 MHz, DMSO-*d*₆) δ 11.01 (s, 1H), 7.63 (s, 1H), 7.37 (d, *J =* 7.6 Hz, 1H), 7.34 (s, 1H), 7.31 (d, *J=* 8.4 Hz, 2H), 7.29 (d, *J =* 7.6 Hz, 1H), 6.99 (d, *J =* 8.5 Hz, 2H), 5.11 (dd, *J=* 13.3, 5.1 Hz, 1H), 4.75 - 4.63 (m, 2H), 4.40 (d, *J* = 17.1 Hz, 1H), 4.24 (d, *J =* 17.1 Hz, 1H), 4.15 (s, 2H), 3.90 - 3.81 (m, 4H), 3.60 (t, *J =* 8.6 Hz, 2H), 3.43 - 3.26 (m, 3H), 3.08 (m, 3H), 2.92 (ddd, *J =* 17.9, 13.3, 5.4 Hz, 1H), 2.80 (t, *J =* 12.0 Hz, 2H), 2.64 - 2.56 (m, 1H), 2.42 (m, 1H), 2.24 (t, *J =* 13.9 Hz, 2H), 2.07 (m, 1H), 1.97 (m, 3H), 1.85 (d, *J =* 12.8 Hz, 2H), 1.81 - 1.64 (m, 4H), 1.32 (qd, J = 12.4, 4.1 Hz, 2H).

### Example 439

### Synthesis of compound 406

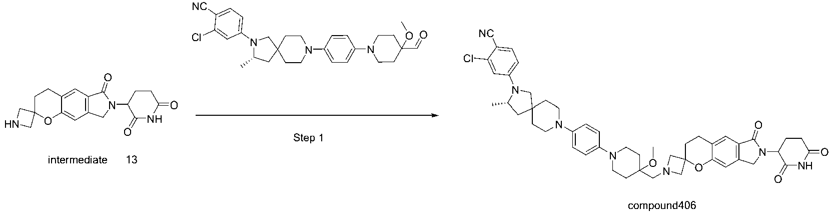

Step 1: (Compound 406) was prepared with reference to step 1 of Compound 267.
¹H NMR (600 MHz, DMSO-*d*₆) δ 10.98 (s, 1H), 7.64 (d, *J =* 8.8 Hz, 1H), 7.53 (s, 3H), 7.20 - 7.01 (m, 3H), 6.82 (d, *J* = 2.3 Hz, 1H), 6.69 (dd, *J =* 9.0, 2.3 Hz, 1H), 5.07 (dd, *J =* 13.3, 5.1 Hz, 1H), 4.57 (s, 1H), 4.45 (d, *J* = 8.5 Hz, 1H), 4.36 (dt, *J =* 19.3, 9.3 Hz, 3H), 4.24 (dd, *J =* 17.3, 4.9 Hz, 1H), 4.07 (q, *J =* 6.6 Hz, 1H), 3.45 (s, 4H), 3.43 - 3.30 (m, 5H), 3.22 (s, 3H), 3.01 (s, 2H), 2.96 - 2.84 (m, 3H), 2.65 - 2.55 (m, 1H), 2.42 - 2.16 (m, 4H), 2.05 - 1.79 (m, 5H), 1.66 (d, *J =* 15.1 Hz, 5H), 1.22 (d, *J =* 6.0 Hz, 4H).LC-MS: [M+H]⁺ = 832.66

### Example 440

### Synthesis of compound 408

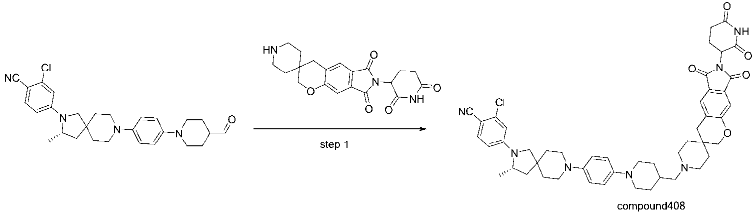

Step 1: (Compound 408) was prepared with reference to step 1 of Compound 267.
¹H NMR (600 MHz, DMSO-d6)δ 11.13 (s, 1H), 9.04 (s, 1H), 7.80 - 7.59 (m, 2H), 7.45 - 6.97 (m, 4H), 6.92 - 6.60 (m, 2H), 5.11 (dd, *J =* 12.9, 5.5 Hz, 1H), 4.33 (s, 1H), 4.06 (d, *J =* 9.7 Hz, 2H), 3.08 (s, 7H), 2.85 (d, *J =* 52.0 Hz, 4H), 2.67-20.56 (m, 3H),2.20 (dd, *J =* 18.0, 10.8 Hz, 3H), 2.02 (m, 3H), 1.87 (s, 3H), 1.75 (m, 4H), 1.63 (d, *J =* 14.0 Hz, 3H), 1.53 - 1.39 (m, 2H), 1.34 - 1.14 (m, 8H). LC-MS: [M+H]⁺ =844.56

### Example 441

### Synthesis of compound 409

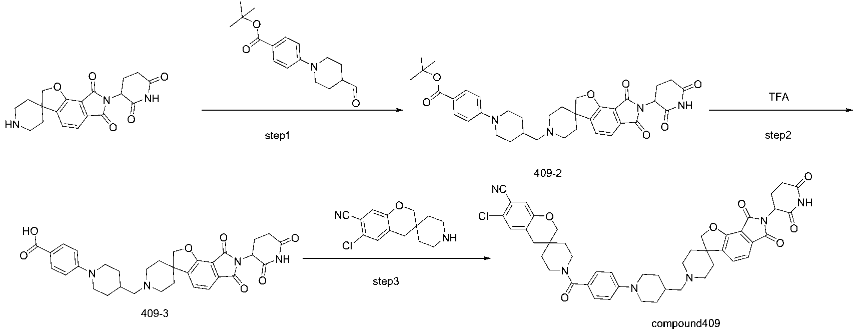

### Step 1: (Compound 409-2) was purified with reference to step 1 of Example 267. LC-MS: [M+H]+ = 643.56

### Step 2: (Compound 409-3) was purified with reference to step 2 of Example 267. LC-MS: [M+H]+ = 587.45

Step 3: (Compound 409) was prepared with reference to step 3 of Example 267.
¹H NMR (600 MHz, DMSO-d6) δ 11.12 (s, 1H), 7.74 (d, *J =* 7.4 Hz, 1H), 7.46 *(d, J=* 16.1 Hz, 2H), 7.41 (d, *J* = 7.3 Hz, 1H), 7.27 (d, *J =* 8.5 Hz, 2H), 6.94 (d, *J=* 8.5 Hz, 2H), 5.10 (dd, *J=* 12.9, 5.4 Hz, 1H), 4.69 (s, 2H), 4.04 (s, 2H), 3.80 (d, *J* = 12.2 Hz, 2H), 3.48 (d, *J =* 36.3 Hz, 2H), 2.89 - 2.82 (m, 4H), 2.73 (d, *J* = 3.2 Hz, 3H), 2.20 (d, *J =* 7.1 Hz, 2H), 2.05 - 1.88 (m, 6H), 1.85 - 1.68 (m, 5H), 1.41 - 1.33 (m, 3H), 1.25 (d, *J =* 9.3 Hz, 6H). LC-MS: [M+H]⁺ =831.50

### Example 442

### Synthesis of compound 410

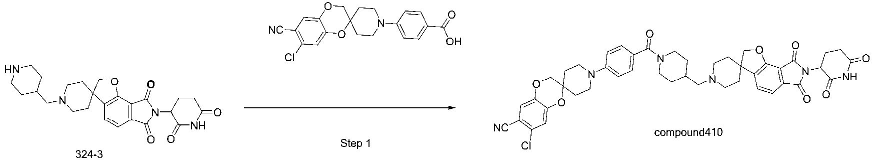

Step 3: (Compound 410) was prepared with reference to step 3 of Example 267.
¹H NMR (600 MHz, DMSO-d6) δ 11.13 (s, 1H), 7.64 (s, 1H), 7.57 (d, *J* = 7.3 Hz, 1H), 7.51 (d, *J* = 7.3 Hz, 1H), 7.35 (s, 1H), 7.31 - 7.27 (m, 2H), 7.02 (dd, *J* = 8.9, 2.6 Hz, 2H), 5.12 (dd, *J* = 12.9, 5.4 Hz, 1H), 4.81 (s, 2H), 4.19 (s, 2H), 3.62 (m, 4H), 3.35 (d, *J =* 35.9 Hz, 1H), 3.22 (ddd, J = 13.4, 10.3, 3.3 Hz, 2H), 3.14 - 3.04 (m, 4H), 2.89 (ddd, J = 17.2, 13.9, 5.5 Hz, 3H), 2.63 - 2.57 (m, 1H), 2.22 (t, *J* = 13.7 Hz, 2H), 2.18 - 2.08 (m, 1H), 2.06 - 1.97 (m, 3H), 1.87 - 1.74 (m, 7H), 1.26 - 1.17 (m, 3H).LC-MS: [M+H]⁺ =833.66

### Example 443

### Synthesis of compound 411

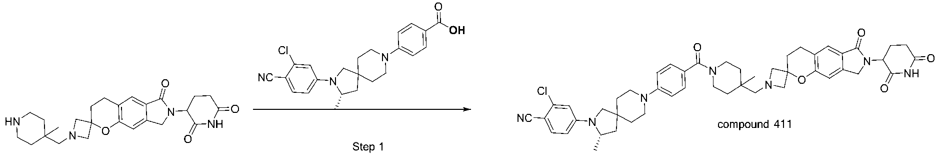

Step 3: (Compound 411) was prepared with reference to step 3 of Example 267.
¹H NMR (600 MHz, DMSO-d6) δ 10.98 (s, 1H), 7.61 (d, *J =* 8.9 Hz, 1H), 7.53 (s, 1H), 7.26 (d, *J =* 8.3 Hz, 2H), 7.07 (d, *J =* 19.5 Hz, 1H), 6.96 (d, *J =* 8.3 Hz, 2H), 6.80 (d, *J =* 2.2 Hz, 1H), 6.66 (dd, *J* = 9.0, 2.4 Hz, 1H), 5.07 (dd, *J =* 13.3, 5.2 Hz, 1H), 4.54 (ddd, *J =* 54.9, 12.2, 6.4 Hz, 2H), 4.39 (dd, *J =* 12.6, 5.3 Hz, 1H), 4.34 (t, *J =* 7.5 Hz, 2H), 4.24 (dd, *J =* 17.0, 4.4 Hz, 1H), 4.04 (h, *J =* 6.4 Hz, 2H), 3.44 (d, *J =* 10.8 Hz, 1H), 3.40 - 3.35 (m, 3H), 3.32 (dd, *J* = 8.8, 5.4 Hz, 3H), 3.28 - 3.17 (m, 4H), 2.92 (dt, *J =* 9.8, 5.3 Hz, 2H), 2.88 (t, *J =* 7.0 Hz, 1H), 2.60 (d, *J =* 14.1 Hz, 1H), 2.35 (td, J = 13.2, 4.7 Hz, 1H), 2.23 (m, 3H), 2.02 - 1.94 (m, 1H), 1.78 - 1.67 (m, 2H), 1.58 (dd, *J =* 12.8, 6.5 Hz, 1H), 1.50 (m, 4H), 1.35 (d, *J* = 17.1 Hz, 2H), 1.21 *(d, J=* 6.1 Hz, 3H), 1.10 (d, *J =* 7.1 Hz, 3H).LC-MS: [M+H]⁺ =844.76

### Example 444

### Synthesis of compound 412

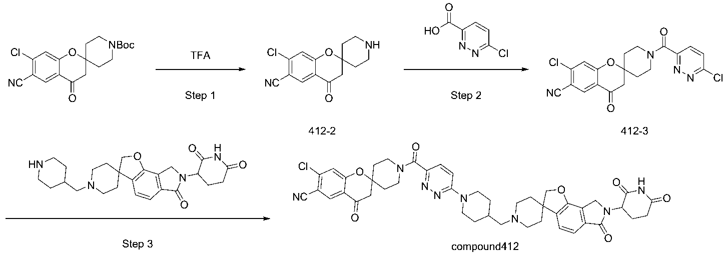

### Step 1: (Compound 413-2) was prepared with reference to step 2 of Example 267. LC-MS: [M+H]⁺ =277.27

### Step 2: (Compound 413-3) LC-MS: [M+H]⁺ =417.29

Compound 413-2 (220.0 mg, 795.03 µmol) and 6-chloropyridine-3-carboxylic acid (126.04 mg, 795.03 µmol) were added sequentially into a glass vial, and the solvent N,N-dimethylformamide (4.0 mL) was added, followed by addition of 1-(3-dimethylaminopropyl)-3-ethylcarbodiimide hydrochloride (228.61 mg, 1.19 mmol) and 1-hydroxybenzotriazole (161.14 mg, 1.19 mmol), and the reaction solution was stirred at room temperature for 2.0 h. After the reaction was completed, a light yellow solid compound 413-2 (75.2 mg, 22.67%) was obtained by Flash purification.

### Step 3: (Compound 413) was prepared with reference to step 3 of Example 19. LC-MS: [M+H]⁺ =833.56

¹H NMR (600 MHz, DMSO-*d*₆) δ 11.01 (s, 1H), 8.11 (d, *J =* 8.9 Hz, 1H), 7.98 (t, *J =* 4.4 Hz, 2H), 7.37 (d, *J* = 7.6 Hz, 1H), 7.29 (d, *J =* 7.6 Hz, 1H), 6.73 (s, 1H), 5.11 (dd, *J =* 13.3, 5.0 Hz, 1H), 4.68 (q, *J =* 9.4 Hz, 2H), 4.40 (d, *J =* 17.2 Hz, 1H), 4.31 (d, *J* = 12.9 Hz, 1H), 4.24 (d, *J =* 17.1 Hz, 1H), 3.89 - 3.77 (m, 2H), 3.66 - 3.54 (m, 4H), 3.38 - 3.29 (m, 2H), 3.18 - 3.04 (m, 3H), 3.01 - 2.81 (m, 5H), 2.60 (d, *J =* 17.3 Hz, 1H), 2.48 - 2.36 (m, 2H), 2.24 (t, *J =* 13.6 Hz, 2H), 2.17 - 2.07 (m, 1H), 2.04 (d, *J =* 12.7 Hz, 1H), 2.02 - 1.86 (m, 5H), 1.86 - 1.76 (m, 2H), 1.48 - 1.35 (m, 2H).

### Example 445

### Synthesis of compound 413

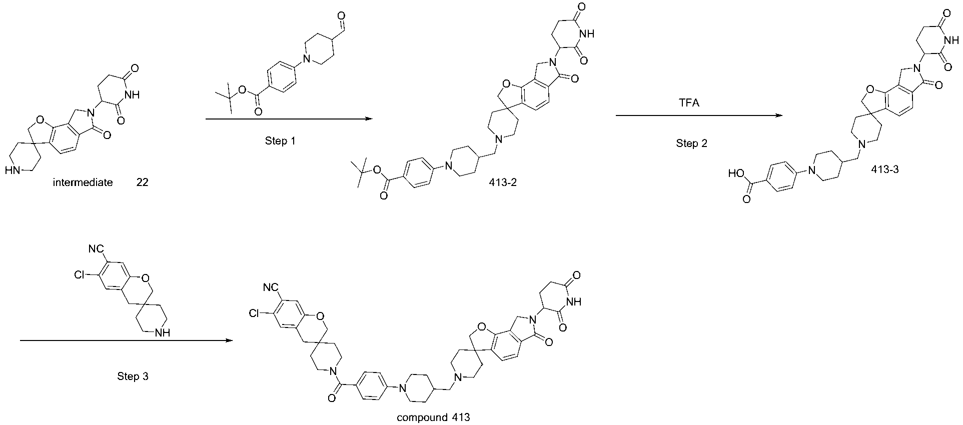

### Step 1: (Compound 413-2) LC-MS: [M+H]⁺ =629.58

The intermediate 22 (50.0 mg, 140.69 umol) and tert-butyl 4-(4-formylpiperidin-1-yl)benzoate (81.42 mg, 281.38 umol) were added sequentially to a glass vial, and the solvent THF (2 mL), Ti(O-iPr)₄(79.97 mg, 281.38 umol) were added and the reaction solution was stirred at 60 °C for 1 h. Then NaBH₃CN (26.52 mg, 422.07 umol) was added and the reaction solution was continued to be stirred at 60 °C for 1 h. After the reaction was completed, the reaction solution was concentrated and then purified by preparative thin-layer chromatography (DCM:MeOH=10:1) to obtain ayellow solid compound 413-2 (50 mg, 56.52%).

### Step 2: (Compound 413-3) was prepared with reference to step 2 of Example 267. LC-MS: [M+H]⁺ =573.48

Step 3: (Compound 413) was prepared with reference to step 3 of Example 267.
¹H NMR (400 MHz, DMSO-d6) δ 11.00 (s, 1H), 7.46 (d, *J =* 11.7 Hz, 2H), 7.40 - 7.34 (m, 1H), 7.29 (d, *J =* 8.1 Hz, 3H), 6.98 (d, *J =* 8.7 Hz, 2H), 5.10 (dd, *J =* 13.3, 5.1 Hz, 1H), 4.72 - 4.62 (m, 2H), 4.40 (d, *J=* 17.2 Hz, 1H), 4.24 (d, *J =* 17.2 Hz, 1H), 4.04 (s, 2H), 3.53 - 3.30 (m, 5H), 3.09 (q, *J =* 5.7 Hz, 4H), 2.97 - 2.88 (m, 1H), 2.86 - 2.75 (m, 4H), 2.65 - 2.56 (m, 1H), 2.42 (m, 1H), 2.37 - 1.89 (m, 8H), 1.85 (d, *J =* 12.2 Hz, 2H), 1.37 (m, 7H). LC-MS: [M+H]⁺=817.56

### Test Example 1

### The degradation activity of the compounds disclosed in the present disclosure on AR protein in LNCaP cells (cell source: ATCC)

AR positive human prostate cancer cells LNCaP FGC in RPMI 1640 (ATCC catalog No.30-2001) containing 10% FBS (Hyclone catalog No. SH30406.05) were inoculated in six-well microplates (Thermo catalog No.140675) at 1.5×10⁵/well. After culturing the cells in CO₂ incubator (ESCO) overnight, different concentrations of the compound of the present disclosure and the positive control sample ARV-110 (source: Energy chemical) solution were added at a volume of 1 µL/well, to reach final concentrations of 100nM and 10nM. Corresponding solvent controls were also set and cultured for 8 hours. After 8 hours of cultivation, the culture medium was removed and the cells were washed with PBS. RIPA lysis buffer containing 1% Protease Inhibitor Cocktail was added, and the total protein extract was obtained by lysis and centrifugation. The protein concentration in the extract was detected by BCA method. Protein electrophoresis was performed using SDS-PAGE, followed by transfer of the protein onto a PVDF (Millipore Sigma catalog No.PSRP010R5) membrane at 100V constant voltage for 60 minutes. The PVDF membrane was then placed in a 1 × TBST solution with 5% milk and blocked at room temperature for 1 hour. A primary antibody solution (Anti Antigen receptor, Cell Signaling Technology catalog No.5153S; 1:2000 dilution) was prepared and incubated overnight at 4°C. The primary antibody solution was discarded and the PVDF membrane was washed three times with 1 × TBST for 5 minutes/time. A secondary antibody solution (Thermo catalog No.SA5-35571;1: 7500 dilution) was prepared, and the secondary antibody dilution was incubated at room temperature for 1 hour. The secondary antibody solution was discarded and the PVDF membrane was washed three times with 1 × TBST for 5 minutes/time. Western blot imaging was performed by using a dual color infrared laser imaging system (Licor catalog No. Odyssey CLX). Bands were analyzed in grayscale using Image Studio software. For each sample, GAPDH protein bands were detected as internal reference. The degradation rates of the AR protein for the compounds and the positive control sample were calculated based on grayscale of the protein bands.

The degradation rates of the AR protein in LNCaP cells by the compounds disclosed in the present disclosure are shown in Table 3.

**Table 3 Degradation rates of the AR protein in LNCaP cells by the compounds disclosed in the present disclosure**

| Compound | 100 nM(%) | 10 nM(%) | Compound | 100 nM(%) | 10 nM(%) |
|---|---|---|---|---|---|
| ARV-110 | 62% | 11% | Compound 207 | 40% | 28% |
| Compound 1 | 82% | 44% | Compound 208 | 38% | 34% |
| Compound 2 | 50% | 66% | Compound 209 | 25% | 49% |
| Compound 5 | 35% | 51% | Compound 210 | 59% | 61% |
| Compound 6 | 46% | 58% | Compound 211 | 28% | 45% |
| Compound 7 | 79% | 86% | Compound 212 | 70% | 79% |
| Compound 8 | 67% | 78% | Compound 213 | 67% | 47% |
| Compound 9 | 66% | 56% | Compound 214 | 68% | 40% |
| Compound 16 | 79% | 64% | Compound 215 | 91% | 86% |
| Compound 17 | 65% | 35%% | Compound 217 | 48% | -2% |
| Compound 18 | 57% | 7% | Compound 218 | 17% | 28% |
| Compound 20 | 48% | 13% | Compound 219 | 21% | -40% |
| Compound 22 | 84% | 71% | Compound 220 | 63% | 32% |
| Compound 24 | 50% | 40% | Compound 221 | 76% | 24% |
| Compound 25 | 43% | 49% | Compound 222 | 75% | 4% |
| Compound 26 | 56% | 61% | Compound 223 | 20% | 27% |
| Compound 27 | 39% | 14% | Compound 224 | 52% | 41% |
| Compound 28 | 60% | 58% | Compound 225 | 7% | -35% |
| Compound 29 | 63% | 35% | Compound 226 | 34% | 52% |
| Compound 30 | 62% | 28% | Compound 227 | 62% | 45% |
| Compound 31 | 69% | 52% | Compound 228 | 56% | 30% |
| Compound 32 | 53% | 22% | Compound 229 | 59% | 49% |
| Compound 33 | 61% | 27% | Compound 230 | 51% | 4% |
| Compound 34 | 70% | 57% | Compound 231 | 28% | 31% |
| Compound 35 | 50% | 17% | Compound 232 | 36% | 33% |
| Compound 36 | 22% | 18% | Compound 233 | 62% | 49% |
| Compound 37 | 29% | 5% | Compound 234 | 81% | 58% |
| Compound 38 | 48% | 29% | Compound 235 | 36% | 39% |
| Compound 42 | 49% | 29% | Compound 236 | 60% | 44% |
| Compound 44 | 84% | 85% | Compound 237 | 39% | 6% |
| Compound 46 | 47% | 35% | Compound 238 | 61% | 31% |
| Compound 47 | 67% | 54% | Compound 239 | 69% | 65% |
| Compound 48 | 91% | 73% | Compound 240 | 30% | 18% |
| Compound 49 | 36% | 24% | Compound 241 | 63% | 50% |
| Compound 50 | 86% | 66% | Compound 242 | 11% | -18% |
| Compound 51 | 49% | 13% | Compound 243 | 58% | 24% |
| Compound 52 | 22% | 43% | Compound 244 | 65% | -5% |
| Compound 53 | 85% | 69% | Compound 245 | 45% | 2% |
| Compound 54 | 76% | 24% | Compound 246 | 43% | 17% |
| Compound 55 | 69% | 44% | Compound 247 | 27% | -10% |
| Compound 56 | 77% | 44% | Compound 248 | -16% | -53% |
| Compound 57 | 72% | 45% | Compound 249 | 51% | -5% |
| Compound 58 | 58% | 33% | Compound 250 | 67% | -14% |
| Compound 59 | 66% | 43% | Compound 251 | 37% | 45% |
| Compound 60 | 64% | 41% | Compound 252 | 70% | 56% |
| Compound 61 | 82% | 65% | Compound 253 | 41% | 37% |
| Compound 62 | 76% | 46% | Compound 254 | 45% | 53% |
| Compound 63 | 65% | 0% | Compound 255 | 44% | 55% |
| Compound 64 | 72% | 44% | Compound 256 | 36% | 47% |
| Compound 65 | 75% | 36% | Compound 257 | -41% | 36% |
| Compound 66 | 74% | 72% | Compound 258 | 67% | 71% |
| Compound 67 | 60% | 37% | Compound 259 | 44% | -16% |
| Compound 69 | 64% | 26% | Compound 260 | 9% | 13% |
| Compound 70 | 70% | 61% | Compound 265 | 61% | 66% |
| Compound 71 | 71% | 37% | Compound 266 | 73% | 49% |
| Compound 72 | 92% | 81% | Compound 267 | 66% | 30% |
| Compound 73 | 74% | 49% | Compound 268 | 31% | 15% |
| Compound 74 | 79% | 39% | Compound 269 | 61% | 35% |
| Compound 75 | 36% | 41% | Compound 270 | 40% | 47% |
| Compound 76 | 74% | 74% | Compound 271 | 10% | -3% |
| Compound 78 | 70% | 67% | Compound 272 | 52% | 45% |
| Compound 79 | 28% | 23% | Compound 273 | 26% | 2% |
| Compound 137 | 21% | 68% | Compound 274 | -5% | -3% |
| Compound 138 | -1% | -9% | Compound 275 | 54% | 30% |
| Compound 139 | 11% | 42% | Compound 276 | 67% | 55% |
| Compound 142 | 52% | 29% | Compound 277 | 60% | 16% |
| Compound 144 | 39% | 68% | Compound 278 | 34% | -3% |
| Compound 147 | 14% | -3% | Compound 279 | 69% | 40% |
| Compound 148 | 36% | 38% | Compound 280 | 52% | 36% |
| Compound 149 | 1% | 1% | Compound 281 | 56% | 20% |
| Compound 150 | 35% | 52% | Compound 282 | 44% | 17% |
| Compound 152 | 24% | 45% | Compound 283 | 66% | 53% |
| Compound 153 | 19% | 23% | Compound 284 | 51% | 18% |
| Compound 154 | 48% | 46% | Compound 285 | 54% | 40% |
| Compound 155 | 1% | 29% | Compound 286 | 34% | 22% |
| Compound 156 | 53% | 52% | Compound 287 | 51% | 28% |
| Compound 157 | 52% | 51% | Compound 288 | 18% | -18% |
| Compound 158 | 37% | 43% | Compound 289 | 63% | 27% |
| Compound 160 | 76% | 67% | Compound 290 | 18% | -14% |
| Compound 164 | 4% | 25% | Compound 291 | 57% | 9% |
| Compound 165 | 25% | 28% | Compound 294 | 57% | 54% |
| Compound 166 | 19% | 36% | Compound 295 | 44% | 1% |
| Compound 167 | 18% | 45% | Compound 296 | 63% | 53% |
| Compound 168 | 26% | 70% | Compound 297 | 40% | 42% |
| Compound 169 | 53% | 75% | Compound 298 | 55% | 27% |
| Compound 171 | 44% | 22% | Compound 190 | 75% | 53% |
| Compound 172 | 33% | 12% | Compound 191 | 4% | -2% |
| Compound 173 | 72% | 75% | Compound 192 | 35% | 37% |
| Compound 176 | 61% | 33% | Compound 193 | 31% | 18% |
| Compound 177 | 53% | 37% | Compound 198 | 54% | 44% |
| Compound 180 | 53% | 12% | Compound 200 | 27% | 44% |
| Compound 183 | 51% | 40% | Compound 201 | 32% | 6% |
| Compound 184 | 41% | 79% | Compound 202 | 40% | 41% |
| Compound 188 | 66% | 68% | Compound 203 | 43% | 37% |
| Compound 189 | 57% | 42% | | | |

Conclusion: The compounds disclosed in the present disclosure are capable of effectively degrading AR protein, and the capabilities of some compounds to degrade AR protein are comparable or even better than that of the positive control sample.

### Test Example 2

### The degradation activity of the compounds disclosed in the present disclosure on AR protein in VCaP cells (cell source: ATCC)

AR positive human prostate cancer cells VCaP in DMEM (GiBco catalog No.11995-065) containing 10% FBS (Hyclone catalog No. SH30406.05) were inoculated in six-well microplates (Thermo catalog No.140675) at 3×10⁵/well. After culturing the cells in CO₂ incubator (ESCO) overnight, different concentrations of the compound of the present disclosure and the positive control sample ARV-110 (source: Energy chemical) solution were added at a volume of 1 µL/well, to reach final concentrations of 5nM and 0.5nM. Corresponding solvent controls were also set and cultured for 8 hours. After 8 hours of cultivation, the culture medium was removed and the cells were washed with PBS. RIPA lysis buffer containing 1% Protease Inhibitor Cocktail was added, and the total protein extract was obtained by lysis and centrifugation. The protein concentration in the extract was detected by BCA method. Protein electrophoresis was performed using SDS-PAGE, followed by transfer of the protein onto a PVDF (Millipore Sigma catalog No.PSRP010R5) membrane at 100V constant voltage for 60 minutes. The PVDF membrane was then placed in a 1 × TBST solution with 5% milk and blocked at room temperature for 1 hour. A primary antibody solution (Anti-Antigen receptor, Cell Signaling Technology catalog No.5153S; 1:2000 dilution) was prepared and incubated overnight at 4°C. The primary antibody solution was discarded and the PVDF membrane was washed three times with 1 × TBST for 5 minutes/time. A secondary antibody solution (Thermo catalog No.SA5-35571; 1: 7500 dilution) was prepared, and the secondary antibody dilution was incubated at room temperature for 1 hour. The secondary antibody solution was discarded and the PVDF membrane was washed three times with 1 × TBST for 5 minutes/time. Western blot imaging was performed by using a dual color infrared laser imaging system (Licor catalog No. Odyssey CLX). Bands were analyzed in grayscale using Image Studio software. For each sample, GAPDH protein bands were detected as internal reference. The degradation rates of the AR protein for the compounds and the positive control sample were calculated based on grayscale of the protein bands.

The degradation rates of the AR protein in VCaP cells by the compounds disclosed in the present disclosure are shown in Table 4.

**Table 4 Degradation rates of the AR protein in VCaP cells by the compounds disclosed in the present disclosure**

| Compound | 100 nM(%) | 10 nM(%) | Compound | 100 nM(%) | 10 nM(%) |
|---|---|---|---|---|---|
| ARV-110 | 85% | 68% | Compound 207 | 37% | -46% |
| Compound 1 | 79% | 65% | Compound 208 | 67% | 17% |
| Compound 2 | 56% | 21% | Compound 209 | 67% | 39% |
| Compound 5 | 51% | 41% | Compound 210 | 71% | 28% |
| Compound 6 | 28% | 13% | Compound 211 | 46% | -4% |
| Compound 7 | 79% | 72% | Compound 212 | 82% | 60% |
| Compound 8 | 76% | 24% | Compound 213 | 75% | 32% |
| Compound 9 | 82% | 87% | Compound 214 | 63% | 7% |
| Compound 16 | 76% | 66% | Compound 215 | 82% | 63% |
| Compound 17 | 80% | 39% | Compound 216 | 80% | 54% |
| Compound 18 | 69% | 59% | Compound 217 | 83% | 49% |
| Compound 20 | 54% | 41% | Compound 218 | 74% | 21% |
| Compound 22 | 64% | 58% | Compound 219 | 70% | 11% |
| Compound 24 | 58% | 47% | Compound 220 | 78% | 22% |
| Compound 25 | 32% | 13% | Compound 221 | 64% | 16% |
| Compound 26 | 56% | 48% | Compound 222 | 61% | 8% |
| Compound 27 | 44% | 33% | Compound 223 | 52% | -3% |
| Compound 28 | 35% | 9% | Compound 224 | 57% | 24% |
| Compound 30 | 64% | 39% | Compound 225 | 30% | -29% |
| Compound 31 | 70% | 67% | Compound 226 | 83% | 74% |
| Compound 32 | 64% | 58% | Compound 227 | 60% | 24% |
| Compound 33 | 52% | 49% | Compound 228 | 18% | -9% |
| Compound 34 | 51% | 39% | Compound 229 | 73% | 33% |
| Compound 35 | 58% | 47% | Compound 230 | 52% | -25% |
| Compound 36 | 19% | 17% | Compound 231 | 63% | 11% |
| Compound 42 | 33% | 45% | Compound 232 | 43% | 37% |
| Compound 44 | 83% | 79% | Compound 233 | 87% | 74% |
| Compound 46 | 59% | 39% | Compound 234 | 72% | 35% |
| Compound 47 | 79% | 82% | Compound 235 | 76% | 35% |
| Compound 48 | 82% | 69% | Compound 236 | 82% | 37% |
| Compound 49 | 61% | 24% | Compound 237 | 23% | -46% |
| Compound 50 | 88% | 77% | Compound 238 | 79% | 54% |
| Compound 51 | 90% | 83% | Compound 239 | 54% | 11% |
| Compound 52 | 26% | 16% | Compound 240 | 58% | 12% |
| Compound 53 | 71% | 62% | Compound 241 | 77% | 29% |
| Compound 54 | 85% | 75% | Compound 242 | 15% | -5% |
| Compound 55 | 80% | 77% | Compound 243 | 68% | 16% |
| Compound 56 | 88% | 79% | Compound 244 | 56% | 3% |
| Compound 57 | 80% | 66% | Compound 245 | 43% | 3% |
| Compound 58 | 93% | 91% | Compound 246 | 61% | -19% |
| Compound 59 | 87% | 86% | Compound 247 | 70% | 53% |
| Compound 60 | 64% | 41% | Compound 248 | 17% | 2% |
| Compound 61 | 38% | 24% | Compound 249 | 56% | -21% |
| Compound 62 | 74% | 66% | Compound 250 | 61% | 15% |
| Compound 63 | 81% | 67% | Compound 251 | 73% | 48% |
| Compound 64 | 73% | 62% | Compound 252 | 87% | 80% |
| Compound 65 | 71% | 50% | Compound 253 | 55% | 10% |
| Compound 66 | 42% | 49% | Compound 254 | 85% | 56% |
| Compound 67 | 72% | 43% | Compound 255 | 82% | 63% |
| Compound 69 | 78% | 50% | Compound 256 | 81% | 48% |
| Compound 70 | 93% | 87% | Compound 257 | 75% | 67% |
| Compound 71 | 66% | 54% | Compound 258 | 84% | 60% |
| Compound 72 | 85% | 75% | Compound 259 | 78% | 33% |
| Compound 73 | 81% | 70% | Compound 260 | 74% | 15% |
| Compound 74 | 65% | 47% | Compound 265 | 78% | 34% |
| Compound 76 | 76% | 84% | Compound 266 | 74% | 21% |
| Compound 78 | 57% | 72% | Compound 267 | 69% | 30% |
| Compound 79 | 42% | 36% | Compound 268 | -32% | -26% |
| Compound 137 | 90% | 80% | Compound 269 | 63% | 7% |
| Compound 138 | 7% | -28% | Compound 270 | 28% | 9% |
| Compound 139 | -31% | -16% | Compound 271 | -5% | -47% |
| Compound 142 | 74% | 26% | Compound 272 | 75% | 38% |
| Compound 144 | 86% | 82% | Compound 273 | 7% | -20% |
| Compound 147 | 47% | 24% | Compound 274 | -13% | 4% |
| Compound 148 | 35% | 18% | Compound 275 | 51% | 7% |
| Compound 149 | 26% | -4% | Compound 276 | 59% | -11% |
| Compound 150 | 86% | 65% | Compound 277 | 54% | 7% |
| Compound 152 | 72% | 51% | Compound 278 | -16% | -30% |
| Compound 153 | 76% | 35% | Compound 279 | 79% | 34% |
| Compound 154 | 86% | 75% | Compound 280 | 77% | 15% |
| Compound *155* | 80% | 58% | Compound 281 | 60% | -11% |
| Compound 156 | 75% | 37% | Compound 282 | 51% | 8% |
| Compound 157 | 84% | 46% | Compound 283 | 45% | 2% |
| Compound 158 | 81% | 62% | Compound 284 | 73% | 9%% |
| Compound 164 | 8% | 4% | Compound 285 | 66 | -5% |
| Compound 165 | 23% | -43% | Compound 286 | -9% | -33% |
| Compound 166 | 60% | 43% | Compound 287 | 13% | -31% |
| Compound 167 | 67% | 33% | Compound 288 | 41% | 7% |
| Compound 168 | 77% | 76% | Compound 289 | 15% | -21% |
| Compound 169 | 82% | 79% | Compound 290 | 6% | -2% |
| Compound 171 | 62% | 46% | Compound 291 | 53% | 21% |
| Compound 172 | -14% | -9% | Compound 294 | 62% | 13% |
| Compound 173 | 86% | 75% | Compound 295 | 37% | -4% |
| Compound 177 | 38% | -14% | Compound 296 | 59% | 0% |
| Compound 180 | 73% | 29% | Compound 297 | 81% | 47% |
| Compound 183 | 84% | 75% | Compound 198 | 84% | 47% |
| Compound 188 | 79% | 63% | Compound 200 | 57% | 25% |
| Compound 191 | 68% | 31% | Compound 201 | 32% | -11% |
| Compound 192 | 86% | 66% | Compound 202 | 72% | 39% |
| Compound 193 | 78% | 21% | Compound 203 | 42% | 15% |

Conclusion: The compounds disclosed in the present disclosure are capable of effectively degrading AR protein, and the capabilities of some compounds to degrade AR protein are comparable or even better than that of the positive control sample.

### Test Example 3

### The inhibitory activity of the compounds disclosed in the present disclosure on the proliferation of LNCaP cells

LNCaP FGC cells (ATCC, CLR-1740) were cultured in RPMI 1640 (ATCC, 30-2001) complete medium containing 10% fetal bovine serum (Hyclone, SH30406.05). On the first day of the experiment, LNCaP FGC cells were inoculated in 96 well plates at a density of 5000 cells per well using RPMI 1640 medium containing 10% fetal bovine serum, with 100 µL of cell suspension per well. The cells were cultured in a cell culture incubator with 5% CO₂ at 37 °C overnight. On the second day, 100 µL of different concentrations of the compounds to be tested of the disclosure and the positive control ARV-110 (source: Energy chemical) prepared with culture medium were added to each well respectively. The final concentration of the compounds was 9 concentration points of 5-fold gradient dilution from 10 µ M, a well containing 100 µL of RPMI 1640 medium containing 10% fetal bovine serum was set as a control and cultured in the cell culture incubator with 5% CO₂ at 37 °C for 96 hours. On the sixth day, 96 well plates were taken out, and 20 µL of WST-8 (Beytime, C0040) reagent was added to each well. The absorbance at 450nm was detected using a microplate reader (TECAN, F50), and the 50% proliferation inhibition concentration (IC₅₀ value) was calculated by logistic regression using Graphpad Prism software based on the concentration and absorbance values of the compounds.

The IC₅₀ values of the compounds disclosed in the present disclosure for inhibiting the proliferation of LNCaP cells are shown in Table 5.

**Table 5 the IC₅₀ values of the compounds disclosed in the present disclosure for inhibiting the proliferation of LNCaP cells**

| Compound | IC₅₀ (µM) | Compound | IC₅₀ (µM) | Compound | IC₅₀ (µM) |
|---|---|---|---|---|---|
| ARV-110 | 0.32 | Compound 100 | 27.9 | Compound 225 | 0.030 |
| Compound 1 | 0.93 | Compound 104 | 5.39 | Compound 226 | 0.0025 |
| Compound 2 | 1.25 | Compound 108 | 10.39 | Compound 227 | 0.17 |
| Compound 5 | 0.71 | Compound 111 | 5.72 | Compound 228 | 2.29 |
| Compound 6 | 5.17 | Compound 112 | 8.18 | Compound 229 | 0.33 |
| Compound 7 | 1.57 | Compound 113 | 5.56 | Compound 230 | 0.29 |
| Compound 8 | 2.09 | Compound 114 | 7.63 | Compound 231 | 3.71 |
| Compound 12 | 0.73 | Compound 115 | 10.85 | Compound 233 | 34.28 |
| Compound 16 | 0.12 | Compound 116 | 10.75 | Compound 234 | 0.27 |
| Compound 17 | 0.80 | Compound 117 | 2.57 | Compound 235 | 0.84 |
| Compound 18 | 4.94 | Compound 119 | 4.11 | Compound 236 | 0.022 |
| Compound 20 | 1.00 | Compound 122 | 13.68 | Compound 237 | 0.051 |
| Compound 21 | 0.56 | Compound 123 | 2.71 | Compound 238 | 0.021 |
| Compound 22 | 0.60 | Compound 124 | 7.91 | Compound 239 | 13.54 |
| Compound 25 | 0.39 | Compound 125 | 6.97 | Compound 240 | 0.033 |
| Compound 26 | 1.20 | Compound 126 | 4.06 | Compound 241 | 68.63 |
| Compound 27 | 0.11 | Compound 127 | 6.28 | Compound 242 | 0.64 |
| Compound 28 | 3.90 | Compound 128 | 4.16 | Compound 243 | 0.12 |
| Compound 29 | 0.93 | Compound 129 | 3.63 | Compound 244 | 0.17 |
| Compound 30 | 1.90 | Compound 130 | 2.66 | Compound 245 | 1.82 |
| Compound 31 | 2.20 | Compound 131 | 4.55 | Compound 246 | 0.87 |
| Compound 32 | 3.60 | Compound 132 | 4.35 | Compound 247 | 0.015 |
| Compound 33 | 2.21 | Compound 133 | 23.7 | Compound 248 | 5.99 |
| Compound 34 | 1.12 | Compound 138 | 71.54 | Compound 249 | 12.11 |
| Compound 35 | 0.80 | Compound 139 | 52.28 | Compound 250 | >90 |
| Compound 36 | 3.20 | Compound 142 | >90 | Compound 251 | 0.15 |
| Compound 37 | 4.53 | Compound 143 | 0.35 | Compound 252 | 6.24 |
| Compound 38 | 2.40 | Compound 147 | 17.01 | Compound 253 | 0.55 |
| Compound 42 | 1.16 | Compound 148 | 8.66 | Compound 254 | 49.10 |
| Compound 44 | 1.14 | Compound 149 | 3.73 | Compound 255 | 50.23 |
| Compound 47 | 0.17 | Compound 150 | 3.13 | Compound 256 | 56.01 |
| Compound 48 | 1.67 | Compound 151 | 72.74 | Compound 257 | 0.014 |
| Compound 49 | 2.81 | Compound 152 | 28.29 | Compound 258 | 0.017 |
| Compound 50 | 1.20 | Compound 153 | 13.56 | Compound 259 | 0.073 |
| Compound 51 | 0.40 | Compound 154 | 39.45 | Compound 260 | 0.004 |
| Compound 52 | 4.50 | Compound 160 | 59.86 | Compound 265 | 44.36 |
| Compound 53 | 0.38 | Compound 161 | 0.44 | Compound 266 | 22.05 |
| Compound 54 | 1.70 | Compound 163 | 13.63 | Compound 267 | 43.20 |
| Compound 55 | 1.00 | Compound 164 | 90.48 | Compound 268 | 88.56 |
| Compound 56 | 0.35 | Compound 165 | 96.58 | Compound 269 | 11.84 |
| Compound 57 | 1.36 | Compound 166 | 71.85 | Compound 270 | 72 |
| Compound 58 | 1.80 | Compound 167 | 80.11 | Compound 271 | 37.89 |
| Compound 59 | 0.40 | Compound 171 | 13.51 | Compound 272 | 0.10 |
| Compound 60 | 2.40 | Compound 172 | 91.41 | Compound 273 | 96.37 |
| Compound 61 | 0.13 | Compound 173 | 12.29 | Compound 274 | 84.89 |
| Compound 62 | 0.82 | Compound 175 | 8.07 | Compound 275 | 0.24 |
| Compound 63 | 0.50 | Compound 186 | 0.85 | Compound 276 | 8.81 |
| Compound 64 | 0.80 | Compound 187 | 0.39 | Compound 277 | 10.73 |
| Compound 65 | 0.50 | Compound 188 | 3.72 | Compound 278 | 59.99 |
| Compound 66 | 0.42 | Compound 189 | 26.92 | Compound 279 | 25.44 |
| Compound 67 | 0.015 | Compound 190 | 5.15 | Compound 280 | 9.25 |
| Compound 69 | 0.39 | Compound 191 | 15.21 | Compound 281 | 2.14 |
| Compound 70 | 0.10 | Compound 192 | 2.59 | Compound 282 | 2.98 |
| Compound 71 | 0.50 | Compound 199 | 1.11 | Compound 283 | 97.56 |
| Compound 72 | 0.05 | Compound 200 | 90.99 | Compound 284 | 5.84 |
| Compound 73 | 0.30 | Compound 207 | 0.87 | Compound 285 | 0.84 |
| Compound 74 | 0.60 | Compound 208 | 0.01 | Compound 286 | 3.72 |
| Compound 75 | 6.05 | Compound 209 | 1.76 | Compound 287 | 70.46 |
| Compound 76 | 2.37 | Compound 210 | 0.008 | Compound 288 | 65.94 |
| Compound 78 | 3.00 | Compound 211 | 0.055 | Compound 289 | 66.04 |
| Compound 79 | 6.10 | Compound 212 | 0.32 | Compound 290 | 60.89 |
| Compound 80 | 0.73 | Compound 213 | 1.32 | Compound 291 | 2.88 |
| Compound 81 | 3.86 | Compound 214 | 3.78 | Compound 294 | 0.077 |
| Compound 82 | 3.39 | Compound 215 | 0.22 | Compound 295 | 0.37 |
| Compound 83 | 7.06 | Compound 216 | 0.006 | Compound 296 | 6.60 |
| Compound 85 | 5.34 | Compound 217 | 0.005 | Compound 297 | 61.41 |
| Compound 86 | 4.53 | Compound 218 | 0.11 | Compound 298 | 12.14 |
| Compound 87 | 5.16 | Compound 219 | 0.26 | Compound 93 | 3.24 |
| Compound 88 | 0.53 | Compound 220 | 0.064 | Compound 98 | 2.63 |
| Compound 89 | 3.24 | Compound 221 | 0.046 | Compound 223 | 0.009 |
| Compound 90 | 6.45 | Compound 222 | 0.88 | Compound 224 | 0.011 |

Conclusion: The compounds disclosed in the present disclosure are capable of effectively inhibiting the proliferation of LNCAP cells, and the capabilities of some compounds to inhibit the proliferation of LNCAP cells are comparable or even better than that of the positive control sample.

### Test Example 4

### The inhibitory activity of the compounds disclosed in the present disclosure on the proliferation of VCaP cells

VCaP cells (ATCC, CRL-2876) were cultured in DMEM (Gibco, 11995-065) complete medium containing 10% fetal bovine serum (Hyclone, SH30406.05). On the first day of the experiment, VCaP FGC cells were inoculated in 96 well plates at a density of 15000 cells per well using DMEM medium containing 10% fetal bovine serum, with 100 µL of cell suspension per well. The cells were cultured in a cell culture incubator with 5% CO₂ at 37°C overnight. On the second day, 100 µL of different concentrations of the compounds to be tested of the disclosure and the positive control ARV-110 (source: Energy chemical) prepared with culture medium were added to each well respectively. The final concentration of the compounds was 9 concentration points of 5-fold gradient dilution from 10 µ M, a well containing 100 µL of DMEM medium containing 10% fetal bovine serum was set as a blank control and cultured in the cell culture incubator with 5% CO₂ at 37 °C for 168 hours. On the ninth day, 96 well plates were taken out, and 20 µL of WST-8 (Beytime, C0040) reagent was added to each well. The absorbance at 450nm was detected using a microplate reader (TECAN, F50), and the 50% proliferation inhibition concentration (IC₅₀ value) was calculated by logistic regression using Graphpad Prism software based on the concentration and absorbance values of the compounds.

The IC₅₀ values of the compounds disclosed in the present disclosure for inhibiting the proliferation of VCaP cells are shown in Table 6.

**Table 6 the IC₅₀ values of the compounds disclosed in the present disclosure for inhibiting the proliferation of VCaP cells**

| Compound | IC₅₀ (µM) | Compound | IC₅₀ (µM) | Compound | IC₅₀ (µM) |
|---|---|---|---|---|---|
| ARV-110 | 0.19 | Compound 101 | 3.75 | Compound 266 | 0.0035 |
| Compound 1 | 1.20 | Compound 102 | 3.88 | Compound 267 | 0.0045 |
| Compound 2 | 3.91 | Compound 103 | 2.74 | Compound 269 | 0.27 |
| Compound 5 | 4.50 | Compound 104 | 2.23 | Compound 270 | 0.11 |
| Compound 6 | 7.50 | Compound 105 | 2.96 | Compound 271 | 0.10 |
| Compound 7 | 3.54 | Compound 112 | 5.12 | Compound 272 | 0.063 |
| Compound 8 | 0.001 | Compound 113 | 2.81 | Compound 273 | 0.97 |
| Compound 12 | 2.83 | Compound 114 | 3.29 | Compound 275 | 0.02 |
| Compound 16 | 0.43 | Compound 123 | 3.38 | Compound 276 | 0.035 |
| Compound 17 | 0.58 | Compound 124 | 2.96 | Compound 277 | 0.073 |
| Compound 18 | 0.11 | Compound 125 | 9.29 | Compound 278 | 9.20 |
| Compound 20 | 0.17 | Compound 130 | 3.61 | Compound 279 | 0.046 |
| Compound 21 | 0.38 | Compound 131 | 2.271 | Compound 280 | 0.045 |
| Compound 22 | 0.23 | Compound 138 | >90 | Compound 281 | 0.0040 |
| Compound 25 | 0.91 | Compound 139 | >90 | Compound 282 | 0.0035 |
| Compound 26 | 0.75 | Compound 142 | 15.13 | Compound 283 | 0.013 |
| Compound 27 | 0.38 | Compound 143 | 0.02 | Compound 284 | 0.019 |
| Compound 28 | 6.20 | Compound 147 | 2.99 | Compound 286 | 0.057 |
| Compound 29 | 1.20 | Compound 148 | 4.01 | Compound 287 | 0.31 |
| Compound 30 | 0.85 | Compound 149 | 3.17 | Compound 288 | 0.074 |
| Compound 31 | 0.08 | Compound 150 | 3.34 | Compound 289 | 0.082 |
| Compound 32 | 0.52 | Compound 152 | >90 | Compound 290 | 9.42 |
| Compound 33 | 1.70 | Compound 154 | 0.01 | Compound 291 | 0.047 |
| Compound 34 | 4.20 | Compound 160 | 0.002 | Compound 294 | 0.002 |
| Compound 35 | 0.50 | Compound 161 | 0.003 | Compound 295 | 0.020 |
| Compound 36 | 7.07 | Compound 163 | 0.65 | Compound 296 | 0.0010 |
| Compound 37 | 3.23 | Compound 164 | 11.76 | Compound 297 | 0.0020 |
| Compound 38 | 5.29 | Compound 165 | >90 | Compound 240 | 0.0075 |
| Compound 42 | 0.10 | Compound 167 | >90 | Compound 241 | 0.0085 |
| Compound 44 | 0.0025 | Compound 171 | 0.001 | Compound 242 | 0.094 |
| Compound 46 | 0.11 | Compound 172 | 78.75 | Compound 243 | 0.018 |
| Compound 47 | 0.051 | Compound 173 | 0.001 | Compound 244 | 0.068 |
| Compound 48 | 0.18 | Compound 175 | 0.01 | Compound 245 | 0.10 |
| Compound 49 | 4.00 | Compound 186 | 0.02 | Compound 246 | 0.12 |
| Compound 50 | 0.028 | Compound 191 | >90 | Compound 248 | 0.21 |
| Compound 51 | 0.018 | Compound 192 | 0.003 | Compound 249 | 0.22 |
| Compound 52 | 6.80 | Compound 199 | 0.57 | Compound 250 | 0.16 |
| Compound 53 | 0.043 | Compound 207 | 0.038 | Compound 251 | 0.27 |
| Compound 54 | 0.047 | Compound 208 | 0.0035 | Compound 254 | 0.0025 |
| Compound 55 | 0.083 | Compound 209 | 0.002 | Compound 255 | 0.011 |
| Compound 56 | 0.06 | Compound 210 | 0.002 | Compound 256 | 0.0075 |
| Compound 57 | 0.038 | Compound 211 | 0.0025 | Compound 257 | 0.0015 |
| Compound 58 | 0.069 | Compound 212 | 0.001 | Compound 259 | 0.0012 |
| Compound 59 | 0.11 | Compound 213 | 0.019 | Compound 260 | 0.001 |
| Compound 60 | 4.40 | Compound 214 | 0.048 | Compound 265 | 0.0015 |
| Compound 61 | 0.96 | Compound 215 | 0.5 | Compound 81 | 0.03 |
| Compound 62 | 0.52 | Compound 218 | 0.0025 | Compound 82 | 4.26 |
| Compound 63 | 0.24 | Compound 219 | 0.06 | Compound 83 | 9.73 |
| Compound 64 | 0.16 | Compound 220 | 0.051 | Compound 84 | 4.57 |
| Compound 65 | 0.10 | Compound 221 | 0.0015 | Compound 85 | 7.40 |
| Compound 66 | 0.53 | Compound 227 | 0.03 | Compound 86 | 2.49 |
| Compound 67 | 0.19 | Compound 228 | 0.47 | Compound 87 | 4.19 |
| Compound 69 | 0.96 | Compound 229 | 0.015 | Compound 88 | 0.17 |
| Compound 70 | 0.28 | Compound 230 | 0.018 | Compound 89 | 0.35 |
| Compound 71 | 0.16 | Compound 231 | 5.12 | Compound 90 | 2.03 |
| Compound 72 | 0.1 | Compound 232 | 4.41 | Compound 91 | 2.70 |
| Compound 73 | 0.20 | Compound 233 | 0.043 | Compound 92 | 4.09 |
| Compound 74 | 0.60 | Compound 234 | 0.036 | Compound 93 | 9.48 |
| Compound 75 | 7.4 | Compound 235 | 0.0020 | Compound 94 | 9.51 |
| Compound 76 | 0.0011 | Compound 236 | 0.001 | Compound 97 | 1.3 |
| Compound 78 | 0.05 | Compound 237 | 0.0045 | Compound 98 | 0.89 |
| Compound 79 | 6.50 | Compound 238 | 0.0016 | Compound 99 | 3.80 |
| Compound 80 | 2.83 | Compound 239 | 0.022 | Compound 100 | 0.62 |

Conclusion: The compounds disclosed in the present disclosure are capable of effectively inhibiting the proliferation of VCaP cells, and the capacity of some compounds to inhibit the proliferation of VCaP cells is better than that of the positive control sample.

The present disclosure has been illustrated through the above embodiments and examples, but it should be understood that the above embodiments and examples are only for the purpose of illustration and description, and are not intended to limit the present disclosure to the scope of the described embodiments and examples. In addition, those skilled in the art are to be understood that the present disclosure is not limited to the above embodiments and examples, and more variations and modifications can be made according to the teachings of the present disclosure, all of which fall within the scope of protection required by the present disclosure. The scope of protection of the present disclosure is defined by the accompanying claims and their equivalent.

## Claims

1. A compound having the structure ofI:
PTM-L-CLM (I),
or an isomer, an isotopic derivative, a polymorph, a prodrug, or a pharmaceutically acceptable salt or a solvate thereof,
wherein:
PTM is a moiety that binds to the androgen receptor;
L is a bond or a chemical linker moiety that covalently connects CLM and the PTM, and has a structure of -(B^{L})_{q}-;
each occurrence of B ^{L} is identical or different and is each independently selected from: a covalent bond, CR^{L1}R^{L2}, O, S, SO, SO₂, NR^{L3}, CO, SiR^{L1}R^{L2}, P(O)R^{L1}, P(O)OR^{L1}, C(=NCN), C( = CNO₂), C₂-C₆ alkenylene, C₂-C₆ alkynylene, C₃-C₁₁ cycloalkylene optionally substituted with 0-6 R^{L1} and/or R^{L2} groups, C₃-C₁₁ heterocyclylene optionally substituted with 0-6 R^{L1} and/or R^{L2} groups, arylene optionally substituted with 0-6 R^{L1} and/or R^{L2} groups, heteroarylene optionally substituted with 0-6 R^{L1} and/or R^{L2} groups, C₆-C₁₆ spirocyclylene optionally substituted with 0-6 R^{L1} and/or R^{L2} groups, and C₆-C₁₆ heterospirocyclylene optionally substituted with 0-6 R^{L1} and/or R^{L2} groups; preferably
R^{L1}, R^{L2}, and R^{L3} are each independently H, halogen, alkyl, haloalkyl, alkoxy, haloalkoxy, alkenyl, haloalkenyl, alkynyl, haloalkynyl, SR^{L4}, NR^{L4}R^{L5}, cycloalkyl, aryl, heteroaryl, heterocyclyl, OR^{L4}, OH, SO₂-R^{L4}, P(O)R^{L4}R^{L5}, Si(OH)₃, SiR^{L4}R^{L5}R^{L6}, COR^{L6}, CN, NO₂, SF₅, SO₂NR^{L4}R^{L5}, CONR^{L4}R^{L5}, - COOR^{L4}, N(R^{L4})CONR^{L5}R^{L4}, or N(R^{L4})SO₂NR^{L4}R^{L5};
R^{L4} and R^{L5} are each independently H, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl;
R^{L6} is each independently H, OH, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl; and
q is an integer greater than or equal to 1;
the CLM is a cereblon E3 ubiquitin ligase binding moiety, selected from the following structures: 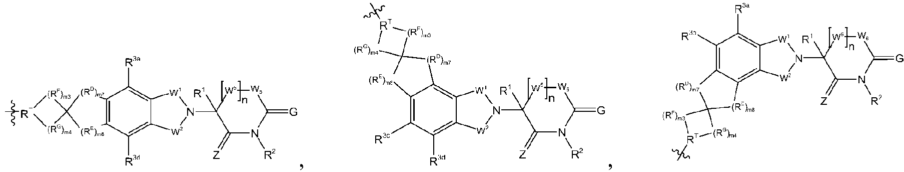 and 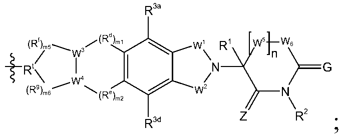
wherein:
W¹ and W² are identical or different, each independently being CR^{a}R^{b}, C(=O), NR^{a}, or SO₂, and at least one of W¹ and W² is C(=O);
G and Z are identical or different and are each independently selected from O, S, and Se;
R^{3a}, R^{3b}, R^{3c}, and R^{3d} are each independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
each occurrence of W⁵, W⁶, R^{d}, R^{e}, R^{f}, R^{g}, R^{D}, R^{E}, R^{F}, and R^{G} are each independently C(R^{m})₂, NR^{m}, O, or S;
W³ and W⁴ are each independently CR^{m} or N;
R^{t} and R^{T} are each independently N or CR^{2h}, and when all of R^{D}, R^{E}, R^{F}, and R^{G} are C(R^{m})₂, R^{T} are CR^{2h};
m1 and m2 are each independently an integer of 0, 1, 2, 3, 4, 5, or 6, and m1+ m2≤6;
m3 is an integer of 0, 1, 2, 3, 4, 5, 6, or 7, m4 is an integer of 1, 2, 3, 4, 5, 6, 7, or 8, and m3+m4≤8;
m5 and m6 are each independently an integer of 0, 1, 2, 3, 4, 5, 6, or 7, and m5+ m6≤7;
m7 and m8 are each independently an integer of 0, 1, 2, 3, 4, 5, 6, or 7, and m7+ m8≤7;
each occurrence of R^{m} is independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, alkylacyl, alkoxycarbonyl, alkylaminocarbonyl, -C₁₋₆ alkylene -ONH₂, -NHO-C₁₋₆ alkyl, -C₁₋₆ alkylene -NH-C₁₋₆ alkylene -ONH₂, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
R^{2h} is selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl, and C₁-C₆ hydroxyalkyl; preferably, R¹ is H, F, Cl, Br, I, C₁-C₃ alkyl, or hydroxyl;
R², R^{a}, and R^{b} are each independently selected from H, deuterium, F, Cl, Br, I, C₁-C₆ alkyl, and C₁-C₆ alkoxy, hydroxyl, C₁-C₆ haloalkyl, and C₁-C₆ hydroxyalkyl; and
n is 0, 1, 2, or 3.

2. A compound, wherein the compound is a compound having the structure of formula I:
PTM-L-CLM (formula I),
or an isomer, an isotopic derivative, a polymorph,a prodrug, or a pharmaceutically acceptable salt or a solvate thereof,
wherein:
PTM is a moiety that binds to the androgen receptor;
L is a bond or a chemical linker that covalently connects CLM and PTM, and has a structure of - (B^{L})_{q}-;
each occurrence of B^{L} is identical or different and is each independently selected from a covalent bond, CR^{L1}R^{L2}, O, S, SO, SO₂, NR^{L3}, CO, SiR^{L1}R^{L2}, P(O)R^{L1}, P(O)OR^{L1}, C(=NCN), C( = CNO₂), C₃-C₁₁ cycloalkylene optionally substituted with 0-6 R^{L1} and/or R^{L2} groups, C₃-C₁₁ heterocyclylene optionally substituted with 0-6 R^{L1} and/or R^{L2} groups, arylene optionally substituted with 0-6 R^{L1} and/or R^{L2} groups, heteroarylene optionally substituted with 0-6 R^{L1} and/or R^{L2} groups, C₆-C₁₆ spirocyclylene optionally substituted with 0-6 R^{L1} and/or R^{L2} groups, and C₆-C₁₆ heterospirocyclylene optionally substituted with 0-6 R^{L1} and/or R^{L2} groups;
R^{L1}, R^{L2}, and R^{L3} are each independently H, halogen, alkyl, haloalkyl, alkoxy, haloalkoxy, alkenyl, haloalkenyl, alkynyl, haloalkynyl, SR^{L4}, NR^{L4}R^{L5}, cycloalkyl, aryl, heteroaryl, heterocyclyl, OR^{L4}, OH, SO₂-R^{L4}, P(O)R^{L4}R^{L5}, Si(OH)₃, Si R^{L4}R^{L5}R^{L6}, COR^{L6}, CN, NO₂, SF₅, SO₂NR^{L4}R^{L5}, CONR^{L4}R^{L5}, N(R^{L4})CONR^{L5}R^{L4}, or N(R^{L4})SO₂NR^{L4}R^{L5};
R^{L4} and R^{L5} are each independently H, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl; R^{L6} is each independently H, OH, halogen, alkyl, haloalkyl, alkoxy, haloalkoxy, alkenyl, haloalkenyl, alkynyl, haloalkynyl, cycloalkyl, halocycloalkyl, heterocyclyl, haloheterocyclyl, aryl, haloaryl, heteroaryl, or haloheteroaryl;
q is an integer greater than or equal to 1; preferably, q is 1, 2, 3, 4, 5, 6, 7, 8, 9, 10, 11, 12, 13, 14, 15, 16, 17, 18, 19, or 20;
the CLM is a cereblon E3 ubiquitin ligase binding moiety, selected from the following structures: 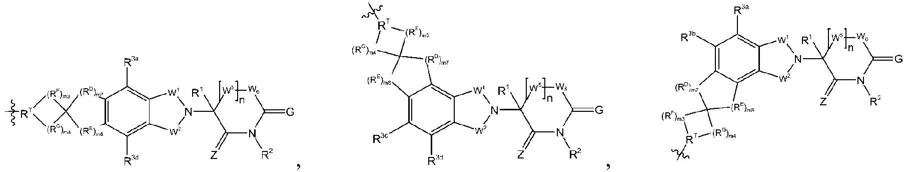 and 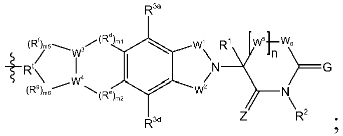
wherein:
W¹ and W² are identical or different, each independently being CR^{a}R^{b}, C(=O), NR^{a}, or SO₂, and at least one of W¹ and W² is C(=O);
G and Z are identical or different and are each independently selected from O, S, and Se;
R^{3a}, R^{3b}, R^{3c}, and R^{3d} are each independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
each occurrence of W⁵, W⁶, R^{d}, R^{e}, R^{f}, R^{g}, R^{D}, R^{E}, R^{F}, and R^{G} are each independently C(R^{m})₂, NR^{m}, O, or S;
W³ and W⁴ are each independently CR^{m} or N;
R^{t} and R^{T} are each independently N or CR^{2h}, and when all of R^{D}, R^{E}, R^{F}, and R^{G} are C(R^{m})₂, R^{T} is CR^{2h};
m1 and m2 are each independently an integer of 0, 1, 2, 3, 4, 5, or 6, and m1+ m2≤6;
m3 is an integer of 0, 1, 2, 3, 4, 5, 6, or 7, m4 is an integer of 1, 2, 3, 4, 5, 6, 7, or 8, and m3+m4≤8;
m5 and m6 are each independently an integer of 0, 1, 2, 3, 4, 5, 6, or 7, and m5+ m6≤7;
m7 and m8 are each independently an integer of 0, 1, 2, 3, 4, 5, 6, or 7, and m7+ m8≤7;
each occurrence of R^{m} is independently selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, alkylacyl, alkoxycarbonyl , alkylaminocarbonyl, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
R^{2h} is selected from H, deuterium, halogen, alkyl, deuterated alkyl, heteroalkyl, alkenyl, alkynyl, alkoxy, hydroxyl, nitro, cyano, amino, cycloalkyl, heterocyclyl, alkylamino, aryl, and heteroaryl, wherein the alkyl, heteroalkyl, alkenyl, alkynyl, alkoxy, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl;
R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl and C₁-C₆ hydroxyalkyl; preferably, R¹ is H, F, Cl, Br, I, C₁-C₃ alkyl, or hydroxyl;
R², R^{a}, and R^{b} are each independently selected from H, deuterium, F, Cl, Br, I, C₁-C₆ alkyl, and C₁-C₆ alkoxy, hydroxyl, C₁-C₆ haloalkyl, and C₁-C₆ hydroxyalkyl; and
n is 0, 1, 2, or 3.

3. The compound according to claim 1 or 2, wherein:
W¹ and W² are identical or different, each independently being CH₂ or C(=O), and at least one of W¹ and W² is C(=O); and/or
G is O; and/or
Z is O; and/or
R^{3a}, R^{3b}, R^{3c}, and R^{3d} are each independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₃-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₃-C₁₀ heteroaryl are each independently optionally substituted with one or more substituents selected from F, Cl, Br, I, C₁-C₆ alkyl, C₁-C₆ heterocyclyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₃-C₁₀ heteroaryl; preferably, R^{3a}, R^{3b}, R^{3c}, and R^{3d} are each independently selected from H, deuterium, F, Cl, Br, I, C₁-C₃ alkyl, and C₁-C₃ alkoxy; and/or
each occurrence of R^{d}, R^{e}, R^{D}, and R^{E} are each independently C(R^{m})₂ or O; and/or
each occurrence of R^{f}, R^{g}, R^{F}, and R^{G} are each independently C(R^{m})₂ or O, and preferably C(R^{m})₂; and/or
W³ and W⁴ are CH; and/or
W⁵ and W⁶ are C(R^{m})₂ or N(R^{m}), and preferably CH₂, CH (C₁-C₆ alkyl), CH (C₁-C₆ haloalkyl), CH(OH), or NH;
R^{2h} is selected from H, deuterium, F, Cl, Br, I, C₁-C₆ alkyl, C₁-C₆ deuterated alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₃-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₃-C₁₀ heteroaryl; preferably, R^{2h} is selected from H, deuterium, F, Cl, Br, I, C₁-C₃ alkyl, and C₁-C₃ alkoxy; and/or
m1 and m2 are each independently an integer of 0, 1, 2, or 3, and m1+ m2≤3, preferably, m1+m2=1 or m1+m2=2; and/or
m3 is an integer of 0, 1, 2, 3, or 4, m4 is an integer of 1, 2, 3, 4, or 5, and m3+m4≤5, preferably, m3+m4=2, m3+m4=3, or m3+m4=4; and/or
each occurrence of m5 and m6 are each independently an integer of 0, 1, 2, 3, or 4, and m5+ m6≤4, preferably, m5+m6=2 or m5+m6=3;
each occurrence of m7 and m8 are each independently an integer of 0, 1, 2, 3, or 4, and m7+ m8≤4, preferably, m7+m8=2 or m7+m8=3; and /or
each occurrence of R^{m} is independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₁-C₆ alkylacyl, C₁-C₆ alkoxycarbonyl , C₁-C₆ alkylaminocarbonyl, -C₁₋₆ alkylene -ONH₂, -NHO-C₁₋₆ alkyl, -C₁₋₆ alkylene -NH-C₁₋₆ alkylene -ONH₂, C₆-C₁₀ aryl, and C₃-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₃-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₃-C₁₀ heteroaryl; preferably, each occurrence of R^{m} is independently selected from H, deuterium, F, Cl, Br, I, C₁-C₃ alkyl, and C₁-C₃ alkoxy; and/or
R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl, and C₁-C₆ hydroxyalkyl; preferably R¹ is H, F, Cl, Br, I, C₁-C₃ alkyl, or hydroxyl; and/or
R² is selected from H, deuterium, F, Cl, Br, I, C₁-C₆ alkyl, C₁-C₆ alkoxy, hydroxyl, C₁-C₆ haloalkyl and C₁-C₆ hydroxyalkyl; preferably R² is H, F, Cl, Br, I, C₁-C₃ alkyl, or hydroxyl; and /or
n is 0 or 1.

4. The compound according to claim 2, wherein:
W¹ and W² are identical or different, each independently being CH₂ or C(=O), and at least one of W¹ and W² is C(=O); and/or
G is O; and /or
Z is O; and /or
R^{3a}, R^{3b}, R^{3c}, and R^{3d} are each independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₃-C₁₀ heteroaryl are each independently optionally substituted with one or more substituents selected from F, Cl, Br, I, C₁-C₆ alkyl, C₁-C₆ heterocyclyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₃-C₁₀ heteroaryl; preferably, R^{3a}, R^{3b}, R^{3c}, and R^{3d} are each independently selected from H, deuterium, F, Cl, Br, I, C₁-C₃ alkyl, and C₁-C₃ alkoxy; and/or
each occurrence of R^{d}, R^{e}, R^{D}, and R^{E} are each independently C(R^{m})₂ or O; and/or
each occurrence of R^{f}, R^{g}, R^{F}, and R^{G} are each independently C(R^{m})₂ or O, preferably C(R^{m})₂; and/or
W³ and W⁴ are CH; and/or
W⁵ and W⁶ are C(R^{m})₂ or N(R^{m}), and preferably CH₂, CH (C₁-C₆ alkyl), CH (C₁-C₆ haloalkyl), CH(OH), or NH;
R^{2h} is selected from H, deuterium, F, Cl, Br, I, C₁-C₆ alkyl, C₁-C₆ deuterated alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₆-C₁₀ aryl, and C₅-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl and C₃-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₃-C₁₀ heteroaryl; preferably, R^{2h} is selected from H, deuterium, F, Cl, Br, I, C₁-C₃ alkyl, and C₁-C₃ alkoxy; and/or
m1 and m2 are each independently an integer of 0, 1, 2, or 3, and m1+ m2≤3, preferably, m1+m2=1 or m1+m2=2; and/or
m3 is an integer of 0, 1, 2, 3, or 4, m4 is an integer of 1, 2, 3, 4, or 5, and m3+m4≤5, preferably, m3+m4=2, m3+m4=3, or m3+m4=4; and/or
each occurrence of m5 and m6 are each independently an integer of 0, 1, 2, 3, or 4, and m5+ m6≤4, preferably, m5+m6=2 or m5+m6=3;
each occurrence of m7 and m8 are each independently an integer of 0, 1, 2, 3, or 4, and m7+ m8≤4, preferably, m7+m8=2 or m7+m8=3; and /or
each occurrence of R^{m} is independently selected from H, deuterium, F, Cl, Br, I, C₁-C₆ alkyl, C₁-C₆ heteroalkyl, C₂-C₆ alkenyl, C₂-C₆ alkynyl, C₁-C₆ alkoxy, hydroxyl, nitro, cyano, amino, C₃-C₈ cycloalkyl, C₄-C₁₀ heterocyclyl, C₁-C₆ alkylamino, C₁-C₆ alkylacyl, C₁-C₆ alkoxycarbonyl , C₁-C₆ alkylaminocarbonyl, C₆-C₁₀ aryl, and C₃-C₁₀ heteroaryl, wherein the C₁-C₆ alkyl, C₁-C₆ heteroalkyl, C₂-C₆ alkenyl, C₂-C₆ alkynyl, C₁-C₆ alkoxy, C₃-C₈ cycloalkyl, C₄-C₁₀ heterocyclyl, C₆-C₁₀ aryl, and C₃-C₁₀ heteroaryl are each independently optionally substituted with one or more substituents selected from F, Cl, Br, I, C₁-C₆ alkyl, C₁-C₆ heteroalkyl, C₂-C₆ alkenyl, C₂-C₆ alkynyl, C₁-C₆ alkoxy, hydroxyl, C₁-C₆ haloalkyl, C₁-C₆ hydroxyalkyl, cyano, amino, nitro, C₃-C₈ cycloalkyl, C₄-C₁₀ heterocyclyl, C₁-C₆ alkylamino, C₆-C₁₀ aryl, and C₃-C₁₀ heteroaryl; preferably, each occurrence of R^{m} is independently selected from H, deuterium, F, Cl, Br, I, C₁-C₃ alkyl, and C₁-C₃ alkoxy; and/or
R¹ is selected from H, deuterium, F, Cl, Br, I, C₁-C₆ alkyl, C₁-C₆ cycloalkyl, C₁-C₆ alkoxy, hydroxyl, C₁-C₆ haloalkyl, and C₁-C₆ hydroxyalkyl; preferably R¹ is H, F, Cl, Br, I, C₁-C₃ alkyl, or hydroxyl; and/or
R² is selected from **H,** deuterium, F, Cl, Br, I, C₁-C₆ alkyl, and C₁-C₆ alkoxy, hydroxyl, C₁-C₆ haloalkyl and C₁-C₆ hydroxyalkyl; preferably R² is **H,** F, Cl, Br, **I,** C₁-C₃ alkyl, or hydroxyl; and/or
n is 0 or 1.

5. The compound according to any one of claims 1-4, wherein the CLM is selected from the following structures: 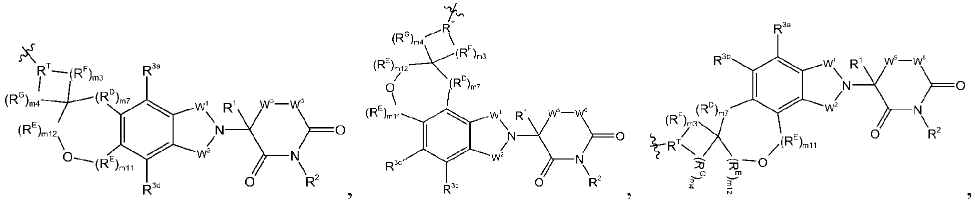 and 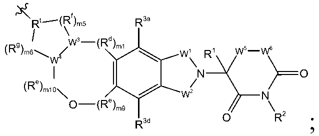 preferably, the CLM is the following structures: 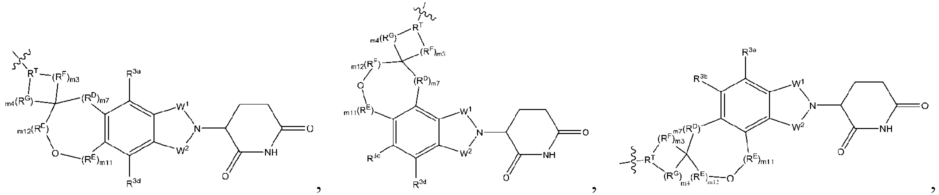 or 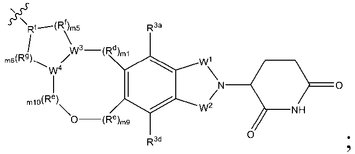 wherein:
W¹, W², W³, W⁴, W⁵, W⁶, R^{3a}, R^{3b}, R^{3c}, R^{3d}, R^{d}, R^{e}, R^{f}, R^{t}, R^{g}, R^{D}, R^{E}, R^{F}, R^{T}, R^{G}, R¹, R², m3, m4, m5, and m6 are as defined in claim 1, 2, 3, or 4;
m1, m9, and m10 are each independently an integer of 0, 1, 2, 3, 4, or 5, and m1+m9+m10≤5; preferably, m1, m9, and m10 are each independently an integer of 0, 1, or 2, and m1+m9+m10≤2; preferably, m1+m9+m10=1 or m1+m9+m10=0; and
m7, m11, and m12 are each independently an integer of 0, 1, 2, 3, 4, 5, or 6, and m7+m11+m12≤6; preferably, m7, m11, and m12 are each independently an integer of 0, 1, 2, or 3, and m7+m11+m12≤3; preferably, m7+m11+m12=1 or m7+m11+m 12=2.

6. The compound according to any one of claims 1-4, wherein the CLM is selected from the following structures: 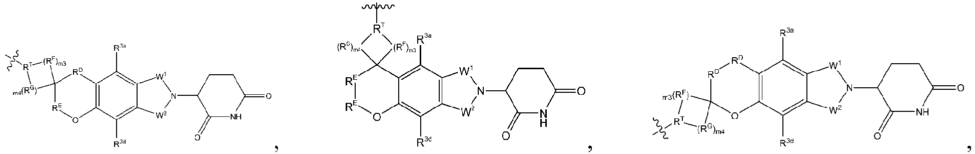 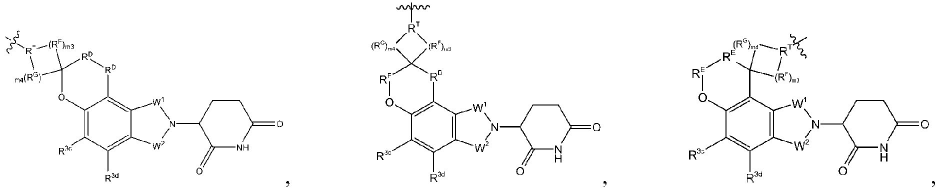 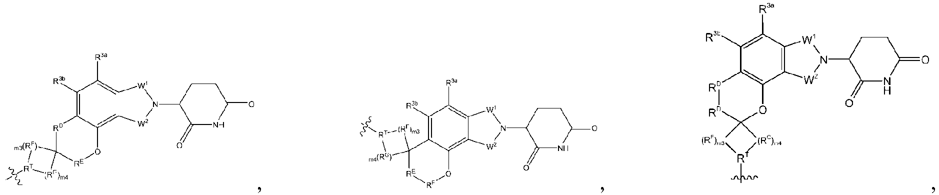 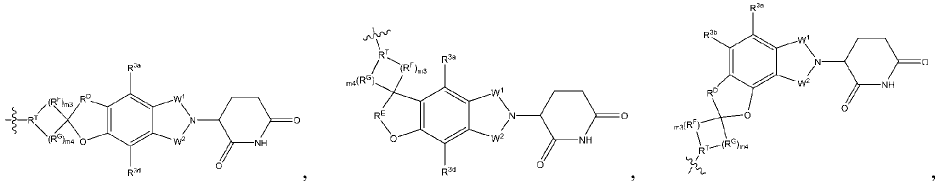 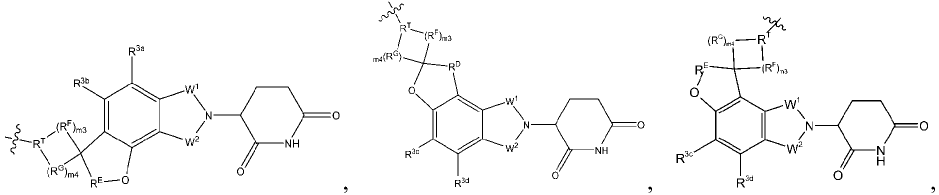 and 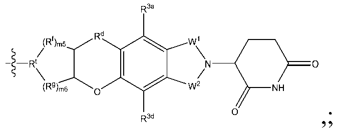 wherein:
W¹, W², R^{3a}, R^{3b}, R^{3c}, R^{3d}, R^{d}, R^{f}, R^{t}, R^{g}, R^{D}, R^{E}, R^{F}, R^{T}, R^{G}, m3, m4, m5, and m6 are as defined in claim 1, 2, 3 or 4.

7. The compound according to any one of claims 1-4, wherein the CLM is selected from the following structures, or an isomer, an isotopic derivative, a polymorph, a prodrug, or a pharmaceutically acceptable salt or a solvate thereof: 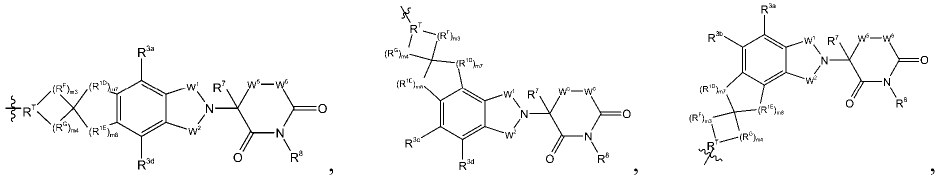 and 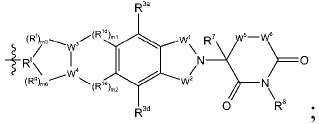 preferably, the CLM is the following structures: 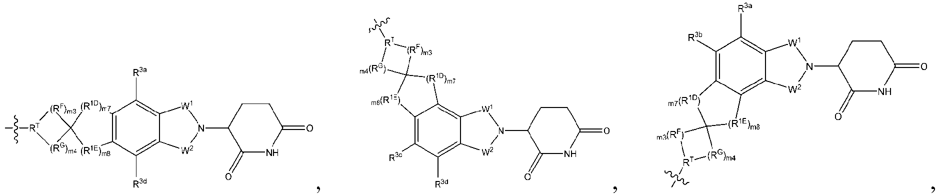 or 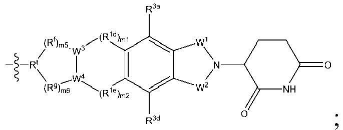 wherein:
W¹, W², W³, W⁴, W⁵, W⁶, R^{3a}, R^{3b}, R^{3c}, R^{3d}, R^{f}, R^{g}, R^{t}, R^{F}, R^{G}, R⁷, R⁸, m1, m2, m3, m4, m5, m6, m7, and m8 are as defined in claim 1, 2, 3, or 4; and
each occurrence of R^{1d}, R^{1E}, R^{1D}, and R^{1E} is independently C(R^{m})₂; and
R^{T} is N or CR^{2h}, and when R^{F} and R^{G} are both C(R^{m})₂, R^{T} is CR^{2h}; and
R^{2h} and R^{m} are as defined in claim 1, 2, 3, or 4.

8. The compound according to any one of claims 1-2, or 5, wherein the CLM is selected from the following structures: 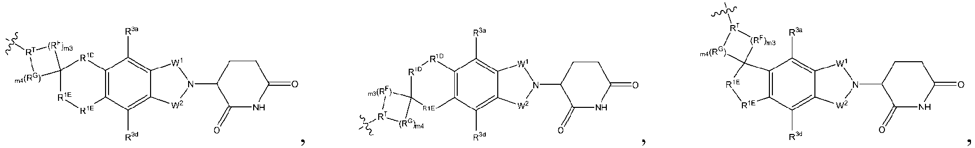 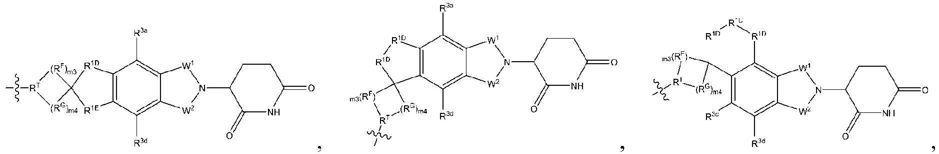 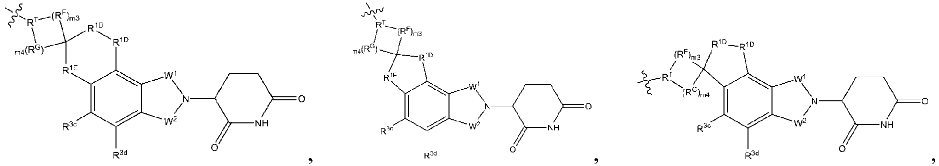 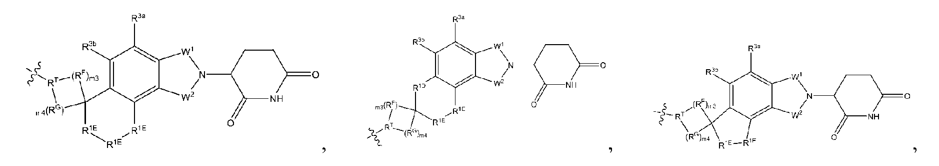 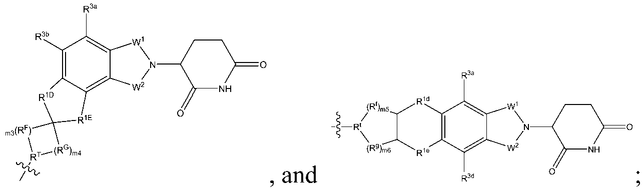 wherein W¹, W², R^{3a}, R^{3b}, R^{3c}, R^{3d}, R^{f}, R^{t}, R^{g}, R^{F}, R^{T}, R^{G}, R^{1d}, R^{1e}, R^{1D}, R^{1E}, m3, m4, m5, and m6 are as defined in claim 1, 2, 3, 4, 5, 6, or 7.

9. The compound according to any one of claims 1-8, wherein the CLM is selected from the following structures:

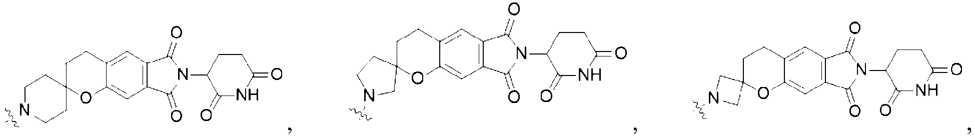

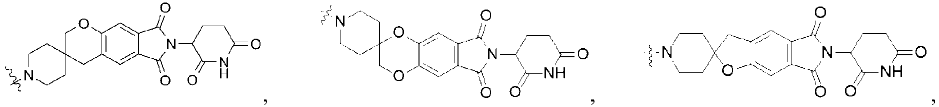

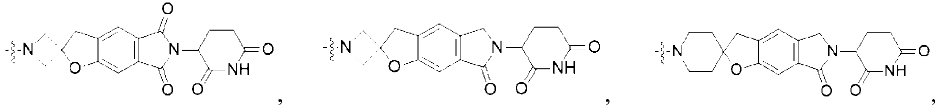

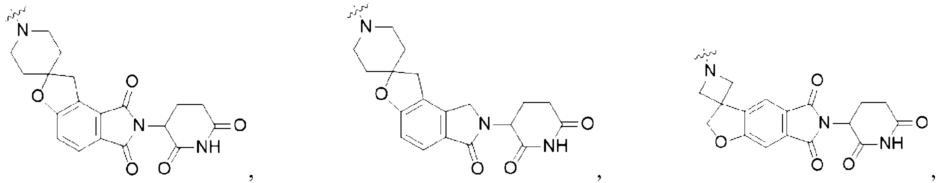

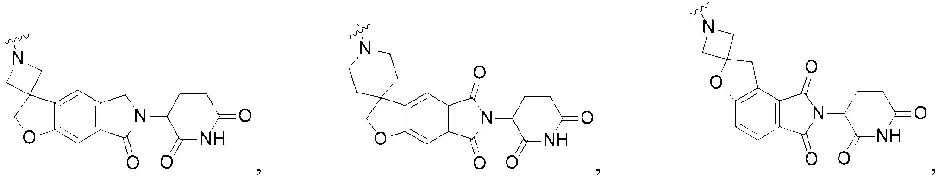

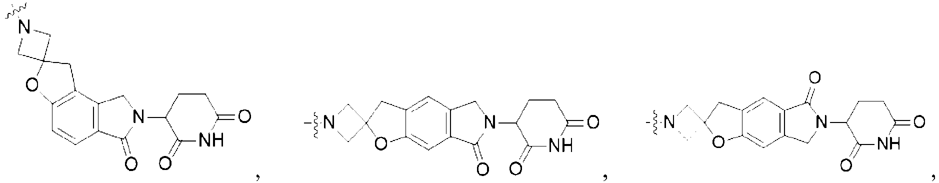

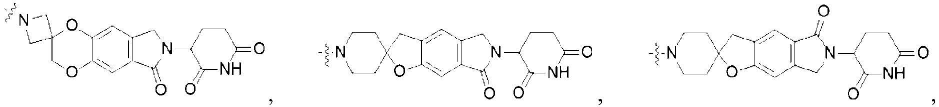

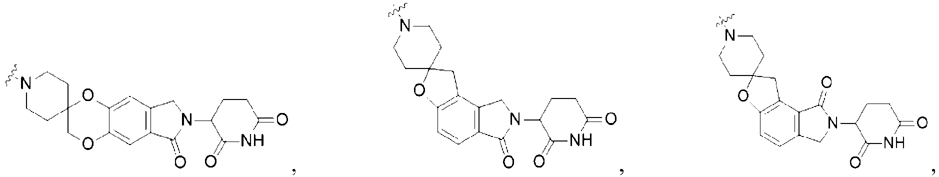

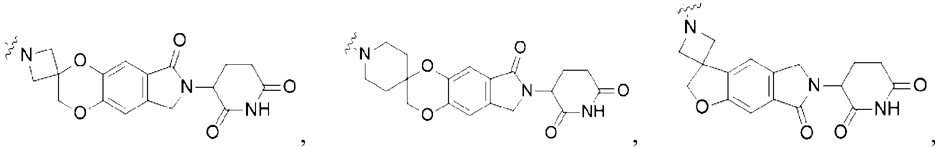

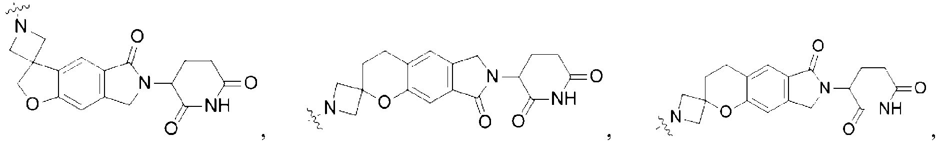

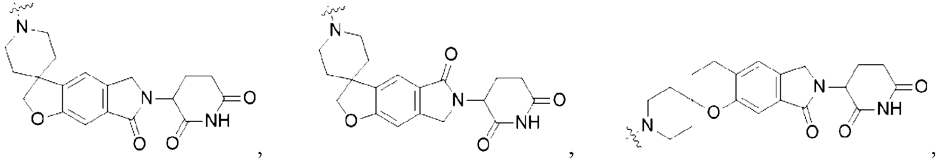

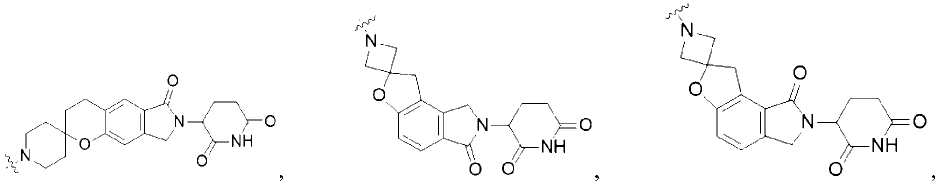

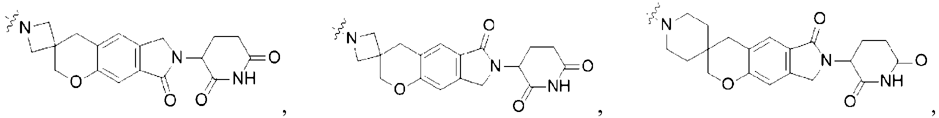

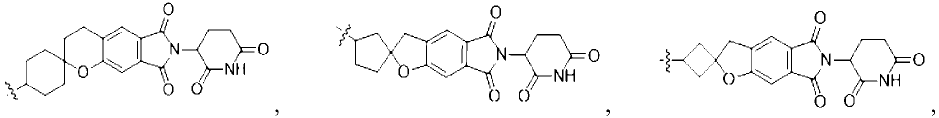

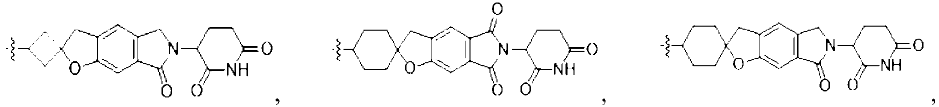

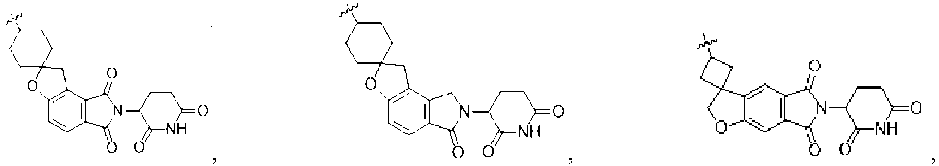

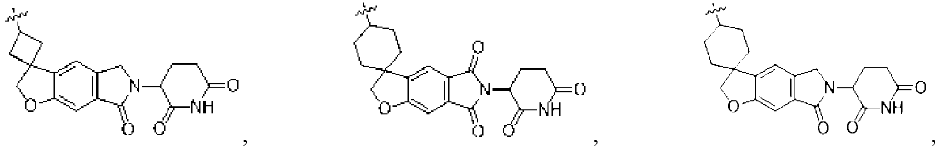

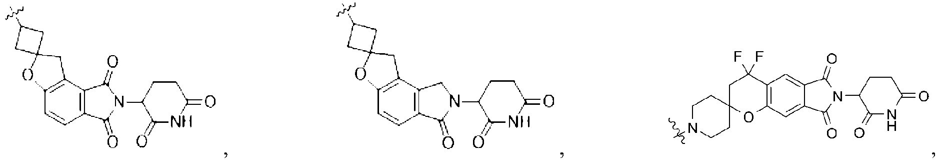

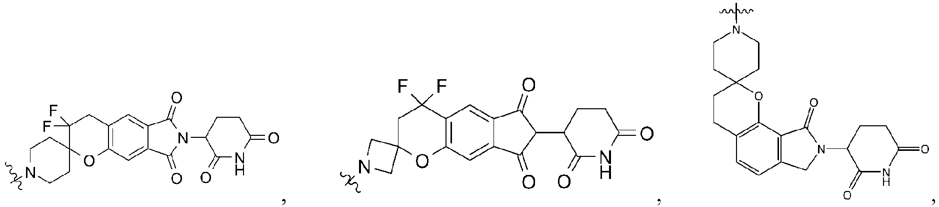

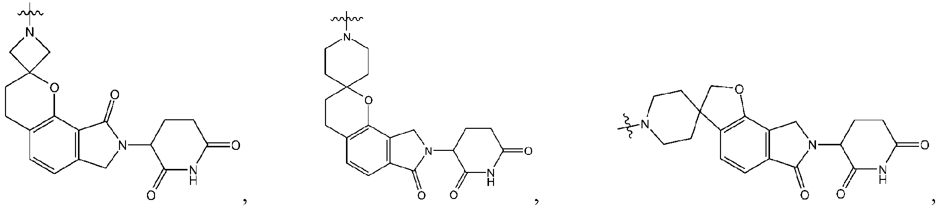

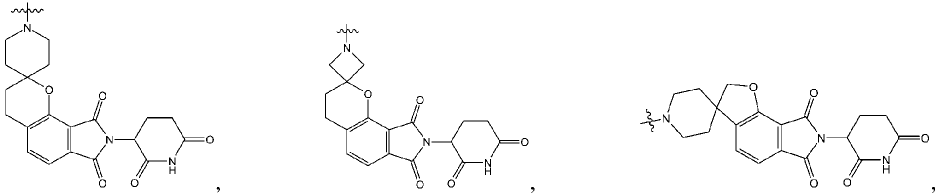

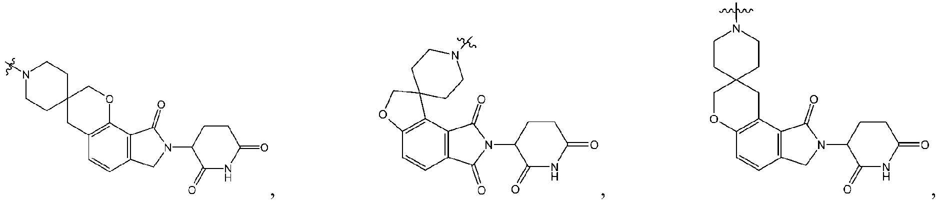

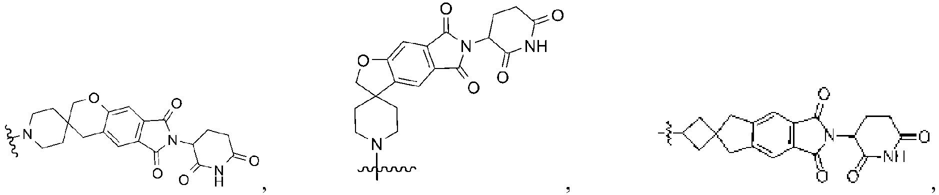

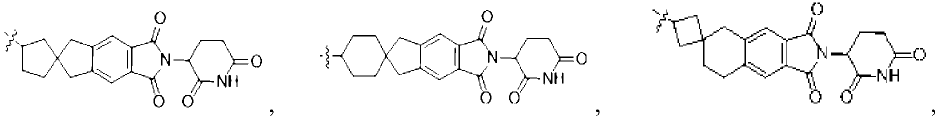

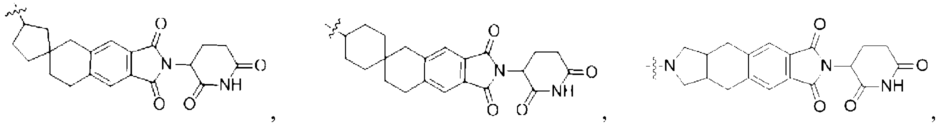

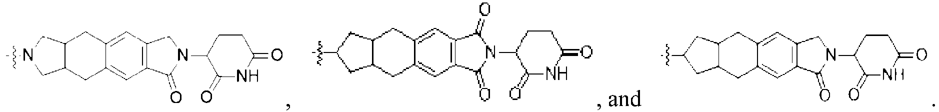

10. The compound according to any one of claims 1-9, wherein:
each occurrence of B^{L} is identical or different and is independently selected from: a covalent bond, CR^{L1}R^{L2}, O, S, SO, SO₂, NR^{L3}, CO, C₂₋₆ alkenylene, C₂₋₆ alkynylene, cycloalkylene, heterocyclylene, spirocyclylene, heterospirocyclylene, arylene, and heteroarylene, wherein the cycloalkylene, heterocyclylene, spirocyclylene, heterospirocyclylene, arylene, and heteroarylene are optionally substituted with 0-6 R^{L1} and/or R^{L2} groups; preferably, each occurrence of B^{L} is identical or different and is independently selected from: a covalent bond, CR^{L1}R^{L2}, O, S, SO, SO₂, NR^{L3}, CO, cycloalkylene, heterocyclylene, spirocyclylene, heterospirocyclylene, arylene, and heteroarylene, wherein the cycloalkylene, heterocyclylene, spirocyclylene, heterospirocyclylene, arylene, and heteroarylene are optionally substituted with 0-6 R^{L1} and/or R^{L2} groups; and/or
each occurrence of R^{L1}, R^{L2}, and R^{L3} are each independently selected from H, F, Cl, Br, I, C₁₋₈ alkyl, C₃₋₁₁ cycloalkyl, C₃₋₁₁ heterocyclyl, C₆₋₁₀ aryl, C₃₋₁₀ heteroaryl, C₁₋₈ alkoxy, C₁₋₈ alkylene-O-C₁₋₈ alkyl, C₁₋₈ alkylene C₃₋₁₁ cycloalkyl, -o-C₃₋₈ cycloalkyl, -o-C₃₋₁₁ heterocyclyl, -O- aryl, -O-heteroaryl, -NH-C₁₋₈ alkyl, -N(C₁₋₈ alkyl)₂, -NH-C₃₋₈ cycloalkyl, -N (C₃₋₈ cycloalkyl)₂, -N(C₃₋₈ cycloalkyl)(C₁₋₈ alkyl), -NH-C₃₋₈ heterocyclyl, -N (C₃₋₈ heterocyclyl)₂, -N(C₃₋₈ heterocyclyl)(C₁₋₈ alkyl), -NH- aryl, -N(aryl)(C₁₋₈ alkyl), -NH- heteroaryl, -N(heteroaryl)(C₁₋₈ alkyl), -OH, -NH₂, -CO-C₁₋₈ alkyl, -CO₂H, -CN, -CF₃, -CHF₂, -CH₂F, -NO₂, -CONH-C₁₋₈ alkyl, -CON(C₁₋₈ alkyl)₂, -N(C₁₋₈ alkyl)CONH(C₁₋₈ alkyl), -N(C₁₋₈ alkyl)CON(C₁₋₈ alkyl)₂, -NHCONH(C₁₋₈ alkyl), -NHCON(C₁₋₈ alkyl)₂, -NHCONH₂, and -COO-C₁₋₈ alkyl, wherein the alkyl, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl; preferably, each occurrence of R^{L1} R^{L2}, and R^{L3} is independently selected from H, F, Cl, Br, I, C₁₋₈ alkyl, C₃₋₁₁ cycloalkyl, C₃₋₁₁ heterocyclyl, C₆₋₁₀ aryl, C₅₋₁₀ heteroaryl, C₁-₈ alkoxy, O-C₃₋₈ cycloalkyl, O-C₃₋₁₁ heterocyclyl, O- aryl, O- heteroaryl, NH-C₁₋₈ alkyl, N(C₁₋₈ alkyl)₂, NH-C₃₋₈ cycloalkyl, N (C₃₋₈ cycloalkyl)₂, N(C₃₋₈ cycloalkyl)(C₁₋₈ alkyl), NH-C₃₋₈ heterocyclyl, N (C₃₋₈ heterocyclyl)₂, N(C₃₋₈ heterocyclyl)(C₁₋₈ alkyl), NH- aryl, N(aryl)(C₁₋₈ alkyl), NH- heteroaryl, N(heteroaryl)(C₁₋₈ alkyl), OH, NH₂, CO-C₁₋₈ alkyl, CO₂H, CN, CF₃, CHF₂, CH₂F, NO₂, CONH-C₁₋₈ alkyl, CON(C₁₋₈ alkyl)₂, N(C₁₋₈ alkyl)CONH(C₁₋₈ alkyl), N(C₁₋₈ alkyl)CON(C₁₋₈ alkyl)₂, NHCONH(C₁₋₈ alkyl), NHCON(C₁₋₈ alkyl)₂, and NHCONH₂, wherein the alkyl, cycloalkyl, heterocyclyl, aryl, and heteroaryl are each independently optionally substituted with one or more substituents selected from halogen, alkyl, heteroalkyl, alkenyl, alkynyl, alkoxy, hydroxyl, haloalkyl, hydroxyalkyl, cyano, amino, nitro, cycloalkyl, heterocyclyl, alkylamino, aryl, and heteroaryl; and/or
q is an integer greater than or equal to 1; preferably 1, 2, 3, 4, 5, 6, 7, 8, 9, 10, 11, 12, 13, 14, 15, 16, 17, 18, 19, or 20.

11. The compound according to any one of claims 1-10, wherein B^{L} is selected from one or more of the following structures: a covalent bond, -O-, -(CH₂)ₖ-, -CO-, -NH-, 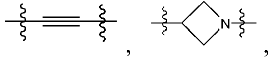 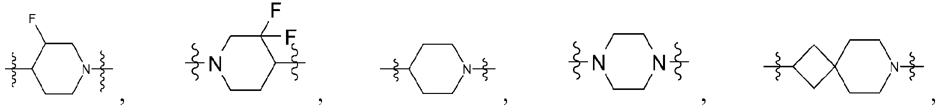 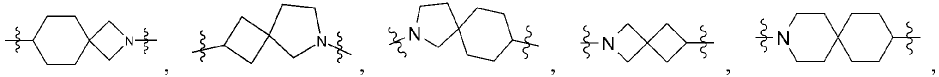 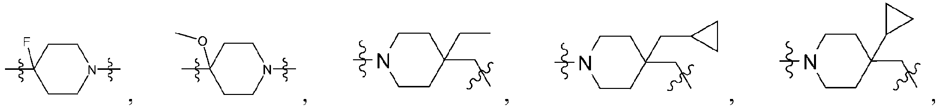 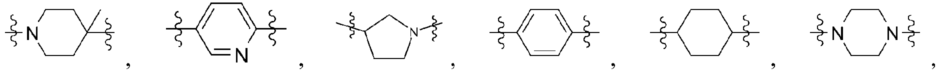 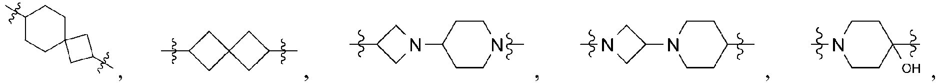 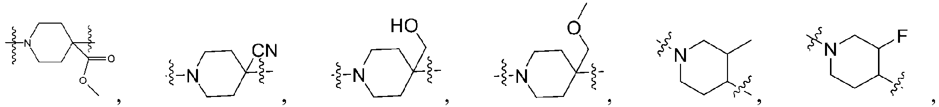 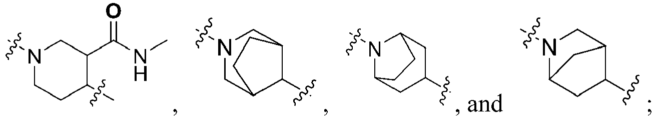 k is 1, 2, 3, 4, 5, 6, 7, 8, 9, or 10.

12. The compound according to any one of claims 1-11, wherein L is selected from the following structures:
a covalent bond, -(CH₂)ⱼ-, -(CH₂)ⱼ-CO-, -NH-(CH₂)ⱼ-, -(CH₂)ⱼ-NH-, -NH-(CH₂)ⱼ-NH-, 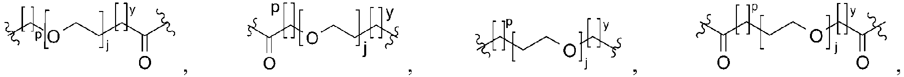 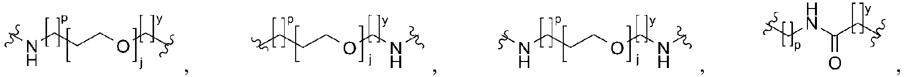 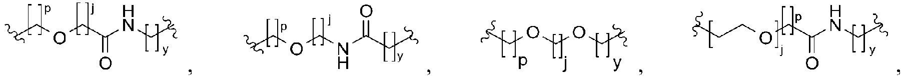 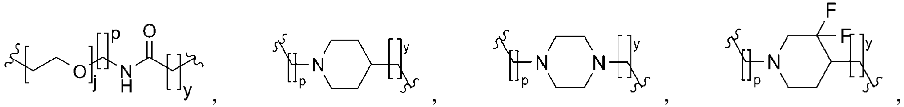 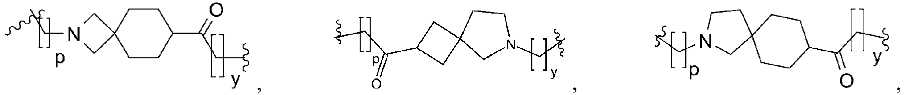 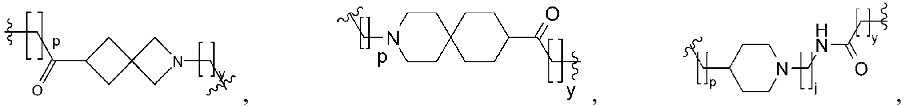 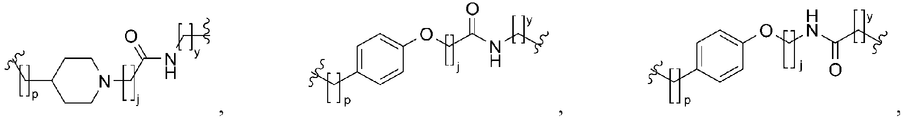 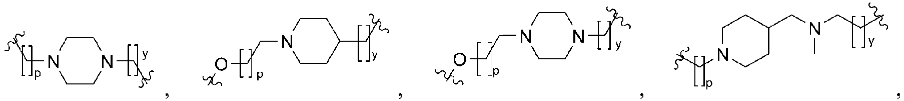 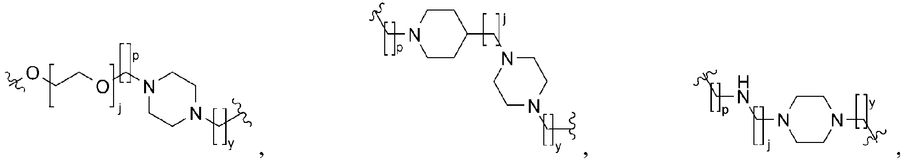 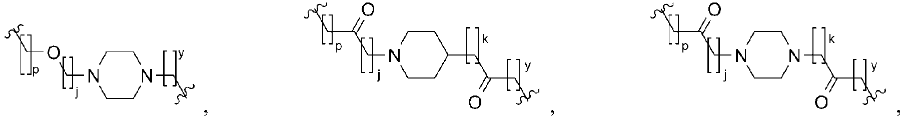 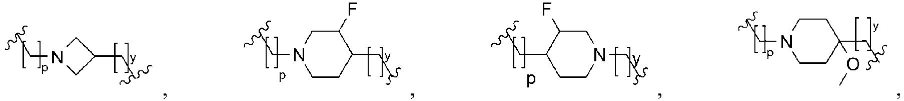 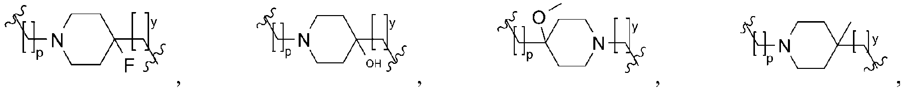 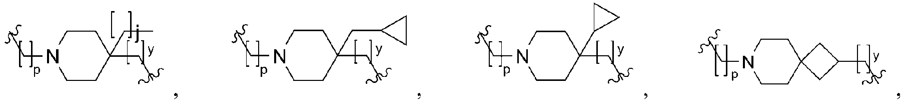 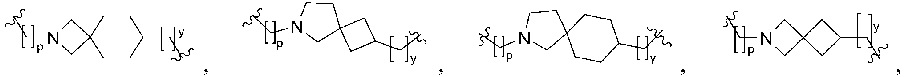 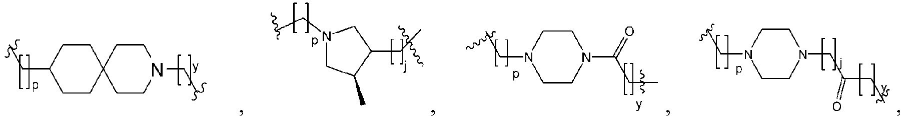 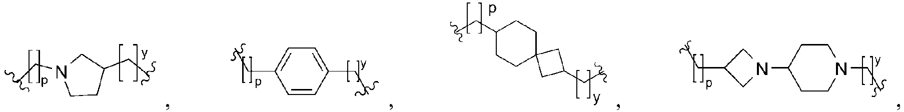 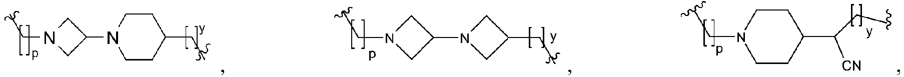 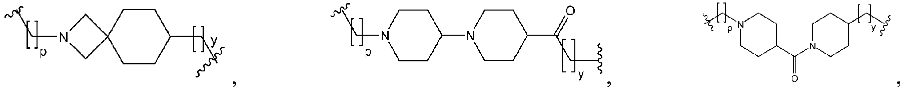 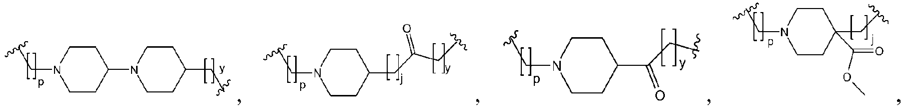 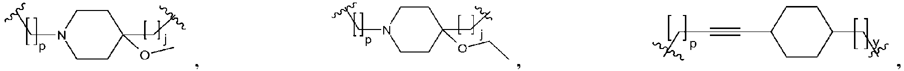 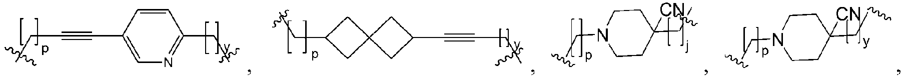 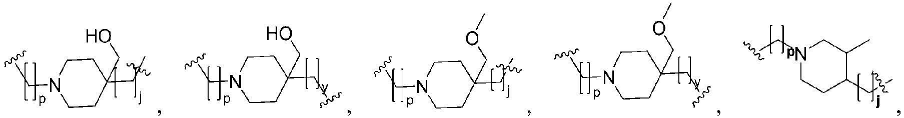 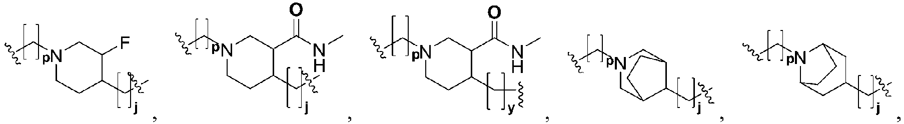 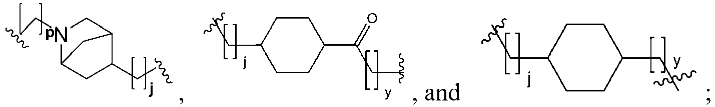
wherein j is 1, 2, 3, 4, 5, 6, 7, 8, 9, or 10;
p and y are 0, 1, 2, 3, 4, 5, 6, 7, 8, 9, or 10;
preferably, L is selected from a covalent bond, -CH₂-, -CH₂-CO-, -(CH₂)₂-, -(CH₂)₃-, -(CH₂)₄-, - (CH₂)₅-, -(CH₂)₆-, -(CH₂)₇-, -(CH₂)₈-, -NH-CH₂-, -NH-(CH₂)₂-, -NH-(CH₂)₃-, -NH-(CH₂)₄-, -NH-(CH₂)₅-, -NH-(CH₂)₆-, -NH-(CH₂)₇-, -NH-(CH₂)₈-, -CO-NH-CH₂-, -CO-NH-(CH₂)₂-, -CO-NH-(CH₂)₃-, -CO-NH-(CH₂)₄-, -CO-NH-(CH₂)₅-, -CO-NH-(CH₂)₆-, -CO-NH-(CH₂)₇-, -CO-NH-(CH₂)₈-, - CH₂-NH-, -(CH₂)₂-NH-, -(CH₂)₃-NH-, -(CH₂)₄-NH-, -(CH₂)₅-NH-, -(CH₂)₆-NH-, -(CH₂)₇-NH-, - (CH₂)₈-NH-, -NH-CH₂-NH-, -NH-(CH₂)₂-NH-, -NH-(CH₂)₃-NH-, -NH-(CH₂)₄-NH-, -NH-(CH₂)₅-NH-, -NH-(CH₂)₆-NH-, -NH-(CH₂)₇-NH-, -NH-(CH₂)₈-NH-, -CO-NH-CH₂-NH-, -CO-NH-(CH₂)₂-NH-, -CO-NH-(CH₂)₃-NH-, -CO-NH-(CH₂)₄-NH-, -CO-NH-(CH₂)₅-NH-, -CO-NH-(CH₂)₆-NH-, - CO-NH-(CH₂)₇-NH-, -CO-NH-(CH₂)₈-NH-, -(CH₂-CH₂-O)-CH₂-CH₂-, -(CH₂-CH₂-O)₂-CH₂-CH₂-, - (CH₂-CH₂-O)₃-CH₂-CH₂-, -NH-(CH₂-CH₂-O)-CH₂-CH₂-, -NH-(CH₂-CH₂-O)₂-CH₂-CH₂-, -NH-(CH₂-CH₂-O)₃-CH₂-CH₂-, -CO-NH-(CH₂-CH₂-O)-CH₂-CH₂-, -CO-NH-(CH₂-CH₂-O)₂-CH₂-CH₂-, -CO-NH-(CH₂-CH₂-O)₃-CH₂-CH₂-, -(CH₂-CH₂-O)-CH₂-CH₂-NH-, -(CH₂-CH₂-O)₂-CH₂-CH₂-NH-, -(CH₂-CH₂-O)₃-CH₂-CH₂-NH-, -NH-(CH₂-CH₂-O)-CH₂-CH₂-NH-, -NH-(CH₂-CH₂-O)₂-CH₂-CH₂-NH-, - NH-(CH₂-CH₂-O)₃-CH₂-CH₂-NH-, -CO-NH-(CH₂-CH₂-O)-CH₂-CH₂-NH-, -CO-NH-(CH₂-CH₂-O)₂-CH₂-CH₂-NH-, -CO-NH-(CH₂-CH₂-O)₃-CH₂-CH₂-NH-, -CH₂-CH₂-(O-CH₂-CH₂)-, -CH₂-CH₂-(O-CH₂-CH₂)₂-, -CH₂-CH₂-(O-CH₂-CH₂)₃-, -NH-CH₂-CH₂-(O-CH₂-CH₂)-, -NH-CH₂-CH₂-(O-CH₂-CH₂)₂-, -NH-CH₂-CH₂-(O-CH₂-CH₂)₃-, -CO-NH-CH₂-CH₂-(O-CH₂-CH₂)-, -CO-NH-CH₂-CH₂-(O-CH₂-CH₂)₂-, -CO-NH-CH₂-CH₂-(O-CH₂-CH₂)₃-, -CH₂-CH₂-(O-CH₂-CH₂)-NH-, -CH₂-CH₂-(O-CH₂-CH₂)₂-NH-, -CH₂-CH₂-(O-CH₂-CH₂)₃-NH-, -NH-CH₂-CH₂-(O-CH₂-CH₂)-NH-, -NH-CH₂-CH₂-(O-CH₂-CH₂)₂-NH-, -NH-CH₂-CH₂-(O-CH₂-CH₂)₃-NH-, -CO-NH-CH₂-CH₂-(O-CH₂-CH₂)-NH-, -CO-NH-CH₂-CH₂-(O-CH₂-CH₂)₂-NH-, -CO-NH-CH₂-CH₂-(O-CH₂-CH₂)₃-NH-,

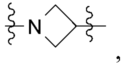

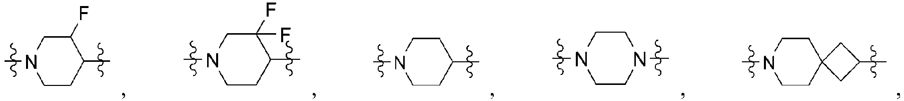

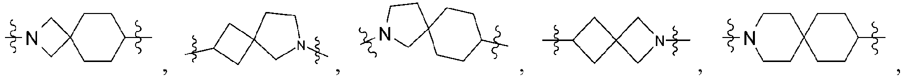

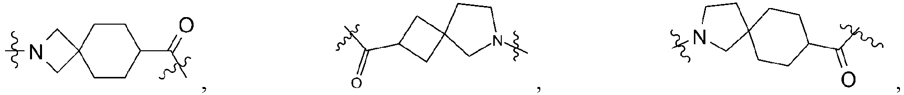

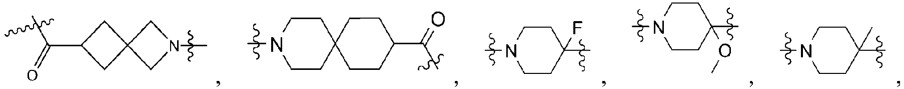

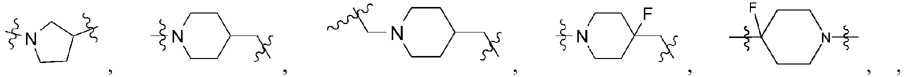

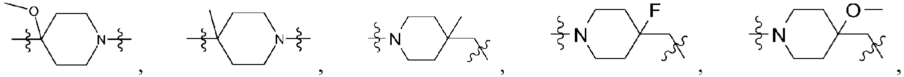

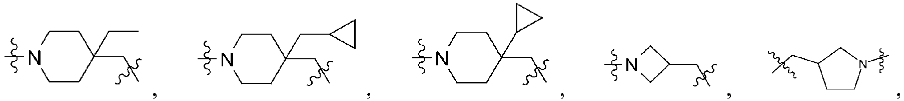

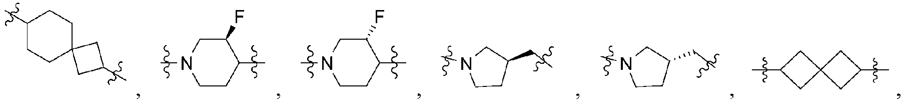

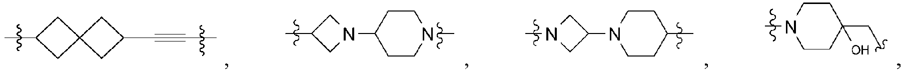

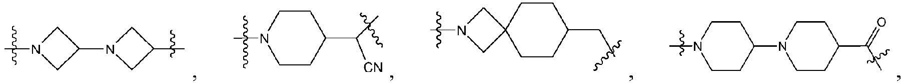

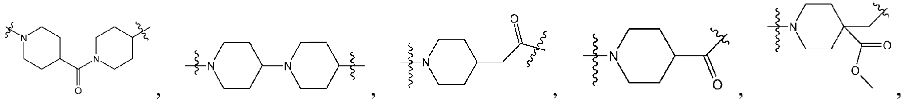

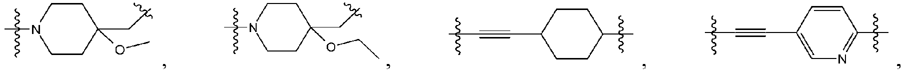

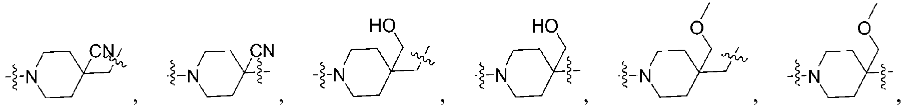

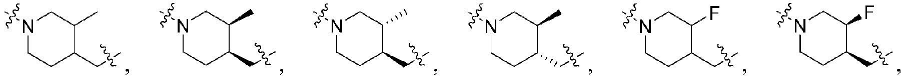

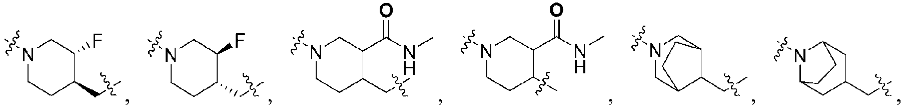

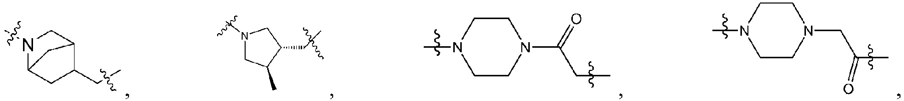

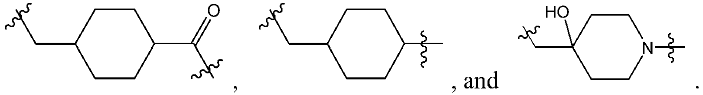

13. The compound according to any one of claims 1-12, wherein the PTM is selected from the following structures: 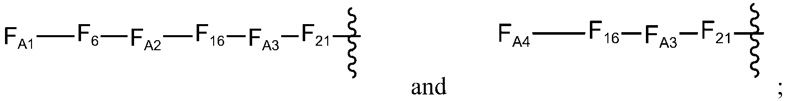
wherein each occurrence of F₆, F₁₆, and F₂₁ are each independently selected from a single bond, NH, SO, S, O, SO₂, alkylene, haloalkylene, heteroalkylene, alkyleneoxy, heteroalkyleneoxy, alkenylene, alkynylene, C(=O), OC(=O), C(=O)O, C(=O)NH, and NHC(=O), or a combination thereof; wherein the alkylene, alkyleneoxy, and alkenylene are optionally substituted with 0, 1, 2, 3, 4, 5, or 6 R_{c};
F_{A1} and F_{A4} are each independently aryl or heteroaryl; the aryl or heteroaryl is optionally substituted with 0, 1, 2, 3, 4, 5, or 6 R_{d}; preferably, F_{A4} is a 9-20 membered benzo-spiroheterocyclyl containing 1, 2, 3, 4, or 5 heteroatoms selected from N, O, and S, optionally substituted with 0, 1, 2, 3, 4, 5, or 6 R_{d};
each occurrence of F_{A3} is independently arylene or heteroarylene; the arylene or heteroarylene is optionally substituted with 0, 1, 2, 3, 4, 5, or 6 R_{c}, preferably substituted with 0, 1, 2, or 3 R_{c};
F_{A2} is cycloalkylene, spirocycloalkylene, heterocycloalkylene, or spiroheterocycloalkylene, and optionally substituted with 0, 1, 2, 3, 4, 5, or 6 R_{d};
each occurrence of R_{c} is independently selected from H, halogen, C₁₋₆ alkyl, C₁₋₆ haloalkly, C₁₋₆ heteroalkyl, C₁₋₆ alkoxy, C₁₋₆ heteroalkoxyl, C₃₋₇ cycloalkyl, C₃₋₇ heterocyclyl, C₆₋₈ aryl, C₅₋₈ heteroaryl, OH, NH₂, CN, and NO₂, preferably H, F, Cl, Br, I, CH₃, OCH₃, CF₃, OH, NH₂, CN, or NO₂;
each occurrence of R_{d} is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, oxo (=O), thioxo (=S), OH, NH₂, CN, and NO₂, preferably oxo (=O), thioxo (=S), H, F, Cl, Br, I, CH₃, OCH₃, CF₃, OH, NH₂, CN, or NO₂.

14. The compound according to claim 13, wherein:
(1) the PTM is selected from the following structures: 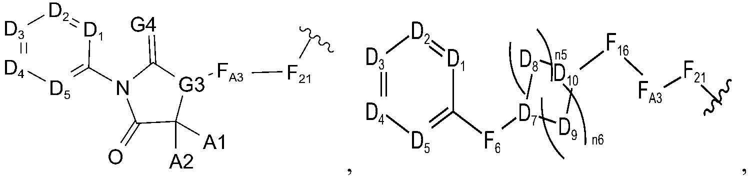 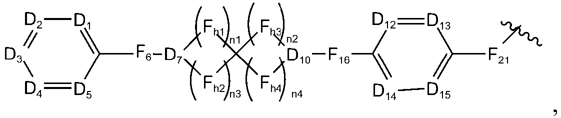 and 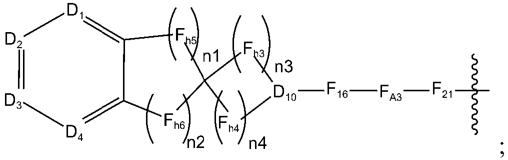 wherein:
each occurrence of F₆ and F₁₆ are each independently selected from a single bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O), wherein the C₁₋₃ alkylene, C₁₋₃ alkylene, and C₂₋₃ alkenylene are optionally substituted with 0-6 R_{c}, preferably substituted with 0-4 R_{c}, preferably substituted with 0, 1, 2, or 3 R_{c};
each occurrence of F_{A3} is independently a 6-10 membered arylene or a 5-13 membered heteroarylene containing 1, 2, 3, 4, or 5 heteroatoms selected from N, O, and/or S, the arylene or heteroarylene is optionally substituted with 0, 1, 2, 3, 4, 5, or 6 R_{c}; preferably, F_{A3} is selected from the following groups: 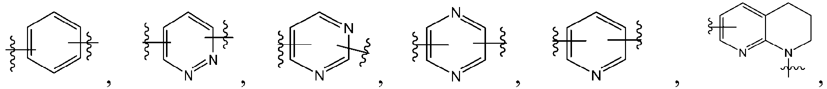 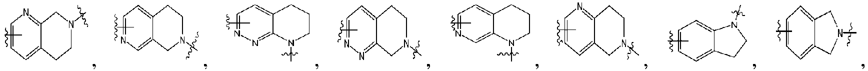 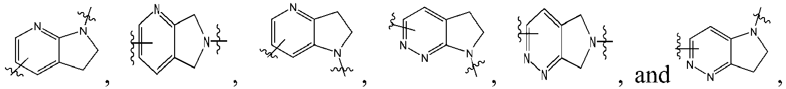 optionally substituted with 0, 1, 2, 3, 4, 5, or 6 R_{c}; wherein 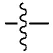 indicates a point of attachment, when the point of attachment on the F_{A3} group is not fixed, it indicates that 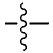 can be attached to any atom on the F_{A3} group that is capable of being connected;
G3 is selected from N and C(R_{c});
each occurrence of F₂₁ is independently selected from a single bond, NH, O, CO, C₁₋₆ alkylene, C₁₋₆ heteroalkylene, C₁₋₆ alkyleneoxy, and C₁₋₆ heteroalkyleneoxy, or a combination thereof, preferably selected from a single bond, NH, O, CO, C₁₋₆ alkylene, -C₁₋₆ alkylene-NH-C₁₋₆ alkylene-, -C₁₋₆ alkylene-O-C₁₋₆ alkylene-, -C₁₋₆ alkylene -C(O)-C₁₋₆ alkylene, -C₁₋₆ alkylene-O-C(O)-C₁₋₆ alkylene, - C₁₋₆ alkylene-C(O)-O-C₁₋₆ alkylene, -C₁₋₆ alkylene-NH-, -C₁₋₆ alkylene-O-, -C₁₋₆ alklyene-C(O)-, -C₁₋₆ alkylene-C(O)-O-, -C₁₋₆ alkylene-O-C(O)-, -NH-C₁₋₆ alkylene-, -O-C₁₋₆ alkylene-, -C(O)-C₁₋₆ alkylene, -C(O)-O-C₁₋₆ alkylene, -O-C(O)-C₁₋₆ alkylene, -NH- C₁₋₆ alkylene-NH-, -O-C₁₋₆ alkylene-O-, and -C(O)-C₁₋₆ alkylene-C(O)-, wherein the C₁₋₆ alkylene, C₁₋₆ heteroalkylene, C₁₋₆ alkyleneoxy, and C₁₋₆ heteroalkyleneoxy is optionally substituted with 0, 1, 2, 3, 4, 5, or 6 R_{c};
each occurrence of A1 and A2 are independently selected from H, C₁₋₆ alkyl, halogen, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, or each occurrence of A1 and A2 together with the carbon atom to which they are attached form a C₄₋₈ cycloalkyl or C₄₋₈ heteroalkyl, wherein the C₁₋₆ alkyl, C₁₋₆ haloalkyl, C₁₋₆ heteroalkyl, C₄₋₈ cycloalkyl, and C₄₋₈ heterocyclyl are optionally substituted with 0, 1, 2, 3, 4, 5, or 6 R_{c}; preferably, each occurrence of A1 and A2 is independently CH₃, or each occurrence of A1 and A2 together with the carbon atom to which they are attached form a C₄₋₆ cycloalkyl or heterocyclyl;
G4 is selected from O and S;
each occurrence of D₁, D₂, D₃, D₄, D₅, D₁₂, D₁₃, D₁₄, D₁₃, D₇, and D₁₀ is independently selected from CR_{c} and N;
each occurrence of D₈ and D₉ are independently selected from C(R_{d})₂ and NR_{d};
each occurrence of n5 and n6 are independently selected from 0, 1, 2, and 3, and n5 and n6 are not simultaneously 0; preferably, n5 and n6 are selected from 1 and 2;
each occurrence of Fₕ₁, Fₕ₂, Fₕ₃, and Fₕ₄ are independently selected from C(R_{c})₂ and NR_{c};
each occurrence of Fₕ₅ and Fₕ₆ are independently selected from C(R_{c})₂, CO, CS, O, S, and NR_{c};
each occurrence of n1, n2, n3, and n4 are independently selected from 1, 2, 3, 4, and 5; preferably, each occurrence of n1, n2, n3, and n4 are independently selected from 1 and 2;
each occurrence of R_{c} is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably R_{c} is H, F, Cl, Br, I, CH₃, OCH₃, CF₃, OH, NH₂, CN, or NO₂;
each occurrence of R_{d} is independently selected from H, halogen, C₁₋₆ alkyl, C₁₋₆ haloalkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, oxo (=O), thioxo (=S), OH, NH₂, CN, and NO₂, preferably R_{d} is oxo (=O), thioxo (=S), H, F, Cl, Br, I, CH₃, CF₃, OCH₃, OH, NH₂, CN, or NO₂;
preferably, 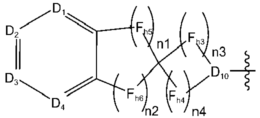 is selected from the following groups: 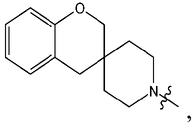 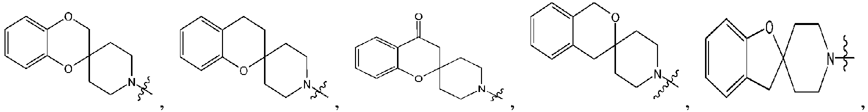 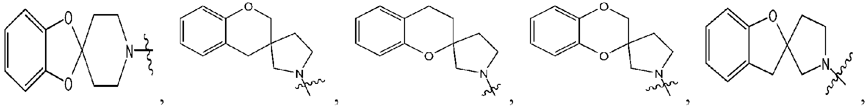 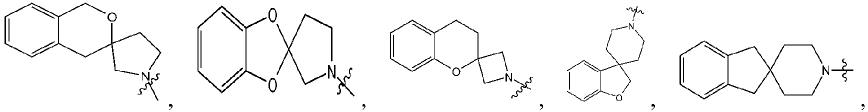 and 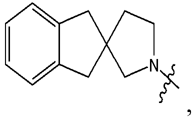 optionally substituted with 0, 1, 2, 3, or 4 R_{d}, wherein 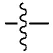 indicates a point of attachment;
preferably, 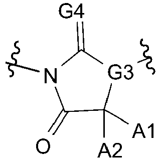 is selected from 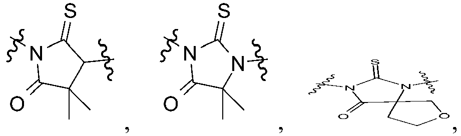 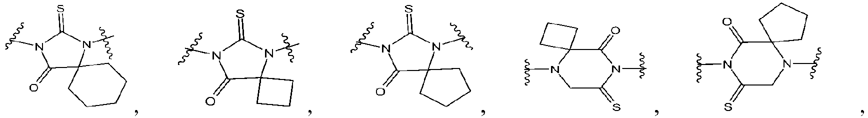 and 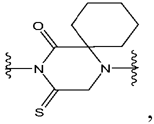 optionally substituted with 0-3 R_{d}; 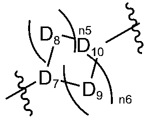 is selected from 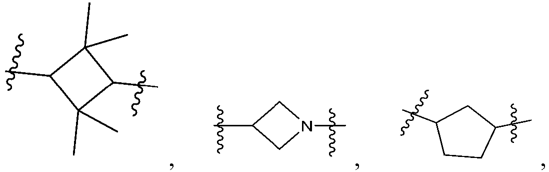 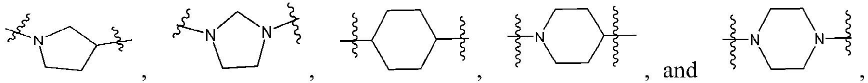 optionally substituted with 0-3 R_{d}; 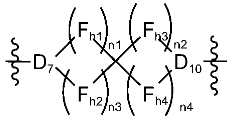 is selected from 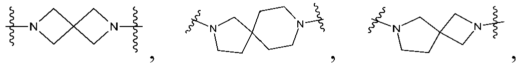 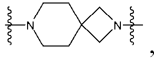 and 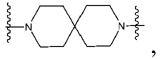 optionally substituted with 0-3 R_{d}; wherein 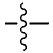 indicates a point of attachment;
or,
(2) the PTM is selected from the following structures: 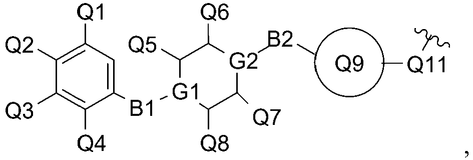 preferably, the PTM is selected from the following structures: 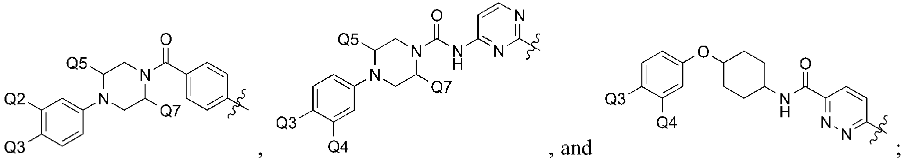 wherein:
B1 and B2 are independently selected from a covalent bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O), wherein the C₁₋₃ alkylene, C₁₋₃ alkyleneoxy, and C₂₋₃ alkenylene are optionally substituted with 0-6 Q10, preferably substituted with 0-4 Q10, preferably substituted with 0, 1, 2, or 3 Q10;
G1 and G2 are independently selected from N and C(Q10);
Q9 is selected from aryl and heteroaryl, wherein the aryl and heteroaryl are optionally substituted with 0-4 Q10;
Q11 is selected from one or more of a covalent bond, NH, O, CO, C₁₋₆ alkylene, C₁₋₆ heteroalkylene, C₁₋₆ alkyleneoxy, and C₁₋₆ heteroalkyleneoxy, preferably selected from a covalent bond, NH, O, CO, C₁₋₆ alkylene, -C₁₋₆ alkylene-NH- C₁₋₆ alkylene-, -C₁₋₆ alkylene-O-C₁₋₆ alkylene-, - C₁₋₆ alkylene-C(O)-C₁₋₆ alkylene, -C₁₋₆ alkylene-O-C(O)-C₁₋₆ alkylene, -C₁₋₆ alkylene-C(O)-O-C₁₋₆ alkylene, -C₁₋₆ alkylene-NH-, -C₁₋₆ alkylene-O-, -C₁₋₆ alkylene-C(O)-, -C₁₋₆ alkylene-C(O)-O-, -C₁₋₆ alkylene-O-C(O)-, -NH-C₁₋₆ alkylene-, -O-C₁₋₆ alkylene-, -C(O)-C₁₋₆ alkylene, -C(O)-O-C₁₋₆ alkylene, -O-C(O)-C₁₋₆ alkylene, -NH-C₁₋₆ alkylene -NH-, -O-C₁₋₆ alkylene -O-, and -C(O)-C₁₋₆ alkylene -C(O)-, wherein the C₁₋₆ alkylene, C₁₋₆ heteroalkylene, C₁₋₆ alkyleneoxy, and C₁₋₆ heteroalkyleneoxy are optionally substituted with 0-6 Q10, preferably substituted with 0, 1, 2, 3, 4, or 5 Q10;
Q1, Q2, Q3, Q4, Q5, Q6, Q7, Q8, and Q10 are independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably H, F, Cl, Br, I, CH₃, CF₃, OH, NH₂, CN, or NO₂;
preferably, the PTM is selected from the following structures: 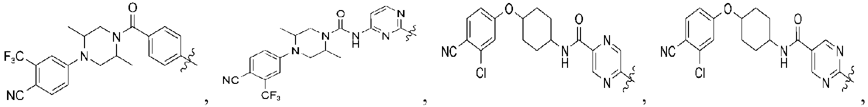 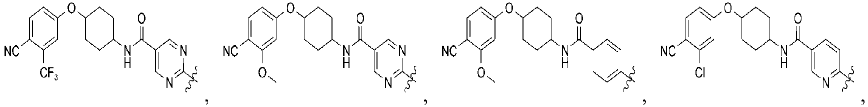 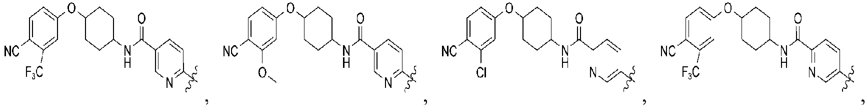 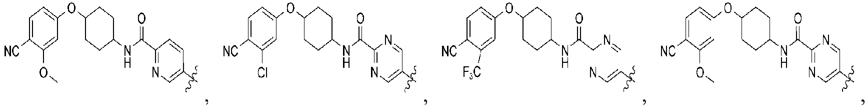 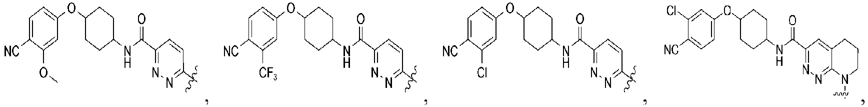 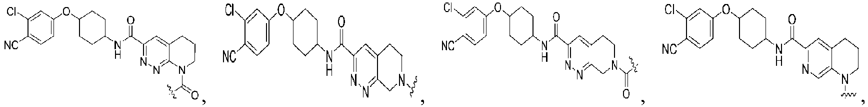 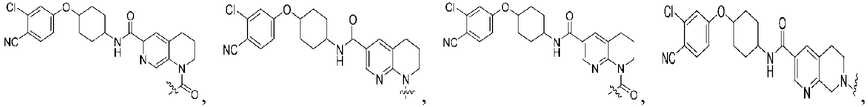 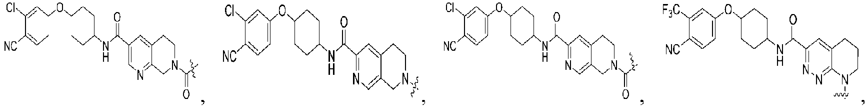 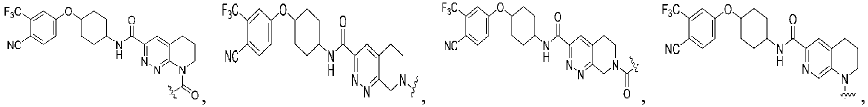 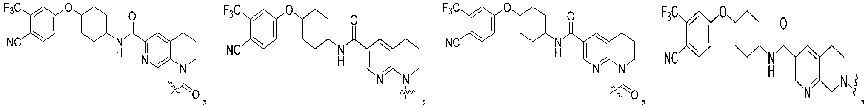 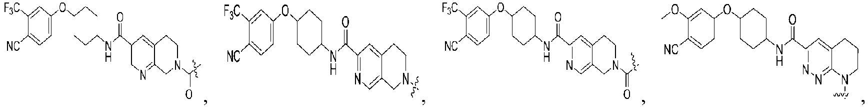 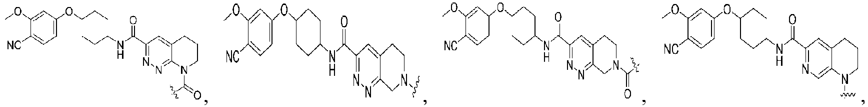 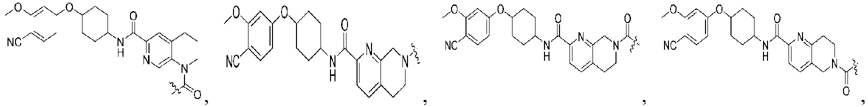 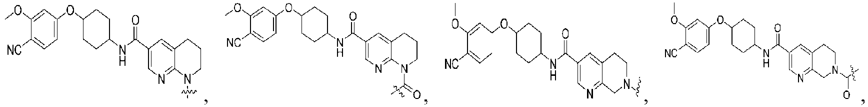 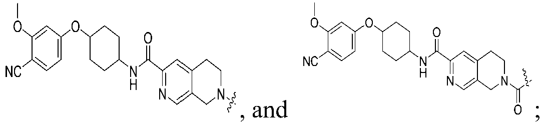
preferably, the PTM is selected from the following structures: 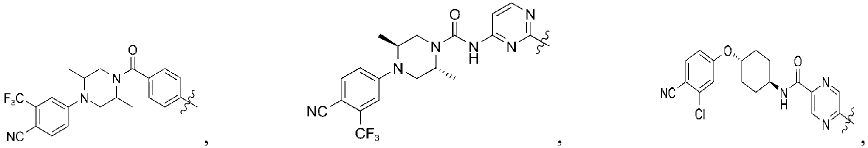 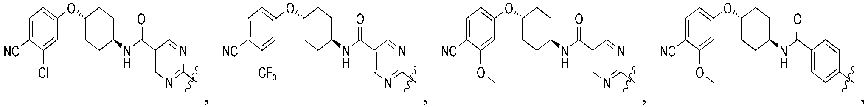 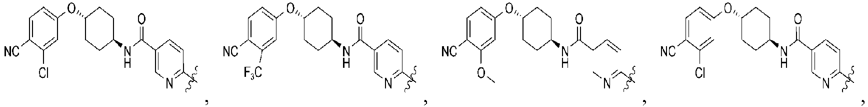 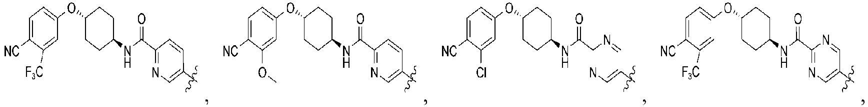 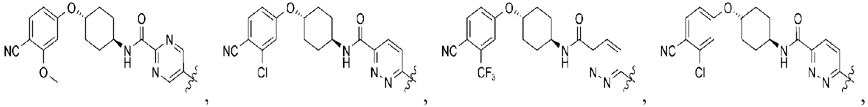 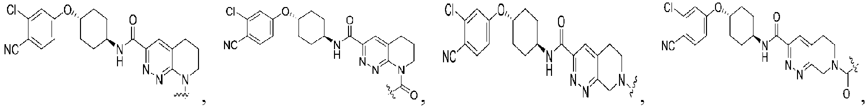 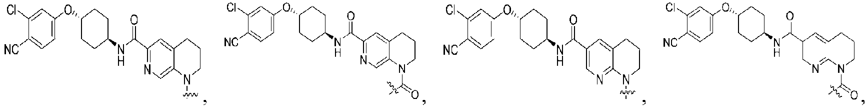 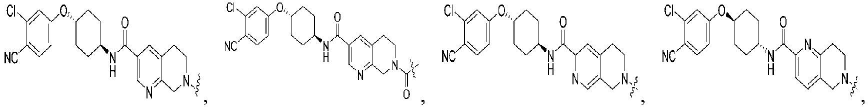 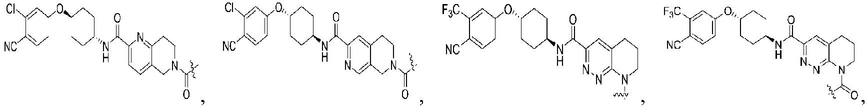 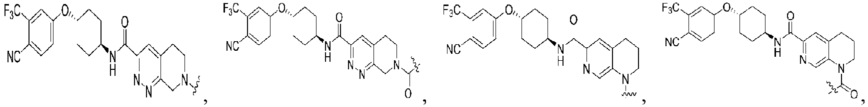 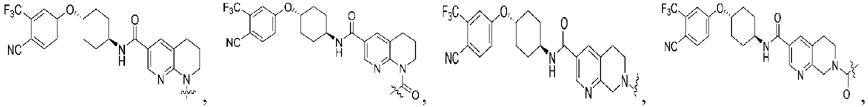 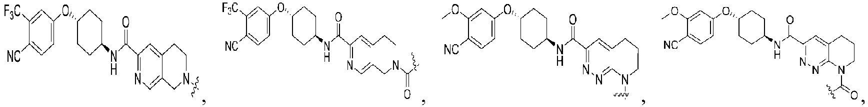 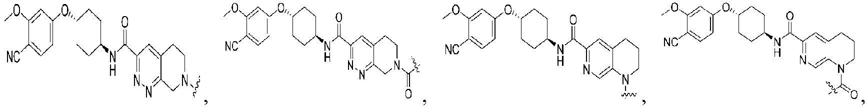 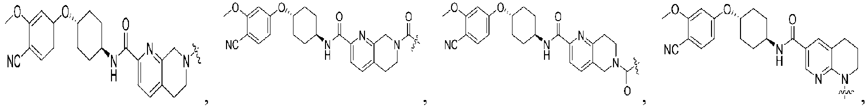 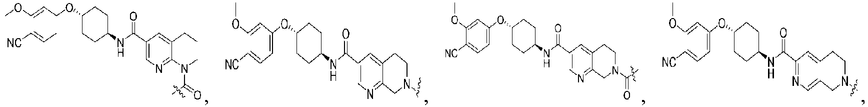 and 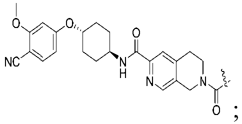 or,
(3) the PTM is selected from the following structures: 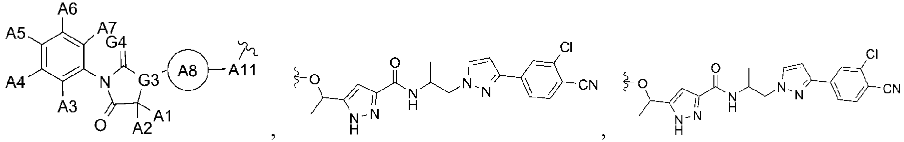 and 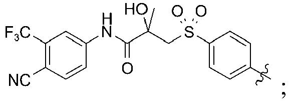 preferably, the PTM is selected from the following structures: 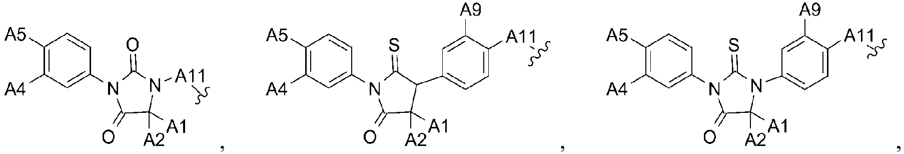 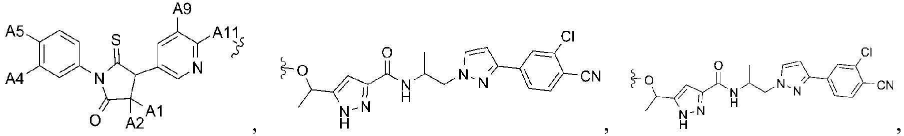 and 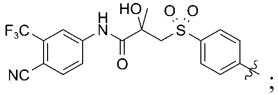 wherein:
A1 and A2 are independently selected from C₁₋₆ alkyl, C₁₋₆ heteroalkyl, and C₄₋₈ cycloalkyl or C₄₋₈ heterocyclyl formed together with the carbon atom or heteroatom to which they are attached, wherein the C₁₋₆ alkyl, C₁₋₆ heteroalkyl, C₄₋₈ cycloalkyl, and C₄₋₈ heterocyclyl are optionally substituted with 0-6 A9, preferably substituted with 0, 1, 2, 3, 4, or 5 A9;
A8 is selected from a covalent bond, aryl, and heteroaryl, wherein the aryl and heteroaryl are optionally substituted with 0-4 A9;
A11 is selected from one or more of a covalent bond, NH, O, CO, C₁₋₆ alkylene, C₁₋₆ heteroalkylene, C₁₋₆ alkyleneoxy and C₁₋₆ heteroalkyleneoxy, preferably selected from a covalent bond, NH, O, CO, C₁₋₆ alkylene, -C₁₋₆ alkylene -NH- C₁₋₆ alkylene -, -C₁₋₆ alkylene -O-C₁₋₆ alkylene -, -C₁₋₆ alkylene -C(O)- C₁₋₆ alkylene, -C₁₋₆ alkylene -O-C(O)-C₁₋₆ alkylene, -C₁₋₆ alkylene -C(O)-O-C₁₋₆ alkylene, -C₁₋₆ alkylene -NH-, -C₁₋₆ alkylene -O-, -C₁₋₆ alkylene -C(O)-, -C₁₋₆ alkylene -C(O)-O-, -C₁₋₆ alkylene -O-C(O)-, -NH-C₁₋₆ alkylene -, -O-C₁₋₆ alkylene -, -C(O)-C₁₋₆ alkylene, -C(O)-O- C₁₋₆ alkylene, -O-C(O)- C₁₋₆ alkylene, -NH- C₁₋₆ alkylene -NH-, -O-C₁₋₆ alkylene -O-, and -C(O)-C₁₋₆ alkylene -C(O)-, wherein the C₁₋₆ alkylene, C₁₋₆ heteroalkylene, C₁₋₆ alkyleneoxy, and C₁₋₆ heteroalkyleneoxy are optionally substituted with 0-6 A9, preferably substituted with 0, 1, 2, 3, 4, or 5 A9;
G3 is selected from N and C(A10);
G4 is selected from O and S; and
each occurrence of A3, A4, A5, A6, A7, A9, and A10 is independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably H, F, Cl, Br, I, CH₃, CF₃, OH, NH₂, CN, or NO₂;
preferably, the PTM is selected from the following structures: 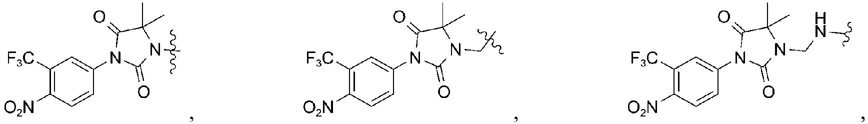 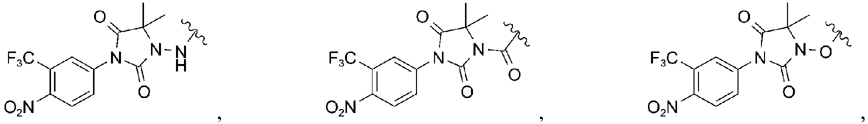 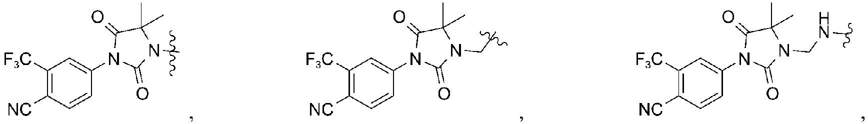 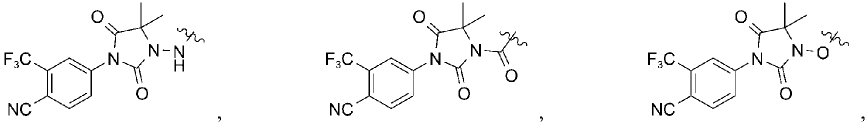 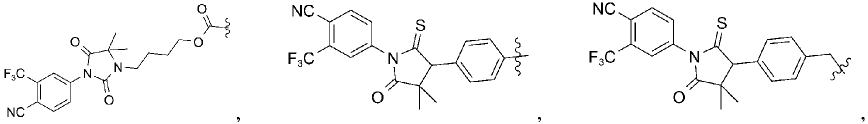 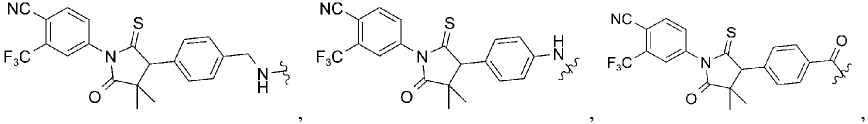 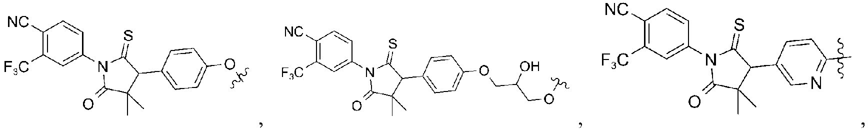 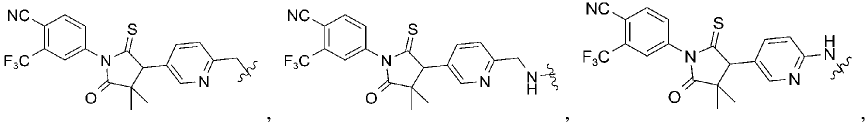 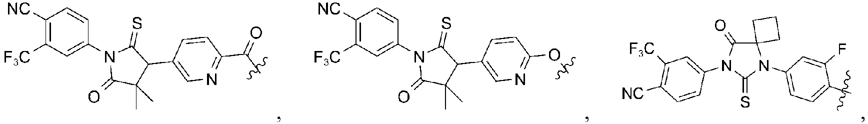 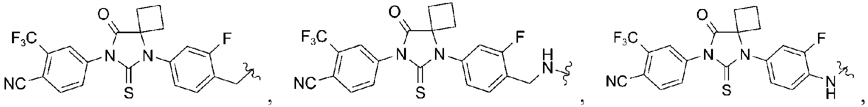 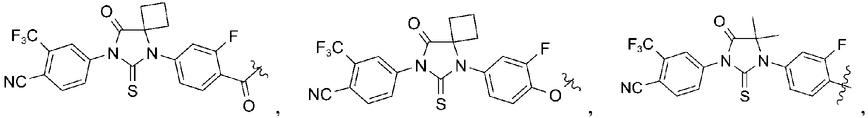 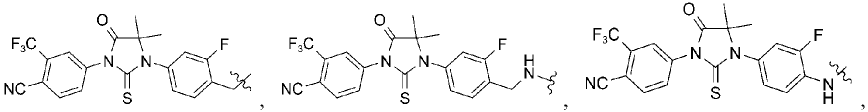 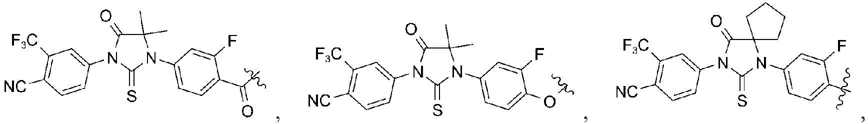 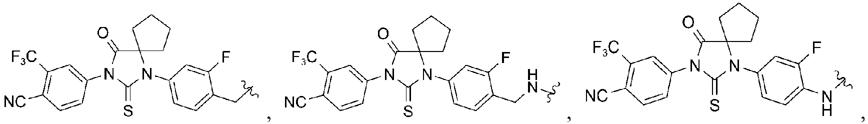 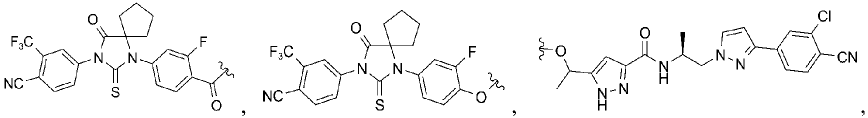 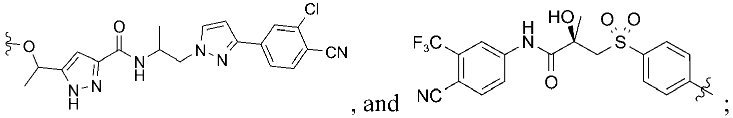
preferably, the PTM is selected from the following structures: 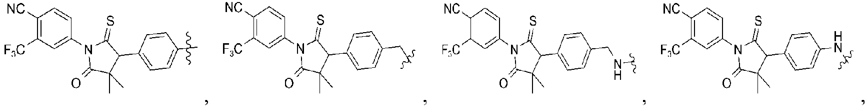 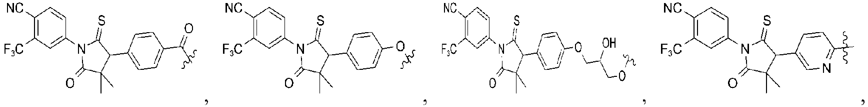 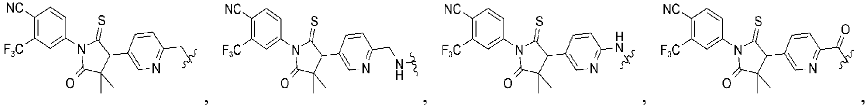 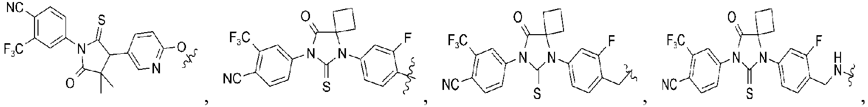 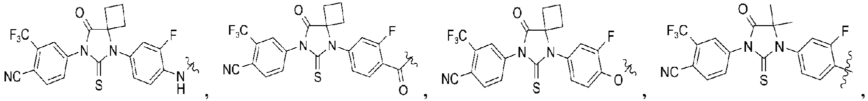 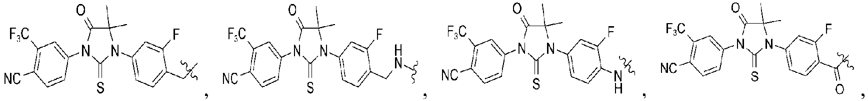 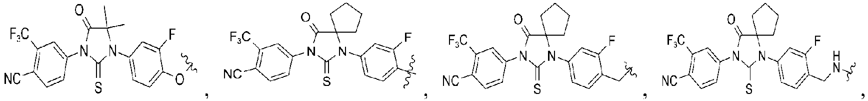 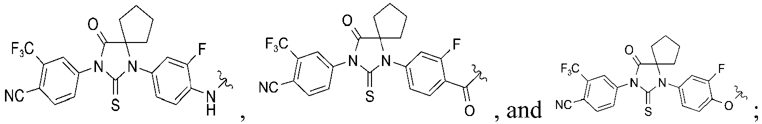 or,
(4) the PTM is selected from the following structures: 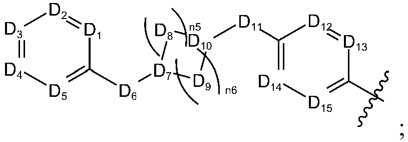 preferably, the PTM is selected from the following structures: 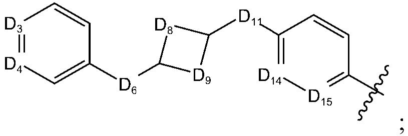
wherein, D₆ and D₁₁ are independently selected from a single bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O); wherein the C₁₋₃ alkylene, C₁₋₃ alkyleneoxy, and C₂₋₃ alkenylene are optionally substituted with 0-6 Rₐₐ, preferably substituted with 0-4 Rₐₐ, preferably substituted with 0, 1, 2, or 3 Rₐₐ;
each occurrence of D₁, D₂, D₃, D₄, D₅, D₁₂, D₁₄, D₁₅, D₇, and D₁₀ are independently selected from CRₐₐ and N;
each occurrence of D₈ and D₉ are independently selected from C(Rₐₐ)₂ and NRₐₐ;
n5 and n6 are independently selected from 1, 2, and 3, preferably selected from 1 and 2;
each occurrence of Rₐₐ is independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably independently selected from H, F, Cl, Br, I, CH₃, CF₃, OH, NH₂, CN, and NO₂; preferably independently selected from H, F, Cl, Br, I, CH₃, CF₃, OH, NH₂, CN, OCH₃, and NO₂;
preferably, the PTM is selected from the following structures: 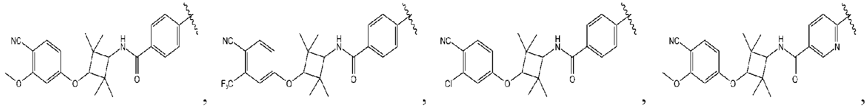 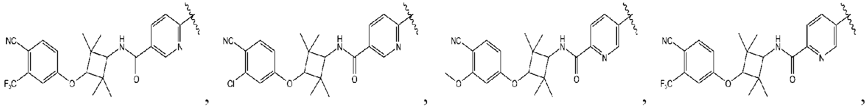 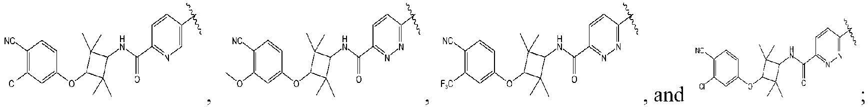
preferably, the PTM is selected from the following structures: 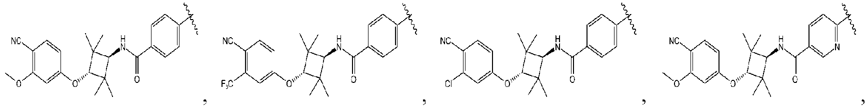 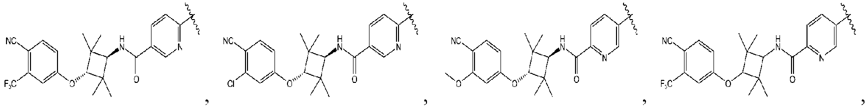 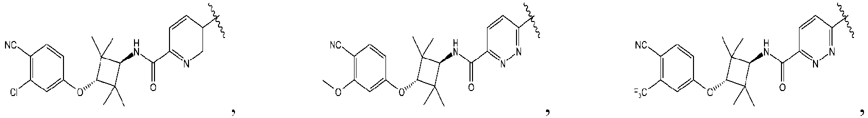 and 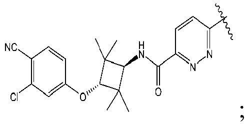 or,
(5) the PTM is selected from the following structures: 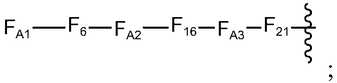 preferably, the PTM is selected from the following structures: 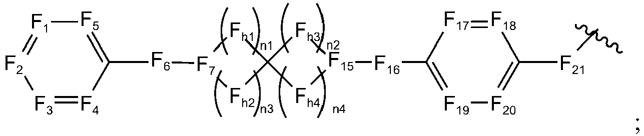 preferably, the PTM is selected from the following structures: 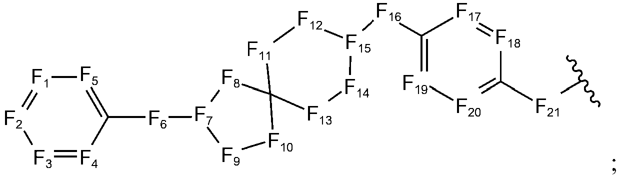 preferably, the PTM is selected from the following structures: 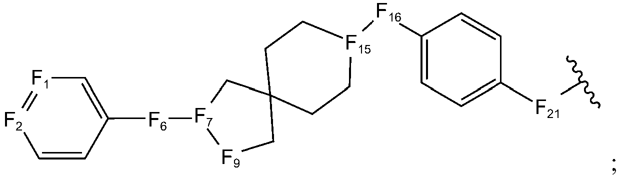
wherein F₆, F₁₆, and F₂₁ are independently selected from a single bond, NH, O, SO₂, C₁₋₃ alkylene, C₁₋₃ alkyleneoxy, C₂₋₃ alkenylene, C₂₋₃ alkynylene, C(=O), OC(=O), C(=O)O, C(=O)NH, and NHC(=O); wherein the C₁₋₃ alkylene, C₁₋₃ alkyleneoxy, and C₂₋₃ alkenylene are optionally substituted with 0-6 R_{c}, preferably substituted with 0-4 R_{c}, preferably substituted with 0, 1, 2, or 3 R_{c};
F_{A1} and F_{A3} are independently a 6-10 membered aryl ring or a 5-8 membered heteroaryl ring; the aryl ring or heteroaryl ring is optionally substituted with 0-6 R_{c}, preferably substituted with 0-4 R_{c}, preferably substituted with 0, 1, 2, or 3 R_{c};
F_{A2} is a 7-13 membered spirocycle or spiroheterocycle containing 0, 1, 2, 3, or 4 nitrogen atoms, and is optionally substituted with 0-6 R_{c}, preferably substituted with 0, 1, 2, or 3 R_{c};
each occurrence of R_{c} is independently selected from H, halogen, C₁₋₆ alkyl, C₁₋₆ heteroalkyl, C₁₋₆ alkoxy, C₁₋₆ heteroalkoxy, C₃₋₇ cycloalkyl, C₃₋₇ heterocyclyl, C₆₋₈ aryl, C₅₋₈ heteroaryl, OH, NH₂, CN, and NO₂, preferably selected from H, F, Cl, Br, I, CH₃, CF₃, OH, NH₂, CN, and NO₂;
wherein:
each occurrence of F₁, F₂, F₃, F₄, F₃, F₇, F₁₅, F₁₇, F₁₈, F₁₉, and F₂₀ are independently selected from CR_{c} and N;
each occurrence of F₇ and F₁₅ are independently selected from CR_{c} and N;
each occurrence of Fₕ₁, Fₕ₂, Fₕ₃, and Fₕ₄ are independently selected from C(R_{c})₂ and NR_{c};
each occurrence of n1, n2, n3, and n4 are independently selected from 1, 2, 3, 4 and 5; preferably, each occurrence of n1, n2, n3, and n4 are independently selected from 1 and 2;
wherein,
each occurrence of F₈, F₉, F₁₀, F₁₁, F₁₂, F₁₃, and F₁₄ are independently selected from C(R_{c})₂ and NR_{c};
preferably, the PTM is the following structures: 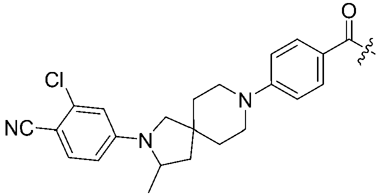 or

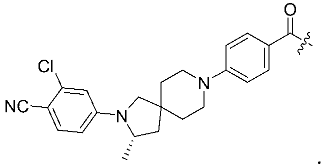

15. The compound according to claim 13 or 14, wherein the compound of formula I is selected from the following structures:
| PTM- | -L- | -CLM |
|---|---|---|
| 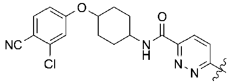 | 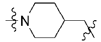 | 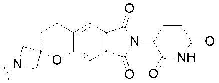 |
| 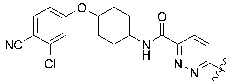 | 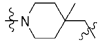 | 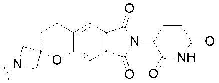 |
| 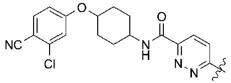 | 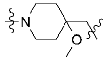 | 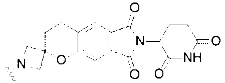 |
| 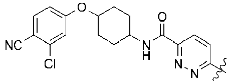 | 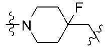 | 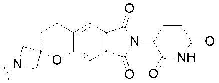 |
| 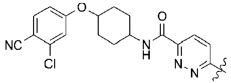 | 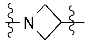 | 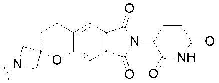 |
| 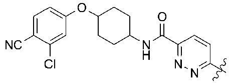 | - | 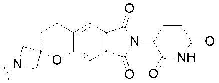 |
| 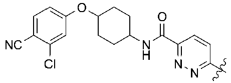 | 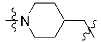 | 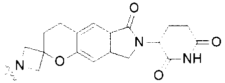 |
| 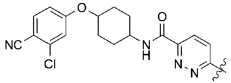 | 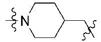 | 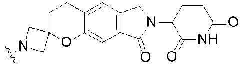 |
| 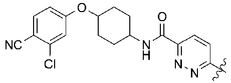 | 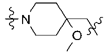 | 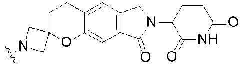 |
| 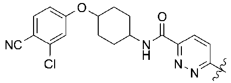 | 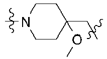 | 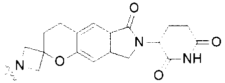 |
| 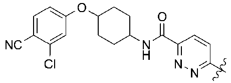 | 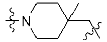 | 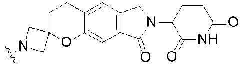 |
| 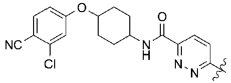 | 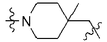 | 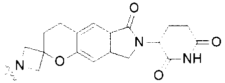 |
| 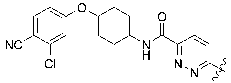 | 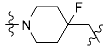 | 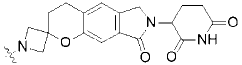 |
| 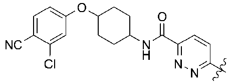 | 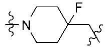 | 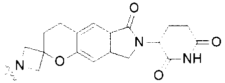 |
| 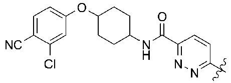 | 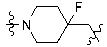 | 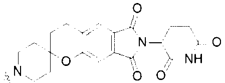 |
| 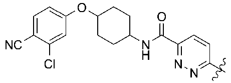 | 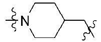 | 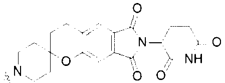 |
| 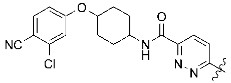 | 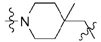 | 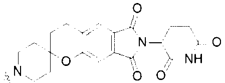 |
| 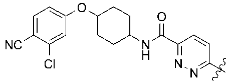 | 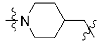 | 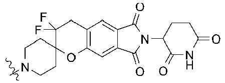 |
| 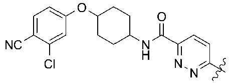 | 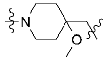 | 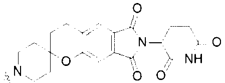 |
| 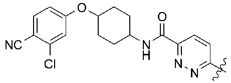 | 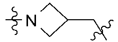 | 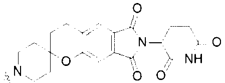 |
| 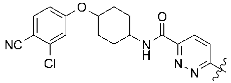 | 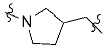 | 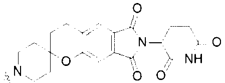 |
| 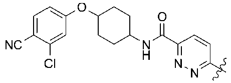 | 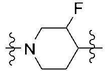 | 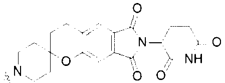 |
| 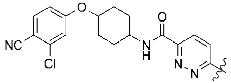 | 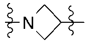 | 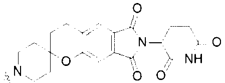 |
| 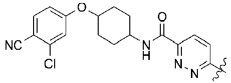 | 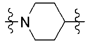 | 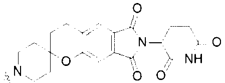 |
| 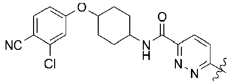 | 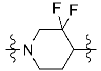 | 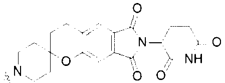 |
| 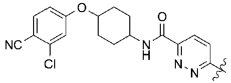 | - | 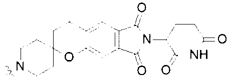 |
| 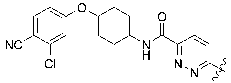 | 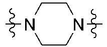 | 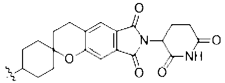 |
| 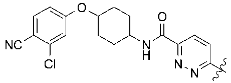 | 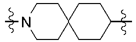 | 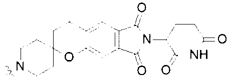 |
| 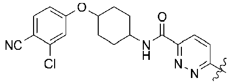 | 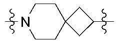 | 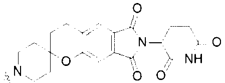 |
| 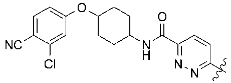 | 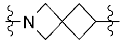 | 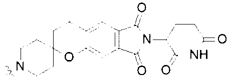 |
| 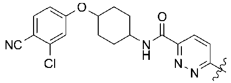 | 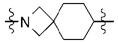 | 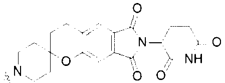 |
| 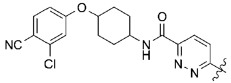 | 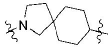 | 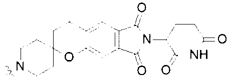 |
| 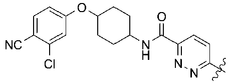 | 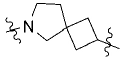 | 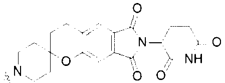 |
| 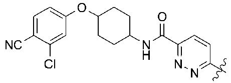 | - | 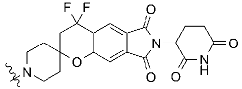 |
| 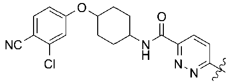 | 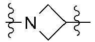 | 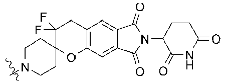 |
| 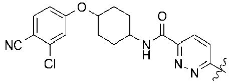 | 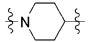 | 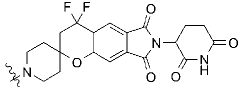 |
| 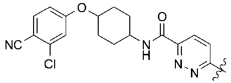 | 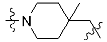 | 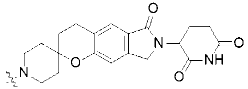 |
| 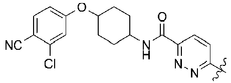 | 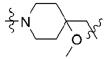 | 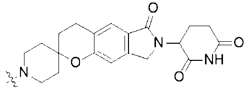 |
| 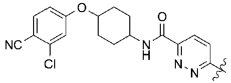 | 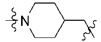 | 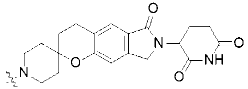 |
| 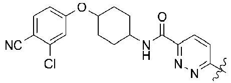 | 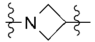 | 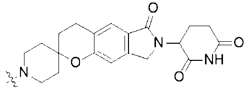 |
| 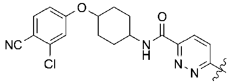 | 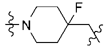 | 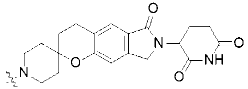 |
| 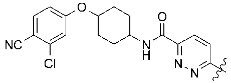 | 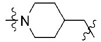 | 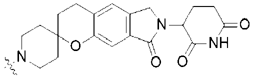 |
| 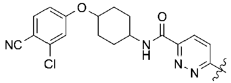 | 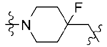 | 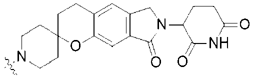 |
| 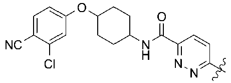 | 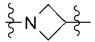 | 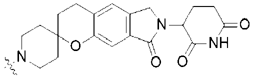 |
| 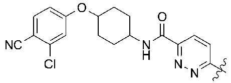 | 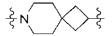 | 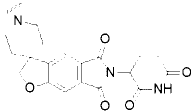 |
| 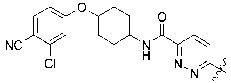 | 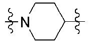 | 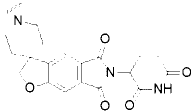 |
| 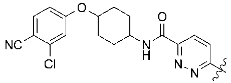 | 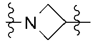 | 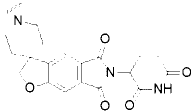 |
| 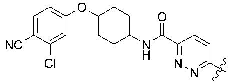 | 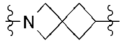 | 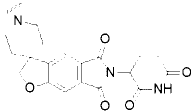 |
| 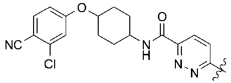 | 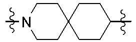 | 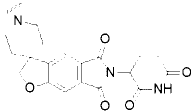 |
| 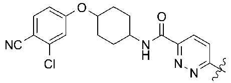 | - | 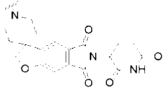 |
| 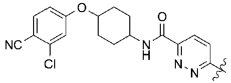 | 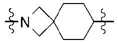 | 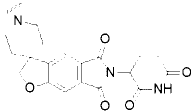 |
| 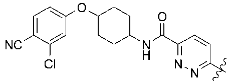 | 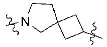 | 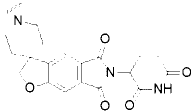 |
| 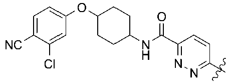 | 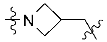 | 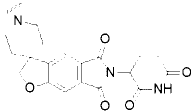 |
| 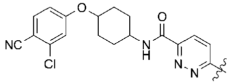 | 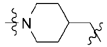 | 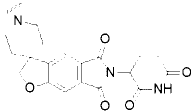 |
| 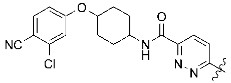 | 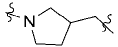 | 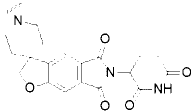 |
| 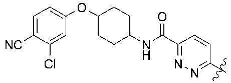 | 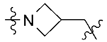 | 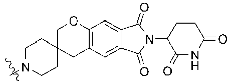 |
| 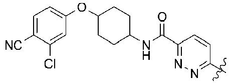 | - | 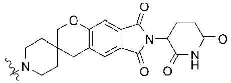 |
| 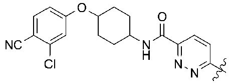 | 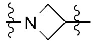 | 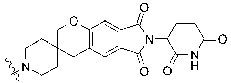 |
| 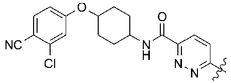 | 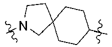 | 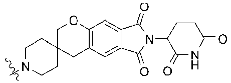 |
| 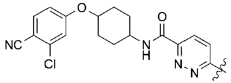 | 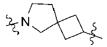 | 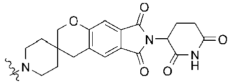 |
| 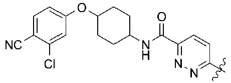 | 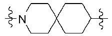 | 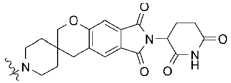 |
| 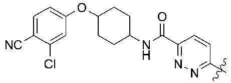 | 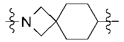 | 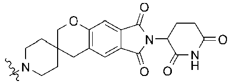 |
| 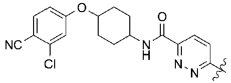 | 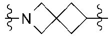 | 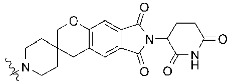 |
| 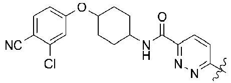 | 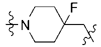 | 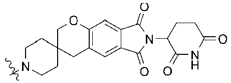 |
| 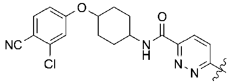 | 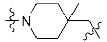 | 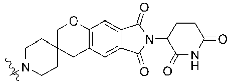 |
| 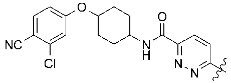 | 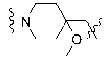 | 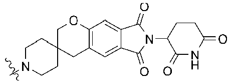 |
| 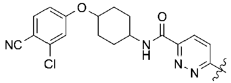 | 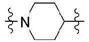 | 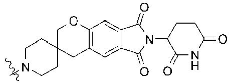 |
| 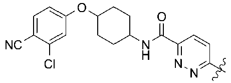 | 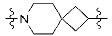 | 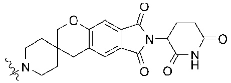 |
| 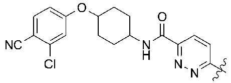 | 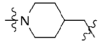 | 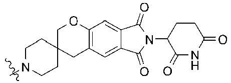 |
| 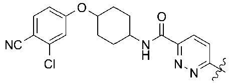 | 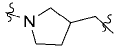 | 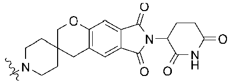 |
| 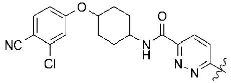 | - | 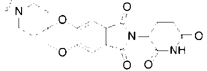 |
| 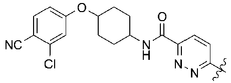 | 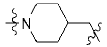 | 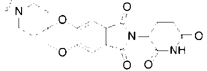 |
| 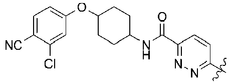 | 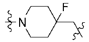 | 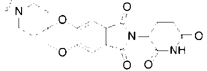 |
| 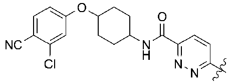 | 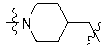 | 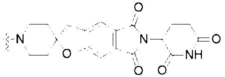 |
| 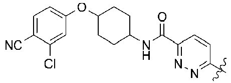 | 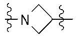 | 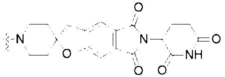 |
| 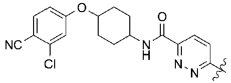 | 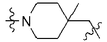 | 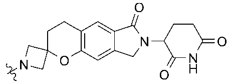 |
| 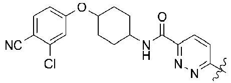 | 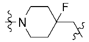 | 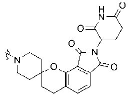 |
| 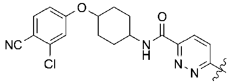 | 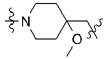 | 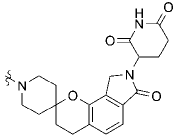 |
| 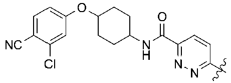 | 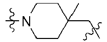 | 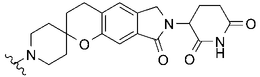 |
| 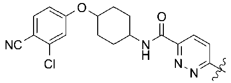 | 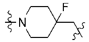 | 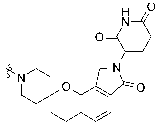 |
| 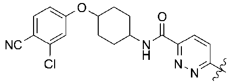 | 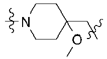 | 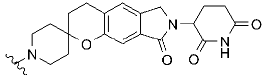 |
| 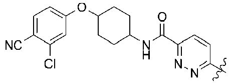 | 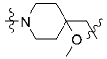 | 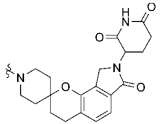 |
| 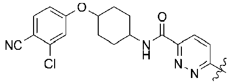 | 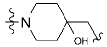 | 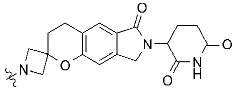 |
| 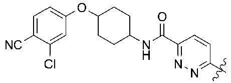 | 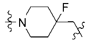 | 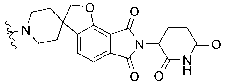 |

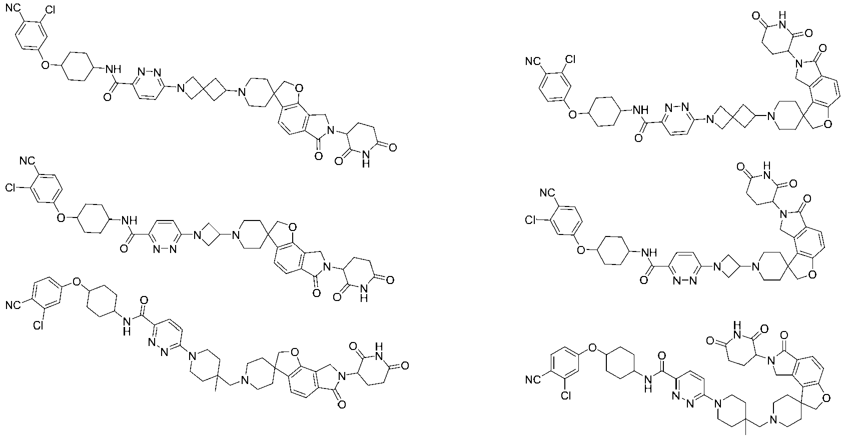

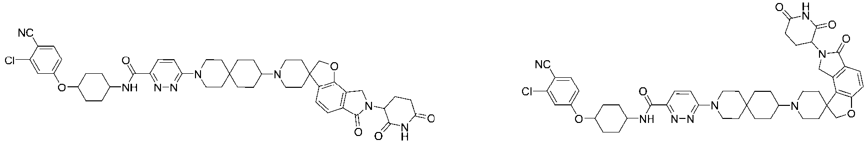

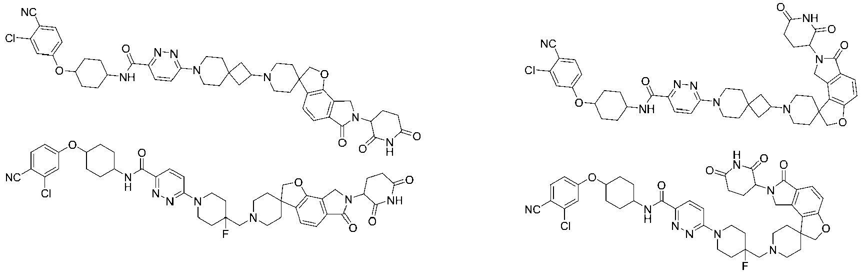

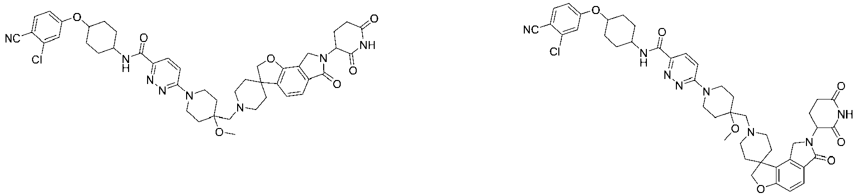

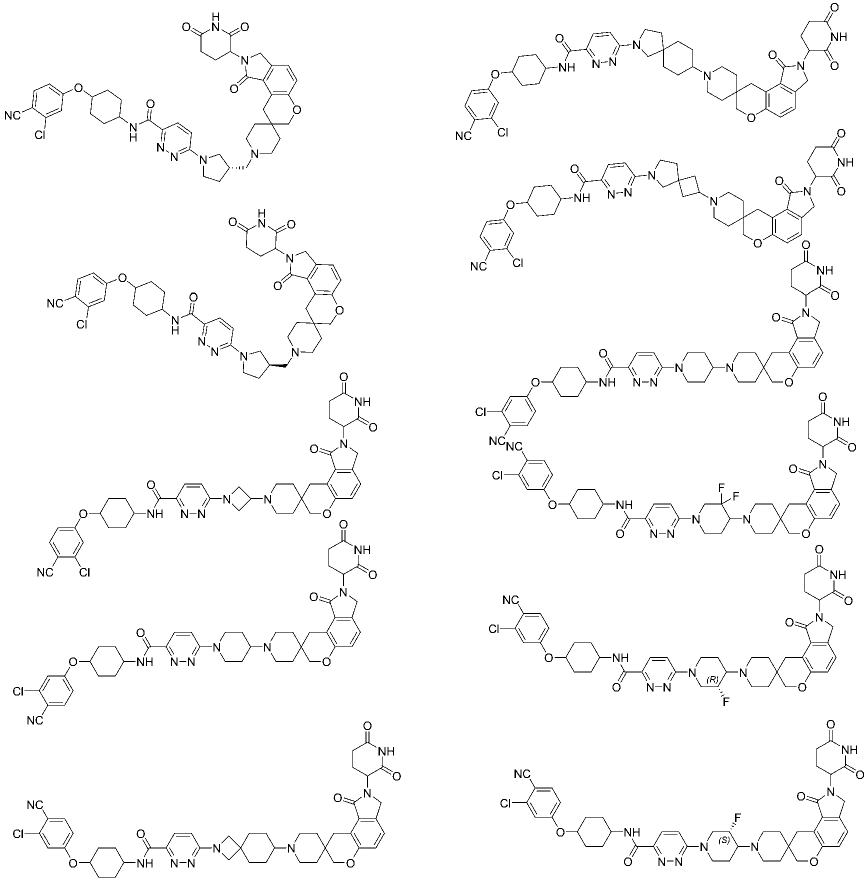

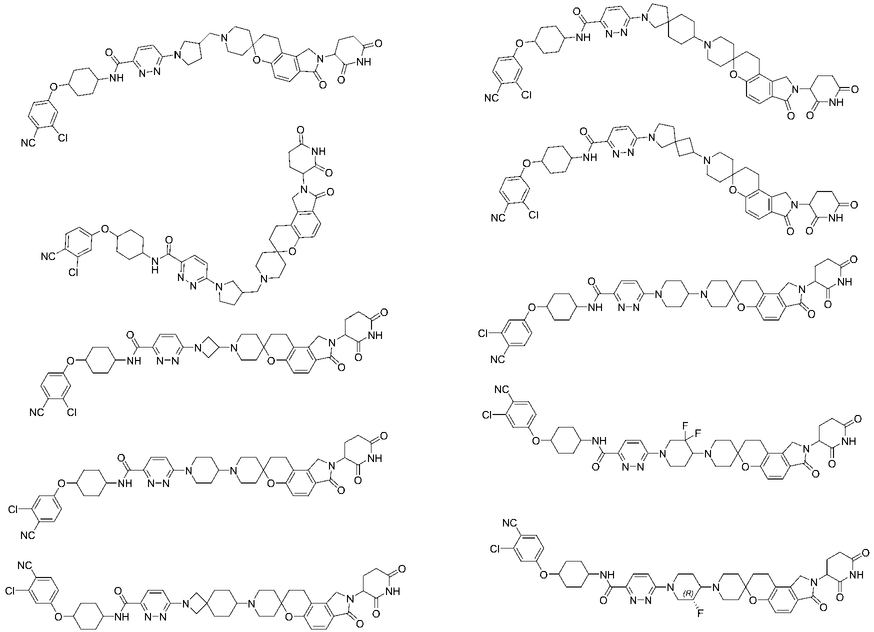

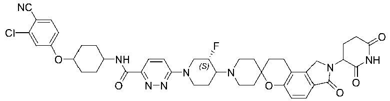

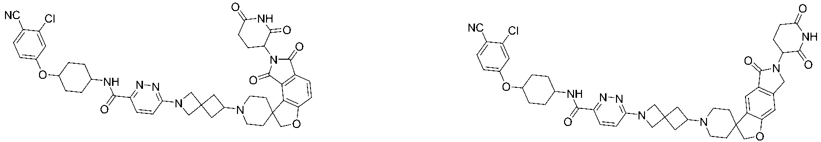

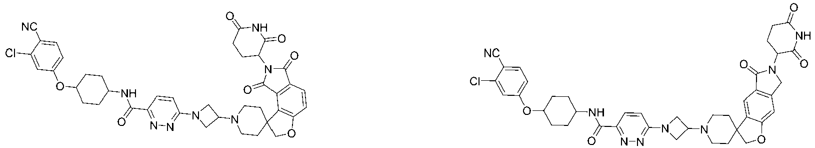

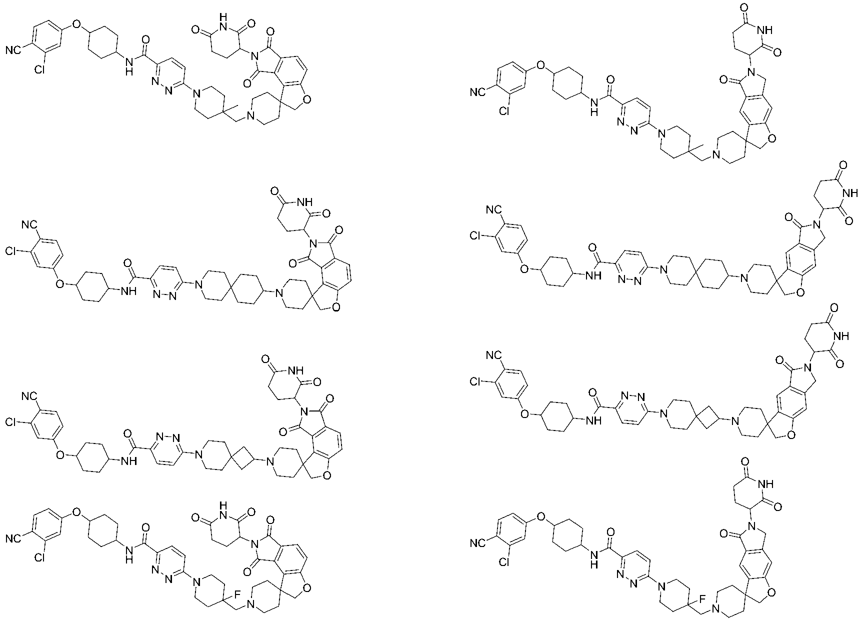

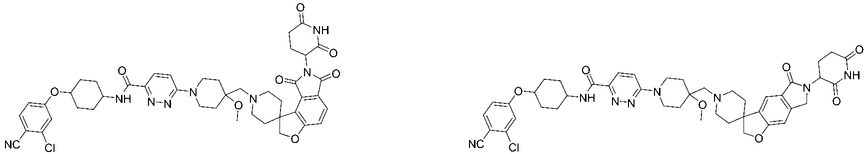

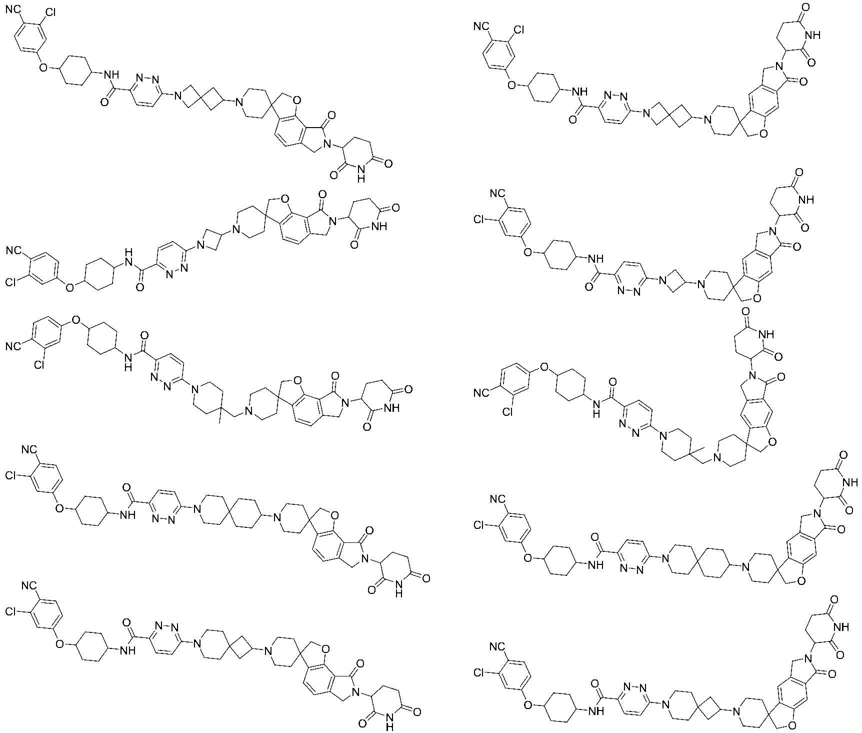

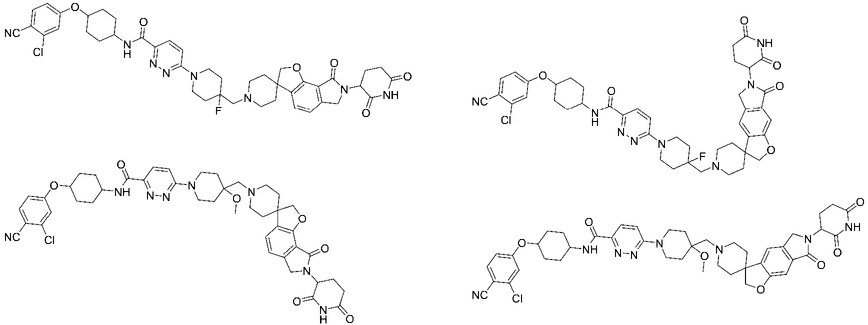

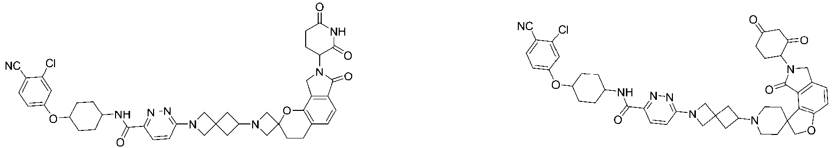

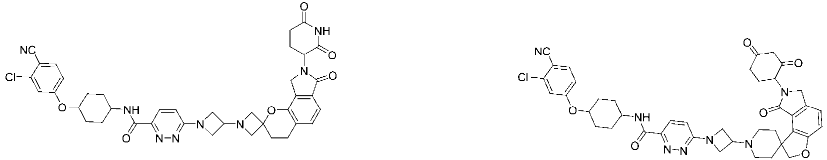

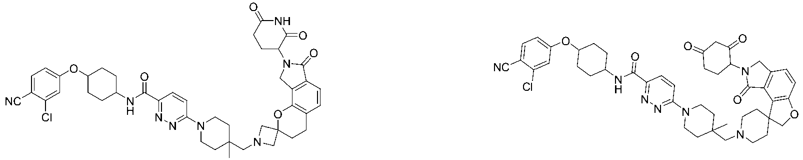

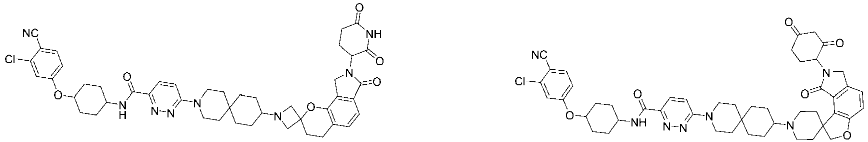

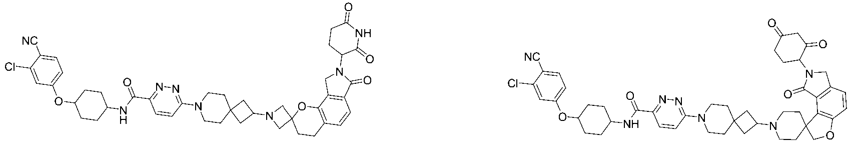

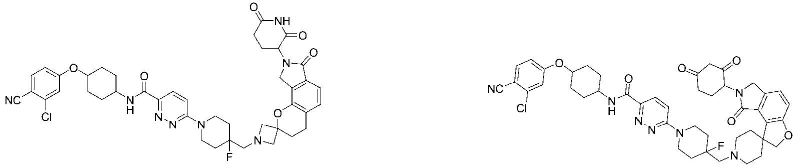

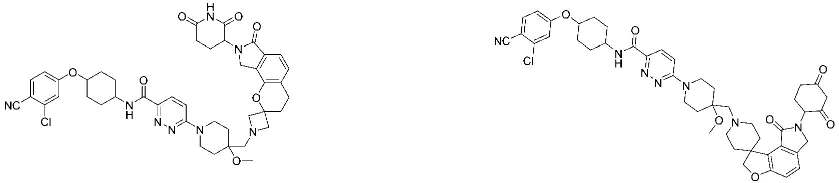

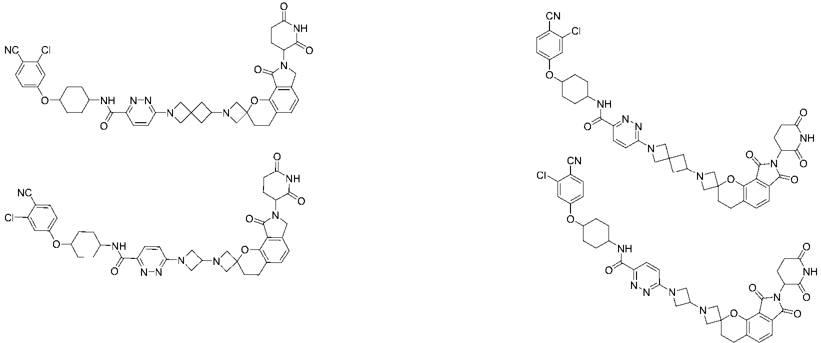

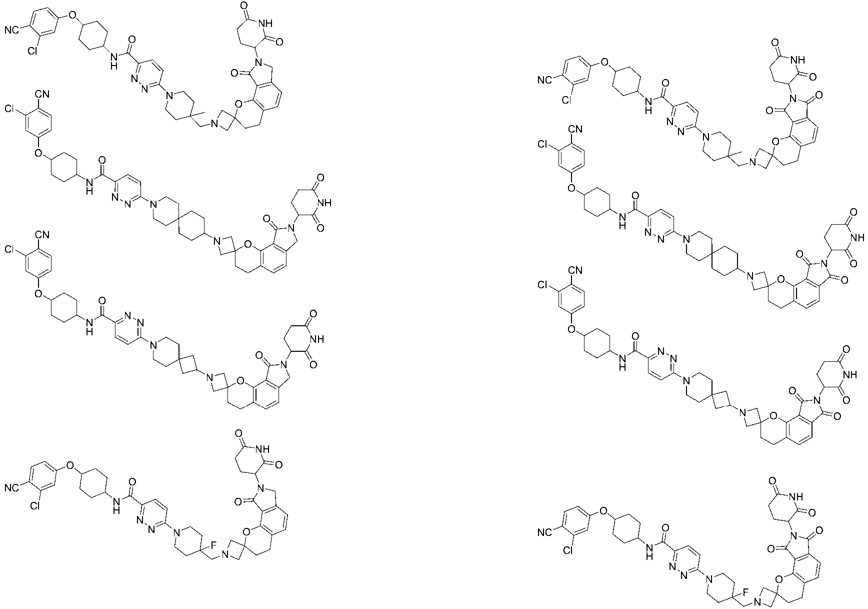

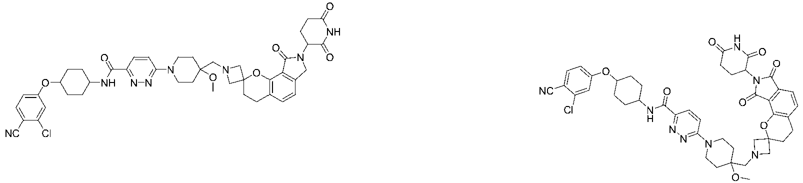

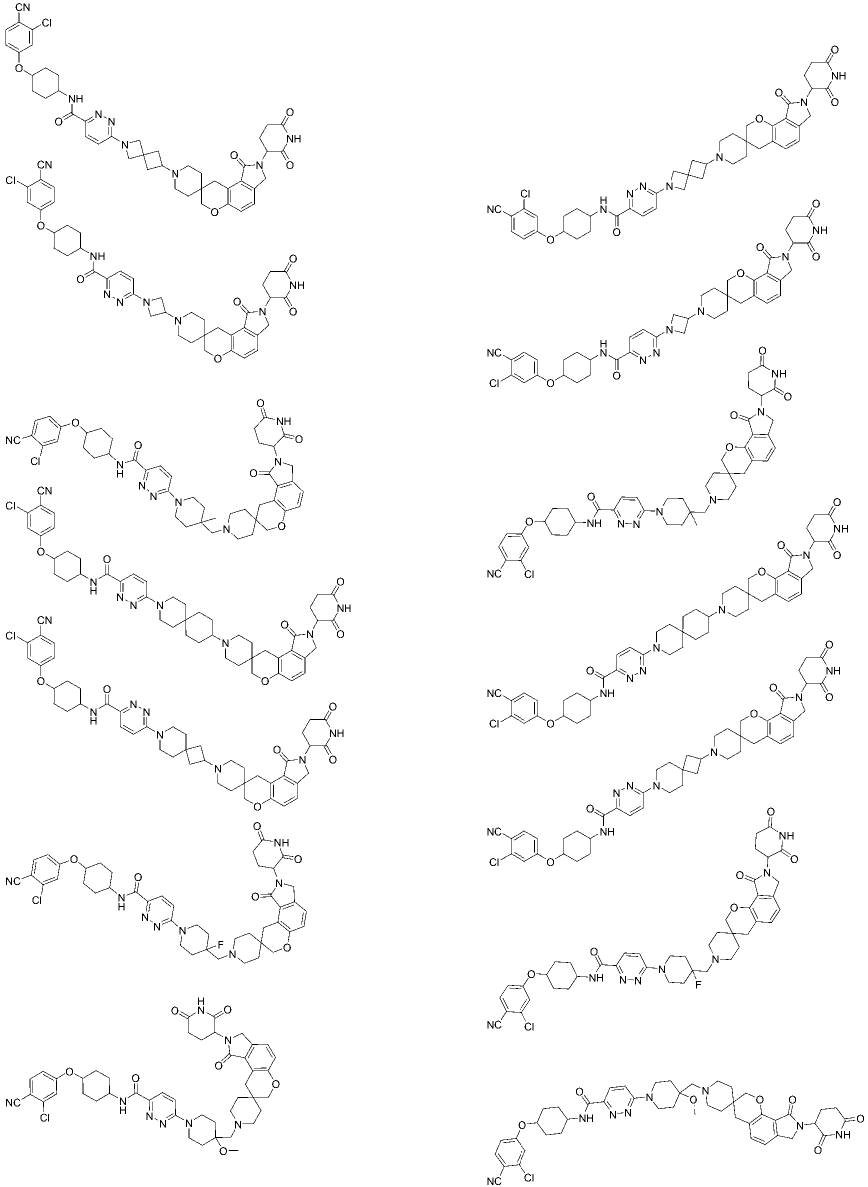

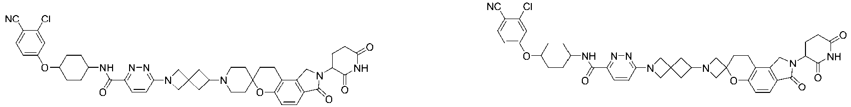

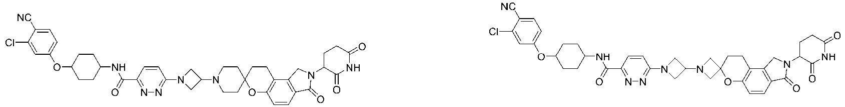

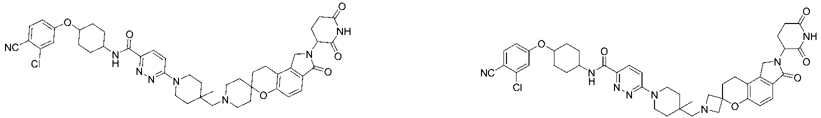

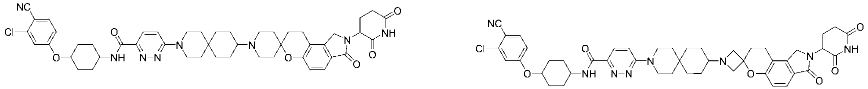

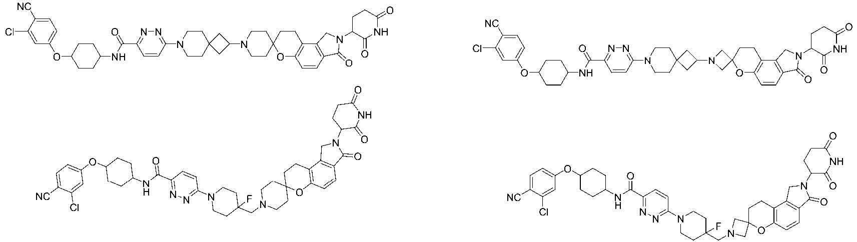

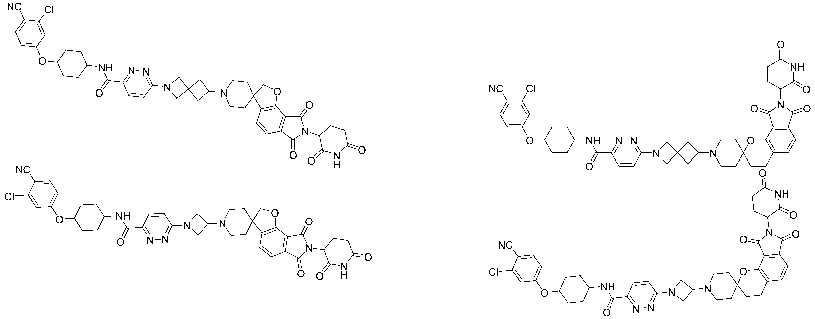

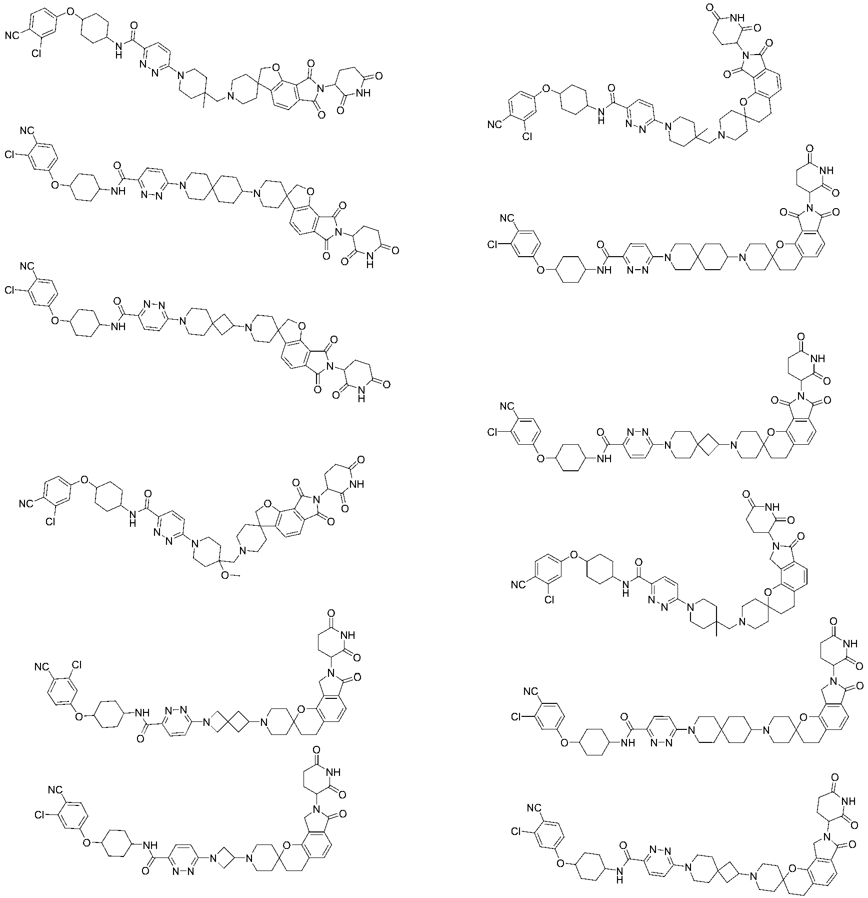

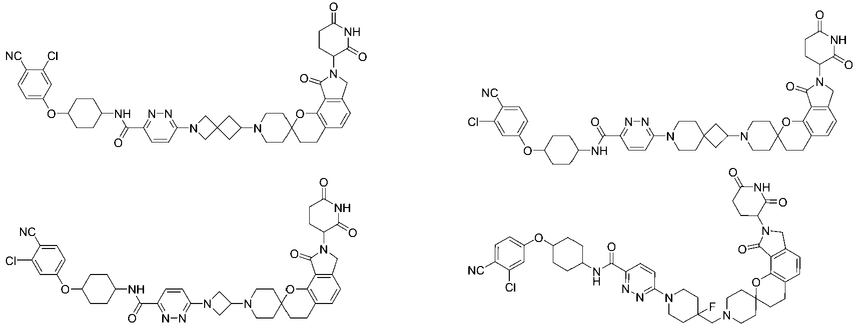

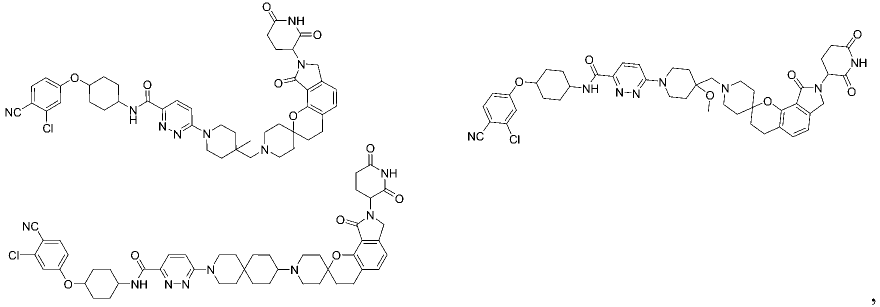

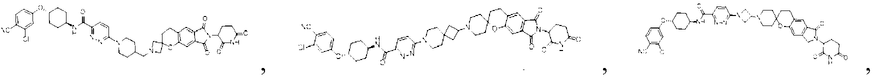

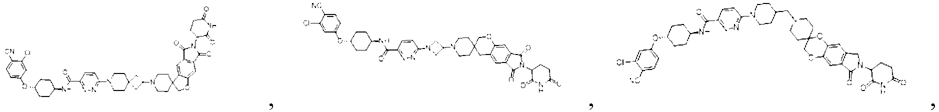

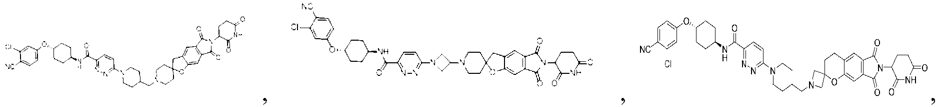

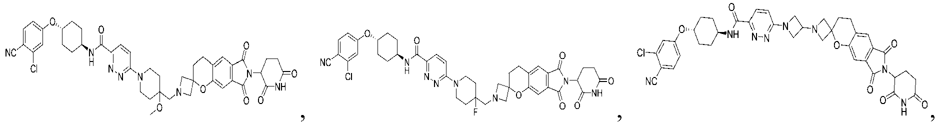

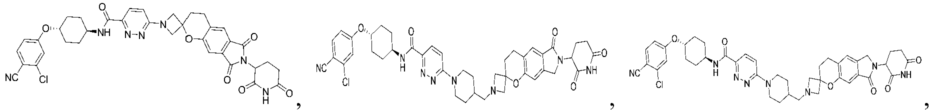

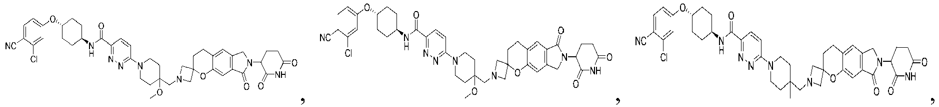

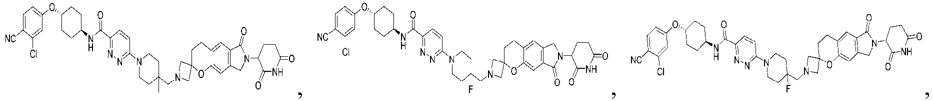

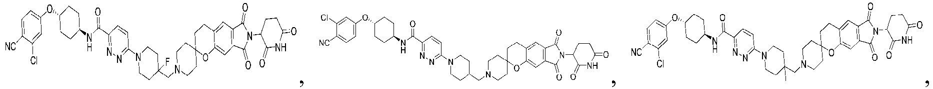

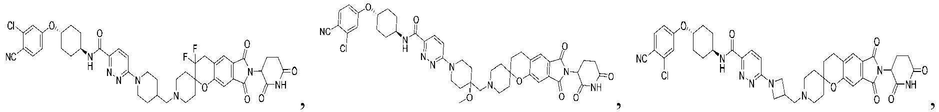

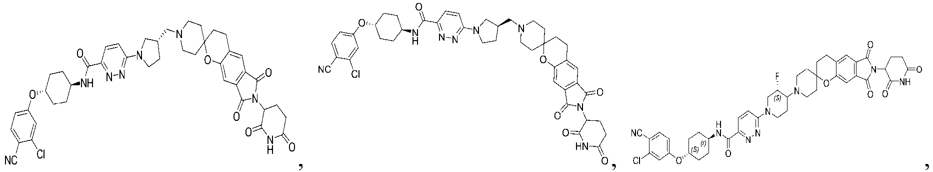

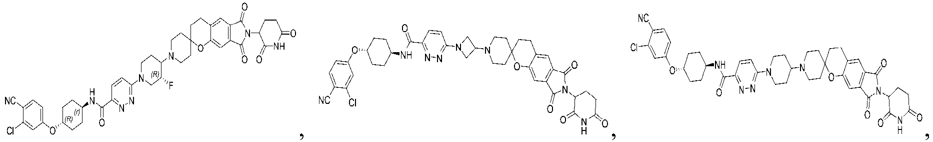

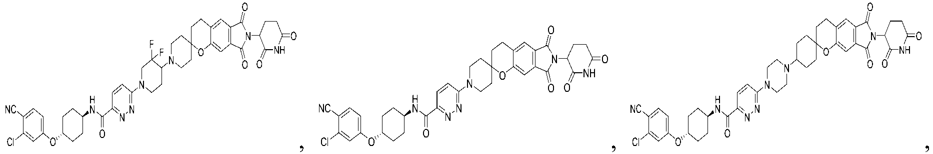

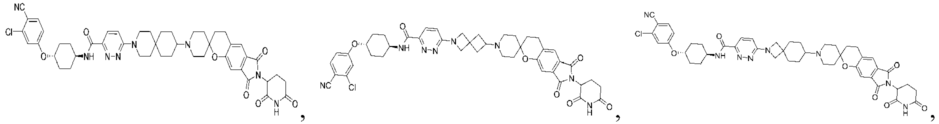

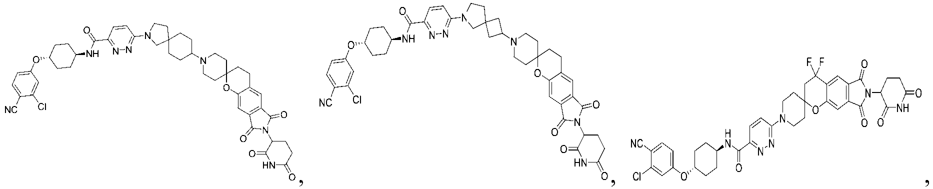

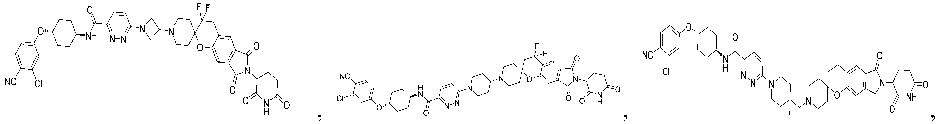

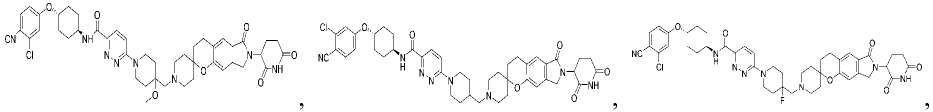

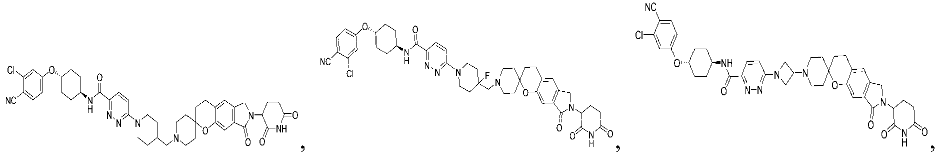

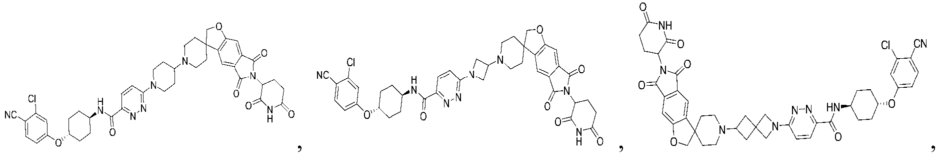

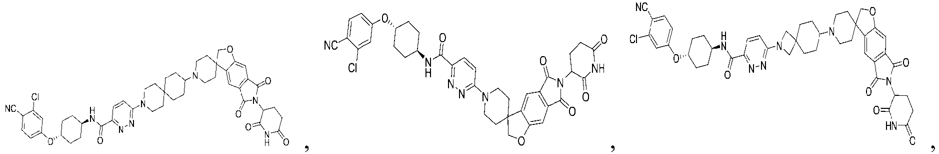

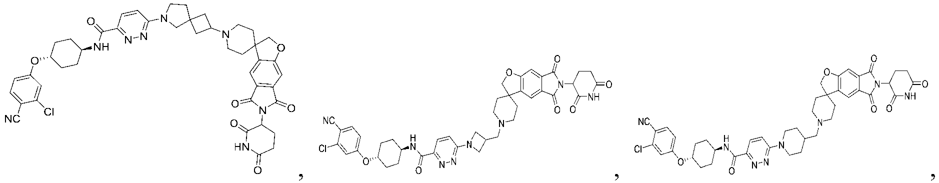

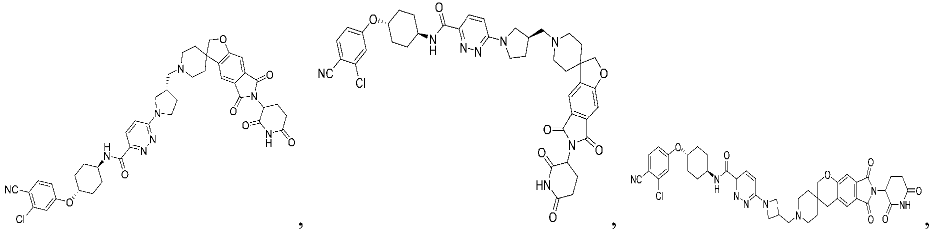

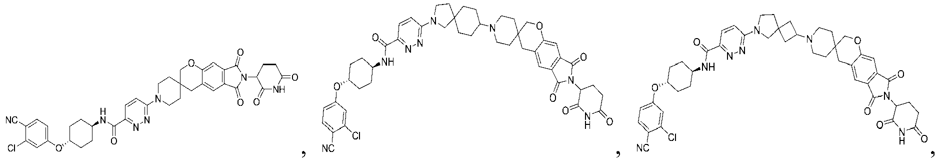

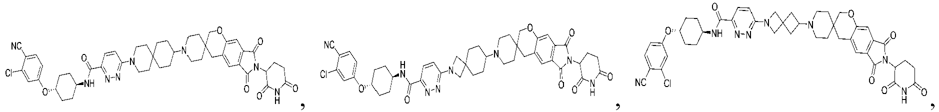

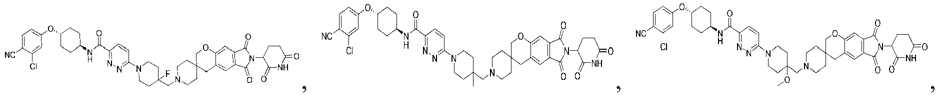

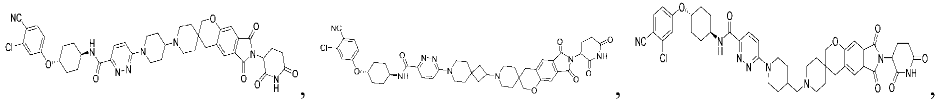

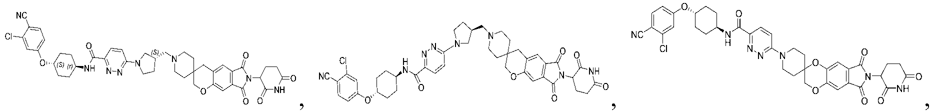

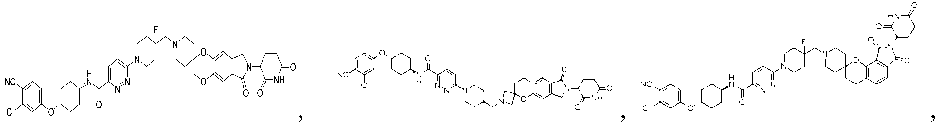

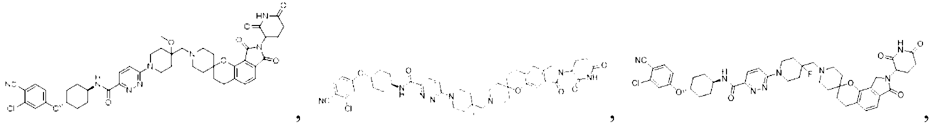

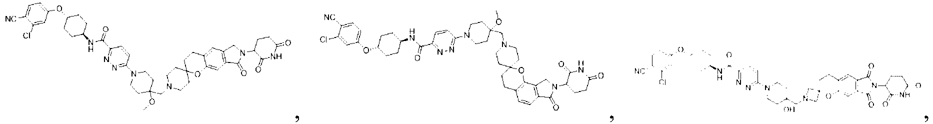

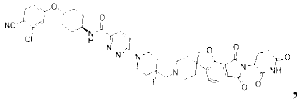

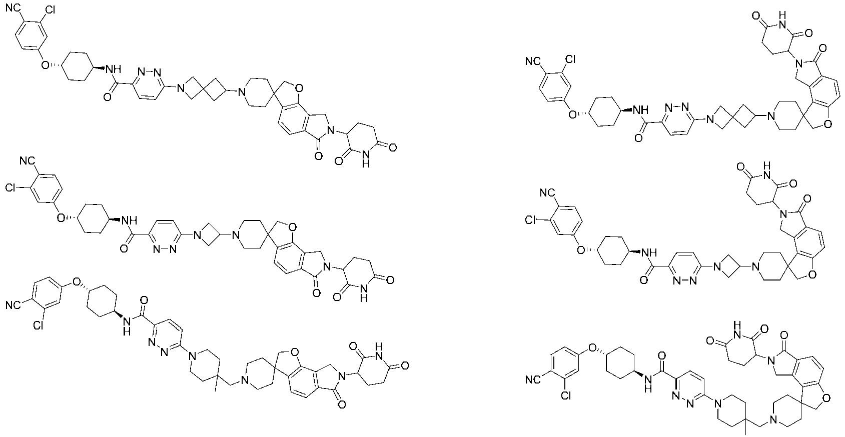

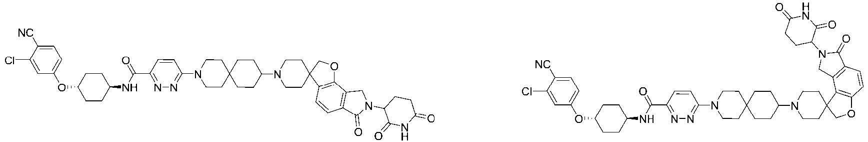

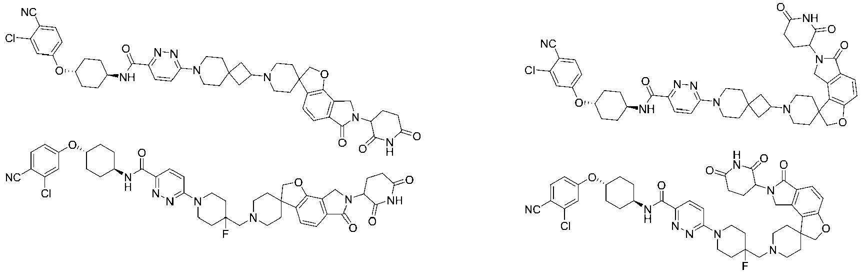

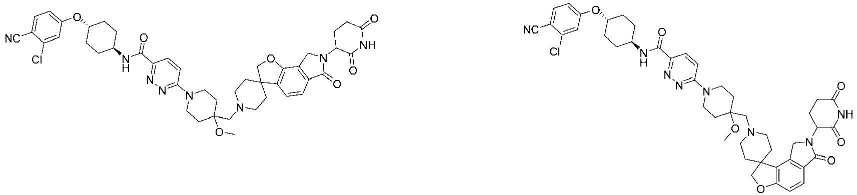

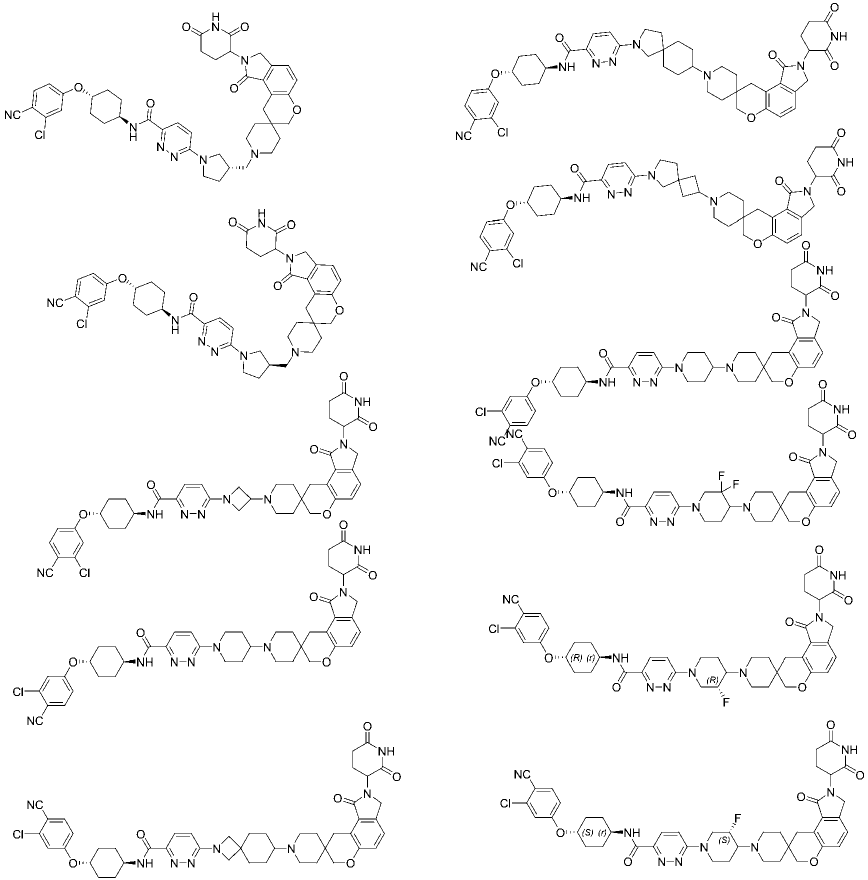

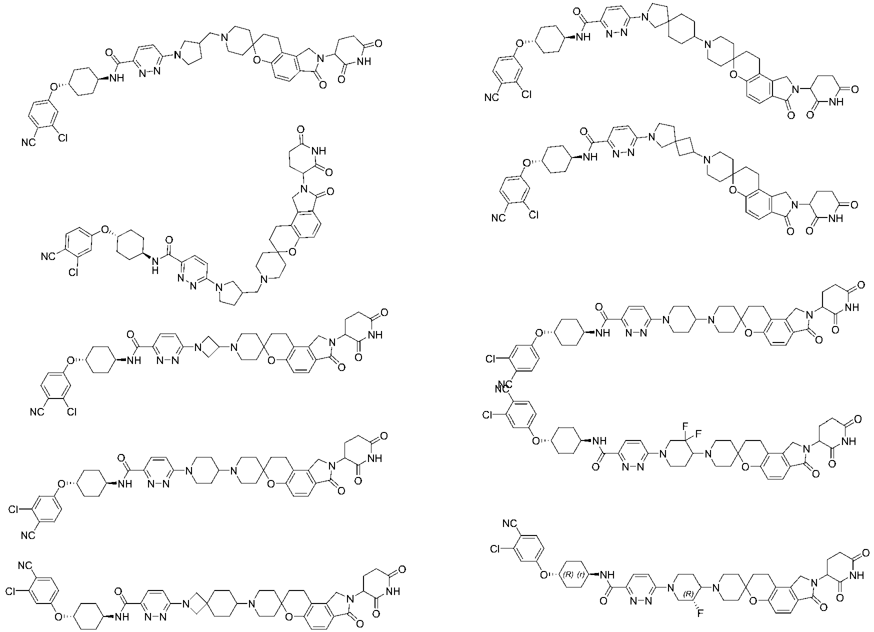

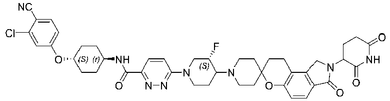

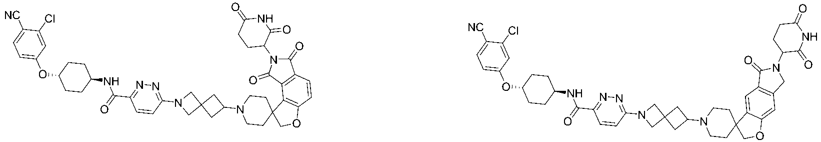

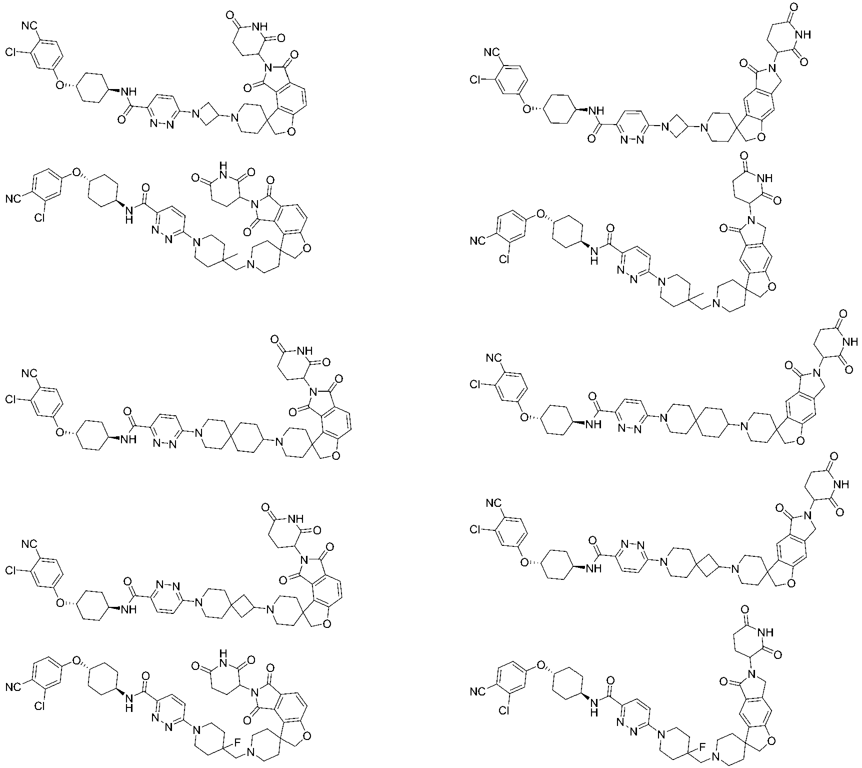

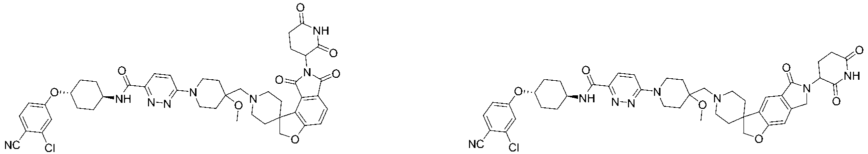

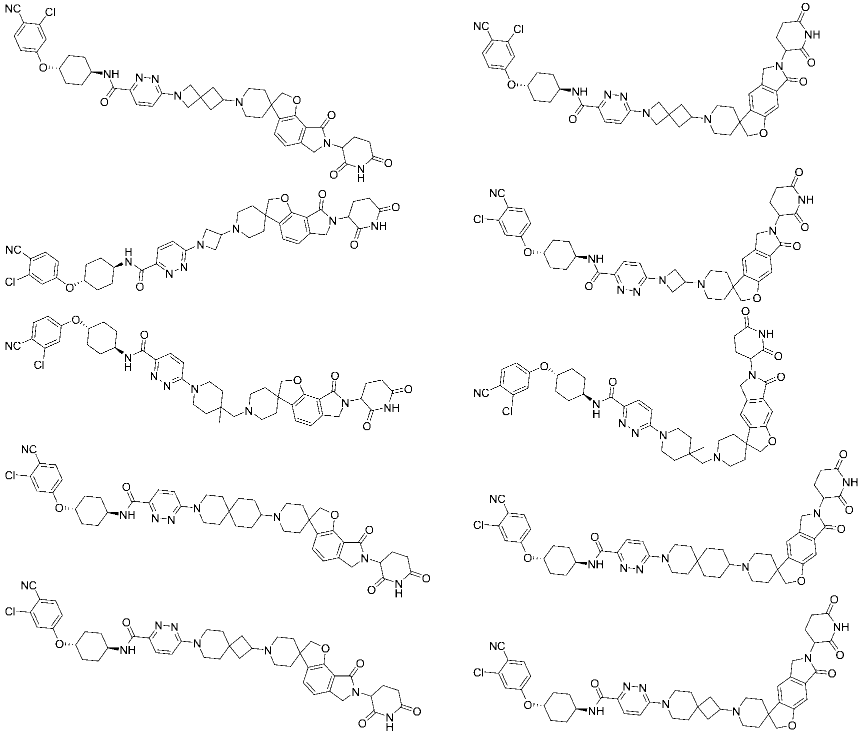

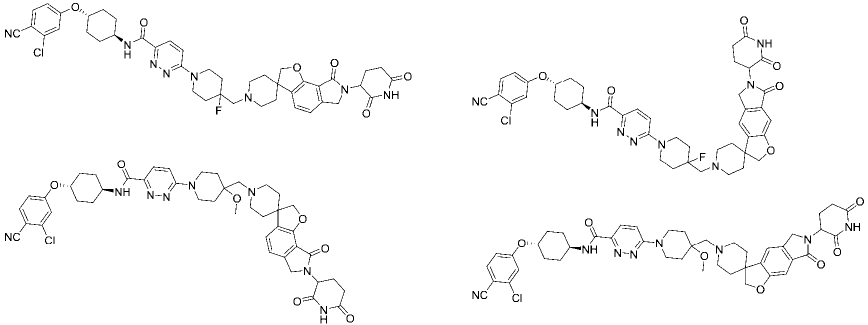

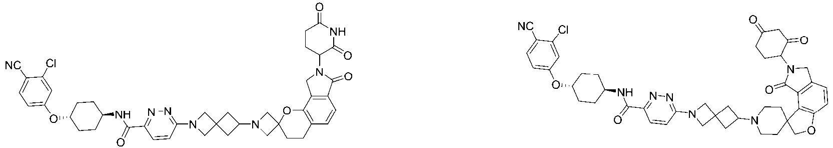

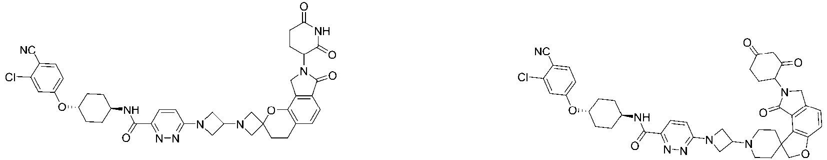

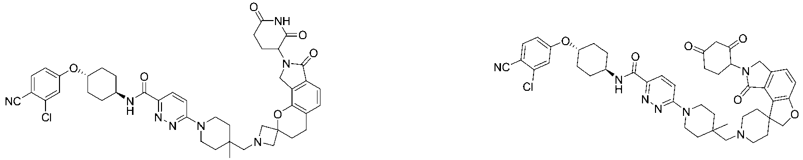

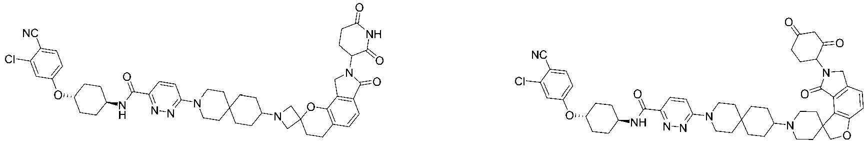

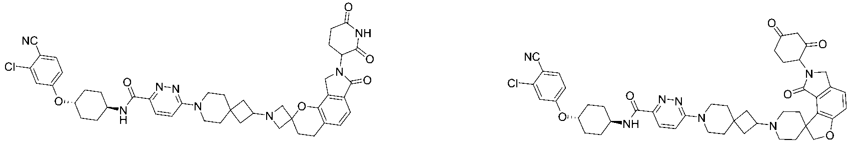

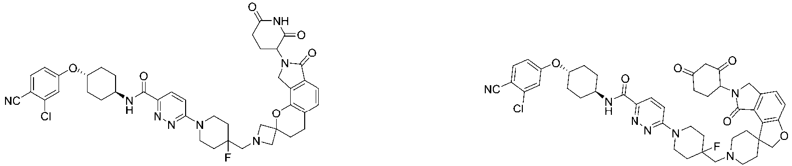

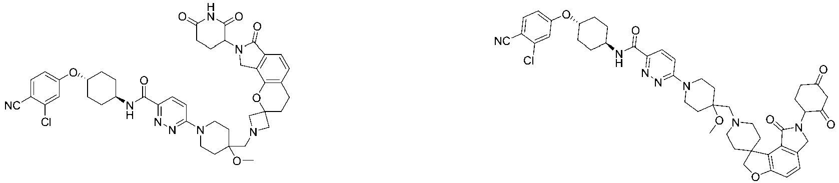

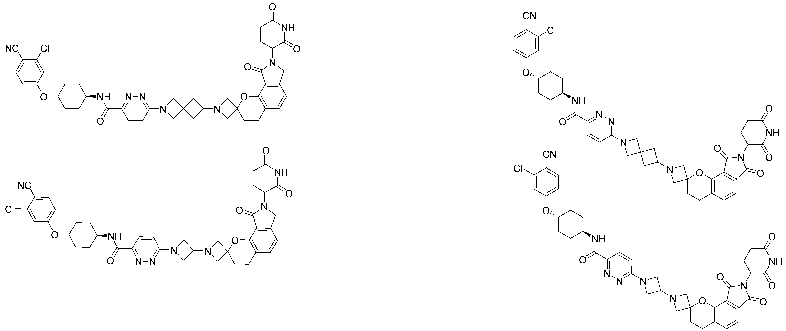

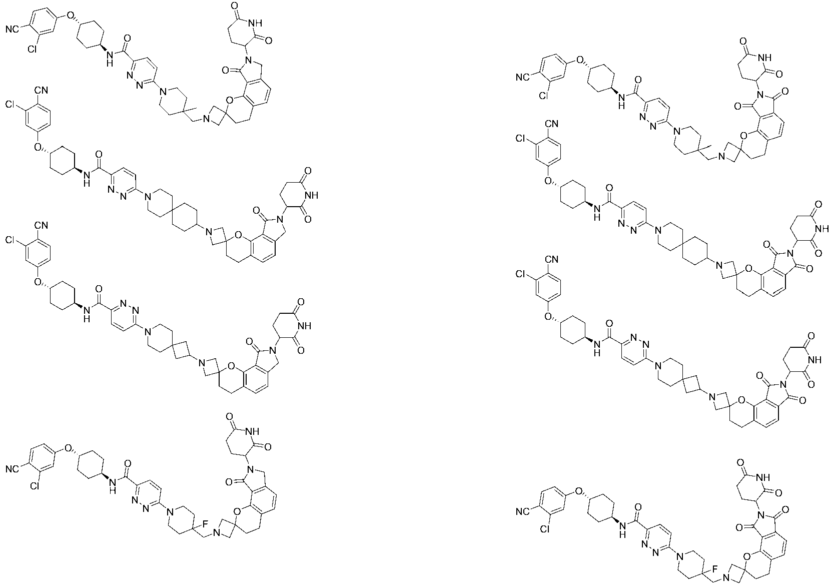

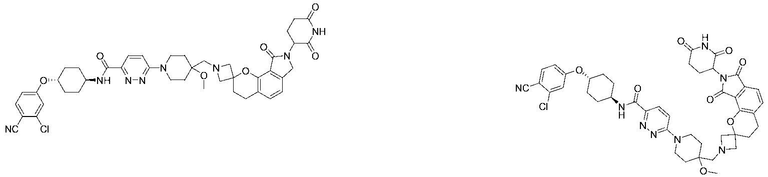

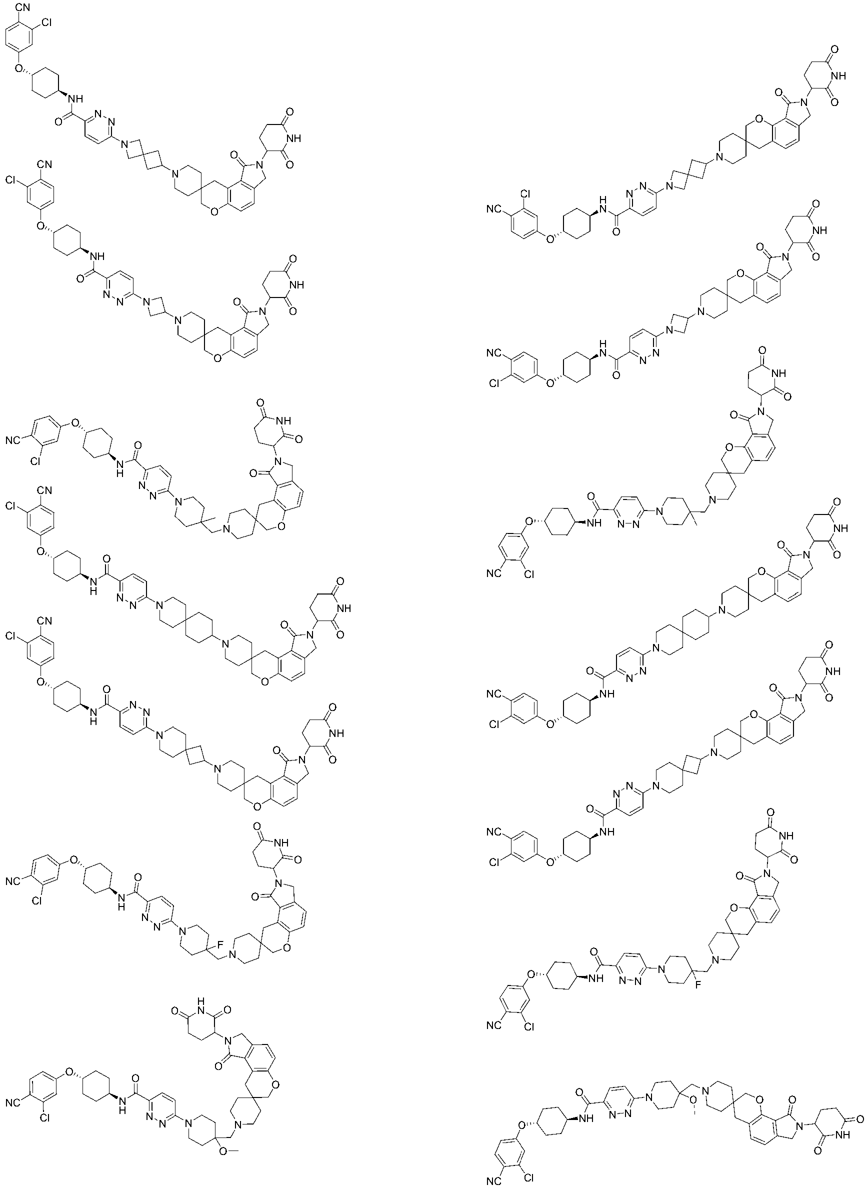

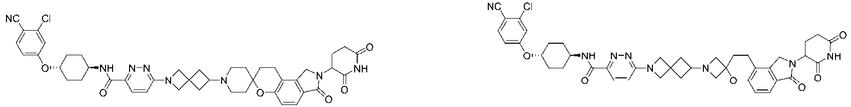

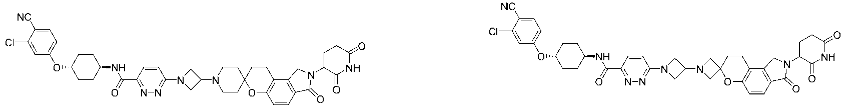

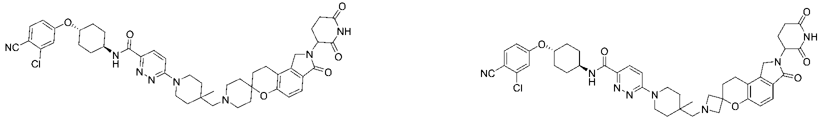

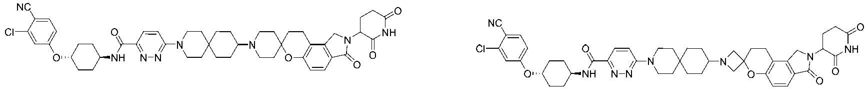

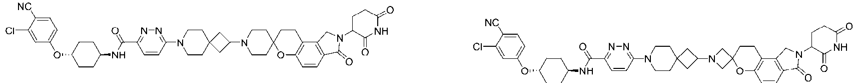

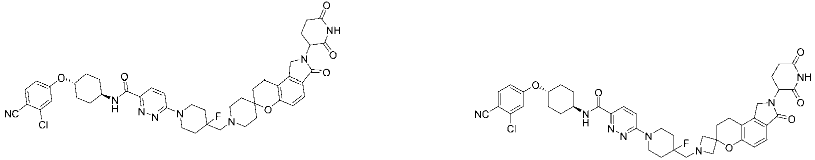

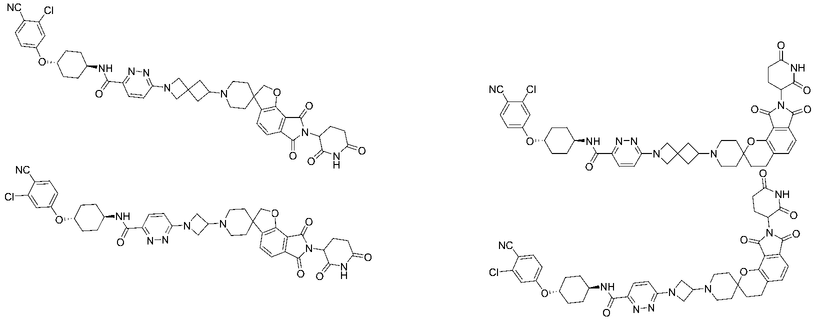

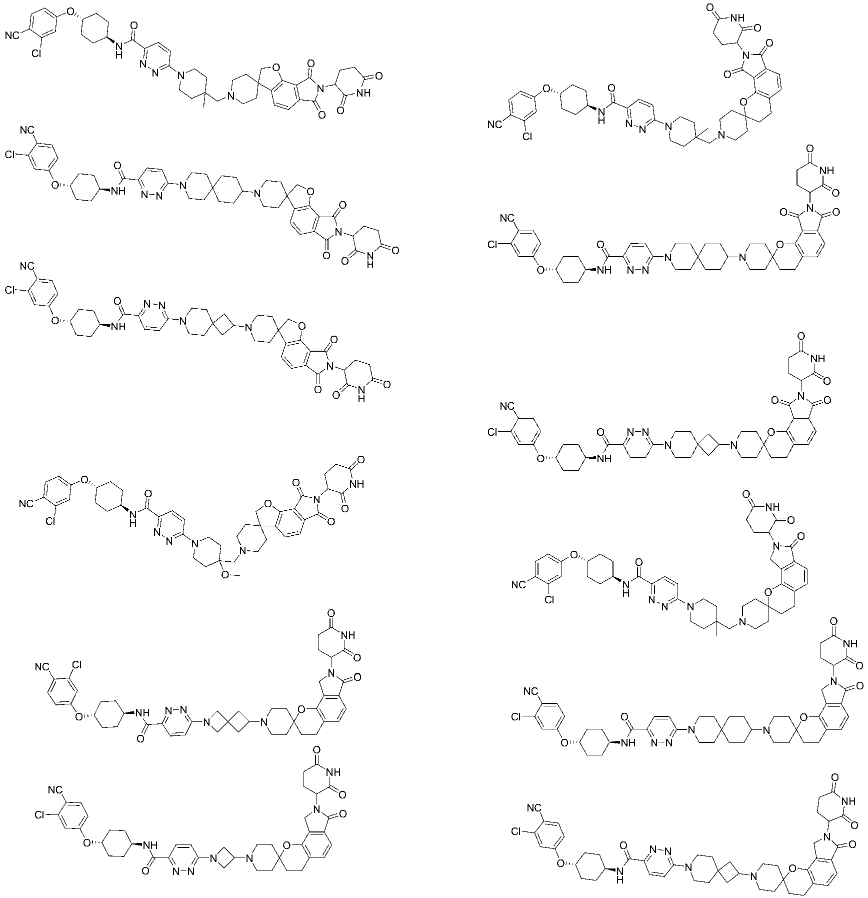

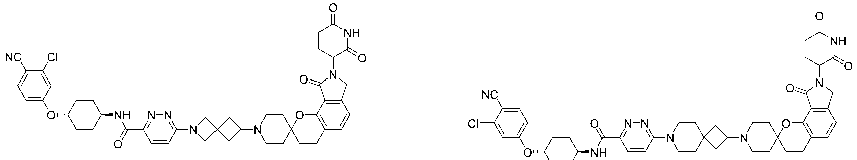

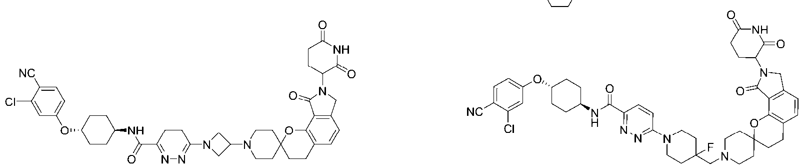

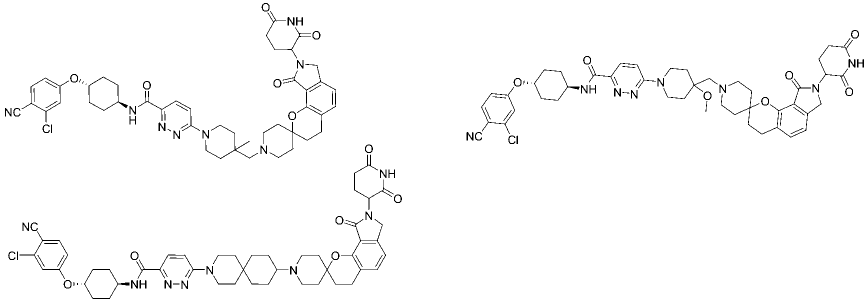

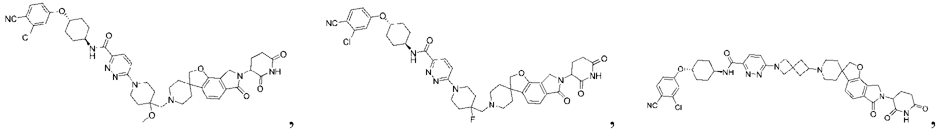

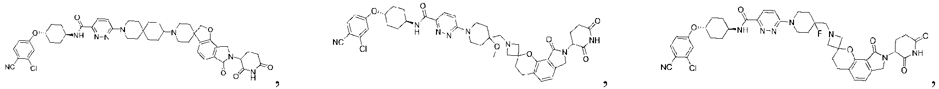

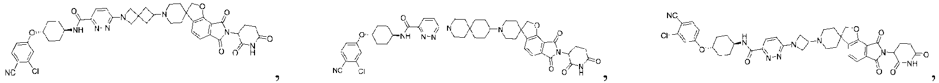

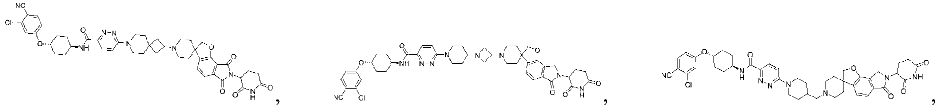

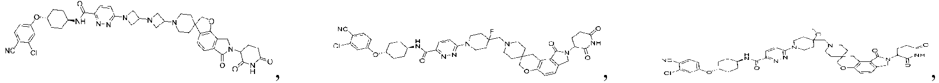

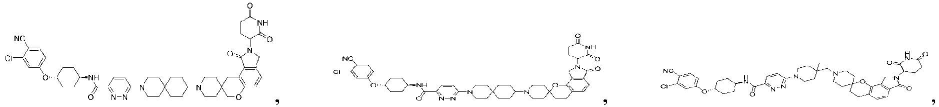

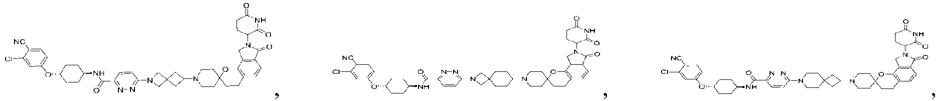

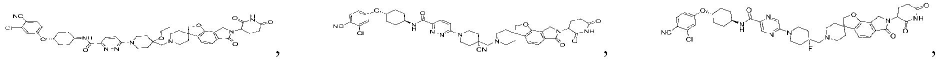

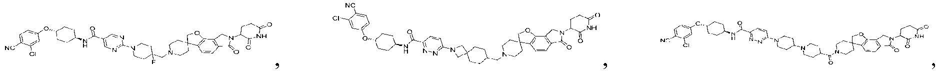

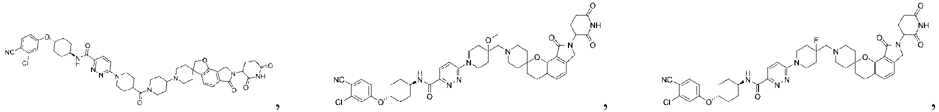

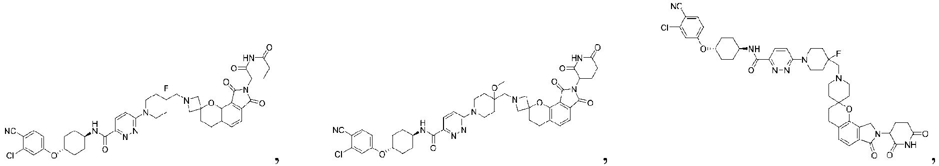

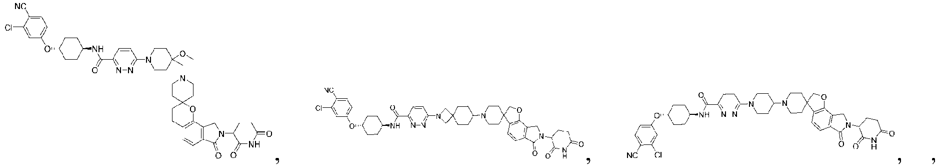

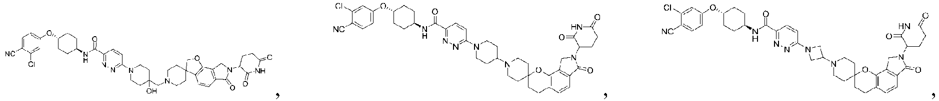

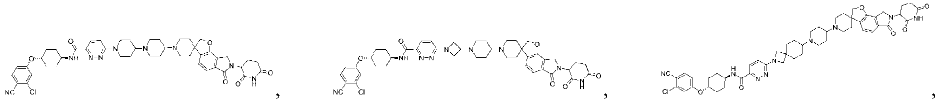

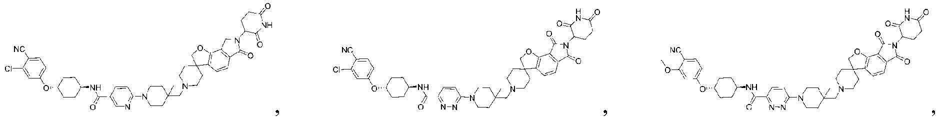

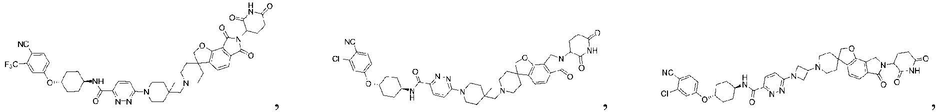

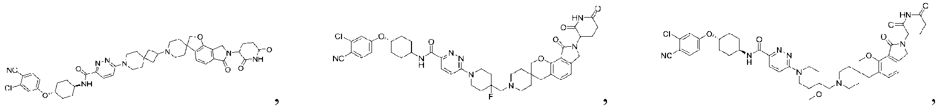

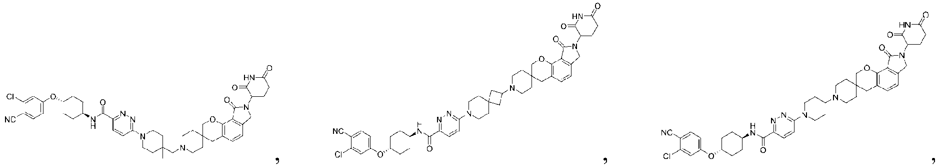

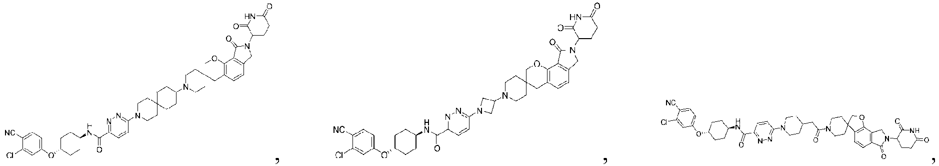

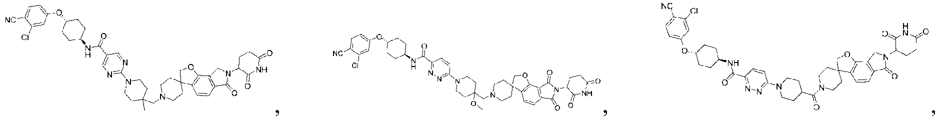

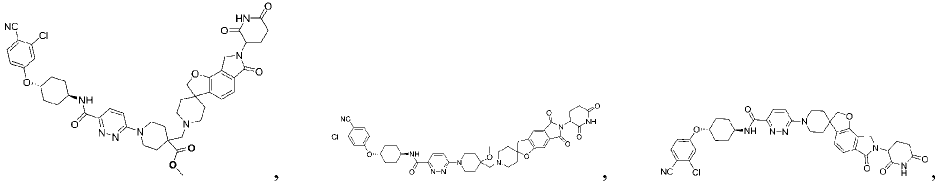

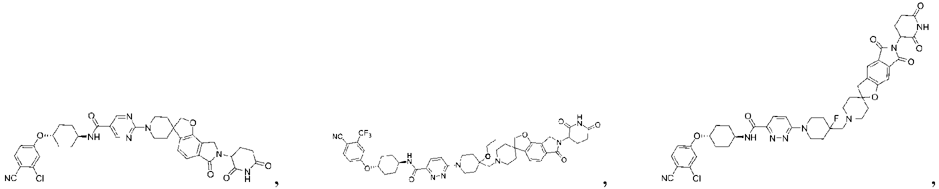

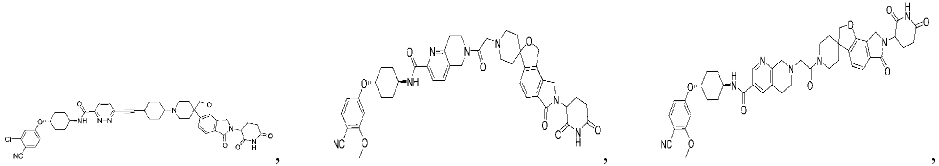

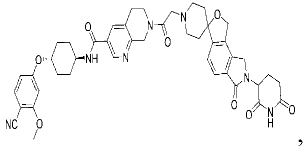

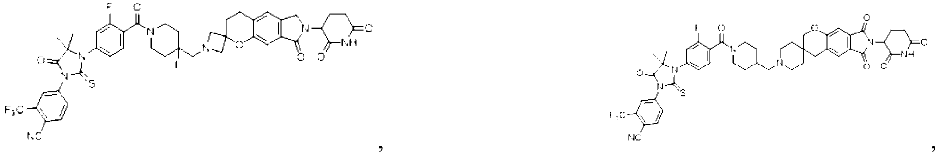

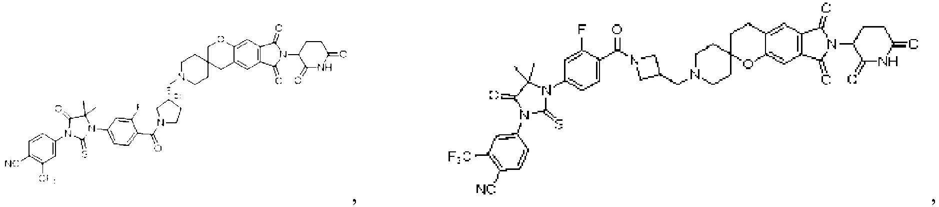

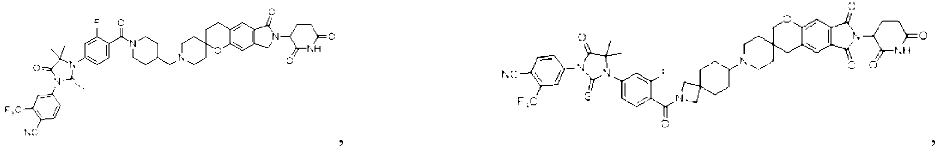

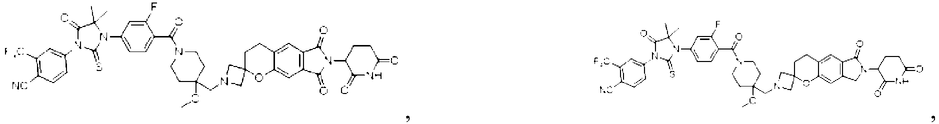

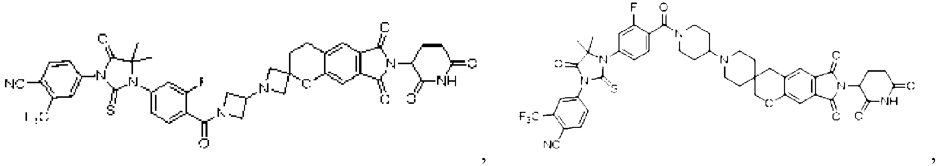

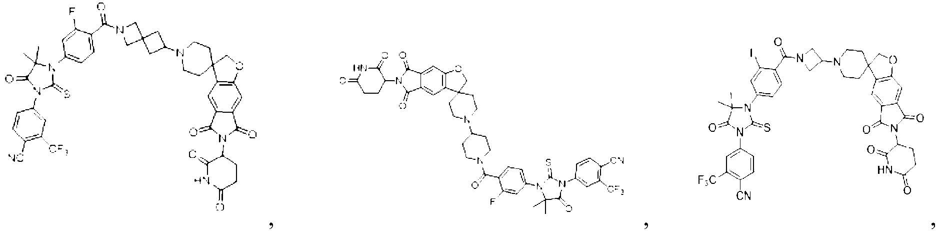

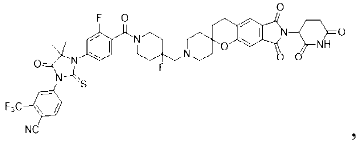

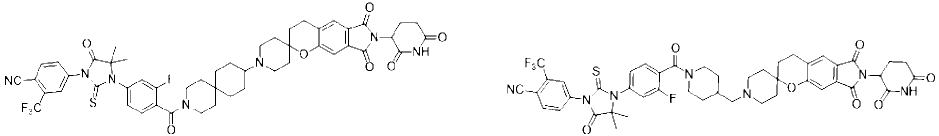

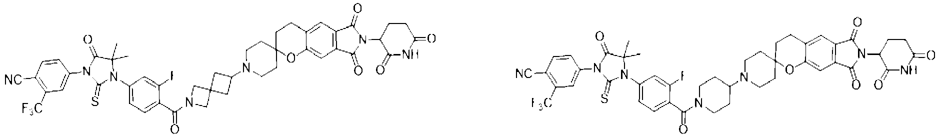

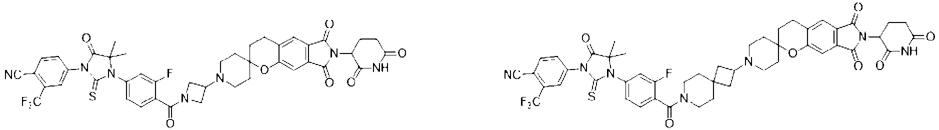

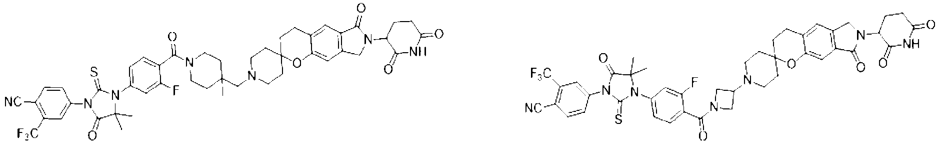

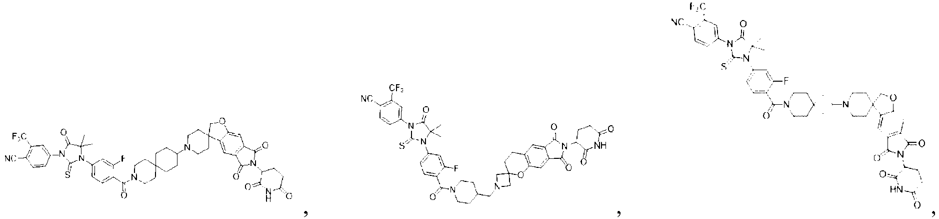

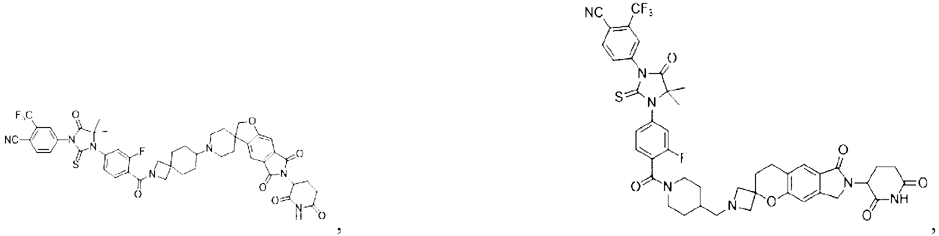

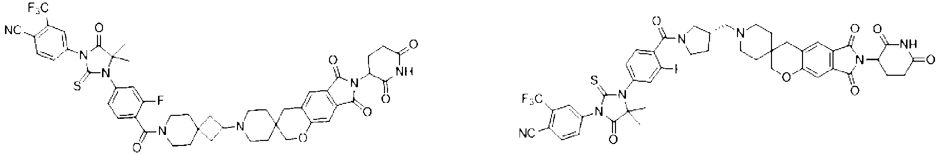

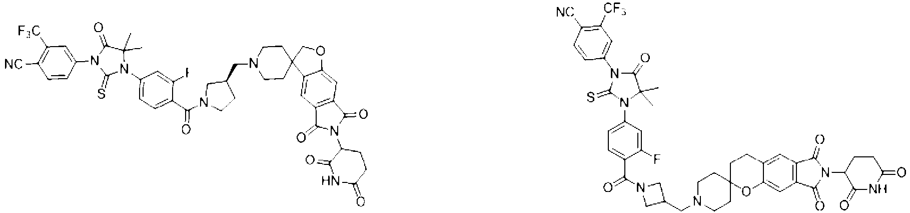

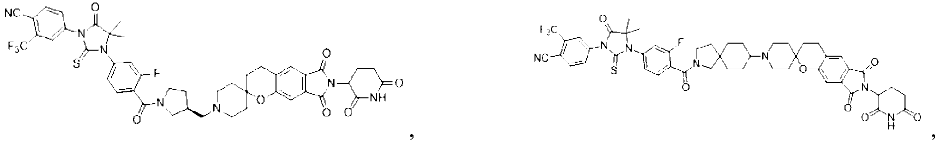

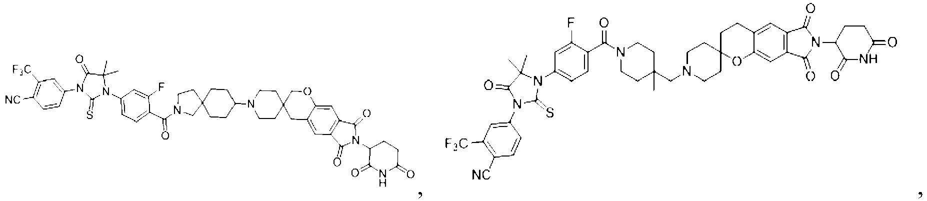

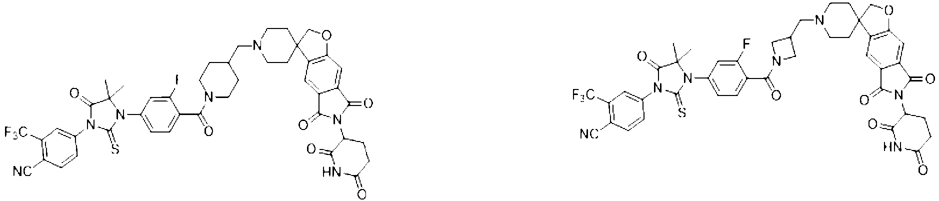

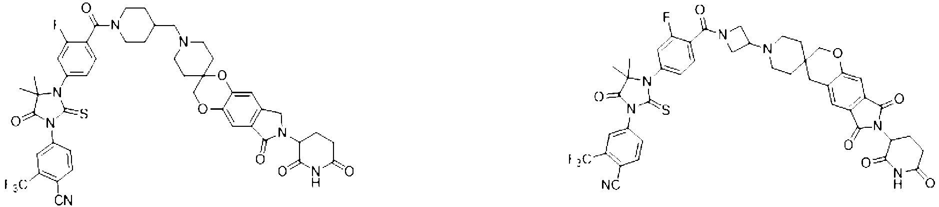

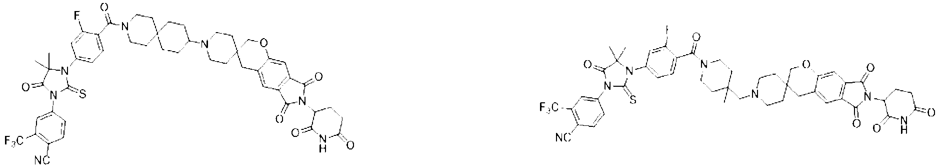

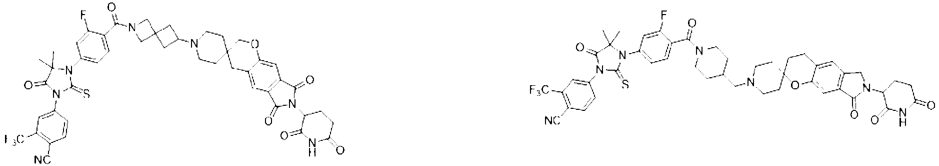

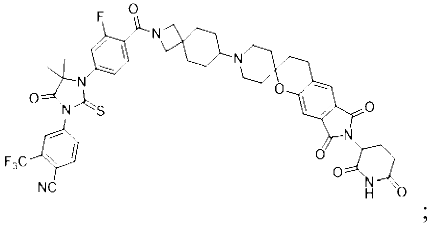

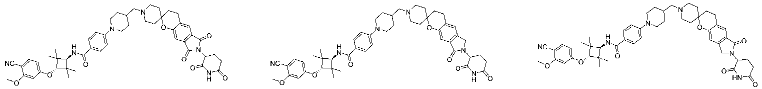

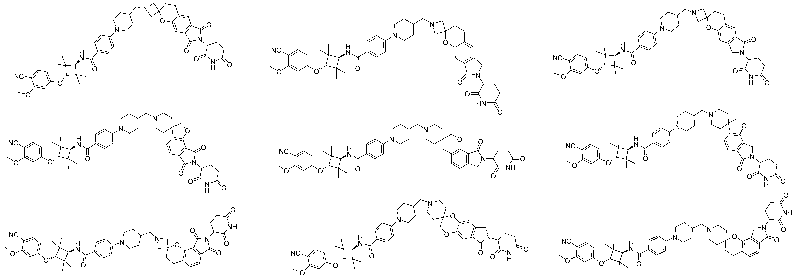

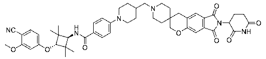

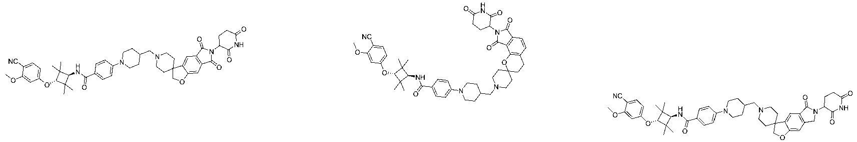

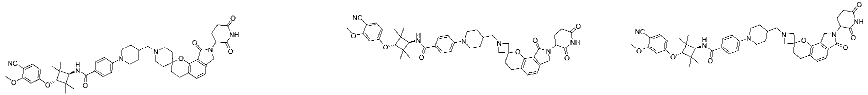

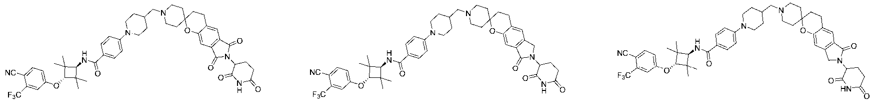

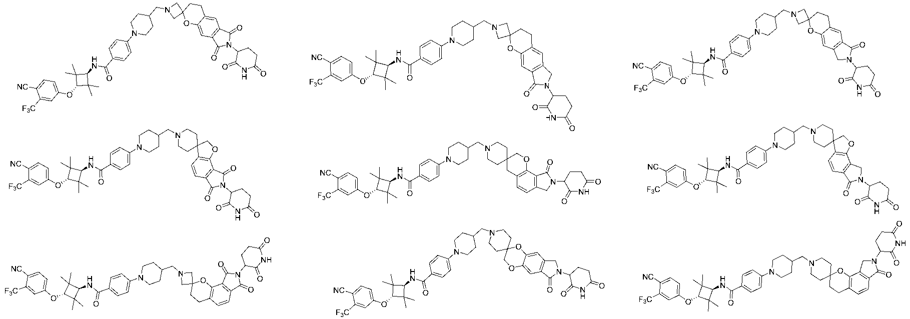

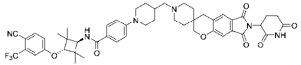

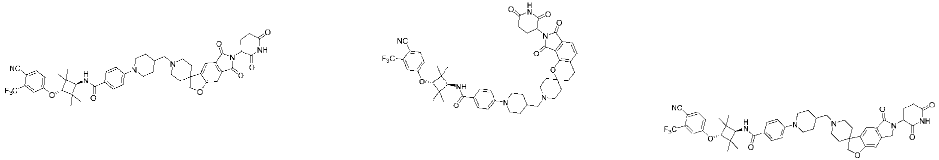

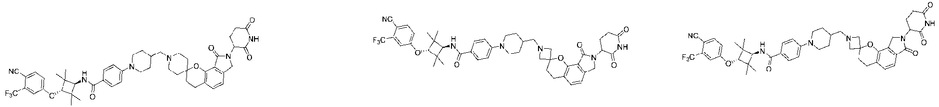

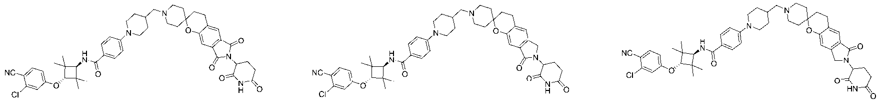

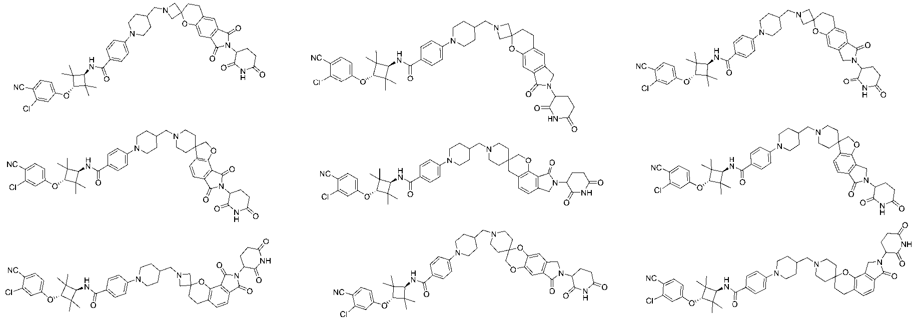

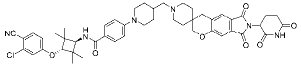

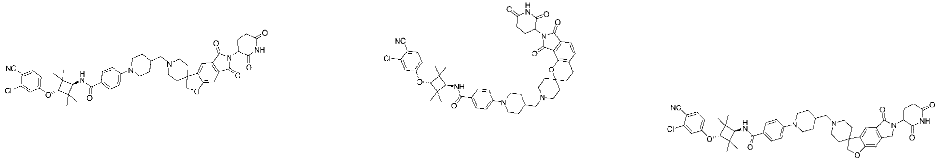

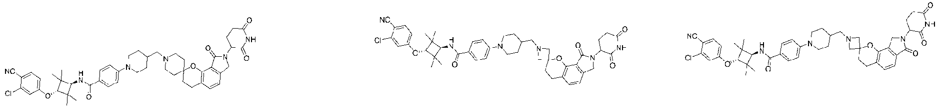

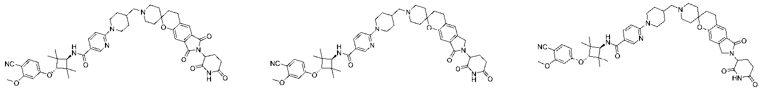

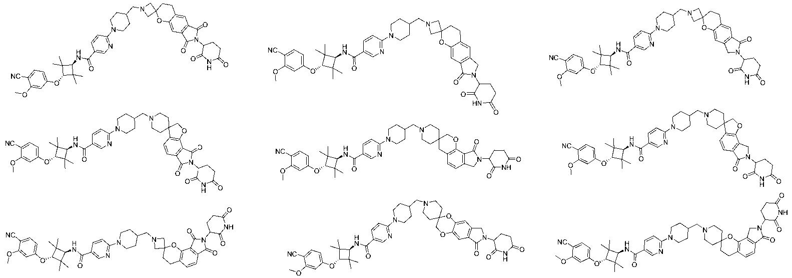

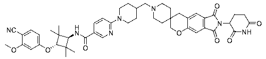

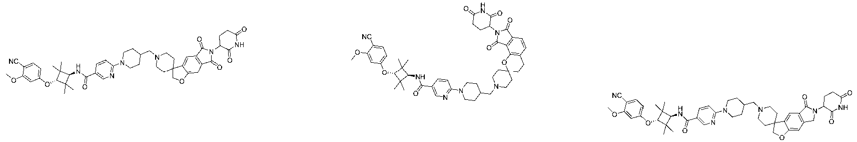

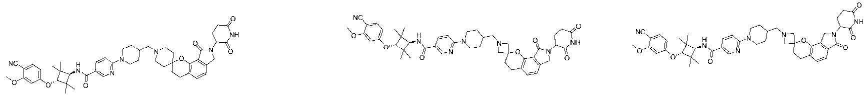

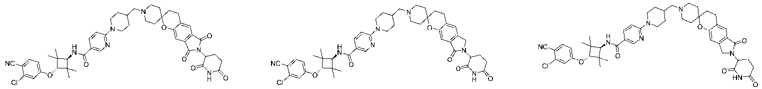

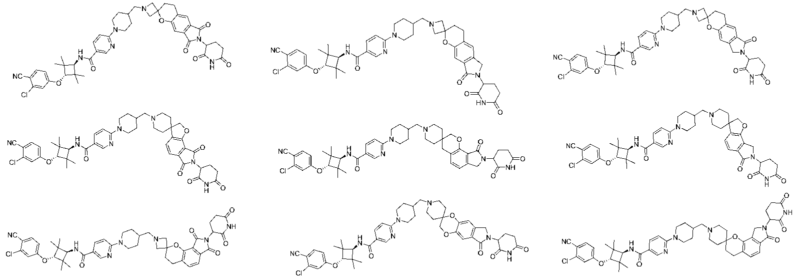

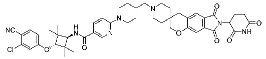

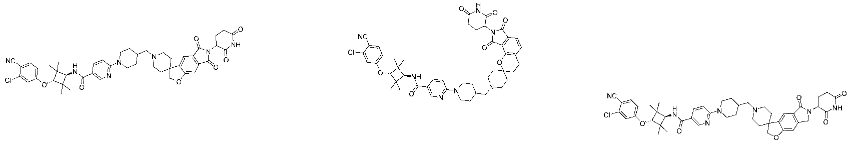

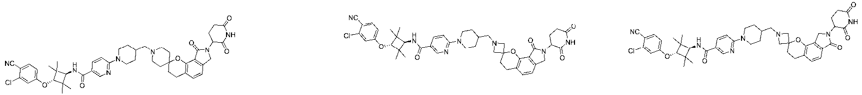

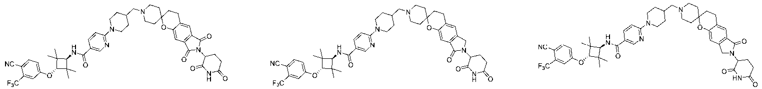

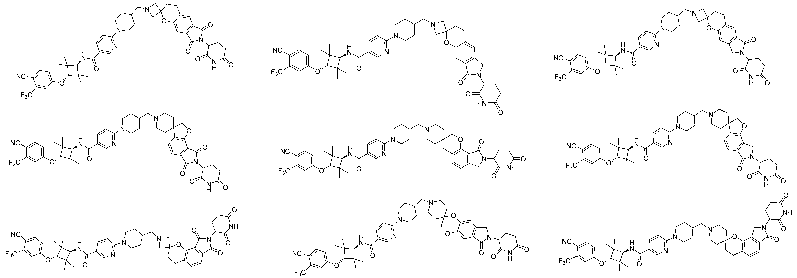

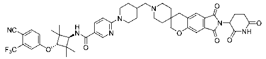

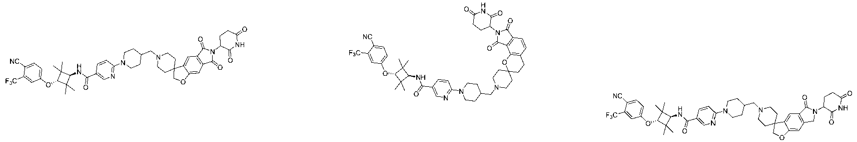

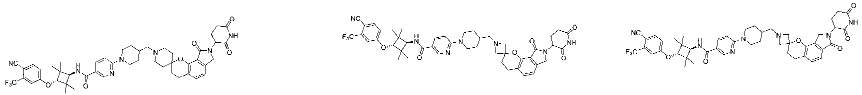

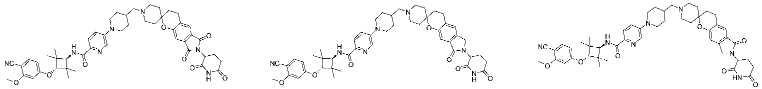

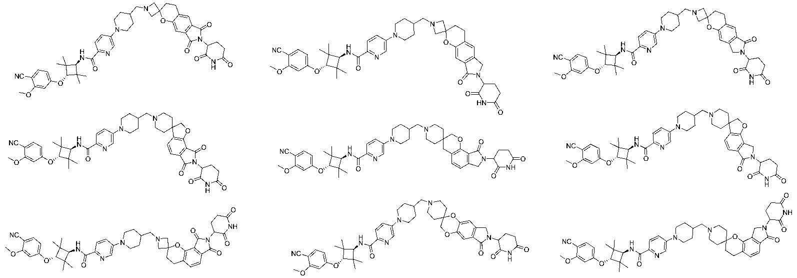

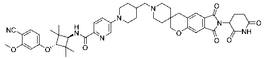

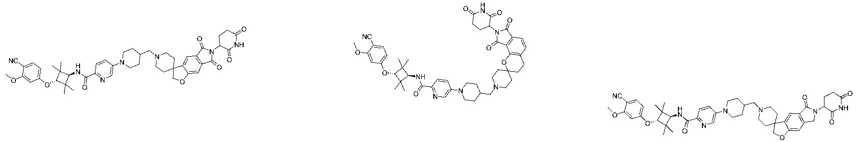

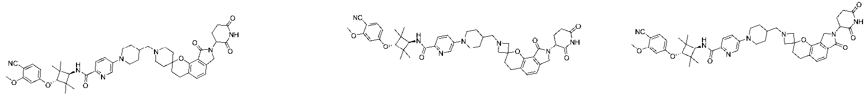

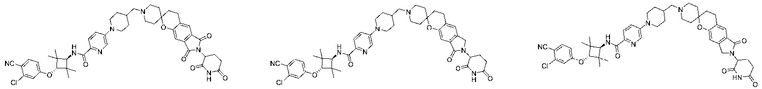

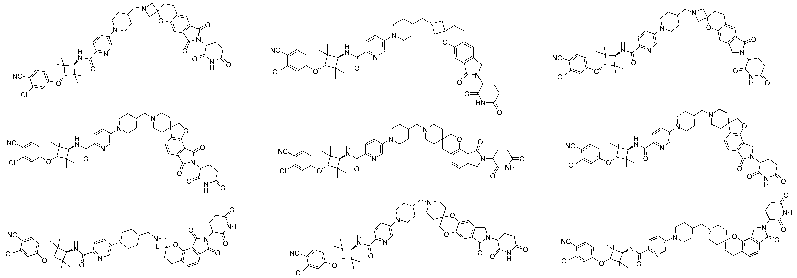

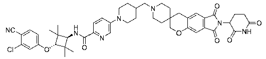

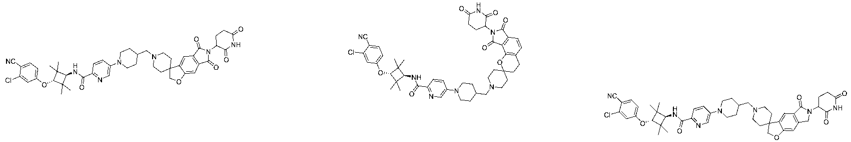

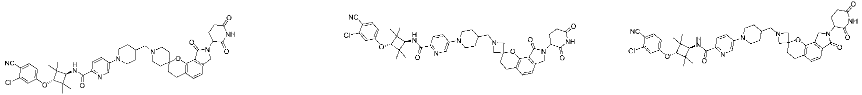

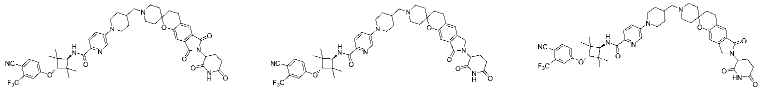

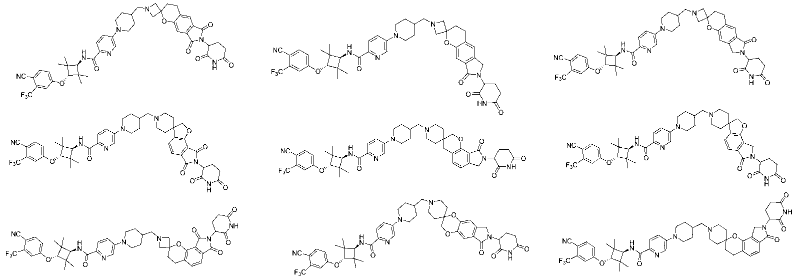

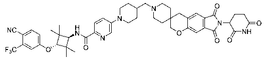

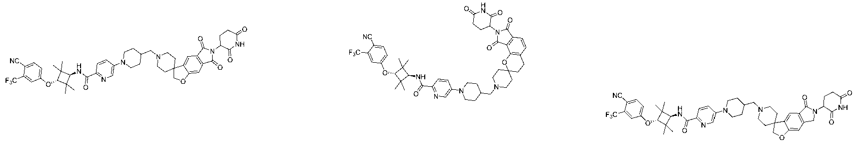

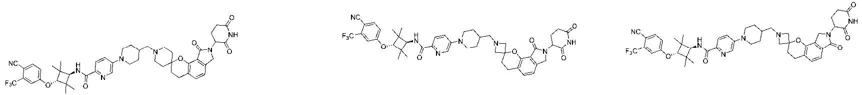

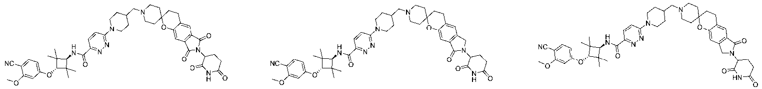

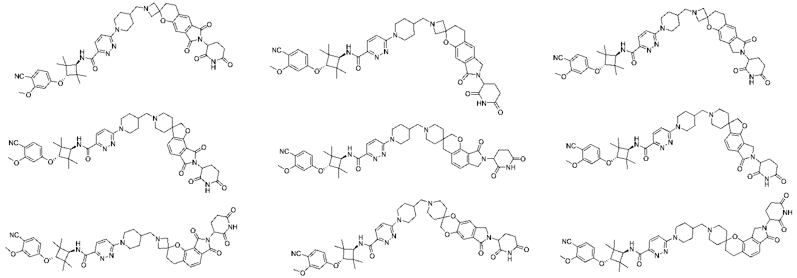

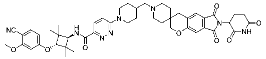

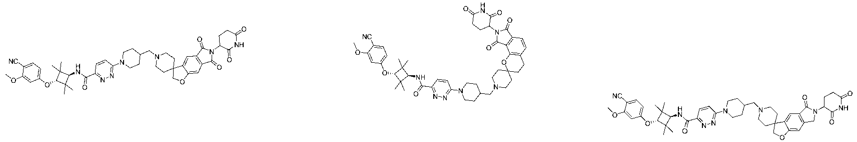

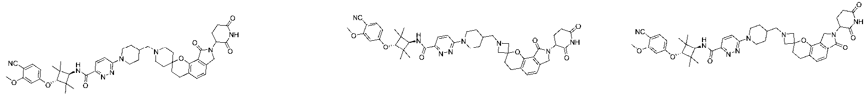

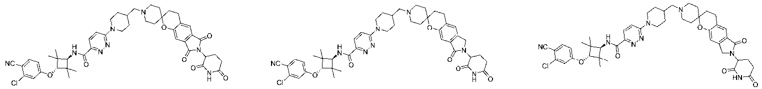

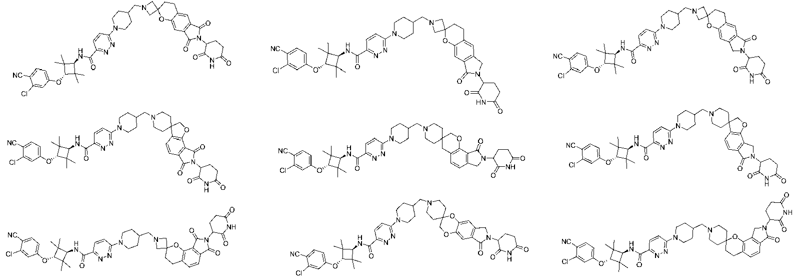

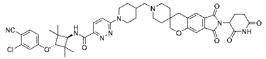

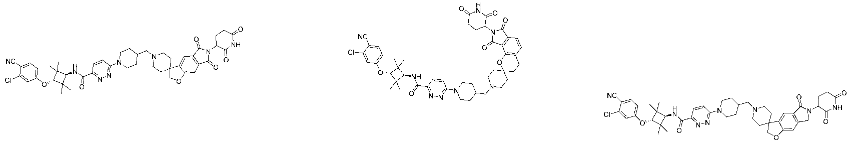

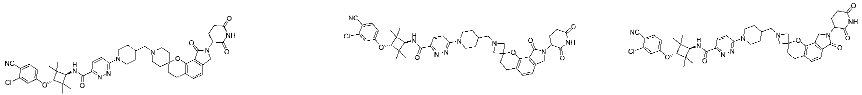

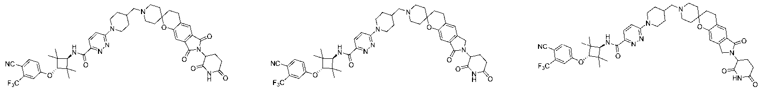

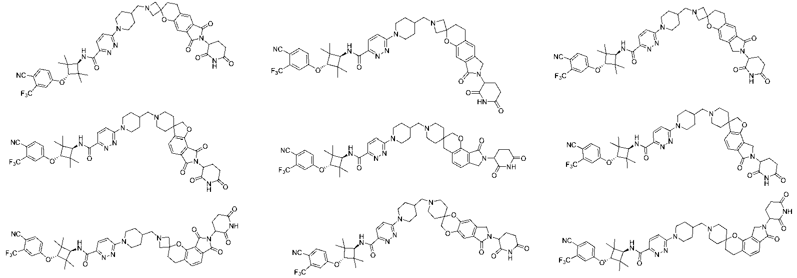

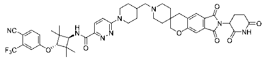

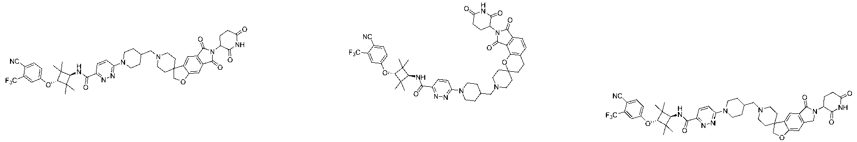

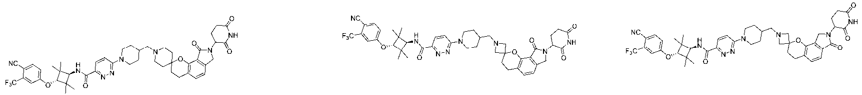

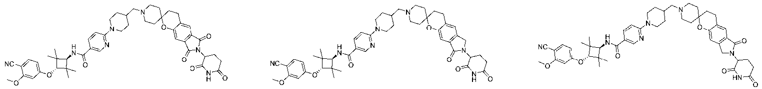

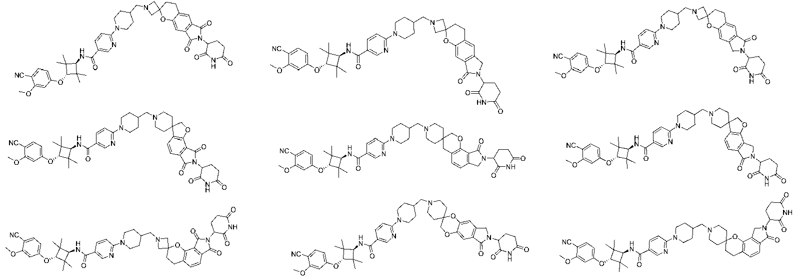

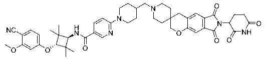

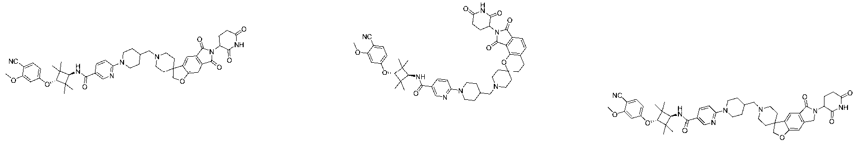

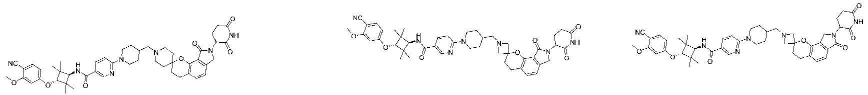

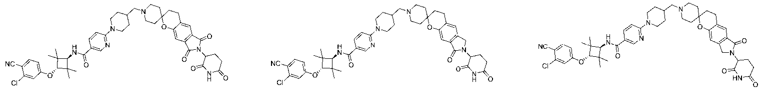

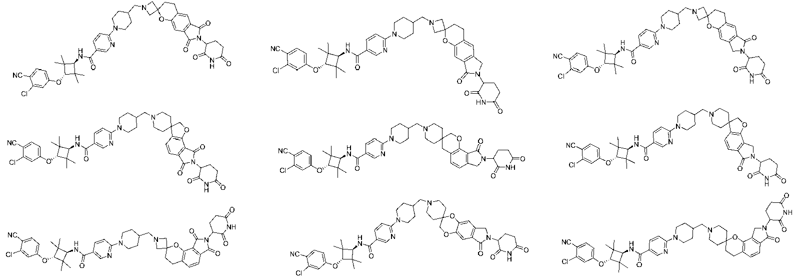

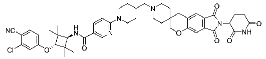

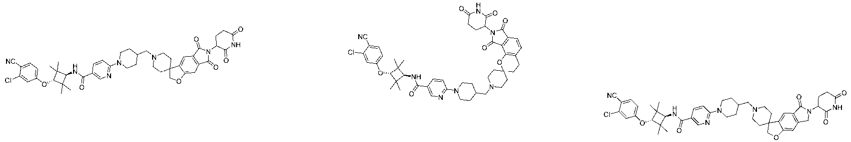

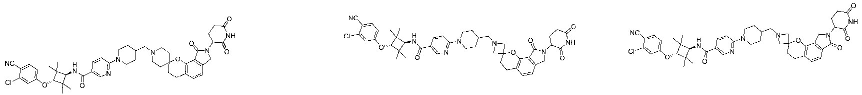

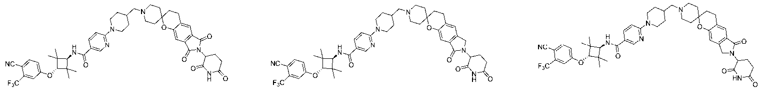

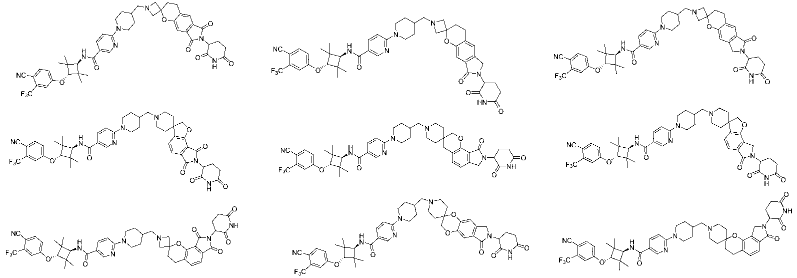

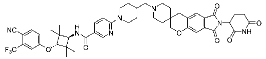

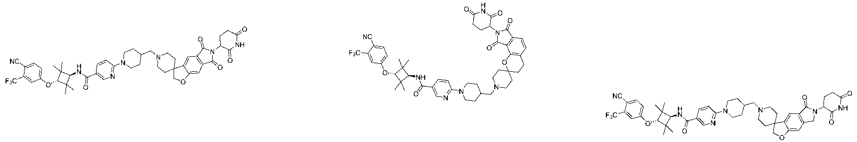

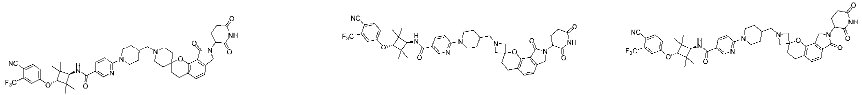

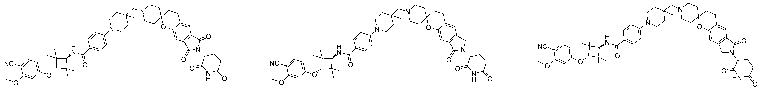

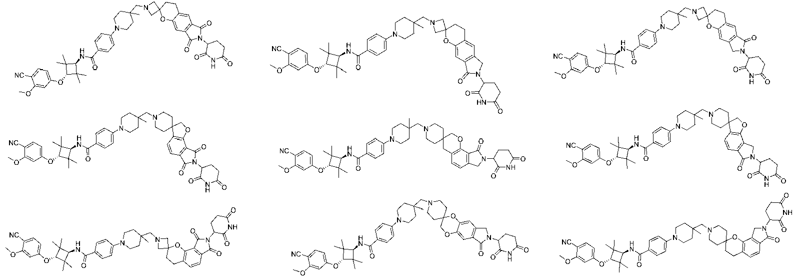

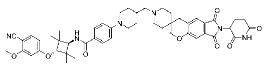

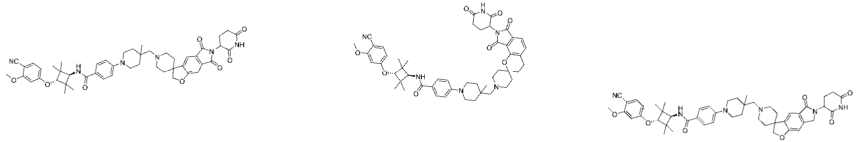

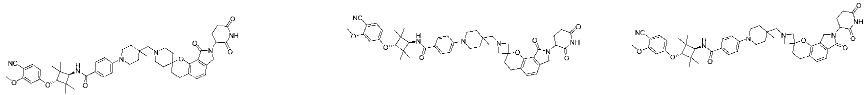

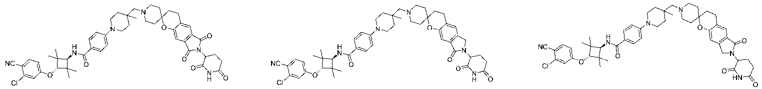

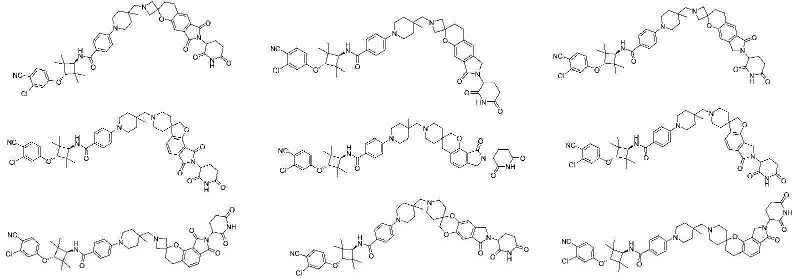

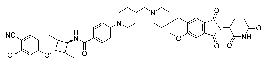

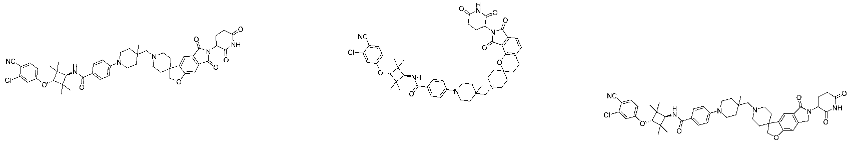

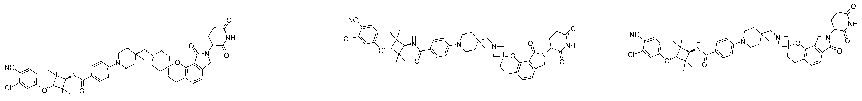

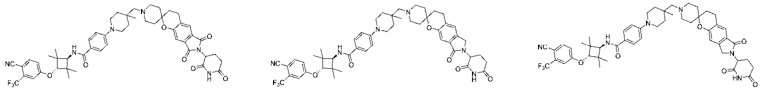

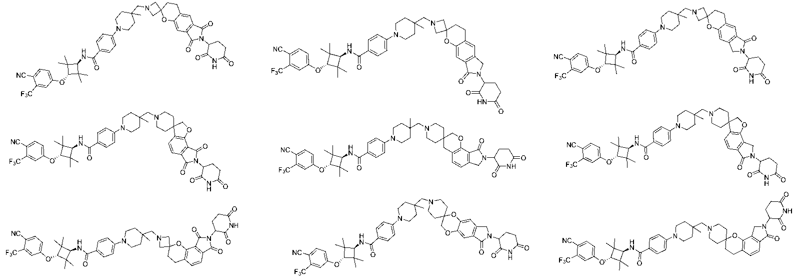

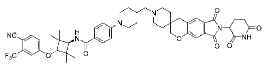

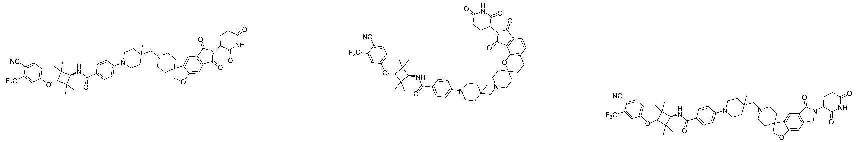

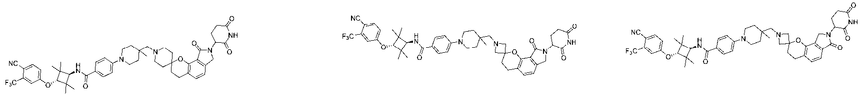

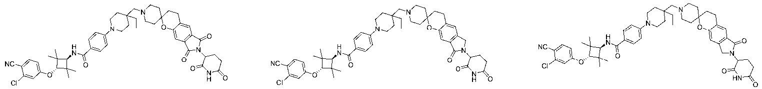

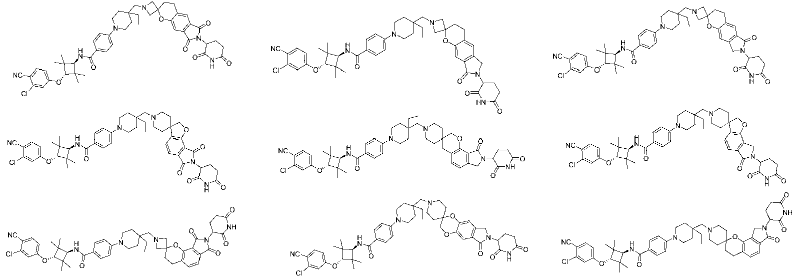

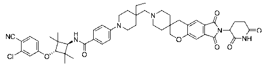

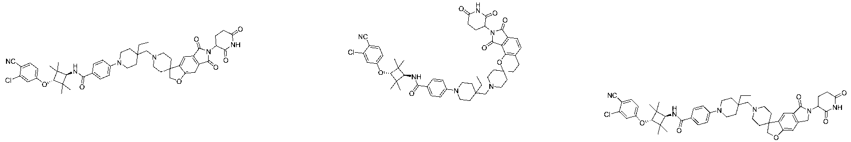

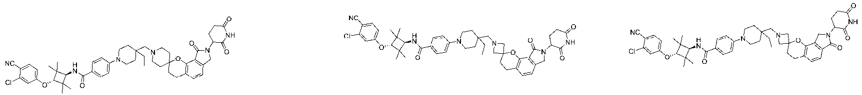

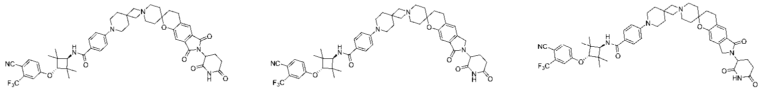

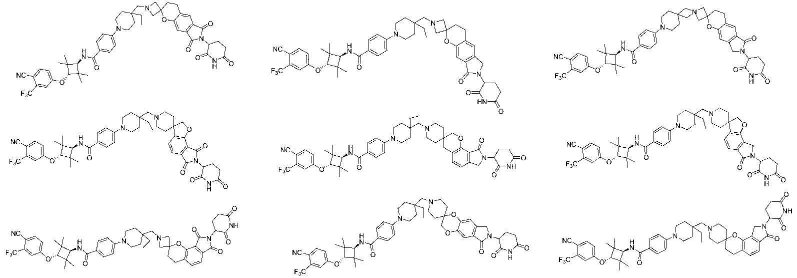

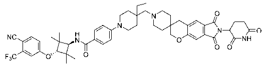

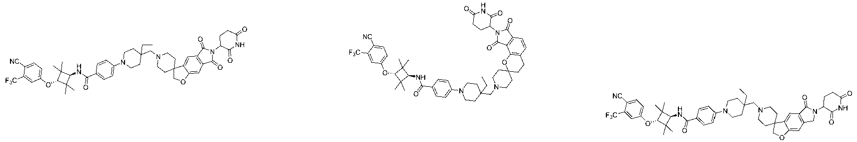

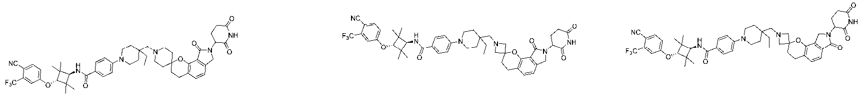

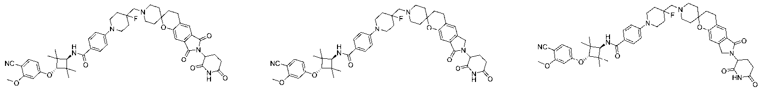

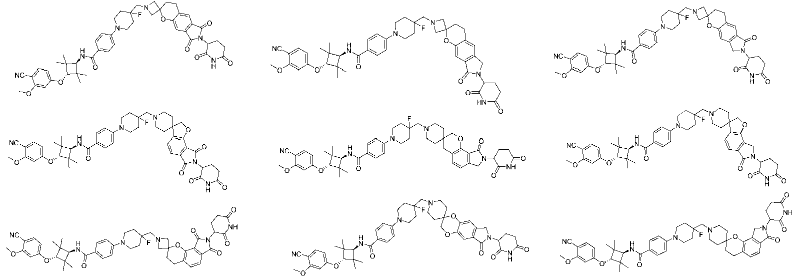

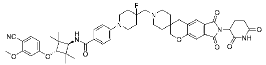

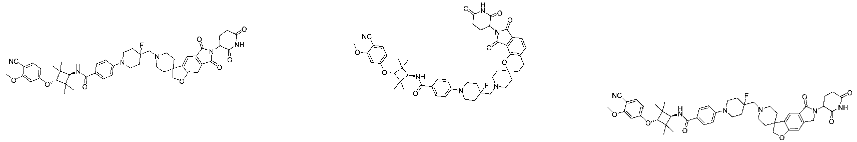

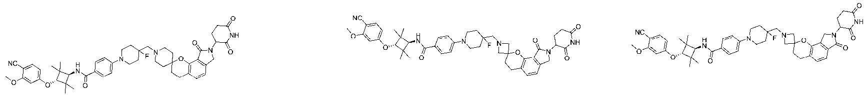

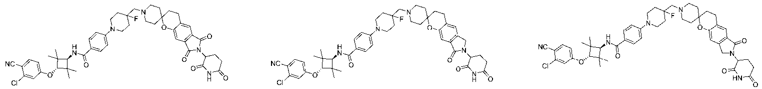

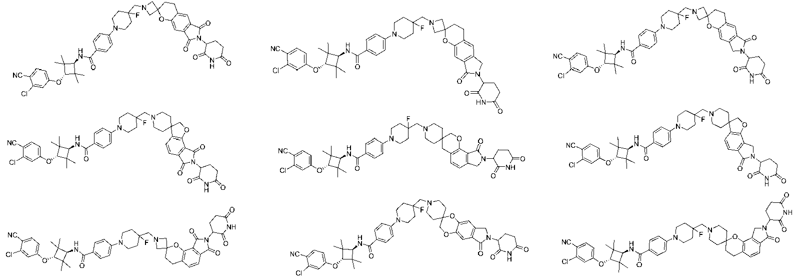

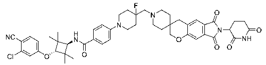

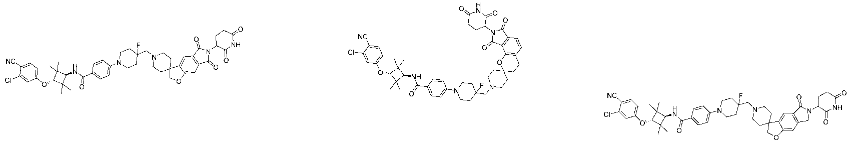

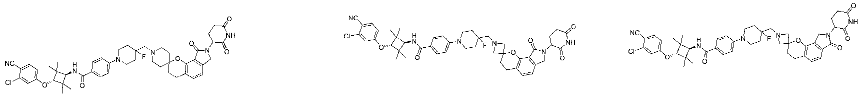

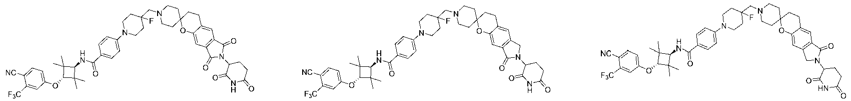

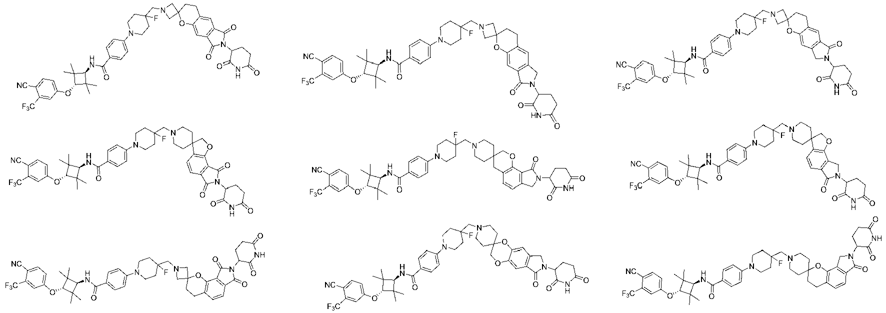

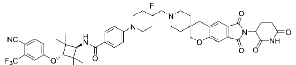

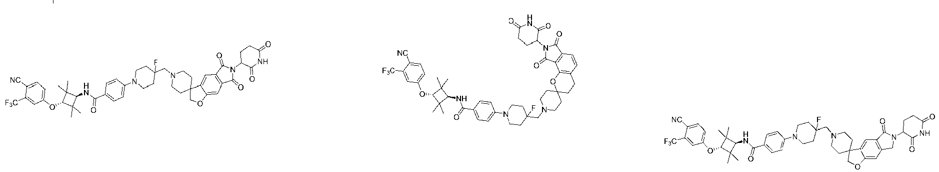

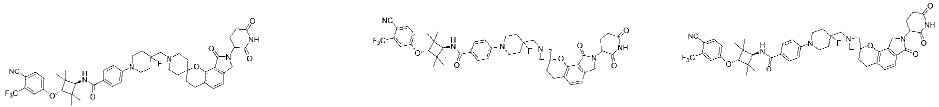

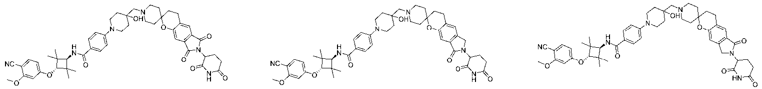

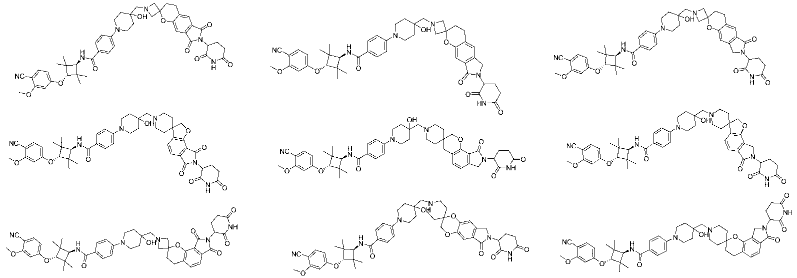

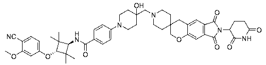

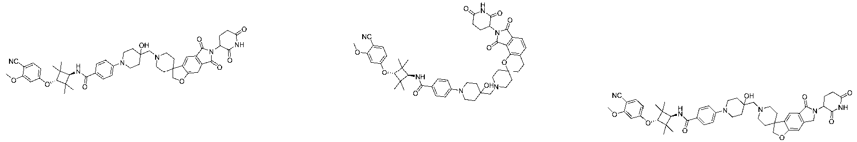

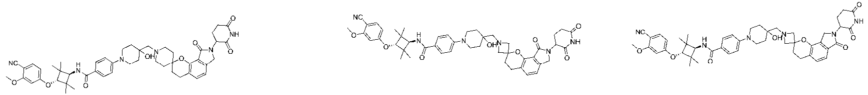

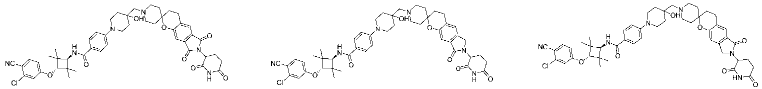

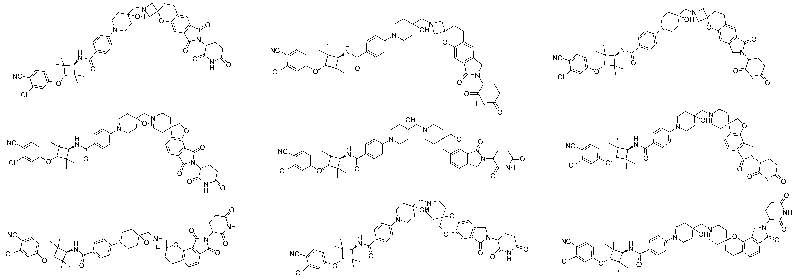

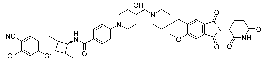

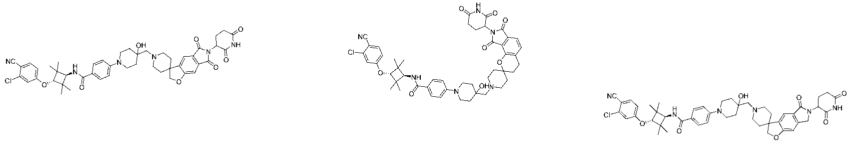

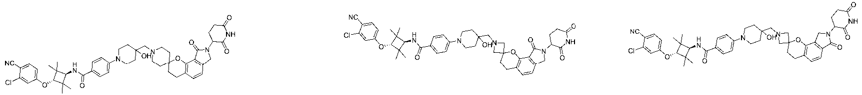

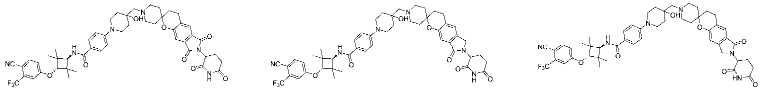

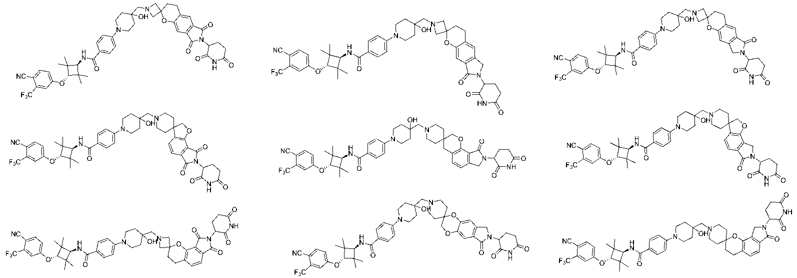

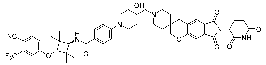

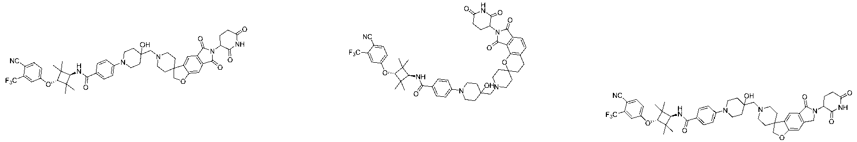

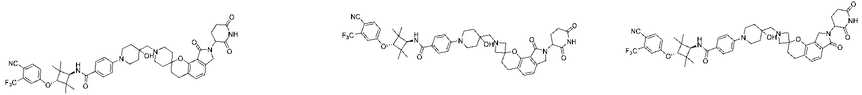

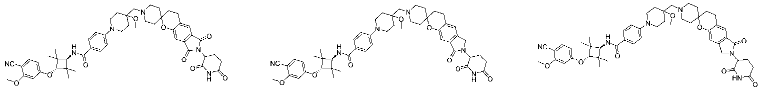

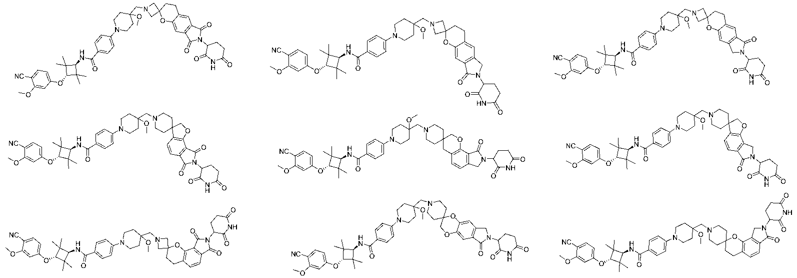

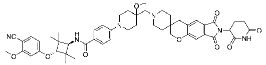

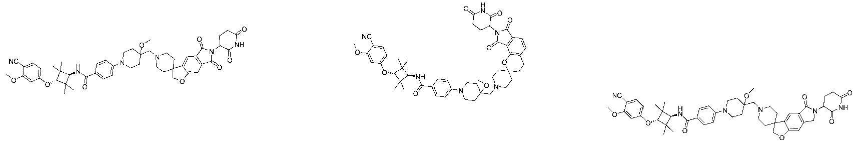

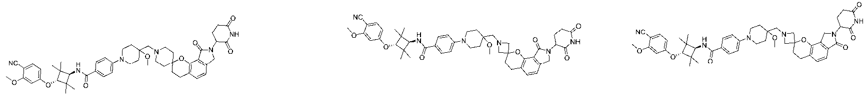

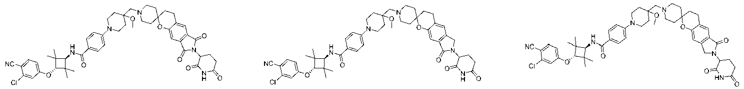

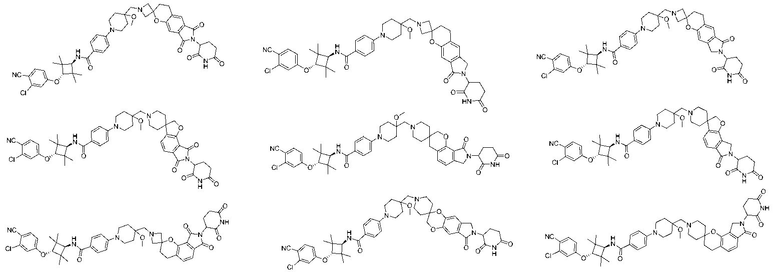

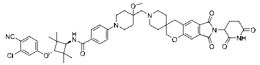

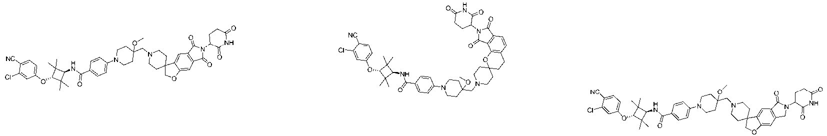

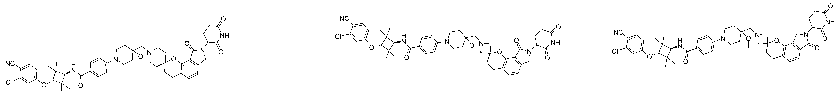

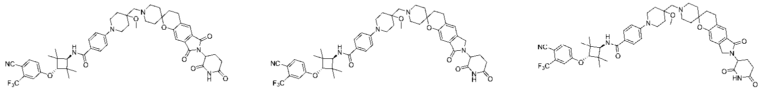

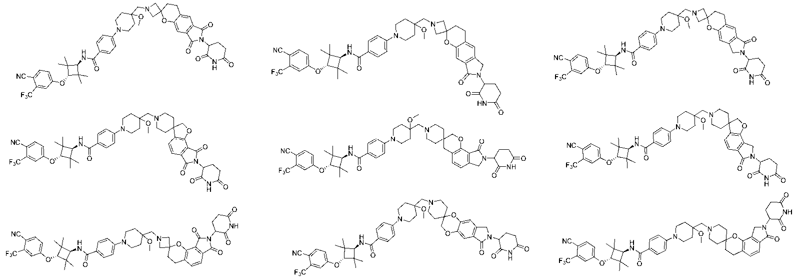

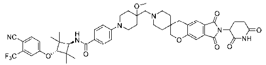

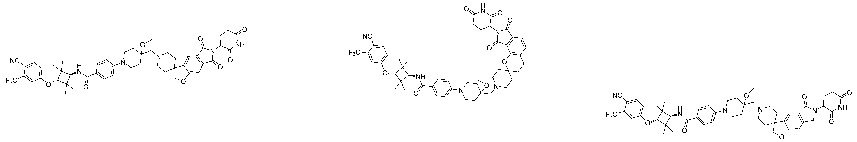

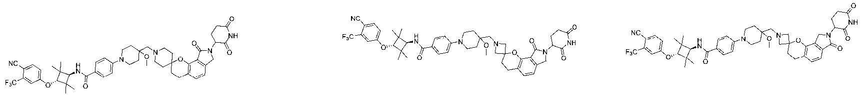

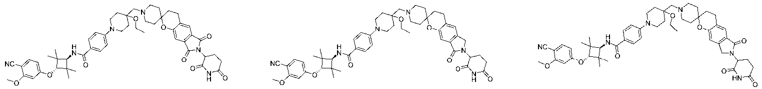

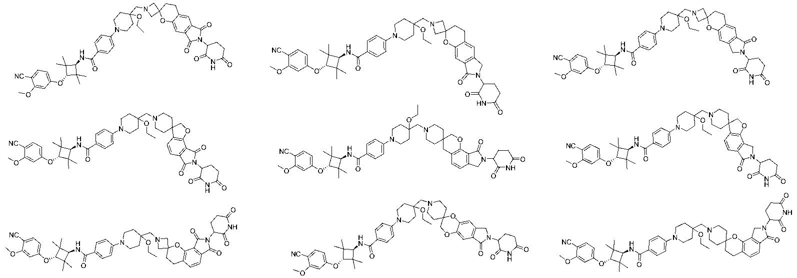

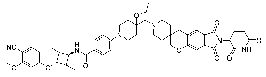

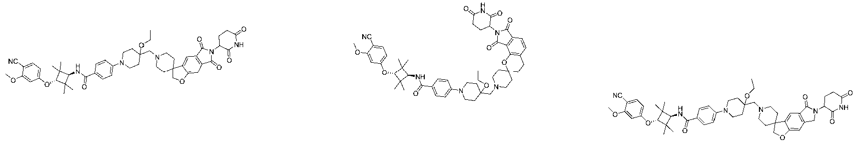

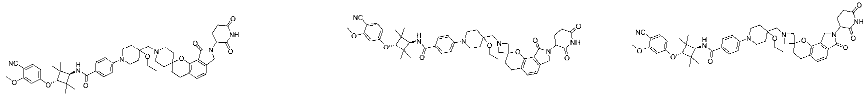

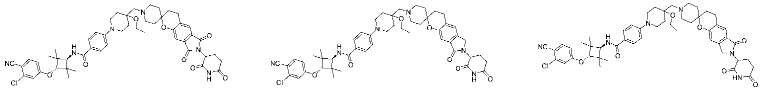

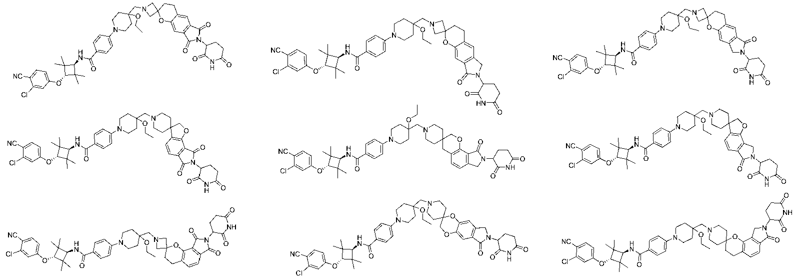

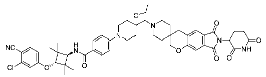

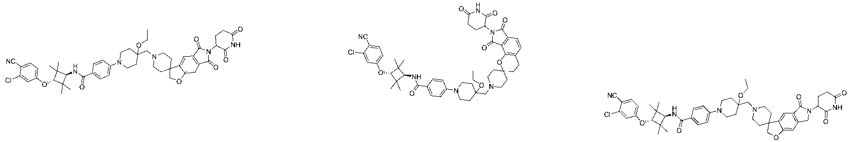

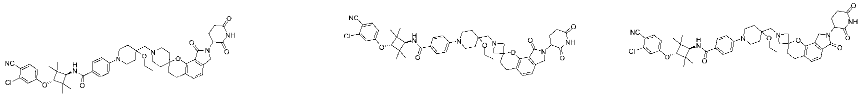

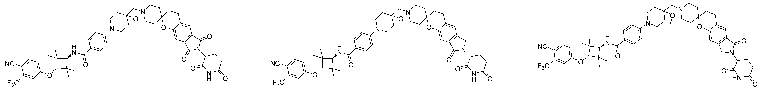

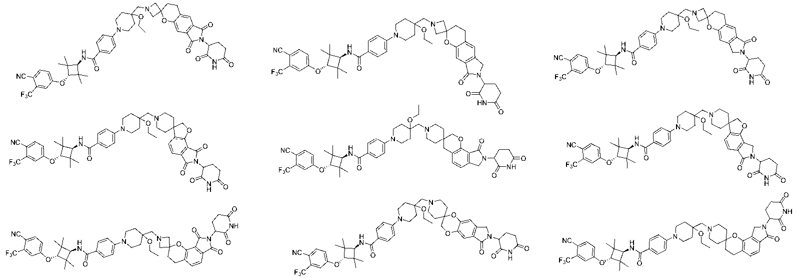

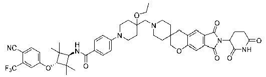

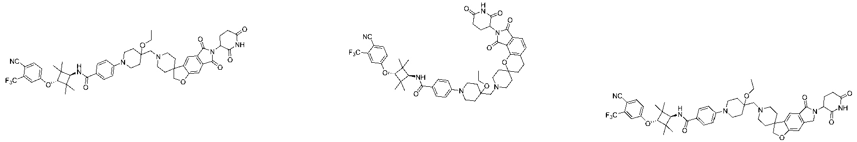

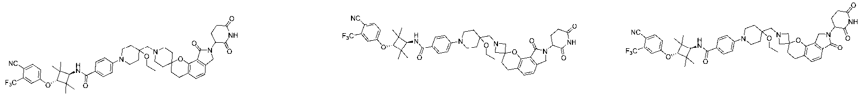

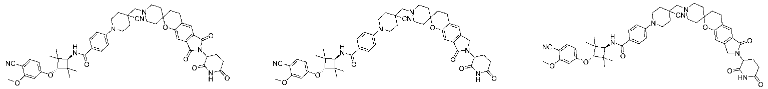

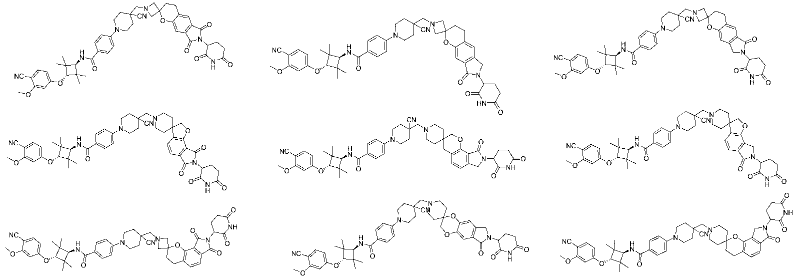

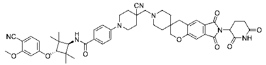

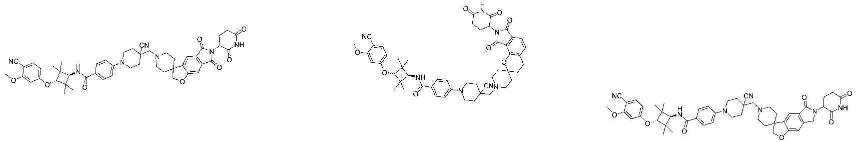

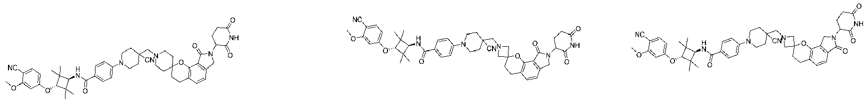

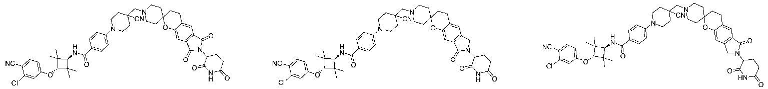

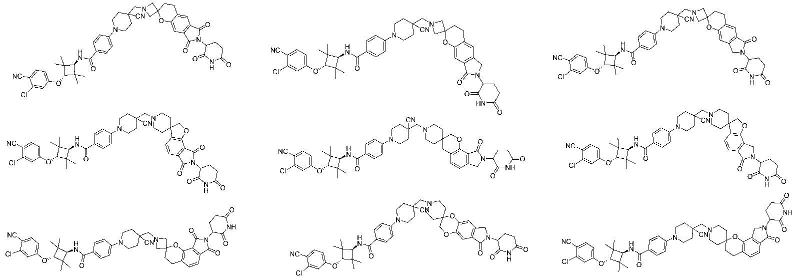

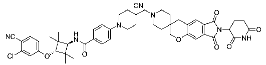

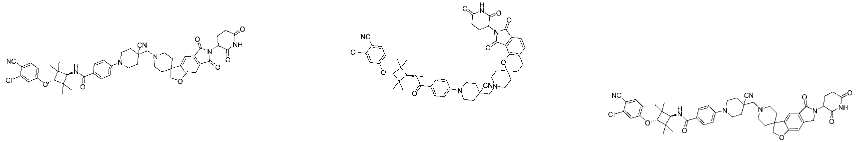

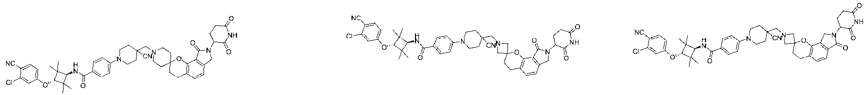

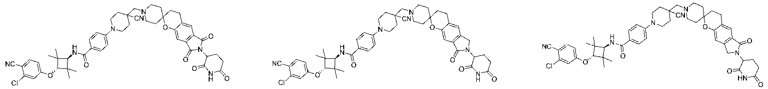

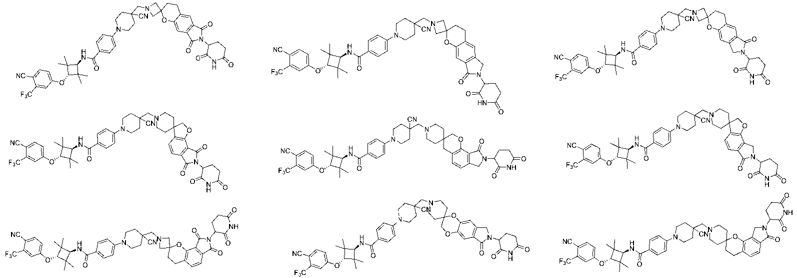

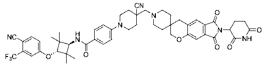

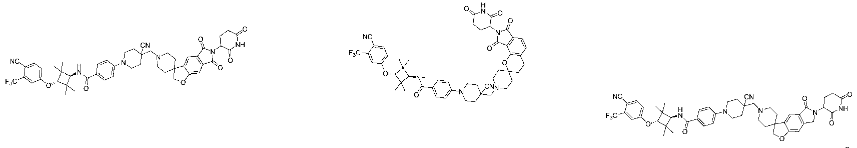

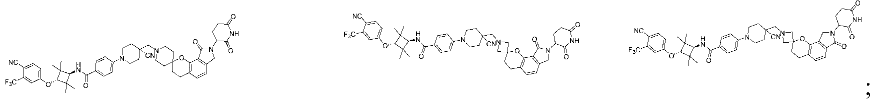

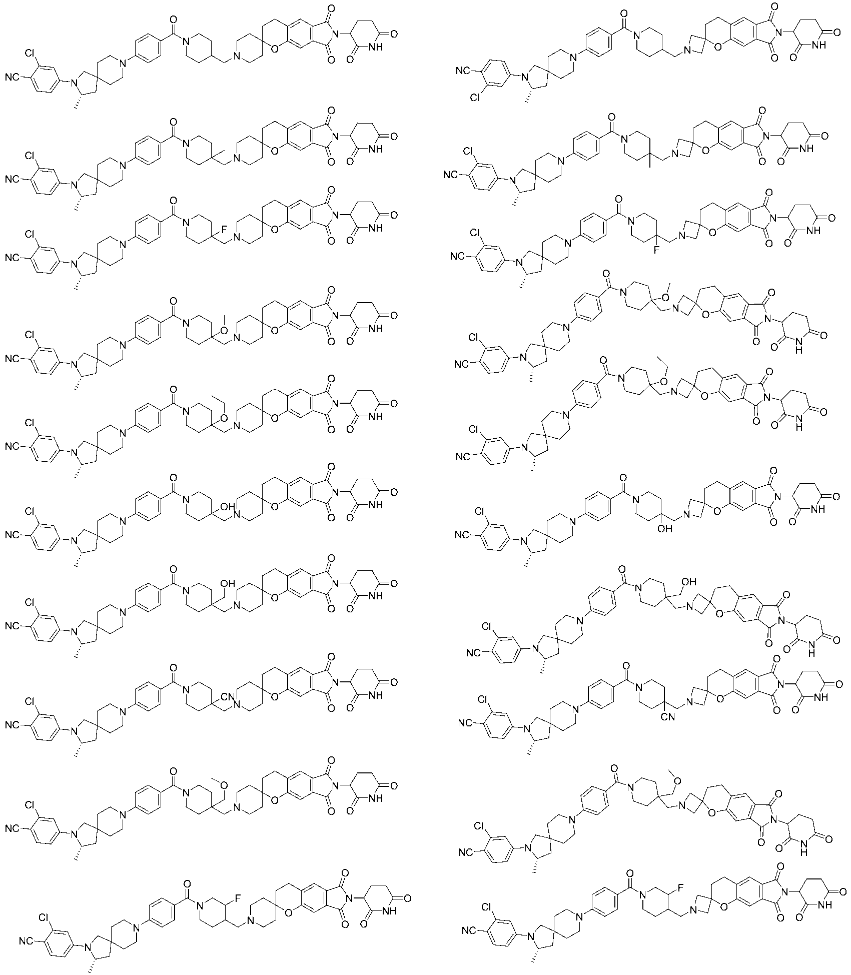

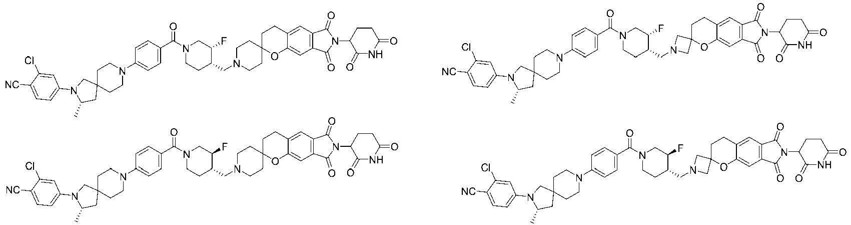

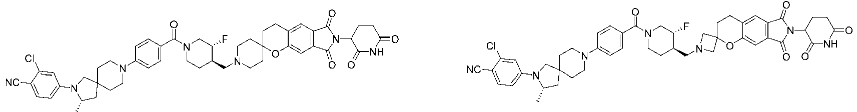

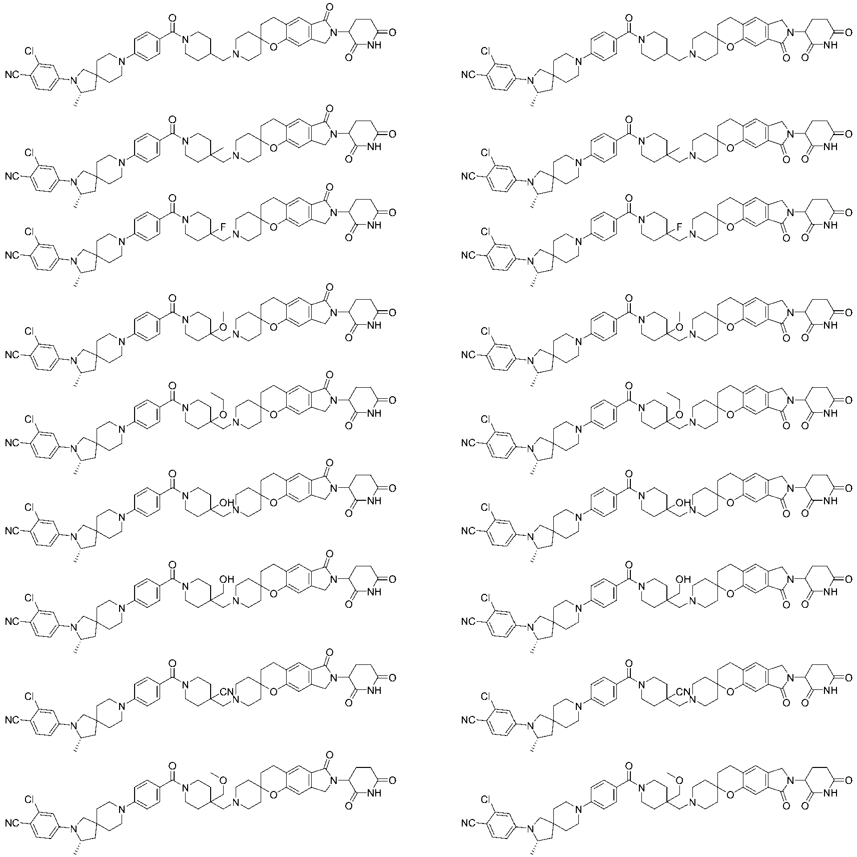

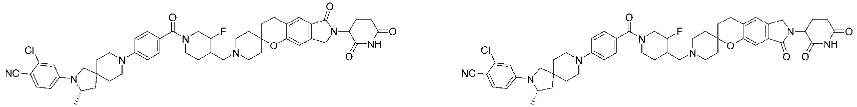

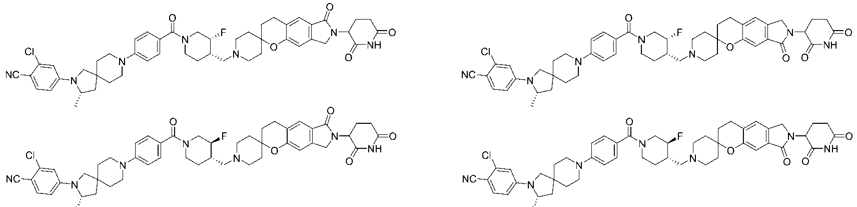

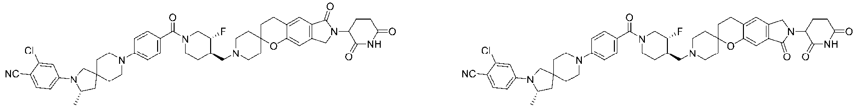

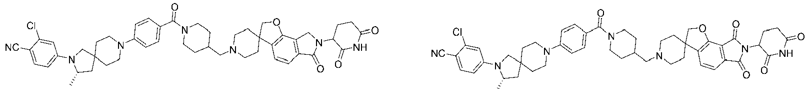

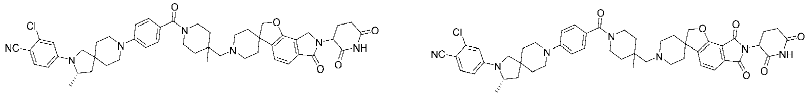

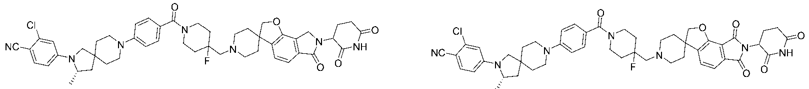

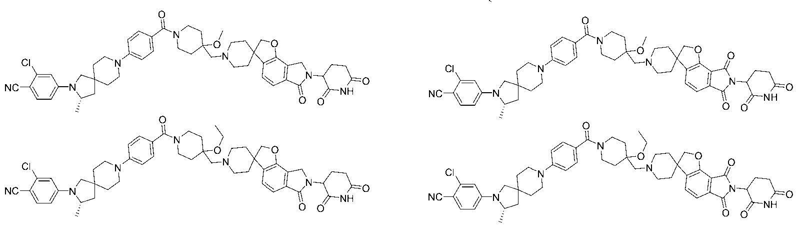

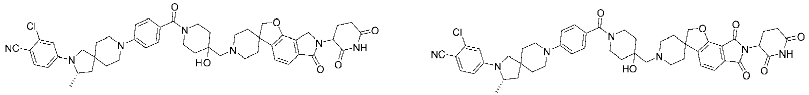

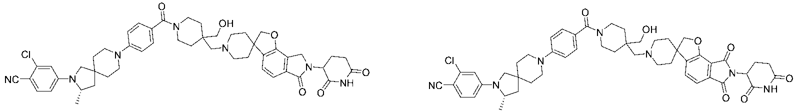

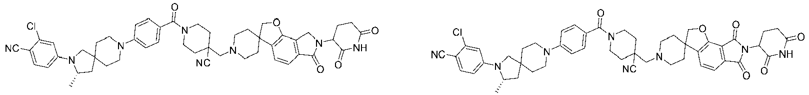

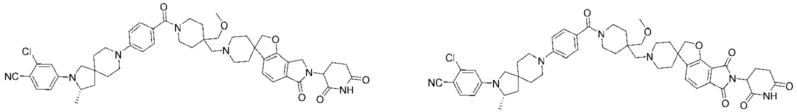

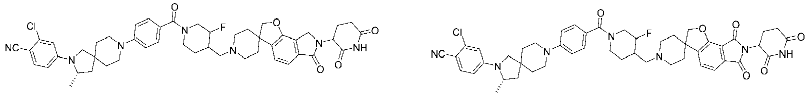

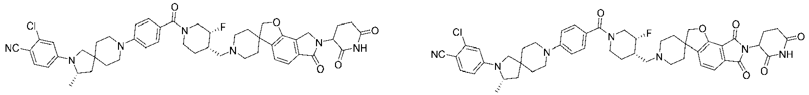

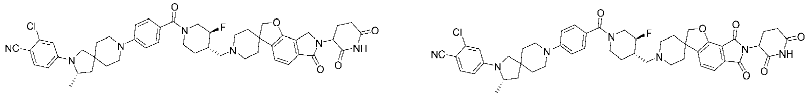

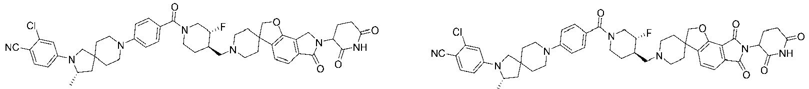

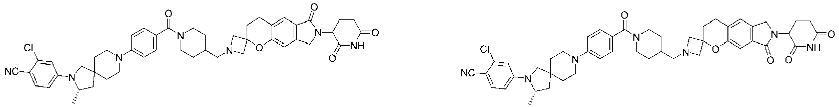

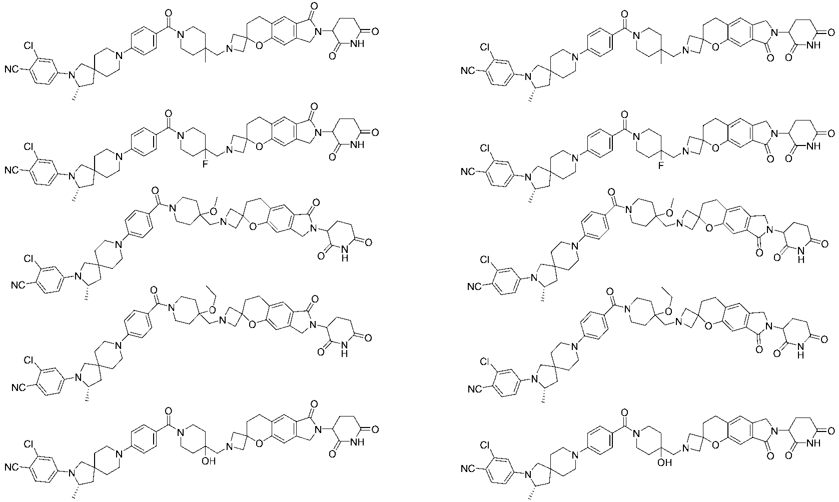

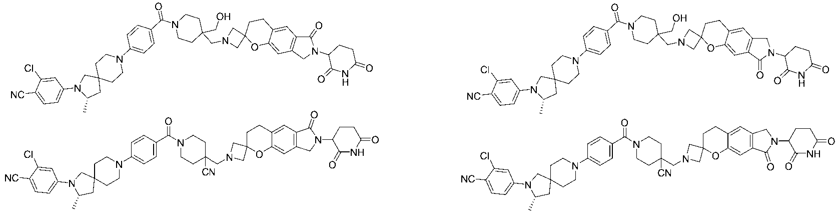

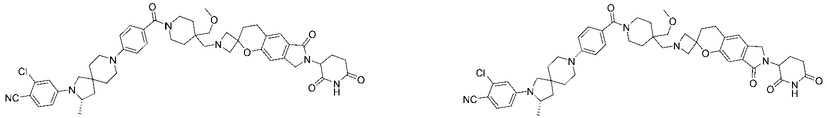

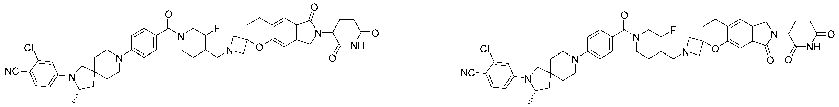

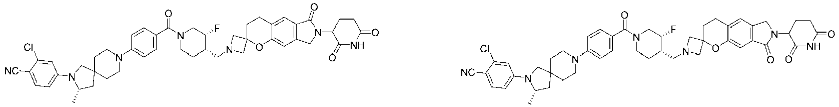

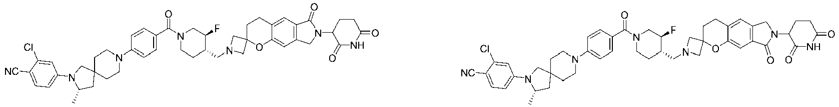

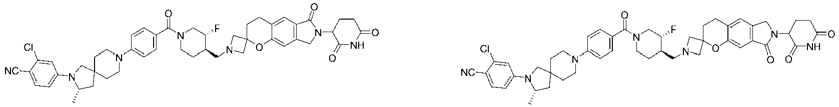

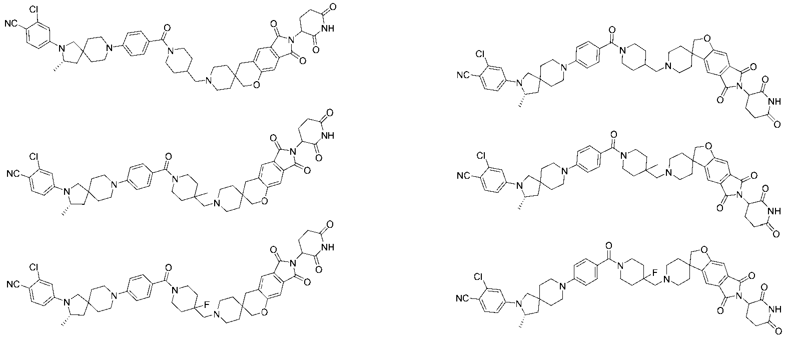

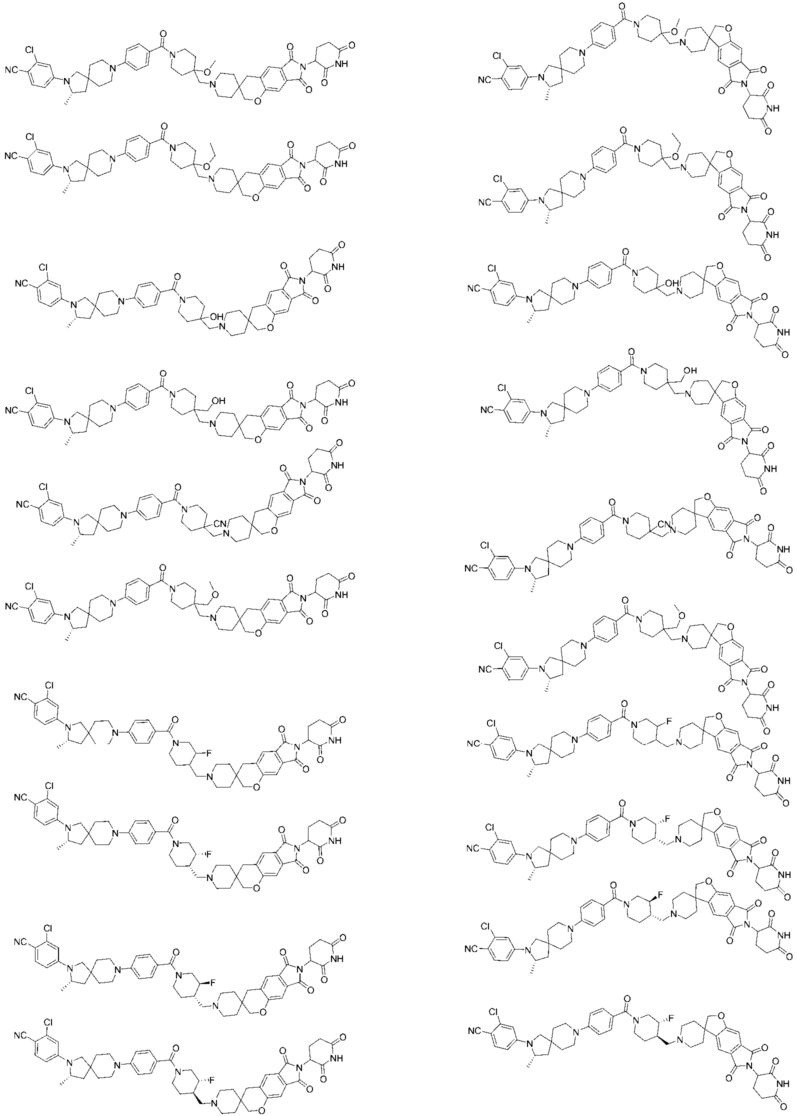

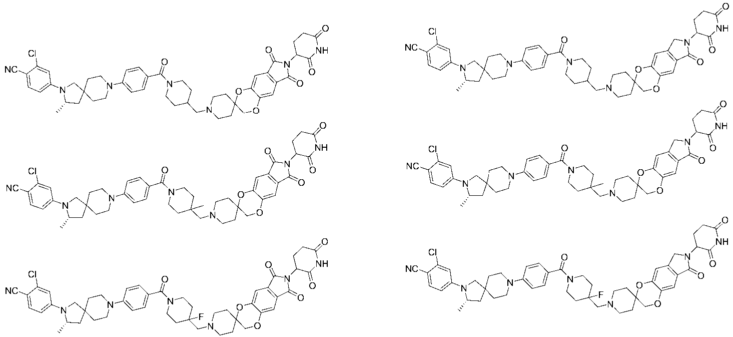

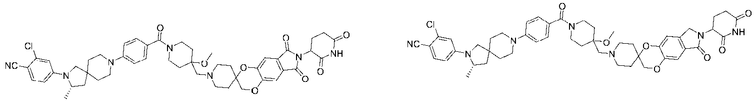

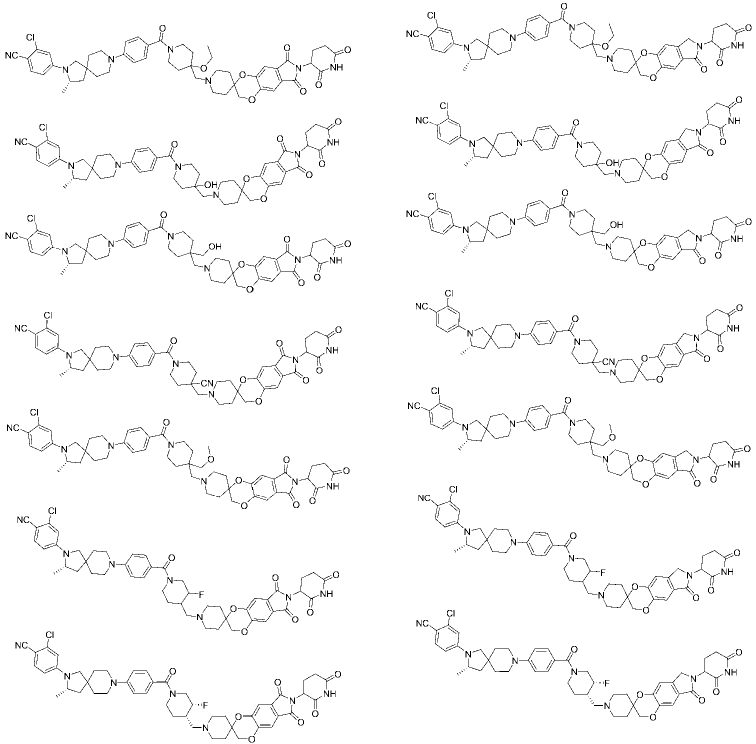

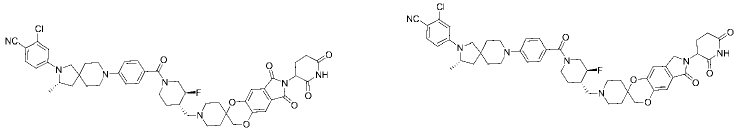

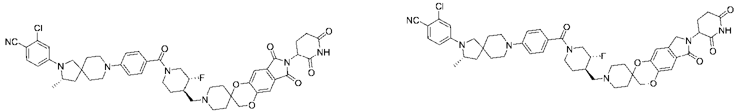

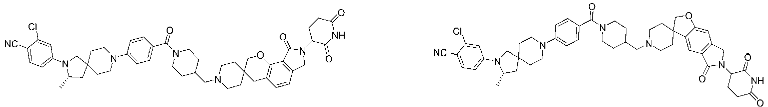

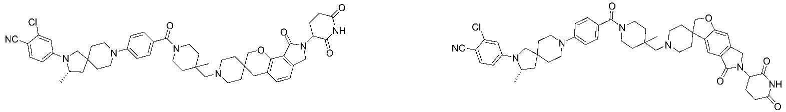

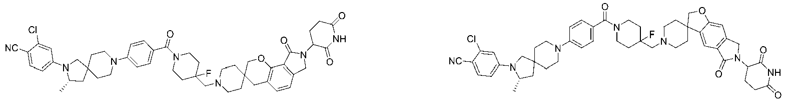

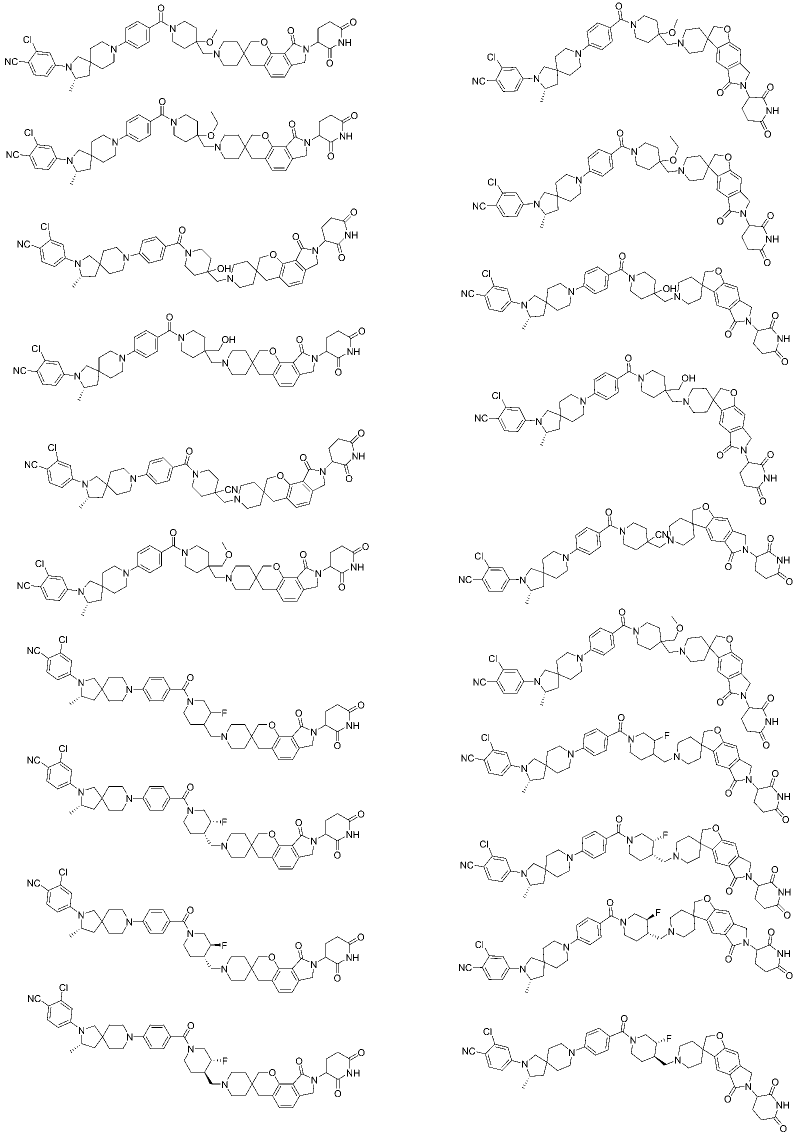

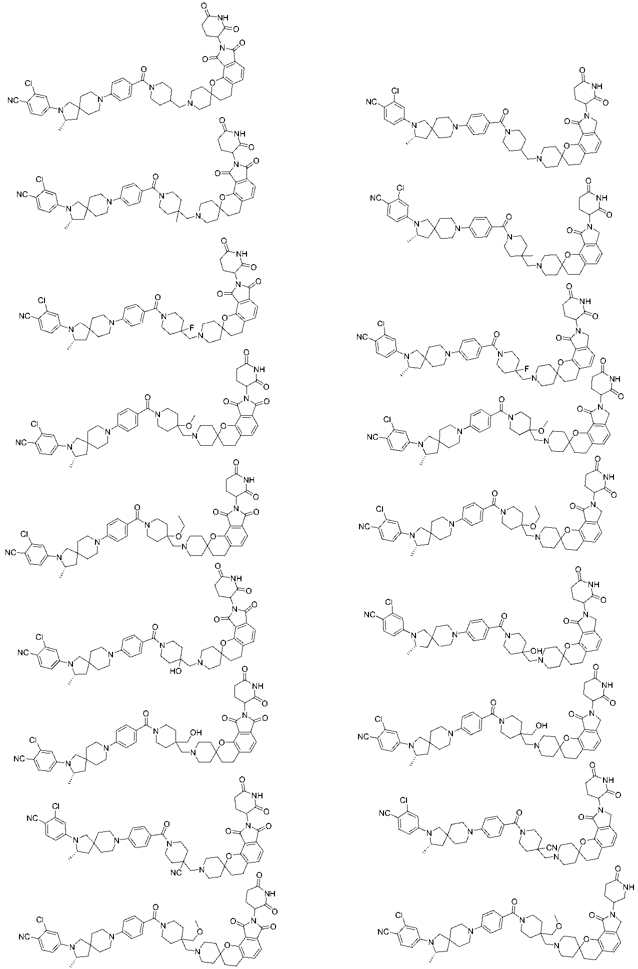

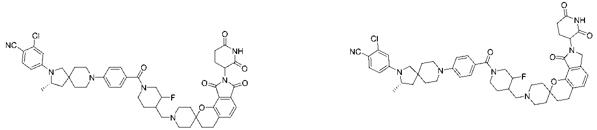

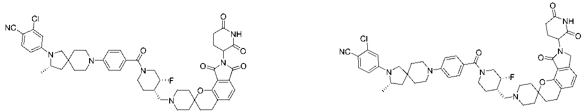

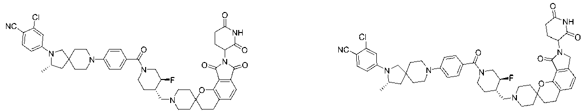

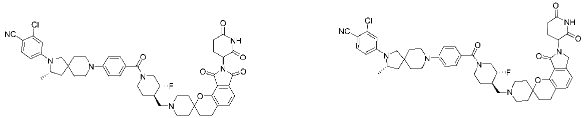

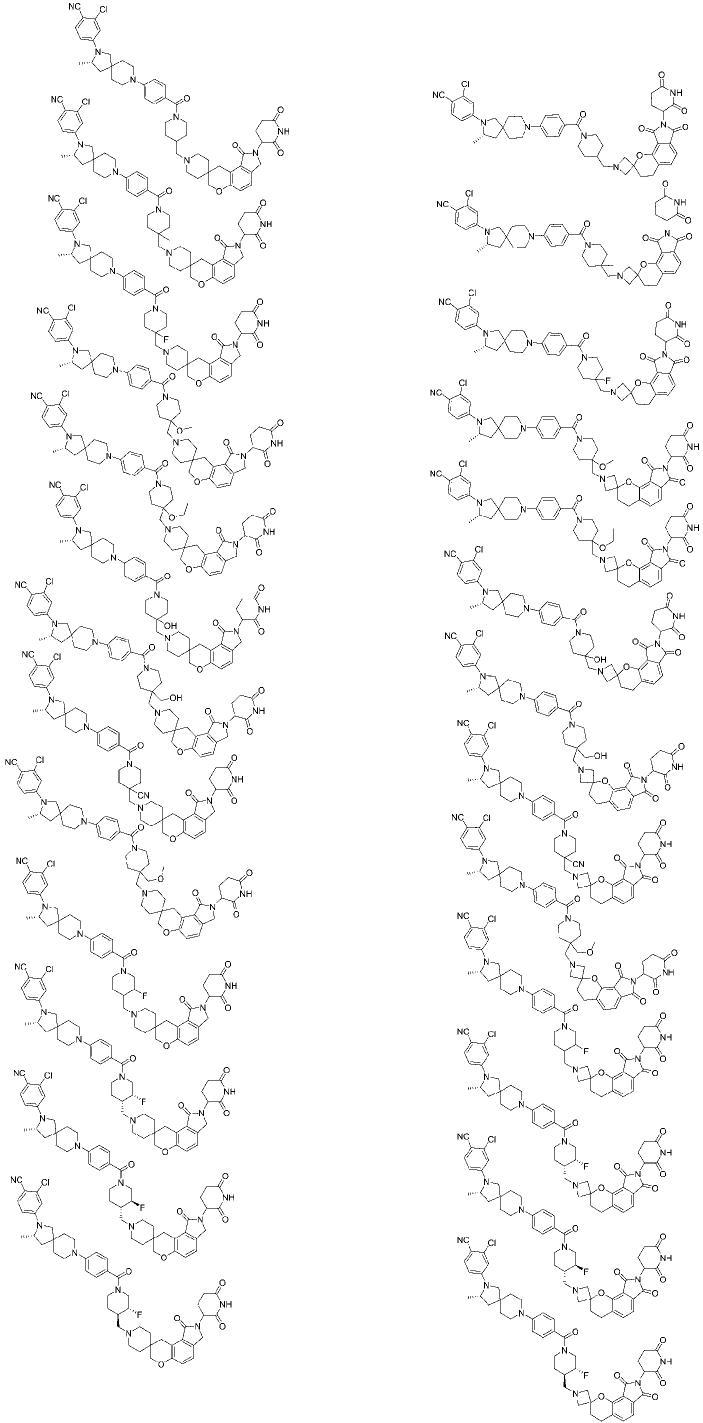

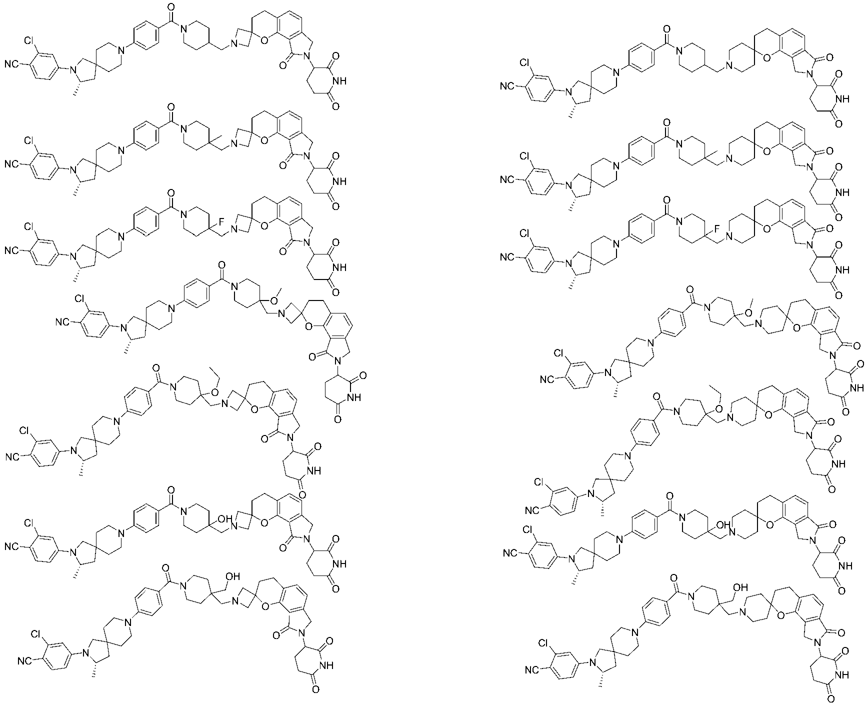

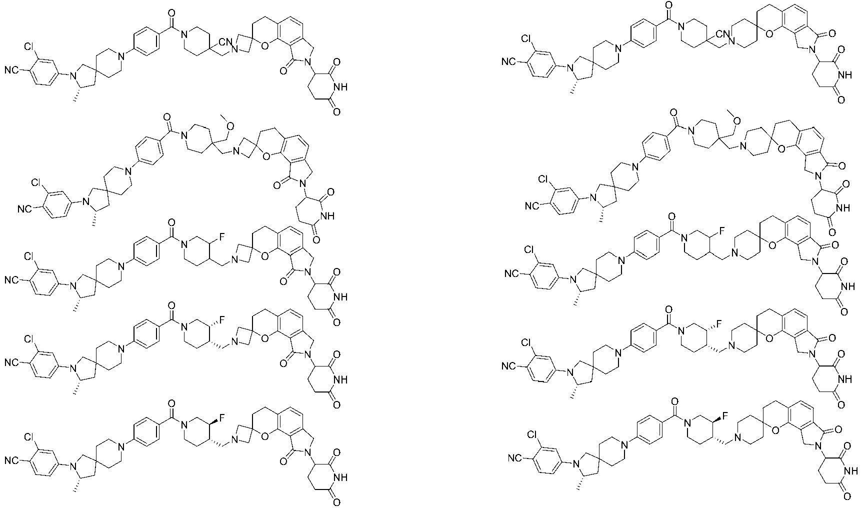

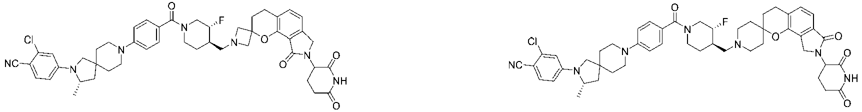

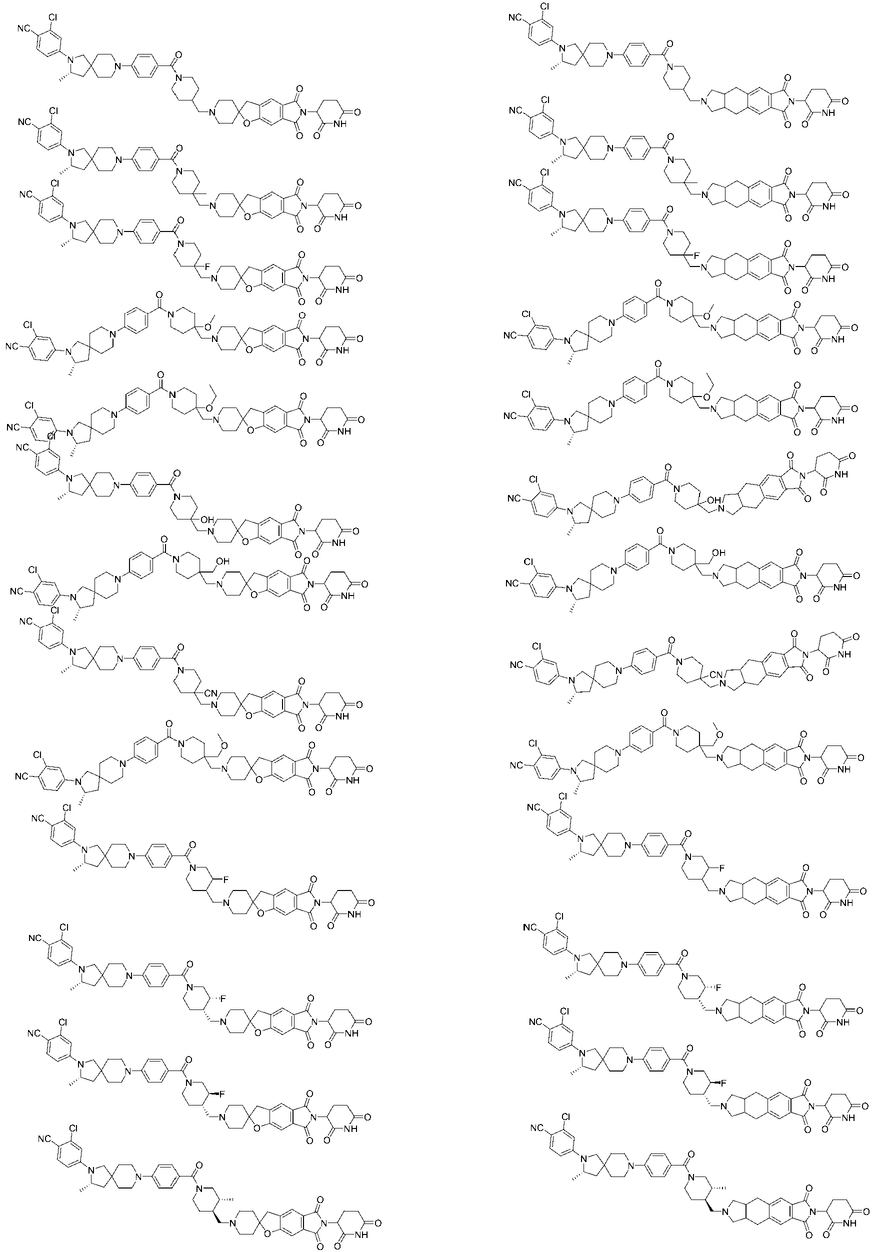

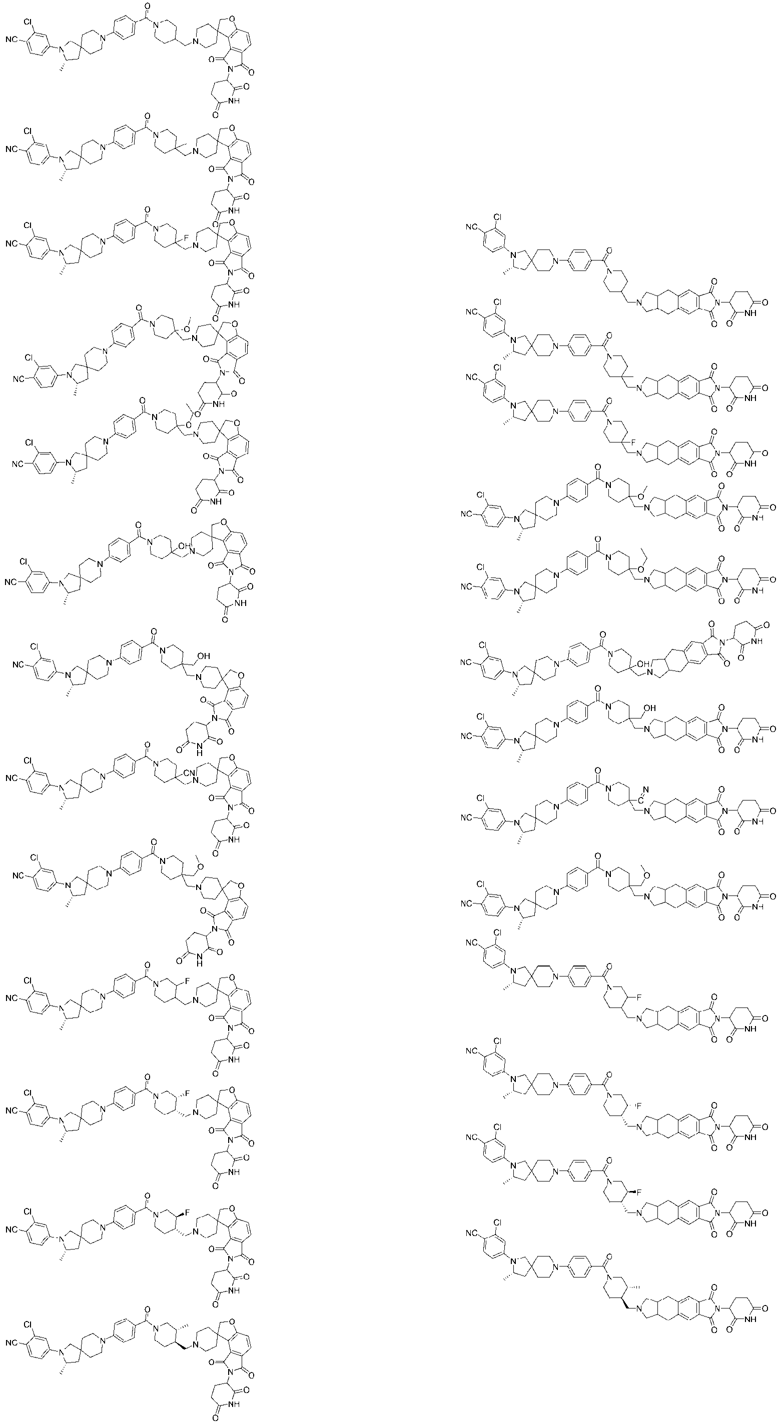

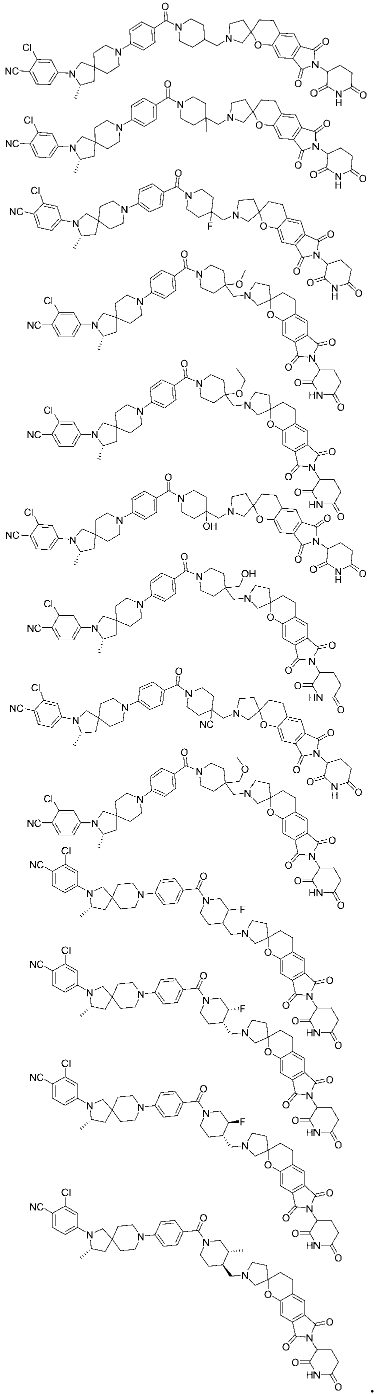

16. The compound according to claim 13 or 14, wherein the PTM is selected from the following structures: 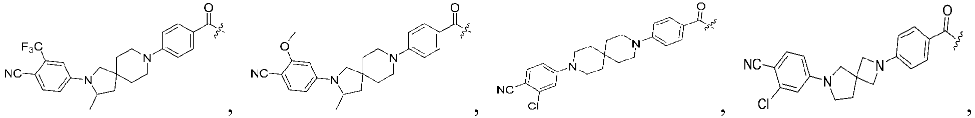 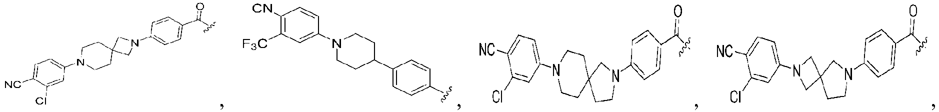 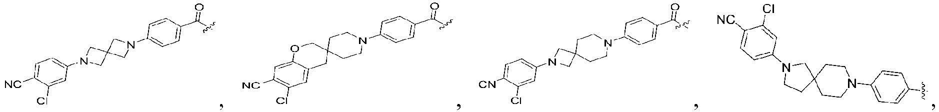 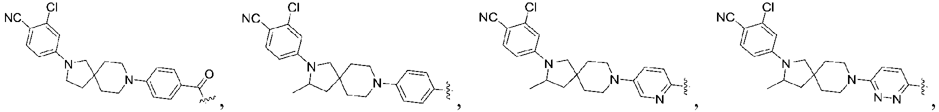 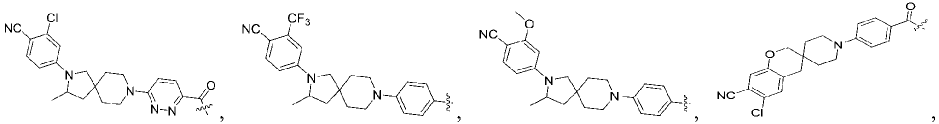 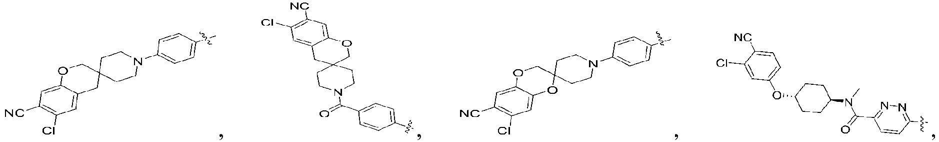 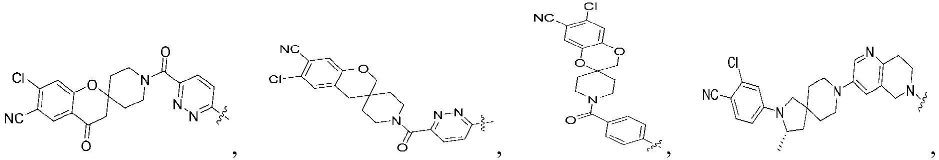 and

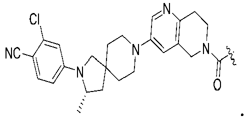

17. The compound according to any one of claims 1-14, wherein the compound of formula I is selected from the folllowing compounds:

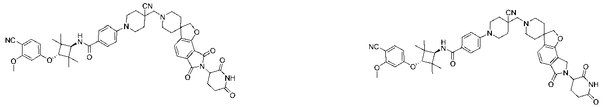

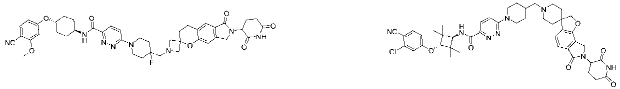

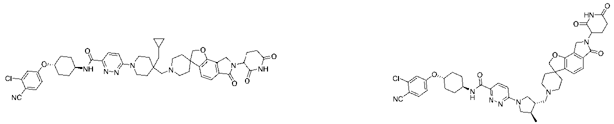

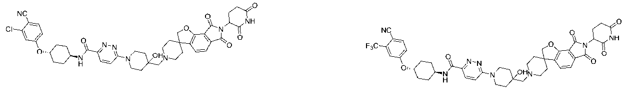

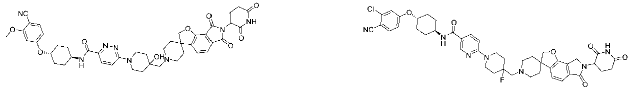

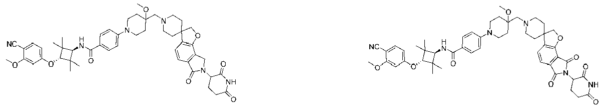

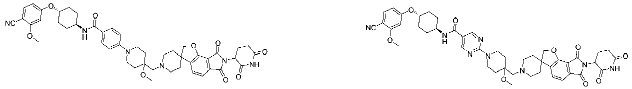

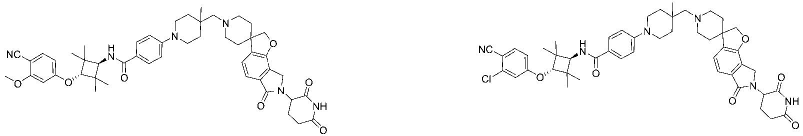

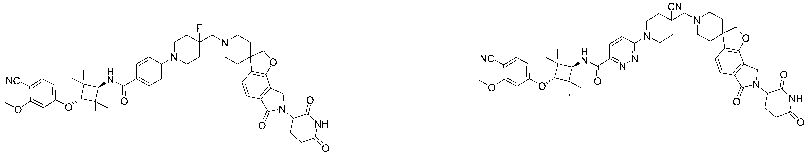

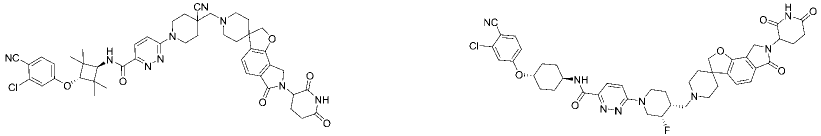

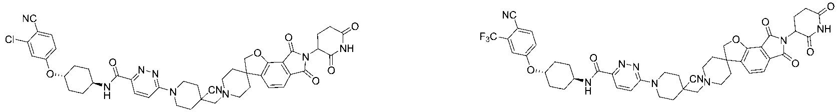

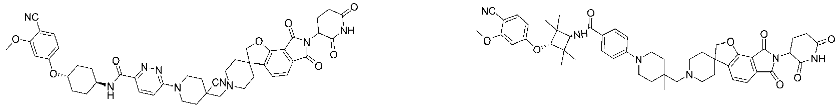

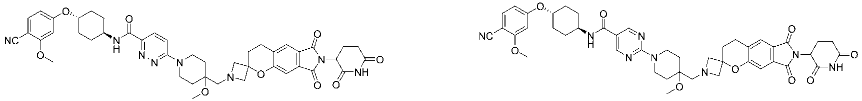

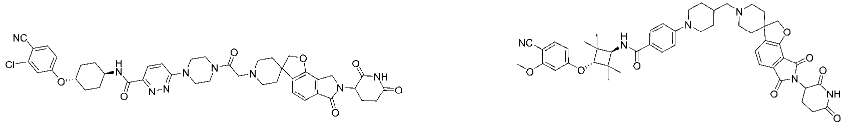

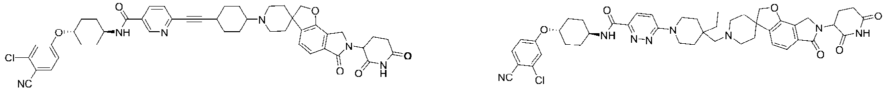

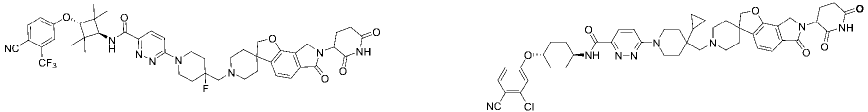

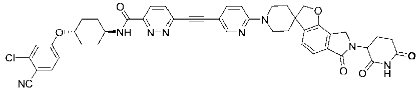

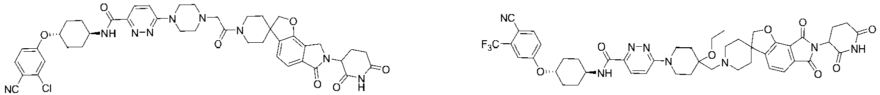

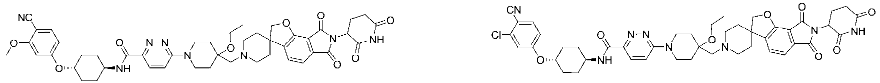

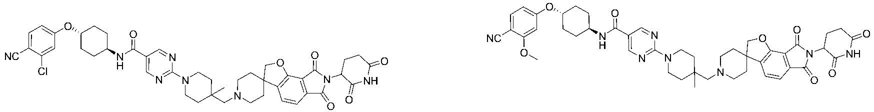

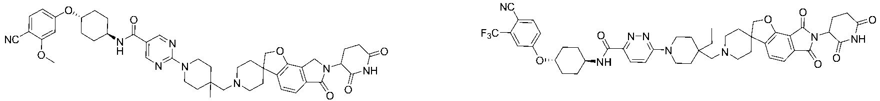

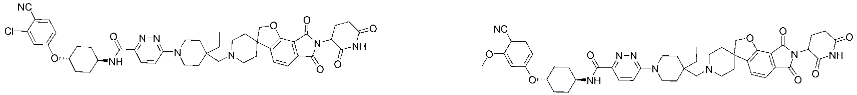

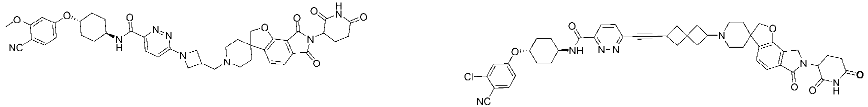

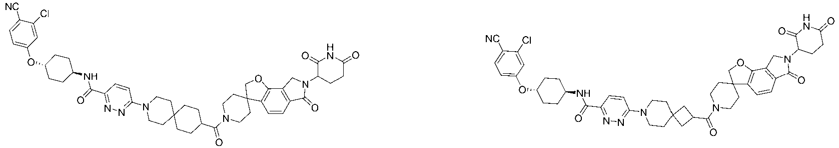

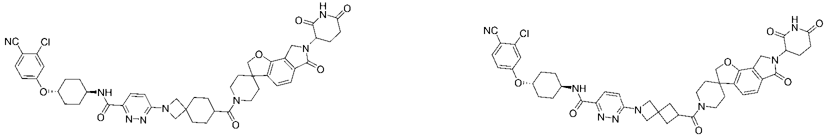

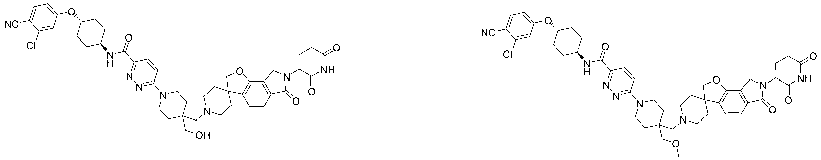

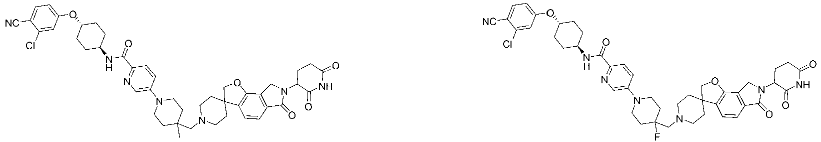

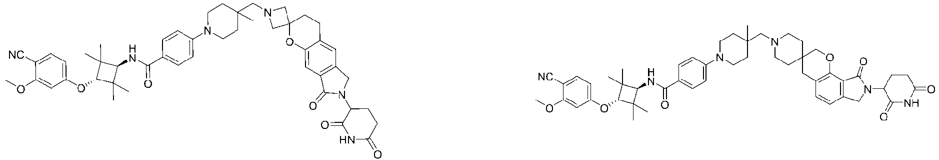

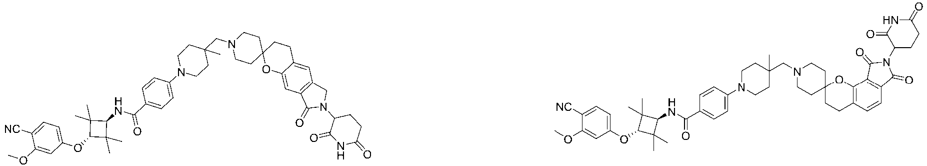

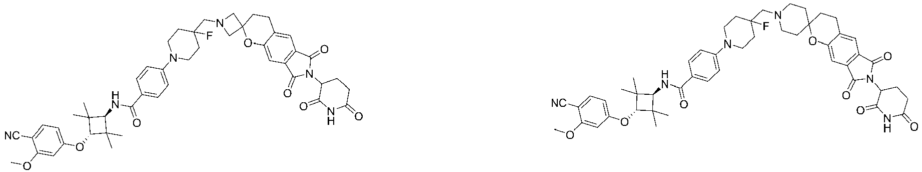

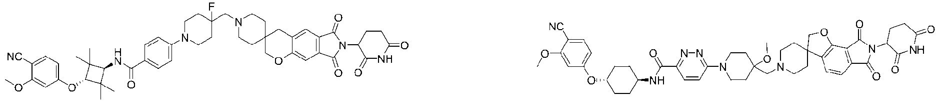

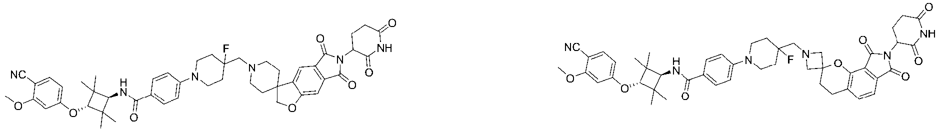

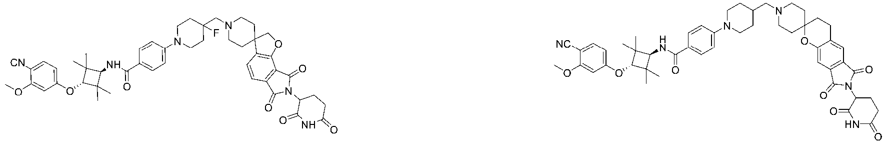

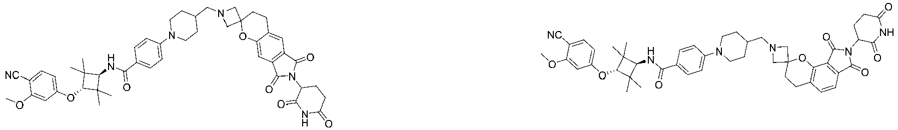

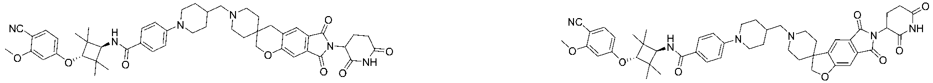

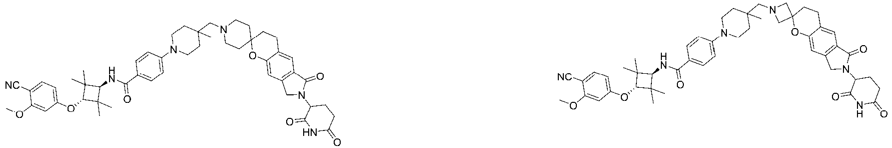

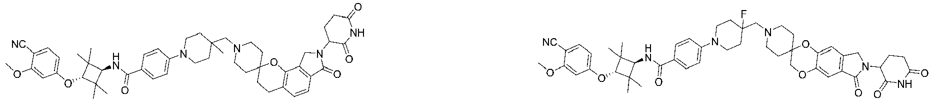

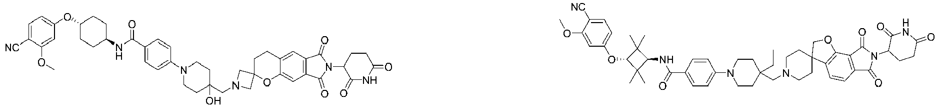

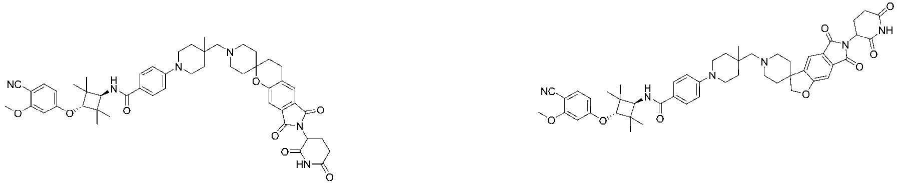

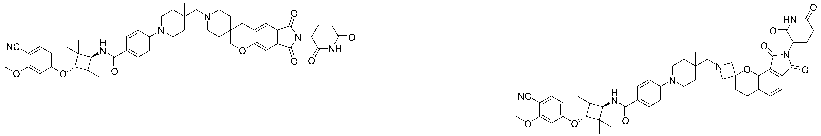

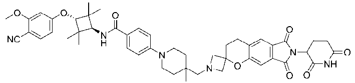

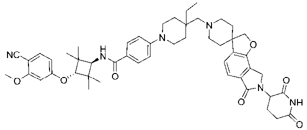

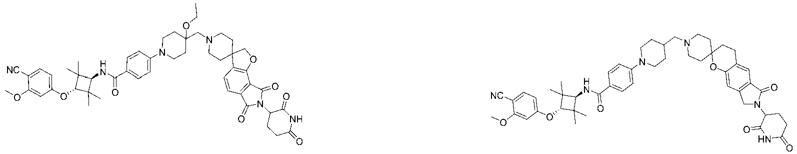

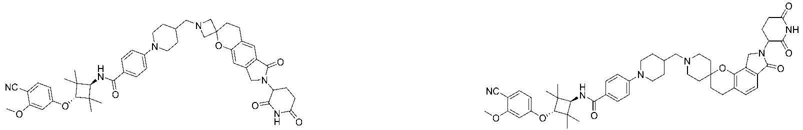

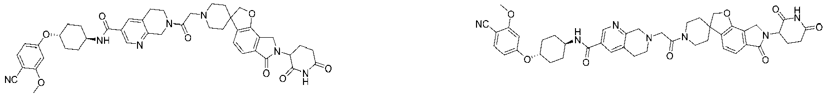

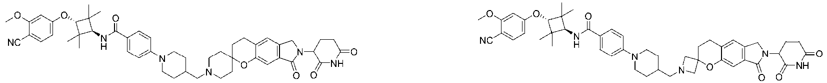

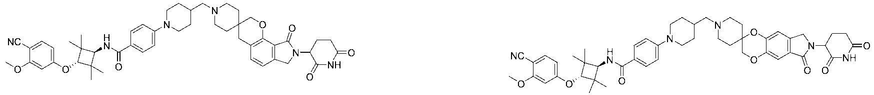

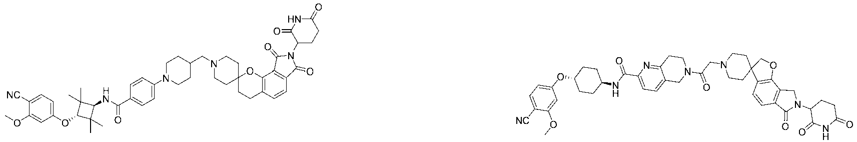

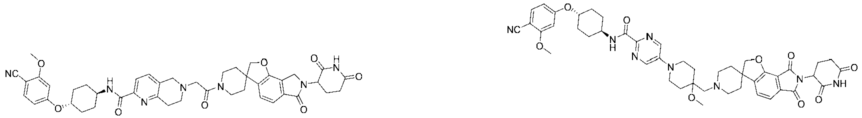

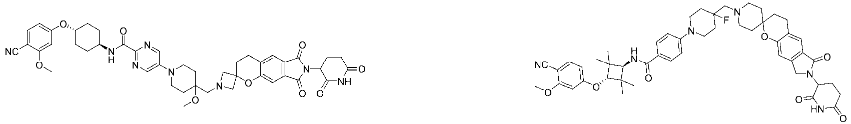

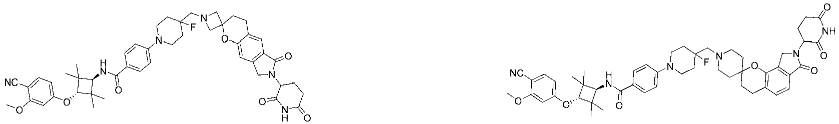

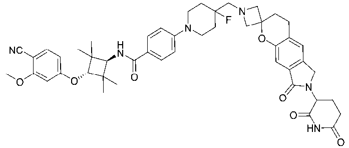

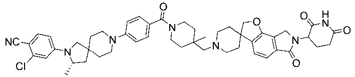

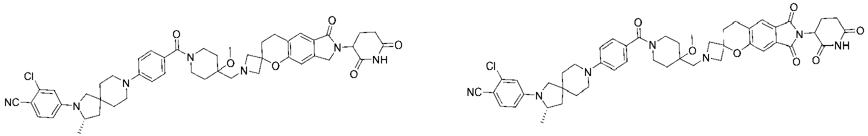

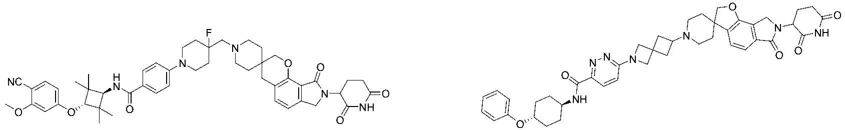

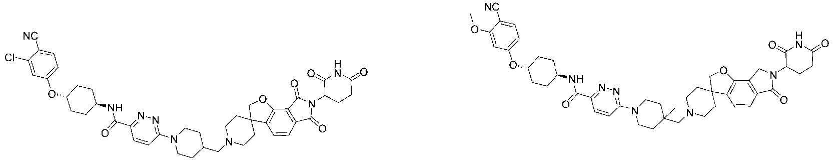

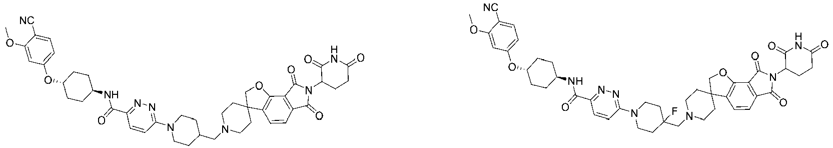

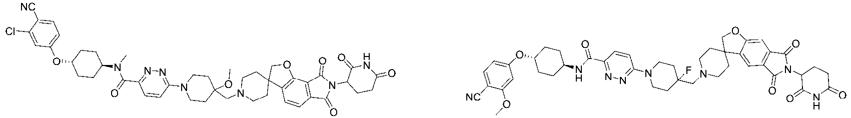

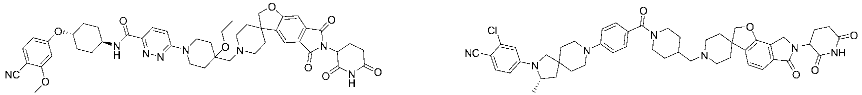

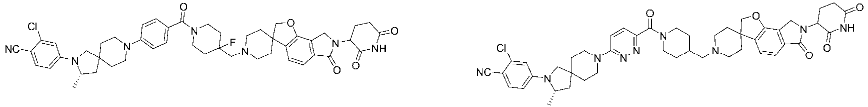

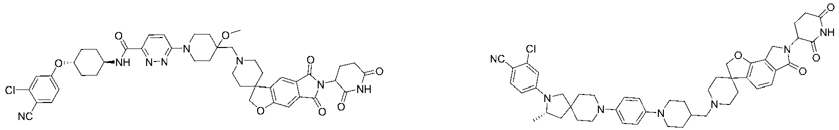

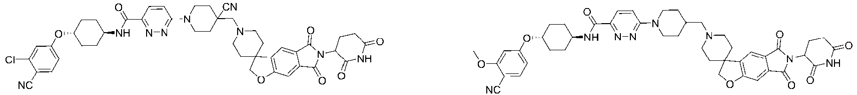

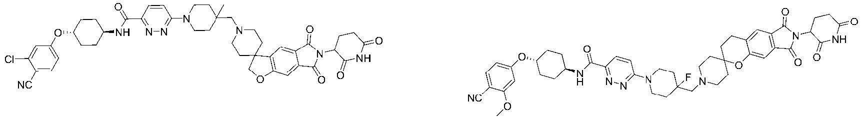

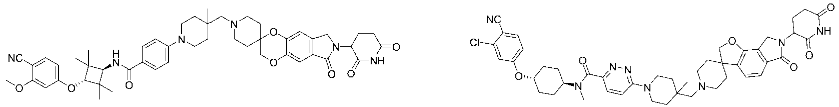

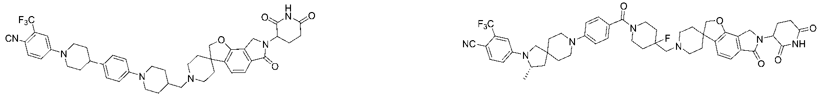

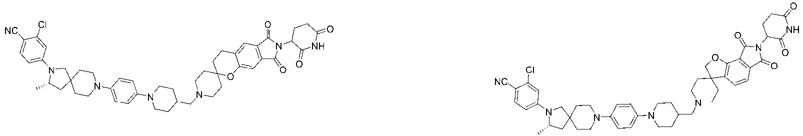

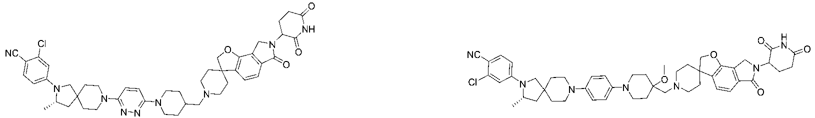

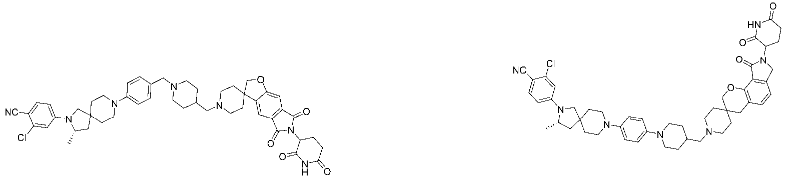

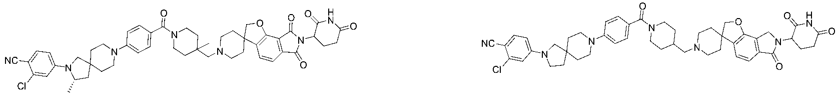

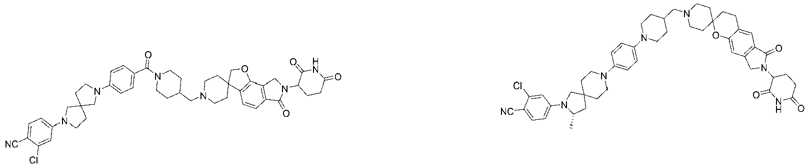

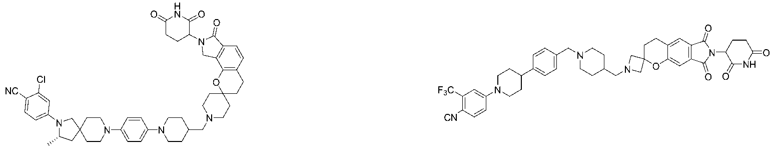

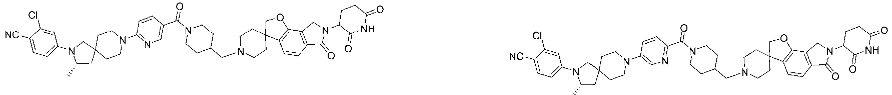

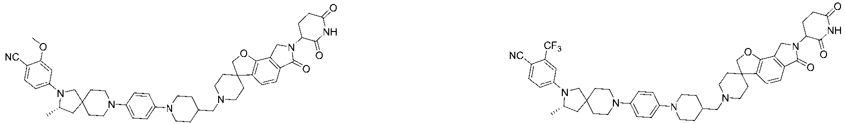

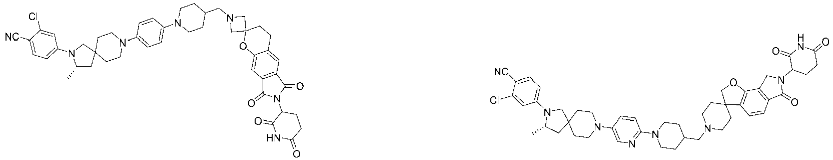

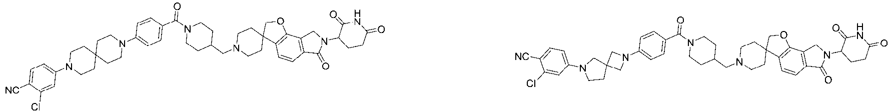

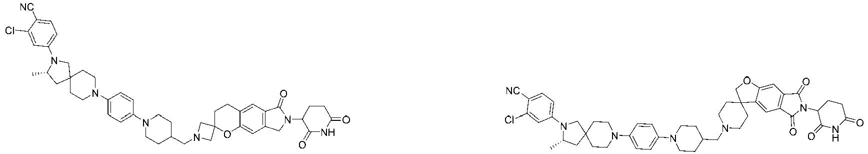

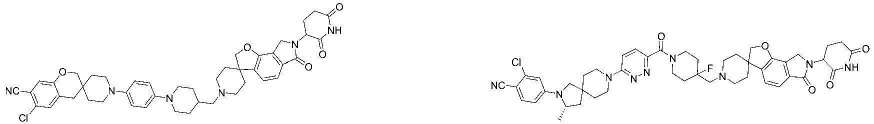

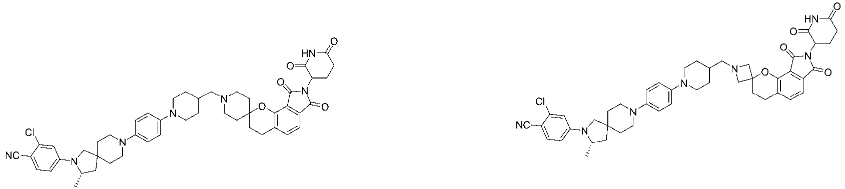

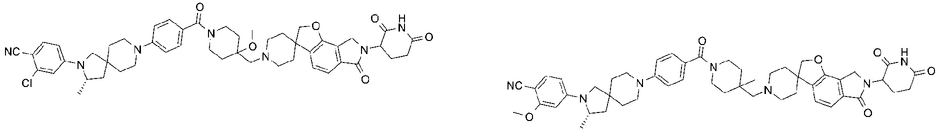

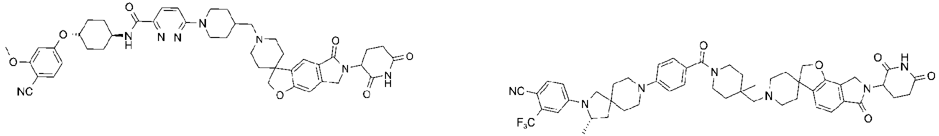

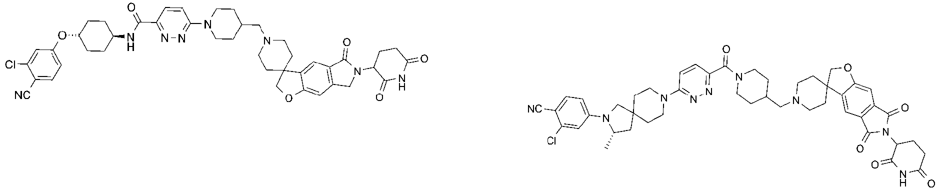

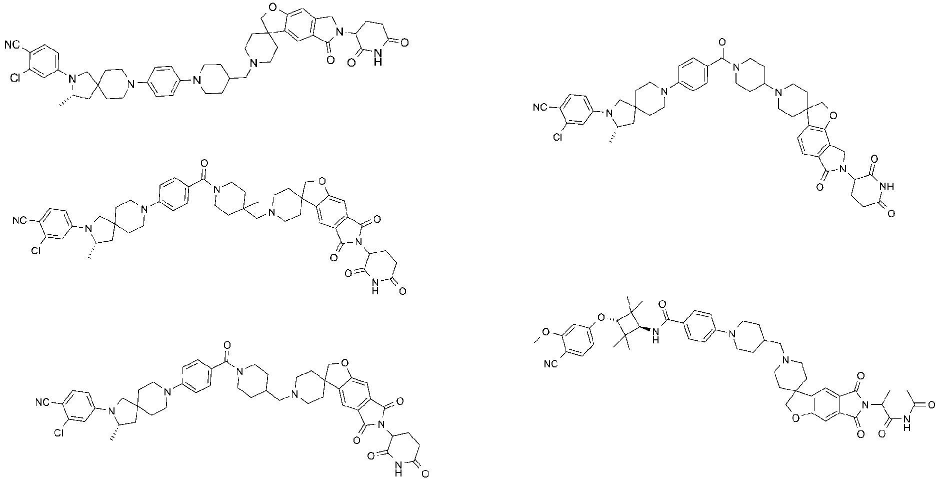

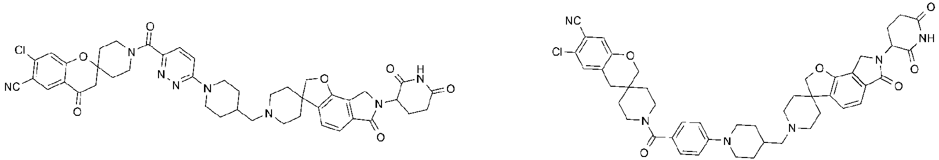

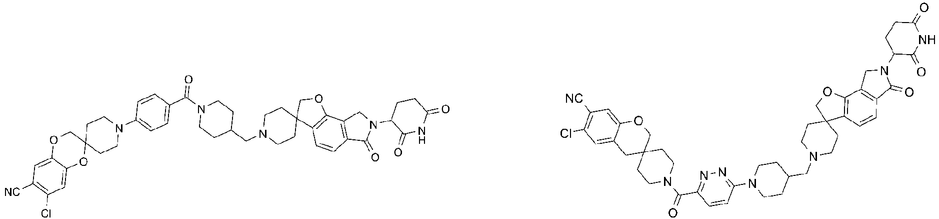

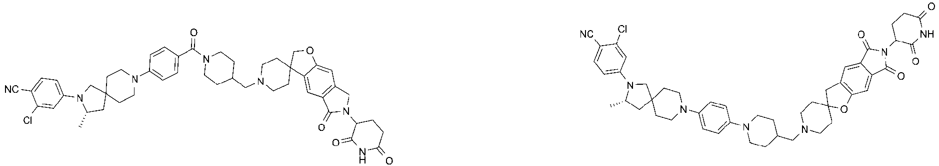

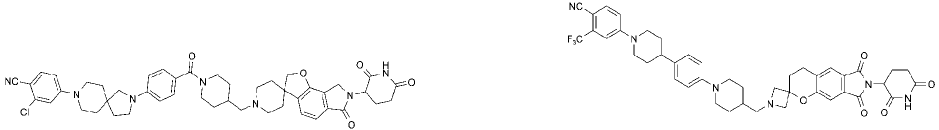

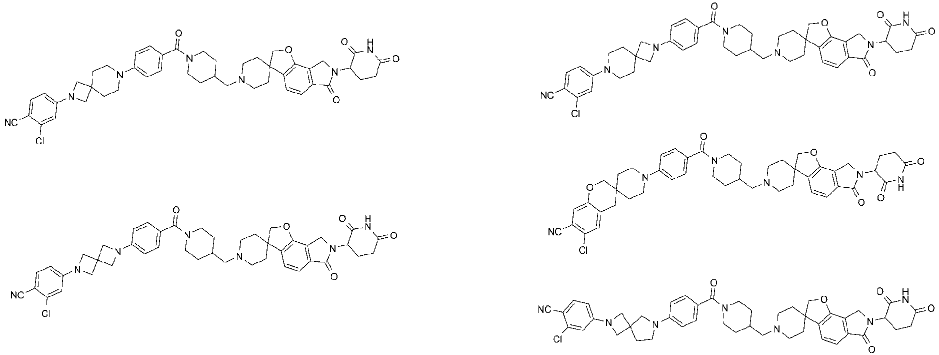

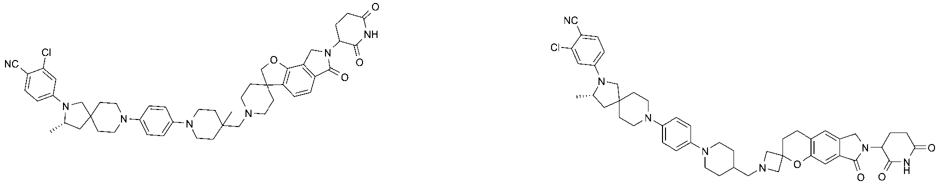

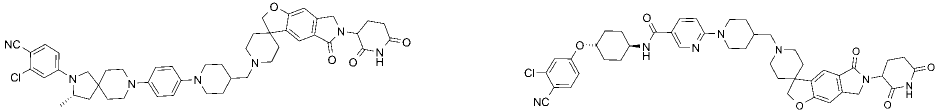

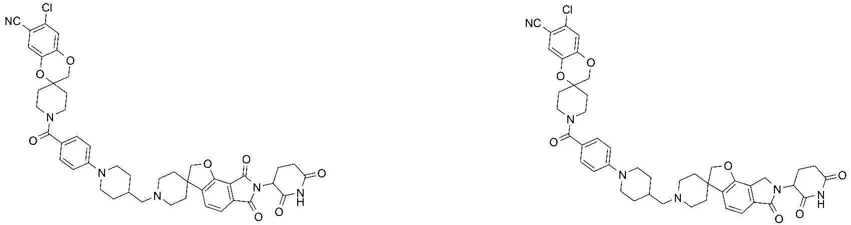

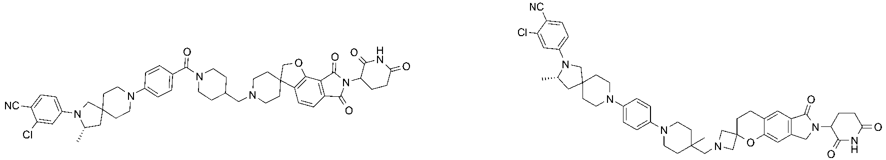

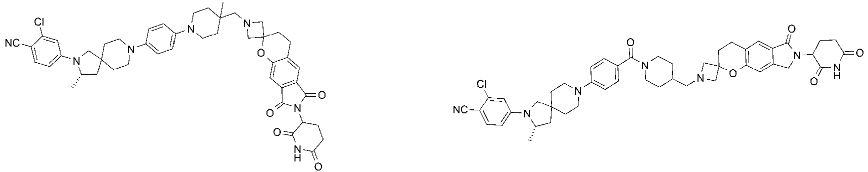

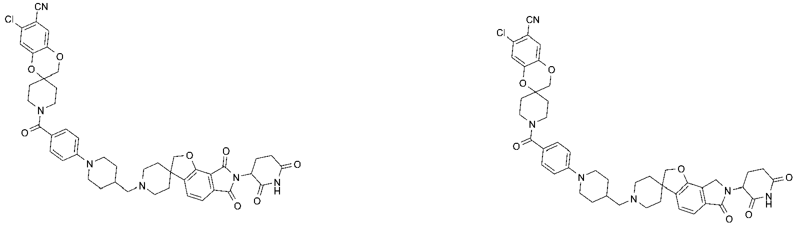

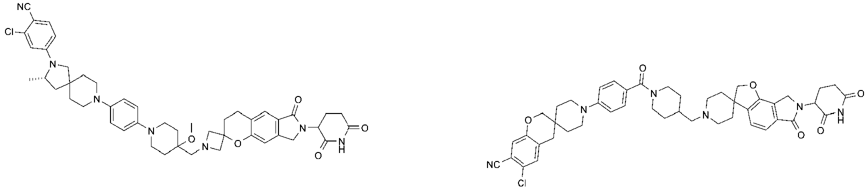

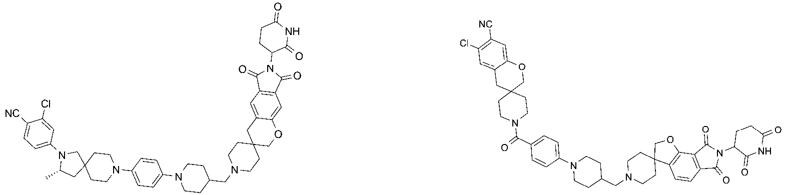

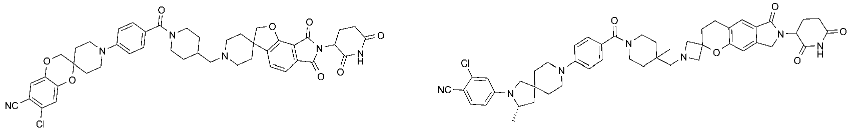

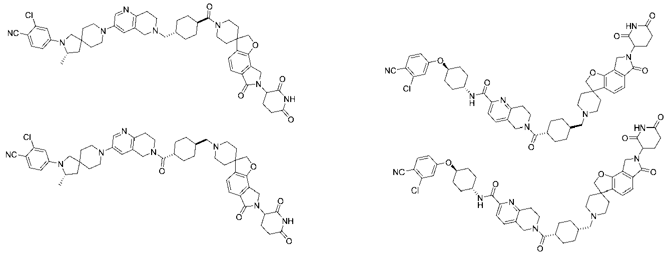

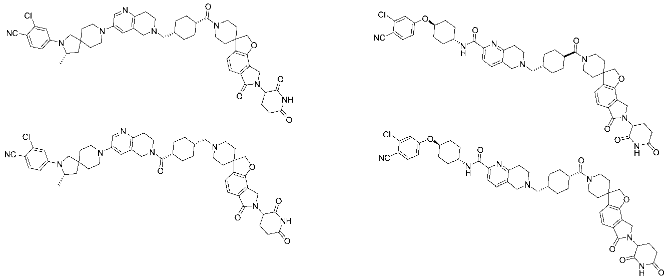

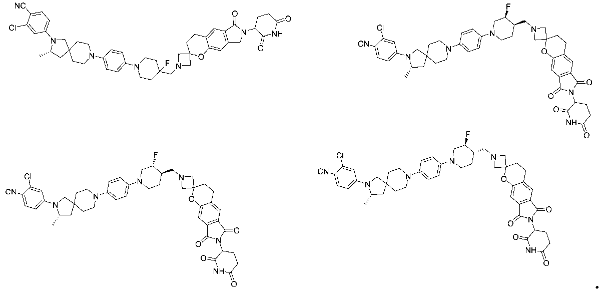

18. A pharmaceutical composition, wherein the pharmaceutical composition comprises the compound according to any one of claims 1-17.

19. The compound according to any one of claims 1-7, or the pharmaceutical composition according to claim 18 for use as a medicament.

20. The compound according to any one of claims 1-17, or the pharmaceutical composition according to claim 18 for use in the treatment of prostate cancer.

21. Use of the compound according to any one of claims 1-17, or the pharmaceutical composition according to claim 18, in the manufacture of a medicament for use in the treatment of cancer; preferably, the cancer is prostate cancer.

22. A method of treating or preventing cancer, comprising administering to a subject in needthereof a therapeutically effective amount of the compound according to any one of claims 1-17, or the pharmaceutical composition according to claim 18; preferably, the cancer is prostate cancer.
